(19) Europäisches Patentamt
European Patent Office
Office européen des brevets

(11) EP 4 778 922 A1

(12) **EUROPEAN PATENT APPLICATION**
published in accordance with Art. 153(4) EPC

(43) Date of publication:
**22.07.2026 Bulletin 2026/30**

(21) Application number: **24864783.6**

(22) Date of filing: **14.09.2024**

(51) International Patent Classification (IPC):
***C07D 403/02*** (2006.01) ***C07D 205/02*** (2006.01)
***C07D 239/38*** (2006.01) ***C07D 239/42*** (2006.01)
***C07D 239/34*** (2006.01) ***A61P 35/00*** (2006.01)
***A61K 31/506*** (2006.01)

(52) Cooperative Patent Classification (CPC):
**A61K 31/506; A61P 35/00; C07D 205/02; C07D 239/34; C07D 239/38; C07D 239/42; C07D 403/02**

(86) International application number:
**PCT/CN2024/119149**

(87) International publication number:
**WO 2025/056068 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**15.09.2023 CN 202311201930**
**17.11.2023 CN 202311547952**
**24.11.2023 CN 202311588615**
**11.12.2023 CN 202311698673**
**01.03.2024 CN 202410239612**
**30.04.2024 CN 202410546567**
**06.06.2024 CN 202410734815**
**30.07.2024 CN 202411040155**
**04.09.2024 CN 202411238360**

(71) Applicant: **Shanghai Jeyou Pharmaceutical Co., Ltd.**
**Shanghai 201315 (CN)**

(72) Inventors:
- **DENG, Gang**
  **Shanghai 201315 (CN)**
- **HUANG, Huoming**
  **Shanghai 201315 (CN)**
- **LIN, Xianfeng**
  **Shanghai 201315 (CN)**
- **PENG, Jianbiao**
  **Shanghai 201315 (CN)**

(74) Representative: **IK-IP LTD**
**8 Devonshire Square**
**London EC2M 4YJ (GB)**

(54) **BICYCLIC COMPOUND, AND PREPARATION METHOD THEREFOR AND PHARMACEUTICAL USE THEREOF**

(57) Disclosed in the present invention are a bicyclic compound, and a preparation method therefor and the pharmaceutical use thereof. Specifically, disclosed in the present invention are a compound as represented by formula (I), an optical isomer thereof or a pharmaceutically acceptable salt thereof, and the use thereof such as serving as an androgen receptor (AR) inhibitor.

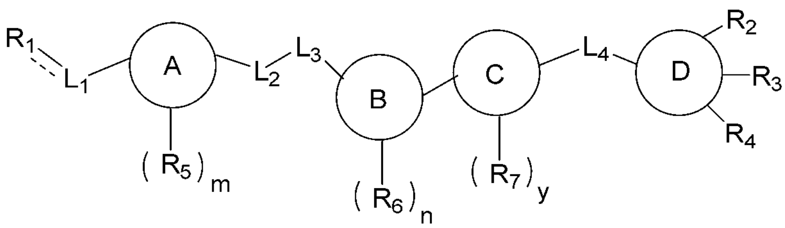

(I)

EP 4 778 922 A1

## Description

The present application claims priority to the following applications:

1) Application No. CN 202311201930.9, filed on September 15, 2023;
2) Application No. CN 202311547952.0, filed on November 17, 2023;
3) Application No. CN 202311588615.6, filed on November 24, 2023;
4) Application No. CN 202311698673.4, filed on December 11, 2023;
5) Application No. CN 202410239612.X, filed on March 01, 2024;
6) Application No. CN 202410546567.2, filed on April 30, 2024;
7) Application No. CN 202410734815.6, filed on June 06, 2024;
8) Application No. CN 202411040155.8, filed on July 30, 2024;
9) Application No. CN 202411238360.5, filed on September 04, 2024.

## TECHNICAL FIELD

The present disclosure belongs to the field of medicinal chemistry. Specifically, the present disclosure relates to a class of bicyclic compounds, preparation methods therefor, and pharmaceutical uses thereof.

## BACKGROUND

Prostate cancer is an androgen-dependent tumor. Androgens can bind to androgen receptors (AR), thereby stimulating the growth of prostate cancer cells and the progression of the disease. Endocrine therapy is one of the conventional treatment methods. For example, the standard treatment for advanced PCa is primarily androgen deprivation therapy (ADT), such as surgical castration (bilateral orchiectomy) or medical castration (e.g., injection of Zoladex). ADT therapy shows significant efficacy in the early stages of treatment. However, as the disease progresses, mutations occur in the AR. The mutated AR becomes more sensitive to low levels of androgens, thereby driving the disease to progress to castration-resistant prostate cancer (CRPC). Almost all patients with advanced prostate cancer will eventually progress to CRPC after receiving endocrine therapy. Furthermore, up to 30% of prostate cancer patients will develop metastatic castration-resistant prostate cancer (mCRPC) within 10 years of initial treatment.

Currently, there are several oral drugs available for the treatment of mCRPC, such as enzalutamide, apalutamide, and darolutamide, which primarily exert their therapeutic effects by binding to the ligand-binding domain of the androgen receptor (AR-LBD), thereby blocking the interaction between AR and DNA. However, after 2 to 3 years of treatment with these drugs, patients are prone to developing drug resistance. The emergence of androgen receptor variants (AR-Vs) lacking the LBD region and mutations in the LBD region are two important mechanisms of drug resistance.

Compared to the normal full-length AR, AR-Vs are truncated forms of AR. These splice variants lack the LBD during their formation, which prevents androgens from binding to AR-Vs. Nevertheless, since AR-Vs retain the N-terminal domain and DNA-binding domain (DBD), they can still bind to genomic DNA and regulate the expression of downstream target genes, exhibiting androgen-independent constitutive activity. This is one of the key mechanisms underlying ADT resistance and CRPC disease progression.

The development of inhibitors targeting the N-terminal domain (NTD) of AR with favorable physicochemical properties and druggability, which suppress the transcriptional function of AR by regulating the N-terminal domain of AR and block androgen receptor signaling pathways, represents a new direction in prostate cancer therapy research. Inhibitors targeting AR-NTD, by acting on the N-terminal domain of AR, will provide profound and broad AR inhibition. This has significant clinical value for the treatment of AR-driven cancers such as prostate cancer, particularly for the treatment of androgen-independent, drug-resistant prostate cancer.

## SUMMARY

In one aspect of the present disclosure, the present disclosure provides a compound of formula (I), an optical isomer thereof, and a pharmaceutically acceptable salt thereof,

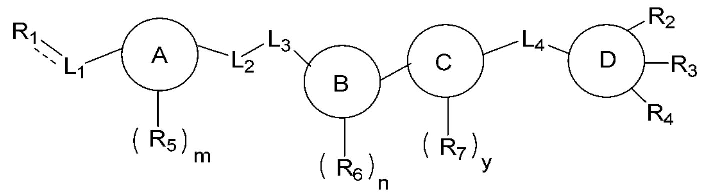

( I )

wherein

$R_1$ is selected from the group consisting of H, halogen, OH, CN, $NH_2$, $H_2N$-S(=O)$_2$-, $C_{1-6}$ alkyl, $C_{2-6}$ alkenyl, $C_{2-6}$ alkynyl, $C_{3-6}$ cycloalkyl, $C_{3-6}$ cycloalkenyl, $C_{3-6}$ cycloalkyl-S(=O)$_2$-, $C_{3-6}$ cycloalkyl-S(=O)(=NH)-, $C_{6-10}$ aryl, 5- to 10-membered heteroaryl, $C_{1-6}$ heteroalkyl, and 3- to 10-membered heterocycloalkyl, wherein the $C_{1-6}$ alkyl, $C_{2-6}$ alkenyl, $C_{2-6}$ alkynyl, $C_{3-6}$ cycloalkyl, $C_{3-6}$ cycloalkenyl, $C_{3-6}$ cycloalkyl-S(=O)$_2$-, $C_{3-6}$ cycloalkyl-S(=O)(=NH)-, $C_{6-10}$ aryl, 5- to 10-membered heteroaryl, $C_{1-6}$ heteroalkyl, and 3- to 10-membered heterocycloalkyl are each optionally substituted with 1, 2, or 3 R;

$R_2$, $R_3$, and $R_4$ are each independently selected from the group consisting of H, CN, F, Cl, Br, OH, $NH_2$, $C_{1-6}$ alkyl, $C_{3-6}$ cycloalkyl, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, 4- to 6-membered heterocycloalkyl-O-, 4- to 6-membered heterocycloalkyl-S-, and 4- to 6-membered heterocycloalkyl-NH-, wherein the $C_{1-6}$ alkyl, $C_{3-6}$ cycloalkyl, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, 4- to 6-membered heterocycloalkyl-O-, 4- to 6-membered heterocycloalkyl-S-, and 4- to 6-membered heterocycloalkyl-NH- are each optionally substituted with 1, 2, or 3 R;

$R_5$, $R_6$, and $R_7$ are each independently selected from the group consisting of H, CN, F, Cl, Br, OH, $NH_2$, C(=O)OH, C(=O)$OCH_3$, -C(=O)$NH_2$, -C(=O)$NHCH_3$, -C(=O)N$(CH_3)_2$, $C_{1-6}$ alkyl, $C_{3-6}$ cycloalkyl, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, 4- to 6-membered heterocycloalkyl-O-, 4- to 6-membered heterocycloalkyl-S-, 4- to 6-membered heterocycloalkyl-NH-, $C_{6-10}$ aryl, 5- to 10-membered heteroaryl, and 3- to 10-membered heterocycloalkyl, wherein the C(=O)$OCH_3$, -C(=O)$NH_2$, -C(=O)$NHCH_3$, -C(=O)N$(CH_3)_2$, $C_{1-6}$ alkyl, $C_{3-6}$ cycloalkyl, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, 4- to 6-membered heterocycloalkyl-O-, 4- to 6-membered heterocycloalkyl-S-, 4- to 6-membered heterocycloalkyl-NH-, $C_{6-10}$ aryl, 5- to 10-membered heteroaryl, and 3- to 10-membered heterocycloalkyl are each optionally substituted with 1, 2, or 3 R;

m, n, and y are each independently selected from the group consisting of 0, 1, 2, 3, and 4;

$L_1$ is selected from the group consisting of a single bond, -NH-, =N-, -O-, -C=C-, -S-, -C(=O)-, -S(=O)-, -S(=O)$_2$-, -$CH_2$-, -$CH_2CH_2$-, -$OCH_2$-, $C_{3-6}$ cycloalkyl, and 3- to 10-membered heterocycloalkyl, wherein the -NH-, -$CH_2$-, -$CH_2CH_2$-, -$OCH_2$-, $C_{3-6}$ cycloalkyl, and 3- to 10-membered heterocycloalkyl are each optionally substituted with 1 or 2 R;

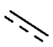 is selected from the group consisting of 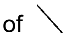 and 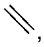, and when 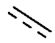 is 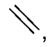, $L_1$ is selected from the group consisting of =N- and 3- to 10-membered heterocycloalkyl;

$L_2$ is selected from the group consisting of a single bond, -C≡C-, -CH=CH-, - $(CR_8R_9)$x-, -O-, -S-, -C(=O)-, -S(=O)-, -S(=O)$_2$-, -$NR_{10}$-, -$C_{1-3}$ alkyl-O-, $C_{3-6}$ cycloalkyl, 4- to 10-membered heterocycloalkyl, $C_{6-10}$ aryl, and 5- to 10-membered heteroaryl, wherein the -$C_{1-3}$ alkyl-O-, $C_{3-6}$ cycloalkyl, 4- to 10-membered heterocycloalkyl, $C_{6-10}$ aryl, and 5- to 10-membered heteroaryl are each optionally substituted with 1, 2, or 3 R;

$L_3$ is selected from the group consisting of a single bond, -C≡C-, -CH=CH-, - $(CR_8R_9)$x-, -O-, -S-, -C(=O)-, -S(=O)-, -S(=O)$_2$-, -$NR_{10}$-, and -$C_{1-3}$ alkyl-O-, wherein the -$C_{1-3}$ alkyl-O- is optionally substituted with 1, 2, or 3 R;

and, $L_2$ and $L_3$ are not simultaneously a single bond;

and, when $L_2$ is selected from the group consisting of $C_{3-6}$ cycloalkyl, 4- to 10-membered heterocycloalkyl, $C_{6-10}$ aryl, and 5- to 10-membered heteroaryl, $L_3$ is not a single bond;

$L_4$ is selected from the group consisting of -$(CR_8R_9)$x-, -O-, -S-, -C(=O)-, -S(=O)-, - S(=O)$_2$-, and -$NR_{10}$-;

$R_8$ and $R_9$ are each independently selected from the group consisting of H, CN, F, Cl, Br, OH, $NH_2$, $C_{1-6}$ alkyl, $C_{3-6}$ cycloalkyl, $C_{6-10}$ aryl, 5- to 10-membered heteroaryl, $C_{1-6}$ heteroalkyl, and 3- to 10-membered heterocycloalkyl, wherein the $C_{1-6}$ alkyl, $C_{3-6}$ cycloalkyl, $C_{6-10}$ aryl, 5- to 10-membered heteroaryl, $C_{1-6}$ heteroalkyl, and 3- to 10-membered heterocycloalkyl are each optionally substituted with 1, 2, or 3 R;

$R_{10}$ is selected from the group consisting of H, $C_{1-6}$ alkyl, $C_{3-6}$ cycloalkyl, $C_{6-10}$ aryl, 5- to 10-membered heteroaryl, $C_{1-6}$ heteroalkyl, and 3- to 10-membered heterocycloalkyl, wherein the $C_{1-6}$ alkyl, $C_{3-6}$ cycloalkyl, $C_{6-10}$ aryl, 5- to 10-membered heteroaryl, $C_{1-6}$ heteroalkyl, and 3- to 10-membered heterocycloalkyl are each optionally substituted with 1, 2, or 3 R;

x is selected from the group consisting of 1, 2, and 3;
ring A is selected from the group consisting of $C_{4-10}$ cycloalkyl, 4- to 10-membered heterocycloalkyl, phenyl, and 5- to 10-membered heteroaryl;
ring B, ring C, and ring D are each independently selected from the group consisting of $C_{4-10}$ cycloalkyl, 4- to 10-membered heterocycloalkyl, phenyl, 5- to 10-membered heteroaryl, benzo-fused $C_{5-6}$ cycloalkyl, benzo-fused 5- to 7-membered heterocycloalkyl, 5- to 6-membered heteroaryl-fused $C_{5-6}$ cycloalkyl, and 5- to 6-membered heteroaryl-fused 5- to 6-membered heterocycloalkyl;
each R is independently selected from the group consisting of H, halogen, =O, =NR', OH, $NH_2$, CN,

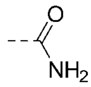,

$C_6$ alkyl, $C_{3-6}$ cycloalkyl, 3- to 6-membered heterocycloalkyl, $C_{1-6}$ alkyl-S(=O)$_2$-, ($C_{1-6}$ alkyl)$_2$-P(=O)-, $C_{1-6}$ alkyl-C(=O)-, $C_{1-6}$ alkyl-C(=O)O-, $C_{1-6}$ alkyl-O-C(=O)-, $C_{1-6}$ alkyl-C(=O)NH-, $C_{1-6}$ alkyl-NH-C(=O)-, $C_{1-6}$ alkyl-S(=O)NH-, $C_{1-6}$ alkyl-S(=O)$_2$NH-, $C_{1-6}$ alkyl-NHS(=O)$_2$-, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, $C_{3-6}$ cycloalkyl-C(=O)-, 4- to 6-membered heterocycloalkyl-O-, 4- to 6-membered heterocycloalkyl-S-, 4- to 6-membered heterocycloalkyl-NH-, and $C_{6-10}$ aryl-$C_{1-6}$ alkyl-, wherein the $C_{1-6}$ alkyl, $C_{3-6}$ cycloalkyl, 3- to 6-membered heterocycloalkyl, $C_{1-6}$ alkyl-S(=O)$_2$-, ($C_{1-6}$ alkyl)$_2$-P(=O)-, $C_{1-6}$ alkyl-C(=O)-, $C_{1-6}$ alkyl-C(=O)O-, $C_{1-6}$ alkyl-O-C(=O)-, $C_{1-6}$ alkyl-C(=O)NH-, $C_{1-6}$ alkyl-NH-C(=O)-, $C_{1-6}$ alkyl-S(=O)NH-, $C_{1-6}$ alkyl-S(=O)$_2$NH-, $C_{1-6}$ alkyl-NHS(=O)$_2$-, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, $C_{3-6}$ cycloalkyl-C(=O)-, 4- to 6-membered heterocycloalkyl-O-, 4- to 6-membered heterocycloalkyl-S-, 4- to 6-membered heterocycloalkyl-NH-, and $C_{6-10}$ aryl-$C_{1-6}$ alkyl- are each optionally substituted with 1, 2, or 3 R';
R' is selected from the group consisting of H, F, Cl, Br, I, OH, $NH_2$, CN,

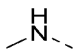, 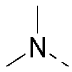,

$CH_3$, $CH_2F$, $CHF_2$, $CF_3$, and $C_{1-6}$ alkyl-S(=O)$_2$-;
the heteroaryl, heteroalkyl, or heterocycloalkyl comprises 1, 2, or 3 heteroatoms or heteroatomic groups independently selected from the group consisting of O, NH, S, C(=O), C(=O)O, C(=O)NH, S(=O), S(=O)$_2$, P(=O), S(=O)$_2$NH, and N.

In some embodiments of the present disclosure, each R is independently selected from the group consisting of H, halogen, OH, $NH_2$, CN,

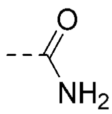

, =O, =NR', $C_{1-3}$ alkyl, $C_{3-6}$ cycloalkyl, 3- to 6-membered heterocycloalkyl, $C_{1-3}$ alkyl-C(=O)-, $C_{1-3}$ alkyl-S(=O)$_2$-, ($C_{1-3}$ alkyl)$_2$-P(=O)-, $C_{1-3}$ alkyl-C(=O)O-, $C_{1-3}$ alkyl-O-C(=O)-, $C_{1-3}$ alkyl-C(=O)NH-, $C_{1-3}$ alkyl-NH-C(=O)-, $C_{1-3}$ alkyl-S(=O)NH-, $C_{1-3}$ alkyl-S(=O)$_2$NH-, $C_{1-3}$ alkyl-NHS(=O)$_2$-, $C_{1-3}$ alkyl-O-, $C_{1-3}$ alkyl-S-, $C_{1-3}$ alkyl-NH-, $C_{3-6}$ cycloalkyl-C(=O)-, 4- to 6-membered heterocycloalkyl-O-, 4- to 6-membered heterocycloalkyl-S-, 4- to 6-membered heterocycloalkyl-NH-, and $C_{6-10}$ aryl-$C_{1-3}$ alkyl-, wherein the $C_{1-3}$ alkyl, $C_{3-6}$ cycloalkyl, 3- to 6-membered heterocycloalkyl, $C_{1-3}$ alkyl-C(=O)-, $C_{1-3}$ alkyl-S(=O)$_2$-, $C_{1-3}$ alkyl-C(=O)O-, $C_{1-3}$ alkyl-O-C(=O)-, $C_{1-3}$ alkyl-C(=O)NH-, $C_{1-3}$ alkyl-NH-C(=O)-, $C_{1-3}$ alkyl-S(=O)NH-, $C_{1-3}$ alkyl-S(=O)$_2$NH-, $C_{1-3}$ alkyl-NHS(=O)$_2$-, $C_{1-3}$ alkyl-O-, $C_{1-3}$ alkyl-S-, $C_{1-3}$ alkyl-NH-, $C_{3-6}$ cycloalkyl-C(=O)-, 4- to 6-membered heterocycloalkyl-O-, 4- to 6-membered heterocycloalkyl-S-, 4- to 6-membered heterocycloalkyl-NH-, and $C_{6-10}$ aryl-$C_{1-3}$ alkyl- are each optionally substituted with 1, 2, or 3 R', and the remaining variables are as defined in the present disclosure.

In some embodiments of the present disclosure, each R is independently selected from the group consisting of H, F, Cl, Br, I, OH, $NH_2$, CN, =O, =NH, =N-CN,

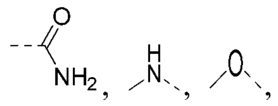

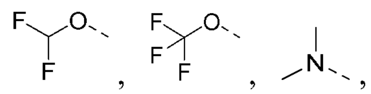

$CH_3$, $CH_2F$, $CHF_2$, $CF_3$,

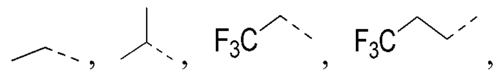

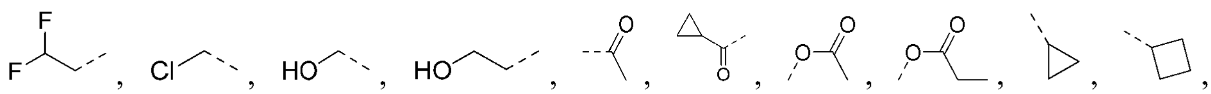

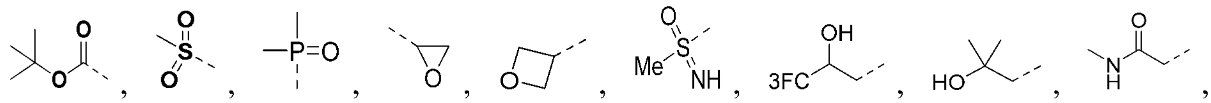

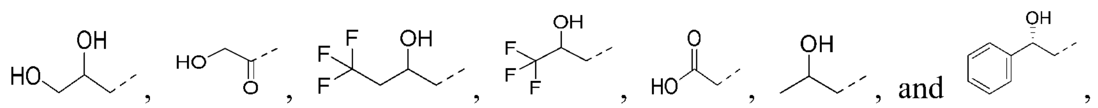

and the remaining variables are as defined in the present disclosure.

In some embodiments of the present disclosure, $R_1$ is selected from the group consisting of H, halogen, OH, CN, $NH_2$, $H_2N$-S(=O)$_2$-, $C_{1-6}$ alkyl, $C_{2-6}$ alkenyl, $C_{2-6}$ alkynyl, $C_{1-6}$ alkyl-O-, $C_{1-6}$ alkyl-S-, $C_{1-6}$ alkyl-S(=O)$_2$-, ($C_{1-6}$ alkyl)$_2$-S(=O)=, ($C_{1-6}$ alkyl)$_2$-P(=O)-, ($C_{1-6}$ alkyl)$_2$-S(=O)=N-, $C_{1-6}$ alkyl-S(=O)$_2$NH-, $C_{1-6}$ alkyl-NHS(=O)$_2$-, $C_{1-6}$ alkyl-NH-, $C_{1-6}$ alkyl-S(=O)(=NH)-, $C_{3-6}$ cycloalkyl, $C_{3-6}$ cycloalkenyl, $C_{3-6}$ cycloalkyl-S(=O)$_2$-, $C_{3-6}$ cycloalkyl-S(=O)(=NH)-, $C_{6-10}$ aryl, 5- to 10-membered heteroaryl, and 3- to 10-membered heterocycloalkyl, wherein the $C_{1-6}$ alkyl, $C_{2-6}$ alkenyl, $C_{2-6}$ alkynyl, $C_{1-6}$ alkyl-O-, $C_{1-6}$ alkyl-S-, $C_{1-6}$ alkyl-S(=O)$_2$-, ($C_{1-6}$ alkyl)$_2$-S(=O)=, ($C_{1-6}$ alkyl)$_2$-P(=O)-, ($C_{1-6}$ alkyl)$_2$-S(=O)=N-, $C_{1-6}$ alkyl-S(=O)$_2$NH-, $C_{1-6}$ alkyl-NHS(=O)$_2$-, $C_{1-6}$ alkyl-NH-, $C_{1-6}$ alkyl-S(=O)(=NH)-, $C_{3-6}$ cycloalkyl, $C_{3-6}$ cycloalkenyl, $C_{3-6}$ cycloalkyl-S(=O)$_2$-, $C_{3-6}$ cycloalkyl-S(=O)(=NH)-, $C_{6-10}$ aryl, 5- to 10-membered heteroaryl, and 3- to 10-membered heterocycloalkyl are each optionally substituted with 1, 2, or 3 R, and the remaining variables are as defined in the present disclosure.

In some embodiments of the present disclosure, $R_1$ is selected from the group consisting of H, F, Cl, Br, I, Me,

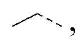

CN, OH, $NH_2$, $H_2N$-S(=O)$_2$-,

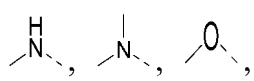

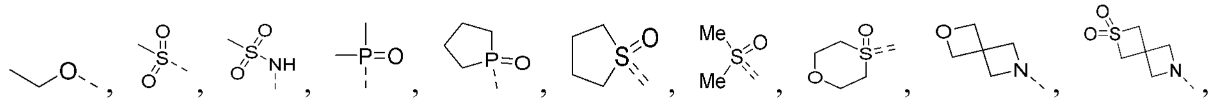

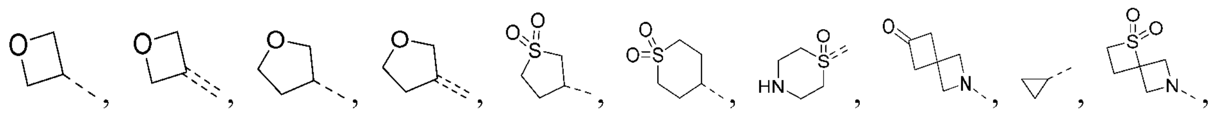

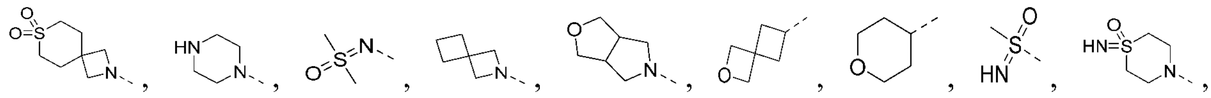

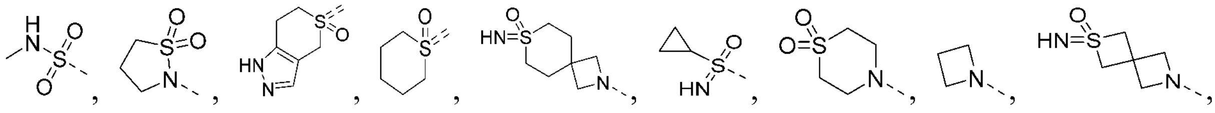

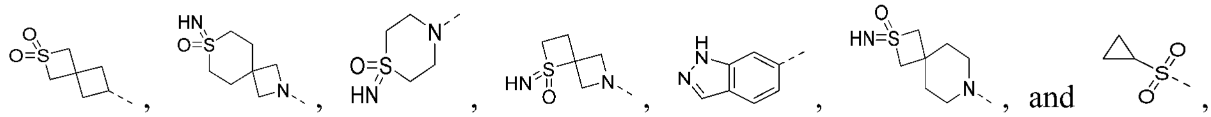
and

wherein the

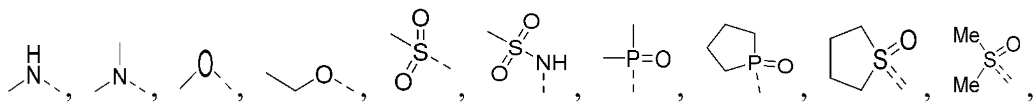

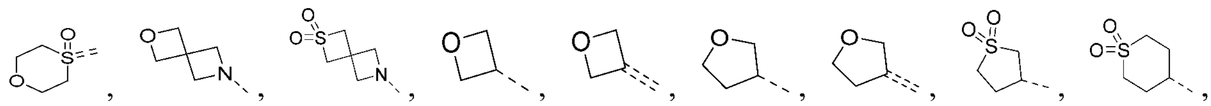

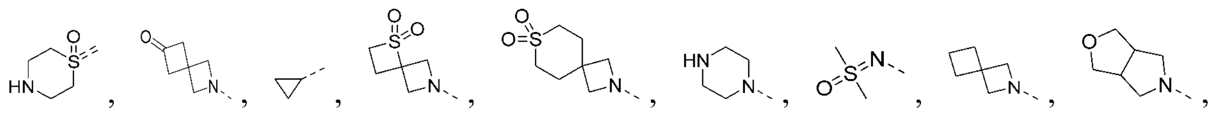

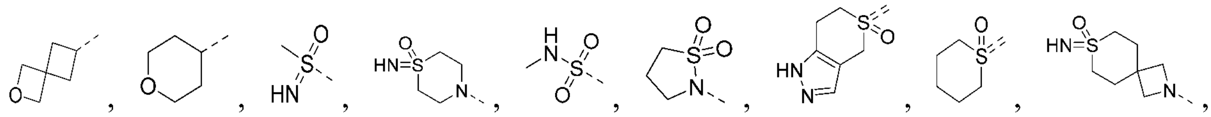

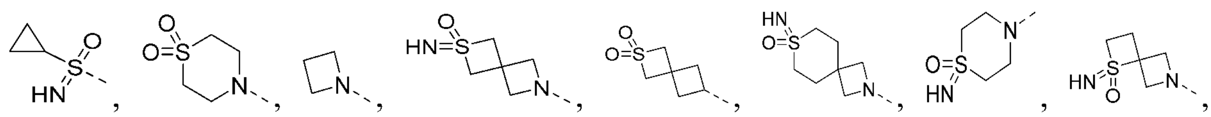

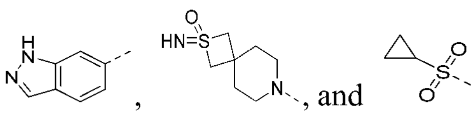
and

are each optionally substituted with 1, 2, or 3 R, and the remaining variables are as defined in the present disclosure.

In some embodiments of the present disclosure, $R_1$ is selected from the group consisting of H, F, Cl, Br, I, Me,

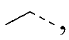

CN, OH, $NH_2$, $H_2N-S(=O)_2-$,

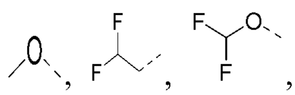

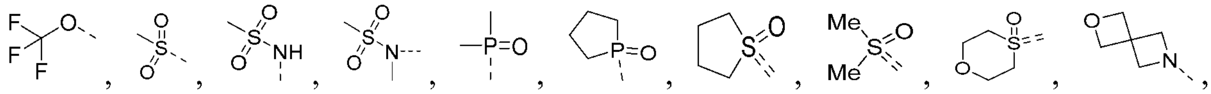
, , , , , , , , , ,

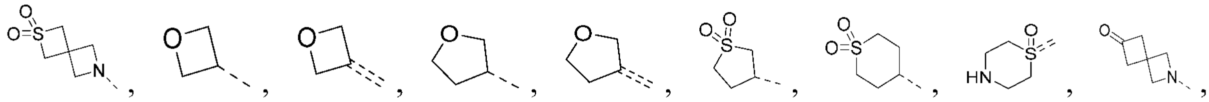
, , , , , , , , ,

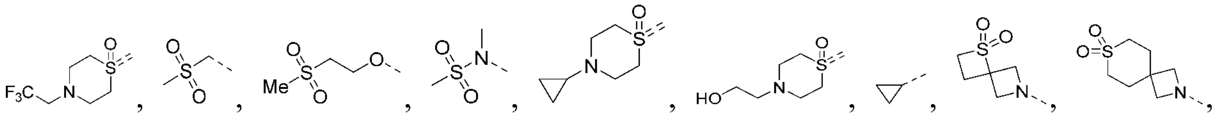
, , , , , , , , ,

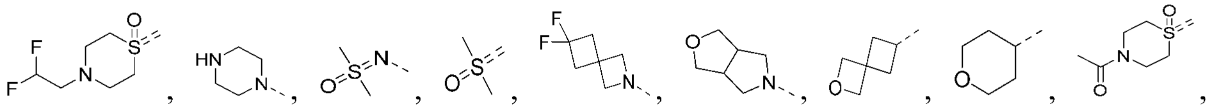
, , , , , , , , , ,

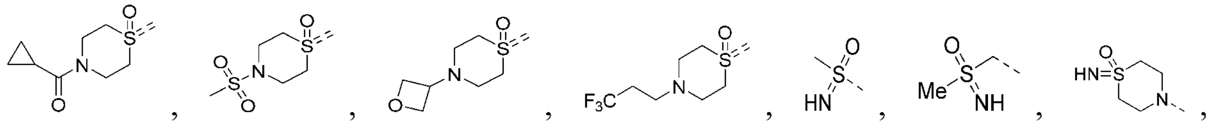
, , , , , , ,

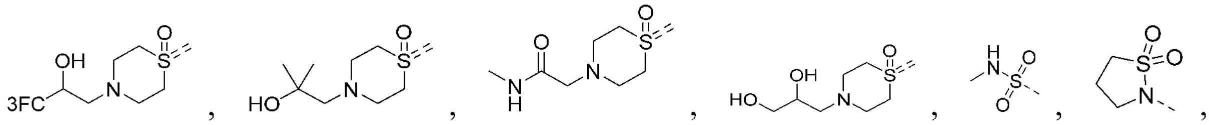
, , , , , ,

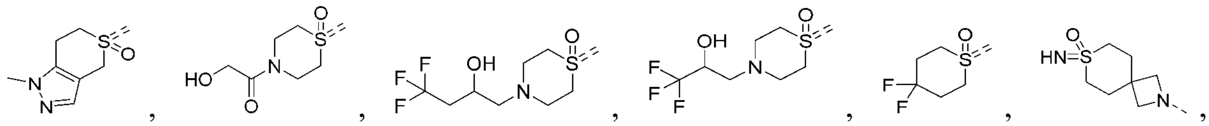
, , , , , ,

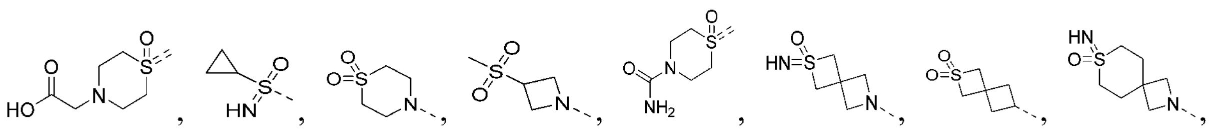
, , , , , , , ,

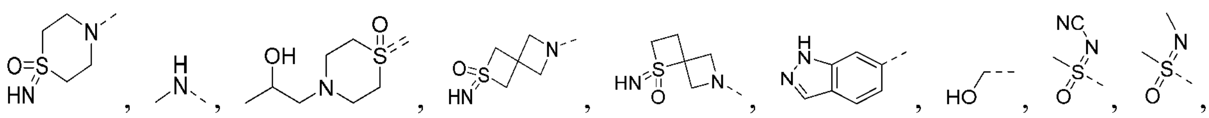
, , , , , , , , ,

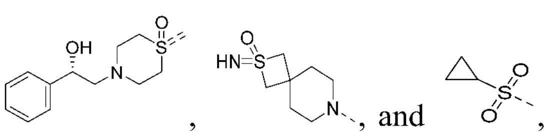
, , and ,

and the remaining variables are as defined in the present disclosure.

In some embodiments of the present disclosure, $L_1$ is selected from the group consisting of a single bond, $-CH_2-$, -NH-, =N-, -O-, -C≡C-, -S-, -C(=O)-, -S(=O)-, $-S(=O)_2-$,

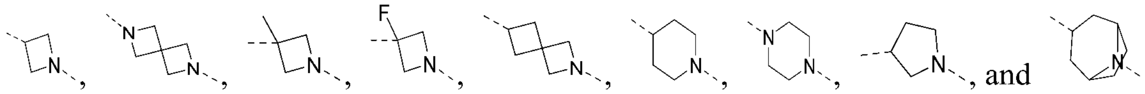
, , , , , , , , and

, and the remaining variables are as defined in the present disclosure.

In some embodiments of the present disclosure, the structural moiety

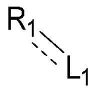

is selected from the group consisting of H, F, Cl, Br, I, $H_2N-S(=O)_2-$,

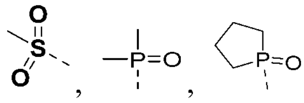 , , ,

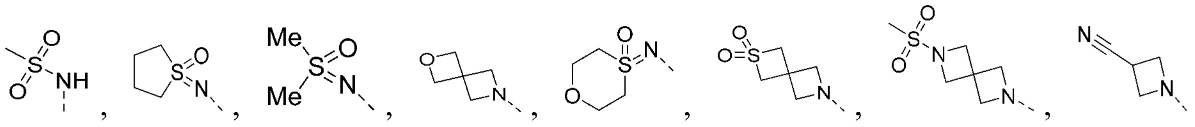 , , , , , , , ,

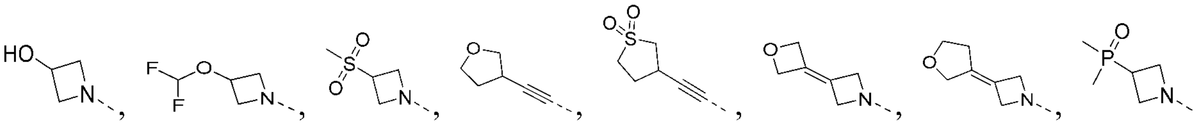 , , , , , , , ,

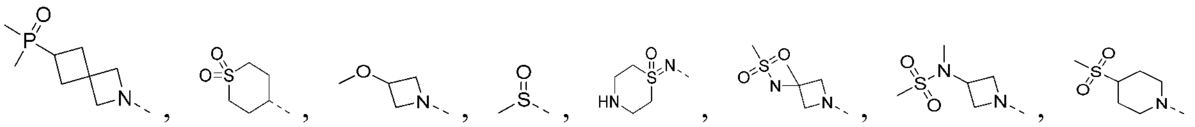 , , , , , , , ,

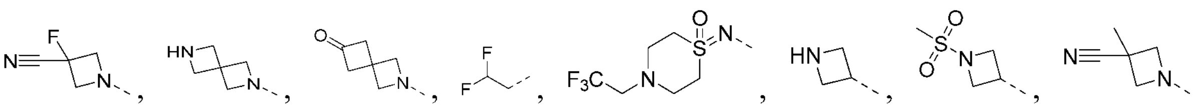 , , , , , , , ,

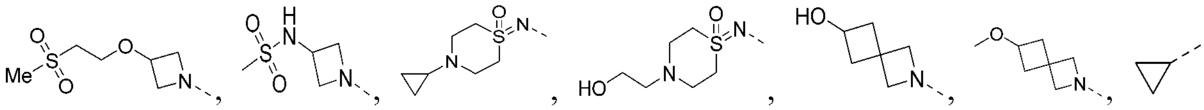 , , , , , , ,

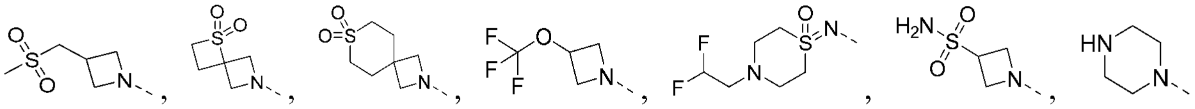 , , , , , , ,

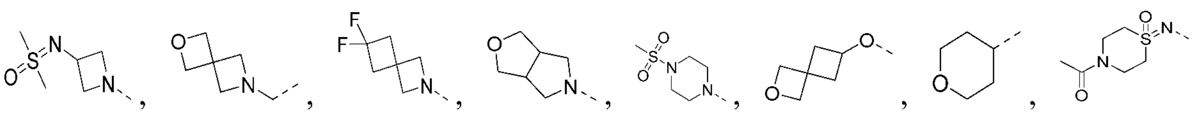 , , , , , , , ,

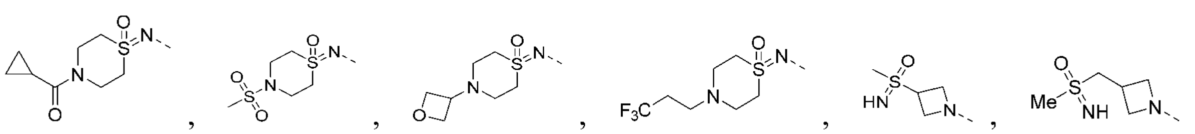 , , , , , ,

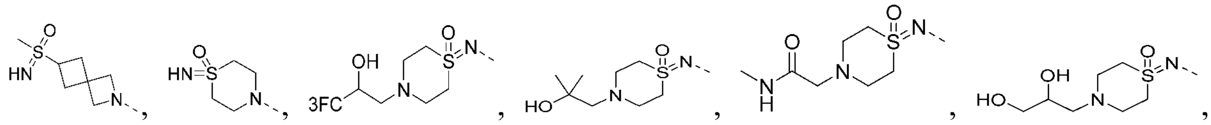

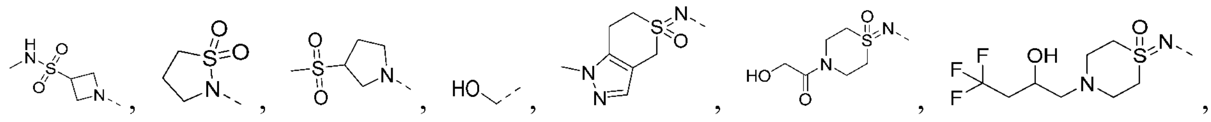

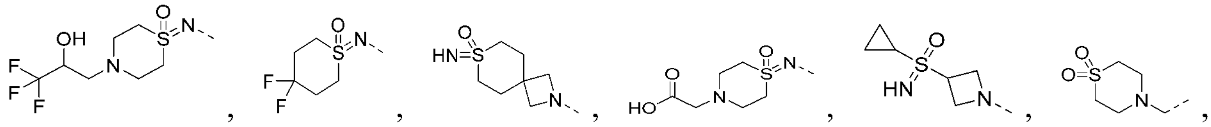

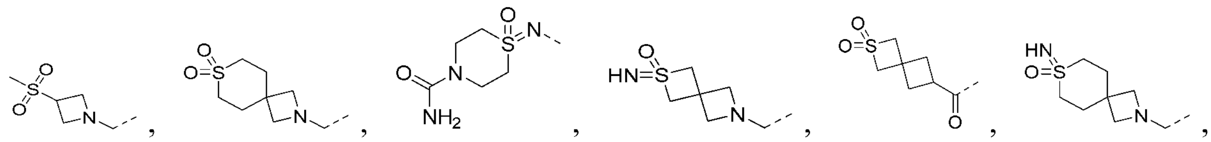

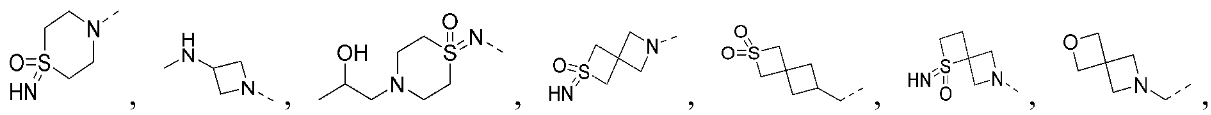

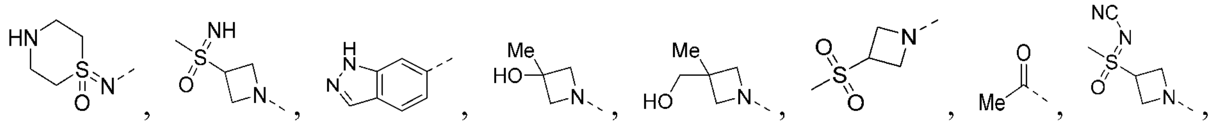

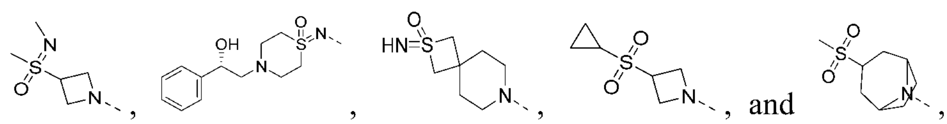

and the remaining variables are as defined in the present disclosure.

In some embodiments of the present disclosure, the structural moiety

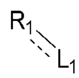

is selected from the group consisting of

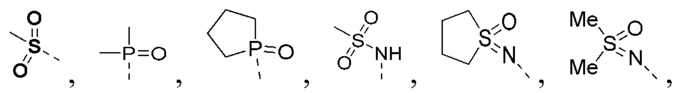

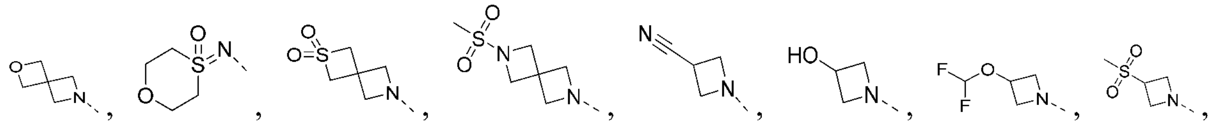

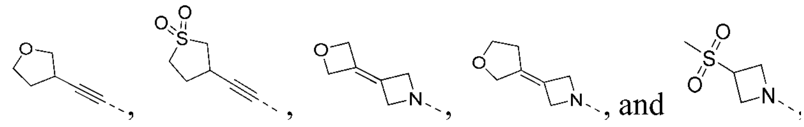

, , , , and

and the remaining variables are as defined in the present disclosure.

In some embodiments of the present disclosure, the structural moiety

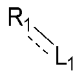

is selected from the group consisting of H, F, Cl, Br, I,

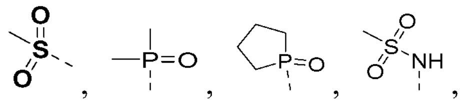

, , , ,

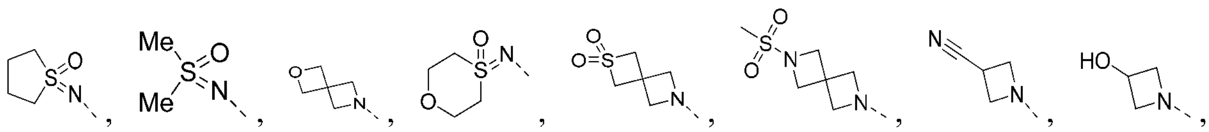

, , , , , , , ,

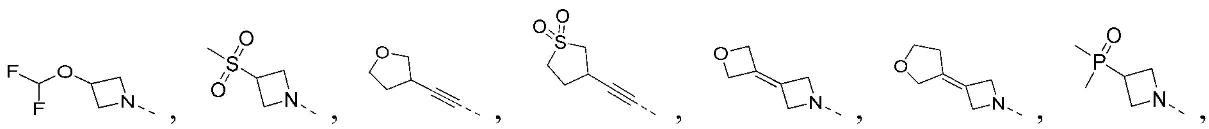

, , , , , , ,

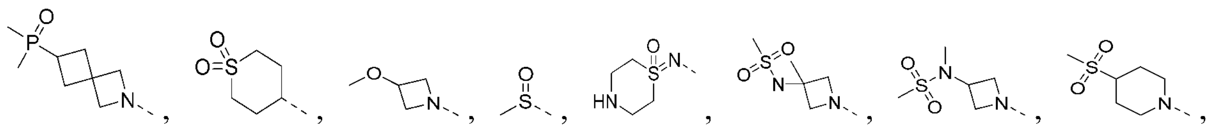

, , , , , , , ,

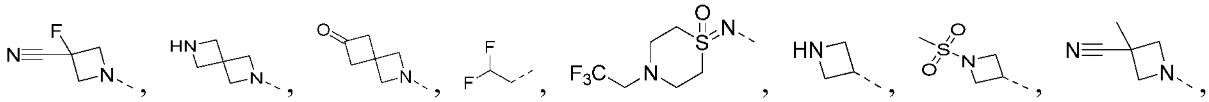

, , , , , , , ,

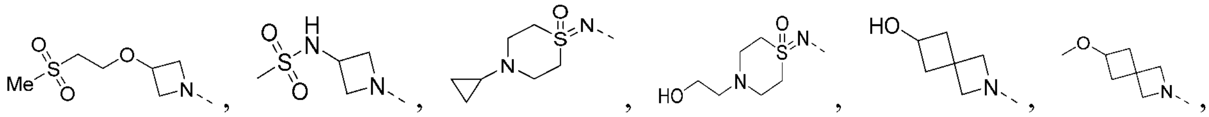

, , , , , ,

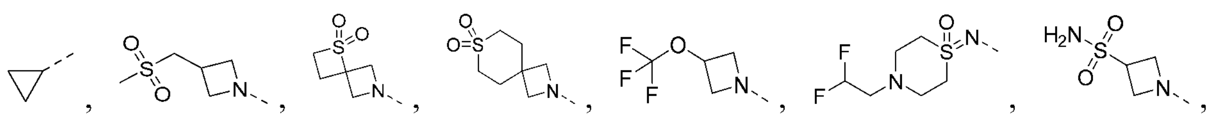

, , , , , , ,

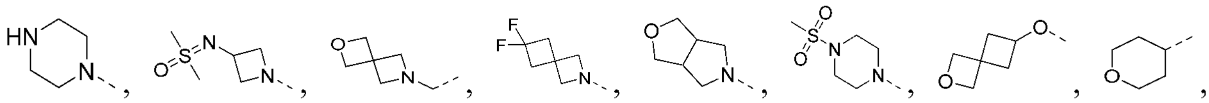

, , , , , , , ,

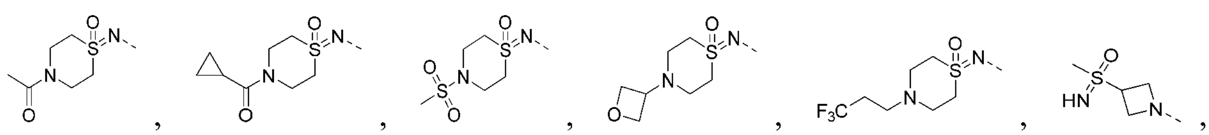

, , , , , ,

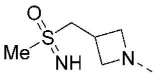, 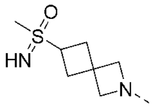, 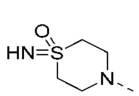, 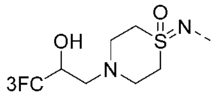, 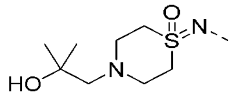, 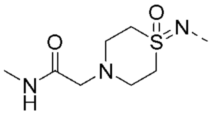, 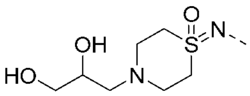, 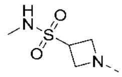, 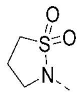, 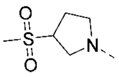, 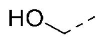, 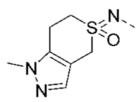, 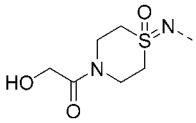, 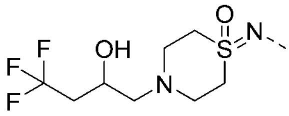, 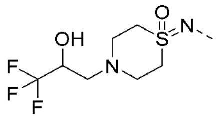, 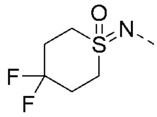, 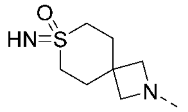, 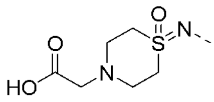, and 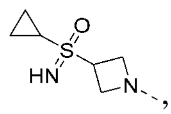,

and the remaining variables are as defined in the present disclosure.

In some embodiments of the present disclosure, the structural moiety

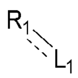

is selected from the group consisting of H, F, Cl, Br, I,

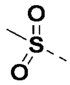, 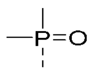, 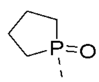, 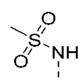, 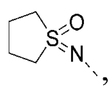, 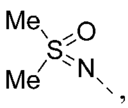, 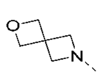, 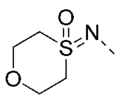, 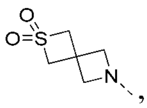, 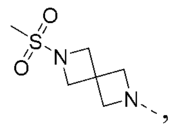, 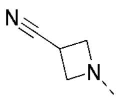, 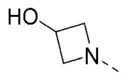, 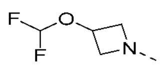, 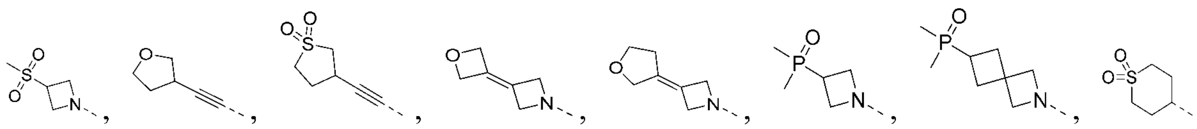, 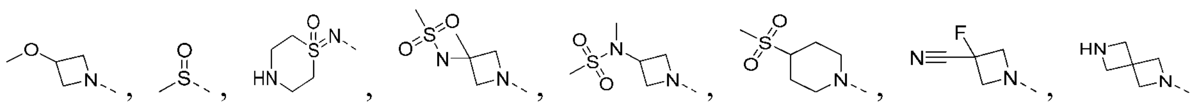, 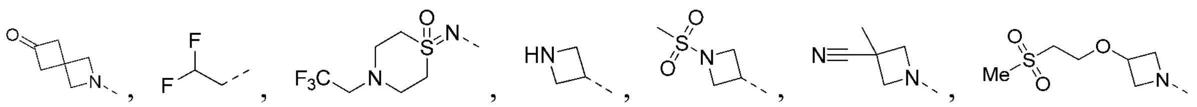, 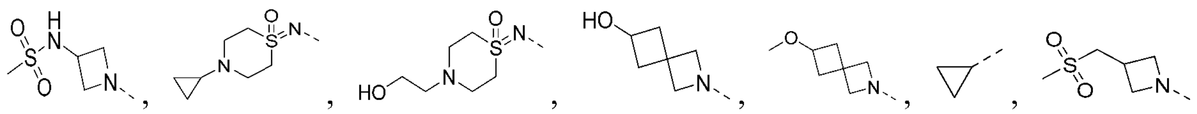,

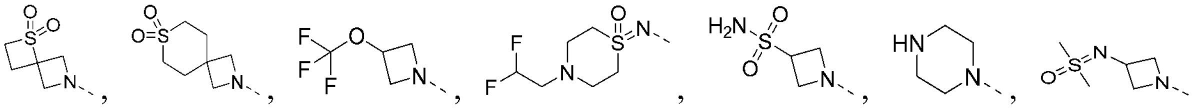,

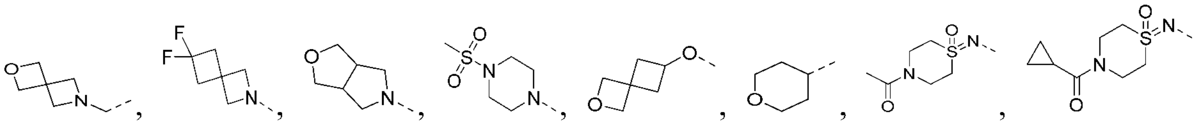,

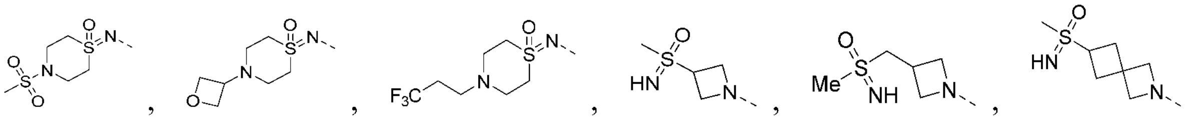,

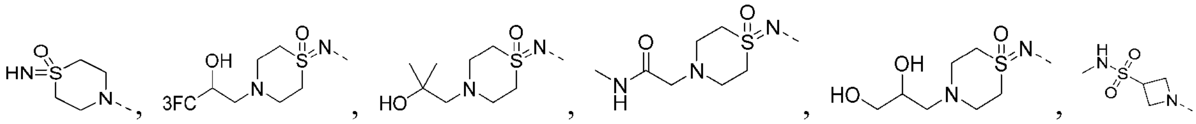,

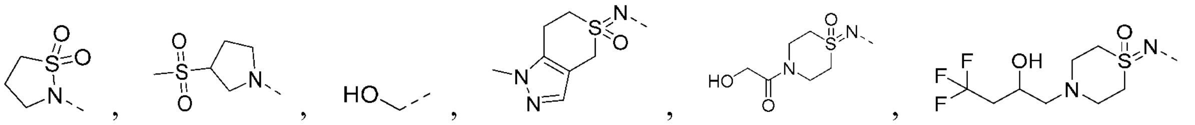,

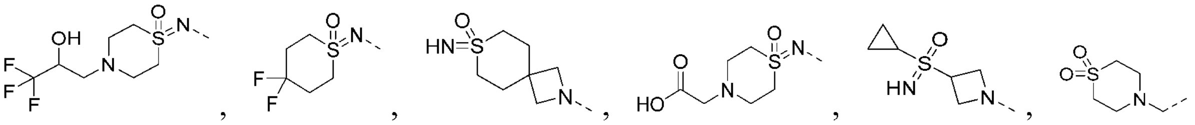,

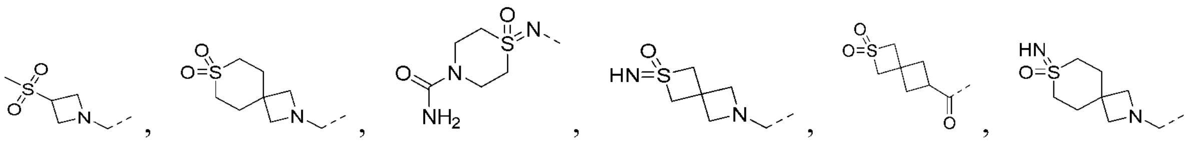,

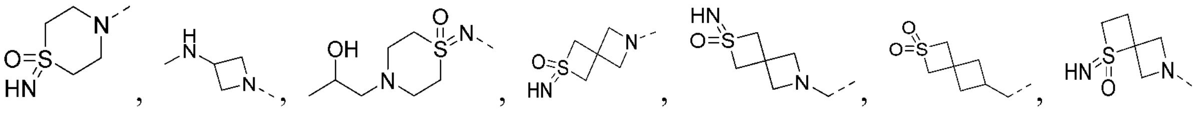,

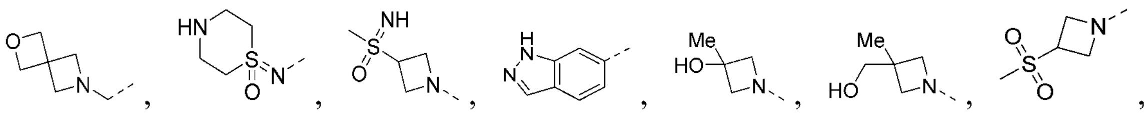,

and

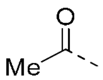,

and the remaining variables are as defined in the present disclosure.

In some embodiments of the present disclosure, $R_2$, $R_3$, and $R_4$ are each independently selected from the group consisting of H, CN, F, Cl, Br, OH, $NH_2$, $C_{1-3}$ alkyl, $C_{1-3}$ alkyl-O-, $C_{1-3}$ alkyl-S-, $C_{1-3}$ alkyl-NH-, $C_{2-3}$ alkenyl-O-, $C_{2-3}$ alkenyl-S-, $C_{2-3}$ alkenyl-NH-, $C_{3-6}$ cycloalkyl-O-, $C_{3-6}$ cycloalkyl-S-, $C_{3-6}$ cycloalkyl-NH-, oxiranyl-O-, and azetidinyl-O-, wherein the $C_{1-3}$ alkyl, $C_{1-3}$ alkyl-O-, $C_{1-3}$ alkyl-S-, $C_{1-3}$ alkyl-NH-, $C_{2-3}$ alkenyl-O-, $C_{2-3}$ alkenyl-S-, $C_{2-3}$ alkenyl-NH-, $C_{3-6}$ cycloalkyl-O-, $C_{3-6}$ cycloalkyl-S-, $C_{3-6}$ cycloalkyl-NH-, oxiranyl-O-, and azetidinyl-O- are each optionally substituted with

1, 2, or 3 R, and the remaining variables are as defined in the present disclosure.

In some embodiments of the present disclosure, $R_2$, $R_3$, and $R_4$ are each independently selected from the group consisting of H, CN, F, Cl, Br, OH, $NH_2$, CN, Me,

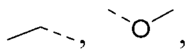

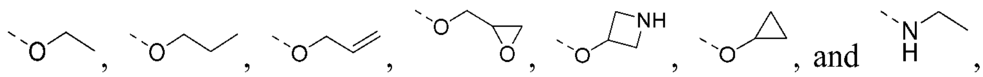

wherein the Me,

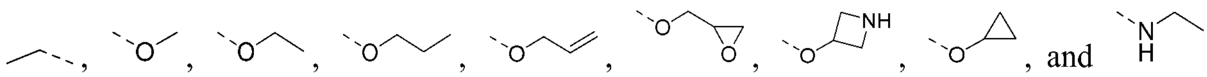

are each optionally substituted with 1, 2, or 3 R, and the remaining variables are as defined in the present disclosure.

In some embodiments of the present disclosure, $R_2$, $R_3$, and $R_4$ are each independently selected from the group consisting of H, CN, F, Cl, Br, OH, $NH_2$, CN, Me,

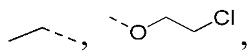

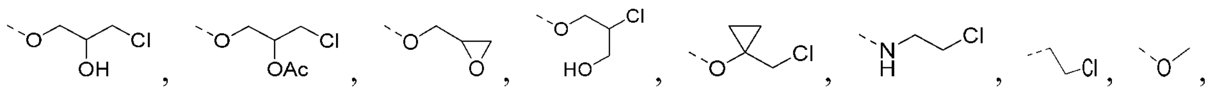

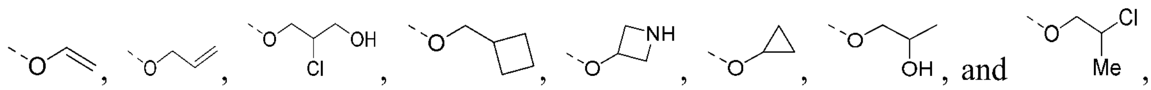

and the remaining variables are as defined in the present disclosure.

In some embodiments of the present disclosure, $R_5$, $R_6$, and $R_7$ are each independently selected from the group consisting of H, CN, F, Cl, Br, OH, $NH_2$, -COOH, $-COOCH_3$, - $C(=O)NH_2$, $-C(=O)NHCH_3$, $-C(=O)N(CH_3)_2$, $C_{1-3}$ alkyl, $C_{1-3}$ alkyl-O-, $C_{1-3}$ alkyl-S-, $C_{1-3}$ alkyl-NH-, $C_{2-3}$ alkenyl-O-, $C_{2-3}$ alkenyl-S-, $C_{2-3}$ alkenyl-NH-, $C_{3-6}$ cycloalkyl-O-, $C_{3-6}$ cycloalkyl-S-, $C_{3-6}$ cycloalkyl-NH-, and oxiranyl-O-, wherein the $C_{1-3}$ alkyl, $C_{1-3}$ alkyl-O-, $C_{1-3}$ alkyl-S-, $C_{1-3}$ alkyl-NH-, $C_{2-3}$ alkenyl-O-, $C_{2-3}$ alkenyl-S-, $C_{2-3}$ alkenyl-NH-, $C_{3-6}$ cycloalkyl-O-, $C_{3-6}$ cycloalkyl-S-, $C_{3-6}$ cycloalkyl-NH-, and oxiranyl-O- are each optionally substituted with 1, 2, or 3 R, and the remaining variables are as defined in the present disclosure.

In some embodiments of the present disclosure, $R_5$, $R_6$, and $R_7$ are each independently selected from the group consisting of H, CN, F, Cl, Br, OH, $NH_2$, CN, Me,

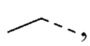

$CH_2F$, $CHF_2$, $CF_3$, COOH, -COOMe,

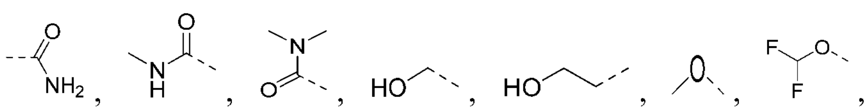

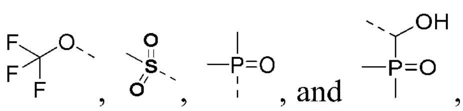

and the remaining variables are as defined in the present disclosure.

In some embodiments of the present disclosure, $R_8$ and $R_9$ are each independently selected from the group consisting of H, CN, F, Cl, Br, OH, $NH_2$, Me, and

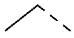,

and the remaining variables are as defined in the present disclosure.

In some embodiments of the present disclosure, $R_{10}$ is selected from the group consisting of H, Me,

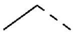, 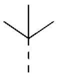, and 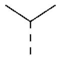

, and the remaining variables are as defined in the present disclosure.

In some embodiments of the present disclosure, $L_2$ is selected from the group consisting of a single bond, $-CH_2-$, $-CH(CH_3)-$, $-OCH_2-$, -C=C-, -CH=CH-, -O-, -S-, -C(=O)-, -S(=O)-, $-S(=O)_2-$, -NH-,

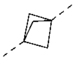, 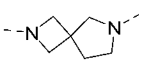, 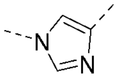, 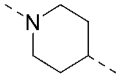, 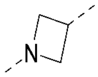, 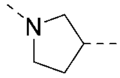,

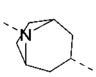, 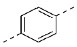, 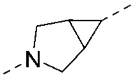, 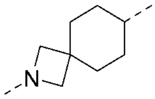, 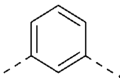, 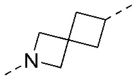, 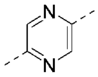, 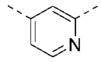, 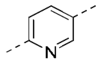,

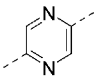, and 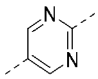

, and the remaining variables are as defined in the present disclosure.

In some embodiments of the present disclosure, $L_3$ is selected from the group consisting of a single bond, $-CH_2-$, $-CH(CH_3)-$, $-OCH_2-$, -C≡C-, -CH=CH-, -O-, -S-, -C(=O)-, -S(=O)-, $-S(=O)_2-$, and -NH-, and the remaining variables are as defined in the present disclosure.

In some embodiments of the present disclosure, $L_2$-$L_3$ is selected from the group consisting of $-CH_2-$, $-CH_2CH_2-$, $-CH(CH_3)-$, -C≡C-, -CH=CH-, -O-, $-OCH_2-$, $-OCH_2CH_2-$, $-OCH(CH_3)-$, $-OCH_2OCH_2-$, $-OCH_2CH_2O-$, -S-, -C(=O)-, -C(=O)O-, -S(=O)-, $-S(=O)_2-$, -NH-, $-CH_2NH-$,

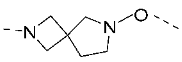, 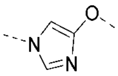, 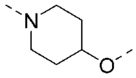, 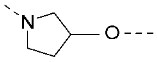, 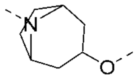, 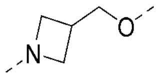,

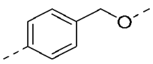, 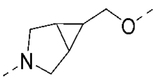, 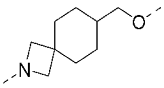, 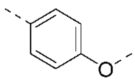, 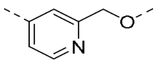, 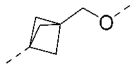,

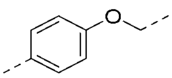, 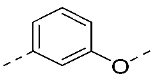, 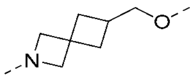, 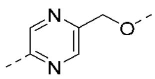, 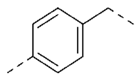, 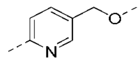,

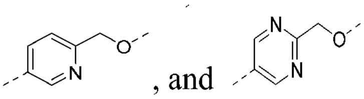, and.

In some embodiments of the present disclosure, $L_4$ is selected from the group consisting of $-CH_2-$, -O-, -S-, -C(=O)-, -S(=O)-, $-S(=O)_2-$, -NH-,

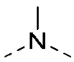, 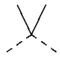, 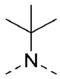, 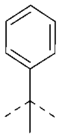,

and

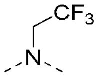,

and the remaining variables are as defined in the present disclosure.

In some embodiments of the present disclosure, ring A is selected from the group consisting of phenyl, pyridinyl, pyrimidinyl, pyrazinyl, thiazolyl, thienyl, oxazolyl, pyridazinyl, cyclobutyl, oxetanyl, tetrahydropyranyl, 2-oxaspiro[3.3]heptanyl, 2,2-dioxo-2-thia-6-azaspiro[3.3]heptyl, tetrahydrofuranyl, azetidinyl, piperidinyl, 1,1-dioxothiomorpholinyl, 2,2-dioxo-2-thia-6-azaspiro[3.3]heptyl, 2-oxo-2-imino-2λ-thiaspiro[3.3]heptyl, cyclohexyl, and 1-oxo-1-imino-1λ6-thiomorpholinyl, and the remaining variables are as defined in the present disclosure.

In some embodiments of the present disclosure, the structural moiety

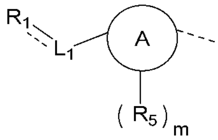

is selected from the group consisting of

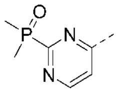, 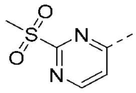,

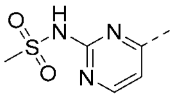, 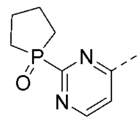, 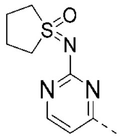, 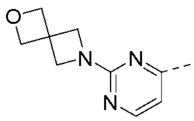, 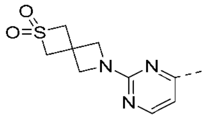, 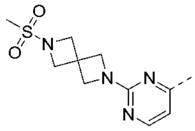,

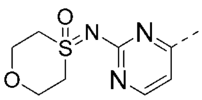, 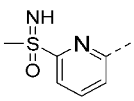, 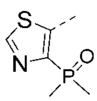, 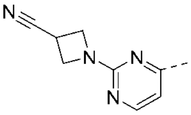, 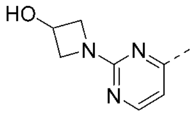, 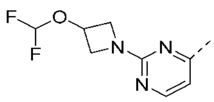,

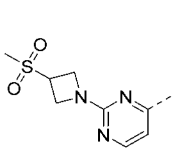, 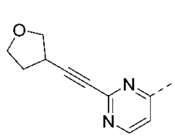, 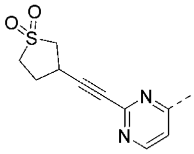, 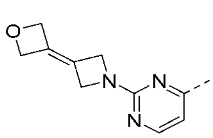, 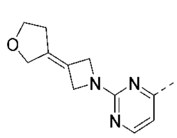,

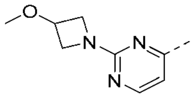, 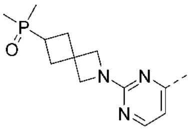, 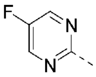, 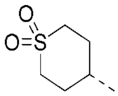, 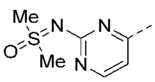, 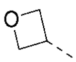, 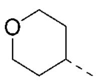, 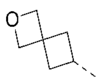,

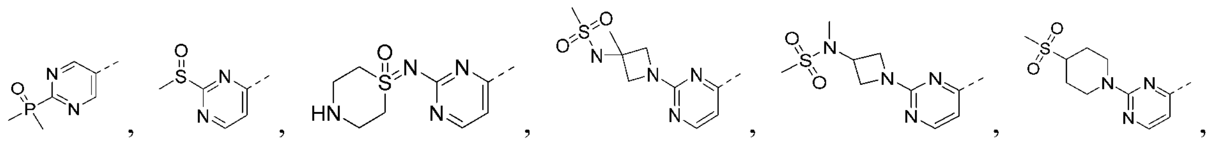 , , , , , ,

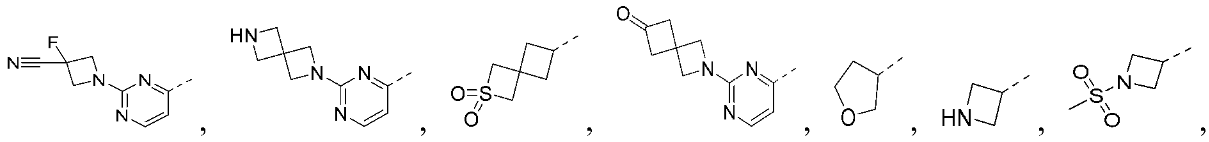 , , , , , , ,

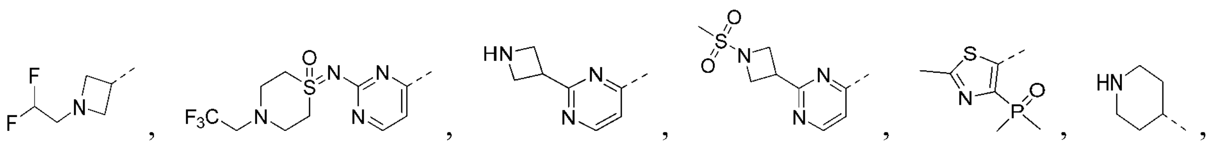 , , , , , ,

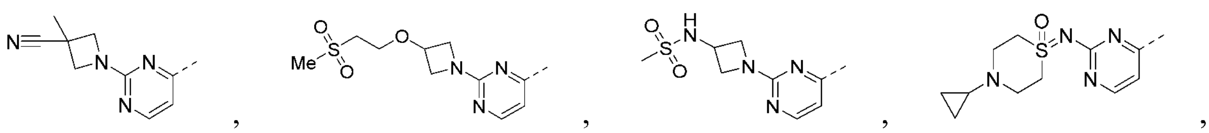 , , , ,

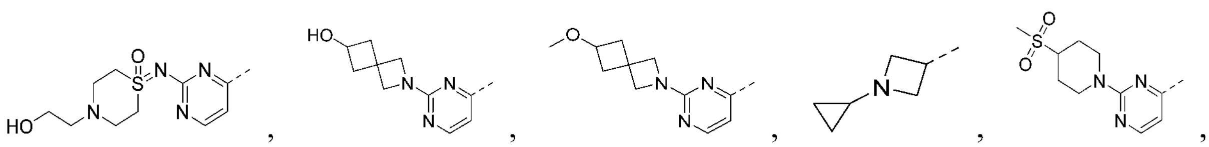 , , , , ,

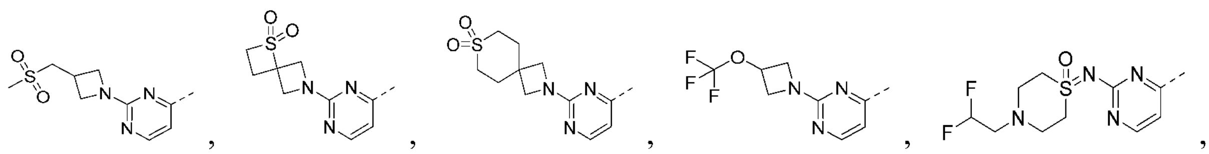 , , , , ,

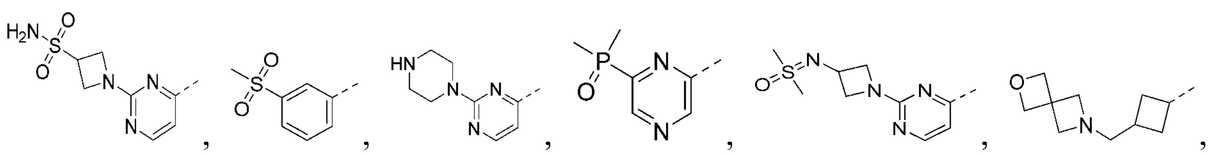 , , , , , ,

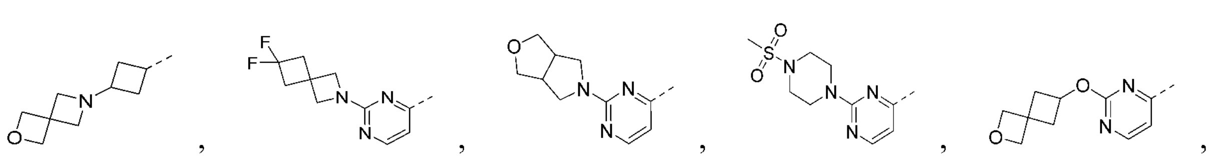 , , , , ,

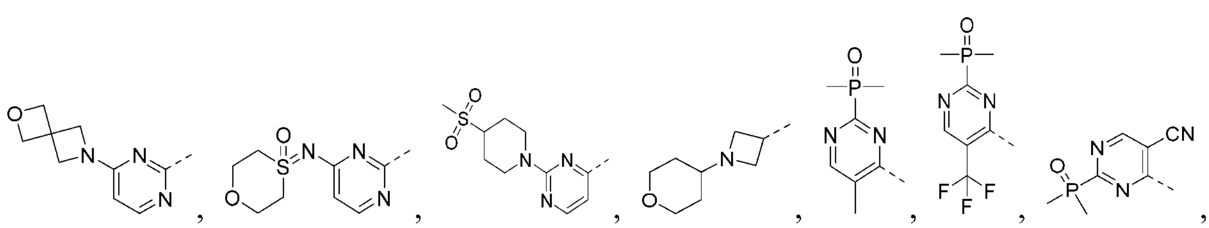 , , , , , , ,

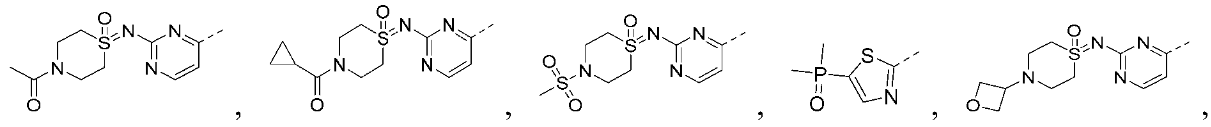

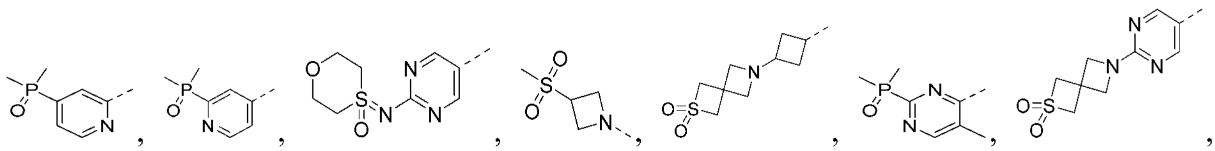

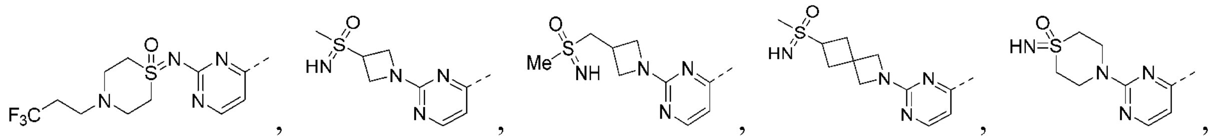

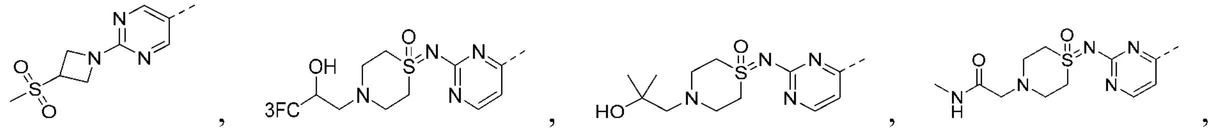

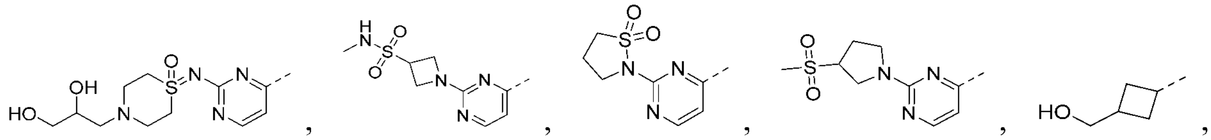

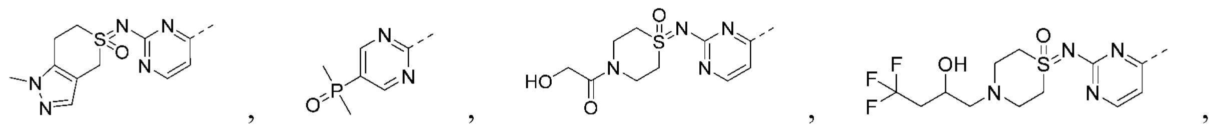

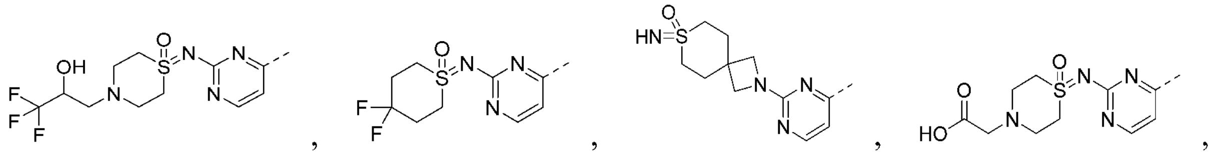

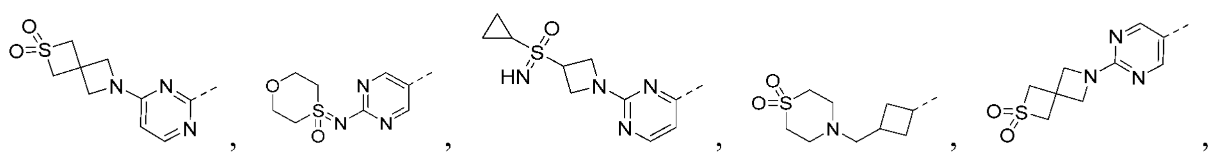

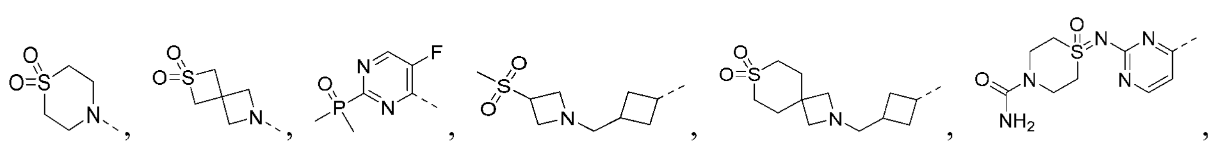

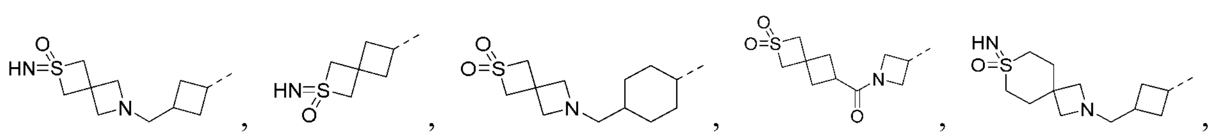

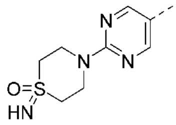, 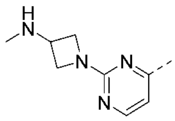, 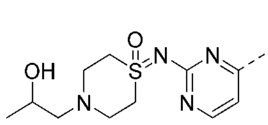, 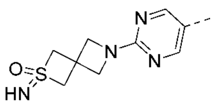, 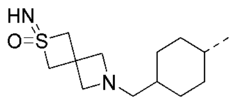,

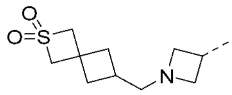, 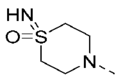, 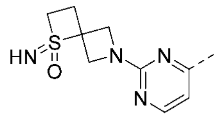, 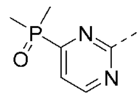, 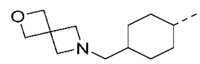,

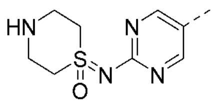, 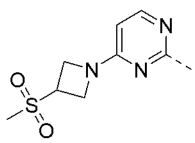, 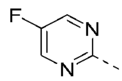, 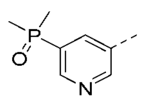, 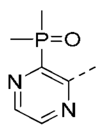, 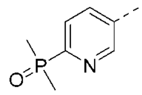,

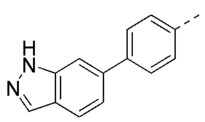, 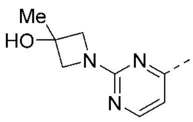, 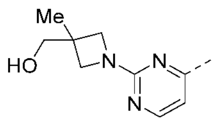, 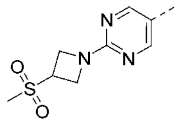, 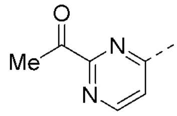,

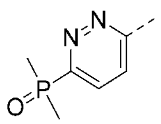, 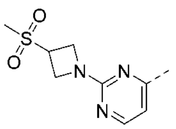, 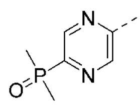, 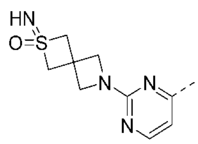, 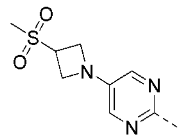, 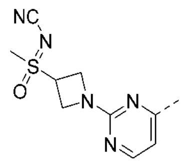,

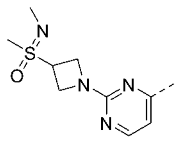, 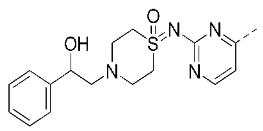, 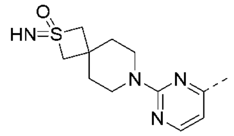, 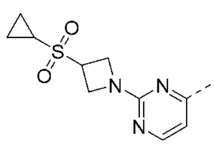, 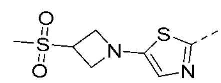,

and

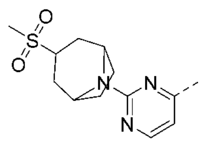,

and the remaining variables are as defined in the present disclosure.

In some embodiments of the present disclosure, ring B is selected from the group consisting of bicyclo[1.1.1]pentyl, cyclobutyl, cyclopentyl, 2,6-diazaspiro[3.3]heptyl, pyrazolyl, piperidinyl, thiazolyl, phenyl, pyridinyl, pyrimidinyl, pyridazinyl, pyrazinyl, oxazolyl, benzocyclopentyl, benzocyclohexyl, 1,2,3,4-tetrahydroquinolinyl, naphthyl, indolyl, isoindolinyl, spiro[cyclopropane-1,3'-indolin]-2'-one, 3(2H)-pyridazinone, 2(1H)-pyridinone, isoquinolinyl, and quinolin-2(1H)-one, and the remaining variables are as defined in the present disclosure.

In some embodiments of the present disclosure, the above structural moiety

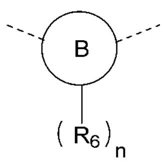

is selected from the group consisting of

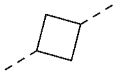, 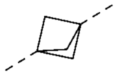, 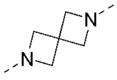,

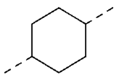, 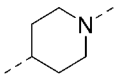, 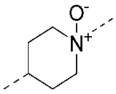, 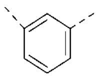, 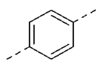, 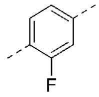, 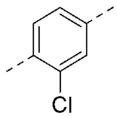, 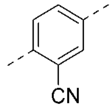, 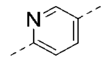,

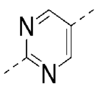, 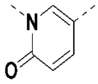, 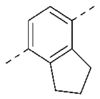, 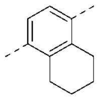, 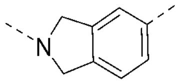, 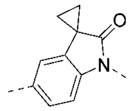, 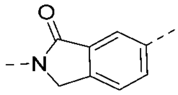, 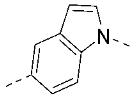,

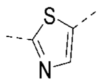, 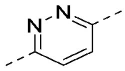, 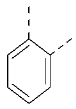, 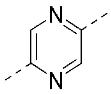, 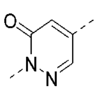, 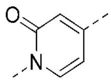, 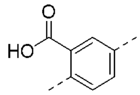, 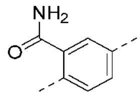,

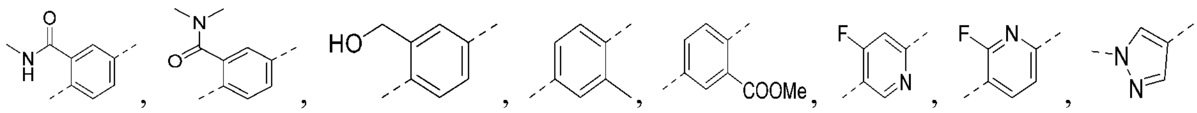, , , , , , , ,

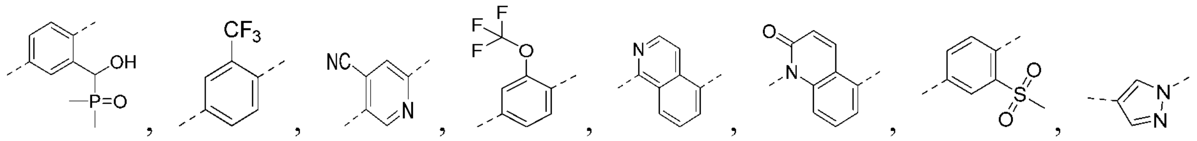, , , , , , , ,

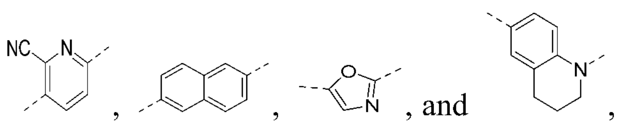, , , and ,

and the remaining variables are as defined in the present disclosure.

In some embodiments of the present disclosure, ring C is selected from the group consisting of azetidinyl, phenyl, pyridinyl, pyrimidinyl, pyridazinyl, pyrazinyl, indolyl, and spiro[cyclopropane-1,3'-indolin]-2'-one, and the remaining variables are as defined in the present disclosure.

In some embodiments of the present disclosure, the structural moiety

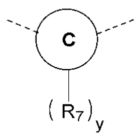

is selected from the group consisting of

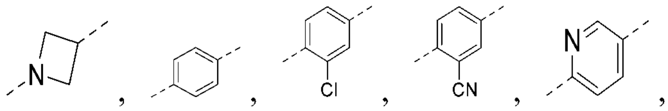, , , , ,

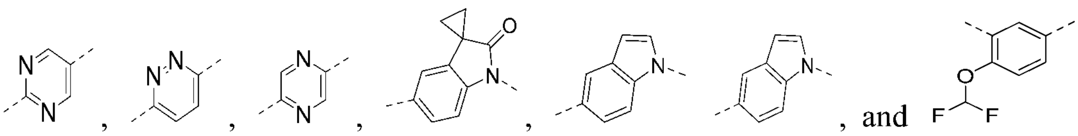, , , , , , and

, and the remaining variables are as defined in the present disclosure.

In some embodiments of the present disclosure, ring D is selected from the group consisting of phenyl, pyridinyl, benzocyclopentyl, benzocyclohexyl, 1H-indazolyl, 2H-indazolyl, and 1H-benzo[d]imidazolyl, and the remaining variables are as defined in the present disclosure.

In some embodiments of the present disclosure, the structural moiety

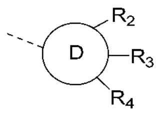

is selected from the group consisting of

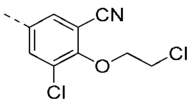 , 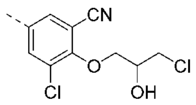 , 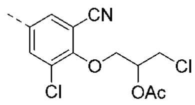 ,

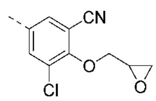 , 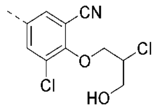 , 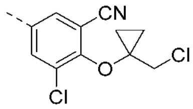 , 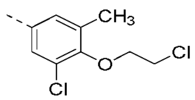 , 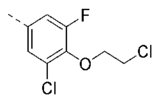 , 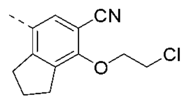 ,

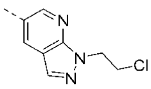 , 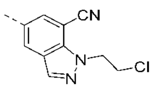 , 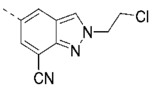 , 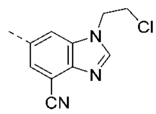 , 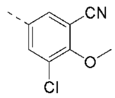 , 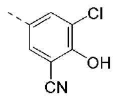 , 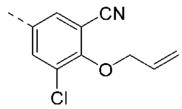 ,

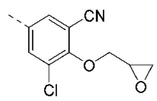 , 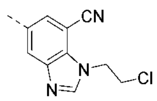 , 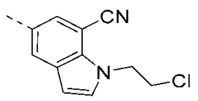 , 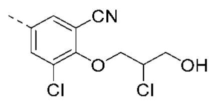 , 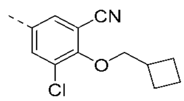 , 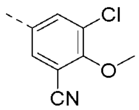 ,

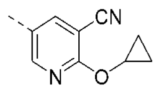 , 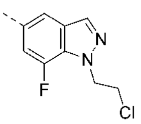 , 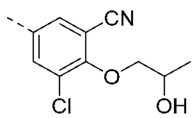 , 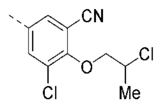 , 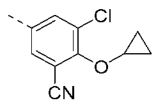 , 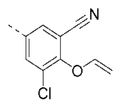 ,

and

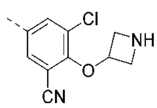 ,

and the remaining variables are as defined in the present disclosure.

The present disclosure also provides a compound of the following formula, an optical isomer thereof, and a pharmaceutically acceptable salt thereof, selected from the group consisting of:

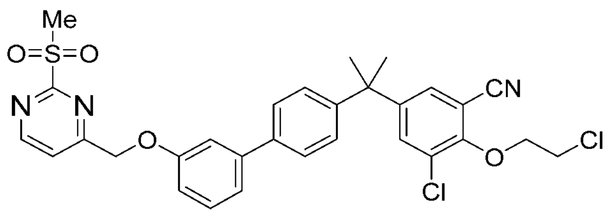 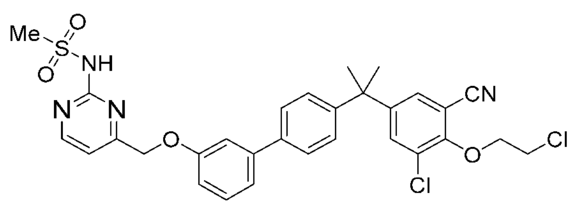

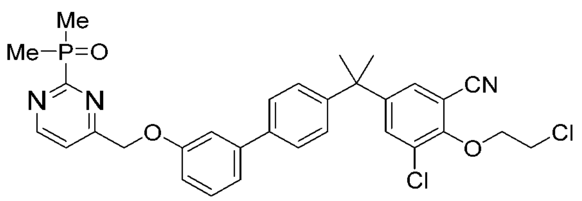

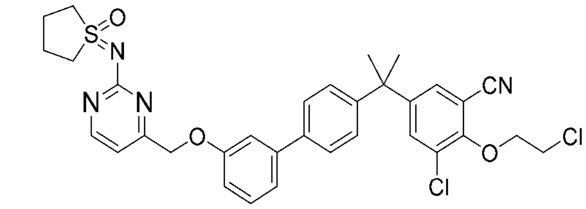

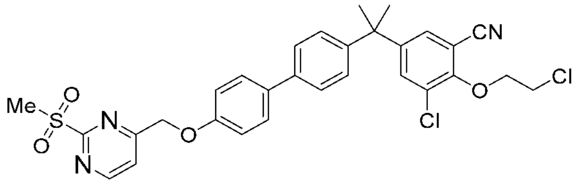

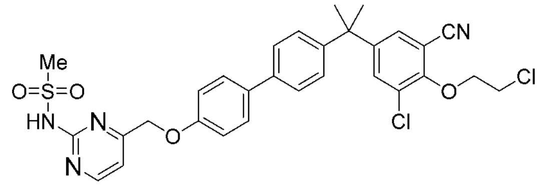

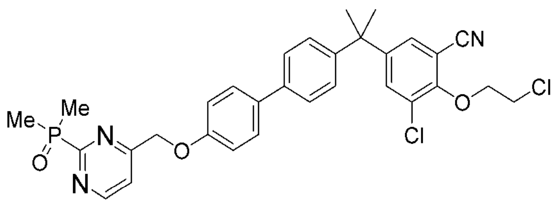

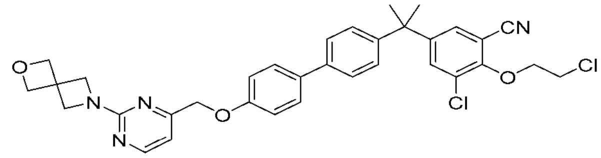

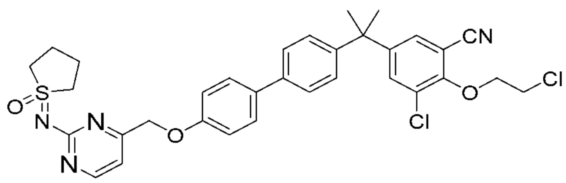

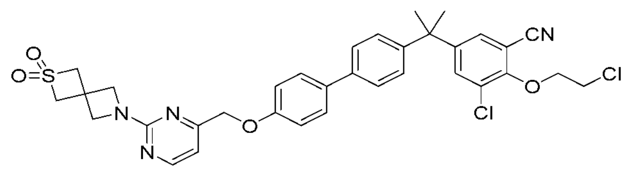

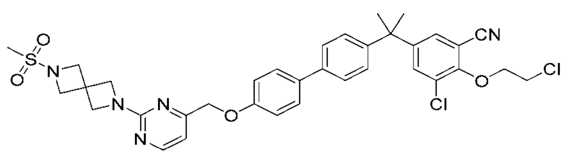

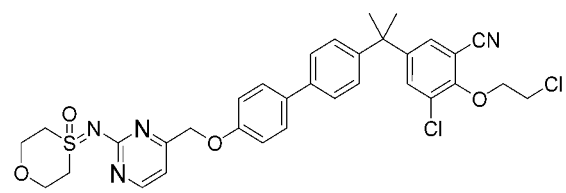

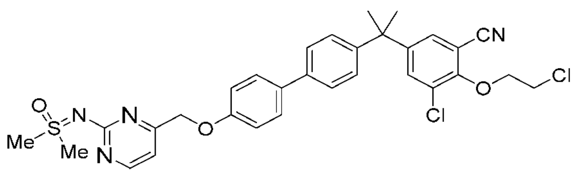

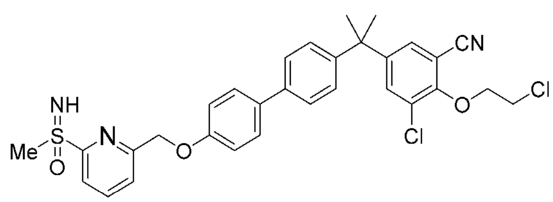

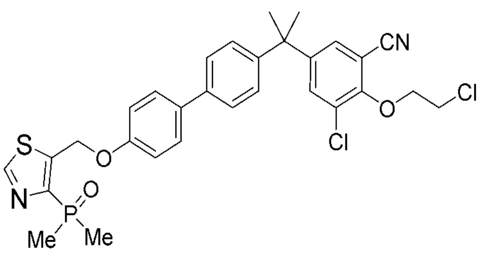

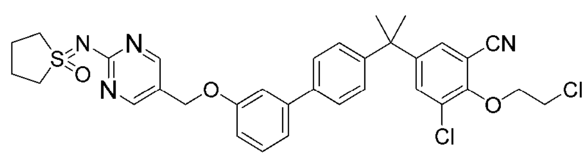

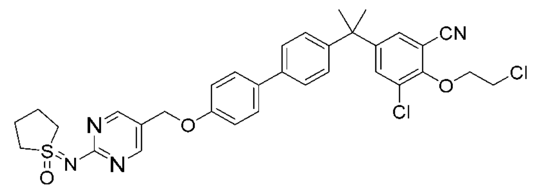

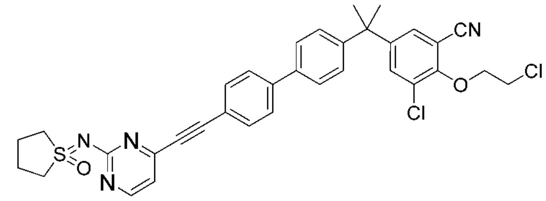

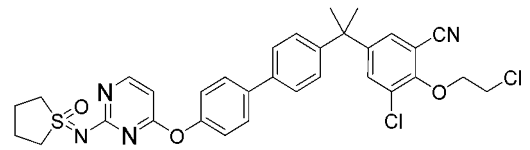

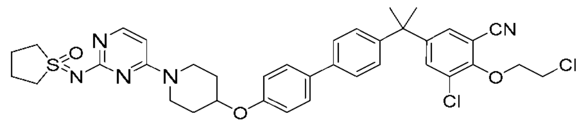

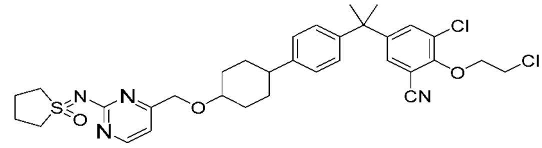

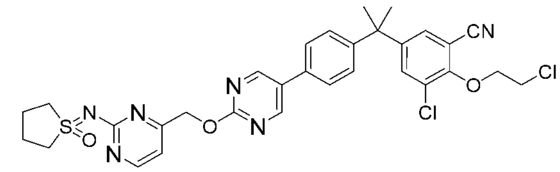

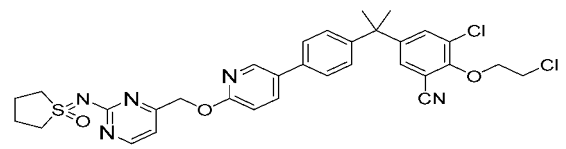

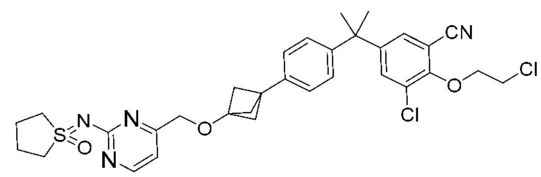

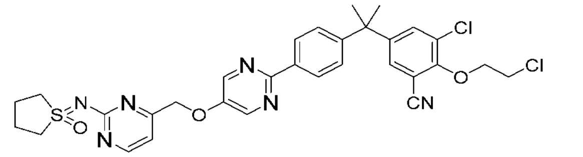

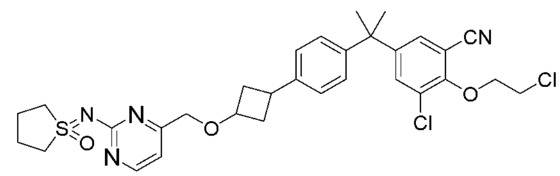

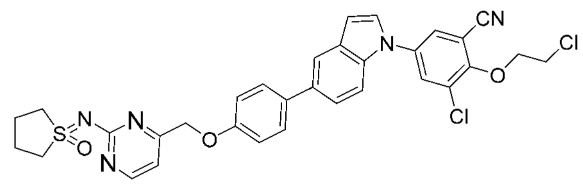

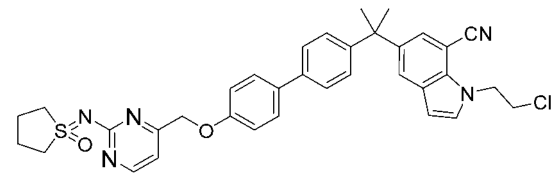

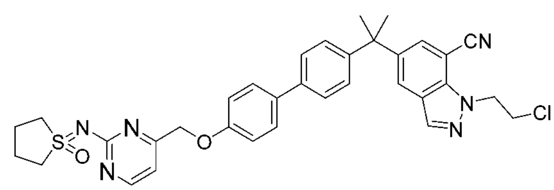

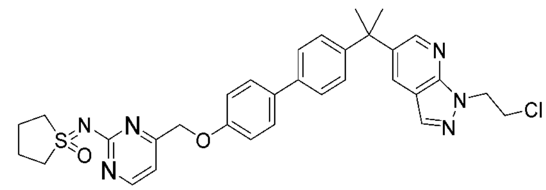

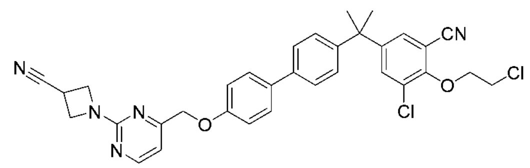

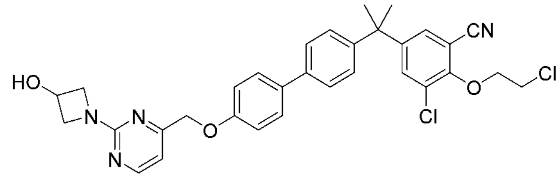

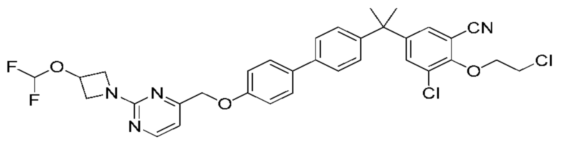

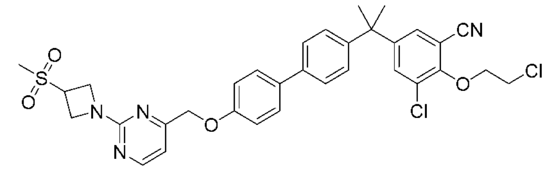

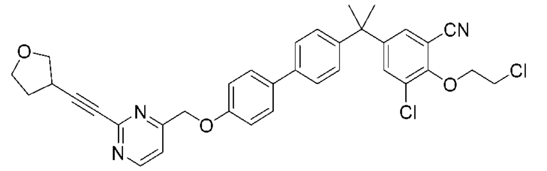

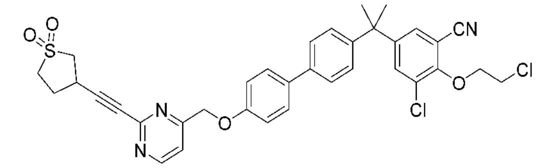

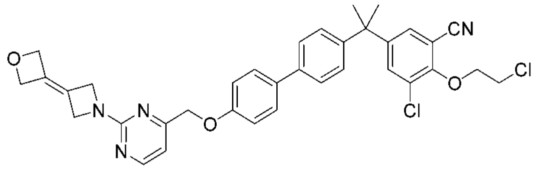

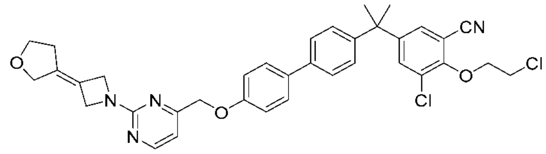

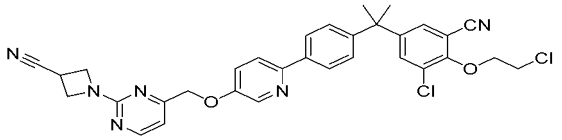

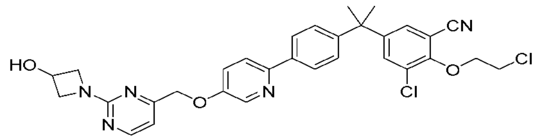

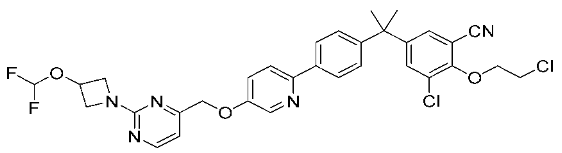

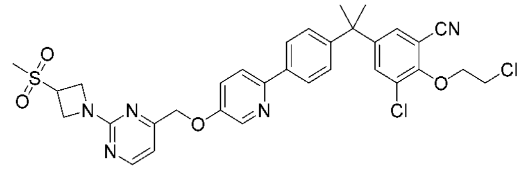

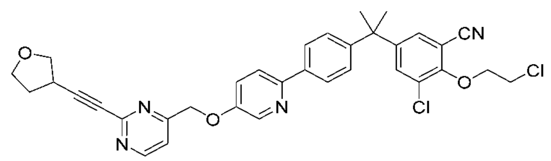

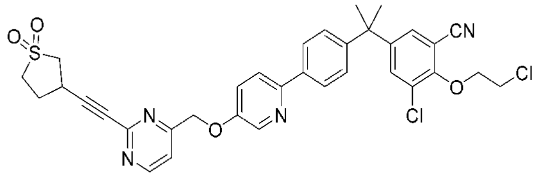

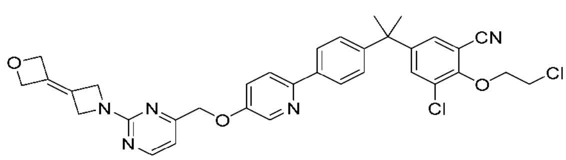

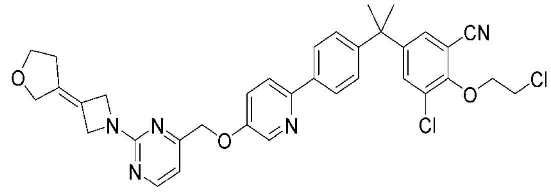

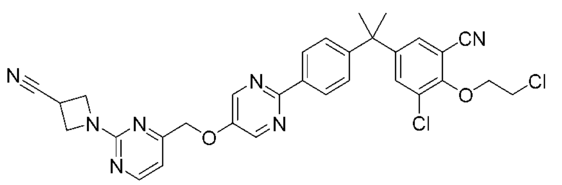

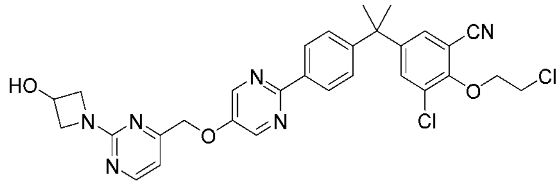

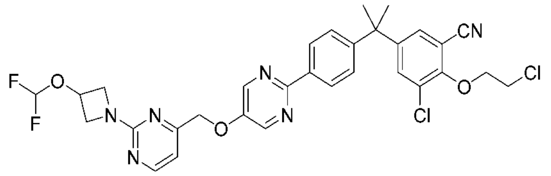

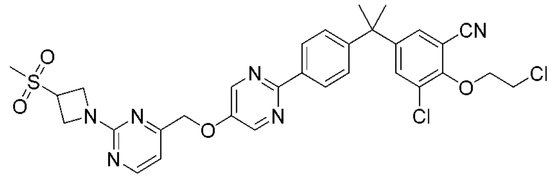

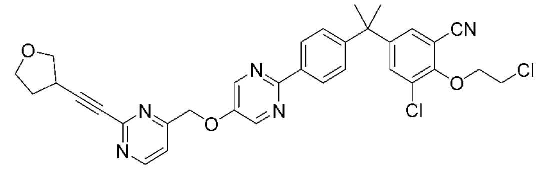

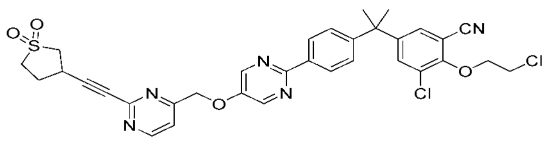

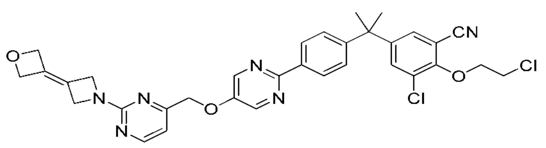

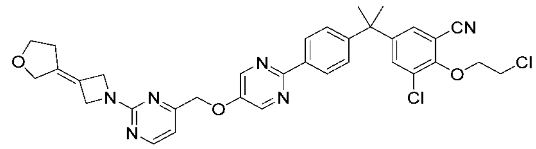

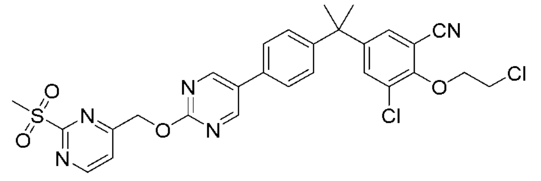

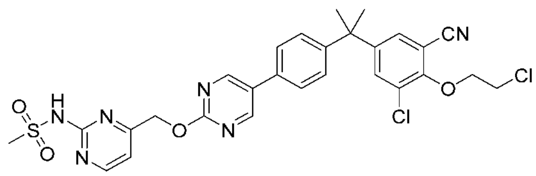

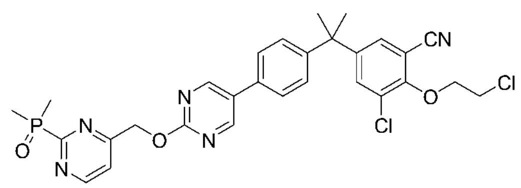

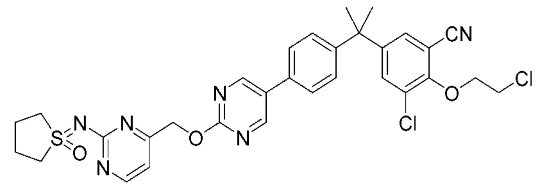

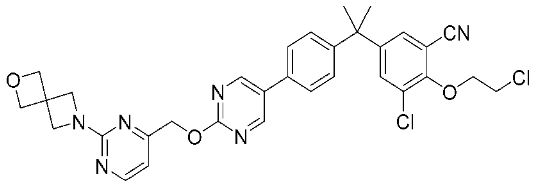

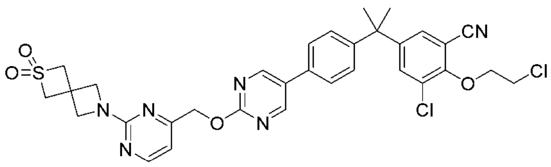

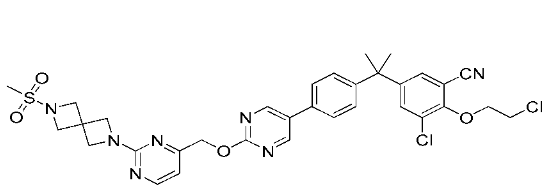

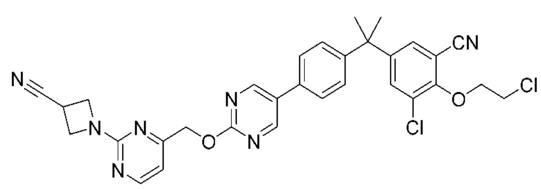

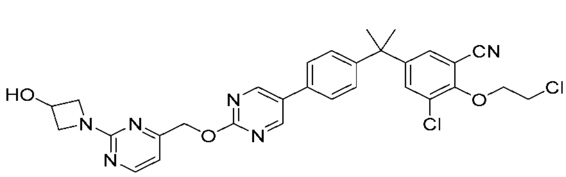

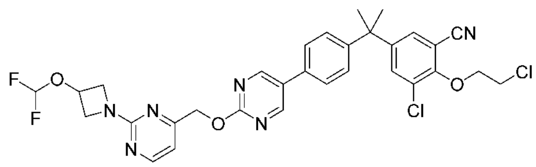

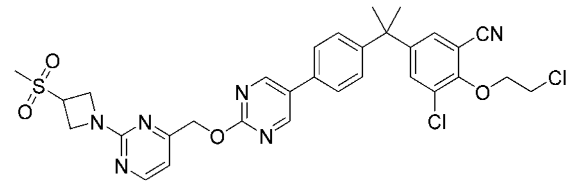
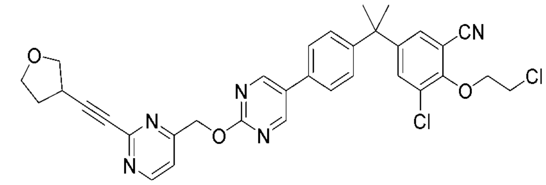
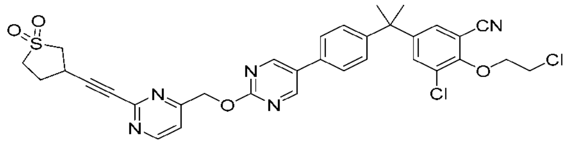
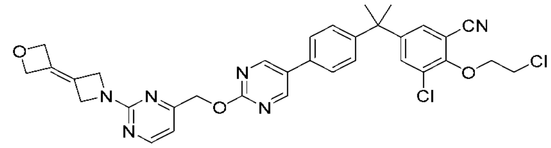
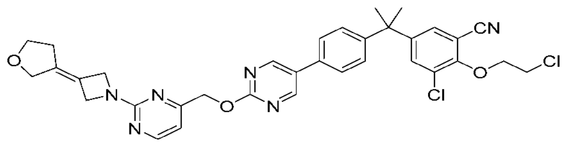
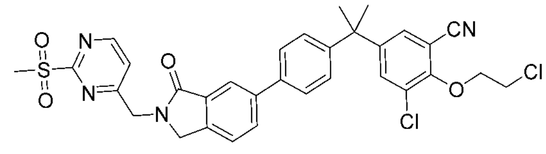
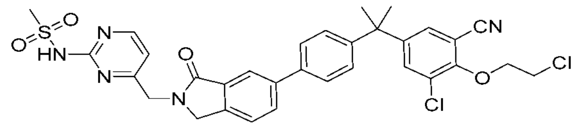
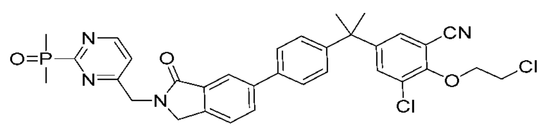
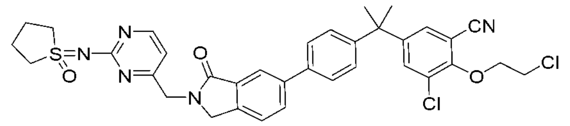
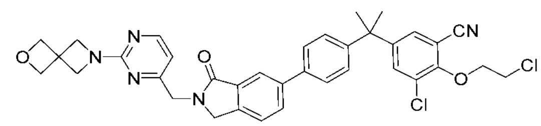
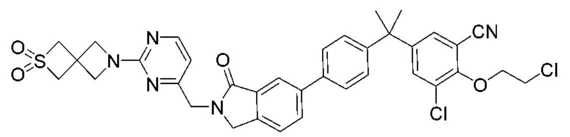
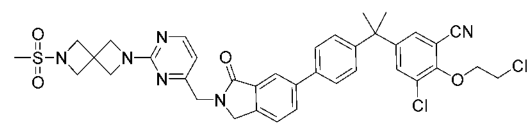
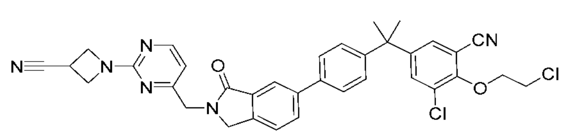
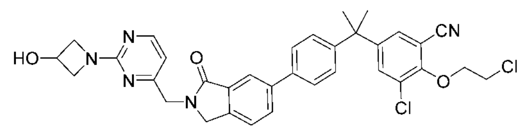
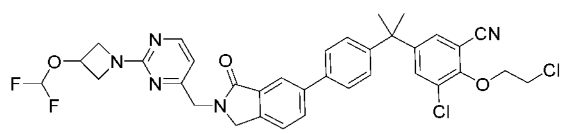
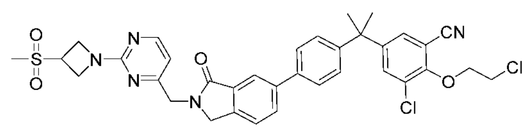
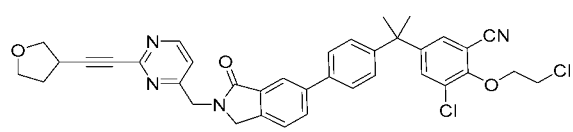
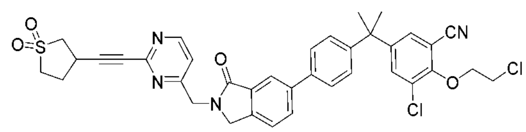
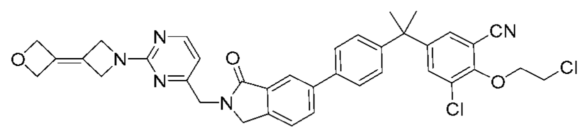
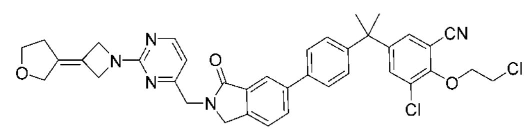

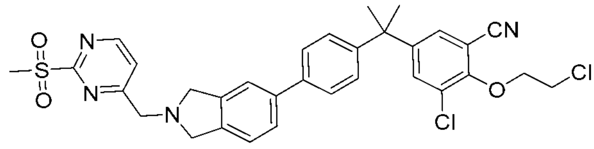

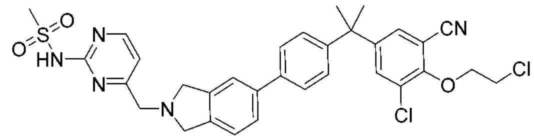

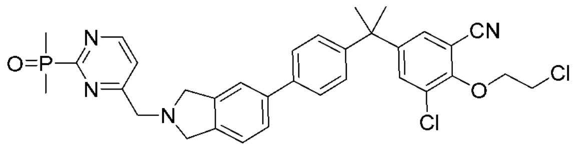

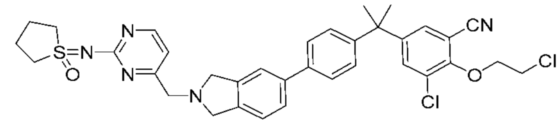

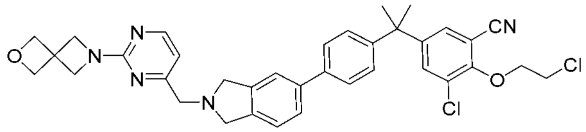

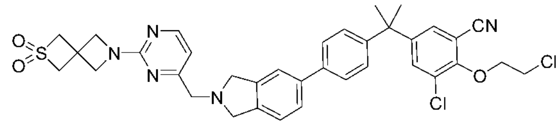

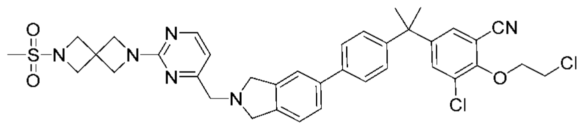

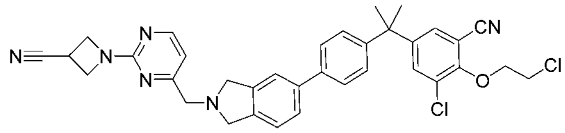

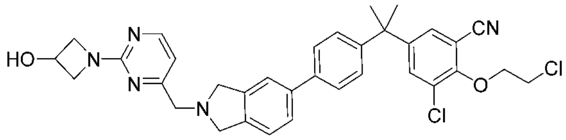

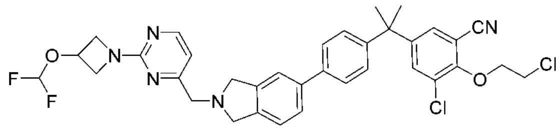

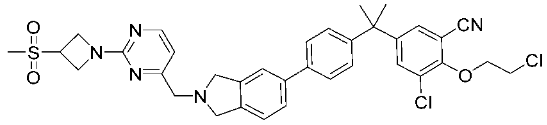

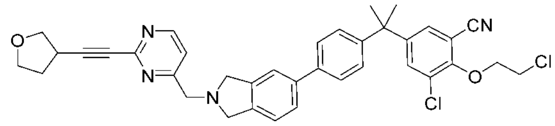

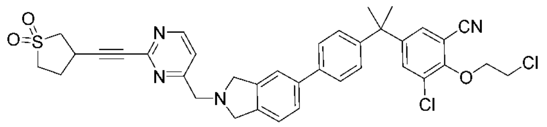

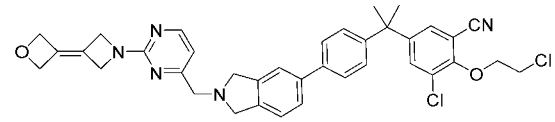

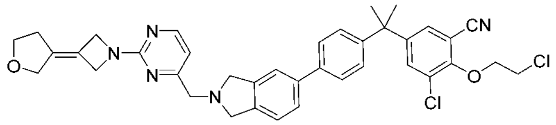

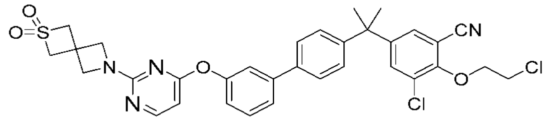

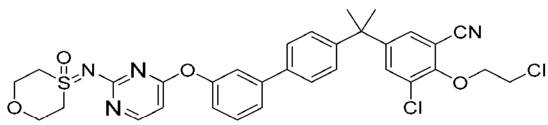

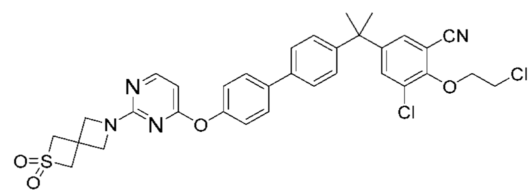

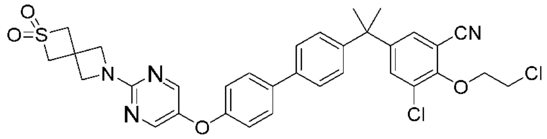

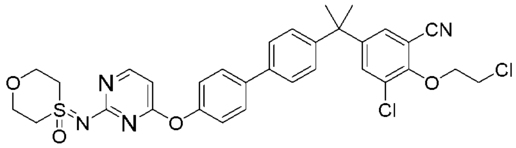

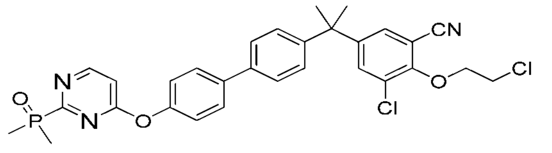

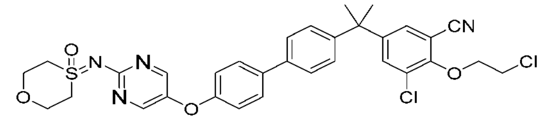

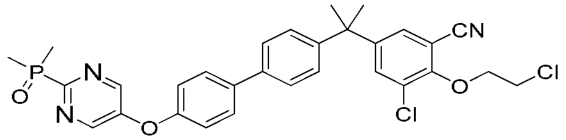

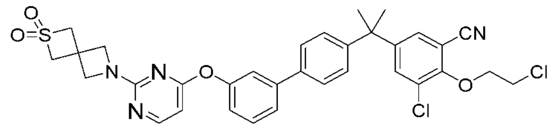

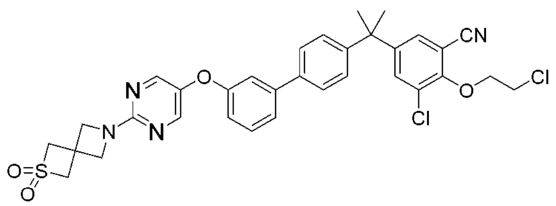

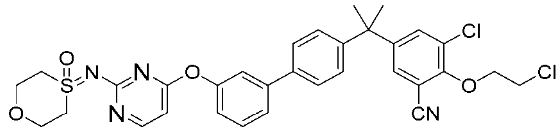

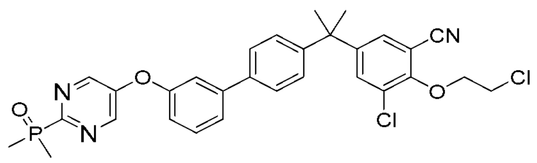

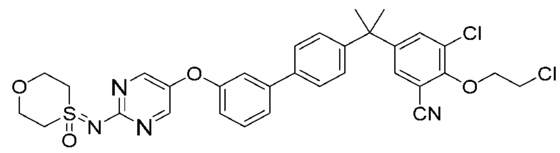

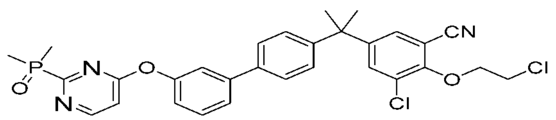

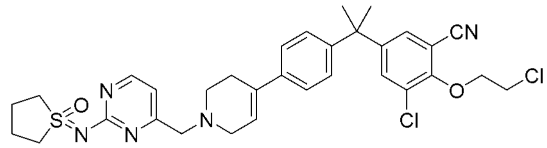

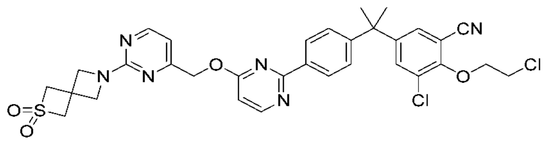

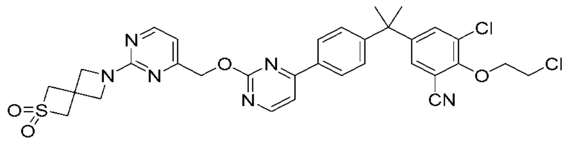

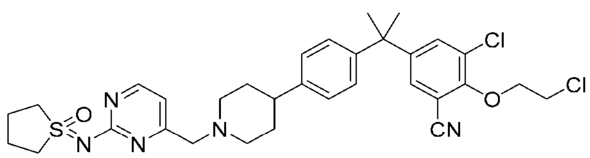

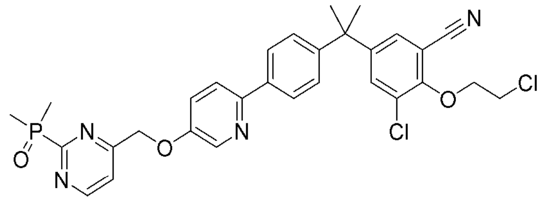

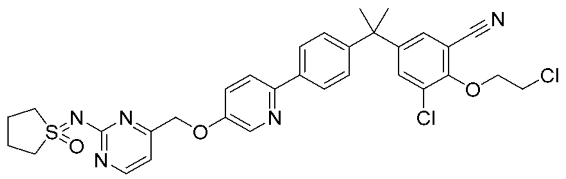

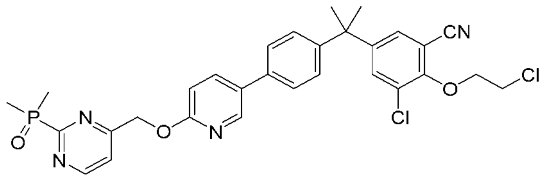

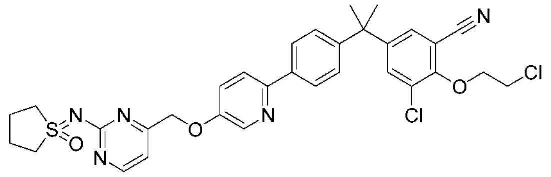

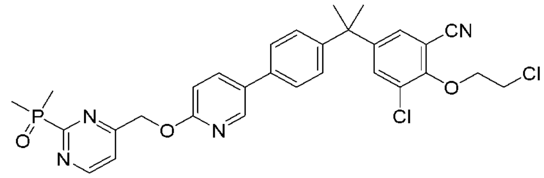

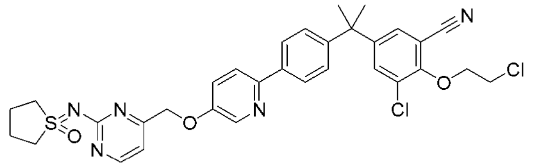

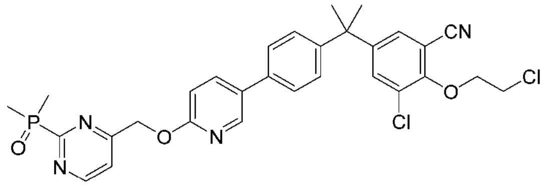

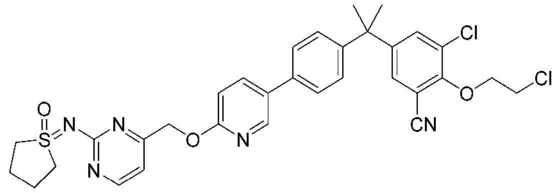

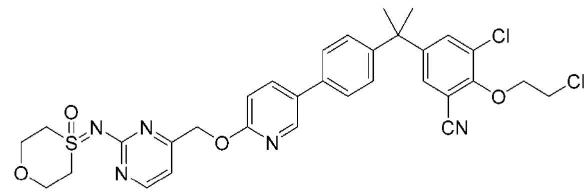

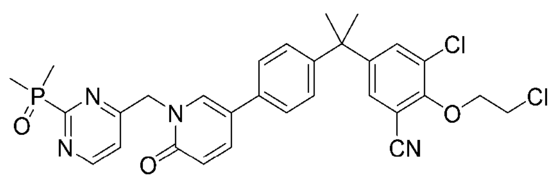

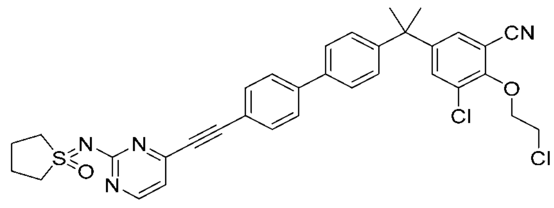

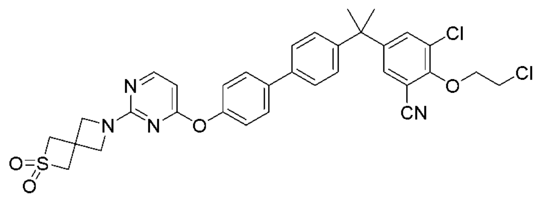

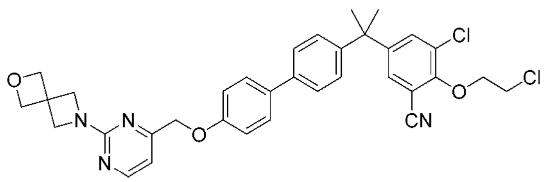

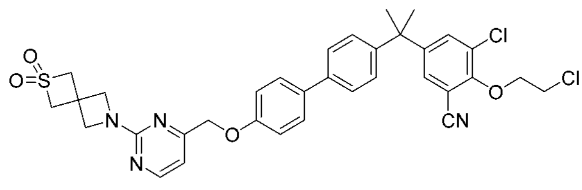

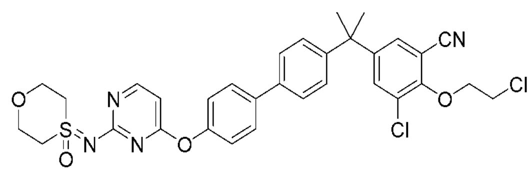

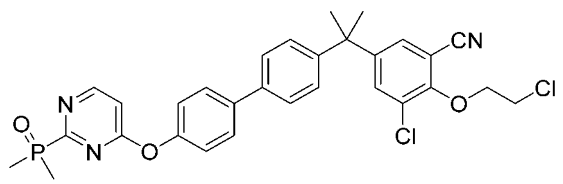

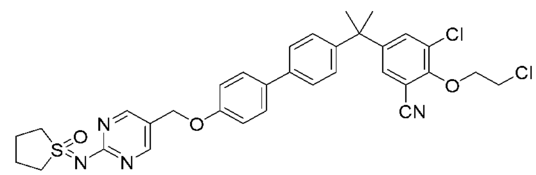

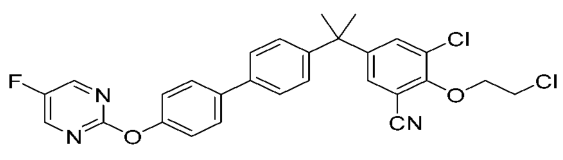

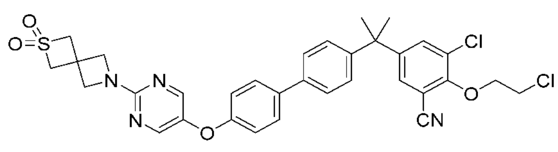

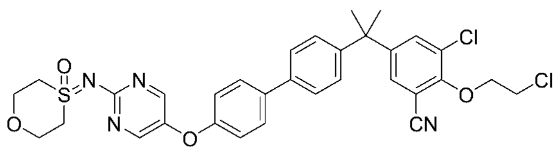
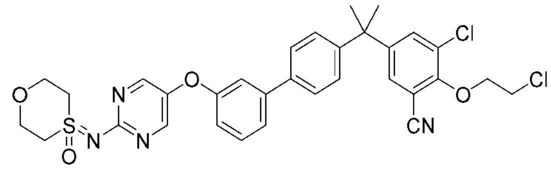
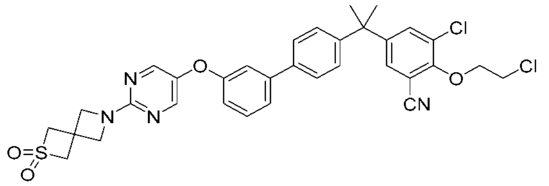
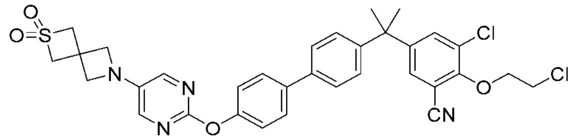
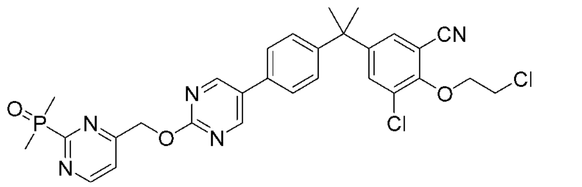
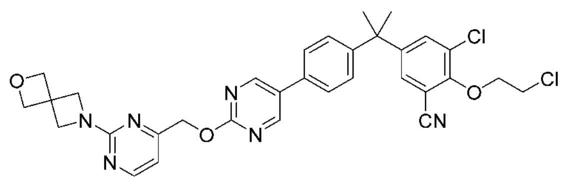
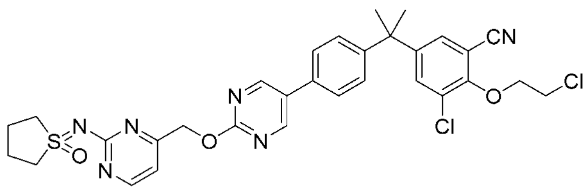
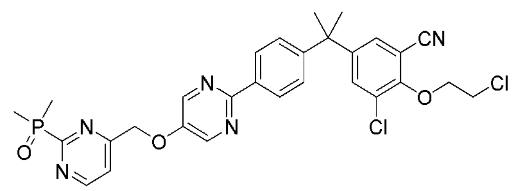
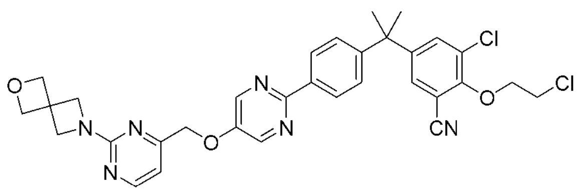
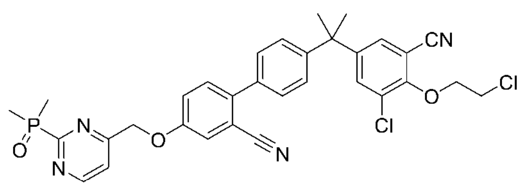
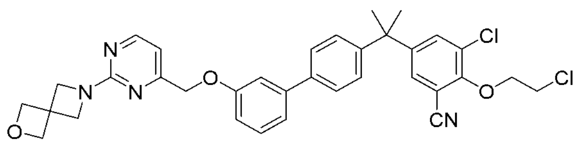
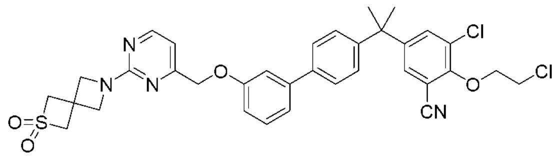
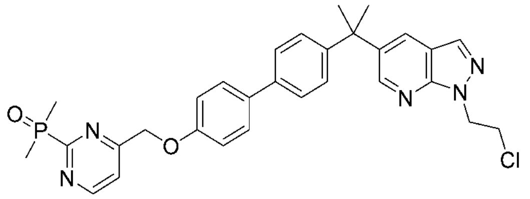
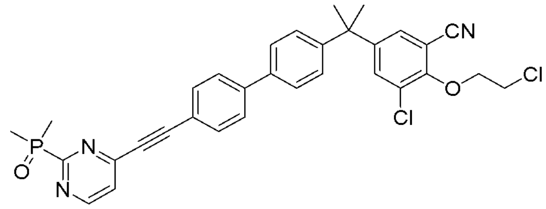
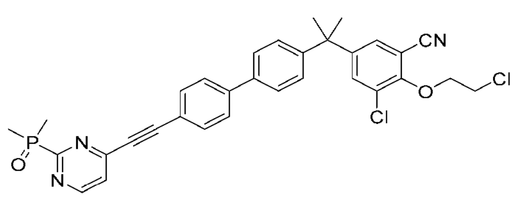
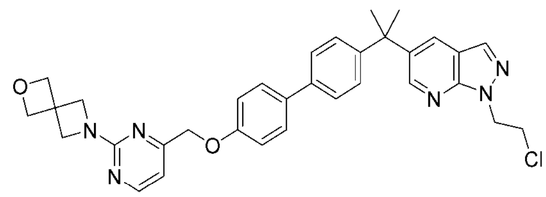

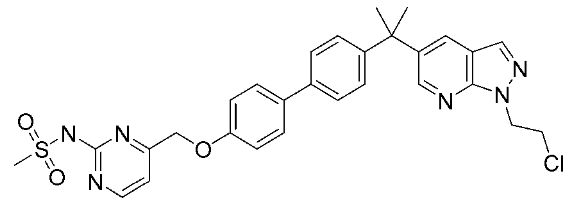

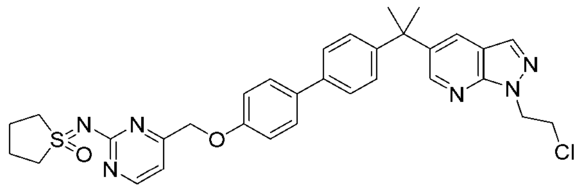

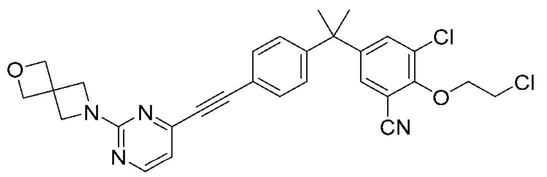

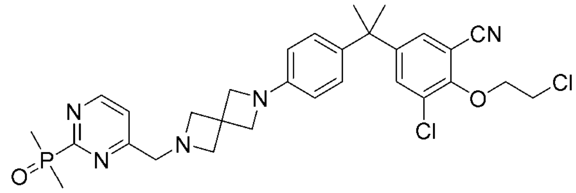

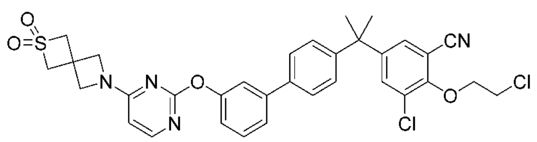

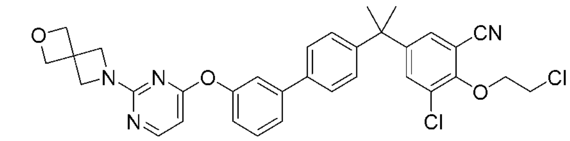

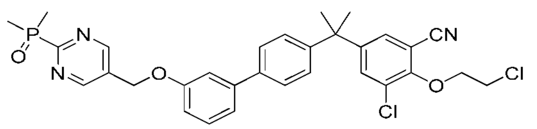

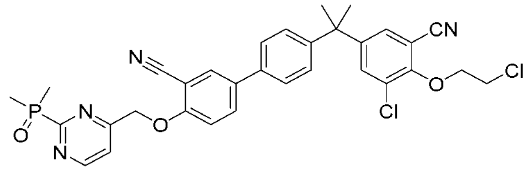

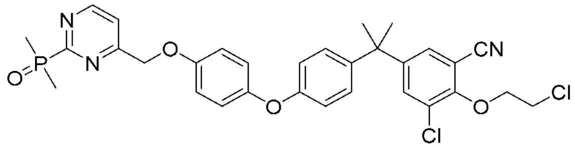

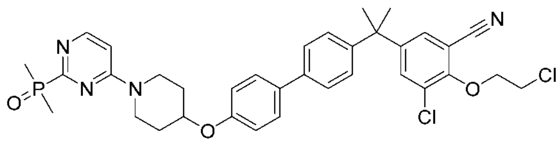

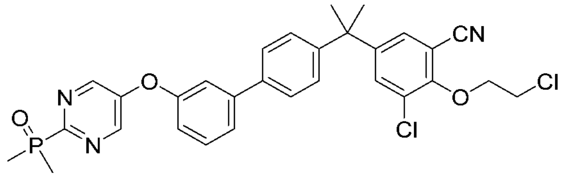

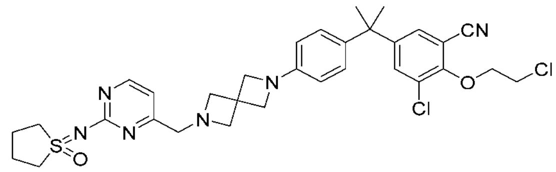

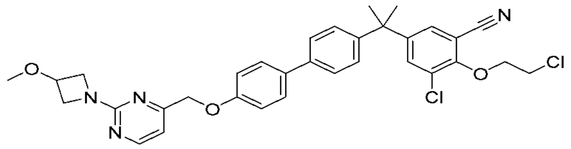

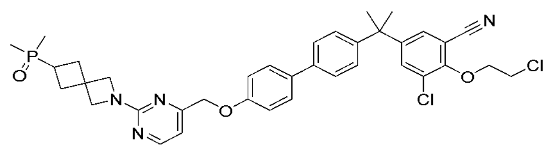

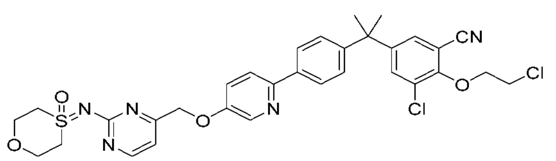

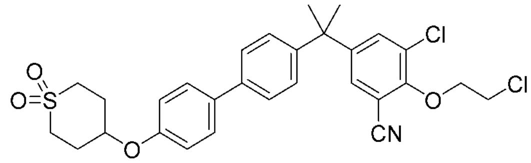

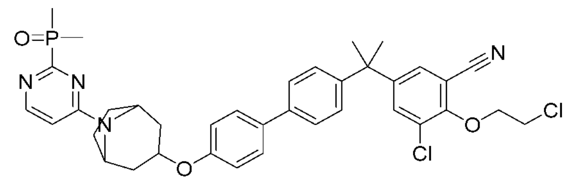

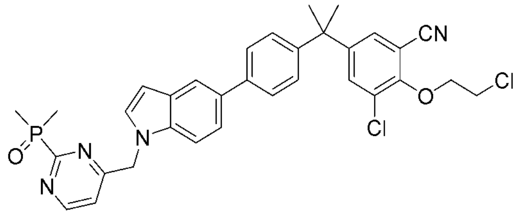

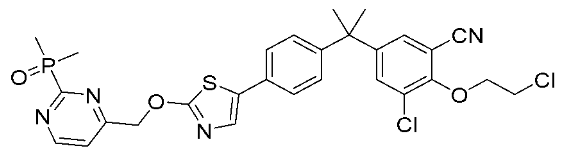

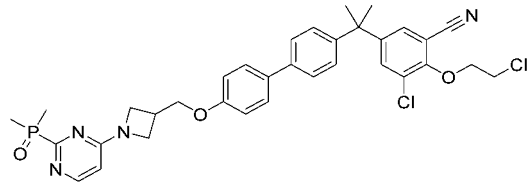

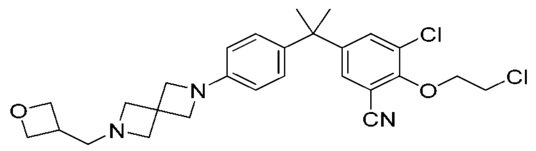

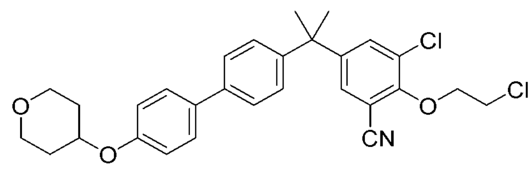

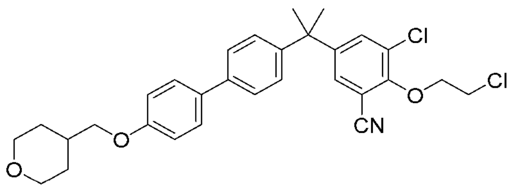

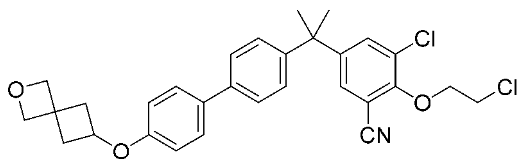

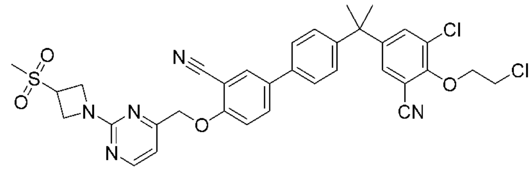

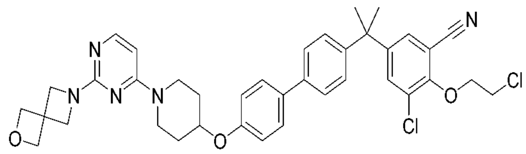

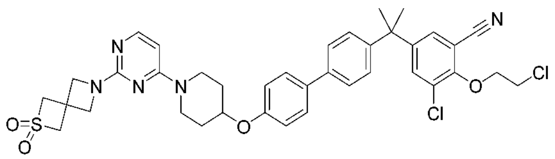

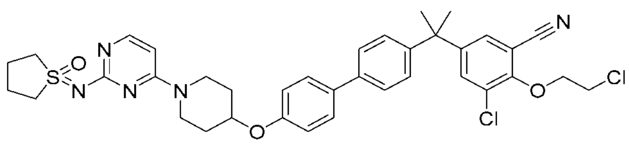

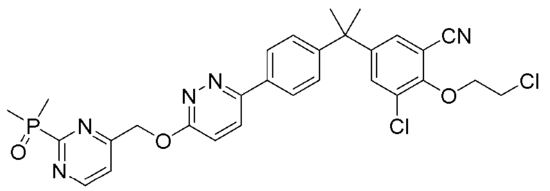

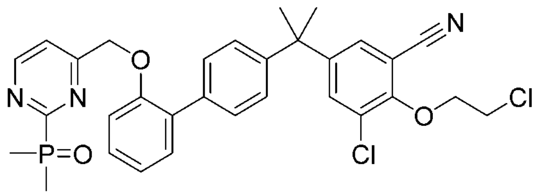

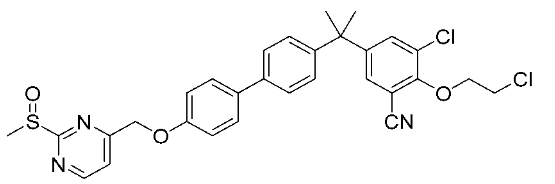

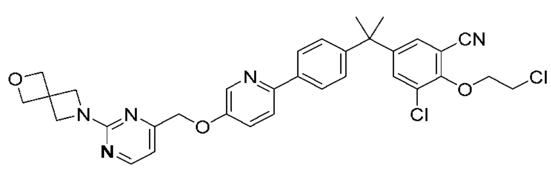

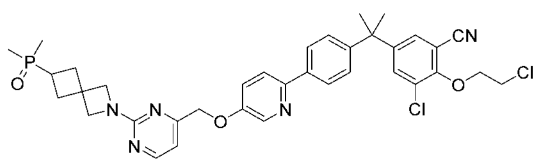

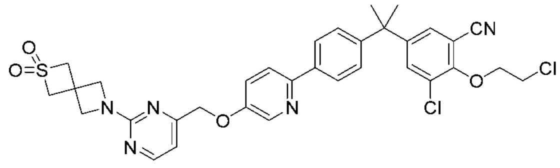

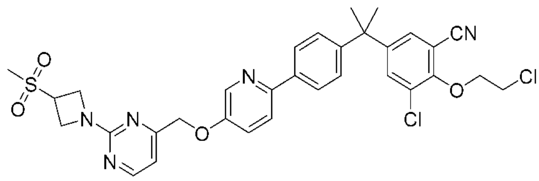

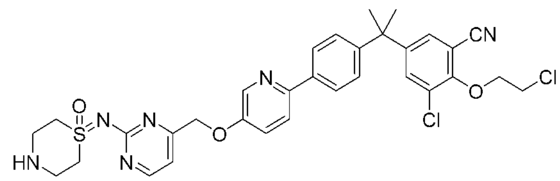

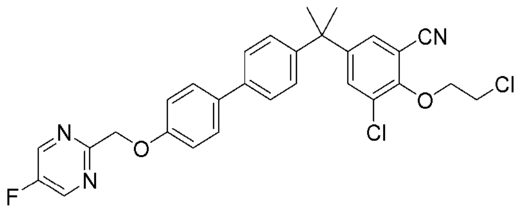

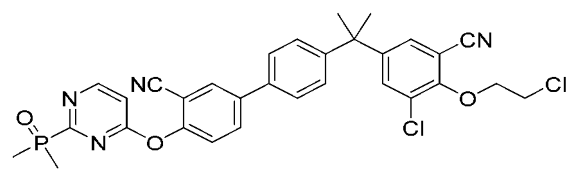

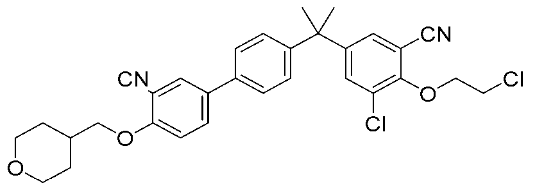

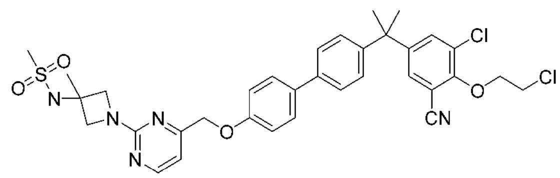

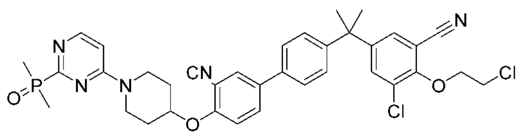

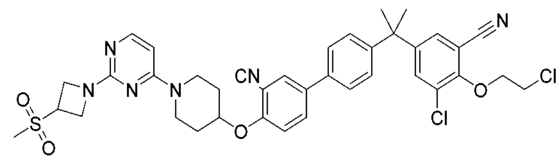

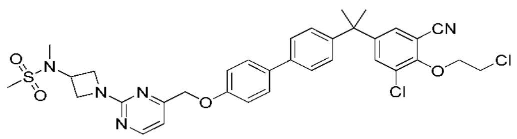

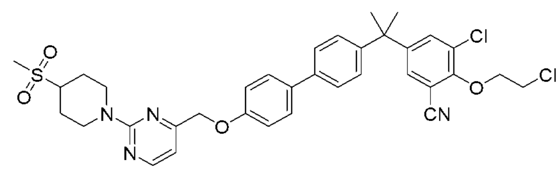

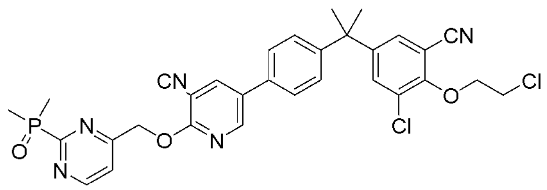

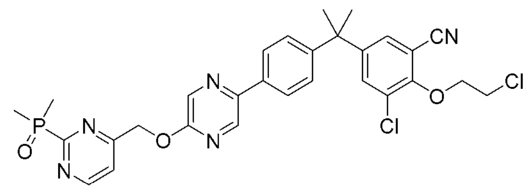

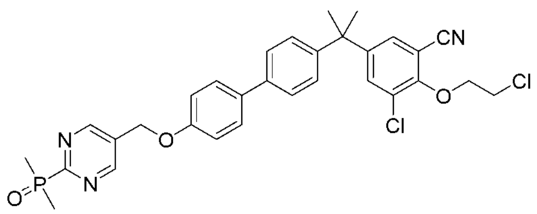

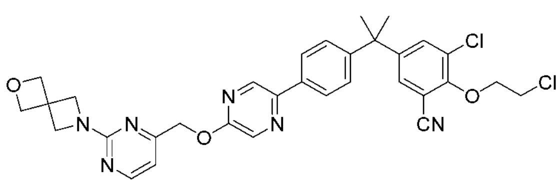

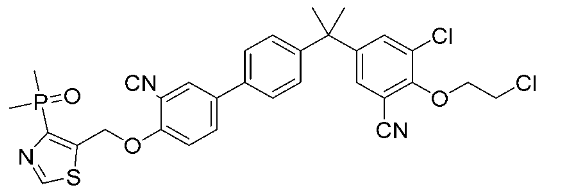

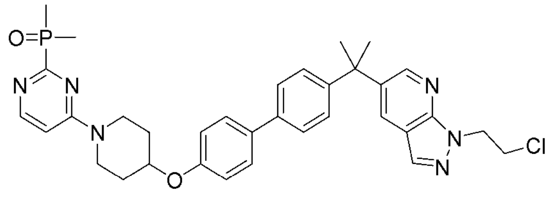

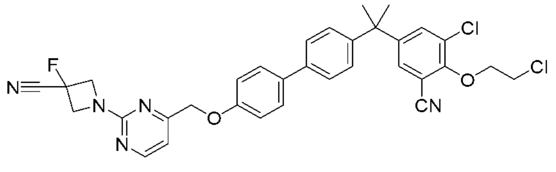

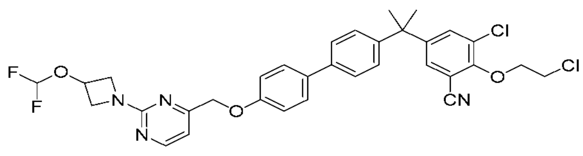

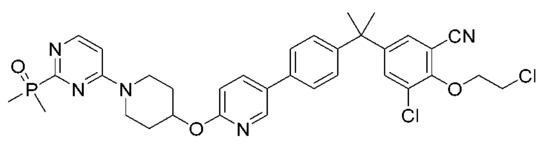

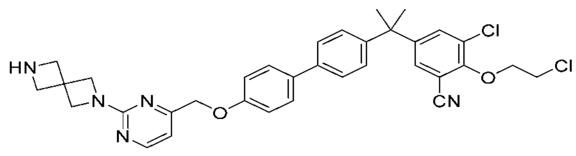

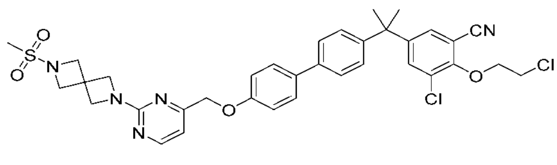

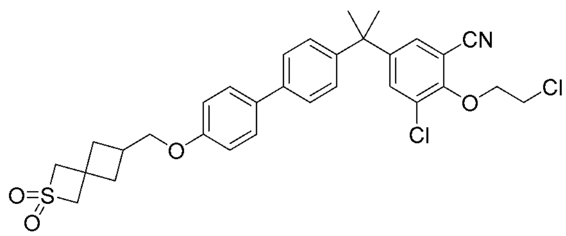

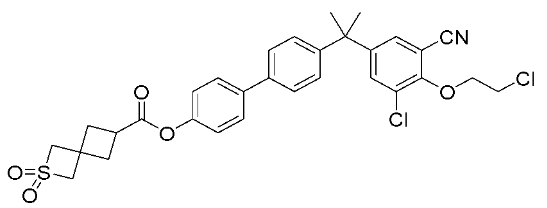

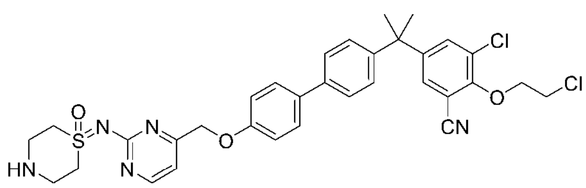

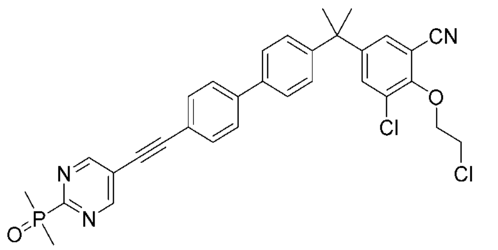

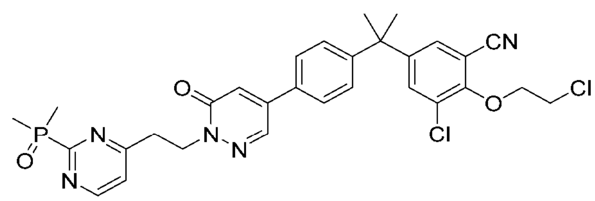

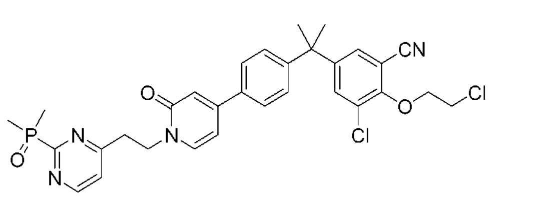

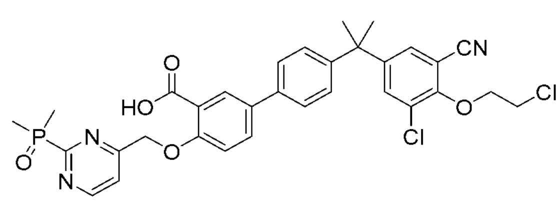

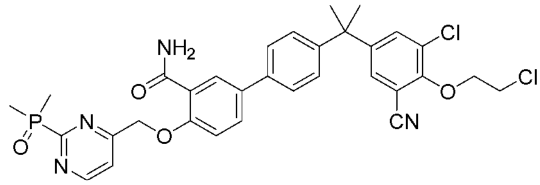
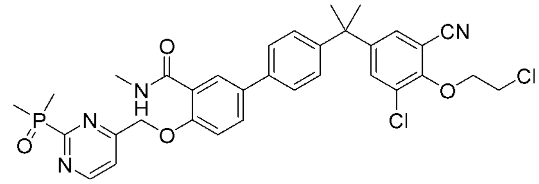

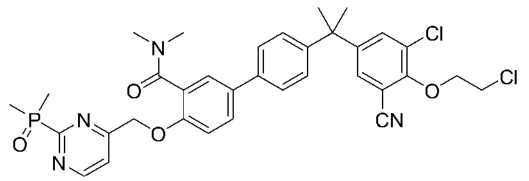
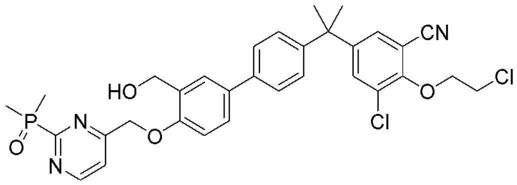

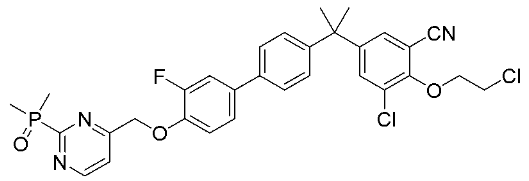
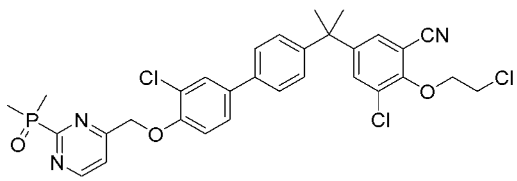

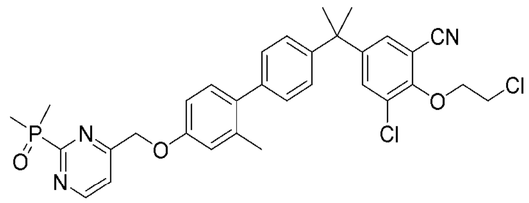
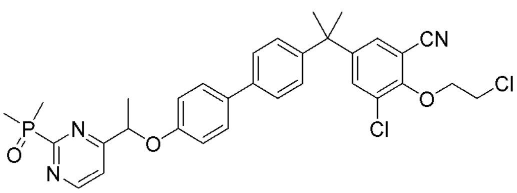

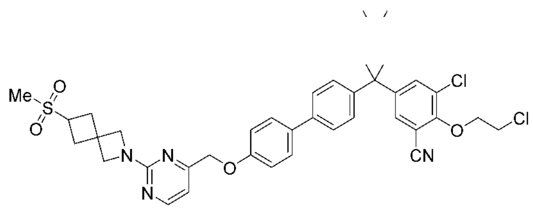
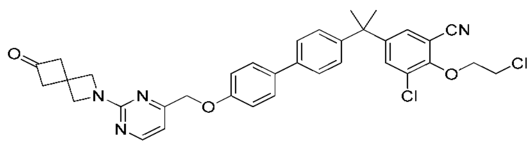

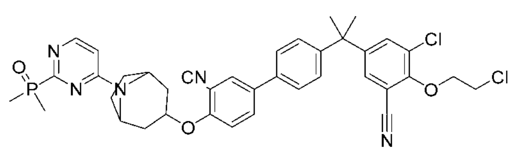
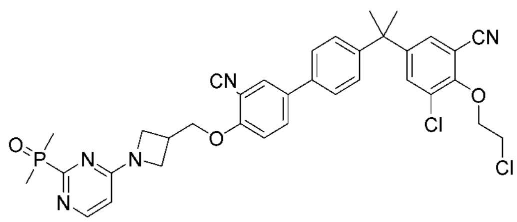

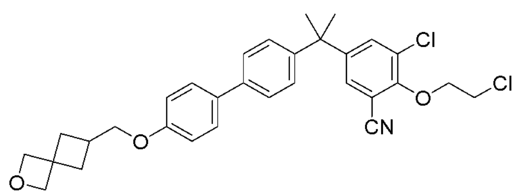
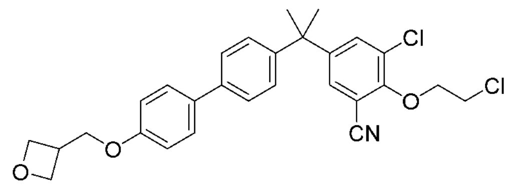

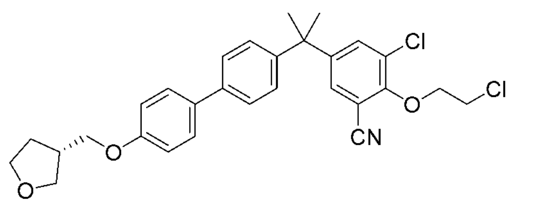

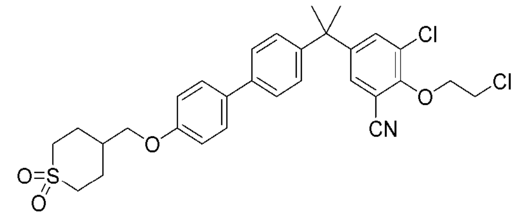

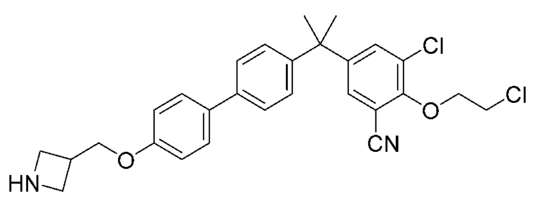

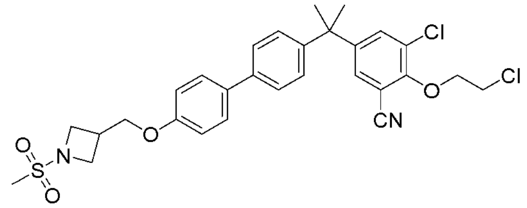

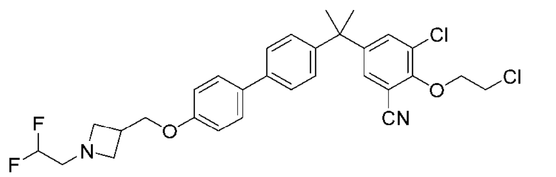

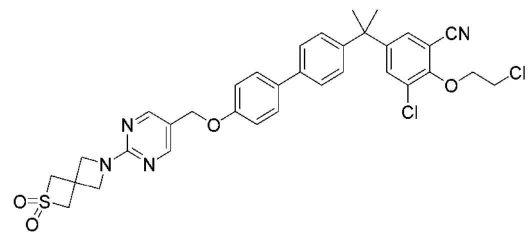

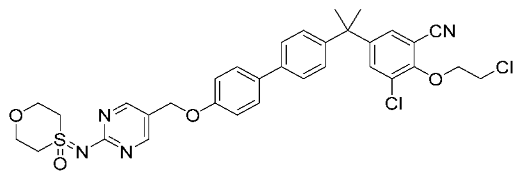

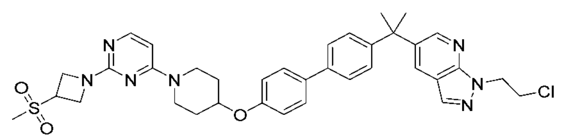

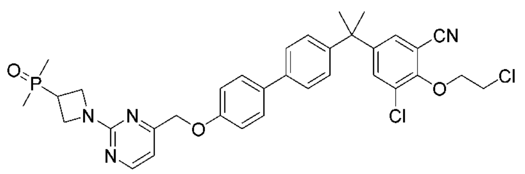

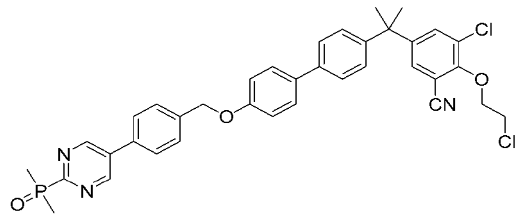

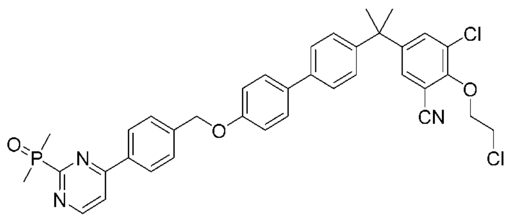

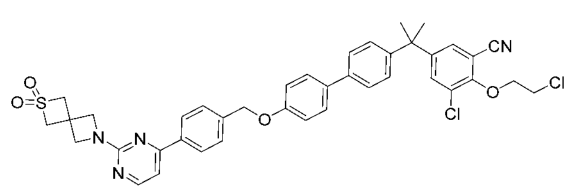

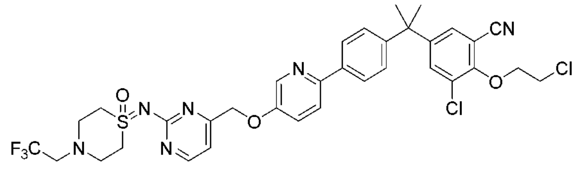

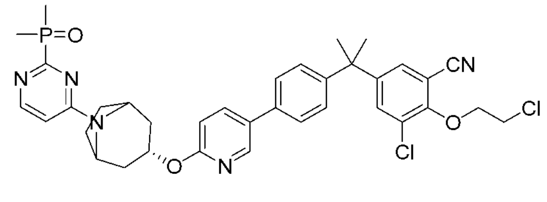

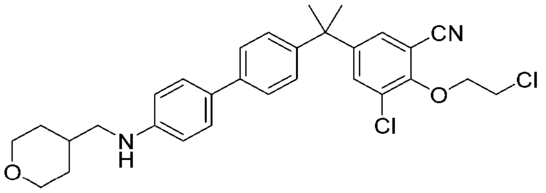
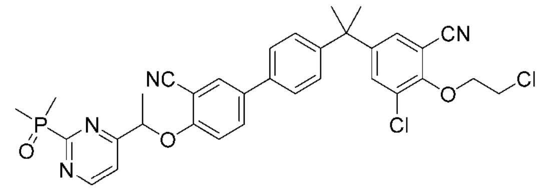
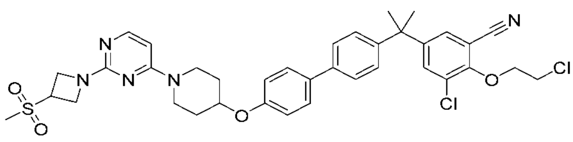
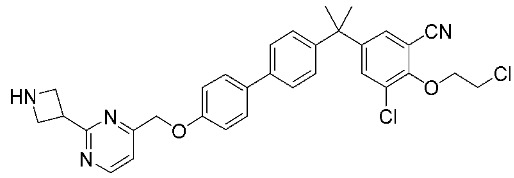
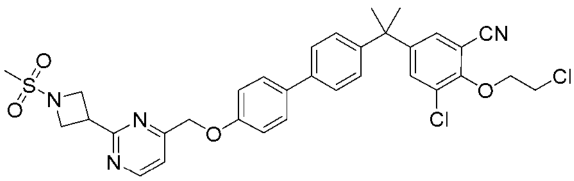
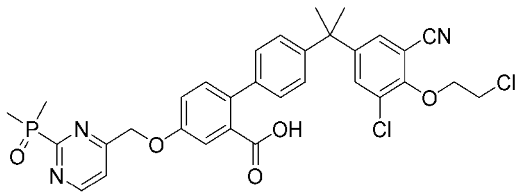
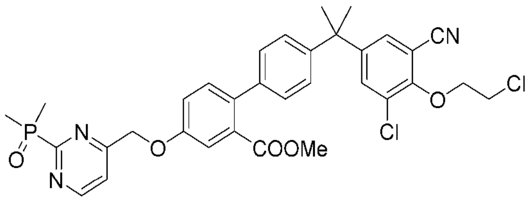
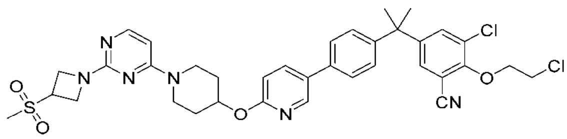
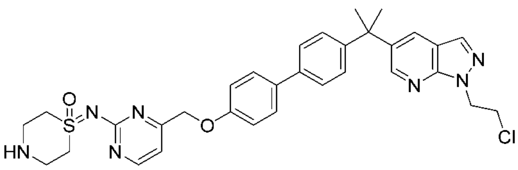
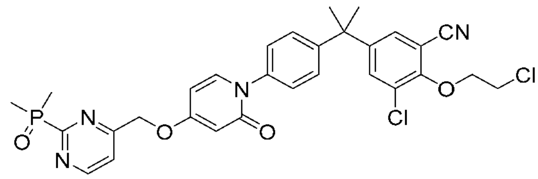
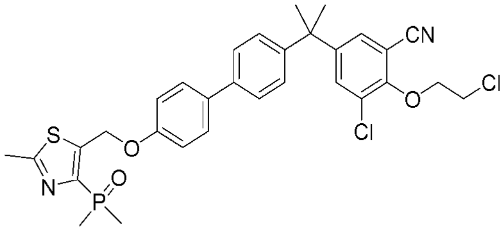
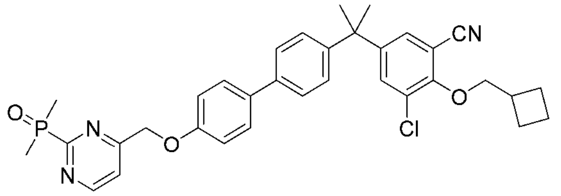
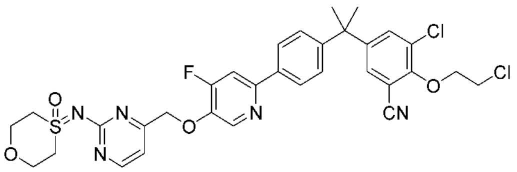
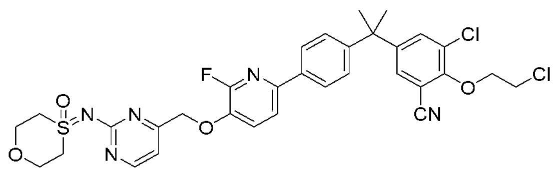
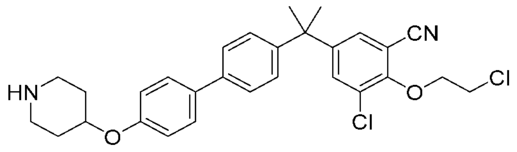
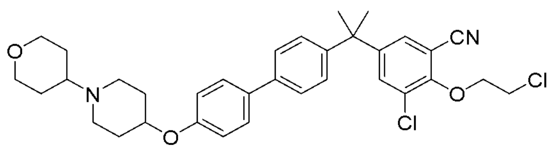

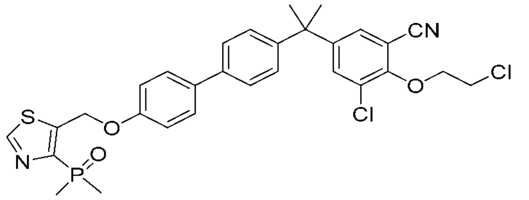

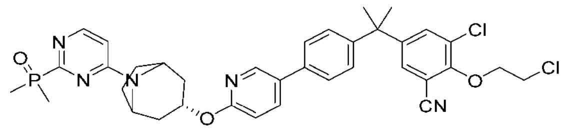

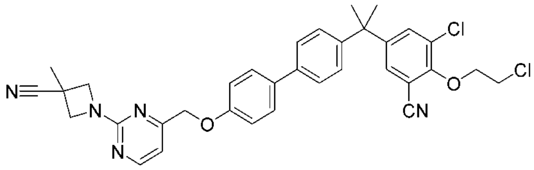

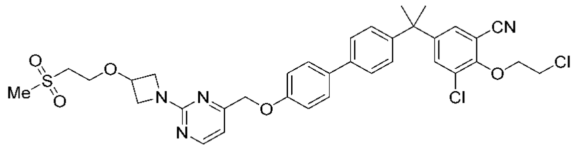

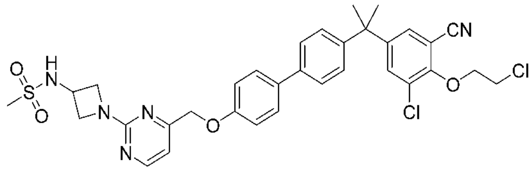

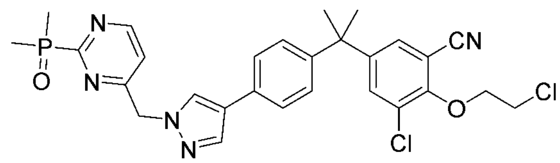

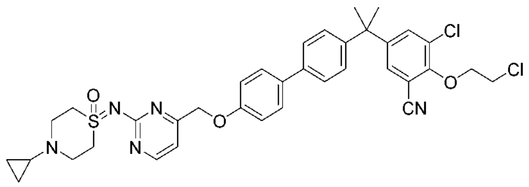

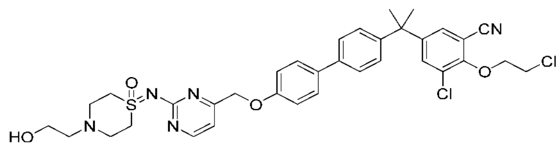

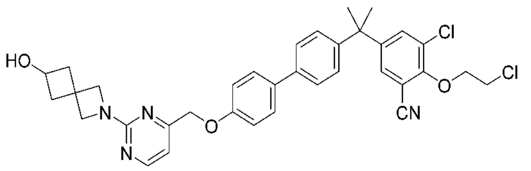

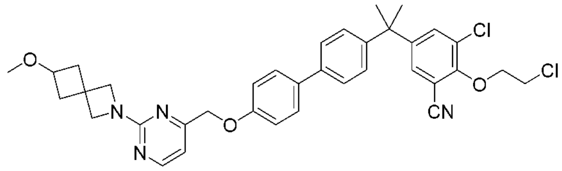

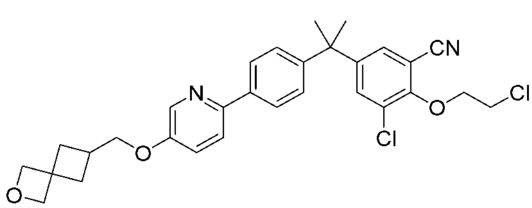

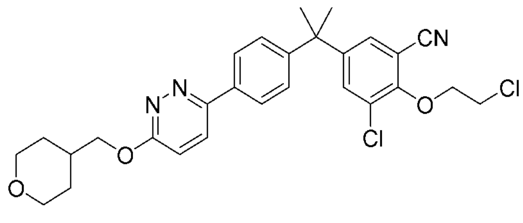

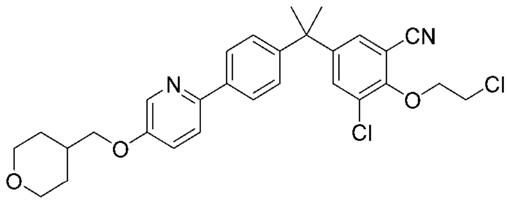

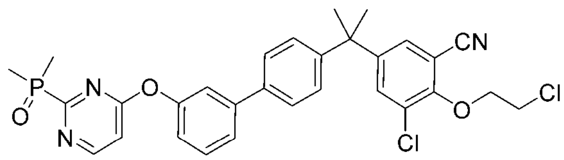

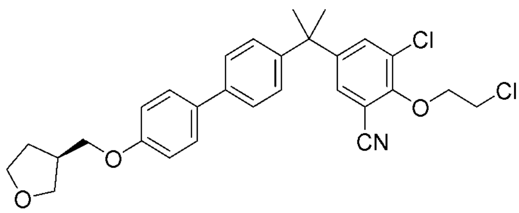

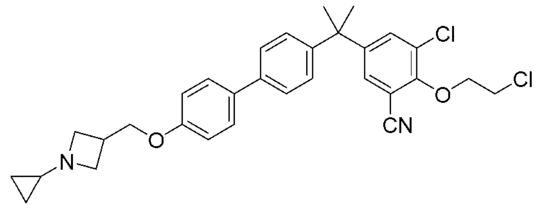

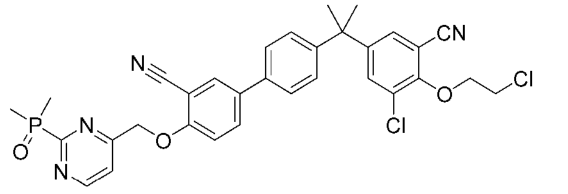

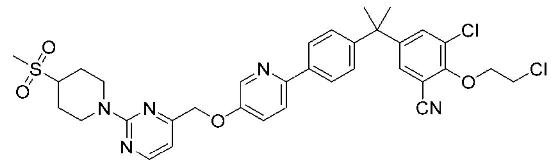

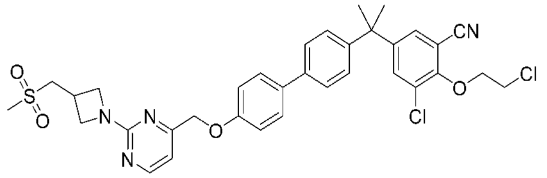

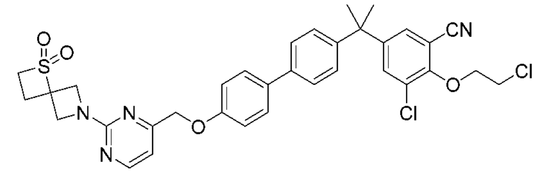

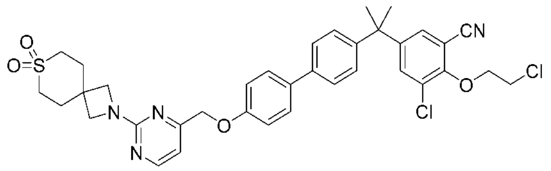

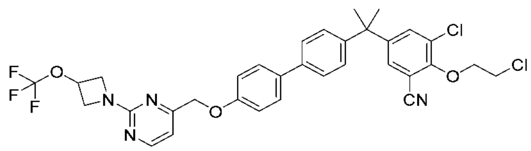

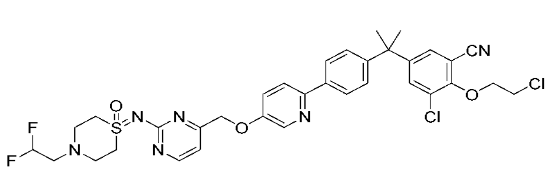

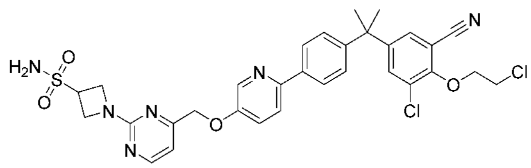

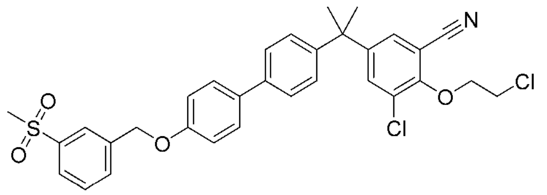

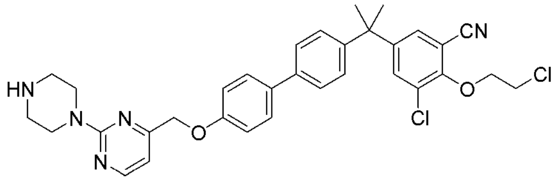

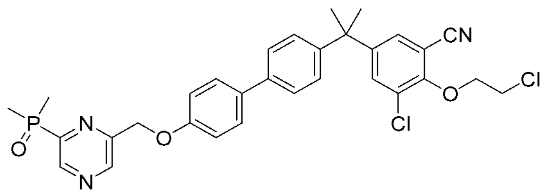

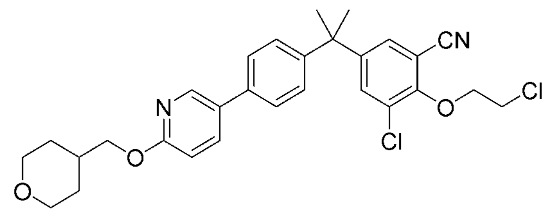

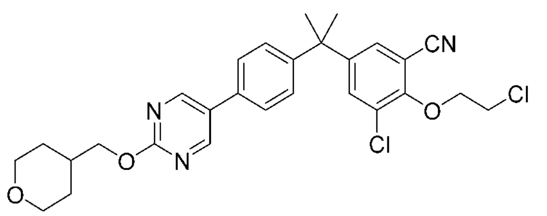

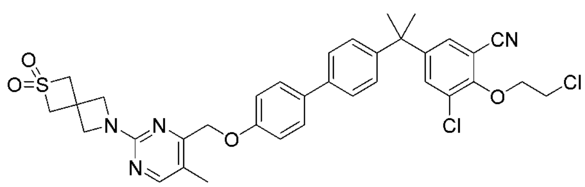

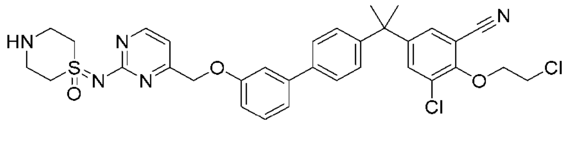

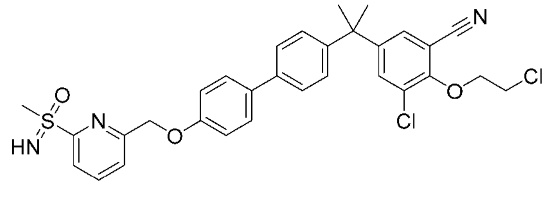

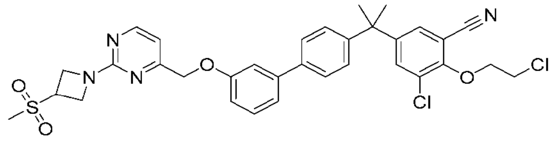

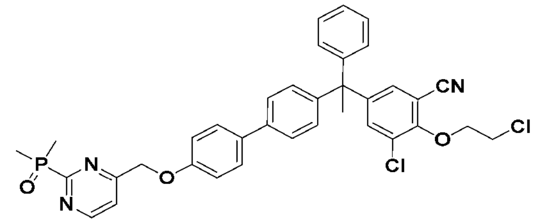

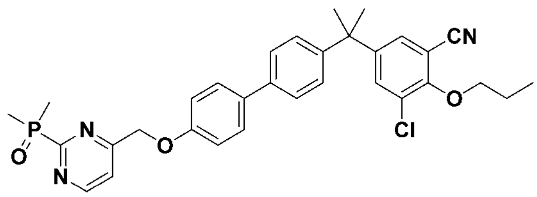

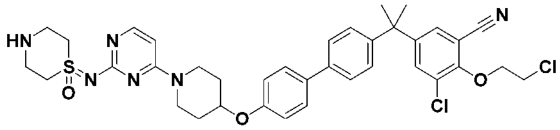

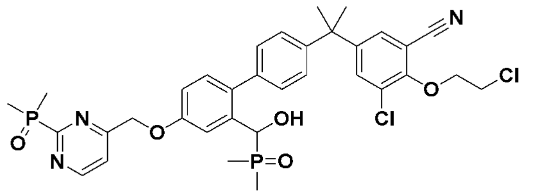

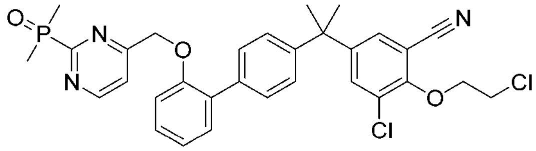

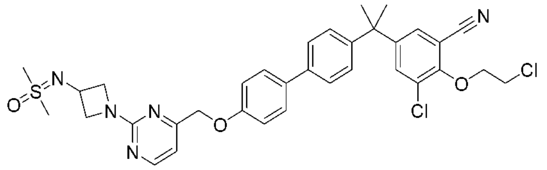

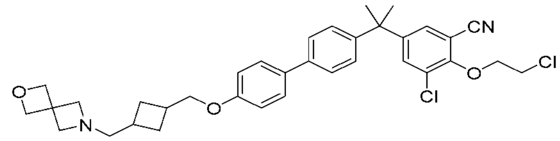

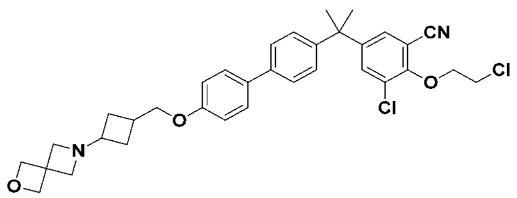

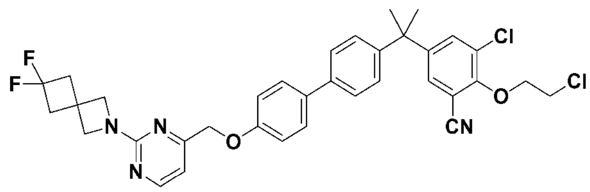

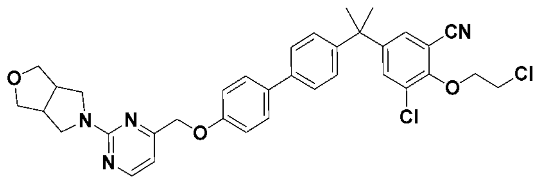

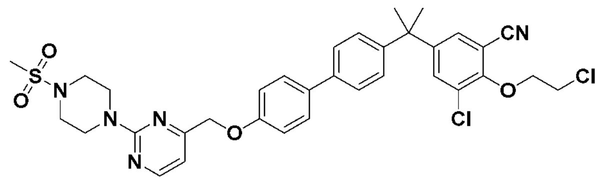

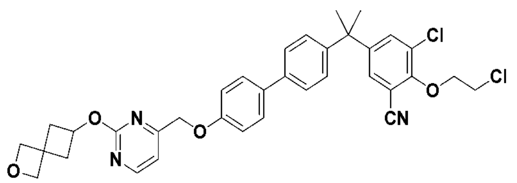

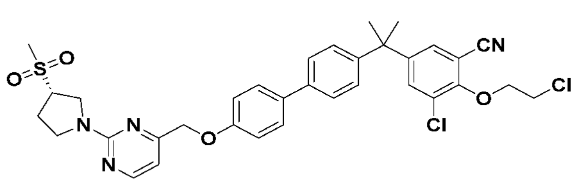

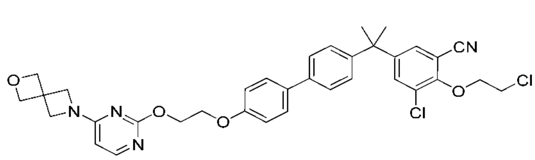

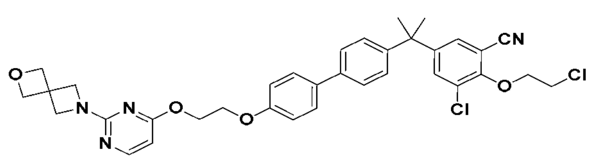

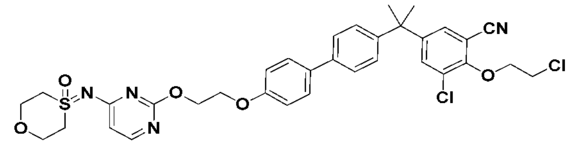

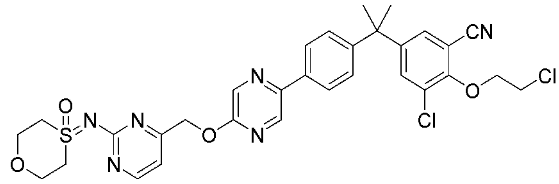

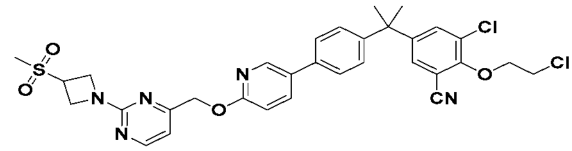

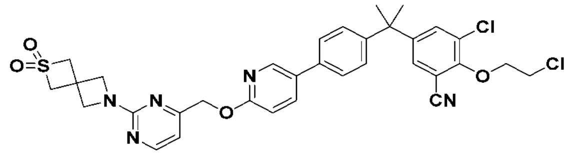

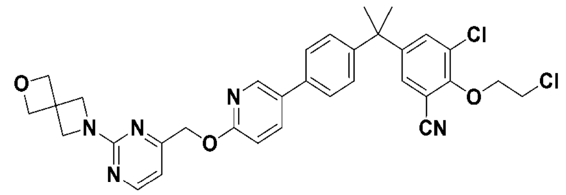

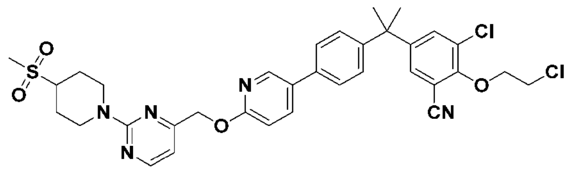

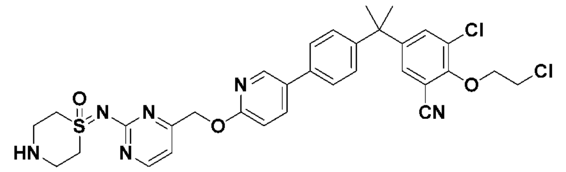

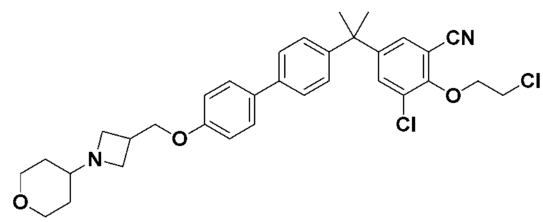

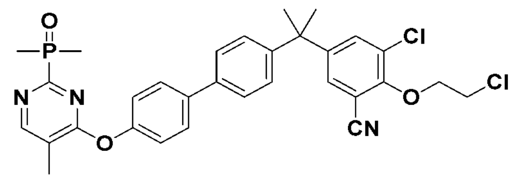

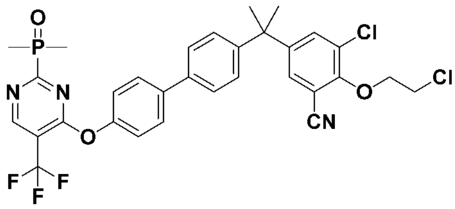

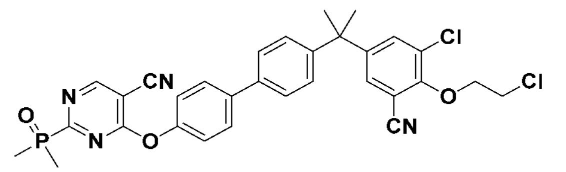

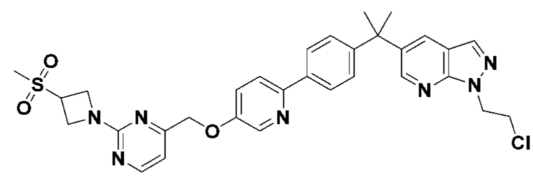

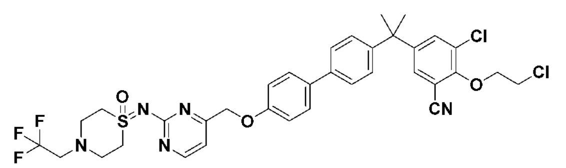

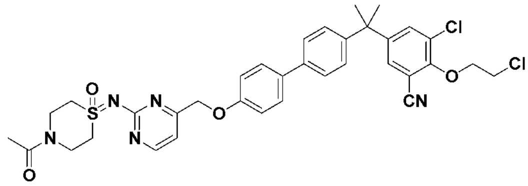

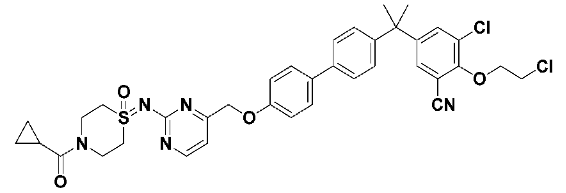

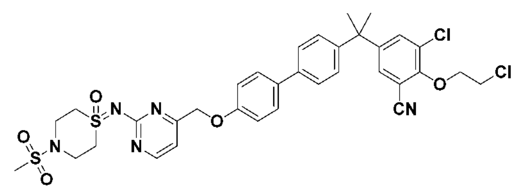

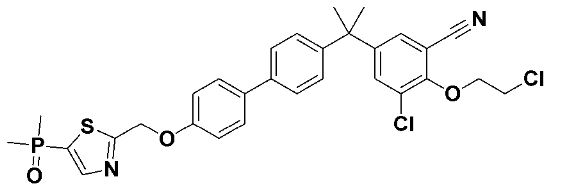

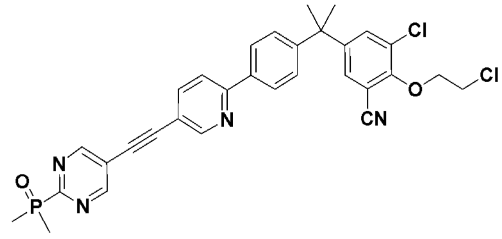

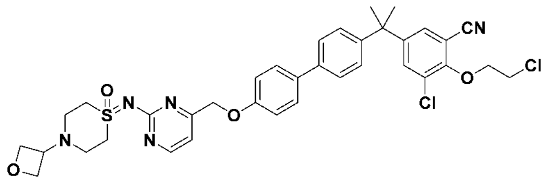

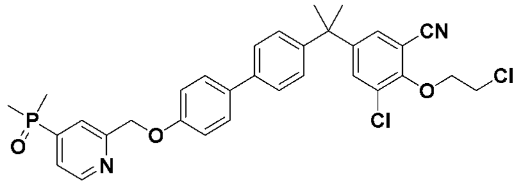

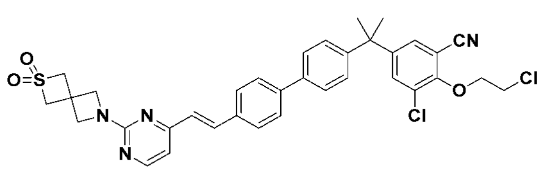

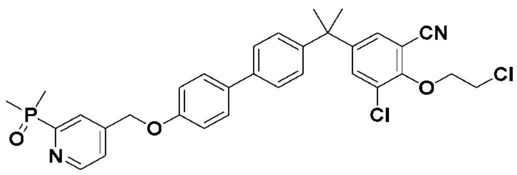

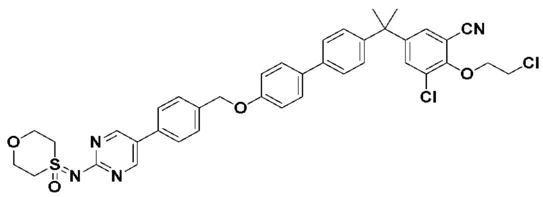

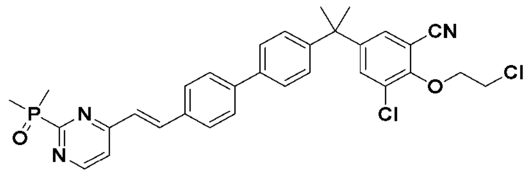

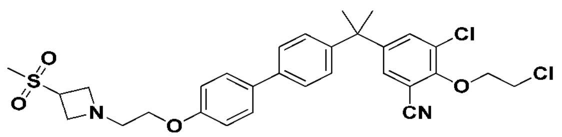

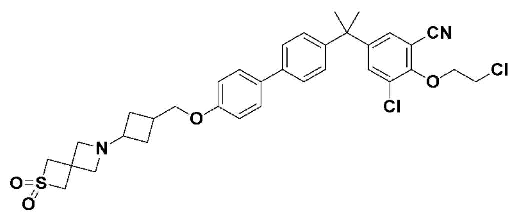

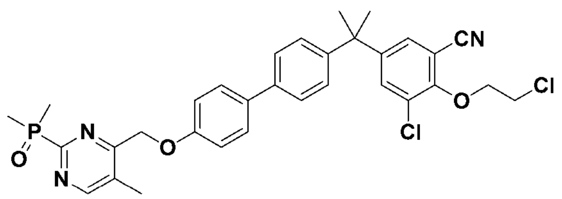

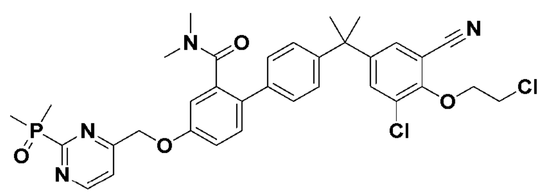

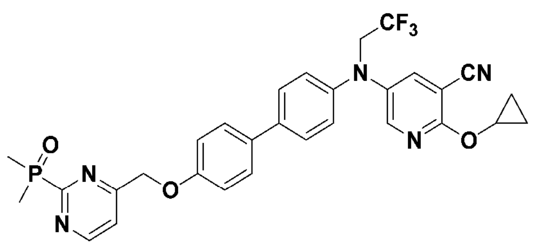

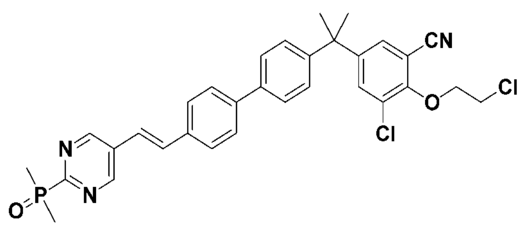

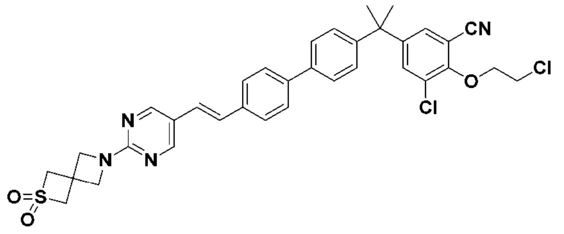

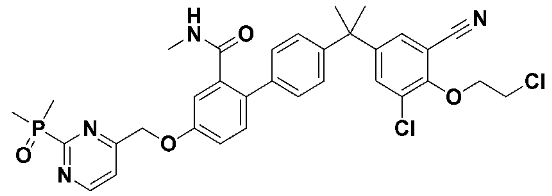

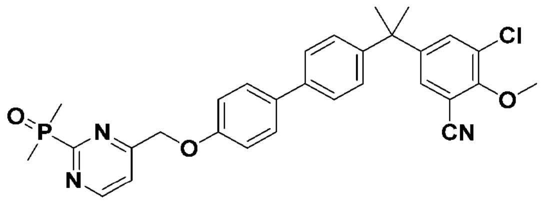

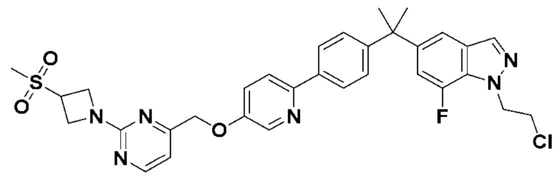

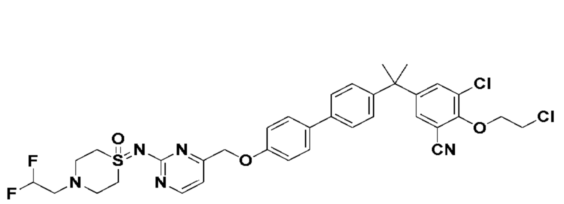

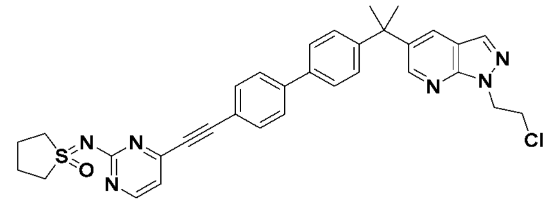

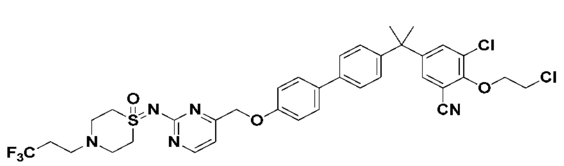

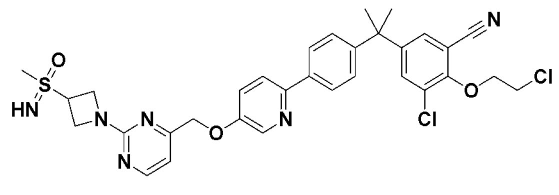

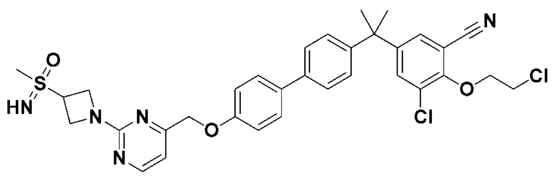

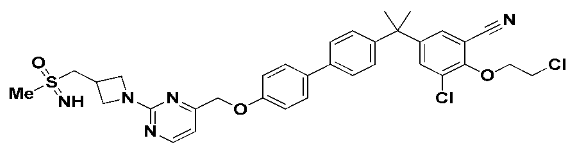

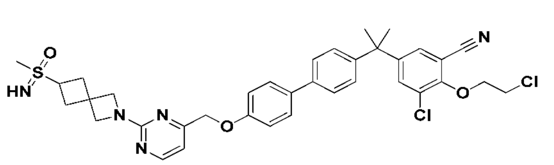

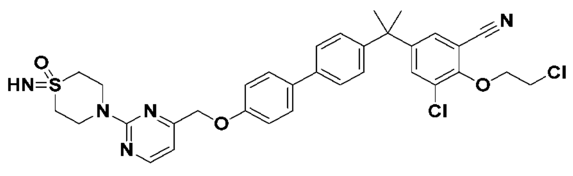

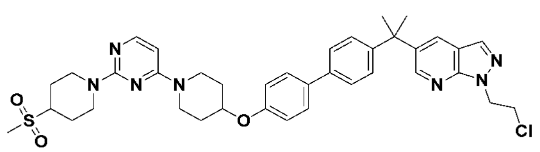

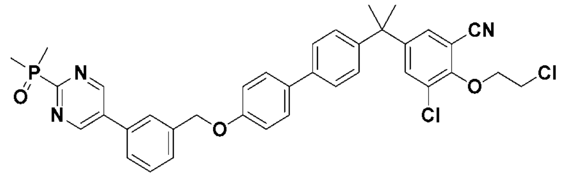

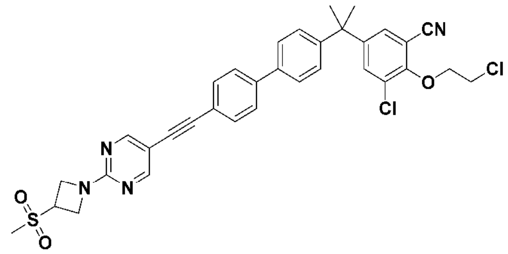

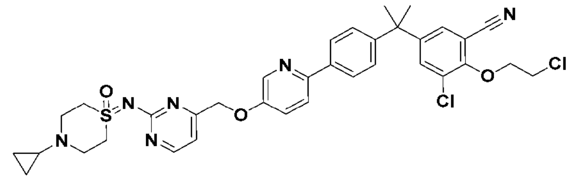

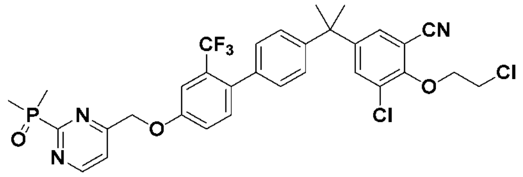

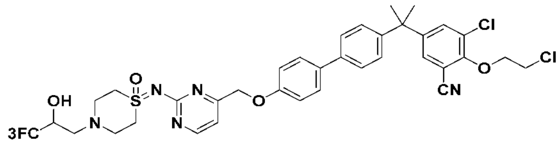

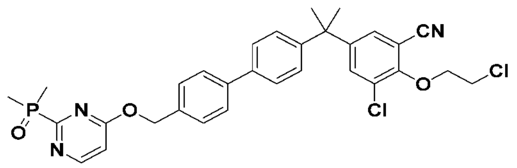

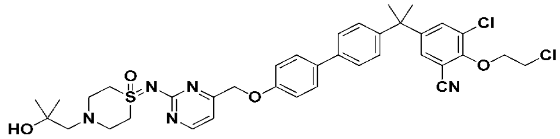

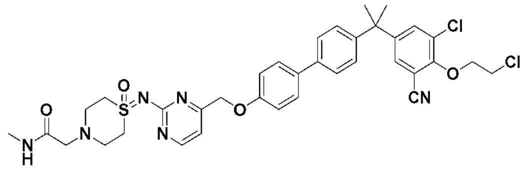

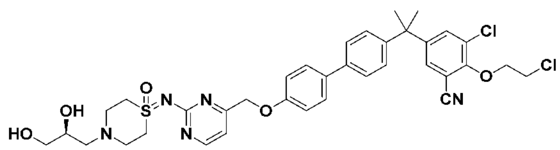

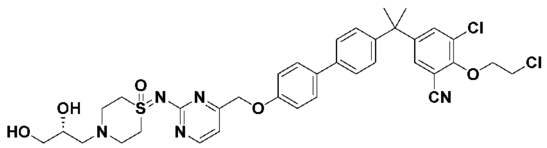

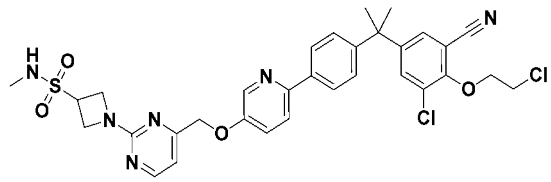

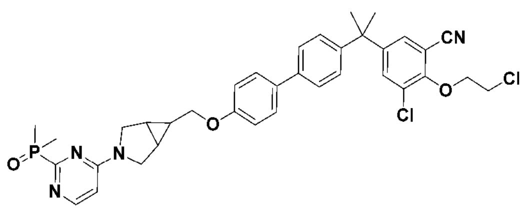

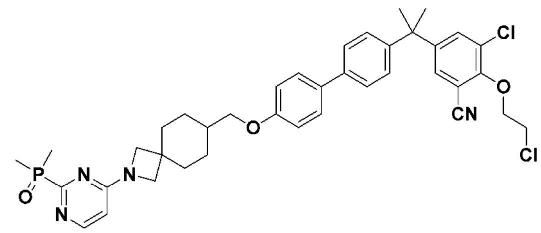

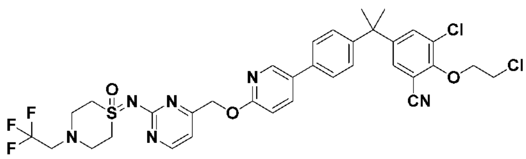

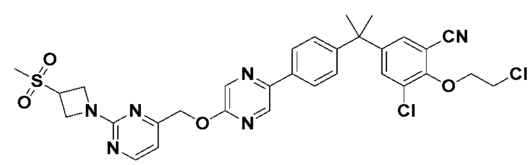

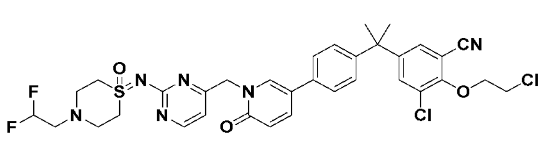

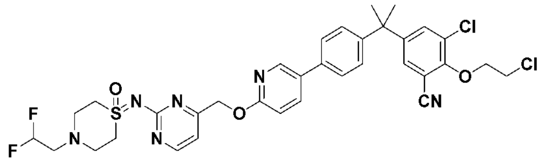

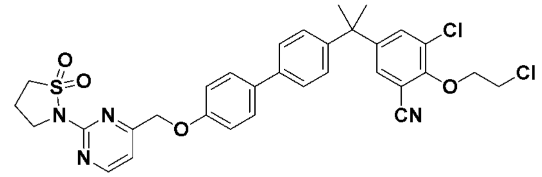

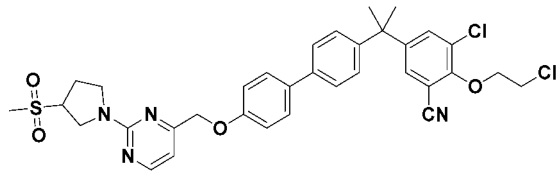

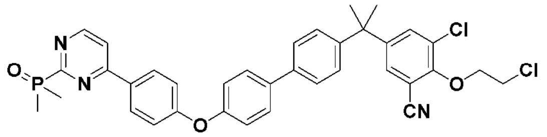

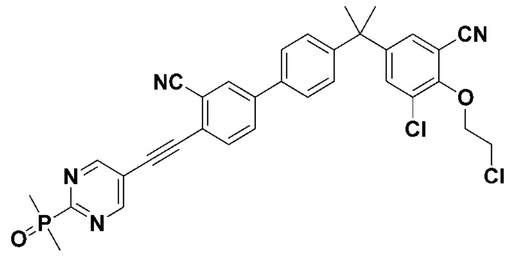

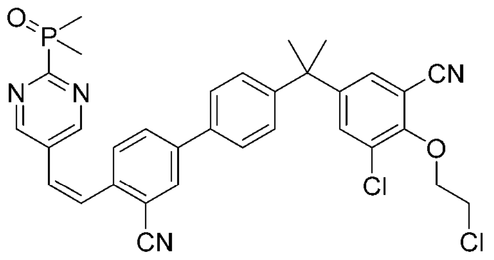

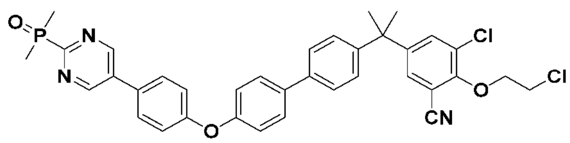

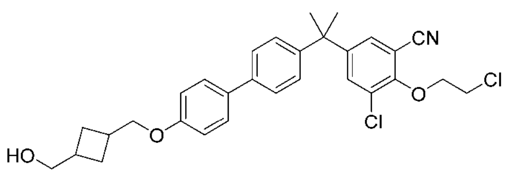

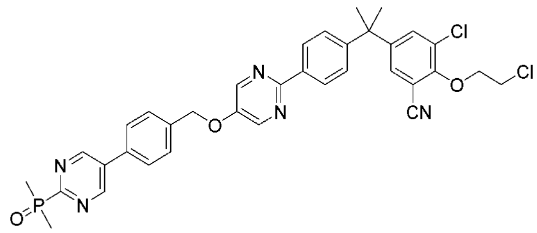

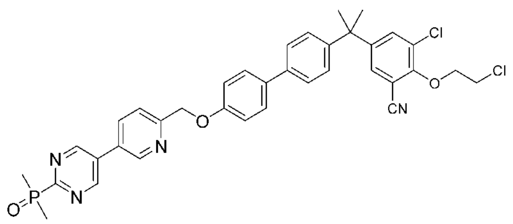

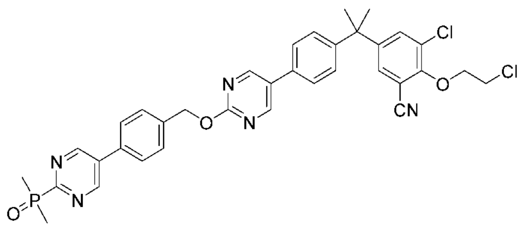

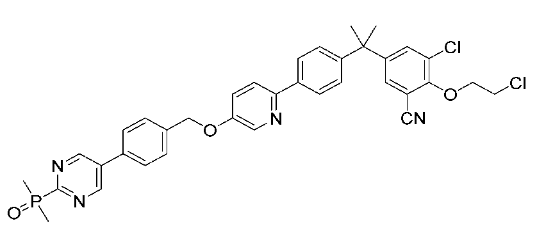

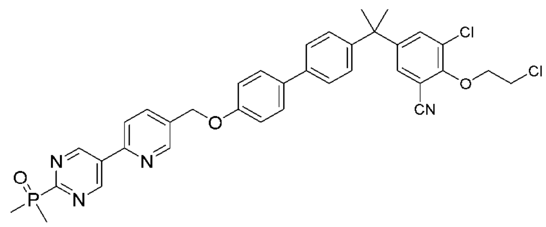

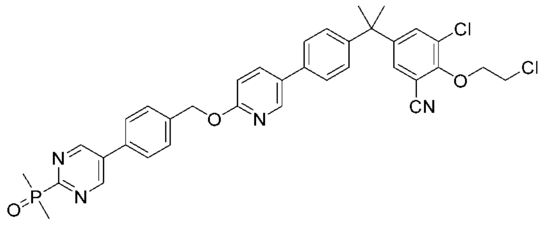

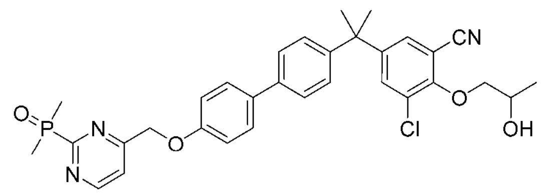

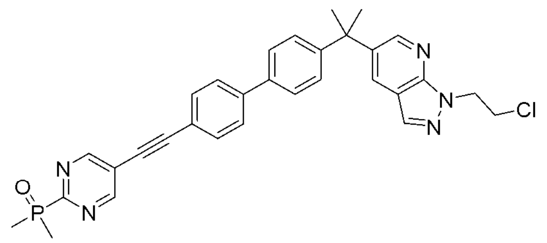

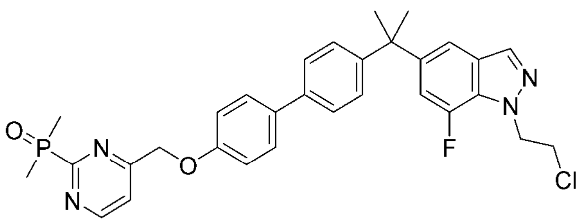

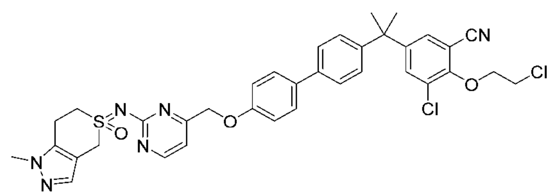

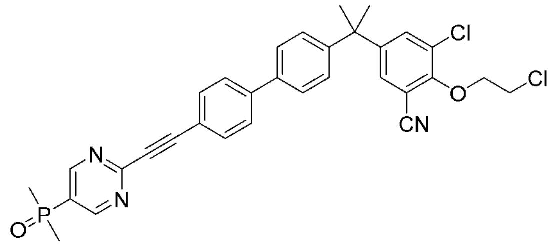

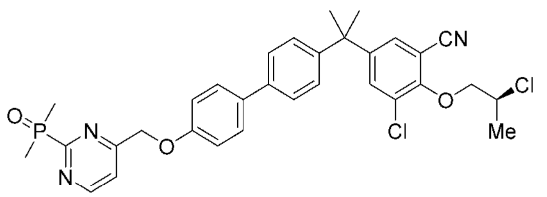

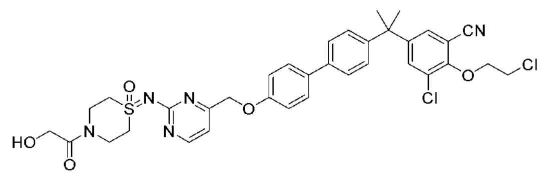

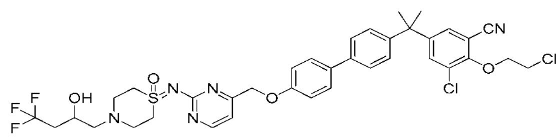

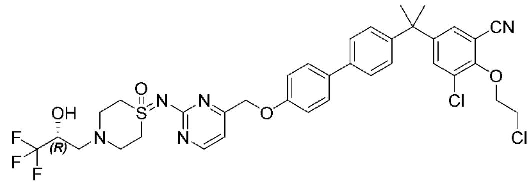

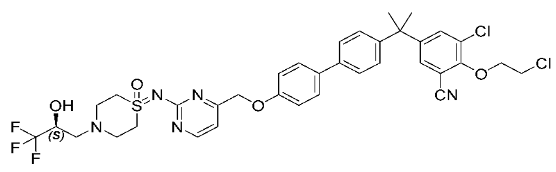

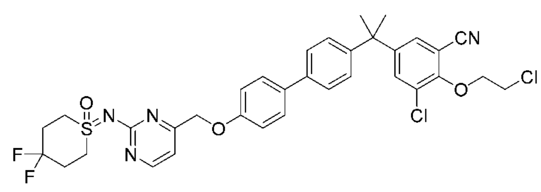

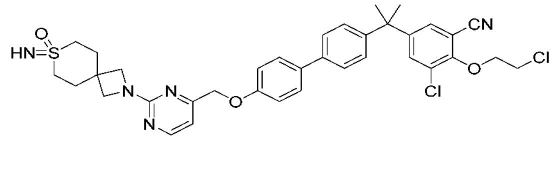

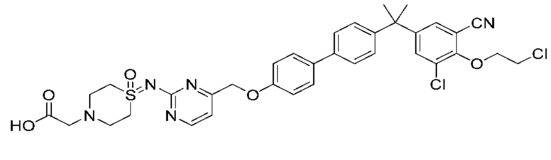

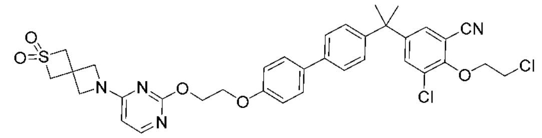

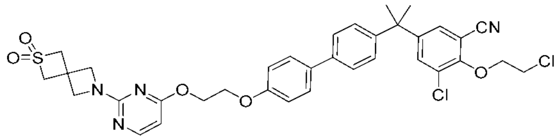

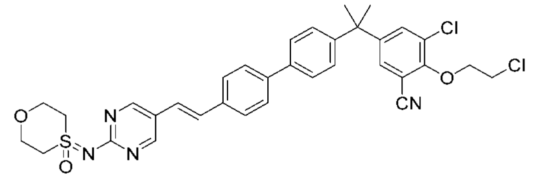

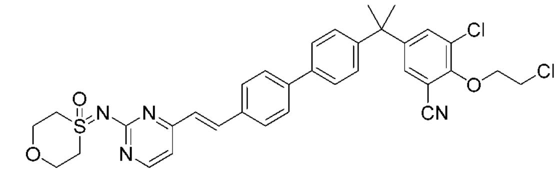

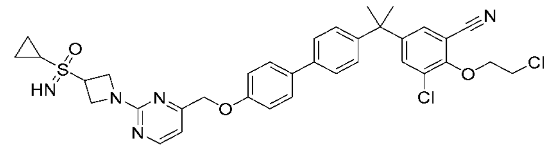

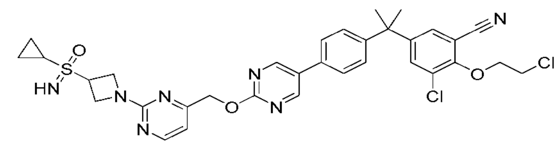

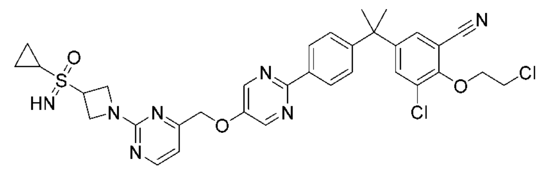

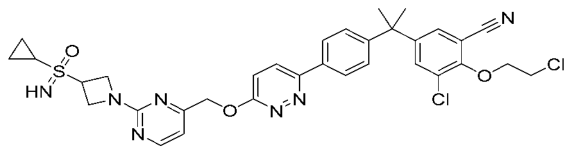

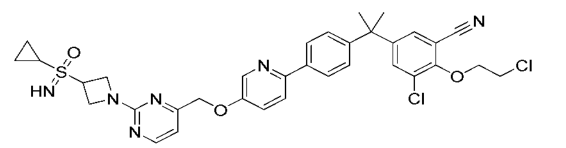

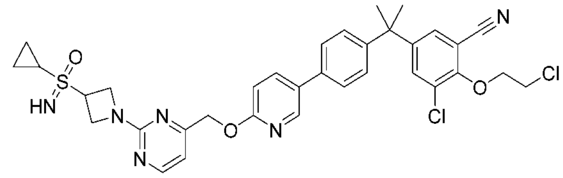

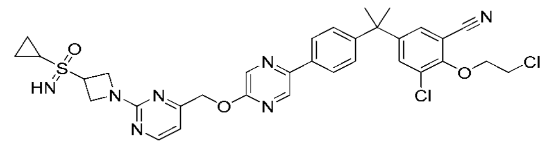

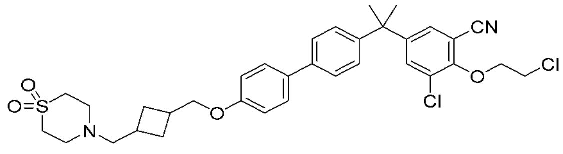

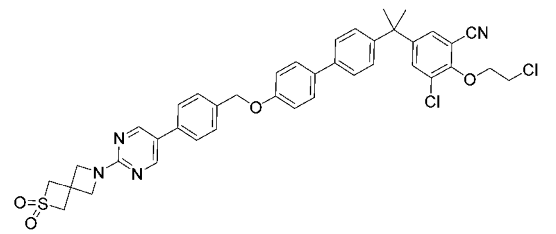

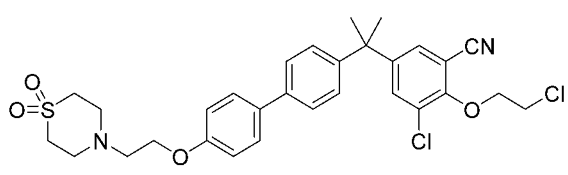

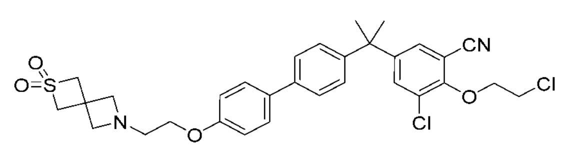

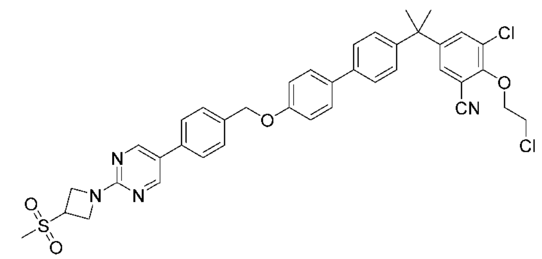

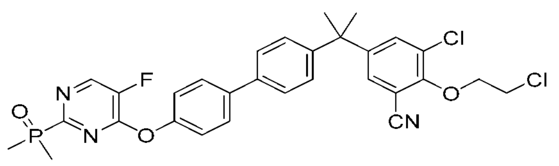

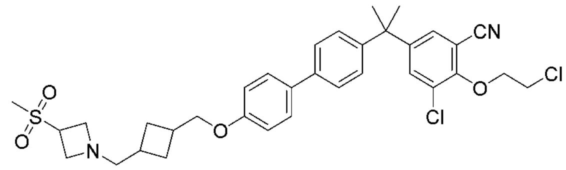

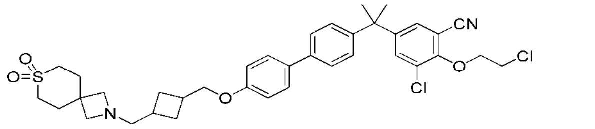

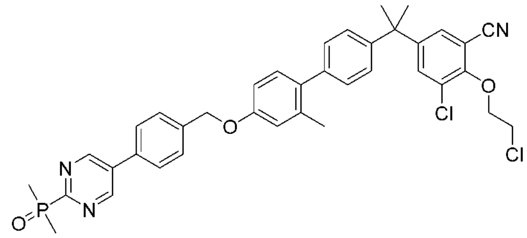

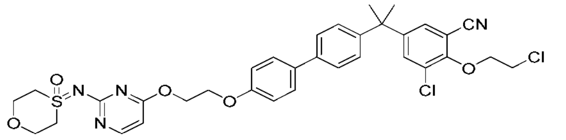

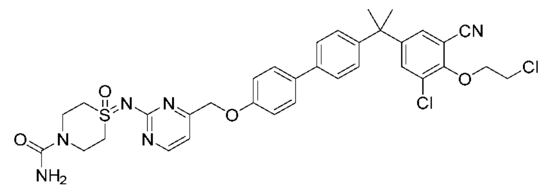

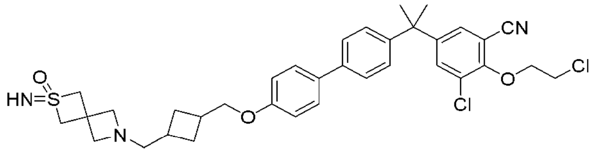

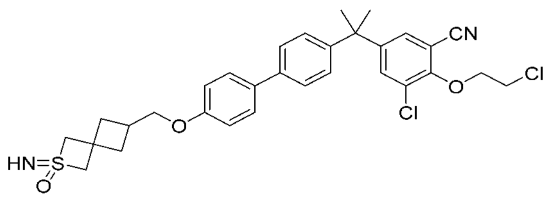

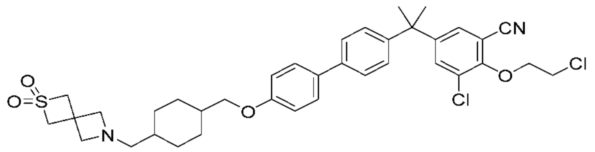

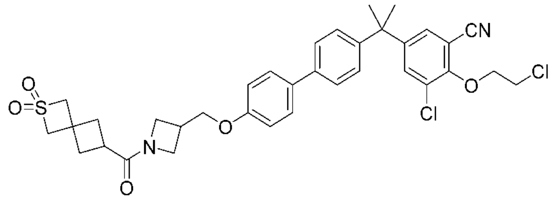

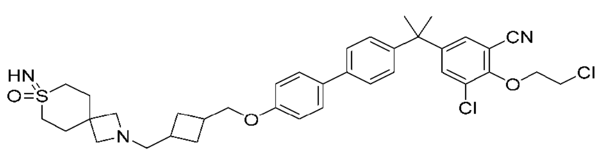

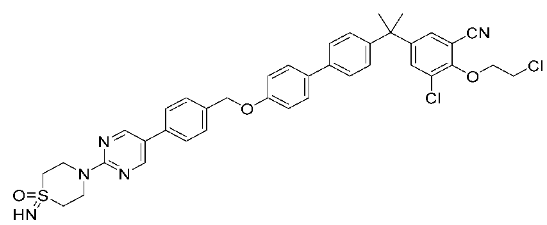

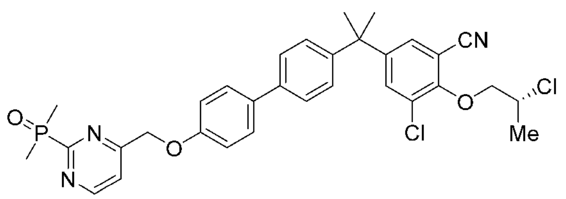

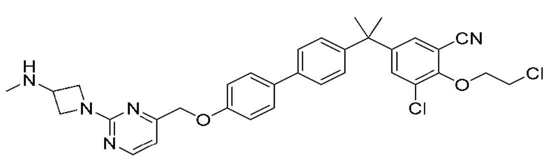

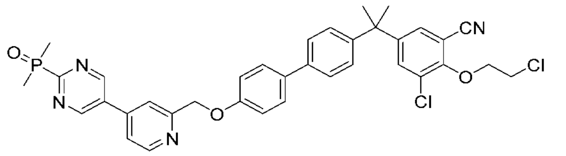

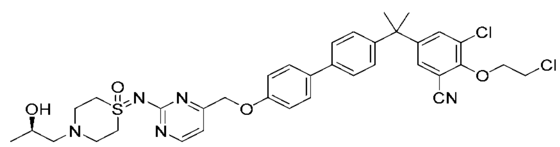

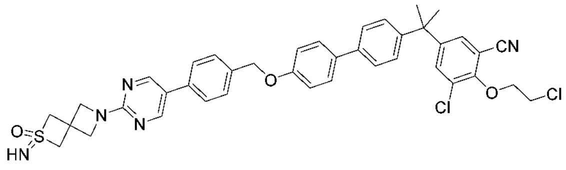

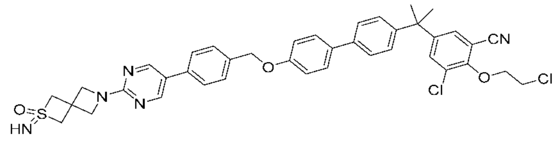

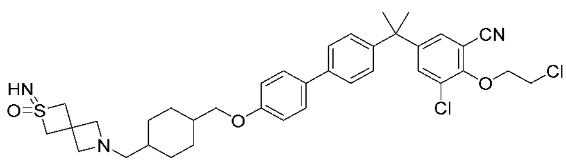

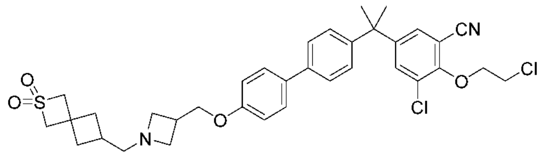

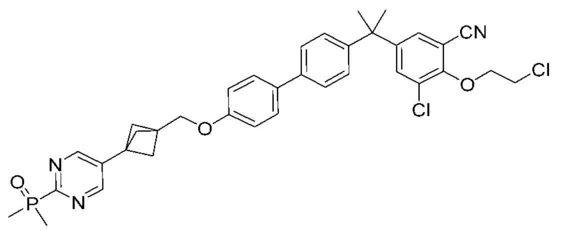

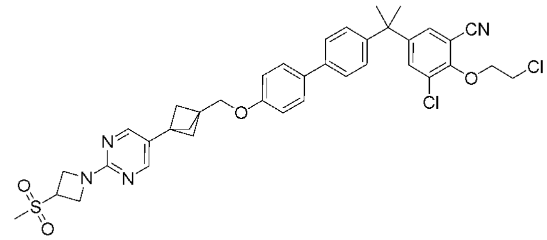

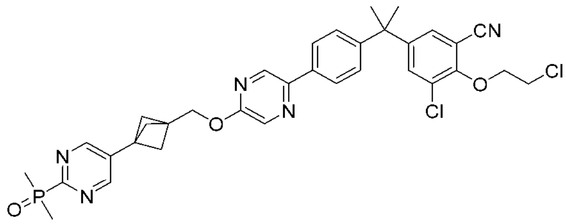

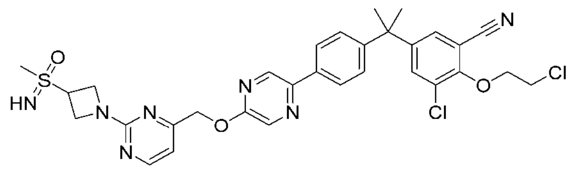

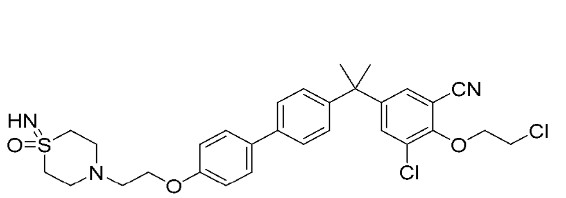

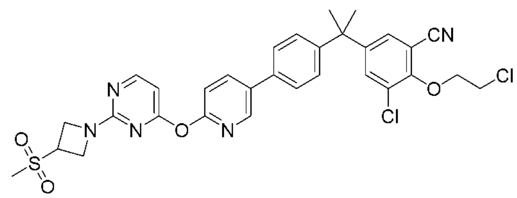

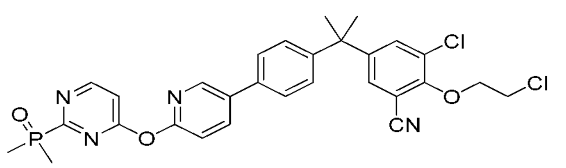

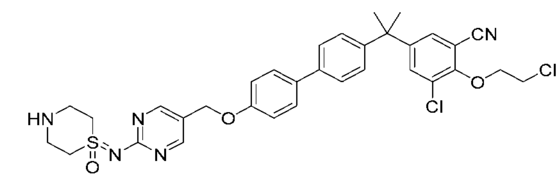

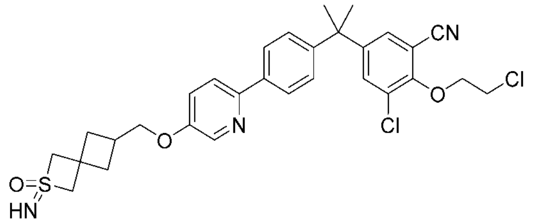

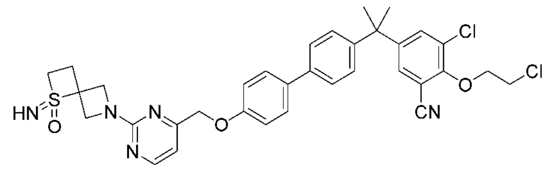

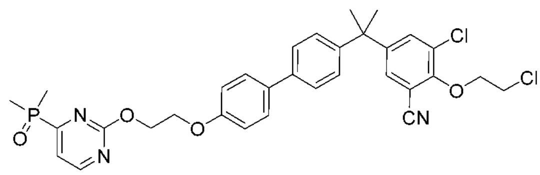

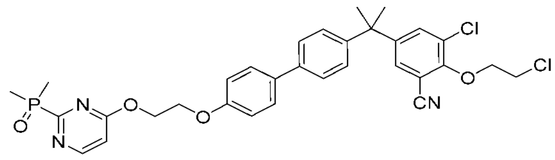

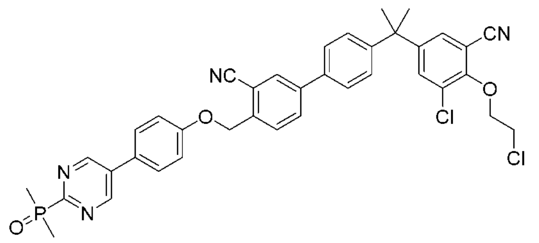

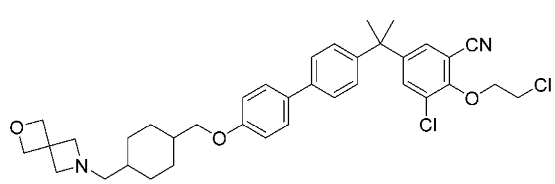

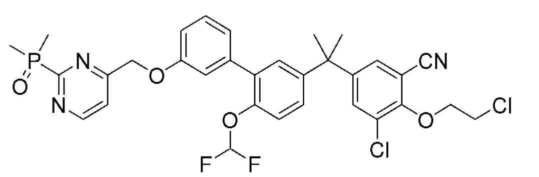

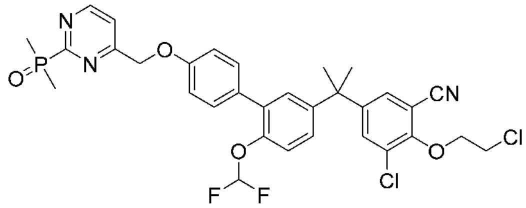

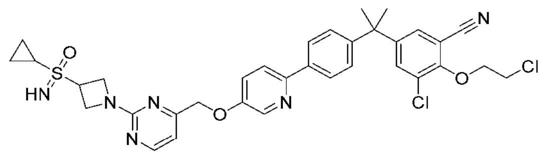

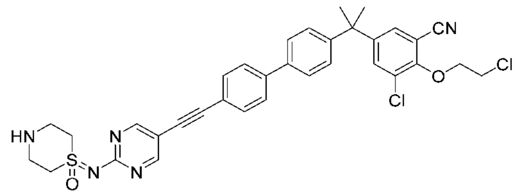

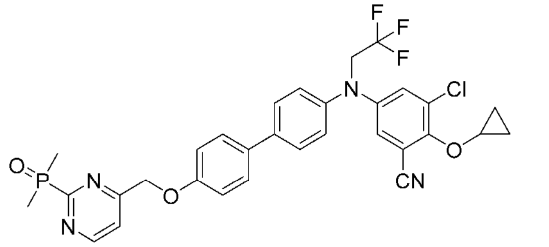

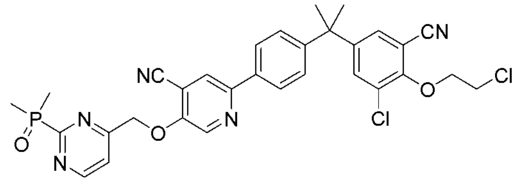

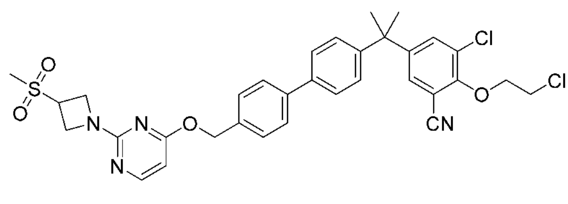

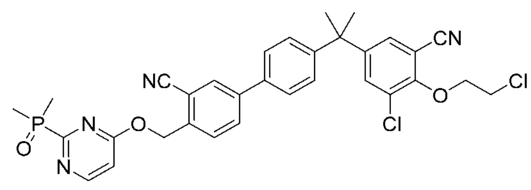

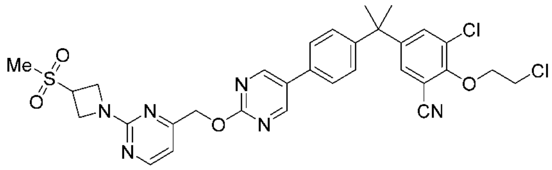

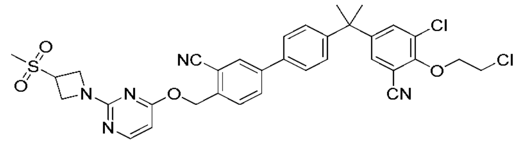

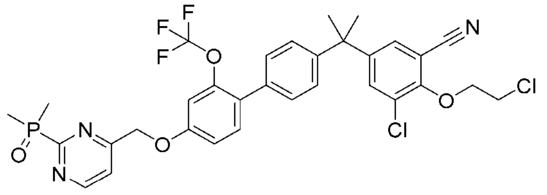

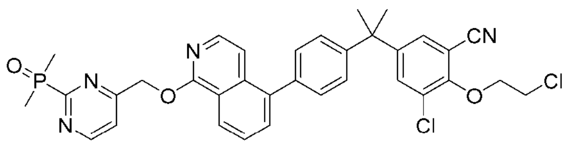

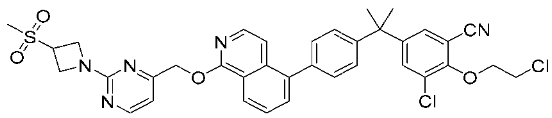

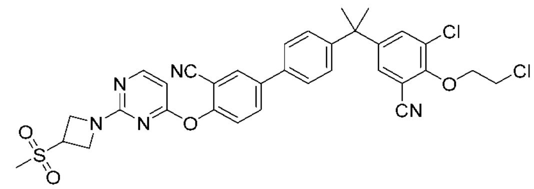

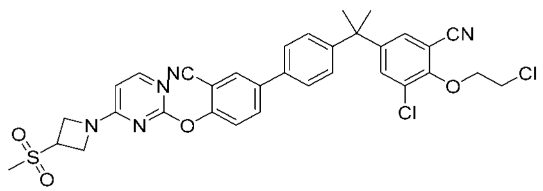

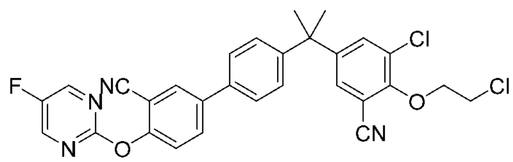

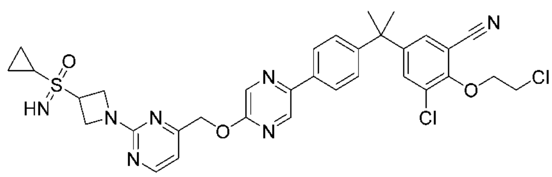

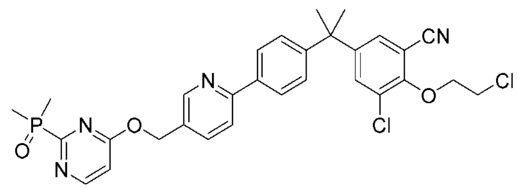

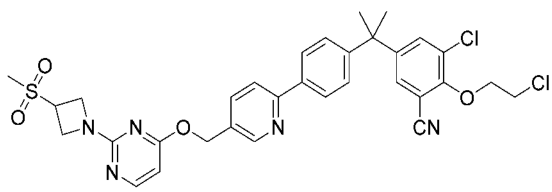

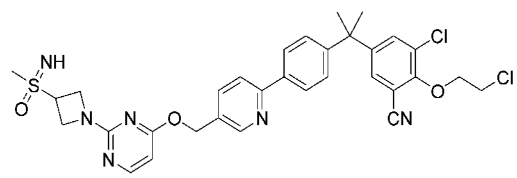

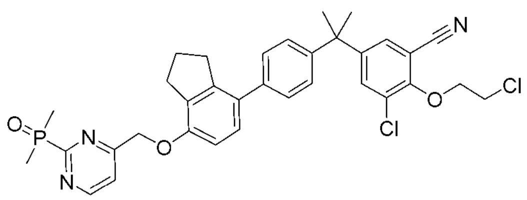

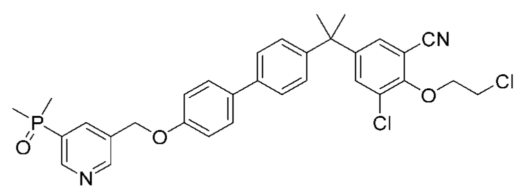

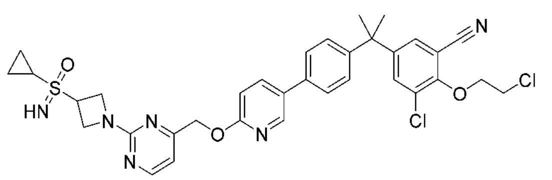

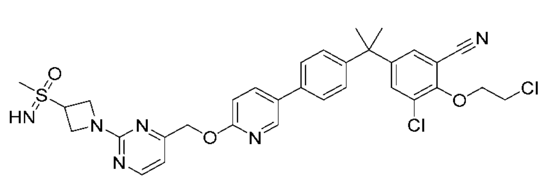

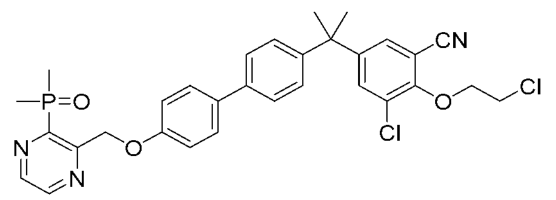

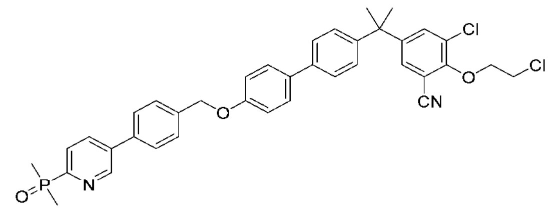

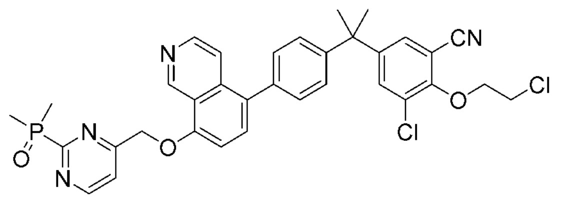

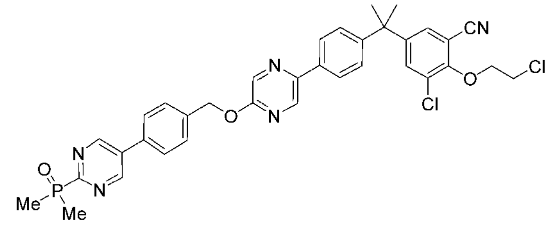

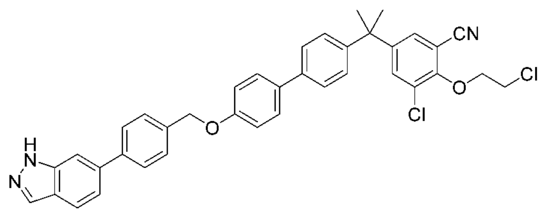

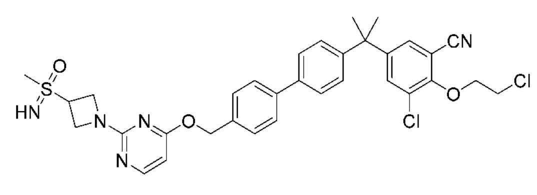

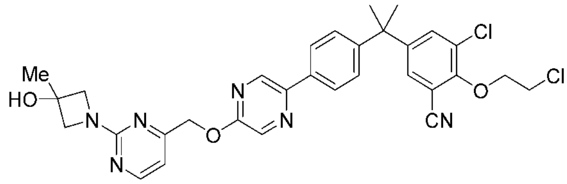

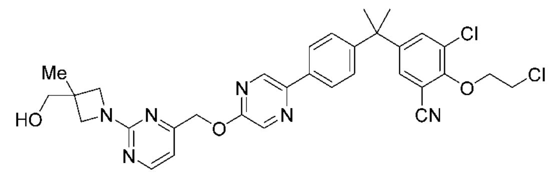

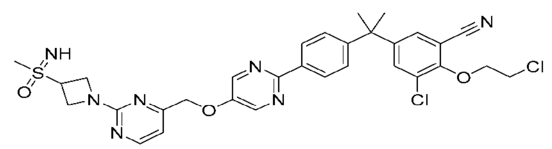

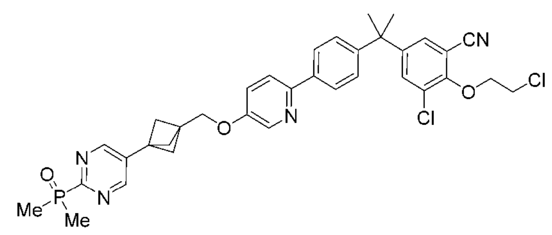

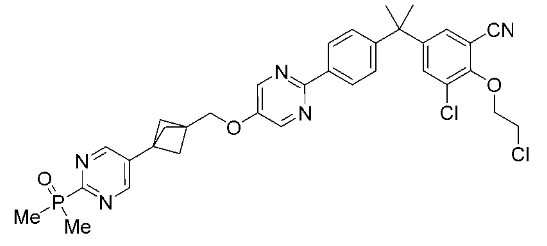

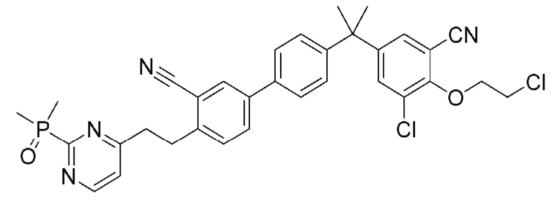

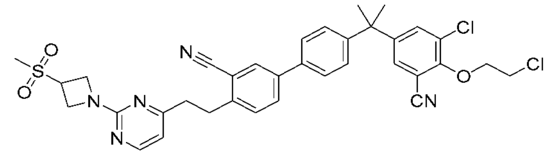

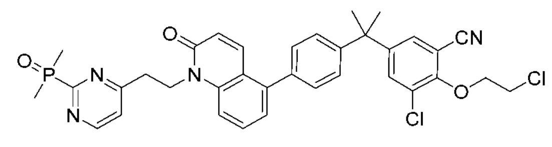

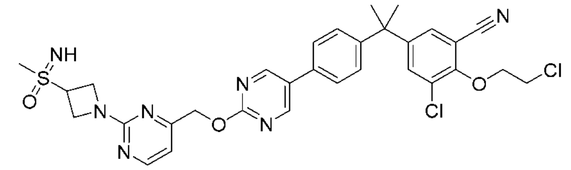

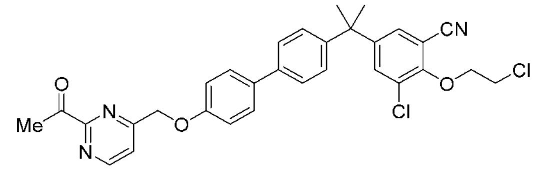

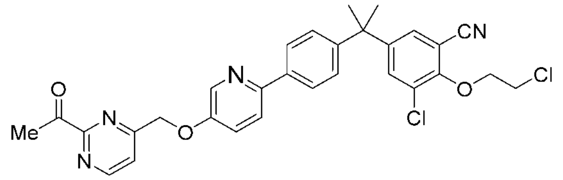

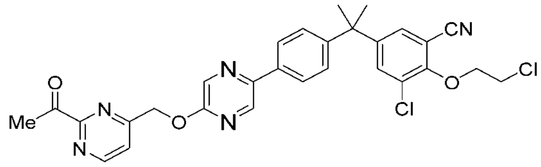

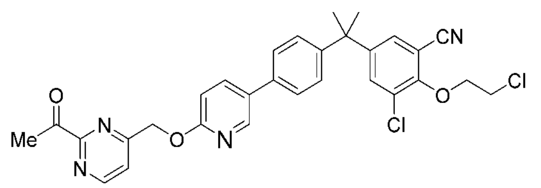

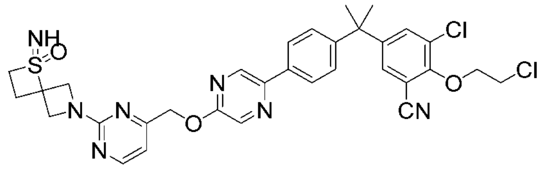

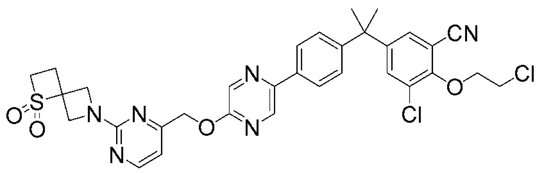

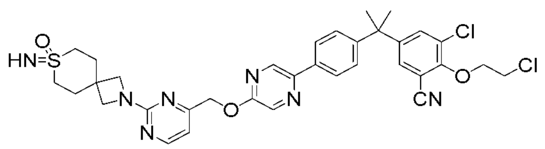

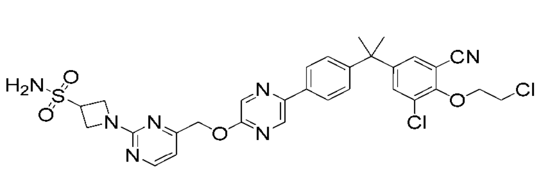

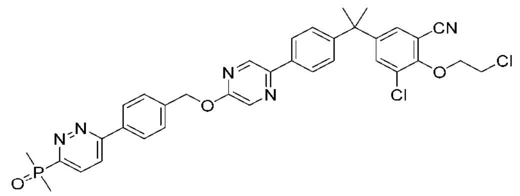

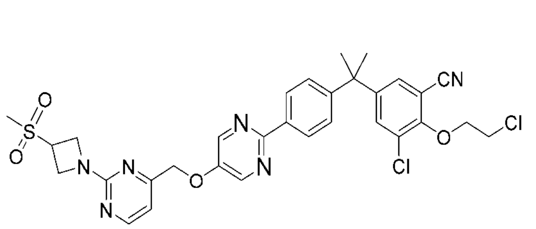

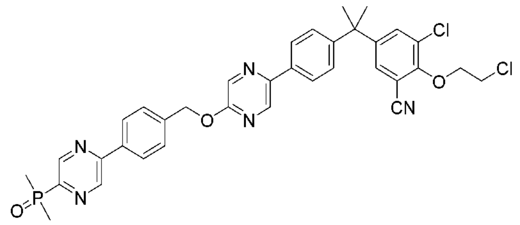

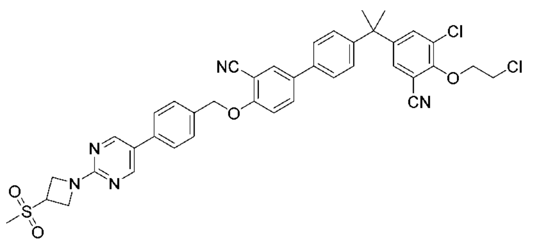

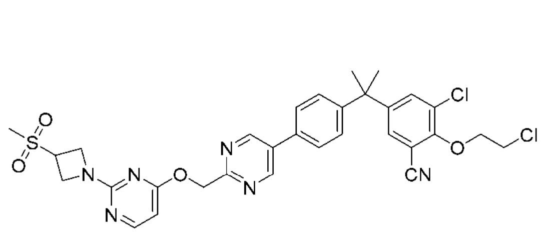

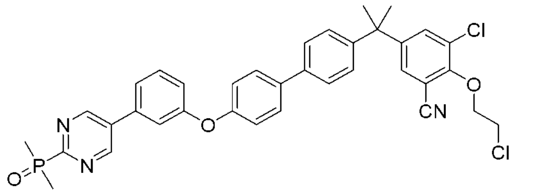

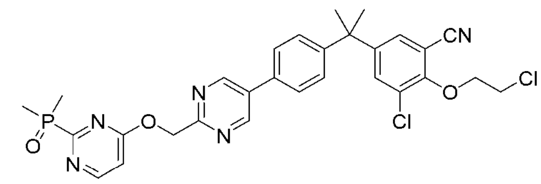

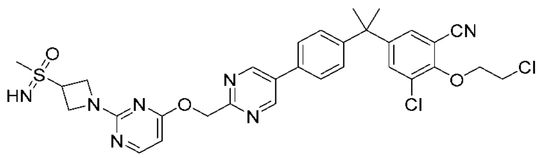

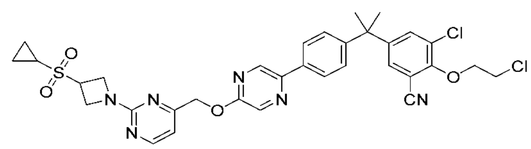

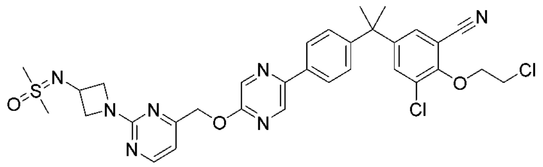

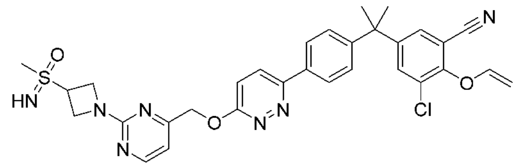

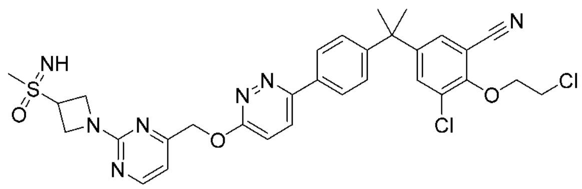

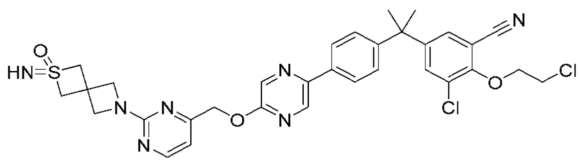

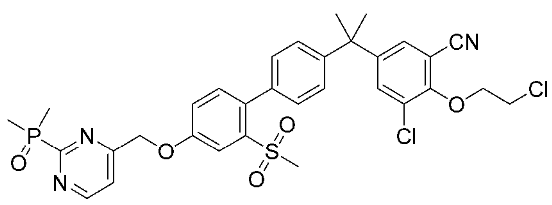

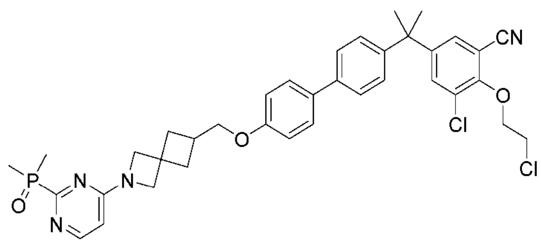

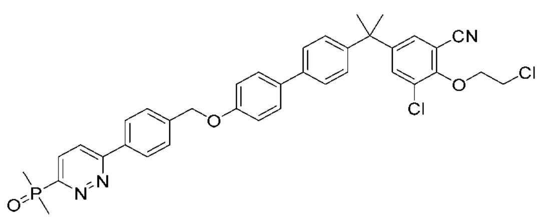

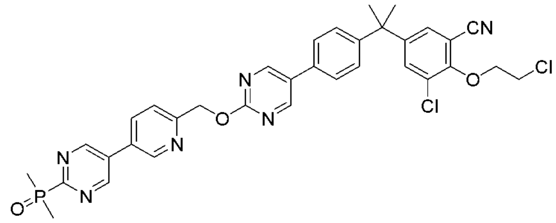

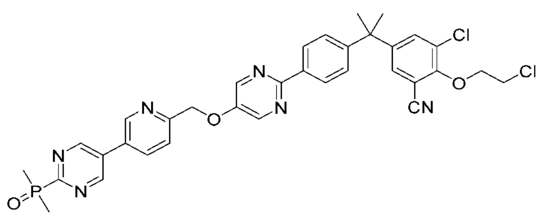

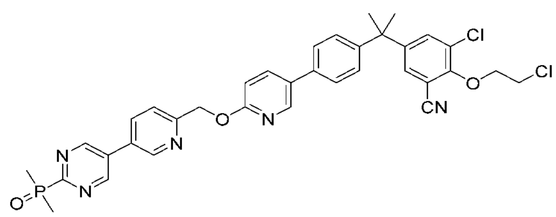

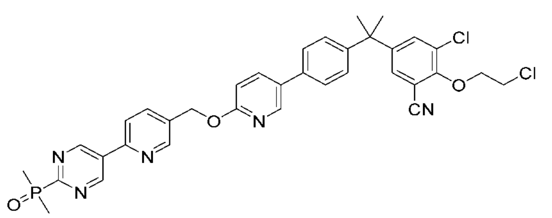

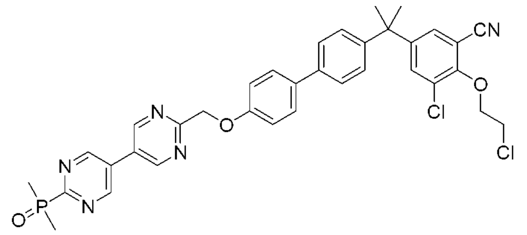

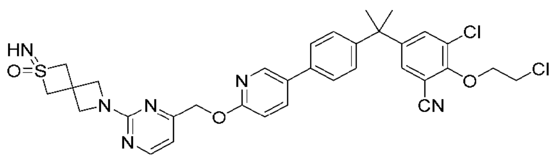

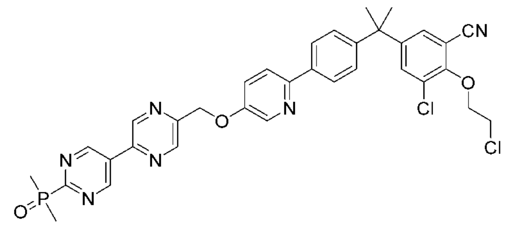

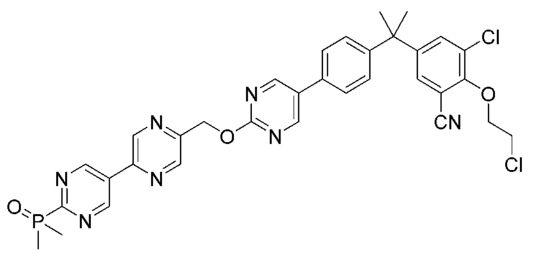

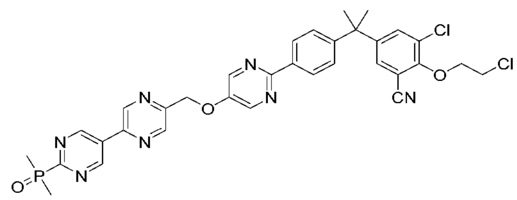

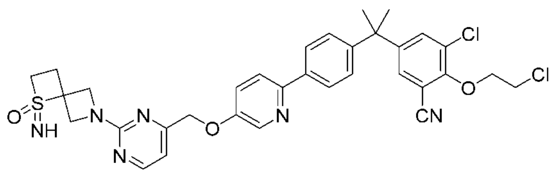

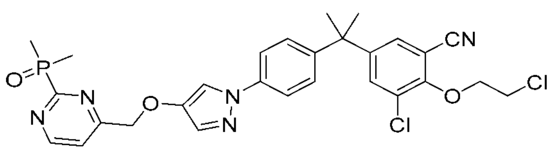

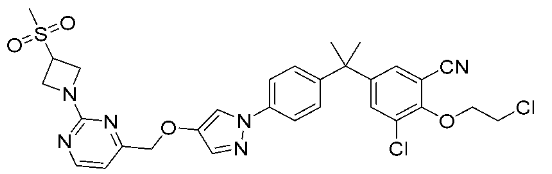

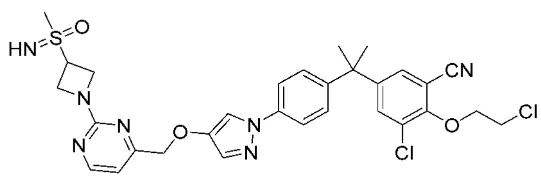

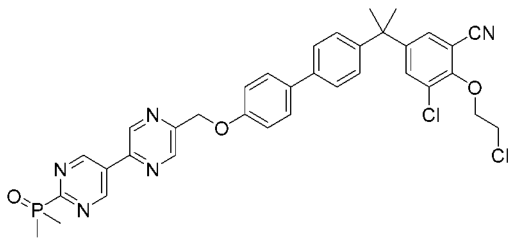

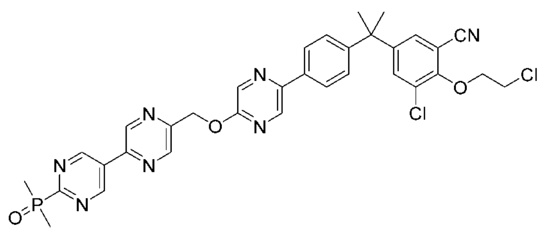

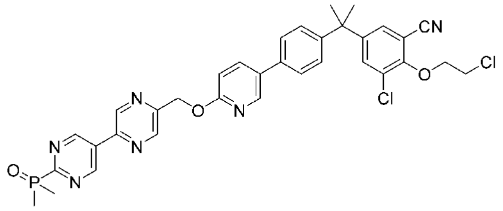

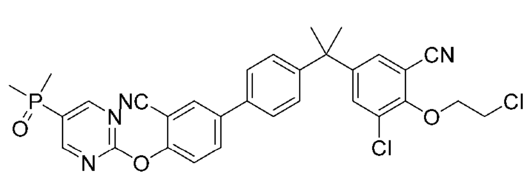

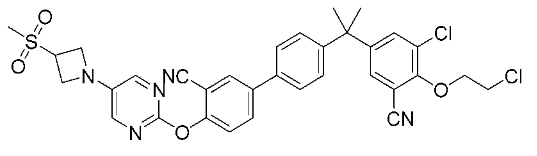

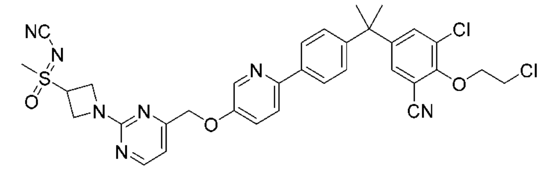

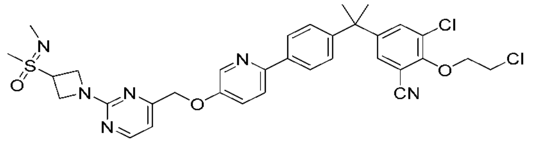

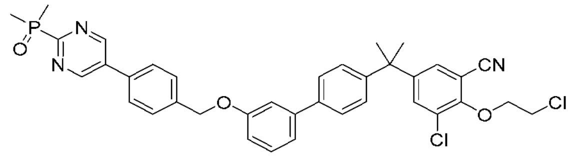

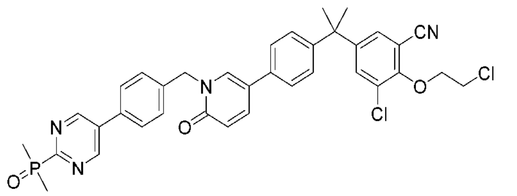

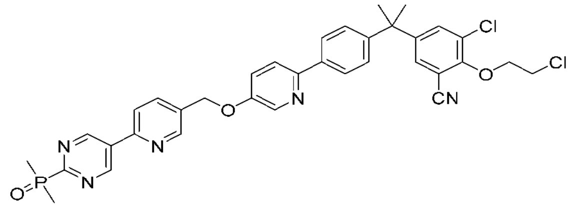

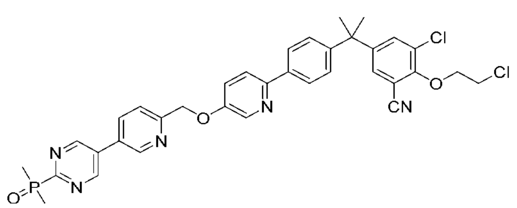

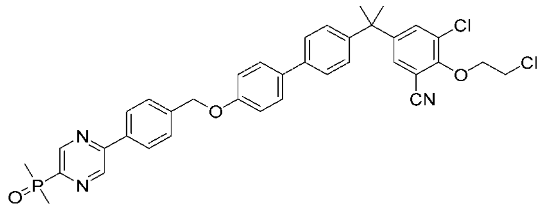

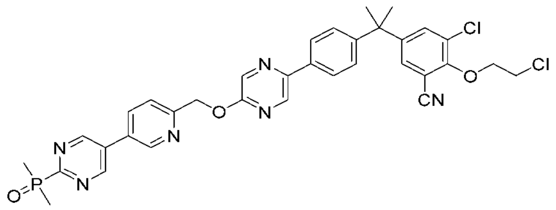

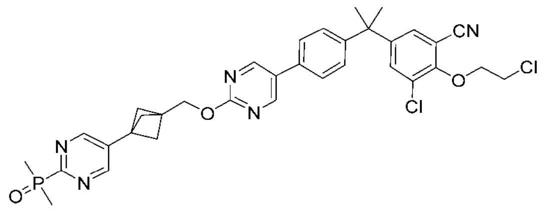

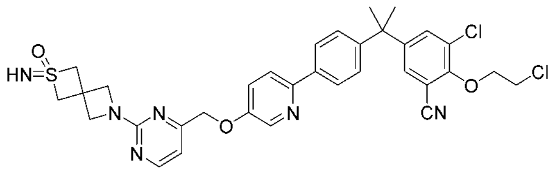

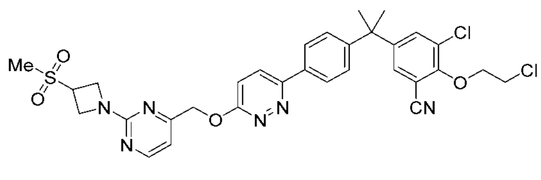

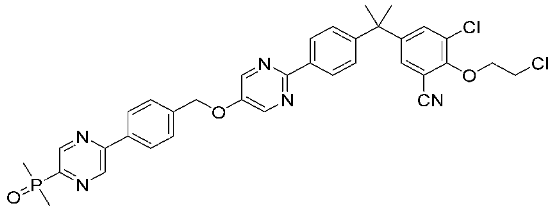

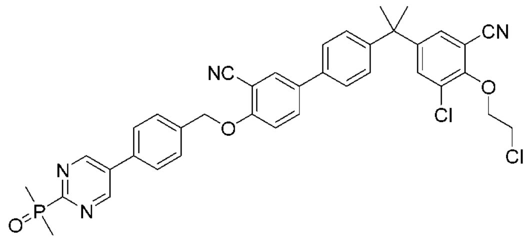

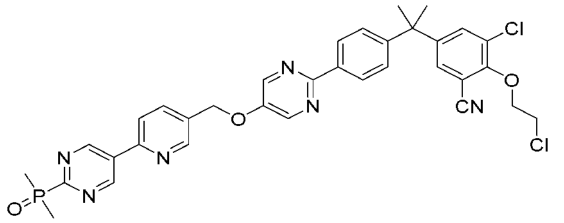

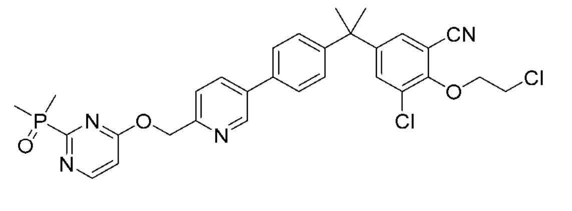

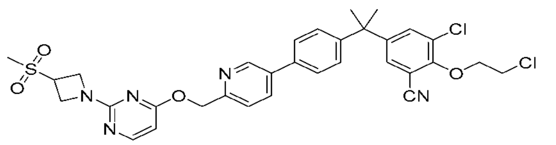

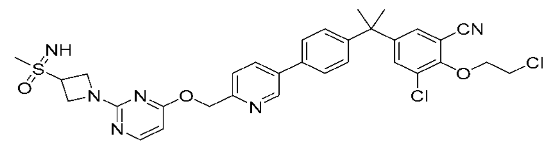

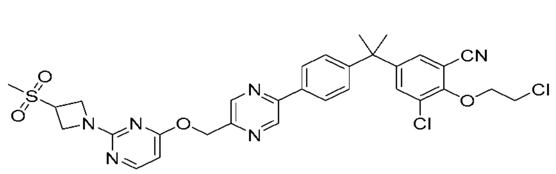

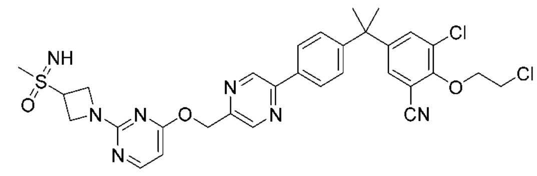

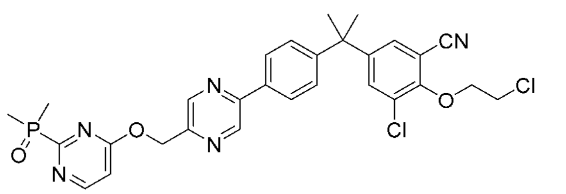

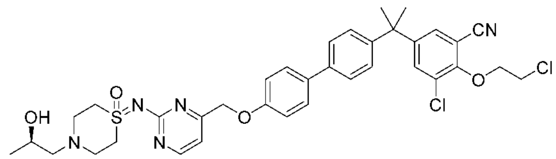

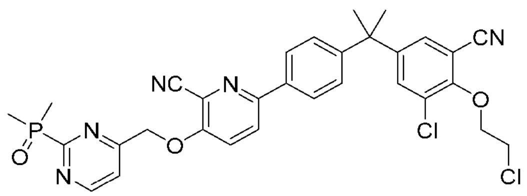

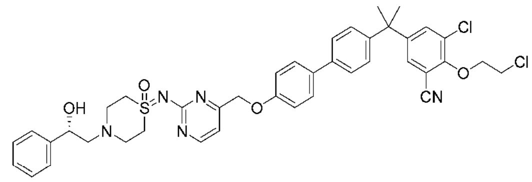

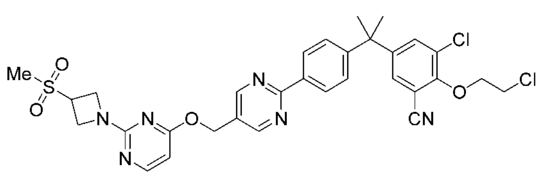

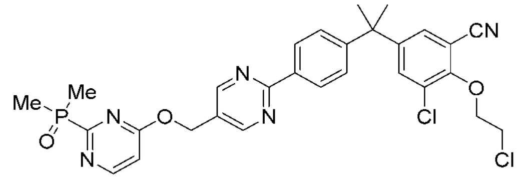

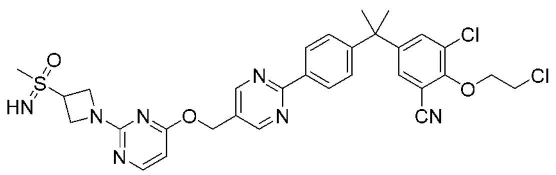

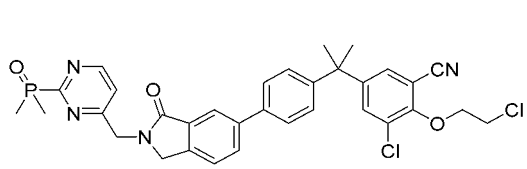

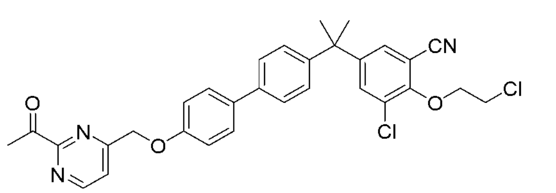

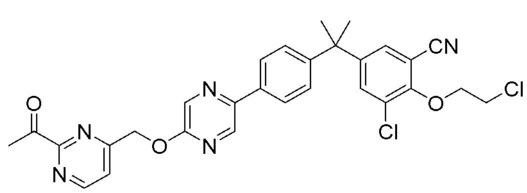

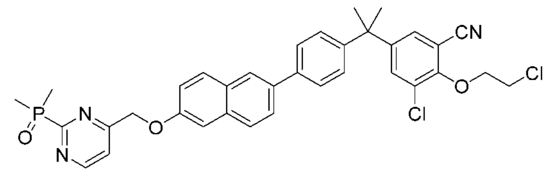
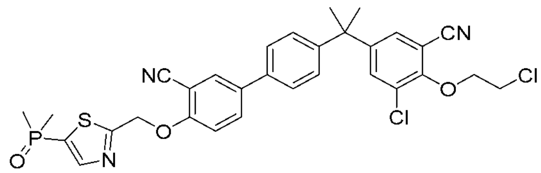

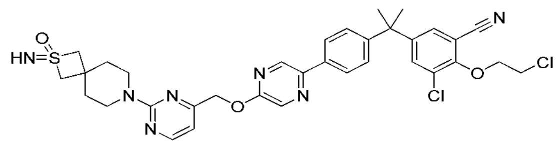
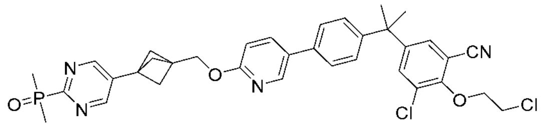

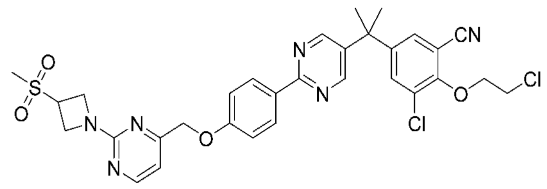
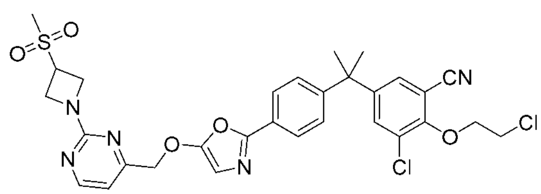

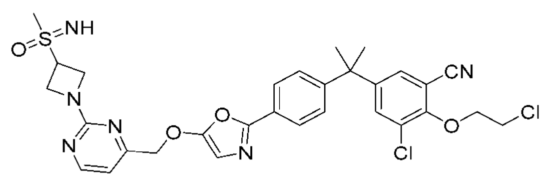
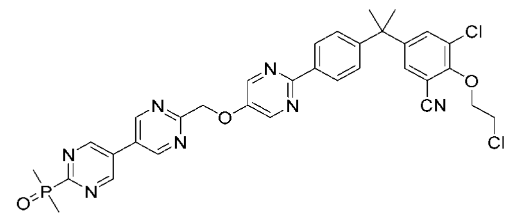

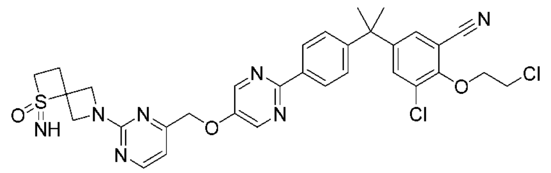
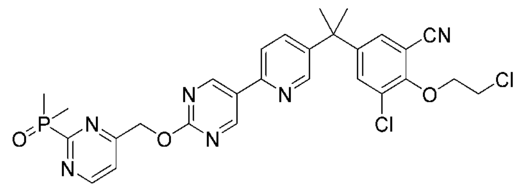

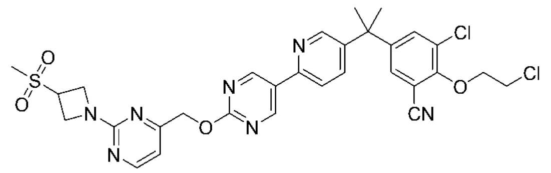
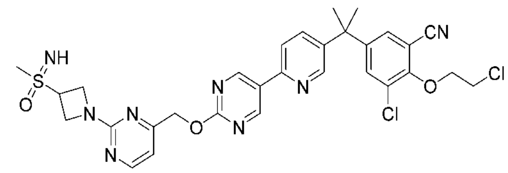

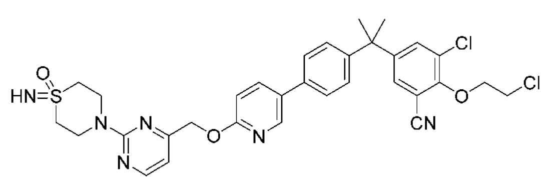
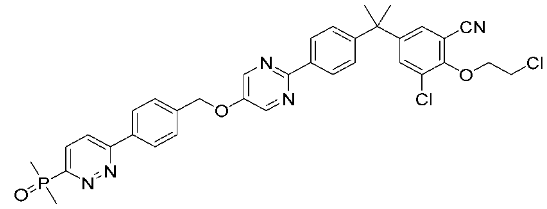

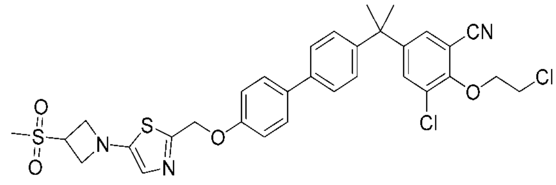
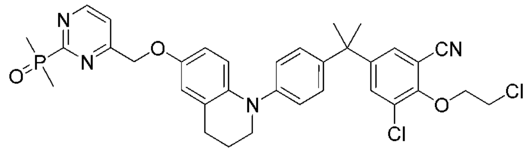

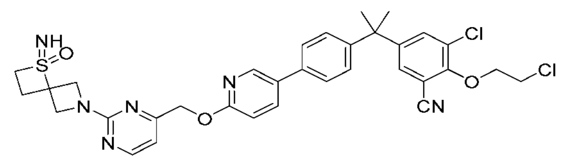

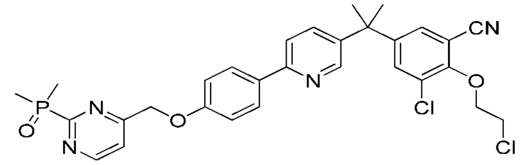

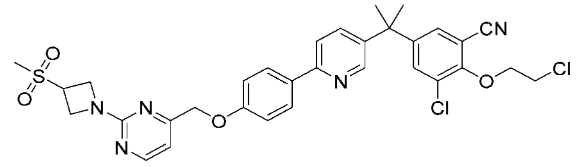

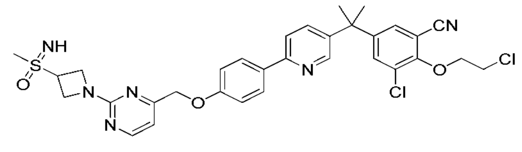

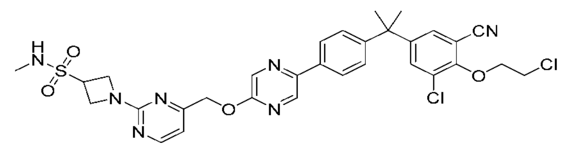

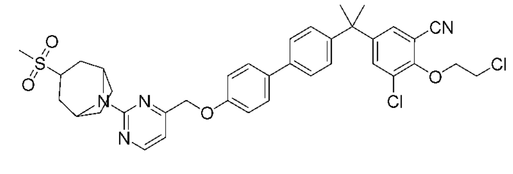

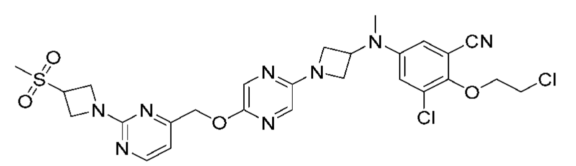

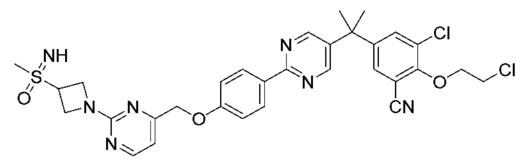

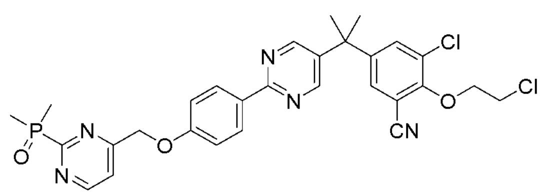

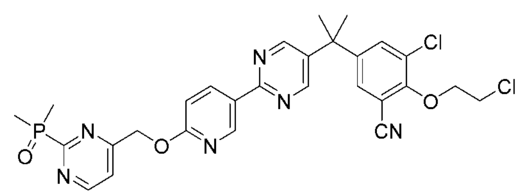

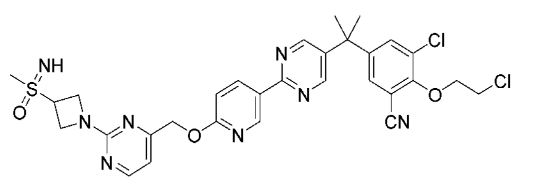

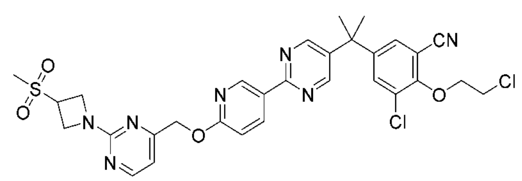

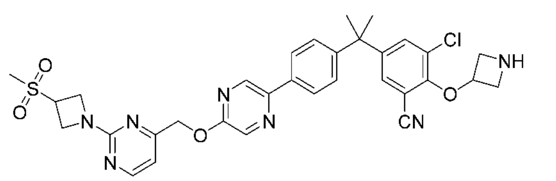

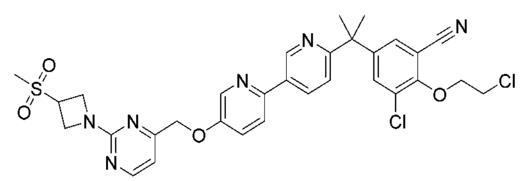

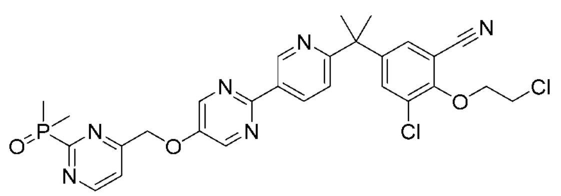

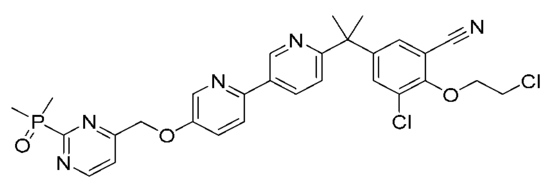

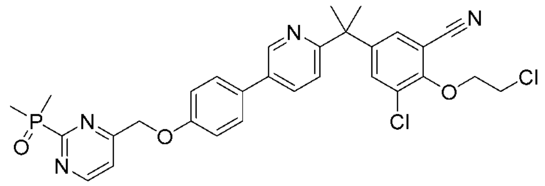

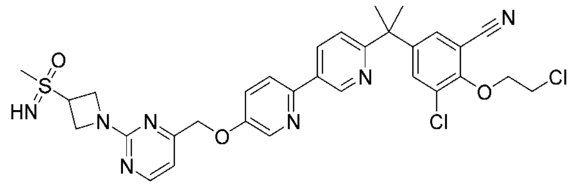

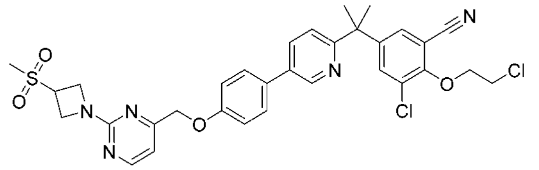

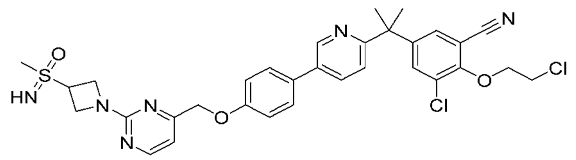

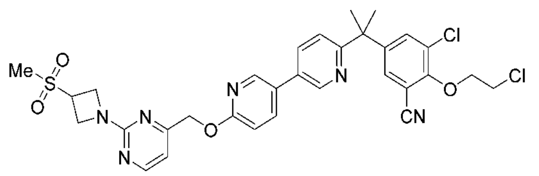

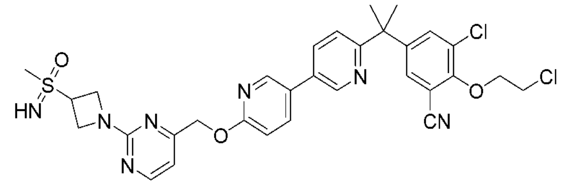

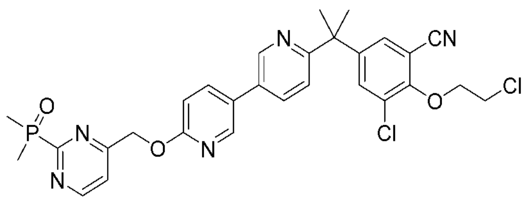

.

The present disclosure also provides a compound of the following formula, an optical isomer thereof, and a pharmaceutically acceptable salt thereof, selected from the group consisting of:

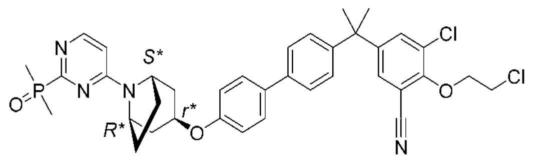

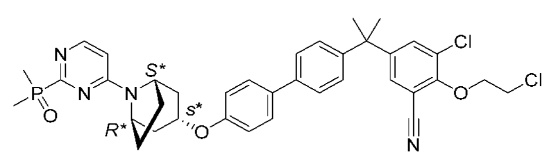

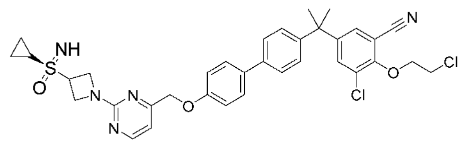

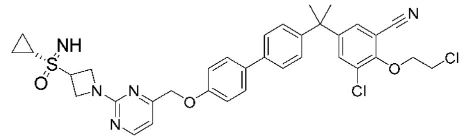

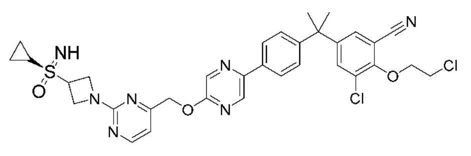

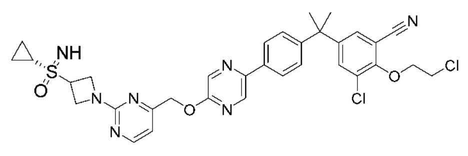

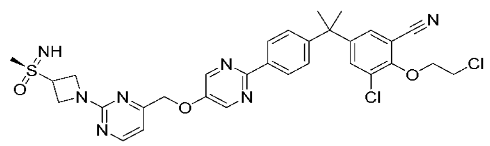

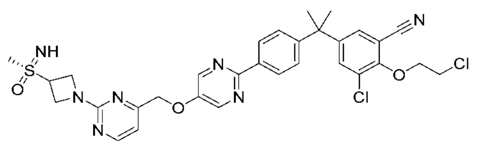

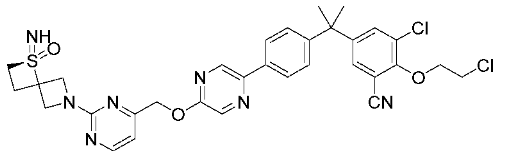

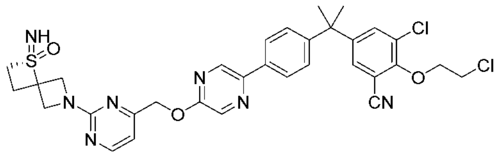

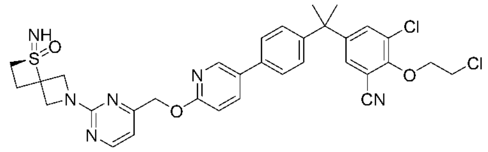

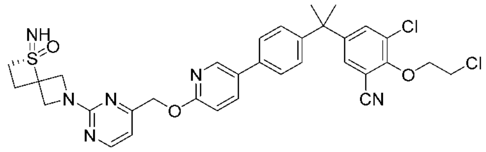
.

In another aspect of the present disclosure, the present disclosure also provides a use of the aforementioned compound, the optical isomer thereof, or the pharmaceutically acceptable salt thereof in the manufacture of a medicament for treating a disease associated with androgen receptor (AR) activity or expression.

In some embodiments of the present disclosure, the disease associated with androgen receptor (AR) activity or expression is selected from the group consisting of prostate cancer, ovarian cancer, breast cancer, bladder cancer, pancreatic cancer, endometrial cancer, hepatocellular carcinoma, renal cell carcinoma, melanoma, mantle cell lymphoma, glioblastoma, salivary gland carcinoma, alopecia, acne, hirsutism, ovarian cysts, polycystic ovary syndrome, precocious puberty, spinal and bulbar muscular atrophy, and age-related macular degeneration.

**Definition and description**

Unless otherwise specified, the following terms and phrases when used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

As used herein, the phrase "at least one" when referring to a list of one or more elements should be understood to mean at least one element selected from the group consisting of any one or more elements in the list of elements, but not necessarily including at least one of each element specified in the list of elements, and not excluding any combination of elements in the list of elements. This definition also allows that elements may optionally be present other than the elements specifically identified within the list of elements to which the phrase "at least one" refers, whether related or unrelated to those elements specifically identified.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt comprises a salt of sodium, potassium, calcium, ammonium, organic amine, magnesium, or similar salts. When the compound of the present disclosure contains a relatively basic functional group, an acid addition salt can be obtained by bringing the neutral form of the compound into contact with a sufficient amount of acid in a solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, trifluoroacetic acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by a conventional chemical method. Generally, such salt can be prepared by reacting the free acid or base form of the compound with a stoichiometric amount of an appropriate base or acid in water or an organic solvent or a mixture thereof.

"Pharmaceutically acceptable carrier" refers to a non-toxic solid, semi-solid, or liquid filler, diluent, encapsulating

material, formulation aid, or carrier commonly used in the art together with a therapeutic agent, which collectively constitutes a "pharmaceutical composition" for administration to a subject. The pharmaceutically acceptable carrier is non-toxic to the recipient at the dosages and concentrations employed and is compatible with other components of the formulation. The pharmaceutically acceptable carrier is suitable for the formulation employed.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound. For example,

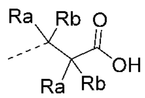

may be selected from the group consisting of

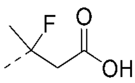, 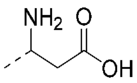, 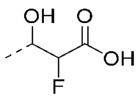, and 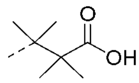.

A hyphen ("-") being not between two letters or symbols indicates the linkage site of a substituent. For example, $C_{1-6}$ alkylcarbonyl- refers to a $C_{1-6}$ alkyl linked to the rest of the molecule through a carbonyl group. However, when the linkage site of a substituent is obvious to those skilled in the art, for example, a halogen substituent, "-" can be omitted.

When the valence bond of a group has a dashed line "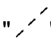", such as in

"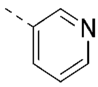",

the dashed line indicates the linkage site of the group to the rest of the molecule.

The term "substituted" or "substituted by..." means one or more hydrogen atoms on a specific atom are substituted by the substituent, including deuterium and hydrogen variables, as long as the valence of the specific atom is normal and the substituted compound is stable. The term "optionally substituted" or "optionally substituted by..." means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 1, 2, or 3 R', the group can be optionally substituted by 1, 2, or 3 R', wherein the definition of R' at each occurrence is independent. Moreover, a combination of the substituent and/or the variant thereof is allowed only when the combination results in a stable compound.

When one of the variables is a single bond, it means that the two groups linked by the single bond are linked directly. For example, when Li in

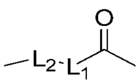

represents a single bond, the structure is actually

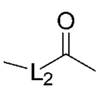.

When the listed substituents do not indicate via which atom it is linked to the substituted group, this substituent can be bonded via any atom, for example, pyridyl, as a substituent, can link to the substituted group via any carbon atom on the pyridine ring.

When the listed linking group does not indicate the direction for linking, the direction for linking is arbitrary, for

example, the linking group L contained in

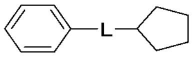

is - $CH_2O$-, then -$CH_2O$- can link phenyl and cyclopentyl to form

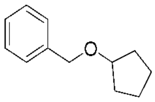

in the direction same as left-to-right reading order, and can link phenyl and cyclopentyl to form

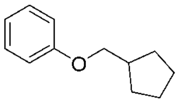

in the direction contrary to left-to-right reading order. A combination of the linking groups, substituents and/or variables thereof is allowed only when such combination can result in a stable compound.

The term "halogen" refers to fluorine, chlorine, bromine, or iodine.

Unless otherwise specified, the number of atoms on a ring is usually defined as the membered number of the ring, such as a "3- to 6-membered ring" is a "ring" with 3 to 6 atoms arranged around it.

Unless otherwise specified, the term "$C_{1-6}$ alkyl" refers to a linear or branched saturated hydrocarbon group consisting of 1 to 6 carbon atoms. The $C_{1-6}$ alkyl includes $C_{1-5}$ alkyl, $C_{1-4}$ alkyl, $C_{1-3}$ alkyl, $C_{1-2}$ alkyl, $C_{2-6}$ alkyl, $C_{2-4}$ alkyl, $C_6$ alkyl, $C_5$ alkyl, *etc.;* it can be monovalent (such as $CH_3$), divalent (-$CH_2$-), or multivalent (such as

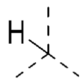

). Examples of $C_{1-6}$ alkyl include, but are not limited to, $CH_3$,

, , 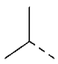, 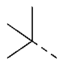, ,

, , , , , ,

,

*etc.*

Unless otherwise specified, "$C_{2-6}$ alkenyl" refers to a linear or branched hydrocarbon group consisting of 2 to 6 carbon atoms containing at least one carbon-carbon double bond, and the carbon-carbon double bond may be located at any position of the group. The $C_{2-6}$ alkenyl includes $C_{2-4}$ alkenyl, $C_{2-3}$ alkenyl, $C_4$ alkenyl, $C_3$ alkenyl, $C_2$ alkenyl, *etc.;* it can be monovalent, divalent, or multivalent. Examples of $C_{2-6}$ alkenyl include, but are not limited to, vinyl, propenyl, butenyl, pentenyl, hexenyl, butadienyl, pentadienyl, hexadienyl, *etc.*

Unless otherwise specified, "$C_{2-3}$ alkenyl" refers to a linear or branched hydrocarbon group consisting of 2 to 3 carbon atoms containing at least one carbon-carbon double bond, and the carbon-carbon double bond may be located at any position of the group. The $C_{2-3}$ alkenyl includes $C_3$ alkenyl and $C_2$ alkenyl; it can be monovalent, divalent, or multivalent. Examples of $C_{2-3}$ alkenyl include, but are not limited to,

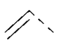, , , ,

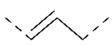,

*etc.*

The term "heteroalkyl" by itself or in combination with another term means a stable linear or branched alkyl group or a combination thereof consisting of a certain number of carbon atoms, and at least one heteroatom or heteroatom group. In some embodiments, the heteroatom is selected from the group consisting of B, O, N, P, and S, wherein the nitrogen, phosphorus, and sulfur atoms are optionally oxidized, and the nitrogen heteroatom is optionally quaternized. In other embodiments, the heteroatom group is selected from the group consisting of -C(=O)O-, -C(=O)-, -C(=S)-, -S(=O), -P(=O)-, $-S(=O)_2-$, -C(=O)N(H)-, -N(H)-, -C(=NH)-, $-S(=O)_2N(H)-$, and -S(=O)N(H)-. In some embodiments, the heteroalkyl is $C_{1-6}$ heteroalkyl; in other embodiments, the heteroalkyl is $C_{1-3}$ heteroalkyl. A heteroatom or heteroatom group may be located at any internal position within a heteroalkyl, including the site where the alkyl group is linked to the rest of the molecule, but the term "alkoxy" is a conventional expression referring to those alkyl groups linked to the rest of the molecule through an oxygen atom. Examples of heteroalkyl include, but are not limited to, $-OCH_3$, - $OCH_2CH_3$, $-OCH_2CH_2CH_3$, $-OCH(CH_3)_2$, $-CH_2-CH_2-O-CH_3$, $-NHCH_3$, $-N(CH_3)_2$, - $NHCH_2CH_3$, $-N(CH_3)(CH_2CH_3)$, $-CH_2-CH_2-NH-CH_3$, $-CH_2-CH_2-N(CH_3)-CH_3$, $-SCH_3$, - $SCH_2CH_3$, $-SCH_2CH_2CH_3$, $-SCH(CH_3)_2$, $-CH_2-S-CH_2-CH_3$, $-CH_2-CH_2-S-CH_3$, $-S(=O)-CH_3$,$-CH_2-CH_2-S(=O)_2-CH_3$,

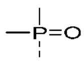,

and up to two heteroatoms may be consecutive, e.g., $-CH_2-NH-OCH_3$.

Unless otherwise specified, the term "$C_{1-6}$ alkoxy" refers to an alkyl group containing 1 to 6 carbon atoms that are connected to the rest of the molecule through an oxygen atom. The $C_{1-6}$ alkoxy includes $C_{1-4}$ alkoxy, $C_{1-3}$ alkoxy, $C_{1-2}$ alkoxy, $C_{2-6}$ alkoxy, $C_{2-4}$ alkoxy, $C_6$ alkoxy, $C_5$ alkoxy, $C_4$ alkoxy, $C_3$ alkoxy, *etc.* Examples of $C_{1-6}$ alkoxy include, but are not limited to, methoxy, ethoxy, propoxy (including n-propoxy and isopropoxy), butoxy (including n-butoxy, isobutoxy, s-butoxy, and t-butoxy), pentyloxy (including n-pentyloxy, isopentyloxy, and neopentyloxy), hexyloxy, *etc.*

Unless otherwise specified, the term "$C_{1-3}$ alkoxy" refers to an alkyl group containing 1 to 3 carbon atoms that are connected to the rest of the molecule through an oxygen atom. The $C_{1-3}$ alkoxy includes $C_{1-3}$ alkoxy, $C_{1-2}$ alkoxy, $C_{2-3}$ alkoxy, $C_1$ alkoxy, $C_2$ alkoxy, $C_3$ alkoxy, *etc.* Examples of $C_{1-3}$ alkoxy include, but are not limited to, methoxy, ethoxy, propoxy (including n-propoxy and isopropoxy), *etc.*

Unless otherwise specified, the term "$C_{1-6}$ alkylamino" refers to an alkyl group containing 1 to 6 carbon atoms that are connected to the rest of the molecule through an amino group. The $C_{1-6}$ alkylamino includes $C_{1-4}$ alkylamino, $C_{1-3}$ alkylamino, $C_{1-2}$ alkylamino, $C_{2-6}$ alkylamino, $C_{2-4}$ alkylamino, $C_6$ alkylamino, $C_5$ alkylamino, $C_4$ alkylamino, $C_3$ alkylamino, $C_2$ alkylamino, *etc.* Examples of $C_{1-6}$ alkylamino include, but are not limited to, $-NHCH_3$, - $N(CH_3)_2$, $-NHCH_2CH_3$, $-N(CH_3)CH_2CH_3$, $-N(CH_2CH_3)(CH_2CH_3)$, $-NHCH_2CH_2CH_3$, - $NHCH(CH_3)_2$, $-NHCH_2CH_2CH_2CH_3$, *etc.*

Unless otherwise specified, the term "$C_{1-3}$ alkylamino" refers to an alkyl group containing 1 to 3 carbon atoms that are connected to the rest of the molecule through an amino group. The $C_{1-3}$ alkylamino includes $C_{1-3}$ alkylamino, $C_{1-2}$ alkylamino, $C_{2-3}$ alkylamino, $C_1$ alkylamino, $C_2$ alkylamino, $C_3$ alkylamino, *etc.* Examples of $C_{1-3}$ alkylamino include, but are not limited to, $-NHCH_3$, $-N(CH_3)_2$, $-NHCH_2CH_3$, $-N(CH_3)CH_2CH_3$, $-NHCH_2CH_2CH_3$, - $NHCH(CH_3)_2$, *etc.*

Unless otherwise specified, the term "$C_{1-6}$ alkylthio" refers to an alkyl group containing 1 to 6 carbon atoms that are connected to the rest of the molecule through a sulfur atom. The $C_{1-6}$ alkylthio includes $C_{1-4}$ alkylthio, $C_{1-3}$ alkylthio, $C_{1-2}$ alkylthio, $C_{2-6}$ alkylthio, $C_{2-4}$ alkylthio, $C_6$ alkylthio, $C_5$ alkylthio, $C_4$ alkylthio, $C_3$ alkylthio, $C_2$ alkylthio, *etc.* Examples of $C_{1-6}$ alkylthio include, but are not limited to, $-SCH_3$, $-SCH_2CH_3$, $-SCH_2CH_2CH_3$, $-SCH(CH_3)_2$, *etc.*

Unless otherwise specified, the term "$C_{1-3}$ alkylthio" refers to an alkyl group containing 1 to 3 carbon atoms that are connected to the rest of the molecule through a sulfur atom. The $C_{1-3}$ alkylthio includes $C_{1-3}$ alkylthio, $C_{1-2}$ alkylthio, $C_{2-3}$ alkylthio, $C_1$ alkylthio, $C_2$ alkylthio, $C_3$ alkylthio, *etc.* Examples of $C_{1-3}$ alkylthio include, but are not limited to, - $SCH_3$, $-SCH_2CH_3$, $-SCH_2CH_2CH_3$, $-SCH(CH_3)_2$, *etc.*

Unless otherwise specified, "$C_{3-6}$ cycloalkyl" refers to a saturated cyclic hydrocarbon group consisting of 3 to 6 carbon atoms in monocyclic and bicyclic systems, and the $C_{3-6}$ cycloalkyl includes $C_{3-5}$ cycloalkyl, $C_{4-5}$ cycloalkyl, $C_{5-6}$ cycloalkyl, *etc.;* it can be monovalent, divalent, or multivalent. Examples of $C_{3-6}$ cycloalkyl groups include, but are not limited to, cyclopropyl, cyclobutyl, cyclopentyl, cyclohexyl, *etc.*

Unless otherwise specified, the term "3- to 10-membered heterocyclyl" by itself or in combination with other terms refers to a saturated or partially unsaturated cyclic group consisting of 3 to 10 ring atoms, respectively, wherein 1, 2, 3, or 4 ring atoms are heteroatoms independently selected from the group consisting of O, S, P and N, and the rest are carbon atoms, wherein the nitrogen atoms are optionally quaternized, and the carbon, nitrogen, phosphorus, and sulfur atoms may be independently and optionally oxidized (*i.e.,* C=O, P=O, NO and $S(O)_p$, p is 1 or 2). It includes monocyclic and bicyclic systems, wherein the bicyclic system includes spiro rings, fused rings, and bridged rings. In addition, in the case of the "3-to 10-membered heterocyclyl", the heteroatom may occupy the position where the heterocyclyl is attached to the rest of the molecule. The 3- to 10-membered heterocyclyl includes 3- to 9-membered, 3- to 8-membered, 3- to 7-membered, 3- to 6-membered, 3- to 5-membered, 3- to 4-membered, 4- to 5-membered, 4- to 6-membered, 4- to 7-membered, 4- to 8-membered, 4-to 9-membered, 5- to 6-membered, 5- to 7-membered, 5- to 8-membered, 5- to 9-membered, 6- to 7-membered, 6- to 8-membered, 6- to 9-membered, 7- to 8-membered, 3-membered, 4-membered, 5-membered, 6-membered, 7-membered, 8-membered, 9-membered, and 10-membered heterocyclyl, *etc.* Examples of 3- to 10-membered heterocyclyl include, but are not limited to, azetidinyl, oxetanyl, thietanyl, 1,3-dioxolane,

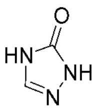 , 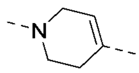 , 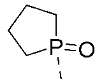 ,

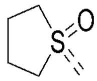 , 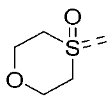 , 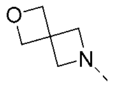 , 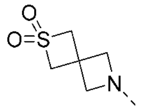 ,

pyrrolidinyl, pyrazolidinyl, imidazolidinyl, tetrahydrothiophenyl (including tetrahydrothiophen-2-yl, tetrahydrothiophen-3-yl, *etc.*), tetrahydrofuranyl (including tetrahydrofuran-2-yl, *etc.*), tetrahydropyranyl, piperidinyl (including 1-piperidinyl, 2-piperidinyl, 3-piperidinyl, *etc.),* piperazinyl (including 1-piperazinyl, 2-piperazinyl, *etc.*)*,* morpholinyl (including 3-morpholinyl, 4-morpholinyl, *etc.),* dioxolyl, dithianyl, isoxazolidinyl, isothiazolidinyl, 1,2-oxazinyl, 1,2-thiazinyl, hexahydropyridazinyl, homopiperazinyl, homopiperidinyl, *etc.*

Unless otherwise specified, the term "5- to 6-membered heterocyclyl" by itself or in combination with other terms refers to a saturated or partially unsaturated cyclic group consisting of 5 to 6 ring atoms, respectively, wherein 1, 2, 3, or 4 ring atoms are heteroatoms independently selected from the group consisting of O, S, and N, and the rest are carbon atoms, wherein the nitrogen atoms are optionally quaternized, and the nitrogen and sulfur heteroatoms are optionally oxidized (i.e., NO and $S(O)_p$, p is 1 or 2). It includes monocyclic and bicyclic systems, wherein the bicyclic system includes spiro rings, fused rings, and bridged rings. In addition, in the case of the "5- to 6-membered heterocyclyl", the heteroatom may occupy the position where the heterocyclyl is attached to the rest of the molecule. The 5- to 6-membered heterocyclyl includes 5-membered heterocyclyl, 6-membered heterocyclyl, *etc.* Examples of 5- to 6-membered heterocyclyl include, but are not limited to, 1,3-dioxolane,

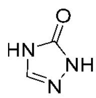 ,

pyrrolidinyl, pyrazolidinyl, imidazolidinyl, tetrahydrothiophenyl (including tetrahydrothiophen-2-yl, tetrahydrothiophen-3-yl, *etc.),* tetrahydrofuranyl (including tetrahydrofuran-2-yl, *etc.),* tetrahydropyranyl, piperidinyl (including 1-piperidinyl, 2-piperidinyl, 3-piperidinyl, *etc.),* piperazinyl (including 1-piperazinyl, 2-piperazinyl, *etc.),* morpholinyl (including 3-morpholinyl, 4-morpholinyl, *etc.),* dioxolyl, dithianyl, isoxazolidinyl, isothiazolidinyl, 1,2-oxazinyl, 1,2-thiazinyl, hexahydropyridazinyl, homopiperazinyl, homopiperidinyl, *etc.*

Unless otherwise specified, the terms "5- to 6-membered heteroaromatic ring" and "5-to 6-membered heteroaryl" in the present disclosure may be used interchangeably, and the term "5- to 6-membered heteroaryl" refers to a monocyclic group consisting of 5 to 6 ring atoms with conjugated $\pi$ electronic system, wherein 1, 2, 3, or 4 ring atoms are heteroatoms independently selected from the group consisting of O, S, and N, and the rest are carbon atoms. Where the nitrogen atom is optionally quaternized, and the nitrogen and sulfur heteroatoms are optionally oxidized (i.e., NO and $S(O)_p$, p is 1 or 2). The 5- to 6-membered heteroaryl may be attached to the rest of the molecule through a heteroatom or a carbon atom. The 5- to 6-membered heteroaryl includes 5-membered and 6-membered heteroaryl. Examples of the 5-to 6-membered heteroaryl include, but are not limited to, pyrrolyl (including *N*-pyrrolyl, 2-pyrrolyl, 3-pyrrolyl, *etc.*), pyrazolyl (including 2-pyrazolyl, 3-pyrazolyl, *etc.),* imidazolyl (including *N*-imidazolyl, 2-imidazolyl, 4-imidazolyl, 5-imidazolyl, *etc.),* oxazolyl (including 2-oxazolyl, 4-oxazolyl, 5-oxazolyl, *etc.*)*,* triazolyl (1*H*-1,2,3-triazolyl, 2*H*-1,2,3-triazolyl, 1*H*-1,2,4-triazolyl, 4*H*-1,2,4-triazolyl, *etc.*), tetrazolyl, isoxazolyl (3-isoxazolyl, 4-isoxazolyl, 5-isoxazolyl, *etc.*)*,* thiazolyl (including 2-thiazolyl, 4-thiazolyl, 5-thiazolyl, *etc.*), furanyl (including 2-furanyl, 3-furanyl, *etc.*)*,* thienyl (including 2-thienyl, 3-thienyl, *etc.*), pyridinyl (including 2-pyridyl, 3-pyridyl, 4-pyridyl, *etc.*), pyrazinyl, or pyrimidinyl (including 2-pyrimidinyl, 4-pyrimidinyl, *etc.).*

Unless otherwise specified, the term "$C_{6-10}$ aryl" by itself or in combination with other terms refers to a monocyclic or bicyclic aromatic ring system having six to ten carbon atoms. Non-limiting exemplary aryl groups include phenyl (abbreviated as "Ph") and naphthyl.

The compound of the present disclosure may contain an unnatural proportion of atomic isotope at one or more atoms that constitute the compound. For example, the compound can be radiolabeled with a radioactive isotope, such as tritium ($^3H$), iodine-125 ($^{125}I$), or C-14 ($^{14}C$). For another example, deuterated drugs can be formed by replacing hydrogen

with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs and the like. All isotopic variations of the compound of the present disclosure, whether radioactive or not, are encompassed within the scope of the present disclosure.

"Optional" or "optionally" means that the subsequent event or condition may occur but not requisite, and the description includes the instance in which the event or condition occurs and the instance in which the event or condition does not occur.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The solvent used in the present disclosure is commercially available.

The compounds of the present disclosure are named according to the conventional naming principles in the art or by ChemDraw® software, and the commercially available compounds use the supplier catalog names.

The compounds disclosed in the present disclosure may have one or more chiral centers, each chiral center independently having an R configuration or an S configuration, or a cis configuration, or a trans configuration. In some compounds disclosed in the present disclosure, chiral centers are labeled as *R, *S, R*, S*, *Cis-, or *Trans-, indicating that the absolute configuration of the chiral center in the compound has not been determined, but the compound has been isolated or chirally resolved and the chiral center is present in a single configuration. The compound is a single-configuration enantiomer monomer, or a single-configuration diastereomer monomer, or a mixture of diastereomers in which the chiral center is present in a single configuration (for example, where the configurations of other chiral centers have not been resolved), or a single-configuration monomer (for example, a single cis-configuration monomer, or a single trans-configuration monomer). When the absolute configuration (R configuration, S configuration, cis configuration, or trans configuration) of a chiral center in a compound disclosed in the present disclosure has not been determined, such compound can be identified based on its peak pattern in the corresponding nuclear magnetic resonance ($^1$H-NMR, $^{31}$P-NMR) spectra, or based on the corresponding retention time (RT or Rt) under corresponding chromatographic column conditions (e.g., column model, column packing material, column dimensions, mobile phase, *etc.).*

The present disclosure is explained in more detail in the following examples. However, it should be understood that these examples are provided to illustrate the present disclosure and are not intended to limit the scope of the present disclosure in any way. Experimental methods in the following examples in which specific conditions are not indicated are usually in accordance with the conventional conditions for this type of reaction, or in accordance with the conditions recommended by the manufacturer. Unless otherwise specified, percentages and parts are by weight. Unless otherwise specified, ratios of liquids are by volume.

The technical and scientific terms used herein that are not specifically defined have the meanings commonly understood by those skilled in the art to which the present disclosure belongs.

DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific embodiments have also been disclosed; for those skilled in the art, it is obvious to make various modifications and improvements to the embodiments of the present disclosure without departing from the conception and scope of the present disclosure.

The experimental materials and reagents used in the following examples can be obtained from commercially available sources unless otherwise specified.

In all examples, $^1$H NMR, $^{13}$C NMR, $^{19}$F NMR, and $^{31}$P NMR spectra were recorded using a Bruker Ascend 400 mHz nuclear magnetic resonance spectrometer, with spectra processed using Topspin software and deuterated solvent serving as an internal deuterium lock. Among them, $^{13}$C NMR, $^{19}$F NMR, and $^{31}$P NMR were decoupled from $^1$H. Assignments are made based on distinct chemical shift/coupling patterns, or according to 2D Cosy, HMBC, HSQC, or NOESY experiments. Peak multiplicity is defined as: s (singlet), d (doublet), t (triplet), q (quartet), m (multiplet), br (broad), br.s (broad singlet); the coupling constant (J) is accurate to 0.1 Hz. Mass spectra were recorded using Agilent 1260 (ESI), Shimadzu LC-MS-2020 (ESI), or Agilent 6215 (ESI) mass spectrometer; reversed-phase preparative HPLC separations were performed using an Agilent 1290 UV-guided fully automated purification system (Xtimate ®Prep C18 OBDTM 21.2 * 250 mm 10 $\mu$m column), a Gilson GX281 UV-guided fully automated purification system (xBridge ®Prep C18 OBDTM 19 * 250 mm 10 $\mu$m column) or a Waters QDa-guided fully automated purification system (SunFire ®Prep C18 OBD 29 * 250 mm 10 $\mu$m column). Unless otherwise specified, separations were performed by using a SepaFlash pre-installed normal-phase silica gel column (Sinopharm Chemical Reagent Co., Ltd.) or a TLC analysis plate (Yantai Jiangyou Silica Gel Development Co., Ltd., model: HSGF254, specification: 2.5 $\times$ 5 cm), and the ratios of eluents are all by volume.

The names of the reagents, represented by chemical formula or alphabetical abbreviations, are as follows:

$CD_3OD$ represents deuterated methanol; DMSO-*d6* represents deuterated dimethyl sulfoxide; Chloroform-*d* or $CDCl_3$ represents deuterated chloroform; AcOH represents acetic acid; $AlCl_3$ represents aluminum trichloride; Aq represents aqueous solution; $N_2$ represents nitrogen; Ar represents argon; $B_2Pin_2$ represents bis(pinacolato)diboron; $BBr_3$ represents boron tribromide; $BH_3$ represents borane; $(Boc)_2O$ represents di-*tert*-butyl dicarbonate; $Et_3SiH$ represents triethylsilane; HATU represents 1-[bis(dimethylamino)methylene]-1H-1,2,3-triazolo[4,5-b]pyridinium 3-oxide hexafluorophosphate; HOBt represents 1-hydroxybenzotriazole; TMAD represents *N,N,N',N'*-tetramethylazodicarboxamide; $K_2CO_3$ represents potassium carbonate; KOAc represents potassium acetate; MeONa represents sodium methoxide; LDA represents lithium diisopropylamide; LiHMDS represents lithium bis(trimethylsilyl)amide; LiOH represents lithium hydroxide; *m*-CPBA represents meta-chloroperoxybenzoic acid; $Na_2CO_3$ represents sodium carbonate; $NaBH_4$ represents sodium borohydride; NaCl represents sodium chloride; $NaHCO_3$ represents sodium bicarbonate; NaOH represents sodium hydroxide; $Na_2SO_4$ represents sodium sulfate; NBS represents *N*-bromosuccinimide; NCS represents *N*-chlorosuccinimide; NIS represents *N*-iodosuccinimide; Oxone represents potassium peroxymonosulfate; n-BuLi represents *n*-butyllithium; $NH_4Cl$ represents ammonium chloride; NMP represents *N*-methyl-2-pyrrolidone; $PBr_3$ represents phosphorus tribromide; $Pd(dppf)Cl_2$ or $PdCl_2(dppf)$ represents [1,1'-bis(diphenylphosphino)ferrocene]dichloropalladium(II); $Pd_2(dba)_3$ represents tris(dibenzylideneacetone)dipalladium(0); $Pd(OAc)_2$ represents palladium acetate; conc. represents concentrated; $(COCl)_2$ represents oxalyl chloride; $Cs_2CO_3$ represents cesium carbonate; CuCl represents copper(I) chloride; CuI represents copper(I) iodide; DCM represents dichloromethane; Dioxane or 1,4-dioxane represents 1,4-dioxane; MeCN, ACN or $CH_3CN$ represents acetonitrile; MeOH or methanol represents methanol; EtOH or ethanol represents ethanol; DEA represents diethylamine; DIPEA or DIEA represents *N,N*-diisopropylethylamine; TEA represents triethylamine; DIAD represents diisopropyl azodicarboxylate; Xantphos represents 4,5-bis(diphenylphosphino)-9,9-dimethylxanthene; DMAP represents 4-dimethylaminopyridine; DMF represents *N,N*-dimethylformamide; DMSO represents dimethyl sulfoxide; EA or EtOAc represents ethyl acetate; PE represents petroleum ether; THF represents tetrahydrofuran; Toluene or tol. represents toluene; $SOCl_2$ represents thionyl chloride; TFA represents trifluoroacetic acid; FA represents formic acid; TMSCN represents trimethylsilyl cyanide; $H_2O$ represents water; HCl represents hydrogen chloride gas; HCl aq. represents hydrochloric acid aqueous solution; °C represents degrees Celsius; rt or RT represents room temperature; h represents hour; min represents minute; g represents gram; mg represents milligram; mL represents milliliter; mmol represents millimole; M represents mole; cm represents centimeter; mm represents millimeter; μm represents micrometer; nm represents nanometer; mL/min represents milliliters per minute; Hz represents Hertz; MHz represents megahertz; bar represents the pressure unit bar; psi represents the pressure unit pounds per square inch; $N_2$ represents nitrogen; HPLC represents high performance liquid chromatography; I.D. represents inner diameter; LCMS or LC-MS represents liquid chromatography-mass spectrometry; m/z represents mass-to-charge ratio; ESI represents electrospray ionization; $CO_2$ represents carbon dioxide; TLC represents thin-layer chromatography; Rf represents the retention factor of a sample after spotting/development on a TLC plate; UV represents ultraviolet; IV represents intravenous injection; PO represents oral administration; rpm represents revolutions per minute; ATCC represents American Type Culture Collection.

**Example 1: Preparation of compound 1**

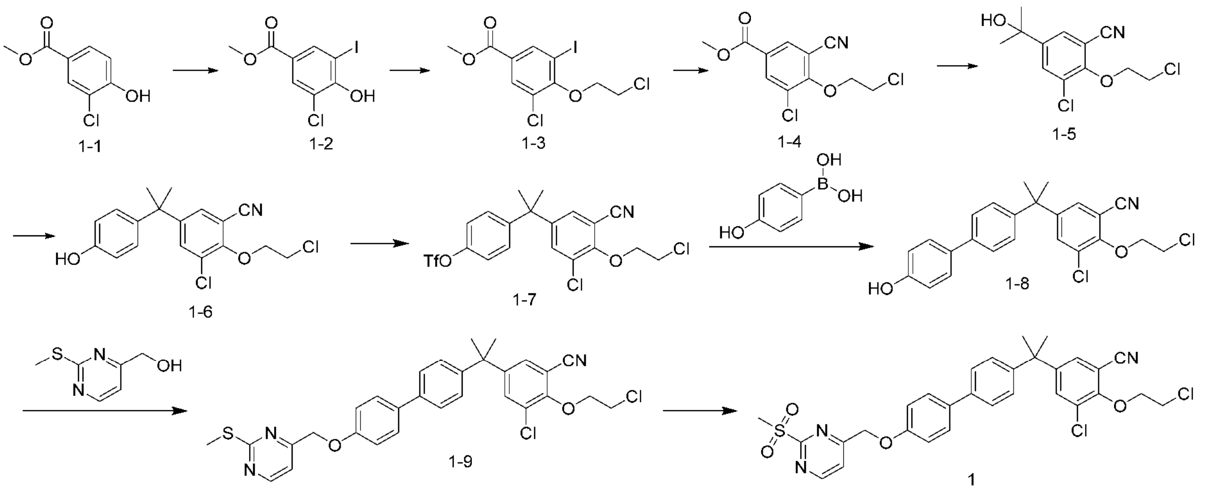

1-1 1-2 1-3 1-4 1-5

1-6 1-7 1-8

1-9 1

**Preparation of compound 1-2**

Compound 1-1 (120.0 g, 643.1 mmol) was dissolved in MeCN (1.2 L), and the mixture was cooled to 0°C in an ice-water bath. *p*-Toluenesulfonic acid monohydrate (122.2 g, 643.1 mmol) was added, and the resulting mixture was stirred at this temperature for 30 min. Subsequently, NIS (173.6 g, 771.1 mmol) was added. After the addition was completed, the ice-water bath was removed, and the reaction system was stirred at room temperature for 16 h. Sodium sulfite was added to the reaction system to quench the reaction, followed by the addition of water (3 L). The mixture was extracted with ethyl acetate (2 L × 2). The organic phase was washed with saturated brine (2 L × 3),dried over anhydrous sodium sulfate, concentrated, and purified by normal-phase silica gel column chromatography (EA/PE = 0-30%) to obtain the title compound **1-2** (168.0 g, white solid, yield: 84%).

**Preparation of compound 1-3**

Compound **1-2** (163.0 g, 0.52 mol) was dissolved in DMF (800 mL), followed by the sequential addition of cesium carbonate (338.9 g, 1.04 mol), compound 1-bromo-2-chloroethane (149.1 g, 1.04 mol), and $H_2O$ (12 mL). The mixture was heated to 65°C and reacted for 16 h. After the reaction was completed, the reaction mixture was filtered. Water (3 L) was added to the filtrate, and the mixture was extracted with ethyl acetate (2 L × 2). The organic phase was washed with saturated brine (2 L × 3), dried over anhydrous sodium sulfate, concentrated, and purified by normal-phase silica gel column chromatography (EA/PE = 0-30%) to obtain the title compound **1-3** (136.9 g, white solid, yield: 70%).

**Preparation of compound 1-4**

Compound **1-3** (168.0 g, 448.0 mmol) was dissolved in DMF (1 L), and CuCN (100.3 g, 1120.0 mmol) and CuI (73.1 g, 385.4 mmol) were added. The mixture was heated to 140°C and reacted for 4 h. The reaction was monitored by TLC (PE/EA = 5/1, product Rf of approximately 0.5) until completion. The mixture was filtered through a thin layer of diatomite, and the filter cake was rinsed with ethyl acetate. The filtrate was collected, water (4 L) was added, and the mixture was extracted with ethyl acetate (3 L × 2). The organic phase was washed with saturated brine (2 L × 3), dried over anhydrous sodium sulfate, concentrated, and purified by normal-phase silica gel column chromatography (EA/PE = 0-20%) to obtain the title compound **1-4** (66.7 g, white solid, yield: 54%). $^1H$ NMR (400 MHz, $CDCl_3$) δ 8.46 (d, *J* = 5.2 Hz, 1H), 6.93 (d, *J* = 5.2 Hz, 1H), 5.32 (s, 2H), 2.51 (s, 3H), 2.05 (s, 2H).

**Preparation of compound 1-5**

Compound **1-4** (5 g, 18 mmol) was added to a three-necked flask, and the system was purged three times with nitrogen. Anhydrous tetrahydrofuran (20 mL) was added, and methylmagnesium bromide (3.0 M, 18 mL, 54 mmol) was added dropwise at -10°C. The reaction system was stirred at 0°C for 2 h, and the reaction was completed. The reaction system was slowly added with saturated aqueous ammonium chloride solution (50 mL) in an ice bath, and extracted with EtOAc (50 mL × 3). The organic phases were combined, washed with saturated brine (20 mL × 2), dried over anhydrous sodium sulfate, and concentrated to obtain a crude product. The crude product was purified by silica gel column chromatography (EtOAc/PE = 0-20%) to obtain the title compound **1-5** (3 g, pale yellow liquid, yield: 60%). $^1H$ NMR (400 MHz, DMSO-d6) δ 7.90 (d, *J* = 2.2 Hz, 1H), 7.83 (d, *J* = 2.2 Hz, 1H), 5.37 (s, 1H), 4.43 (t, *J* = 4.0 Hz, 2H), 3.97 (t, *J* = 4.0 Hz, 2H) ,1.43 (s, 6H).

**Preparation of compound 1-6**

Compound **1-5** (3 g, 10.9 mmol) was added to a three-necked flask, followed by the addition of anhydrous THF (30 mL) and phenol (3 g, 32.8 mmol). Boron trifluoride diethyl etherate (9.71 g, 32.8 mmol) was added at 0°C. The reaction system was stirred at 0°C for 3 h. After the reaction was completed, ice water (20 mL) was slowly added to quench the reaction. The mixture was extracted with EtOAc (30 mL × 3). The organic phases were combined, washed with saturated brine (20 mL × 2), dried over anhydrous sodium sulfate, and concentrated to obtain a crude product. The crude product was purified by normal-phase silica gel column chromatography (EtOAc/PE = 0-20%) to obtain the title compound **1-6** (2.3 g, pale yellow liquid, yield: 57%). $^1H$ NMR (400 MHz, DMSO-d6) δ 9.29 (s, 1H), 7.61 (d, *J* = 2.2 Hz, 1H), 7.54 (d, *J* = 2.2 Hz, 1H), 7.04 (d, *J* = 8.6 Hz, 2H), 6.69 (d, *J* = 8.6 Hz, 2H), 4.41 (t, *J* =4.0 Hz, 2H), 3.95 (t, *J* = 4.0 Hz 2H), 1.60 (s, 6H).

**Synthesis of compound 1-7**

Compound **1-6** (5 g, 14.28 mmol), DCM (50 mL), and pyridine (2.26 g, 28.55 mmol, 2.30 mL) were added to a 250 mL three-necked flask, stirred to dissolve, and cooled to 0°C. $Tf_2O$ (6.04 g, 21.41 mmol, 3.60 mL) was added dropwise at

5°C, and the mixture was stirred and maintained at the temperature for 3 h. TLC monitoring indicated that the reaction was complete. The reaction mixture was poured into saturated sodium bicarbonate solution (100 mL) and extracted twice with DCM (60 mL) The organic phases were combined, washed with water (100 mL), and evaporated to dryness under reduced pressure to obtain a brown-black liquid. Purification was performed using an automated column chromatography system (EA:PE = 0 to 15%) to obtain the title compound **1-7** (6.15 g). LC-MS (ESI): m/z 482.2 $[M+H]^+$.

**Synthesis of compound 1-8**

Compound **1-7** (1 g, 2.07 mmol), 4-hydroxyphenylboronic acid (400.00 mg, 2.90 mmol), 1,4-dioxane (20 mL), $H_2O$ (10 mL), Pd(dppf)$Cl_2$ (160.00 mg, 218.67 μmol), and $Na_2CO_3$ (550.00 mg, 5.19 mmol) were added to a 100 mL single-necked flask. The system was purged three times with nitrogen, and the mixture was stirred at 90°C for 10 h. LCMS monitoring indicated that the reaction was complete. Saturated ammonium chloride (100 mL) and ethyl acetate (80 mL) were added, mixed, and filtered through a pad of diatomite to remove insoluble matter. The filtrate was allowed to stand, separated into layers, and the phases were separated. The aqueous phase was extracted once with ethyl acetate (50 mL). The organic phases were combined, dried over anhydrous sodium sulfate, evaporated to dryness under reduced pressure, and purified by an automated column chromatography system (EA:PE = 0 to 30%) to obtain 0.61 g of the title compound **1-8.** LC-MS (ESI): m/z 424.0 $[M-H]^-$. $^1H$ NMR (400 MHz, $CDCl_3$) δ 7.50 - 7.44 (m, 5H), 7.38 (d, *J* = 2.4 Hz, 1H), 7.21 (d, *J* = 8.4 Hz, 2H), 6.90 (d, *J* = 8.6 Hz, 2H), 4.90 (br.s, 1H), 4.43 (t, *J* = 6.2 Hz, 2H), 3.88 (t, *J* = 6.2 Hz, 2H), 1.69 (s, 6H).

**Synthesis of compound 1-9**

Compound **1-8** (0.61 g, 1.43 mmol), 2-methylthiopyrimidin-4-ylmethanol (265 mg, 1.70 mmol),TMAD (495 mg, 2.87 mmol) and DCM (10 mL) were added to a 50 mL single-necked flask. The mixture was cooled to 25°C. $Bu_3P$ (607.50 mg, 3.00 mmol) was added dropwise. After the addition was completed, the mixture was slowly warmed to room temperature (25°C) and stirred for 16 h. LCMS indicated the presence of the molecular ion peak of the target product. The mixture was evaporated to dryness under reduced pressure, and purified by an automated column chromatography system (EA/PE = 0 to 20%) to obtain 0.61 g of the title compound **1-9.** LC-MS (ESI): m/z 564.3 $[M+H]^+$. $^1H$ NMR (400 MHz, $CDCl_3$) δ 8.54 (d, *J* = 4.8 Hz, 1H), 7.54 - 7. 46 (m, 5H), 7.37 (d, *J* = 2.4 Hz, 1H), 7.24 - 7.20 (m, 3H), 7.02 (d, *J* = 8.8 Hz, 2H), 5.14 (s, 2H), 4.43 (t, *J* = 6.4 Hz, 2H), 3.88 (t, *J* = 6.4 Hz, 2H), 2.60 (s, 3H), 1.69 (s, 6H).

**Synthesis of compound 1**

Compound **1-9** (0.61 g, 1.08 mmol) and DCM (10 mL) were added to a 100 mL single-necked flask. The mixture was cooled to 0°C, and m-CPBA (465 mg, 2.29 mmol, 85% purity) was added. The mixture was slowly warmed to room temperature (25°C) and stirred for 16 h. TLC indicated that the reaction was complete, and LCMS indicated the presence of the molecular ion peak of the target product. Saturated sodium bicarbonate solution (50 mL) was added, and the mixture was extracted twice with DCM (50 mL). The organic phases were combined, washed with water (30 mL), dried over anhydrous sodium sulfate, subjected to suction filtration, and the filtrate was evaporated to dryness under reduced pressure. The resulting residue was purified by an automated column chromatography system (EA/PE = 0 to 50%) to obtain 0.49 g of the title compound 1. LC-MS (ESI): m/z 596.3 $[M+H]^+$. $^1H$ NMR (400 MHz, $CDCl_3$) δ 8.93 (d, *J* = 5.2 Hz, 1H), 7.85 (d, *J* = 5.2 Hz, 1H), 7.55 (d, *J* = 8.8 Hz, 2H), 7.50 (d, *J* = 4.2 Hz, 1H), 7.48 (d, *J* = 1.9 Hz, 2H), 7.37 (d, *J* = 2.4 Hz, 1H), 7.23 (d, *J* = 8.4 Hz, 2H), 7.03 (d, *J* = 8.8 Hz, 2H), 5.34 (s, 2H), 4.43 (t, *J* = 6.2 Hz, 2H), 3.88 (t, *J* = 6.2 Hz, 2H), 3.39 (s, 3H), 1.70 (s, 6H).

**Example 2: Preparation of compound 2**

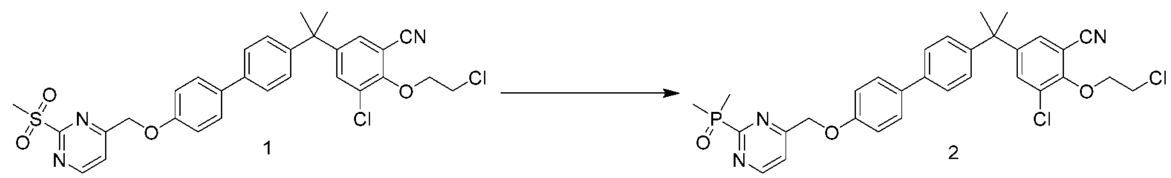

**Synthesis of compound 2**

Compound **1** (150 mg, 251.46 μmol), dimethylphosphine oxide (412.50 mg, 5.29 mmol), $CH_3CN$ (4 mL), and $K_2CO_3$ (75.00 mg, 542.65 μmol) were added to a microwave tube. The mixture was heated to 100°C and stirred under microwave irradiation for 3 h. TLC monitoring indicated that the starting material was completely consumed. Water (30 mL) was added, and the mixture was extracted twice with ethyl acetate (40 mL). The organic phases were combined, washed with water (30 mL), dried over anhydrous sodium sulfate, subjected to suction filtration, and the filtrate was evaporated to dryness under reduced pressure. The resulting residue was purified by an automated column chromatography system to obtain 220 mg of a viscous substance. The viscous substance was purified by preparative high-performance liquid chromatography (preparation method: chromatographic column: Pntulips ZZ-C18 10 μm 250 × 20 mm; column temperature: 25°C; mobile phase: water (0.1% FA) - acetonitrile; gradient elution with the proportion of acetonitrile in the mobile phase from 50% to 70% over 12 min; flow rate: 30 mL/min), and lyophilized to obtain 53.77 mg of the title compound 2. LC-MS (ESI): m/z 594.4 $[M+H]^+$. $^1H$ NMR (400 MHz, $CDCl_3$) δ 8.89 (s, 1H), 7.70 (s, 1H), 7.54 (d, *J* = 8.4 Hz, 2H), 7.49 (d, *J* = 8.4 Hz, 2H), 7.49 (d, *J* = 2.3 Hz, 1H), 7.37 (d, *J* = 2.3 Hz, 1H), 7.23 (d, *J* = 8.4 Hz, 2H), 7.03 (d, *J* = 8.4 Hz, 2H), 5.30 (s, 2H), 4.43 (t, *J* = 6.2 Hz, 2H), 3.88 (t, *J* = 6.2 Hz, 2H), 1.91 (d, *J* = 13.4 Hz, 6H), 1.70 (s, 6H). $^{31}P$ NMR (162 MHz, DMSO-d6) δ 35.11 (s, 1P).

**Example 3: Preparation of compound 3**

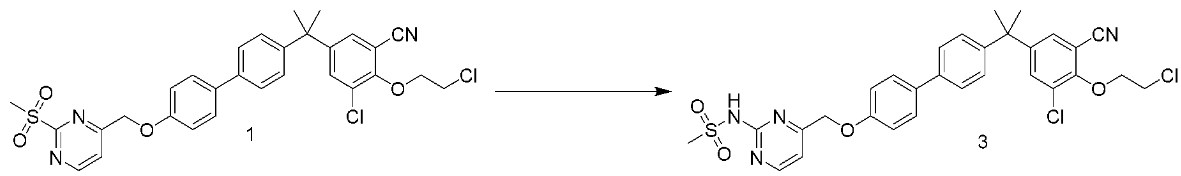

1

3

**Synthesis of compound 3**

Compound 1 (150 mg, 251.46 μmol), methanesulfonamide (120 mg, 1.26 mmol), $CH_3CN$ (3 mL), and $K_2CO_3$ (174 mg, 1.26 mmol) were added to a microwave tube. The mixture was heated to 100°C and stirred for 2 h. TLC indicated the reaction was complete. The reaction mixture was poured into water, and ethyl acetate (20 mL) was added. The pH was adjusted to 6 to 7, and the mixture was subjected to suction filtration. The filter cake was washed with water. Methanol (6 mL) and ethyl acetate (4 mL) were added to the obtained filter cake, and the mixture was heated to reflux for 1 h. The mixture was cooled to room temperature and stirred for 16 h, subjected to suction filtration, and evaporated to dryness under reduced pressure to obtain 89.28 mg of the title compound 3. LC-MS (ESI): m/z 611.4 $[M+H]^+$. $^1H$ NMR (400 MHz, DMSO-d6) δ 11.37 (br.s, 1H), 8.63 (d, *J* = 5.1 Hz, 1H), 7.69 (d, *J* = 2.3 Hz, 1H), 7.63 (d, *J* = 2.4 Hz, 1H), 7.61 (d, *J* = 8.8 Hz, 2H), 7.56 (d, *J* = 8.4 Hz, 2H), 7.30 (d, *J* = 8.5 Hz, 2H), 7.23 (d, *J* = 5.1 Hz, 1H), 7.10 (d, *J* = 8.8 Hz, 2H), 5.19 (s, 2H), 4.42 (t, *J* = 4.8 Hz, 2H), 3.96 (t, *J* = 4.8 Hz, 2H), 3.37 (s, 3H), 3.31 (s, 6H).

**Example 4: Preparation of compound** 4

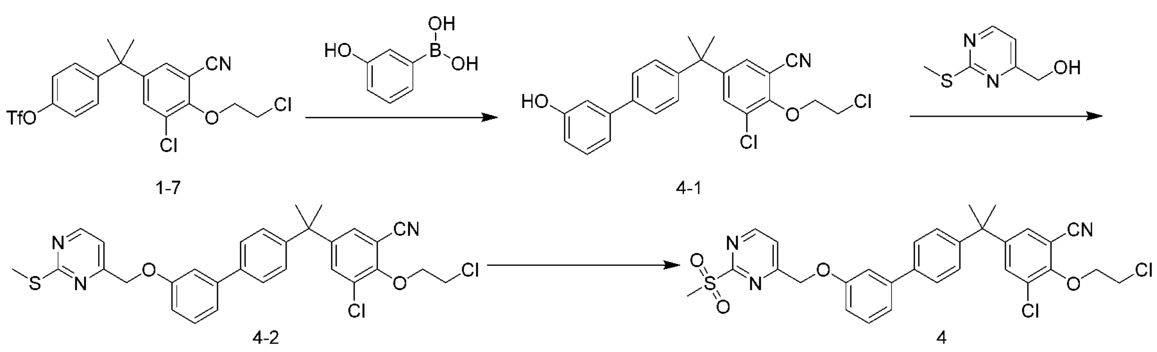

1-7

4-1

4-2

4

**Preparation of compound 4-1**

Compound **1-7** (1 g, 2.07 mmol) was dissolved in 1,4-dioxane (20.0 mL). 3-Hydroxyphenylboronic acid (400 mg, 2.9 mmol), potassium carbonate (860 mg, 6.2 mmol), [1,1'-bis(diphenylphosphino)ferrocene]dichloropalladium(II) (228 mg, 0.31 mmol), and water (2.0 mL) were added. The reaction system was purged three times with nitrogen and stirred at 100°C for 5 h. After the reaction was completed, water (10 mL) was added, and the mixture was extracted with dichloromethane (20 mL × 3). The organic phases were combined, washed twice with saturated brine (20 mL), dried over anhydrous sodium sulfate, and concentrated to obtain a crude product. The crude product was purified by column chromatography (EA:PE = 0 to 10%) to obtain 700 mg of the title compound **4-1.** LC-MS (ESI): m/z 426.2 $[M+H]^+$.

**Preparation of compound 4-2**

Compound **4-1** (450 mg, 1.06 mmol) was dissolved in dichloromethane (10.0 mL), followed by the sequential addition of 2-methylthiopyrimidin-4-ylmethanol (181 mg, 1.16 mmol) and *N*,*N*,*N*,*N*-tetramethylazodicarboxamide (365 mg, 2.1 mmol). The system was purged three times with nitrogen, and then tributylphosphine (427 mg, 2.1 mmol) was added under a nitrogen atmosphere. The reaction system was stirred at room temperature for 1 h. After the reaction was completed, water (20 mL) was added, and the mixture was extracted three times with dichloromethane (20 mL). The organic phases were combined, washed twice with saturated brine (20 mL), dried over anhydrous sodium sulfate, and concentrated to obtain a crude product. The crude product was purified by column chromatography (EA:PE = 0 to 10%) to obtain 450 mg of the title compound **4-2.** LC-MS (ESI): m/z 564.2 $[M+H]^+$.

**Preparation of compound 4**

Compound 4-2 (150 mg, 0.42 mmol) was dissolved in THF (2.0 mL), and a solution of Oxone (576 mg, 1.6 mmol) in $H_2O$ (2.0 mL) was added. The reaction system was stirred at room temperature for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **4.** LC-MS (ESI): m/z 596.2 $[M+H]^+$. $^1H$ NMR (400 MHz, $CDCl_3$) δ 8.93 (d, *J* = 5.1 Hz, 1H), 7.86 (d, *J* = 5.1 Hz, 1H), 7.52 (d, *J* = 8.4 Hz, 2H), 7.49 (d, *J* = 2.4 Hz, 1H), 7.40 (t, *J* = 8.0 Hz, 1H), 7.37 (d, *J* = 2.4 Hz, 1H), 7.28 - 7.24 (m, 3H), 7.20 (t, *J* = 2.6 Hz, 1H), 6.94 (ddd, *J* = 8.2, 2.6, 0.9 Hz, 1H), 5.36 (d, *J* = 0.8 Hz, 2H), 4.43 (t, *J* = 6.2 Hz, 2H), 3.88 (t, *J* = 6.2 Hz, 2H), 3.39 (s, 3H), 1.71 (s, 6H).

**Example 5: Preparation of compound 5**

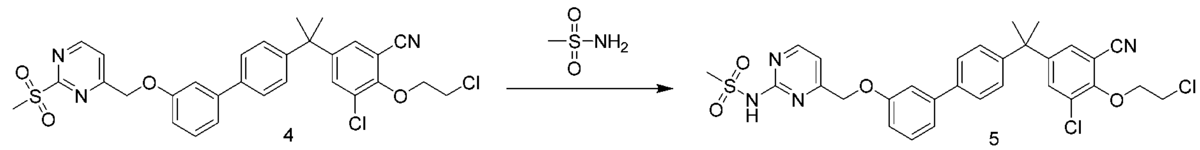

**Preparation of compound 5**

Compound **4** (50 mg, 0.08 mmol) was dissolved in THF (2.0 mL), followed by the sequential addition of methanesulfonamide (95 mg, 0.12 mmol) and potassium carbonate (30 mg, 0.21 mmol). The reaction system was stirred at 80°C for 4 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound **5.** LC-MS (ESI): m/z 611.2 $[M+H]^+$. $^1H$ NMR (400 MHz, DMSO-d6) δ 11.38 (br.s, 1H), 8.64 (d, *J*= 5.1 Hz, 1H), 7.71 (d, *J* = 2.3 Hz, 1H), 7.65 (d, *J* = 2.3 Hz, 1H), 7.62 (d, *J* = 8.3 Hz, 2H), 7.39 (t, *J* = 7.9 Hz, 1H), 7.34 (d, *J* = 8.3 Hz, 2H), 7.31 (s, 1H), 7.27 (t, *J* = 6.6 Hz, 2H), 7.03 (dd, *J* = 8.3, 2.5 Hz, 1H), 5.24 (s, 2H), 4.44 (t, *J* = 5.2 Hz, 2H), 3.97 (t, *J* = 5.2

Hz, 2H), 3.37 (s, 3H), 1.70 (s, 6H).

**Example 6: Preparation of compound 6**

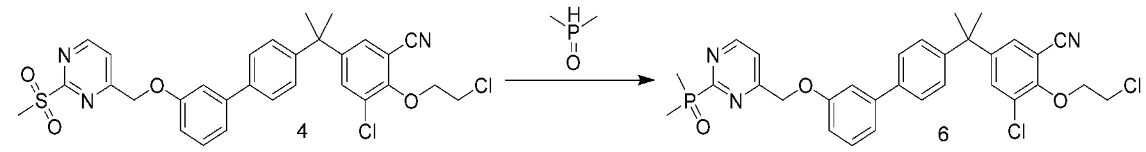

4 6

**Preparation of compound 6**

Compound **4** (50 mg, 0.08 mmol) was dissolved in tetrahydrofuran (2.0 mL), and dimethylphosphine oxide (20 mg, 0.025 mmol) was added. The reaction system was stirred at 80°C for 5 h. After the reaction was completed, water (20 mL) was added, and the mixture was extracted three times with ethyl acetate (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 22 mg of the title compound **6.** LC-MS (ESI): m/z 594.2 $[M+H]^+$. $^1H$ NMR (400 MHz, DMSO-d6) δ 8.93 (d, *J* = 5.1 Hz, 1H), 7.69 (dd, *J* = 5.2, 3.2 Hz, 1H), 7.63 (d, *J* = 2.3 Hz, 1H), 7.59 - 7.55 (m, 2H), 7.54 (s, 1H), 7.32 (t, *J* = 7.9 Hz, 1H), 7.28 (s, 1H), 7.25 (d, *J* = 8.3 Hz, 2H), 7.23 - 7.18 (m, 1H), 6.98 (dd, *J* = 8.2, 2.5 Hz, 1H), 5.32 (s, 2H), 4.36 (t, *J* = 5.2 Hz, 2H), 3.89 (t, *J* = 5.2 Hz, 2H), 1.69 (d, *J* = 13.7 Hz, 6H), 1.62 (s, 6H). $^{31}P$ NMR (162 MHz, DMSO-*d6)* δ 33.99 (s, 1P).

**Example 7: Preparation of compound 7**

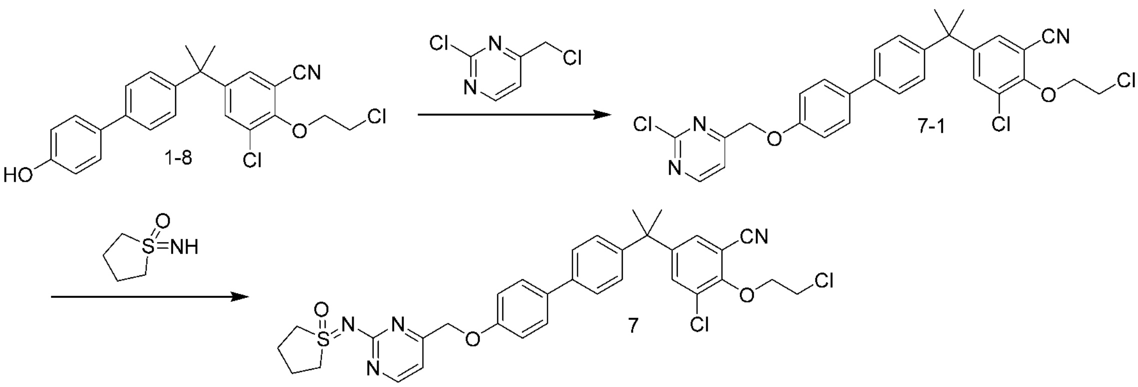

1-8 7-1 7

**Preparation of compound 7-1**

Compound **1-8** (190 mg, 445.66 μmol), DMF (4 mL), 2-chloro-4-(chloromethyl)pyrimidine (119.85 mg, 735.26 μmol, 85 μL), and $K_2CO_3$ (125 mg, 904.45 μmol) were added to a 100 mL single-necked flask. The mixture was stirred at room temperature for 5 h. TLC indicated that the reaction was essentially complete. Water (50 mL) was added, and the mixture was extracted twice with ethyl acetate (40 mL). The organic phases were combined, washed with water (50 mL), dried over anhydrous sodium sulfate, subjected to suction filtration, and the filtrate was evaporated to dryness under reduced pressure. The residue was purified by an automated column chromatography system (EA:PE = 10 to 100%) to obtain 210 mg of the title compound **7-1.** LC-MS (ESI): m/z 552.2 $[M+H]^+$. $^1H$ NMR (400 MHz, $CDCl_3$) δ 8.65 (d, *J* = 5.0 Hz, 1H), 7.56 (d, *J* = 4.5 Hz, 1H), 7.54 (d, *J* = 8.9 Hz, 2H), 7.49 (d, *J* = 8.4 Hz, 2H), 7.49 (d, *J* = 2.4 Hz, 1H), 7.37 (d, *J* = 2.4 Hz, 1H), 7.23 (d, *J* = 8.4 Hz, 2H), 7.02 (d, *J* = 8.8 Hz, 2H), 5.20 (d, *J* = 0.8 Hz, 2H), 4.43 (d, *J* = 6.2 Hz, 2H), 3.88 (d, *J* = 6.2 Hz, 2H), 1.70 (s, 6H).

**Preparation of compound 7**

Compound **7-1** (210 mg, 0.38 mmol) was dissolved in DMF (2.0 mL), followed by the sequential addition of tris(dibenzylideneacetone)dipalladium (70 mg, 0.08 mmol), 4,5-bis(diphenylphosphino)-9,9-dimethylxanthene (44 mg, 0.08 mmol), DIEA (148 mg, 0.8 mmol), and 1-imino-1-oxothiolane (50 mg, 0.42 mmol). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 37 mg of the title compound 7. LC-MS (ESI): m/z 635.2 $[M+H]^+$. $^1H$ NMR (400 MHz, DMSO-d6) δ 8.47 (d, *J* = 5.1 Hz, 1H), 7.69 (d, *J* = 2.3 Hz, 1H), 7.63 (d, *J* = 2.3 Hz, 1H), 7.60 (d, *J* = 8.8 Hz, 2H), 7.55 (d, *J* = 8.5 Hz, 2H), 7.30 (d, *J* = 8.5 Hz, 2H), 7.08 (d, *J* = 8.8 Hz, 2H), 6.98 (d, *J* = 5.1 Hz, 1H), 5.10 (s, 2H), 4.42 (t, *J*= 5.2 Hz, 2H), 3.95 (t, *J* = 5.2 Hz, 2H), 3.62 - 3.52 (m, 2H), 3.40 - 3.31 (m, 2H), 2.27 - 2.16 (m, 2H), 2.15 -2.05 (m, 2H), 1.68 (s, 6H).

**Example 8: Preparation of compound** 8

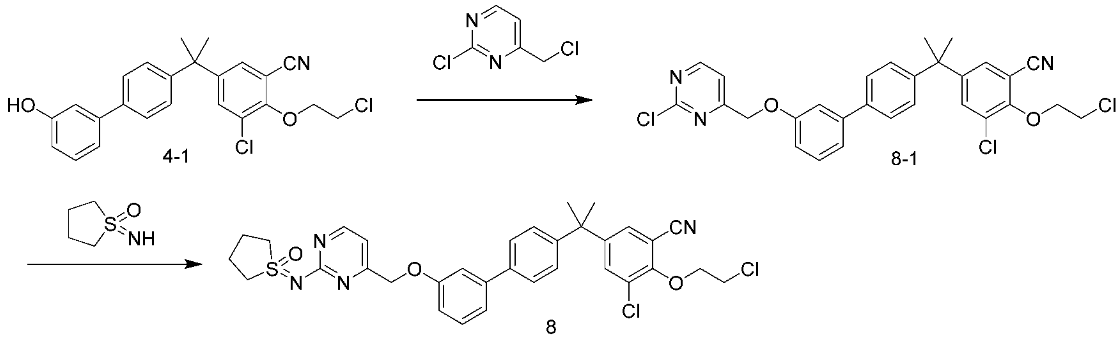

4-1

8-1

8

**Preparation of compound 8-1**

Compound **4-1** (105 mg, 0.25 mmol) was dissolved in acetonitrile (2.0 mL), followed by the sequential addition of 2-chloro-4-(chloromethyl)pyrimidine (40 mg, 0.25 mmol) and potassium carbonate (102 mg, 0.75 mmol). The reaction system was purged three times with nitrogen and stirred at 100°C for 5 h. After the reaction was completed, water (10 mL) was added, and the mixture was extracted three times with dichloromethane (20 mL). The organic phases were combined, washed twice with saturated brine (20 mL), dried over anhydrous sodium sulfate, and concentrated to obtain a crude product. The crude product was purified by column chromatography (EA:PE = 0 to 30%) to obtain 127 mg of the title compound 8-1. LC-MS (ESI): m/z 552.0 $[M+H]^+$.

**Preparation of compound 8**

Compound **8-1** (127 mg, 0.23 mmol) was dissolved in DMF (2.0 mL), followed by the sequential addition of tris(dibenzylideneacetone)dipalladium (42 mg, 0.05 mmol), 4,5-bis(diphenylphosphino)-9,9-dimethylxanthene (27 mg, 0.05), DIEA (88 mg, 0.7 mmol), and 1-imino-1-oxothiolane (30 mg, 0.25 mmol). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2 mg of the title compound **8.** LC-MS (ESI): m/z 635.2 $[M+H]^+$. $^1H$ NMR (400 MHz, DMSO-$d_6$) δ 8.47 (d, *J* = 5.1 Hz, 1H), 7.71 (d, *J* = 2.4 Hz, 1H), 7.65 (d, *J* = 2.3 Hz, 1H), 7.60 (d, *J* = 8.5 Hz, 2H), 7.37 (t, *J* = 7.9 Hz, 1H), 7.33 (d, *J* = 8.5 Hz, 2H), 7.28 - 7.23 (m, 2H), 7.02 - 6.97 (m, 2H), 5.14 (s, 2H), 4.43 (t, *J* = 5.2 Hz, 2H), 3.96 (t, *J*= 5.2 Hz, 2H), 3.62 - 3.51 (m, 2H), 3.40 - 3.32 (m, 2H), 2.25 - 2.14 (m, 2H), 2.14 - 2.04 (m, 2H), 1.70 (s, 6H).

**Example 9: Preparation of compound 9**

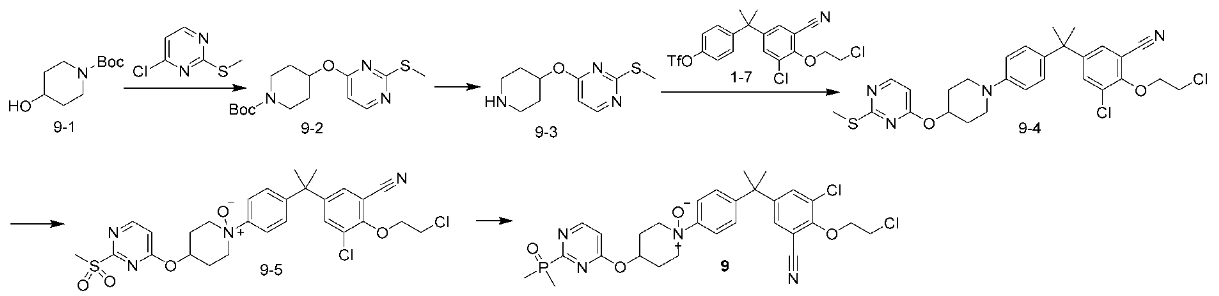


**Preparation of compound 9-2**

Under a nitrogen atmosphere, compound **9-1** (500 mg, 3.11 mmol), 2-methylthio-4-chloropyrimidine (626.50 mg, 3.11 mmol), and THF (10 mL) were added to a reaction flask. NaH (149.40 mg, 3.74 mmol, 60% purity) was added in portions at 0°C, and the mixture was slowly warmed to room temperature and stirred for 16 h. The reaction was monitored by LCMS until completion. Water (10 mL) was added, and then the mixture was extracted three times with dichloromethane (10 mL). The organic phases were combined, dried, and concentrated to obtain a crude product. The crude product was purified by normal-phase silica gel column chromatography (EtOAc/PE = 0 to 10%) to obtain 1.0 g of the title compound **9-2.** LC-MS (ESI): 326.2 $[M+H]^+$.

**Preparation of compound 9-3**

Under a nitrogen atmosphere, compound **9-2** (1 g, 3.07 mmol) and DCM (10 mL) were added to a reaction flask. A solution of hydrogen chloride in dioxane (4.0 M, 30.73 mmol, 7.68 mL) was added dropwise at 0°C, and the mixture was stirred at room temperature for 3 h. After the reaction was completed, the supernatant was decanted. Water (20 mL) was added to dissolve the residue, and then the pH was adjusted to basic with cesium carbonate. The mixture was extracted three times with DCM (10 mL), dried over anhydrous sodium sulfate, filtered, and subjected to rotary evaporation until dryness to obtain 530 mg of the title compound **9-3.** LC-MS (ESI): 226.2 $[M+H]^+$.

**Preparation of compound 9-4**

Under a nitrogen atmosphere, compound **9-3** (112.12 mg, 497.62 μmol), compound **1-7** (200 mg, 414.68 μmol), cesium carbonate (405.33 mg, 1.24 mmol), $Pd(OAc)_2$ (9.31 mg, 41.47 μmol), BINAP (51.64 mg, 82.94 μmol), and dioxane (5 mL) were added to a microwave tube, and the mixture was stirred at 110°C for 2 h. The mixture was filtered, and the filtrate was subjected to rotary evaporation until dryness. The crude product was purified by column chromatography (EA/PE = 0 to 100%) to obtain 180 mg of the title compound **9-4.** LC-MS (ESI): 557.2 $[M+H]^+$.

**Preparation of compound 9-5**

At 0°C, compound **9-4** (30 mg, 53.81 μmol) and DCM (1 mL) were added to a reaction flask, followed by the addition of *m*-CPBA (27.86 mg, 161.43 μmol). The mixture was slowly warmed to room temperature and stirred for 2 h. Saturated aqueous sodium carbonate solution (10 mL) was added, and the mixture was extracted three times with dichloromethane (10 mL). The organic phases were combined, dried, and concentrated to obtain a crude product. The crude product was purified by normal-phase silica gel column chromatography (EtOAc/PE = 0 to 100%) to obtain 10 mg of the title compound **9-5.** LC-MS (ESI): 605.2 $[M+H]^+$.

**Preparation of compound 9**

Compound **9-5** (10 mg, 16.51 μmol), dimethylphosphine oxide (3.87 mg, 49.54 μmol), $Cs_2CO_3$ (16.14 mg, 49.54 μmol), and acetonitrile (2 mL) were added to a reaction flask and stirred at 80°C for 2 h. The mixture was filtered and subjected to rotary evaporation until dryness to obtain 10 mg of the crude product of the title compound **9.** LC-MS (ESI): 603.2 $[M+H]^+$.

**Example 10: Preparation of compound 10**

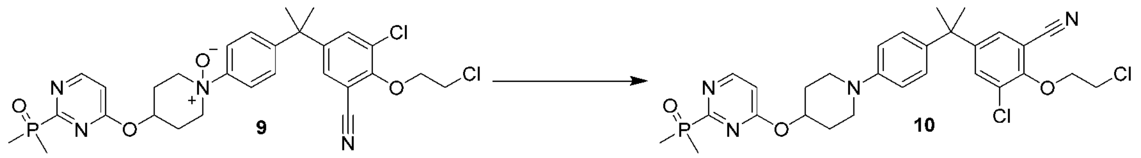

9 10

**Preparation of compound 10**

Under a nitrogen atmosphere, compound **9** (50 mg, 82.85 μmol), CuI (31.56 mg, 165.71 μmol), DIEA (21.42 mg, 165.71 μmol, 28.86 μL)and THF (1 mL) were added to a reaction flask, and the mixture was stirred at room temperature for 2 h. The mixture was filtered and subjected to rotary evaporation until dryness. Water (2 mL) was added, and then the mixture was extracted three times with dichloromethane (2 mL). The organic phases were combined, dried, and concentrated to obtain a crude product. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 10% to 80% acetonitrile gradient elution over 30 min; flow rate: 30 mL/min) to obtain 13 mg of the title compound **10.** LC-MS (ESI): 587.2 $[M+H]^+$. $^1$H NMR (400 MHz, DMSO-$d_6$) δ 8.66 (dd, *J* = 5.9, 1.0 Hz, 1H), 7.62 (d, *J* = 2.3 Hz, 1H), 7.56 (d, *J* = 2.3 Hz, 1H), 7.09 (d, *J* = 8.6 Hz, 2H), 7.04 (dd, *J* = 5.9, 2.7 Hz, 1H), 6.92 (d, *J* = 8.6 Hz, 2H), 5.36 - 5.24 (m, 1H), 4.41 (t, *J* = 5.2 Hz, 2H), 3.95 (t, *J* = 5.2 Hz, 2H), 3.55 - 3.45 (m, 2H), 3.13 - 3.03 (m, 2H), 2.17 - 2.08 (m, 2H), 1.87 - 1.77 (m, 2H), 1.74 (d, *J* = 13.7 Hz, 6H), 1.62 (s, 6H). $^{31}$P NMR (162 MHz, DMSO-$d_6$) δ 34.05 (s, 1P).

**Example 11: Preparation of compound 11**

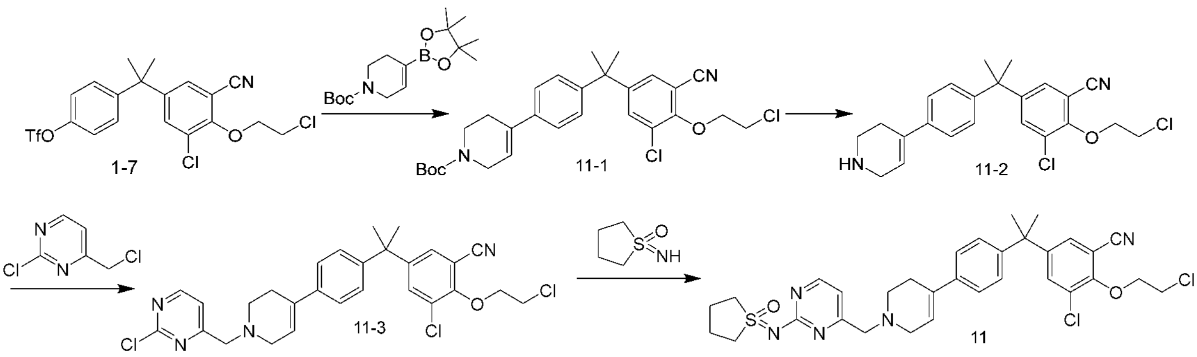

1-7 11-1 11-2 11-3 11

**Preparation of compound 11-1**

Compound **1-7** (1 g, 1.87 mmol) and (N-tert-butoxycarbonyl)-1,2,3,6-tetrahydropyridine-4-boronic acid pinacol ester (309 mg, 1.87 mmol) were dissolved in 1,4-dioxane (10.0 mL), followed by the sequential addition of cesium carbonate (1.22 g, 3.73 mmol) and [1,1'-bis(diphenylphosphino)ferrocene]dichloropalladium(II) dichloromethane complex (272.93 mg, 373.00 μmol). The system was purged three times with nitrogen, and then stirred at 105°C for 5 h. After the reaction was completed, the reaction mixture was cooled to room temperature, and the insoluble matter in the reaction mixture was removed by filtration. Saturated aqueous sodium chloride solution (10 mL) was added to the filtrate, and the mixture was extracted three times with ethyl acetate (10 mL). The organic phases were combined, dried, and concentrated to obtain a crude product. The crude product was purified by normal-phase silica gel column chromatography (EtOAc/PE = 0 to 40%) to obtain 820 mg of the title compound **11-1** in 77.22% yield. LC-MS (ESI): 459.2 $[M+H\text{-}t\text{-}Bu]^+$.

**Preparation of compound 11-2**

Compound **11-1** (1.0 g, 1.94 mmol) was dissolved in dichloromethane (10 mL), and the reaction system was cooled to 0°C, followed by dropwise addition of trifluoroacetic acid (3 mL). The reaction was continued with stirring for 3 h. Upon completion, the reaction mixture was concentrated to obtain a crude product **11-2** (0.8 g), which was used directly in

the next step. LC-MS (ESI): m/z 415.0 $[M+H]^+$.

**Preparation of compound 11-3**

Compound **11-2** (450 mg, 1.08 mmol) and potassium carbonate (150 mg, 1.08 mmol) were added to a single-necked flask. Anhydrous acetonitrile (5 mL) was added to the single-necked flask, and the mixture was cooled to 0°C. A solution of 2-chloro-4-(chloromethyl)pyrimidine (177 mg, 1.08 mmol) in acetonitrile (1 mL) was slowly added dropwise. The reaction system was stirred at 0°C for 60 min, and then quenched with saturated sodium bicarbonate solution (20 mL). The mixture was extracted three times with EtOAc (15 mL). The organic phases were combined, dried, and concentrated to obtain a crude product. The crude product was purified by normal-phase silica gel column chromatography (EtOAc/PE = 0 to 30%) to obtain 200 mg of the title compound **11-3** in 34% yield. LC-MS (ESI): m/z 541.2 $[M+H]^+$.

**Preparation of compound 11**

Compound **11-3** (40 mg, 0.074 mmol), DIEA (28 mg, 0.029 mmol), 1-iminotetrahydrothiophene-1-oxide (26 mg, 0.224 mmol), tris(dibenzylideneacetone)dipalladium (7 mg, 0.007 mmol), and 4,5-bis(diphenylphosphino)-9,9-dimethylxanthene (8 mg, 0.014 mmol) were dissolved in 1,4-dioxane. The reaction system was purged three times with nitrogen and stirred under microwave irradiation at 100°C for 2 h. After the reaction was completed, the reaction system was filtered, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain the title compound 11 (8 mg). LC-MS (ESI): m/z 624.3 $[M+H]^+$. $^1H$ NMR (400 MHz, Chloroform-d) δ 8.50 (d, *J* = 4.8 Hz, 1H), 7.42 (d, *J* = 2.4 Hz, 1H), 7.34 - 7.30 (m, 4H), 7.14 (d, *J* = 8.4 Hz, 2H), 6.03 (s, 1H), 4.42 (t, *J* = 6.2 Hz, 2H), 4.01 - 3.94 (m, 2H), 3.87 (t, *J* = 6.2 Hz, 2H), 3.75 - 3.68 (m, 2H), 3.64 - 3.56 (m, 2H), 3.46 - 3.39 (m, 2H), 3.21-3.06 (m, 2H), 2.84 - 2.74 (m, 2H), 2.41 - 2.31 (m, 2H), 2.31 - 2.21 (m, 2H), 1.66 (s, 6H).

**Example 12: Preparation of compound 12**

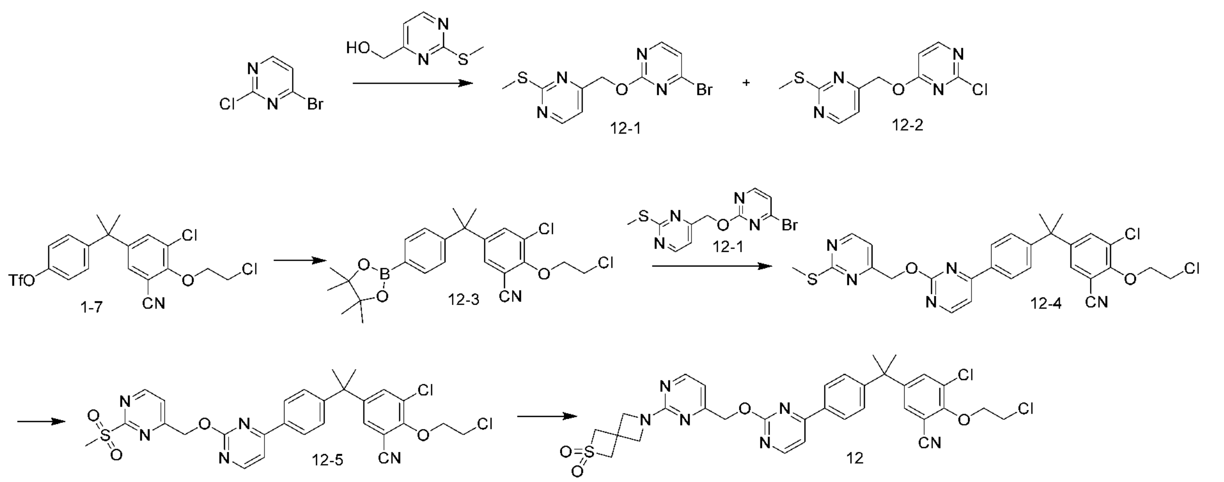
12-1
12-2
1-7
12-3
12-1
12-4
12-5
12

**Preparation of compounds 12-1 and 12-2**

At room temperature, compound 2-chloro-4-bromopyrimidine (619.15 mg, 3.20 mmol), compound (2-(methylthio)pyrimidin-4-yl)methanol (500 mg, 3.20 mmol), $Cs_2CO_3$ (2.09 g, 6.40 mmol), and acetonitrile (5 mL) were added to a reaction flask, and the mixture was stirred at 80°C for 16 h. The mixture was filtered, and the filtrate was concentrated to obtain a crude product. The crude product was purified by normal-phase silica gel column chromatography (EtOAc/PE = 0 to 20%) to obtain the title compound **12-1** (200 mg, yield: 20%) and compound **12-2** (500 mg, yield: 58%). Compound **12-1,** LC-MS (ESI): m/z 313.0 $[M+H]^+$; compound **12-2,** LC-MS (ESI): m/z 269.0 $[M+H]^+$.

**Preparation of compound 12-3**

Compound **1-7** (11.0 g, 22.81 mmol) and bis(pinacolato)diboron (5.79 g, 22.81 mmol) were dissolved in 1,4-dioxane (110.0 mL). [1,1'-Bis(diphenylphosphino)ferrocene]dichloropalladium(II) (0.83 g, 1.14 mmol) and an aqueous potassium carbonate solution (2 mol/L, 28.50 mL, 57.02 mmol) were then added. The system was purged four times with argon, heated to 110°C, and stirred for 16 h. After the reaction was completed, the reaction system was cooled to room temperature. Insoluble solids were removed by filtration through a pad of diatomite, and the filter cake was rinsed with ethyl acetate. The filtrates were combined, water (150 mL) was added, and the mixture was extracted twice with ethyl acetate (300 mL). The organic phases were combined, dried over anhydrous sodium sulfate and concentrated. The residue was purified by column chromatography (ethyl acetate/petroleum ether = 0 to 10%) to obtain 10.0 g of the title compound **12-3** in 86.9% yield. LC-MS (ESI): m/z 477.2 $[M+H_2O]^+$.

**Preparation of compound 12-4**

Under a nitrogen atmosphere, compound **12-3** (400 mg, 869.19 μmol), compound **12-1** (544.41 mg, 1.74 mmol), $K_2CO_3$ (360.38 mg, 2.61 mmol), $Pd(dppf)Cl_2$ (63.60 mg, 86.92 μmol), $H_2O$ (1 mL), and 1,4-dioxane (5 mL) were added to a reaction flask, and the mixture was stirred at 80°C for 2 h. The mixture was subjected to rotary evaporation until dryness, and the crude product was purified by normal-phase silica gel column chromatography (PE/EtOAc = 0 to 50%) to obtain 320 mg of the title compound **12-4** in 64% yield. LC-MS (ESI): 566.2 $[M+H]^+$.

**Preparation of compound 12-5**

Compound **12-4** (100 mg, 176.52 μmol) was dissolved in DCM (1 mL). At 0°C, m-CPBA (67.02 mg, 388.35 μmol) was added to the reaction flask. The temperature was slowly raised to room temperature and the mixture was stirred for 2 h. DCM (5 mL) was added to the system, and the mixture was washed three times with saturated aqueous sodium carbonate solution (5 mL), dried over anhydrous sodium sulfate, and subjected to rotary evaporation until dryness to obtain 100 mg of the title compound **12-5** in 94% yield. LC-MS (ESI): 598.2 $[M+H]^+$.

**Preparation of compound 12**

2-Thia-6-azaspiro[3.3]heptane 2,2-dioxide hydrochloride (46.03 mg, 250.63 μmol), compound **12-5** (100 mg, 167.08 μmol), DIEA (215.95 mg, 1.67 mmol, 291.03 μL), and acetonitrile (1 mL) were added to a reaction flask, and the mixture was stirred at 80°C for 2 h. The mixture was subjected to rotary evaporation until dryness to obtain a crude product of the target compound. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 10% to 60% acetonitrile gradient elution over 30 min; flow rate: 30 mL/min) to obtain 30 mg of the title compound 12. LC-MS (ESI): 665.0 $[M+H]^+$. $^1H$ NMR (400 MHz, DMSO-$d_6$) δ 8.66 (d, *J* = 5.2 Hz, 1H), 8.35 (d, *J* = 5.2 Hz, 1H), 8.10 - 8.08 (m, 2H), 7.72 (d, *J* = 5.2 Hz, 1H), 7.69 (d, *J* = 2.4 Hz, 1H), 7.63 (d, *J* = 2.4 Hz, 1H), 7.42 - 7.40 (m, 2H), 6.74 (d, *J* = 4.8 Hz, 1H), 5.37 (s, 2H), 4.49 (s, 4H), 4.42 (t, *J* = 5.2 Hz, 2H), 4.26 (s, 4H), 3.95 (t, *J* = 5.2 Hz, 2H), 1.70 (s, 6H).

**Example 13: Preparation of compound 13**

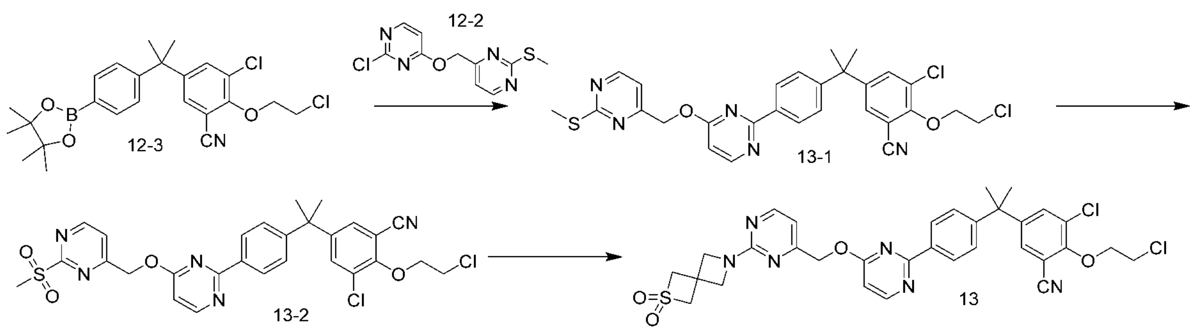
12-2
12-3
13-1
13-2
13

**Preparation of compound 13-1**

Under a nitrogen atmosphere, compound **12-3** (400 mg, 869.19 μmol), compound **12-2** (350.36 mg, 1.30 mmol), $K_2CO_3$ (360.38 mg, 2.61 mmol), Pd(dppf)$Cl_2$ (63.60 mg, 86.92 μmol), 1,4-dioxane (5 mL), and $H_2O$ (1 mL) were added to a reaction flask, and the reaction was carried out at 100°C for 16 h. Water (10 mL) was added, and the mixture was extracted three times with DCM (10 mL). The organic phases were combined, dried, and concentrated to obtain a crude product. The crude product was purified by normal-phase silica gel column chromatography (EtOAc/PE = 0 to 100%) to obtain 400 mg of the title compound **13-1** in 81% yield. LC-MS (ESI): 566.1 $[M+H]^+$.

**Preparation of compound 13-2**

Compound **13-1** (100 mg, 176.52 μmol) and DCM (1 mL) were added to a reaction flask, and m-CPBA (67.02 mg, 388.35 μmol) was added at 0°C. The mixture was slowly warmed to room temperature and stirred for 2 h. DCM (5 mL) was added, and the mixture was washed three times with saturated aqueous sodium carbonate solution (5 mL), dried over anhydrous sodium sulfate, and subjected to rotary evaporation until dryness to obtain100 mg of the title compound13-2in 94% yield. LC-MS (ESI): 598.2 $[M+H]^+$.

**Preparation of compound 13**

2-Thia-6-azaspiro[3.3]heptane 2,2-dioxide hydrochloride (46.03 mg, 250.63 μmol), compound **13-2** (100 mg, 167.08 μmol), DIEA (215.95 mg, 1.67 mmol, 291.03 μL), and acetonitrile (1 mL) were added to a reaction flask, and the mixture was stirred at 80°C for 2 h. The mixture was subjected to rotary evaporation until dryness to obtain a crude product of the target compound. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 10% to 60% acetonitrile gradient elution over 30 min; flow rate: 30 mL/min) to obtain 30 mg of the title compound 13. LC-MS (ESI): 665.0 $[M+H]^+$. $^1$H NMR (400 MHz, DMSO-$d_6$) δ 8.66 (d, *J* = 5.6 Hz, 1H), 8.36 (d, *J* = 5.2 Hz, 1H), 8.24 - 8.21 (m, 2H), 7.67 (d, *J* = 2.4 Hz, 1H), 7.61 (d, *J* = 2.4 Hz, 1H), 7.39 - 7.36 (m, 2H), 7.01 (d, *J* = 5.6 Hz, 1H), 6.79 (d, *J* = 5.2 Hz, 1H), 5.44 (s, 2H), 4.49 (s, 4H), 4.42 (t, *J* = 5.2 Hz, 2H), 4.25 (s, 4H), 3.95 (t, *J* = 5.2 Hz, 2H), 1.69 (s, 6H).

**Example 14: Preparation of compound 14**

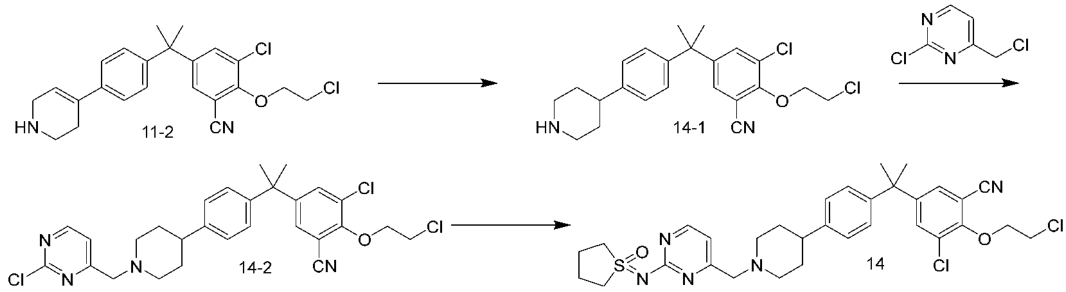

**Preparation of compound 14-1**

Compound **11-2** (0.4 g, 0.963 mmol) and anhydrous methanol (5 mL) were added to a single-necked flask, followed by the addition of Pd/C (0.17 g). The reaction system was purged three times with hydrogen, and the reaction was carried out under a hydrogen atmosphere (atmospheric pressure) for 12 h. Upon completion, the mixture was filtered through a pad of diatomite, and the filtrate was concentrated to obtain 400 mg of the crude product of the title compound **14-1,** which was used directly in the next step. LC-MS (ESI): m/z 417.2 $[M+H]^+$.

**Preparation of compound 14-2**

Compound **14-1** (0.400 g, 0.958 mmol), DIEA (371 mg, 2.88 mmol), and anhydrous acetonitrile (5 mL) were added to a single-necked flask. The mixture was cooled to 0°C. A solution of 2-chloro-4-(chloromethyl)pyrimidine (163 mg, 0.958 mmol) in acetonitrile (1 mL) was slowly added dropwise at 0°C, and the reaction mixture was stirred at 0°C for 60 min. The reaction was quenched by the addition of saturated sodium bicarbonate solution (10 mL) to the reaction system, and

the mixture was extracted three times with EtOAc (15 mL). The organic phases were combined, dried, and concentrated to obtain a crude product. The crude product was purified by normal-phase silica gel column chromatography (EtOAc/PE = 0 to 30%) to obtain the title compound **14-3** (280 mg) in 53% yield. LC-MS (ESI): m/z 543.2 $[M+H]^+$.

**Preparation of compound 14**

Compound **14-2** (280 mg, 0.517 mmol), DIEA (200 mg, 1.55 mmol), 1-iminotetrahydrothiophene-1-oxide (119 mg, 0.184 mmol), tris(dibenzylideneacetone)dipalladium (47 mg, 0.051 mmol), 4,5-bis(diphenylphosphino)-9,9-dimethylxanthene (60 mg, 0.1034 mmol), and 1,4-dioxane (5 mL) were added to a reaction flask. The reaction system was purged three times with nitrogen and reacted under microwave irradiation at 100°C for 2 h. After the reaction was completed, the reaction mixture was cooled to room temperature, filtered, and water (15 mL) was added. The mixture was extracted three times with dichloromethane (20 mL). The organic phases were combined, dried, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 100 mg of the title compound **14.** LC-MS (ESI): m/z 626.4 $[M+H]^+$. $^1H$ NMR (400 MHz, DMSO-$d_6$) δ 8.41 (d, *J* = 5.1 Hz, 1H), 7.66 (d, *J* = 2.3 Hz, 1H), 7.60 (d, *J* = 2.3 Hz, 1H), 7.22 - 7.15 (m, 4H), 6.97 (d, *J* = 5.1 Hz, 1H), 4.41 (t, *J* = 5.4 Hz, 2H), 3.95 (t, *J* = 5.4 Hz, 2H), 3.52-3.61 (m, 4H), 3.31 - 3.38 (m, 6H), 2.95 - 2.98 (m, 2H), 2.46 (m, 1H), 2.17 - 2.26 (m, 4H), 2.08 - 2.14 (m, 2H), 1.64 (s, 6H).

**Example 15: Preparation of compound 15**

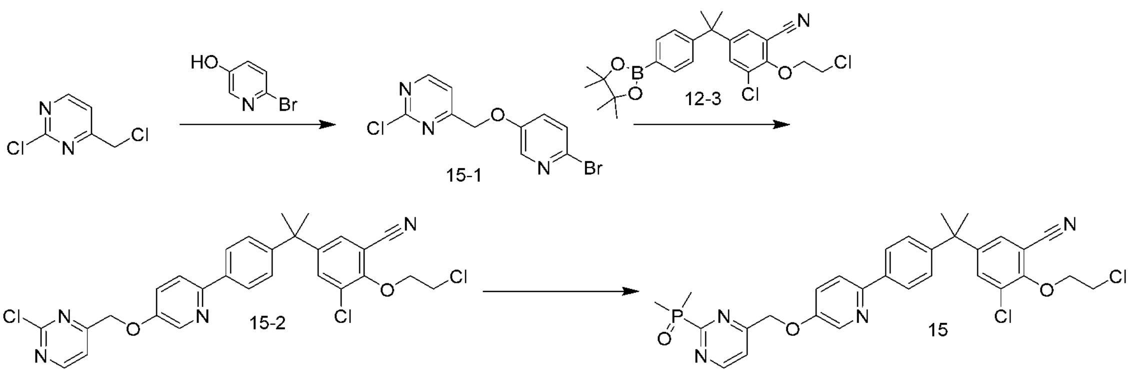


**Preparation of compound 15-1**

Compound 2-bromo-5-hydroxypyridine (300 mg, 1.72 mmol), compound 2-chloro-4-(chloromethyl)pyrimidine (421.57 mg, 2.59 mmol), $K_2CO_3$ (476.58 mg, 3.45 mmol), and acetonitrile (3 mL) were added to a reaction flask, and the mixture was stirred at 70°C for 16 h, filtered, and the filtrate was concentrated to obtain a crude product. The crude product was purified by normal-phase silica gel column chromatography (EtOAc/PE = 0 to 20%) to obtain 300 mg of the title compound **15-1** in 57% yield. LC-MS (ESI): 300.0 $[M+H]^+$.

**Preparation of compound 15-2**

Under a nitrogen atmosphere, compound **12-3** (100 mg, 217.30 μmol), compound **15-1** (97.96 mg, 325.95 μmol), $K_2CO_3$ (90.09 mg, 651.89 μmol), Pd(dppf)$Cl_2$ (31.80 mg, 43.46 μmol), 1,4-dioxane (1 mL), and $H_2O$ (0.2 mL) were added to a reaction flask, and the mixture was stirred at 70°C for 3 h. The mixture was subjected to rotary evaporation until dryness, and the crude product was purified by normal-phase silica gel column chromatography (EtOAc/PE = 0 to 50%) to obtain 50 mg of the title compound **15-2** in 41% yield. LC-MS (ESI): 553.2 $[M+H]^+$.

**Preparation of compound 15**

Dimethylphosphine oxide (21.14 mg, 270.82 μmol) and compound **15-2** (100 mg, 180.55 μmol) were dissolved in DMF (1 mL), then $Pd_2(dba)_3$ (16.52 mg, 18.05 μmol), Xantphos (20.89 mg, 36.11 μmol), and DIEA (116.67 mg, 902.75 μmol, 157.24 μL) were added. The system was purged three times with nitrogen and reacted under microwave irradiation at 120°C for 2 h. The mixture was filtered and subjected to rotary evaporation until dryness to obtain a crude product of the

target compound. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 10% to 80% acetonitrile gradient elution over 30 min; flow rate: 30 mL/min) to obtain 30 mg of the title compound **15.** LC-MS (ESI): 595.2 [M+H]$^+$. $^1$H NMR (400 MHz, DMSO-$d_6$) δ 9.02 (d, *J* = 5.2 Hz, 1H), 8.50 (d, *J* = 3.2 Hz, 1H), 7.97 - 7.95 (m, 2H), 7.92 (d, *J* = 8.8 Hz, 1H), 7.78 (dd, *J* = 5.2, 3.2 Hz, 1H), 7.69 (d, *J* = 2.4 Hz, 1H), 7.62 (d, *J* = 2.4 Hz, 1H), 7.60 (dd, *J* = 8.8, 3.2 Hz, 1H), 7.35 - 7.33 (m, 2H), 5.44 (s, 2H), 4.42 (t, *J* = 5.2 Hz, 2H), 3.95 (t, *J* = 5.2 Hz, 2H), 1.76 (d, *J* = 13.6 Hz, 6H), 1.69 (s, 6H). $^{31}$P NMR (162 MHz, DMSO-$d_6$) δ 34.16 (s, 1P).

**Example 16: Preparation of compound 16**

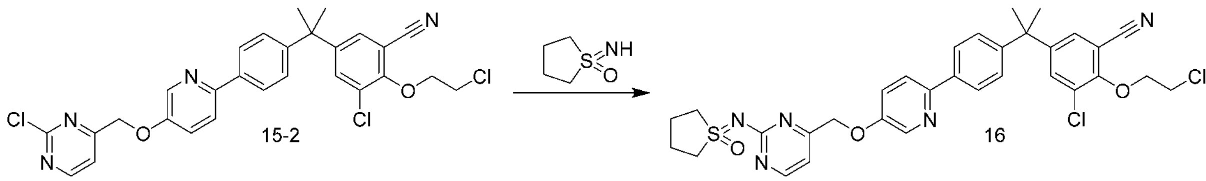
15-2
16

**Preparation of compound 16**

Compound **15-2** (100 mg, 180.55 μmol), 1-iminotetrahydrothiophene 1-oxide (32.28 mg, 270.82 μmol), cesium carbonate (117.65 mg, 361.10 μmol), Xantphos (20.89 mg, 36.11 μmol), $Pd_2(dba)_3$ (16.53 mg, 18.05 μmol), and dioxane (1 mL) were added to a microwave tube. The mixture was stirred at 100°C for 2 h under a nitrogen atmosphere. The mixture was filtered and subjected to rotary evaporation until dryness to obtain a crude product. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 10% to 80% acetonitrile gradient elution over 30 min; flow rate: 30 mL/min) to obtain 20 mg of the title compound **16.** LC-MS (ESI): 636.3 [M+H]$^+$. $^1$H NMR (400 MHz, DMSO-$d_6$) δ 8.49 (d, *J* = 5.2 Hz, 1H), 8.44 (d, *J* = 2.8 Hz, 1H), 7.95 - 7.93 (m, 2H), 7.89 (d, *J* = 8.8 Hz, 1H), 7.69 (d, *J* = 2.4 Hz, 1H), 7.62 (d, *J* = 2.4 Hz, 1H), 7.52 (dd, *J* = 8.8, 2.8 Hz, 1H), 7.34 - 7.32 (m, 2H), 7.02 (d, *J* = 5.2 Hz, 1H), 5.19 (s, 2H), 4.42 (t, *J* = 5.2 Hz, 2H), 3.95 (t, *J* = 5.2 Hz, 2H), 3.61 - 3.54 (m, 2H), 3.26 - 3.21 (m, 2H), 2.24 - 2.17 (m, 2H), 2.14 - 2.07 (m, 2H), 1.68 (s, 6H).

**Example 17: Preparation of compound 17**

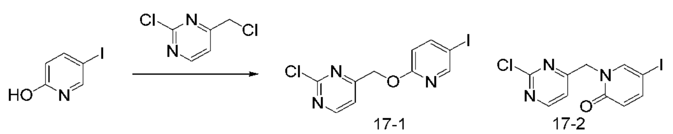
17-1
17-2

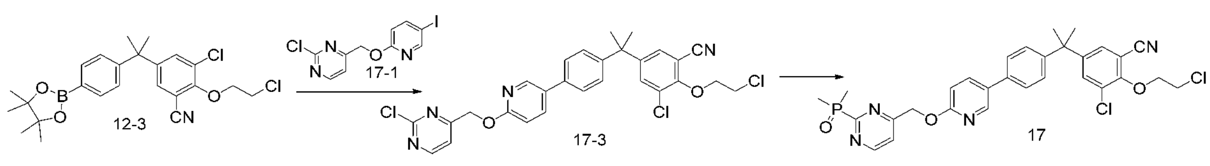
17-1
12-3
17-3
17

**Preparation of compounds 17-1 and 17-2**

Compound 2-hydroxy-5-iodopyridine (0.990 g, 4.48 mmol), potassium carbonate (1.236 g, 8.96 mmol), and 2-chloro-4-(chloromethyl)pyrimidine (0.730 g, 4.48 mmol) were dissolved in DMF (10 mL). The reaction system was heated to 80°C and stirred for 3 h. Upon completion, the mixture was diluted with ethyl acetate (50 mL) and washed three times with a 5% aqueous lithium chloride solution. The organic phase was separated, dried, filtered, and concentrated to obtain a crude product. The crude product was purified by normal-phase silica gel column chromatography (EtOAc/PE = 0 to 100%) to obtain 210 mg of the title compound **17-1** (yield: 13.5%) and 800 mg of the title compound **17-2** (yield: 51%). Compound **17-1,** LC-MS (ESI): m/z 348.0 [M+H]$^+$; compound **17-2,** LC-MS (ESI): m/z 348.0 [M+H]$^+$.

**Preparation of compound 17-3**

Compound **17-1** (0.250 g, 0.543 mmol), compound **12-3** (0.189 g, 0.543 mmol), potassium carbonate (0.187 g, 1.36 mmol), and Pd(dppf)$Cl_2$ (0.039 g, 0.054 mmol) were added to a single-necked flask. 1,4-Dioxane (8 mL) and water (2 mL) were added to the single-necked flask. The reaction system was purged three times with nitrogen, heated to 90°C, and reacted for 6 h. After the reaction was complete, the mixture was cooled to room temperature, filtered through diatomite, and the filtrate was concentrated to obtain a crude product. The crude product was purified by normal-phase silica gel column chromatography (EtOAc/PE = 0 to 30%) to obtain 250 mg of the title compound **17-3** in 83% yield. LC-MS (ESI): m/z 553.1 $[M+H]^+$.

**Preparation of compound 17**

Compound **17-3** (70 mg, 0.126 mmol), DIEA (49 mg, 0.379 mmol), dimethylphosphine oxide (30 mg, 0.379 mmol), tris(dibenzylideneacetone)dipalladium (12 mg, 0.012 mmol), and 4,5-bis(diphenylphosphino)-9,9-dimethylxanthene (7 mg, 0.012 mmol) were dissolved in DMF (5 mL). The reaction system was purged three times with nitrogen and stirred under microwave irradiation at 130°C for 2 h. After the reaction was completed, the reaction mixture was cooled to room temperature, and water (10 mL) was added. The reaction system was filtered, extracted three times with dichloromethane (20 mL), washed three times with a 5% aqueous lithium chloride solution, and the organic phases were separated. The organic phases were combined, dried, filtered, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound **17.** LC-MS (ESI): m/z 595.2 $[M+H]^+$. $^1$H NMR (400 MHz, Chloroform-d) δ 8.84 (d, *J* = 5.2 Hz, 1H), 8.34 (d, *J* = 2.4 Hz, 1H), 7.89 (dd, *J* = 8.4, 2.4 Hz, 1H), 7.56 - 7.53 (m, 1H), 7.50 (m, 1H), 7.48 (d, *J* = 8.4 Hz, 2H), 7.36 (d, *J* = 2.4 Hz, 1H), 7.27 (d, *J* = 8.4 Hz, 2H), 7.01 (d, *J* = 8.4 Hz, 1H), 5.63 (s, 2H), 4.44 (t, *J* = 6.2 Hz, 2H), 3.89 (t, *J* = 6.2 Hz, 2H), 1.90 (d, *J* = 13.5 Hz, 6H), 1.71 (s, 6H). $^{31}$P NMR (162 MHz, Chloroform-d) δ 35.16.

**Example 18: Preparation of compound 18**

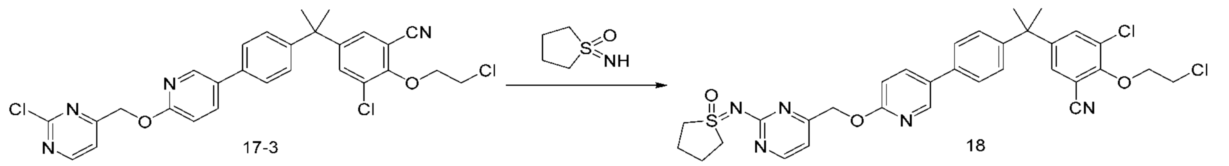

**Preparation of compound 18**

Compound **17-3** (45 mg, 0.081 mmol), DIEA (31 mg, 0.243 mmol), 1-iminotetrahydrothiophene-1-oxide (30 mg, 0.379 mmol), tris(dibenzylideneacetone)dipalladium (7 mg, 0.008 mmol), and 4,5-bis(diphenylphosphino)-9,9-dimethylxanthene (5 mg, 0.008 mmol) were dissolved in 1,4-dioxane (5 mL). The reaction system was purged three times with nitrogen and stirred under microwave irradiation at 100°C for 2 h. After the reaction was completed, the reaction system was cooled to room temperature, filtered, and then the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound **18.** LC-MS (ESI): m/z 636.2 $[M+H]^+$. $^1$H NMR (400 MHz, Chloroform-*d*) δ 8.47 (d, *J* = 5.2 Hz, 1H), 8.33 (d, *J* = 2.8 Hz, 1H), 7.84 (dd, *J* = 8.4, 2.4 Hz, 1H), 7.48 (d, *J* = 2.3 Hz, 1H), 7.47 (d, *J* = 8.4 Hz, 2H), 7.36 (d, *J* = 2.4 Hz, 1H), 7.26 (d, *J* = 8.4 Hz, 2H), 7.01 (d, *J* = 5.2 Hz, 1H), 6.96 (dd, *J* = 8.4, 0.8 Hz, 1H), 5.46 (s, 2H), 4.43 (t, *J* = 6.2 Hz, 2H), 3.88 (t, *J* = 6.2 Hz, 2H), 3.74 - 3.68 (m, 2H), 3.44 - 3.37 (m, 2H), 2.38 - 2.33 (m, 2H), 2.31 - 2.25 (m, 2H), 1.70 (s, 6H).

**Example 19: Preparation of compound 19**

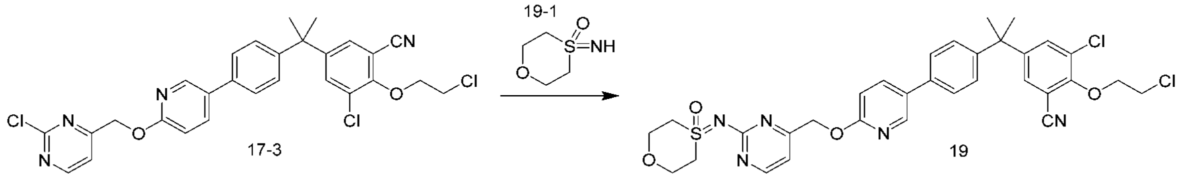


### Preparation of compound 19

Compound **17-3** (75 mg, 0.135 mmol), DIEA (53 mg, 0.406 mmol), **19-1** (27 mg, 0.203 mmol), tris(dibenzylideneacetone)dipalladium (12 mg, 0.013 mmol), and 4,5-bis(diphenylphosphino)-9,9-dimethylxanthene (8 mg, 0.013 mmol) were dissolved in 1,4-dioxane (5 mL). The reaction system was purged three times with nitrogen and stirred under microwave irradiation at 100°C for 2 h. After the reaction was completed, the reaction system was cooled to room temperature, filtered, and then concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 10 mg of the title compound **19.** LC-MS (ESI): m/z 652.2 $[M+H]^+$. $^1H$ NMR (400 MHz, Chloroform-*d*) δ 8.43 (d, *J* = 5.2 Hz, 1H), 8.33 (d, *J* = 2.5 Hz, 1H), 7.84 (dd, *J* = 8.6, 2.5 Hz, 1H), 7.48 (d, *J* = 2.3 Hz, 1H), 7.46 (d, *J* = 8.4 Hz, 2H), 7.36 (d, *J* = 2.3 Hz, 1H), 7.25 (d, *J* = 8.4 Hz, 2H), 6.98 (d, *J* = 5.2 Hz, 1H), 6.95 (d, *J* = 8.6 Hz, 1H), 5.44 (s, 2H), 4.43 (t, *J* = 6.1 Hz, 2H), 4.24 - 4.13 (m, 4H), 3.88 (t, *J* = 6.1 Hz, 2H), 3.86 - 3.81 (m, 2H), 3.52 - 3.47 (m, 2H), 1.69 (s, 6H).

### Example 20: Preparation of compound 20

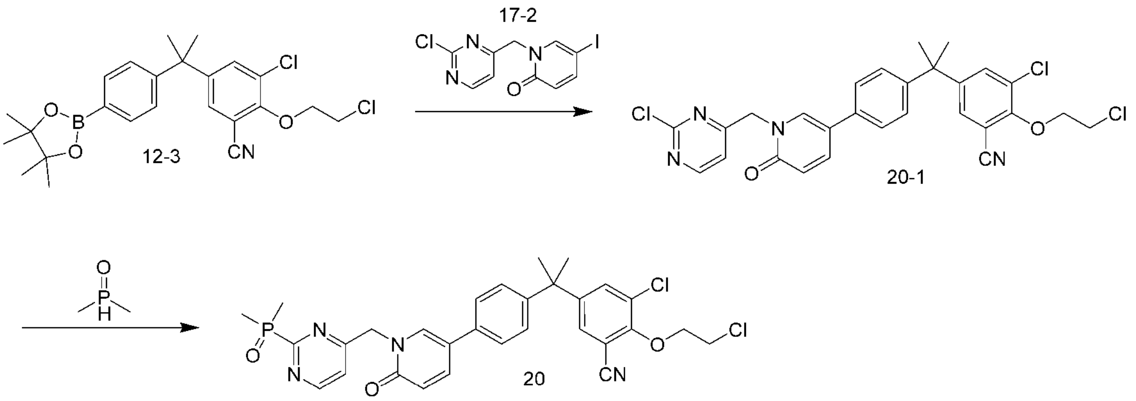


### Preparation of compound 20-1

Compound **17-2** (0.120 g, 0.347 mmol), compound **12-3** (0.160 g, 0.347 mmol), potassium carbonate (0.144 g, 1.04 mmol), and Pd(dppf)$Cl_2$ (0.025 g, 0.035 mmol) were added to a single-necked flask. 1,4-Dioxane (8 mL) and water (2 mL) were added to the single-necked flask. The reaction system was purged three times with nitrogen, heated to 90°C, and reacted for 6 h. After the reaction was completed, the reaction mixture was filtered through a pad of diatomite, and the filtrate was concentrated to obtain a crude product. The crude product was purified by normal-phase silica gel column chromatography (EtOAc/PE = 0 to 100%) to obtain 140 mg of the title compound **20-1** in 72.7% yield. LC-MS (ESI): m/z 553.1$[M+H]^+$.

### Preparation of compound 20

Compound **20-1** (100 mg, 0.180 mmol), DIEA (116 mg, 0.896 mmol), dimethylphosphine oxide (70 mg, 0.896 mmol), tris(dibenzylideneacetone)dipalladium (27 mg, 0.030 mmol), and 4,5-bis(diphenylphosphino)-9,9-dimethylxanthene (17 mg, 0.030 mmol) were dissolved in DMF (5 mL). The reaction system was purged three times with nitrogen and stirred under microwave irradiation at 130°C for 2 h. After the reaction was completed, the reaction system was filtered, and the filtrate was concentrated to obtain a crude product. The crude product was subjected to preparative purification (preparative method: mobile phase: A: 0.1% formic acid in water; B: acetonitrile; chromatographic column: Welch Ultimate

AQ-C18 250 × 21.2 mm; column temperature: 25°C; gradient: 45% to 65% acetonitrile gradient elution over 12 min; flow rate: 30 mL/min) to obtain 24 mg of the title compound **20.** LC-MS (ESI): m/z 595.2 $[M+H]^+$. $^1H$ NMR (400 MHz, Chloroform-*d*) δ 8.82 (d, *J* = 4.4 Hz, 1H), 7.71 (m, 2H), 7.46 (d, *J* = 2.3 Hz, 1H), 7.41 (br.s, 1H), 7.37 (d, *J* = 7.9 Hz, 2H), 7.32 (d, *J* = 2.3 Hz, 1H), 7.23 (d, *J* = 7.9 Hz, 2H), 6.72 (d, *J* = 9.3 Hz, 1H), 5.34 (s, 2H), 4.42 (t, *J* = 6.0 Hz, 2H), 3.87 (t, *J* = 6.0 Hz, 2H), 1.84 (d, *J* = 13.6 Hz, 6H), 1.68 (s, 6H). $^{31}P$ NMR (162 MHz, Chloroform-*d*) δ 35.80.

**Example 21: Preparation of compound 21**

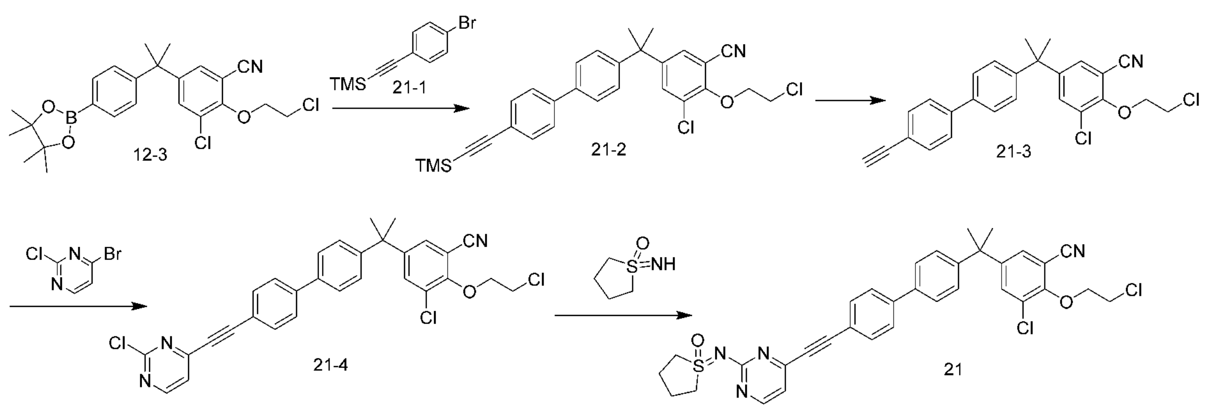
12-3, 21-1, 21-2, 21-3, 21-4, 21

**Preparation of compound 21-2**

Compound **12-3** (448 mg, 1.18 mmol) and (4-bromophenylethynyl)trimethylsilane (330 mg, 1.30 mmol) were dissolved in 1,4-dioxane (5 mL), followed by the sequential addition of potassium carbonate (327 mg, 2.37 mmol) and [1,1'-bis(diphenylphosphino)ferrocene]dichloropalladium(II) dichloromethane complex (43.3 mg, 59.2 μmol). The system was purged three times with nitrogen, and then stirred at 105°C for 5 h. After the reaction was completed, the reaction mixture was cooled to room temperature, filtered, and saturated aqueous sodium chloride solution (10 mL) was added to the filtrate. The mixture was extracted three times with ethyl acetate (10 mL). The organic phases were combined, dried, and concentrated to obtain a crude product. The crude product was purified by normal-phase silica gel column chromatography (EtOAc/PE = 0 to 35%) to obtain 380 mg of the title compound **21-2** in 63% yield. LC-MS (ESI): 506.2 $[M+H]^+$.

**Preparation of compound 21-3**

Compound **21-2** (300 mg, 592 μmol) and cesium carbonate (385 mg, 1.18 μmol) were added to MeOH (4 mL), and the mixture was stirred at 25°C for 4 h. After the reaction was completed, the reaction mixture was filtered. Saturated sodium chloride solution (10 mL) was added to the filtrate, and the mixture was extracted three times with dichloromethane (10 mL). The organic phases were combined, dried, and concentrated. The residue was purified by normal-phase silica gel column chromatography (EtOAc/PE = 0 to 25%) to obtain 280 mg of the title compound **21-3** in 65% yield. LC-MS (ESI): 434.1 $[M+H]^+$.

**Preparation of compound 21-4**

Compound **21-3** (250 mg, 575.6 μmol), 2-chloro-4-bromopyrimidine (134 mg, 690.7 μmol), bis(triphenylphosphine)palladium(II) chloride (20.2 mg, 28.8 μmol), copper(I) iodide (5.5 mg, 28.8 μmol), and N,N-diisopropylethylamine (149 mg, 1.15 mmol) were added to acetonitrile (3 mL). The reaction mixture was stirred at 80°C for 4 h. After the reaction was completed, the reaction system was quenched with saturated aqueous sodium chloride solution (15 mL) and extracted three times with ethyl acetate (15 mL). The organic phases were combined, dried, and concentrated to obtain a crude product. The crude product was purified by normal-phase silica gel column chromatography (EtOAc/PE = 0 to 30%) to obtain 150 mg of the title compound **21-4** in 47% yield. LC-MS (ESI): 546.1 $[M+H]^+$.

**Preparation of compound 21**

Compound **21-4** (80 mg, 146.29 μmol) was dissolved in N,N-dimethylformamide (1 mL), followed by the sequential addition of 1-iminotetrahydrothiophene 1-oxide (26.15 mg, 219.43 umol), *N,N*-diisopropylethylamine

(37.81 mg, 292.57 μmol), tris(dibenzylideneacetone)dipalladium (5.35 mg, 7.31 μmol), and 4,5-bis(diphenylphosphino)-9,9-dimethylxanthene (4.23 mg, 7.31 μmol). The reaction system was purged three times with nitrogen and stirred under microwave irradiation at 120°C for 2 h. After the reaction was completed, the reaction system was filtered, water (15 mL) was added, and the mixture was extracted three times with dichloromethane (20 mL). The organic phases were combined, dried, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 45% acetonitrile gradient elution over 12 min; flow rate: 30 mL/min) to obtain 26 mg of the title compound **21.** LC-MS (ESI): m/z 629.2 $[M+H]^+$. $^1$H NMR (400 MHz, DMSO-$d_6$) δ 8.69 (br.s, 2H), 7.75 (d, *J* = 8.5 Hz, 2H), 7.72 (d, *J* = 2.3 Hz, 1H), 7.68 (d, *J* = 8.5 Hz, 2H), 7.66 (d, *J* = 2.4 Hz, 1H), 7.63 (d, *J* = 8.4 Hz, 2H), 7.37 (d, *J* = 8.5 Hz, 2H), 4.43 (t, *J* = 5.1 Hz, 2H), 3.97 (t, *J* = 5.1 Hz, 2H), 3.68 - 3.61 (m, 2H), 3.46 - 3.39 (m, 2H), 2.26 - 2.21 (m, 2H), 2.16 - 2.08 (m, 2H), 1.70 (s, 6H).

**Example 22: Preparation of compound 22**

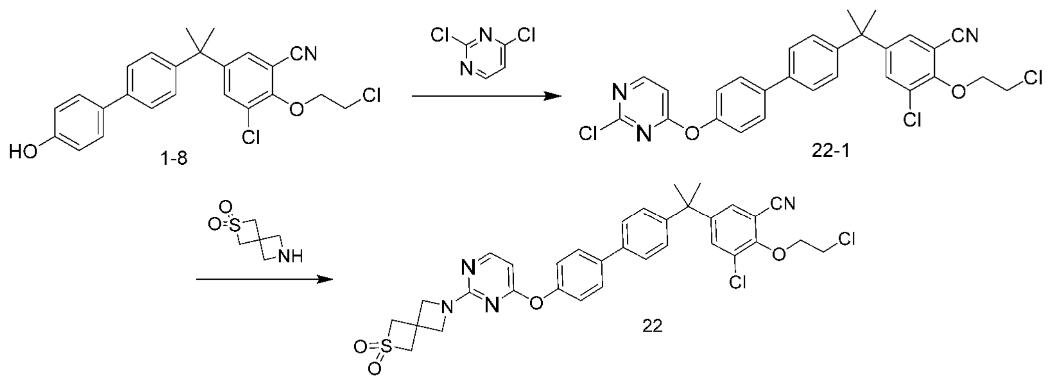
1-8
22-1
22

**Preparation of compound 22-1**

Compound **1-8** (100 mg, 0.23 mmol) was dissolved in DMF (4 mL), followed by the sequential addition of 2,4-dichloropyrimidine (42 mg, 0.28 mmol) and cesium carbonate (230 mg, 0.7 mmol). The reaction system was stirred at room temperature for 16 h. After the reaction was completed, water (10 mL) was added to the reaction mixture, and the mixture was extracted twice with ethyl acetate (30 mL). The organic phases were combined, washed once with saturated brine (3 mL), dried over anhydrous sodium sulfate, and concentrated to obtain 100 mg of the crude product of title compound **3.** LC-MS (ESI): m/z 538.2 $[M+H]^+$.

**Preparation of compound 22**

Compound **22-1** (50 mg, 0.09 mmol) was dissolved in DMF (5.0 mL), followed by the sequential addition of 2-thia-6-azaspiro[3.3]heptane 2,2-dioxide (21 mg, 0.11 mmol) and cesium carbonate (91 mg, 0.28 mmol). The reaction system was stirred at room temperature for 16 h. After the reaction was completed, the reaction mixture was quenched by adding water (10 mL), and the mixture was extracted twice with ethyl acetate (30 mL). The organic phases were combined, washed once with saturated brine (5 mL), dried over anhydrous sodium sulfate, filtered, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 3.4 mg of the title compound **22.** LC-MS (ESI): m/z 649.3 $[M+H]^+$. $^1$H NMR (400 MHz, DMSO-$d_6$) δ 8.26 (d, *J* = 5.6 Hz, 1H), 7.72 (d, *J* = 8.8 Hz, 2H), 7.71 (m, 1H), 7.64 (d, *J* = 8.8 Hz, 2H), 7.63 (m, 1H), 7.35 (d, *J* = 8.5 Hz, 2H), 7.27 (d, *J* = 8.5 Hz, 2H), 6.24 (d, *J* = 5.6 Hz, 1H), 4.45 (s, 4H), 4.43 (t, *J* = 5.6 Hz, 2H), 4.16 (s, 4H), 3.96 (t, *J* = 5.6 Hz, 2H), 1.70 (s, 6H).

**Example 23: Preparation of compound 23**

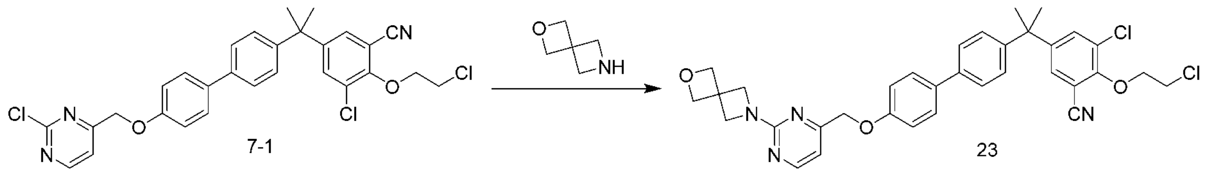

7-1

23

**Preparation of compound 23**

Compound **7-1** (100 mg, 0.19 mmol) was dissolved in acetonitrile (2 mL), followed by the sequential addition of 2-oxa-6-azaspiro[3,3]heptane (31 mg, 0.19 mmol) and DIEA (50 mg, 0.38 mmol). The reaction system was stirred at 85°C for 12 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 12.8 mg of the title compound **23.** LC-MS (ESI): m/z 615.3 $[M+H]^+$. $^1H$ NMR (400 MHz, DMSO-$d_6$) δ 8.35 (d, *J* = 4.8 Hz, 1H), 7.69 (d, *J* = 2.4 Hz, 1H), 7.63 (d, *J* = 2.4 Hz, 1H), 7.59 (d, *J* = 8.8 Hz, 2H), 7.55 (d, *J* = 8.2 Hz, 2H), 7.30 (d, *J* = 8.2 Hz, 2H), 7.06 (d, *J* = 8.8 Hz, 2H), 6.77 (d, *J* = 4.8 Hz, 1H), 5.04 (s, 2H), 4.72 (s, 4H), 4.42 (t, *J* = 5.1 Hz, 2H), 4.21 (s, 4H), 3.95 (t, *J* = 5.1 Hz, 2H), 1.68 (s, 6H).

**Example 24: Preparation of compound 24**

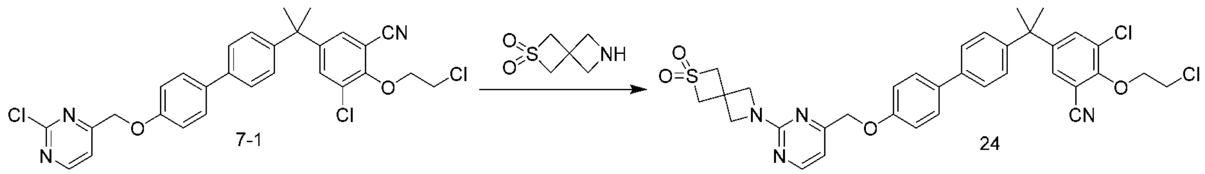

7-1

24

**Preparation of compound 24**

Compound **7-1** (100 mg, 0.19 mmol) was dissolved in acetonitrile (2 mL), followed by the sequential addition of 2-thia-6-azaspiro[3.3]heptane 2,2-dioxide (28 mg, 0.19 mmol) and DIEA (50 mg, 0.38 mmol). The reaction system was stirred at 85°C for 12 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 17.6 mg of the title compound 24. LC-MS (ESI): m/z 663.2 $[M+H]^+$. $^1H$ NMR (400 MHz, DMSO-$d_6$) 8.38 (d, *J* = 5.0 Hz, 1H), 7.69 (s, 1H), 7.63 (s, 1H), 7.59 (d, *J* = 8.4 Hz, 2H), 7.55 (d, *J* = 8.0 Hz, 2H), 7.30 (d, *J* = 8.0 Hz, 2H), 7.07 (d, *J* = 8.4 Hz, 2H), 6.82 (d, *J* = 5.0 Hz, 1H), 5.06 (s, 2H), 4.51 (s, 4H), 4.42 (t, *J* = 5.2 Hz, 2H), 4.28 (s, 4H), 3.95 (t, *J* = 5.2 Hz, 2H), 1.68 (s, 6H).

**Example 25: Preparation of compound 25**

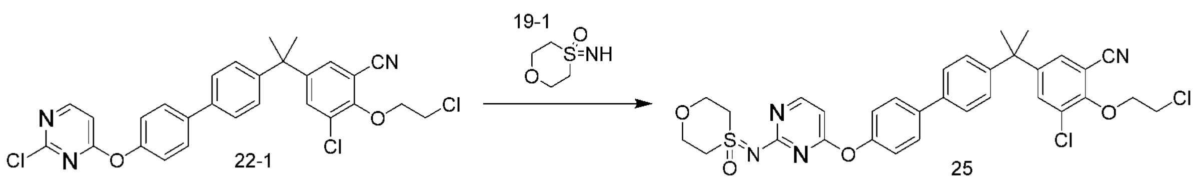


22-1

25

**Preparation of compound 25**

Compound **22-1** (60 mg, 0.11 mmol) was dissolved in 1,4-dioxane (6 mL), followed by the addition of **19-1** (23 mg, 0.16 mmol), $Pd_2(dba)_3$ (11 mg, 0.01 mmol), X-phos (13 mg, 0.02 mmol), and DIEA (72 mg, 0.56 mmol). The system was purged three times with nitrogen and stirred at 120°C overnight. After the reaction was completed, the reaction mixture was filtered through diatomite, and the filter cake was rinsed with an appropriate amount of ethyl acetate. Water (20 mL) was added to the filtrate, and the mixture was extracted twice with ethyl acetate (50 mL). The organic phases were combined, washed once with saturated brine (20 mL), dried over anhydrous sodium sulfate, and concentrated to obtain a crude product. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15.0 mg of the title compound **25.** LC-MS (ESI): m/z 637.2 $[M+H]^+$. $^1H$ NMR (400 MHz, DMSO-$d_6$) δ 8.34 (d, *J* = 5.6 Hz, 1H), 7.73 (d, *J* = 2.4 Hz, 1H), 7.72 (d, *J* = 8.4 Hz, 2H), 7.67 (d, *J* = 2.3 Hz, 1H), 7.63 (d, *J* = 8.4 Hz, 2H), 7.36 (d, *J* = 8.4 Hz, 2H), 7.26 (d, *J* = 8.6 Hz, 2H), 6.59 (d, *J* = 5.6 Hz, 1H), 4.43 (t, *J* = 5.2 Hz, 2H), 3.96 (t, *J* = 5.2 Hz, 2H), 3.83 - 3.89 (m, 4H), 3.58 - 3.64 (m, 4H), 1.70 (s, 6H).

**Example 26: Preparation of compound 26**

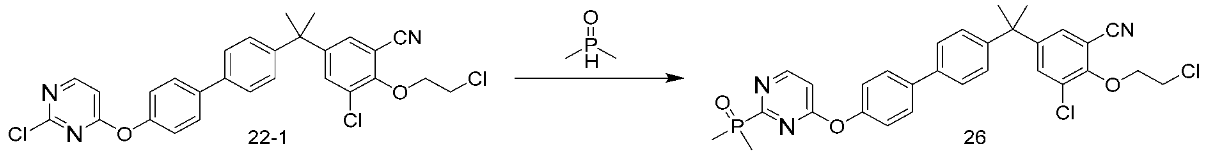

22-1

26

**Preparation of compound 26**

Compound **22-1** (110 mg, 0.20 mmol) was dissolved in 1,4-dioxane (6 mL), and dimethylphosphine oxide (26 mg, 0.31 mmol), $Pd_2(dba)_3$ (19 mg, 0.02 mmol), X-phos (23 mg, 0.04 mmol), and DIEA (122 mg, 0.94 mmol) were added. The system was purged three times with nitrogen and stirred at 120°C overnight. After the reaction was completed, the reaction mixture was filtered through diatomite, and the filter cake was dried with an appropriate amount of ethyl acetate. Water (30 mL) was added to the filtrate, and the mixture was extracted three times with ethyl acetate (60 mL). The organic phases were combined, washed twice with saturated brine (50 mL), dried over anhydrous sodium sulfate, and concentrated to obtain a crude product. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 6.5 mg of the title compound 8. LC-MS (ESI): m/z 580.2 $[M+H]^+$. $^1H$ NMR (400 MHz, DMSO-$d_6$) δ 8.86 (d, *J* = 6.0 Hz, 1H), 7.79 (d, *J* = 8.8 Hz, 2H), 7.73 (d, *J* = 2.0, 1H), 7.64 - 7.68 (m, 3H), 7.36 - 7.39 (m, 4H), 7.24 (dd, *J* = 5.6, 2.4 Hz, 1H), 4.44 (t, *J* = 5.6 Hz, 2H), 3.97 (t, *J* = 5.6 Hz, 2H), 1.71 (s, 6H), 1.65 (d, *J* = 14.0 Hz, 6H).

**Example 27: Preparation of compound 27**

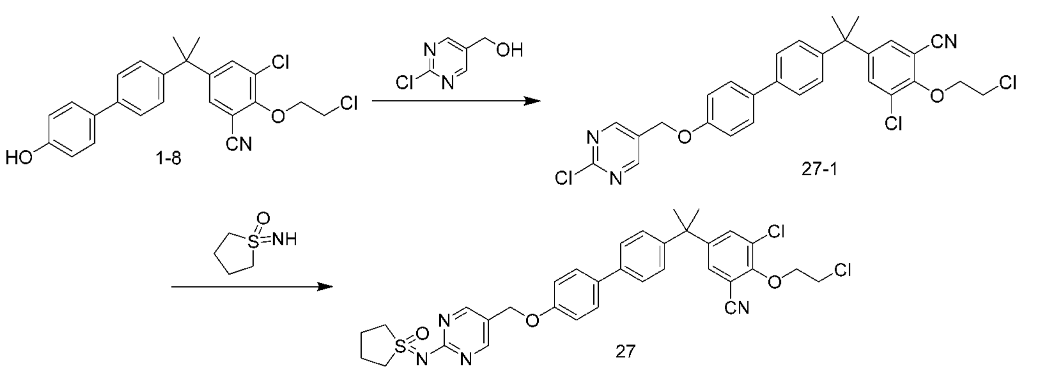

1-8

27-1

27

**Preparation of compound 27-1**

Compound **1-8** (250 mg, 0.59 mmol) was added to a three-necked flask, followed by the sequential addition of anhydrous DCM (10 mL), (2-chloropyrimidin-5-yl)methanol (110 mg, 0.76 mmol), and *N,N,N',N'*-tetramethylazodicarboxamide (202 mg, 1.17 mmol). The system was purged three times with nitrogen, and tributylphosphine (237 mg, 1.17 mmol) was added at 0°C. The reaction was stirred at 25°C for 3 h. After the reaction was completed, water (20 mL) was added, and the mixture was extracted twice with DCM (20 mL). The organic phases were combined, dried, and concentrated to obtain a crude product. The crude product was purified by normal-phase silica gel column chromatography (EtOAc/PE = 0 to 30%) to obtain 150 mg of the title compound **27-1** in 46% yield. LC-MS (ESI): 552.1 $[M+H]^+$.

**Preparation of compound 27**

Compound **27-1** (100 mg, 0.18 mmol) was dissolved in DMF (2 mL), followed by the sequential addition of tris(dibenzylideneacetone)dipalladium (17 mg, 0.02 mmol), 4,5-bis(diphenylphosphino)-9,9-dimethylxanthene (21 mg, 0.04), DIEA (70 mg, 0.54 mmol), and 1-imino-1-oxothiolane (65 mg, 0.54 mmol). The reaction system was stirred at 120°C for 3 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 45 mg of the title compound **5.** LC-MS (ESI): m/z 635.3 $[M+H]^+$. $^1$H NMR (400 MHz, DMSO-$d_6$) δ 8.59 (s, 2H), 7.71 (d, *J* = 2.4 Hz, 1H), 7.65 (d, *J* = 2.4 Hz, 1H), 7.63 - 7.54 (m, 4H), 7.33 - 7.28 (m, 2H), 7.13 - 7.08 (m, 2H), 5.06 (s, 2H), 4.43 (t, *J* = 5.6 Hz, 2H), 3.97 (t, *J* = 5.6 Hz, 2H), 3.65 - 3.54 (m, 2H), 3.43 - 3.34 (m, 2H), 2.28 - 2.03 (m, 4H), 1.69 (s, 6H).

**Example 28: Preparation of compound 28**

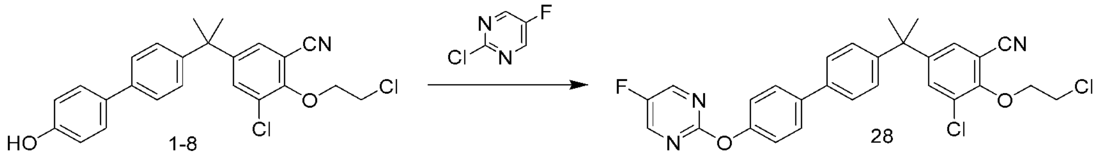

**Preparation of compound 28**

Compound **1-8** (200 mg, 0.47 mmol) was dissolved in DMF (5 mL), followed by the sequential addition of 2-chloro-5-fluoropyrimidine (62 mg, 0.47 mmol) and potassium carbonate (138 mg, 0.94 mmol). The reaction system was stirred at 50°C for 16 h. After the reaction was completed, insoluble solids were removed by filtration. Water (30 mL) was added to the filtrate, and the mixture was extracted twice with ethyl acetate (30 mL). The organic phases were combined, washed twice with saturated brine (20 mL), dried over anhydrous sodium sulfate, and concentrated to obtain a crude product. The crude product was purified by column chromatography (petroleum ether: ethyl acetate = 5:1) to obtain a crude product. The crude product was further subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 94 mg of the title compound **28.** LC-MS (ESI): m/z 522.4 $[M+H]^+$. $^1$H NMR (400 MHz, DMSO-$d_6$) δ 8.76 (s, 2H), 7.69 - 7.73 (m, 3H), 7.66 (d, J = 2.4 Hz, 1H), 7.62 - 7.64 (m, 2H), 7.34 - 7.36 (m, 2H), 7.27 -7.30 (m, 2H), 4.43 (m, *J* = 5.6 Hz, 2H), 3.96 (t, *J* = 5.6 Hz, 2H), 1.73 (s, 6H). $^{19}$F NMR (376 MHz, DMSO-$d_6$) δ -147.83.

**Example 29: Preparation of compound 29**

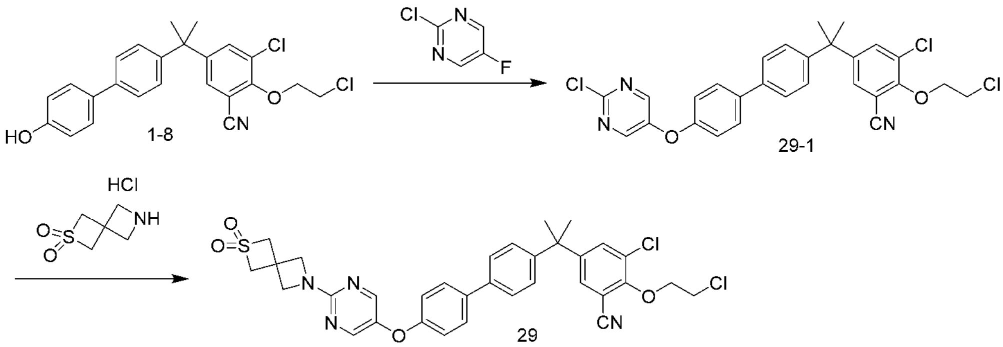


1-8 29-1 29

### Preparation of compound 29-1

Compound **1-8** (200 mg, 0.47 mmol) was dissolved in DMF (5 mL), followed by the sequential addition of 2-chloro-5-fluoropyrimidine (62 mg, 0.47 mmol) and potassium carbonate (138 mg, 0.94 mmol). The reaction system was stirred at 50°C for 16 h. After the reaction was completed, insoluble solids were removed by filtration. Water (30 mL) was added to the filtrate, and the mixture was extracted three times with ethyl acetate (30 mL). The organic phases were combined, washed twice with saturated brine (20 mL), dried over anhydrous sodium sulfate, and concentrated to obtain a crude product. The crude product was purified by column chromatography (petroleum ether: ethyl acetate = 5:1) to obtain 60 mg of the title compound **29-1.**

### Preparation of compound 29

Compound **29-1** (30 mg, 0.06 mmol) was dissolved in DMF (0.5 mL), followed by the sequential addition of 2-thia-6-azaspiro[3.3]heptane 2,2-dioxide hydrochloride (12 mg, 0.07 mmol) and potassium carbonate (54 mg, 0.17 mmol). The reaction system was stirred at 60°C for 16 h. After the reaction was completed, water (5 mL) was added to the reaction mixture to quench the reaction. The mixture was extracted three times with ethyl acetate (5 mL). The organic phases were combined, washed once with saturated brine (5 mL), dried over anhydrous sodium sulfate, and concentrated. The crude product was purified by column chromatography (petroleum ether: ethyl acetate = 5:1), followed by preparative separation (preparative method: mobile phase: A: 0.1% formic acid in water; B: acetonitrile; chromatographic column: Welch Ultimate AQ-C 18 250 × 21.2 mm; column temperature: 25°C; gradient: 45% to 65% acetonitrile gradient elution over 12 min; flow rate: 30 mL/min) to obtain 20 mg of the title compound **29.** LC-MS (ESI): m/z 649.2 $[M+H]^+$. $^1H$ NMR (400 MHz, DMSO-$d_6$) δ 8.38 (s, 2H), 7.71 (d, *J* = 2.4 Hz, 1H), 7.62 - 7.66 (m, 3H), 7.55 - 7.58 (m, 2H), 7.31 - 7.33 (m, 2H), 7.00 - 7.04 (m, 2H), 4.53 (s, 4H), 4.43 (t, *J* = 5.2 Hz, 2H), 4.29 (s, 4H), 3.96 (t, *J* = 5.2 Hz, 2H), 1.69 (s, 6H).

### Example 30: Preparation of compound 30

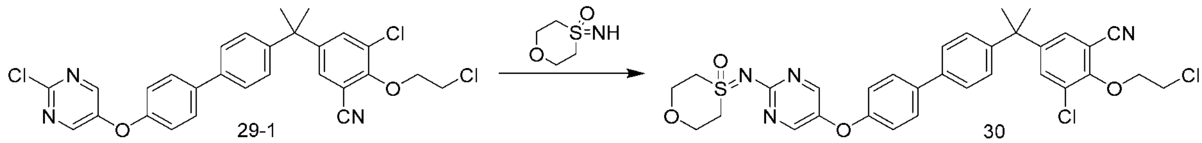


29-1 30

### Preparation of compound 30

Compound **29-1** (20 mg, 0.04 mmol) was dissolved in toluene (1 mL), followed by the sequential addition of **19-1** (10 mg, 0.07 mmol), palladium acetate (1 mg, 0.004 mmol), cesium carbonate (24 mg, 0.07 mmol), and 4,5-bis(diphenylphosphino)-9,9-dimethylxanthene (4 mg, 0.007 mmol). The reaction mixture was purged three times with nitrogen and then stirred at 105°C for 16 h. After the reaction was completed, insoluble solids were removed by filtration. Water (10 mL) was added to the filtrate, and the mixture was extracted three times with ethyl acetate (10 mL). The organic phases were combined, washed twice with saturated brine (10 mL), dried over anhydrous sodium sulfate, and concentrated to obtain a crude product. The crude product was purified by column chromatography (petroleum ether: ethyl acetate = 1:1) to obtain a crude product. The crude product was further subjected to preparative purification (preparative method: mobile phase: A: 0.1% formic acid in water; B: acetonitrile; chromatographic column: Welch Ultimate AQ-C18 250 × 21.2 mm; column

temperature: 25°C; gradient: 45% to 65% acetonitrile gradient elution over 12 min; flow rate: 30 mL/min) to obtain 94 mg of the title compound **30.** LC-MS (ESI): m/z 637.04 $[M+H]^+$. $^1$H NMR (400 MHz, DMSO-$d_6$) δ 8.41 (s, 2H), 7.72 (d, *J* = 2.4 Hz, 1H), 7.63 - 7.68 (m, 3H), 7.57 - 7.59 (m, 2H), 7.32 - 7.34 (m, 2H), 7.07 - 7.11 (m, 2H), 4.43 (t, *J* = 5.2 Hz, 2H), 4.08 - 4.14 (m, 2H), 3.94 - 4.00 (m, 4H), 3.77 - 3.83 (m 2H), 3.56 - 3.62 (m, 2H), 1.69 (s, 6H).

**Example 31: Preparation of compound 31**

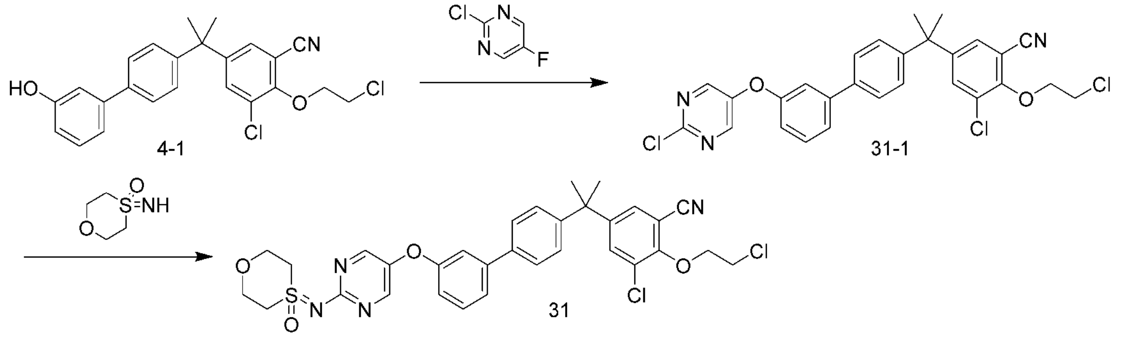
4-1
31-1
31

**Preparation of compound 31-1**

Compound **4-1** (300 mg, 0.70 mmol) was dissolved in DMF (3 mL), followed by the sequential addition of 2-chloro-5-fluoropyrimidine (111 mg, 0.84 mmol) and potassium carbonate (194 mg, 1.41 mmol). The reaction system was stirred at 50°C for 16 h. After the reaction was completed, insoluble solids were removed by filtration. Water (30 mL) was added to the filtrate, and the mixture was extracted twice with ethyl acetate (30 mL). The organic phases were combined, washed once with saturated brine (20 mL), dried over anhydrous sodium sulfate, and concentrated to obtain a crude product. The crude product was purified by column chromatography (petroleum ether: ethyl acetate = 8:1) to obtain 80 mg of the title compound 31-1. LC-MS (ESI): m/z 538.2 $[M+H]^+$.

**Preparation of compound 31**

Compound **31-1** (30 mg, 0.06 mmol) was dissolved in toluene (1 mL), followed by the sequential addition of **19-1** (15 mg, 0.11 mmol), palladium acetate (1 mg, 0.006 mmol), cesium carbonate (36 mg, 0.11 mmol), and 4,5-bis(diphenylphosphino)-9,9-dimethylxanthene (6 mg, 0.01 mmol). The reaction mixture was purged three times with nitrogen and then stirred at 105°C for 16 h. After the reaction was completed, insoluble solids were removed by filtration. Water (10 mL) was added to the filtrate, and the mixture was extracted three times with ethyl acetate (10 mL). The organic phases were combined, washed twice with saturated brine (10 mL), dried over anhydrous sodium sulfate, and concentrated to obtain a crude product. The crude product was purified by column chromatography (dichloromethane: methanol = 10:1) to obtain a crude product. The crude product was further subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 12 mg of the title compound 31. LC-MS (ESI): m/z 637.2 $[M+H]^+$. $^1$H NMR (400 MHz, DMSO-$d_6$) δ 8.41 (s, 2H), 7.70 (d, *J* = 2.0 Hz, 1H), 7.64 (d, *J* = 2.0 Hz, 1H), 7.59 - 7.61 (m, 2H), 7.40 - 7.47 (m, 2H), 7.29 - 7.34 (m, 3H), 6.96 - 6.99 (m, 1H), 4.42 (t, *J* = 5.2 Hz, 2H), 4.06 - 4.12 (m, 2H), 3.92 - 3.98 (m, 4H), 3.77 - 3.82 (m, 2H), 3.55 - 3.61 (m, 2H), 1.68 (s, 6H).

**Example 32: Preparation of compound 32**

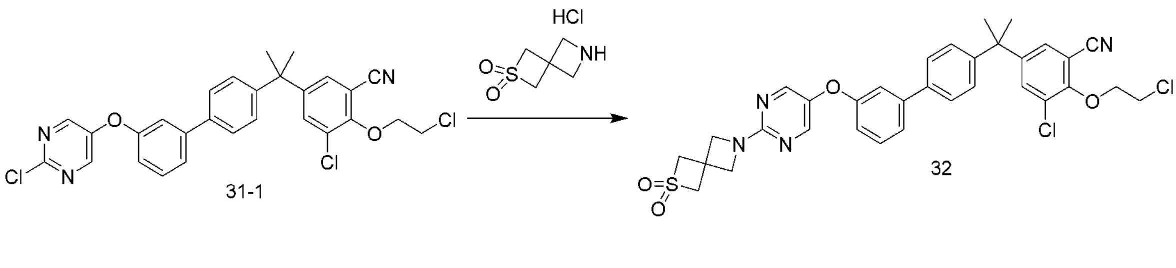

31-1
32

**Preparation of compound 32**

Compound **31-1** (20 mg, 0.04 mmol) was dissolved in DMF (0.5 mL), followed by the sequential addition of 2-thia-6-azaspiro[3.3]heptane 2,2-dioxide hydrochloride (10 mg, 0.06 mmol) and cesium carbonate (45 mg, 0.14 mmol). The reaction mixture was purged three times with nitrogen and then stirred at 60°C for 16 h. After the reaction was completed, insoluble solids were removed by filtration. Water (10 mL) was added to the filtrate, and the mixture was extracted twice with ethyl acetate (10 mL). The organic phases were combined, washed once with saturated brine (10 mL), dried over anhydrous sodium sulfate, and concentrated to obtain a crude product. The crude product was purified by column chromatography (petroleum ether: ethyl acetate = 2:1) to obtain a crude product. The crude product was further subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 3 mg of the title compound **32.** LC-MS (ESI): m/z 649.2 $[M+H]^+$. $^1$H NMR (400 MHz, DMSO-$d_6$) δ 8.38 (s, 2H), 7.71 (d, *J* = 2.4 Hz, 1H), 7.64 (d, *J* = 2.4 Hz, 1H), 7.56 - 7.58 (m, 2H), 7.35 - 7.44 (m, 2H), 7.31 - 7.34 (m, 2H), 7.20 (t, *J* = 2.0 Hz, 1H), 6.89 - 6.92 (m, 1H), 4.52 (s, 4H), 4.42 (t, *J* = 5.2 Hz, 2H), 4.28 (s, 4H), 3.96 (t, *J* = 5.2 Hz, 2H), 1.68 (s, 6H).

**Example 33: Preparation of compound 33**

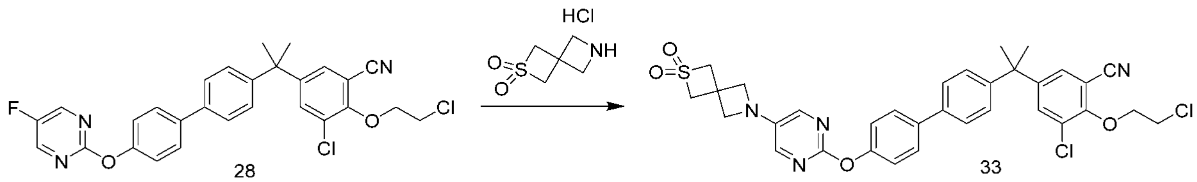


**Preparation of compound 33**

Compound **28** (20 mg, 0.04 mmol) was dissolved in DMF (0.5 mL), followed by the sequential addition of 2-thia-6-azaspiro[3.3]heptane 2,2-dioxide hydrochloride (8 mg, 0.05 mmol) and cesium carbonate (25 mg, 0.08 mmol). The reaction mixture was stirred at 90°C for 16 h. After the reaction was completed, the reaction mixture was cooled to room temperature, and insoluble solids were removed by filtration. Water (10 mL) was added to the filtrate, and the mixture was extracted three times with ethyl acetate (10 mL). The organic phases were combined, washed twice with saturated brine (10 mL), dried over anhydrous sodium sulfate, and concentrated to obtain a crude product. The crude product was purified by column chromatography (petroleum ether: ethyl acetate = 2:1) to obtain a crude product. The crude product was further subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 3 mg of the title compound **33.** $^1$H NMR (400 MHz, DMSO-$d_6$) δ 8.76 (s, 2H), 7.73 (d, *J* = 2.3 Hz, 1H), 7.71 (d, *J* = 8.6 Hz, 2H), 7.66 (d, *J* = 2.3 Hz, 1H), 7.63 (d, *J* = 8.4 Hz, 2H), 7.35 (d, *J* = 8.4 Hz, 2H), 7.29 (d, *J* = 8.6 Hz, 2H), 4.42 (s, 4H), 4.34 (t, *J* = 5.1 Hz, 2H), 4.30 (t, *J* = 5.1 Hz, 2H), 4.11 (s, 4H), 1.70 (s, 6H).

**Example 34: Preparation of compound 34**

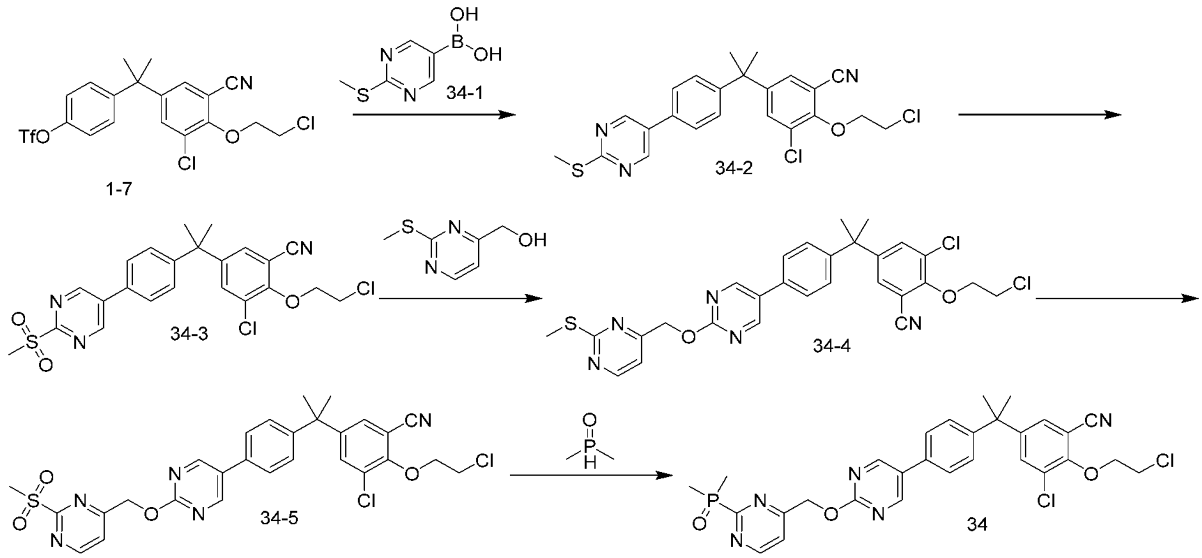


**Preparation of compound 34-2**

Compound **1-7** (300 mg, 0.62 mmol) was dissolved in 1,4-dioxane (20 mL) and water (5 mL), followed by the sequential addition of compound **34-1** (170 mg, 0.75 mmol), potassium carbonate (258 mg, 1.87 mmol), and Pd(dppf)$Cl_2$ (45 mg, 0.06 mmol). The reaction system was purged three times with nitrogen and stirred at 100°C for 4 h. After the reaction was completed, water (10 mL) was added, and the mixture was extracted three times with ethyl acetate (20 mL). The organic phases were combined, washed once with saturated brine (20 mL), dried over anhydrous sodium sulfate, and concentrated to obtain a crude product. The crude product was purified by column chromatography (PE:EA = 30%) to obtain 290 mg of the title compound **34-2.** LC-MS (ESI): m/z 458.2 $[M+H]^+$.

**Preparation of compound 34-3**

Compound **34-2** (100 mg, 0.22 mmol) was dissolved in dichloromethane (20 mL), and m-chloroperoxybenzoic acid (112 mg, 0.66 mmol) was added thereto at 0°C. The reaction system was stirred at 25°C for 1 h. After the reaction was completed, water (10 mL) was added, and the mixture was extracted three times with dichloromethane (20 mL). The organic phases were combined, washed three times with saturated sodium carbonate solution (20 mL), dried over anhydrous sodium sulfate, and concentrated to obtain a crude product of the title compound **34-3,** which was used directly in the next step without purification. LC-MS (ESI): m/z 490.0 $[M+H]^+$.

**Preparation of compound 34-4**

Compound **34-3** (90 mg, 0.18 mmol) was dissolved in acetonitrile (20 mL), followed by the sequential addition of compound (2-(methylthio)pyrimidin-4-yl)methanol (34 mg, 0.22 mmol) and potassium carbonate (75 mg, 0.54 mmol). The reaction system was stirred at 70°C for 1 h. After the reaction was completed, water (10 mL) was added, and the mixture was extracted three times with ethyl acetate (20 mL). The organic phases were combined, washed twice with saturated brine (20 mL), dried over anhydrous sodium sulfate, and concentrated to obtain a crude product. The crude product was purified by column chromatography (PE:EA = 50%) to obtain 100 mg of the title compound **34-4.** LC-MS (ESI): m/z 566.2 $[M+H]^+$.

**Preparation of compound 34-5**

Compound **34-4** (100 mg, 0.18 mmol) was dissolved in dichloromethane (20 mL), and m-chloroperoxybenzoic acid (91 mg, 0.54 mmol) was added thereto at 0°C. The reaction system was stirred at 25°C for 1 h. After the reaction was completed, water (10 mL) was added, and the mixture was extracted three times with dichloromethane (20 mL). The organic phases were combined, washed three times with saturated sodium carbonate solution (20 mL), dried over anhydrous sodium sulfate, and concentrated to obtain a crude product of the title compound **34-5,** which was used directly in the next step without purification. LC-MS (ESI): m/z 598.2 $[M+H]^+$.

**Preparation of compound 34**

Compound **34-5** (100 mg, 0.17 mmol) was dissolved in acetonitrile (2.0 mL), followed by the sequential addition of dimethylphosphine oxide (39 mg, 0.51 mmol) and potassium carbonate (69 mg, 0.51 mmol). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 7.6 mg of the title compound **34.** LC-MS (ESI): m/z 596.4 $[M+H]^+$. $^1$H NMR (400 MHz, DMSO-$d_6$) δ 8.96 - 8.94 (m, 3H), 7.69 (d, *J* = 2.3 Hz, 1H), 7.67 (d, *J* = 8.4 Hz, 2H), 7.64 (d, *J* = 2.3 Hz, 1H), 7.63 - 7.61 (m, 1H), 7.37 (d, *J* = 8.4 Hz, 2H), 5.63 (s, 2H), 4.42 (t, *J* = 5.2 Hz, 2H), 3.95 (t, *J* = 5.2 Hz, 2H), 1.72 (d, *J* = 13.8 Hz, 6H), 1.69 (s, 6H). $^{31}$P NMR (162 MHz, Chloroform-d) δ 34.23 (s, 1P).

**Example 35: Preparation of compound 35**

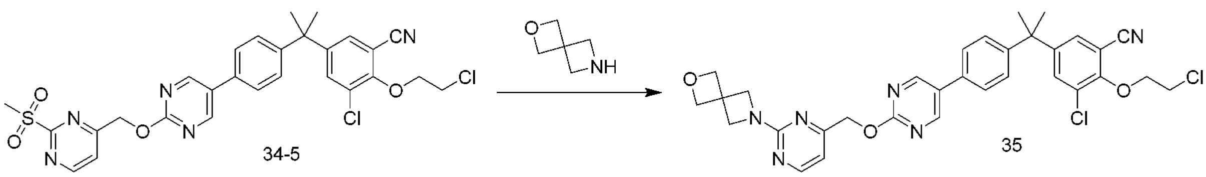

**Preparation of compound 35**

Compound **34-5** (100 mg, 0.17 mmol) was dissolved in acetonitrile (2.0 mL), followed by the sequential addition of 2-oxa-6-azaspiro[3,3]heptane (50 mg, 0.51 mmol) and potassium carbonate (69 mg, 0.51 mmol). The reaction system was stirred at 50°C for 1 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 38.6 mg of the title compound **35.** LC-MS (ESI): m/z 617.2 $[M+H]^+$. $^1$H NMR (400 MHz, Chloroform-d) δ 8.73 (s, 2H), 8.30 (d, *J* = 5.1 Hz, 1H), 7.48 (d, *J* = 2.3 Hz, 1H), 7.47 (d, *J* = 8.4 Hz, 2H), 7.35 (d, *J* = 2.3 Hz, 1H), 7.30 (d, *J* = 8.4 Hz, 2H), 6.78 (d, *J* = 5.1 Hz, 1H), 5.41 (s, 2H), 4.86 (s, 4H), 4.43 (t, *J* = 6.1 Hz, 2H), 4.30 (s, 4H), 3.88 (t, *J* = 6.1 Hz, 2H), 1.70 (s, 6H).

**Example 36: Preparation of compound 36**

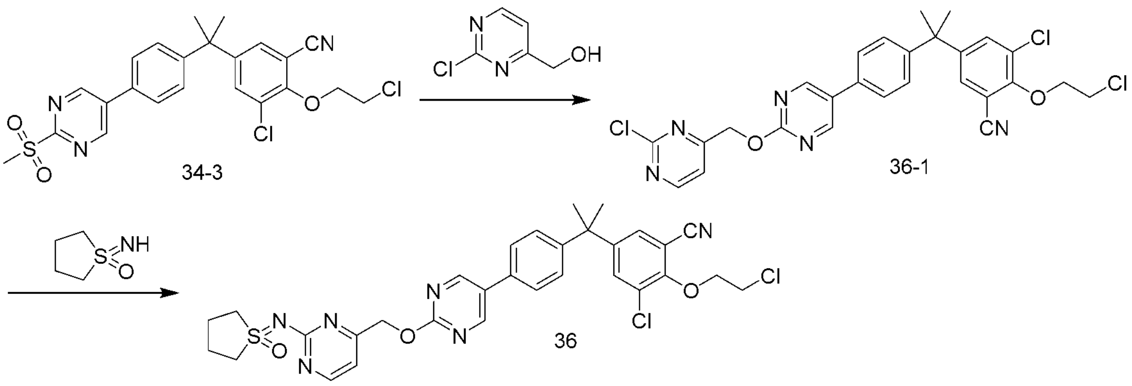

**Preparation of compound 36-1**

Compound **34-3** (290 mg, 0.59 mmol) was dissolved in acetonitrile (20 mL), followed by the sequential addition of (2-chloropyrimidin-4-yl)methanol (102 mg, 0.71 mmol) and potassium carbonate (243 mg, 1.78 mmol). The reaction system was stirred at 70°C for 1 h. After the reaction was completed, water (10 mL) was added, and the mixture was extracted three times with ethyl acetate (20 mL). The organic phases were combined, washed twice with saturated brine (20 mL), dried over anhydrous sodium sulfate, and concentrated to obtain a crude product. The crude product was purified by column chromatography (PE:EA = 50%) to obtain 250 mg of the title compound **36-1.** LC-MS (ESI): m/z 554.2 $[M+H]^+$.

**Preparation of compound 36**

Compound **36-1** (100 mg, 0.18 mmol) was dissolved in DMF (2 mL), followed by the sequential addition of tris(dibenzylideneacetone)dipalladium (42 mg, 0.05 mmol), 4,5-bis(diphenylphosphino)-9,9-dimethylxanthene (27 mg, 0.05 mmol), DIEA (68 mg, 0.54 mmol), and 1-imino-1-oxothiolane (43 mg, 0.36 mmol). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 36.3 mg of the title compound **36.** LC-MS (ESI): m/z 637.2 $[M+H]^+$. $^1H$ NMR (400 MHz, Chloroform-*d*) δ 8.73 (s, 2H), 8.46 (d, *J* = 5.4 Hz, 1H), 7.48 (d, *J* = 2.3 Hz, 1H), 7.47 (d, *J* = 8.4 Hz, 2H), 7.35 (d, *J* = 2.3 Hz, 1H), 7.30 (d, *J* = 8.4 Hz, 2H), 7.05 (d, *J* = 5.4 Hz, 1H), 5.50 (s, 2H), 4.44 (t, *J* = 6.1 Hz, 2H), 3.88 (t, *J* = 6.1 Hz, 2H), 3.74 - 3.67 (m, 2H), 3.44 - 3.38 (m, 2H), 2.40 - 2.24 (m, 4H), 1.70 (s, 6H).

**Example 37: Preparation of compound 37**

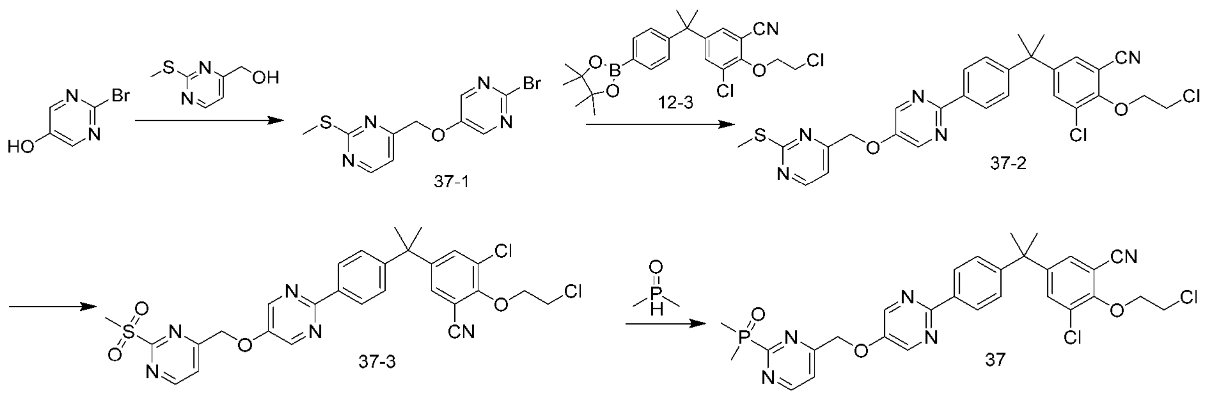


**Preparation of compound 37-1**

Compound 2-bromo-5-hydroxypyrimidine (500 mg, 2.86 mmol) was dissolved in dichloromethane (20 mL), followed by the sequential addition of compound (2-(methylthio)pyrimidin-4-yl)methanol (538 mg, 3.42 mmol), *N,N,N',N'*-tetramethylazodicarboxamide (982 mg, 5.71 mmol), and tri-n-butylphosphine (1722 mg, 8.57 mmol). The reaction system was purged three times with nitrogen and stirred at 25°C for 4 h. After the reaction was completed, water (10 mL) was added, and the mixture was extracted three times with dichloromethane (20 mL). The organic phases were combined, washed twice with saturated brine (20 mL), dried over anhydrous sodium sulfate, and concentrated to obtain a crude product. The crude product was purified by column chromatography (PE:EA = 25%) to obtain 600 mg of the title compound **37-1.** LC-MS (ESI): m/z 313.0 $[M+H]^+$.

**Preparation of compound 37-2**

Compound **37-1** (500 mg, 1.60 mmol) was dissolved in 1,4-dioxane (20 mL) and water (5 mL), followed by the sequential addition of compound **12-3** (734 mg, 1.60 mmol), potassium carbonate (657 mg, 4.80 mmol), and $Pd(dppf)Cl_2$

(117 mg, 0.16 mmol). The reaction system was purged three times with nitrogen and stirred at 100°C for 4 h. After the reaction was completed, water (10 mL) was added, and the mixture was extracted three times with ethyl acetate (20 mL). The organic phases were combined, washed twice with saturated brine (20 mL), dried over anhydrous sodium sulfate, and concentrated to obtain a crude product. The crude product was purified by column chromatography (PE:EA = 30%) to obtain 400 mg of the title compound **37-2.** LC-MS (ESI): m/z 566.4$[M+H]^+$.

**Preparation of compound 37-3**

Compound **37-2** (100 mg, 0.18 mmol) was dissolved in dichloromethane (20 mL), and m-chloroperoxybenzoic acid (91 mg, 0.54 mmol) was added thereto at 0°C. The reaction system was stirred at 25°C for 1 h. After the reaction was completed, water (10 mL) was added, and the mixture was extracted three times with dichloromethane (20 mL). The organic phases were combined, washed three times with saturated sodium carbonate solution (20 mL), dried over anhydrous sodium sulfate, and concentrated to obtain a crude product of the title compound **37-3,** which was used directly in the next step without purification. LC-MS (ESI): m/z 598.4 $[M+H]^+$.

**Preparation of compound 37**

Compound **37-3** (100 mg, 0.17 mmol) was dissolved in acetonitrile (2.0 mL), followed by the sequential addition of dimethylphosphine oxide (39 mg, 0.51 mmol) and potassium carbonate (69 mg, 0.51 mmol). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 12 mg of the title compound **37.** LC-MS (ESI): m/z 596.2 $[M+H]^+$. $^1H$ NMR (400 MHz, Chloroform-d) δ 8.95 (s, 1H), 8.56 (s, 2H), 8.30 (d, *J* = 8.4 Hz, 2H), 7.71 (s, 1H), 7.45 (d, *J* = 2.4 Hz, 1H), 7.38 (d, *J* = 2.4 Hz, 1H), 7.30 (d, *J* = 8.4 Hz, 1H), 5.38 (s, 2H), 4.42 (t, *J* = 6.2 Hz, 2H), 3.88 (t, *J* = 6.2 Hz, 2H), 1.91 (d, *J* = 13.4 Hz, 6H), 1.71 (s, 6H). $^{31}P$ NMR (162 MHz, Chloroform-d) δ 34.92 (s, 1P).

**Example 38: Preparation of compound 38**

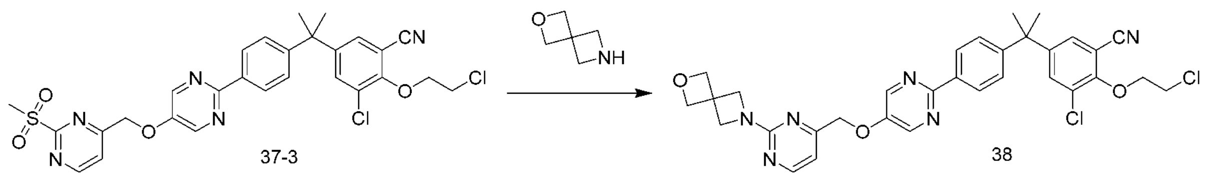

**Preparation of compound 38**

Compound **37-3** (100 mg, 0.17 mmol) was dissolved in acetonitrile (2.0 mL), followed by the sequential addition of 2-oxa-6-azaspiro[3,3]heptane (50 mg, 0.51 mmol) and potassium carbonate (69 mg, 0.51 mmol). The reaction system was stirred at 50°C for 1 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 32 mg of the title compound **38.** LC-MS (ESI): m/z 617.3 $[M+H]^+$. $^1H$ NMR (400 MHz, Chloroform-*d*) δ 8.51 (s, 2H), 8.35 (d, *J* = 5.2 Hz, 1H), 8.27 (d, *J* = 8.4 Hz, 2H), 7.44 (d, *J* = 2.4 Hz, 1H), 7.37 (d, *J* = 2.4 Hz, 1H), 7.28 (d, *J* = 8.4 Hz, 2H), 6.81 (d, *J* = 5.2 Hz, 1H), 5.11 (s, 2H), 4.87 (s, 4H), 4.42 (t, *J* = 6.1 Hz, 2H), 4.36 (s, 4H), 3.87 (t, *J* = 6.1 Hz, 2H), 1.70 (s, 6H).

**Example 39: Preparation of compound 39**

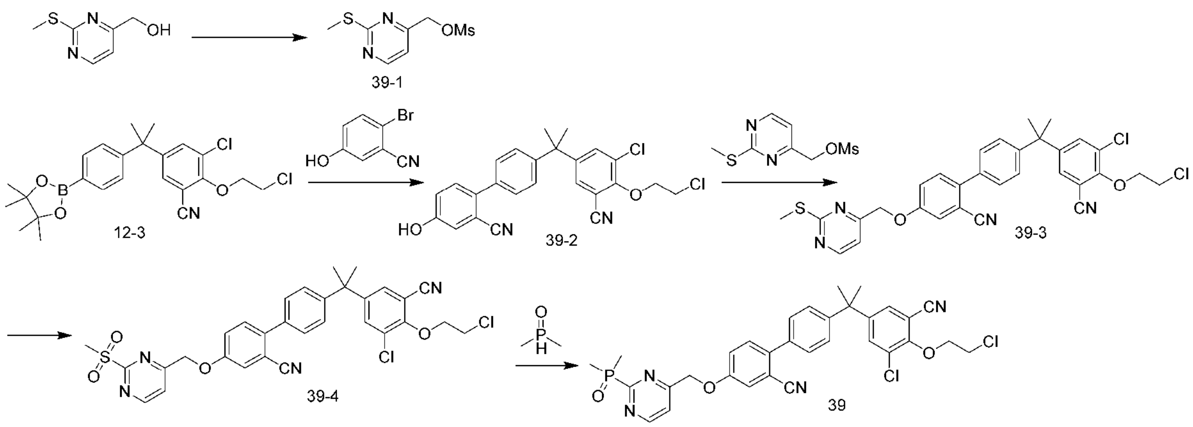

39-1, 12-3, 39-2, 39-3, 39-4, 39

**Preparation of compound 39-1**

Compound (2-(methylthio)pyrimidin-4-yl)methanol (2.4 g, 15.36 mmol) was dissolved in dichloromethane (40 mL), and triethylamine (3.1 g, 30.72 mmol) was added. The mixture was cooled to 0°C, and MsCl (2.6 g, 23.04 mmol) was added dropwise. After the addition was completed, the mixture was warmed to room temperature and stirred for 2 h. The reaction was monitored by LCMS until completion. The solvent was removed by concentration, and ice water (30 mL) was added to quench the reaction. The mixture was extracted three times with ethyl acetate (50 mL). The organic phases were combined, washed with saturated brine (50 mL), and dried over anhydrous sodium sulfate. The concentrate was purified by column chromatography (ethyl acetate/petroleum ether = 0 to 100%) to obtain 1.7 g of the title compound **39-1** in 47.2% yield. LC-MS (ESI): m/z $[M+H]^+$: 235.1.

**Preparation of compound 39-2**

Compound **12-3** (1.2 g, 2.61 mmol) and 2-bromo-5-hydroxybenzonitrile (0.6 g, 2.87 mmol) were dissolved in 1,4-dioxane (25 mL). [1,1'-Bis(diphenylphosphino)ferrocene]dichloropalladium(II) (130 mg, 0.18 mmol) and an aqueous potassium carbonate solution (2 mol/L, 3.9 mL, 7.80 mmol) were then added. The system was purged four times with argon, heated to 110°C, and stirred for 16 h until the reaction was complete. The reaction system was cooled to room temperature and adjusted to weakly acidic with 2 mol/L hydrochloric acid aqueous solution. Insoluble matter was filtered off through a pad of diatomite, and the filter cake was rinsed with ethyl acetate. The filtrates were combined and separated into layers. The organic phase was washed once with saturated brine, dried over anhydrous sodium sulfate, and concentrated. The residue was subjected to column chromatography (ethyl acetate/petroleum ether = 0 to 40%) to obtain 1.1 g of the title compound **39-2** in 93.5% yield. LC-MS (ESI): m/z $[M-H]^-$: 449.1.

**Preparation of compound 39-3**

Compound **39-2** (400 mg, 0.89 mmol) and compound **39-1** (219 mg, 0.93 mmol) were dissolved in acetonitrile (8.0 mL). Cesium carbonate (580 mg, 1.78 mmol) was added at room temperature, and the mixture was stirred at room temperature for 2 h. The reaction was monitored by LCMS until completion. The mixture was filtered, and the filter cake was rinsed with ethyl acetate. The filtrates were combined and concentrated. The resulting residue was subjected to column chromatography (ethyl acetate/petroleum ether = 0 to 30%) to obtain 480 mg of the title compound **39-3** in 91.9% yield. LC-MS (ESI): m/z $[M+Na]^+$: 611.1.

**Preparation of compound 39-4**

Compound **39-3** (460 mg, 0.78 mmol) was dissolved in a mixed solvent of THF (10 mL) and water (10 mL). Potassium peroxymonosulfate triple salt (1.92 g, 3.12 mmol) was added at room temperature, and the mixture was stirred at room temperature for 16 h. The reaction was monitored by LCMS until completion. Water (30 mL) was added, and the mixture was extracted twice with ethyl acetate (50 mL). The organic phases were combined, washed with saturated brine (50 mL), dried over anhydrous sodium sulfate, and concentrated to obtain 425 mg of the title compound **39-4** in 87.6% yield.

**Preparation of compound 39**

Compound **39-4** (415 mg, 0.68 mmol) and dimethylphosphine oxide (532 mg, 6.80 mmol) were dissolved in acetonitrile (8 mL). Potassium carbonate (752.0 mg, 5.44 mmol) was added at room temperature, and the mixture was heated to 85°C and stirred for 16 h. The reaction was monitored by LCMS until substantial completion. Saturated brine (30 mL) was added to quench the reaction, and the mixture was extracted three times with ethyl acetate (50 mL). The organic phases were combined, washed with saturated brine (50 mL), dried over anhydrous sodium sulfate, and concentrated. The resulting residue was subjected to column chromatography (methanol/dichloromethane = 0 to 10 %) to obtain 130 mg of a crude product. The crude product was subjected to preparative purification (prep-HPLC (Waters 2767/Qda, Column: XBridge XBridge C18 19 * 250 mm, 10 μm; Mobile Phase A: 0.03% $NH_3H_2O$/H2O, B: ACN; flow rate: 20 mL/min; gradient: 59% to 59%; Retention Time: 9.2-10.7 min of 16 min)) to obtain 43.11 mg of the title compound **39.** LC-MS (ESI): m/z $[M+H]^+$: 619.4. $^1$H NMR (400 MHz, DMSO-*d6*) δ 9.02 (d, *J* = 5.2 Hz, 1H), 7.77 (dd, *J* = 4.8, 3.2 Hz, 1H), 7.74 (d, *J* = 2.4 Hz, 1H), 7.71 (d, *J* = 2.8 Hz, 1H), 7.68 (d, *J* = 2.3 Hz, 1H), 7.59 (d, *J* = 8.8 Hz, 1H), 7.52 - 7.48 (m, 3H), 7.40 (d, *J* = 8.4 Hz, 2H), 5.45 (s, 2H), 4.43 (t, *J* =5.2 Hz, 2H), 3.96 (t, *J* =5.2 Hz, 2H), 1.76 (d, *J* = 13.6 Hz, 6H), 1.72 (s, 6H). $^{31}$P NMR (162 MHz, DMSO-*d6*) δ 34.04 (s,1P).

**Example 40: Preparation of compound 40**

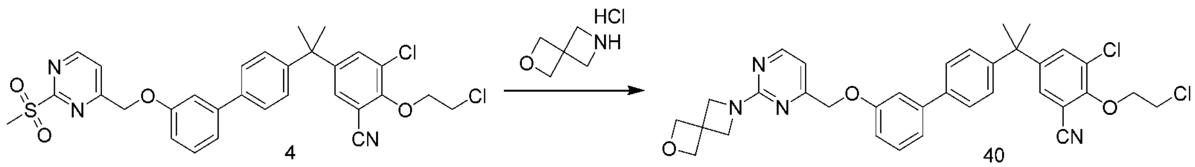


**Preparation of compound 40**

Compound **4** (73.32 mg, 122.92 μmol) was added to a reaction flask, followed by the addition of THF (2 mL). 2-Oxa-6-azaspiro[3.3]heptane hydrochloride (20 mg, 147.50 μmol) and $K_2CO_3$ (42.47 mg, 307.30 μmol) were added at room temperature. The system was purged three times with nitrogen, and the reaction was carried out at 80°C for 5 h. LCMS monitoring indicated that the reaction was complete, and the molecular ion peak of the target product was observed. The reaction mixture was cooled to room temperature, water (30 mL) was added, and the mixture was extracted three times with EA (30 mL). The organic phases were combined, dried over anhydrous sodium sulfate, subjected to suction filtration, and the filtrate was evaporated to dryness under reduced pressure. The resulting crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 10% to 60% acetonitrile gradient elution over 30 min; flow rate: 30 mL/min) to obtain 26 mg of the title compound **40.** LC-MS (ESI): m/z 615.3 $[M+H]^+$. $^1$H NMR (400 MHz, DMSO-*d6*) δ 8.35 (d, *J* = 4.8 Hz, 1H), 7.71 (d, *J* = 2.4 Hz, 1H), 7.65 (d, *J* = 2.4 Hz, 1H), 7.60 - 7.64 (m, 2H), 7.37 (t, *J* = 7.6 Hz, 1H), 7.31 - 7.34 (m, 2H), 7.27 - 7.28 (m, 1H), 7.24 - 7.26 (m, 1H), 6.96 - 6.99 (m, 1H), 6.80 (d, *J* = 5.2 Hz, 1H), 5.08 (s, 2H), 4.71 (s, 4H), 4.43 (t, *J* = 5.2 Hz, 2H), 4.20 (s, 4H), 3.96 (t, *J* = 5.2 Hz, 2H), 1.69 (s, 6H).

**Example 41: Preparation of compound 41**

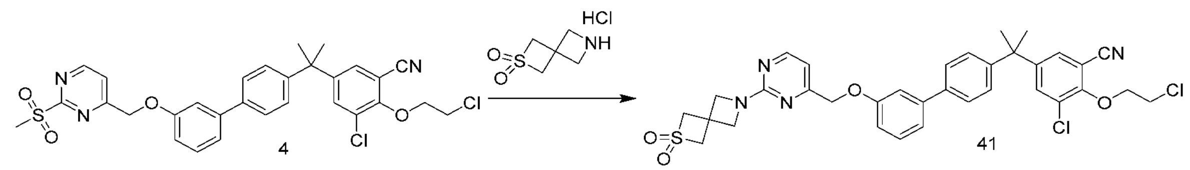


**Preparation of compound 41**

Compound **4** (54.13 mg, 90.75 μmol), THF (2 mL), 2-thia-6-azaspiro[3.3]heptane 2,2-dioxide hydrochloride (20 mg, 108.90 μmol), and $K_2CO_3$ (31.36 mg, 226.87 μmol) were added to a reaction flask. The system was purged three times with nitrogen, and the reaction was carried out at 80°C for 5 h. LCMS monitoring indicated that the reaction was

complete, and the molecular ion peak of the target product was observed. The reaction mixture was cooled to room temperature, water (30 mL) was added, and the mixture was extracted three times with EA (30 mL). The organic phases were combined, evaporated to dryness under reduced pressure, and purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 10% to 60% acetonitrile gradient elution over 30 min; flow rate: 30 mL/min) to obtain 25 mg of the title compound 41. LC-MS (ESI): m/z 663.2 $[M+H]^+$. $^1$H NMR (400 MHz, DMSO-*d6*) δ 9.40 (d, *J* = 5.2 Hz, 1H), 7.71 (d, *J* = 2.4 Hz, 1H), 7.65 (d, *J* = 2.5 Hz, 1H), 7.60 - 7.63 (m, 2H), 7.37 (t, *J* = 8.0 Hz, 1H), 7.31 - 7.35 (m, 2H), 7.28 - 7.29 (m, 1H), 7.24 - 7.27 (m, 1H), 6.97 - 7.00 (m, 1H), 6.86 (d, *J* = 4.8 Hz, 1H), 5.09 (s, 2H), 4.50 (s, 4H), 4.43 (t, *J* = 5.2 Hz, 2H), 4.27 (s, 4H), 3.96 (t, *J* = 5.2 Hz, 2H), 1.69 (s, 6H).

**Example 42: Preparation of compound 42**

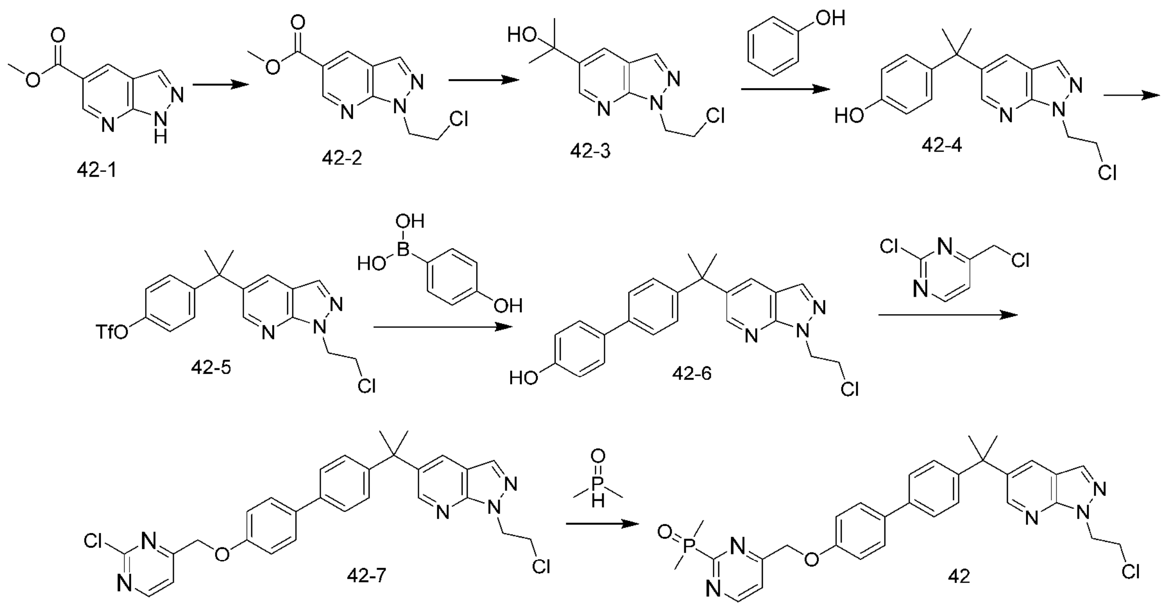

42-1 42-2 42-3 42-4 42-5 42-6 42-7 42

**Preparation of compound 42-2**

Compound **42-1** (200 mg, 1.13 mmol) and 1-bromo-2-chloro-ethane (194.28 mg, 1.35 mmol, 112.76 μL) were weighed and dissolved in acetonitrile (10 mL). $Cs_2CO_3$ (735.65 mg, 2.26 mmol) was added. The reaction mixture was reacted at 80°C for 4 h. LCMS indicated the disappearance of the starting material and the formation of the product. The reaction mixture was poured into water and extracted twice with ethyl acetate (50 mL). The organic phases were combined, washed with water (30 mL), dried over anhydrous sodium sulfate, subjected to suction filtration, and the filtrate was evaporated to dryness under reduced pressure. The resulting residue was purified by column chromatography (EtOAc/PE: = 0 to 30%) to obtain 180 mg of the title compound **42-2** in 66.53% yield. LC-MS (ESI): m/z 240.2 $[M+H]^+$.

**Preparation of compound 42-3**

Compound **42-2** (4.4 g, 18.36 mmol) was weighed and dissolved in tetrahydrofuran (50 mL), and the system was purged three times with $N_2$. Methylmagnesium bromide (3 M, 110.16 mmol, 36.72 mL) was added dropwise at 0°C. The reaction mixture was stirred at 0°C for 2 h. LCMS indicated the disappearance of the starting material and the formation of the product. The reaction mixture was poured into saturated $NH_4Cl$ solution (50 mL). The mixture was extracted twice with ethyl acetate (200 + 150 mL). The organic phases were combined, washed with water (150 mL), dried over anhydrous sodium sulfate, subjected to suction filtration, and the filtrate was evaporated to dryness under reduced pressure. The resulting residue was purified by column chromatography (EtOAc/PE: = 0 to 15%) to obtain 3.5 g of the title compound in 79.5% yield. LC-MS (ESI): m/z 240.2 $[M+H]^+$.

**Preparation of compound 42-4**

Compound **42-3** and phenol (6.48 g, 68.84 mmol) were weighed and added to 1,2-dichloroethane (40 mL), followed by the addition of anhydrous aluminum trichloride (3.67 g, 27.53 mmol). After the system was purged three times with $N_2$, the reaction mixture was stirred at 100°C for 3 h. TLC indicated the disappearance of the starting material and the formation of a new spot. The reaction mixture was poured into water (40 mL). The mixture was extracted twice with dichloromethane (60 mL + 40 mL). The organic phases were combined, washed with water (30 mL), dried over anhydrous sodium sulfate, subjected to suction filtration, and the filtrate was evaporated to dryness under reduced pressure. The resulting residue was purified by column chromatography (EtOAc/PE: = 0 to 15%) to obtain 3.6 g of the title compound **42-4** in 82.8% yield. LC-MS (ESI): m/z 316.4 $[M+H]^+$.

**Preparation of compound 42-5**

Compound **42-4** (1 g, 3.17 mmol) and triethylamine (961.28 mg, 9.50 mmol, 1.32 mL) were weighed and added to dichloromethane (15 mL). The system was purged three times with $N_2$, and trifluoromethanesulfonic anhydride (1.07 g, 3.80 mmol, 639.30 μL) was added dropwise at 0°C. The reaction mixture was stirred at room temperature for 1 h. LCMS indicated the disappearance of the starting material and the formation of the product. The reaction mixture was evaporated to dryness under reduced pressure, and the resulting residue was purified by column chromatography (EtOAc/PE: = 0 to 5%) to obtain 900 mg of the title compound **42-5** in 63.4% yield. LC-MS (ESI): m/z 448.2 $[M+H]^+$.

**Preparation of compound 42-6**

Compound **42-5** (698.46 mg, 1.56 mmol), 4-hydroxyphenylboronic acid (236.62 mg, 1.72 mmol), [1,1'-bis(diphenylphosphino)ferrocene]dichloropalladium(II) dichloromethane complex (114.11 mg, 155.96 μmol), potassium carbonate (538.85 mg, 3.90 mmol), dioxane (10 mL), and water (5 mL) were added to a 100 mL single-necked flask. The system was purged three times with nitrogen and stirred at 90°C for 2 h. LCMS indicated that the reaction was complete, and the main peak was the molecular ion peak of the target product. The reaction mixture was evaporated to dryness under reduced pressure and purified by column chromatography (EA/PE = 0 to 15%) to obtain the title compound **42-6.** LC-MS (ESI): m/z 392.2 $[M+H]^+$.

**Preparation of compound 42-7**

**42-6** and 2-chloro-4-(chloromethyl)pyrimidine (228.77 mg, 1.40 mmol) were weighed and added to acetonitrile (8 mL), followed by the addition of potassium carbonate (352.66 mg, 2.55 mmol). The reaction mixture was stirred at 70°C for 3 h. The reaction mixture was poured into water. The mixture was extracted twice with dichloromethane (20 mL + +15 mL). The organic phases were combined, washed with water (15 mL), dried over anhydrous sodium sulfate, subjected to suction filtration, and the filtrate was evaporated to dryness under reduced pressure. The resulting residue was purified by column chromatography (EtOAc/PE = 0 to 15%) to obtain 540 mg of the title compound **42-7** in 81.6% yield. LC-MS (ESI): m/z 518.4 $[M+H]^+$.

**Preparation of compound 42**

Compound **42-7** (100 mg, 192.89 μmol), dimethylphosphine oxide (45.16 mg, 578.66 μmol), 4,5-bis(diphenylphosphino)-9,9-dimethylxanthene (22.32 mg, 38.58 μmol), and *N,N*-diisopropylethylamine (74.79 mg, 578.66 μmol, 100.79 μL) were weighed and added to DMF (3 mL), and tris(dibenzylideneacetone)dipalladium (17.66 mg, 19.29 μmol) was added. The system was purged three times with $N_2$. The reaction mixture was heated under microwave irradiation at 120°C for 3 h. LCMS indicated the disappearance of the starting material and the formation of the product. The reaction mixture was subjected to preparative purification (preparative method: mobile phase: A: 0.1% formic acid in water; B: acetonitrile; chromatographic column: Welch Ultimate Polar-RP 250 × 30 mm; column temperature: 25°C; gradient: 50% to 70% acetonitrile gradient elution over 8 min; flow rate: 50 mL/min) to obtain 41.8 mg of the title compound **42.** LC-MS (ESI): m/z 560.2 $[M+H]^+$. $^1$H NMR (400 MHz, DMSO-*d6*) δ 9.00 (d, *J* = 5.2 Hz, 1H), 8.40 (d, *J* = 2.4 Hz, 1H), 8.19 (d, *J* = 2.0 Hz, 1H), 8.17 (s, 1H), 7.73 (dd, *J* = 5.2, 3.2 Hz, 1H), 7.61 (d, *J* = 8.8 Hz, 2H), 7.55 (d, *J* = 8.4 Hz, 2H), 7.31 (d, *J* = 8.4 Hz, 2H), 7.14 (d, *J* = 8.8 HZ, 2H), 5.35 (s, 2H), 4.75 (t, *J* = 5.6 Hz, 2H), 4.12 (t, *J* = 5.6 Hz, 2H), 1.76 (d, *J* = 13.6 Hz, 6H), 1.77 (s, 6H). $^{31}$P NMR (162 MHz, DMSO-$d_6$) δ 34.07 (s, 1P).

**Example 43: Preparation of compound 43**

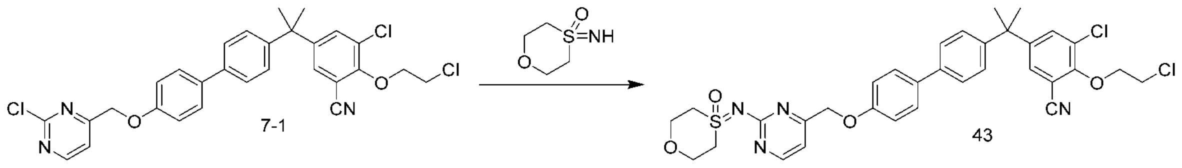

Preparation of compound 43

Compound **7-1** (200 mg, 0.36 mmol) was dissolved in DMF (2.0 mL), followed by the sequential addition of tris(dibenzylideneacetone)dipalladium (33 mg, 0.04 mmol), 4,5-bis(diphenylphosphino)-9,9-dimethylxanthene (42 mg, 0.07), DIEA (94 mg, 0.7 mmol), and 1,4-oxathiane sulfoximine (73 mg, 0.54 mmol). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 82 mg of the title compound **43.** LC-MS (ESI): m/z 651.3 $[M+H]^+$. $^1H$ NMR (400 MHz, DMSO-$d_6$) δ 8.46 (d, *J* = 5.0 Hz, 1H), 7.71 (d, *J* = 2.3 Hz, 1H), 7.64 (d, *J* = 2.3 Hz, 1H), 7.60 (d, *J* = 8.8 Hz, 2H), 7.56 (d, *J* = 8.4 Hz, 2H), 7.30 (d, *J* = 8.4 Hz, 2H), 7.07 (d, *J* = 8.8 Hz, 2H), 7.00 (d, *J* = 5.0 Hz, 1H), 5.12 (s, 2H), 4.42 (t, *J* = 5.2 Hz, 2H), 4.05 - 4.09 (m, 2H), 3.96 (t, *J* = 5.2 Hz, 2H), 3.97 - 3.93 (m, 2H), 3.80 - 3.74 (m, 2H), 3.59 - 3.54 (m, 2H), 1.68 (s, 6H).

**Example 44: Preparation of compound 44**

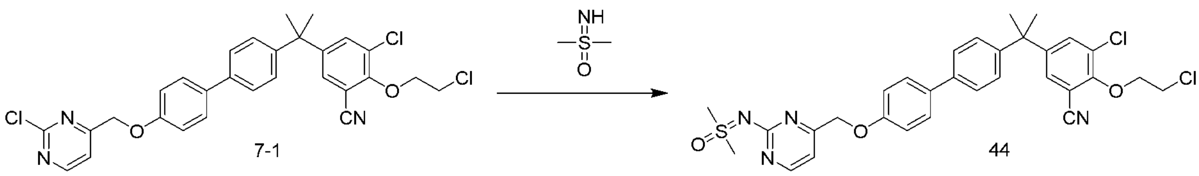

**Preparation of compound 44**

Compound **7-1** (200 mg, 0.36 mmol) was dissolved in DMF (2.0 mL), followed by the sequential addition of tris(dibenzylideneacetone)dipalladium (33 mg, 0.04 mmol), 4,5-bis(diphenylphosphino)-9,9-dimethylxanthene (42 mg, 0.07), DIEA (94 mg, 0.7 mmol), and dimethyl sulfoximine (51 mg, 0.54 mmol). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 17 mg of the title compound **44.** LC-MS (ESI): m/z 609.2 $[M+H]^+$. $^1H$ NMR (400 MHz, DMSO-$d_6$) δ 8.45 (d, *J* = 5.1 Hz, 1H), 7.70 (d, *J* = 2.4 Hz, 1H), 7.64 (d, *J* = 2.4 Hz, 1H), 7.60 (d, *J* = 8.8 Hz, 2H), 7.55 (d, *J* = 8.5 Hz, 2H), 7.30 (d, *J* = 8.5 Hz, 2H), 7.07 (d, *J* = 8.8 Hz, 2H), 6.95 (d, *J* = 5.1 Hz, 1H), 5.10 (s, 2H), 4.42 (t, *J* = 5.2 Hz, 2H), 3.96 (t, *J* = 5.2 Hz, 2H), 3.40 (s, 6H), 1.68 (s, 6H).

**Example 45: Preparation of compound 45**

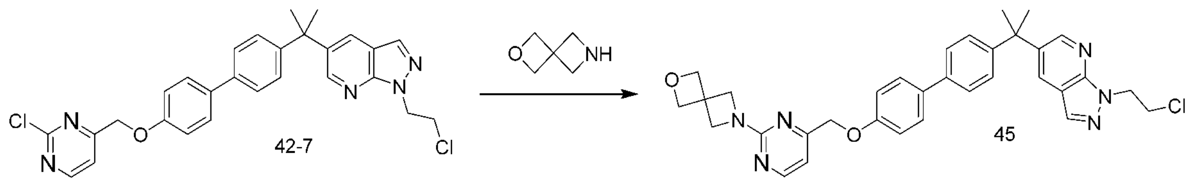

42-7 → 45

**Preparation of compound 45**

Compound **42-7** (80 mg, 154.31 μmol) and 2-oxa-6-azaspiro[3.3]heptane (16.83 mg, 169.74 μmol) were weighed and added to ethanol (2 mL), and triethylamine (46.84 mg, 462.93 μmol, 64.57 μL) was added. The system was purged three times with nitrogen. The reaction mixture was heated under microwave irradiation at 80°C for 3 h. LCMS indicated the disappearance of the starting material and the formation of the product. The reaction mixture was subjected to preparative purification (preparative method: chromatographic column: Pntulips ZZ-C18 10 μm 250 × 20 mm; column temperature: 25°C; mobile phase: water (0.1% FA) - acetonitrile; gradient elution with the proportion of acetonitrile in the mobile phase from 50% to 70% over 8 min, then from 70% to 90% acetonitrile over 4 min; flow rate: 30 mL/min) to obtain 42 mg of the title compound **45.** LC-MS (ESI): m/z 582.2 [M+H]+. $^{1}$H NMR (400 MHz, DMSO-*d6*) δ 8.39 (d, *J* = 2.3 Hz, 1H), 8.35 (d, *J* = 5.0 Hz, 1H), 8.18 (d, *J* = *2.3* Hz, 1H), 8.17 (s, 1H), 7.58 (d, *J* = 8.8 Hz, 2H), 8.54 (d, *J* = 8.4 Hz, 2H), 7.31 (d, *J* = 8.4 Hz, 2H), 7.06 (d, *J* = 8.8 Hz, 2H), 6.76 (d, *J* = 5.0 Hz, 1H), 5.04 (s, 2H), 4.75 (t, *J* = 5.8 Hz, 2H), 4.72 (s, 4H), 4.21 (s, 4H), 4.12 (t, *J* = 5.8 Hz, 2H), 1.77 (s, 6H).

**Example 46: Preparation of compound 46**

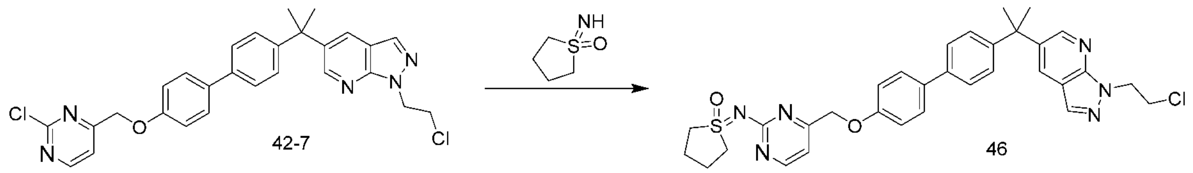

42-7 → 46

**Preparation of compound 46**

Compound **42-7** (80 mg, 154.31 μmol) and compound 1-iminotetrahydrothiophene 1-oxide (27.59 mg, 231.47 μmol) were weighed and added to DMF (2 mL), followed by the addition of tris(dibenzylideneacetone)dipalladium (14.13 mg, 15.43 μmol), 4,5-bis(diphenylphosphino)-9,9-dimethylxanthene (17.86 mg, 30.86 μmol), and triethylamine (46.84 mg, 462.93 μmol, 64.57 μL). The reaction mixture was heated under microwave irradiation at 120°C for 3 h. LCMS indicated the disappearance of the starting material and the formation of the product. The reaction mixture was subjected to preparative purification (preparation method: chromatographic column: Pntulips ZZ-C18 10 μm 250 × 20 mm; column temperature: 25°C; mobile phase: water (0.1% FA) - acetonitrile; gradient elution with the proportion of acetonitrile in the mobile phase from 50% to 70% over 8 min; flow rate: 30 mL/min) to obtain 24.5 mg of the title compound **46.** LC-MS (ESI): m/z 601.2 [M+H]$^{+}$. $^{1}$H NMR (400 MHz, DMSO-*d6*) δ 8.46 (d, *J* = 5.0, 1H), 8.39 (d, *J* = 2.3 Hz, 1H), 8.18 (d, *J* = 2.2 Hz, 1H), 8.17 (s, 1H), 7.58 (d, *J* = 8.8 Hz, 2H), 7.54 (d, *J* = 8.5 Hz, 2H), 7.31 (d, *J* = 8.5 Hz, 2H), 7.07 (d, *J* = 8.8 Hz, 2H), 6.98 (d, *J* = 5.0 Hz, 1H), 5.10 (s, 2H), 4.75 (t, *J* = 5.8 Hz, 2H), 4.12 (t, *J* = 5.8 Hz, 2H), 3.61 - 3.54 (m, 2H), 3.39 - 3.32 (m, 2H), 2.26 - 2.17 (m, 2H), 2.11 - 2.04 (m, 2H), 1.77 (s, 6H).

**Example 47: Preparation of compound 47**

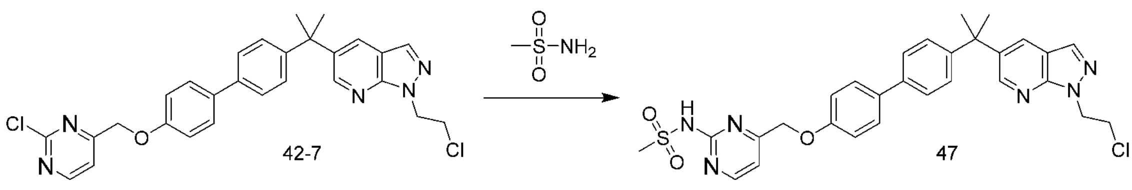

42-7 → 47

**Preparation of compound 47**

Compound **42-7** (100 mg, 192.89 μmol) and methanesulfonamide (36.70 mg, 385.78 μmol) were weighed and added to acetonitrile (3 mL), and cesium carbonate (125.69 mg, 385.78 μmol, 173.25 μL) was added. The reaction mixture was stirred at 80°C for 3 h. LCMS indicated the disappearance of the starting material and the formation of the product. The reaction mixture was subjected to preparative purification (preparative method: mobile phase: A: 0.1% formic acid in water; B: acetonitrile; chromatographic column: Welch Ultimate Polar-RP 250 × 30 mm; column temperature: 25°C; gradient: 60% to 80% acetonitrile gradient elution over 8 min; flow rate: 50 mL/min) to obtain 6.1 mg of the title compound **47.** LC-MS (ESI): m/z 577.2 $[M+H]^+$. $^1$H NMR (400 MHz, DMSO-*d6*) δ 8.40 (d, *J* = 2.2 Hz, 1H), 8.36 (m, 2H), 8.18 (d, *J* = 2.4 Hz, 1H), 7.58 (d, *J* = 8.8 Hz, 2H), 7.54 (d, *J* = 8.5 Hz, 2H), 7.31 (d, *J* = 8.5 Hz, 2H), 7.07 (d, *J* = 8.8 Hz, 2H), 6.83 (br.s, 2H), 5.04 (s, 2H), 4.75 (t, *J* = 5.8 Hz, 2H), 4.13 (t, *J* = 5.8 Hz, 2H), 3.08 (s, 3H), 1.77 (s, 6H).

**Example 48: Preparation of compound 48**

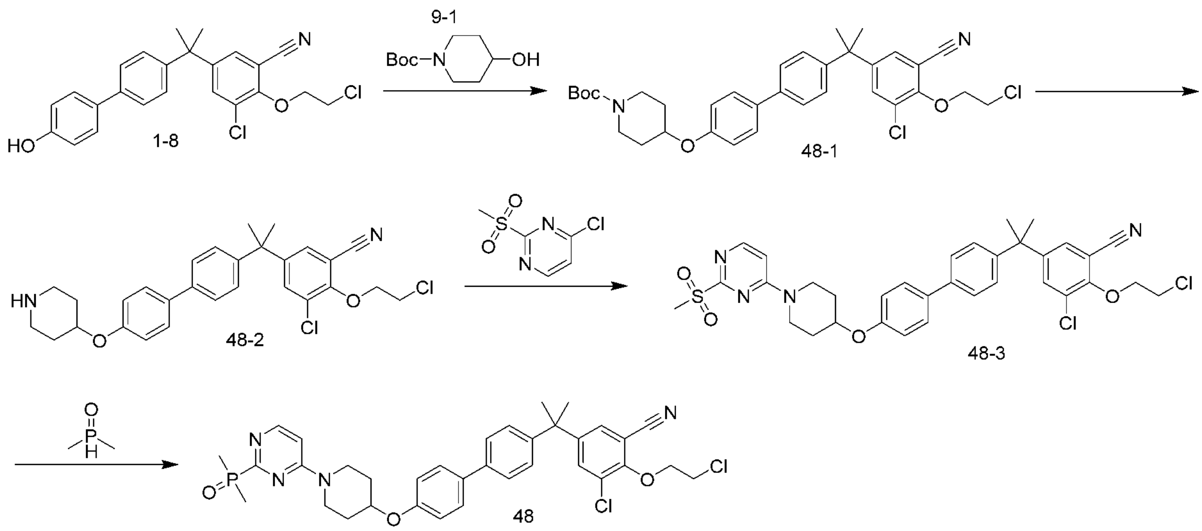

1-8
48-1
48-2
48-3
48

**Preparation of compound 48-1**

Compound **1-8** (260 mg, 0.62 mmol), compound **9-1** (145 mg, 0.72 mmol), and N,N,N,N-tetramethylazodicarboxamide (215 mg, 1.24 mmol) were dissolved in dichloromethane (20 mL), and tributylphosphine (375 mg, 1.86 mmol) was added thereto. The reaction system was stirred at 25°C for 1 h. After the reaction was completed, water (10 mL) was added, and the mixture was extracted three times with dichloromethane (20 mL). The organic phases were combined, washed twice with saturated brine (20 mL), dried over anhydrous sodium sulfate, and concentrated to obtain a crude product. The crude product was purified by column chromatography (PE:EA = 30%) to obtain 200 mg of the title compound **48-1.** LC-MS (ESI): m/z 553.2 [M+H-*t*Bu]$^+$.

**Preparation of compound 48-2**

Compound **48-1** (200 mg, 0.3 mmol) was dissolved in dichloromethane (5 mL), and a solution of hydrogen chloride in 1,4-dioxane (4 M, 10 mL) was added thereto at 0°C. The reaction system was stirred at 25°C for 1 h. After the reaction was completed, the solvent was directly removed by rotary evaporation to obtain a crude product **48-2.** The crude product was used directly in the next step without purification. LC-MS (ESI): m/z 509.2 $[M+H]^+$.

**Preparation of compound 48-3**

Compound **48-2** (200 mg, 0.39 mmol) was dissolved in methanol (10 mL), followed by the sequential addition of 4-chloro-2-(methylsulfonyl)pyrimidine (89 mg, 0.47 mmol) and triethylamine (79 mg, 0.78 mmol). The reaction system was stirred at 25°C for 1 h. After the reaction was completed, water (10 mL) was added, and the mixture was extracted three times with ethyl acetate (20 mL). The organic phases were combined, washed twice with saturated brine (20 mL), dried over anhydrous sodium sulfate, and concentrated to obtain a crude product. The crude product was purified by column

chromatography (PE:EA = 60%) to obtain 200 mg of the title compound **48-3.** LC-MS (ESI): m/z 665.2 $[M+H]^+$.

**Preparation of compound 48**

Compound **48-3** (100 mg, 0.15 mmol) was dissolved in acetonitrile (5 mL), followed by the sequential addition of dimethylphosphine oxide (35 mg, 0.45 mmol) and potassium carbonate (62 mg, 0.45 mmol). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 37.8 mg of the title compound **48.** LC-MS (ESI): m/z 663.3 $[M+H]^+$. $^1H$ NMR (400 MHz, Chloroform-*d*) δ 8.29 (d, *J* = 6.1 Hz, 1H), 7.53 (d, *J* = 8.6 Hz, 2H), 7.49 (d, *J* = 8.4 Hz, 2H), 7.49 (d, *J* = 2.3 Hz, 1H), 7.37 (d, *J* = 2.3 Hz, 1H), 7.23 (d, *J* = 8.4 Hz, 2H), 7.00 (d, *J* = 8.6 Hz, 2H), 6.62 (br.s, 1H), 4.68 (br.s, 1H), 4.43 (t, *J* = 6.2 Hz, 2H), 3.91 (m, 4H), 3.88 (t, *J* = 6.2 Hz, 2H), 2.06 - 1.95 (m, 4H), 1.88 (d, *J* = 13.5 Hz, 6H), 1.70 (s, 6H). $^{31}P$ NMR (162 MHz, Chloroform-d) δ 35.95 (s, 1P).

**Example 49: Preparation of compound 49**

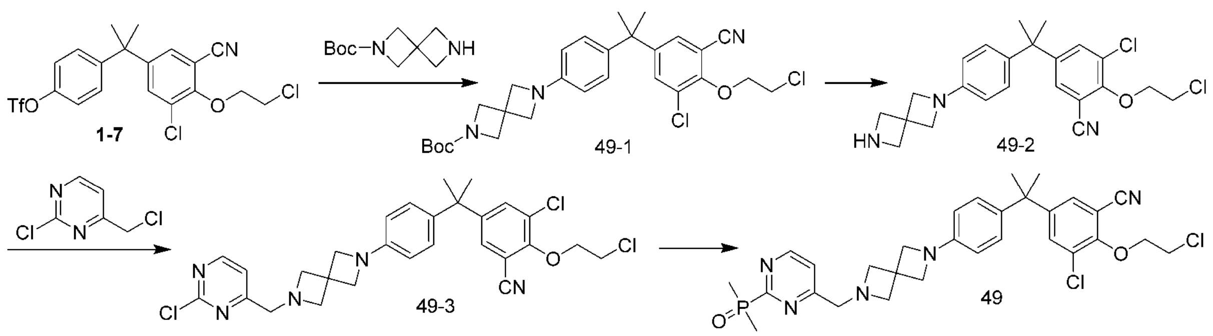


**Preparation of compound 49-1**

Compound **1-7** (1 g, 2.07 mmol) was dissolved in DMF (10 mL), followed by the sequential addition of tris(dibenzylideneacetone)dipalladium (190 mg, 0.21 mmol), 2,2'-bis(diphenylphosphino)-1,1'-binaphthyl (240 mg, 0.42 mmol), cesium carbonate (536 mg, 4.15 mmol), and *tert*-butyl 2,6-diazaspiro[3.3]heptane-2-carboxylate (493 mg, 2.49 mmol). The reaction system was stirred at 70°C for 16 h. After the reaction was completed, water (10 mL) was added, and the mixture was extracted three times with dichloromethane (20 mL). The organic phases were combined, washed twice with saturated brine (20 mL), dried over anhydrous sodium sulfate, and concentrated to obtain a crude product. The crude product was purified by column chromatography (PE:EA = 30%) to obtain 560 mg of the title compound **49-1.** LC-MS (ESI): m/z 530.0 $[M+H]^+$.

**Preparation of compound 49-2**

Compound **49-1** (560 mg, 1.06 mmol) was dissolved in a solution of hydrochloric acid in methanol (4 N, 3.0 mL, 12 mmol), and the reaction system was stirred at room temperature for 3 h. After the reaction was completed, water (10 mL) was added, and the mixture was extracted three times with dichloromethane (20 mL). The organic phases were combined, washed twice with saturated brine (20 mL), dried over anhydrous sodium sulfate, and concentrated to obtain 500 mg of a crude product of the title compound **49-2.** LC-MS (ESI): m/z 430.0 $[M+H]^+$.

**Preparation of compound 49-3**

Compound **49-2** (300 mg, 0.70 mmol) was dissolved in acetonitrile (10 mL), followed by the sequential addition of compound 2-chloro-4-(chloromethyl)pyrimidine (171 mg, 1.05 mmol) and cesium carbonate (682 mg, 2.10 mmol). The reaction system was stirred at 25°C for 1 h. After the reaction was completed, water (10 mL) was added, and the mixture was extracted three times with ethyl acetate (20 mL). The organic phases were combined, washed twice with saturated

brine (20 mL), dried over anhydrous sodium sulfate, and concentrated to obtain a crude product. The crude product was purified by column chromatography (PE:EA = 70%) to obtain 200 mg of the title compound **49-3.** LC-MS (ESI): m/z 556.2 $[M+H]^+$.

**Preparation of compound 49**

Compound **49-3** (100 mg, 0.18 mmol) was dissolved in DMF (5.0 mL), followed by the sequential addition of dimethylphosphine oxide (42 mg, 0.54 mmol), DIPEA (70 mg, 0.54 mmol), tris(dibenzylideneacetone)dipalladium (18 mg, 0.02 mmol), and 4,5-bis(diphenylphosphino)-9,9-dimethylxanthene (27 mg, 0.05 mmol). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 6 mg of the title compound **49.** LC-MS (ESI): m/z 598.2 $[M+H]^+$. $^1H$ NMR (400 MHz, Chloroform-*d*) δ 8.82 (d, *J* = 5.1 Hz, 1H), 7.49 (dd, *J* = 5.1, 3.3 Hz, 1H), 7.44 (d, *J* = 2.4 Hz, 1H), 7.29 (d, *J* = 2.3 Hz, 1H), 7.02 (d, *J* = 8.6 Hz, 2H), 6.40 (d, *J* = 8.6 Hz, 2H), 4.40 (t, *J* = 6.2 Hz, 2H), 3.99 (s, 4H), 3.93 (s, 2H), 3.87 (t, *J* = 6.2 Hz, 2H), 3.68 (s, 4H), 1.89 (d, *J* = 13.6 Hz, 6H), 1.61 (s, 6H). $^{31}P$ NMR (162 MHz, Chloroform-*d*) δ 36.12 (s, 1P).

**Example 50: Preparation of compound 50**

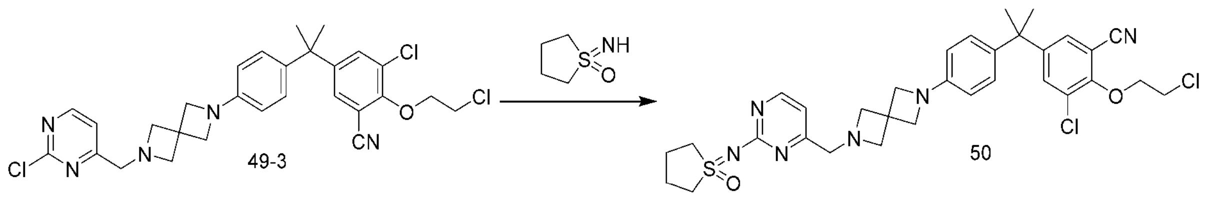

**Preparation of compound 50**

Compound **49-3** (100 mg, 0.18 mmol) was dissolved in DMF (5 mL), followed by the sequential addition of 1-iminotetrahydrothiophene-1-oxide (42 mg, 0.54 mmol), DIPEA (70 mg, 0.54 mmol), tris(dibenzylideneacetone)dipalladium (18 mg, 0.02 mmol), and 4,5-bis(diphenylphosphino)-9,9-dimethylxanthene (27 mg, 0.05 mmol). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 4.7 mg of the title compound **50.** LC-MS (ESI): m/z 639.2 $[M+H]^+$. $^1H$ NMR (400 MHz, *Chloroform-d*) δ 8.48 (d, *J* = 5.0 Hz, 1H), 7.43 (d, *J* = 2.4 Hz, 1H), 7.28 (d, *J* = 2.4 Hz, 1H), 7.01 (d, *J* = 8.6 Hz, 2H), 6.94 (d, *J* = 5.0 Hz, 1H), 6.38 (d, *J* = 8.6 Hz, 2H), 4.40 (t, *J* = 6.2 Hz, 2H), 4.03 - 3.97 (m, 8H), 3.87 (t, *J* = 6.2 Hz, 2H), 3.75 - 3.66 (m, 2H), 3.49 - 3.42 (m, 2H), 2.37 - 2.27 (m, 4H), 1.61 (s, 6H).

**Example 51: Preparation of compound 51**

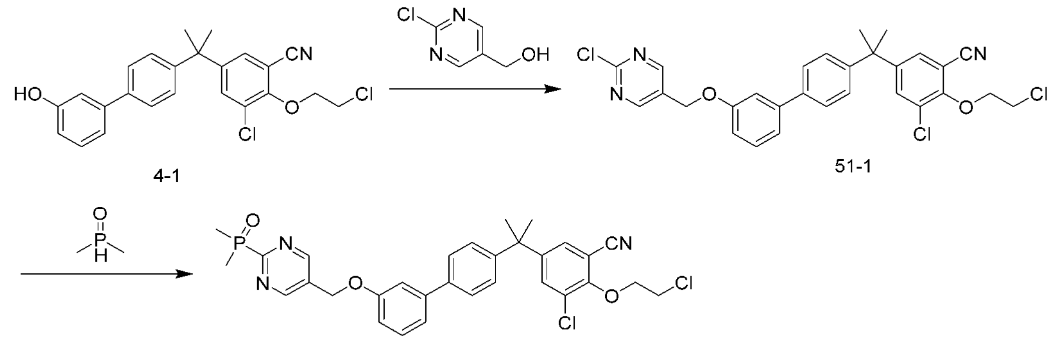

4-1

51-1

51

### Preparation of compound 51-1

Compound **4-1** (250 mg, 0.59 mmol) was added to a three-necked flask, followed by the sequential addition of anhydrous DCM (10 mL), (2-chloropyrimidin-5-yl)methanol (110 mg, 0.76 mmol), and *N,N,N',N'*-tetramethylazodicarboxamide (202 mg, 1.17 mmol). The system was purged three times with nitrogen, and tributylphosphine (237 mg, 1.17 mmol) was added at 0°C. The reaction was stirred at 25°C for 3 h. After the reaction was completed, water (20 mL) was added, and the mixture was extracted twice with DCM (20 mL). The organic phases were combined, dried, and concentrated to obtain a crude product. The crude product was purified by normal-phase silica gel column chromatography (EtOAc/PE = 0 to 30%) to obtain 150 mg of the title compound **51-1** in 46% yield. LC-MS (ESI): 552.2 $[M+H]^+$.

### Preparation of compound 51

Compound **51-1** (100 mg, 0.18 mmol) was dissolved in DMF (2.0 mL), followed by the sequential addition of tris(dibenzylideneacetone)dipalladium (17 mg, 0.02 mmol), 4,5-bis(diphenylphosphino)-9,9-dimethylxanthene (21 mg, 0.04), DIEA (70 mg, 0.54 mmol), and dimethylphosphine oxide (42 mg, 0.54 mmol). The reaction system was stirred at 120°C for 3 h in a microwave reactor. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 54 mg of the title compound **51.** LC-MS (ESI): m/z 594.2 $[M+H]^+$. $^1$H NMR (400 MHz, DMSO-$d_6$) δ 9.10 (s, 2H), 7.71 (d, *J* = 2.3 Hz, 1H), 7.65 (d, *J* = 2.3 Hz, 1H), 7.62 (d, *J* = 8.6 Hz, 2H), 7.40 (t, *J* = 7.9 Hz, 1H), 7.34 (d, *J* = 8.6 Hz, 2H), 7.34 - 7.32 (m, 1H), 7.27 (d, *J* = 8.0 Hz, 1H), 7.05 (dd, *J* = 8.2, 2.5 Hz, 1H), 5.34 (s, 2H), 4.42 (t, *J* = 5.2 Hz, 2H), 3.96 (t, *J* = 5.2 Hz, 2H), 1.77 (d, *J* = 13.7 Hz, 6H), 1.69 (s, 6H). $^{31}$P NMR (162 MHz, DMSO-$d_6$) δ 34.04 (s, 1P).

### Example 52: Preparation of compound 52

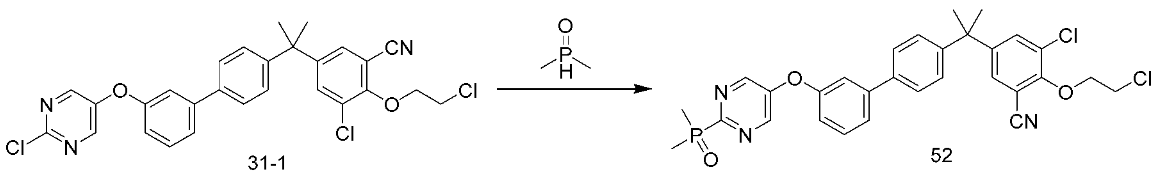

31-1

52

### Preparation of compound 52

Compound **31-1** (30 mg, 0.06 mmol) was added to a microwave tube and dissolved in dioxane (0.5 mL), followed by the sequential addition of dimethylphosphine oxide (7 mg, 0.07 mmol), tris(dibenzylideneacetone)dipalladium (5 mg, 0.006 mmol), 4,5-bis(diphenylphosphino)-9,9-dimethylxanthene (6 mg, 0.01 mmol), and *N,N*-diisopropylethylamine (24 mg, 0.19 mmol). The reaction mixture was stirred at 120°C under a nitrogen atmosphere for 16 h. After the reaction was completed, the reaction mixture was filtered to remove insoluble matter. Water (5 mL) was added, and the mixture was extracted three times with ethyl acetate (5 mL). The organic phases were combined, washed once with saturated brine (5

mL), dried over anhydrous sodium sulfate, and concentrated. The crude product was purified by column chromatography (dichloromethane: methanol = 10:1), followed by preparative separation (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 10 mg of the title compound **52.** LC-MS (ESI): m/z 580.2 $[M+H]^+$. $^1H$ NMR (400 MHz, DMSO-$d_6$) δ 8.79 (s, 2H), 7.70 (d, *J* = 2.3 Hz, 1H), 7.65 (d, *J* = 8.5 Hz, 2H), 7.64 (d, *J* = 2.4 Hz, 1H), 7.58 - 7.52 (m, 3H), 7.34 (d, *J* = 8.5 Hz, 2H), 7.21 - 7.18 (m, 1H), 4.42 (t, *J* = 5.2 Hz, 2H), 3.96 (t, *J* = 5.2 Hz, 2H), 1.75 (d, *J* = 13.8 Hz, 6H), 1.69 (s, 6H).

**Example 53: Preparation of compound 53**

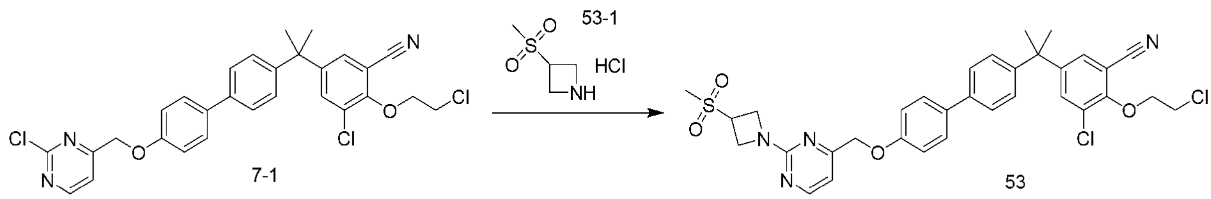


**Preparation of compound 53**

Compound **7-1** (100 mg, 180.87 μmol) and compound **53-1** (34.15 mg, 198.96 μmol) were dissolved in acetonitrile (2 mL), and triethylamine (54.91 mg, 542.62 μmol, 75.68 μL) was added. The reaction mixture was purged three times with nitrogen. The mixture was heated at 80°C for 2 h under microwave irradiation. LCMS indicated the disappearance of the starting material and the formation of the product. The reaction mixture was subjected to preparative purification (preparative method: chromatographic column: Pntulips ZZ-C18 10 μm 250 × 20 mm; column temperature: 25°C; mobile phase: water (0.1% FA)-acetonitrile; acetonitrile ratio in mobile phase: 50% to 70% over 8 min, 70% to 90% over 4 min, and 95% to 95% over 4 min; flow rate: 30 mL/min) to obtain 40 mg of the title compound **53.** LC-MS (ESI): m/z 651.2 $[M+H]^+$. $^1H$ NMR (400 MHz, DMSO-*d6*) δ 8.42 (d, *J* = 5.0 Hz, 1H), 7.71 (d, *J* = 2.4 Hz, 1H), 7.64 (d, *J* = 2.4 Hz, 1H), 7.60 (d, *J* = 8.8 Hz, 2H), 7.56 (d, *J* = 8.4 Hz, 2H), 7.30 (d, *J* = 8.0 Hz, 2H), 7.08 (d, *J* = 8.8 Hz, 2H), 6.86 (d, *J* = 5.0 Hz, 1H), 5.09 (s, 2H), 4.42 (t, *J* = 5.1 Hz, 2H), 4.41 - 4.39 (m, 1H), 4.34 (t, *J* = 8.4 Hz, 2H), 4.25 - 4.22 (m, 2H), 3.96 (t, *J* = 5.1 Hz, 2H), 3.07 (s, 3H), 1.68 (s, 6H).

**Example 54: Preparation of compound 54**

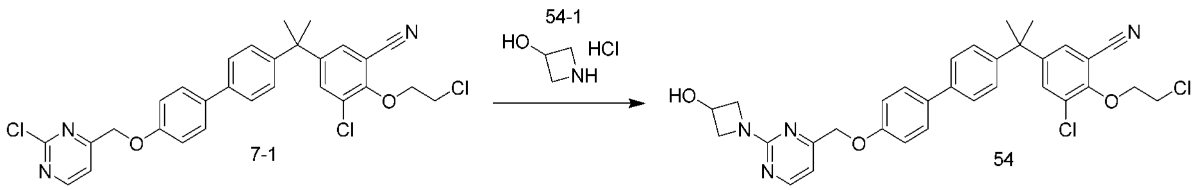


**Preparation of compound 54**

Compound **7-1** (100 mg, 180.87 μmol) and compound **54-1** (21.80 mg, 198.96 μmol) were dissolved in acetonitrile (2 mL), and triethylamine (54.91 mg, 542.62 μmol, 75.68 μL) was added. The reaction mixture was purged three times with nitrogen. The mixture was heated at 80°C for 2 h under microwave irradiation. LCMS indicated the disappearance of the starting material and the formation of the product. The reaction mixture was subjected to preparative purification (preparative method: mobile phase: A: 0.1% formic acid in water; B: acetonitrile; chromatographic column: Welch Ultimate Polar-RP 250 × 30 mm; column temperature: 25°C; gradient: acetonitrile from 70% to 90% over 8 min; flow rate: 50 mL/min) to obtain 54.9 mg of the title compound 54. LC-MS (ESI): m/z 589.2 $[M+H]^+$. $^1H$ NMR (400 MHz, DMSO-d6) δ 8.34 (d, *J* = 5.0, 1H), 7.70 (d, *J* = 2.3 Hz, 1H), 7.63 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.07 (d, *J* = 8.8 Hz, 2H), 6.74 (d, *J* = 5.0 Hz, 1H), 5.71 (s, 1H), 5.04 (s, 2H), 4.56 (s, 1H), 4.42 (t, *J* = 5.2 Hz, 2H), 4.24 (dd, *J* = 9.3, 6.6 Hz, 2H), 3.96 (t, *J* = 5.2 Hz, 2H), 3.79 (dd, *J* = 9.6, 4.5 Hz, 2H), 1.68 (s, 6H).

**Example 55: Preparation of compound 55**

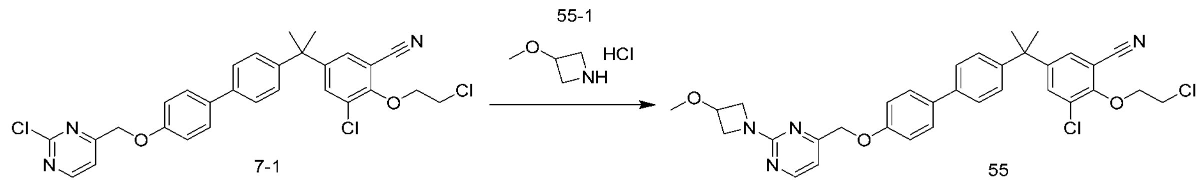

7-1
55

**Preparation of compound 55**

Compound **7-1** (100 mg, 180.87 μmol) and compound **55-1** (24.59 mg, 198.96 μmol) were weighed and dissolved in acetonitrile (3 mL), and triethylamine (54.91 mg, 542.62 μmol, 75.68 μL) was added. The reaction mixture was stirred at 80°C for 3 h. LCMS indicated the disappearance of the starting material and the formation of the product. The reaction mixture was subjected to preparative purification (mobile phase: A: 0.1% formic acid in water; B: acetonitrile; chromatographic column: Welch Ultimate Polar-RP 250 × 30 mm; column temperature: 25°C; gradient: 80% to 95% acetonitrile gradient elution over 8 min; flow rate: 50 mL/min) to obtain 65.7 mg of the title compound **55.** LC-MS (ESI): m/z 603.2 $[M+H]^+$. $^1$H NMR (400 MHz, DMSO-*d6*) 8.36 (d, *J* = 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.4 Hz, 2H), 7.30 (d, *J* = 8.4 Hz, 2H), 7.07 (d, *J* = 8.8 Hz, 2H), 6.77 (d, *J* = 5.0 Hz, 1H), 5.05 (s, 2H), 4.42 (t, *J* = 5.2 Hz, 2H), 4.34 - 4.29 (m, 1H), 4.25 - 4.21 (m, 2H), 3.96 (t, *J* = 5.2 Hz, 2H), 3.85 (dd, *J* = 10.0, 3.9 Hz, 2H), 3.25 (s, 3H), 1.68 (s, 6H).

**Example 56: Preparation of compound 56**

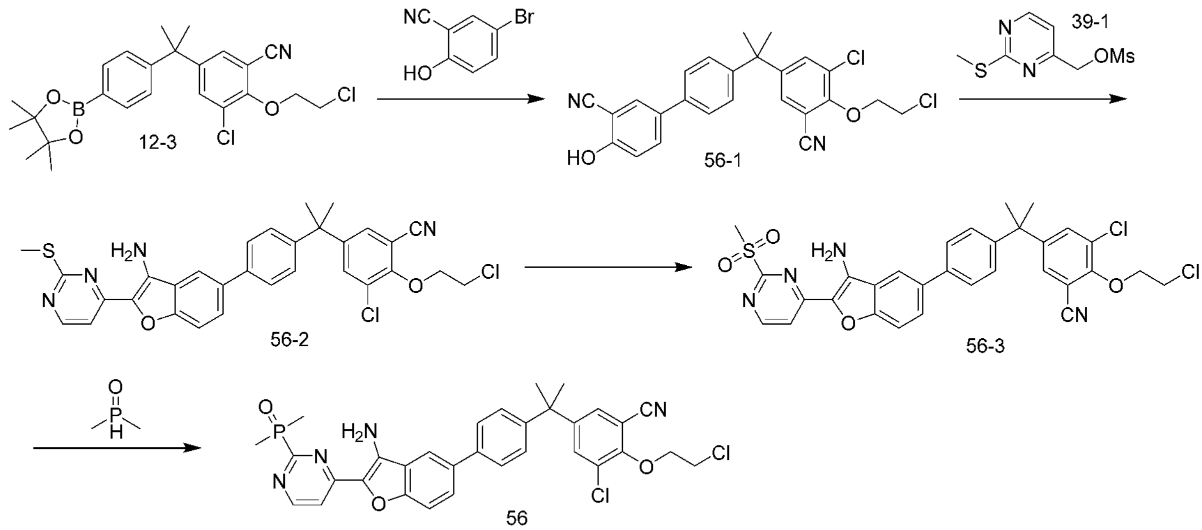

12-3
56-1
56-2
56-3
56

**Preparation of compound 56-1**

Compound **12-3** (1.30 g, 2.82 mmol) and 5-bromo-2-hydroxybenzonitrile (0.73 g, 3.67 mmol) were dissolved in 1,4-dioxane (13 mL). [1,1'-Bis(diphenylphosphino)ferrocene]dichloropalladium(II) (210 mg, 0.28 mmol) and an aqueous potassium carbonate solution (2 mol/L, 4.20 mL, 7.80 mmol) were then added. The system was purged four times with argon, heated to 110°C, and stirred for 4 h. After the reaction was completed, the reaction mixture was cooled to room temperature, acidified with an aqueous hydrochloric acid solution (2.0 M), and filtered through a pad of diatomite. The filter cake was rinsed with ethyl acetate. The filtrates were combined, washed once with saturated brine, dried over anhydrous sodium sulfate, and concentrated. The resulting residue was subjected to column chromatography (ethyl acetate/petroleum ether = 0 to 50%) to obtain 1.0 g of the title compound **56-1** in 72% yield. LC-MS (ESI): m/z $[M-H]^-$: 449.2.

## Preparation of compound 56-2

Compound **56-1** (800 mg, 1.77 mmol) and compound **39-1** (456.18 mg, 1.95 mmol) were dissolved in acetonitrile (16 mL). Cesium carbonate (1.15 g, 3.54 mmol) was added at room temperature, and the mixture was heated to 80°C and stirred for 2 h. LCMS indicated that the reaction was complete. The mixture was filtered, and the filter cake was rinsed with ethyl acetate. The filtrates were combined and evaporated to dryness under reduced pressure. The resulting residue was subjected to column chromatography (ethyl acetate/petroleum ether = 0 to 30%) to obtain 800 mg of the title compound **56-2** in 73.5% yield. LC-MS (ESI): m/z $[M+H]^+$: 589.1.

## Preparation of compound 56-3

Compound **56-2** (188 mg, 0.32 mmol) was dissolved in acetone (4.0 mL). Sodium tungstate (10 mg, 0.032 mmol) and hydrogen peroxide (218 mg, 1.92 mmol, 30 %) were added sequentially at room temperature. The mixture was heated to 40°C and stirred for 16 h. LCMS analysis indicated that the reaction was complete. At 0°C, an aqueous sodium sulfite solution (30 mL) was added, and the mixture was extracted three times with ethyl acetate (50 mL). The organic phases were combined, washed with saturated brine (50 mL), dried over anhydrous sodium sulfate, subjected to suction filtration, and concentrated to obtain 200 mg of a crude product of the title compound **56-3,** which was used directly in the next step without purification. LC-MS (ESI): m/z $[M+H]^+$: 620.8.

## Preparation of compound 56

Compound **56-3** (200 mg, 0.32 mmol) and dimethylphosphine oxide (125 mg, 1.60 mmol) were dissolved in acetonitrile (4.0 mL). Potassium carbonate (177 mg, 1.28 mmol) was added at room temperature, and the mixture was heated to 85°C and stirred for 2 h. LCMS analysis indicated approximately 2% product and 71% starting material. *N,N*-Dimethylacetamide (4.0 mL) was added. The mixture was stirred at 85°C overnight. The reaction was monitored by LCMS until completion. The mixture was cooled to room temperature, saturated brine (30 mL) was added, and the mixture was extracted three times with ethyl acetate (50 mL). The organic phases were combined, washed with saturated brine (50 mL), dried over anhydrous sodium sulfate, subjected to suction filtration, and the filtrate was concentrated. The resulting residue was subjected to column chromatography (methanol/dichloromethane = 0 to 10%) to obtain 120 mg of crude product. The crude product was subjected to preparative purification (prep-HPLC (Waters 2767/Qda, Column: XBridge XBridge C18 19 * 250 mm, 10 $\mu$m; mobile phase A: 0.03% $NH_3H_2O/H_2O$, mobile phase B: ACN; flow rate: 20 mL/min; gradient: 70% mobile phase B gradient elution over 16 min; target compound collection time: 8.0-9.2 min)) to obtain 18 mg of the title compound **56.** LC-MS (ESI): m/z 619.3 $[M+H]^+$. $^1H$ NMR (400 MHz, DMSO-*d6*) $\delta$ 8.75 (d, *J* = 5.7 Hz, 1H), 8.29 (d, *J* = 1.6 Hz, 1H), 7.78 (dd, *J* = 8.7, 1.9 Hz, 1H), 7.72 (d, *J* = 2.3 Hz, 1H), 7.66 (d, *J* = 8.5 Hz, 2H), 7.66 - 7.64 (m, 1H), 7.61 (d, *J* = 8.7 Hz, 1H), 7.53 (dd, *J* = 5.6, 3.3 Hz, 1H), 7.39 (d, *J* = 8.5 Hz, 2H), 6.87 (s, 2H), 4.43 (t, *J* = 5.2 Hz, 2H), 3.96 (t, *J* = 5.2 Hz, 2H), 1.80 (d, *J* = 13.6 Hz, 6H), 1.71 (s, 6H). $^{31}P$ NMR (162 MHz, DMSO-*d6*) $\delta$ 33.51 (s, 1P).

## Example 57: Preparation of compound 57

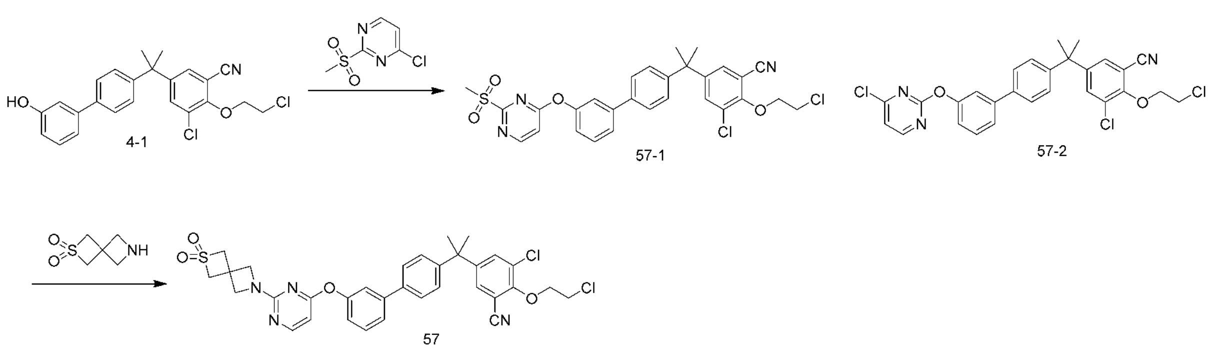

4-1

57-1

57-2

57

## Preparation of compounds 57-1 and 57-2

Compound **4-1** (255.80 mg, 0.60 mmol) was dissolved in acetonitrile (6 mL), followed by the sequential addition of 4-chloro-2-(methylsulfonyl)pyrimidine (115.57 mg, 0.60 mmol) and potassium carbonate (165.85 mg, 1.20 mmol). The reaction system was purged three times with nitrogen and stirred at 25°C for 5 h. After the reaction was completed, water

(10 mL) was added, and the mixture was extracted three times with dichloromethane (20 mL). The organic phases were combined, washed twice with saturated brine (20 mL), dried over anhydrous sodium sulfate, and concentrated to obtain a crude product. The crude product was purified by column chromatography (PE:EA = 30%) to obtain 180 mg of the title compound **57-1,** LC-MS (ESI): m/z 582.2 $[M+H]^+$, and 100 mg of the title compound **57-2,** LC-MS (ESI): m/z 538.2 $[M+H]^+$.

**Preparation of compound 57**

Compound **57-1** (30.5 mg, 52.43 μmol) was dissolved in DMF (2 mL), followed by the sequential addition of $Cs_2CO_3$ (34.2 mg, 104.87 μmol) and 2-thia-6-azaspiro[3.3]heptane 2,2-dioxide hydrochloride (10.59 mg, 57.68 μmol). The reaction system was stirred at 25°C for 2 h. After the reaction was completed, insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5.5 mg of the title compound **57.** LC-MS (ESI): m/z 649.2 $[M+H]^+$. $^1$H NMR (400 MHz, $CDCl_3$) δ 8.18 (d, *J* = 5.6 Hz, 1H), 7.45 - 7.54 (m, 5H), 7.34 - 7.35 (m, 2H), 7.25 - 7.27 (m, 2H), 7.09 - 7.12 (m, 1H), 6.20 (d, *J* = 5.6 Hz, 1H), 4.43 (t, *J* = 6.4 Hz, 2H), 4.33 - 4.35 (m, 8H), 3.88 (t, *J* = 6.4 Hz, 2H), 1.70 (s, 6H).

**Example 58: Preparation of compound 58**

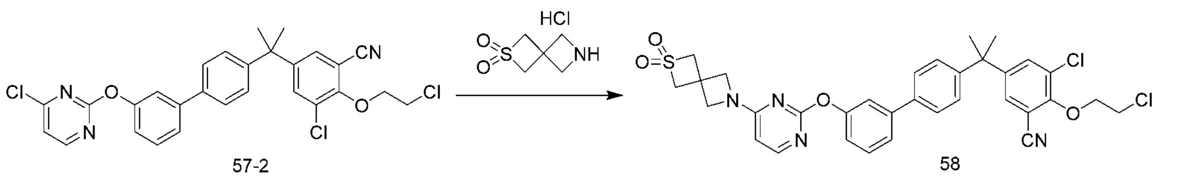


57-2

58

**Preparation of compound 58**

Compound **57-2** (28.25 mg, 52.43 μmol) was dissolved in DMF (2 mL), followed by the sequential addition of $Cs_2CO_3$ (34.17 mg, 104.87 μmol) and 2-thia-6-azaspiro[3.3]heptane 2,2-dioxide hydrochloride (10.59 mg, 57.68 μmol). The reaction system was stirred at 25°C for 2 h. After the reaction was completed, insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 8 mg of the title compound **58.** LC-MS (ESI): m/z 649.2 $[M+H]^+$. $^1$H NMR (400 MHz, $CDCl_3$) δ 8.07 (br.s, 1H), 7.52 (d, *J* = 8.0 Hz, 2H), 7.48 (d, *J* = 2.4 Hz, 1H), 7.45 - 7.46 (m, 2H), 7.38 (s, 1H), 7.36 (d, *J* = 2.4 Hz, 1H), 7.24 (d, *J* = 7.6 Hz, 2H), 7.15 (s, 1H), 6.01 (br.s, 1H), 4.42 (t, *J* = 5.2 Hz, 2H), 4.35 - 4.39 (m, 8H), 3.88 (t, *J* = 5.2 Hz, 2H), 1.69 (s, 6H).

**Example 59: Preparation of compound 59**

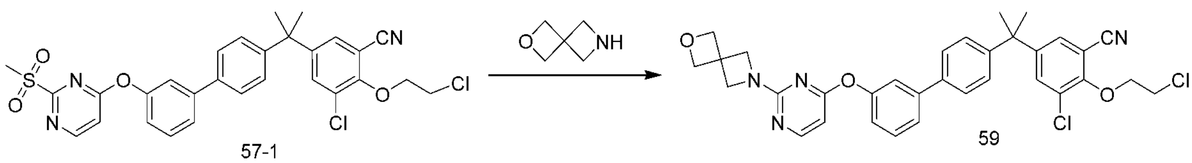

57-1

59

**Preparation of compound 59**

Compound **57-1** (30.54 mg, 52.43 μmol) was dissolved in DMF (2 mL), followed by the sequential addition of $Cs_2CO_3$ (34.17 mg, 104.87 μmol) and 2-oxa-6-azaspiro[3.3]heptane (7.82 mg, 57.68 μmol). The reaction system was stirred at 25°C for 2 h. After the reaction was completed, insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 7.5 mg of the title compound **59.** LC-MS (ESI): m/z

601.2 [M+H]+. $^1$H NMR (400 MHz, $CDCl_3$) δ 8.15 (d, *J* = 6.0 Hz, 1H), 7.53 (d, *J* = 8.0 Hz, 2H), 7.45 - 7.48 (m, 3H), 7.35 - 7.36 (m, 2H), 7.25 - 7.27 (m, 2H), 7.10 - 7.13 (m, 1H), 6.09 (d, *J* = 5.6, 1H), 4.82 (s, 4H), 4.43 (t, *J* = 5.6 Hz, 2H), 4.27 (s, 4H), 3.88 (t, *J* = 5.6 Hz, 2H), 1.70 (s, 6H).

**Example 60: Preparation of compound 60**

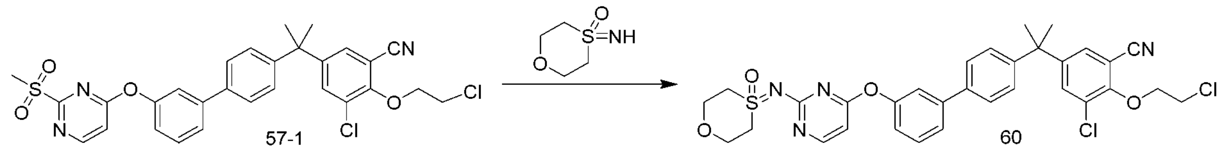

**Preparation of compound 60**

Compound **57-1** (58.3 mg, 100.09 μmol) was dissolved in acetonitrile (2 mL), followed by the sequential addition of $Cs_2CO_3$ (65.22 mg, 200.17 μmol) and 1,4-oxathiane sulfoximine (27.06 mg, 200.17 μmol). The reaction system was stirred under microwave irradiation at 120°C for 1 h. After the reaction was completed, insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 3.5 mg of the title compound **60.** LC-MS (ESI): m/z 637.2 [M+H]+. $^1$H NMR (400 MHz, $CDCl_3$) δ 8.35 (d, *J* = 3.6 Hz, 1H), 7.54 (d, *J* = 8.0 Hz, 2H), 7.50 - 7.45 (m, 3H), 7.36 - 7.35 (m, 2H), 7.26 - 7.24 (m, 2H), 7.13 (d, *J* = 6.6 Hz, 1H), 6.46 (d, *J* = 5.7 Hz, 1H), 4.43 (t, *J* = 6.2 Hz, 2H), 4.03 - 3.95 (m, 4H), 3.88 (t, *J* = 6.2 Hz, 2H), 3.55 - 3.48 (m, 2H), 3.23 - 3.13 (m, 2H), 1.69 (s, 6H).

**Example 61: Preparation of compound 61**

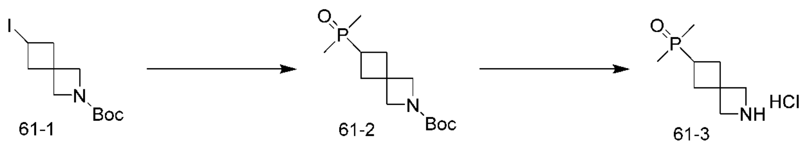

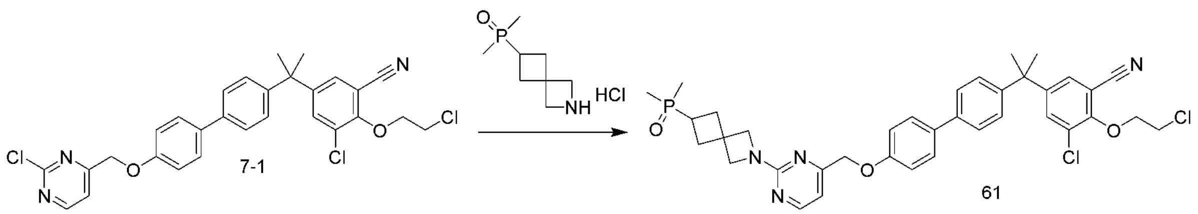

**Preparation of compound 61-2**

Under a nitrogen atmosphere, dimethylphosphine oxide (966 mg, 12.38 mmol) was dissolved in THF (20 mL), and NaHMDS (2 M, 12.38 mmol, 6.2 mL) was added dropwise at 0°C, then the mixture was slowly warmed to room temperature and stirred for 1 h. After the system was cooled to 0°C, a solution of **61-1** (2 g, 6.19 mmol) in THF (5 mL) was added dropwise, then the mixture was slowly warmed to room temperature and stirred for 48 h. The system was directly subjected to rotary evaporation until dryness. Ethyl acetate (20 mL) was added, and the mixture was stirred for 1 h, filtered, and the filtrate was subjected to rotary evaporation until dryness. The crude product was subjected to column chromatography (EA/PE = 0 to 10%) to obtain 580 mg of the target compound **61-2** in 34% yield. LC-MS (ESI): m/z 218.2 [M-56+H]+.

**Preparation of compound 61-3**

At 0°C, compound **61-2** (200 mg, 731.78 μmol) was dissolved in DCM (4 mL), and a solution of HCl in 1,4-dioxane (1 mL, 4 M) was added dropwise. The mixture was stirred for 2 h. The supernatant was discarded, and the residue was

subjected to rotary evaporation until dryness to obtain 100 mg of the target compound **61-3** in 78% yield. The product was used directly in the next step without purification.

**Preparation of compound 61**

Compound **7-1**(100 mg, 180.87 μmol) was added to a reaction flask, followed by the addition of acetonitrile (1 mL). **61-3**(47 mg, 271.31 μmol) and DIPEA (1 mL) were added at room temperature. The reaction was carried out under microwave irradiation at 80°C for 2 h. The reaction was monitored by LCMS until completion. The reaction mixture was cooled to room temperature, water (30 mL) was added, and the mixture was extracted three times with EA (30 mL). The organic phases were combined, dried over anhydrous sodium sulfate, subjected to suction filtration, and the filtrate was evaporated to dryness under reduced pressure. The resulting crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 10% to 60% acetonitrile gradient elution over 30 min; flow rate: 30 mL/min) to obtain 50 mg of the title compound **61.** LC-MS (ESI): m/z 689.4 $[M+H]^+$. $^1H$ NMR (400 MHz, DMSO-$d_6$) δ 8.33 (d, *J* = 5.0 Hz, 1H), 7.70 (d, *J* = 2.4 Hz, 1H), 7.63 (d, *J* = 2.4 Hz, 1H), 7.59 (d, *J* = 8.8 Hz, 2H), 7.55 (d, *J* = 8.5 Hz, 2H), 7.30 (d, *J* = 8.5 Hz, 2H), 7.06 (d, *J* = 8.8 Hz, 2H), 6.74 (d, *J* = 5.0 Hz, 1H), 5.03 (s, 2H), 4.42 (t, *J* = 5.6 Hz, 2H), 4.09 (s, 2H), 3.98 - 3.94 (m, 4H), 2.49 - 2.33 (m, 5H), 1.68 (s, 6H), 1.29 (d, *J* = 12.8 Hz, 6H). $^{31}P$ NMR (162 MHz, DMSO-$d_6$) δ 41.04 (s, 1P).

**Example 62: Preparation of compound 62**

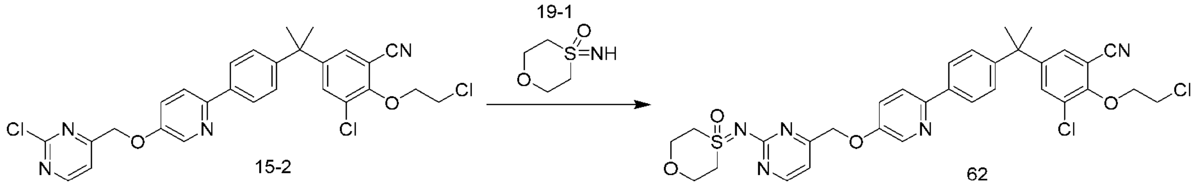


15-2

62

**Preparation of compound 62**

Compound **15-2** (100 mg, 180.55 μmol) was dissolved in 1,4-dioxane (1 mL), followed by the sequential addition of **19-1** (36.61 mg, 270.82 μmol), $Pd_2(dba)_3$ (16.53 mg, 18.05 μmol), DIEA (24 mg, 0.07 mmol), and Xantphos (20.89 mg, 36.11 μmol). The system was purged three times with nitrogen and stirred at 100°C for 2 h. After the reaction was completed, insoluble solids were removed by filtration. Water (10 mL) was added to the filtrate, and the mixture was extracted three times with ethyl acetate (10 mL). The organic phases were combined, washed twice with saturated brine (10 mL), dried over anhydrous sodium sulfate, and concentrated to obtain a crude product. The crude product was purified by column chromatography (petroleum ether: ethyl acetate = 1:1) to obtain a crude product. The crude product was further subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound 62. LC-MS (ESI): m/z 652.3 $[M+H]^+$. $^1H$ NMR (400 MHz, DMSO-$d_6$) δ 8.48 (d, *J* = 5.1 Hz, 1H), 8.44 (d, *J* = 3.0 Hz, 1H), 7.94 (d, *J* = 8.1 Hz, 2H), 7.89 (d, *J* = 8.8 Hz, 1H), 7.69 (d, *J* = 2.4 Hz, 1H), 7.62 (d, *J* = 2.4 Hz, 1H), 7.52 (dd, *J* = 8.8, 3.0 Hz, 1H), 7.33 (d, *J* = 8.1 Hz, 2H), 7.03 (d, *J* = 5.1 Hz, 1H), 5.20 (s, 2H), 4.42 (t, *J* = 5.2 Hz, 2H), 4.10 - 4.04 (m, 2H), 3.95 (t, *J* = 5.1 Hz, 2H), 3.93 - 3.89 (m, 2H), 3.81 - 3.76 (m, 2H), 3.60 - 3.54 (m, 2H), 1.69 (s, 6H).

**Example 63: Preparation of compound 63**

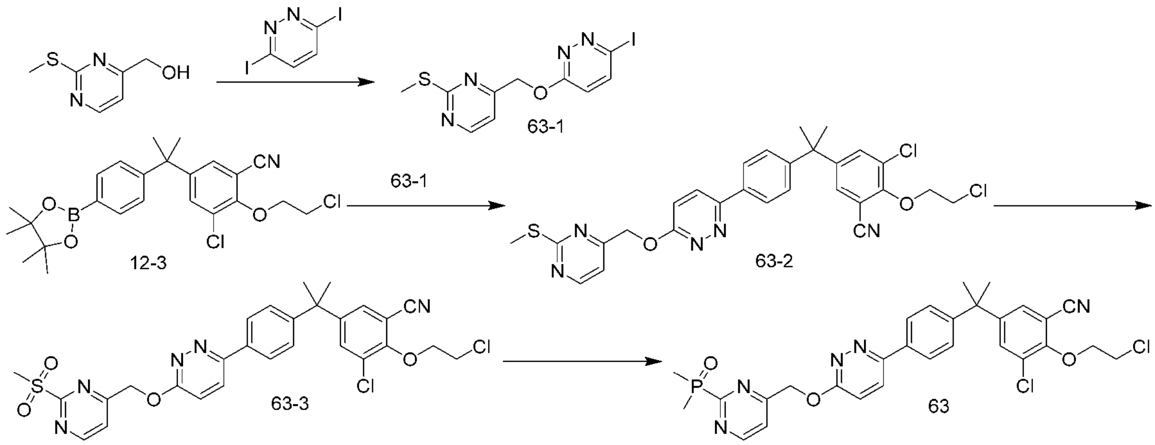


**Preparation of compound 63-1**

Compound (2-(methylthio)pyrimidin-4-yl)methanol (800 mg, 5.12 mmol) and 3,6-diiodopyridazine (1.7 g, 5.12 mmol) were dissolved in anhydrous tetrahydrofuran (10 mL). The reaction system was cooled to 0°C, and sodium hydride (410 mg, 10.24 mmol) was added in portions. Upon completion, the reaction system was stirred at 25°C for 5 h. After the reaction was completed, the reaction system was quenched with saturated aqueous ammonium chloride solution (20 mL) and extracted three times with ethyl acetate (30 mL). The organic phases were combined, washed twice with saturated brine (20 mL), dried over anhydrous sodium sulfate, subjected to suction filtration, and the filtrate was concentrated to obtain a crude product. The crude product was purified by column chromatography (EA/PE = 0 to 20%) to obtain 550 mg of the title compound **63-1** in 29.8% yield. LC-MS (ESI): m/z 361.0 $[M+H]^+$.

**Preparation of compound 63-2**

Compound **63-1** (200 mg, 0.555 mmol) was dissolved in 1,4-dioxane (10 mL) and water (3 mL), followed by the sequential addition of compound **12-3** (255 mg, 0.555 mmol), potassium carbonate (230 mg, 1.67 mmol), and Pd(dppf)$Cl_2$ (40 mg, 0.055 mmol). The reaction system was purged three times with nitrogen and stirred at 90°C for 5 h. After the reaction was completed, water (30 mL) was added, and the mixture was extracted three times with ethyl acetate (20 mL). The organic phases were combined, washed twice with saturated brine (20 mL), dried over anhydrous sodium sulfate, and subjected to suction filtration, and the filtrate was concentrated to obtain a crude product. The crude product was purified by column chromatography (EA/PE = 0 to 20%) to obtain 200 mg of the title compound **63-2** in 63% yield. LC-MS (ESI): m/z 566.2 $[M+H]^+$.

**Preparation of compound 63-3**

Compound **63-2** (119 mg, 0.211 mmol) was dissolved in dichloromethane (10 mL), and m-chloroperoxybenzoic acid (108 mg, 0.633 mmol) was added in portions at room temperature. The reaction system was stirred at room temperature for 2 h. After the reaction was completed, the reaction system was quenched with saturated aqueous sodium sulfite solution (10 mL) and extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by column chromatography (EA/PE = 0 to 60%) to obtain 80 mg of the title compound **63-3** in 63% yield. LC-MS (ESI): m/z 598.2 $[M+H]^+$.

**Preparation of compound 63**

Compound **63-3** (35 mg, 0.035 mmol) was dissolved in acetonitrile (2 mL), followed by the sequential addition of potassium carbonate (15 mg, 0.105 mmol) and dimethylphosphine oxide (8 mg, 0.105 mmol). The reaction system was stirred at 80°C for 1 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5 mg of the title compound **63.** LC-MS (ESI): m/z 596.2 $[M+H]^+$. $^1H$ NMR (400 MHz, Chloroform-d) δ 8.86 (s, 1H), 7.95 (d, *J* = 8.2 Hz, 2H), 7.88 (d, *J* = 9.1 Hz, 1H), 7.54 (s, 1H), 7.46 (d, *J* = 2.3 Hz, 1H), 7.36 (d, *J* = 2.3 Hz, 1H), 7.32 (d, *J* = 8.2

Hz, 2H), 7.24 (s, 1H), 5.81 (s, 2H), 4.43 (t, *J* = 6.1 Hz, 2H), 3.87 (t, *J* = 6.1 Hz, 2H), 1.88 (d, *J* = 13.5 Hz, 6H), 1.70 (s, 6H). $^{31}P$ NMR (162 MHz, Chloroform-d) δ 35.44.

**Example 64: Preparation of compound 64**

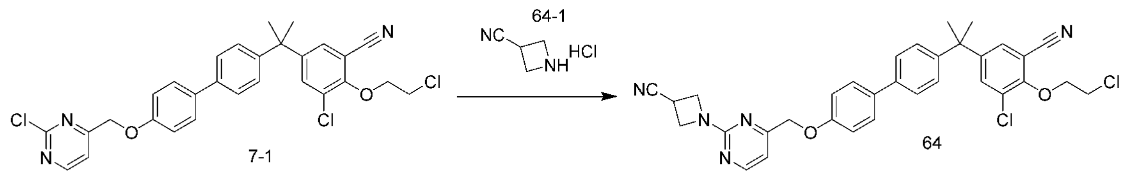


7-1 64

**Preparation of compound 64**

Compound **7-1** (100 mg, 180.87 μmol) and compound **64-1** (23.59 mg, 198.96 μmol) were weighed and dissolved in acetonitrile (2 mL), and triethylamine (54.91 mg, 542.62 μmol, 75.68 μL) was added. The reaction mixture was purged three times with nitrogen. The mixture was heated at 80°C for 2 h under microwave irradiation. LCMS indicated the disappearance of the starting material and the formation of the product. The reaction mixture was subjected to preparative purification to obtain 60.5 mg of the title compound **64.** LC-MS (ESI): m/z 598.2 $[M+H]^+$. $^1H$ NMR (400 MHz, DMSO-$d_6$) δ 8.41 (d, *J* = 5.0 Hz, 1H), 7.70 (d, *J* = 2.4 Hz, 1H), 7.64 (d, *J* = 2.3 Hz, 1H), 7.60 (d, *J* = 8.8 Hz, 2H), 7.56 (d, *J* = 8.4Hz, 2H), 7.30 (d, *J* = 8.4 Hz, 2H), 7.08 (d, *J* = 8.8 Hz, 2H), 6.87 (d, *J* = 5.0 Hz, 1H), 5.08 (s, 2H), 4.42 (t, *J* = 5.2 Hz, 2H), 4.34 (t, *J* = 8.8 Hz, 2H), 4.19 (dd, *J* = 8.6, 5.8 Hz, 2H), 3.96 (t, *J* = 5.2 Hz, 2H), 3.90 - 3.85 (m, 1H), 1.68 (s, 6H).

**Example 65: Preparation of compound 65**

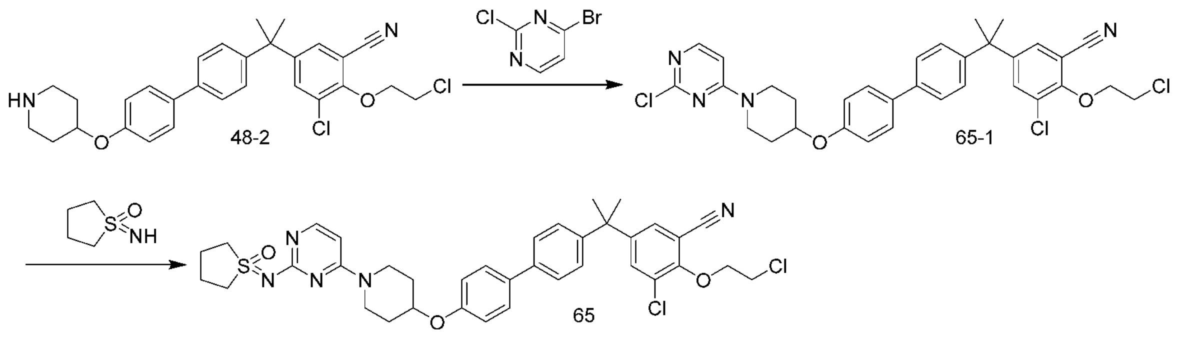

48-2 65-1 65

**Preparation of compound 65-1**

Compound **48-2** (200 mg, 0.39 mmol) was dissolved in methanol (10 mL), followed by the sequential addition of compound 2-chloro-4-bromopyrimidine (90 mg, 0.47 mmol) and triethylamine (79 mg, 0.78 mmol). The reaction system was stirred at 25°C for 1 h. After the reaction was completed, water (10 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by column chromatography (PE:EA = 60%) to obtain 200 mg of the title compound **65-1.** LC-MS (ESI): m/z 621.2 $[M+H]^+$.

**Preparation of compound 65**

Compound **65-1** (100 mg, 0.16 mmol) was dissolved in DMF (5 mL), followed by the sequential addition of 1-imino-1-oxothiolane (57 mg, 0.48 mmol), DIPEA(62 mg, 0.48 mmol), tris(dibenzylideneacetone)dipalladium (18 mg, 0.02 mmol), and 4,5-bis(diphenylphosphino)-9,9-dimethylxanthene (27 mg, 0.05 mmol). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined,

washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 23.6 mg of the title compound **65.** LC-MS (ESI): m/z 704.3 $[M+H]^+$. $^1H$ NMR (400 MHz, Chloroform-d) δ 8.04 (d, *J* = 6.6 Hz, 1H), 7.54 - 7.45 (m, 5H), 7.37 (d, *J* = 2.3 Hz, 1H), 7.22 (d, *J* = 8.4 Hz, 2H), 6.99 (d, *J* = 8.7 Hz, 2H), 6.20 (d, *J* = 6.6 Hz, 1H), 4.68 - 4.63 (m, 1H), 4.43 (t, *J* = 6.2 Hz, 2H), 3.94 - 3.84 (m, 2H), 3.88 *(t, J* = 6.2 Hz, 2H), 3.83 - 3.66 (m, 4H), 3.42 - 3.30 (m, 2H), 2.37 - 2.22 (m, 4H), 2.04 - 1.89 (m, 4H), 1.69 (s, 6H).

**Example 66 and Example 67: Preparation of compounds 66 and 67**

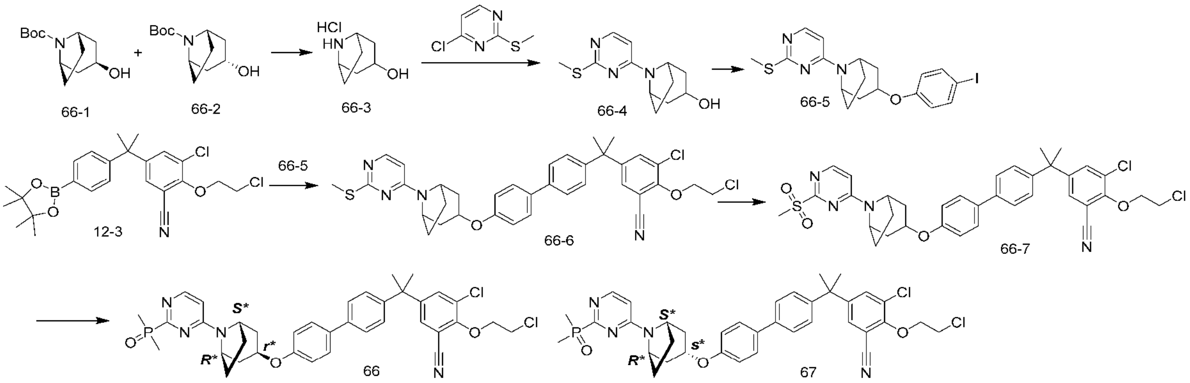


**Preparation of compound 66-3**

Under a nitrogen atmosphere at 0°C, compound **66-1** (0.5 g, 2.20 mmol) and compound **66-2** (0.5 g, 2.20 mmol) were dissolved in DCM (10 mL), and a solution of HCl in 1,4-dioxane (5 mL, 4 M) was slowly added dropwise. The mixture was stirred at 0°C for 2 h. The supernatant was discarded, and the residue was subjected to rotary evaporation until dryness to obtain 560 mg of a crude product of the title compound **66-3,** which was used directly in the next step.

**Preparation of compound 66-4**

Compound **66-3** (705.98 mg, 4.40 mmol), 4-chloro-2-(methylthio)pyrimidine (560 mg, 4.40 mmol), triethylamine (4.45 g, 43.95 mmol, 6.13 mL)and acetonitrile (10 mL)were added to a reaction flask, and the mixture was stirred at 80°C for 16 h. The reaction mixture was subjected to rotary evaporation until dryness. The crude product was purified by column chromatography (EA/PE = 100%) to obtain 800 mg of the title compound **66-4** in 72% yield. LC-MS (ESI): m/z 252.2.

**Preparation of compound 66-5**

Compound 4-iodophenol (500 mg, 2.27 mmol), compound **66-4** (571 mg, 2.27 mmol), CMBP (cyanomethylene(tri-n-butyl)phosphorane, 1.1 g, 4.55 mmol), and toluene (5 mL) were added to a reaction flask. The mixture was stirred at 110°C for 2 h under a nitrogen atmosphere. The reaction mixture was subjected to rotary evaporation until dryness. The crude product was purified by column chromatography (EA/PE = 50%) to obtain 900 mg of the title compound **66-5** in 87% yield. LC-MS (ESI): m/z 454.0.

**Preparation of compound 66-6**

Under an air atmosphere, compound **66-5** (500 mg, 1.10 mmol), compound **12-3** (507.57 mg, 1.10 mmol), $K_2CO_3$ (457.29 mg, 3.31 mmol), $Pd(dppf)Cl_2$ (161.40 mg, 220.59 μmol), 1,4-dioxane (5 mL), and water (1 mL) were added to a reaction flask, and the mixture was stirred at 100°C for 16 h. The mixture was subjected to rotary evaporation until dryness, and the crude product was purified by column chromatography (EA/PE = 100%) to obtain 350 mg of the title compound **66-6** in 48% yield. LC-MS (ESI): m/z 659.5.

**Preparation of compound 66-7**

Compound **66-6** (350 mg, 530.57 μmol) was dissolved in DCM (3 mL). Under a nitrogen atmosphere, *m*-CPBA (274.7 mg, 1.59 mmol) was added in portions at 0°C, and the reaction was carried out at room temperature for 2 h. The reaction was quenched by adding saturated aqueous sodium thiosulfate solution (5 mL) to the reaction system, and the mixture was extracted three times with DCM (5 mL). The organic phases were combined, washed three times with saturated aqueous sodium carbonate solution (5 mL), dried over anhydrous sodium sulfate, and subjected to rotary evaporation until dryness to obtain 300 mg of a crude product of the title compound **66-7** in 81% yield. LC-MS (ESI): m/z 691.3.

**Preparation of compound 66 and compound 67**

Compound **66-7** (300 mg, 433.74 μmol), $Cs_2CO_3$ (424 mg, 1.30 mmol), dimethylphosphine oxide (80.74 mg, 1.30 mmol), and acetonitrile (3 mL) were added to a reaction flask, and the mixture was stirred at 80°C for 2 h. The mixture was filtered and subjected to rotary evaporation until dryness. The crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 10% to 70% acetonitrile gradient elution over 30 min; flow rate: 30 mL/min) to obtain 20 mg of title compound **66** and 20 mg of title compound **67.**

**Compound 66:** HPLC analysis method (chromatographic column: Agilent ZORBAX Extend-C18 4.6 * 150 mm, 3.5 μm; column temperature: 30°C; mobile phase: water (0.1 mL/1 L trifluoroacetic acid)-acetonitrile (0.4 mL/4 L trifluoroacetic acid); the content of acetonitrile in the mobile phase was gradually increased from 5% to 95% over 8 min, and the column was flushed with 95% acetonitrile phase for 7 min; flow rate: 1 mL/min), retention time RT = 8.194 min. LC-MS (ESI): m/z 689.3 $[M+H]^+$. $^1H$ NMR (400 MHz, DMSO-$d_6$) δ 8.33 (d, *J* = 6.1 Hz, 1H), 7.70 (d, *J* = 2.3 Hz, 1H), 7.63 (d, *J*= 2.3 Hz, 1H), 7.54 (d, *J* = 8.8 Hz, 2H), 7.53 (d, *J* = 8.3 Hz, 2H), 7.29 (d, *J* = 8.3 Hz, 2H), 7.04 (d, *J* = 8.8 Hz, 2H), 6.88 (dd, *J* = 6.2, 3.2 Hz, 1H), 5.09 - 4.96 (m, 1H), 4.92 (brs, 1H), 4.55 (brs, 1H), 4.42 (t, *J* = 5.2 Hz, 2H), 3.95 (t, *J* = 5.2 Hz, 2H), 2.24 - 2.13 (m, 2H), 2.02 (brs 4H), 1.68 (d, *J* = 13.5 Hz, 6H), 1.67 (s, 6H), 1.62 - 1.46 (m, 2H). $^{31}P$ NMR (162 MHz, DMSO-$d_6$) δ 33.95(s, 1P).

**Compound 67:** HPLC analysis method (chromatographic column: Agilent ZORBAX Extend-C18 4.6 * 150 mm, 3.5 μm; column temperature: 30°C; mobile phase: water (containing 0.1 mL/1 L trifluoroacetic acid) - acetonitrile (containing 0.4 mL/4 L trifluoroacetic acid); the content of acetonitrile in the mobile phase was gradually increased from 5% to 95% over 8 min, and the column was flushed with 95% acetonitrile phase for 7 min; flow rate: 1 mL/min), retention time RT = 8.522 min. LC-MS (ESI): m/z 689.3 $[M+H]^+$. $^1H$ NMR (400 MHz, DMSO-$d_6$) δ 8.30 (d, *J* = 6.2 Hz, 1H), 7.70 (d, *J* = 2.3 Hz, 1H), 7.64 (d, *J* = 2.3 Hz, 1H), 7.59 (d, *J* = 8.6 Hz, 2H), 7.55 (d, *J* = 8.4 Hz, 2H), 7.30 (d, *J* = 8.4 Hz, 2H), 6.98 (d, *J* = 8.6 Hz, 2H), 6.84 (dd, *J* = 6.2, 3.2 Hz, 1H), 4.83 (brs, 1H), 4.70 (t, *J* = 4.9 Hz, 1H), 4.50 (brs, 1H), 4.42 (t, *J* = 5.2 Hz, 2H), 3.95 (t, *J* = 5.2 Hz, 2H), 2.23 (d, *J* = 7.1 Hz, 2H), 2.10 - 1.97 (m, 4H), 1.94 (d, *J* = 14.8 Hz, 2H), 1.68 (s, 6H), 1.67 (d, *J* = 13.6 Hz, 6H). $^{31}P$ NMR (162 MHz, DMSO-$d_6$) δ 33.67(s, 1P).

**Example 68: Preparation of compound 68**

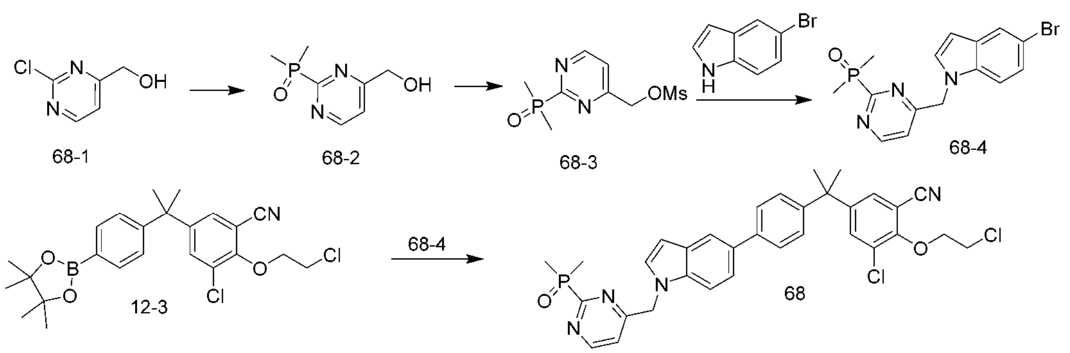


68-1 68-2 68-3 68-4 12-3 68

**Preparation of compound 68-2**

Under a nitrogen atmosphere, compound **68-1** (100 mg, 691.76 μmol), dimethylphosphine oxide (80.99 mg, 1.04 mmol), DIEA (268 mg, 2.08 mmol, 361.48 μL), Xtantphos (80.05 mg, 138.35 μmol), $Pd_2(dba)_3$ (63.35 mg, 69.18 μmol),

and DMF (2 mL) were added to a microwave tube, and the mixture was stirred at 140°C for 2 h. The mixture was filtered, and the filtrate was subjected to rotary evaporation until dryness. The crude product was purified by column chromatography (MeOH/DCM = 0 to 20%) to obtain 100 mg of title compound **68-2** in 77% yield. LC-MS (ESI): m/z 187.2.

**Preparation of compound 68-3**

Under a nitrogen atmosphere, compound **68-2** (500 mg, 2.69 mmol) and triethylamine (543.6 mg, 5.37 mmol, 749.28 μL) were dissolved in DCM (11 mL). Methanesulfonyl chloride (369.23 mg, 3.22 mmol) was added dropwise at 0°C, and the mixture was stirred for 2 h. After the reaction was completed, the reaction was quenched by adding water (10 mL) and extracted three times with DCM (10 mL). The organic phases were combined, subjected to rotary evaporation until dryness, and purified by column chromatography (EA/PE = 0 to 15%) to obtain 660 mg of the title compound **68-3** in 92% yield. LC-MS (ESI): m/z 265.0.

**Preparation of compound 68-4**

Compound 5-bromoindole (323.48 mg, 1.22 mmol) and DMF (4 mL) were added to a reaction flask, and NaH (53.04 mg, 1.33 mmol, 60% purity) was added at 0°C. After stirring for 20 min, a solution of compound **68-3** (200 mg, 1.02 mmol) in DMF (1 mL) was added dropwise. The mixture was stirred at 0°C for 2 h. The reaction was quenched by adding water (10 mL), extracted three times with DCM (10 mL), and the organic phases were combined and subjected to rotary evaporation until dryness. The crude product was purified by column chromatography (MeOH/DCM = 0 to 5%) to obtain 90 mg of the title compound **68-4** in 24% yield. LC-MS (ESI): m/z 364.0.

**Preparation of compound 68**

Under a nitrogen atmosphere, compound **12-3** (101 mg, 219.67 μmol), compound **68-4** (80 mg, 219.67 μmol), $K_2CO_3$ (91.1 mg, 659.02 μmol), Pd(dppf)$Cl_2$ (32 mg, 43.93 μmol), 1,4-dioxane (0.5 mL), and $H_2O$ (0.2 mL) were added to a reaction flask, and the mixture was stirred at 100°C for 16 h. The mixture was filtered and subjected to rotary evaporation until dryness. The resulting crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 5% to 75% acetonitrile gradient elution over 30 min; flow rate: 30 mL/min) to obtain 20 mg of title compound **68.** LC-MS (ESI): m/z 617.2 $[M+H]^+$. $^1$H NMR (400 MHz, DMSO-$d_6$) δ 8.84 (d, *J* = 5.2 Hz, 1H), 7.85 (d, *J* = 1.7 Hz, 1H), 7.72 (d, *J* = 2.4 Hz, 1H), 7.65 (d, *J* = 2.3 Hz, 1H), 7.60 (d, *J* = 8.1 Hz, 2H), 7.56 (d, *J* = 3.1 Hz, 1H), 7.49 (d, *J* = 8.6 Hz, 1H), 7.41 (dd, *J* = 8.6, 1.7 Hz, 1H), 7.31 (d, *J* = 8.1 Hz, 2H), 6.96 (dd, *J* = 5.2, 3.1 Hz, 1H), 6.61 (d, *J* = 3.1 Hz, 1H), 5.67 (s, 2H), 4.42 (t, *J* = 5.2 Hz, 2H), 3.96 (t, *J* = 5.2 Hz, 2H), 1.72 (d, *J* = 13.7 Hz, 6H), 1.69 (s, 6H). $^{31}$P NMR (162 MHz, DMSO-$d_6$) δ 34.20 (s, 1P).

**Example 69: Preparation of compound 69**

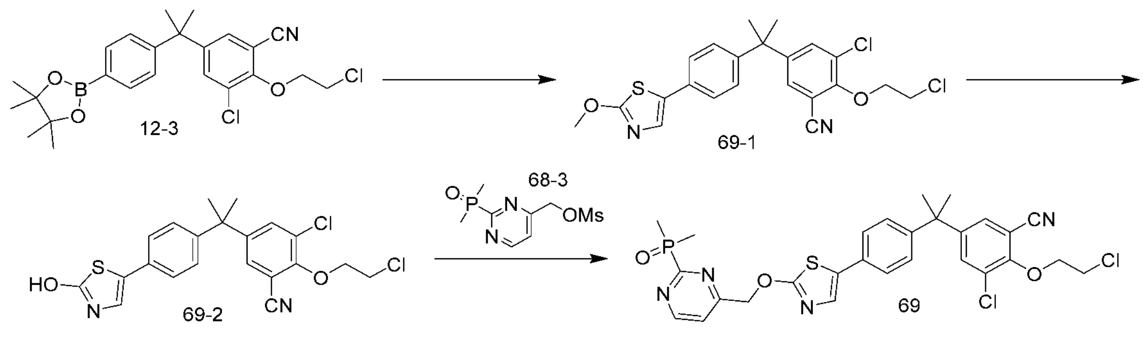

**Preparation of compound 69-1**

Compound **12-3** (500 mg, 1.09 mmol) was dissolved in a solution of 1,4-dioxane (5.0 mL) and water (2.0 mL), followed by the sequential addition of [1,1'-bis(diphenylphosphino)ferrocene]dichloropalladium(II) dichloromethane complex (199 mg, 0.21 mmol), 5-bromo-2-methoxythiazole (317 mg, 1.63 mmol), and potassium carbonate (300 mg, 2.18 mmol). The reaction system was stirred at 100°C for 2 h. After the reaction was completed, water (10 mL) was added

to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was purified by column chromatography (EA/PE = 0 to 30%) to obtain 300 mg of the title compound **69-1.** LC-MS (ESI): m/z 447.2 $[M+H]^+$.

**Preparation of compound 69-2**

Compound **69-1** (300 mg, 0.67 mmol) was dissolved in 1,4-dioxane (3 mL). The reaction mixture was cooled to 0°C, and dioxane hydrochloride (4 mol/L, 1.7 mL, 6.70 mmol) was added. The reaction system was stirred at room temperature for 2 h. After the reaction was completed, water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was purified by column chromatography (EA/PE = 0 to 50%) to obtain 100 mg of the title compound **69-2.** LC-MS (ESI): m/z 433.0 $[M+H]^+$.

**Preparation of compound 69**

Compound **69-2** (100 mg, 0.23 mmol) was dissolved in acetonitrile (5 mL), followed by the sequential addition of **68-3** (73 mg, 0.28 mmol) and cesium carbonate (150 mg, 0.46 mmol). The reaction system was stirred at 80°C for 2 h. After the reaction was completed, water (10 mL) was added to the filtrate, and the mixture was extracted three times with dichloromethane (20 mL). The organic phases were combined, washed twice with saturated brine (20 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 3 mg of the title compound **69.** LC-MS (ESI): m/z 601.2 $[M+H]^+$. $^1H$ NMR (400 MHz, DMSO-$d_6$) δ 8.95 (d, *J* = 5.3 Hz, 1H), 7.68 (s, 1H), 7.67 (d, *J* = 2.3 Hz, 1H), 7.60 (d, *J* = 2.3 Hz, 1H), 7.53 (dd, *J* = 5.3, 3.2 Hz, 1H), 7.37 (d, *J* = 8.6 Hz, 2H), 7.29 (d, *J* = 8.6 Hz, 2H), 5.17 (s, 2H), 4.41 (t, *J* = 5.2 Hz, 2H), 3.95 (t, *J* = 5.2 Hz, 2H), 1.70 (d, *J* = 13.7 Hz, 6H), 1.65 (s, 6H). $^{31}P$ NMR (162 MHz, DMSO-$d_6$) δ 34.31 (s, 1P).

**Example 70: Preparation of compound 70**

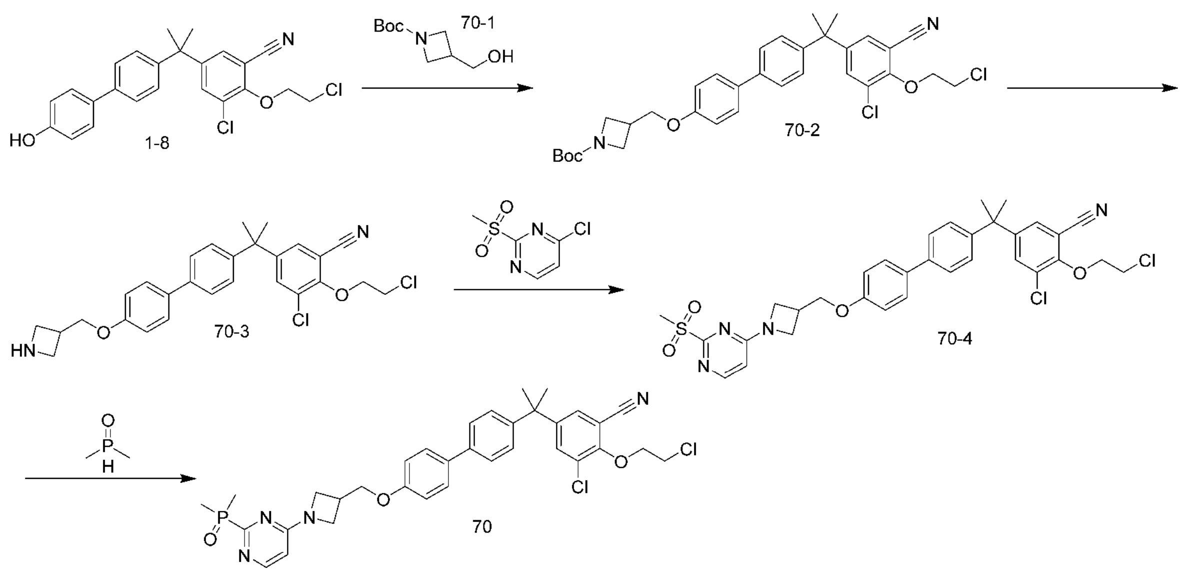


**Preparation of compound 70-2**

Compound **1-8** (500 mg, 1.17 mmol) and compound **70-1** (241.55 mg, 1.29 mmol) were weighed and added to a 30 mL microwave tube, followed by the addition of CMBP (566 mg, 2.35 mmol) and toluene (10 mL). The system was purged three times with nitrogen. The reaction mixture was heated at 100°C for 2 h. LCMS indicated the disappearance of the starting material and the formation of the product. The reaction mixture was subjected to rotary evaporation until

dryness and purified by column chromatography (EtOAc/PE = 0 to 5%) to obtain 600 mg of the title compound **70-2** in 85.9% yield. LC-MS (ESI): m/z 539.2 $[M-56+H]^+$.

**Preparation of compound 70-3**

At 0°C, compound **70-2** (600 mg, 1.01 mmol) was weighed and dissolved in DCM (5 mL). A solution of hydrogen chloride in 1,4-dioxane (4 M, 10.07 mmol, 2.5 mL) was added dropwise. The reaction mixture was stirred at room temperature for 3 h. LCMS indicated the disappearance of the starting material and the formation of the product. The reaction mixture was poured into saturated $Na_2CO_3$ solution (50 mL). The organic phase was extracted three times with DCM (30 mL × 3), washed three times with saturated brine (30 mL), dried over anhydrous $Na_2SO_4$, filtered, and evaporated to dryness under reduced pressure to obtain 400 mg of the title compound **70-3** in 80.1% yield. The product was used directly in the next step without purification. LC-MS (ESI): m/z 495.2 $[M+H]^+$.

**Preparation of compound 70-4**

Compound **70-3** (400 mg, 807.36 μmol) and 4-chloro-2-(methylsulfonyl)pyrimidine (233.3 mg, 1.21 mmol) were dissolved in DCM (10 mL), and DIPEA (208.7 mg, 1.61 mmol, 281.26 μL) was added. The reaction mixture was stirred at 0°C for 2 h. LCMS indicated the disappearance of the starting material and the formation of the product. The reaction mixture was subjected to rotary evaporation until dryness and purified by column chromatography (EtOAc/PE = 0 to 50%) to obtain 300 mg of the title compound **70-4** in 57% yield. LC-MS (ESI): m/z 651.2 $[M+H]^+$.

**Preparation of compound 70**

Compound **70-4** (300 mg, 460.40 μmol) and dimethylphosphine oxide (359.4 mg, 4.60 mmol) were weighed and dissolved in $CH_3CN$ (8 mL), and $Cs_2CO_3$ (450 mg, 1.38 mmol) was added. The reaction mixture was stirred at 80°C for 2 h. LCMS indicated the disappearance of the starting material and the formation of the product. The reaction mixture was filtered, and the filtrate was poured into water (20 mL) and extracted three times with ethyl acetate (25 mL). The organic phases were combined, washed with water, washed three times with saturated brine (25 mL), dried over anhydrous sodium sulfate, filtered, and subjected to rotary evaporation until dryness. The resulting residue was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 55% to 75% acetonitrile gradient elution over 12 min; flow rate: 30 mL/min) to obtain 58 mg of the title compound 70. LC-MS (ESI): m/z 649.2 $[M+H]^+$. $^1H$ NMR (400 MHz, DMSO-$d_6$) δ 8.27 (dd, *J* = 6.0, 1.0 Hz, 1H), 7.70 (d, *J* = 2.3 Hz, 1H), 7.64 (d, *J* = 2.3 Hz, 1H), 7.59 (d, *J* = 8.8 Hz, 2H), 7.56 (d, *J* = 8.5 Hz, 2H), 7.30 (d, *J* = 8.5 Hz, 2H), 7.04 (d, *J* = 8.8 Hz, 2H), 6.50 (dd, *J* = 6.0, 3.2 Hz, 1H), 4.42 (t, *J* = 5.3 Hz, 2H), 4.29 - 4.19 (m, 4H), 3.95 (t, *J* = 5.3 Hz, 2H), 3.94 - 3.90 (m, 2H), 3.28 - 3.17 (m, 1H), 1.68 (s, 6H), 1.65 (d, *J* = 13.5 Hz, 6H). $^{31}P$ NMR (162 MHz, DMSO-$d_6$) δ 33.36 (s, 1P).

**Example 71: Preparation of compound 71**

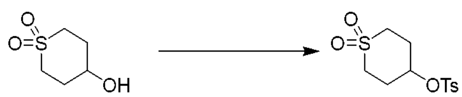

71-1 71-2

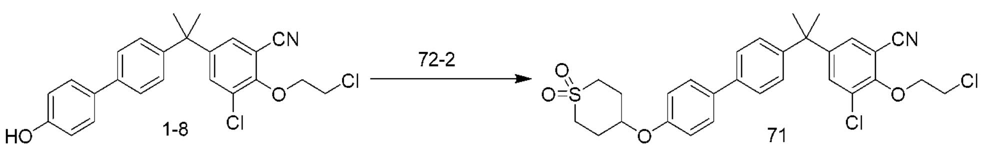


1-8 71

**Preparation of compound 71-2**

Compound **71-1** (100 mg, 0.67 mmol) was dissolved in dichloromethane (2 mL), followed by the sequential addition of 4-methylbenzenesulfonyl chloride (140 mg, 0.74 mmol) and DMAP (8.2 mg, 0.07 mmol). The mixture was cooled to 0°C, and triethylamine (203 mg, 2.01 mmol) was added dropwise. The reaction system was stirred at 25°C for 4 h.

After the reaction was completed, water (10 mL) was added, and the mixture was extracted three times with ethyl acetate (20 mL). The organic phases were combined, washed twice with saturated brine (20 mL), dried over anhydrous sodium sulfate, and concentrated to obtain a crude product. The crude product was purified by column chromatography (EA/PE = 0 to 30%) to obtain 120 mg of the title compound **71-2.**

**Preparation of compound 71**

Compound **71-2** (100 mg, 0.23 mmol) was dissolved in DMF (2 mL), followed by the sequential addition of **1-8** (79 mg, 0.26 mmol) and cesium carbonate (153 mg, 0.47 mmol). The reaction system was stirred at 90°C for 4 h. After the reaction was completed, insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 11 mg of the title compound **71.** LC-MS (ESI): m/z 558.2 $[M+H]^+$. $^1H$ NMR (400 MHz, DMSO-$d_6$) δ 7.81 - 7.69 (m, 2H), 7.65 - 7.55 (m, 4H), 7.31 (dd, *J* = 9.1, 2.7 Hz, 2H), 7.11 (d, *J* = 8.8 Hz, 2H), 4.80 - 4.73 (m, 1H), 4.48 - 4.37 (m, 2H), 4.01 - 3.92 (m, 2H), 3.24 - 3.17 (m, 4H), 2.22 (dt, *J* = 14.1, 8.0 Hz, 4H), 1.69 (d, *J* = 6.7 Hz, 6H).

**Example 72: Preparation of compound 72**

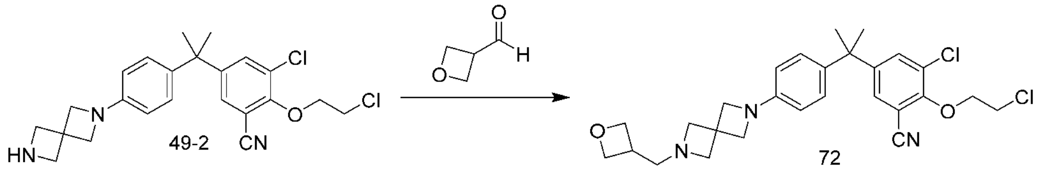

49-2

72

**Preparation of compound 72**

Compound **49-2** (100 mg, 0.23 mmol) was dissolved in dichloromethane (3 mL), followed by the sequential addition of oxetane-3-carbaldehyde (86 mg, 0.93 mmol) and triethylamine (118 mg, 1.16 mmol). The reaction system was stirred at 25°C for 0.5 h. Sodium triacetoxyborohydride (246 mg, 1.16 mmol) was added, and the mixture was stirred for another 3 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 41 mg of the title compound **72.** LC-MS (ESI): m/z 500.2 $[M+H]^+$. $^1H$ NMR (400 MHz, Chloroform-d) δ 7.44 (d, *J* = 2.3 Hz, 1H), 7.26 (d, *J* = 2.3 Hz, 1H), 7.02 (d, *J* = 8.6 Hz, 2H), 6.39 (d, *J* = 8.6 Hz, 2H), 4.84 (t, *J* = 6.9 Hz, 2H), 4.46 - 4.37 (m, 4H), 4.11 (brs, 4H), 4.03 (s, 4H), 3.87 (t, *J* = 6.2 Hz, 2H), 3.38 - 3.21 (m, 3H), 1.61 (s, 6H).

**Example 73: Preparation of compound 73**

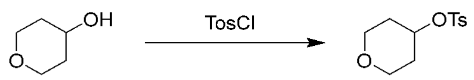


73-1

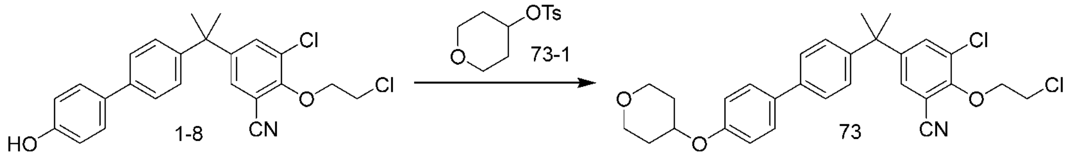


1-8

73

**Preparation of compound 73-1**

Compound 4-hydroxypyran (200 mg, 1.96 mmol) was dissolved in dichloromethane (2 mL), followed by the sequential addition of *p*-toluenesulfonyl chloride (411 mg, 2.15 mmol) and 4-dimethylaminopyridine (24 mg, 0.20 mmol). The mixture was cooled to 0°C, and triethylamine (595 mg, 5.87 mmol) was added dropwise. The reaction system was stirred at 25°C for 12 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by column chromatography (PE:EA = 30%) to obtain 380 mg of the title compound **73-1.**

**Preparation of compound 73**

Compound **1-8** (150 mg, 0.35 mmol) was dissolved in dimethyl sulfoxide (2.0 mL), followed by the sequential addition of compound **73-1** (99 mg, 0.39 mmol) and cesium carbonate (230 mg, 0.70 mmol). The reaction system was stirred at 60°C for 12 h. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 41 mg of the title compound **73.** LC-MS (ESI): m/z 510.2 $[M+H]^+$. $^1$H NMR (400 MHz, Chloroform-*d*) δ 7.54 - 7.49 (m, 3H), 7.49 - 7.46 (m, 2H), 7.40 (dd, *J* = 20.6, 2.3 Hz, 1H), 7.22 (dd, *J* = 8.3, 3.4 Hz, 2H), 6.99 (d, *J* = 8.7 Hz, 2H), 4.53 (m, 1H), 4.42 (t, *J* = 6.2 Hz, 2H), 4.01 (m, 2H), 3.88 (t, *J* = 6.2 Hz, 2H), 3.60 (m, 2H), 2.12 - 2.01 (m, 2H), 1.82 (m, 2H), 1.70 (s, 6H).

**Example 74: Preparation of compound 74**

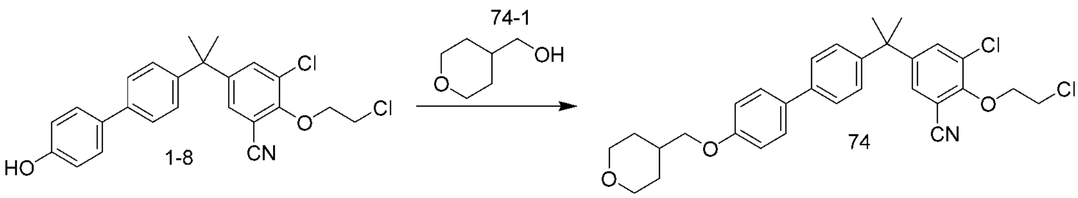


**Preparation of compound 74**

Compound **1-8** (100 mg, 0.23 mmol) was dissolved in anhydrous tetrahydrofuran (5 mL), followed by the sequential addition of (tetrahydro-2H-pyran-4-yl)methanol (30 mg, 0.26 mmol) and triphenylphosphine (123 mg, 0.47 mmol). The system was purged three times with nitrogen, cooled to 0°C, and diisopropyl azodicarboxylate (95 mg, 0.47 mmol) was added dropwise. The reaction system was stirred at 25°C for 12 h. After the reaction was completed, water (10 mL) was added to the reaction mixture, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2 mg of the title compound **74.** LC-MS (ESI): m/z 524.3 $[M+H]^+$. $^1$H NMR (400 MHz, Chloroform-*d*) δ 7.53 - 7.46 (m, 5H), 7.38 (d, *J* = 2.3 Hz, 1H), 7.22 (d, *J* = 8.4 Hz, 2H), 6.96 (d, *J* = 8.8 Hz, 2H), 4.42 (t, *J* = 6.2 Hz, 2H), 4.03 (dd, *J* = 11.2, 3.4 Hz, 2H), 3.88 (t, *J* = 6.2 Hz, 2H), 3.85 (d, *J* = 6.5 Hz, 2H), 3.46 (td, *J* = 11.9, 2.0 Hz, 2H), 2.14 - 2.04 (m, 1H), 1.78 (d, *J* = 12.9 Hz, 2H), 1.69 (s, 6H), 1.49 (m, 2H).

**Example 75: Preparation of compound 75**

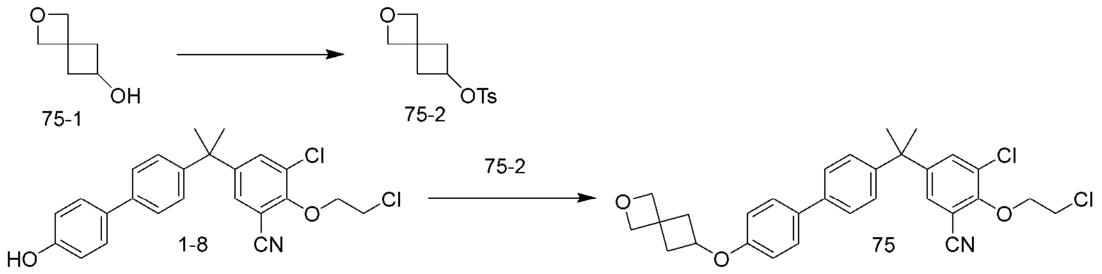


75-1 75-2 1-8 75

### Preparation of compound 75-2

Compound **75-1** (130 mg, 1.14 mmol) was dissolved in dichloromethane (2 mL), followed by the sequential addition of *p*-toluenesulfonyl chloride (191 mg, 1.14 mmol) and 4-dimethylaminopyridine (14 mg, 0.11 mmol). The mixture was cooled to 0°C, and triethylamine (345 mg, 3.41 mmol) was added dropwise. The reaction system was stirred at 25°C for 12 h. After the reaction was completed, water (10 mL) was added, and the mixture was extracted three times with dichloromethane (20 mL). The organic phases were combined, washed twice with saturated brine (20 mL), dried over anhydrous sodium sulfate, and subjected to suction filtration, and the filtrate was concentrated to obtain a crude product. The crude product was purified by column chromatography (EA/PE = 0 to 30%) to obtain 235 mg of the title compound **75-2.** LC-MS (ESI): m/z 286.2 $[M+H_2O]^+$.

### Preparation of compound 75

Compound **1-8** (100 mg, 0.23 mmol) was dissolved in dimethyl sulfoxide (2 mL), followed by the sequential addition of compound **75-2** (69 mg, 0.26 mmol) and cesium carbonate (153 mg, 0.47 mmol). The reaction system was stirred at 60°C for 12 h. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 8 mg of the title compound **4.** LC-MS (ESI): m/z 544.2 $[M+Na]^+$. $^1H$ NMR (400 MHz, Chloroform-*d*) δ 7.52 - 7.44 (m, 5H), 7.37 (d, *J* = 2.3 Hz, 1H), 7.22 (d, *J* = 8.5 Hz, 2H), 6.84 (d, *J* = 8.7 Hz, 2H), 4.77 (s, 2H), 4.71 (s, 2H), 4.59 - 4.52 (m, 1H), 4.43 (t, *J* = 6.2 Hz, 2H), 3.88 (t, *J* = 6.2 Hz, 2H), 2.90 - 2.79 (m, 2H), 2.43 - 2.35 (m, 2H), 1.69 (s, 6H).

### Example 76: Preparation of compound 76

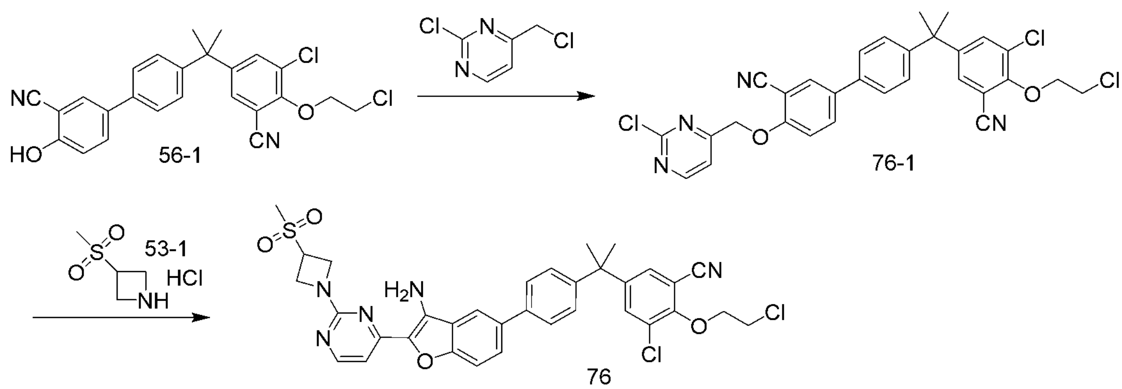


56-1 76-1 76

### Preparation of compound 76-1

Compound **56-1** (250 mg, 553.90 μmol) and compound 2-chloro-4-(chloromethyl)pyrimidine (99.3 mg, 609.29 μmol) were dissolved in $CH_3CN$ (5 mL), and $K_2CO_3$ (229.7 mg, 1.66 mmol) was added. The reaction mixture was stirred at 85°C for 2 h. LCMS indicated the disappearance of the starting material and the formation of the product. The reaction mixture was used directly in the next step without workup and purification. LC-MS (ESI): m/z 577.2 $[M+H]^+$.

**Preparation of compound 76**

Compound **53-1** (114.1 mg, 664.49 μmol) and triethylamine (112.1 mg, 1.11 mmol, 154.47 μL) were added to the above reaction mixture. The reaction mixture was stirred at 80°C for 12 h. LCMS indicated the disappearance of the starting material and the formation of the product. The reaction mixture was filtered and then subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 75% acetonitrile gradient elution over 12 min; flow rate: 30 mL/min) to obtain 96.7 mg of the title compound **76.** LC-MS (ESI): m/z 676.2 $[M+H]^+$. $^1H$ NMR (400 MHz, DMSO-$d_6$) δ 8.31 (d, *J* = 5.4 Hz, 1H), 8.25 (d, *J* = 1.9 Hz, 1H), 7.75 - 7.70 (m, 2H), 7.65 (d, *J* = 8.6 Hz, 2H), 7.65 (d, *J* = 2.3 Hz, 1H), 7.57 (d, *J* = 8.6 Hz, 1H), 7.38 (d, *J* = 8.6 Hz, 2H), 6.85 (d, *J* = 5.3 Hz, 1H), 6.51 (s, 2H), 4.44 - 4.37 (m, 3H), 4.43 (t, *J* = 5.2 Hz, 2H), 4.34 - 4.26 (m, 2H), 3.96 (t, *J* = 5.2 Hz, 2H), 3.10 (s, 3H), 1.71 (s, 6H).

**Example 77: Preparation of compound 77**

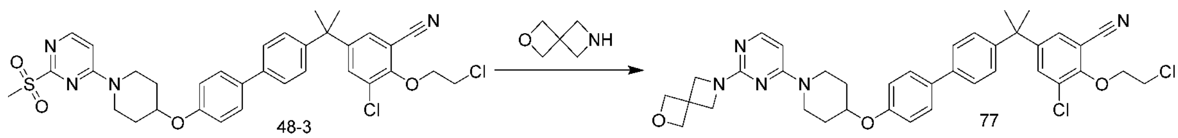

**Preparation of compound 77**

Compound **48-3** (100 mg, 0.15 mmol) was dissolved in acetonitrile (5 mL), followed by the sequential addition of 2-oxa-6-azaspiro[3.3]heptane (45 mg, 0.45 mmol) and potassium carbonate (62 mg, 0.45 mmol). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 34.3 mg of the title compound 77. LC-MS (ESI): m/z 684.2 $[M+H]^+$. $^1H$ NMR (400 MHz, Chloroform-d) δ 7.90 (d, *J* = 6.4 Hz, 1H), 7.52 (d, *J* = 8.8 Hz, 2H), 7.49 (d, *J* = 8.4 Hz, 2H), 7.49 (d, *J* = 2.4 Hz, 1H), 7.37 (d, *J* = 2.4 Hz, 1H), 7.23 (d, *J* = 8.4 Hz, 2H), 7.00 (d, *J* = 8.8 Hz, 2H), 5.99 (d, *J* = 6.5 Hz, 1H), 4.83 (s, 4H), 4.68 - 4.58 (m, 1H), 4.43 (t, *J* = 6.2 Hz, 2H), 4.28 (s, 4H), 3.88 (t, *J* = 6.2 Hz, 2H), 3.87 - 3.82 (m, 2H), 3.70 - 3.59 (m, 2H), 2.05 - 1.94 (m, 2H), 1.94 - 1.86 (m, 2H), 1.70 (s, 6H).

**Example 78: Preparation of compound 78**

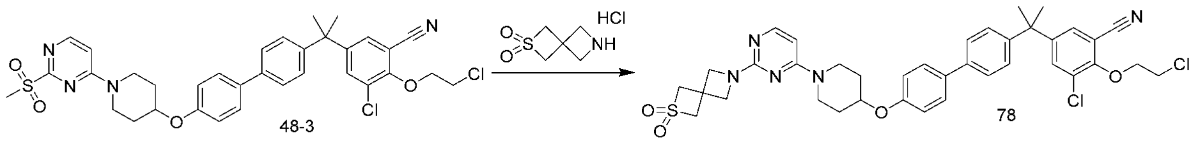

**Preparation of compound 78**

Compound **48-3** (100 mg, 0.15 mmol) was dissolved in acetonitrile (5 mL), followed by the sequential addition of 2-thia-6-azaspiro[3.3]heptane 2,2-dioxide hydrochloride (66 mg, 0.45 mmol) and potassium carbonate (62 mg, 0.45 mmol). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65%

acetonitrile gradient elution over 12 min; flow rate: 30 mL/min) to obtain 27.2 mg of the title compound **78.** LC-MS (ESI): m/z 732.2 [M+H]$^+$. $^1$H NMR (400 MHz, Chloroform-d) δ 7.93 (d, *J* = 6.3 Hz, 1H), 7.52 (d, *J* = 8.8 Hz, 2H), 7.49 (d, *J* = 8.4 Hz, 2H), 7.49 (d, *J* = 2.4 Hz, 1H), 7.37 (d, *J* = 2.3 Hz, 1H), 7.23 (d, *J* = 8.4 Hz, 2H), 7.00 (d, *J* = 8.8 Hz, 2H), 6.03 (d, *J* = 6.3 Hz, 1H), 4.66 - 4.57 (m, 1H), 4.43 (t, *J* = 6.2 Hz, 2H), 4.35 (s, 4H), 4.31 (s, 4H), 3.88 (t, *J* = 6.2 Hz, 2H), 3.87 - 3.80 (m, 2H), 3.68 - 3.57 (m, 2H), 2.04 - 1.93 (m, 2H), 1.93 - 1.81 (m, 2H), 1.70 (s, 6H).

**Example 79: Preparation of compound 79**

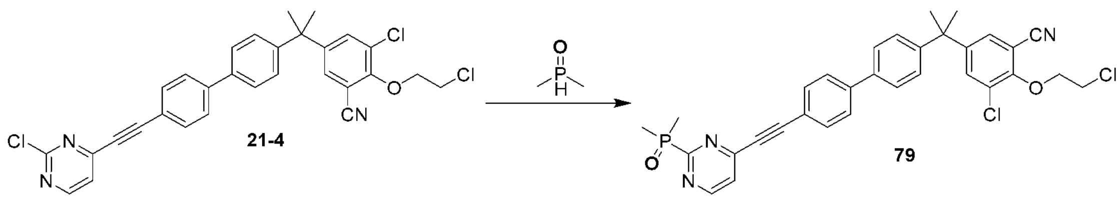

**Preparation of compound 79**

Compound **21-4** (30 mg, 54.86 μmol) was dissolved in *N,N*-dimethylformamide (0.5 mL), followed by the sequential addition of dimethylphosphine oxide (6.4 mg, 82.29 μmol), *N,N*-diisopropylethylamine (14.2 mg, 109.71 μmol), tris(dibenzylideneacetone)dipalladium (2 mg, 2.74 μmol), and 4,5-bis(diphenylphosphino)-9,9-dimethylxanthene (1.6 mg, 2.74 μmol). The reaction system was purged three times with nitrogen and stirred under microwave irradiation at 120°C for 2 h. After the reaction was completed, the reaction system was filtered. Water (20 mL) was added to the filtrate, and the mixture was extracted three times with dichloromethane (20 mL). The organic phases were combined, dried, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 45% acetonitrile gradient elution over 12 min; flow rate: 30 mL/min) to obtain 4.4 mg of the title compound **79.** LC-MS (ESI): m/z 588.2 [M+H]$^+$. $^1$H NMR (400 MHz, DMSO-$d_6$) δ 9.19 (s, 2H), 7.81 (d, *J* = 8.4 Hz, 2H), 7.72 (d, *J* = 8.4 Hz, 2H), 7.72 (d, *J* = 2.3 Hz, 1H), 7.70 (d, *J* = 8.4 Hz, 2H), 7.65 (d, *J* = 2.3 Hz, 1H), 7.38 (d, *J* = 8.4 Hz, 2H), 4.43 (t, *J* = 5.2 Hz, 2H), 3.96 (t, *J* = 5.2 Hz, 2H), 1.79 (d, *J* = 13.8 Hz, 6H), 1.70 (s, 6H). $^{31}$P NMR (162 MHz, DMSO-$d_6$) δ 34.43.

**Example 80: Preparation of compound 80**

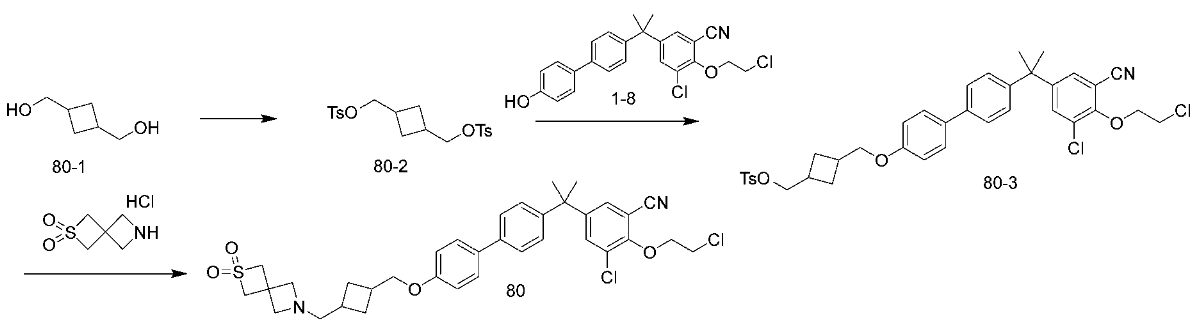

**Preparation of compound 80-2**

Compound **80-1** (250 mg, 2.15 mmol) was dissolved in dichloromethane (12 mL). Triethylamine (653.4 mg, 6.46 mmol, 0.9 mL) and DMAP (52.6 mg, 430.45 μmol) were added separately at room temperature, followed by slow addition of *p*-toluenesulfonyl chloride (902.70 mg, 4.73 mmol). The mixture was stirred at room temperature overnight. After the reaction was completed, dichloromethane was added to the reaction mixture for dilution, silica gel was added, and the mixture was concentrated. The crude product was purified using an automated column chromatography system (EA/PE = 0 to 30%) to obtain 683 mg of title compound **80-2** in 74.8% yield. LC-MS (ESI): m/z 442.2 [M+18]$^+$. $^1$H NMR (400 MHz, Chloroform-*d*) δ 7.80 - 7.74 (m, 4H), 7.38 - 7.32 (m, 4H), 3.98 (d, *J* = 6.6 Hz, 2H), 3.88 (d, *J* = 6.2 Hz, 2H), 2.59 - 2.47 (m, 2H),

2.45 (s, 6H), 2.09 - 2.01 (m, 1H), 1.83 (t, *J* = 7.5 Hz, 2H), 1.53 - 1.42 (m, 1H).

**Preparation of compound 80-3**

Compound **1-8** (60 mg, 140.73 μmol) was dissolved in DMF (1.5 mL), and compound **80-2** (65.7 mg, 154.81 μmol) and cesium carbonate (68.8 mg, 211.10 μmol) were added. The reaction mixture was stirred at 60°C overnight. After the reaction was completed, the reaction mixture was cooled to room temperature. Saturated aqueous ammonium chloride solution was added, and the mixture was extracted with ethyl acetate. The organic phases were combined, washed with saturated brine, dried over anhydrous sodium sulfate, filtered, and concentrated. The crude product was purified using an automated column chromatography system (EA/PE = 0 to 25%) to obtain 75.7 mg of the title compound **80-3** in 79.3% yield. LC-MS (ESI): m/z 695.3 $[M+18]^+$.

**Preparation of compound 80**

Compound **80-3** (75.7 mg, 111.54 μmol) was dissolved in acetonitrile (1.5 mL), and compound 2-thia-6-azaspiro [3.3]heptane 2,2-dioxide hydrochloride (22.5 mg, 122.70 μmol) and DIPEA (43.3 mg, 334.63 μmol, 58.3 μL) were added. The reaction mixture was stirred overnight at 90°C in a sealed tube. After the reaction was completed, the reaction mixture was cooled to room temperature, diluted with a small amount of acetonitrile, and purified by preparative chromatography (preparative method: mobile phase: A: 10 mM aqueous ammonium bicarbonate solution; B: acetonitrile; chromatographic column: Welch Ultimate AQ-C18 250 × 21.2 mm; column temperature: 25°C; gradient: B%: 80%-90% 8 min, 95% 6 min; flow rate: 30 mL/min) to obtain 2 mg of the title compound **80.** LC-MS (ESI): m/z 653.4 $[M+H]^+$.

**Example 81: Preparation of compound 81**

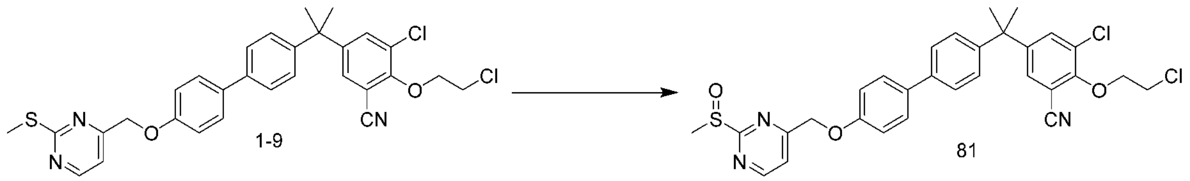

1-9 81

**Preparation of compound 81**

Compound **1-9** (30 mg, 53.14 μmol) and DCM (3 mL) were added to a reaction flask, followed by the addition of mCPBA (11.33 mg, 55.80 μmol, 85% purity) to the reaction system. The mixture was stirred at room temperature for 2 h. The reaction was quenched by adding saturated aqueous sodium thiosulfate solution (3 mL), and the mixture was extracted three times with DCM (3 mL). The organic phases were combined and subjected to rotary evaporation until dryness. The crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 10% to 80% acetonitrile gradient elution over 12 min; flow rate: 30 mL/min) to obtain 20 mg of title compound **81.** LC-MS (ESI): m/z 580.0 $[M+H]^+$. $^1H$ NMR (400 MHz, DMSO-$d_6$) δ 9.01 (d, *J* = 4.8 Hz, 1H), 7.96 - 7.43 (m, 7H), 7.30 (d, *J* = 7.9 Hz, 2H), 7.14 (d, *J* = 8.2 Hz, 2H), 5.37 (s, 2H), 4.42 (s, 2H), 3.96 (s, 2H), 2.89 (s, 3H), 1.68 (s, 6H).

**Example 82: Preparation of compound 82**

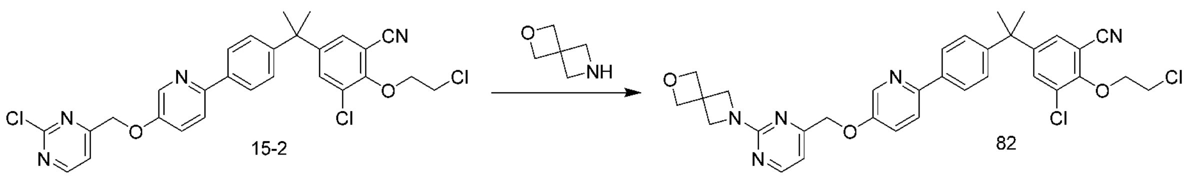

15-2 82

**Preparation of compound 82**

2-Oxa-6-azaspiro[3.3]heptane (21.5 mg, 216.66 μmol), compound 15-2 (80 mg, 144.44 μmol), DIPEA (186.7 mg, 1.44 mmol, 251.6 μL) and acetonitrile (1 mL) were added to a reaction flask, and the mixture was stirred under microwave irradiation at 80°C for 2 h. After the reaction was completed, the reaction mixture was cooled to room temperature and subjected to rotary evaporation until dryness. The crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 10% to 80% acetonitrile gradient elution over 12 min; flow rate: 30 mL/min) to obtain 50 mg of title compound **82.** LC-MS (ESI): m/z 616.4 $[M+H]^+$. $^1H$ NMR (400 MHz, DMSO-$d_6$) δ 8.43 (d, *J* = 3.0 Hz, 1H), 8.37 (d, *J* = 5.0 Hz, 1H), 7.94 (d, *J* = 8.5 Hz, 2H), 7.89 (d, *J* = 8.8 Hz, 1H), 7.70 (d, *J* = 2.3 Hz, 1H), 7.62 (d, *J* = 2.3 Hz, 1H), 7.52 (dd, *J* = 8.8, 3.0 Hz, 1H), 7.33 (d, *J* = 8.6 Hz, 2H), 6.80 (d, *J* = 5.0 Hz, 1H), 5.13 (s, 2H), 4.72 (s, 4H), 4.42 (t, *J* = 5.2 Hz, 2H), 4.20 (s, 4H), 3.96 (t, *J* = 5.2 Hz, 2H), 1.69 (s, 6H).

**Example 83: Preparation of compound 83**

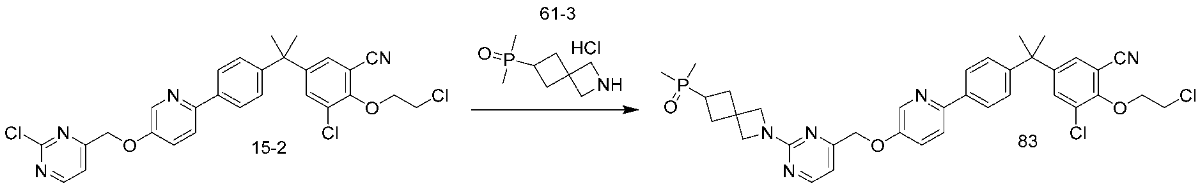


**Preparation of compound 83**

Compound **15-2** (100 mg, 180.55 μmol), compound **61-3** (46.9 mg, 270.82 μmol), DIPEA (233 mg, 1.81 mmol, 314.48 μL), and acetonitrile (717 μL) were added to a reaction flask, and the mixture was stirred under microwave irradiation at 80°C for 2 h. The mixture was subjected to rotary evaporation until dryness. The crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 10% to 80% acetonitrile gradient elution over 12 min; flow rate: 30 mL/min) to obtain 50 mg of title compound **83.** LC-MS (ESI): m/z 690.6 $[M+H]^+$. $^1H$ NMR (400 MHz, DMSO-$d_6$) δ 8.43 (d, *J* = 3.0 Hz, 1H), 8.35 (d, *J* = 5.0 Hz, 1H), 7.95 (d, *J* = 8.6 Hz, 2H), 7.89 (d, *J* = 8.8 Hz, 1H), 7.70 (d, *J* = 2.4 Hz, 1H), 7.62 (d, *J* = 2.3 Hz, 1H), 7.52 (dd, *J* = 8.9, 3.0 Hz, 1H), 7.33 (d, *J* = 8.6 Hz, 2H), 6.78 (d, *J* = 5.0 Hz, 1H), 5.13 (s, 2H), 4.42 (t, *J* = 5.6 Hz, 2H), 4.08 (s, 2H), 3.99 - 3.91 (m, 4H), 2.49 - 2.41 (m, 1H), 2.40 - 2.30 (m, 4H), 1.69 (s, 6H), 1.28 (d, *J* = 12.7 Hz, 6H). $^{31}P$ NMR (162 MHz, DMSO-$d_6$) δ 40.72 (s, 1P).

**Example 84: Preparation of compound 84**

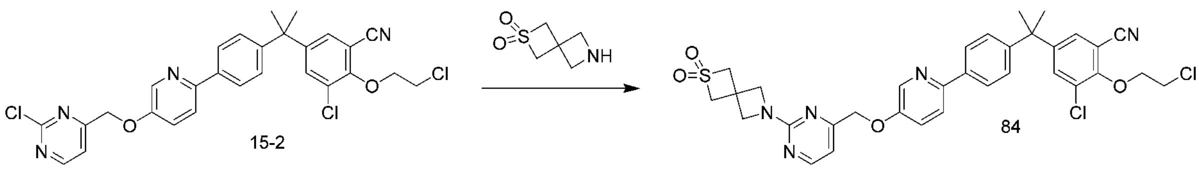


**Preparation of compound 84**

Compound **15-2** (76 mg, 138.44 μmol), 2-thia-6-azaspiro[3.3]heptane 2,2-dioxide (30.6 mg, 207.65 μmol) , DIPEA (178.9 mg, 1.38 mmol, 241 μL) , and acetonitrile (790 μL) were added to a reaction flask, and the mixture was stirred under microwave irradiation at 80°C for 2 h. The mixture was subjected to rotary evaporation until dryness. The crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 10% to 80% acetonitrile gradient elution over 12 min; flow rate: 30 mL/min) to obtain 50 mg of title compound **84.** LC-MS (ESI): m/z 664.4 $[M+H]^+$. $^1H$ NMR (400 MHz, DMSO-$d_6$) δ 8.44 (d, *J* = 3.0 Hz, 1H), 8.40 (d, *J* = 5.0 Hz, 1H), 7.95 (d, *J* = 8.2 Hz, 2H), 7.89 (d, *J* = 8.8 Hz, 1H), 7.69 (d, *J* = 2.4 Hz, 1H), 7.62 (d, *J* = 2.3 Hz, 1H), 7.52 (dd, *J* = 8.8, 3.1 Hz, 1H),

7.33 (d, *J* = 8.1 Hz, 2H), 6.86 (d, *J* = 5.0 Hz, 1H), 5.15 (s, 2H), 4.51 (s, 4H), 4.42 (t, *J* = 5.1 Hz, 2H), 4.28 (s, 4H), 3.96 (t, *J* = 5.1 Hz, 2H), 1.69 (s, 6H).

**Example 85: Preparation of compound 85**

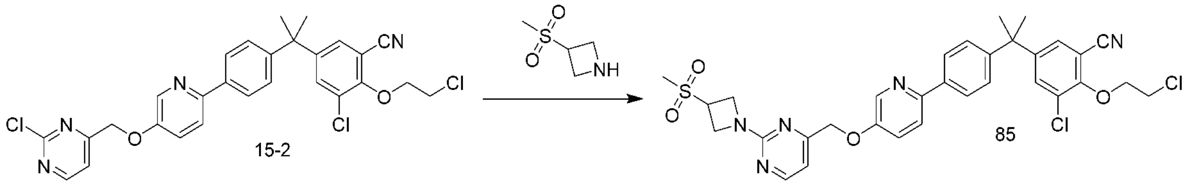
15-2
85

**Preparation of compound 85**

3-Methanesulfonyl-azetidine (29.29 mg, 216.66 μmol), compound **15-2** (80 mg, 144.44 μmol), DIPEA (187 mg, 1.44 mmol, 251.6 μL), and acetonitrile were added to a reaction flask, and the mixture was stirred under microwave irradiation at 80°C for 2 h. The mixture was subjected to rotary evaporation until dryness. The crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 10% to 80% acetonitrile gradient elution over 12 min; flow rate: 20 mL/min) to obtain 50 mg of title compound 54. LC-MS (ESI): m/z 652.4 $[M+H]^+$. $^1$H NMR (400 MHz, DMSO-$d_6$) δ 8.46 - 8.41 (m, 2H), 7.95 (d, *J* = 8.6 Hz, 2H), 7.89 (d, *J* = 8.8 Hz, 1H), 7.70 (d, *J* = 2.3 Hz, 1H), 7.62 (d, *J* = 2.4 Hz, 1H), 7.53 (dd, *J* = 8.8, 3.0 Hz, 1H), 7.33 (d, *J* = 8.6 Hz, 2H), 6.90 (d, *J* = 5.0 Hz, 1H), 5.18 (s, 2H), 4.46 - 4.38 (m, 3H), 4.34 (t, *J* = 8.6 Hz, 2H), 4.28 - 4.17 (m, 2H), 3.96 (t, *J* = 5.2 Hz, 2H), 3.06 (s, 3H), 1.69 (s, 6H).

**Example 86: Preparation of compound 86**

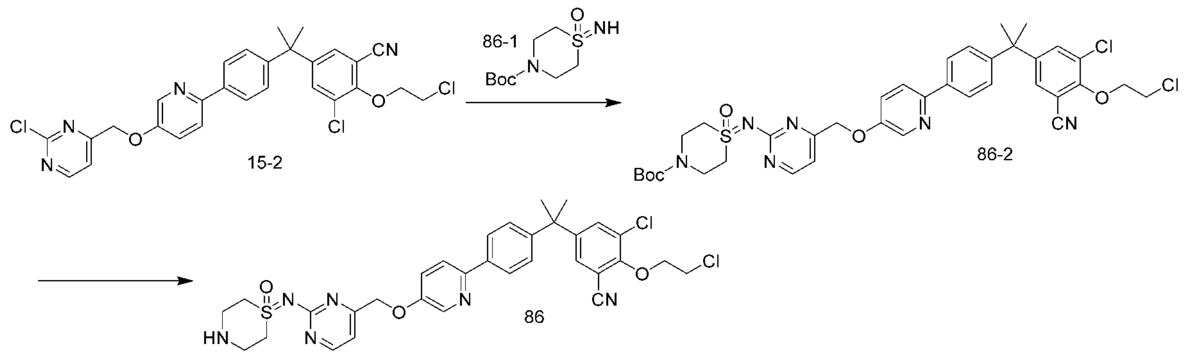
15-2
86-1
86-2
86

**Preparation of compound 86-2**

Compound **15-2** (500 mg, 902.75 μmol), compound **86-1** (317 mg, 1.35 mmol), DIPEA (350 mg, 2.71 mmol, 472 μL), Xantphos (105 mg, 180.55 μmol), $Pd_2(dba)_3$ (83 mg, 90.27 μmol), and dioxane (5 mL) were added to a microwave tube. Under a nitrogen atmosphere, the mixture was stirred under microwave irradiation at 100°C for 2 h. The mixture was subjected to rotary evaporation until dryness. The crude product was purified by silica gel column chromatography (MeOH/DCM = 0 to 10%) to obtain 300 mg of the title compound **86-2** in 44% yield. LC-MS (ESI): m/z 751.4 $[M+H]^+$.

**Preparation of compound 86**

Compound **86-2** (300 mg, 399.08 μmol) and DCM (10 mL) were added to a reaction flask, followed by the addition of TFA (455 mg, 3.99 mmol). The mixture was stirred at room temperature for 2 h. The mixture was subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain

220 mg of the title compound **86.** LC-MS (ESI): m/z 651.2 [M+H]+. $^1$H NMR (400 MHz, DMSO-$d_6$) δ 8.47 (d, *J* = 5.0 Hz, 1H), 8.44 (d, *J* = 3.0 Hz, 1H), 7.95 (d, *J* = 8.5 Hz, 2H), 7.89 (d, *J* = 8.8 Hz, 1H), 7.70 (d, *J* = 2.4 Hz, 1H), 7.63 (d, *J* = 2.3 Hz, 1H), 7.52 (dd, *J* = 8.8, 3.0 Hz, 1H), 7.33 (d, *J* = 8.6 Hz, 2H), 7.01 (d, *J* = 5.0 Hz, 1H), 5.20 (s, 2H), 4.42 (t, *J* = 5.2 Hz, 2H), 3.96 (t, *J* = 5.2 Hz, 2H), 3.74 - 3.59 (m, 2H), 3.45 - 3.34 (m, 2H), 3.28 - 3.18 (m, 2H), 3.15 - 2.97 (m, 2H), 1.69 (s, 6H).

**Example 87: Preparation of compound 87**

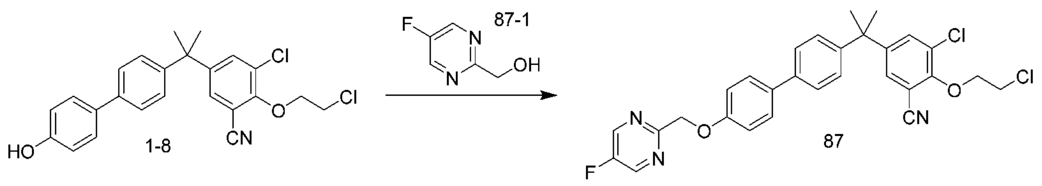

**Preparation of compound 87**

Compound **1-8** (100 mg, 0.23 mmol) was dissolved in THF (5 mL). Compound **87-1** (33 mg, 0.26 mmol) and $PPh_3$ (123 mg, 0.47 mmol) were added. The system was purged three times with nitrogen. DIAD (95 mg, 0.47 mmol) was added to the reaction system at 80°C, and stirring was continued overnight. After the reaction was completed, water (20 mL) was added, and a portion of THF was removed by rotary evaporation. The mixture was extracted three times with ethyl acetate (50 mL). The organic phases were combined, washed once with saturated brine (50 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; acetonitrile elution over 12 min, flow rate: 30 mL/min) to obtain 12 mg of the title compound **87.** LC-MS (ESI): m/z 536.6 [M+H]+. $^1$H NMR (400 MHz, DMSO-$d_6$) δ 8.94 (d, *J* = 0.9 Hz, 2H), 7.70 (d, *J* = 2.3 Hz, 1H), 7.64 (d, *J* = 2.4 Hz, 1H), 7.60 - 7.51 (m, 3H), 7.45 (m, 1H), 7.30 (d, *J* = 8.4 Hz, 2H), 7.05 (d, *J* = 8.8 Hz, 2H), 5.33 (s, 2H), 4.41 (t, *J* = 5.3 Hz, 2H), 3.96 (t, *J* = 5.3 Hz, 2H), 1.68 (s, 6H). $^{19}$F NMR (376 MHz, DMSO-$d_6$) δ -138.90.

**Example 88: Preparation of compound 88**

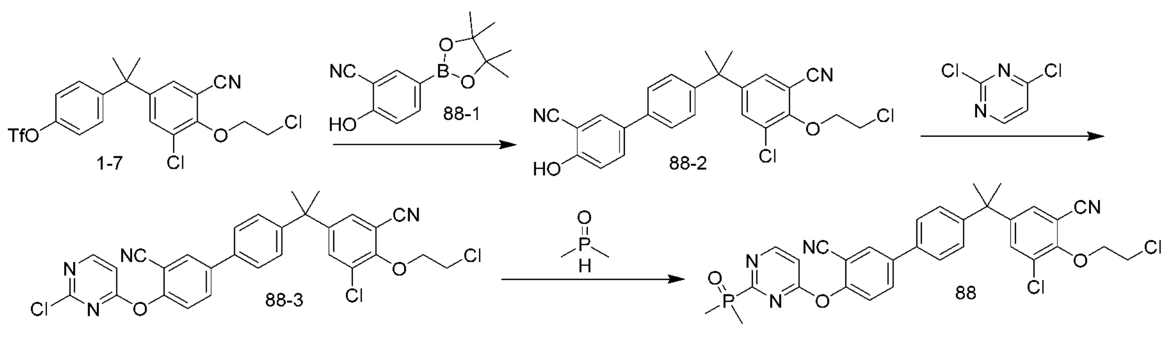

**Preparation of compound 88-2**

Compound **1-7** (100 mg, 0.20 mmol), compound **88-1** (66 mg, 0.27 mmol), potassium carbonate (44 mg, 0.57 mmol), and Pd(dppf)$Cl_2$ (15 mg, 0.02 mmol) were dissolved in 1,4-dioxane (6 mL) and water (2 mL). The reaction system was purged three times with nitrogen and stirred under microwave irradiation at 90°C for 2 h. After the reaction was completed, the reaction mixture was cooled to room temperature and filtered through a pad of diatomite. The filter cake was rinsed with ethyl acetate (30 mL). The filtrates were combined, water (30 mL) was added, and the mixture was extracted three times with ethyl acetate (50 mL). The organic phases were combined, washed once with saturated brine (50 mL), dried over anhydrous sodium sulfate, and concentrated to obtain a crude product. The crude product was purified by silica gel column chromatography (EA/PE = 0 to 25%) to obtain 90 mg of the title compound **88-2.** LC-MS (ESI): m/z 451.0 [M+H]+.

**Preparation of compound 88-3**

Compound **88-2** (50 mg, 0.11 mmol) was dissolved in DMF (2 mL), followed by the sequential addition of 2,4-dichloropyrimidine (20 mg, 0.13 mmol) and cesium carbonate (73 mg, 0.22 mmol). The reaction system was stirred at room temperature for 2 h. After the reaction was completed, water (10 mL) was added to the reaction mixture, and the mixture was extracted three times with ethyl acetate (30 mL). The organic phases were combined, washed once with saturated brine (10 mL), dried over anhydrous sodium sulfate, and concentrated. The resulting crude product was purified by silica gel column chromatography (EA/PE = 0 to 25%) to obtain 60 mg of the title compound 5. LC-MS (ESI): m/z 563.2 $[M+H]^+$.

**Preparation of compound 88**

Compound **88-3** (60 mg, 0.11 mmol), dimethylphosphine oxide (12 mg, 0.16 mmol), DIPEA (69 mg, 0.53 mmol), Xantphos (12 mg, 0.02 mmol), and $Pd_2(dba)_3$ (10 mg, 0.01 mmol) were dissolved in 1,4-dioxane (2 mL). The reaction system was purged three times with nitrogen and stirred under microwave irradiation at 120°C for 2 h. After the reaction was completed, the reaction mixture was filtered through a pad of diatomite, and the filtrate was rinsed with ethyl acetate (30 mL). The filtrates were combined, water (30 mL) was added, and the mixture was extracted three times with ethyl acetate (50 mL). The organic phases were combined, washed once with saturated brine (50 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20.7 mg of the title compound **88.** LC-MS (ESI): m/z 605.4 $[M+H]^+$. $^1H$ NMR (400 MHz, DMSO-$d_6$) δ 8.97 (d, *J* = 6.0 Hz, 1H), 8.33 (d, *J* = 2.3 Hz, 1H), 8.15 (dd, *J* = 8.7, 2.4 Hz, 1H), 7.76 (d, *J* = 8.5 Hz, 2H), 7.72 (d, *J* = 2.4 Hz, 1H), 7.69 - 7.64 (m, 2H), 7.52 (dd, *J* = 5.8, 2.6 Hz, 1H), 7.39 (d, *J* = 8.5 Hz, 2H), 4.43 (t, *J* = 5.6 Hz, 2H), 3.96 (t, *J* = 5.6 Hz, 2H), 1.71 (s, 6H), 1.62 (d, *J* = 13.8 Hz, 6H). $^{31}P$ NMR (162 MHz, DMSO-$d_6$) δ 34.36.

**Example 89: Preparation of compound 89**

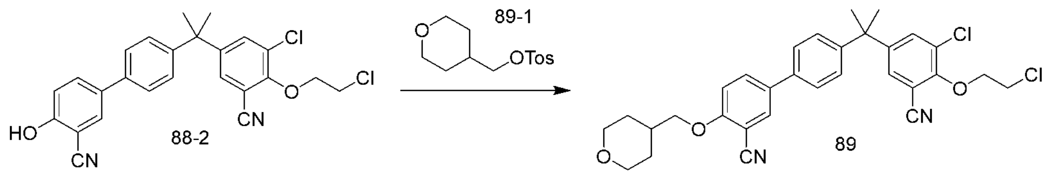


**Preparation of compound 89**

Compound **88-2** (50 mg, 0.11 mmol) was dissolved in THF (5 mL). Compound **89-1** (33 mg, 0.13 mmol) and $Cs_2CO_3$ (72 mg, 0.22 mmol) were added, and the reaction system was stirred at 60°C overnight. After the reaction was completed, water (25 mL) was added, and the mixture was extracted three times with ethyl acetate (50 mL). The organic phases were combined, washed once with saturated brine (50 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; acetonitrile elution over 12 min, flow rate: 30 mL/min) to obtain 10 mg of the title compound **89.** LC-MS (ESI): m/z 549.3 $[M+H]^+$. $^1H$ NMR (400 MHz, DMSO-$d_6$) δ 8.03 (d, *J* = 2.4 Hz, 1H), 7.95 (dd, *J* = 8.9, 2.4 Hz, 1H), 7.70 (d, *J* = 2.3 Hz, 1H), 7.67 - 7.58 (m, 3H), 7.33 (d, *J* = 8.5 Hz, 3H), 4.42 (t, *J* = 5.2 Hz, 2H), 4.05 (d, *J* = 6.4 Hz, 2H), 3.96 (t, *J* = 5.2 Hz, 2H), 3.93 - 3.86 (m, 2H), 3.49 - 3.21 (m, 1H), 2.18 - 1.93 (m, 2H), 1.77 - 1.60 (m, 8H), 1.45 - 1.31 (m, 2H).

**Example 90: Preparation of compound 90**

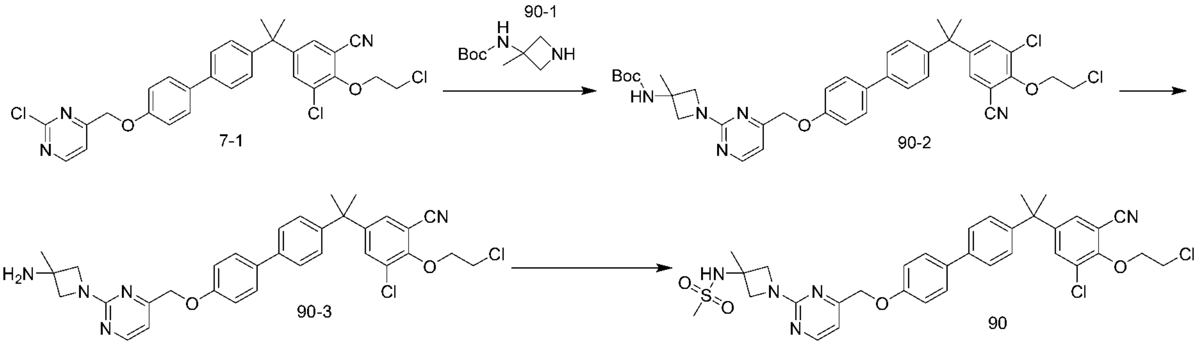


7-1 90-2 90-3 90

### Preparation of compound 90-2

Compound **7-1** (90 mg, 0.16 mmol) and compound **90-1** (56 mg, 0.16 mmol) were dissolved in acetonitrile (10 mL), and potassium carbonate (66 mg, 0.48 mmol) was added. After the addition was completed, the reaction system was stirred at 60°C for 3 h. LCMS monitoring indicated that the starting material was completely consumed. Water (20 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by column chromatography (EA/PE = 0 to 50%) to obtain 100 mg of the title compound **90-2.** LC-MS (ESI): m/z 702.4 $[M+H]^+$.

### Preparation of compound 90-3

Compound **90-2** (100 mg, 0.14 mmol) was dissolved in DCM (5 mL), and HCl solution (3 M, 5 mL) was added. After the addition was completed, the reaction system was stirred at room temperature for 3 h. After the reaction was completed, the solvent was directly removed by rotary evaporation to obtain a crude product of the title compound **90-3.** The crude product was used directly in the next step without purification. LC-MS (ESI): m/z 602.2 $[M+H]^+$.

### Preparation of compound 90

Compound **90-3** (100 mg, 0.17 mmol) was dissolved in dichloromethane (10 mL), followed by the sequential addition of methanesulfonyl chloride (23 mg, 0.20 mmol) and triethylamine (52 mg, 0.51 mmol). The reaction system was stirred at 25°C for 1 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 23 mg of the title compound **90.** LC-MS (ESI): m/z 680.2 $[M+H]^+$. $^1H$ NMR (400 MHz, Chloroform-*d*) δ 8.32 (d, *J* = 5.4 Hz, 1H), 7.56 - 7.44 (m, 5H), 7.37 (d, *J* = 2.3 Hz, 1H), 7.22 (d, *J* = 8.4 Hz, 2H), 7.01 (d, *J* = 8.7 Hz, 2H), 6.94 (d, *J* = 5.3 Hz, 1H), 5.06 (s, 2H), 4.49 - 4.32 (m, 4H), 4.20 (d, *J* = 9.5 Hz, 2H), 3.88 (t, *J* = 6.2 Hz, 2H), 3.09 (s, 3H), 1.81 (s, 3H), 1.69 (s, 6H).

### Example 91: Preparation of compound 91

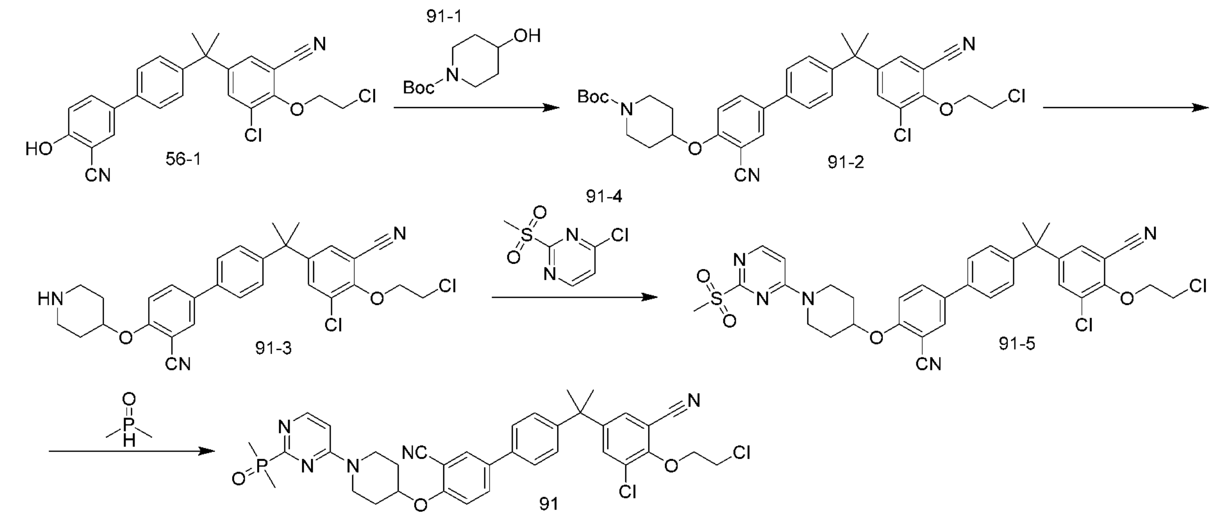


**Preparation of compound 91-2**

Compound **56-1** (250 mg, 0.56 mmol), compound **91-1** (145 mg, 0.67 mmol), and cyanomethylene(tri-n-butyl) phosphorane (270 mg, 1.12 mmol) were dissolved in toluene (20 mL). The reaction system was stirred at 100°C for 16 h. After the reaction was completed, water (10 mL) was added, and the mixture was extracted three times with ethyl acetate (20 mL). The organic phases were combined, washed twice with saturated brine (20 mL), dried over anhydrous sodium sulfate, filtered, and the filtrate was concentrated to obtain a crude product. The crude product was purified by column chromatography (EA/PE = 0 to 30%) to obtain 200 mg of the title compound **91-2.** LC-MS (ESI): m/z 578.2 $[M+H-56]^+$.

**Preparation of compound 91-3**

Compound **91-2** (200 mg, 0.3 mmol) was dissolved in dichloromethane (5 mL), and a solution of hydrogen chloride in 1,4-dioxane (4 M, 10 mL) was added thereto at 0°C. The reaction system was stirred at 25°C for 1 h. After the reaction was completed, the solvent was concentrated to dryness to obtain a crude product of the title compound **91-3.** The product was used directly in the next step without purification. LC-MS (ESI): m/z 534.2 $[M+H]^+$.

**Preparation of compound 91-5**

Compound **91-3** (200 mg, 0.37 mmol) was dissolved in methanol (10 mL), followed by the sequential addition of compound **91-4** (85 mg, 0.44 mmol) and triethylamine (75 mg, 0.74 mmol). The reaction system was stirred at 25°C for 1 h. After the reaction was completed, water (10 mL) was added, and the mixture was extracted three times with ethyl acetate (20 mL). The organic phases were combined, washed twice with saturated brine (20 mL), dried over anhydrous sodium sulfate, filtered, and the filtrate was concentrated to obtain a crude product. The crude product was purified by column chromatography (EA/PE = 0 to 70%) to obtain 80 mg of the title compound **91-5.** LC-MS (ESI): m/z 690.2 $[M+H]^+$.

**Preparation of compound 91**

Compound **91-5** (60 mg, 0.09 mmol) was dissolved in acetonitrile (5 mL), followed by the sequential addition of dimethylphosphine oxide (21 mg, 0.27 mmol) and potassium carbonate (37 mg, 0.27 mmol). The reaction system was stirred at 80°C for 16 h. After the reaction was completed, the reaction mixture was cooled to room temperature, and insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 13.4 mg of the title compound **91.** LC-MS (ESI): m/z 688.3 $[M+H]^+$. $^1$H NMR (400 MHz, Methanol-$d_4$) δ 8.28 (dd, $J$ = 6.5, 1.5 Hz, 1H), 7.96 - 7.85 (m, 2H), 7.63 - 7.49 (m, 4H), 7.41 - 7.30 (m, 3H), 6.91 (dd, $J$ = 6.4, 3.3 Hz, 1H), 5.03 - 4.93 (m, 1H), 4.43 (t, $J$ = 5.2 Hz, 2H), 4.06 - 3.81 (m, 6H), 2.17 - 2.04 (m, 2H), 2.01 - 1.89 (m, 2H), 1.83 (d, $J$ = 13.7 Hz, 6H), 1.72 (s, 6H). $^{31}$P NMR (162 MHz, Methanol-$d_4$) δ 40.87 (s, 1P).

**Example 92: Preparation of compound 92**

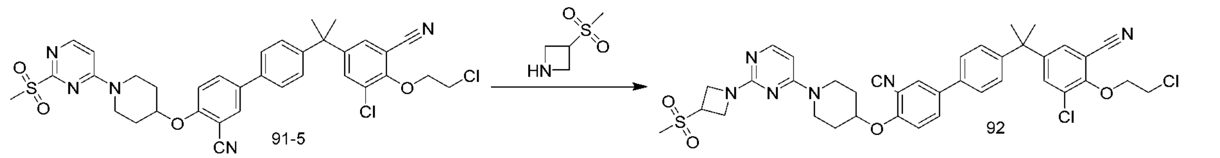

**Preparation of compound 92**

Compound **91-5** (60 mg, 0.09 mmol) was dissolved in acetonitrile (5 mL), followed by the sequential addition of compound 3-(methylsulfonyl)azetidine (36 mg, 0.27 mmol) and potassium carbonate (37 mg, 0.27 mmol). The reaction system was stirred at 80°C for 16 h. After the reaction was completed, the reaction mixture was cooled to room temperature, and insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 2.1 mg of the title compound **92.** LC-MS (ESI): m/z 745.2 $[M+H]^+$. $^1H$ NMR (400 MHz, Chloroform-d) δ 7.86 (d, *J* = 6.8 Hz, 1H), 7.80 (d, *J* = 2.3 Hz, 1H), 7.75 (dd, *J* = 8.7, 2.4 Hz, 1H), 7.49 - 7.43 (m, 3H), 7.34 (d, *J* = 2.4 Hz, 1H), 7.28 (s, 2H), 7.09 (d, *J* = 8.8 Hz, 1H), 6.11 (d, *J* = 6.9 Hz, 1H), 4.85 (s, 1H), 4.57 (s, 4H), 4.43 (t, *J* = 6.1 Hz, 2H), 4.14 - 3.91 (m, 3H), 3.88 (t, *J* = 6.1 Hz, 2H), 3.84 - 3.76 (m, 2H), 2.96 (s, 3H), 2.09 - 1.98 (m, 4H), 1.70 (s, 6H).

**Example 93: Preparation of compound 93**

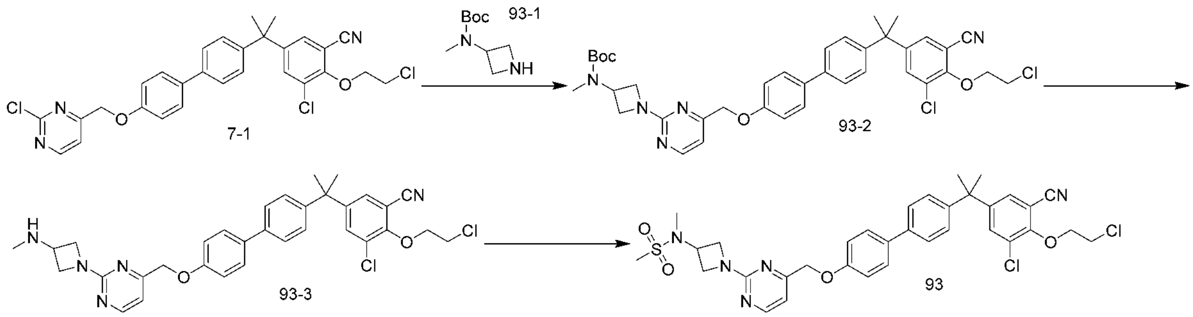

**Preparation of compound 93-2**

Compound **7-1** (90 mg, 0.16 mmol) and compound **93-1** (56 mg, 0.16 mmol) were dissolved in acetonitrile (10 mL), and potassium carbonate (66 mg, 0.48 mmol) was added thereto. After the addition was completed, the reaction system was stirred at 60°C for 3 h. LCMS monitoring indicated that the starting material was completely consumed. The mixture was cooled to room temperature, water (20 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by column chromatography (EA/PE = 0 to 50%) to obtain 100 mg of the title compound **93-2.** LC-MS (ESI): m/z 702.3$[M+H]^+$.

**Preparation of compound 93-3**

Compound **93-2** (100 mg, 0.14 mmol) was dissolved in DCM (5 mL), and a hydrochloric acid solution (3 M, 5 mL) was added. After the addition was completed, the reaction system was stirred at room temperature for 3 h. After the reaction was completed, the solvent was concentrated to obtain a crude product of the title compound **93-3,** which was

used directly in the next step without purification. LC-MS (ESI): m/z 602.2 [M+H]$^+$.

**Preparation of compound 93**

Compound **93-3** (100 mg, 0.17 mmol) was dissolved in dichloromethane (10 mL), followed by the sequential addition of methanesulfonyl chloride (23 mg, 0.20 mmol) and triethylamine (52 mg, 0.51 mmol). The reaction system was stirred at 25°C for 1 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 23.6 mg of the title compound **93.** LC-MS (ESI): m/z 680.2 [M+H]$^+$. $^1$H NMR (400 MHz, Chloroform-*d*) δ 8.35 (d, *J* = 5.2 Hz, 1H), 7.53 (d, *J* = 8.8 Hz, 2H), 7.50 - 7.45 (m, 3H), 7.37 (d, *J* = 2.4 Hz, 1H), 7.22 (d, *J* = 8.4 Hz, 2H), 7.01 (d, *J* = 8.8 Hz, 2H), 6.92 (d, *J* = 5.1 Hz, 1H), 5.05 (s, 2H), 4.91 - 4.81 (m, 1H), 4.51 - 4.39 (m, 4H), 4.31 (s, 2H), 3.88 (t, *J* = 6.2 Hz, 2H), 3.03 (s, 3H), 2.84 (s, 3H), 1.69 (s, 6H).

**Example 94: Preparation of compound 94**

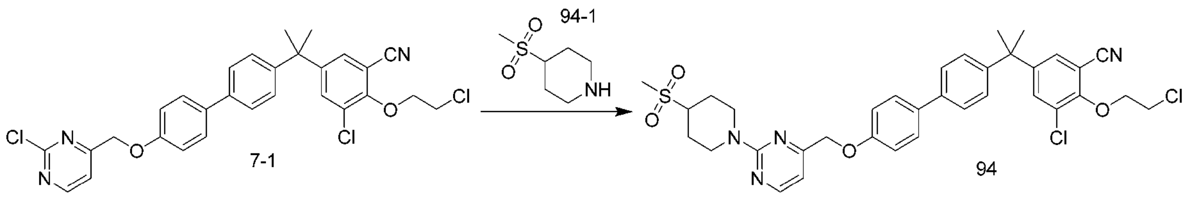


7-1

94

**Preparation of compound 94**

Compound **7-1** (30 mg, 0.054 mmol) was dissolved in acetonitrile (2 mL), followed by the sequential addition of potassium carbonate (15 mg, 0.108 mmol) and **94-1** (9 mg, 0.054 mmol). The reaction system was stirred at 80°C for 16 h. After the reaction was completed, the reaction mixture was cooled to room temperature, and insoluble solids were removed by filtration. The filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5 mg of the title compound **94.** LC-MS (ESI): m/z 679.2 [M+H]$^+$. $^1$H NMR (400 MHz, DMSO-$d_6$) δ 8.39 (d, *J* = 4.9 Hz, 1H), 7.70 (d, *J* = 2.3 Hz, 1H), 7.63 (d, *J* = 2.3 Hz, 1H), 7.59 (d, *J* = 8.8 Hz, 2H), 7.55 (d, *J* = 8.4 Hz, 2H), 7.30 (d, *J* = 8.5 Hz, 2H), 7.07 (d, *J* = 8.8 Hz, 2H), 6.73 (d, *J* = 5.0 Hz, 1H), 5.07 (s, 2H), 4.89 - 4.76 (m, 2H), 4.42 (t, *J* = 5.2 Hz, 2H), 3.96 (t, *J* = 5.2 Hz, 2H), 3.47 - 3.36 (m, 1H), 3.06 - 2.85 (m, 2H), 2.94 (s, 3H), 2.15 - 2.00 (m, 2H), 1.68 (s, 6H), 1.60 - 1.41 (m, 2H).

**Example 95: Preparation of compound 95**

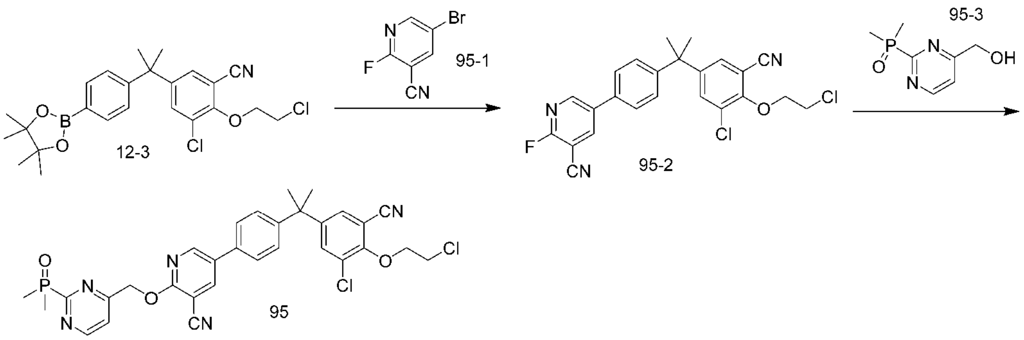


12-3

95-2

95

**Preparation of compound 95-2**

Compound **12-3** (100 mg, 0.497 mmol) was dissolved in 1,4-dioxane (10 mL) and water (3 mL), followed by the sequential addition of compound **95-1** (460 mg, 0.229 mmol), potassium carbonate (172 mg, 1.24 mmol), and Pd(dppf)$Cl_2$ (36 mg, 0.050 mmol). The reaction system was purged three times with nitrogen and stirred at 91°C for 2 h. After the reaction was completed, water (30 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by column chromatography (EA/PE = 0 to 20%) to obtain 210 mg of the title compound **95-2** in 93% yield. LC-MS (ESI): m/z 454.0 $[M+H]^+$.

**Preparation of compound 95**

Compound **95-2** (210 mg, 0.462 mmol) was dissolved in acetonitrile (8 mL), followed by the sequential addition of potassium carbonate (128 mg, 0.924 mmol) and **95-3** (186 mg, 0.462 mmol). The reaction system was stirred at 70°C for 0.5 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5 mg of the title compound **95.** LC-MS (ESI): m/z 620.2 $[M+H]^+$. $^1$H NMR (400 MHz, DMSO-$d_6$) δ 8.98 (d, *J* = 5.2 Hz, 1H), 8.73 (d, *J* = 2.5 Hz, 1H), 8.71 (d, *J* = 2.5 Hz, 1H), 7.71 - 7.63 (m, 5H), 7.36 (d, *J* = 8.5 Hz, 2H), 5.74 (s, 2H), 4.42 (t, *J* = 5.2 Hz, 2H), 3.95 (t, *J* = 5.2 Hz, 2H), 1.70 (d, *J* = 13.6 Hz, 6H), 1.69 (s, 6H). $^{31}$P NMR (162 MHz, DMSO-$d_6$) δ 34.17.

**Example 96: Preparation of compound 96**

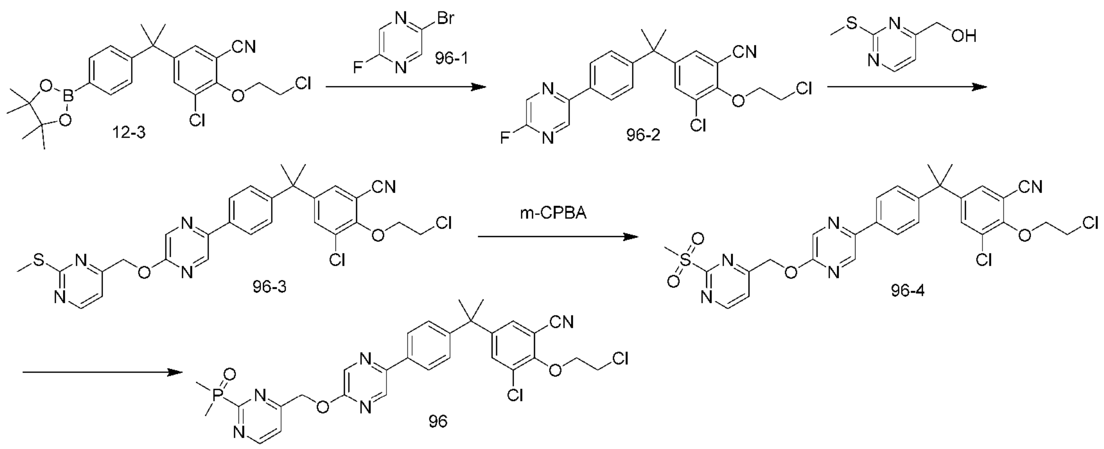


**Preparation of compound 96-2**

2-Bromo-5-fluoropyrazine (380 mg, 2.15 mmol), compound 12-3 (988.1 mg, 2.15 mmol), Pd(dppf)$Cl_2$ (157 mg, 214.72 μmol), and potassium carbonate (741 mg, 5.37 mmol) were weighed and added to a mixed solvent of $H_2O$ (5 mL) and 1,4-dioxane (15 mL). The system was purged three times with nitrogen, and the reaction was carried out at 100°C for 5 h. The reaction was monitored by LCMS until completion. The reaction mixture was poured into ice water and extracted with ethyl acetate (20 mL × 2). The organic phases were combined, dried over anhydrous sodium sulfate, subjected to suction filtration, and the filtrate was concentrated to obtain a crude product. The crude product was purified by normal-phase silica gel column chromatography (EA/PE = 0 to 40%) to obtain 750 mg of the title compound **96-2** in 81% yield. LC-MS (ESI): m/z 430.2 $[M+H]^+$.

**Preparation of compound 96-3**

Compound (2-(methylthio)pyrimidin-4-yl)methanol (279 mg, 1.78 mmol) was weighed and added to anhydrous tetrahydrofuran (20 mL), and sodium hydride (142.8 mg, 5.95 mmol, 60% purity) was added in an ice-water bath. The

reaction was carried out for 5 min, and then compound **96-2** (640 mg, 1.49 mmol) was added. After the addition was completed, the reaction was carried out at room temperature for 3 h. After the reaction was monitored by LCMS until completion, the reaction mixture was poured into ice water and extracted with ethyl acetate (20 mL × 2). The organic phases were combined, dried over anhydrous sodium sulfate, subjected to suction filtration, and the filtrate was concentrated to obtain a crude product. The crude product was purified by silica gel column chromatography (EA/PE = 0 to 40%) to obtain 560 mg of the title compound **96-3** in 66.5% yield. LC-MS (ESI): m/z 566.3[M+H]$^+$.

**Preparation of compound 96-4**

Compound **96-3** (250 mg, 441.31 μmol) was weighed and dissolved in dichloromethane (10 mL), and *m*-CPBA (305 mg, 1.77 mmol) was added with stirring. The reaction was carried out at room temperature for 2 h. After the reaction was monitored by LCMS until completion, the reaction mixture was poured into ice water and extracted with dichloromethane (20 mL × 2), followed by washing with saturated aqueous sodium bicarbonate solution. The organic phases were combined, dried over anhydrous sodium sulfate, subjected to suction filtration, and the filtrate was concentrated to obtain a crude product. The crude product was purified by silica gel column chromatography (EA/PE = 0 to 80%) to obtain 250 mg of the title compound **96-4** in 95% yield. LC-MS (ESI): m/z 598.2 [M+H]$^+$.

**Preparation of compound 96**

Compound **96-4** (180 mg, 300.75 μmol) and cesium carbonate (196 mg, 601.50 μmol) were weighed and added to acetonitrile (8 mL). After stirring for 5 min, dimethylphosphine oxide (47 mg, 601.50 μmol) was added. After the addition was completed, the reaction was carried out at 60°C for 2 h. After the reaction was monitored by LCMS until completion, the reaction mixture was cooled to room temperature, filtered, and the filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 24 mg of the title compound **96.** LC-MS (ESI): m/z 596.2 [M+H]$^+$. $^1$H NMR (400 MHz, DMSO-$d_6$) δ 8.95 (d, *J* = 5.2 Hz, 1H), 8.74 (d, *J* = 1.4 Hz, 1H), 8.59 (d, *J* = 1.4 Hz, 1H), 7.96 (d, *J* = 8.5 Hz, 2H), 7.73 - 7.66 (m, 2H), 7.63 (d, *J* = 2.3 Hz, 1H), 7.37 (d, *J* = 8.6 Hz, 2H), 5.63 (s, 2H), 4.42 (t, *J* = 5.2 Hz, 2H), 3.96 (t, *J* = 5.2 Hz, 2H), 1.72 (d, *J* = 13.7 Hz, 6H), 1.69 (s, 6H). $^{31}$P NMR (162 MHz, DMSO-$d_6$) δ 34.12 (s, 1P).

**Example 97: Preparation of compound 97**

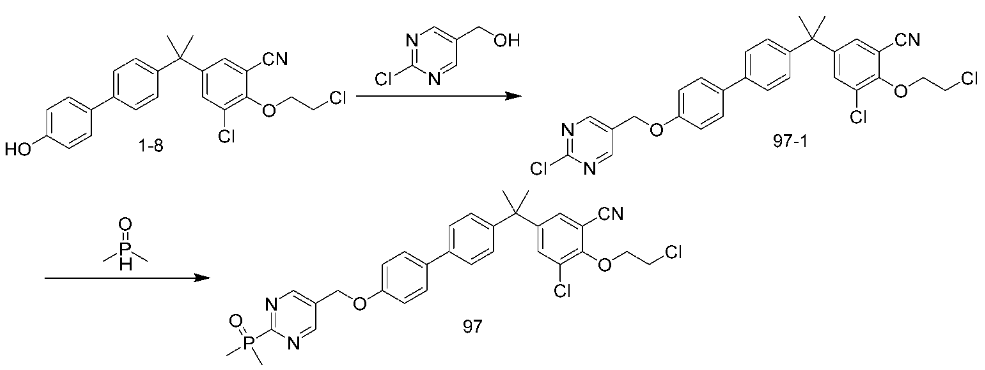


**Preparation of compound 97-1**

Compound **1-8** (200 mg, 469.12 μmol) was added to a three-necked flask, followed by the sequential addition of anhydrous DCM (10 mL), (2-chloropyrimidin-5-yl)methanol (88 mg, 609.85 μmol), and *N,N,N',N'*-tetramethylazodicarboxamide (162 mg, 938.23 μmol). The system was purged three times with nitrogen, and tributylphosphine (190 mg, 938.23 μmol) was added at 0°C. The reaction was stirred at 25°C for 3 h. After the reaction was completed, water (20 mL) was added, and the mixture was extracted with DCM (20 mL × 2). The organic phases were combined, dried, and concentrated to obtain a crude product. The crude product was purified by silica gel column chromatography (EtOAc/PE = 0 to 30%) to obtain 100 mg of the title compound **97-1** in 38.6% yield. LC-MS (ESI): 552.1 [M+H]$^+$.

**Preparation of compound 97**

Compound **97-1** (60 mg, 108.52 μmol) was dissolved in DMF (2 mL), followed by the sequential addition of tris(dibenzylideneacetone)dipalladium (10 mg, 10.85 μmol), 4,5-bis(diphenylphosphino)-9,9-dimethylxanthene (13 mg, 21.70 μmol), DIPEA (42 mg, 325.57 μmol), and dimethylphosphine oxide (25 mg, 325.57 μmol). The reaction system was stirred at 120°C for 3 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound **97.** LC-MS (ESI): m/z 594.3 $[M+H]^+$. $^1H$ NMR (400 MHz, DMSO-$d_6$) δ 9.09 (s, 2H), 7.70 (d, *J* = 2.3 Hz, 1H), 7.66 - 7.59 (m, 3H), 7.57 (d, *J* = 8.5 Hz, 2H), 7.30 (d, *J* = 8.5 Hz, 2H), 7.14 (d, *J* = 8.8 Hz, 2H), 5.31 (s, 2H), 4.42 (t, *J* = 5.2 Hz, 2H), 3.96 (t, *J* = 5.2 Hz, 2H), 1.77 (d, *J* = 13.7 Hz, 6H), 1.68 (s, 6H). $^{31}P$ NMR (162 MHz, DMSO-$d_6$) δ 34.02 (s, 1P).

**Example 98: Preparation of compound 98**

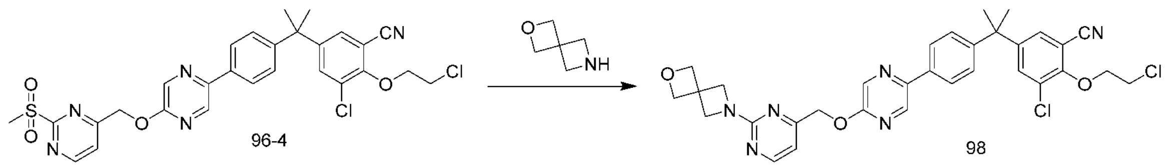


**Preparation of compound 98**

Compound **96-4** (60 mg, 100.25 μmol) and DIPEA (26 mg, 200.50 μmol) were weighed and added to acetonitrile (3 mL). After stirring for 5 min, 2-oxa-6-azaspiro[3.3]heptane (15 mg, 150.38 μmol) was added. After the addition was completed, the reaction was carried out at 60°C for 2 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 35 mg of the title compound **98.** LC-MS (ESI): m/z 617.4 $[M+H]^+$. $^1H$ NMR (400 MHz, DMSO-$d_6$) δ 8.74 (d, *J* = 1.4 Hz, 1H), 8.52 (d, *J* = 1.3 Hz, 1H), 8.31 (d, *J* = 5.0 Hz, 1H), 7.96 (d, *J* = 8.5 Hz, 2H), 7.70 (d, *J* = 2.3 Hz, 1H), 7.63 (d, *J* = 2.3 Hz, 1H), 7.37 (d, *J* = 8.5 Hz, 2H), 6.70 (d, *J* = 5.0 Hz, 1H), 5.33 (s, 2H), 4.70 (s, 4H), 4.42 (t, *J* = 5.2 Hz, 2H), 4.18 (s, 4H), 3.95 (t, *J* = 5.2 Hz, 2H), 1.69 (s, 6H).

**Example 99: Preparation of compound 99**

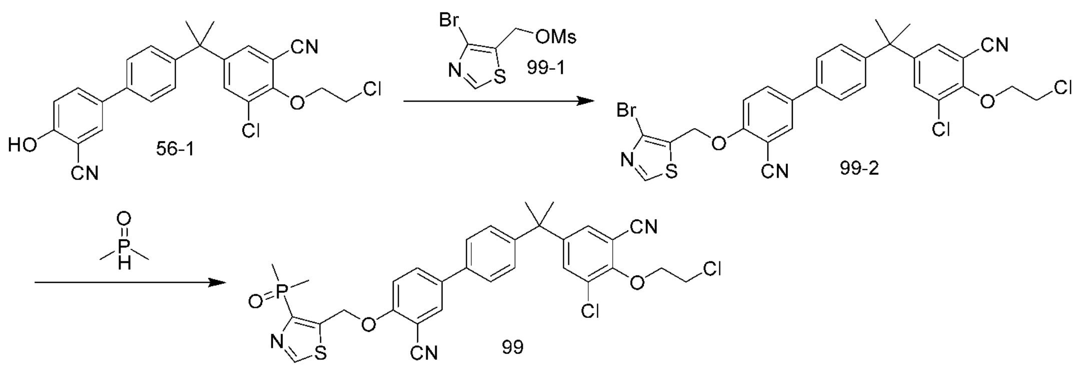

**Preparation of compound 99-2**

Compound **99-1** (60 mg, 220.47 μmol), compound **56-1** (109 mg, 24 μmol), and cesium carbonate (143 mg, 440 μmol) were dissolved in acetonitrile (2 mL), and the system was purged three times with nitrogen and then stirred at 75°C for 3 h. After the reaction was completed, water (15 mL) was added, and the mixture was extracted three times with dichloromethane (15 mL). The organic phases were combined, washed twice with saturated brine (10 mL), dried over anhydrous sodium sulfate, subjected to suction filtration, and the filtrate was concentrated to obtain a crude product. The crude product was purified by silica gel column chromatography (EA/PE = 0 to 20%) to obtain 105 mg of the title compound **99-2.** LC-MS (ESI): m/z 625.1 [M+H]+.

**Preparation of compound 99**

Compound **99-2** (85 mg, 141.11 μmol) was dissolved in DMF (2 mL), and $Pd_2(dba)_3$ (5.16 mg, 7.06 μmol), 4,5-bis(diphenylphosphino)-9,9-dimethylxanthene (4 mg, 7.06 μmol), DIPEA (36 mg, 282.22 μmol, 49.16 μL), and dimethylphosphine oxide (22 mg, 282.22 μmol) were added sequentially. The reaction system was stirred under microwave irradiation at 120°C for 2 h. After the reaction was completed, insoluble solids were removed by filtration. Water (10 mL) was added to the filtrate, and the mixture was extracted three times with dichloromethane (10 mL). The organic phases were combined, washed twice with saturated brine (15 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 45% acetonitrile gradient elution over 12 min; flow rate: 30 mL/min) to obtain 13 mg of the title compound **99.** LC-MS (ESI): m/z 624.2 [M+H]+. $^1$H NMR (400 MHz, DMSO-$d_6$) δ 9.27 (s, 1H), 8.08 (d, *J* = 2.4 Hz, 1H), 8.00 (dd, *J* = 8.9, 2.4 Hz, 1H), 7.70 (d, *J* = 2.3 Hz, 1H), 7.68 - 7.59 (m, 3H), 7.51 (d, *J* = 9.0 Hz, 1H), 7.33 (d, *J* = 8.5 Hz, 2H), 6.00 (s, 2H), 4.42 (t, *J* = 5.2 Hz, 2H), 3.96 (t, *J* = 5.2 Hz, 2H), 1.75 (d, *J* = 13.9 Hz, 6H), 1.68 (s, 6H). $^{31}$P NMR (162 MHz, DMSO-$d_6$) δ 34.17 (s, 1P).

**Example 100: Preparation of compound 100**

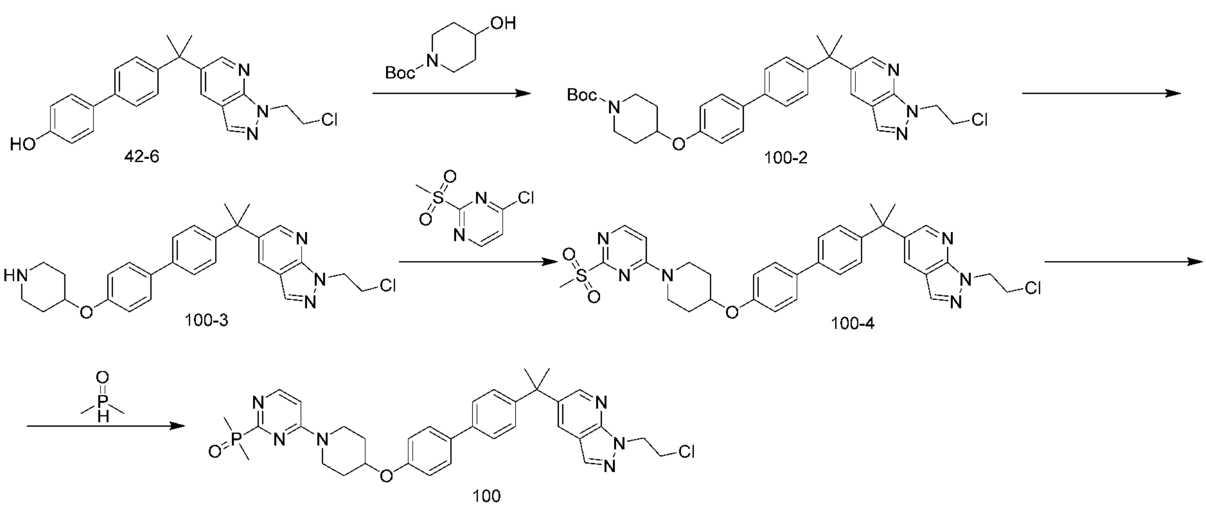

42-6 100-2 100-3 100-4 100

**Preparation of compound 100-2**

Compound **42-6** (620 mg, 1.58 mmol) and cyanomethylene(tri-n-butyl)phosphorane (572 mg, 2.37 mmol) were dissolved in toluene (7 mL). The reaction system was stirred at 100°C for 3 h. After the reaction was completed, the reaction mixture was cooled to room temperature. After the reaction system was filtered, saturated aqueous sodium chloride solution (15 mL) was added to the filtrate, and the mixture was extracted three times with ethyl acetate (10 mL). The organic phases were combined, dried, and concentrated to obtain a crude product. The crude product was purified by silica gel column chromatography (EtOAc/PE = 0 to 30%) to obtain 580 mg of the off-white title compound **100-2** in 63% yield. LC-MS (ESI): 575.4 [M+H]+.

**Preparation of compound 100-3**

Compound **100-2** (580 mg, 1.01 mol) was added to dichloromethane (6 mL), and trifluoromethanesulfonic acid (0.5 mL) was added dropwise in an ice bath. The reaction system was stirred at room temperature for 2 h. After the reaction was completed, the reaction mixture was concentrated to obtain 460 mg of a crude product of the title compound **100-3** in 96% yield. LC-MS (ESI): 475.3 $[M+H]^+$.

**Preparation of compound 100-4**

Compound **100-3** (200 mg, 421 μmol) and triethylamine (92 mg, 842 μmol) were dissolved in DCM (6 mL). 4-Chloro-2-(methylsulfonyl)pyrimidine (97 mg, 505 μmol) was added in portions in an ice bath. The reaction system was stirred at room temperature for 2 h. After the reaction was completed, the reaction system was filtered, then saturated aqueous sodium chloride solution (10 mL) was added to the filtrate, and the mixture was extracted three times with ethyl acetate (10 mL). The organic phases were combined, dried, subjected to suction filtration, and the filtrate was concentrated to obtain a crude product. The crude product was purified by silica gel column chromatography (EtOAc/PE = 0 to 20%) to obtain 165 mg of the title compound **100-4** as a white solid in 62% yield. LC-MS (ESI): 631.2 $[M+H]^+$.

**Preparation of compound 100**

Compound **100-4** (90 mg, 142 μmol), dimethylphosphine oxide (33 mg, 426 μmol), and cesium carbonate (93 mg, 284 μmol) were added to acetonitrile (3 mL). The reaction system was stirred at 70°C for 2 h. After the reaction was completed, the reaction mixture was cooled to room temperature. Saturated brine (5 mL) was added to the reaction mixture, and the mixture was extracted three times with EA (8 mL). The organic phases were combined, dried, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 30% acetonitrile gradient elution over 12 min; flow rate: 30 mL/min) to obtain 18 mg of the title compound **100.** LC-MS (ESI): m/z 629.5 $[M+H]^+$. $^1$H NMR (400 MHz, DMSO-$d_6$) δ 8.40 (d, *J* = 2.2 Hz, 1H), 8.29 (dd, *J* = 6.3, 1.3 Hz, 1H), 8.19 (d, *J* = 2.3 Hz, 1H), 8.17 (s, 1H), 7.63 - 7.48 (m, 4H), 7.31 (d, *J* = 8.5 Hz, 2H), 7.08 (d, *J* = 8.8 Hz, 2H), 6.96 (dd, *J* = 6.4, 3.1 Hz, 1H), 4.78 - 4.68 (m, 3H), 4.19 - 3.96 (m, 4H), 3.61 - 3.49 (m, 2H), 2.10 - 1.98 (m, 2H), 1.78 (s, 6H), 1.72 - 1.58 (m, 8H). $^{31}$P NMR (162 MHz, DMSO-$d_6$) δ 33.57.

**Example 101: Preparation of compound 101**

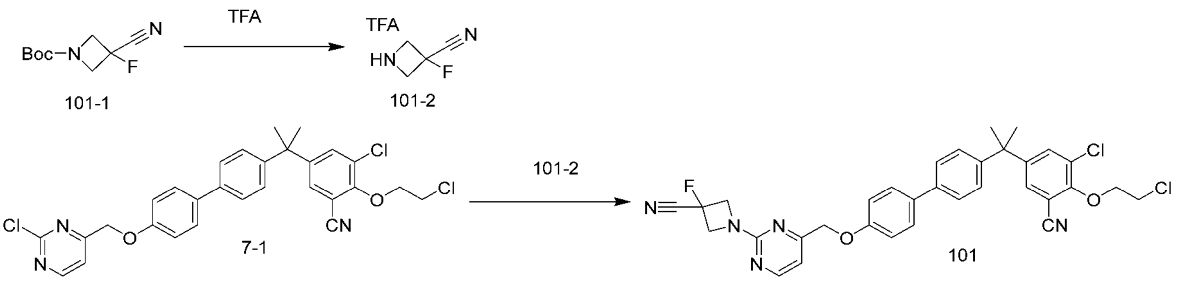

101-1 101-2 7-1 101

**Preparation of compound 101-2**

Compound **101-1** (100 mg, 1.0 mol) was added to dichloromethane (2 mL), and trifluoromethanesulfonic acid (0.3 mL) was added dropwise in an ice bath. The reaction system was stirred at room temperature for 2 h. After the reaction was completed, the reaction mixture was concentrated to obtain 110 mg of a crude product of the title compound in 92% yield.

**Preparation of compound 101**

Compound **101-2** (15 mg, 148.31 μmol), compound **7-1** (41 mg, 74.16 μmol), and TEA (15 mg, 148.31 μmol) were added to acetonitrile (2 mL). The reaction system was stirred at 70°C for 2 h. After the reaction was completed, the reaction mixture was cooled to room temperature. Saturated brine (5 mL) was added to the reaction mixture, and the mixture was extracted three times with EA (8 mL). The organic phases were combined, dried, subjected to suction filtration,

and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 30% acetonitrile gradient elution over 12 min; flow rate: 30 mL/min) to obtain 11 mg of the title compound **101** in 17% yield. LC-MS (ESI): m/z 616.4 $[M+H]^+$. $^1H$ NMR (400 MHz, DMSO-$d_6$) δ 8.46 (d, $J$ = 5.0 Hz, 1H), 7.70 (d, $J$ = 2.4 Hz, 1H), 7.64 (d, $J$ = 2.3 Hz, 1H), 7.60 (d, $J$ = 8.8 Hz, 2H), 7.56 (d, $J$ = 8.4 Hz, 2H), 7.30 (d, $J$ = 8.5 Hz, 2H), 7.08 (d, $J$ = 8.8 Hz, 2H), 6.95 (d, $J$ = 5.0 Hz, 1H), 5.10 (s, 2H), 4.74 (dd, $J$ = 11.2, 1.6 Hz, 1H), 4.69 (dd, $J$ = 11.2, 1.6 Hz, 1H), 4.48 (dd, $J$ = 11.3, 1.6 Hz, 1H), 4.46 - 4.38 (m, 3H), 3.96 (t, $J$ = 5.2 Hz, 2H), 1.68 (s, 6H).

**Example 102: Preparation of compound 102**

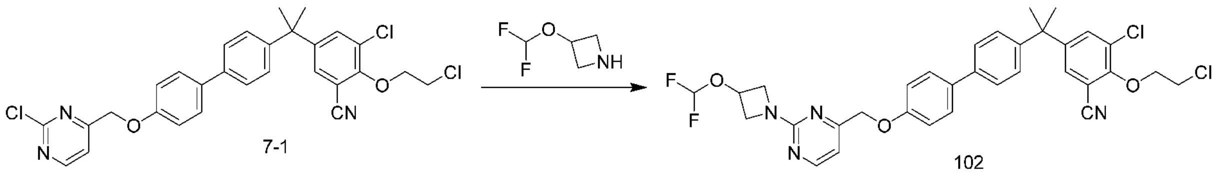

7-1

102

**Preparation of compound 102**

Compound **7-1** (65 mg, 117.57 μmol), 3-(difluoromethoxy)azetidine (18 mg, 141 μmol), and TEA (24 mg, 235.13 μmol) were added to acetonitrile (2 mL). The reaction system was stirred at 90°C for 2 h. After the reaction was completed, the reaction mixture was cooled to room temperature. Saturated brine (5 mL) was added to the reaction mixture, and the mixture was extracted three times with EA (8 mL). The organic phases were combined, dried, and subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 30% acetonitrile gradient elution over 12 min; flow rate: 30 mL/min) to obtain 14 mg of the title compound **102.** LC-MS (ESI): m/z 639.3 $[M+H]^+$. $^1H$ NMR (400 MHz, DMSO-$d_6$) δ 8.39 (d, $J$ = 5.0 Hz, 1H), 7.70 (d, $J$ = 2.3 Hz, 1H), 7.64 (d, $J$ = 2.4 Hz, 1H), 7.59 (d, $J$ = 8.7 Hz, 2H), 7.55 (d, $J$ = 8.4 Hz, 2H), 7.30 (d, $J$ = 8.4 Hz, 2H), 7.07 (d, $J$ = 8.8 Hz, 2H), 6.82 (d, $J$ = 5.0 Hz, 1H), 6.80 (d, $J$ = 74.8 Hz, 1H), 5.13 - 5.00 (m, 1H), 5.08 (s, 2H), 4.46 - 4.33 (m, 4H), 4.07 - 3.89 (m, 4H), 1.68 (s, 6H).

**Example 103: Preparation of compound 103**

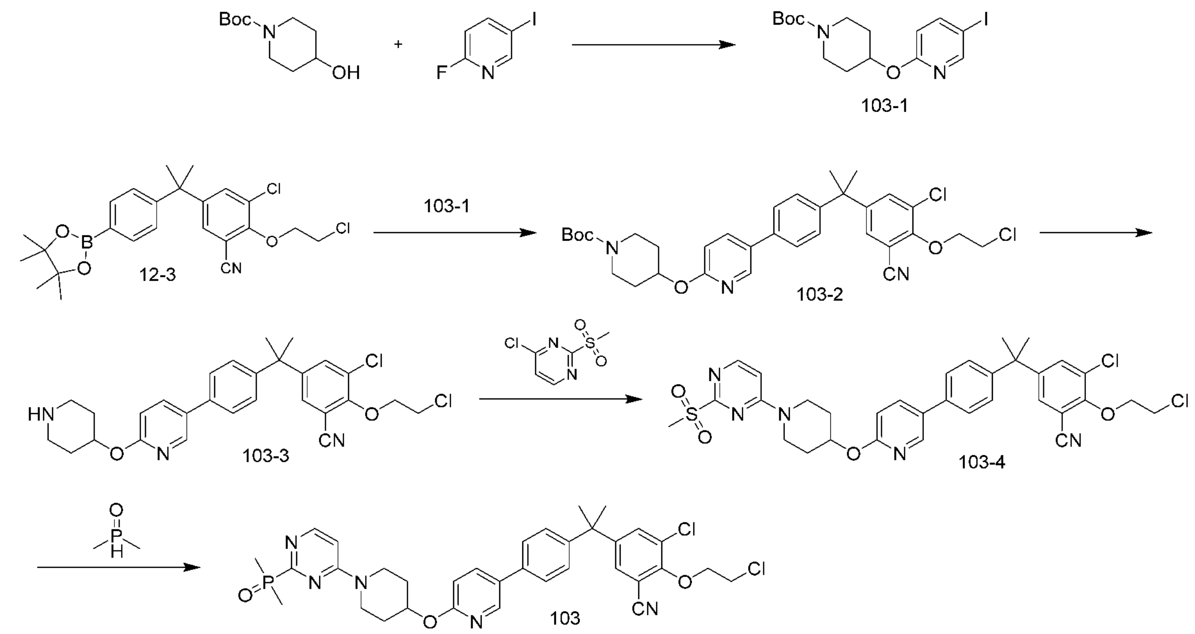


103-1

12-3

103-2

103-3

103-4

103

**Preparation of compound 103-1**

*tert*-Butyl 4-hydroxypiperidine-1-carboxylate (500 mg, 2.48 mmol) was dissolved in tetrahydrofuran (5 mL). The system was purged three times with nitrogen, and then sodium hydride (149 mg, 3.72 mmol, 60% purity) was added in an ice bath. After 30 min, 2-fluoro-5-iodopyridine (663 mg, 2.98 mmol) was added, and the reaction system was stirred at 15°C for 15 h. After the reaction was completed, the reaction mixture was filtered. Saturated aqueous sodium chloride solution (15 mL) was added to the filtrate, and the mixture was extracted three times with ethyl acetate (10 mL). The organic phases were combined, dried, and concentrated to obtain a crude product. The crude product was purified by silica gel column chromatography (EtOAc/PE = 0 to 30%) to obtain 470 mg of the off-white title compound **103-1** in 46.8% yield. LC-MS (ESI): 405.2 $[M+H]^+$.

**Preparation of compound 103-2**

Compound **103-1** (470 mg, 1.16 mmol) was dissolved in 1,4-dioxane (10 mL), followed by the sequential addition of cesium carbonate (754 mg, 2.32 mmol) and [1,1'-bis(diphenylphosphino)ferrocene]dichloropalladium(II) dichloromethane complex (46 mg, 5.8 μmol). The system was purged three times with nitrogen and then stirred at 105°C for 3 h. After the reaction was completed, the reaction mixture was cooled to room temperature and filtered. Saturated aqueous sodium chloride solution (20 mL) was added to the filtrate, and the mixture was extracted three times with ethyl acetate (10 mL). The organic phases were combined, dried, and concentrated to obtain a crude product. The crude product was purified by silica gel column chromatography (EtOAc/PE = 0 to 40%) to obtain 320 mg of the off-white title compound **103-2** in 45% yield. LC-MS (ESI): 610.2 $[M+H]^+$.

**Preparation of compound 103-3**

Compound **103-2** (320 mg, 524 μmol) was added to dichloromethane (6 mL), and trifluoromethanesulfonic acid (0.5 mL) was added dropwise in an ice bath. The reaction system was stirred at room temperature for 2 h. After the reaction was completed, the reaction mixture was concentrated to obtain 255 mg of a crude product of the title compound **103-3** in 95% yield.

**Preparation of compound 103-4**

Compound **103-3** (255 mg, 499 μmol) and triethylamine (101 mg, 998 μmol) were dissolved in DCM (4 mL). 4-Chloro-2-(methylsulfonyl)pyrimidine (98 mg, 505 μmol) was added in portions in an ice bath, and the mixture was stirred at room temperature for 2 h. After the reaction was completed, the reaction mixture was filtered. Saturated aqueous sodium chloride solution (10 mL) was added to the filtrate, and the mixture was extracted three times with ethyl acetate (10 mL). The organic phases were combined, dried, subjected to suction filtration, and the filtrate was concentrated to obtain a crude product. The crude product was purified by silica gel column chromatography (EtOAc/PE = 0 to 20%) to obtain 206 mg of the title compound **103-4** in 61% yield. LC-MS (ESI): 666.4 $[M+H]^+$.

**Preparation of compound 103**

Compound **103-4** (100 mg, 150 μmol), dimethylphosphine oxide (35 mg, 450 μmol), and cesium carbonate (98 mg, 300 μmol) were added to acetonitrile (3 mL), and the mixture was stirred at 90°C for 2 h. After the reaction was completed, the reaction mixture was cooled to room temperature. Saturated brine (10 mL) was added to the reaction mixture, and the mixture was extracted three times with EA (10 mL). The organic phases were combined, dried, and subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 30% acetonitrile gradient elution over 12 min; flow rate: 30 mL/min) to obtain 22 mg of the title compound. LC-MS (ESI): m/z 664.4 $[M+H]^+$. $^1$H NMR (400 MHz, DMSO-$d_6$) δ 8.47 (s, 1H), 8.30 (dd, *J* = 6.3, 1.2 Hz, 1H), 8.01 (dd, *J* = 8.6, 2.6 Hz, 1H), 7.71 (d, *J* = 2.4 Hz, 1H), 7.65 (d, *J* = 2.3 Hz, 1H), 7.60 (d, *J* = 8.5 Hz, 2H), 7.34 (d, *J* = 8.5 Hz, 2H), 6.97 (dd, *J* = 6.4, 3.1 Hz, 1H), 6.89 (d, *J* = 8.6 Hz, 1H), 5.40 - 5.27 (m, 1H), 4.42 (t, 2H), 4.23 - 4.03 (m, 2H), 3.96 (t, 2H), 3.52 (t, *J* = 11.1 Hz, 2H), 3.44 - 3.36 (m, 2H), 2.09 (d, *J* = 13.2 Hz, 2H), 1.69 (s, 6H), 1.67 (d, *J* = 13.6 Hz, 6H). $^{31}$P NMR (162 MHz, DMSO-$d_6$) δ 33.56 (s, 1P).

**Example 104: Preparation of compound 104**

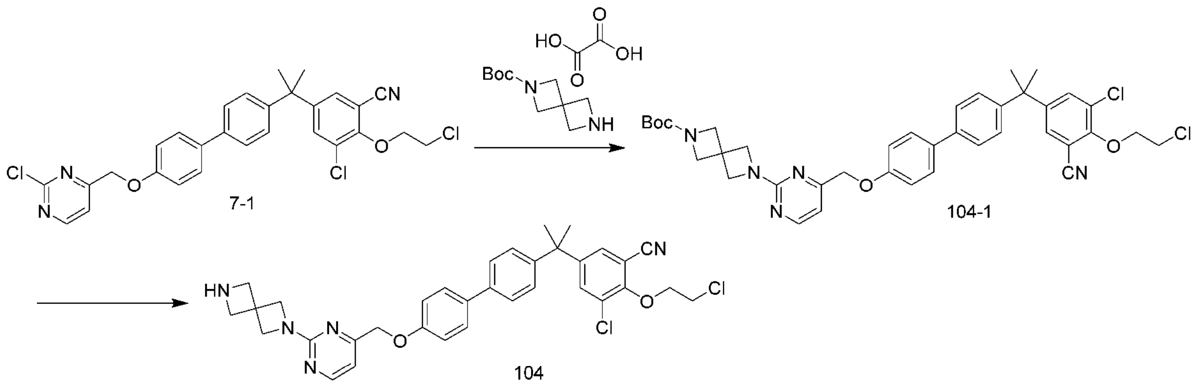

7-1 104-1 104

### Preparation of compound 104-1

Compound **7-1** (30 mg, 0.054 mmol) and tert-butyl 2,6-diazaspiro[3.3]heptane-2-carboxylate oxalate (26 mg, 0.054 mmol) were dissolved in anhydrous acetonitrile (2 mL), and potassium carbonate (15 mg, 0.108 mmol) was added. The reaction system was stirred at 80°C for 12 h. After the reaction was completed, the reaction mixture was cooled to room temperature, insoluble solids were removed by filtration, and the filtrate was concentrated to obtain a crude product. The crude product was purified by column chromatography (EA/PE = 0 to 20%) to obtain 38 mg of the title compound **104-1** in 98% yield. LC-MS (ESI): m/z 714.3 $[M+H]^+$.

### Preparation of compound 104

Compound **104-1** (40 mg, 0.056 mmol) was dissolved in dichloromethane (3 mL), and trifluoroacetic acid (1 mL) was added dropwise in an ice bath. The reaction system was stirred and maintained at temperature for 2 h. After the reaction was completed, the reaction mixture was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 12 mg of the title compound **104.** LC-MS (ESI): m/z 614.3 $[M+H]^+$. $^1H$ NMR (400 MHz, Chloroform-d) δ 8.26 (d, *J* = 5.1 Hz, 1H), 7.48 - 7.38 (m, 5H), 7.31 - 7.29 (m, 1H), 7.17 - 7.12 (m, 2H), 6.97 - 6.90 (m, 2H), 6.80 (d, *J* = 4.9 Hz, 1H), 4.95 (s, 2H), 4.36 (t, *J* = 6.2 Hz, 2H), 4.27 (s, 4H), 4.23 (s, 4H), 3.81 (t, *J* = 6.2 Hz, 2H), 1.62 (s, 6H).

### Example 105: Preparation of compound 105

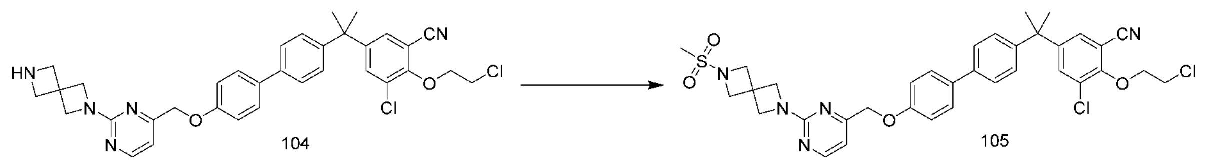

104 105

### Preparation of compound 105

Compound **104** (60 mg, 0.098 mmol) and triethylamine (39 mg, 0.390 mmol) were dissolved in dichloromethane (2 mL). The system was cooled to 0°C, and a solution of methanesulfonyl chloride (45 mg, 0.390 mmol) in dichloromethane (1 mL) was added dropwise. After the addition was completed, the reaction system was stirred at 0°C for 1 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **105.** LC-MS (ESI): m/z 692.2 $[M+H]^+$. $^1H$ NMR (400 MHz, DMSO-$d_6$) δ 8.36 (d, *J*= 5.1 Hz, 1H), 7.70 (s, 1H), 7.67 - 7.46 (m, 5H), 7.30 (d, *J* = 8.0 Hz, 2H), 7.07 (d, *J* = 8.2 Hz, 2H), 6.78 (d, *J* = 5.1 Hz, 1H), 5.05 (s, 2H), 4.42 (t, *J* = 5.1 Hz, 2H), 4.20 (s, 4H), 4.08 (s, 4H), 3.95 (t, *J* = 5.2 Hz, 2H), 3.01 (s, 3H), 1.68 (s, 6H).

**Example 106: Preparation of compound 106**

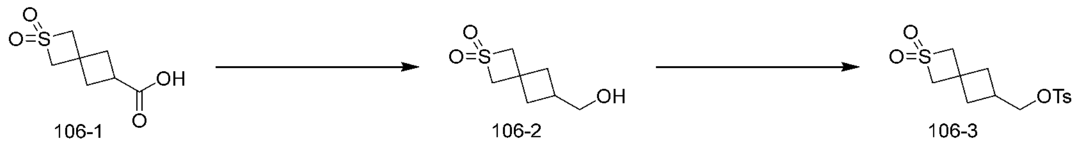


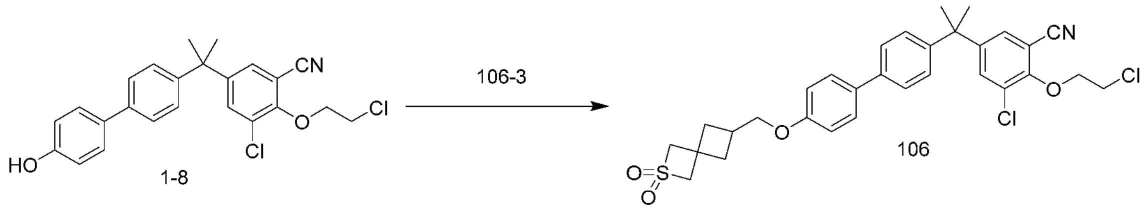


**Preparation of compound 106-2**

Compound **106-1** (50 mg, 0.26 mmol) was dissolved in tetrahydrofuran (1 mL) in a 25 mL two-necked flask, and the mixture was cooled to 0°C under a nitrogen atmosphere. A solution of borane in tetrahydrofuran (0.32 mL, 0.32 mmol, 1 M) was slowly added. The reaction mixture was stirred at 0°C for 2 h. After the reaction was completed, the reaction mixture was quenched with methanol, and the mixture was concentrated under reduced pressure to obtain 70 mg of a crude product of the title compound **106-2.**

**Preparation of compound 106-3**

Compound **106-2** (70 mg, 0.19 mmol) was dissolved in dichloromethane (2 mL) in a 25 mL two-necked flask, followed by the sequential addition of DMAP (2 mg, 0.02 mmol) and triethylamine (40 mg, 0.40 mmol). Under a nitrogen atmosphere, p-toluenesulfonyl chloride (45 mg, 0.24 mmol) was added at 0°C, and the reaction mixture was stirred at room temperature for 2 h. After the reaction was completed, the reaction mixture was concentrated under reduced pressure. The resulting crude product was purified by column chromatography (EA/PE = 0 to 20%) to obtain 43 mg of the title compound **106-3.** $^{1}$H NMR (400 MHz, Chloroform-d) δ 7.81 - 7.73 (m, 2H), 7.42 - 7.31 (m, 2H), 4.12 (s, 2H), 4.03 (s, 2H), 3.99 (d, *J* = 5.4 Hz, 2H), 2.68 - 2.59 (m, 1H), 2.46 (s, 3H), 2.44 - 2.36 (m, 2H), 2.23 - 2.14 (m, 2H).

**Preparation of compound 106**

Compound **106-3** (30 mg, 0.09 mmol) was dissolved in DMF (0.5 mL) in a 25 mL single-necked flask, followed by the sequential addition of compound **1-8** (50 mg, 0.12 mmol) and cesium carbonate (59 mg, 0.18 mmol). The reaction mixture was stirred at 80°C for 2 h. After the reaction was completed, the reaction mixture was cooled to room temperature and subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 13.4 mg of the title compound **106.** LC-MS (ESI): m/z 584.2 [M+H]$^{+}$. $^{1}$H NMR (400 MHz, DMSO-$d_6$) δ 7.70 (d, *J* = 2.3 Hz, 1H), 7.64 (d, *J* = 2.3 Hz, 1H), 7.56 (t, *J* = 9.0 Hz, 4H), 7.30 (d, *J* = 8.5 Hz, 2H), 7.00 (d, *J* = 8.8 Hz, 2H), 4.47 - 4.38 (m, 2H), 4.27 (s, 2H), 4.20 (s, 2H), 4.05 - 3.88 (m, 4H), 2.74 - 2.63 (m, 1H), 2.47 - 2.40 (m, 2H), 2.25 - 2.14 (m, 2H), 1.69 (d, *J* = 6.8 Hz, 6H).

**Example 107: Preparation of compound 107**

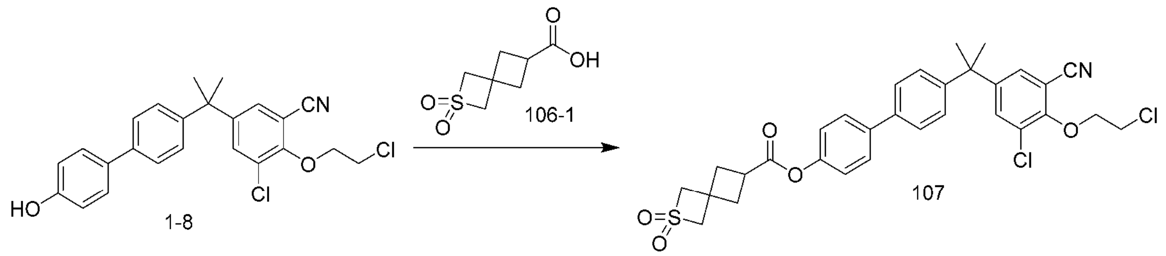

### Preparation of compound 107

Compound **106-1** (50 mg, 0.26 mmol) was dissolved in dichloromethane (2 mL) in a 25 mL single-necked flask, followed by the sequential addition of compound **1-8** (110 mg, 0.32 mmol), DMAP (3 mg, 0.03 mmol), DIPEA (68 mg, 0.53 mmol), and EDCI (60 mg, 0.32 mmol). The mixture was stirred at room temperature for 16 h. After the reaction was completed, the reaction mixture was washed with water (10 mL). The organic phase was dried over anhydrous sodium sulfate, filtered, and the filtrate was subjected to rotary evaporation until dryness under reduced pressure. The crude product was purified by silica gel column chromatography (EA/PE = 0 to 30%), and further subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 61 mg of the title compound **107.** LC-MS (ESI): m/z 598.4 $[M+H]^+$. $^1H$ NMR (400 MHz, DMSO-$d_6$) δ 7.76 - 7.67 (m, 3H), 7.65 (d, *J* = 2.3 Hz, 1H), 7.61 (d, *J* = 8.1 Hz, 2H), 7.34 (d, *J* = 8.1 Hz, 2H), 7.22 (d, *J* = 8.7 Hz, 2H), 4.42 (t, *J* = 5.1 Hz, 2H), 4.33 (s, 2H), 4.24 (s, 2H), 3.96 (t, *J* = 5.1 Hz, 2H), 3.45 (p, *J* = 8.5 Hz, 1H), 2.66 (d, *J* = 8.5 Hz, 4H), 1.69 (s, 6H).

### Example 108: Preparation of compound 108

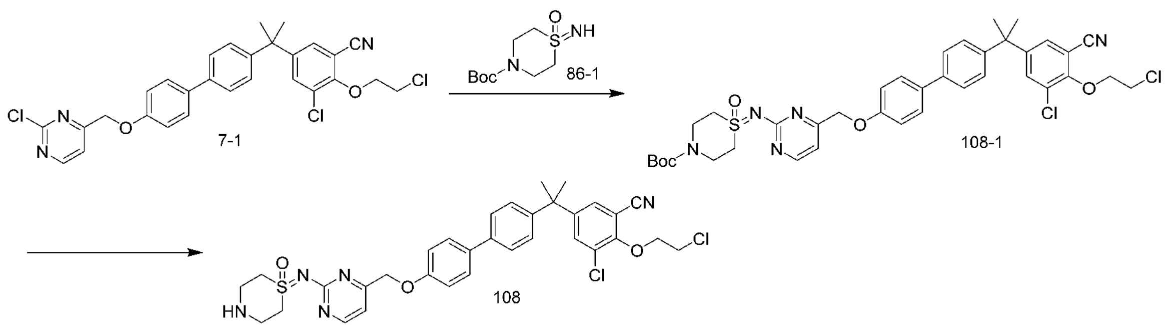

### Preparation of compound 108-1

Compound **7-1** (190 mg, 343.66 μmol) was dissolved in DMF (3 mL), followed by the sequential addition of tris(dibenzylideneacetone)dipalladium (31.5 mg, 34.37 μmol), 4,5-bis(diphenylphosphino)-9,9-dimethylxanthene (40 mg, 68.73 μmol), DIEA (133 mg, 1.03 mmol), and compound **86-1** (161 mg, 687.31 μmol). The reaction system was stirred at 120°C for 3 h. After the reaction was monitored by LCMS until completion, the reaction mixture was poured into ice water and extracted twice with ethyl acetate (20 mL). The organic phases were combined, dried over anhydrous sodium sulfate, subjected to suction filtration, and the filtrate was concentrated to obtain a crude product. The crude product was purified by silica gel column chromatography (EA/PE = 0 to 40%) to obtain 150 mg of the title compound **108-1** in 58% yield. LC-MS (ESI): m/z 750.4 $[M+H]^+$.

### Preparation of compound 108

Compound **108-1** (130 mg, 0.17 mmol) was dissolved in dichloromethane (5 mL), and a solution of hydrogen chloride in 1,4-dioxane (5 mL, 4 M) was added dropwise with stirring. After the addition was completed, the reaction was carried out at room temperature overnight. After the reaction was monitored by LCMS until completion, the reaction mixture was concentrated to obtain a crude product. The crude product was subjected to preparative purification (preparative method: mobile phase: A: 0.1% formic acid in water; B: acetonitrile; chromatographic column: Welch Ultimate

AQ-C18 250 × 21.2 mm; column temperature: 25°C; gradient: 45% to 65% acetonitrile gradient elution over 12 min; flow rate: 30 mL/min) to obtain 56 mg of the title compound **108.** LC-MS (ESI): m/z 650.6 $[M+H]^+$. $^1H$ NMR (400 MHz, DMSO-$d_6$) δ 8.44 (d, *J* = 5.0 Hz, 1H), 7.70 (d, *J* = 2.4 Hz, 1H), 7.64 (d, *J* = 2.3 Hz, 1H), 7.60 (d, *J* = 8.8 Hz, 2H), 7.55 (d, *J* = 8.4 Hz, 2H), 7.30 (d, *J* = 8.4 Hz, 2H), 7.07 (d, *J* = 8.8 Hz, 2H), 6.96 (d, *J* = 5.1 Hz, 1H), 5.10 (s, 2H), 4.42 (t, *J* = 5.2 Hz, 2H), 3.96 (t, *J* = 5.2 Hz, 2H), 3.67 - 3.55 (m, 2H), 3.32 - 3.26 (m, 2H), 3.21 - 3.08 (m, 2H), 3.05 - 2.92 (m, 2H), 1.68 (s, 6H).

**Example 109: Preparation of compound 109**

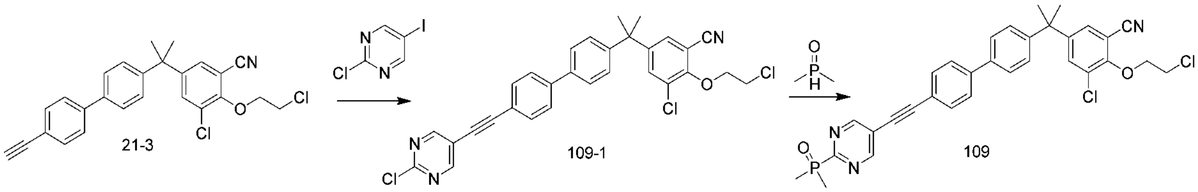
21-3
109-1
109

**Preparation of compound 109-**

Compound **21-3** (190 mg, 343.66 μmol), 2-chloro-5-iodopyrimidine (161 mg, 687.31 μmol), bis(triphenylphosphine)palladium(II) dichloride (81 mg, 115.11 μmol), copper(I) iodide (91 mg, 115.11 μmol), and triethylamine (582 mg, 5.76 mmol) were added to acetonitrile (10 mL). The reaction was stirred at 50°C for 4 h. The reaction was monitored by LCMS until completion. The reaction mixture was poured into ice water and extracted twice with ethyl acetate (20 mL). The organic phases were combined, dried over anhydrous sodium sulfate, subjected to suction filtration, and the filtrate was concentrated to obtain a crude product. The crude product was purified by silica gel column chromatography (EA/PE = 0 to 40%) to obtain 100 mg of the title compound **109-1** in 32% yield. LC-MS (ESI): m/z 546.2 $[M+H]^+$.

**Preparation of compound 109**

Compound **109-1** (110 mg, 201.14 μmol) was dissolved in DMF (3 mL), followed by the sequential addition of tris(dibenzylideneacetone)dipalladium (19 mg, 20.11 μmol), 4,5-bis(diphenylphosphino)-9,9-dimethylxanthene (24 mg, 40.23 μmol), DIEA (78 mg, 603.43 μmol), and dimethylphosphine oxide (47 mg, 603.43 μmol). The reaction system was stirred at 120°C for 3 h. After the reaction was completed, the reaction mixture was cooled to room temperature, and insoluble solids were removed by filtration. Water (10 mL) was added to the filtrate, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 45 mg of the title compound **109.** LC-MS (ESI): m/z 588.2 $[M+H]^+$. $^1H$ NMR (400 MHz, DMSO-$d_6$) δ 9.19 (s, 2H), 7.81 (d, *J* = 8.5 Hz, 2H), 7.72 (d, *J* = 8.4 Hz, 2H), 7.71 (m, 1H), 7.70 (d, *J* = 8.4 Hz, 2H), 7.65 (d, *J* = 2.3 Hz, 1H), 7.38 (d, *J* = 8.5 Hz, 2H), 4.43 (t, *J* = 5.2 Hz, 2H), 3.96 (t, *J* = 5.2 Hz, 2H), 1.79 (d, *J* = 13.8 Hz, 6H), 1.70 (s, 6H). $^{31}P$ NMR (162 MHz, DMSO-$d_6$) δ 34.38 (s, 1P).

**Example 110: Preparation of compound 110**

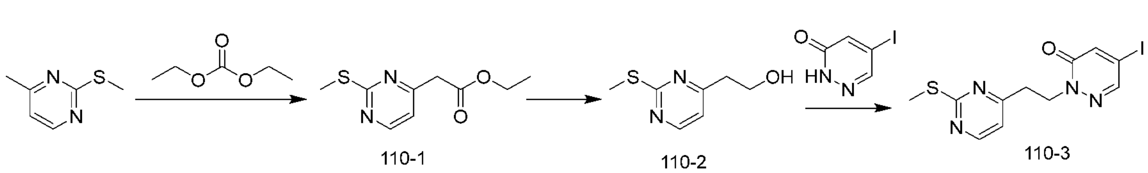
110-1
110-2
110-3

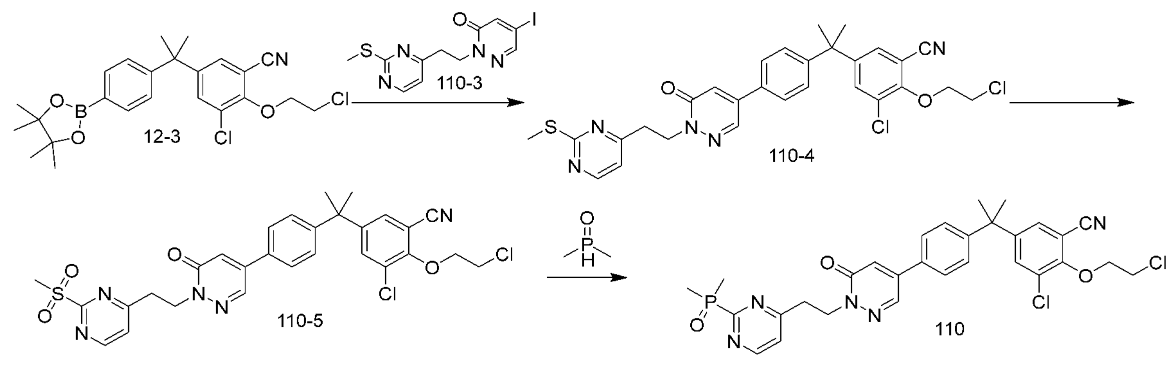
12-3
110-3
110-4
110-5
110

### Preparation of compound 110-1

Compound 4-methyl-2-(methylthio)pyrimidine (10.2 g, 72.75 mmol) was dissolved in anhydrous THF (30 mL). Under an argon atmosphere, the temperature was lowered to -10°C. At this temperature, a solution of NaHMDS (1 M in THF, 218 mL, 218 mmol) was slowly added dropwise to the reaction system, and the mixture was stirred at -10°C for 30 min. Compound diethyl carbonate (10.3 g, 87.31 mmol) was slowly added dropwise to the reaction system. After the addition was completed, the mixture was allowed to warm to room temperature naturally and stirred for 3 h. After the reaction was completed, saturated aqueous ammonium chloride solution (100 mL) was added dropwise to quench the reaction, followed by the addition of a hydrochloric acid solution (100 mL, 2 M). The mixture was extracted three times with ethyl acetate (200 mL). The organic phases were combined, dried over anhydrous sodium sulfate, subjected to suction filtration, and the filtrate was concentrated. The resulting residue was purified by silica gel column chromatography (EA/PE = 0 to 30%) to obtain 12.5 g of the pale yellow title compound in 81% yield. LC-MS (ESI): m/z $[M+H]^+$: 213.1.

### Preparation of compound 110-2

Compound **110-1** (10.5 g, 49.47 mmol) was dissolved in anhydrous THF (50 mL). Under an argon atmosphere, the temperature was lowered to -5°C, and DIBAL-H (49.5 mL, 74.10 mmol, 1.5 M solution in toluene) was added dropwise. The mixture was allowed to warm to room temperature naturally and stirred for 2 h. After the reaction was completed, water (50 mL) was added, and the mixture was extracted three times with ethyl acetate (200 mL). The organic phases were combined, dried over anhydrous sodium sulfate, subjected to suction filtration, and the filtrate was concentrated. The resulting residue was purified by silica gel column chromatography (EA/PE = 0 to 20%) to obtain 4.5 g of the title compound **110-2** as a brown oil in 36% yield. LC-MS (ESI): m/z $[M+H]^+$: 171.1.

### Preparation of compound 110-3

Compound **110-2** (1 g, 5.87 mmol), compound 5-iodopyridazin-3(2H)-one (1.43 g, 6.46 mmol), and triphenylphosphine (1.43 g, 8.80 mmol) were dissolved in anhydrous THF (20 mL). Under an argon atmosphere, the temperature was lowered to 0°C, and DEAD (1.53 g, 8.80 mmol) was added dropwise at 0°C. After the addition was completed, the mixture was allowed to warm to room temperature naturally and stirred for 16 h. After the reaction was completed, water (20 mL) was added, and the mixture was extracted three times with ethyl acetate (50 mL). The organic phases were combined, dried over anhydrous sodium sulfate, subjected to suction filtration, and concentrated. The resulting residue was purified by silica gel column chromatography (EA/PE = 0 to 40%) to obtain 1.1 g of the title compound **110-3** as a white solid in 50% yield. LC-MS (ESI): m/z 374.8 $[M+H]^+$.

### Preparation of compound 110-4

Compounds **12-3** (1.0 g, 2.17 mmol) and **110-3** (812 mg, 2.17 mmol) were dissolved in a mixed solvent of 1,4-dioxane (15 mL) and water (1 mL). [1,1'-Bis(diphenylphosphino)ferrocene]dichloropalladium(II) (159 mg, 0.22 mmol) and potassium carbonate (900 mg, 6.51 mmol) were added. The system was purged four times with argon, heated to 110°C, and stirred for 3 h. After the reaction was completed, the reaction mixture was cooled to room temperature, water (20 mL) was added, and the mixture was extracted three times with ethyl acetate (50 mL). The organic phases were combined, dried over anhydrous sodium sulfate, subjected to suction filtration, and the filtrate was concentrated. The resulting residue was purified by silica gel column chromatography (EA/PE = 0 to 50%) to obtain 660 mg of the title compound **110-4** as a yellow oil in 52% yield. LC-MS (ESI): m/z 579.9 $[M+H]^+$.

**Preparation of compound 110-5**

Compound **110-4** (300 mg, 0.52 mmol) was dissolved in THF (2 mL) and water (2 mL), and potassium peroxymonosulfate (1.28 g, 2.08 mmol) was added. The mixture was stirred at room temperature for 16 h. The reaction was monitored by LCMS until the starting material was consumed. After the reaction was completed, water (10 mL) was added, and the mixture was extracted three times with ethyl acetate (10 mL). The organic phases were combined, dried over anhydrous sodium sulfate, subjected to suction filtration, and the filtrate was concentrated to obtain 300 mg of a crude product of the title compound **110-5** in 95% yield. The product was used directly in the next step without purification.

**Preparation of compound 110**

Compound **110-5** (300 mg, 0.49 mmol) and dimethylphosphine oxide (191 mg, 1.96 mmol) were dissolved in acetonitrile (3 mL). Potassium carbonate (271 mg, 3.72 mmol) was added at room temperature, and the mixture was heated to 70°C and stirred for 2 h. The reaction was monitored by LCMS until completion. Water (10 mL) was added, and the mixture was extracted twice with ethyl acetate (20 mL). The organic phases were combined and concentrated. The resulting residue was subjected to preparative purification (preparative column: Atlantis TM T3 prep OBD TM, 19 * 250 mm * 10 μm; flow rate: 20 mL/min; mobile phase: A - 0.1% aqueous FA solution, B - acetonitrile; gradient: 56 to 64% acetonitrile content, retention time 7.4 to 8.0 min) to obtain 21.7 mg of the title compound **110.** LC-MS (ESI): m/z 610.3 $[M+H]^+$.

$^1$H NMR (400 MHz, DMSO-$d_6$) δ 8.84 (d, *J* = 5.1 Hz, 1H), 8.30 (d, *J* = 2.1 Hz, 1H), 7.77 (d, *J* = 8.4 Hz, 2H), 7.71 (d, *J* = 2.1 Hz, 1H), 7.65 (d, *J* = 2.1 Hz, 1H), 7.59 (dd, *J* = 5.0, 3.3 Hz, 1H), 7.39 (d, *J* = 8.4 Hz, 2H), 7.20 (d, *J* = 2.0 Hz, 1H), 4.51 (t, *J* = 6.8 Hz, 2H), 4.42 (t, *J* = 5.2 Hz, 2H), 3.96 *(t, J*= 5.2 Hz, 2H), 3.33 - 3.27 (m, 2H), 1.69 (s, 6H), 1.67 (d, *J* = 13.6 Hz, 6H). $^{31}$P NMR (162 MHz, DMSO-*d6*) δ 33.60 (s,1P).

**Example 111: Preparation of compound 111**

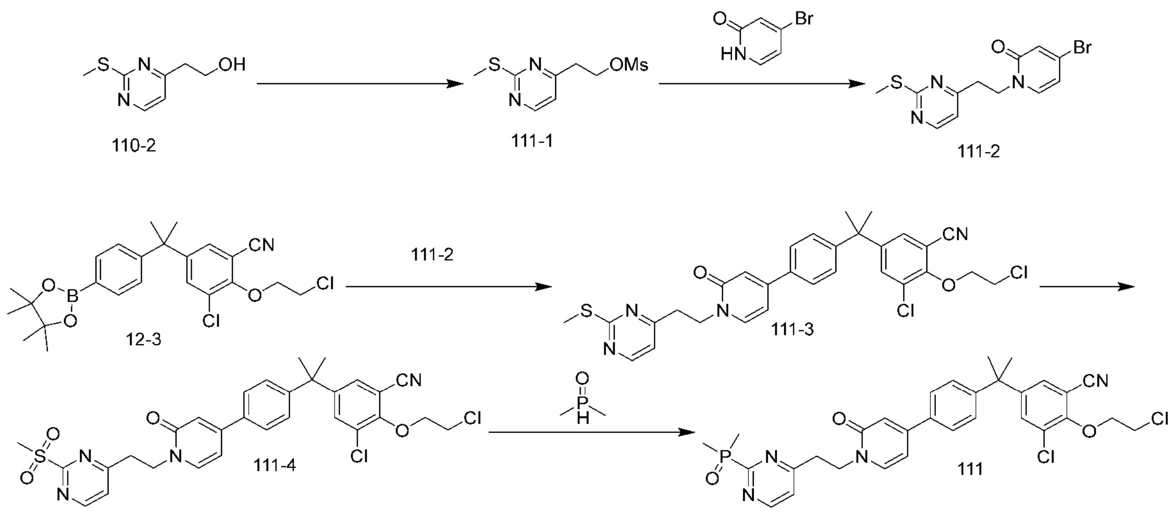


**Preparation of compound 111-1**

Compound **110-2** (2 g, 11.75 mmol) and triethylamine (3.6 g, 35.25 mmol) were dissolved in THF (20 mL). The system was cooled to 0°C, and methanesulfonyl chloride (3.4 g, 29.38 mmol) was slowly added dropwise. The mixture was warmed to room temperature and stirred for 1 h. The reaction was monitored by LCMS until completion. Ice water (20 mL) was added to quench the reaction, and the mixture was extracted three times with ethyl acetate (50 mL). The organic phases were combined, dried over anhydrous sodium sulfate, subjected to suction filtration, and the filtrate was concentrated. The resulting residue was purified by normal-phase silica gel column chromatography (PE/EA = 0 to 70%) to obtain 2.1 g of the title compound **111-1** in 70% yield. LC-MS (ESI): m/z 153.1 $[M+H]^+$.

**Preparation of compound 111-2**

Compound **111-1** (2 g, 8.05 mmol) and compound 4-bromopyridin-2(1H)-one (1.68 g, 9.66 mmol) were dissolved

in 1,4-dioxane (20 mL). Cesium carbonate (2.23 g, 16.10 mmol) was added at room temperature, and the mixture was heated to 90°C and stirred for 3 h. The reaction was monitored by LCMS until completion. Ice water (20 mL) was added to quench the reaction, and the mixture was extracted three times with ethyl acetate (50 mL). The organic phases were combined, dried over anhydrous sodium sulfate, subjected to suction filtration, and the filtrate was concentrated. The resulting residue was purified by silica gel column chromatography (EA/PE = 0 to 100%) to obtain 1.4 g of the title compound **111-2** in 50% yield. LC-MS (ESI): m/z 326.0 $[M+H]^+$.

**Preparation of compound 111-3**

Compounds **12-3** (1.00 g, 2.17 mmol) and **111-2** (0.78 g, 2.39 mmol) were dissolved in 1,4-dioxane (15 mL). [1,1'-Bis(diphenylphosphino)ferrocene]dichloropalladium(II) (0.16 g, 0.22 mmol) and an aqueous potassium carbonate solution (2 mol/L, 3.3 mL, 6.51 mmol) were added. The system was purged four times with argon, heated to 110°C, and stirred for 3 h. After the reaction was completed, the reaction mixture was cooled to room temperature, and water (30 mL) was added to quench the reaction. The mixture was filtered through a pad of diatomite, and the filter cake was rinsed with ethyl acetate (50 mL). The filtrates were combined and extracted three times with ethyl acetate (50 mL). The organic phases were combined, dried over anhydrous sodium sulfate, subjected to suction filtration, and the filtrate was concentrated. The resulting residue was purified by silica gel column chromatography (MeOH/DCM = 0 to 5%) to obtain 1 g of the title compound **111-3** in 64% yield. LC-MS (ESI): m/z 578.9 $[M+H]^+$.

**Preparation of compound 111-4**

Compound **111-3** (200.00 mg, 0.35 mmol) was dissolved in a mixed solvent of tetrahydrofuran (2 mL) and water (2 mL). Potassium peroxymonosulfate triple salt (0.86 g, 1.40 mmol) was added, and the mixture was stirred at room temperature for 16 h. The reaction was monitored by LCMS until completion. The reaction was quenched with water (5 mL) and extracted three times with ethyl acetate (10 mL). The organic phases were combined, dried over anhydrous sodium sulfate, subjected to suction filtration, and the filtrates were combined and concentrated. The resulting residue was purified by preparative TLC to obtain 50 mg of the title compound **111-4** in 24% yield. LC-MS (ESI): m/z 611.1 $[M+H]^+$.

**Preparation of compound 111**

Compound **111-4** (250 mg, 0.041 mmol) and dimethylphosphine oxide (16 mg, 0.21 mmol) were dissolved in acetonitrile (1 mL). Potassium carbonate (17 mg, 0.12 mmol) was added at room temperature, and the mixture was heated to 85°C and stirred for 16 h. The reaction was monitored by LCMS until completion. The mixture was cooled to room temperature, filtered through a pad of diatomite, and rinsed with acetonitrile (5 mL). The filtrates were combined, concentrated, and then purified by preparative TLC to obtain a crude product. The crude product was further purified by reversed-phase preparative chromatography (prep-HPLC (Waters 2767/Qda, Column: Sunfire C18 19 * 250 mm, 10 μm; Mobile Phase A: 0.1% FA/$H_2O$, B: ACN; flow rate: 20 mL/min; gradient: 51% to 51%; Retention Time: 7.4-8.5 min of 16 min)) to obtain 2.7 mg of the title compound 111. LC-MS (ESI): m/z 609.3 $[M+H]^+$. $^1H$ NMR (400 MHz, Chloroform-d) δ 8.71 (s, 1H), 7.50 (d, *J* = 8.3 Hz, 2H), 7.45 (d, *J* = 2.3 Hz, 1H), 7.37 (d, *J* = 7.1 Hz, 1H), 7.33 (d, *J* = 2.3 Hz, 1H), 7.26 - 7.22 (m, 3H), 6.77 (s, 1H), 6.37 (d, *J* = 6.7 Hz, 1H), 4.50 - 4.40 (m, 4H), 3.87 (t, *J*= 6.1 Hz, 2H), 3.41 (t, *J* = 6.1 Hz, 2H), 1.88 (d, *J* = 13.5 Hz, 6H), 1.68 (s, 6H). $^{31}P$ NMR (162 MHz, $CDCl_3$) δ 35.24 (s,1P).

**Examples 112 and 113: Preparation of compounds 112 and 113**

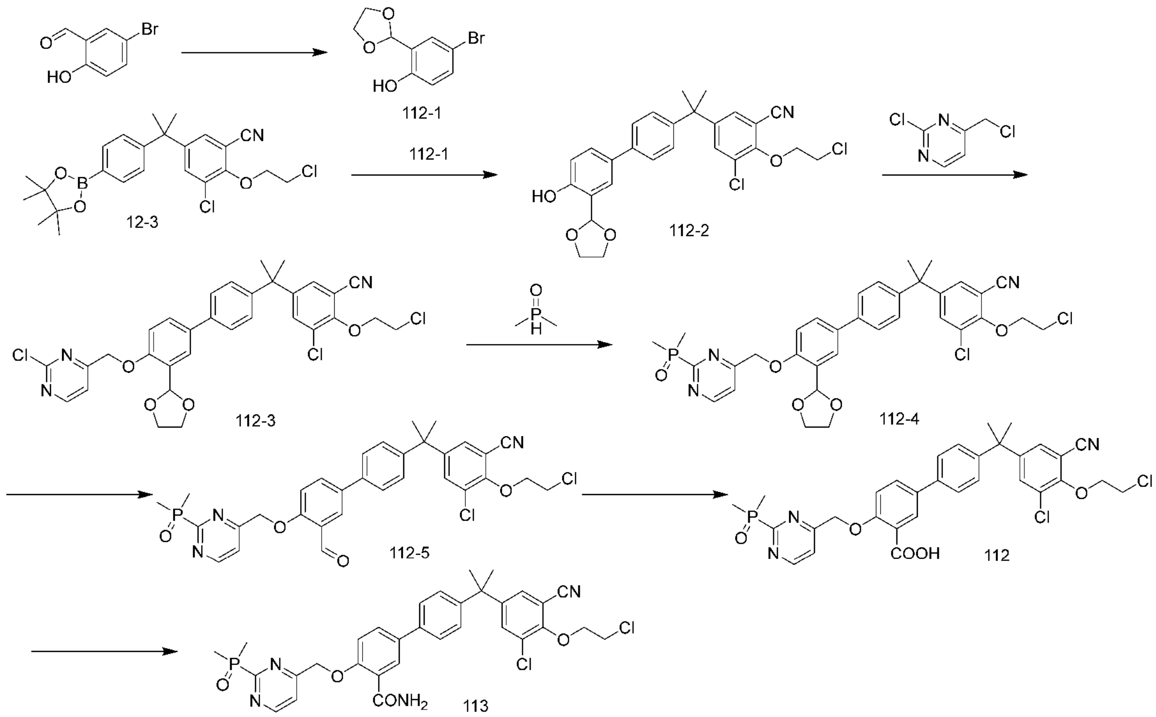

112-1 12-3 112-2 112-3 112-4 112-5 112 113

## Preparation of compound 112-1

2-Hydroxy-5-bromobenzaldehyde (3 g, 14.59 mmol) was dissolved in toluene (90 mL), and ethylene glycol (2.8 g, 44.77 mmol) andp-toluenesulfonic acid (257 mg, 1.49 mmol) were added. The reaction system was heated to 160°C and stirred for 6 h. The reaction was monitored by LCMS until completion. The system was cooled to room temperature and quenched with ice water (50 mL). The mixture was extracted three times with ethyl acetate (100 mL). The organic phases were combined, dried over anhydrous sodium sulfate, subjected to suction filtration, and the filtrate was concentrated. The resulting residue was purified by normal-phase silica gel column chromatography (EA/0.1% TEA + PE = 0 to 30%) to obtain 1.6 g of the title compound **112-1** in 44% yield. LC-MS (ESI): m/z 244.7 $[M-H]^-$.

## Preparation of compound 112-2

Compound **12-3** (400 mg, 0.87 mmol), compound **112-1** (213 mg, 0.87 mmol), $Pd(dppf)Cl_2$ (64 mg, 0.087 mmol), and potassium carbonate (361 mg, 2.61 mmol) were dissolved in 1,4-dioxane (15 mL). The system was purged with argon, heated to 110°C, and stirred for 2 h. The reaction was monitored by LCMS until completion. The system was cooled to room temperature, filtered through a pad of diatomite, and rinsed with ethyl acetate. The filtrates were combined and concentrated. The resulting residue was subjected to silica gel column chromatography (EA/0.1% TEA + PE = 0 to 50%) to obtain 200 mg of the title compound **112-2** in 50% yield. LC-MS (ESI): m/z 495.8 $[M-H]^-$.

## Preparation of compound 112-3

Compound **112-2** (800 mg, 1.60 mmol) and 2-chloro-4-(chloromethyl)pyrimidine (286 mg, 1.77 mmol) were dissolved in acetonitrile (8 mL). Cesium carbonate (1.6 g, 4.8 mmol) was added at room temperature, and the mixture was heated to 70°C and stirred for 2 h. The reaction was monitored by LCMS until completion. The mixture was cooled to room temperature, filtered, and the filter cake was rinsed with ethyl acetate. The filtrates were combined and concentrated. The resulting residue was purified by silica gel column chromatography (EA/PE = 0 to 50%) to obtain 760 mg of the title compound **112-3** in 76% yield. LC-MS (ESI): m/z 624.1 $[M+3]^+$.

## Preparation of compound 112-4

Compound **112-3** (760 mg, 1.22 mmol) and dimethylphosphine oxide (380 mg, 4.88 mmol) were dissolved in DMF (7 mL). Palladium acetate (27 mg, 0.12 mmol), 1,3-bis(diphenylphosphino)propane (50 mg, 0.12 mmol), and DIPEA (790 mg, 6.1 mmol) were added at room temperature, and the system was purged three times with argon. The reaction mixture was stirred under microwave irradiation at 120°C for 30 min. The reaction was monitored by LCMS until

completion. Water (30 mL) was added, and the mixture was extracted three times with ethyl acetate (50 mL), washed with saturated brine (50 mL), dried over anhydrous sodium sulfate, subjected to suction filtration, and the filtrate was concentrated. The resulting residue was purified by silica gel column chromatography (MeOH/DCM = 0 to 10%) to obtain 800 mg of the title compound **112-4** in 98% yield. LC-MS (ESI): m/z 666.1 $[M+H]^+$.

**Preparation of compound 112-5**

Compound **112-4** (800 mg, 1.2 mmol) was dissolved in tetrahydrofuran (5 mL). Hydrochloric acid (4 mL, 1 M) was added at room temperature, and the mixture was stirred at room temperature for 2 h. The reaction was monitored by LCMS until completion. An aqueous solution (30 mL) was added, and the mixture was extracted three times with ethyl acetate (50 mL), washed with saturated brine (50 mL), dried over anhydrous sodium sulfate, subjected to suction filtration, and the filtrate was concentrated. The resulting residue was purified by silica gel column chromatography (MeOH/DCM = 0 to 15%) to obtain 356 mg of the title compound **112-5** in 48% yield. LC-MS (ESI): m/z 622.1 $[M+H]^+$.

**Preparation of compound 112**

Compound **112-5** (356 mg, 0.57 mmol) was dissolved in a mixed solvent of *tert*-butanol (5 mL) and water (1 mL). Sodium chlorite (64 mg, 0.71 mmol), sodium dihydrogen phosphate (680 mg, 5.7 mmol), and 2-methyl-2-butene (400 mg, 5.7 mmol) were added at room temperature, and the mixture was stirred at room temperature for 16 h. The reaction was monitored by LCMS until completion. Saturated brine (30 mL) was added, and the mixture was extracted three times with ethyl acetate (50 mL). The organic phase was washed with saturated brine (50 mL), dried over anhydrous sodium sulfate, subjected to suction filtration, and the filtrate was concentrated. The resulting residue was purified by silica gel column chromatography (MeOH/DCM = 0 to 15%) to obtain 300 mg of the title compound 112. LC-MS (ESI): m/z 636.0 $[M-H]^-$. $^1H$ NMR (400 MHz, DMSO-$d_6$) $\delta$ 9.00 (d, *J* = 5.1 Hz, 1H), 8.10 - 8.02 (m, 1H), 7.84 (s, 1H), 7.71 (d, *J* = 2.1 Hz, 1H), 7.68 - 7.59 (m, 2H), 7.56 (d, *J* = 8.2 Hz, 2H), 7.31 (d, *J* = 8.2 Hz, 2H), 7.20 (d, *J* = 8.7 Hz, 1H), 5.37 (s, 2H), 4.42 (t, *J* = 5.2 Hz, 2H), 3.96 (t, *J* = 5.2 Hz, 2H), 1.77 (d, *J* = 13.7 Hz, 6H), 1.68 (s, 6H). $^{31}P$ NMR (162 MHz, DMSO-$d_6$) $\delta$ 34.48 (s,1P).

**Preparation of compound 113**

Compound **112** (50 mg, 0.078 mmol) was dissolved in DMF (1 mL). Ammonium chloride (6.3 mg, 0.12 mmol), HATU (44 mg, 0.12 mmol), and DIPEA (30 mg, 0.23 mmol) were added at room temperature, and the mixture was stirred at room temperature for 2 h. The reaction was monitored by LCMS until completion. The mixture was purified by preparative HPLC (Waters 2767/Qda, Column: XBridge C18 19 * 250 mm, 10 $\mu$m; mobile phase A: 0.1% TFA/$H_2O$, mobile phase B: acetonitrile; flow rate: 20 mL/min; elution gradient: 55% to 65%; retention time: 8.0-9.2 min of 16 min) to obtain 6 mg of the title compound **113.** LC-MS (ESI): m/z 637.3 $[M+H]^+$. $^1H$ NMR (400 MHz, DMSO-$d_6$) $\delta$ 9.01 (d, *J* = 5.2 Hz, 1H), 8.00 (d, *J* = 2.5 Hz, 1H), 7.98 (s, 1H), 7.77 (dd, *J* = 5.1, 3.2 Hz, 1H), 7.74 (dd, *J* = 8.6, 2.5 Hz, 1H), 7.70 (d, *J* = 2.3 Hz, 1H), 7.64 (d, *J* = 2.3 Hz, 2H), 7.59 (d, *J* = 8.4 Hz, 2H), 7.33 (d, *J* = 8.4 Hz, 2H), 7.26 (d, *J* = 8.8 Hz, 1H), 5.50 (s, 2H), 4.42 (t, *J* = 5.2 Hz, 2H), 3.96 (t, *J* = 5.2 Hz, 2H), 1.76 (d, *J* = 13.7 Hz, 6H), 1.69 (s, 6H). $^{31}P$ NMR (162 MHz, DMSO-d6) $\delta$ 34.02 (s,1P).

**Example 114: Preparation of compound 114**

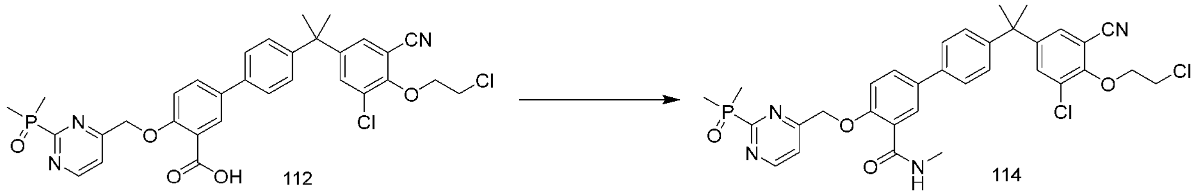

**Preparation of compound 114**

Compound **112** (50 mg, 0.078 mmol) was dissolved in DMF (1 mL). Methylamine hydrochloride (7.9 mg, 0.12 mmol), HATU (44 mg, 0.12 mmol), and DIPEA (30 mg, 0.23 mmol) were added at room temperature, and the mixture was stirred at room temperature for 2 h. The reaction was monitored by LCMS until completion. The mixture was filtered, and the resulting filtrate was purified by reversed-phase preparative chromatography (preparative HPLC: Waters 2767/Qda, column: XBridge XBridge C18 19 * 250 mm, 10 $\mu$m; mobile phase A: 0.1%FA/$H_2O$, mobile phase B: ACN; flow rate: 20

mL/min; elution gradient: 59% to 69%; retention time: 7.4-8.6 min of 16 min) to obtain 16.4 mg of the title compound **114.** LC-MS (ESI): m/z 651.3 $[M+H]^+$. $^1H$ NMR (400 MHz, DMSO-$d_6$) δ 9.02 (d, *J* = 5.2 Hz, 1H), 8.40 (q, *J* = 4.7 Hz, 1H), 7.93 (d, *J* = 2.4 Hz, 1H), 7.78 - 7.68 (m, 3H), 7.64 (d, *J* = 2.3 Hz, 1H), 7.59 (d, *J* = 8.4 Hz, 2H), 7.33 (d, *J* = 8.4 Hz, 2H), 7.25 (d, *J* = 8.7 Hz, 1H), 5.49 (s, 2H), 4.42 (t, *J* = 5.2 Hz, 2H), 3.96 (t, *J* = 5.2 Hz, 2H), 2.87 (d, *J* = 4.6 Hz, 3H), 1.76 (d, *J* = 13.7 Hz, 6H), 1.69 (s, 6H). $^{31}P$ NMR (162 MHz, DMSO-$d_6$) δ 34.01 (s, 1P).

**Example 115: Preparation of compound 115**

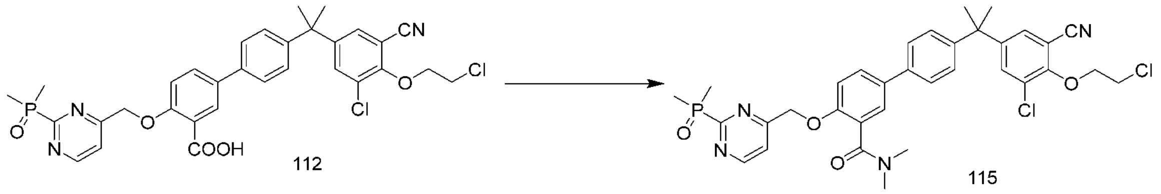
112
115

**Preparation of compound 115**

Compound **112** (50 mg, 0.078 mmol) was dissolved in DMF (1 mL). Dimethylamine hydrochloride (9.5 mg, 0.12 mmol), HATU (44 mg, 0.12 mmol), and DIPEA (30 mg, 0.23 mmol) were added at room temperature, and the reaction mixture was stirred at room temperature for 2 h. The reaction was monitored by LCMS until completion. The reaction mixture was filtered, and the resulting filtrate was purified by reversed-phase preparative chromatography (Waters 2767/Qda, column: XBridge C18 19 * 250 mm, 10 μm; mobile phase A: 0.1%FA/$H_2O$, mobile phase B: ACN; flow rate: 20 mL/min; gradient: 59% to 69%; retention time: 8.0-8.8 min of 16 min) to obtain 9.6 mg of the title compound **115.** LC-MS (ESI): m/z 665.3 $[M+H]^+$. $^1H$ NMR (400 MHz, DMSO-$d_6$) δ 9.03 (d, *J* = 5.2 Hz, 1H), 7.73 - 7.66 (m, 2H), 7.63 (d, *J* = 2.3 Hz, 1H), 7.62 - 7.57 (m, 3H), 7.52 (d, *J* = 2.3 Hz, 1H), 7.31 (d, *J* = 8.5 Hz, 2H), 7.23 (d, *J* = 8.8 Hz, 1H), 5.41 (s, 2H), 4.42 (t, *J* = 5.2 Hz, 2H), 3.96 (t, *J* = 5.2 Hz, 2H), 3.05 (s, 3H), 2.86 (s, 3H), 1.76 (d, *J* = 13.7 Hz, 6H), 1.68 (s, 6H). $^{31}P$ NMR (162 MHz, DMSO-$d_6$) δ 33.99 (s,1P).

**Example 116: Preparation of compound 116**

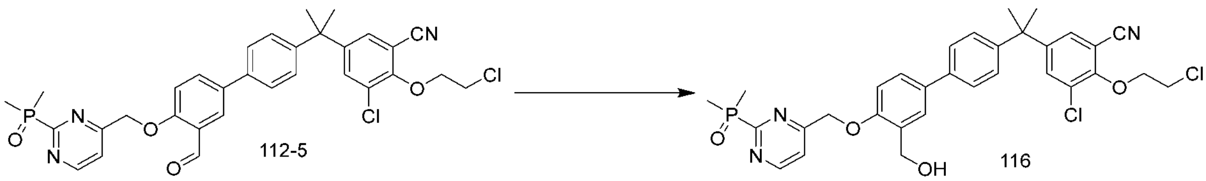
112-5
116

**Preparation of compound 116**

Compound **112-5** (100 mg, 0.16 mmol) was dissolved in methanol (5 mL). Sodium borohydride (24 mg, 0.643 mmol) was added at room temperature, and the mixture was stirred at room temperature for 2 h. The reaction was monitored by LCMS until completion. The mixture was subjected to rotary evaporation until dryness. The resulting residue was purified by reversed-phase preparative chromatography (Waters 2767/Qda, column: XBridge XBridge C18 19 * 250 mm, 10 μm; mobile phase A: 0.1% FFA/$H_2O$, mobile phase B: ACN; flow rate: 20 mL/min; gradient: 50% to 59%; retention time: 7.0-8.0 min of 16 min) to obtain 20.2 mg of the title compound 116. LC-MS (ESI): m/z 624.3 $[M+H]^+$. $^1H$ NMR (400 MHz, DMSO-$d_6$) δ 9.01 (d, *J = 5.1* Hz, 1H), 7.77 - 7.73 (m, 1H), 7.72 - 7.68 (m, 2H), 7.63 (d, *J* = 2.1 Hz, 1H), 7.56 (d, *J* = 8.2 Hz, 2H), 7.50 (d, 1H), 7.32 (d, *J* = 8.3 Hz, 2H), 7.09 (d, *J* = 8.6 Hz, 1H), 5.36 (s, 2H), 5.17 (t, *J* = 5.7 Hz, 1H), 4.70 (d, *J* = 5.6 Hz, 2H), 4.42 (t, *J* = 5.2 Hz, 2H), 3.96 (t, *J* = 5.2 Hz, 2H), 1.77 (d, *J* = 13.7 Hz, 6H), 1.69 (s, 6H). $^{31}P$ NMR (162 MHz, DMSO-$d_6$) δ 34.00 (s,1P).

**Example 117: Preparation of compound 117**

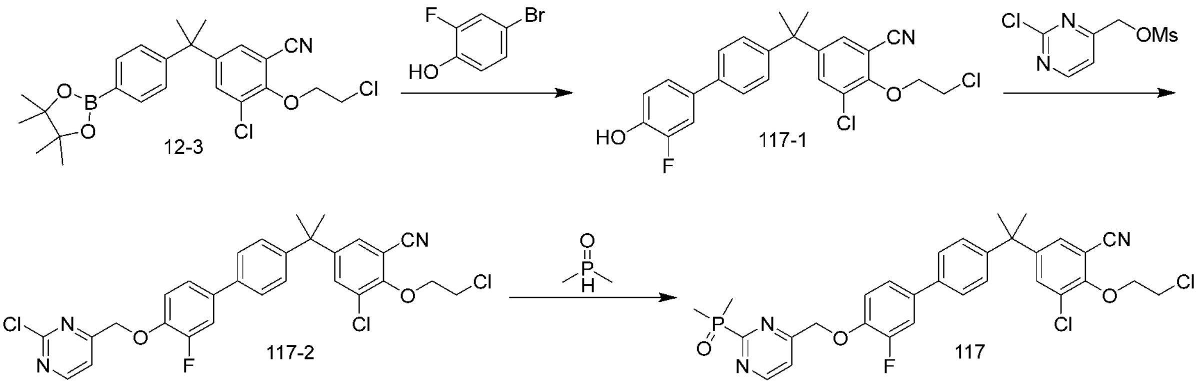

12-3 117-1 117-2 117

### Preparation of compound 117-1

Compound 12-3 (500 mg, 1.09 mmol) and 2-fluoro-4-bromophenol (250 mg, 1.31 mmol) were dissolved in 1,4-dioxane (8 mL). Pd(dppf)$Cl_2$ (80 mg, 0.11 mmol) and an aqueous potassium carbonate solution (2 mol/L, 1.6 mL, 3.27 mmol) were added. The system was purged four times with argon, heated to 110°C, and stirred for 3 h. The reaction was monitored by LCMS until completion, and then cooled to room temperature. The pH was adjusted to acidic with 2 mol/L aqueous hydrochloric acid solution. The mixture was filtered through a pad of diatomite, and the filter cake was rinsed with ethyl acetate (30 mL). The filtrates were combined, washed once with saturated brine (80 mL), dried over anhydrous sodium sulfate, subjected to suction filtration, and the filtrate was concentrated. The resulting residue was purified by silica gel column chromatography (EA/PE = 0 to 30%) to obtain 300 mg of the title compound **117-1** in 62.4% yield. LC-MS (ESI): m/z 441.9 $[M-H]^-$.

### Preparation of compound 117-2

Compound **117-1** (240 mg, 0.54 mmol) and compound (2-chloropyrimidin-4-yl)methyl methanesulfonate (132.25 mg, 0.59 mmol) were dissolved in acetonitrile (3 mL). Cesium carbonate (264 mg, 0.81 mmol) was added at room temperature, and the mixture was heated to 70°C and stirred for 1.5 h. The reaction was monitored by TLC until completion. The mixture was filtered, and the filter cake was rinsed with acetonitrile (10 mL). The filtrates were combined and concentrated to obtain 400 mg of a crude product of the title compound **117-2**. The crude product was used directly in the next step without purification.

### Preparation of compound 117

Compound **117-2** (200 mg, 0.35 mmol), dimethylphosphine oxide (109 mg, 1.40 mmol), DIPEA (227 mg, 1.75 mmol), 1,3-bis(diphenylphosphino)propane (22 mg, 0.052 mmol), and solvent DMF (2 mL) were added to a microwave tube. The mixture was stirred until a clear solution was obtained. Palladium acetate (16 mg, 0.07 mmol) was added at room temperature. The system was purged three times with argon, heated to 120°C, and the reaction was carried out under microwave irradiation for 0.5 h. The reaction was monitored by LCMS until completion. The mixture was diluted with DMF (2 mL), filtered, and then purified by reversed-phase preparative chromatography (Waters 2767/Qda, column: XBridge C18 19 * 250 mm, 10 μm; mobile phase A: 0.03% $NH_4OH/H_2O$, mobile phase B: ACN; flow rate: 20 mL/min; elution gradient: 65% to 65%; retention time: 8.60-9.50 min of 16 min) to obtain 48.5 mg of the title compound **117.** LC-MS (ESI): m/z 612.3 $[M+H]^+$. $^1H$ NMR (400 MHz, DMSO-$d_6$) δ 9.02 (d, *J* = 5.2 Hz, 1H), 7.73 (dd, *J* = 5.2, 3.2 Hz, 1H), 7.70 (d, *J* = 2.3 Hz, 1H), 7.66 - 7.56 (m, 4H), 7.49 - 7.41 (m, 1H), 7.38 - 7.26 (m, 3H), 5.43 (s, 2H), 4.42 (t, *J* = 5.2 Hz, 2H), 3.96 (t, *J* = 5.2 Hz, 2H), 1.76 (d, *J* = 13.7 Hz, 6H), 1.68 (s, 6H). $^{19}F$ NMR (376 MHz, DMSO-$d_6$) δ -133.99 (s,1F). $^{31}P$ NMR (162 MHz, DMSO-$d_6$) δ 34.03 (s,1P).

### Example 118: Preparation of compound 118

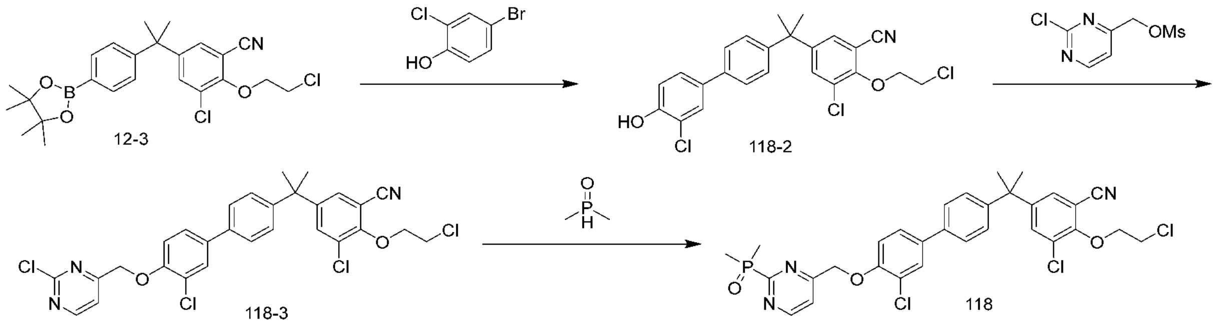


**Preparation of compound 118-2**

Compound **12-3** (300 mg, 0.65 mmol) and 4-bromo-2-chlorophenol (162 mg, 15.65 mmol) were dissolved in 1,4-dioxane (5 mL). Pd(dppf)$Cl_2$ (24 mg, 0.033 mmol) and an aqueous potassium carbonate solution (2 mol/L, 1 mL, 2.00 mmol) were added. The system was purged four times with argon, heated to 110°C, and stirred for 2 h. After the reaction was completed, the reaction system was cooled to room temperature. The pH was adjusted to acidic with 2 mol/L aqueous hydrochloric acid solution. The mixture was filtered through a pad of diatomite, and the filter cake was rinsed with ethyl acetate (100 mL). The filtrates were combined and washed once with saturated brine (50 mL). The organic phase was dried over anhydrous sodium sulfate, subjected to suction filtration, and the filtrate was concentrated. The resulting residue was purified by silica gel column chromatography (EA/PE = 0 to 50%) to obtain 250 mg of the title compound **118-2** in 83.2% yield. LC-MS (ESI): m/z 457.8 $[M-H]^-$.

**Preparation of compound 118-3**

Compound **118-2** (250 mg, 0.54 mmol) and (2-chloropyrimidin-4-yl)methyl methanesulfonate (138 mg, 0.62 mmol) were dissolved in acetonitrile (5.0 mL). Cesium carbonate (264 mg, 0.81 mmol) was added at room temperature, and the mixture was heated to 70°C and stirred for 1 h. The reaction was monitored by LCMS until completion. The mixture was cooled to room temperature, filtered, and the filter cake was rinsed with ethyl acetate. The filtrates were combined and concentrated. The resulting residue was subjected to silica gel column chromatography (EA/PE = 0 to 30%) to obtain 270 mg of the title compound **118-3** in 85% yield. LC-MS (ESI): m/z 586.1 $[M+H]^+$.

**Preparation of compound 118**

Compound **118-3** (270 mg, 0.46 mmol), dimethylphosphine oxide (144 mg, 1.84 mmol), DIPEA (298 mg, 2.30 mmol), 1,3-bis(diphenylphosphino)propane (28 mg, 0.07 mmol), and solvent DMF (2 mL) were added to a microwave tube. The mixture was stirred until a clear solution was obtained. Palladium acetate (10 mg, 0.046 mmol) was added at room temperature. The system was purged with argon, and the reaction was carried out under microwave irradiation at 120°C for 0.5 h. The reaction was monitored by LCMS until completion. The reaction mixture was purified by reversed-phase chromatography (10 to 80% ACN/0.1% aqueous FA) and silica gel column chromatography (MeOH/DCM = 0 to 5%) to obtain 179 mg of the title compound **118.** LC-MS (ESI): m/z 628.3 $[M+H]^+$. $^1H$ NMR (400 MHz, DMSO-$d_6$) δ 9.04 (d, $J$ = 5.2 Hz, 1H), 7.79 (d, $J$ = 2.3 Hz, 1H), 7.76 (dd, $J$ = 5.1, 3.2 Hz, 1H), 7.70 (d, $J$ = 2.3 Hz, 1H), 7.64 (d, $J$ = 2.3 Hz, 1H), 7.61 (dd, $J$ = 8.5, 1.7 Hz, 3H), 7.32 (dd, $J$ = 8.6, 1.4 Hz, 3H), 5.46 (s, 2H), 4.42 (t, $J$ = 5.2 Hz, 2H), 3.96 (t, $J$ = 5.2 Hz, 2H), 1.76 (d, $J$ = 13.7 Hz, 6H), 1.68 (s, 6H). $^{31}P$ NMR (162 MHz, DMSO-$d_6$) δ 34.02 (s,1P).

**Example 119: Preparation of compound 119**

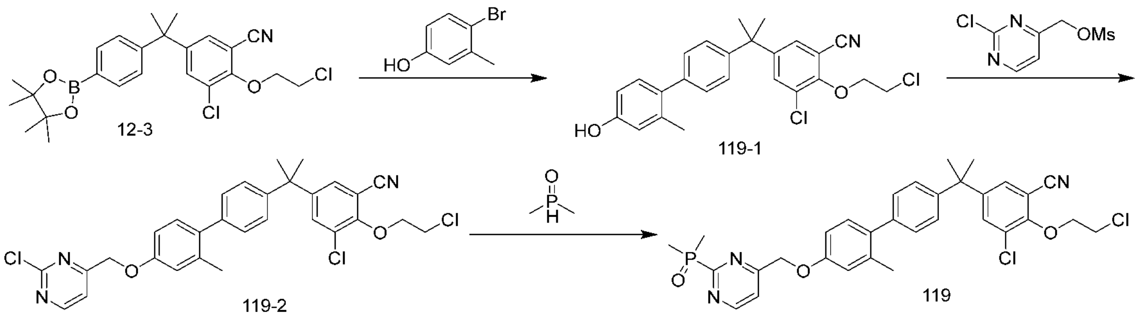
12-3
119-1
119-2
119

**Preparation of compound 119-1**

Compound **12-3** (300 mg, 0.65 mmol) and 3-methyl-4-bromophenol (146 mg, 0.78 mmol) were dissolved in 1,4-dioxane (10 mL). Pd(dppf)$Cl_2$ (24 mg, 0.033 mmol) and potassium carbonate (269 mg, 1.95 mmol) were added. The system was purged four times with argon, heated to 110°C, and stirred for 3 h. After the reaction was completed, the reaction mixture was cooled to room temperature, acidified with 2 mol/L aqueous hydrochloric acid solution, filtered through a pad of diatomite, and the filter cake was rinsed with ethyl acetate (30 mL). The filtrates were combined and washed once with saturated brine (10 mL). The organic phase was dried over anhydrous sodium sulfate, subjected to suction filtration, and the filtrate was concentrated. The resulting residue was purified by silica gel column chromatography (EA/PE = 0 to 50%) to obtain 47.3 mg of the title compound **119-1** in 88% yield. LC-MS (ESI): m/z 438.0 $[M-H]^-$.

**Preparation of compound 119-2**

Compound **119-1** (200 mg, 0.45 mmol) and (2-chloropyrimidin-4-yl)methyl methanesulfonate (77 mg, 0.47 mmol) were dissolved in acetonitrile (5 mL). Cesium carbonate (440 mg, 1.35 mmol) was added at room temperature, and the mixture was heated to 70°C and stirred for 2 h. The reaction was monitored by LCMS until completion. The mixture was filtered, and the filter cake was rinsed with ethyl acetate. The filtrates were combined and concentrated. The resulting residue was subjected to silica gel column chromatography (EA/PE = 0 to 60%) to obtain 230 mg of the title compound **119-2** in 89% yield. LC-MS (ESI): m/z 566.1 $[M+H]^+$.

**Preparation of compound 119**

Compound **119-2** (150 mg, 0.26 mmol), dimethylphosphine oxide (81 mg, 1.04 mmol), DIPEA (201 mg, 1.56 mmol), 1,3-bis(diphenylphosphino)propane (32 mg, 0.078 mmol), and solvent DMF (2 mL) were added to a microwave tube. The mixture was stirred until a clear solution was obtained. Palladium acetate (18 mg, 0.078 mmol) was added at room temperature. The system was purged with argon, heated to 120°C, and the reaction was carried out under microwave irradiation for 0.5 h. The reaction was monitored by LCMS until completion. The mixture was cooled to room temperature, water (10 mL) was added, and the mixture was extracted twice with ethyl acetate (20 mL). The organic phases were combined and concentrated. The resulting residue was purified by reversed-phase preparative chromatography to obtain 47.3 mg of the title compound **119.** LC-MS (ESI): m/z 608.3 $[M+H]^+$. $^1H$ NMR (400 MHz, DMSO-$d_6$) $\delta$ 9.01 (d, $J$ = 4.7 Hz, 1H), 7.75 - 7.69 (m, 2H), 7.67 (d, $J$ = 2.3 Hz, 1H), 7.32 - 7.21 (m, 4H), 7.14 (d, $J$ = 8.4 Hz, 1H), 7.03 (d, $J$ = 2.4 Hz, 1H), 6.92 (dd, $J$ = 8.5, 2.5 Hz, 1H), 5.32 (s, 2H), 4.43 (t, $J$ = 5.2 Hz, 2H), 3.96 (t, $J$ = 5.2 Hz, 2H), 2.22 (s, 3H), 1.77 (d, $J$ = 13.7 Hz, 6H), 1.69 (s, 6H). $^{31}P$ NMR (162 MHz, DMSO-$d_6$) $\delta$ 34.05 (s, 1P).

**Example 120: Preparation of compound 120**

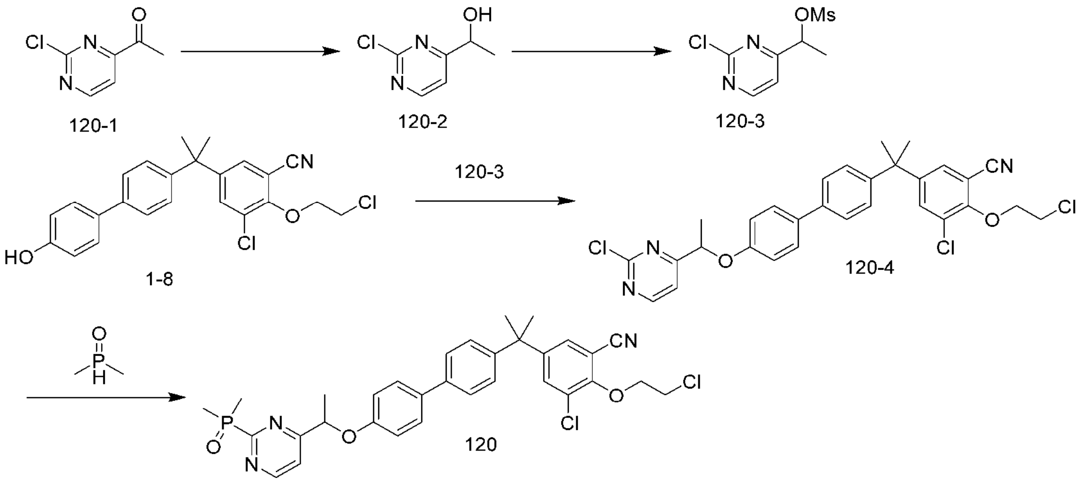

120-1 120-2 120-3 1-8 120-4 120

**Preparation of compound 120-2**

Compound **120-1** (1.00 g, 6.39 mmol) was dissolved in a mixed solvent of tetrahydrofuran (5 mL) and water (5 mL). The reaction system was cooled to 0°C, and sodium borohydride (0.27 g, 7.16 mmol) was added in portions slowly. The mixture was stirred at room temperature for 1 h. The reaction was monitored by LCMS until completion. The system was cooled to 0°C, quenched with saturated ammonium chloride solution (20 mL), and extracted three times with ethyl acetate (30 mL). The organic phases were combined, dried over anhydrous sodium sulfate, subjected to suction filtration, and the filtrate was concentrated to obtain 1 g of a crude product of the title compound **120-2** in 59% yield. LC-MS (ESI): m/z 159.2 $[M+H]^+$.

**Preparation of compound 120-3**

Compound **120-2** (0.90 g, 5.68 mmol) was dissolved in tetrahydrofuran (10 mL). The system was purged with argon, cooled to 0°C, and TEA (1.72 g, 17.04 mmol) was added. The mixture was stirred for 5 min, methanesulfonyl chloride (1.63 g, 14.2 mmol) was added slowly, and the mixture was warmed to room temperature and stirred for 1 h. The reaction was monitored by LCMS until completion. Ice water (30 mL) was added to quench the reaction, and the mixture was extracted three times with ethyl acetate (30.0 mL). The organic phases were combined, dried over anhydrous sodium sulfate, subjected to suction filtration, and the filtrate was concentrated. The resulting residue was purified by silica gel column chromatography (EA/PE = 0 to 47%) to obtain 1.23 g of the title compound **120-3** in 41% yield. LC-MS (ESI): m/z 237.0 $[M+H]^+$.

**Preparation of compound 120-4**

Compound **1-8** (200 mg, 0.47 mmol) and **120-3** (122.4 mg, 0.52 mmol) were dissolved in acetonitrile (5 mL). Cesium carbonate (230 mg, 0.70 mmol) was added at room temperature, and the mixture was heated to 80°C and stirred for 2 h. The reaction was monitored by LCMS until completion. The reaction mixture was filtered through a pad of diatomite, and the filter cake was rinsed with ethyl acetate. The filtrates were combined and extracted twice with ethyl acetate (30 mL). The organic phases were combined and concentrated. The resulting residue was purified by silica gel column chromatography (EA/PE = 0 to 50%) to obtain 200 mg of the title compound **120-4** in 74% yield. LC-MS (ESI): m/z 565.9 $[M+H]^+$.

**Preparation of compound 120**

Compound **120-4** (200 mg, 0.35 mmol), dimethylphosphine oxide (109 mg, 1.4 mmol), DIPEA (226.17 mg, 1.75 mmol), 1,3-bis(diphenylphosphino)propane (22 mg, 0.052 mmol), and solvent DMF (2 mL) were added to a microwave tube. The mixture was stirred until a clear solution was obtained. Palladium acetate (16 mg, 0.07 mmol) was added. The system was purged four times with argon, and the reaction was carried out under microwave irradiation at 120°C for 0.5 h. The reaction was monitored by LCMS until completion. The mixture was diluted with DMF (2 mL) and purified by reversed-phase preparative chromatography (ACN/0.1% formic acid in water = 10 to 90%) to obtain 49 mg of the title product **120.** LC-MS (ESI): m/z 608.3 $[M+H]^+$. $^1$H NMR (400 MHz, DMSO-$d_6$) δ 8.95 (d, *J* = 5.2 Hz, 1H), 7.71 - 7.66 (m, 2H), 7.62 (d, *J* = *2.3* Hz, 1H), 7.55 (d, *J* = 8.8 Hz, 2H), 7.52 (d, *J* = 8.4 Hz, 2H), 7.28 (d, *J* = 8.4 Hz, 2H), 7.02 (d, *J* = 8.8 Hz, 2H), 5.60 (q, *J* = 6.5 Hz, 1H), 4.42 (t, *J* = 5.2 Hz, 2H), 3.95 (t, *J* = 5.2 Hz, 2H), 1.77 (dd, *J* = 13.7, 3.0 Hz, 6H), 1.67 (s, 6H), 1.65 (d, *J* = 6.6 Hz, 3H).

$^{31}P$ NMR (162 MHz, DMSO-$d_6$) δ 34.16 (s, 1P).

**Example 121: Preparation of compound 121**

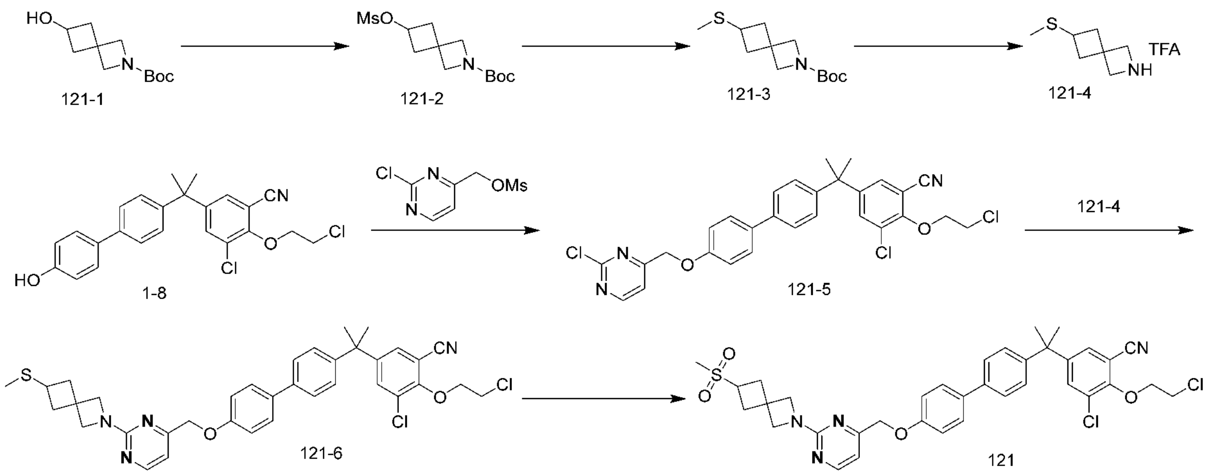


**Preparation of compound 121-2**

Compound **121-1** (1 g, 4.69 mmol) and triethylamine (1.42 g, 14.07 mmol) were dissolved in dichloromethane (10 mL). The mixture was cooled to 0°C, and MsCl (0.54 mL, 7.04 mmol) was slowly added dropwise. The mixture was stirred for 2 h. The reaction was monitored by TLC (DCM/MeOH = 20/1) until completion. The mixture was concentrated to obtain 1.37 g of a crude product of compound **121-2**, which was used directly in the next step without purification.

**Preparation of compound 121-3**

Compound **121-2** (1.37 g, 0.47 mmol) was dissolved in DMF (25 mL). Sodium methanethiolate (0.66 g, 9.40 mmol) was added, and the mixture was heated to 60°C and stirred for 4 h. The reaction was monitored by TLC (DCM/MeOH = 20/1) until completion. Water (30 mL) was added to quench the reaction, and the mixture was extracted three times with ethyl acetate (60 mL). The organic phases were combined, washed three times with saturated brine (100 mL), dried over anhydrous sodium sulfate, subjected to suction filtration, and the filtrate was concentrated. The resulting residue was purified by silica gel column chromatography (MeOH/DCM = 0 to 10%) to obtain 300 mg of the title compound **121-3** in 26.2% yield. $^1H$ NMR (400 MHz, $CDCl_3$) δ 3.92 (s, 2H), 3.89 (s, 2H), 3.25 (p, $J$ = 7.6 Hz, 1H), 2.58 - 2.50 (m, 2H), 2.17 - 2.10 (m, 2H), 2.03 (s, 3H), 1.43 (s, 9H).

**Preparation of compound 121-4**

Compound **121-3** (200 mg, 0.82 mmol) was dissolved in DCM (4 mL). Trifluoroacetic acid (930 mg, 8.20 mmol) was added at room temperature, and the mixture was stirred for 16 h. The reaction was monitored by TLC (DCM/MeOH = 20/1) until completion. The reaction mixture was concentrated to obtain 700 mg of the crude compound **121-4,** which was used directly in the next step without purification.

**Preparation of compound 121-5**

Compound **1-8** (2.1 g, 4.93 mmol) and (2-chloropyrimidin-4-yl)methyl methanesulfonate (1.32 g, 5.92 mmol) were dissolved in acetonitrile (20 mL). Cesium carbonate (2.41 g, 7.39 mmol) was added at room temperature, and the mixture was heated to 70°C and stirred for 2 h. The reaction was monitored by LCMS until completion. The reaction mixture was cooled to room temperature, filtered through a pad of diatomite, and the filter cake was rinsed with ethyl acetate. The filtrates were combined and concentrated. The resulting residue was purified by silica gel column chromatography (EA/PE = 0 to 30%) to obtain 1.4 g of the title compound **121-5** in 50.6% yield. LC-MS (ESI): m/z 551.8 $[M+H]^+$.

**Preparation of compound 121-6**

Compound **121-5** (300 mg, 0.54 mmol) was dissolved in EtOH (5 mL). Compound **121-4** (350 mg, 2.44 mmol) and triethylamine (546 mg, 5.40 mmol) were added at room temperature, and the mixture was heated to 100°C and stirred for 6 h. The reaction was monitored by LCMS until completion. The mixture was cooled to room temperature, quenched with water (10 mL), and extracted three times with ethyl acetate (20 mL). The organic phases were combined, dried over anhydrous sodium sulfate, and concentrated. The resulting residue was purified by silica gel column chromatography (EA/PE = 0 to 30%) to obtain 270 mg of the title compound **121-6** in 72% yield. LC-MS (ESI): m/z 659.2 $[M+H]^+$.

**Preparation of compound 121**

Compound **121-6** (100 mg, 0.15 mmol) was dissolved in a mixed solvent of THF (2 mL) and water (2 mL). Potassium peroxymonosulfate triple salt (369 mg, 0.60 mmol) was added at room temperature, and the mixture was stirred at room temperature for 16 h. The reaction was monitored by LCMS until completion. The reaction was quenched with water (5 mL) and extracted three times with ethyl acetate (10 mL). The organic phases were combined, dried over anhydrous sodium sulfate, subjected to suction filtration, and the filtrate was concentrated. The resulting residue was purified by reversed-phase preparative chromatography (0.1% ammonia water/acetonitrile = 10 to 90%) was performed to obtain 25 mg of the title compound **121.** LC-MS (ESI): m/z 691.3 $[M+H]^+$. $^1H$ NMR (400 MHz, DMSO-$d_6$) δ 8.34 (d, *J* = 5.0 Hz, 1H), 7.70 (d, *J* = 2.3 Hz, 1H), 7.63 (d, *J* = 2.3 Hz, 1H), 7.62 - 7.57 (m, 2H), 7.57 - 7.52 (m, 2H), 7.30 (d, *J* = 8.5 Hz, 2H), 7.07 (d, *J* = 8.8 Hz, 2H), 6.76 (d, *J* = 5.0 Hz, 1H), 5.04 (s, 2H), 4.42 (t, *J* = 5.2 Hz, 2H), 4.09 (s, 2H), 4.01 (s, 2H), 3.96 (t, *J* = 5.2 Hz, 2H), 3.88 (p, *J* = 8.0 Hz, 1H), 2.89 (s, 3H), 2.61 - 2.53 (m, 4H), 1.68 (s, 6H).

**Example 122: Preparation of compound 122**

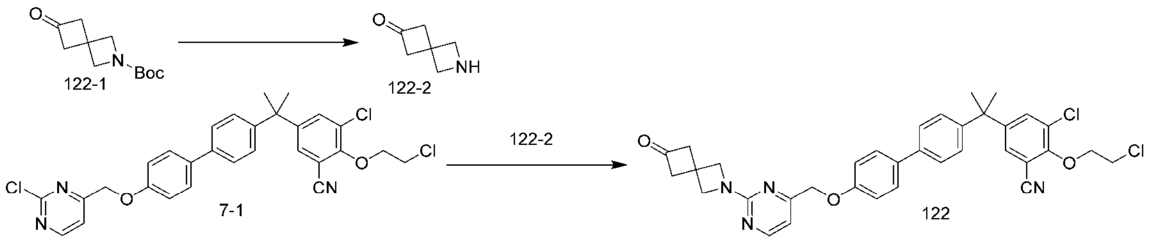

122-1
122-2
7-1
122

**Preparation of compound 122-2**

Compound **122-1** (1 g, 4.73 mmol) was dissolved in dichloromethane (10 mL), and the temperature was lowered to 0°C. A solution of hydrogen chloride in dioxane (4 M, 15 mL) was added dropwise. After the addition was completed, the mixture was warmed to room temperature and stirred for 3 h. TLC indicated completion of the reaction. The reaction mixture was concentrated to obtain 1.1 g of a crude product of the title compound **122-2,** which was used directly in the next step without purification.

**Preparation of compound 122**

Compound **7-1** (300 mg, 0.54 mmol), compound **122-2** (300 mg, 2.7 mmol), and triethylamine (546 mg, 5.4 mmol) were dissolved in anhydrous ethanol (5 mL). The mixture was heated to 100°C and stirred for 16 h. After the reaction was completed, water (20 mL) was added, and the mixture was extracted three times with ethyl acetate (50 mL). The organic phases were combined, dried over anhydrous sodium sulfate, subjected to suction filtration, and the filtrate was concentrated. The resulting residue was subjected to preparative purification (column: SunFire Sunfire C18, 19 * 250 mm * 10 μm; flow rate: 20 mL/min; mobile phase: A-0.1% aqueous FA solution, B-acetonitrile; gradient: 97 to 91% acetonitrile content, retention time: 10.2 min of 16 min) to obtain 26 mg of the title compound **122.** LC-MS (ESI): m/z 627.3 $[M+H]^+$. $^1H$ NMR (400 MHz, DMSO-$d_6$) δ 8.37 (d, *J*= 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.4 Hz, 2H), 7.30 (d, *J* = 8.5 Hz, 2H), 7.07 (d, *J* = 8.8 Hz, 2H), 6.78 (d, *J* = 5.0 Hz, 1H), 5.05 (s, 2H), 4.42 (t, *J* = 5.2 Hz, 2H), 4.25 (s, 4H), 3.96 (t, *J* = 5.2 Hz, 2H), 3.37 (s, 4H), 1.68 (s, 6H).

**Example 123: Preparation of compound 123**

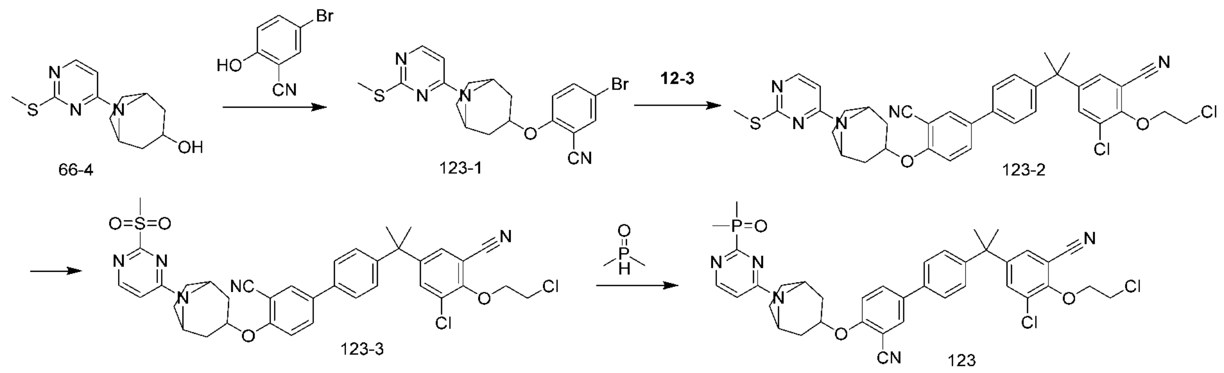


66-4 123-1 123-2 123-3 123

**Preparation of compound 123-1**

Compound **66-4** (1 g, 3.98 mmol), CMBP (1.92 mg, 7.96 mmol), and 5-bromo-2-hydroxybenzonitrile (1.2 g, 5.97 mmol) were dissolved in toluene (20 mL). The reaction system was stirred at 120°C for 3 h. After the reaction was completed, the reaction mixture was cooled to room temperature. Water (20 mL) was added to the reaction mixture, and the mixture was extracted three times with dichloromethane (30 mL). The organic phases were combined, washed twice with saturated brine (20 mL), dried over anhydrous sodium sulfate, subjected to suction filtration, and the filtrate was concentrated. The resulting residue was purified by column chromatography (EA/PE = 0 to 30%) to obtain 600 mg of the title compound **123-1.** LC-MS (ESI): m/z 431.0 $[M+H]^+$.

**Preparation of compound 123-2**

Compound **123-1** (600 mg, 1.39 mmol) was dissolved in a solution of 1,4-dioxane (10 mL) and water (3 mL), followed by the sequential addition of [1,1'-bis(diphenylphosphino)ferrocene]dichloropalladium(II) dichloromethane complex (103 mg, 0.14 mmol), potassium carbonate (383 mg, 2.78 mmol), and compound **12-3** (959 mg, 2.09 mmol). The reaction system was stirred at 90°C for 5 h. After the reaction was completed, the reaction mixture was cooled to room temperature, water (20 mL) was added, and the mixture was extracted three times with dichloromethane (30 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 500 mg of the title compound **123-2**. LC-MS (ESI): m/z 684.3 $[M+H]^+$.

**Preparation of compound 123-3**

Compound **123-2** (500 mg, 0.73 mmol) was dissolved in dichloromethane (10 mL), and mCPBA was added in an ice bath. The reaction system was stirred at 25°C for 1 h. After the reaction was completed, water (20 mL) was added to the reaction mixture, and the mixture was extracted three times with dichloromethane (30 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 400 mg of the title compound **123-3**. LC-MS (ESI): m/z 716.4 $[M+H]^+$.

**Preparation of compound 123**

Compound 123-3 (170 mg, 0.24 mmol) was dissolved in acetonitrile (3 mL), and dimethylphosphine oxide (37 mg, 0.48 mmol) and DIPEA (93 mg, 0.72 mmol) were added. The reaction system was stirred at 80°C for 2 h. After the reaction was completed, the reaction mixture was cooled to room temperature, water (3 mL) was added, and the mixture was extracted three times with dichloromethane (5 mL). The organic phases were combined, washed twice with saturated brine (3 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 21

mg of the title compound 123. LC-MS (ESI): m/z 714.2 [M+H]+. $^{1}$H NMR (400 MHz, DMSO-$d_6$) δ 8.32 (d, *J* = 6.4 Hz, 1H), 8.12 - 7.89 (m, 2H), 7.71 (d, *J* = 2.3 Hz, 1H), 7.67 - 7.59 (m, 3H), 7.33 (d, *J* = 8.5 Hz, 2H), 7.23 (d, *J* = 12.3 Hz, 1H), 6.95 - 6.82 (m, 1H), 4.90 (s, 2H), 4.52 (s, 1H), 4.42 (t, *J* = 5.2 Hz, 2H), 3.96 (t, *J* = 5.2 Hz, 2H), 2.42 - 2.13(m, 4H), 2.09 - 1.93 (m, 4H), 1.74 - 1.62 (m, 12H). $^{31}$P NMR (162 MHz, DMSO-$d_6$) δ 33.46 (s, 1H).

**Example 124: Preparation of compound 124**

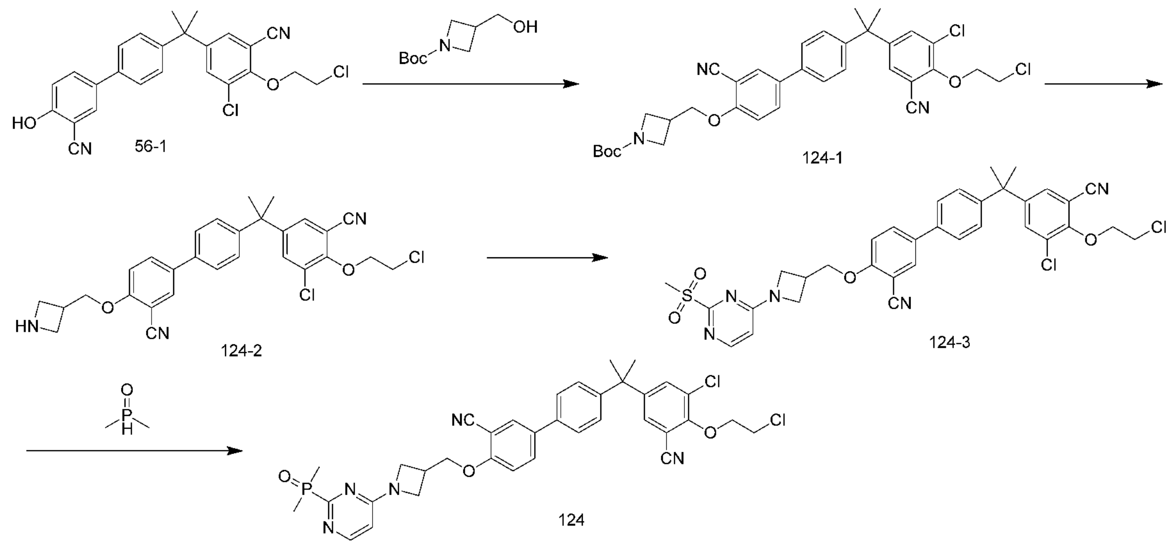


**Preparation of compound 124-1**

Compound **56-1** (330 mg, 0.73 mmol) was dissolved in acetonitrile (5 mL), and *tert*-butyl 3-(hydroxymethyl) azetidine-1-carboxylate (205 mg, 1.09 mmol) and cesium carbonate (714 mg, 2.19 mmol) were added. The reaction system was stirred at 80°C for 2 h. After the reaction was completed, the reaction mixture was cooled to room temperature, water (5 mL) was added, and the mixture was extracted three times with dichloromethane (10 mL). The organic phases were combined, washed twice with saturated brine (5 mL), dried over anhydrous sodium sulfate, subjected to suction filtration, and the filtrate was concentrated to obtain a crude product. The crude product was purified by silica gel column chromatography (EA/PE = 0 to 30%) to obtain 250 mg of the title compound 124-1. LC-MS (ESI): m/z 564.2 [M+H-56]+.

**Preparation of compound 124-2**

Compound **124-1** (250 mg, 0.40 mmol) was dissolved in dichloromethane (5 mL), and TFA (456 mg, 4.00 mmol) was added. The reaction system was stirred at 25°C for 2 h. After the reaction was completed, water (5 mL) was added, and the pH was adjusted to 7-8. The mixture was extracted three times with dichloromethane (10 mL). The organic phases were combined, washed twice with saturated brine (5 mL), dried over anhydrous sodium sulfate, subjected to suction filtration, and the filtrate was concentrated to obtain a crude product. The crude product was purified by column chromatography (EA/PE = 0 to 30%) to obtain 150 mg of the title compound **124-2.** LC-MS (ESI): m/z 520.0 [M+H]+.

**Preparation of compound 124-3**

Compound **124-2** (150 mg, 0.29 mmol) was dissolved in acetonitrile (3 mL), followed by the sequential addition of 4-chloro-2-(methylsulfonyl)pyrimidine (84 mg, 0.44 mmol) and DIPEA (112 mg, 0.87 mmol). The reaction system was stirred at 80°C for 2 h. After the reaction was completed, the reaction mixture was cooled to room temperature, water (5 mL) was added, and the mixture was extracted three times with dichloromethane (10 mL). The organic phases were combined, washed twice with saturated brine (5 mL), dried over anhydrous sodium sulfate, subjected to suction filtration, and the filtrate was concentrated to obtain a crude product. The crude product was purified by silica gel column chromatography (EA/PE = 0 to 30%) to obtain 80 mg of the title compound **124-3.** LC-MS (ESI): m/z 676.2 [M+H]+.

**Preparation of compound 124**

Compound 124-3 (80 mg, 0.12 mmol) was dissolved in acetonitrile (3 mL), and dimethylphosphine oxide (19 mg, 0.24 mmol) and DIPEA (47 mg, 0.36 mmol) were added. The reaction system was stirred at 80°C for 2 h. After the reaction was completed, the reaction mixture was cooled to room temperature, water (3 mL) was added, and the mixture was extracted three times with dichloromethane (5 mL). The organic phases were combined, washed twice with saturated brine (3 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 14 mg of the title compound 124. LC-MS (ESI): m/z 674.2 $[M+H]^+$. $^1$H NMR (400 MHz, DMSO-$d_6$) δ 8.27 (dd, *J* = 6.0, 1.0 Hz, 1H), 8.04 (d, *J* = 2.4 Hz, 1H), 7.98 (dd, *J* = 8.9, 2.4 Hz, 1H), 7.70 (d, *J* = 2.3 Hz, 1H), 7.64 (d, *J* = 8.5 Hz, 2H), 7.64 (d, *J* = 2.4 Hz, 1H), 7.38 (d, *J* = 8.9 Hz, 1H), 7.33 (d, *J* = 8.5 Hz, 2H), 6.51 (dd, *J* = 6.1, 3.2 Hz, 1H), 4.47 - 4.39 (m, 4H), 4.24 (t, *J* = 8.7 Hz, 2H), 4.02 - 3.93 (m, 4H), 3.30 - 3.25 (m, 1H), 1.69 (s, 6H), 1.65 (d, *J* = 13.5 Hz, 6H). $^{31}$P NMR (162 MHz, DMSO-$d_6$) δ 33.78(s, 1H).

**Example 125: Preparation of compound 125**

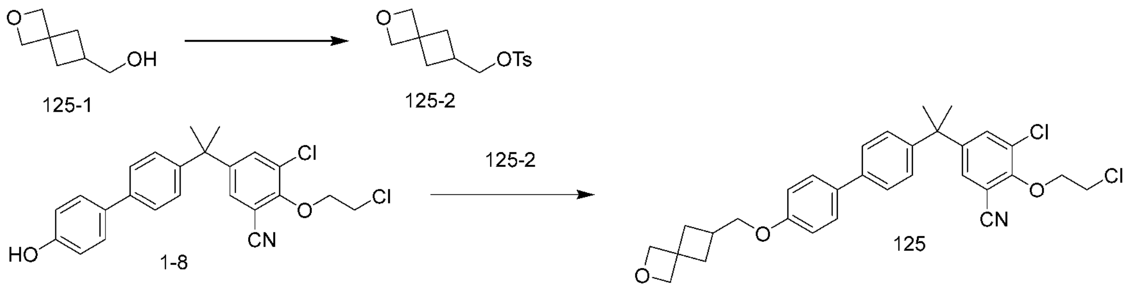


125-1 125-2 1-8 125

**Preparation of compound 125-2**

Compound **125-1** (100 mg, 0.78 mmol) was dissolved in dichloromethane (2 mL), and p-toluenesulfonyl chloride (149 mg, 0.78 mmol) and 4-dimethylaminopyridine (10 mg, 0.08 mmol) were added. The mixture was cooled to 0°C, and triethylamine (158 mg, 1.6 mmol) was added dropwise. The reaction system was stirred at 25°C for 2 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 111 mg of the title compound **125-2.** LC-MS (ESI): m/z 283.0 $[M+H]^+$.

**Preparation of compound 125**

Compound **1-8** (100 mg, 0.23 mmol) was dissolved in dimethyl sulfoxide (2 mL), followed by the addition of compound **125-2** (69 mg, 0.26 mmol) and cesium carbonate (153 mg, 0.47 mmol). The reaction system was stirred at 60°C for 12 h. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15 mg of the title compound 125. LC-MS (ESI): m/z 536.2 $[M+H]^+$. $^1$H NMR (400 MHz, Chloroform-d) δ 7.55 - 7.46 (m, 5H), 7.38 (d, *J* = 2.4 Hz, 1H), 7.25 - 7.20 (m, 2H), 6.98 - 6.89 (m, 2H), 4.72 (s, 2H), 4.69 (s, 2H), 4.42 (t, *J* = 6.2 Hz, 2H), 3.95 - 3.85 (m, 4H), 2.63 - 2.53 (m, 1H), 2.50 - 2.38 (m, 2H), 2.19 - 2.09 (m, 2H), 1.69 (s, 6H).

**Example 126: Preparation of compound 126**

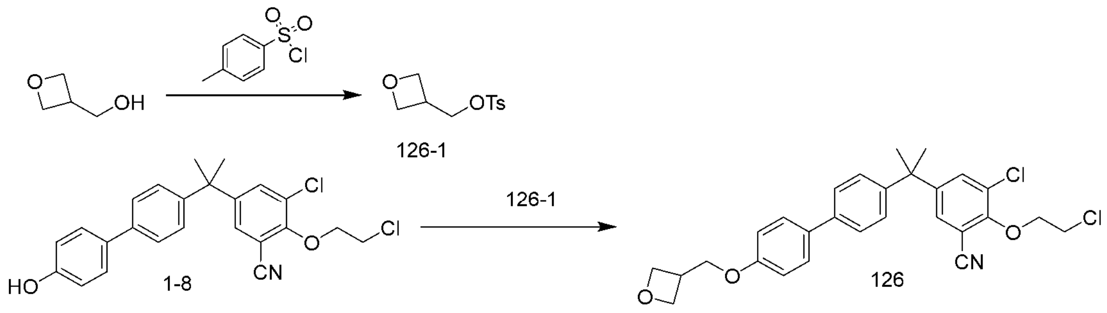

126-1
1-8
126

**Preparation of compound 126-1**

Compound oxetan-3-ylmethanol (100 mg, 1.14 mmol) was dissolved in dichloromethane (2 mL), followed by the sequential addition of p-toluenesulfonyl chloride (238 mg, 1.25 mmol) and 4-dimethylaminopyridine (14 mg, 0.11 mmol). The mixture was cooled to 0°C, and triethylamine (230 mg, 2.27 mmol) was added dropwise. The reaction system was stirred at 25°C for 12 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 120 mg of the title compound **126-1.** LC-MS (ESI): m/z 243.0 $[M+H]^+$.

**Preparation of compound 126**

Compound **1-8** (30 mg, 0.07 mmol) was dissolved in dimethyl sulfoxide (2 mL), followed by the sequential addition of compound **126-1** (19 mg, 0.77 mmol) and cesium carbonate (46 mg, 0.14 mmol). The reaction system was stirred at 60°C for 12 h. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15 mg of the title compound 126. LC-MS (ESI): m/z 496.3 $[M+H]^+$. $^1$H NMR (400 MHz, Chloroform-d) δ 7.61 - 7.45 (m, 5H), 7.38 (d, *J* = 2.3 Hz, 1H), 7.22 (d, *J* = 8.2 Hz, 2H), 6.99 (d, *J* = 8.7 Hz, 2H), 4.90 (t, *J* = 7.6 Hz, 2H), 4.60 (t, *J* = 6.0 Hz, 2H), 4.43 (t, *J* = 6.2 Hz, 2H), 4.25 (d, *J* = 6.7 Hz, 2H), 3.88 (t, *J* = 6.2 Hz, 2H), 3.54 - 3.41 (m, 1H), 1.69 (s, 6H).

**Example 127: Preparation of compound 127**

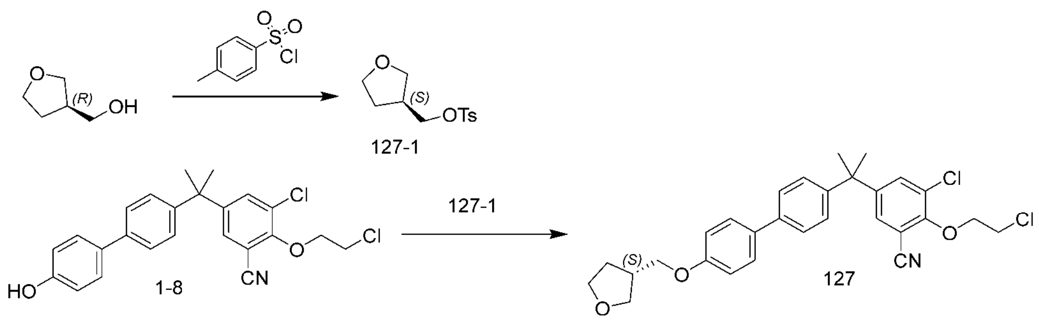

127-1
1-8
127

**Preparation of compound 127-1**

Compound (R)-(tetrahydrofuran-3-yl)methanol (100 mg, 0.98 mmol) was dissolved in dichloromethane (2 mL), followed by the addition of p-toluenesulfonyl chloride (205 mg, 1.08 mmol) and 4-dimethylaminopyridine (12 mg, 0.10 mmol). The mixture was cooled to 0°C, and triethylamine (199 mg, 1.96 mmol) was added dropwise. The reaction system was stirred at 25°C for 12 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 232 mg of the

title compound **127-1.** LC-MS (ESI): m/z 257.0 [M+H]+.

**Preparation of compound 127**

Compound **1-8** (40 mg, 0.09 mmol) was dissolved in dimethyl sulfoxide (2 mL), followed by the addition of compound **127-1** (33 mg, 0.10 mmol) and cesium carbonate (61 mg, 0.19 mmol). The reaction system was stirred at 60°C for 12 h. After the reaction was completed, the reaction mixture was cooled to room temperature and filtered. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15 mg of the title compound **127.** LC-MS (ESI): m/z 510.2. [M+H]+. $^1$H NMR (400 MHz, Chloroform-d) δ 7.54 - 7.47 (m, 5H), 7.38 (d, *J* = 2.3 Hz, 1H), 7.22 (d, *J* = 8.4 Hz, 2H), 6.96 (d, *J* = 8.8 Hz, 2H), 4.42 (t, *J* = 6.2 Hz, 2H), 4.00 - 3.86 (m, 6H), 3.84 - 3.76 (m, 1H), 3.73 (dd, *J* = 8.9, 5.3 Hz, 1H), 2.83 - 2.72 (m, 1H), 2.19 - 2.07 (m, 1H), 1.80 - 1.74 (m, 1H), 1.69 (s, 6H).

**Example 128: Preparation of compound 128**

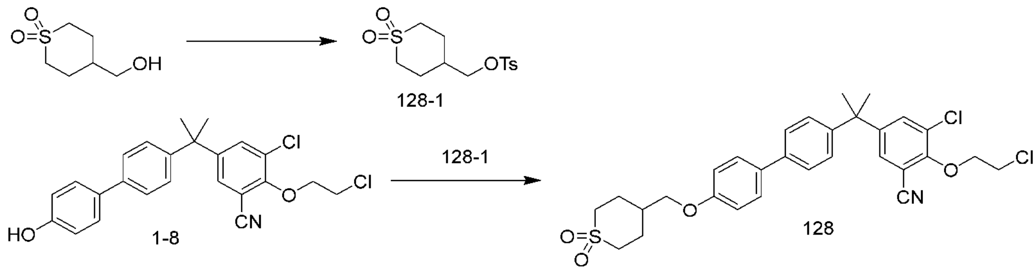


128-1

1-8

128

**Preparation of compound 128-1**

Compound 4-(hydroxymethyl)tetrahydro-2H-thiopyran 1,1-dioxide (100 mg, 0.61 mmol) was dissolved in dichloromethane (2 mL), followed by the addition of p-toluenesulfonyl chloride (128 mg, 0.67 mmol) and 4-dimethylaminopyridine (7 mg, 0.06 mmol). The mixture was cooled to 0°C, and triethylamine (123 mg, 1.22 mmol) was added dropwise. The reaction system was stirred at 25°C for 12 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 232 mg of the title compound **128-1.** LC-MS (ESI): m/z 319.0 [M+H]+.

**Preparation of compound 128**

Compound **1-8** (60 mg, 0.12 mmol) was dissolved in dimethyl sulfoxide (2 mL), followed by the addition of compound **128-1** (33 mg, 0.13 mmol) and cesium carbonate (76 mg, 0.23 mmol). The reaction system was stirred at 60°C for 12 h. After the reaction was completed, the reaction mixture was cooled to room temperature and filtered. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15 mg of the title compound **128.** LC-MS (ESI): m/z 572.1 [M+H]+. $^1$H NMR (400 MHz, Chloroform-d) δ 7.57 - 7.45 (m, 5H), 7.37 (d, *J* = 2.3 Hz, 1H), 7.22 (d, *J* = 8.4 Hz, 2H), 6.95 (d, *J* = 8.8 Hz, 2H), 4.43 (t, *J* = 8.0 Hz, 2H), 3.91 (d, *J* = 4.8 Hz, 2H), 3.88 (t, *J* = 6.2 Hz, 2H), 3.20 - 3.10 (m, 2H), 3.10 - 3.01 (m, 2H), 2.37 - 2.27 (m, 2H), 2.11 - 2.03 (m, 3H), 1.69 (s, 6H).

**Example 129: Preparation of compound 129**

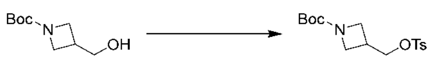

129-1

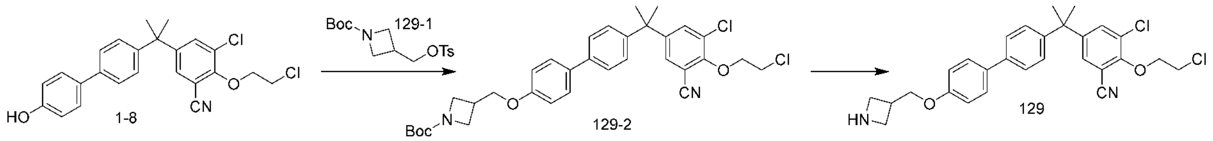


1-8

129-2

129

**Preparation of compound 129-1**

Compound tert-butyl 3-(hydroxymethyl)azetidine-1-carboxylate (200 mg, 1.1 mmol) was dissolved in dichloromethane (2 mL), followed by the addition of p-toluenesulfonyl chloride (224 mg, 1.17 mmol) and 4-(dimethylamino) pyridine (13 mg, 0.11 mmol). The mixture was cooled to 0°C, and triethylamine (216 mg, 2.14 mmol) was added dropwise. The reaction system was stirred at 25°C for 12 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 232 mg of the title compound **129-1.** LC-MS (ESI): m/z 286.2 $[M+H-56]^+$.

**Preparation of compound 129-2**

Compound **1-8** (100 mg, 0.23 mmol) was dissolved in dimethyl sulfoxide (2 mL), followed by the addition of compound **129-1** (104 mg, 0.30 mmol) and cesium carbonate (153 mg, 0.47 mmol). The reaction system was stirred at 80°C for 12 h. After the reaction was completed, the reaction mixture was cooled to room temperature. Water (10 mL) was added to the reaction mixture, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 135 mg of the title compound **129-2.** LC-MS (ESI): m/z 539.2 $[M+H-56]^+$.

**Preparation of compound 129**

Compound **129-2** (132 mg, 0.22 mmol) was dissolved in dichloromethane (2 mL). The mixture was cooled to 0°C, and trifluoroacetic acid (1 mL) was added dropwise. The reaction system was stirred at 25°C for 3 h. After the reaction was completed, the volatiles were removed by evaporation under reduced pressure. The resulting residue was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15 mg of the title compound **129.** LC-MS (ESI): m/z 495.2 $[M+H]^+$. $^1H$ NMR (400 MHz, Chloroform-d) δ 8.62 (s, 1H), 7.57 - 7.42 (m, 4H), 7.37 (d, *J* = 2.3 Hz, 1H), 7.22 (d, *J* = 8.1 Hz, 2H), 7.00 (d, *J* = 8.1 Hz, 2H), 4.42 (t, *J* = 6.2 Hz, 2H), 4.23 - 4.07 (m, 4H), 4.07 - 3.96 (m, 2H), 3.88 (t, *J* = 6.2 Hz, 2H), 3.37 - 3.29 (m, 1H), 1.69 (s, 6H).

**Example 130: Preparation of compound 130**

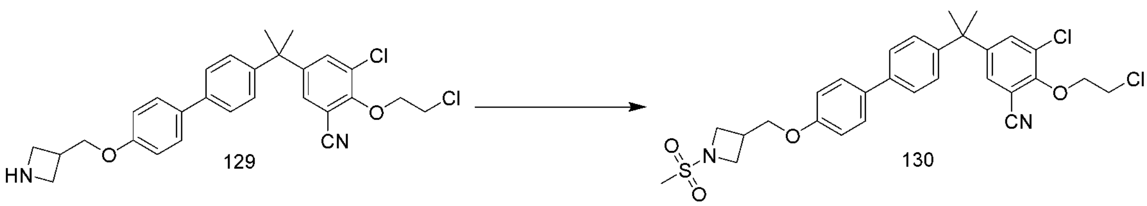

129

130

**Preparation of compound 130**

Compound 129 (30 mg, 0.06 mmol) was dissolved in dimethyl sulfoxide (2 mL). Triethylamine (17 mg, 0.17 mmol) was added, and the mixture was cooled to 0°C. Methanesulfonyl chloride (7 mg, 0.06 mmol) was added dropwise, and the reaction system was stirred at 25°C for 3 h. After the reaction was completed, the reaction mixture was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column

temperature: 25°C; gradient: 45% to 65% acetonitrile gradient elution over 12 min; flow rate: 30 mL/min) to obtain 5.5 mg of the title compound **130.** LC-MS (ESI): m/z 573.3 $[M+H]^+$. $^1$H NMR (400 MHz, Chloroform-d) δ 7.55 - 7.46 (m, 5H), 7.37 (d, *J* = 2.4 Hz, 1H), 7.23 (d, *J* = 8.4 Hz, 2H), 6.97 (d, *J* = 8.7 Hz, 2H), 4.43 (t, *J* = 6.1 Hz, 2H), 4.22 - 4.04 (m, 4H), 3.93 (t, 2H), 3.88 (t, *J* = 6.2 Hz, 2H), 3.17 - 3.04 (m, 1H), 2.93 (s, 3H), 1.69 (s, 6H).

**Example 131: Preparation of compound 131**

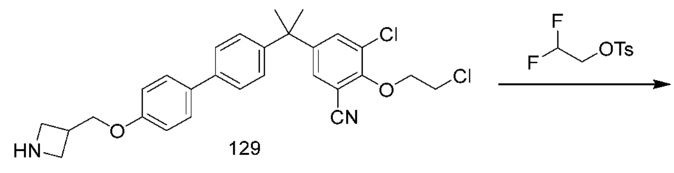
129
131

**Preparation of compound 131**

Compound **129** (60 mg, 0.12 mmol) was dissolved in acetonitrile (2 mL), and compound 2,2-difluoroethyl p-toluenesulfonate (37 mg, 0.16 mmol) and DIPEA (78 mg, 0.61 mmol) were added. The reaction system was stirred at 90°C for 12 h. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5 mg of the title compound **131.** LC-MS (ESI): m/z 559.2. $[M+H]^+$. $^1$H NMR (400 MHz, Chloroform-d) δ 7.55 - 7.45 (m, 5H), 7.38 (d, *J* = 2.4 Hz, 1H), 7.22 (d, *J* = 8.4 Hz, 2H), 6.97 (d, *J* = 8.7 Hz, 2H), 5.81 (t, *J* = 55.9 Hz, 1H), 4.42 (t, *J* = 6.2 Hz, 2H), 4.12 (d, *J* = 6.2 Hz, 2H), 3.88 (t, *J* = 6.2 Hz, 2H), 3.71 - 3.59 (m, 2H), 3.37 - 3.28 (m, 2H), 3.08 - 2.96 (m, 1H), 2.97 - 2.80 (m, 2H), 1.69 (s, 6H). $^{19}$F NMR (376 MHz, Chloroform-*d*) δ -120.85.

**Example 132: Preparation of compound 132**

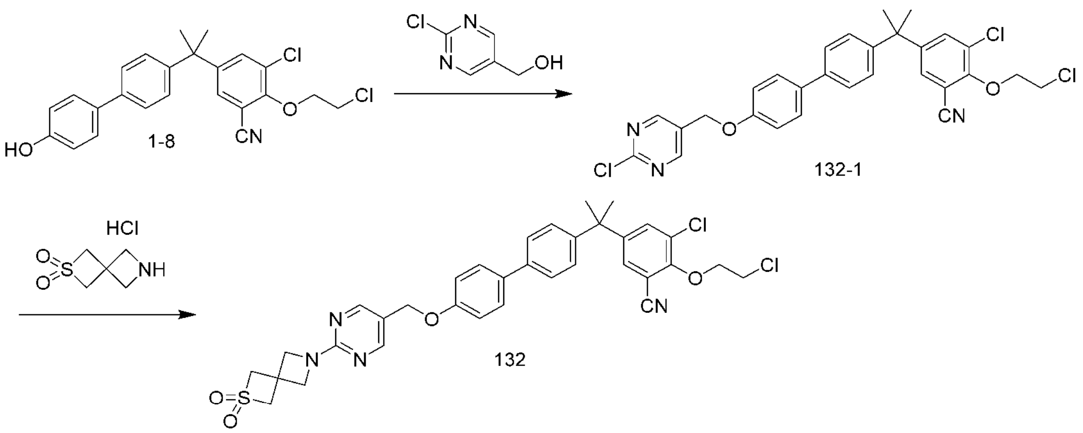

1-8
132-1
132

**Preparation of compound 132-1**

Compound (2-chloropyrimidin-5-yl)methanol (150 mg, 1.04 mmol), compound **1-8** (221 mg, 518.82 μmol), and triphenylphosphine (272 mg, 1.04 mmol) were dissolved in acetonitrile (2 mL). The system was purged three times with nitrogen, heated to 90°C, and DIAD (210 mg, 1.04 mmol) was added. The reaction system was stirred at 90°C for 2 h. After the reaction was completed, the reaction mixture was cooled to room temperature and concentrated. The crude product was purified by silica gel column chromatography (EA/PE = 0 to 20%) to obtain 250 mg of the title compound **132-1.** LC-MS (ESI): m/z 552.2 $[M+H]^+$.

**Preparation of compound 132**

Compound **132-1**(25 mg, 45.22 μmol) was dissolved in ethanol (2 mL), followed by the addition of DIPEA (12 mg, 90.44 μmol) and 2-thio-6-azaspiro[3.3]heptane 2,2-dioxide hydrochloride (10 mg, 54.26 μmol). The reaction system was stirred at 90°C for 3 h. After the reaction was completed, insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 8 mg of the title compound **132.** LC-MS (ESI): m/z 663.3 $[M+H]^+$. $^1H$ NMR (400 MHz, DMSO-$d_6$) δ 8.50 (s, 2H), 7.78 - 7.45 (m, 6H), 7.30 (d, *J* = 7.6 Hz, 2H), 7.08 (s, 2H), 5.00 (s, 2H), 4.50 (s, 4H), 4.42 (s, 2H), 4.27 (s, 4H), 3.96 (s, 2H), 1.68 (s, 6H).

**Example 133: Preparation of compound 133**

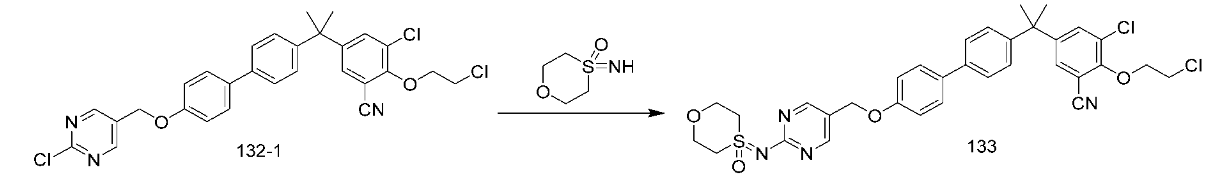
132-1
133

**Preparation of compound 133**

Compound **132-1** (55 mg, 98.63 μmol) was dissolved in DMF (2 mL), followed by the sequential addition of DIPEA (64 mg, 493.15 μmol), 4,4-dihydro-4-imino-1,4-oxathiane-4-oxide (40 mg, 295.89 μmol), XantPhos (121 mg, 19.73 μmol), $Pd_2(dba)_3$ (9 mg, 9.86 μmol). The reaction system was stirred at 110°C for 2 h. After the reaction was completed, the reaction mixture was cooled to room temperature, and insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15 mg of the title compound **133.** LC-MS (ESI): m/z 651.2 $[M+H]^+$. $^1H$ NMR (400 MHz, DMSO-$d_6$) δ 8.54 (s, 2H), 7.60 (d, *J* = 2.4 Hz, 1H), 7.59 - 7.54 (m, 3H), 7.52 (d, *J* = 8.4 Hz, 2H), 7.27 (d, *J* = 8.5 Hz, 2H), 7.06 (d, *J* = 8.8 Hz, 2H), 5.02 (s, 2H), 4.39 (t, *J* = 5.2 Hz, 2H), 4.11 - 4.04 (m, 2H), 3.99 - 3.88 (m, 4H), 3.83 - 3.74 (m, 2H), 3.61 - 3.49 (m, 2H), 1.65 (s, 6H).

**Example 134: Preparation of compound 134**

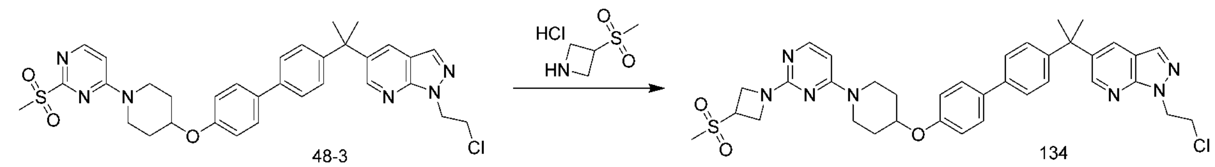

48-3
134

**Preparation of compound 134**

Compound 48-3 (60 mg, 95 μmol), 3-(methylsulfonyl)azetidine hydrochloride (32 mg, 186 μmol), and cesium carbonate (62 mg, 190 μmol) were added to acetonitrile (2 mL). The reaction system was stirred at 120°C for 16 h. After the reaction was completed, the reaction mixture was cooled to room temperature. Saturated brine (5 mL) was added to the reaction mixture, and the mixture was extracted three times with ethyl acetate (8 mL). The organic phases were combined, dried, and subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 5% to 35% acetonitrile gradient elution over 12 min; flow rate: 30 mL/min) to obtain 22 mg of the title compound 134. LC-MS (ESI): m/z 686.5 [M+H]+. 1H NMR (400 MHz, DMSO-$d_6$) δ 8.40 (d, *J = 2.3* Hz, 1H), 8.19 (d, *J* = 2.2 Hz, 1H), 8.17 (s, 1H), 7.88 (d, *J* = 6.1 Hz, 1H), 7.60 - 7.50 (m, 4H), 7.31 (d, *J* = 8.5 Hz, 2H), 7.07 (d, *J* = 8.9 Hz, 2H), 6.25 (d, *J* = 6.2 Hz, 1H), 4.75 (t, *J* = 5.8 Hz, 2H),

4.71 - 4.63 (m, 1H), 4.40 - 4.28 (m, 1H), 4.21 (t, *J* = 8.8 Hz, 2H), 4.15 - 4.08 (m, 4H), 4.04 - 3.92 (m, 2H), 3.45 - 3.37 (m, 2H), 3.03 (s, 3H), 2.03 - 1.96 (m, 2H), 1.78 (s, 6H), 1.64 - 1.55 (m, 2H).

**Example 135: Preparation of compound 135**

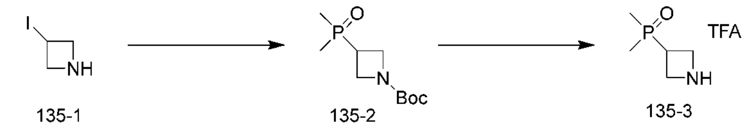

135-1 135-2 135-3

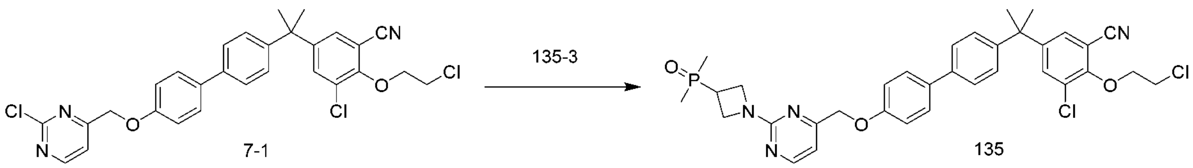


7-1 135

**Preparation of compound 135-2**

Dimethylphosphine oxide (0.55 g, 7.06 mmol) was dissolved in THF (20 mL). The reaction mixture was cooled to 0°C, and sodium bis(trimethylsilyl)amide (7.1 mL, 7.10 mmol, 1 M in THF) was added slowly. The reaction system was stirred at room temperature for 1 h. The reaction mixture was cooled to 0°C, and **135-1** (1.00 g, 3.53 mmol) was added. The reaction system was stirred at room temperature for 48 h. After the reaction was completed, water (20 mL) was added to the filtrate, and the mixture was extracted three times with dichloromethane (30 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (MeOH/DCM = 0 to 10%) to obtain 300 mg of the title compound **135-2.** LC-MS (ESI): m/z 178.2 $[M-56+H]^+$.

**Preparation of compound 135-3**

Compound **135-2** (300 mg, 1.29 mmol) was dissolved in dichloromethane (5 mL), and TFA (441 mg, 3.87 mmol) was added. The reaction system was stirred at room temperature for 2 h. After the reaction was completed, the reaction mixture was concentrated under reduced pressure to obtain 150 mg of the crude title compound **135-3.** LC-MS (ESI): m/z 134.2 $[M+H]^+$.

**Preparation of compound 135**

Compound **7-1** (100 mg, 0.18 mmol) was dissolved in acetonitrile (5 mL), followed by the sequential addition of azetidin-3-yldimethylphosphine oxide (46 mg, 0.27 mmol) and DIPEA (70 mg, 0.54 mmol). The reaction system was stirred at 80°C for 2 h. After the reaction was completed, the reaction mixture was cooled to room temperature, water (5 mL) was added, and the mixture was extracted three times with dichloromethane (5 mL). The organic phases were combined, washed twice with saturated brine (5 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 27 mg of the title compound **135.** LC-MS (ESI): m/z 649.5 $[M+H]^+$. $^1H$ NMR (400 MHz, DMSO-$d_6$) δ 8.38 (d, *J* = 5.0 Hz, 1H), 7.70 (d, *J* = 2.3 Hz, 1H), 7.64 (d, *J* = 2.3 Hz, 1H), 7.60 (d, *J* = 8.8 Hz, 2H), 7.55 (d, *J* = 8.4 Hz, 2H), 7.30 (d, *J* = 8.5 Hz, 2H), 7.07 (d, *J* = 8.8 Hz, 2H), 6.79 (d, *J* = 5.0 Hz, 1H), 5.06 (s, 2H), 4.42 (t, *J* = 5.2 Hz, 2H), 4.27 - 4.06 (m, 4H), 3.96 (t, *J* = 5.2 Hz, 2H), 3.08 - 2.99 (m, 1H), 1.68 (s, 6H), 1.40 (d, *J* = 12.9 Hz, 6H). $^{31}P$ NMR (162 MHz, DMSO-$d_6$) δ 40.19 (s, 1P).

**Example 136: Preparation of compound 136**

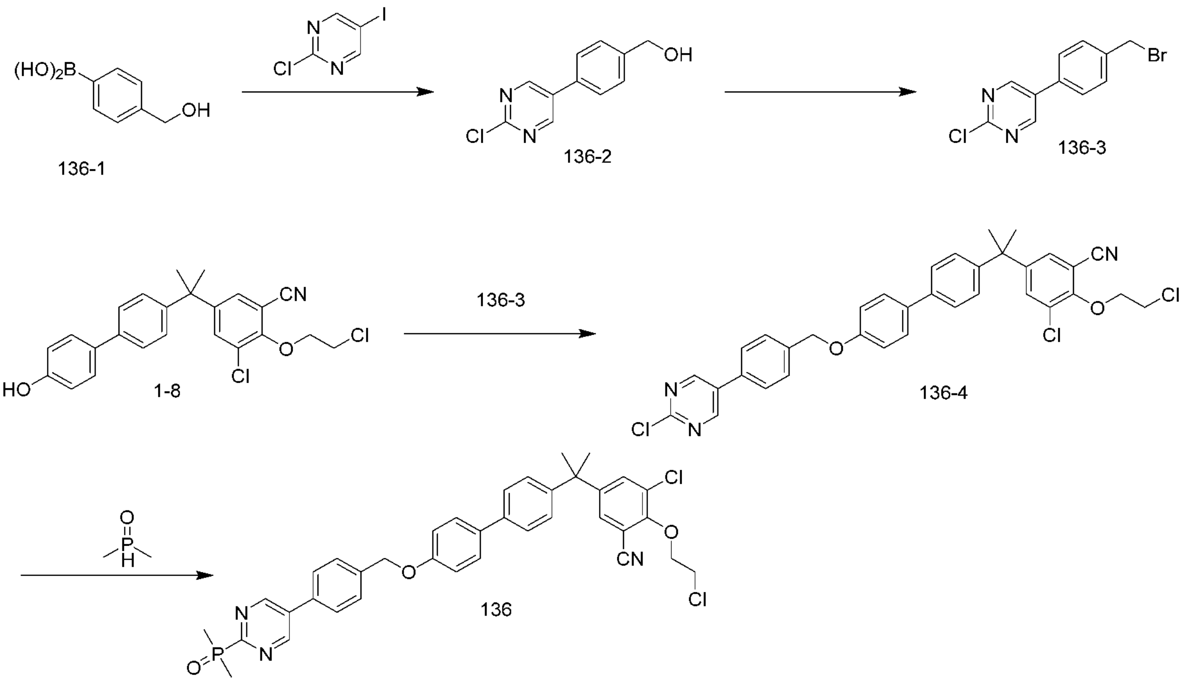


**Preparation of compound 136-2**

Compound **136-1** (1.3 g, 8.56 mmol) and 2-chloro-5-iodopyrimidine (1.87 g, 7.78 mmol) were dissolved in 1,4-dioxane (16 mL) and water (4 mL), followed by the sequential addition of $K_2CO_3$ (3.2 g, 22.3 mmol) and Pd(dppf)$Cl_2$ (284 mg, 0.38 mmol). The reaction system was purged three times with nitrogen and stirred at 60°C for 2 h. After the reaction was completed, the reaction mixture was cooled to room temperature and filtered through a pad of diatomite. The filter cake was rinsed with ethyl acetate (30 mL). Water (50 mL) was added to the filtrate, and the mixture was extracted three times with ethyl acetate (150 mL). The organic phases were combined, washed once with saturated brine (50 mL), dried over anhydrous sodium sulfate, subjected to suction filtration, and the filtrate was concentrated. The resulting crude product was purified by silica gel column chromatography to obtain 1.6 g of the title compound **136-2.** LC-MS (ESI): m/z 221.0 $[M+H]^+$.

**Preparation of compound 136-3**

Compound **136-2** (1.48 g, 6.71 mmol) was dissolved in THF (4 mL), followed by the sequential addition of carbon tetrabromide (3.34 g, 10.0 mmol) and $PPh_3$ (2.64 g, 10.0 mmol). The reaction system was stirred at 15°C for 15 h. After the reaction was completed, THF was evaporated to dryness under reduced pressure, water (50 mL) was added, and the mixture was extracted three times with ethyl acetate (100 mL). The organic phases were combined, washed once with saturated brine (50 mL), dried over anhydrous sodium sulfate, subjected to suction filtration, and the filtrate was concentrated to obtain a crude product. The crude product was purified by silica gel column chromatography to obtain 2 g of the title compound **136-3.** LC-MS (ESI): m/z 283.0 $[M+H]^+$.

**Preparation of compound 136-4**

Compound **1-8** (50 mg, 0.12 mmol) was dissolved in DMF (3 mL), followed by the sequential addition of **136-3** (40 mg, 0.14 mmol) and cesium carbonate (73 mg, 0.23 mmol). The reaction system was stirred at 60°C for 16 h. After the reaction was completed, water (10 mL) was added to the reaction mixture, and the mixture was extracted three times with ethyl acetate (30 mL). The organic phases were combined, washed once with saturated brine (10 mL), dried over anhydrous sodium sulfate, subjected to suction filtration, and the filtrate was concentrated to obtain 70 mg of a crude product of the title compound **136-4.** LC-MS (ESI): m/z 628.3 $[M+H]^+$.

**Preparation of compound 136**

Compound **136-4** (120 mg, 0.19 mmol) was dissolved in DMF (4 mL), followed by the sequential addition of dimethylphosphine oxide (74 mg, 0.95 mmol), DIPEA (123 mg, 0.95 mmol), X-phos (21.6 mg, 0.04 mmol), and $Pd_2(dba)_3$

(18 mg, 0.02 mmol). The reaction system was purged three times with nitrogen and stirred under microwave irradiation at 120°C for 2 h. After the reaction was completed, the reaction mixture was filtered through diatomite, and the filtrate was rinsed with ethyl acetate (30 mL). The filtrates were combined, water (30 mL) was added, and the mixture was extracted three times with ethyl acetate (50 mL). The organic phases were combined, washed once with saturated brine (50 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile elution over 12 min; flow rate: 30 mL/min) to obtain 5.6 mg of the title compound 136. LC-MS (ESI): m/z 670.4 $[M+H]^+$. $^1H$ NMR (400 MHz, DMSO-*d$_6$)* *J* = 7.60 (d, *J = 8.7* Hz, 2H), 7.56 (d, *J* = 8.3 Hz, 2H), 7.30 (d, *J* = 8.4 Hz, 2H), 7.11 (d, *J* = 8.8 Hz, 2H), 5.26 (s, 2H), 4.42 (t, *J* = 5.2 Hz, 2H), 3.96 (t, *J* = 5.2 Hz, 2H), 1.80 (d, *J* = 13.7 Hz, 6H), 1.68 (s, 6H). $^{31}P$ NMR (162 MHz, DMSO-*d*$_6$) δ 33.88 (s, 1P).

**Example 137: Preparation of compound 137**

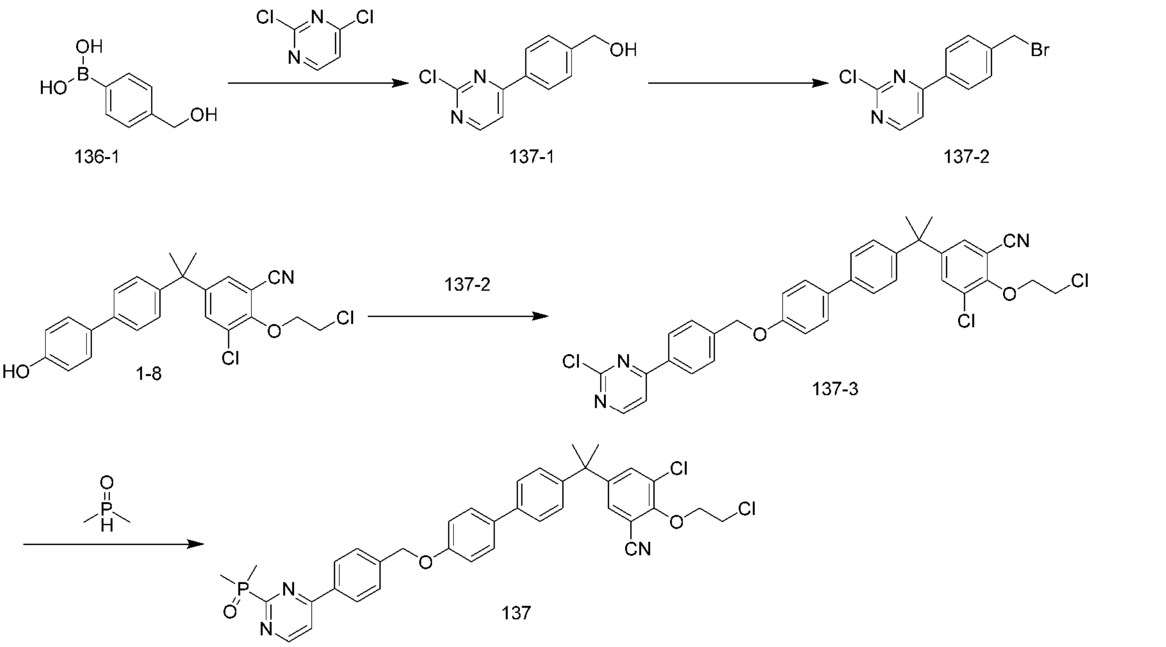


136-1 137-1 137-2

1-8 137-3

137

**Preparation of compound 137-1**

Compound 136-1 (1.1 g, 7.24 mmol) was dissolved in 1,4-dioxane (16 mL) and water (4 mL). 2,4-Dichloropyrimidine (1.62 g, 10.8 mmol), $K_2CO_3$ (3 g, 21.7 mmol), and Pd(dppf)$Cl_2$ (530 mg, 0.72 mmol) were added. The reaction system was purged three times with nitrogen and stirred at 80°C for 2 h. After the reaction was completed, the reaction mixture was filtered through diatomite, and the filtrate was rinsed with ethyl acetate (30 mL). Water (50 mL) was added to the filtrate, and the mixture was extracted three times with ethyl acetate (150 mL). The organic phases were combined, washed once with saturated brine (50 mL), dried over anhydrous sodium sulfate, subjected to suction filtration, and the filtrate was concentrated to obtain a crude product. The crude product was purified by silica gel column chromatography to obtain 1.18 g of the title compound **137-1.** LC-MS (ESI): m/z 221.0 $[M+H]^+$.

**Preparation of compound 137-2**

Compound **137-1** (1.1 g, 4.99 mmol) was dissolved in THF (4 mL), followed by the addition of carbon tetrabromide (2.48 g, 7.48 mmol) and $PPh_3$ (1.96 g, 7.48 mmol). The reaction system was stirred at 15°C for 15 h. After the reaction was completed, tetrahydrofuran was removed by evaporation under reduced pressure, water (50 mL) was added, and the mixture was extracted three times with ethyl acetate (100 mL). The organic phases were combined, washed once with saturated brine (50 mL), dried over anhydrous sodium sulfate, subjected to suction filtration, and the filtrate was concentrated to obtain a crude product. The crude product was purified by silica gel column chromatography to obtain 1.1 g of the title compound **137-2.** LC-MS (ESI): m/z 283.0 $[M+H]^+$.

**Preparation of compound 137-3**

Compound **1-8** (50 mg, 0.12 mmol) was dissolved in DMF (3 mL), followed by the sequential addition of **137-2** (40 mg, 0.14 mmol) and cesium carbonate (73 mg, 0.23 mmol). The reaction system was stirred at 60°C for 16 h. After the reaction was completed, water (10 mL) was added to the reaction mixture, and the mixture was extracted three times with ethyl acetate (30 mL). The organic phases were combined, washed once with saturated brine (10 mL), dried over anhydrous sodium sulfate, subjected to suction filtration, and the filtrate was concentrated to obtain 70 mg of a crude product of the title compound **137-3.** LC-MS (ESI): m/z 628.0 $[M+H]^+$.

**Preparation of compound 137**

Compound **137-3** (80 mg, 0.13 mmol) was dissolved in DMF (4 mL), followed by the sequential addition of dimethylphosphine oxide (50 mg, 0.64 mmol), DIPEA (82 mg, 0.64 mmol), X-phos (15 mg, 0.02 mmol), and $Pd_2(dba)_3$ (12 mg, 0.01 mmol). The reaction system was purged three times with nitrogen and stirred under microwave irradiation at 120°C for 2 h. After the reaction was completed, the reaction mixture was filtered through diatomite, and the filter cake was rinsed with 30 mL of ethyl acetate. Water (30 mL) was added to the filtrate, and the mixture was extracted three times with ethyl acetate (50 mL). The organic phases were combined, washed once with saturated brine (50 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile elution over 12 min; flow rate: 30 mL/min) to obtain 3.8 mg of the title compound 137. LC-MS (ESI): m/z 670.5 $[M+H]^+$. $^1H$ NMR (400 MHz, DMSO-$d_6$) δ 9.03 (d, $J$ = 5.4 Hz, 1H), 8.32 (d, $J$ = 8.2 Hz, 2H), 8.22 (dd, $J$ = 5.5, 3.2 Hz, 1H), 7.70 (d, $J$ = 2.3 Hz, 1H), 7.68 (d, $J$ = 8.2 Hz, 2H), 7.64 (d, $J$ = 2.3 Hz, 1H), 7.60 (d, $J$ = 8.7 Hz, 2H), 7.56 (d, $J$ = 8.4 Hz, 2H), 7.30 (d, $J$ = 8.4 Hz, 2H), 7.11 (d, $J$ = 8.8 Hz, 2H), 5.28 (s, 2H), 4.42 (t, $J$ = 5.2 Hz, 2H), 3.96 (t, $J$ = 5.2 Hz, 2H), 1.83 (d, $J$ = 13.7 Hz, 6H), 1.68 (s, 6H). $^{31}P$ NMR (162 MHz, DMSO-$d_6$) δ 34.39 (s, 1P).

**Example 138: Preparation of compound 138**

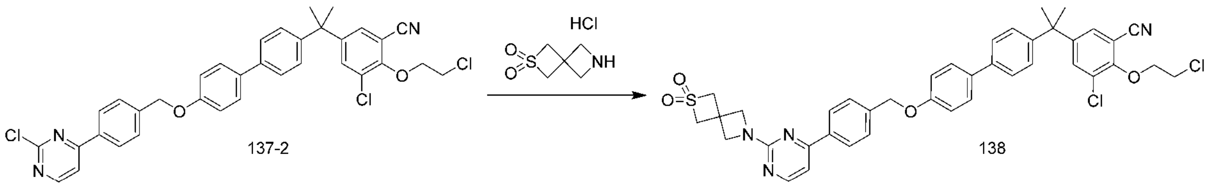

137-2

138

**Preparation of compound 138**

Compound **137-2** (40 mg, 0.06 mmol) was dissolved in EtOH (2 mL), followed by the sequential addition of 2-thio-6-azaspiro[3.3]heptane 2,2-dioxide hydrochloride (14 mg, 0.08 mmol) and DIPEA (17 mg, 0.13 mmol). The reaction system was stirred under microwave irradiation at 90°C for 2 h. After the reaction was completed, the reaction mixture was subjected to rotary evaporation until dryness. The resulting crude product was purified by silica gel column chromatography (EA/PE = 0 to 25%) to obtain 1.95 mg of the title compound **138.** LC-MS (ESI): m/z 739.3 $[M+H]^+$. $^1H$ NMR (400 MHz, DMSO-$d_6$) δ 8.45 (d, $J$ = 5.2 Hz, 1H), 8.15 (d, $J$ = 8.3 Hz, 2H), 7.70 (s, 1H), 7.64 (d, $J$ = 2.4 Hz, 1H), 7.60 (dd, $J$ = 8.7, 2.7 Hz, 4H), 7.56 (d, $J$ = 8.5 Hz, 2H), 7.34 - 7.25 (m, 3H), 7.10 (d, $J$ = 8.8 Hz, 2H), 5.24 (s, 2H), 4.52 (s, 4H), 4.42 (t, $J$ = 5.2 Hz, 2H), 4.34 (s, 4H), 3.96 (t, $J$ = 5.2 Hz, 2H), 1.68 (s, 6H).

**Example 139: Preparation of compound 139**

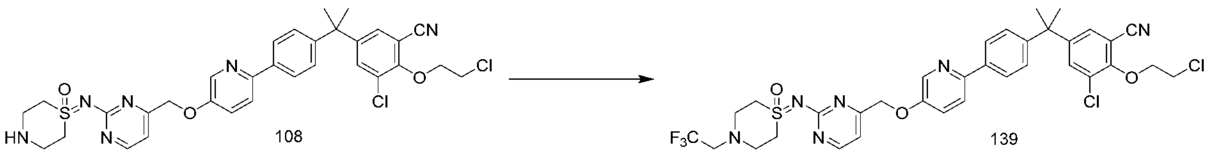

108

139

**Preparation of compound 139**

Compound **108** (20 mg, 30.69 μmol), DIPEA (40 mg, 306.93 μmol, 53.46 μL), 2,2,2-trifluoroethyl trifluoromethanesulfonate (14.25 mg, 61.39 μmol), and DMF (1 mL) were added to a reaction flask, and the mixture was stirred at room temperature for 16 h. After the reaction was completed, water (10 mL) was added, and the mixture was extracted three times with dichloromethane (20 mL). The organic phases were combined, subjected to rotary evaporation until dryness, and concentrated to obtain a crude product. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 10% to 90% acetonitrile gradient elution over 20 min; flow rate: 30 mL/min) to obtain 5 mg of the title compound 139. LC-MS (ESI): m/z 733.2 $[M+H]^+$. $^1$H NMR (400 MHz, DMSO-$d_6$) δ 8.47 (d, *J* = 5.0 Hz, 1H), 8.44 (d, *J* = 3.0 Hz, 1H), 7.95 (d, 2H), 7.89 (d, *J* = 8.8 Hz, 1H), 7.70 (d, *J* = 2.3 Hz, 1H), 7.62 (d, *J* = 2.3 Hz, 1H), 7.52 (dd, *J* = 8.8, 3.0 Hz, 1H), 7.35 - 7.31 (m, 2H), 7.02 (d, *J* = 5.1 Hz, 1H), 5.20 (s, 2H), 4.42 (t, *J* = 5.2 Hz, 2H), 3.95 (t, *J* = 5.2 Hz, 2H), 3.74 - 3.62 (m, 2H), 3.54 - 3.44 (m, 4H), 3.21 - 3.07 (m, 4H), 1.69 (s, 6H). $^{19}$F NMR (376 MHz, DMSO-$d_6$) δ -68.74 (s, 3F).

**Example 140: Preparation of compound 140**

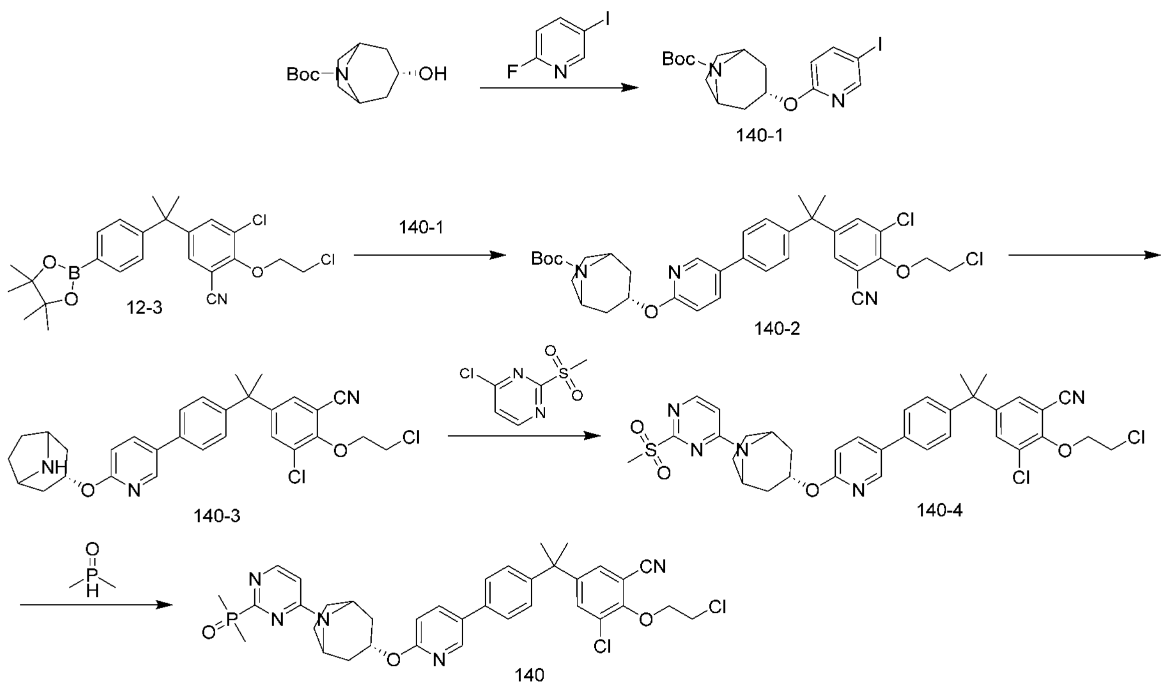


**Preparation of compound 140-1**

Compound *tert*-butyl 3-hydroxy-8-azabicyclo[3.2.1]octane-8-carboxylate (397 mg, 1.97 mmol) was dissolved in tetrahydrofuran (5 mL). The reaction system was purged three times with nitrogen, and then sodium hydride (144 mg, 3.59 mmol, 60% purity) was added in an ice bath. After 30 min under the ice bath, 2-fluoro-5-iodopyridine (400 mg, 1.79 mmol) was added. After the addition was completed, the reaction system was stirred at 20°C for 15 h. After the reaction was completed, the reaction mixture was filtered. Saturated aqueous sodium chloride solution (15 mL) was added to the filtrate, and the mixture was extracted three times with ethyl acetate (10 mL). The organic phases were combined, dried over anhydrous sodium sulfate, subjected to suction filtration, and the filtrate was concentrated to obtain a crude product. The crude product was purified by normal-phase silica gel column chromatography (EtOAc/PE = 0 to 30%) to obtain 380 mg of the title compound **140-1**in 52% yield. LC-MS (ESI): m/z 431.3 $[M+H]^+$.

**Preparation of compound 140-2**

Compound **140-1**(268 mg, 663 μmol) and compound **12-3**(320 mg, 663 μmol) were dissolved in 1,4-dioxane (5 mL), followed by the sequential addition of potassium carbonate (183 mg, 1.33 mmol) and [1,1'-bis(diphenylphosphino)ferrocene]dichloropalladium(II) dichloromethane complex (27 mg, 33.17 μmol). The system was purged three times with nitrogen and stirred at 105°C for 3 h. After the reaction was completed, the reaction mixture was cooled to room temperature and filtered. Saturated aqueous sodium chloride solution (15 mL) was added to the filtrate, and the mixture was extracted three times with ethyl acetate (10 mL). The organic phases were combined, dried, subjected to suction filtration, and concentrated to obtain a crude product. The crude product was purified by normal-phase silica gel column chromatography (EtOAc/PE = 0 to 35%) to obtain 275 mg of the title compound **140-2** in 67% yield. LC-MS (ESI): m/z 636.4 $[M+H]^+$.

**Preparation of compound 140-3**

Compound **140-2** (190 mg, 311.19 μmol) was added to dichloromethane (4 mL), and trifluoromethanesulfonic acid (0.5 mL) was added dropwise in an ice bath. The mixture was stirred at room temperature for 2 h. After the reaction was completed, the reaction mixture was concentrated to obtain 142 mg of a crude product of the title compound **140-3** in 89% yield.

**Preparation of compound 140-4**

Compound **140-3** (130 mg, 254.68 μmol) and triethylamine (52 mg, 509.35 μmol, 71 μL) were dissolved in DCM (3 mL). 4-Chloro-2-(methylsulfonyl)pyrimidine (54 mg, 280.14 μmol) was added in an ice bath, and the reaction system was stirred at room temperature for 2 h. After the reaction was completed, the reaction mixture was filtered. Saturated aqueous sodium chloride solution (10 mL) was added to the filtrate, and the mixture was extracted three times with ethyl acetate (10 mL). The organic phases were combined, dried, filtered, and concentrated to obtain a crude product. The crude product was purified by normal-phase silica gel column chromatography (EtOAc/PE = 0 to 40%) to obtain 105 mg of the title compound **140-4** in 61% yield. LC-MS (ESI): m/z 692.8 $[M+2H]^+$.

**Preparation of compound 140**

Compound **140-4** (25 mg, 37.5 μmol), dimethylphosphine oxide (6 mg, 75.0 μmol), and cesium carbonate (25 mg, 75.0 μmol) were added to acetonitrile (1 mL). The reaction system was stirred at 90°C for 2 h. After the reaction was completed, the reaction mixture was cooled to room temperature. Saturated brine (10 mL) was added to the reaction mixture, and the mixture was extracted three times with ethyl acetate (10 mL). The organic phases were combined, dried, and subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 5% to 30% acetonitrile gradient elution over 12 min; flow rate: 30 mL/min) to obtain 8 mg of the title compound **140.** LC-MS (ESI): m/z 690.4 $[M+H]^+$. $^1$H NMR (400 MHz, DMSO-$d_6$) δ 8.47 (d, *J* = 2.6 Hz, 1H), 8.34 (d, *J* = 6.0 Hz, 1H), 7.96 (dd, *J* = 8.7, 2.6 Hz, 1H), 7.71 (d, *J* = 2.3 Hz, 1H), 7.65 (d, *J* = 2.4 Hz, 1H), 7.59 (d, *J* = 8.4 Hz, 2H), 7.33 (d, *J* = 8.4 Hz, 2H), 6.90 (dd, *J* = 6.2, 3.1 Hz, 1H), 6.77 (d, *J* = 8.6 Hz, 1H), 5.68 - 5.59 (m, 1H), 5.07 - 4.74 (m, 2H), 4.73 - 4.46 (m, 2H), 4.42 (t, *J* = 5.2 Hz, 2H), 3.96 (t, *J* = 5.2 Hz, 2H), 2.27 - 2.19 (m, 2H), 2.11 - 2.02 (m, 2H), 1.99 - 1.90 (m, 2H), 1.67 (d, *J* = 13.6 Hz, 6H), 1.68 (s, 6H). $^{31}$P NMR (162 MHz, DMSO-$d_6$) δ 33.72 (s, 1P).

**Example 141: Preparation of compound 141**

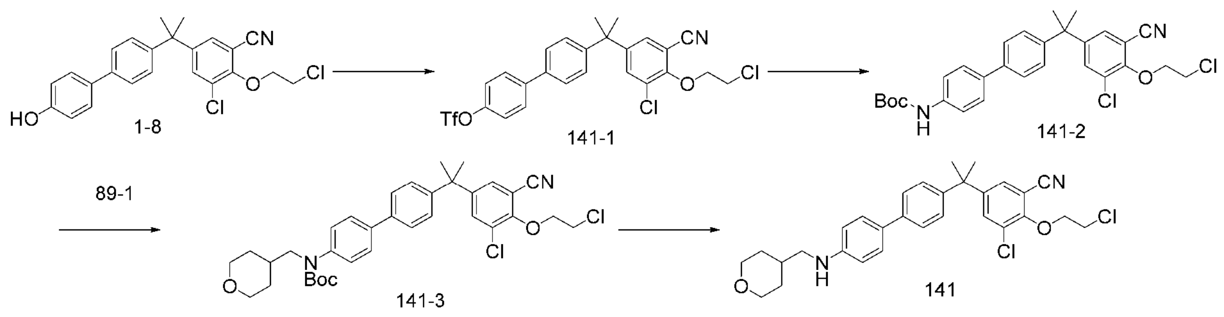

1-8
141-1
141-2
141-3
141

**Preparation of compound 141-1**

Compound **1-8** (10 g, 23.46 mmol) was dissolved in DCM (100 mL), and trifluoromethanesulfonic anhydride (13.24 g, 46.91 mmol) was added at 0°C, followed by the dropwise addition of triethylamine (4.75 g, 46.91 mmol) at 0°C. After the addition was completed, the reaction system was stirred at 25°C for 1 h. After the reaction was completed, water (50 mL) was added, and the mixture was extracted three times with dichloromethane (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 90%) to obtain 12 g of the title compound **141-1.** LC-MS (ESI): m/z 558.2 $[M+H]^+$.

**Preparation of compound 141-2**

Compound **141-1** (53 mg, 0.179 mmol) was dissolved in THF (2 mL), followed by the sequential addition of $Pd_2(dba)_3$ (17 mg, 0.018 mmol), Xantphos (21 mg, 0.036 mmol), $Cs_2CO_3$ (117 mg, 0.36 mmol), and tert-butyl carbamate (32 mg, 0.27 mmol). The reaction system was stirred at 100°C for 16 h. After the reaction was completed, the reaction mixture was cooled to room temperature, and insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 50%) to obtain 70 mg of the title compound **141-2.** LC-MS (ESI): m/z 525.2 $[M+H]^+$.

**Preparation of compound 141-3**

Compound **141-2** (53 mg, 0.101 mmol) was dissolved in THF (2 mL). NaH (12 mg, 0.303 mmol) was added at 0°C, and the mixture was stirred for 1 h. **89-1** (41 mg, 0.151 mmol) was added, and the mixture was stirred at 60°C for 12 h. After the reaction was completed, the reaction mixture was cooled to room temperature, and insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 50%) to obtain 32 mg of the title compound **141-3.** LC-MS (ESI): m/z 523.2 $[M+H-100]^+$.

**Preparation of compound 141**

Compound **141-3** (32 mg, 0.05 mmol) was dissolved in DCM (0.5 mL). NaH (171 mg, 1.5 mmol) was added, and the mixture was stirred for 3 h. The mixture was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% gradient elution over 12 min; flow rate: 30 mL/min) to obtain 12 mg of the title compound. LC-MS (ESI): m/z 523.2 $[M+H]^+$. $^1$H NMR (400 MHz, Chloroform-d) δ 7.52 - 7.42 (m, 5H), 7.38 (d, *J* = 2.4 Hz, 1H), 7.23 - 7.17 (m, 2H), 6.88 (s, 2H), 4.42 (t, *J* = 6.2 Hz, 2H), 4.04 - 3.93 (m, 2H), 3.88 (t, *J* = 6.2 Hz, 2H), 3.36 (td, *J* = 11.8, 2.0 Hz, 2H), 3.10 (d, *J* = 6.8 Hz, 2H), 1.94 (d, *J* = 4.0 Hz, 1H), 1.75 (d, *J* = 13.5 Hz, 2H), 1.69 (s, 6H), 1.46 - 1.31 (m, 2H).

**Example 142: Preparation of compound 142**

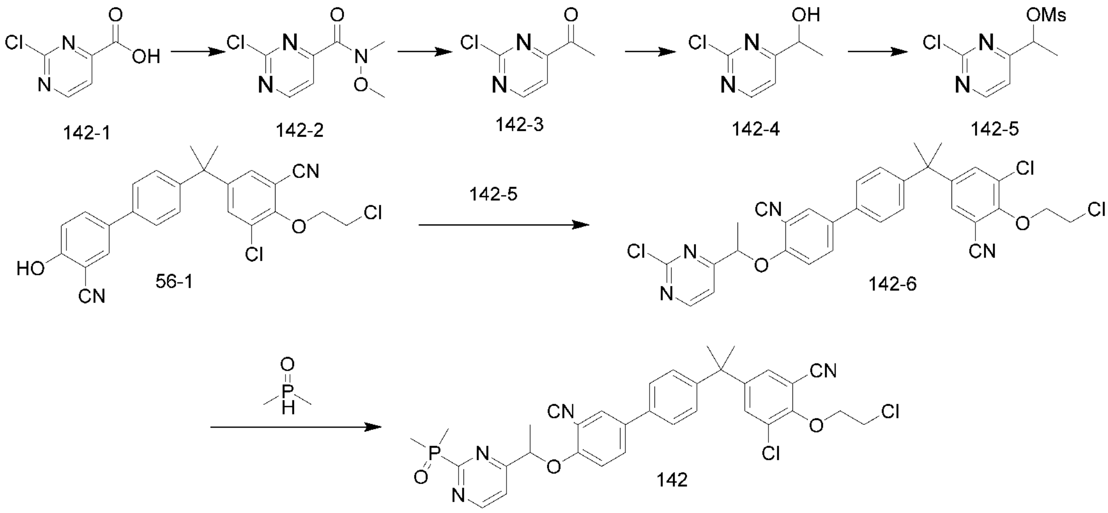

142-1 142-2 142-3 142-4 142-5

56-1 142-5 142-6

142

### Preparation of compound 142-2

Compound **142-1** (3 g, 18.92 mmol), N,O-dimethylhydroxylamine hydrochloride (1.73 g, 28.38 mmol), and DIPEA (7.34 g, 56.77 mmol, 9.89 mL) were dissolved in DCM (50 mL), and HATU (8.57 g, 22.71 mmol) was added. After the addition was completed, the reaction system was stirred at 25°C for 16 h. After the reaction was completed, water (50 mL) was added, and the mixture was extracted three times with dichloromethane (50 mL). The organic phases were combined, washed twice with saturated brine (50 mL), dried over anhydrous sodium sulfate, filtered, and the filtrate was concentrated to obtain a crude product. The crude product was purified by silica gel column chromatography (EA/PE = 0 to 50%) to obtain 2 g of the title compound **142-2.** LC-MS (ESI): m/z 202.0 $[M+H]^+$.

### Preparation of compound 142-3

Compound **142-2** (2 g, 9.92 mmol) was dissolved in THF (50 mL), and methylmagnesium bromide (3 M, 3.31 mL) was added at 0°C. After the addition was completed, the reaction system was stirred at room temperature for 2 h. After the reaction was completed, saturated ammonium chloride solution (100 mL) was added, and the mixture was extracted three times with ethyl acetate (100 mL). The organic phases were combined, washed twice with saturated brine (100 mL), dried over anhydrous sodium sulfate, subjected to suction filtration, and the filtrate was concentrated to obtain a crude product. The crude product was purified by silica gel column chromatography (PE/EA = 100 to 70%) to obtain 1 g of the title compound **142-3.** LC-MS (ESI): m/z 156.9 $[M+H]^+$.

### Preparation of compound 142-4

Compound **142-3** (1 g, 6.39 mmol) was dissolved in MeOH (50 mL), and sodium borohydride (241 mg, 6.39 mmol) was added. After the addition was completed, the reaction system was stirred at 25°C for 1 h. After the reaction was completed, saturated ammonium chloride (100 mL) was added, and the mixture was extracted three times with ethyl acetate (100 mL). The organic phases were combined, washed twice with saturated brine (100 mL), dried over anhydrous sodium sulfate, subjected to suction filtration, and the filtrate was concentrated to obtain a crude product. The crude product was purified by silica gel column chromatography (PE/EA = 100 to 60%) to obtain 1 g of the title compound **142-4.** LC-MS (ESI): m/z 158.9 $[M+H]^+$.

### Preparation of compound 142-5

Compound **142-4** (1 g, 6.31 mmol) and triethylamine (638 mg, 6.31 mmol) were dissolved in DCM (50 mL), and methanesulfonyl chloride (722 mg, 6.31 mmol) was added thereto. After the addition was completed, the reaction system was stirred at 25°C for 1 h. After the reaction was completed, the solvent was directly removed by rotary evaporation. The crude product was used directly in the next step without purification. LC-MS (ESI): m/z 237.0 $[M+H]^+$.

### Preparation of compound 142-6

Compound **142-5** (200 mg, 0.84 mmol) and compound **56-1** (381 mg, 0.84 mmol) were dissolved in DMF (10 mL),

and cesium carbonate (275.33 mg, 2.52 mmol) was added. After the addition was completed, the reaction system was stirred at 80°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, subjected to suction filtration, and the filtrate was concentrated to obtain a crude product. The crude product was purified by silica gel column chromatography (PE/EA = 100 to 60%) to obtain 100 mg of the title compound **142-6.** LC-MS (ESI): m/z 591.2 $[M+H]^+$.

**Preparation of compound 142**

Compound **142-6** (100 mg, 0.17 mmol), dimethylphosphine oxide (40 mg, 0.51 mmol), $Pd_2(dba)_3$ (18 mg, 0.02 mmol), Xantphos (27 mg, 0.05 mmol), and DIPEA (66 mg, 0.51 mmol) were dissolved in DMF (5 mL). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 12.9 mg of the title compound **142.** LC-MS (ESI): m/z 633.2 $[M+H]^+$. $^1H$ NMR (400 MHz, Chloroform-d) δ 8.89 (d, *J* = 5.2 Hz, 1H), 7.80 (d, *J* = 2.3 Hz, 1H), 7.71 (dd, *J* = 5.2, 3.2 Hz, 1H), 7.66 (dd, *J* = 8.8, 2.4 Hz, 1H), 7.47 (d, *J* = 2.4 Hz, 1H), 7.43 (d, *J* = 8.4 Hz, 2H), 7.34 (d, *J* = 2.4 Hz, 1H), 7.25 (d, *J* = 6.5 Hz, 2H), 6.94 (d, *J* = 8.8 Hz, 1H), 5.63 (q, *J* = 6.6 Hz, 1H), 4.43 (t, *J* = 6.1 Hz, 2H), 3.87 (t, *J* = 6.1 Hz, 2H), 1.91 (d, *J* = 13.2 Hz, 6H), 1.80 (d, *J* = 6.6 Hz, 3H), 1.69 (s, 6H). $^{31}P$ NMR (162 MHz, Chloroform-d) δ 35.38 (s, 1P).

**Example 143: Preparation of compound 143**

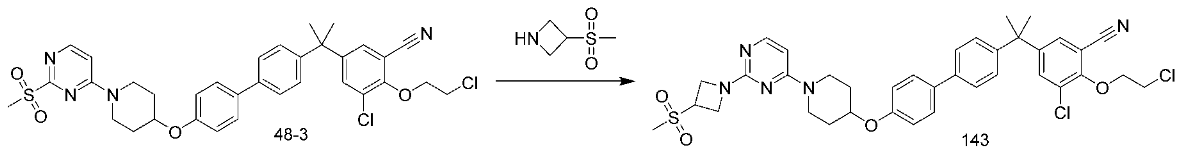

48-3 143

**Preparation of compound 143**

Compound **48-3** (100 mg, 0.15 mmol) was dissolved in acetonitrile (5 mL), followed by the addition of 3-(methylsulfonyl)azetidine (60 mg, 0.45 mmol) and potassium carbonate (62 mg, 0.45 mmol). The reaction system was stirred at 80°C for 16 h. After the reaction was completed, the reaction mixture was cooled to room temperature, and insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 27.2 mg of the title compound **143.** LC-MS (ESI): m/z 720.3 $[M+H]^+$. $^1H$ NMR (400 MHz, Chloroform-d) δ 7.85 (d, *J* = 6.6 Hz, 1H), 7.56 - 7.47 (m, 5H), 7.37 (d, *J* = 2.4 Hz, 1H), 7.23 (d, *J* = 8.3 Hz, 2H), 7.00 (d, *J* = 8.5 Hz, 2H), 6.09 (d, *J* = 6.7 Hz, 1H), 4.76 - 4.62 (m, 1H), 4.63 - 4.47 (m, 4H), 4.43 (t, *J* = 6.2 Hz, 2H), 4.12 - 4.03 (m, 1H), 3.94 - 3.71 (m, 6H), 2.95 (s, 3H), 2.02 - 1.93 (m, 4H), 1.70 (s, 6H).

**Example 144: Preparation of compound 144**

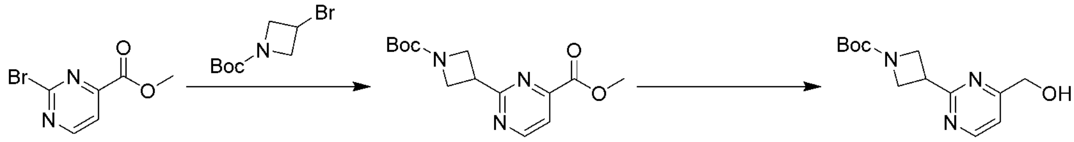

144-1 144-2 144-3

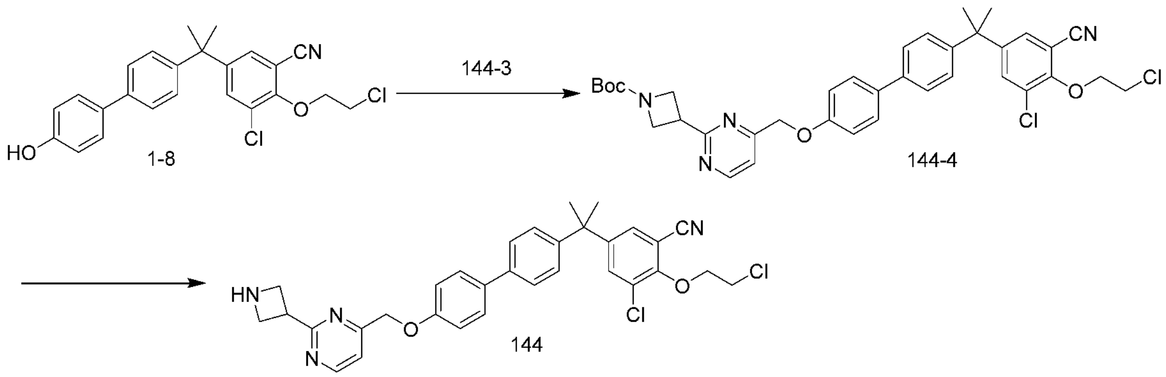


1-8

144-4

144

**Preparation of compound 144-2**

Compound **144-1** (300 mg, 1.38 mmol) was dissolved in 1,4-dioxane (2 mL), followed by the sequential addition of *tert*-butyl 3-bromoazetidine-1-carboxylate (326 mg, 1.38 mmol), 2,6-dimethylpyridine (148 mg, 1.38 mmol), tris(trimethylsilyl)silane (344 mg, 1.38 mmol), iridium(III) [4,4'-bis(1,1-dimethylethyl)-2,2'-bipyridine-N1,N1']bis[3,5-difluoro-2-[5-(trifluoromethyl)-2-pyridinyl-N]phenyl-C] hexafluorophosphate (155 mg, 0.138 mmol), and [4,4'-bis(1,1-dimethylethyl)-2,2'-bipyridine]nickel(II) dichloride (55 mg, 0.138 mmol). The reaction system was purged three times with nitrogen and stirred at room temperature under irradiation with 34 W blue light for 12 h. After the reaction was completed, water (10 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 50 mg of the title compound **144-2.** $^1$H NMR (400 MHz, Chloroform-d) δ 9.00 (d, *J* = 5.0 Hz, 1H), 7.87 (d, *J* = 4.9 Hz, 1H), 4.38 - 4.26 (m, 4H), 4.20 - 4.14 (m, 1H), 4.03 (s, 3H), 1.46 (s, 9H).

**Preparation of compound 144-3**

Compound **144-2** (50 mg, 0.170 mmol) was dissolved in tetrahydrofuran (2 mL) and cooled to 0°C. DIBAL-H (0.5 mL, 1 mol /L in THF) was added dropwise. After the addition was completed, the reaction system was stirred at room temperature for 1 h. After the reaction was completed, water (10 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 15 mg of the title compound **144-3.** LC-MS (ESI): m/z 210.0 $[M+H-56]^+$.

**Preparation of compound 144-4**

Compound **144-3** (30 mg, 0.113 mmol) was dissolved in dichloromethane (2 mL), followed by the sequential addition of compound 1-8 (48 mg, 0.113 mmol) and DIAD (27 mg, 0.169 mmol). A solution of tributylphosphine (34 mg, 0.169 mmol) in dichloromethane (0.5 mL) was added dropwise to the reaction system at 0°C. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 50 mg of the title compound **144-4.** LC-MS (ESI): m/z 673.2 $[M+H]^+$.

**Preparation of compound 144**

Compound **144-4** (45 mg, 0.0668 mmol) was dissolved in dichloromethane (3 mL). TFA (1 mL) was added dropwise to the reaction system at 0°C. After the addition was completed, the reaction was maintained at this temperature for 1 h. After the reaction was completed, the reaction mixture was concentrated to obtain a crude product. The crude product was purified by silica gel column chromatography (EA/PE = 0 to 50%) to obtain the title compound 144. LC-MS (ESI): m/z 573.2 $[M+H]^+$.

**Example 145: Preparation of compound 145**

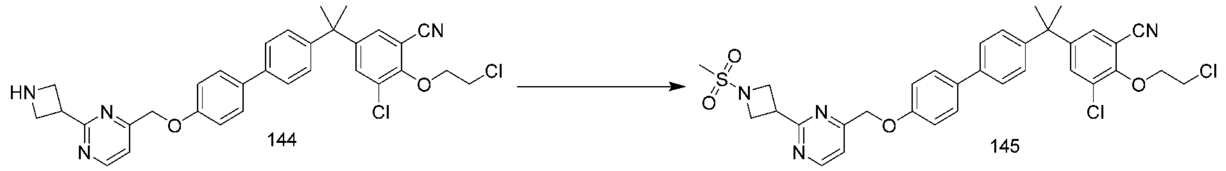

144

145

**Preparation of compound 145**

Compound **144** (20 mg, 0.035 mmol) was dissolved in acetonitrile (2 mL), followed by the sequential addition of TEA (7 mg, 0.070 mmol) and methanesulfonyl chloride (8 mg, 0.070 mmol). The reaction system was stirred at 0°C for 1 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5 mg of the title compound **145.** LC-MS (ESI): m/z 651.3 $[M+H]^+$. $^1$H NMR (400 MHz, Chloroform-*d*) δ 8.76 (d, *J* = 5.1 Hz, 1H), 7.54 (d, *J* = 8.7 Hz, 2H), 7.49 (dq, *J* = 6.4, 2.4 Hz, 4H), 7.37 (d, *J* = 2.3 Hz, 1H), 7.23 (d, *J* = 8.4 Hz, 2H), 7.03 (d, *J* = 8.7 Hz, 2H), 5.19 (s, 2H), 4.42 (t, *J* = 6.2 Hz, 2H), 4.35 (p, *J* = 7.8 Hz, 4H), 4.18 - 4.09 (m, 1H), 3.88 (t, *J* = 6.2 Hz, 2H), 2.97 (s, 3H), 1.69 (s, 6H).

**Example 146: Preparation of compound 146**

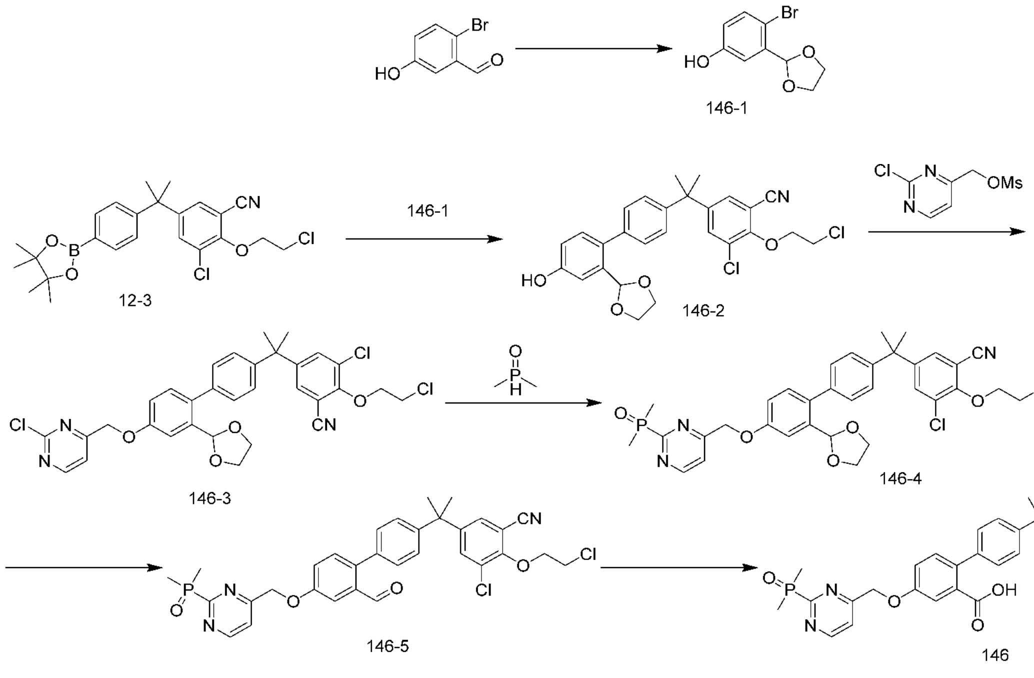


146-1

12-3

146-2

146-3

146-4

146-5

146

**Preparation of compound 146-1**

Compound 2-bromo-5-hydroxybenzaldehyde (11 g, 54.72 mmol) was dissolved in toluene (90 mL), and ethylene glycol (11.2 g, 180.58 mmol) and p-toluenesulfonic acid monohydrate (0.19 g, 1.09 mmol) were added. The reaction system was heated to 160°C and stirred for 6 h. The reaction was monitored by LCMS until completion. The system was cooled to room temperature, quenched with ice water (100 mL), and extracted three times with ethyl acetate (300 mL). The organic phases were combined, dried over anhydrous sodium sulfate, subjected to suction filtration, and the filtrate was concentrated. The resulting residue was subjected to normal-phase silica gel column chromatography (EA/0.1% TEA +

PE = 0 to 30%) to obtain 9 g of the title compound **146-1** in 63% yield. LC-MS (ESI): m/z 244.6 $[M-H]^-$.

**Preparation of compound 146-2**

Compound **146-1** (500 mg, 1.09 mmol), compound **12-3** (290 mg, 1.20 mmol), Pd(dppf)$Cl_2$ (79 mg, 0.11 mmol), and potassium carbonate (450 mg, 3.27 mmol) were added to a mixed solvent of dioxane (6 mL) and water (1 mL). The reaction system was heated to 110°C and stirred for 2 h. The reaction was monitored by LCMS until completion. The mixture was cooled to room temperature, filtered through a pad of diatomite, and rinsed with ethyl acetate. The filtrates were combined and concentrated. The resulting residue was purified by normal-phase silica gel column chromatography (EA/0.1% TEA + PE = 0 to 50%) to obtain 600 mg of a crude product of the title compound **146-2,** which was used directly in the next step without purification. LC-MS (ESI): m/z 496.0 $[M-H]^-$.

**Preparation of compound 146-3**

Compound **146-2** (500 mg, 1.00 mmol) and (2-chloropyrimidin-4-yl)methyl methanesulfonate (245 mg, 1.10 mmol) were dissolved in acetonitrile (6 mL). Cesium carbonate (490 mg, 1.50 mmol) was added, and the system was heated to 70°C and stirred for 2 h. The reaction was monitored by LCMS until completion. The system was cooled to room temperature, filtered through a pad of diatomite, and rinsed with acetonitrile. The filtrates were combined and concentrated. The resulting residue was purified by normal-phase silica gel column chromatography (EA/0.1% TEA + PE = 0 to 25%) to obtain 400 mg of the title compound **146-3** in 63% yield. LC-MS (ESI): m/z 623.7 $[M-H]^-$.

**Preparation of compound 146-4**

Compound **146-3** (300 mg, 0.48 mmol), dimethylphosphine oxide (150 mg, 1.92 mmol), dry DIPEA (310 mg, 2.40 mmol), DPPP (29 mg, 0.072 mmol), and palladium acetate (21 mg, 0.096 mmol) were dissolved in DMF (2 mL). The system was purged with argon, heated to 120°C, and the reaction was carried out under microwave irradiation for half an hour. The reaction was monitored by LCMS until completion. The system was cooled to 0°C, quenched with saturated sodium chloride solution (5 mL), and extracted three times with ethyl acetate (5 mL). The organic phases were combined, dried over anhydrous sodium sulfate, subjected to suction filtration, and the filtrate was concentrated to obtain 500 mg of a crude product of the title compound **146-4.** LC-MS (ESI): m/z 666.0 $[M+H]^+$.

**Preparation of compound 146-5**

Compound **146-4** (200 mg, 0.30 mmol) was dissolved in tetrahydrofuran (5 mL),and hydrochloric acid (1 mL, 1 mol/L) was added. The mixture was stirred at room temperature for 1 h, and the reaction was monitored by LCMS until completion. The reaction was quenched with ice water and extracted three times with ethyl acetate (10 mL). The organic phases were combined, dried over anhydrous sodium sulfate, subjected to suction filtration, and the filtrate was concentrated to obtain 400 mg of a crude product of the title compound **146-5,** which was used directly in the next step without purification.

**Preparation of compound 146**

Compound **146-5** (400 mg, 0.64 mmol), $NaClO_2$ (73 mg, 0.80 mmol), $NaH_2PO_4$ (768 mg, 6.40 mmol), and 2-methyl-2-butene (449 mg, 6.40 mmol) were added to a microwave tube containing *t*-BuOH/$H_2O$ = 5/1 (6 mL), and the mixture was stirred for 1 h. The reaction was monitored by LCMS until completion. The reaction was quenched with ice water and extracted three times with ethyl acetate (10 mL). The organic phases were combined, dried over anhydrous sodium sulfate, subjected to suction filtration, and the filtrate was concentrated. The residue was purified by preparative liquid chromatography (Waters 2767/Qda, column: Sunfire C18 19 * 250 mm, 10 μm; mobile phase A: 0.1% FA/$H_2O$, B: acetonitrile; flow rate: 20 mL/min; elution gradient: 55% to 55%; retention time: 9.0-10.4 min of 16 min) to obtain 31.4 mg of the title compound **146.** LC-MS (ESI): m/z 638.3 $[M+H]^+$. $^1$H NMR (400 MHz, DMSO-$d_6$) δ 9.00 (d, *J= 5.2* Hz, 1H), 7.73 (dd, *J* = 5.0, 3.2 Hz, 1H), 7.70 (d, *J* = 2.3 Hz, 1H), 7.64 (d, *J* = 2.3 Hz, 1H), 7.34 - 7.19 (m, 6H), 7.11 (dd, *J* = 8.6, 2.8 Hz, 1H), 5.35 (s, 2H), 4.42 (t, *J* = 5.2 Hz, 2H), 3.96 (t, *J* = 5.2 Hz, 2H), 1.77 (d, *J* = 13.7 Hz, 6H), 1.68 (s, 6H). $^{31}$P NMR (162 MHz, DMSO-$d_6$) δ 34.25 (s, 1P).

**Example 147: Preparation of compound 147**

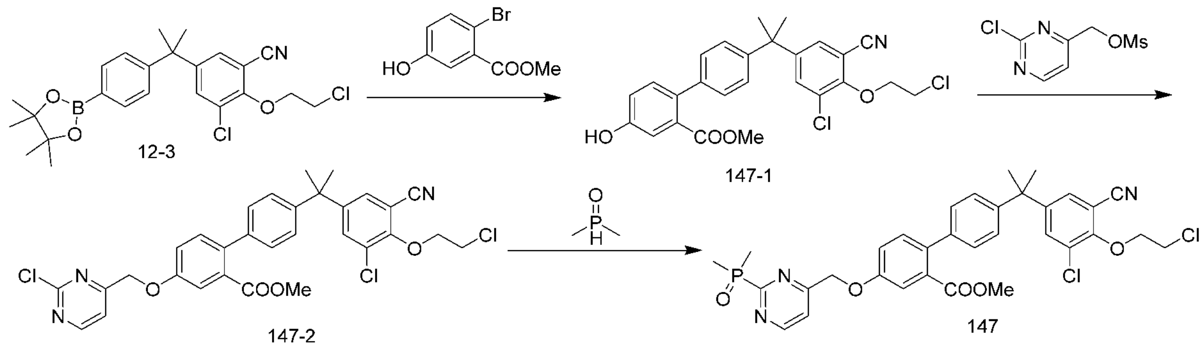

12-3

147-1

147-2

147

**Preparation of compound 147-1**

Compound **12-3** (1 g, 2.17 mmol) and methyl 2-bromo-5-hydroxybenzoate (600 mg, 2.60 mmol) were dissolved in 1,4-dioxane (15 mL). Pd(dppf)$Cl_2$ (159 mg, 0.22 mmol) and potassium carbonate (900 mg, 6.51 mmol) were added. The system was purged four times with argon, heated to 110°C, and stirred for 3 h. The reaction system was cooled to room temperature, acidified with 2 mol/L aqueous hydrochloric acid solution, filtered through a pad of diatomite, and the filter cake was rinsed with ethyl acetate (30 mL). The filtrates were combined and washed once with saturated brine (10 mL). The organic phase was dried over anhydrous sodium sulfate, subjected to suction filtration, and the filtrate was concentrated. The resulting residue was purified by silica gel column chromatography (EA/PE = 0 to 50%) to obtain 1.09 g of the title compound **147-1** in 95% yield. LC-MS (ESI): m/z 481.9 $[M-H]^-$.

**Preparation of compound 147-2**

Compound **147-1** (600 mg, 1.24 mmol) and (2-chloropyrimidin-4-yl)methyl methanesulfonate (290 mg, 1.30 mmol) were dissolved in acetonitrile (5 mL). Cesium carbonate (1.2 g, 3.72 mmol) was added at room temperature, and the mixture was heated to 70°C and stirred for 2 h. The reaction was monitored by LCMS until completion. The mixture was cooled to room temperature, filtered, and the filter cake was rinsed with ethyl acetate. The filtrates were combined and concentrated. The resulting residue was purified by silica gel column chromatography (EA/PE = 0 to 60%) to obtain 650 mg of the title compound **147-2** in 86% yield. LC-MS (ESI): m/z 611.1 $[M+H]^+$.

**Preparation of compound 147**

Compound **147-2** (500 m, 0.82 mmol), dimethylphosphine oxide (256 mg, 3.28 mmol), DIPEA (636 mg, 4.92 mmol), 1,3-bis(diphenylphosphino)propane (102 mg, 0.25 mmol), and solvent DMF (2 mL) were added to a microwave tube. The mixture was stirred until a clear solution was obtained. Palladium acetate (55 mg, 0.25 mmol) was added at room temperature. The system was purged with argon, and the reaction was carried out under microwave irradiation at 120°C for 0.5 h. The reaction was monitored by LCMS until completion. Water (10 mL) was added, and the mixture was extracted twice with ethyl acetate (20 mL). The organic phases were combined and concentrated. The resulting residue was subjected to preparative purification (preparative column: Atlantis TM T3 prep OBD TM, 19 * 250 mm * 10 μm; flow rate: 20 mL/min; mobile phase: A - 0.1% aqueous FA solution, B - acetonitrile; gradient: 70 to 80%, retention time 7.4 to 8.2 min) to obtain 37.1 mg of the title compound **147.** LC-MS (ESI): m/z 652.3 $[M+H]^+$. $^1H$ NMR (400 MHz, DMSO-$d_6$) δ 9.01 (d, *J* = 5.2 Hz, 1H), 7.77 (dd, *J* = 5.1, 3.2 Hz, 1H), 7.66 (d, *J* = 2.3 Hz, 1H), 7.61 (d, *J* = 2.3 Hz, 1H), 7.44 - 7.38 (m, 2H), 7.34 - 7.25 (m, 3H), 7.20 (d, *J* = 8.3 Hz, 2H), 5.40 (s, 2H), 4.42 (t, *J* = 5.2 Hz, 2H), 3.96 (t, *J* = 5.2 Hz, 2H), 3.57 (s, 3H), 1.76 (d, *J* = 13.7 Hz, 6H), 1.69 (s, 6H). $^{31}P$ NMR (162 MHz, DMSO-$d_6$) δ 34.03 (s, 1P).

**Example 148: Preparation of compound 148**

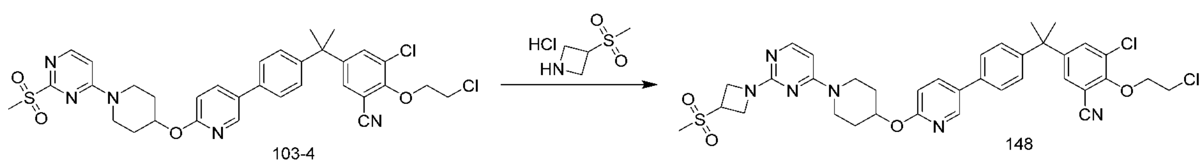

103-4

148

**Preparation of compound 148**

Compound **103-4** (55 mg, 82 μmol), 3-(methylsulfonyl)azetidine hydrochloride (28 mg, 165 μmol), and cesium carbonate (53 mg, 165 μmol) were added to acetonitrile (2 mL). The reaction system was stirred at 120°C for 2 h. After the reaction was completed, the reaction mixture was cooled to room temperature. Saturated brine (5 mL) was added to the reaction mixture, and the mixture was extracted three times with ethyl acetate (7 mL). The organic phases were combined, dried, and subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 35% acetonitrile gradient elution over 12 min; flow rate: 30 mL/min) to obtain 21 mg of the title compound **148.** LC-MS (ESI): m/z 721.4 $[M+H]^+$. $^1H$ NMR (400 MHz, DMSO-$d_6$) δ 8.47 (d, *J* = 2.6 Hz, 1H), 8.00 (dd, *J* = 8.7, 2.6 Hz, 1H), 7.89 (d, *J*= 6.0 Hz, 1H), 7.71 (d, *J* = 2.3 Hz, 1H), 7.65 (d, *J* = 2.4 Hz, 1H), 7.60 (d, *J* = 8.4 Hz, 2H), 7.34 (d, *J* = 8.4 Hz, 2H), 6.89 (d, *J* = 8.6 Hz, 1H), 6.26 (d, *J*= 6.1 Hz, 1H), 5.35 - 5.25 (m, 1H), 4.43 (t, *J* = 5.2 Hz, 2H), 4.38 - 4.29 (m, 1H), 4.21 (t, *J* = 8.8 Hz, 2H), 4.16 - 4.10 (m, 2H), 4.09 - 3.99 (m, 2H), 3.96 (t, *J* = 5.2 Hz, 2H), 3.43 - 3.34 (m, 2H), 3.03 (s, 3H), 2.09 - 2.01 (m, 2H), 1.69 (s, 6H), 1.66 - 1.58 (m, 2H).

**Example 149: Preparation of compound 149**

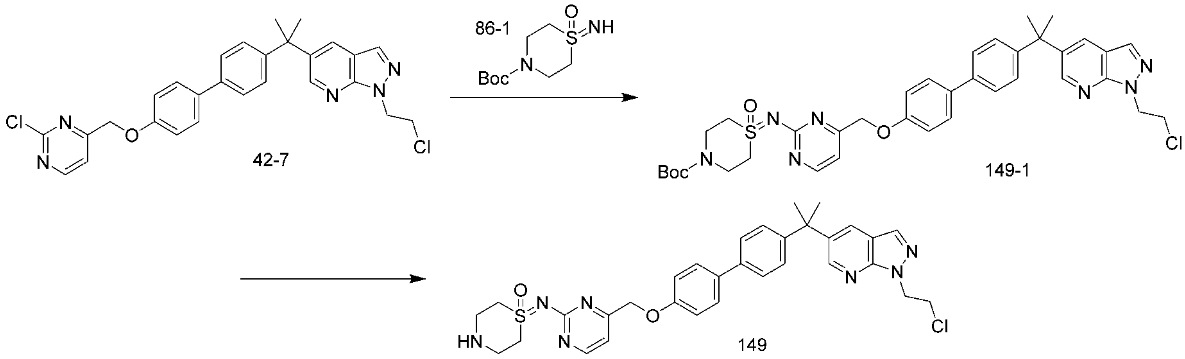


42-7

149-1

149

**Preparation of compound 149-1**

Compound **42-7** (210 mg, 405 μmol) was dissolved in DMF (3 mL), followed by the sequential addition of $Pd_2(dba)_3$ (31.5 mg, 34.37 μmol), Xantphos (40 mg, 68.73 μmol), DIPEA (105 mg, 812 μmol), and compound **86-1** (161 mg, 687.31 μmol). The reaction system was stirred at 120°C for 3 h. After the reaction was monitored by LCMS until completion, the reaction mixture was cooled to room temperature, poured into ice water, and extracted twice with ethyl acetate (20 mL). The organic phases were combined, dried over anhydrous sodium sulfate, subjected to suction filtration, and the filtrate was concentrated to obtain a crude product. The crude product was purified by silica gel column chromatography (EA/PE = 0 to 40%) to obtain 120 mg of the title compound **149-1** in 41% yield. LC-MS (ESI): m/z 716.4 $[M+H]^+$.

**Preparation of compound 149**

Compound **149-1** (60 mg, 84 μmol) was dissolved in dichloromethane (2 mL), and a solution of hydrogen chloride in 1,4-dioxane (0.5 mL, 4 M) was added dropwise with stirring. After the addition was completed, the reaction was carried out at room temperature overnight. After the reaction was monitored by LCMS until completion, the reaction mixture was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 45% acetonitrile gradient elution over 12 min; flow rate: 30 mL/min) to obtain 36 mg of the title compound **149.** LC-MS (ESI): m/z 616.4 $[M+H]^+$. $^1H$ NMR (400 MHz, DMSO-$d_6$) δ 8.44 (d, *J*= 5.0 Hz, 1H), 8.39 (d, *J*= 2.2 Hz, 1H), 8.19 (d, *J*= 2.2 Hz, 1H), 8.17 (s, 1H), 7.58 (d, *J* = 8.8 Hz, 2H), 7.54 (d, *J* = 8.4 Hz, 2H), 7.31 (d, *J* = 8.5 Hz, 2H), 7.06 (d, *J* = 8.8 Hz, 2H), 6.97 (d, *J* = 5.0 Hz, 1H), 5.10 (s, 2H), 4.75 (t, *J*= 5.8 Hz, 2H), 4.12 (t, *J*= 5.8 Hz, 2H), 3.68 - 3.60 (m, 2H), 3.40 - 3.34 (m, 2H), 3.24 - 3.16 (m, 2H), 3.09 - 3.01 (m, 2H), 1.77 (s, 6H).

**Example 150: Preparation of compound 150**

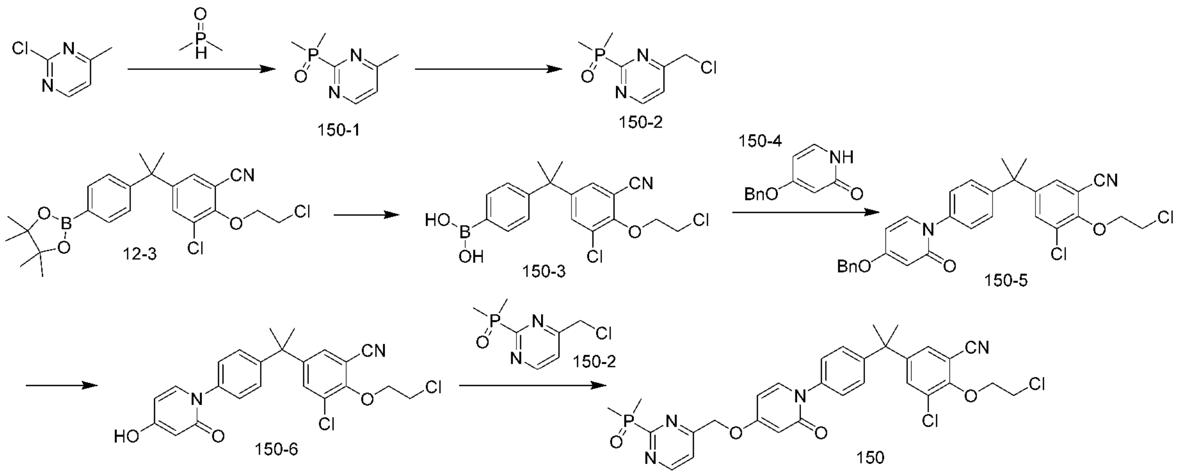


**Preparation of compound 150-1**

2-Chloro-4-methylpyrimidine (1.0 g, 7.78 mmol), dimethylphosphine oxide (2.4 g, 31.12 mmol), DIPEA (5.0 g, 38.9 mmol), 1,3-bis(diphenylphosphino)propane (481 mg, 1.17 mmol), and solvent DMF (10 mL) were added to a microwave tube. The mixture was stirred until a clear solution was obtained. Palladium acetate (350 mg, 1.56 mmol) was added at room temperature. The system was purged with argon, and the reaction was carried out under microwave irradiation at 120°C for 0.5 h. The reaction was monitored by LCMS until completion. Water (10 mL) was added, and the mixture was extracted three times with ethyl acetate (50 mL). The organic phase was washed three times with saturated brine (50 mL), dried over anhydrous sodium sulfate, subjected to suction filtration, and the filtrate was concentrated. The resulting residue was purified by silica gel column chromatography (MeOH/DCM = 0 to 10%) to obtain 1.1 g of the title compound **150-1** in 83% yield. LC-MS (ESI): m/z 171.2 [M+H]$^+$.

**Preparation of compound 150-2**

Compound **150-1** (1.1 g, 6.46 mmol), AIBN (106 mg, 0.65 mmol), and NCS (518 mg, 3.88 mmol) were dissolved in acetonitrile (20 mL). The system was purged with argon, and the mixture was heated to 80°C and stirred for 6 h. The mixture was cooled to room temperature, filtered, and the filter cake was rinsed with acetonitrile (10 mL). The filtrates were combined and concentrated under reduced pressure. The resulting residue was purified by reversed-phase preparative chromatography to obtain 100 mg of the title compound **150-2** in 7% yield. LC-MS (ESI): m/z 205.0 [M+H]$^+$.

**Preparation of compound 150-3**

Compound **12-3** (5.0 g, 10.86 mmol) was dissolved in acetone (50 mL) and water (50 mL). Ammonium acetate (2.51 g, 32.58 mmol) and sodium periodate (7 g, 32.58 mmol) were added at room temperature, and the mixture was stirred at room temperature for 16 h. The reaction was monitored by TLC until completion. The mixture was extracted three times with ethyl acetate (50 mL). The organic phases were combined, washed with saturated brine (50 mL), dried over anhydrous sodium sulfate, subjected to suction filtration, and concentrated under reduced pressure. The resulting residue was purified by silica gel column chromatography (EA/PE = 0 to 50%) to obtain 2 g of the title compound **150-3** in 49% yield. $^1$H NMR (400 MHz, DMSO-$d_6$) δ 7.85 - 7.70 (m, 2H), 7.68 - 7.52 (m, 2H), 7.23 *(t, J*= 7.1 Hz, 2H), 4.42 (t, *J* = 5.2 Hz, 2H), 3.96 (t, *J* = 5.2 Hz, 2H), 1.66 (s, 6H).

**Preparation of compound 150-5**

Compound **150-3** (500 mg, 1.32 mmol) was dissolved in DCE (10 mL). Compound **197-4** (320 mg, 1.59 mmol), pyridine (261 mg, 3.31 mmol), and copper(II) acetate (553 mg, 3.04 mmol) were added at room temperature. The system was purged with oxygen and stirred at 50°C for 5 h. The reaction was monitored by TLC until completion. Water (10 mL) was added, and the mixture was extracted three times with DCM (20 mL). The organic phases were combined, dried over

anhydrous sodium sulfate, subjected to suction filtration, and the filtrate was concentrated. The resulting residue was purified by silica gel column chromatography (EA/PE = 0 to 40%) to obtain 400 mg of the title compound **150-5** in 51% yield. LC-MS (ESI): m/z 533.1 $[M+H]^+$.

**Preparation of compound 150-6**

Compound **150-5** (400 mg, 0.750 mmol) was dissolved in DCM (5 mL). Boron tribromide (563 mg, 2.25 mmol) was added at room temperature, and the mixture was stirred at room temperature for 2 h. The reaction was monitored by TLC until completion. The reaction mixture was concentrated to obtain 300 mg of the title compound **150-6** in 90% yield. LC-MS (ESI): m/z 443.0 $[M+H]^+$.

**Preparation of compound 150**

Compounds **150-6** (100 mg, 0.225 mmol), **150-2** (46 mg, 0.225 mmol), cesium carbonate (147 mg, 0.451 mmol), and acetonitrile (2 mL) were added to a microwave tube. The reaction system was reacted at 70°C for 2 h, and the reaction was monitored by LCMS until completion. The mixture was cooled to room temperature, filtered, and subjected to preparative purification (Waters 2767/Qda, column: Sunfire C18 19 * 250 mm, 10 μm; mobile phase A: 0.1% FA/$H_2O$, B: ACN; flow rate: 20 mL/min; elution gradient: 57% to 57%; retention time: 7.2-8.2 min of 16 min) to obtain 9.6 mg of the title compound **150.** LC-MS (ESI): m/z 611.3 $[M+H]^+$. $^1H$ NMR (400 MHz, DMSO-$d_6$) δ 9.03 (d, *J* = 5.2 Hz, 1H), 7.77 - 7.70 (m, 2H), 7.67 (d, *J* = 2.3 Hz, 1H), 7.64 (d, *J* = 7.7 Hz, 1H), 7.37 (d, *J* = 8.7 Hz, 2H), 7.30 (d, *J* = 8.6 Hz, 2H), 6.19 (dd, *J* = 7.7, 2.7 Hz, 1H), 5.97 (d, *J* = 2.7 Hz, 1H), 5.35 (s, 2H), 4.43 (t, *J* = 5.2 Hz, 2H), 3.96 *(t, J*= 5.2 Hz, 2H), 1.77 (d, *J* = 13.7 Hz, 6H), 1.70 (s, 6H). $^{31}P$ NMR (162 MHz, DMSO-$d_6$) δ 34.09 (s, 1P).

**Example 151: Preparation of compound 151**

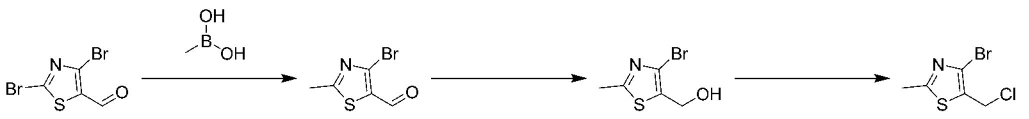

151-1 151-2 151-3 151-4

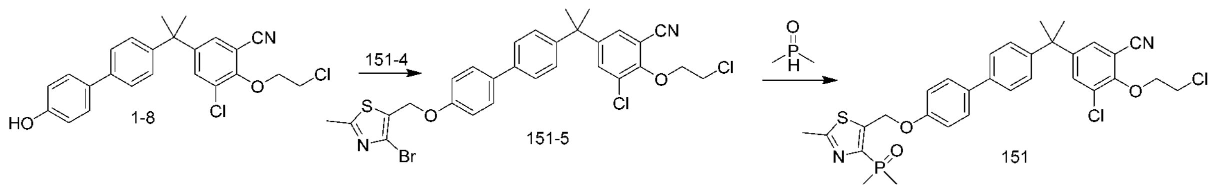


1-8 151-5 151

**Preparation of compound 151-2**

Compound **151-1** (4 g, 14.76 mmol) and methylboronic acid (970 mg, 16.24 mmol) were dissolved in 1,4-dioxane (40 mL). Potassium carbonate (4.08 g, 29.52 mmol) and tetrakis(triphenylphosphine)palladium (1.71 g, 1.48 mmol) were added at room temperature, and the system was purged with argon. The mixture was heated to 110°C and stirred for 16 h. The reaction was monitored by TLC until completion. The mixture was cooled to room temperature, filtered, and the filter cake was rinsed with ethyl acetate. The filtrates were combined and concentrated. The resulting residue was purified by silica gel column chromatography (EA/PE = 0 to 50%) to obtain 280 mg of the title compound **151-2** in 9% yield. $^1H$ NMR (400 MHz, $CDCl_3$) δ 9.92 (s, 1H), 2.80 (s, 3H).

**Preparation of compound 151-3**

Compound **151-2** (280 mg, 1.03 mmol) was dissolved in methanol (2 mL). Sodium borohydride (120 mg, 3.09 mmol) was added at 0°C, and the mixture was stirred at room temperature for 2 h. The reaction was monitored by LCMS until completion. The reaction mixture was acidified with 1 mol/L hydrochloric acid solution and extracted three times with ethyl acetate (10 mL). The organic phases were combined, washed with saturated brine (10 mL), dried over anhydrous sodium sulfate, subjected to suction filtration, and the filtrate was concentrated. The resulting residue was purified by silica gel column chromatography (EA/PE = 0 to 35%) to obtain 172 mg of the title compound **151-3** in 80% yield. LC-MS (ESI):

m/z 207.8 $[M+H]^+$.

**Preparation of compound 151-4**

Compound **151-3** (120 mg, 0.58 mmol) was dissolved in dichloromethane (1 mL). Thionyl chloride (140 mg, 1.16 mmol) was added at 0°C, and the mixture was stirred at room temperature for 2 h. The reaction was monitored by LCMS until completion. The reaction mixture was subjected to rotary evaporation until dryness to obtain 100 mg of a crude product of the title compound **151-4** in 77% yield. LC-MS (ESI): m/z 225.9 $[M+H]^+$.

**Preparation of compound 151-5**

Compound **1-8** (200 mg, 0.47 mmol) was dissolved in acetonitrile (2 mL). **151-4** (120 mg, 0.52 mmol), potassium iodide (7.8 mg, 0.047 mmol), and potassium carbonate (130 mg, 0.94 mmol) were added at room temperature, and the mixture was stirred at 45°C for 16 h. The reaction was monitored by LCMS until completion. The reaction mixture was filtered, and the filtrate was concentrated. The resulting residue was purified by silica gel column chromatography (EA/PE = 0 to 35%) to obtain 150 mg of the title compound **151-5** in 52% yield. LC-MS (ESI): m/z 615.4 $[M+H]^+$.

**Preparation of compound 151**

Compound **151-5** (150 mg, 0.24 mmol) was dissolved in *N,N*-dimethylformamide (2 mL). Dimethylphosphine oxide (75 mg, 0.96 mmol), palladium acetate (5.4 mg, 0.024 mmol), 1,3-bis(diphenylphosphino)propane (10 mg, 0.024 mmol), and DIPEA (160 mg, 1.2 mmol) were added at room temperature. The system was purged with argon for 1 min and stirred under microwave irradiation at 120°C for 0.5 h. The reaction was monitored by LCMS until completion. The mixture was cooled to room temperature, filtered, and the filtrate was concentrated. The resulting residue was subjected to preparative purification (Waters 2767/Qda, column: Xbridge C18 19 * 250 mm, 10 μm; mobile phase: A: 0.1% FA/$H_2O$, B: ACN; flow rate: 20 mL/min; gradient: 77% to 87%; retention time: 9.0-9.8 min of 16 min) to obtain 23 mg of the title compound **151.** LC-MS (ESI): m/z 613.0 $[M+H]^+$. $^1$H NMR (400 MHz, DMSO-$d_6$) δ 7.70 (d, *J* = 2.3 Hz, 1H), 7.63 (d, *J* = 2.3 Hz, 1H), 7.60 (d, *J* = 8.8 Hz, 2H), 7.55 (d, *J* = 8.4 Hz, 2H), 7.30 (d, *J* = 8.5 Hz, 2H), 7.11 (d, *J* = 8.8 Hz, 2H), 5.77 (s, 2H), 4.42 (t, *J* = 5.2 Hz, 2H), 3.95 (t, *J* = 5.2 Hz, 2H), 2.65 (s, 3H), 1.70 (d, *J* = 13.8 Hz, 6H), 1.68 (s, 6H). $^{31}$P NMR (162 MHz, DMSO-*d6*) δ 33.42 (s,1P).

**Example 152: Preparation of compound 152**

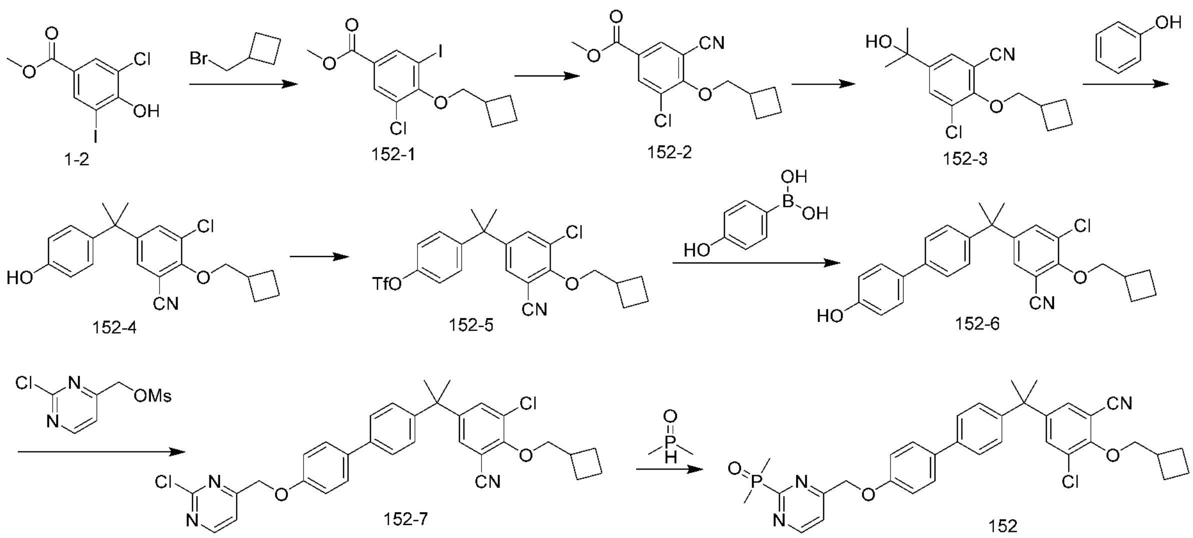

1-2, 152-1, 152-2, 152-3, 152-4, 152-5, 152-6, 152-7, 152

**Preparation of compound 152-1**

Compound **1-2** (15 g, 48 mmol) and (bromomethyl)cyclobutane (28.6 g, 192 mmol) were dissolved in acetonitrile (200 mL). Cesium carbonate (31.3 g, 96 mmol) was added at room temperature, and the mixture was stirred at 70°C for 16 h. The reaction was monitored by TLC until completion. The mixture was filtered, and the filtrate was concentrated. The

resulting residue was purified by silica gel column chromatography (EA/PE = 0 to 30%) to obtain 13 g of the title compound **152-1** in 71% yield. $^{1}$H NMR (400 MHz, $CDCl_3$) δ 8.25 (d, *J* = 6.0 Hz, 1H), 7.93 (d, *J* = 6.0 Hz, 1H), 3.95 (d, *J*= 6.4 Hz, 2H), 3.82 (s, 3H), 2.77 - 2.82 (m, 1H), 2.04 - 2.09 (m, 2H), 1.88 - 1.92 (m, 4H).

**Preparation of compound 152-2**

Compound **152-1** (12 g, 31.53 mmol) and copper(I) cyanide (11.3 g, 126.12 mmol) were dissolved in DMF (120 mL), and the mixture was stirred at 100°C for 16 h. TLC monitoring indicated that the reaction was complete. The mixture was cooled to room temperature, filtered, and the filtrate was concentrated. The resulting residue was purified by silica gel column chromatography (EA/PE = 0 to 5%) to obtain 5 g of the title compound **152-2 in** 56.7% yield. $^{1}$H NMR (400 MHz, $CDCl_3$) δ 8.17 (d, *J* = 2.4 Hz, 1H), 8.09 (d, *J* = 2.4 Hz, 1H), 4.25 (d, *J* = 6.8 Hz, 2H), 3.86 (s, 3H), 2.77 - 2.84 (m, 1H), 2.08 - 2.12 (m, 2H), 1.88 - 1.89 (m, 4H).

**Preparation of compound 152-3**

Compound **152-2** (5 g, 17.88 mmol) was dissolved in dry tetrahydrofuran (50 mL). Methylmagnesium bromide (24 mL, 71.52 mmol) was added at -10°C, and the mixture was stirred at room temperature for 2 h. The reaction was monitored by TLC until completion. Saturated ammonium chloride solution (100 mL) was added to quench the reaction, and the mixture was extracted three times with ethyl acetate (50 mL). The organic phases were combined, washed with saturated brine (50 mL), dried over anhydrous sodium sulfate, subjected to suction filtration, and the filtrate was concentrated. The resulting residue was purified by silica gel column chromatography (EA/PE = 0 to 20%) to obtain 4.5 g of the title compound **152-3** in 89% yield. 1H NMR (400 MHz, $CDCl_3$) δ 7.65 (d, *J* = 2.3 Hz, 1H), 7.51 (d, *J= 2.3* Hz, 1H), 4.09 (d, *J* = 6.7 Hz, 2H), 2.76 - 2.78 (m, 1H), 2.13 - 2.04 (m, 2H), 1.86 - 1.89 (m, 4H), 1.49 (s, 6H).

**Preparation of compound 152-4**

Compound **152-3** (4.5 g, 16.09 mmol) was dissolved in dichloromethane (45 mL). Phenol (1.6 g, 17.70 mol) was added at room temperature, followed by the addition of boron trifluoride diethyl etherate (6.85 g, 48.27 mmol) at 0°C. The mixture was stirred at room temperature for 3 h, and the reaction was monitored by TLC until completion. Water (100 mL) was added, and the mixture was extracted three times with dichloromethane (50 mL). The organic phases were combined, washed with saturated brine (50 mL), dried over anhydrous sodium sulfate, subjected to suction filtration, and the filtrate was subjected to rotary evaporation until dryness to obtain 4.7 g of a crude product of the title compound **152-4** in 82% yield. LC-MS (ESI): m/z 354.1 $[M-H]^-$.

**Preparation of compound 152-5**

Compound **152-4** (3.7 g, 10.40 mmol) was dissolved in dichloromethane (40 mL). Pyridine (1.65 g, 20.8 mmol) was added at room temperature, followed by the addition of trifluoromethanesulfonic anhydride (4.4 g, 15.60 mmol) at 0°C. The mixture was stirred at room temperature for 3 h. The reaction was monitored by TLC until completion. An aqueous solution (50 mL) was added, and the mixture was extracted three times with dichloromethane (50 mL). The organic phases were combined, washed with saturated brine (50 mL), dried over anhydrous sodium sulfate, subjected to suction filtration, and the filtrate was concentrated. The resulting residue was purified by silica gel column chromatography (EA/PE = 0 to 25%) to obtain 4.2 g of the title compound **152-5** in 82% yield. $^{1}$H NMR (400 MHz, $CDCl_3$) δ 7.39 (m, 1H), 7.33 - 7.29 (m, 1H), 7.28 - 7.20 (m, 4H), 4.18 (d, *J* = 6.7 Hz, 2H), 2.91 - 2.78 (m, 1H), 2.25 - 2.07 (m, 2H), 2.05 - 1.90 (m, 4H), 1.67 (s, 6H).

**Preparation of compound 152-6**

Compound **152-5** (3.6 g, 7.38 mmol) was dissolved in 1,4-dioxane (40 mL) and water (10 mL). 4-Hydroxyphenylboronic acid (1.22 g, 8.86 mmol), Pd(dppf)$Cl_2$ (0.54 g, 0.74 mmol), and potassium carbonate (3.06 g, 22.14 mmol) were added at room temperature. The system was purged with nitrogen and stirred at 100°C for 3 h. The reaction was monitored by LCMS until completion. The mixture was cooled to room temperature and filtered. The pH of the filtrate was adjusted to acidic, and an aqueous solution (50 mL) was added. The mixture was extracted three times with ethyl acetate (50 mL). The organic phases were combined, washed with saturated brine (50 mL), dried over anhydrous sodium sulfate, subjected to suction filtration, and the filtrate was concentrated. The resulting residue was purified by silica gel column chromatography (EA/PE = 0 to 25%) to obtain 3 g of the title compound **152-6** in 94% yield. LC-MS (ESI): m/z 429.9 $[M-H]^-$.

**Preparation of compound 152-7**

Compound **152-6** (1 g, 2.32 mmol) was dissolved in acetonitrile (20 mL). (2-Chloropyrimidin-4-yl)methyl methanesulfonate (0.62 g, 2.78 mmol) and cesium carbonate (2.27 g, 6.96 mmol) were added at room temperature. The reaction mixture was stirred at 70°C for 3 h. The reaction was monitored by LCMS until completion. The mixture was cooled to room temperature, filtered, and the filtrate was concentrated. The resulting residue was purified by silica gel column chromatography (EA/PE = 0 to 25%) to obtain 150 mg of the title compound **152-7** in 0.12% yield. LC-MS (ESI): m/z 558.1 $[M+H]^+$.

**Preparation of compound 152**

Compound **152-7** (100 mg, 0.18 mmol), dimethylphosphine oxide (56 mg, 0.72 mmol), palladium acetate (4 mg, 0.018 mmol), 1,3-bis(diphenylphosphino)propane (7.4 mg, 0.018 mmol), and DIPEA (120 mg, 0.9 mmol) were dissolved in DMF (1 mL). The system was purged with argon, and the reaction was carried out under microwave irradiation at 120°C for 0.5 h. The mixture was cooled to room temperature, filtered, and subjected to preparative purification (Waters 2767/Qda, column: Xbridge C18 19 * 250 mm, 10 μm; mobile phase: A: 0.03 % $NH_4OH/H_2O$, B: ACN; flow rate: 20 mL/min; elution gradient: 77% to 77%; retention time: 9.70-10.30 min of 18 min) to obtain 17 mg of the title compound **152.** LC-MS (ESI): m/z 600.1 $[M+H]^+$. $^1H$ NMR (400 MHz, DMSO-$d_6$) δ 9.00 (d, *J*= 5.1 Hz, 1H), 7.73 (dd, *J* = 5.2, 3.2 Hz, 1H), 7.68 (d, *J* = 2.3 Hz, 1H), 7.65 - 7.59 (m, 3H), 7.56 (d, J= 8.5 Hz, 2H), 7.30 (d, *J* = 8.5 Hz, 2H), 7.14 (d, *J* = 8.8 Hz, 2H), 5.35 (s, 2H), 4.11 (d, *J* = 6.6 Hz, 2H), 2.80 - 2.64 (m, 1H), 2.14 - 1.98 (m, 2H), 1.98 - 1.82 (m, 4H), 1.77 (d, *J*= 13.7 Hz, 6H), 1.68 (s, 6H). $^{31}P$ NMR (162 MHz, DMSO-*d6*) δ 34.01 (s,1P).

**Example 153: Preparation of compound 153**

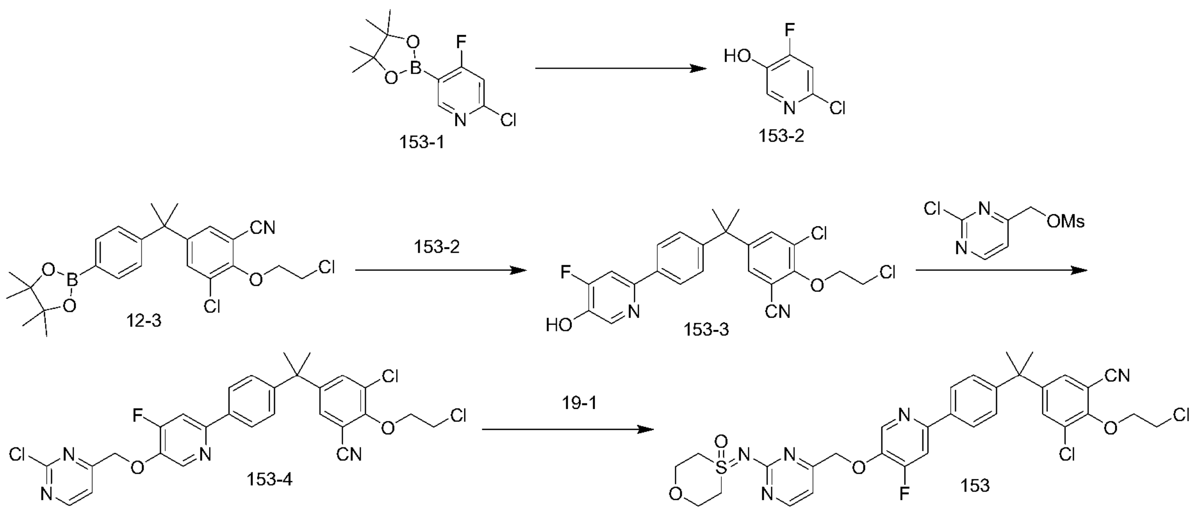


**Preparation of compound 153-2**

Compound **153-1** (1 g, 3.88 mmol) was dissolved in THF (10 mL). The mixture was cooled to 0°C, and sodium hydroxide (160 mg, 3.88 mmol) and hydrogen peroxide (400 mg, 11.64 mmol) were added to the reaction mixture. The mixture was stirred at 0°C for 3 h and then stirred at room temperature for 16 h. The reaction was monitored by LCMS until completion. The system was cooled to room temperature, quenched with ice water (20 mL), and extracted three times with ethyl acetate (30 mL). The organic phases were combined, dried over anhydrous sodium sulfate, subjected to suction filtration, and the filtrate was concentrated. The resulting residue was purified by normal-phase silica gel column chromatography (EA/PE = 0 to 30%) to obtain 240 mg of the title compound **153-2** in 41% yield. LC-MS (ESI): m/z 146.1 $[M-H]^-$.

**Preparation of compound 153-3**

Compounds **12-3** (626 mg, 1.36 mmol) and **153-2** (100 mg, 0.68 mmol) were dissolved in a mixed solvent of 1,4-dioxane (10 mL) and water (0.6 mL). Pd(dppf)$Cl_2$ (50 mg, 0.068 mmol) and potassium carbonate (376 mg, 2.72 mmol) were added. The system was purged four times with argon, heated to 110°C, and stirred for 16 h. The reaction was monitored by LCMS until completion. The reaction system was cooled to room temperature, acidified with 2 mol/L aqueous hydrochloric acid solution, filtered through a pad of diatomite, and the filter cake was rinsed with ethyl acetate (10 mL). The filtrates were combined, washed once with saturated brine (20 mL), dried over anhydrous sodium sulfate, subjected to suction filtration, and the filtrate was concentrated. The resulting residue was purified by normal-phase silica gel column chromatography (EA/PE = 0 to 40%) to obtain 130 mg of the title compound **153-3** in 38% yield. LC-MS (ESI): m/z 445.0 $[M+H]^+$.

**Preparation of compound 153-4**

Compound **153-3** (100 mg, 0.22 mmol) and (2-chloropyrimidin-4-yl)methyl methanesulfonate (54 mg, 0.24 mmol) were dissolved in acetonitrile (5 mL). Cesium carbonate (108 g, 0.33 mmol) was added, and the mixture was heated to 70°C and stirred for 1 h. The reaction was monitored by LCMS until completion. The system was cooled to room temperature, filtered through a pad of diatomite, and rinsed with acetonitrile. The filtrates were combined and concentrated. The resulting residue was purified by normal-phase silica gel column chromatography (EA/PE = 0 to 35%) to obtain 60 mg of the title compound **153-4** in 44.4% yield. LC-MS (ESI): m/z 570.9 $[M+H]^+$.

**Preparation of compound 153**

Compound **153-4** (60 mg, 0.10 mmol), compound **19-1** (27.0 mg, 0.20 mmol), t-Bu-XPhos Pd-G3 (8 mg, 0.01 mmol), and cesium carbonate (97.8 mg, 0.30 mmol) were added to 1,4-dioxane (1 mL). The system was purged with argon, heated to 100°C, and reacted under microwave irradiation for 2 h. The mixture was cooled to 0°C, quenched with saturated sodium chloride solution (3 mL), and extracted three times with ethyl acetate (10 mL). The organic phases were combined, dried over anhydrous sodium sulfate, subjected to suction filtration, and the filtrate was concentrated. The resulting residue was subjected to preparative purification (Waters 2767/Qda, column: Xbridge C18 19 * 250 mm, 10 μm; mobile phase: A: 0.03 % $NH_4OH/H_2O$, B: ACN; flow rate: 20 mL/min; elution gradient: 65% to 65%; retention time: 8.6-9.6 min of 16 min) to obtain 10 mg of the title compound **153.** LC-MS (ESI): m/z 670.3 $[M+H]^+$. $^1$H NMR (400 MHz, DMSO-$d_6$) δ 8.56 (d, *J* = 10.7 Hz, 1H), 8.50 (d, *J* = 5.0 Hz, 1H), 7.99 (d, *J* = 12.8 Hz, 1H), 7.98 (d, *J* = 8.5 Hz, 1H), 7.69 (d, *J* = 2.3 Hz, 1H), 7.62 (d, *J* = 2.3 Hz, 1H), 7.35 (d, *J* = 8.5 Hz, 2H), 7.04 (d, *J* = 5.0 Hz, 1H), 5.32 (s, 2H), 4.42 (t, *J* = 5.2 Hz, 2H), 4.12 - 4.02 (m, 2H), 3.99 - 3.87 (m, 4H), 3.84 - 3.74 (m, 2H), 3.63 - 3.52 (m, 2H), 1.69 (s, 6H). $^{19}$F NMR (376 MHz, DMSO-$d_6$) δ -124.82 (s, 1F).

**Example 154: Preparation of compound 154**

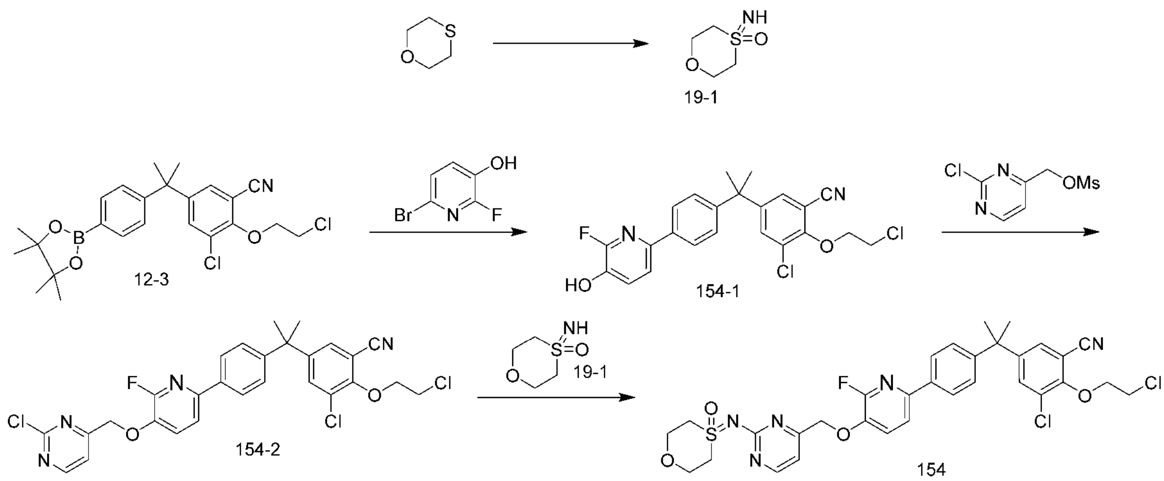


**Preparation of compound 19-1**

Compound thiomorpholine (5 g, 48.0 mmol), ammonium carbamate (3.75 g, 48 mmol), and (diacetoxyiodo) benzene (32.5 g, 100.80 mmol) were dissolved in methanol (50 mL). The reaction was carried out at room temperature for 1 h, and the reaction was monitored by TLC until completion. The reaction mixture was concentrated, adsorbed onto silica

gel, and purified by normal-phase silica gel column chromatography (MeOH/DCM = 0 to 10%) to obtain 4.4 g of the title compound **19-1** in 62% yield. $^1$H NMR (400 MHz, $CDCl_3$) δ 4.14 - 3.98 (m, 4H), 3.16 - 3.05 (m, 4H).

### Preparation of compound 154-1

Compound **12-3** (500 mg, 1.09 mmol) and 6-bromo-2-fluoropyridin-3-ol (209 mg, 1.09 mmol) were dissolved in a mixed solvent of 1,4-dioxane (10 mL) and water (2 mL). Pd(dppf)$Cl_2$ (80 mg, 0.11 mmol) and potassium carbonate (377 mg, 2.73 mmol) were added. The system was purged four times with argon, heated to 110°C, and stirred for 16 h. The reaction was monitored by LCMS until completion. The reaction system was cooled to room temperature, quenched with ice water, and acidified to an acidic pH with 2 mol/L hydrochloric acid solution. The mixture was extracted three times with ethyl acetate (30 mL). The organic phases were combined, dried over anhydrous sodium sulfate, subjected to suction filtration, and the filtrate was concentrated. The resulting residue was purified by normal-phase silica gel column chromatography (EA/PE = 0 to 30%) to obtain 300 mg of the title compound **154-1** in 62% yield. LC-MS (ESI): m/z 442.8 $[M-H]^-$.

### Preparation of compound 154-2

Compound **154-1** (210 mg, 0.47 mmol) and (2-chloropyrimidin-4-yl)methyl methanesulfonate (210 mg, 0.47 mmol) were dissolved in acetonitrile (5 mL). Cesium carbonate (108 g, 0.33 mmol) was added, and the system was heated to 70°C and stirred for 1 h. The reaction was monitored by LCMS until completion. The system was cooled to room temperature, filtered through a pad of diatomite, and rinsed with acetonitrile. The filtrates were combined and concentrated. The residue was purified by normal-phase silica gel column chromatography (EA/PE = 0 to 35%) to obtain 110 mg of the title compound **154-2** in 38% yield. LC-MS (ESI): m/z 571.1 $[M+H]^+$.

### Preparation of compound 154

Compound **154-2** (80 mg, 0.14 mmol), compound **19-1** (19 mg, 0.14 mmol), and t-Bu XPhos Pd G3 (11 mg, 0.014 mmol) were dissolved in dioxane (1 mL), and cesium carbonate (137 mg, 0.42 mmol) was added. The system was purged with argon and reacted under microwave irradiation at 100°C for 1 h. After the reaction was complete, the system was cooled to room temperature. Saturated sodium chloride solution (3 mL) was added to quench the reaction, and the mixture was extracted three times with ethyl acetate (5 mL). The organic phases were combined, dried over anhydrous sodium sulfate, subjected to suction filtration, and the filtrate was concentrated. The resulting residue was subjected to preparative purification (Waters 2767/Qda, column: Xbridge C18 19 * 250 mm, 10 μm; mobile phase: A: 0.03 % $NH_4OH/H_2O$, B: ACN; flow rate: 20 mL/min; elution gradient: 65% to 65%; retention time: 10.20-11.20 min of 16 min) to obtain 11 mg of the title compound **154.** LC-MS (ESI): m/z 670.3 $[M+H]^+$. $^1$H NMR (400 MHz, DMSO-$d_6$) δ 8.49 (d, *J* = 5.0 Hz, 1H), 7.91 (d, *J* = 8.5 Hz, 2H), 7.84 (d, *J* = 8.3 Hz, 1H), 7.80 - 7.70 (m, 1H), 7.69 (d, *J* = 2.3 Hz, 1H), 7.62 (d, *J* = 2.3 Hz, 1H), 7.35 (d, *J* = 8.5 Hz, 2H), 7.01 (d, *J* = 5.0 Hz, 1H), 5.26 (s, 2H), 4.42 (t, *J* = 5.2 Hz, 2H), 4.11 - 4.03 (m, 2H), 3.98 - 3.88 (m, 4H), 3.85 - 3.75 (m, 2H), 3.66 - 3.54 (m, 2H), 1.69 (s, 6H). $^{19}$F NMR (376 MHz, DMSO-*d6*) δ -84.17 (s, 1F).

### Example 155: Preparation of compound 155

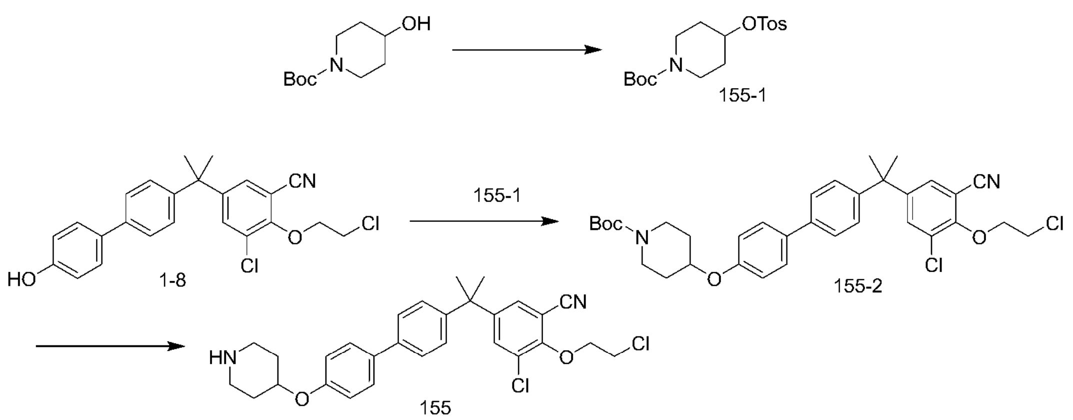


155-1

1-8

155-2

155

**Preparation of compound 155-1**

Compound *tert*-butyl 4-hydroxypiperidine-1-carboxylate (600 mg, 3.0 mmol) was dissolved in DCM (10 mL), followed by the sequential addition of *p*-toluenesulfonyl chloride (626 mg, 3.60 mmol) and DMAP (36 mg, 0.3 mmol). The mixture was cooled to 0°C, and TEA (904 mg, 0.9 mmol) was added. The reaction system was warmed to room temperature and stirred overnight. After the reaction was completed, water (100 mL) was added, and the mixture was extracted with DCM (300 mL). The organic phase was washed once with saturated brine, dried over anhydrous sodium sulfate, subjected to suction filtration, and the filtrate was subjected to rotary evaporation until dryness. The resulting crude product was purified by silica gel column chromatography (EA/PE = 0 to 25%) to obtain 800 mg of the title compound **155-1.** LC-MS (ESI): m/z 300.0 $[M+H-56]^+$.

**Preparation of compound 155-2**

Compound **1-8** (100 mg, 0.23 mmol) was dissolved in DMF (3 mL), followed by the addition of **155-1** (92 mg, 0.26 mmol) and cesium carbonate (153 mg, 0.46 mmol). The reaction system was stirred at 60°C for 16 h. After the reaction was completed, water (50 mL) was added to the reaction mixture, and the mixture was extracted three times with ethyl acetate (100 mL). The organic phases were combined, washed once with saturated brine (100 mL), dried over anhydrous sodium sulfate, subjected to suction filtration, and the filtrate was concentrated to obtain 120 mg of a crude product of the title compound **155-2.** LC-MS (ESI): m/z 553.8 $[M+H-56]^+$.

**Preparation of compound 155**

Compound **155-2** (20 mg, 0.03 mmol) was dissolved in DCM/TFA (2 mL, v/v = 3/1), and the mixture was stirred at room temperature for 2 h. After the reaction was completed, a portion of DCM and TFA was removed from the reaction mixture by rotary evaporation at 45°C. Water was added, and the mixture was extracted three times with DCM (50 mL). The organic phases were combined, washed once with saturated brine (50 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 3 mg of the title compound **155.** $^1$H NMR (400 MHz, DMSO-$d_6$) δ 7.70 (d, *J* = 2.3 Hz, 1H), 7.64 (d, *J* = 2.3 Hz, 1H), 7.59 (d, *J* = 8.7 Hz, 2H), 7.55 (d, *J* = 8.4 Hz, 2H), 7.30 (d, *J* = 8.4 Hz, 2H), 7.06 (d, *J* = 8.8 Hz, 2H), 4.64 (s, 1H), 4.42 (t, *J* = 5.2 Hz, 2H), 3.96 (t, *J* = 5.2 Hz, 2H), 3.24 - 3.16 (m, 2H), 3.05 - 2.93 (m, 2H), 2.13 - 2.01 (m, 2H), 1.81 - 1.71 (m, 2H), 1.68 (s, 6H).

**Example 156: Preparation of compound 156**

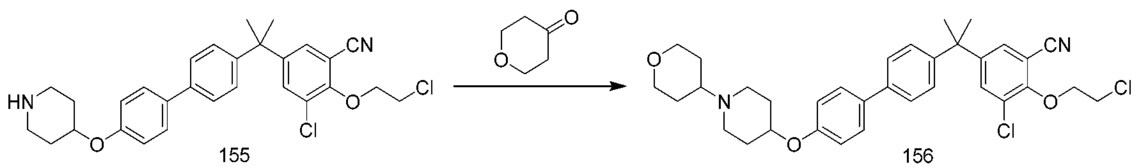

**Preparation of compound 156**

Compound **155** (90 mg, 0.17 mmol) was dissolved in DCM (5 mL), followed by the sequential addition of tetrahydro-4H-pyran-4-one (22 mg, 0.19 mmol). The mixture was cooled to 0°C, and $NaBH(AcO)_3$ (75 mg, 0.35 mmol) was added. The reaction system was warmed to room temperature and stirred overnight. After the reaction was completed, the reaction mixture was quenched with water (100 mL) and extracted with DCM (300 mL). The organic phase was washed once with saturated brine, dried over anhydrous sodium sulfate, subjected to suction filtration, and the filtrate was subjected to rotary evaporation until dryness. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile elution over 12 min; flow rate: 30 mL/min) to obtain 7 mg of the title compound **156.** LC-MS (ESI): m/z 593.4 $[M+H]^+$. $^1$H NMR (400 MHz, DMSO-$d_6$) δ 7.70 (d, *J* = 2.3 Hz, 1H), 7.64 (d, *J* = 2.3 Hz, 1H), 7.56 (d, *J* = 8.5 Hz, 2H), 7.54 (d, *J* = 8.2 Hz, 2H), 7.29 (d, *J* = 8.2 Hz, 2H), 7.01 (d, *J* = 8.7 Hz, 2H), 4.42 (t, *J*= 5.2 Hz, 2H), 3.96 (t, *J*= 5.1 Hz, 2H), 3.92 - 3.80 (m, 1H), 3.30 - 3.09 (m, 4H), 2.84 - 2.64 (m, 3H), 2.43 -

2.30 (m, 2H), 2.29 - 2.15 (m, 2H), 2.04 - 1.90 (m, 2H), 1.78 - 1.54 (m, 8H), 1.01 *(t, J*= 7.1 Hz, 2H).

**Example 157: Preparation of compound 157**

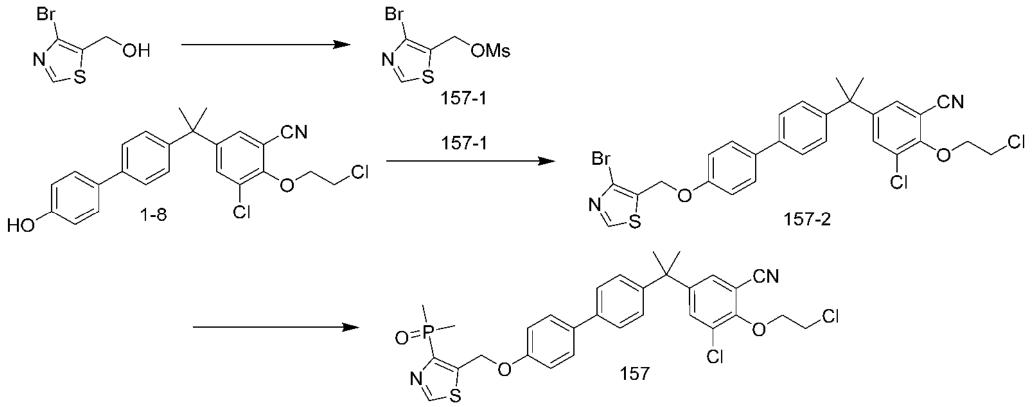

157-1

1-8

157-1

157-2

157

**Preparation of compound 157-1**

Compound (4-bromothiazol-5-yl)methanol (120 mg, 618.40 μmol) was dissolved in dichloroethane (2 mL), and the system was purged three times with nitrogen. Methanesulfonyl chloride (70.84 mg, 618.40 μmol) was added dropwise in an ice bath, and the mixture was stirred at 25°C for 3 h. After the reaction was completed, water (10 mL) was added, and the mixture was extracted three times with dichloromethane (10 mL). The organic phases were combined, washed twice with saturated brine (10 mL), dried over anhydrous sodium sulfate, subjected to suction filtration, and the filtrate was concentrated to obtain a crude product. The crude product was purified by silica gel column chromatography (EA/PE = 0 to 20%) to obtain 127 mg of the title compound **157-1.** LC-MS (ESI): m/z 271.9 $[M+H]^+$.

**Preparation of compound 157-2**

Compound **157-1** (60 mg, 309.2 μmol) was dissolved in acetonitrile (2 mL), followed by the sequential addition of cesium carbonate (143.31 mg, 440.95 μmol) and **1-8** (103 mg, 242.52 μmol). The system was purged three times with nitrogen and stirred at 75°C for 3 h. After the reaction was completed, water (15 mL) was added, and the mixture was extracted three times with dichloromethane (15 mL). The organic phases were combined, washed twice with saturated brine (10 mL), dried over anhydrous sodium sulfate, subjected to suction filtration, and the filtrate was concentrated to obtain a crude product. The crude product was purified by silica gel column chromatography (EA/PE = 0 to 20%) to obtain 110 mg of the title compound **157-2.** LC-MS (ESI): m/z 601.1 $[M+H]^+$.

**Preparation of compound 157**

Compound **157-2** (100 mg, 166.01 μmol) was dissolved in DMF (2 mL), followed by the sequential addition of $Pd_2(dba)_3$ (6 mg, 8.30 μmol), Xantphos (5 mg, 8.30 μmol), DIPEA (43 mg, 332.02 μmol), and dimethylphosphine oxide (26 mg, 332.02 μmol). The mixture was stirred under microwave irradiation at 120°C for 2 h. After the reaction was completed, insoluble solids were removed by filtration. Water (10 mL) was added to the filtrate, and the mixture was extracted three times with dichloromethane (10 mL). The organic phases were combined, washed twice with saturated brine (15 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 35% acetonitrile gradient elution over 12 min; flow rate: 30 mL/min) to obtain 16 mg of the title compound **157.** LC-MS (ESI): m/z 599.2 $[M+H]^+$. $^1H$ NMR (400 MHz, DMSO-$d_6$) δ 9.23 (d, *J* = 0.8 Hz, 1H), 7.70 (d, *J* = 2.4 Hz, 1H), 7.64 (d, *J* = 2.3 Hz, 1H), 7.61 (d, *J* = 8.8 Hz, 2H), 7.56 (d, *J* = 8.5 Hz, 2H), 7.30 (d, *J* = 8.5 Hz, 2H), 7.12 (d, *J* = 8.8 Hz, 2H), 5.83 (s, 2H), 4.42 (t, *J*= 5.2 Hz, 2H), 3.96 (t, *J* = 5.2 Hz, 2H), 1.73 (d, *J* = 13.8 Hz, 6H), 1.68 (s, 6H). $^{31}P$ NMR (162 MHz, DMSO-$d_6$) δ 33.78 (s, 1P).

**Example 158: Preparation of compound 158**

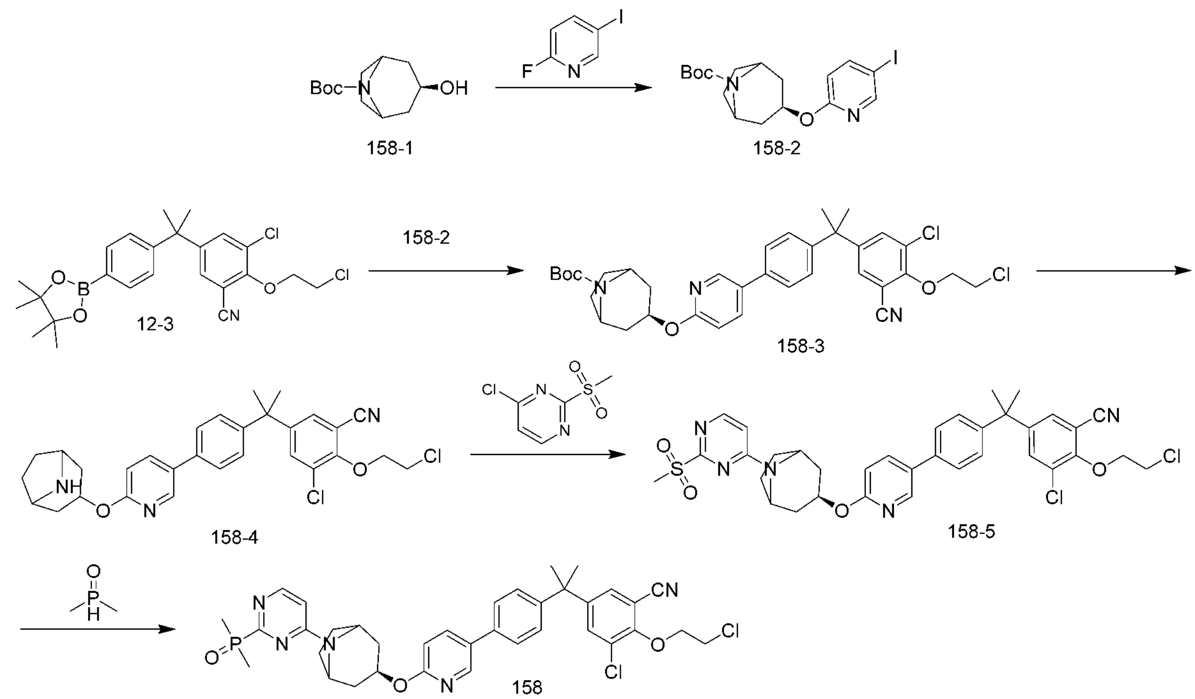

158-1
158-2
12-3
158-3
158-4
158-5
158

**Preparation of compound 158-2**

Compound **158-1** (397 mg, 1.97 mmol) was dissolved in tetrahydrofuran (5 mL). The reaction system was purged three times with nitrogen, and then sodium hydride (144 mg, 3.59 mmol, 60% purity) was added in an ice bath. After 30 min under the ice bath, 2-fluoro-5-iodopyridine (400 mg, 1.79 mmol) was added. After the addition was completed, the reaction system was stirred at 20°C for 15 h. After the reaction was completed, the reaction mixture was filtered. Saturated aqueous sodium chloride solution (15 mL) was added to the filtrate, and the mixture was extracted three times with ethyl acetate (10 mL). The organic phases were combined, dried over anhydrous sodium sulfate, subjected to suction filtration, and the filtrate was concentrated to obtain a crude product. The crude product was purified by normal-phase silica gel column chromatography (EtOAc/PE = 0 to 30%) to obtain 380 mg of the title compound **158-2**in 52% yield. LC-MS (ESI): m/z 431.3 $[M+H]^+$.

**Preparation of compound 158-3**

Compound **158-2**(268 mg, 663 μmol) and compound **12-3**(320 mg, 663 μmol) were dissolved in 1,4-dioxane (5 mL), followed by the sequential addition of potassium carbonate (183 mg, 1.33 mmol) and [1,1'-bis(diphenylphosphino) ferrocene]dichloropalladium(II) dichloromethane complex (27 mg, 33.17 μmol). The system was purged three times with nitrogen and stirred at 105°C for 3 h. After the reaction was completed, the reaction mixture was cooled to room temperature and filtered. Saturated aqueous sodium chloride solution (15 mL) was added to the filtrate, and the mixture was extracted three times with ethyl acetate (10 mL). The organic phases were combined, dried, and concentrated to obtain a crude product. The crude product was purified by normal-phase silica gel column chromatography (EtOAc/PE = 0 to 35%) to obtain 275 mg of the title compound **158-3**in 67% yield. LC-MS (ESI): m/z 636.4 $[M+H]^+$.

**Preparation of compound 158-4**

Compound **158-3** (190 mg, 311.19 μmol) was added to dichloromethane (4 mL), and trifluoromethanesulfonic acid (0.5 mL) was added dropwise in an ice bath. The mixture was stirred at room temperature for 2 h. After the reaction was completed, the reaction mixture was concentrated to obtain 142 mg of a crude product of the title compound **158-4** in 89% yield.

**Preparation of compound 158-5**

Compound **158-4** (130 mg, 254.68 μmol) and triethylamine (52 mg, 509.35 μmol, 71 μL) were dissolved in DCM (3 mL). 4-Chloro-2-(methylsulfonyl)pyrimidine (54 mg, 280.14 μmol) was added in an ice bath, and the reaction system was stirred at room temperature for 2 h. After the reaction was completed, the reaction mixture was filtered. Saturated aqueous sodium chloride solution (10 mL) was added to the filtrate, and the mixture was extracted three times with ethyl acetate (10 mL). The organic phases were combined, dried, filtered, and concentrated to obtain a crude product. The crude product was purified by normal-phase silica gel column chromatography (EtOAc/PE = 0 to 40%) to obtain 105 mg of the title compound **158-5** in 61% yield. LC-MS (ESI): m/z 692.8 $[M+2H]^+$.

**Preparation of compound 158**

Compound **158-5** (25 mg, 37.5 μmol), dimethylphosphine oxide (6 mg, 75.0 μmol), and cesium carbonate (25 mg, 75.0 μmol) were added to acetonitrile (1 mL). The reaction system was stirred at 90°C for 2 h. After the reaction was completed, the reaction mixture was cooled to room temperature. Saturated brine (10 mL) was added to the reaction mixture, and the mixture was extracted three times with ethyl acetate (10 mL). The organic phases were combined, dried, and subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 5% to 30% acetonitrile gradient elution over 12 min; flow rate: 30 mL/min) to obtain 8 mg of the title compound **158.** LC-MS (ESI): m/z 690.3 $[M+H]^+$. $^1$H NMR (400 MHz, DMSO-$d_6$) δ 8.45 (d, *J* = 2.6 Hz, 1H), 8.29 (d, *J* = 6.1 Hz, 1H), 8.02 (dd, *J* = 8.6, 2.6 Hz, 1H), 7.71 (d, *J* = 2.3 Hz, 1H), 7.65 (d, *J* = 2.3 Hz, 1H), 7.60 (d, *J* = 8.3 Hz, 2H), 7.33 (d, *J* = 8.1 Hz, 2H), 6.92 (d, *J* = 8.6 Hz, 1H), 6.84 (dd, *J* = 6.2, 3.1 Hz, 1H), 5.29 (d, *J* = 5.1 Hz, 1H), 5.03 - 4.66 (m, 2H), 4.60 - 4.31 (m, 4H), 3.96 (t, *J* = 5.2 Hz, 2H), 2.31 - 2.21 (m, 2H), 2.19 - 1.99 (m, 4H), 1.69 (s, 6H), 1.67 (d, *J* = 13.7 Hz, 6H). $^{31}$P NMR (162 MHz, DMSO-$d_6$) δ 33.51 (s, 1P).

**Example 159: Preparation of compound 159**

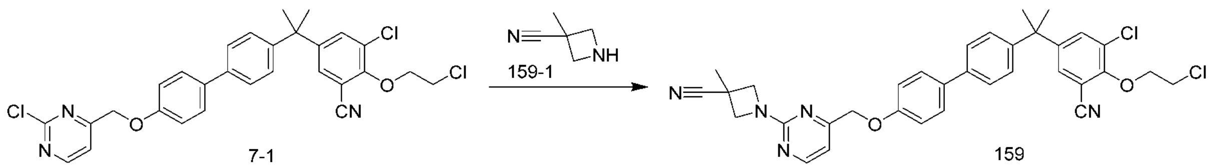


7-1 159

**Preparation of compound 159**

Compound **7-1**(65 mg, 117 μmol), 3-methylazetidine-3-carbonitrile (11 mg, 117 μmol), and TEA (24 mg, 236 μmol) were added to acetonitrile (2 mL). The reaction system was stirred at 70°C for 2 h. After the reaction was completed, the reaction mixture was cooled to room temperature. Saturated brine (5 mL) was added to the reaction mixture, and the mixture was extracted three times with ethyl acetate (8 mL). The organic phases were combined, dried, and subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 30% acetonitrile gradient elution over 12 min; flow rate: 30 mL/min) to obtain 18 mg of the title compound **159.** LC-MS (ESI): m/z 613.0 $[M+2H]^+$. $^1$H NMR (400 MHz, DMSO-$d_6$) δ 8.41 (d, *J* = 5.0 Hz, 1H), 7.70 (d, *J* = 2.3 Hz, 1H), 7.67 - 7.47 (m, 5H), 7.30 (d, *J* = 8.0 Hz, 2H), 7.08 (d, *J* = 8.2 Hz, 2H), 6.87 (d, *J* = 5.1 Hz, 1H), 5.08 (s, 2H), 4.42 (t, *J* = 5.1 Hz, 2H), 4.34 *(d, J*= 8.7 Hz, 2H), 4.03 *(d, J*= 8.8 Hz, 2H), 3.96 *(t, J*= 5.1 Hz, 2H), 1.68 (s, 6H), 1.66 (s, 3H).

**Example 160: Preparation of compound 160**

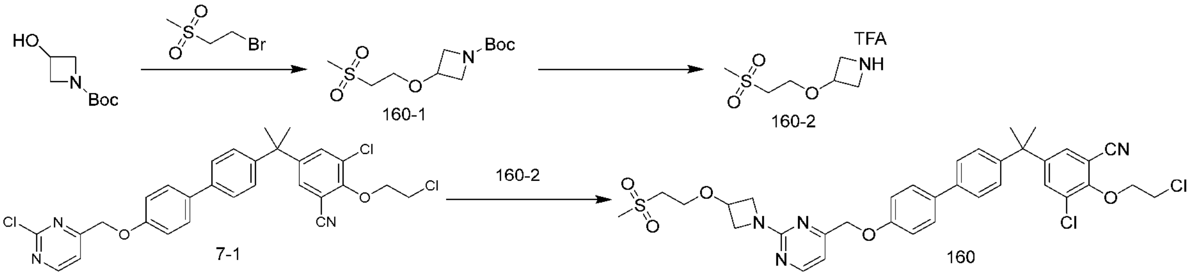


160-1 160-2 7-1 160

### Preparation of compound 160-1

*tert*-Butyl 3-hydroxyazetidine-1-carboxylate (500 mg, 2.89 mmol) was added to THF (6 mL). The system was purged three times with nitrogen, and sodium hydride (231 mg, 5.77 mmol, 60% purity) was added in an ice bath. After 30 min, 2-bromoethyl methyl sulfone (702 mg, 3.75 mmol) was added, and the mixture was warmed to room temperature and stirred for 12 h. After the reaction was completed, saturated brine (15 mL) was added dropwise to the reaction mixture, and the mixture was extracted three times with ethyl acetate (10 mL). The organic phases were combined, dried, subjected to suction filtration, and the filtrate was concentrated to obtain a crude product. The crude product was purified by normal-phase silica gel column chromatography (EtOAc/PE = 0 to 35%) to obtain the title compound 160-1 (95 mg, yield: 11%). $^1H$ NMR (400 MHz, Chloroform-d) δ 4.34 - 4.22 (m, 1H), 4.12 - 4.06 (m, 2H), 3.87 - 3.77 (m, 4H), 3.28 - 3.20 (m, 2H), 3.01 (s, 3H), 1.43 (s, 9H).

### Preparation of compound 160-2

Compound **160-1** (90 mg, 322.17 μmol) was added to dichloromethane (3 mL), and trifluoromethanesulfonic acid (0.3 mL) was added dropwise in an ice bath. The reaction system was stirred at room temperature for 2 h. After the reaction was completed, the reaction mixture was concentrated to obtain 53 mg of a crude product of the title compound **160-2** in 92% yield.

### Preparation of compound 160

Compound **7-1** (60 mg, 109 μmol), compound **160-2** (20 mg, 109 μmol), and TEA (22 mg, 218 μmol) were added to acetonitrile (2 mL). The reaction system was stirred at 90°C for 2 h. After the reaction was completed, the reaction mixture was cooled to room temperature. Saturated brine (5 mL) was added to the reaction mixture, and the mixture was extracted three times with ethyl acetate (8 mL). The organic phases were combined, dried, and subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 10% to 25% acetonitrile gradient elution over 12 min; flow rate: 30 mL/min) to obtain 12 mg of the title compound **160.** $^1H$ NMR (400 MHz, DMSO-$d_6$) δ 8.36 (d, *J* = 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.07 (d, *J* = 8.8 Hz, 2H), 6.78 (d, *J* = 5.0 Hz, 1H), 5.05 (s, 2H), 4.47 (m, 1H), 4.42 (t, *J* = 5.2Hz, 2H), 4.29 - 4.23 (m, 2H), 3.96 (t, *J* = 5.2Hz, 2H), 3.89 (dd, *J* = 10.0, 3.9 Hz, 2H), 3.78 (t, *J*= 5.7 Hz, 2H), 3.42 (t, *J* = 5.7 Hz, 2H), 3.00 (s, 3H), 1.68 (s, 6H).

### Example 161: Preparation of compound 161

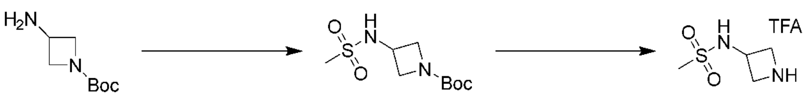

161-1 161-2 161-3

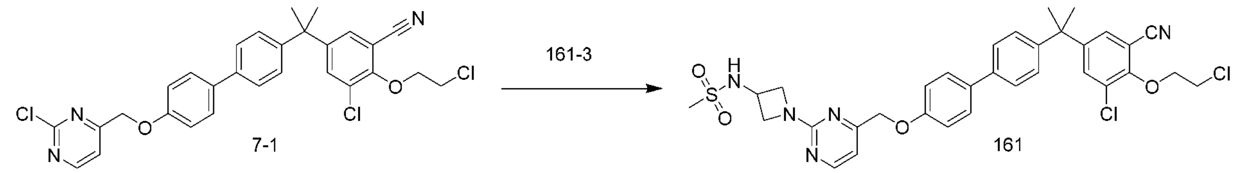


**Preparation of compound 161-2**

Compound **161-1** (100 mg, 0.58 mmol), methanesulfonyl chloride (66 mg, 0.58 mmol), and DIPEA (150 mg, 1.16 mmol) were dissolved in dichloromethane (5 mL). The reaction system was stirred at room temperature for 2 h. After the reaction was completed, water (5 mL) was added, and the mixture was extracted three times with dichloromethane (5 mL). The organic phases were combined, washed twice with saturated brine (5 mL), dried over anhydrous sodium sulfate, subjected to suction filtration, and the filtrate was concentrated to obtain 100 mg of a crude product of the title compound **161-2.** LC-MS (ESI): m/z 195.0 $[M-56+H]^+$.

**Preparation of compound 161-3**

Compound **161-2** (100 mg, 0.40 mmol) was dissolved in dichloromethane (3 mL). TFA (137 mg, 1.20 mmol) was added, and the mixture was stirred at room temperature for 3 h. After the reaction was completed, the reaction mixture was concentrated to obtain 60 mg of a crude product of the title compound **161-3.** LC-MS (ESI): m/z 151.2 $[M+H]^+$.

**Preparation of compound 161**

Compound **7-1** (100 mg, 0.18 mmol) and compound **161-3** (60 mg, 0.40 mmol) were dissolved in acetonitrile (3 mL). DIPEA (70 mg, 0.54 mmol) was added, and the reaction system was stirred at 80°C for 2 h. After the reaction was completed, water (3 mL) was added, and the mixture was extracted three times with dichloromethane (5 mL). The organic phases were combined, washed twice with saturated brine (3 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 11 mg of the title compound **161.** LC-MS (ESI): m/z 666.3 $[M+H]^+$. $^1H$ NMR (400 MHz, DMSO-$d_6$) δ 8.37 (d, *J* = 5.0 Hz, 1H), 7.86 (d, *J* = 8.0 Hz, 1H), 7.70 (d, *J* = 2.4 Hz, 1H), 7.64 (d, *J* = 2.4 Hz, 1H), 7.59 (d, *J* = 8.8 Hz, 2H), 7.56 (d, *J* = 8.4 Hz, 2H), 7.30 (d, *J* = 8.5 Hz, 2H), 7.07 (d, *J* = 8.8 Hz, 2H), 6.80 (d, *J* = 5.0 Hz, 1H), 5.06 (s, 2H), 4.42 (t, *J* = 5.2 Hz, 2H), 4.39 - 4.25 (m, 3H), 3.99 - 3.89 (m, 4H), 2.94 (s, 3H), 1.68 (s, 6H).

**Example 162: Preparation of compound 162**

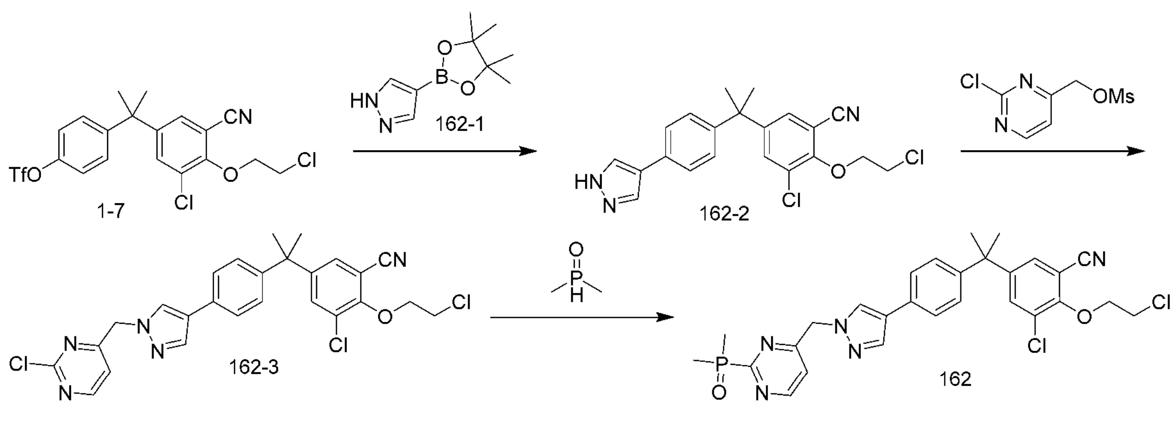


**Preparation of compound 162-2**

Compounds **1-7** (1.5 g, 3.11 mmol) and **162-1** (0.91 g, 4.67 mmol) were dissolved in 1,4-dioxane (25 mL). Pd(dppf)$Cl_2$ (0.23 g, 0.31 mmol) and an aqueous potassium carbonate solution (2 mol/L, 3 mL, 6.22 mmol) were then

added. The system was purged four times with argon, heated to 110°C, and stirred for 16 h. The reaction was monitored by LCMS until completion. The reaction system was cooled to room temperature and filtered through a pad of diatomite. The filter cake was rinsed with ethyl acetate (30 mL). The filtrates were combined and washed once with saturated brine (80 mL). The organic phase was dried over anhydrous sodium sulfate, subjected to suction filtration, and the filtrate was concentrated. The residue was purified by normal-phase silica gel column chromatography (MeOH/DCM = 0 to 8%) to obtain 1.3 g of the title compound **162-2** in 96% yield. LC-MS (ESI): m/z 400.0 $[M+H]^+$.

**Preparation of compound 162-3**

Compound **162-2** (150 mg, 0.37 mmol) and (2-chloropyrimidin-4-yl)methyl methanesulfonate (91 mg, 0.41 mmol) were dissolved in acetonitrile (3 mL). Cesium carbonate (181 mg, 0.55 mmol) was added at room temperature, and the mixture was heated to 80°C and stirred for 2 h. The reaction was monitored by LCMS until completion. The mixture was filtered, and the filter cake was rinsed with acetonitrile (10 mL). The filtrates were combined and concentrated, and the resulting residue was purified by preparative liquid chromatography to obtain 30 mg of the title compound **162-3** in 9% yield, which was used directly in the next step without purification. LC-MS (ESI): m/z 526.1 $[M+H]^+$.

**Preparation of compound 162**

Compound **162-2** (30 mg, 0.057 mmol), dimethylphosphine oxide (18 mg, 0.23 mmol), DIPEA (37 mg, 0.29 mmol), 1,3-bis(diphenylphosphino)propane (4 mg, 0.0086 mmol), and solvent DMF (1 mL) were added to a microwave tube. The mixture was stirred until a clear solution was obtained. Palladium acetate (3 mg, 0.011 mmol) was added at room temperature. The system was purged three times with argon, and the reaction was carried out under microwave irradiation at 120°C for 0.5 h. The reaction was monitored by LCMS until completion. The mixture was cooled to room temperature, filtered, and the filtrate subjected to preparative purification (Waters 2767/Qda, column: Sunfire C18 19 * 250 mm, 10 μm; mobile phase A: 0.1% $FA/H_2O$, B: acetonitrile; flow rate: 20 mL/min; elution gradient: 57% to 57%; retention time: 7.2-8.2 min of 16 min) to obtain 11 mg of the title compound 162. LC-MS (ESI): m/z 568.3 $[M+H]^+$. $^1H$ NMR (400 MHz, DMSO-$d_6$) δ 8.91 (d, *J* = 5.2 Hz, 1H), 8.35 (s, 1H), 7.99 (s, 1H), 7.67 (d, *J* = 2.3 Hz, 1H), 7.60 (d, *J* = 2.3 Hz, 1H), 7.54 (d, *J* = 8.4 Hz, 2H), 7.25 (d, *J* = 8.4 Hz, 2H), 7.11 (dd, *J* = 5.2, 3.1 Hz, 1H), 5.59 (s, 2H), 4.42 (t, *J* = 5.2 Hz, 2H), 3.96 (t, *J* = 5.2 Hz, 2H), 1.74 *(d, J=* 13.7 Hz, 7H), 1.67 (s, 6H). $^{31}P$ NMR (162 MHz, DMSO-$d_6$) δ 34.08 (s, 1P).

**Example 163: Preparation of compound 163**

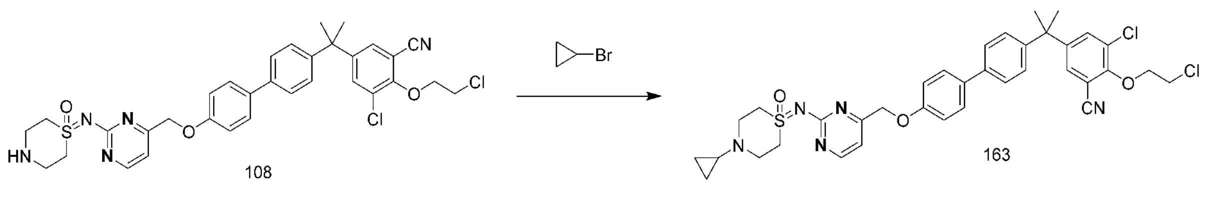

108

163

**Preparation of compound 163**

Compound **108** (500 mg, 0.77 mmol) and bromocyclopropane (280 mg, 2.31 mmol) were dissolved in acetonitrile (5 mL). Potassium carbonate (530 mg, 3.85 mmol) was added at room temperature, and the mixture was stirred at 90°C for 2 h. The reaction was monitored by LCMS until completion. The mixture was cooled to room temperature, filtered, and the filtrate was purified by preparative liquid chromatography (Waters 2767/Qda, column: Xbridge C18 19 * 250 mm, 10 μm; mobile phase A: 0.03% $NH_3H_2O/H_2O$, B: ACN; flow rate: 20 mL/min; elution gradient: 72% to 72%; retention time: 9.80-10.7 min of 16 min) to obtain 2.93 mg of the target compound **163.** LC-MS (ESI): m/z 690.4 $[M+H]^+$. $^1H$ NMR (400 MHz, DMSO-$d_6$) δ 8.44 (d, *J* = 5.0 Hz, 1H), 7.70 (d, *J* = 2.3 Hz, 1H), 7.63 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.07 (d, *J* = 8.8 Hz, 2H), 6.97 (d, *J* = 5.1 Hz, 1H), 5.84 - 5.72 (m, 1H), 5.24 - 5.12 (m, 2H), 5.11 (s, 2H), 4.42 (t, *J= 5.2* Hz, 2H), 3.96 (t, *J* = 5.2 Hz, 2H), 3.70 - 3.61 (m, 2H), 3.52 - 3.43 (m, 2H), 3.10 (d, *J* = 6.4 Hz, 2H), 2.96 - 2.88 (m, 2H), 2.86 - 2.77 (m, 2H), 1.68 (s, 6H).

**Example 164: Preparation of compound 164**

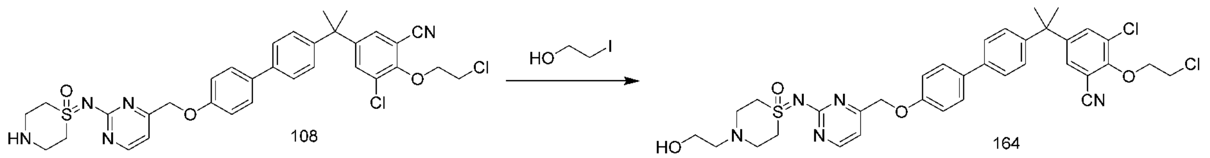

108

164

**Preparation of compound** 164

Compound 108 (500 mg, 0.77 mmol) and 2-iodoethanol (400 mg, 2.31 mmol) were dissolved in acetonitrile (5 mL). Potassium carbonate (530 mg, 3.85 mmol) was added at room temperature, and the mixture was stirred at 90°C for 2 h. The reaction was monitored by LCMS until completion. The mixture was cooled to room temperature, filtered, and the filtrate was purified by preparative liquid chromatography (Waters 2767/Qda, Column: Xbridge C18 19 * 250 mm, 10 μm; mobile phase A: 0.03% $NH_3H_2O/H_2O$, B: ACN; flow rate: 20 mL/min; gradient elution: 62% to 62%; retention time: 9.20-10.20 min of 17 min) to obtain 10.4 mg of the title compound **164.** LC-MS (ESI): m/z 694.4 $[M+H]^+$. $^1H$ NMR (400 MHz, DMSO-$d_6$) δ 8.44 (d, *J* = 5.0 Hz, 1H), 7.70 (d, *J* = 2.3 Hz, 1H), 7.64 (d, *J*= 2.3 Hz, 1H), 7.60 (d, *J* = 8.7 Hz, 2H), 7.56 (d, *J* = 8.3 Hz, 2H), 7.30 (d, *J* = 8.4 Hz, 2H), 7.07 (d, *J* = 8.7 Hz, 2H), 6.97 (d, *J* = 5.1 Hz, 1H), 5.11 (s, 2H), 4.42 (t, *J* = 5.2 Hz, 2H), 3.96 (t, *J*= 5.2 Hz, 2H), 3.79 - 3.55 (m, 3H), 3.54 - 3.42 (m, 4H), 3.08 - 2.96 (m, 2H), 2.97 - 2.84 (m, 2H), 2.57 (t, *J* = 5.8 Hz, 2H), 1.68 (s, 6H).

**Example 165: Preparation of compound 165**

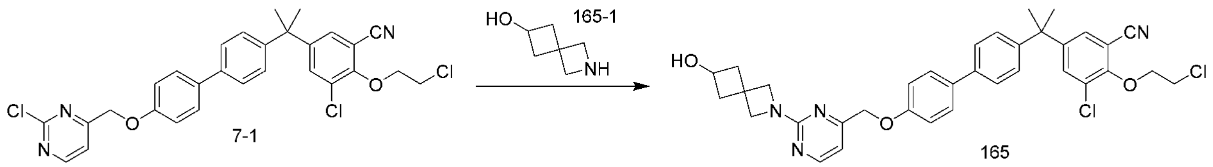

165-1

7-1

165

**Preparation of compound 165**

Compounds **7-1** (100 mg, 0.181 mmol) and **165-1** (41 mg, 0.361 mmol) were dissolved in ethanol (3 mL). Triethylamine (55 mg, 0.542 mmol) was added at room temperature, and the mixture was stirred at 70°C for 5 h. The reaction was monitored by LCMS until completion. The mixture was cooled to room temperature, filtered, and the filtrate was purified by preparative liquid chromatography (Waters 2767/Qda, column: Xbridge C18 19 * 250 mm, 10 μm; mobile phase A: 0.03% $NH_3H_2O/H_2O$, B: ACN; flow rate: 20 mL/min; elution gradient: 72% to 77%; retention time: 8.50-9.50 min of 16 min) to obtain 63 mg of the title compound **165.** LC-MS (ESI): m/z $[M+H]^+$: 629.4. $^1H$ NMR (400 MHz, DMSO-$d_6$) δ 8.32 (d, *J*= 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.06 (d, *J* = 8.8 Hz, 2H), 6.73 (d, *J* = 5.0 Hz, 1H), 5.06 - 5.00 (m, 3H), 4.42 (t, *J* = 5.2 Hz, 2H), 4.03 - 3.94 (m, 7H), 2.48 - 2.42 (m, 2H), 2.05 - 1.98 (m, 2H), 1.68 (s, 6H).

**Example 166: Preparation of compound 166**

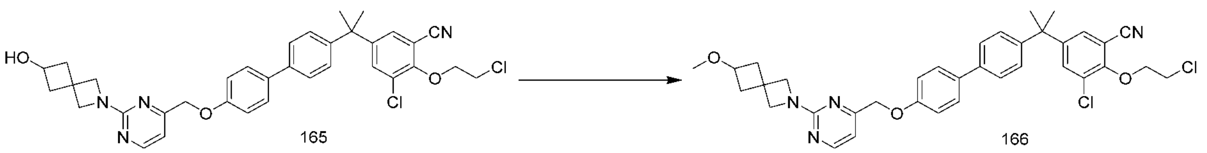

165

166

**Preparation of compound 166**

Compound **165** (40 mg, 0.064 mmol) was dissolved in anhydrous THF (3 mL). Sodium hydride (8 mg, 0.190 mmol, 60% purity) was added at room temperature, and the mixture was stirred for 10 min. Iodomethane (14 mg, 0.095 mmol) was added dropwise, and the reaction was carried out for 2 h. The reaction was monitored by LCMS until completion. The reaction was quenched with saturated ammonium chloride (5 mL) and extracted twice with ethyl acetate

(20 mL). The organic phase was subjected to rotary evaporation until dryness under reduced pressure. The resulting residue was purified by preparative liquid chromatography (Waters 2767/Qda, Column: Xbridge C18 19 * 250 mm, 10 μm; mobile phase A: 0.1% FA/$H_2O$, B: ACN; flow rate: 20 mL/min; elution gradient: 86% to 87%; retention time: 9.0-10.5 min of 17 min) to obtain 4.3 mg of the title compound **166.** LC-MS (ESI): m/z 643.4 $[M+H]^+$. $^1H$ NMR (400 MHz, DMSO-$d_6$) δ 8.33 (d, *J* = 5.0 Hz, 1H), 7.69 (d, *J* = 2.3 Hz, 1H), 7.63 (d, *J* = 2.3 Hz, 1H), 7.59 (d, *J* = 8.7 Hz, 2H), 7.55 (d, *J* = 8.4 Hz, 2H), 7.30 (d, *J* = 8.4 Hz, 2H), 7.06 (d, *J* = 8.8 Hz, 2H), 6.74 (d, *J* = 5.0 Hz, 1H), 5.03 (s, 2H), 4.42 (t, *J* = 5.2 Hz, 2H), 4.03 (s, 2H), 4.00 - 3.93 (m, 4H), 3.81 - 3.75 (m, 1H), 3.11 (s, 3H), 2.49 - 2.41 (m, 2H), 2.09 - 2.00 (m, 2H), 1.68 (s, 6H).

**Example 167: Preparation of compound 167**

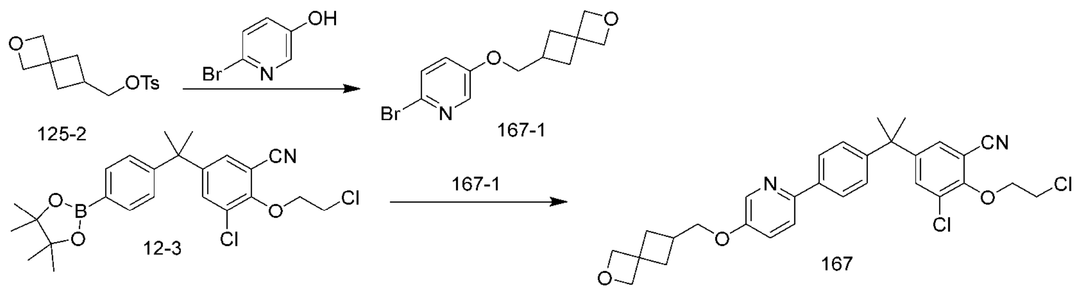

125-2
167-1
12-3
167

**Preparation of compound 167-1**

Compound **125-2** (30 mg, 0.11 mmol) was dissolved in *N,N*-dimethylformamide (0.5 mL), followed by the sequential addition of 2-bromo-5-hydroxypyridine (22 mg, 0.13 mmol), potassium carbonate (69 mg, 0.21 mmol), and tetrabutylammonium chloride (1 mg, 0.005 mmol). The reaction system was stirred at 70°C for 16 h. After the reaction was completed, water (10 mL) was added, and the mixture was extracted three times with ethyl acetate (5 mL). The organic phases were combined, washed twice with saturated brine (5 mL), dried over anhydrous sodium sulfate, subjected to suction filtration, and the filtrate was concentrated to obtain a crude product. The crude product was purified by silica gel column chromatography (EA/PE = 0 to 20%) to obtain 25 mg of the title compound **167-1.** LC-MS (ESI): m/z 284.4 $[M+H]^+$.

**Preparation of compound 167**

Compound **167-1** (20 mg, 0.04 mmol) was dissolved in dioxane (0.5 mL) and water (0.1 mL), followed by the sequential addition of compound **12-3** (27 mg, 0.06 mmol), Pd(dppf)$Cl_2$ (4 mg, 0.006 mmol), and potassium carbonate (16 mg, 0.09 mmol). The reaction system was stirred at 100°C for 16 h. After the reaction was completed, insoluble solids were removed by filtration. Water (5 mL) was added to the filtrate, and the mixture was extracted three times with ethyl acetate (5 mL). The organic phases were combined, washed twice with saturated brine (5 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2.1 mg of the title compound **167.** LC-MS (ESI): m/z 537.4 $[M+H]^+$. $^1H$ NMR (400 MHz, $CDCl_3$) δ 8.39 (s, 1H), 7.92 (d, *J* = 7.9 Hz, 2H), 7.71 (d, *J* = 8.6 Hz, 1H), 7.48 (d, *J* = 2.3 Hz, 1H), 7.40 (d, *J* = 2.4 Hz, 1H), 7.28 (m, 3H), 4.76 (s, 2H), 4.71 (s, 2H), 4.45 (t, *J* = 6.2 Hz, 2H), 4.00 (d, *J* = 5.8 Hz, 2H), 3.90 (t, *J* = 6.2 Hz, 2H), 2.65 (m, 1H), 2.49 (dd, *J* = 12.6, 8.4 Hz, 2H), 2.18 (dd, *J* = 12.8, 7.0 Hz, 2H), 1.72 (s, 6H).

**Example 168: Preparation of compound 168**

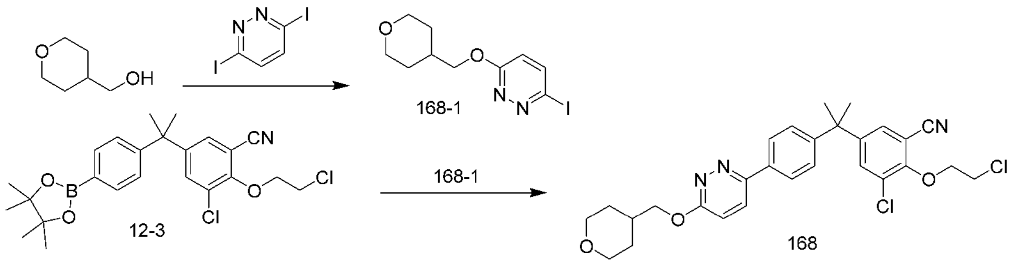

168-1
12-3
168

### Preparation of compound 168-1

Compound (tetrahydro-2H-pyran-4-yl)methanol (50 mg, 0.43 mmol) was dissolved in N,N-dimethylformamide (0.5 mL). The system was purged three times with nitrogen, cooled to 0°C, and sodium hydride (19 mg, 0.52 mmol) was added. The reaction system was stirred for 0.5 h, then 3,6-diiodopyridazine (143 mg, 0.43 mmol) was added, and the reaction system was stirred at 25°C for 16 h. After the reaction was completed, water (5 mL) was added, and the mixture was extracted three times with ethyl acetate (5 mL). The organic phases were combined, washed twice with saturated brine (5 mL), dried over anhydrous sodium sulfate, subjected to suction filtration, and the filtrate was concentrated to obtain a crude product. The crude product was purified by silica gel column chromatography (EA/PE = 0 to 30%) to obtain 60 mg of the title compound **168-1.** LC-MS (ESI): m/z 321.0 $[M+H]^+$.

### Preparation of compound 168

Compound **168-1** (50 mg, 0.16 mmol) was dissolved in dioxane (1 mL) and water (0.2 mL), followed by the sequential addition of compound **12-3** (72 mg, 0.16 mmol), Pd(dppf)$Cl_2$ (11 mg, 0.01 mmol), and potassium carbonate (43 mg, 0.31 mmol). The reaction system was stirred at 90°C for 16 h. After the reaction was completed, insoluble solids were removed by filtration. Water (5 mL) was added to the filtrate, and the mixture was extracted three times with ethyl acetate (5 mL). The organic phases were combined, washed twice with saturated brine (5 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 22 mg of the title compound **168.** LC-MS (ESI): m/z 526.2 $[M+H]^+$. $^1$H NMR (400 MHz, DMSO-$d_6$) δ 8.14 (d, *J* = 9.3 Hz, 1H), 8.00 (d, *J* = 8.6 Hz, 2H), 7.71 (d, *J* = 2.4 Hz, 1H), 7.65 (d, *J* = 2.3 Hz, 1H), 7.40 (d, *J* = 8.5 Hz, 2H), 7.30 (d, *J* = 9.3 Hz, 1H), 4.42 (t, *J* = 5.2 Hz, 2H), 4.34 (d, *J* = 6.5 Hz, 2H), 3.96 (t, *J* = 5.2 Hz, 2H), 3.93 - 3.83 (m, 2H), 3.33 - 3.30 (m, 2H), 2.17 - 2.08 (m, 1H), 1.71 (s, 6H), 1.70 - 1.64 (m, 2H), 1.44 - 1.29 (m, 2H).

### Example 169: Preparation of compound 169

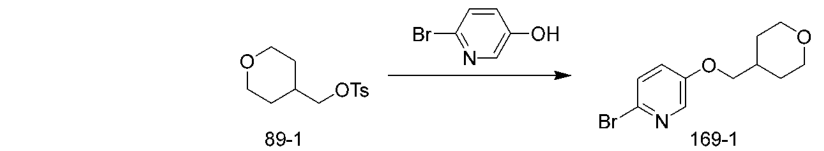

89-1 169-1

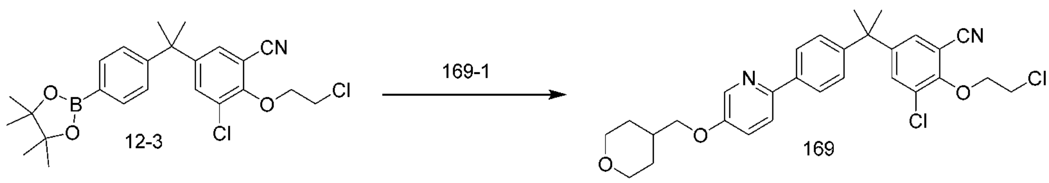

12-3
169

### Preparation of compound 169-1

Compound **89-1** (50 mg, 0.18 mmol) was dissolved in N,N-dimethylformamide (0.5 mL), followed by the sequential addition of potassium carbonate (51 mg, 0.37 mmol) and 2-bromo-5-hydroxypyridine (32 mg, 0.18 mmol). The reaction system was stirred at 80°C for 16 h. After the reaction was completed, water (5 mL) was added, and the

mixture was extracted three times with ethyl acetate (5 mL). The organic phases were combined, washed twice with saturated brine (5 mL), dried over anhydrous sodium sulfate, subjected to suction filtration, and the filtrate was concentrated to obtain a crude product. The crude product was purified by silica gel column chromatography (EA/PE = 0 to 30%) to obtain 42 mg of the title compound **169-1.** LC-MS (ESI): m/z 272.0 $[M+H]^+$.

**Preparation of compound 169**

Compound **169-1** (24 mg, 0.08 mmol) was dissolved in dioxane (0.5 mL) and water (0.1 mL), followed by the addition of **12-3** (40 mg, 0.08 mmol), Pd(dppf)$Cl_2$ (6 mg, 0.008 mmol), and potassium carbonate (24 mg, 0.17 mmol). The reaction system was stirred at 100°C for 16 h. After the reaction was completed, insoluble solids were removed by filtration. Water (5 mL) was added to the filtrate, and the mixture was extracted three times with ethyl acetate (5 mL). The organic phases were combined, washed twice with saturated brine (5 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 3.6 mg of the title compound **169.** LC-MS (ESI): m/z 525.2 $[M+H]^+$. $^1H$ NMR (400 MHz, DMSO-$d_6$) δ 8.35 (d, *J* = 3.0 Hz, 1H), 7.94 (d, *J* = 8.5 Hz, 2H), 7.87 (d, *J* = 8.8 Hz, 1H), 7.70 (d, *J* = 2.3 Hz, 1H), 7.62 (d, *J* = 2.3 Hz, 1H), 7.46 (dd, *J* = 8.8, 3.0 Hz, 1H), 7.33 (d, *J* = 8.5 Hz, 2H), 4.42 (t, *J* = 5.2 Hz, 2H), 4.08 - 3.93 (m, 4H), 3.93 - 3.83 (m, 2H), 3.31 (s, 2H), 2.06 - 1.97 (m, 1H), 1.88 - 1.55 (m, 8H), 1.41 - 1.28 (m, 2H).

**Example 170: Preparation of compound 170**

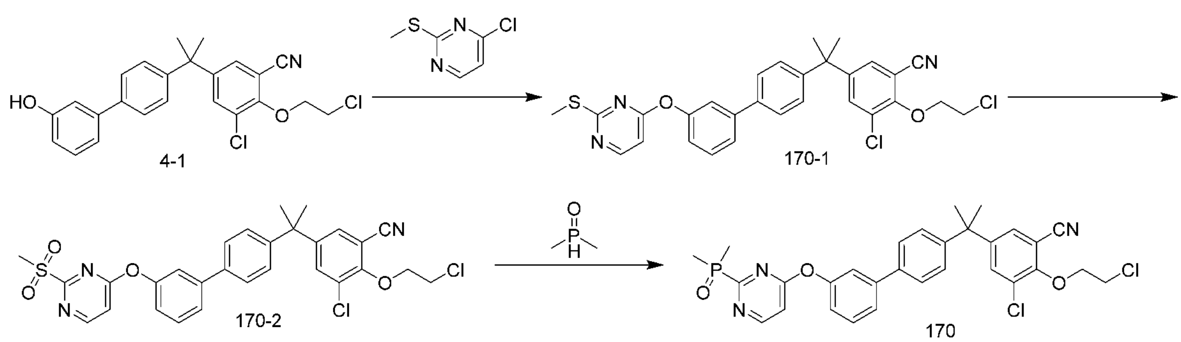

4-1 170-1 170-2 170

**Preparation of compound 170-1**

Compound **4-1** (427 mg, 1.0 mmol) was dissolved in DMF (10 mL). 2-(Methylthio)-4-chloropyrimidine (161 mg, 1.0 mmol) and cesium carbonate (653 mg, 2.0 mmol) were added. The reaction system was purged three times with nitrogen and stirred at 60°C for 2 h. After the reaction was completed, water (50 mL) was added, and the mixture was extracted three times with ethyl acetate (50 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 50%) to obtain 450 mg of the title compound **170-1.** LC-MS (ESI): m/z 550.1$[M+H]^+$.

**Preparation of compound 170-2**

Compound **170-1** (500 mg, 0.91 mmol) was dissolved in dichloromethane (5 mL). *m*-Chloroperoxybenzoic acid (392 mg, 2.27 mmol) was added, and the reaction system was stirred at 20°C for 2 h. After the reaction was completed, insoluble solids were removed by filtration. Water (20 mL) was added to the filtrate, and the mixture was extracted three times with dichloromethane (30 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 50%) to obtain 450 mg of the title compound **170-2.**

**Preparation of compound 170**

Compound **170-2** (59 mg, 0.1 mmol) was dissolved in acetonitrile (2 mL). Dimethylphosphine oxide (16 mg, 0.2 mmol) and cesium carbonate (65 mg, 0.2 mmol) were added. The reaction system was purged three times with nitrogen and stirred at 100°C for 2 h. After the reaction was completed, insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 16 mg of the title compound **170.** LC-MS (ESI): m/z 580.2 $[M+H]^+$. $^1H$ NMR (400 MHz, DMSO-$d_6$) δ 8.78 (d, *J* = 5.8 Hz, 1H), 7.71 - 7.42 (m, 7H), 7.34 (d, *J*= 8.1 Hz, 2H), 7.25 - 7.13 (m, 2H), 4.42 (t, *J* = 5.5 Hz, 2H), 3.89 (t, *J*= 5.5 Hz, 2H), 1.75 (d, *J* = 13.8 Hz, 6H), 1.72 (s, 6H).

**Example 171: Preparation of compound 171**

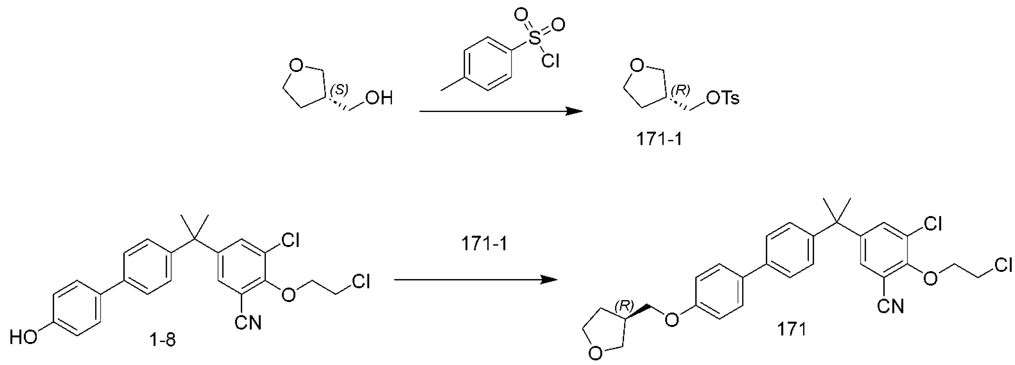


**Preparation of compound 171-1**

Compound (*S*)-(tetrahydrofuran-3-yl)methanol (100 mg, 0.98 mmol) was dissolved in dichloromethane (2 mL), followed by the addition of *p*-toluenesulfonyl chloride (205 mg, 1.08 mmol) and 4-dimethylaminopyridine (12 mg, 0.10 mmol). The mixture was cooled to 0°C, and triethylamine (199 mg, 1.96 mmol) was added dropwise. The reaction system was stirred at 25°C for 12 h. After the reaction was completed, water (10 mL) was added, and the mixture was extracted three times with dichloromethane (20 mL). The organic phases were combined, washed twice with saturated brine (20 mL), dried over anhydrous sodium sulfate, and the filtrate was concentrated to obtain a crude product. The crude product was purified by silica gel column chromatography (EA/PE = 0 to 30%) to obtain 122 mg of the title compound **171-1.** LC-MS (ESI): m/z 257.0 $[M+H]^+$.

**Preparation of compound 171**

Compound **1-8** (40 mg, 0.09 mmol) was dissolved in dimethyl sulfoxide (2.0 mL), followed by the addition of compound **171-1** (33 mg, 0.10 mmol) and cesium carbonate (61 mg, 0.19 mmol). The reaction system was stirred at 60°C for 12 h. The mixture was cooled to room temperature and filtered. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 16 mg of the title compound **171.** LC-MS (ESI): m/z 510.2. $[M+H]^+$. $^1H$ NMR (400 MHz, Chloroform-*d*) δ 7.54 - 7.47 (m, 5H), 7.38 (d, *J* = 2.3 Hz, 1H), 7.22 (d, *J* = 8.4 Hz, 2H), 6.96 (d, *J* = 8.8 Hz, 2H), 4.42 (t, *J* = 6.1 Hz, 2H), 4.00 - 3.86 (m, 6H), 3.84 - 3.76 (m, 1H), 3.73 (dd, *J* = 8.8, 5.3 Hz, 1H), 2.84 - 2.70 (m, 1H), 2.18 - 2.07 (m, 1H), 1.81 - 1.72 (m, 1H), 1.69 (s, 6H).

**Example 172: Preparation of compound 172**

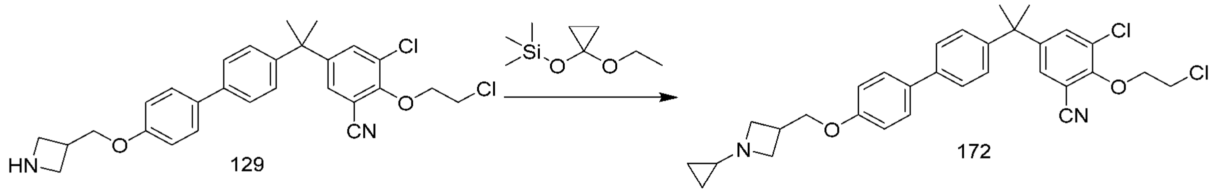

129

172

### Preparation of compound 172

Compound **129** (40 mg, 0.22 mmol) was dissolved in anhydrous methanol (1 mL). 1-Ethoxy-1-trimethylsiloxycyclopropane (79 mg, 0.46 mmol), sodium cyanoborohydride (22 mg, 0.34 mmol), and acetic acid (23 mg, 0.38 mmol) were added. The reaction system was stirred at 90°C for 12 h. After the reaction was completed, the reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 0.85 mg of the title compound **172.** LC-MS (ESI): m/z 535.1 $[M+H]^+$. $^1$H NMR (400 MHz, Chloroform-*d*) δ 7.56 (d, *J* = 8.6 Hz, 2H), 7.53 - 7.47 (m, 3H), 7.36 (d, *J* = 2.4 Hz, 1H), 7.23 (d, *J* = 8.3 Hz, 2H), 7.01 (d, *J* = 8.7 Hz, 2H), 4.43 (t, 2H), 4.23 - 4.05 (m, 4H), 3.87 (t, *J* = 6.2 Hz, 2H), 3.50 - 3.34 (m, 2H), 2.93 - 2.85 (m, 1H), 2.22 (t, *J* = 7.6 Hz, 1H), 1.70 (s, 6H), 0.96 - 0.85 (m, 4H).

### Example 173: Preparation of compound 173

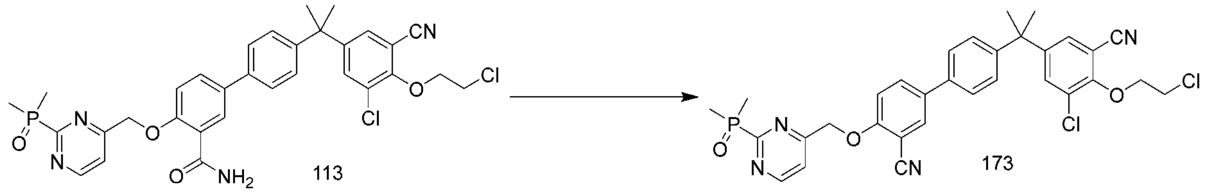

113

173

### Preparation of compound 173

Compound 113 (30 mg, 0.047 mmol) was dissolved in tetrahydrofuran (1 mL). Pyridine (11 mg, 0.14 mmol) was added at room temperature, followed by the addition of trifluoroacetic anhydride (30 mg, 0.14 mmol) in an ice bath. The mixture was stirred at room temperature for 2 h. The reaction was monitored by LCMS until completion. The reaction mixture was subjected to preparative purification (Waters 2767/Qda, Column: XBridge C18 19 * 250 mm, 10 μm; mobile phase A: 0.1% FA/$H_2O$, B: ACN; flow rate: 20 mL/min; elution gradient: 70% to 80%; retention time: 7.8-8.4 min of 16 min) to obtain 4.6 mg of the title compound **173.** LC-MS (ESI): m/z 619.3 $[M+H]^+$. $^1$H NMR (400 MHz, DMSO-$d_6$) δ 9.06 (d, *J* = 5.2 Hz, 1H), 8.12 (d, *J* = 2.3 Hz, 1H), 7.97 (dd, *J* = 8.9, 2.4 Hz, 1H), 7.74 (dd, *J* = 5.1, 3.2 Hz, 1H), 7.70 (d, *J* = 2.3 Hz, 1H), 7.65 (d, *J* = 8.5 Hz, 2H), 7.64 (s, 1H), 7.41 (d, *J* = 9.0 Hz, 1H), 7.34 (d, *J* = 8.5 Hz, 2H), 5.56 (s, 2H), 4.42 (t, *J* = 5.2 Hz, 2H), 3.96 (t, *J* = 5.2 Hz, 2H), 1.75 (d, *J* = 13.7 Hz, 6H), 1.69 (s, 6H). $^{31}$P NMR (162 MHz, DMSO-$d_6$) δ 34.07 (s,1P).

### Example 174: Preparation of compound 174

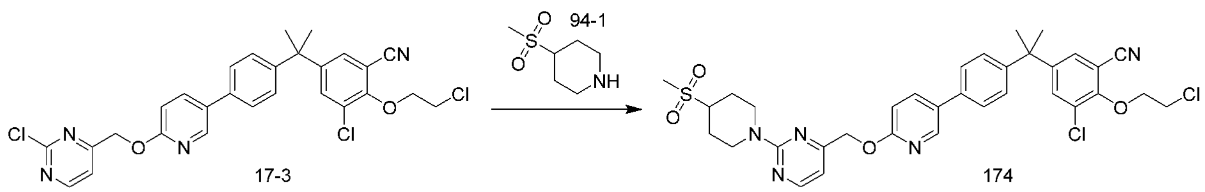

94-1

17-3

174

### Preparation of compound 174

Compound **17-3** (50 mg, 0.090 mmol) was dissolved in acetonitrile (2 mL), followed by the sequential addition of potassium carbonate (25 mg, 0.18 mmol) and compound **94-1** (15 mg, 0.091 mmol). The reaction system was stirred at

80°C for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15 mg of the title compound **174.** LC-MS (ESI): m/z 680.4 $[M+H]^+$. $^1H$ NMR (400 MHz, DMSO-$d_6$) δ 8.44 (d, *J* = 3.0 Hz, 1H), 8.41 (d, *J* = 4.9 Hz, 1H), 7.95 (d, *J* = 8.5 Hz, 2H), 7.89 (d, *J* = 8.8 Hz, 1H), 7.70 (d, *J*= 2.3 Hz, 1H), 7.62 (d, *J* = 2.3 Hz, 1H), 7.53 (dd, *J* = 8.8, 3.0 Hz, 1H), 7.33 (d, *J* = 8.6 Hz, 2H), 6.77 (d, *J* = 4.9 Hz, 1H), 5.17 (s, 2H), 4.80 (d, *J*= 13.2 Hz, 2H), 4.42 (t, *J* = 5.2 Hz, 2H), 3.96 (t, *J*= 5.2 Hz, 2H), 3.43 - 3.40 (m, 1H), 2.98 - 2.87 (m, 2H), 2.93 (s, 3H), 2.12 - 1.98 (m, 2H), 1.69 (s, 6H), 1.54 - 1.38 (m, 2H).

**Example 175: Preparation of compound 175**

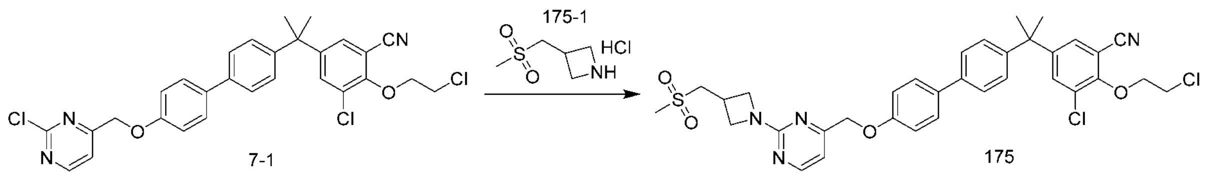


**Preparation of compound 175**

Compound **7-1** (60 mg, 0.108 mmol) was dissolved in ethanol (2 mL), followed by the sequential addition of DIPEA (14 mg, 0.108 mmol) and compound **175-1** (22 mg, 0.108 mmol). The reaction system was stirred at 80°C for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15 mg of the title compound **175.** LC-MS (ESI): m/z 665.5 $[M+H]^+$. $^1H$ NMR (400 MHz, DMSO-$d_6$) δ 8.36 (d, *J* = 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.07 (d, *J*= 8.8 Hz, 2H), 6.77 (d, *J*= 5.0 Hz, 1H), 5.05 (s, 2H), 4.42 (t, *J* = 5.2 Hz, 2H), 4.22 (t, *J* = 8.5 Hz, 2H), 3.96 (t, *J* = 5.2 Hz, 2H), 3.91 (dd, *J* = 8.9, 6.0 Hz, 2H), 3.56 (d, *J* = 7.5 Hz, 2H), 3.24 - 3.18 (m, 1H), 2.98 (s, 3H), 1.68 (s, 6H).

**Example 176: Preparation of compound 176**

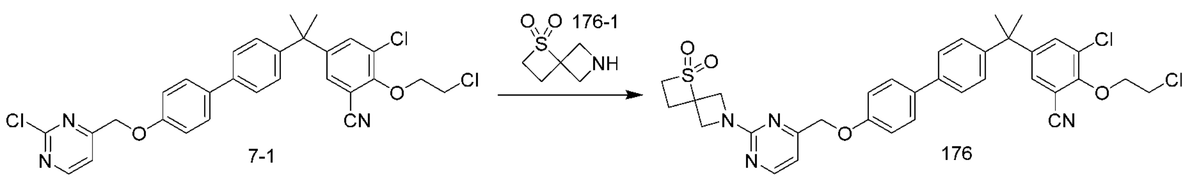


**Preparation of compound 176**

Compound **7-1** (50 mg, 0.090 mmol) was dissolved in ethanol (2 mL), followed by the sequential addition of DIPEA (23 mg, 0.18 mmol) and **176-1** (26 mg, 0.10 mmol). The mixture was stirred at 80°C for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **176.** LC-MS (ESI): m/z 663.2 $[M+H]^+$. $^1H$ NMR (400 MHz, DMSO-$d_6$) δ 8.43 (d, *J* = 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.08 (d, *J* = 8.7 Hz, 2H), 6.88 (d, *J* = 5.0 Hz, 1H), 5.09 (s, 2H), 4.44 (d, *J* = 9.4 Hz, 2H), 4.42 (t, *J* = 5.2 Hz, 2H), 4.33 (d, *J* = 9.4 Hz, 2H), 4.17 - 4.10 (m, 2H), 3.96 (t, *J* = 5.2 Hz, 2H), 2.47 - 2.41 (m, 2H), 1.68 (s, 6H).

**Example 177: Preparation of compound 177**

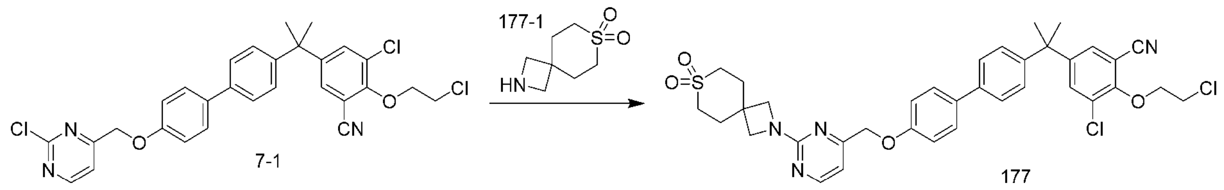


**Preparation of compound 177**

Compound **7-1** (50 mg, 0.09 mmol) was dissolved in ethanol (2 mL), followed by the sequential addition of DIPEA (23 mg, 0.180 mmol) and compound **177-1** (15 mg, 0.100 mmol). The reaction system was stirred at 80°C for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **177.** LC-MS (ESI): m/z 691.3 $[M+H]^+$. $^1H$ NMR (400 MHz, DMSO-$d_6$) δ 8.35 (d, *J* = 5.0 Hz, 1H), 7.70 (d, *J* = 2.3 Hz, 1H), 7.63 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.07 (d, *J* = 8.8 Hz, 2H), 6.76 (d, *J* = 5.0 Hz, 1H), 5.03 (s, 2H), 4.42 (t, *J* = 5.2 Hz, 2H), 3.96 (t, *J* = 5.2 Hz, 2H), 3.85 (s, 4H), 3.15 - 3.07 (m, 4H), 2.26 - 2.19 (m, 4H), 1.68 (s, 6H).

**Example 178: Preparation of compound 178**

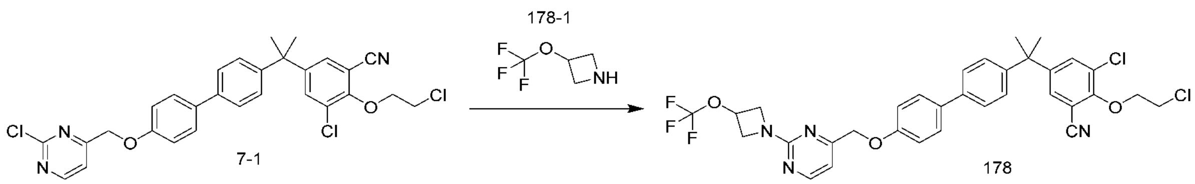


**Preparation of compound 178**

Compound **7-1** (50 mg, 0.09 mmol) was dissolved in acetonitrile (3 mL), and 3-(trifluoromethoxy)azetidine (25 mg, 0.14 mmol) and cesium carbonate (88 mg, 0.27 mmol) were added. The reaction system was stirred at 80°C for 3 h. After the reaction was completed, water (5 mL) was added, and the mixture was extracted three times with dichloromethane (5 mL). The organic phases were combined, washed twice with saturated brine (5 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2 mg of the title compound **178.** LC-MS (ESI): m/z 657.2 $[M+H]^+$. $^1H$ NMR (400 MHz, DMSO-$d_6$) δ 8.41 (d, *J* = 5.0 Hz, 1H), 7.70 (d, *J*= *2.3* Hz, 1H), 7.63 (d, *J* = 2.3 Hz, 1H), 7.57 (dd, *J* = 16.2, 8.2 Hz, 4H), 7.30 (d, *J* = 8.1 Hz, 2H), 7.07 (d, *J* = 8.7 Hz, 2H), 6.85 (d, *J* = 5.0 Hz, 1H), 5.41 - 5.22 (m, 1H), 5.07 (s, 2H), 4.55 - 4.31 (m, 4H), 4.18 - 4.06 (m, 2H), 3.96 (t, *J* = 5.1 Hz, 2H), 1.68 (s, 6H). $^{19}F$ NMR (376 MHz, DMSO-$d_6$) δ -58.31.

**Example 179: Preparation of compound 179**

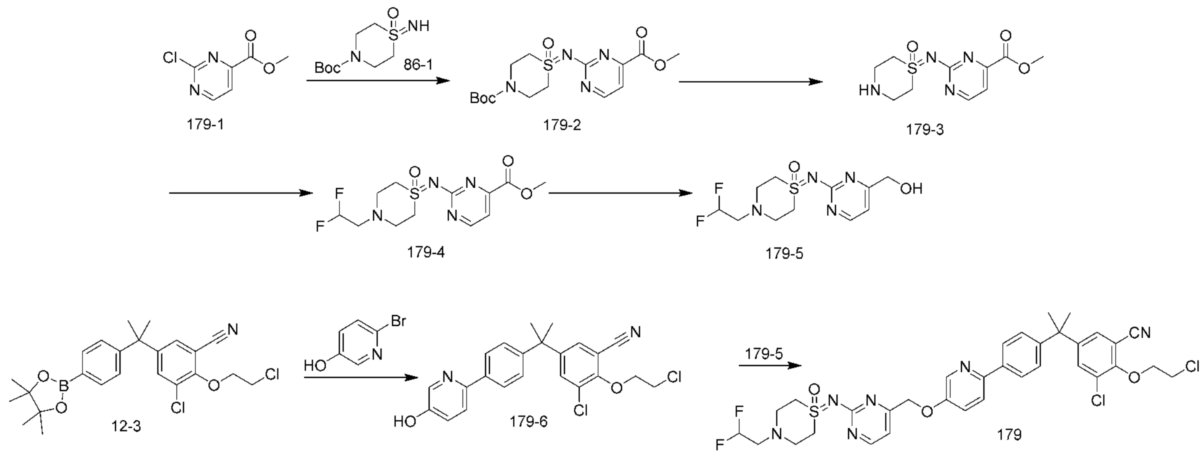


### Preparation of compound 179-2

Under a nitrogen atmosphere, compound **179-1** (500 mg, 2.90 mmol), compound **86-1** (815 mg, 3.48 mmol), DIEA (1.12 g, 8.69 mmol, 1.51 mL), Xantphos (335 mg, 579.48 μmol), $Pd_2(dba)_3$ (265 mg, 289.74 μmol), and 1,4-dioxane (5 mL) were added to a microwave tube, and the mixture was stirred under microwave irradiation at 100°C for 2 h. After the reaction was completed, the reaction mixture was subjected to rotary evaporation until dryness. The crude product was purified by silica gel column chromatography (MeOH/DCM = 0 to 2%) to obtain 600 mg of the title compound **179-2** in 55% yield. LC-MS (ESI): m/z 371.2 $[M+H]^+$.

### Preparation of compound 179-3

Compound **179-2** (0.8 g, 2.16 mmol) and DCM (10 mL) were added to a reaction flask, and TFA (2.46 g, 21.60 mmol) was added at 0°C. The temperature was slowly raised to room temperature, and the mixture was stirred for 2 h. The reaction mixture was subjected to rotary evaporation until dryness to obtain 580 mg of a crude product of the title compound **179-3.** The product was used directly in the next step without purification.

### Preparation of compound 179-4

Compound **179-3** (100 mg, 369.95 μmol), DIPEA (478 mg, 3.70 mmol, 645 μL), 2,2-difluoroethyl trifluoromethanesulfonate (172 mg, 739.90 μmol), and acetonitrile (2 mL) were added to a reaction flask, and the mixture was stirred at room temperature for 16 h. The reaction mixture was subjected to rotary evaporation until dryness and purified by preparative HPLC to obtain 20 mg of the title compound **179-4** in 16% yield. LC-MS (ESI): m/z 335.2 $[M+H]^+$.

### Preparation of compound 179-5

At 0°C, compound **179-4** (140 mg, 418.73 μmol), THF (2 mL), and MeOH (2 mL) were added to a reaction flask. $NaBH_4$ (32 mg, 837.47 μmol) was added in portions at 0°C, and the mixture was stirred for 1 h. The reaction was quenched with water (0.5 mL), subjected to rotary evaporation until dryness, and the crude product was purified by silica gel column chromatography (MeOH/DCM = 0 to 5%) to obtain 100 mg of the title compound **179-5** in 77% yield. LC-MS (ESI): m/z 307.2 $[M+H]^+$.

### Preparation of compound 179-6

Compound **12-3** (2 g, 4.35 mmol) was dissolved in 1,4-dioxane (10 mL). 2-Bromo-5-hydroxypyridine (908 mg, 5.22 mmol), potassium carbonate (1.8 g, 13.04 mmol), $Pd(dppf)Cl_2$ (636 mg, 869.19 μmol), and water (2 mL) were added. The reaction system was purged three times with nitrogen and stirred at 100°C for 5 h. After the reaction was completed, water (10 mL) was added, and the mixture was extracted with dichloromethane (20 mL × 3). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 50%) to obtain 300 mg of the title compound **179-6.** LC-MS (ESI): m/z 427.2 $[M+H]^+$.

**Preparation of compound 179**

Under a nitrogen atmosphere, compound **179-6** (33 mg, 76.36 μmol), DCM (2 mL), compound **179-5** (24 mg, 76.36 μmol), and TMAD (40 mg, 229.08 μmol) were added to a reaction flask. The system was cooled to 0°C, and then tributylphosphine (46 mg, 229.08 μmol) was added dropwise to the reaction system. The mixture was slowly warmed to room temperature and stirred for 2 h. After the reaction was completed, the reaction mixture was subjected to rotary evaporation until dryness under reduced pressure. The resulting crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 10% to 90% acetonitrile gradient elution over 20 min; flow rate: 30 mL/min) to obtain 10 mg of the title compound **179.** LC-MS (ESI): m/z 715.4 $[M+H]^+$. $^1H$ NMR (400 MHz, DMSO-$d_6$) δ 8.47 (d, *J* = 5.1 Hz, 1H), 8.44 (d, *J* = 3.0 Hz, 1H), 7.95 (d, *J* = 8.5 Hz, 2H), 7.89 (d, *J* = 8.8 Hz, 1H), 7.69 (d, *J* = 2.3 Hz, 1H), 7.62 (d, *J* = 2.4 Hz, 1H), 7.52 (dd, *J* = 8.8, 3.0 Hz, 1H), 7.33 (d, *J* = 8.6 Hz, 2H), 7.01 (d, *J* = 5.0 Hz, 1H), 6.11 (tt, *J* = 55.7, 4.2 Hz, 1H), 5.20 (s, 2H), 4.42 (t, *J* = 5.2 Hz, 2H), 3.96 (t, *J* = 5.2 Hz, 2H), 3.75 - 3.61 (m, 2H), 3.54 - 3.43 (m, 2H), 3.21 - 3.00 (m, 5H), 2.97 (d, *J* = 4.3 Hz, 1H), 1.69 (s, 6H). $^{19}F$ NMR (376 MHz, DMSO-$d_6$) δ -119.59 (s, 2F).

**Example 180: Preparation of compound 180**

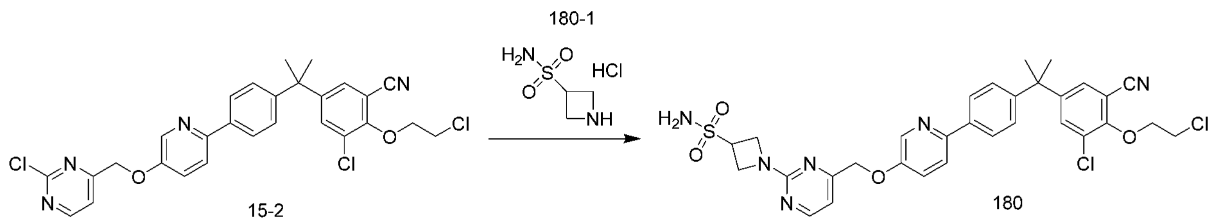


**Preparation of compound 180**

Compound **180-1** (30 mg, 207.65 μmol), compound **15-2** (80 mg, 138.44 μmol), DIPEA(179 mg, 1.38 mmol, 241.13 μL), and acetonitrile (1 mL) were added to a reaction flask, and the mixture was stirred under microwave irradiation at 80°C for 2 h. The mixture was subjected to rotary evaporation until dryness. The crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 10% to 80% acetonitrile gradient elution over 20 min; flow rate: 30 mL/min) to obtain 40 mg of title compound **180.** LC-MS (ESI): m/z 653.2 $[M+H]^+$. $^1H$ NMR (400 MHz, DMSO-$d_6$) δ 8.45 (d, *J* = 3.0 Hz, 1H), 8.42 (d, *J* = 5.0 Hz, 1H), 7.95 (d, *J*= 8.5 Hz, 2H), 7.89 (d, *J* = 8.8 Hz, 1H), 7.70 (d, *J* = 2.3 Hz, 1H), 7.62 (d, *J* = 2.3 Hz, 1H), 7.53 (dd, *J* = 8.8, 3.0 Hz, 1H), 7.33 (d, *J*= 8.5 Hz, 2H), 7.20 (s, 2H), 6.87 (d, *J*= 5.0 Hz, 1H), 5.17 (s, 2H), 4.42 (t, *J* = 5.2 Hz, 2H), 4.36 - 4.26 (m, 2H), 4.25 - 4.12 (m, 3H), 3.96 (t, *J* = 5.2 Hz, 2H), 1.69 (s, 6H).

**Example 181: Preparation of compound 181**

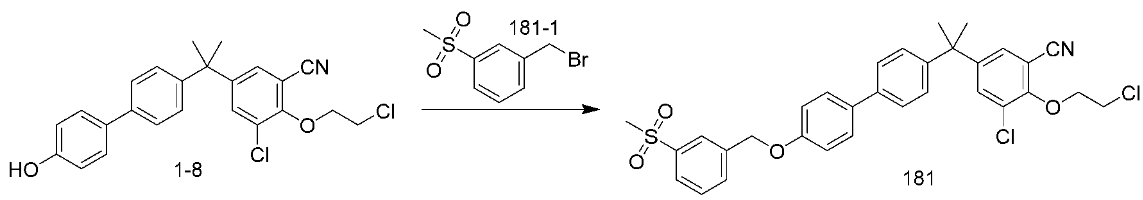


**Preparation of compound 181**

Compound 1-8 (50 mg, 117.28 μmol) and compound 181-1 (44 mg, 175.92 μmol) were dissolved in acetonitrile (2 mL), followed by the addition of $K_2CO_3$ (49 mg, 351.84 μmol). The reaction mixture was stirred at 85°C for 2 h. LCMS indicated the disappearance of the starting material. The mixture was cooled to room temperature, filtered, and the filtrate was subjected to rotary evaporation until dryness. The resulting crude product was purified by preparative liquid

chromatography (preparative method: mobile phase: A: 0.1% formic acid in water; B: acetonitrile; chromatographic column: Agilent 10 Prep-C18 250 × 21.2 mm; column temperature: 25°C; gradient: 20% to 70% acetonitrile gradient elution over 20 min; flow rate: 30 mL/min) to obtain 40 mg of title compound **181.** LC-MS (ESI): m/z 594.2 $[M+H]^+$. $^1H$ NMR (400 MHz, DMSO-$d_6$) δ 8.04 (s, 1H), 7.91 (d, *J* = 7.9 Hz, 1H), 7.83 (d, *J*= 7.8 Hz, 1H), 7.74 - 7.67 (m, 2H), 7.64 (d, *J* = 2.3 Hz, 1H), 7.61 (d, *J* = 8.7 Hz, 2H), 7.56 (d, *J* = 8.4 Hz, 2H), 7.30 (d, *J* = 8.4 Hz, 2H), 7.12 (d, *J* = 8.9 Hz, 2H), 5.28 (s, 2H), 4.42 (t, *J* = 5.2 Hz, 2H), 3.96 (t, *J* = 5.2 Hz, 2H), 3.24 (s, 3H), 1.68 (s, 6H).

**Example 182: Preparation of compound 182**

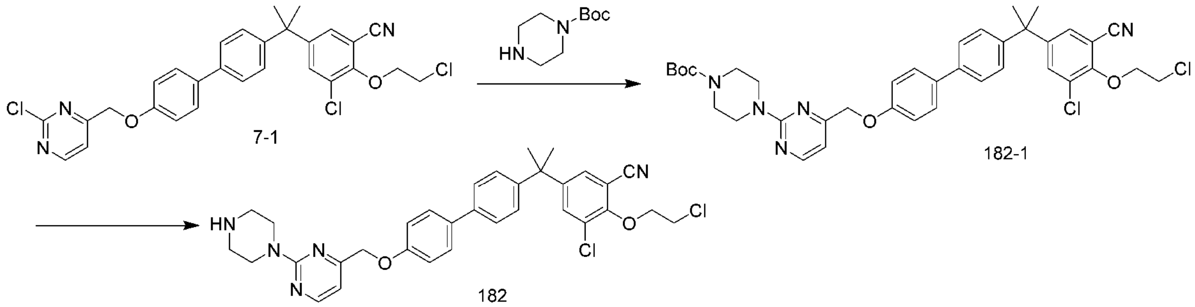

**Preparation of compound 182-1**

Compound **7-1** (20 mg, 0.03 mmol) was dissolved in 1,4-dioxane (2 mL), followed by the sequential addition of *tert*-butyl piperazine-1-carboxylate (8 mg, 0.04 mmol) and TEA (11 mg, 0.1 mmol). The mixture was subjected to microwave irradiation for 2 h. After the reaction was completed, water (20 mL) was added, and the mixture was extracted three times with DCM (50 mL). The organic phases were combined, washed once with saturated brine (50 mL), dried over anhydrous sodium sulfate, subjected to suction filtration, and the filtrate was concentrated to obtain 25 mg of a crude product of the title compound **182-1.** LC-MS (ESI): m/z 646.7 $[M-56+H]^+$.

**Preparation of compound 182**

Compound **182-1** (25 mg, 0.03 mmol) was dissolved in DCM/TFA (2 mL, V/V = 3/1), and the mixture was stirred at room temperature for 2 h. After the reaction was completed, a portion of DCM and TFA was removed by rotary evaporation at 45°C. Water (20 mL) was added, and the mixture was extracted three times with DCM (50 mL). The organic phases were combined, washed once with saturated brine (50 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile elution over 12 min; flow rate: 30 mL/min) to obtain 1.9 mg of the title compound **182.** LC-MS (ESI): m/z 602.3 $[M+H]^+$. $^1H$ NMR (400 MHz, DMSO-$d_6$) δ 8.43 (d, *J* = 5.0 Hz, 1H), 7.70 (d, *J* = 2.4 Hz, 1H), 7.64 (d, *J* = 2.3 Hz, 1H), 7.60 (d, *J* = 8.8 Hz, 2H), 7.55 (d, *J* = 8.4 Hz, 2H), 7.30 (d, *J*= 8.4 Hz, 2H), 7.07 (d, *J* = 8.8 Hz, 2H), 6.81 (d, *J* = 5.0 Hz, 1H), 5.08 (s, 2H), 4.42 (t, *J* = 5.2 Hz, 2H), 3.96 (t, *J* = 5.2 Hz, 2H), 3.88 (d, *J* = 5.5 Hz, 4H), 3.09 (s, 4H), 1.68 (s, 6H).

**Example 183: Preparation of compound 183**

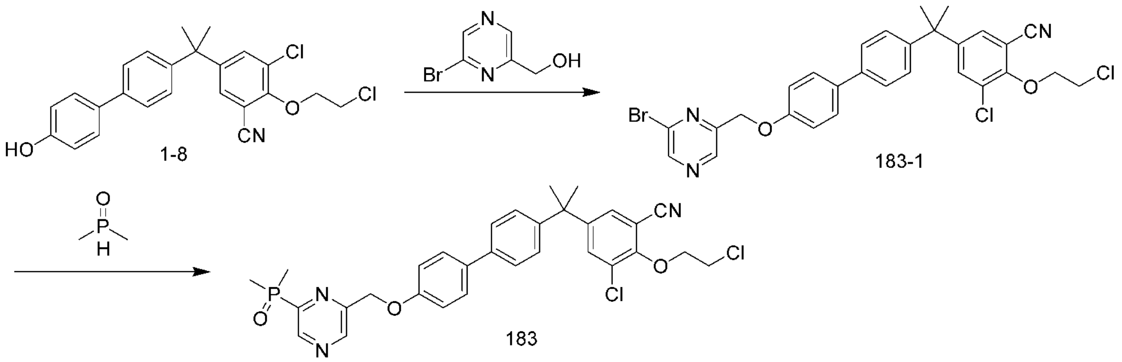

1-8 183-1 183

**Preparation of compound 183-1**

Compound **1-8** (100 mg, 0.23 mmol) was dissolved in DCE (10 mL), followed by the sequential addition of (6-bromopyrazin-2-yl)methanol (53 mg, 0.28 mmol) and TMAD (81 mg, 0.47 mmol). The system was purged three times with nitrogen. Tri-n-butylphosphine (100 mg, 0.49 mmol) was added at 0°C, and the reaction system was stirred at 80°C overnight. After the reaction was completed, water (30 mL) was added, and the mixture was extracted three times with ethyl acetate (100 mL). The organic phases were combined, washed once with saturated brine (100 mL), dried over anhydrous sodium sulfate, subjected to suction filtration, and the filtrate was concentrated to obtain a crude product. The crude product was purified by column chromatography (EA/PE = 0 to 25%) to obtain 69 mg of the title compound **183-1.** LC-MS (ESI): m/z 596.2 $[M+H]^+$.

**Preparation of compound 183**

Compound **183-1** (69 mg, 0.12 mmol), dimethylphosphine oxide (14 mg, 0.17 mmol), DIPEA (75 mg, 0.58 mmol), X-Phos (14 mg, 0.02 mmol), and $Pd_2(dba)_3$ (11 mg, 0.01 mmol) were dissolved in DMF (2 mL). The reaction system was purged three times with nitrogen and stirred under microwave irradiation at 120°C for 2 h. After the reaction was completed, the reaction mixture was cooled to room temperature and filtered through a pad of diatomite. The filter cake was rinsed with ethyl acetate (30 mL). Water (30 mL) was added to the filtrate, and the mixture was extracted three times with ethyl acetate (50 mL). The organic phases were combined, washed once with saturated brine (50 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 6 mg of the title compound **183.** LC-MS (ESI): m/z 594.0 $[M+H]^+$. $^1H$ NMR (400 MHz, DMSO-$d_6$) δ 9.04 (d, $J$ = 1.3 Hz, 1H), 8.98 (d, $J$ = 3.3 Hz, 1H), 7.70 (d, $J$ = 2.3 Hz, 1H), 7.64 (d, $J$ = 2.4 Hz, 1H), 7.62 (d, $J$ = 8.7 Hz, 2H), 7.57 (d, $J$ = 8.3 Hz, 2H), 7.30 (d, $J$ = 8.3 Hz, 2H), 7.16 (d, $J$ = 8.8 Hz, 2H), 5.38 (s, 2H), 4.42 (t, $J$ = 5.2 Hz, 2H), 3.96 (t, $J$ = 5.2 Hz, 2H), 1.73 (d, $J$ = 13.8 Hz, 6H), 1.68 (s, 6H).

**Example 184: Preparation of compound 184**

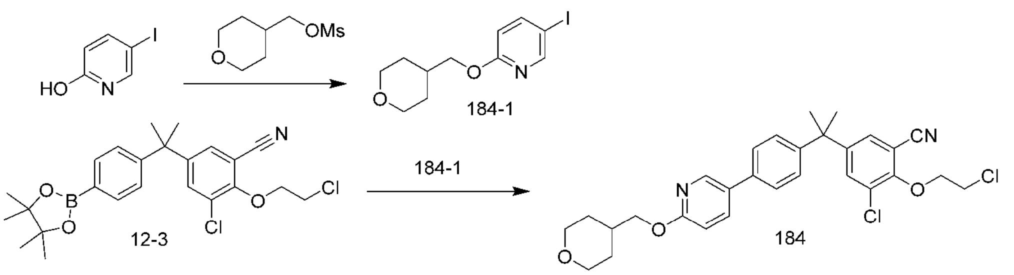


184-1 12-3 184

**Preparation of compound 184-1**

In a 10 mL microwave tube, (tetrahydro-2H-pyran-4-yl)methyl methanesulfonate (150 mg, 0.55 mmol) and 5-iodo-1H-pyridin-2-one (122 mg, 0.55 mmol) were dissolved in *N,N*-dimethylformamide (4 mL). The mixture was stirred at

80°C under a nitrogen atmosphere with microwave irradiation for 2 h. After the reaction was completed, water (20 mL) was added to the reaction mixture, and the mixture was extracted three times with ethyl acetate (10 mL). The organic phases were combined, washed with saturated brine (10 mL), dried over anhydrous sodium sulfate, subjected to suction filtration, and the filtrate was evaporated to dryness under reduced pressure. The resulting residue was purified by silica gel column chromatography (EA/PE = 0 to 50%) to obtain 80 mg of the title compound **184-1.** $^1$H NMR (400 MHz, DMSO-$d_6$) δ 8.36 (d, *J* = 2.3 Hz, 1H), 7.97 (dd, *J* = 8.7, 2.4 Hz, 1H), 6.72 (d, *J* = 8.7 Hz, 1H), 4.07 (d, *J* = 6.6 Hz, 2H), 3.90 - 3.82 (m, 2H), 3.33 - 3.25 (m, 2H), 2.04 - 1.93 (m, 1H), 1.62 (d, *J* = 10.7 Hz, 2H), 1.37 - 1.22 (m, 2H).

**Preparation of compound 184**

In a 25 mL single-necked flask, compound **184-1** (30 mg, 0.09 mmol) was dissolved in dioxane (0.5 mL) and water (0.1 mL), followed by the sequential addition of compound **12-3** (43 mg, 0.09 mmol), potassium carbonate (26 mg, 0.18 mmol), and Pd(dppf)$Cl_2$ (7 mg, 0.009 mmol). The reaction mixture was stirred at 95°C for 2 h. After the reaction was completed, the reaction mixture was cooled to room temperature, filtered, and subjected to rotary evaporation until dryness under reduced pressure. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 6.74 mg of the title compound **184.** LC-MS (ESI): m/z 525.2 $[M+H]^+$. $^1$H NMR (400 MHz, DMSO-$d_6$) δ 8.45 (dd, *J* = 2.6, 0.8 Hz, 1H), 8.00 (dd, *J* = 8.6, 2.6 Hz, 1H), 7.72 (d, *J* = 2.3 Hz, 1H), 7.66 (d, *J* = 2.4 Hz, 1H), 7.60 (d, *J* = 8.5 Hz, 2H), 7.34 (d, *J* = 8.5 Hz, 2H), 6.89 (dd, *J* = 8.5, 0.8 Hz, 1H), 4.43 (t, *J* = 5.2 Hz, 2H), 4.16 (d, *J* = 6.6 Hz, 2H), 3.97 (t, *J* = 5.2 Hz, 2H), 3.93 - 3.82 (m, 2H), 3.43 - 3.25 (m, 2H), 2.08 - 1.99 (m, 1H), 1.79 - 1.61 (m, 8H), 1.39 - 1.28 (m, 2H).

**Example 185: Preparation of compound 185**

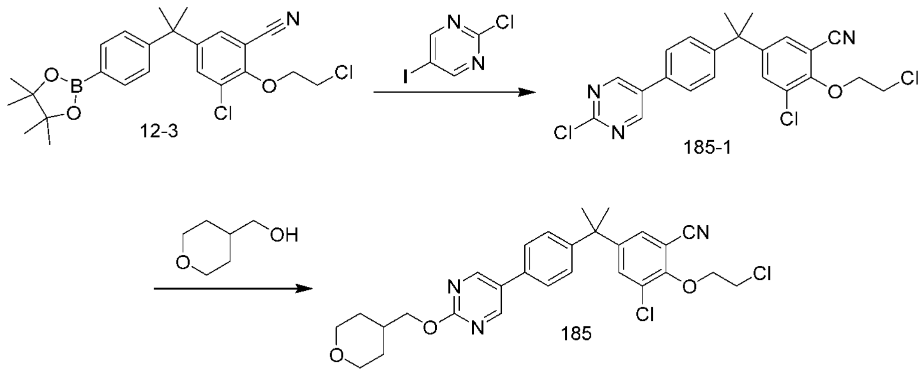

**Preparation of compound 185-1**

In a 25 mL single-necked flask, compound 2-chloro-5-iodopyrimidine (50 mg, 0.21 mmol) was dissolved in dioxane (0.5 mL) and water (0.1 mL), followed by the addition of compound **12-3** (80 mg, 0.13 mmol), potassium carbonate (48 mg, 0.35 mmol), and Pd(dppf)$Cl_2$ (13 mg, 0.02 mmol). The reaction mixture was stirred at 100°C for 16 h. After the reaction was completed, the reaction mixture was cooled to room temperature, filtered, and subjected to rotary evaporation until dryness under reduced pressure. The resulting crude product was purified by silica gel column chromatography (EA/PE = 0 to 30%) to obtain 60 mg of the title compound **185-1.** LC-MS (ESI): m/z 446.3 $[M+H]^+$.

**Preparation of compound 185**

In a 25 mL two-necked flask, compound **185-1** and (tetrahydro-2H-pyran-4-yl)methanol (50 mg, 0.11 mmol) were dissolved in tetrahydrofuran (1 mL). The system was purged three times with nitrogen, and sodium hydride (7 mg, 0.20 mmol) was added at 0°C. The reaction mixture was stirred at room temperature for 16 h. After the reaction was completed, methanol was added to the reaction mixture to quench the reaction, and the mixture was subjected to rotary evaporation until dryness under reduced pressure. The resulting crude product was purified by silica gel column chromatography (EA/PE = 0 to 30%) and subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5.5 mg of the title compound

**185.** LC-MS (ESI): m/z 526.2 [M+H]$^+$. $^1$H NMR (400 MHz, DMSO-$d_6$) δ 8.90 (s, 2H), 7.71 (d, *J* = 2.4 Hz, 1H), 7.69 - 7.62 (m, 3H), 7.37 (d, *J* = 8.4 Hz, 2H), 4.42 (t, *J* = 5.2 Hz, 2H), 4.21 (d, *J* = 6.6 Hz, 2H), 3.96 (t, *J* = 5.2 Hz, 2H), 3.92 - 3.83 (m, 2H), 3.40 - 3.26 (m, 2H), 2.11 - 2.01 (m, 1H), 1.75 - 1.61 (m, 8H), 1.42 - 1.26 (m, 2H).

**Example 186: Preparation of compound 186**

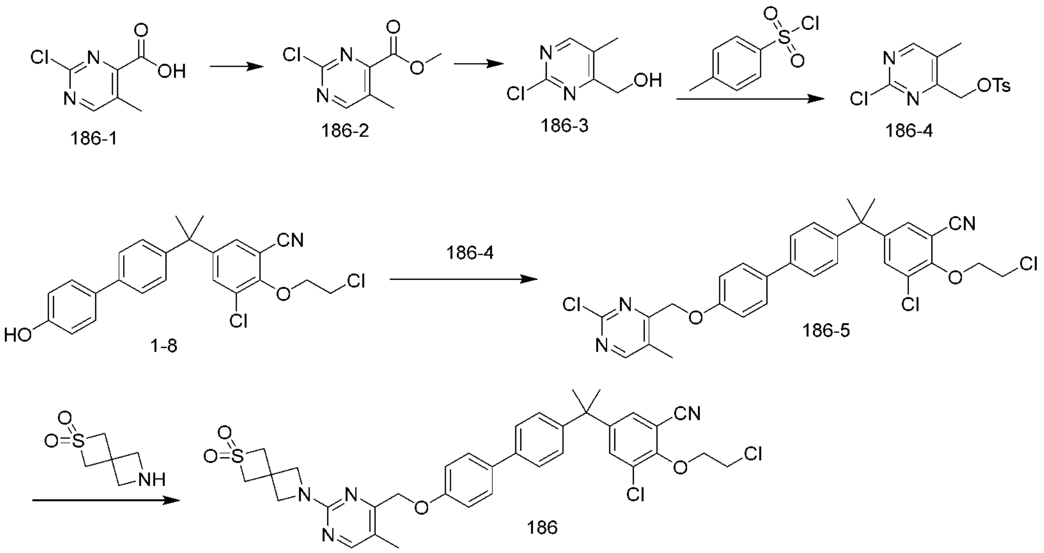

186-1 186-2 186-3 186-4
1-8 186-5
186

**Preparation of compound 186-2**

In a 25 mL two-necked flask, compound **186-1** (200 mg, 1.16 mmol) was dissolved in dichloromethane (5 mL), followed by the addition of *N,N*-dimethylformamide (0.2 mL). The reaction mixture was cooled to 0°C, and then oxalyl chloride (220 mg, 1.74 mmol) was slowly added dropwise. The reaction mixture was stirred at room temperature for 2 h, and methanol (5 mL) was added to quench the reaction. The reaction mixture was subjected to rotary evaporation until dryness under reduced pressure. The resulting crude product was purified by silica gel column chromatography (EA/PE = 0 to 30%) to obtain 190 mg of the title compound **186-2.** LC-MS (ESI): m/z 187.2 [M+H]$^+$.

**Preparation of compound 186-3**

In a 25 mL two-necked flask, compound **186-2** (190 mg, 1.02 mmol) was dissolved in dichloromethane (2 mL). The reaction mixture was cooled to 0°C, and then diisobutylaluminum hydride (2 mL, 2.04 mmol, 1 mol/L in tetrahydrofuran) was slowly added dropwise. The reaction mixture was stirred at room temperature for 16 h, and sodium sulfate decahydrate was added to quench the reaction. The reaction mixture was filtered, and the filtrate was subjected to rotary evaporation until dryness under reduced pressure to obtain 200 mg of a crude product of the title compound **186-3.** $^1$H NMR (400 MHz, DMSO-$d_6$) δ 8.52 (q, *J* = 0.7 Hz, 1H), 5.45 (t, *J* = 6.1 Hz, 1H), 4.55 (d, *J* = 6.0 Hz, 2H), 2.27 (s, 3H).

**Preparation of compound 186-4**

Compound **186-3** (180 mg, 1.14 mmol) and dichloromethane (2 mL) were added to a 25 mL single-necked flask, followed by the sequential addition of 4-(dimethylamino)pyridine (14 mg, 0.11 mmol) and triethylamine (230 mg, 2.27 mmol). The reaction mixture was cooled to 0°C, and then *p*-toluenesulfonyl chloride (325 mg, 1.70 mmol) was added slowly. The reaction mixture was stirred at room temperature for 16 h, and water (10 mL) was added to quench the reaction. The mixture was extracted three times with ethyl acetate (10 mL). The organic phases were combined, dried over anhydrous sodium sulfate, subjected to suction filtration, and the filtrate was subjected to rotary evaporation until dryness under reduced pressure. The residue was purified by silica gel column chromatography (EA/PEA = 0 to 30%) to obtain 110 mg of the title compound **186-4.**

**Preparation of compound 186-5**

In a 25 mL two-necked flask, compound **186-4** (100 mg, 0.32 mmol) was dissolved in N,N-dimethylformamide (2 mL), followed by the sequential addition of compound **1-8** (136 mg, 0.32 mmol) and cesium carbonate (208 mg, 0.64 mmol). The reaction mixture was stirred at 60°C for 16 h. Water (20 mL) was added to the reaction mixture, which was extracted three times with ethyl acetate (10 mL). The organic phases were combined, subjected to rotary evaporation until dryness under reduced pressure, and the resulting crude product was purified by silica gel column chromatography (EA/PE = 0 to 50%) to obtain 45 mg of the title compound 186-5. LC-MS (ESI): m/z 566.3 $[M+H]^+$.

**Preparation of compound 186**

In a 10 mL microwave tube, compound **186-5** (35 mg, 0.06 mmol) was dissolved in ethanol (2 mL), followed by the sequential addition of 2-thia-6-azaspiro[3.3]heptane 2,2-dioxide (18 mg, 0.12 mmol) and *N*,*N*-diisopropylethylamine (32 mg, 0.25 mmol). The reaction mixture was stirred under microwave irradiation at 100°C for 4 h. After the reaction was completed, the reaction mixture was cooled to room temperature and subjected to rotary evaporation until dryness under reduced pressure. The resulting residue was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 11.5 mg of the title compound **186.** LC-MS (ESI): m/z 677.3 $[M+H]^+$. $^1H$ NMR (400 MHz, Chloroform-*d*) δ 8.15 (s, 1H), 7.57 - 7.45 (m, 5H), 7.37 (d, *J* = 2.3 Hz, 1H), 7.23 (d, *J* = 8.2 Hz, 2H), 7.03 (d, *J* = 8.4 Hz, 2H), 5.09 (s, 2H), 4.53 - 4.40 (m, 6H), 4.38 (s, 4H), 3.88 (t, *J* = 6.2 Hz, 2H), 2.27 (s, 3H), 1.69 (s, 6H).

**Example 187: Preparation of compound 187**

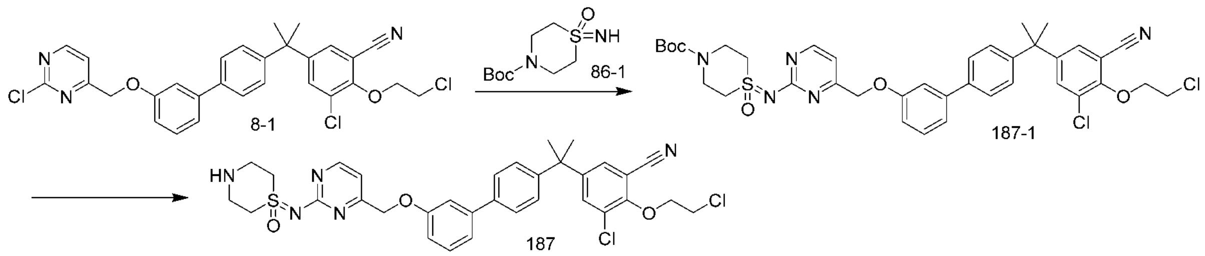

**Preparation of compound 187-1**

Compound **8-1** (200 mg, 361.74 μmol) and compound **86-1** (424 mg, 1.81 mmol) were dissolved in 1,4-dioxane (2 mL). $Pd_2(dba)_3$ (67 mg, 72.35 μmol), Xantphos (84 mg, 144.70 μmol), and DIPEA (94 mg, 723.49 μmol, 126.02 μL) were added, and the system was purged three times with nitrogen. The reaction mixture was heated under microwave irradiation at 120°C for 2 h. LCMS indicated the disappearance of the starting material and the formation of the product. The reaction mixture was cooled to room temperature and subjected to rotary evaporation until dryness. The resulting residue was purified by silica gel column chromatography (EA/PE = 0 to 80%) to obtain 200 mg of the title compound **187-1** in 74% yield. LC-MS (ESI): m/z 750.8 $[M+H]^+$.

**Preparation of compound 187**

Compound **187-1** (200 mg, 266.41 μmol) was dissolved in DCM (5 mL), and TFA (91 mg, 799.22 μmol) was added dropwise. The reaction mixture was stirred at room temperature for 1 h. LCMS indicated the disappearance of the starting material and the formation of the product. The reaction mixture was subjected to rotary evaporation until dryness, followed by preparative purification (preparative method: mobile phase: A: 0.1% formic acid in water; B: acetonitrile; chromatographic column: Welch Ultimate AQ-C18 250 × 21.2 mm; column temperature: 25°C; gradient: 35% to 55% acetonitrile gradient elution over 12 min; flow rate: 30 mL/min) to obtain 51 mg of the title compound **187.** LC-MS (ESI): m/z 650.4 $[M+H]^+$. $^1H$ NMR (400 MHz, DMSO-$d_6$) δ 8.45 (d, *J* = 5.1 Hz, 1H), 7.71 (s, 1H), 7.65 (s, 1H), 7.60 (d, *J* = 7.9 Hz, 2H), 7.43 - 7.31 (m, 3H), 7.29 - 7.23 (m, 2H), 7.05 - 6.94 (m, 2H), 5.14 (s, 2H), 4.43 (t, *J* = 5.1 Hz, 2H), 3.96 (t, *J* = 5.1 Hz, 2H), 3.74 - 3.63 (m, 2H), 3.46 - 3.36 (m, 2H), 3.28 - 3.16 (m, 2H), 3.14 - 2.95 (m, 2H), 1.69 (s, 6H).

**Example 188: Preparation of compound 188**

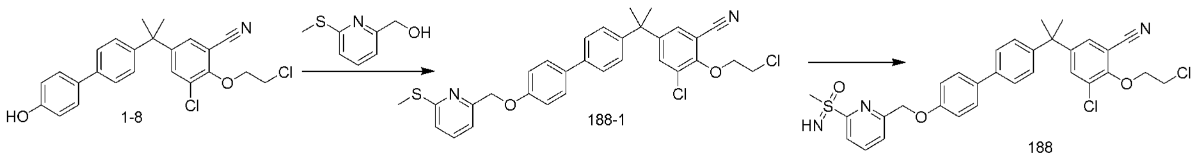

**Preparation of compound 188-1**

Compound **1-8** (100 mg, 234.56 μmol) and (6-(methylthio)pyridin-2-yl)methanol (40 mg, 258.01 μmol) were weighed and dissolved in toluene (5 mL). cyanomethylene(tri-n-butyl)phosphorane (113 mg, 469.12 μmol) was added. The system was purged three times with nitrogen. The reaction mixture was stirred at 100°C for 16 h. LCMS indicated the formation of the product. The reaction mixture was subjected to rotary evaporation until dryness and purified by silica gel column chromatography (EA/PE = 0 to 30%) to obtain 80 mg of the title compound **188-1** in 60.5% yield. LC-MS (ESI): m/z 563.6 $[M+H]^+$.

**Preparation of compound 188**

Compound **188-1** (80 mg, 141.96 μmol) was dissolved in methanol (5 mL). Iodobenzene diacetate (114 mg, 354.90 μmol) and ammonium carbonate (14 mg, 141.96 μmol) were added. The reaction mixture was stirred at room temperature for 1 h. LCMS indicated the disappearance of the starting material and the formation of the product. The reaction mixture was filtered, and the filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 50% to 70% acetonitrile gradient elution over 12 min; flow rate: 30 mL/min) to obtain 11.3 mg of the title compound 188. LC-MS (ESI): m/z 594.2 $[M+H]^+$. $^1H$ NMR (400 MHz, DMSO-$d_6$) δ 8.15 (t, *J* = 7.8 Hz, 1H), 8.03 (d, *J* = 8.2 Hz, 1H), 7.78 (d, *J* = 7.7 Hz, 1H), 7.70 (d, *J* = 2.4 Hz, 1H), 7.64 (d, *J* = 2.3 Hz, 1H), 7.61 (d, *J* = 8.8 Hz, 2H), 7.56 (d, *J* = 8.4 Hz, 2H), 7.30 (d, *J* = 8.5 Hz, 2H), 7.13 (d, *J* = 8.8 Hz, 2H), 5.32 (s, 2H), 4.47 (s, 1H), 4.42 (t, *J* = 5.2 Hz, 2H), 3.96 (t, *J* = 5.2 Hz, 2H), 3.17 (s, 3H), 1.68 (s, 6H).

**Example 189: Preparation of compound 189**

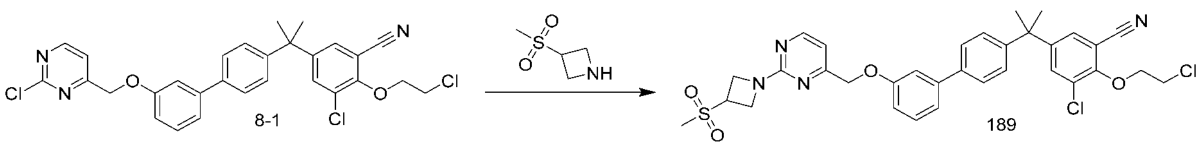

**Preparation of compound 189**

Compound **8-1** (100 mg, 180.87 μmol) and compound 3-(methylsulfonyl)azetidine (27 mg, 198.96 μmol) were dissolved in acetonitrile (2 mL), and TEA (37 mg, 361.74 μmol, 50.45 μL) was added. The reaction mixture was stirred under microwave irradiation at 80°C for 2 h. LCMS indicated the disappearance of the starting material and the formation of the product. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 17 mg of the title compound **189.** LC-MS (ESI): m/z 651.4 $[M+H]^+$. $^1H$ NMR (400 MHz, DMSO-$d_6$) δ 8.42 (d, *J* = 5.0 Hz, 1H), 7.71 (d, *J* = 2.3 Hz, 1H), 7.65 (d, *J* = 2.3 Hz, 1H), 7.61 (d, *J* = 8.5 Hz, 2H), 7.40 - 7.31 (m, 3H), 7.30 - 7.24 (m, 2H), 7.02 - 6.97 (m, 1H), 6.89 (d, *J* = 5.0 Hz, 1H), 5.12 (s, 2H), 4.46 - 4.38 (m, 3H), 4.34 (t, *J* = 8.7 Hz, 2H), 4.27 - 4.21 (m, 2H), 3.96 (t, *J* = 5.2 Hz, 2H), 3.06 (s, 3H), 1.69 (s, 6H).

**Example 190: Preparation of compound 190**

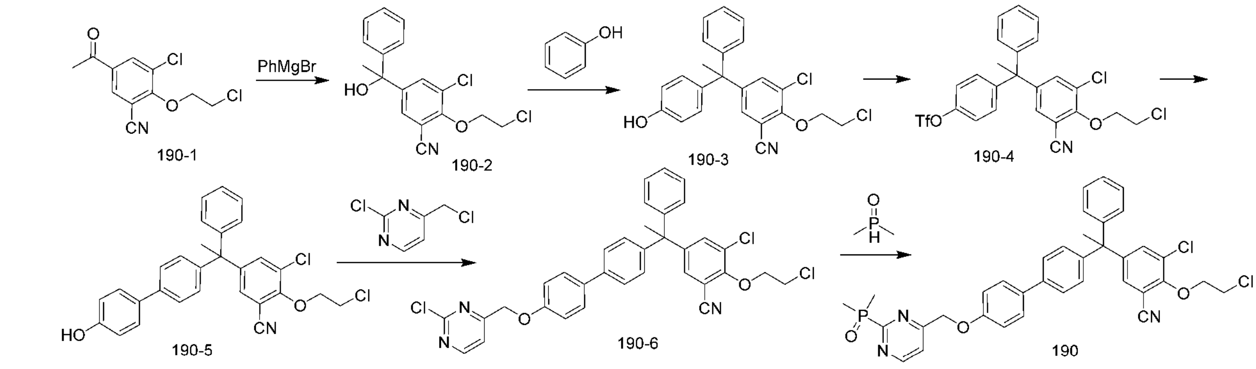


**Preparation of compound 190-2**

Compound **190-1** (800 mg, 3.1 mmol) was dissolved in tetrahydrofuran (50 mL), and phenylmagnesium bromide (4.7 mL, 1 mol/L) was added thereto at 0°C. After the addition was completed, the reaction system was stirred at 25°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 90%) to obtain 600 mg of the title compound **190-2.** LC-MS (ESI): m/z 336.0 $[M+H]^+$.

**Preparation of compound 190-3**

Compound **190-2** (600 mg, 1.79 mmol) and phenol (185 mg, 1.96 mmol) were dissolved in carbon tetrachloride (30 mL). Boron trifluoride diethyl etherate (1.05 g, 48%) was added to thereto at 0°C. After the addition was completed, the reaction system was stirred at 25°C for 16 h. After the reaction was completed, water (50 mL) was added, and the mixture was extracted three times with dichloromethane (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 90%) to obtain 300 mg of the title compound **190-3.** LC-MS (ESI): m/z 412.0 $[M+H]^+$.

**Preparation of compound 190-4**

Compound 190-3 (300 mg, 0.73 mmol) and *N*-phenylbis(trifluoromethanesulfonyl)imide (312 mg, 0.88 mmol) were dissolved in DCM (20 mL), and triethylamine (148 mg, 1.46 mmol) was added thereto. After the addition was completed, the reaction system was stirred at 25°C for 2 h. After the reaction was completed, water (50 mL) was added, and the mixture was extracted three times with dichloromethane (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 90%) to obtain 280 mg of the title compound **190-4.**

**Preparation of compound 190-5**

Compound **190-4** (280 mg, 0.51 mmol), 4-hydroxyphenylboronic acid (141 mg, 1.02 mmol), and potassium carbonate (211 mg, 1.53 mmol) were dissolved in 1,4-dioxane (20 mL) and water (5 mL), and Pd(dppf)$Cl_2$ (74 mg, 0.1 mmol) was added thereto. After the addition was completed, the reaction system was stirred at 80°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 70%) to obtain 150 mg of the title compound **190-5.** LC-MS (ESI): m/z 488.2 $[M+H]^+$.

**Preparation of compound 190-6**

Compound **190-5** (150 mg, 0.31 mmol) and cesium carbonate (200 mg, 0.62 mmol) were dissolved in acetonitrile (20 mL), and 2-chloro-4-(chloromethyl)pyrimidine (76 mg, 0.46 mmol) was added thereto. After the addition was completed, the reaction system was stirred at 80°C for 16 h. After the reaction was completed, water (50 mL) was

added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 65%) to obtain 100 mg of the title compound **190-6.** LC-MS (ESI): m/z 614.2 $[M+H]^+$.

**Preparation of compound 190**

Compound **190-6** (100 mg, 0.16 mmol), dimethylphosphine oxide (38 mg, 0.48 mmol), $Pd_2(dba)_3$ (18 mg, 0.02 mmol), Xantphos (27 mg, 0.05 mmol), and DIPEA (62 mg, 0.48 mmol) were dissolved in DMF (5 mL). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 28.8 mg of the title compound **190.** LC-MS (ESI): m/z 656.2 $[M+H]^+$. $^1H$ NMR (400 MHz, Chloroform-d) δ 8.88 (s, 1H), 7.69 (t, $J$ = 4.0 Hz, 1H), 7.62 - 7.53 (m, 2H), 7.53 - 7.46 (m, 2H), 7.43 (d, $J$ = 2.4 Hz, 1H), 7.37 - 7.27 (m, 3H), 7.23 (d, $J$ = 2.4 Hz, 1H), 7.13 - 7.06 (m, 4H), 7.06 - 7.00 (m, 2H), 5.30 (s, 2H), 4.46 (t, $J$ = 6.1 Hz, 2H), 3.89 (t, $J$ = 6.2 Hz, 2H), 2.18 (s, 3H), 1.91 (d, $J$ = 13.5 Hz, 6H). $^{31}P$ NMR (162 MHz, Chloroform-d) δ 35.36 (s, 1P).

**Example 191: Preparation of compound 191**

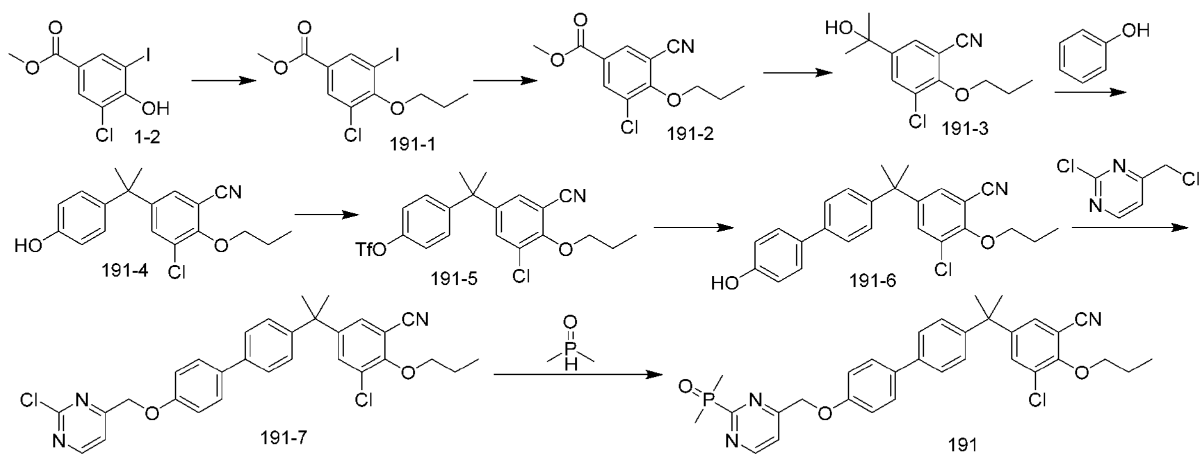

**Preparation of compound 191-1**

Compound **1-2** (3 g, 9.62 mmol) and cesium carbonate (6.3 g, 19.23 mmol) were dissolved in acetonitrile (50 mL), and 1-iodopropane (2.5 g, 14.43 mmol) was added thereto. After the addition was completed, the reaction system was stirred at 70°C for 1 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 90%) to obtain 3.1 g of the title compound **191-1.** LC-MS (ESI): m/z 355.0 $[M+H]^+$.

**Preparation of compound 191-2**

Compound **191-1** (3.1 g, 8.73 mmol) was dissolved in DMF (50 mL), and copper(I) iodide (3.3 g, 17.46 mmol) was added thereto. After the addition was completed, the reaction system was stirred at 120°C for 16 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 90%) to obtain 2 g of the title compound **191-2.** LC-MS (ESI): m/z 254.0 $[M+H]^+$.

**Preparation of compound 191-3**

Compound **191-2** (2 g, 7.87 mmol) was dissolved in tetrahydrofuran (50 mL), and methylmagnesium bromide (10.5 mL, 3 mol/L) was added thereto at 0°C. After the addition was completed, the reaction system was stirred at 25°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 85%) to obtain 1.6 g of the title compound **191-3.** LC-MS (ESI): m/z 254.0 $[M+H]^+$.

**Preparation of compound 191-4**

Compound **191-3** (1.6 g, 6.30 mmol) and phenol (567 mg, 7.56 mmol) were dissolved in dichloromethane (50 mL). Boron trifluoride diethyl etherate (3.7 g, 48%) was added to thereto at 0°C. After the addition was completed, the reaction system was stirred at 25°C for 16 h. After the reaction was completed, water (50 mL) was added, and the mixture was extracted three times with dichloromethane (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 85%) to obtain 1.2 g of the title compound **191-4.** LC-MS (ESI): m/z 330.2 $[M+H]^+$.

**Preparation of compound 191-5**

Compound **191-4** (1.2 g, 3.64 mmol) and *N*-phenylbis(trifluoromethanesulfonyl)imide (1.95 g, 5.45 mmol) were dissolved in DCM (20 mL), and triethylamine (735 mg, 7.28 mmol) was added thereto. After the addition was completed, the reaction system was stirred at 25°C for 2 h. After the reaction was completed, water (50 mL) was added, and the mixture was extracted three times with dichloromethane (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 90%) to obtain 1.2 g of the title compound **191-5.**

**Preparation of compound 191-6**

Compound **191-5** (1.2 g, 2.60 mmol), 4-hydroxyphenylboronic acid (538 mg, 3.90 mmol), and potassium carbonate (718 mg, 5.20 mmol) were dissolved in 1,4-dioxane (50 mL) and water (10 mL), and $Pd(dppf)Cl_2$ (366 mg, 0.5 mmol) was added thereto. After the addition was completed, the reaction system was stirred at 80°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 75%) to obtain 900 mg of the title compound **191-6.** LC-MS (ESI): m/z 406.2 $[M+H]^+$.

**Preparation of compound 191-7**

Compound **191-6** (900 mg, 2.22 mmol) and cesium carbonate (1.4 g, 4.44 mmol) were dissolved in acetonitrile (20 mL), and compound 2-chloro-4-(chloromethyl)pyrimidine (209 mg, 3.33 mmol) was added thereto. After the addition was completed, the reaction system was stirred at 80°C for 16 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 70%) to obtain 900 mg of the compound **191-7.** LC-MS (ESI): m/z 532.2 $[M+H]^+$.

**Preparation of compound 191**

Compound **191-7** (100 mg, 0.19 mmol), dimethylphosphine oxide (45 mg, 0.57 mmol), $Pd_2(dba)_3$ (18 mg, 0.02 mmol), Xantphos (27 mg, 0.05 mmol), and DIPEA (74 mg, 0.57 mmol) were dissolved in DMF (5 mL). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative

purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 55.52 mg of the title compound **191.** LC-MS (ESI): m/z 574.2 [M+H]+. $^1$H NMR (400 MHz, Chloroform-d) δ 8.88 (dd, *J* = 5.2, 0.6 Hz, 1H), 7.73 - 7.64 (m, 1H), 7.57 - 7.52 (m, 2H), 7.51 - 7.47 (m, 2H), 7.46 (d, *J* = 2.4 Hz, 1H), 7.35 (d, *J* = 2.4 Hz, 1H), 7.25 - 7.20 (m, 2H), 7.07 - 6.96 (m, 2H), 5.30 (s, 2H), 4.15 (t, *J* = 6.6 Hz, 2H), 1.92 (d, *J* = 13.6 Hz, 6H), 1.84 - 1.92 (m, 2H), 1.69 (s, 6H), 1.09 (t, *J* = 7.4 Hz, 3H). $^{31}$P NMR (162 MHz, Chloroform-d) δ 35.62 (s, 1P).

**Example 192: Preparation of compound 192**

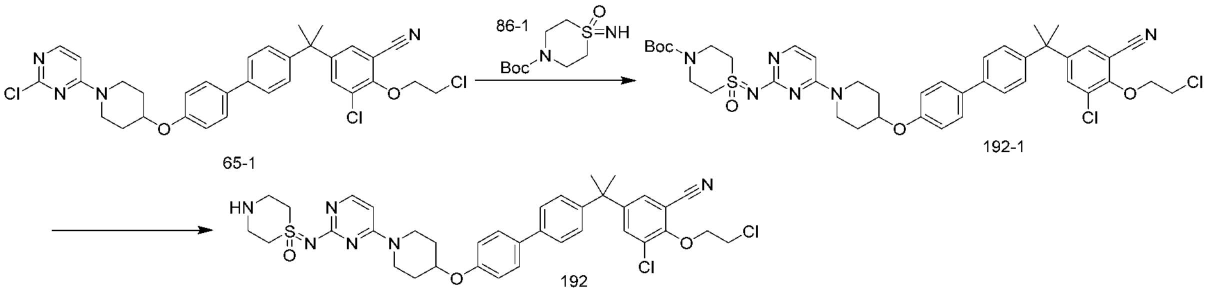


65-1

192-1

192

**Preparation of compound 192-1**

Compound **65-1** (60 mg, 0.10 mmol), compound **86-1** (68 mg, 0.30 mmol), $Pd_2(dba)_3$ (18 mg, 0.02 mmol), Xantphos (27 mg, 0.05 mmol), and DIPEA (39 mg, 0.30 mmol) were dissolved in DMF (5 mL). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (DCM/MeOH = 100% to 90%) to obtain 50 mg of compound **192-1.** LC-MS (ESI): m/z 719.4 [M-Boc+H]+.

**Preparation of compound 192**

Compound **192-1** (60 mg, 0.07 mmol) was dissolved in DCM (5 mL). A solution of hydrogen chloride in dioxane (3 mL, 4 mol/L) was added thereto, and the reaction system was stirred at 25°C for 1 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound **192.** LC-MS (ESI): m/z 719.4 [M+H]+. $^1$H NMR (400 MHz, Chloroform-d) δ 8.02 (d, *J* = 6.7 Hz, 1H), 7.55 - 7.45 (m, 5H), 7.37 (d, *J* = 2.3 Hz, 1H), 7.22 (d, *J* = 8.4 Hz, 2H), 6.99 (d, *J* = 8.7 Hz, 2H), 6.21 (d, *J* = 6.7 Hz, 1H), 4.71 - 4.61 (m, 1H), 4.42 (t, *J* = 6.2 Hz, 2H), 3.92 - 3.81 (m, 4H), 3.81 - 3.69 (m, 4H), 3.46 - 3.30 (m, 6H), 2.08 - 1.90 (m, 4H), 1.69 (s, 6H).

**Example 193: Preparation of compound 193**

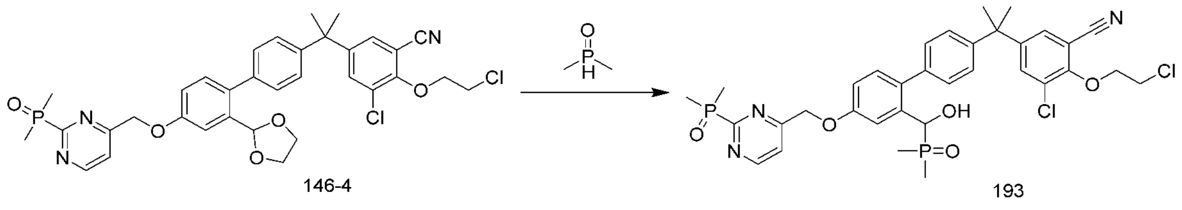

146-4

193

**Preparation of compound 193**

Compound **146-4** (300 mg, 0.45 mmol) and dimethylphosphine oxide (70 mg, 0.90 mmol) were dissolved in DCM (10 mL), and a solution of hydrogen chloride in dioxane (10 mL, 4 mol/L) was added. The reaction system was stirred at 25°C for 1 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 191 mg of the title compound **193.** LC-MS (ESI): m/z 700.2 $[M+H]^+$. $^1$H NMR (400 MHz, Chloroform-*d*) δ 8.86 (d, *J* = 5.1 Hz, 1H), 7.68 (t, *J* = 4.1 Hz, 1H), 7.50 - 7.44 (m, 2H), 7.39 - 7.31 (m, 3H), 7.24 - 7.19 (m, 3H), 6.97 (dd, *J* = 8.5, 2.5 Hz, 1H), 5.29 (s, 2H), 5.17 (d, *J* = 3.7 Hz, 1H), 4.43 (t, *J* = 6.1 Hz, 2H), 3.88 (t, *J* = 6.1 Hz, 2H), 1.88 (dd, *J* = 13.6, 2.5 Hz, 6H), 1.69 (s, 6H), 1.28 (dd, *J* = 39.4, 12.5 Hz, 6H). $^{31}$P NMR (162 MHz, Chloroform-*d*) δ 48.23 (s, 1P), δ 35.67 (s, 1P).

**Example 194: Preparation of compound 194**

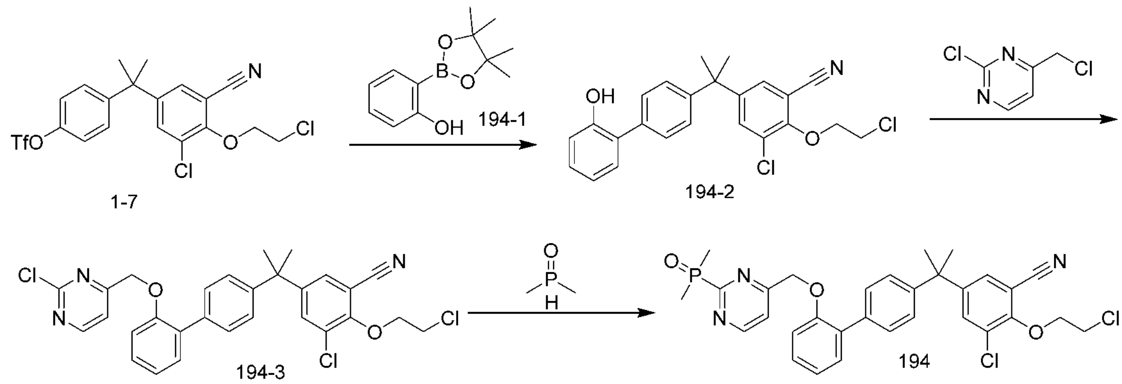

1-7
194-2
194-3
194

**Preparation of compound 194-2**

Compound **1-7** (300 mg, 622.02 μmol) and compound **194-1** (151 mg, 684.22 μmol) were dissolved in 1,4-dioxane (5 mL) and $H_2O$ (2.5 mL). Pd(dppf)$Cl_2$ (46 mg, 62.20 μmol) and $K_2CO_3$ (215 mg, 1.56 mmol) were added, and the system was purged three times with nitrogen. The reaction mixture was stirred at 90°C for 1 h. LCMS indicated the disappearance of the starting material and the formation of the product. The reaction mixture was cooled to room temperature and subjected to rotary evaporation until dryness under reduced pressure. The resulting residue was purified by silica gel column chromatography (EA/PE = 0 to 40%) to obtain 240 mg of the title compound **194-2.** LC-MS (ESI): m/z 426.3 $[M+H]^+$.

**Preparation of compound 194-3**

Compound **194-2** (271 mg, 636.25 μmol) and 2-chloro-4-(chloromethyl)pyrimidine (114 mg, 699.88 μmol) were dissolved in acetonitrile (5 mL), and $K_2CO_3$ (264 mg, 1.91 mmol) was added. The reaction mixture was stirred at 85°C for 2 h. LCMS indicated the disappearance of the starting material and the formation of the product. The reaction mixture was poured into water (30 mL) and extracted twice with ethyl acetate (60+40 mL). The organic phases were combined, washed with water (30 mL), dried over anhydrous sodium sulfate, subjected to suction filtration, and the filtrate was evaporated to dryness under reduced pressure. The resulting residue was purified by silica gel column chromatography (EA/PE = 0 to 35%) to obtain 300 mg of the title compound **194-3.** LC-MS (ESI): m/z 552.8 $[M+H]^+$.

**Preparation of compound 194**

Compound **194-3** (80 mg, 144.70 μmol) and dimethylphosphine oxide (57 mg, 723.49 μmol) were dissolved in DMF (2 mL). $Pd_2(dba)_3$ (14 mg, 14.47 μmol), Xantphos (17 mg, 28.94 μmol), and DIPEA (38 mg, 289.39 μmol, 50.41 μL) were added, and the reaction mixture was heated under microwave irradiation at 120°C for 2 h. LCMS indicated the

disappearance of the starting material and the formation of the product. The reaction mixture was cooled to room temperature, filtered, and subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 42 mg of the title compound **194.** LC-MS (ESI): m/z 594.3 $[M+H]^+$.

$^1H$ NMR (400 MHz, DMSO-$d_6$) δ 8.90 (d, *J* = 5.2 Hz, 1H), 7.74 (d, *J* = 2.4 Hz, 1H), 7.68 (d, *J* = 2.4 Hz, 1H), 7.55 (d, *J* = 8.3 Hz, 2H), 7.51 - 7.45 (m, 1H), 7.40 - 7.27 (m, 4H), 7.14 (d, *J* = 8.2 Hz, 1H), 7.07 (t, *J* = 7.4 Hz, 1H), 5.32 (s, 2H), 4.43 (t, 2H), 3.96 (t, 2H), 1.73 (d, *J* = 13.6 Hz, 6H), 1.71 (s, 6H). $^{31}P$ NMR (162 MHz, DMSO-$d_6$) δ 33.96 (s, 1P).

**Example 195: Preparation of compound 195**

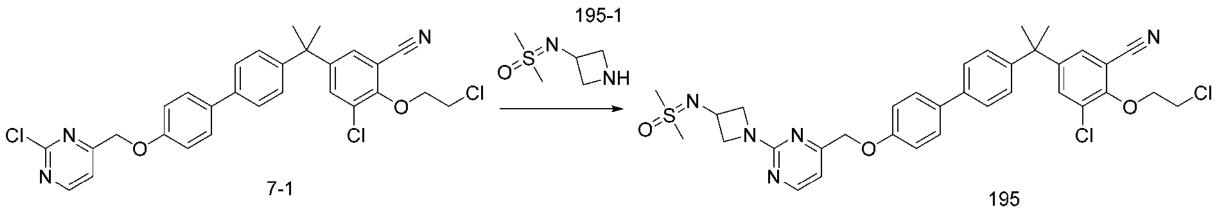


**Preparation of compound 195**

Compound **7-1** (100 mg, 180.87 μmol) and compound **195-1** (40.1 mg, 217.05 μmol) were dissolved in acetonitrile (1 mL), and triethylamine (36.6 mg, 361.74 μmol, 50.45 μL) was added. The reaction mixture was heated under microwave irradiation at 90°C for 4 h. LCMS indicated the disappearance of the starting material and the formation of the product. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 50% to 70% acetonitrile gradient elution over 12 min; flow rate: 30 mL/min) to obtain 74.64 mg of the title compound **195.** LC-MS (ESI): m/z 664.2 $[M+H]^+$. $^1H$ NMR (400 MHz, DMSO-$d_6$) δ 8.33 (d, *J* = 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.4 Hz, 2H), 7.30 (d, *J* = 8.5 Hz, 2H), 7.07 (d, *J* = 8.8 Hz, 2H), 6.73 (d, *J* = 5.0 Hz, 1H), 5.04 (s, 2H), 4.42 (t, *J* = 5.2 Hz, 2H), 4.38 - 4.24 (m, 3H), 3.96 (t, *J* = 5.2 Hz, 2H), 3.77 (dd, *J* = 8.3, 4.5 Hz, 2H), 3.03 (s, 6H), 1.68 (s, 6H).

**Example 196: Preparation of compound 196**

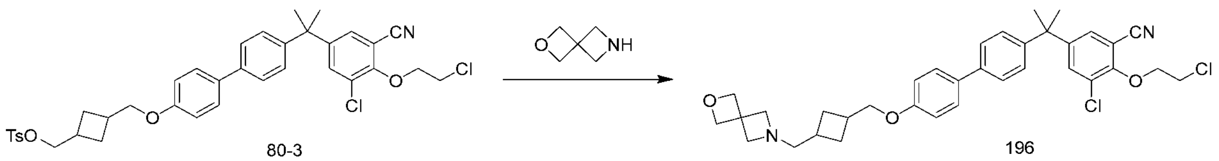


**Preparation of compound 196**

Compound **80-3** (55.7 mg, 82.07 μmol) was dissolved in acetonitrile (1.5 mL), and compound 2-oxa-6-azaspiro [3.3]heptane (12 mg, 123.11 μmol) and DIPEA (27 mg, 205.18 μmol, 36 μL) were added. The reaction mixture was stirred in a sealed tube at 90°C overnight. After the reaction was complete, the reaction mixture was cooled to room temperature, diluted with a small amount of acetonitrile, and purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 75% to 95% over 8 min; flow rate: 30 mL/min) to obtain 4 mg of the title compound **196.** LC-MS (ESI): m/z 605.3 $[M+H]^+$. $^1H$ NMR (400 MHz, $CDCl_3$) δ 7.52-7.48 (m, 5H), 7.39-7.36 (m, 1H), 7.22 (d, *J* = 8.1 Hz, 2H), 7.00-6.94 (m, 2H), 5.02 (s, 1H), 4.71-4.63 (m, 2H), 4.43 (t, *J* = 6.2 Hz, 2H), 4.15-4.01 (m, 1H), 3.93-3.86 (m, 1H), 3.88 (t, *J* = 6.2 Hz, 2H), 3.83-3.74 (m, 1H), 3.74-3.49 (m, 2H), 3.16-3.08 (m, 2H), 2.78 (m, 2H), 2.39-1.83 (m, 6H), 1.69 (s, 6H).

**Example 197: Preparation of compound 197**

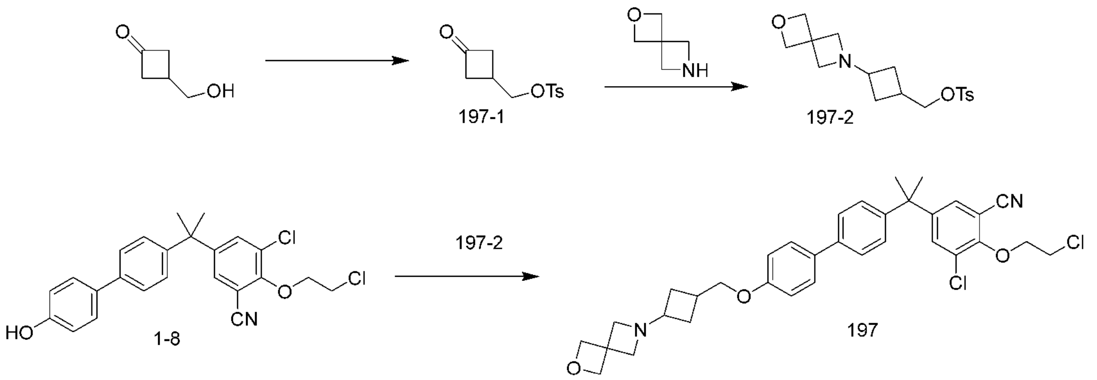

**Preparation of compound 197-1**

Compound 3-(hydroxymethyl)cyclobutan-1-one (1.5 g, 14.98 mmol) was dissolved in tetrahydrofuran (30 mL). Triethylamine (3 g, 29.97 mmol, 4.18 mL) and DMAP (183 mg, 1.50 mmol) were added separately at room temperature, followed by the slow addition of *p*-toluenesulfonyl chloride (3.43 g, 17.98 mmol). The reaction mixture was stirred at room temperature overnight. The reaction mixture was filtered through a pad of diatomite, and the filter cake was washed with ethyl acetate. The filtrates were combined and concentrated. The resulting residue was purified by an automated column chromatography system (EA/PE = 0 to 30%) to obtain 3.28 g of the title compound **197-1** in 86% yield. LC-MS (ESI): m/z 254.9 $[M+H]^+$.

**Preparation of compound 197-2**

Compound **197-1** (100 mg, 393.23 μmol) was dissolved in 1,2-dichloroethane (3 mL). 2-Oxa-6-azaspiro[3.3]heptane (59 mg, 589.85 μmol) and acetic acid (5 mg, 78.65 μmol) were added. The reaction mixture was stirred at room temperature for 0.5 h, then $NaBH(OAc)_3$ (250.03 mg, 1.18 mmol) was added, and the mixture was stirred at room temperature overnight. Water and ethyl acetate were added to the reaction mixture. The organic phase was separated and washed with saturated brine. The aqueous phase was extracted once with dichloromethane. The organic phases were combined, dried over anhydrous sodium sulfate, filtered, and the filtrate was concentrated to dryness by rotary evaporation to obtain 53.4 mg of a crude product of the title compound **197-2.** The product was used directly in the next step without purification. LC-MS (ESI): m/z 338.4 $[M+H]^+$.

**Preparation of compound 197**

Compound **1-8** (53 mg, 158.25 μmol) was dissolved in DMF (1.5 mL), and compound **197-2** (68 mg, 58.33 μmol) and cesium carbonate (77.3 mg, 237.38 μmol) were added separately. The reaction mixture was stirred at 60°C overnight. After the reaction was complete, the mixture was cooled to room temperature, diluted with a small amount of methanol, filtered, and purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 30-50% 8 min, 50-60% 2 min; flow rate: 30 mL/min) to obtain 12 mg of the title compound **197.** LC-MS (ESI): m/z 591.4 $[M+H]^+$. $^1H$ NMR (400 MHz, Chloroform-d) δ 7.52 - 7.45 (m, 5H), 7.37 (d, *J* = 2.4 Hz, 1H), 7.24 - 7.20 (m, 2H), 6.98 - 6.93 (m, 2H), 4.79 (s, 4H), 4.42 (t, *J* = 6.1 Hz, 2H), 3.98 (d, *J* = 5.7 Hz, 2H), 3.88 (t, *J* = 6.2 Hz, 2H), 3.80 - 3.50 (m, 3H), 3.41 - 3.29 (m, 1H), 2.85 (s, 1H), 2.30 - 2.03 (m, 5H), 1.69 (s, 6H).

**Example 198: Preparation of compound 198**

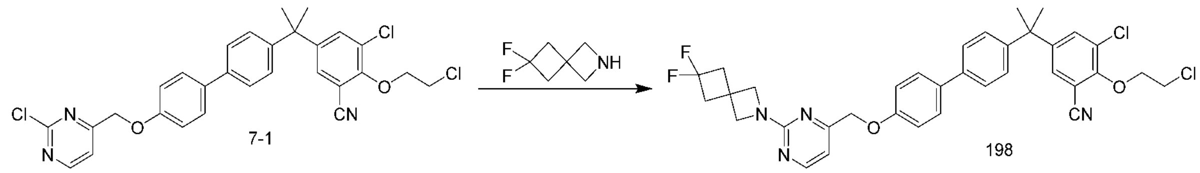

7-1

198

### Preparation of compound 198

Compound **7-1** (60 mg, 0.1 mmol), 6,6-difluoro-2-azaspiro[3.3]heptane (20 mg, 0.15 mmol), and DIPEA (20 mg, 0.15 mmol) were dissolved in anhydrous ethanol (5 mL), and the reaction was carried out at 80°C for 2 h. After the reaction was monitored by LCMS until completion, the reaction mixture was cooled to room temperature and filtered. The crude filtrate was subjected to preparative purification (preparative method: chromatographic column: Welch Xtimate C18 250 × 21.2 mm; column temperature: 25°C; mobile phase: water (10 mM $NH_4HCO_3$)-acetonitrile; mobile phase: 45% to 65% acetonitrile gradient elution over 12 min; flow rate: 30 mL/min) to obtain 32.5 mg of the title compound **198.** LC-MS (ESI): m/z 649.3 $[M+H]^+$. $^1H$ NMR (400 MHz, DMSO-$d_6$) δ 8.36 (d, *J* = 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.07 (d, *J* = 8.8 Hz, 2H), 6.78 (d, *J* = 5.0 Hz, 1H), 5.05 (s, 2H), 4.42 (t, *J* = 5.2 Hz, 2H), 4.14 (s, 4H), 3.96 (t, *J* = 5.2 Hz, 2H), 2.88 (t, *J* = 12.6 Hz, 4H), 1.68 (s, 6H). $^{19}F$ NMR (376 MHz MHz, DMSO-$d_6$) δ -90.35 (s, 2F).

### Example 199: Preparation of compound 199

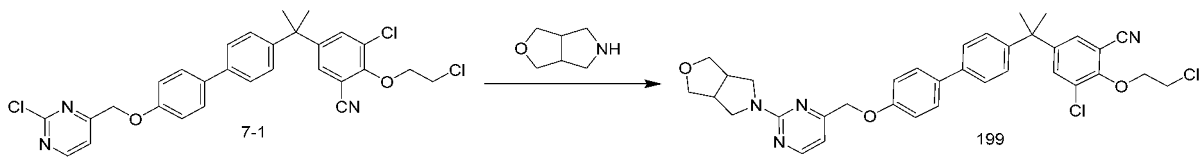

7-1

199

### Preparation of compound 199

Compound **7-1** (60 mg, 0.1 mmol), 6H-1H-furo[3,4-c]pyrrole (17 mg, 0.15 mmol), and DIEA (20 mg, 0.15 mmol) were dissolved in anhydrous ethanol (5 mL), and the reaction was carried out at 80°C for 2 h. After the reaction was monitored by LCMS until completion, the reaction mixture was cooled to room temperature and filtered. The crude filtrate was subjected to preparative purification (preparative method: chromatographic column: Welch Xtimate C18 250 × 21.2 mm; column temperature: 25°C; mobile phase: water (10 mM $NH_4HCO_3$)-acetonitrile; mobile phase: 45% to 65% acetonitrile gradient elution over 12 min; flow rate: 30 mL/min) to obtain 20 mg of the title compound **199.** LC-MS (ESI): m/z 629.4 $[M+H]^+$. $^1H$ NMR (400 MHz, DMSO-*$d_6$*) δ 8.35 (d, *J* = 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.07 (d, *J* = 8.8 Hz, 2H), 6.71 (d, *J* = 5.0 Hz, 1H), 5.05 (s, 2H), 4.42 (t, *J* = 5.2 Hz, 2H), 3.96 (t, *J* = 5.2 Hz, 2H), 3.89 - 3.77 (m, 2H), 3.76 - 3.64 (m, 2H), 3.57 (dd, *J* = 8.9, 3.3 Hz, 2H), 3.46 (dd, *J*= 11.7, 3.1 Hz, 2H), 3.09 - 2.93 (m, 2H), 1.68 (s, 6H).

### Example 200: Preparation of compound 200

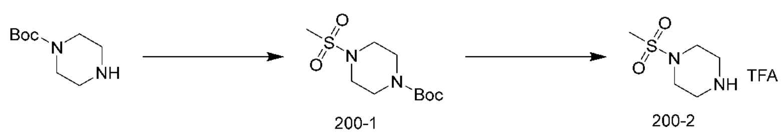

200-1

200-2

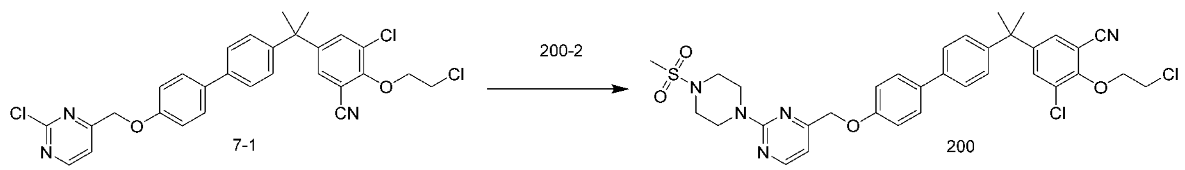


7-1

200

**Preparation of compound 200-1**

N-Boc-piperazine (250 mg, 1.34 mmol) was dissolved in DCM (20 mL), then TEA (250 mg, 1.34 mmol) was added, and the mixture was cooled to 0°C. Methanesulfonyl chloride (168 mg, 1.47 mmol) was added dropwise. After the addition was completed, the temperature was slowly raised to room temperature, and the reaction was carried out for 2 h. After the reaction was completed, water (10 mL) was added. The mixture was mixed, separated into layers, and the phases were separated. The organic phase was concentrated to obtain 320 mg of the title compound **200-1.** The product was used directly in the next step without purification. LC-MS (ESI): m/z 209.0 $[M-55+H]^+$.

**Preparation of compound 200-2**

Compound **200-1** (200 mg, crude) was dissolved in DCM (20 mL), then TFA (10 mL) was added, and the reaction was carried out at room temperature for 2 h. After the reaction was completed, the reaction mixture was concentrated to obtain 210 mg of a crude product of the title compound **200-2.** The product was used directly in the next step without purification.

**Preparation of compound 200**

Compound **7-1** (100 mg, 0.18 mmol), compound **200-2** (59 mg, 0.36 mmol), and DIEA (69 mg, 0.54 mmol) were dissolved in anhydrous ethanol (5 mL), and the reaction was carried out at 80°C for 2 h. After the reaction was monitored by LCMS until completion, the reaction mixture was cooled to room temperature and filtered. The filtrate was subjected to preparative purification (preparative method: chromatographic column: Welch Xtimate C18 250 × 21.2 mm; column temperature: 25°C; mobile phase: water (10 mM $NH_4HCO_3$)-acetonitrile; mobile phase: 45% to 65% acetonitrile gradient elution over 12 min; flow rate: 30 mL/min) to obtain 21.4 mg of the title compound **200.** LC-MS (ESI): m/z 680.0 $[M+H]^+$. $^1H$ NMR (400 MHz, DMSO-$d_6$) δ 8.42 (d, *J* = 5.0 Hz, 1H), 7.70 (d, *J* = 2.4 Hz, 1H), 7.64 (d, *J* = 2.3 Hz, 1H), 7.60 (d, *J* = 8.8 Hz, 2H), 7.56 (d, *J* = 8.5 Hz, 2H), 7.30 (d, *J* = 8.5 Hz, 2H), 7.08 (d, *J* = 8.8 Hz, 2H), 6.78 (d, *J* = 5.0 Hz, 1H), 5.08 (s, 2H), 4.42 (t, *J* = 5.2 Hz, 2H), 3.96 (t, *J* = 5.2 Hz, 2H), 3.92 - 3.80 (m, 4H), 3.22 - 3.11 (m, 4H), 2.89 (s, 3H), 1.68 (s, 6H).

**Example 201: Preparation of compound 201**

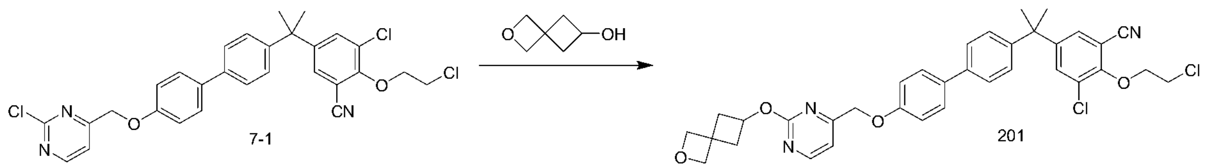

**Preparation of compound 201**

Compound **7-1** (60 mg, 0.1 mmol), 2-oxaspiro[3.3]heptan-6-ol (17 mg, 0.15 mmol), and cesium carbonate (49 mg, 0.15 mmol) were dissolved in anhydrous DMF (5 mL), and the reaction was carried out at 80°C for 2 h. After the reaction was monitored by LCMS until completion, the reaction mixture was cooled to room temperature and filtered. The filtrate was subjected to preparative purification (preparative method: chromatographic column: Welch Xtimate C18 250 × 21.2 mm; column temperature: 25°C; mobile phase: water (10 mM $NH_4HCO_3$)-acetonitrile; mobile phase: 45% to 65% acetonitrile gradient elution over 12 min; flow rate: 30 mL/min) to obtain 11.2 mg of the title compound **201.** LC-MS (ESI): m/z 630.0 $[M+H]^+$. $^1H$ NMR (400 MHz, DMSO-$d_6$) δ 8.59 (d, *J* = 5.0 Hz, 1H), 7.70 (d, *J* = 2.3 Hz, 1H), 7.64 (d, *J* = 2.3 Hz, 1H), 7.60 (d, *J* = 8.8 Hz, 2H), 7.56 (d, *J* = 8.5 Hz, 2H), 7.30 (d, *J* = 8.5 Hz, 2H), 7.21 (d, *J* = 5.0 Hz, 1H), 7.09 (d, *J* = 8.8 Hz, 2H), 5.17 (s, 2H), 5.05 - 4.92 (m, 1H), 4.61 (s, 2H), 4.54 (s, 2H), 4.42 (t, *J* = 5.2 Hz, 2H), 3.96 (t, *J* = 5.2 Hz, 2H), 2.80 - 2.69 (m, 2H), 2.30 - 2.18 (m, 2H), 1.68 (s, 6H).

**Example 202: Preparation of compound 202**

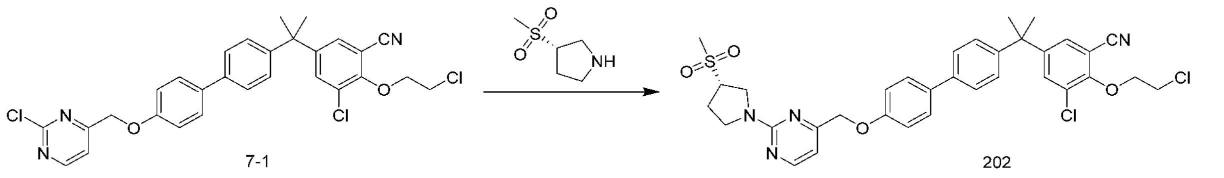

7-1 202

**Preparation of compound 202**

Compound **7-1** (100 mg, 0.18 mmol), (S)-3-(methylsulfonyl)pyrrolidine (40 mg, 0.27 mmol), and DIEA (35 mg, 0.27 mmol) were dissolved in anhydrous ethanol (5 mL), and the reaction was carried out at 80°C for 2 h. After the reaction was monitored by LCMS until completion, the reaction mixture was cooled to room temperature and filtered. The filtrate was subjected to preparative purification (preparative method: chromatographic column: Welch Xtimate C18 250 × 21.2 mm; column temperature: 25°C; mobile phase: water (10 mM $NH_4HCO_3$)-acetonitrile; mobile phase: 45% to 65% acetonitrile gradient elution over 12 min; flow rate: 30 mL/min) to obtain 90.3 mg of the title compound **202.** LC-MS (ESI): m/z 665.0 [M+H]$^+$. $^1$H NMR (400 MHz, DMSO-$d_6$) δ 8.39 (d, *J* = 4.9 Hz, 1H), 7.71 (d, *J* = 2.3 Hz, 1H), 7.64 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.08 (d, *J* = 8.8 Hz, 2H), 6.76 (d, *J* = 5.0 Hz, 1H), 5.08 (s, 2H), 4.42 (t, *J* = 5.2 Hz, 2H), 4.14 - 4.03 (m, 1H), 3.96 (t, *J* = 5.2 Hz, 2H), 3.94 - 3.79 (m, 2H), 3.75 - 3.64 (m, 1H), 3.64 - 3.53 (m, 1H), 3.07 (s, 3H), 2.44 - 2.29 (m, 2H), 1.68 (s, 6H).

**Examples 203 and 204: Preparation of compounds 203 and 204**

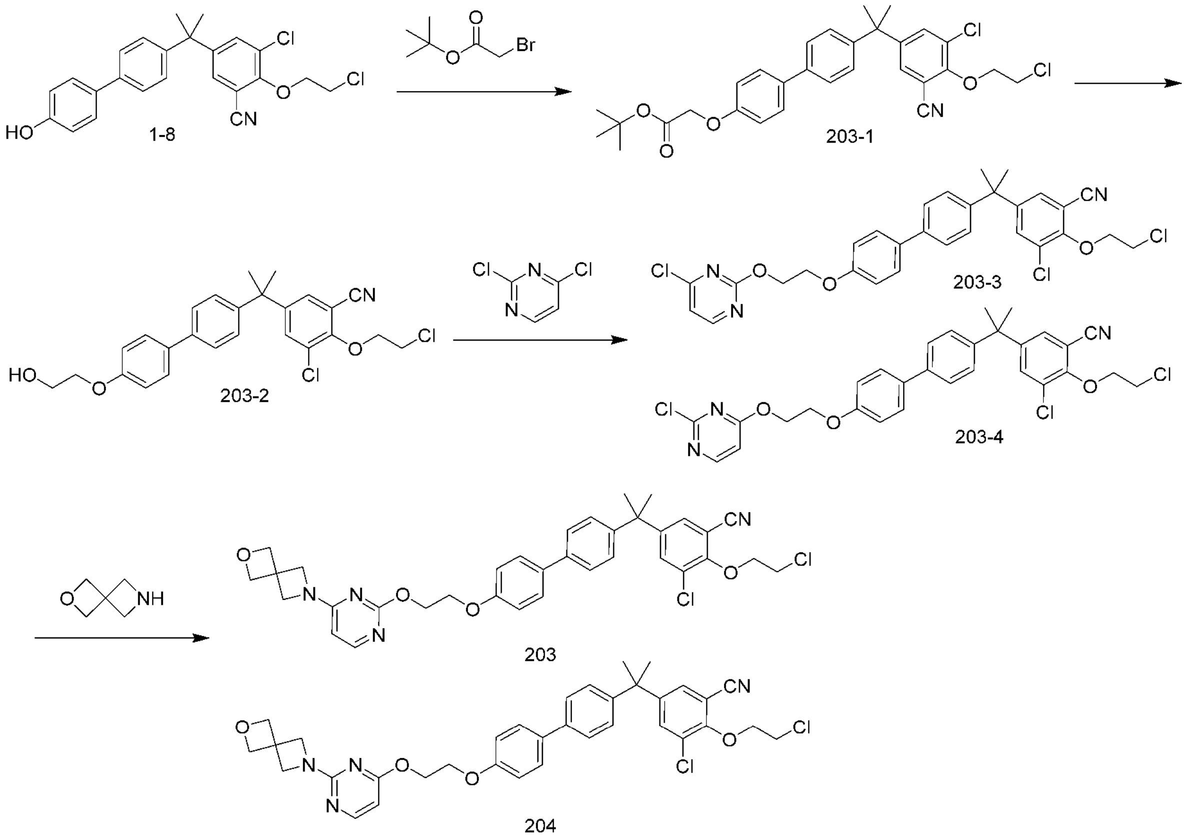

1-8 203-1 203-2 203-3 203-4 203 204

**Preparation of compound 203-1**

Compound **1-8** (1 g, 2.35 mmol) was dissolved in DMF (10 mL), followed by the sequential addition of *tert*-butyl bromoacetate (0.54 g, 2.81 mmol) and cesium carbonate (2.29 g, 7.04 mmol). The reaction system was purged three times with nitrogen and stirred at 50°C for 4 h. After the reaction was completed, water (10 mL) was added, and the mixture was extracted three times with dichloromethane (50 mL). The organic phases were combined, washed twice with saturated

brine (20 mL), dried over anhydrous sodium sulfate, subjected to suction filtration, and the filtrate was concentrated to obtain a crude product. The crude product was purified by silica gel column chromatography (DCM/PE = 0 to 90%) to obtain 1.2 g of the title compound **203-1** in 94.7% yield. LC-MS (ESI): m/z 540.4 $[M+H]^+$.

**Preparation of compound 203-2**

Compound **203-1** (1.1 g, 2.04 mmol) was dissolved in tetrahydrofuran (50 mL). Under an argon atmosphere at -40°C, $LiAlH_4$ (0.895 mL, 2.24 mmol, 2.5 mol/L in tetrahydrofuran solution) was added dropwise. The reaction system was stirred at -40°C for 2 h. After the reaction was completed, $H_2O$ (0.1 mL), a NaOH solution (0.1 mL, 15%), and $H_2O$ (0.3 mL) were added sequentially and slowly at -20°C, and the mixture was stirred at room temperature for 30 min. Insoluble solids were removed by filtration. The filtrate was dried over anhydrous sodium sulfate, subjected to suction filtration, and concentrated to obtain a crude product. The crude product was purified by silica gel column chromatography (DCM/PE = 0 to 90%) to obtain 240 mg of the title compound **203-2** in 25.1% yield. LC-MS (ESI): m/z 470.4 $[M+H]^+$.

**Preparation of compounds 203-3 and 203-4**

Compound **203-2** (150 mg, 0.318 mmol) was dissolved in DMF (5 mL), followed by the sequential addition of 2,4-dichloropyrimidine (57 mg, 0.382 mmol) and potassium carbonate (220 mg, 0.956 mmol). The reaction system was purged three times with nitrogen and stirred at 60°C for 24 h. After the reaction was completed, water (10 mL) was added, and the mixture was extracted three times with dichloromethane (50 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (DCM/PE = 0 to 100%) to obtain 140 mg of a mixture of the title compounds 203-3 and 203-4 in 75.3% yield. LC-MS (ESI): m/z 582.6 $[M+H]^+$.

**Preparation of compounds 203 and 204**

The mixture of compounds **203-3** and **203-4** (50 mg, 0.085 mmol) was dissolved in DMF (3 mL), followed by the sequential addition of 2-oxa-6-azaspiro[3.3]heptane (43 mg, 0.42 mmol) and cesium carbonate (84 mg, 0.257 mmol). The reaction system was purged three times with nitrogen and stirred at room temperature for 3 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 10 mg of the title compound **203** and 0.3 mg of the title compound **204.**

Compound 203: LC-MS (ESI): m/z 645.6 $[M+H]^+$. $^1H$ NMR (400 MHz, DMSO-$d_6$) δ 7.98 (d, *J* = 5.8 Hz, 1H), 7.70 (d, *J* = 2.4 Hz, 1H), 7.64 (d, *J* = 2.3 Hz, 1H), 7.61 - 7.54 (m, 4H), 7.30 (d, *J* = 8.5 Hz, 2H), 7.04 (d, *J* = 8.8 Hz, 2H), 6.06 (d, *J* = 5.8 Hz, 1H), 4.70 (s, 4H), 4.54 - 4.49 (m, 2H), 4.42 (t, *J* = 5.2 Hz, 2H), 4.33 - 4.28 (m, 2H), 4.17 (s, 4H), 3.96 (t, 2H), 1.68 (s, 6H).

Compound **204:** LC-MS (ESI): m/z 645.6 $[M+H]^+$. $^1H$ NMR (400 MHz, DMSO-$d_6$) δ 8.08 (d, *J* = 5.6 Hz, 1H), 7.70 (d, *J* = 2.3 Hz, 1H), 7.64 (d, *J* = 2.4 Hz, 1H), 7.61 - 7.54 (m, 4H), 7.30 (d, *J* = 8.5 Hz, 2H), 7.04 (d, *J* = 8.8 Hz, 2H), 6.16 (d, *J* = 5.6 Hz, 1H), 4.70 (s, 4H), 4.61 - 4.57 (m, 2H), 4.42 (t, *J* = 5.2 Hz, 2H), 4.36 - 4.32 (m, 2H), 4.17 (s, 4H), 3.96 (t, *J* = 5.2 Hz, 2H), 1.68 (s, 6H).

**Example 205: Preparation of compound 205**

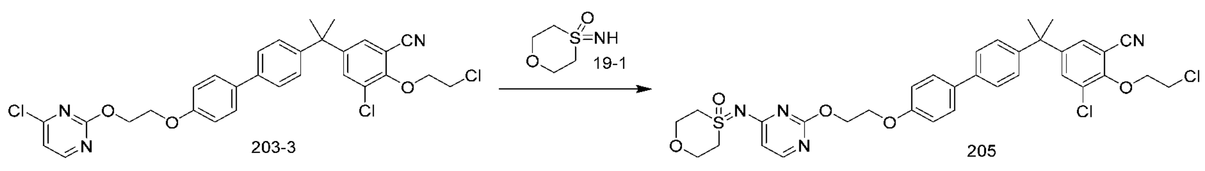

**Preparation of compound 205**

Compound **203-3** (60 mg, 0.102 mmol) was dissolved in DMF (4 mL), followed by the sequential addition of $Pd_2(dba)_3$ (10 mg, 0.010 mmol), Xantphos (12 mg, 0.020 mmol), DIPEA (40 mg, 0.308 mmol), and compound **19-1** (68 mg, 0.503 mmol). The reaction system was stirred at 120°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 4.8 mg of the title compound **205.** LC-MS (ESI): m/z 681.6 $[M+H]^+$. $^1H$ NMR (400 MHz, DMSO-$d_6$) δ 8.18 (d, *J* = 5.7 Hz, 1H), 7.70 (d, *J* = 2.4 Hz, 1H), 7.64 (d, *J* = 2.4 Hz, 1H), 7.59 (d, *J* = 8.8 Hz, 2H), 7.56 (d, *J* = 8.5 Hz, 2H), 7.30 (d, *J* = 8.5 Hz, 2H), 7.05 (d, *J* = 8.8 Hz, 2H), 6.41 (d, *J* = 5.7 Hz, 1H), 4.59 (t, *J* = 4.5 Hz, 2H), 4.42 (t, *J* = 5.2 Hz, 2H), 4.35 (t, *J* = 4.5 Hz, 2H), 4.13 - 4.03 (m, 2H), 4.02 - 3.86 (m, 4H), 3.86 - 3.76 (m, 2H), 3.63 - 3.53 (m, 2H), 1.68 (s, 6H).

**Example 206: Preparation of compound 206**

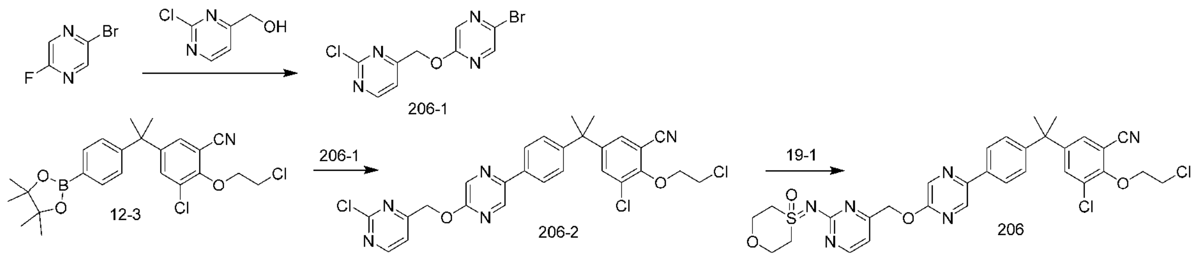


**Preparation of compound 206-1**

(2-Chloropyrimidin-4-yl)methanol (441.09 mg, 3.05 mmol) and potassium carbonate (703 mg, 5.09 mmol) were weighed and added to acetonitrile (10 mL). After stirring for 5 min, 2-fluoro-5-bromopyrazine (450 mg, 2.54 mmol) was added. After the addition was completed, the reaction was carried out at 75°C for 4 h. After the reaction was monitored by LCMS until completion, the reaction mixture was poured into ice water and extracted twice with ethyl acetate (20 mL). The organic phases were combined, dried over anhydrous sodium sulfate, subjected to suction filtration, and the filtrate was concentrated to obtain a crude product. The crude product was purified by normal-phase silica gel column chromatography (EA/PE = 0 to 40%) to obtain 250 mg of the title compound **206-1** in 32.6% yield. LC-MS (ESI): m/z 301.0 $[M+H]^+$.

**Preparation of compound 206-2**

Compound **12-3** (220 mg, 729.62 μmol), compound **206-1** (370 mg, 802.58 μmol), $Pd_2(dba)_3$ (53 mg, 72.96 μmol), and potassium carbonate (252 mg, 1.82 mmol) were added to a mixed solvent of $H_2O$ (2 mL) and 1,4-dioxane (10 mL). The system was purged three times with nitrogen, and the reaction was carried out at 100°C for 5 h. After the reaction was monitored by LCMS until completion, the reaction mixture was poured into ice water and extracted twice with ethyl acetate (20 mL). The organic phases were combined, dried over anhydrous sodium sulfate, subjected to suction filtration, and the filtrate was concentrated to obtain a crude product. The crude product was purified by normal-phase silica gel column chromatography (EA/PE = 0 to 40%) to obtain 200 mg of the title compound **206-2** in 49.4% yield. LC-MS (ESI): m/z 554.4 $[M+H]^+$.

**Preparation of compound 206**

Compound **206-2** (80 mg, 144.18 μmol) was dissolved in 1,4-dioxane (3 mL), followed by the sequential addition of $Pd_2(dba)_3$ (14 mg, 14.42 μmol), Xantphos (17 mg, 28.84 μmol), DIEA (56 mg, 432.55 μmol), and compound **19-1** (59 mg, 432.55 μmol). The system was purged three times with nitrogen, and the reaction was carried out under microwave irradiation at 120°C for 3 h. After the reaction was monitored by LCMS until completion, the reaction mixture was cooled to room temperature and filtered. The crude filtrate was subjected to preparative purification (preparative method: mobile

phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 40 mg of the title compound **206.** LC-MS (ESI): m/z 653.5 $[M+H]^+$. $^1H$ NMR (400 MHz, DMSO-$d_6$) δ 8.75 (d, *J* = 1.4 Hz, 1H), 8.53 (d, *J* = 1.4 Hz, 1H), 8.43 (d, *J* = 5.1 Hz, 1H), 7.96 (d, *J* = 8.6 Hz, 2H), 7.70 (d, *J* = 2.3 Hz, 1H), 7.63 (d, *J* = 2.4 Hz, 1H), 7.37 (d, *J* = 8.6 Hz, 2H), 6.96 (d, *J* = 5.1 Hz, 1H), 5.40 (s, 2H), 4.42 (t, *J* = 5.2 Hz, 2H), 4.10 - 4.00 (m, 2H), 3.99 - 3.86 (m, 2H), 3.95 (t, *J* = 5.2 Hz, 2H), 3.81 - 3.69 (m, 2H), 3.60 - 3.48 (m, 2H), 1.69 (s, 6H).

**Example 207: Preparation of compound 207**

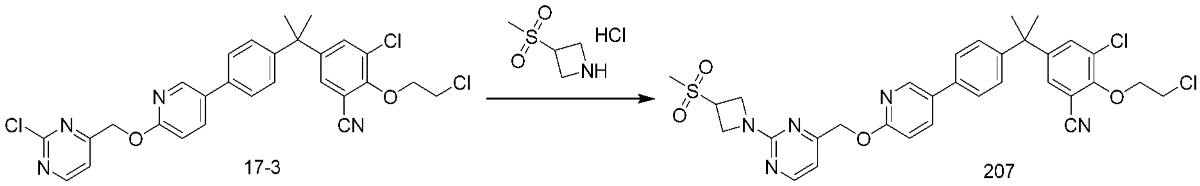


17-3 207

**Preparation of compound 207**

Compound **17-3** (50 mg, 90.27 μmol) was added to a microwave tube, followed by the addition of anhydrous ethanol (2 mL), compound 3-(methylsulfonyl)azetidine hydrochloride (24 mg, 135.41 μmol), and DIPEA (35 mg, 270.82 μmol). The system was purged three times with nitrogen, and the reaction was carried out under microwave irradiation at 90°C for 3 h. After the reaction was monitored by LCMS until completion, the solvent was removed by evaporation under reduced pressure. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 40 mg of the title compound **207.** LC-MS (ESI): m/z 652.4 $[M+H]^+$. $^1H$ NMR (400 MHz, DMSO-*d*$_6$) δ 8.43 (d, *J* = 2.5 Hz, 1H), 8.36 (d, *J* = 5.1 Hz, 1H), 8.07 (dd, *J* = 8.6, 2.6 Hz, 1H), 7.71 (d, *J* = 2.3 Hz, 1H), 7.65 (d, *J* = 2.4 Hz, 1H), 7.60 (d, *J* = 8.5 Hz, 2H), 7.33 (d, *J* = 8.5 Hz, 2H), 7.07 (d, *J* = 8.6 Hz, 1H), 6.73 (d, *J* = 5.0 Hz, 1H), 5.32 (s, 2H), 4.42 (t, *J* = 5.2 Hz, 2H), 4.41 - 4.36 (m, 1H), 4.33 (t, *J* = 8.8 Hz, 2H), 4.25 - 4.18 (m, 2H), 3.96 (t, *J* = 5.2 Hz, 2H), 3.06 (s, 3H), 1.68 (s, 6H).

**Example 208: Preparation of compound 208**

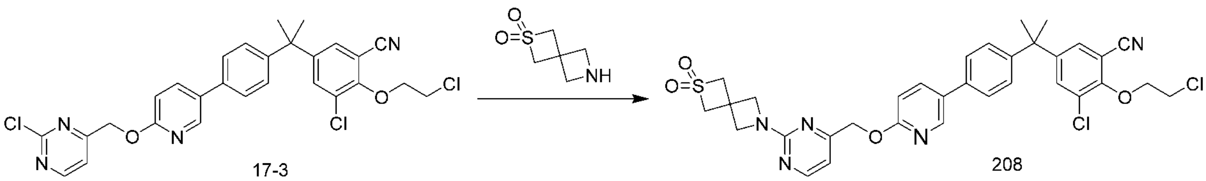

17-3 208

**Preparation of compound 208**

Compound **17-3** (60 mg, 108.33 μmol) was added to a microwave tube, followed by the sequential addition of anhydrous ethanol (2 mL), compound 2-thia-6-azaspiro[3.3]heptane 2,2-dioxide (24 mg, 162.49 μmol), and DIPEA (42 mg, 324.99 μmol). The system was purged three times with nitrogen, and the reaction was carried out under microwave irradiation at 90°C for 3 h. After the reaction was monitored by LCMS until completion, the solvent was removed by evaporation under reduced pressure. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 45 mg of the title compound **208.** LC-MS (ESI): m/z 664.2 $[M+H]^+$. $^1H$ NMR (400 MHz, DMSO-*d*$_6$) δ 8.42 (d, *J* = 2.5 Hz, 1H), 8.33 (d, *J* = 5.0 Hz, 1H), 8.07 (dd, *J* = 8.6, 2.6 Hz, 1H), 7.71 (d, *J* = 2.4 Hz, 1H), 7.64 (d, *J* = 2.3 Hz, 1H), 7.60 (d, *J* = 8.4 Hz, 2H), 7.33 (d, *J* = 8.5 Hz, 2H), 7.06 (d, *J* = 8.6 Hz, 1H), 6.70 (d, *J* = 5.0 Hz, 1H), 5.30 (s, 2H), 4.51 (s, 4H), 4.42 (t, *J* = 5.2 Hz, 2H), 4.26 (s, 4H), 3.96 (t, *J* = 5.2 Hz, 2H), 1.68 (s, 6H).

**Example 209: Preparation of compound 209**

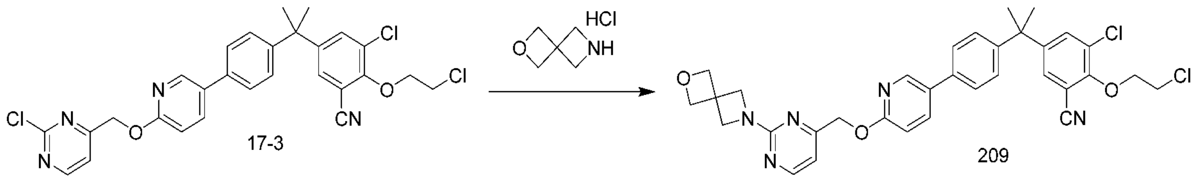


**Preparation of compound 209**

Compound **17-3** (60 mg, 108.33 μmol) was added to a microwave tube, followed by the sequential addition of anhydrous ethanol (2 mL), compound 2-oxa-6-azaspiro[3.3]heptane hydrochloride (16 mg, 162.49 μmol), and DIPEA (42 mg, 324.99 μmol). The system was purged three times with nitrogen, and the reaction was carried out under microwave irradiation at 90°C for 3 h. After the reaction was monitored by LCMS until completion, the mixture was cooled to room temperature, and the solvent was removed by evaporation under reduced pressure. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 40 mg of the title compound **209.** LC-MS (ESI): m/z 616.4 $[M+H]^+$. $^1H$ NMR (400 MHz, DMSO-$d_6$) δ 8.41 (d, *J* = 2.5 Hz, 1H), 8.29 (d, *J* = 5.0 Hz, 1H), 8.06 (dd, *J* = 8.7, 2.6 Hz, 1H), 7.71 (d, *J* = 2.3 Hz, 1H), 7.64 (d, *J* = 2.3 Hz, 1H), 7.60 (d, *J* = 8.4 Hz, 2H), 7.33 (d, *J* = 8.4 Hz, 2H), 7.05 (d, *J* = 8.6 Hz, 1H), 6.64 (d, *J* = 5.0 Hz, 1H), 5.28 (s, 2H), 4.71 (s, 4H), 4.42 (t, *J* = 5.2 Hz, 2H), 4.19 (s, 4H), 3.95 (t, *J* = 5.2 Hz, 2H), 1.68 (s, 6H).

**Example 210: Preparation of compound 210**

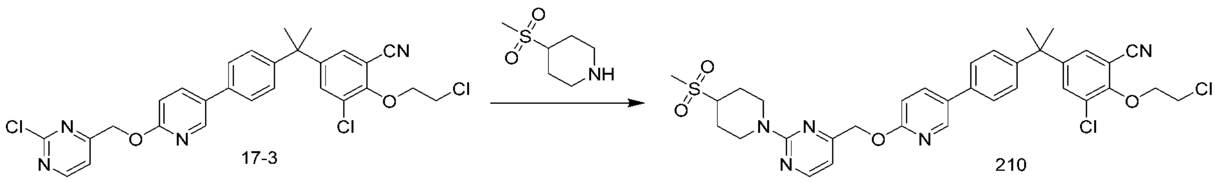

**Preparation of compound 210**

Compound **17-3** (60 mg, 108.33 μmol) was added to a microwave tube, followed by the sequential addition of anhydrous ethanol (2 mL), 4-(methylsulfonyl)piperidine (27 mg, 162.49 μmol), and DIPEA (42 mg, 324.99 μmol). The system was purged three times with nitrogen, and the reaction was carried out under microwave irradiation at 90°C for 3 h. After the reaction was monitored by LCMS until completion, the mixture was cooled to room temperature, and the solvent was removed by evaporation under reduced pressure. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 13 mg of the title compound **210.** LC-MS (ESI): m/z 680.4 $[M+H]^+$. $^1H$ NMR (400 MHz, DMSO-$d_6$) δ 8.42 (d, *J* = 2.5 Hz, 1H), 8.33 (d, *J* = 5.0 Hz, 1H), 8.06 (dd, *J* = 8.6, 2.6 Hz, 1H), 7.71 (d, *J* = 2.3 Hz, 1H), 7.64 (d, *J* = 2.4 Hz, 1H), 7.60 (d, *J* = 8.4 Hz, 2H), 7.33 (d, *J* = 8.5 Hz, 2H), 7.06 (d, *J* = 8.6 Hz, 1H), 6.62 (d, *J* = 5.0 Hz, 1H), 5.32 (s, 2H), 4.85 - 4.72 (m, 2H), 4.42 (t, *J* = 5.2Hz, 2H), 3.96 (t, *J* = 5.2Hz, 2H), 3.45 - 3.40 (m, 1H), 2.96 - 2.85 (m, 2H), 2.93 (s, 3H), 2.12 - 2.00 (m, 2H), 1.68 (s, 6H), 1.55 - 1.41 (m, 2H).

**Example 211: Preparation of compound 211**

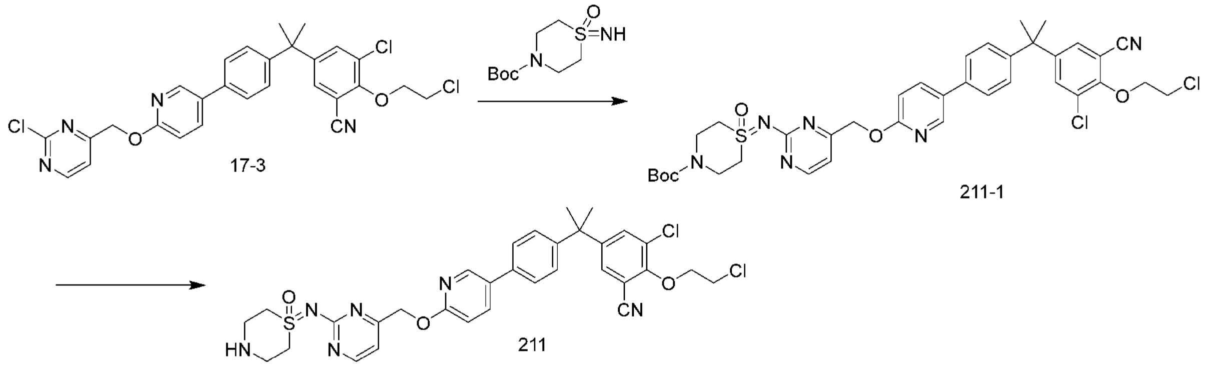

17-3

211-1

211

**Preparation of compound 211-1**

Compound **17-3** (300 mg, 541.65 μmol) was dissolved in DMF (8 mL), followed by the sequential addition of $Pd_2(dba)_3$ (50 mg, 54.16 μmol), Xantphos (63 mg, 108.33 μmol), DIEA (210 mg, 1.62 mmol), and *tert*-butyl 1-imino-116-thiomorpholine-4-carboxylate 1-oxide (381 mg, 1.62 mmol). The reaction system was stirred at 120°C for 3 h. After the reaction was monitored by LCMS until completion, the reaction mixture was cooled to room temperature, poured into ice water. and extracted twice with ethyl acetate (20 mL). The organic phases were combined, dried over anhydrous sodium sulfate, subjected to suction filtration, and the filtrate was concentrated to obtain a crude product. The crude product was purified by normal-phase silica gel column chromatography (EA/PE = 0 to 40%) to obtain 300 mg of the title compound **211-1** in 73.7% yield. LC-MS (ESI): m/z 751.6 $[M+H]^+$.

**Preparation of compound 211**

Compound **211-1** (280 mg, 372.48 μmol) was dissolved in dichloromethane (5 mL), and a solution of hydrogen chloride in 1,4-dioxane (5 mL, 4 M) was added dropwise with stirring. After the addition was completed, the reaction was carried out at room temperature overnight. After the reaction was monitored by LCMS until completion, the reaction mixture was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 90 mg of the title compound **211.** LC-MS (ESI): m/z 651.4 $[M+H]^+$. $^1H$ NMR (400 MHz, DMSO-$d_6$) δ 8.43 (d, *J* = 2.6 Hz, 1H), 8.39 (d, *J* = 5.1 Hz, 1H), 8.07 (dd, *J* = 8.6, 2.5 Hz, 1H), 7.71 (d, *J* = 2.3 Hz, 1H), 7.65 (d, *J* = 2.3 Hz, 1H), 7.60 (d, *J* = 8.5 Hz, 2H), 7.33 (d, *J* = 8.5 Hz, 2H), 7.06 (d, *J* = 8.6 Hz, 1H), 6.86 (d, *J* = 5.1 Hz, 1H), 5.33 (s, 2H), 4.42 (t, *J* = 5.2 Hz, 2H), 3.96 (t, *J* = 5.2 Hz, 2H), 3.63 - 3.51 (m, 2H), 3.32 - 3.22 (m, 2H), 3.19 - 3.07 (m, 2H), 3.04 - 2.92 (m, 2H), 1.68 (s, 6H).

**Example 212: Preparation of compound 212**

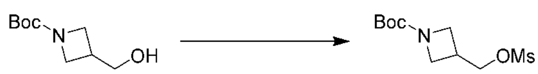

212-1

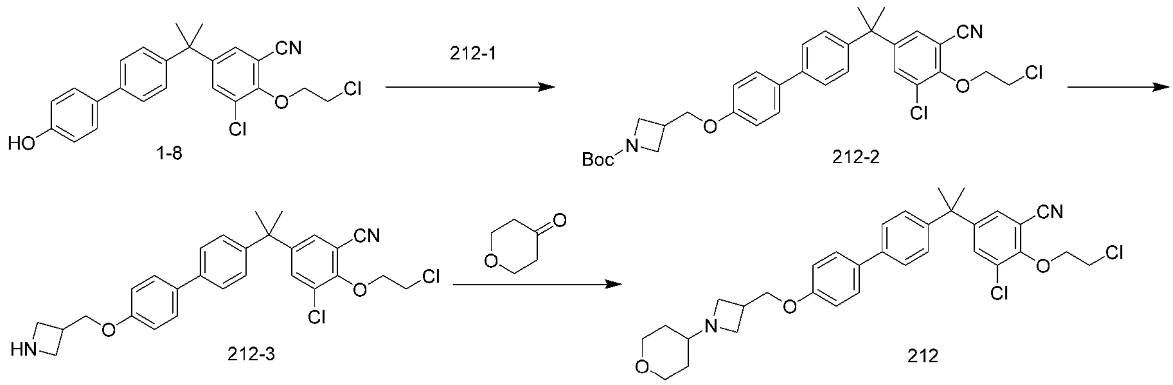


1-8, 212-2, 212-3, 212

### Preparation of compound 212-1

Compound *tert*-butyl 3-(hydroxymethyl)azetidine-1-carboxylate (4.5 g, 24.03 mmol) was dissolved in dichloromethane (50 mL). In an ice bath, triethylamine (7.28 g, 72.09 mmol) was added, followed by the dropwise addition of methanesulfonyl chloride (4.13 g, 36.05 mmol). The reaction system was reacted from 0°C to room temperature for 2 h. After the reaction was completed, water (30 mL) was added, and the mixture was extracted three times with dichloromethane (30 mL). The organic phases were combined, washed twice with saturated brine (20 mL), dried over anhydrous sodium sulfate, subjected to suction filtration, and the filtrate was concentrated to obtain 6.3 g of a crude product of the title compound **212-1.** LC-MS (ESI): m/z 210.0 $[M+H-56]^+$.

### Preparation of compound 212-2

Compound **1-8** (500 mg, 1.17 mmol) was dissolved in DMF (5 mL). At 0°C, sodium hydride (56 mg, 1.40 mmol) was added, and the mixture was stirred at this temperature for 15 min. A solution of compound **212-1** (341 mg, 1.29 mmol) in DMF (5 mL) was added dropwise. The reaction system was stirred at room temperature for 15 h. After the reaction was completed, water (50 mL) was added, and the mixture was extracted three times with ethyl acetate (40 mL). The organic phases were combined, washed twice with saturated brine (30 mL), dried over anhydrous sodium sulfate, subjected to suction filtration, and the filtrate was concentrated to obtain a crude product. The crude product was purified by silica gel column chromatography (EA/PE = 0 to 15%) to obtain 550 mg of the title compound **212-2** in 79% yield. LC-MS (ESI): m/z 539.6 $[M+H-56]^+$.

### Preparation of compound 212-3

Compound **212-2** (120 mg, 0.20 mmol) was dissolved in DCM (5 mL), and TFA (0.5 mL) was added dropwise in an ice bath. The reaction system was stirred at room temperature for 2 h. After the reaction was completed, the organic solvent and volatiles were removed by evaporation under reduced pressure to obtain 95 mg of a crude product of the title compound **212-3.** LC-MS (ESI): m/z 495.2 $[M+H]^+$.

### Preparation of compound 212

Compound **212-3** (50 mg, 0.10 mmol) was dissolved in DCM (5 mL), and compound tetrahydropyranone (13 mg, 0.13 mmol) and sodium triacetoxyborohydride (32 mg, 0.15 mmol) were added sequentially at 0°C. The reaction system was stirred at room temperature for 2 h. After the reaction was completed, methanol was added to quench the reaction, and the mixture was filtered. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 46 mg of the title compound **212.** LC-MS (ESI): m/z 579.2 $[M+H]^+$. $^1H$ NMR (400 MHz, DMSO-$d_6$) δ 7.70 (d, *J* = 2.3 Hz, 1H), 7.64 (d, *J* = 2.3 Hz, 1H), 7.62 (d, *J* = 8.6 Hz, 2H), 7.56 (d, *J* = 8.5 Hz, 2H), 7.31 (d, *J* = 8.4 Hz, 2H), 7.05 (d, *J* = 8.7 Hz, 2H), 4.42 (t, *J* = 5.2 Hz, 2H), 4.37 - 4.01 (m, 4H), 3.96 (t, *J* = 5.1 Hz, 2H), 3.95 - 3.70 (m, 4H), 3.31 - 3.04 (m, 4H), 1.93 - 1.76 (m, 2H), 1.68 (s, 6H), 1.37 - 1.19 (m, 2H).

### Example 213: Preparation of compound 213

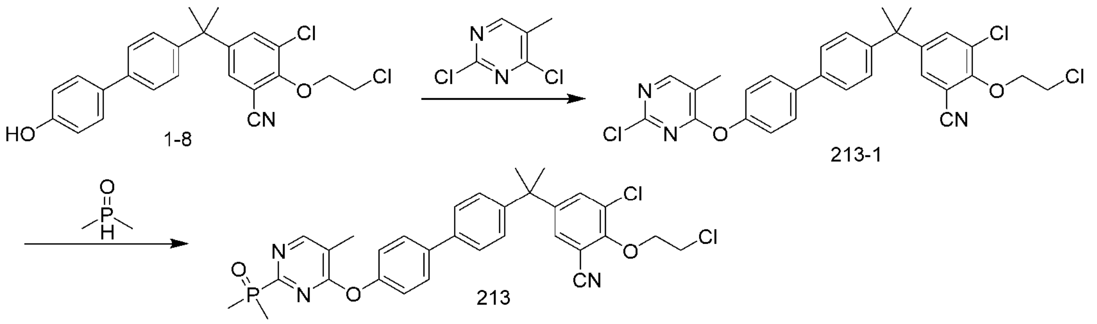

## Preparation of compound 213-1

Compound **1-8** (20 mg, 0.05 mmol) was dissolved in tetrahydrofuran (2 mL) and cooled to 0°C. Sodium hydride (0.5 mg, 0.94 mmol) was added, and the mixture was stirred at this temperature for 15 min. 2,4-Dichloro-5-methylpyrimidine (12 mg, 0.07 mmol) was added, and the reaction system was stirred at 25°C for 12 h. After the reaction was completed, water (5 mL) was added, and the mixture was extracted three times with dichloromethane (10 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 45%) to obtain 14 mg of the title compound **213-1.** LC-MS (ESI): m/z 552.6 $[M+H]^+$.

## Preparation of compound 213

Compound **213-1** (40 mg, 0.09 mmol) was dissolved in dioxane (2 mL), followed by the addition of compound dimethylphosphine oxide (33 mg, 0.10 mmol), Xantphos (2 mg, 4.1 μmol), and triethylamine (61 mg, 0.19 mmol). The system was purged three times with nitrogen, and $Pd_2(dba)_3$ (61 mg, 0.19 mmol) was added. The reaction system was stirred at 90°C for 12 h. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 16 mg of the title compound **213.** LC-MS (ESI): m/z 594.2. $[M+H]^+$.

## Example 214: Preparation of compound 214

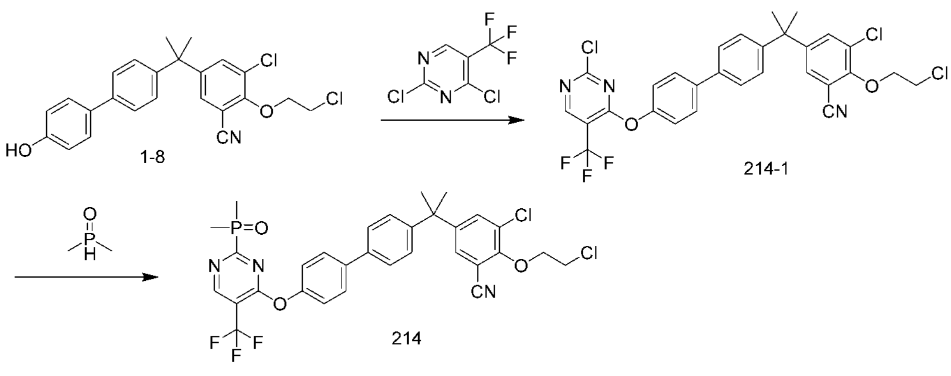

## Preparation of compound 214-1

Compound **1-8** (60 mg, 0.14 mmol) was dissolved in tetrahydrofuran (2 mL) and cooled to 0°C. Sodium hydride (7 mg, 0.28 mmol) was added, and the mixture was stirred for 15 min. 2,4-Dichloro-5-(trifluoromethyl)pyrimidine (34 mg, 0.15 mmol) was added, and the reaction system was stirred at 25°C for 12 h. After the reaction was completed, water (5 mL) was added, and the mixture was extracted three times with dichloromethane (10 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 45%) to obtain 35 mg of the title compound **214-1.** LC-MS (ESI): m/z 606.2 $[M+H]^+$.

**Preparation of compound 214**

Compound **214-1** (90 mg, 0.15 mmol) was dissolved in dioxane (2 mL), followed by the sequential addition of compound dimethylphosphine oxide (14 mg, 0.18 mmol), Xantphos (17 mg, 0.03 mmol), and triethylamine (75 mg, 0.74 mmol). The system was purged three times with nitrogen, and $Pd_2(dba)_3$ (14 mg, 14 μmol) was added. The reaction system was stirred at 90°C for 6 h. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 16 mg of the title compound **214.** LC-MS (ESI): m/z 648.2 $[M+H]^+$. $^1H$ NMR (400 MHz, Chloroform-*d*) δ 8.99 (s, 1H), 7.66 (d, *J* = 8.6 Hz, 2H), 7.56 (d, *J* = 8.2 Hz, 2H), 7.50 (d, *J* = 2.4 Hz, 1H), 7.37 (d, *J* = 2.4 Hz, 1H), 7.28 (d, *J* = 8.2 Hz, 2H), 7.27 (d, *J* = 8.5 Hz, 2H), 4.44 (t, *J* = 6.1 Hz, 2H), 3.89 (t, *J* = 6.1 Hz, 2H), 1.73 (d, *J* = 13.6 Hz, 6H), 1.71 (s, 6H). $^{31}P$ NMR (162 MHz, Chloroform-*d*) δ 35.88 (s, 1P). $^{19}F$ NMR (376 MHz, Chloroform-*d*) δ -63.50 (s, 3F).

**Example 215: Preparation of compound 215**

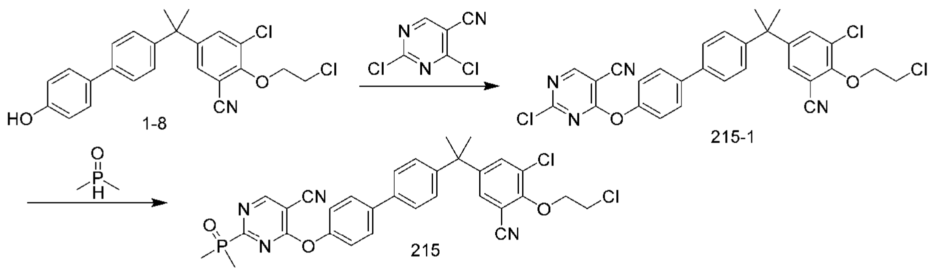

**Preparation of compound 215-1**

Compound **1-8** (60 mg, 0.14 mmol) was dissolved in acetonitrile (2 mL), followed by the sequential addition of potassium carbonate (39 mg, 0.28 mmol) and 2,4-dichloro-5-cyanopyrimidine (34 mg, 0.15 mmol). The reaction system was stirred at 50°C for 12 h. After the reaction was completed, water (5 mL) was added, and the mixture was extracted three times with dichloromethane (10 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 45%) to obtain 55 mg of the title compound **215-1.**

**Preparation of compound 215**

Compound **215-1** (24 mg, 0.04 mmol) was dissolved in dioxane (2 mL), followed by the sequential addition of compound dimethylphosphine oxide (7 mg, 0.08 mmol), Xantphos (4 mg, 8.3 μmol), and triethylamine (21 mg, 0.21 mmol). The system was purged three times with nitrogen, and $Pd_2(dba)_3$ (14 mg, 4.1 μmol) was added. The reaction system was stirred at 90°C for 6 h. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 6 mg of the title compound **215.** LC-MS (ESI): m/z 605.3 $[M+H]^+$. $^1H$ NMR (400 MHz, Chloroform-*d*) δ 9.02 (s, 1H), 7.67 (d, *J* = 8.6 Hz, 2H), 7.56 (d, *J* = 8.1 Hz, 2H), 7.50 (d, *J* = 2.3 Hz, 1H), 7.37 (d, *J* = 2.3 Hz, 1H), 7.31 - 7.25 (m, 4H), 4.44 (t, *J* = 6.1 Hz, 2H), 3.89 (t, *J* = 6.1 Hz, 2H), 1.73 (d, *J* = 13.6 Hz, 6H), 1.72 (s, 6H). $^{31}P$ NMR (162 MHz, Chloroform-d) δ 36.18 (s, 1P).

**Example 216: Preparation of compound 216**

**Preparation of compound 216-1**

Compound **42-5** (6 g, 13.40 mmol), bis(pinacolato)diboron (3.74 g, 14.74 mmol), and potassium acetate (3.3 g, 33.50 mmol) were dissolved in 1,4-dioxane (60 mL). Xantphos (0.49 g, 0.67 mmol) was then added, and the system was purged four times with argon, then heated to 110°C, and stirred for 16 h. The reaction was monitored by LCMS until completion. The reaction system was cooled to room temperature and filtered through a pad of diatomite, and the filter cake was rinsed with ethyl acetate (50 mL). The filtrates were combined, concentrated, and purified by normal-phase silica gel

column chromatography (EA/PE = 0 to 30%) to obtain 6 g of the title compound **216-1** in 96.5% yield. LC-MS (ESI): m/z 425.8 $[M+H]^+$.

**Preparation of compound 216-2**

Compound **216-1** (2 g, 4.70 mmol) and compound 2-bromo-5-hydroxypyridine (0.9 g, 5.17 mmol) were dissolved in 1,4-dioxane (20 mL). Pd(dppf)$Cl_2$ (340 mg, 0.47 mmol), potassium carbonate (1.62 g, 11.75 mmol), and water (4 mL) were added. The system was purged four times with argon, heated to 110°C, and stirred for 2 h. The reaction was monitored by LCMS until completion. The reaction system was cooled to room temperature and filtered through a pad of diatomite, and the filter cake was rinsed with ethyl acetate (20 mL). The filtrates were combined, the organic phase was concentrated, and purified by normal-phase silica gel column chromatography (EA/PE = 0 to 70%) to obtain 1 g of the title compound **216-2** in 54.2% yield. LC-MS (ESI): m/z 390.8 $[M-H]^-$.

**Preparation of compound 216-3**

Compound **216-2** (500 mg, 1.27 mmol) and (2-chloropyrimidin-4-yl)methyl methanesulfonate (311 mg, 1.40 mmol) were dissolved in acetonitrile (10 mL). Cesium carbonate (621 mg, 1.91 mmol) was then added to the reaction mixture, and the mixture was stirred at 70°C for 1 h. The reaction was monitored by LCMS until completion. Ice water (20 mL) was added to quench the reaction, and the mixture was extracted three times with ethyl acetate (50 mL). The organic phases were combined, dried over anhydrous sodium sulfate, subjected to suction filtration, and the filtrate was concentrated and purified by normal-phase silica gel column chromatography (EA/PE = 0 to 100%) to obtain 300 mg of the title compound **216-3** in 45.1% yield. LC-MS (ESI): m/z 518.7 $[M+H]^+$.

**Preparation of compound 216**

Compound **216-3** (100 mg, 0.19 mmol) and 3-(methylsulfonyl)azetidine hydrochloride (36 mg, 0.21 mmol) were dissolved in ethanol (2 mL), and triethylamine (77 mg, 0.76 mmol) was added. The system was purged with argon, heated to 100°C, and reacted in a sealed tube for 6 h. The reaction was monitored by LCMS until completion. The mixture was cooled to 0°C, quenched with saturated sodium chloride solution (3 mL), and extracted three times with ethyl acetate (5 mL). The organic phases were combined, dried over anhydrous sodium sulfate, subjected to suction filtration, and the filtrate was concentrated. The resulting residue was purified by preparative reversed-phase chromatography (ACN/0.1% FA $H_2O$ = 5% to 75%) to obtain 23.67 mg of the title compound **216.** LC-MS (ESI): m/z 618.3 $[M+H]^+$. $^1H$ NMR (400 MHz, DMSO-$d_6$) δ 8.46 - 8.42 (m, 2H), 8.39 (d, *J* = 2.2 Hz, 1H), 8.18 (d, *J* = 2.2 Hz, 1H), 8.16 (s, 1H), 7.94 (d, *J* = 8.5 Hz, 2H), 7.87 (d, *J* = 8.8 Hz, 1H), 7.53 (dd, *J* = 8.8, 3.0 Hz, 1H), 7.34 (d, *J* = 8.5 Hz, 2H), 6.90 (d, *J* = 5.0 Hz, 1H), 5.17 (s, 2H), 4.75 (t, *J* = 5.8 Hz, 2H), 4.44 - 4.38 (m, 1H), 4.34 (t, *J* = 8.6 Hz, 2H), 4.26 - 4.20 (m, 2H), 4.12 (t, *J* = 5.8 Hz, 2H), 3.06 (s, 3H), 1.78 (s, 6H).

**Example 217: Preparation of compound 217**

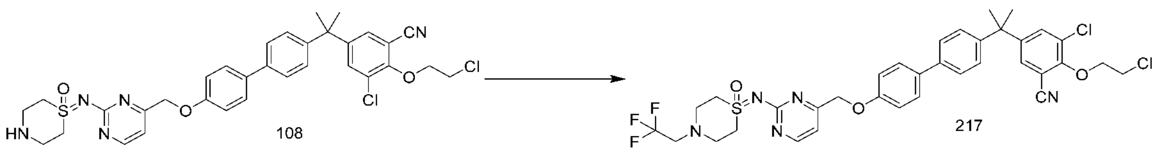

**Preparation of compound 217**

Compound **108** (100 mg, 0.153 mmol) and DIEA (100 mg, 0.768 mmol) were dissolved in DMF (2 mL). Trifluoroethyl trifluoromethanesulfonate (179 mg, 0.768 mmol) was added dropwise, and the reaction was carried out at room temperature for 1 h. The reaction was monitored by LCMS until completion. The reaction mixture was subjected to preparative purification (Waters 2767/QDA: column: Pursuit XRs C18, 21.2 × 250 mm, 10 μm; mobile phase: A: 0.1% FA in $H_2O$, B: ACN; flow rate: 20 mL/min; elution gradient: 77% to 87%; retention time: 8.6-10.0 min of 16 min) to obtain 19.56 mg of the title compound **217.** LC-MS (ESI): m/z 732.2 $[M+H]^+$. $^1H$ NMR (400 MHz, DMSO-$d_6$) δ 8.47 (d, *J* = 5.1 Hz, 1H), 7.70 (d, *J* = 2.3 Hz, 1H), 7.63 (d, *J* = 2.3 Hz, 1H), 7.60 (d, *J* = 8.8 Hz, 2H), 7.56 (d, *J* = 8.5 Hz, 2H), 7.30 (d, *J* = 8.5 Hz, 2H), 7.07 (d, *J* = 8.8 Hz, 2H), 7.01 (d, *J* = 5.1 Hz, 1H), 5.13 (s, 2H), 4.42 (t, *J* = 5.2 Hz, 2H), 3.96 (t, *J* = 5.2 Hz, 2H), 3.75 - 3.65 (m, 2H), 3.63 - 3.29 (m, 4H), 3.25 - 3.09 (m, 4H), 1.68 (s, 6H). $^{19}F$ NMR (376 MHz, DMSO-*d6*) δ -68.77 (s, 3F).

**Example 218: Preparation of compound 218**

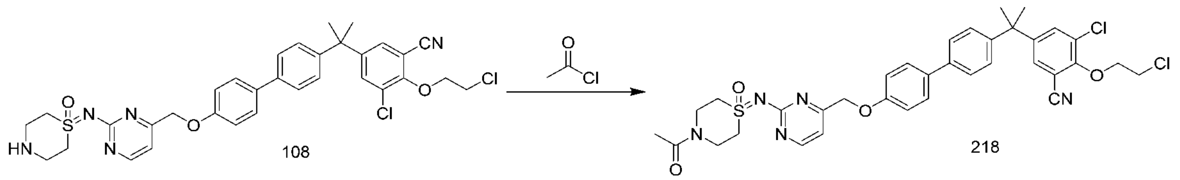

108 218

**Preparation of compound 218**

Compound **108** (100 mg, 0.15 mmol) and triethylamine (61 mg, 0.60 mmol) were dissolved in DCM (2 mL). The system was purged with argon, and then acetyl chloride (24 mg, 0.30 mmol) was added dropwise. The reaction was carried out at room temperature for half an hour. The reaction was monitored by LCMS until completion. The reaction mixture was concentrated, and the resulting residue was subjected to preparative purification (prep-HPLC: Waters 2767/QDA; column: Sunfire C18, 19 × 250 mm, 10 μm; mobile phase: A: 0.1% FA in $H_2O$, B: ACN; flow rate: 20 mL/min; elution gradient: 68-68%; retention time: 6.8-9.6 min of 16 min) to obtain 36.5 mg of the title compound **218.** LC-MS (ESI): m/z 692.3 $[M+H]^+$. $^1H$ NMR (400 MHz, DMSO-$d_6$) δ 8.47 (d, *J* = 5.1 Hz, 1H), 7.70 (d, *J* = 2.3 Hz, 1H), 7.63 (d, *J* = 2.3 Hz, 1H), 7.60 (d, *J* = 8.8 Hz, 2H), 7.56 (d, *J* = 8.5 Hz, 2H), 7.30 (d, *J* = 8.5 Hz, 2H), 7.08 (d, *J* = 8.8 Hz, 2H), 7.00 (d, *J* = 5.1 Hz, 1H), 5.12 (s, 2H), 4.42 (t, *J* = 5.2 Hz, 2H), 4.13 - 4.05 (m, 1H), 3.96 (t, *J* = 5.2 Hz, 2H), 3.95 - 3.91 (m, 1H), 3.80 - 3.59 (m, 5H), 3.47 - 3.40 (m, 1H), 2.06 (s, 3H), 1.68 (s, 6H).

**Example 219: Preparation of compound 219**

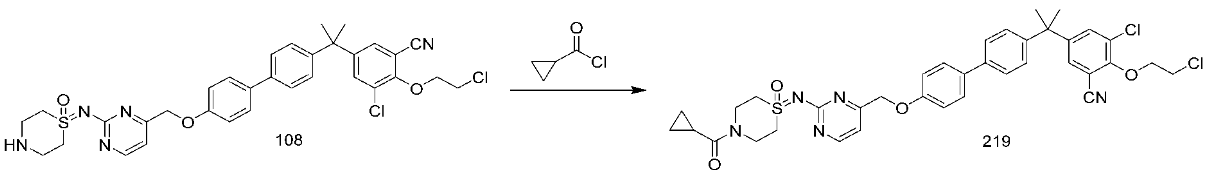

108 219

**Preparation of compound 219**

Compound **108** (60 mg, 0.092 mmol) and triethylamine (37 mg, 0.37 mmol) were dissolved in DCM (2 mL). The system was purged with argon, and then cyclopropanecarbonyl chloride (16 mg, 0.18 mmol) was added dropwise. The reaction was carried out at room temperature for 1 h. The reaction was monitored by LCMS until completion. The reaction mixture was concentrated, and the resulting residue was subjected to preparative purification (Waters 2767/QDA; column: Sunfire C18, 19 * 250 mm, 10 μm; mobile phase: A: 0.1% FA in $H_2O$, B: ACN; flow rate: 20 mL/min; gradient: 40 to 70 %; retention time: 9-11.4 min of 16 min) to obtain 57.8 mg of the title compound **219.** LC-MS (ESI): m/z 718.2 $[M+H]^+$. $^1H$ NMR (400 Hz, DMSO-$d_6$) δ 8.48 (d, *J* = 5.1 Hz, 1H), 7.70 (d, *J* = 2.3 Hz, 1H), 7.64 (d, *J* = 2.3 Hz, 1H), 7.61 (d, *J* = 8.8 Hz, 2H), 7.56 (d, *J* = 8.5 Hz, 2H), 7.30 (d, *J* = 8.5 Hz, 2H), 7.07 (d, *J* = 8.8 Hz, 2H), 7.02 (d, *J* = 5.1 Hz, 1H), 5.14 (s, 2H), 4.42 (t, *J* = 5.2 Hz, 2H), 4.29 - 4.02 (m, 2H), 3.96 (t, *J* = 5.2 Hz, 2H), 3.84 - 3.45 (m, 6H), 2.02 - 1.94 (m, 1H), 1.68 (s, 6H), 0.89 - 0.59 (m, 4H).

**Example 220: Preparation of compound 220**

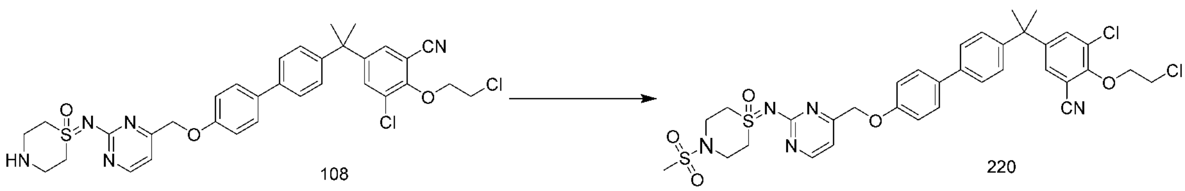

108 220

**Preparation of compound 220**

Compound **108** (100 mg, 0.153 mmol) and triethylamine (47 mg, 0.461 mmol) were dissolved in DCM (2 mL). The system was purged with argon, and MsCl (35 mg, 0.307 mmol) was added dropwise. The reaction was stirred at room temperature for 1 h. The reaction was monitored by LCMS until completion. The reaction mixture was concentrated, and the resulting residue was subjected to preparative purification (Waters 2767/QDA; column: Pursuit XRs C18, 21.2 * 250 mm, 10 μm; mobile phase: A: 0.1% FA in $H_2O$, B: ACN; flow rate: 20 mL/min; elution gradient: 72 to 82 %; retention time: 8.6-9.4 min of 16 min) to obtain 7.71 mg of the title compound **220.** LC-MS (ESI): m/z 728.4 $[M+H]^+$. $^1H$ NMR (400 MHz, DMSO-*d*$_6$) δ 8.48 (d, *J* = 5.1 Hz, 1H), 7.70 (d, *J* = 2.3 Hz, 1H), 7.64 (d, *J* = 2.3 Hz, 1H), 7.60 (d, *J* = 8.8 Hz, 2H), 7.56 (d, *J* = 8.4 Hz, 2H), 7.30 (d, *J* = 8.5 Hz, 2H), 7.08 (d, *J* = 8.8 Hz, 2H), 7.02 (d, *J* = 5.1 Hz, 1H), 5.12 (s, 2H), 4.42 (t, *J* = 5.2 Hz, 2H), 3.96 (t, *J* = 5.2 Hz, 2H), 3.90 - 3.82 (m, 2H), 3.81 - 3.72 (m, 2H), 3.71 - 3.61 (m, 2H), 3.61 - 3.51 (m, 2H), 3.04 (s, 3H), 1.68 (s, 6H).

**Example 221: Preparation of compound 221**

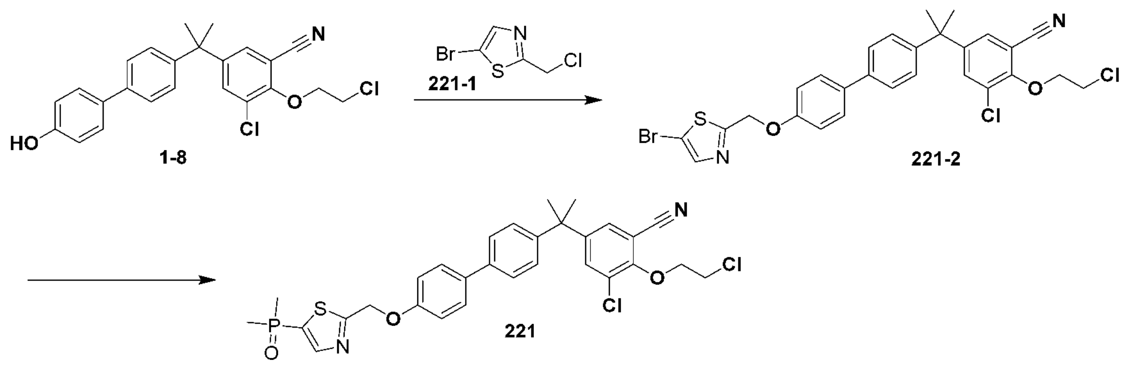


**Preparation of compound 221-2**

Compound **1-8** (200 mg, 469.12 μmol) and 5-bromo-2-chloromethylthiazole (100 mg, 469.12 μmol) were dissolved in acetonitrile (5 mL), and potassium carbonate (130 mg, 938.23 μmol) was added. The reaction mixture was stirred at 80°C for 12 h. LCMS indicated the disappearance of the starting material and the formation of the product. The reaction mixture was poured into water and extracted twice with ethyl acetate (30 mL). The organic phases were combined, washed with saturated brine, dried over anhydrous sodium sulfate, filtered, and the filtrate was subjected to rotary evaporation until dryness. The resulting residue was purified by silica gel column chromatography (EtOAc/PE = 0 to 35%) to obtain 260 mg of the title compound **221-2.** LC-MS (ESI): m/z 601.2 $[M+H]^+$.

**Preparation of compound 221**

Compound **221-2** (200 mg, 332.02 μmol), dimethylphosphine oxide (52 mg, 664.05 μmol), $Pd_2(dba)_3$ (611 mg, 66.40 μmol), Xantphos (77 mg, 132.81 μmol), and DIPEA (86 mg, 664.05 μmol) were dissolved in DMF (2 mL). The reaction system was stirred at 100°C for 16 h. After the reaction was completed, the reaction mixture was cooled to room temperature, and insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 66 mg of the title compound **221.** LC-MS (ESI): m/z 599.2 $[M+H]^+$. $^1H$ NMR (400 MHz, DMSO-*d*$_6$) δ 8.21 (d, *J* = 3.9 Hz, 1H), 7.71 (d, *J* = 2.3 Hz, 1H), 7.64 (d, *J* = 2.4 Hz, 1H), 7.63 (d, *J* = 8.8 Hz, 2H), 7.57 (d, *J* = 8.4 Hz, 2H), 7.31 (d, *J* = 8.5 Hz, 2H), 7.15 (d, *J* = 8.9 Hz, 2H), 5.56 (s, 2H), 4.42 (t, *J* = 5.2 Hz, 2H), 3.96 (t, *J* = 5.2 Hz, 2H), 1.78 (d, *J* = 13.9 Hz, 6H), 1.68 (s, 6H). $^{31}P$ NMR (162 MHz, DMSO-d$_6$) δ 27.71 (s, 1P).

**Example 222: Preparation of compound 222**

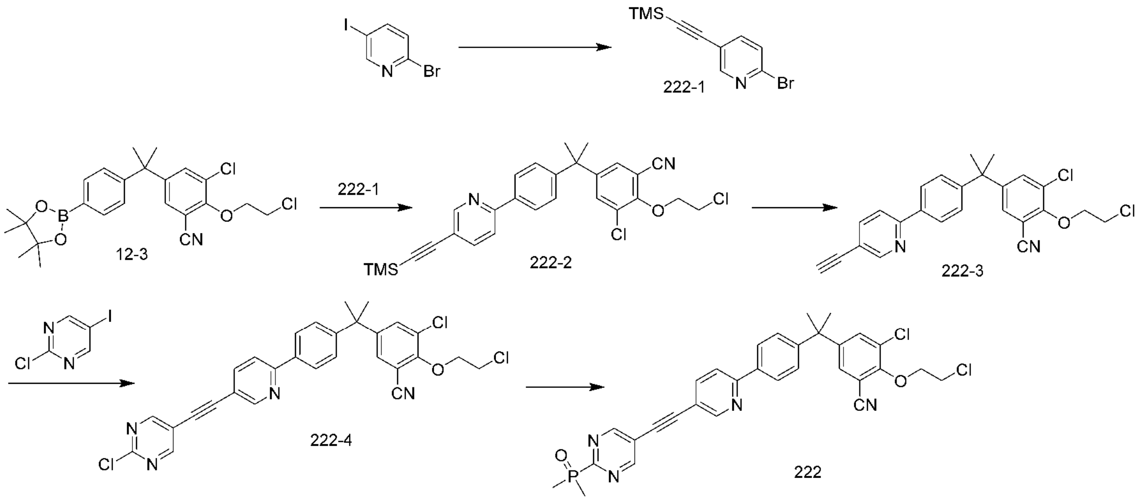
222-1
12-3
222-2
222-3
222-4
222

### Preparation of compound 222-1

Under a nitrogen atmosphere, 2-bromo-5-iodopyridine (3 g, 10.57 mmol), $Pd(PPh_3)_2Cl_2$ (1.48 g, 2.11 mmol), CuI (604 mg, 3.17 mmol), TEA (3.21 g, 31.70 mmol, 4.42 mL), trimethylsilylacetylene (1.14 g, 11.62 mmol) and THF (30 mL) were added to a sealed tube. The reaction system was stirred at 50°C in the sealed tube for 16 h. After the reaction was completed, the reaction mixture was subjected to rotary evaporation until dryness, and the crude product was purified by silica gel column chromatography (EA/PE = 0 to 100%) to obtain 1.49 g of the title compound **222-1.** LC-MS (ESI): m/z 254.0 $[M+H]^+$.

### Preparation of compound 222-2

Under a nitrogen atmosphere, compound **12-3** (905 mg, 1.97 mmol), compound **222-1** (0.5 g, 1.97 mmol), [1,1'-bis(diphenylphosphino)ferrocene]dichloropalladium(II) dichloromethane complex (144 mg, 196.70 μmol), $K_2CO_3$ (814 mg, 5.90 mmol), dioxane (10 mL) and $H_2O$ (2 mL) were added to a reaction flask. The system was purged three times with nitrogen, and the reaction was carried out at 100°C for 5 h. The mixture was subjected to rotary evaporation until dryness, and the crude product was purified by silica gel column chromatography (PE/EA = 100% to 90%) to obtain 250 mg of the title compound **222-2.** LC-MS (ESI): m/z 507.2 $[M+H]^+$.

### Preparation of compound 222-3

Compound **222-2** (250 mg, 492.59 μmol), KF (143 mg, 2.46 mmol) and methanol (20 mL) were added to a reaction flask, and the reaction was carried out at room temperature for 16 h. The mixture was subjected to rotary evaporation until dryness, and the crude product was purified by silica gel column chromatography (EA/PE = 0 to 20%) to obtain 200 mg of the title compound **222-3.** LC-MS (ESI): m/z 435.2 $[M+H]^+$.

### Preparation of compound 222-4

Compound **222-3** (200 mg, 459.41 μmol), 2-chloro-5-iodopyridine (165 mg, 689.11 μmol), bis(triphenylphosphine)palladium(II) chloride (64 mg, 91.88 μmol), CuI (109 mg, 137.82 μmol), TEA (465 mg, 4.59 mmol, 640.76 μL) and THF (10 mL) were added to a reaction flask. The system was purged three times with nitrogen, and the reaction was carried out at 50°C for 4 h. The mixture was subjected to rotary evaporation until dryness, and the crude product was purified by silica gel column chromatography (PE/EA = 100% to 50%) to obtain 240 mg of the title compound **222-4.** LC-MS (ESI): m/z 547.1 $[M+H]^+$.

### Preparation of compound 222

Compound **222-4** (120 mg, 219.03 μmol) was dissolved in DMF (1 mL), followed by the sequential addition of dimethylphosphine oxide (52 mg, 657.10 μmol), DIEA (85 mg, 657.10 μmol, 114.46 μL),$Pd_2(dba)_3$ (20 mg, 21.90 μmol) and Xantphos (25 mg, 43.81 μmol). The reaction system was purged three times with nitrogen and reacted under microwave irradiation at 100°C for 1 h. The mixture was filtered, and the crude product was subjected to preparative purification (preparative method: mobile phase: A: 0.1% formic acid in water; B: acetonitrile; chromatographic column:

Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound **222.** LC-MS (ESI): m/z 589.2 $[M+H]^+$. $^1H$ NMR (400 MHz, DMSO-$d_6$) δ 9.22 (s, 2H), 8.90 (d, *J* = 2.1 Hz, 1H), 8.25 - 7.96 (m, 2H), 8.10 (d, *J* = 8.6 Hz, 2H), 7.70 (d, *J* = 2.3 Hz, 1H), 7.63 (d, *J* = 2.3 Hz, 1H), 7.40 (d, *J* = 8.4 Hz, 2H), 4.42 (t, *J* = 5.2 Hz, 2H), 3.96 (t, *J* = 5.2 Hz, 2H), 1.79 (d, *J* = 13.8 Hz, 6H), 1.70 (s, 6H). $^{31}P$ NMR (162 MHz, DMSO-$d_6$) δ 34.61 (s, 1P).

**Example 223: Preparation of compound 223**

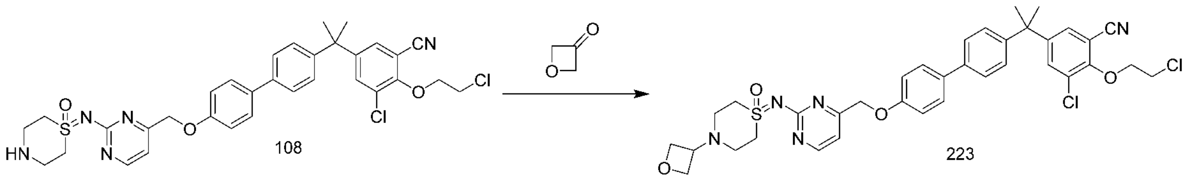

108

223

**Preparation of compound 223**

Compound **108** (100 mg, 0.15 mmol), compound oxetan-3-one (54 mg, 0.75 mmol), and sodium triacetoxyborohydride (160 mg, 0.75 mmol) were dissolved in dichloromethane (2 mL), and the system was reacted at room temperature for 2 h. The residue was concentrated and subjected to preparative purification (Waters 2767/Qda, column: Xbridge C18 19 * 250 mm, 10 μm; mobile phase A: 0.1% FA/$H_2O$, B: ACN; flow rate: 20 mL/min; elution gradient: 70% to 80%; retention time: 9.0-9.8 min of 16 min) to obtain 15.9 mg of the title compound **223.** LC-MS (ESI): m/z 706.3 $[M+H]^+$. $^1H$ NMR (400 MHz, DMSO-$d_6$) δ 8.45 (d, *J* = 5.0 Hz, 1H), 7.70 (d, *J* = 2.3 Hz, 1H), 7.63 (d, *J* = 2.3 Hz, 1H), 7.60 (d, *J* = 8.8 Hz, 2H), 7.56 (d, *J* = 8.4 Hz, 2H), 7.30 (d, *J* = 8.4 Hz, 2H), 7.06 (d, *J* = 8.8 Hz, 2H), 6.98 (d, *J* = 5.0 Hz, 1H), 5.12 (s, 2H), 4.50 (t, *J* = 6.6 Hz, 2H), 4.42 (t, *J* = 5.2 Hz, 2H), 4.38 (t, *J* = 6.1 Hz, 2H), 3.96 (t, *J* = 5.2 Hz, 2H), 3.74 - 3.60 (m, 3H), 3.56 - 3.43 (m, 2H), 2.86 - 2.73 (m, 2H), 2.72 - 2.61 (m, 2H), 1.68 (s, 6H).

**Example 224: Preparation of compound 224**

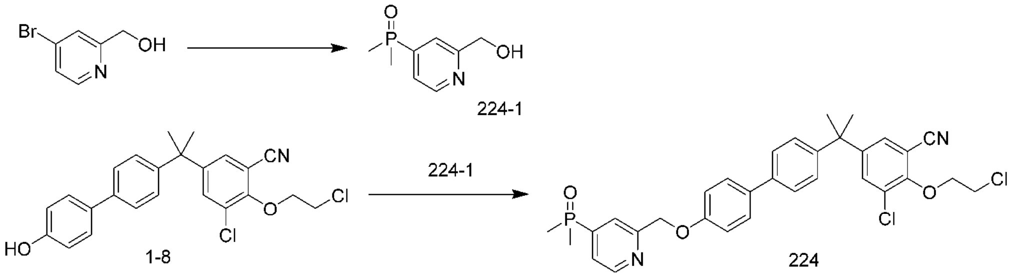


224-1

1-8

224

**Preparation of compound 224-1**

(4-Bromopyridin-2-yl)methanol (500 mg, 2.66 mmol) was dissolved in 1,4-dioxane (5 mL), followed by the sequential addition of compound dimethylphosphine oxide (623 mg, 7.98 mmol), DIPEA (1.03 g, 7.98 mmol), Xantphos (31 mg, 0.053 mmol), and $Pd_2(dba)_3$ (24 mg, 0.027 mmol). The reaction system was purged three times with nitrogen and stirred at 120°C for 2 h. After the reaction was completed, water (30 mL) was added, and the mixture was extracted three times with ethyl acetate (20 mL). The organic phases were combined, washed twice with saturated brine (20 mL), dried over anhydrous sodium sulfate, and subjected to suction filtration, and the filtrate was concentrated to obtain a crude product. The crude product was purified by silica gel column chromatography (DCM/MeOH = 100 to 90%) to obtain 400 mg of the title compound **224-2** in 81% yield. LC-MS (ESI): m/z 186.0 $[M+H]^+$.

**Preparation of compound 224**

Compound **224-1** (100 mg, 0.234 mmol) was dissolved in dichloromethane (5 mL), followed by the sequential addition of compound **1-8** (56 mg, 0.304 mmol) and DIAD (31 mg, 0.352 mmol). A solution of tributylphosphine (71 mg,

0.352 mmol) in dichloromethane (0.5 mL) was added dropwise to the reaction system at 0°C. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 100 mg of the title compound **224.** LC-MS (ESI): m/z 593.4 $[M+H]^+$. $^1H$ NMR (400 MHz, DMSO-$d_6$) δ 8.75 (t, *J* = 4.4 Hz, 1H), 7.91 (d, *J* = 11.5 Hz, 1H), 7.75 - 7.67 (m, 1H), 7.70 (d, *J* = 2.2 Hz, 1H), 7.64 (d, *J* = 2.3 Hz, 1H), 7.61 (d, *J* = 8.8 Hz, 2H), 7.56 (d, *J* = 8.4 Hz, 2H), 7.30 (d, *J* = 8.4 Hz, 2H), 7.14 (d, *J* = 8.8 Hz, 2H), 5.28 (s, 2H), 4.42 (t, *J* = 5.2 Hz, 2H), 3.95 (t, *J* = 5.2 Hz, 2H), 1.70 (d, *J* = 13.6 Hz, 6H), 1.68 (s, 6H). $^{31}P$ NMR (162 MHz, Chloroform-d) δ 32.46 (s, 1P).

**Example 225: Preparation of compound 225**

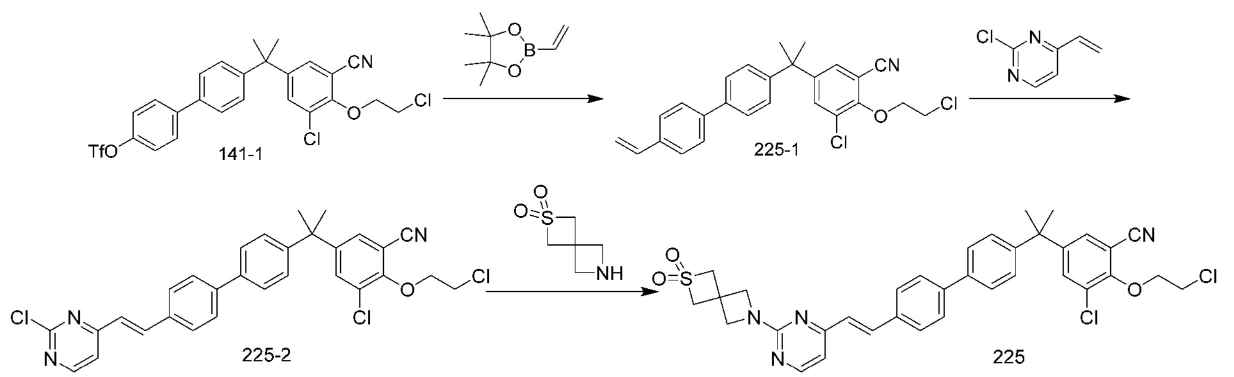

**Preparation of compound 225-1**

Compound **141-1** (1.4 g, 2.50 mmol) was dissolved in dioxane (12 mL) and water (3 mL). At 0°C, vinylboronic acid pinacol ester (1.35 g, 8.75 mmol), sodium carbonate (530 mg, 5.00 mmol), and Pd(dppf)$Cl_2$ (183 mg, 0.25 mmol) were added thereto. Under a nitrogen atmosphere, the reaction system was stirred at 100°C for 1 h. After the reaction was completed, saturated sodium chloride solution (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 80%) to obtain 0.82 g of the title compound **225-1.**

**Preparation of compound 225-2**

Compound **225-1** (300 mg, 0.69 mmol) and 2-chloro-4-vinylpyrimidine (241.6 mg, 1.72 mmol) were dissolved in dichloroethane (10 mL), and Hoveyda-Grubbs catalyst (86 mg, 0.14 mmol) was added thereto. After the addition was completed, the reaction system was stirred at 100°C for 12 h under $N_2$ atmosphere. After the reaction was completed, the reaction mixture was filtered, and the filtrate was concentrated to obtain a crude product. The crude product was purified by silica gel column chromatography (PE/EA = 100% to 80%) to obtain 160 mg of the title compound **225-2.** LC-MS (ESI): m/z 548.2 $[M+H]^+$.

**Preparation of compound 225**

Compound **225-2** (15 mg, 0.03 mmol), 2-thia-6-azaspiro[3.3]heptane 2,2-dioxide hydrochloride (7.5 mg, 0.04 mmol), and DIPEA (8.5 mg, 0.08 mmol) were dissolved in EtOH (1 mL). The reaction system was stirred at 100°C for 2 h. After the reaction was completed, the reaction mixture was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5 mg of the title compound **225.** LC-MS (ESI): m/z 659.2 $[M+H]^+$.

**Example 226: Preparation of compound 226**

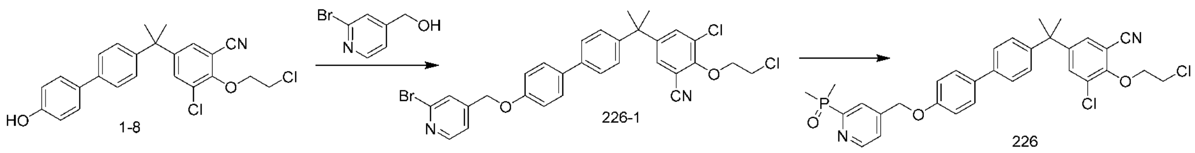

**Preparation of compound 226-1**

Compound **1-8** (226 mg, 530.10 μmol) and (2-bromopyridin-4-yl)methanol (109.6 mg, 583.11 μmol) were dissolved in THF (5 mL). Triphenylphosphine (278.1 mg, 1.06 mmol) was added, and the reaction system was purged three times with a nitrogen balloon. The mixture was cooled to 0°C in an ice-water bath, and DIAD (214.4 mg, 1.06 mmol, 210 μL) was added slowly. The reaction mixture was gradually warmed to room temperature and stirred overnight. LC-MS monitoring indicated that a significant amount of starting material remained unreacted. Additional (2-bromopyridin-4-yl) methanol (109.6 mg, 583.11 μmol), triphenylphosphine (278.1 mg, 1.06 mmol), and DIAD (214.4 mg, 1.06 mmol, 210 μL) were added, and the mixture was heated to 60°C and stirred for 4.5 h. After the reaction was completed, the reaction mixture was diluted with ethyl acetate, adsorbed onto silica gel, and concentrated to dryness by rotary evaporation. The crude product was purified by silica gel column chromatography (PE/EA = 4:1) to obtain 240 mg of the title compound **226-1.** LC-MS (ESI): m/z 595.2 $[M+H]^+$.

**Preparation of compound 226**

The crude compound **226-1** (240 mg, 402.45 μmol) and dimethylphosphine oxide (94.2 mg, 1.21 mmol) were dissolved in 1,4-dioxane (4 mL). $Pd_2(dba)_3$ (37 mg, 40.25 μmol), Xantphos (47 mg, 80.49 μmol), and DIPEA (156 mg, 1.21 mmol, 210 μL) were added separately. The mixture was bubbled with nitrogen for 1 min and stirred under microwave irradiation at 110°C for 2 h in a sealed tube. After the reaction was completed, the reaction mixture was filtered through a membrane filter, diluted with ethyl acetate, adsorbed onto silica gel, and concentrated to dryness by rotary evaporation. The resulting crude product was purified by silica gel column chromatography (DCM/MeOH = 94:6), and then further purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 70 to 90% 8 min; flow rate: 30 mL/min) to obtain 18.2 mg of the title compound **226.** LC-MS (ESI): m/z 593.2 $[M+H]^+$. $^1H$ NMR (400 MHz, Chloroform-*d*) δ 8.78 (d, *J* = 5.2 Hz, 1H), 8.32 (d, *J* = 5.7 Hz, 1H), 7.69 (d, *J* = 4.9 Hz, 1H), 7.54 (d, *J* = 8.7 Hz, 2H), 7.49 (d, *J* = 8.4 Hz, 2H), 7. 49 (d, *J* = 2.4 Hz, 1H), 7.37 (d,*J* = 2.3 Hz, 1H), 7.23 (d, *J* = 8.4 Hz, 2H), 7.04 (d, *J* = 8.7 Hz, 2H), 5.24 (s, 2H), 4.43 (t, *J* = 6.2 Hz, 2H), 3.88 (t, *J* = 6.2 Hz, 2H), 1.91 (d, *J* = 13.5 Hz, 6H), 1.70 (s, 6H). $^{31}P$ NMR (162 MHz, Chloroform-*d*) δ 36.17 (s, 1P).

**Example 227: Preparation of compound 227**

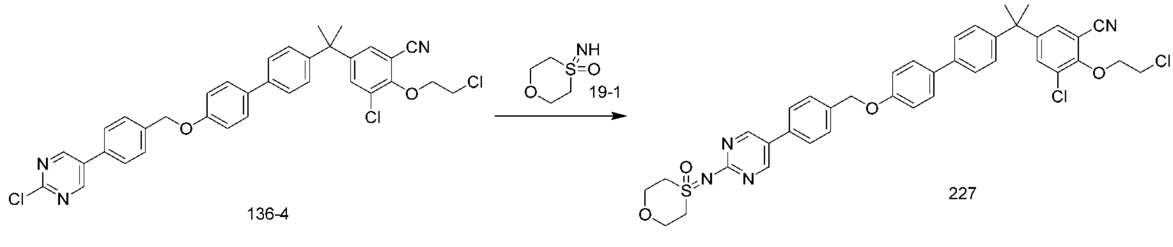

**Preparation of compound 227**

Compound **136-4** (60 mg, 0.10 mmol), compound **19-1** (19.4 mg, 0.14 mmol), DIEA (61.5 mg, 0.48 mmol), X-phox (11.0 mg, 0.02 mmol), and $Pd_2(dba)_3$ (8.7 mg, 0.01 mmol) were dissolved in 1,4-dioxane (3 mL). The reaction system was purged three times with nitrogen and stirred in an oil bath at 120°C for 16 h. After the reaction was completed, the reaction mixture was filtered through diatomite, and the filter cake was rinsed with ethyl acetate (50 mL). The filtrates were

combined, water (50 mL) was added, and the mixture was extracted three times with ethyl acetate (50 mL). The organic phases were combined, washed once with saturated brine (50 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 7.2 mg of the title compound 227. LC-MS (ESI): m/z 727.0 $[M+H]^+$. $^1H$ NMR (400 MHz, DMSO-$d_6$) δ 8.81 (s, 2H), 7.76 - 7.68 (m, 3H), 7.64 (d, *J* = 2.3 Hz, 1H), 7.62 - 7.51 (m, 6H), 7.30 (d, *J* = 8.4 Hz, 2H), 7.10 (d, *J* = 8.9 Hz, 2H), 5.20 (s, 2H), 4.42 (t, *J* = 5.2 Hz, 2H), 4.17 - 4.05 (m, 2H), 4.02 - 3.91 (m, 4H), 3.90 - 3.79 (m, 2H), 3.69 - 3.56 (m, 2H), 1.68 (s, 6H).

**Example 228: Preparation of compound 228**

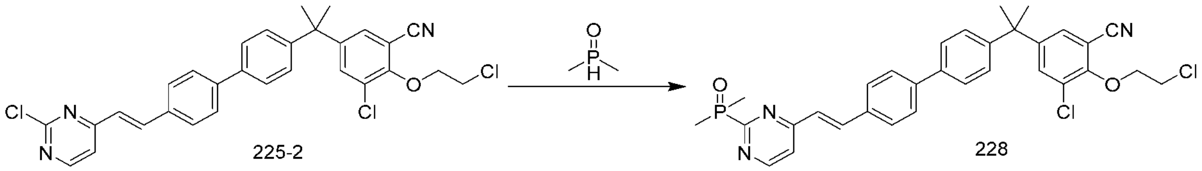
225-2
228

**Preparation of compound 228**

Compound **225-2** (27.4 mg, 0.05 mmol), dimethylphosphine oxide (19.5 mg, 0.25 mmol), $Pd_2(dba)_3$ (4.6 mg, 0.005 mmol), Xantphos (27 mg, 0.01 mmol), and DIPEA (32.3 mg, 0.25 mmol) were dissolved in DMF (2 mL). Under a nitrogen atmosphere, the reaction system was stirred at 120°C for 2 h in a microwave reactor. After the reaction was completed, insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 11 mg of compound **228.** LC-MS (ESI): m/z 590.2 $[M+H]^+$. $^1H$ NMR (400 MHz, Chloroform-*d*) δ 8.78 (s, 1H), 8.02 (d, *J* = 15.8 Hz, 1H), 7.74 - 7.61 (m, 4H), 7.58 (d, *J* = 7.8 Hz, 2H), 7.49 (s, 1H), 7.44 - 7.34 (m, 3H), 7.22 - 7.07 (m, 2H), 4.43 (t, *J* = 6.3 Hz, 2H), 3.88 (t, *J* = 6.2 Hz, 2H), 1.93 (d, *J* = 13.4 Hz, 6H), 1.71 (s, 6H). $^{31}P$ NMR (162 MHz, Chloroform-*d*) δ 34.73 (s, 1P).

**Example 229: Preparation of compound 229**

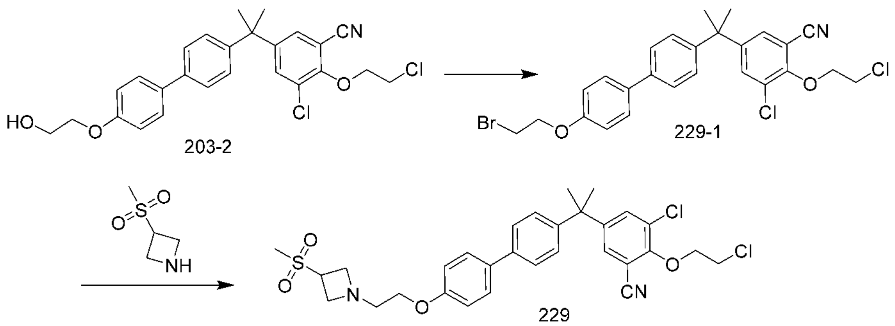
203-2
229-1
229

**Preparation of compound 229-1**

Compound **203-2** (300 mg, 0.63 mmol) and carbon tetrabromide (635 mg, 1.91 mmol) were dissolved in dichloromethane (10 mL). Triphenylphosphine (502 mg, 1.91 mmol) was added thereto at 0°C. After the addition was completed, the reaction system was stirred at 25°C for 16 h. After the reaction was completed, the reaction mixture was concentrated and purified by silica gel column chromatography (PE/DCM = 100% to 5%) to obtain 200 mg of the title compound **229-1.** $^1H$ NMR (400 MHz, Chloroform-d) δ 7.55 - 7.45 (m, 5H), 7.38 (d, *J* = 2.4 Hz, 1H), 7.25 - 7.20 (m, 2H), 7.01 - 6.96 (m, 2H), 4.43 (t, *J* = 6.2 Hz, 2H), 4.34 (t, *J* = 6.3 Hz, 2H), 3.88 (t, *J* = 6.2 Hz, 2H), 3.67 (t, *J* = 6.3 Hz, 2H), 1.69 (s, 6H).

**Preparation of compound 229**

Compound **229-1** (30 mg, 0.056 mmol) and potassium carbonate (39 mg, 0.28 mmol) were dissolved in *N*,*N*-dimethylformamide (5 mL), and 3-(methylsulfonyl)azetidine (22 mg, 0.16 mmol) was added thereto. After the addition was completed, the reaction system was stirred at 80°C for 1 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 4 mg of the title compound **229.** LC-MS (ESI): m/z 587.6 $[M+H]^+$.

$^1$H NMR (400 MHz, DMSO-$d_6$) δ 7.70 (d, *J* = 2.4 Hz, 1H), 7.63 (d, *J* = 2.3 Hz, 1H), 7.56 (t, *J* = 8.8 Hz, 4H), 7.30 (d, *J* = 8.0 Hz, 2H), 6.99 (d, *J* = 8.4 Hz, 2H), 4.42 (t, *J* = 5.0 Hz, 2H), 4.20 - 4.11 (m, 1H), 4.05 - 3.89 (m, 4H), 3.59 (t, *J* = 8.1 Hz, 2H), 3.46 (t, *J* = 7.2 Hz, 2H), 2.94 (s, 3H), 2.80 (t, *J* = 5.3 Hz, 2H), 1.68 (s, 6H).

**Example 230: Preparation of compound 230**

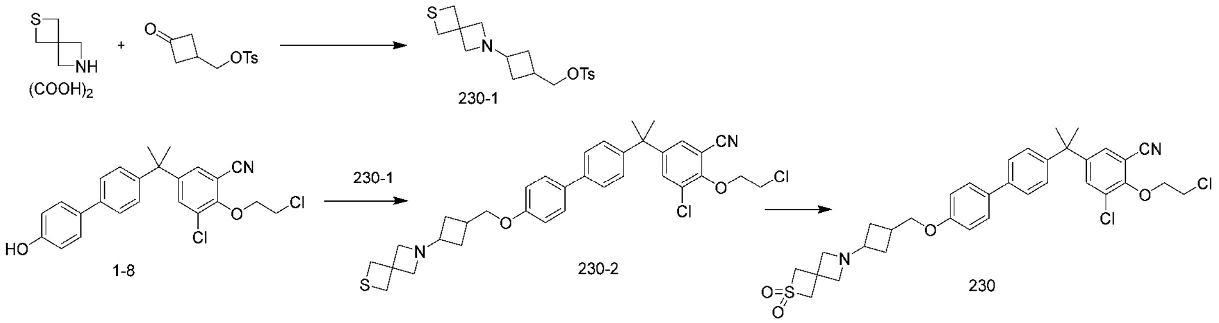

$(COOH)_2$ 230-1 1-8 230-2 230

**Preparation of compound 230-1**

(3-Oxocyclobutyl)methyl 4-methylbenzenesulfonate (300 mg, 1.18 mmol) was dissolved in DCE (8 mL). Compound 2-thia-6-azaspiro[3.3]heptane oxalate (283.5 mg, 884.78 μmol) and acetic acid (14.2 mg, 235.94 μmol) were added. The reaction mixture was stirred at room temperature for half an hour, then sodium triacetoxyborohydride (750.1 mg, 3.54 mmol) was added, and the mixture was stirred at room temperature overnight. Water and ethyl acetate were added to the reaction mixture. The organic phase was separated, washed with saturated brine, and the aqueous phase was extracted once with dichloromethane. The organic phases were combined and dried over anhydrous sodium sulfate. The mixture was filtered, concentrated to dryness by rotary evaporation, and dried under reduced pressure with an oil pump to obtain 240 mg of a crude product of the title compound **230-1,** which was used directly in the next step. LC-MS (ESI): m/z 354.2 $[M+H]^+$.

**Preparation of compound 230-2**

Compound **230-1** (240.3 mg, 679.78 μmol) was dissolved in DMF (5 mL), and compound **1-8** (231.9 mg, 543.82 μmol) and cesium carbonate (332.2 mg, 1.02 mmol) were added separately. The reaction mixture was stirred at 70°C overnight. The mixture was cooled to room temperature. Saturated aqueous ammonium chloride solution, water, and ethyl acetate were added. The organic phase was separated, washed with saturated brine, dried over anhydrous sodium sulfate, filtered, and concentrated to dryness by rotary evaporation. The crude product was purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 30 to 50% over 8 min, 50 to 70% over 2 min; flow rate: 30 mL/min) to obtain 35 mg of the title compound **230-2.** LC-MS (ESI): m/z 607.4 $[M+H]^+$.

**Preparation of compound 230**

Compound **230-2** (12 mg, 19.75 μmol) was dissolved in DCM (1 mL), cooled to 0°C in an ice-water bath, and

*m*-CPBA (9.6 mg, 47.4 μmol, 85% purity) was added. The mixture was allowed to warm to room temperature naturally and stirred for 25 min. A small amount of aqueous sodium carbonate solution and water were added to the reaction mixture. The mixture was extracted with dichloromethane. The organic phase was separated and dried over anhydrous sodium sulfate. The mixture was filtered, concentrated to dryness by rotary evaporation, and purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 20 to 40% over 8 min, 40 to 50% over 4 min, held at 50% for 2 min; flow rate: 30 mL/min) to obtain 1.4 mg of the title compound **230.** LC-MS (ESI): m/z 639.2 [M+H]+.

**Example 231: Preparation of compound 231**

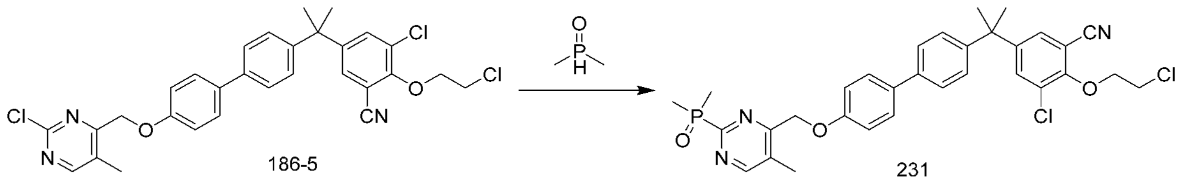

**Preparation of compound 231**

Compound **186-5** (100 mg, 176.40 μmol) and dimethylphosphine oxide (41.3 mg, 529.19 μmol) were dissolved in 1,4-dioxane (3 mL). $Pd_2(dba)_3$ (16.2 mg, 17.64 μmol), Xantphos (20.4 mg, 35.28 μmol), and DIPEA (68.4 mg, 529.19 μmol, 92 μL) were added separately. The mixture was bubbled with nitrogen for 1 min and stirred under microwave irradiation at 120°C for 2 h in a sealed tube. After the reaction was completed, the reaction mixture was filtered through a membrane filter and purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 70 to 95% 8 min; flow rate: 30 mL/min) to obtain 56 mg of the title compound **231.** LC-MS (ESI): m/z 608.8 [M+H]+. $^1$H NMR (400 MHz, Chloroform-*d*) δ 8.69 (s, 1H), 7.51 (d, *J* = 8.8 Hz, 2H), 7.48 (d, *J* = 2.3 Hz, 1H), 7.47 (d, *J* = 8.4 Hz, 2H), 7.37 (d, *J* = 2.3 Hz, 1H), 7.22 (d, *J* = 8.4 Hz, 2H), 7.04 (d, *J* = 8.8 Hz, 2H), 5.30 (s, 2H), 4.42 (t, *J* = 6.2 Hz, 2H), 3.88 (t, *J* = 6.2 Hz, 2H), 2.49 (s, 3H), 1.87 (d, *J* = 13.6 Hz, 6H), 1.69 (s, 6H). $^{31}$P NMR (162 MHz, Chloroform-d) δ 35.35 (s, 1P).

**Example 232: Preparation of compound 232**

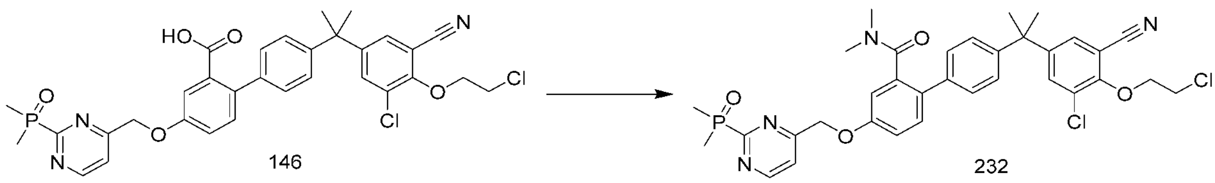

**Preparation of compound 232**

Compound **146** (100 mg, 0.16 mmol), dimethylamine hydrochloride (26 mg, 0.32 mmol), and DIPEA (62 mg, 0.48 mmol) were dissolved in DMF (5 mL), and HATU (122 mg, 0.32 mmol) was added thereto. The reaction system was stirred at 25°C for 1 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 4.62 mg of the title compound **232.** LC-MS (ESI): m/z 665.3 [M+H]+. $^1$H NMR (400 MHz, Chloroform-*d*) δ 8.89 (s, 1H), 7.67 (s, 1H), 7.42 (d, *J* = 2.4 Hz, 1H), 7.40 - 7.33 (m, 3H), 7.29 (d, *J* = 2.4 Hz, 1H), 7.21 (d, *J* = 8.4 Hz, 2H), 7.11 - 7.00 (m, 2H), 5.30 (s, 2H), 4.43 (t, *J* = 6.1 Hz, 2H), 3.88 (t, *J* = 6.1 Hz, 2H), 2.85 (s, 3H), 2.43 (s, 3H), 1.91 (d, *J* = 13.5 Hz, 6H), 1.69 (s, 6H). $^{31}$P NMR (162 MHz, Chloroform-d) δ 35.75 (s, 1P).

**Example 233: Preparation of compound 233**

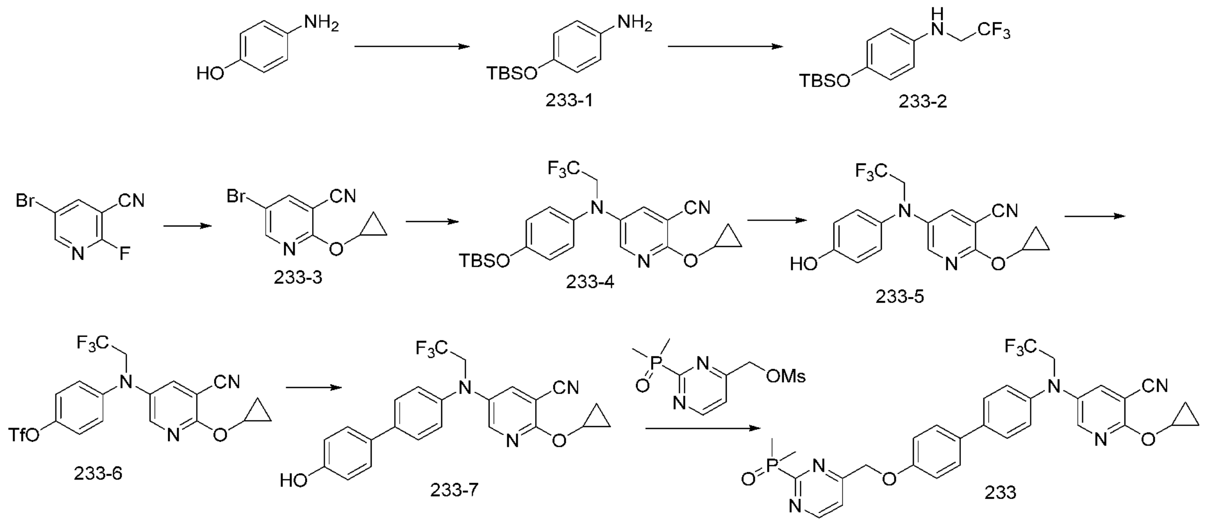
233-1
233-2
233-3
233-4
233-5
233-6
233-7
233

**Preparation of compound 233-1**

4-Aminophenol (2 g, 18.33 mmol) was dissolved in dichloromethane (20 mL), followed by the sequential addition of imidazole (2.5 g, 36.66 mmol) and TBSCl (2.93 g, 19.43 mmol). The reaction system was purged three times with nitrogen and stirred at room temperature for 2 h. After the reaction was completed, water (30 mL) was added, and the mixture was extracted three times with dichloromethane (50 mL). The organic phases were combined, washed twice with saturated brine (30 mL), dried over anhydrous sodium sulfate, subjected to suction filtration, and the filtrate was concentrated to obtain a crude product. The crude product was purified by silica gel column chromatography (EA/PE = 0 to 10%) to obtain 3.5 g of the title compound **233-1** in 85% yield. $^1$H NMR (400 MHz, Chloroform-*d*) δ 6.68 - 6.63 (m, 2H), 6.61 - 6.56 (m, 2H), 3.39 (s, 2H), 0.96 (s, 9H), 0.15 (s, 6H).

**Preparation of compound 233-2**

Compound **233-1** (2 g, 8.95 mmol) was dissolved in tetrahydrofuran (20 mL), followed by the sequential addition of TEA (2.72 g, 26.86 mmol) and 2,2,2-trifluoroethyl trifluoromethanesulfonate (6.23 g, 26.86 mmol). The reaction system was purged three times with nitrogen and stirred at 80°C for 24 h. After the reaction was completed, water (30 mL) was added, and the mixture was extracted three times with ethyl acetate (50 mL). The organic phases were combined, washed twice with saturated brine (30 mL), dried over anhydrous sodium sulfate, subjected to suction filtration, and the filtrate was concentrated to obtain a crude product. The crude product was purified by silica gel column chromatography (EA/PE = 0 to 10%) to obtain 2.1 g of the title compound **233-2** in 76% yield. LC-MS (ESI): m/z 306.2 $[M+H]^+$.

**Preparation of compound 233-3**

Cyclopropanol (1.1 g, 18.91 mmol) was dissolved in tetrahydrofuran (20 mL), and sodium hydride (0.756 g, 49.91 mmol, 60% in oil) was added thereto at 0°C. The mixture was stirred at this temperature for half an hour, and a solution of 5-bromo-2-fluoro-3-cyanopyridine (1.9 g, 9.45 mmol) in tetrahydrofuran (10 mL) was added dropwise. After the addition was completed, the reaction system was stirred at 25°C for 0.5 h. After the reaction was completed, saturated ammonium chloride solution (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 85%) to obtain 2 g of the title compound **233-3.** LC-MS (ESI): m/z 239.0 $[M+H]^+$.

**Preparation of compound 233-4**

Compound **233-3** (420 mg, 1.38 mmol) was dissolved in toluene (10 mL), followed by the sequential addition of compound **233-2** (394 mg, 1.65 mmol), sodium *tert*-butoxide (158 mg, 1.65 mmol), tri-*tert*-butylphosphine (56 mg, 0.0275

mmol), and $Pd_2(dba)_3$ (126 mg, 0.138 mmol). The reaction system was purged three times with nitrogen and stirred at room temperature for 12 h. After the reaction was completed, water (10 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 200 mg of the title compound **233-4.** LC-MS (ESI): m/z 464.4 $[M+H]^+$.

**Preparation of compound 233-5**

Compound **233-4** (0.23 g, 0.496 mmol) was dissolved in tetrahydrofuran (5 mL), and TBAF (259 mg, 0.496 mmol) was added. The reaction system was purged three times with nitrogen and stirred at room temperature for 2 h. After the reaction was completed, water (30 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by column chromatography (EA/PE = 0 to 40%) to obtain 160 mg of the title compound **233-5** in 92% yield. LC-MS (ESI): m/z 350.2 $[M+H]^+$.

**Preparation of compound 233-6**

Compound **233-5** (160 mg, 0.458 mmol) was dissolved in dichloromethane (5 mL), followed by the sequential addition of compound TEA (46 mg, 0.485 mmol) and N-phenylbis(trifluoromethanesulfonyl)imide (163 mg, 0.458 mmol). The reaction system was purged three times with nitrogen and stirred at room temperature for 2 h. After the reaction was completed, water (30 mL) was added, and the mixture was extracted three times with dichloromethane (50 mL). The organic phases were combined, washed twice with saturated brine (30 mL), dried over anhydrous sodium sulfate, subjected to suction filtration, and the filtrate was concentrated to obtain a crude product. The crude product was purified by silica gel column chromatography (EA/PE = 0 to 30%) to obtain 110 mg of the title compound **233-6** in 49% yield. LC-MS (ESI): m/z 482.2 $[M+H]^+$.

**Preparation of compound 233-7**

Compound **233-6** (100 mg, 0.207 mmol) was dissolved in 1,4-dioxane (3 mL) and water (1 mL), followed by the sequential addition of compound 4-hydroxyphenylboronic acid (37 mg, 0.27 mmol), potassium carbonate (29 mg, 0.207 mmol), and $Pd(dppf)Cl_2$ dichloromethane complex (17 mg, 0.0207 mmol). The reaction system was purged three times with nitrogen and stirred at 91°C for 2 h. After the reaction was completed, water (30 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 50%) to obtain 45 mg of the title compound **233-7** in 51% yield. LC-MS (ESI): m/z 426.2 $[M+H]^+$.

**Preparation of compound 233**

Compound **233-7** (45 mg, 0.106 mmol) was dissolved in acetonitrile (2 mL), followed by the sequential addition of potassium carbonate (22 mg, 0.158 mmol) and (2-(dimethoxyphosphoryl)pyrimidin-4-yl)methyl methanesulfonate (36 mg, 0.137 mmol). The reaction system was stirred at 70°C for 0.5 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 27 mg of the title compound **233.** LC-MS (ESI): m/z 594.2 $[M+H]^+$. $^1H$ NMR (400 MHz, DMSO-$d_6$) δ 9.00 (d, *J* = 5.2 Hz, 1H), 8.33 (d, *J* = 2.9 Hz, 1H), 8.19 (d, *J* = 2.9 Hz, 1H), 7.73 (dd, *J* = 5.2, 3.2 Hz, 1H), 7.58 (d, *J* = 8.7 Hz, 2H), 7.55 (d, *J* = 8.7 Hz, 2H), 7.13 (d, *J* = 8.8 Hz, 2H), 7.01 (d, *J* = 8.7 Hz, 2H), 5.34 (s, 2H), 4.69 (q, *J* = 9.2 Hz, 2H), 4.42 - 4.35 (m, 1H), 1.77 (d, *J* = 13.7 Hz, 6H), 0.84 - 0.76 (m, 4H). $^{31}P$ NMR (162 MHz, Chloroform-*d*) δ 34.06 (s, 1P). $^{19}F$ NMR (376 MHz, DMSO-$d_6$) δ -68.40 (d, 3F).

**Example 234: Preparation of compound 234**

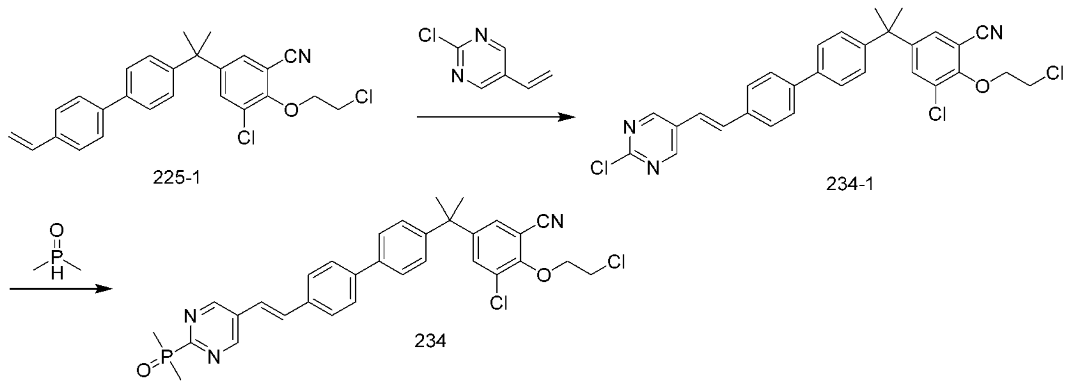
225-1
234-1
234

**Preparation of compound 234-1**

Compound **225-1** (300 mg, 0.69 mmol) and 2-chloro-5-vinylpyrimidine (241.6 mg, 1.72 mmol) were dissolved in dichloroethane (10 mL), and Hoveyda-Grubbs catalyst (86 mg, 0.14 mmol) was added thereto. After the addition was completed, the reaction system was purged with nitrogen and stirred at 100°C for 12 h. After the reaction was completed, the reaction mixture was filtered, and the filtrate was concentrated to obtain a crude product. The crude product was purified by silica gel column chromatography (PE/EA = 100% to 80%) to obtain 160 mg of the title compound **234-1.** LC-MS (ESI): m/z 548.2 $[M+H]^+$.

**Preparation of compound 234**

Compound **225-2** (27.4 mg, 0.05 mmol), dimethylphosphine oxide (19.5 mg, 0.25 mmol), $Pd_2(dba)_3$ (4.6 mg, 0.005 mmol), Xantphos (27 mg, 0.01 mmol), and DIPEA (32.3 mg, 0.25 mmol) were dissolved in DMF (2 mL). Under a nitrogen atmosphere, the reaction system was stirred at 120°C for 2 h in a microwave reactor. After the reaction was completed, the reaction mixture was cooled to room temperature, and insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 16 mg of compound **234.** LC-MS (ESI): m/z 590.2 $[M+H]^+$. $^1H$ NMR (400 MHz, Chloroform-*d*) δ 8.99 (s, 2H), 7.64 (s, 4H), 7.58 (d, *J* = 8.4 Hz, 2H), 7.49 (d, *J* = 2.3 Hz, 1H), 7.48 - 7.32 (m, 2H), 7.27 (d, *J* = 7.8 Hz, 2H), 7.12 - 7.02 (m, 1H), 4.43 (t, *J*= 6.1 Hz, 2H), 3.88 (t, *J* = 6.1 Hz, 2H), 1.90 (d, *J*= 13.5 Hz, 6H), 1.71 (s, 6H). $^{31}P$ NMR (162 MHz, Chloroform-*d*) δ 34.73 (s, 1P).

**Example 235: Preparation of compound 235**

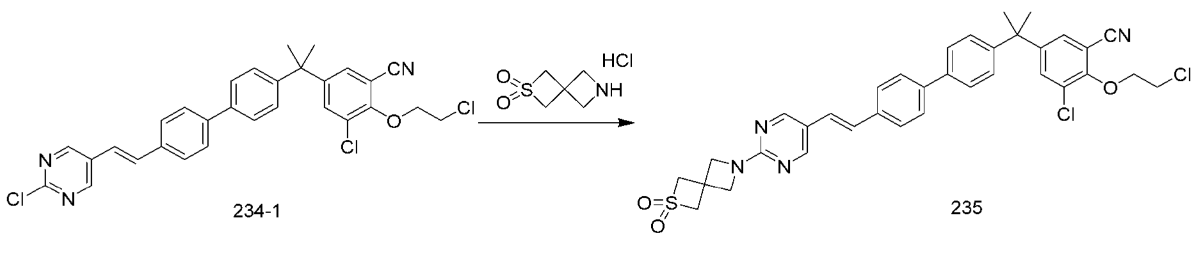

234-1
235

**Preparation of compound 235**

Compound **234-1** (27.4 mg, 0.05 mmol), 2-thia-6-azaspiro[3.3]heptane 2,2-dioxide hydrochloride (23 mg, 0.13 mmol), and DIPEA (32.3 mg, 0.25 mmol) were dissolved in EtOH (2 mL). The reaction system was stirred at 100°C for 2 h. After the reaction was completed, the reaction mixture was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 10 mg of the title compound **235.** LC-MS (ESI): m/z 659.2 $[M+H]^+$. $^1H$ NMR (400 MHz, Chloroform-*d*) δ 8.54 (s, 2H), 7.63 - 7.54 (m, 6H), 7.49 (d, *J* = 2.4 Hz, 1H), 7.38 (d, *J* = 2.3 Hz, 1H), 7.25 (s, 2H), 7.04 (d, *J* = 16.2 Hz, 1H), 6.93 (d, *J* = 16.2 Hz, 1H), 4.56 - 4.31 (m, 10H), 3.88 (t, *J* = 6.1 Hz, 2H), 1.71 (s,

6H).

**Example 236: Preparation of compound 236**

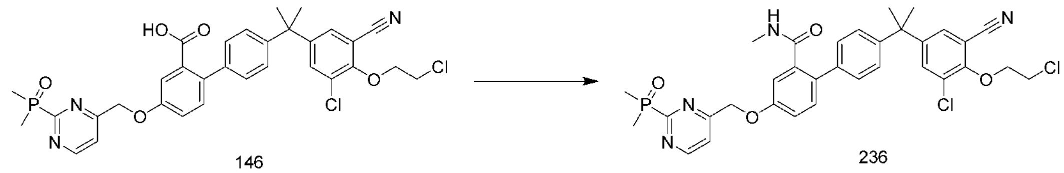

146 236

**Preparation of compound 236**

Compound **146** (100 mg, 0.16 mmol), methylamine hydrochloride (22 mg, 0.32 mmol), and DIPEA (62 mg, 0.48 mmol) were dissolved in DMF (5 mL), and HATU (122 mg, 0.32 mmol) was added thereto. The reaction system was stirred at 25°C for 1 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 4.62 mg of the title compound **236.** LC-MS (ESI): m/z 651.2 $[M+H]^+$. $^1H$ NMR (400 MHz, Chloroform-*d*) δ 8.91 (s, 1H), 7.68 (s, 1H), 7.44 (d, *J* = 2.3 Hz, 1H), 7.38 - 7.27 (m, 5H), 7.22 (d, *J* = 8.0 Hz, 2H), 7.09 (d, *J* = 8.4 Hz, 1H), 5.31 (s, 2H), 4.43 (t, *J* = 6.1 Hz, 2H), 3.88 (t, *J* = 6.1 Hz, 2H), 2.68 (s, 3H), 1.91 (d, *J* = 13.3 Hz, 6H), 1.69 (s, 6H). $^{31}P$ NMR (162 MHz, Chloroform-*d*) δ 33.19 (s, 1P).

**Example 237: Preparation of compound 237**

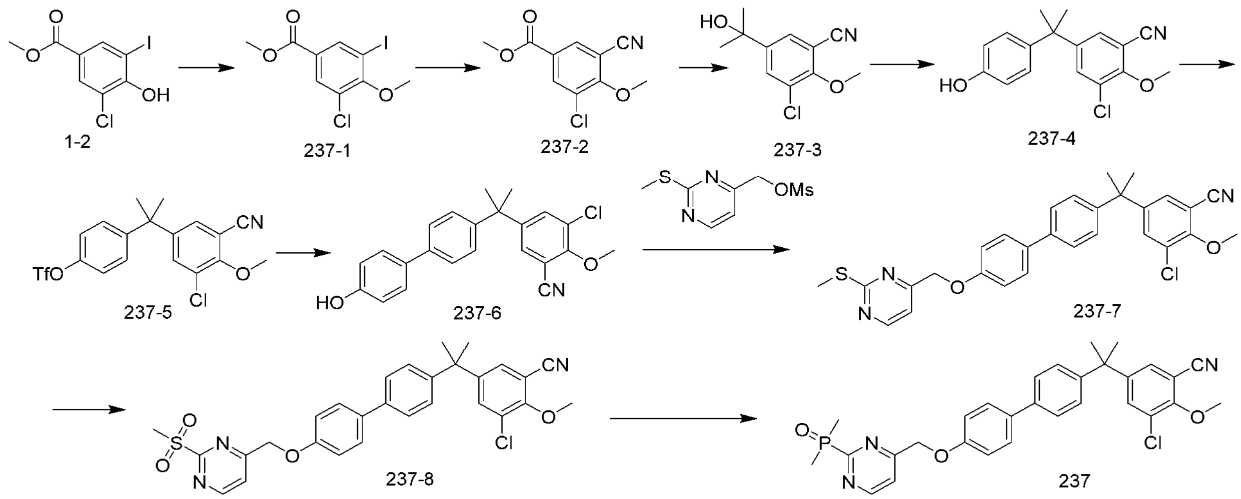

**Preparation of compound 237-1**

Compound **1-2** (5 g, 16 mmol) and cesium carbonate (10.43 g, 32 mmol) were dissolved in DMF (50 mL), and iodomethane (1.5 mL, 24 mmol) was added thereto. After the addition was completed, the system was stirred at room temperature for 16 h. Ice water (100 mL) was added to the system to quench the reaction, and then the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by normal-phase silica gel column chromatography (EtOAc/PE = 0 to 10%) to obtain 4.2 g of the title compound **237-1** in 80% yield.

**Preparation of compound 237-2**

Compound **237-1** (4.2 g, 12.9 mmol) was dissolved in DMF (50 mL), and copper(I) cyanide (2.3 g, 25.8 mmol) was added thereto. After the addition was completed, the system was stirred at 120°C for 16 h. The system was diluted with water (50 mL) and extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by normal-phase silica gel column chromatography (EtOAc/PE (v/v) = 0 to 10%) to obtain 2.5 g of the title compound **237-2** in 86% yield.

**Preparation of compound 237-3**

Compound **237-2** (2.5 g, 11.1 mmol) was dissolved in tetrahydrofuran (50 mL). At -78°C, methylmagnesium bromide (3.0 M, 11.1 mL) was added thereto. After the addition was completed, the system was stirred at room temperature for 2 h. Saturated ammonium chloride solution (50 mL) was added to the reaction system. After the reaction was quenched, the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by normal-phase silica gel column chromatography (EtOAc/PE (v/v) = 0 to 25%) to obtain 2.3 g of the title compound **237-3** in 92% yield.

**Preparation of compound 237-4**

Compound **237-3** (2.3 g, 10.2 mmol) and phenol (1.2 g, 12.2 mmol) were dissolved in dichloromethane (50 mL). At 0°C, boron trifluoride diethyl etherate (48%, 2.68 mL) was added. After the addition was completed, the system was stirred at room temperature for 2 h. Water (50 mL) was added to the system to quench the reaction, and then the mixture was extracted three times with ethyl acetate (50 mL). The organic phases were combined, washed three times with saturated brine (50 mL), dried over anhydrous sodium sulfate, filtered, and the filtrate was concentrated to obtain a crude product. The crude product was purified by normal-phase silica gel column chromatography (EtOAc/PE (v/v) = 0 to 25%) to obtain the title compound **237-4** in 49% yield. LC-MS (ESI): 300.2 $[M-H]^-$.

**Preparation of compound 237-5**

Compound **237-4** (8 g, 26.49 mmol) and N-phenylbis(trifluoromethanesulfonyl)imide (14.18 g, 39.73 mmol) were dissolved in DCM (100 mL), and triethylamine (5.35 g, 52.98 mmol) was added thereto. After the addition was completed, the reaction system was stirred at 25°C for 2 h. After the reaction was completed, water (100 mL) was added, and the mixture was extracted three times with dichloromethane (100 mL). The organic phases were combined, washed twice with saturated brine (100 mL), dried over anhydrous sodium sulfate, filtered, and the filtrate was concentrated to obtain a crude product. The crude product was purified by silica gel column chromatography (PE/EA = 100% to 90%) to obtain 1.2 g of the title compound **237-5.**

**Preparation of compound 237-6**

Compound **237-5** (8 g, 18.43 mmol), 4-hydroxyphenylboronic acid (3.81 g, 27.65 mmol), and potassium carbonate (5.12 g, 36.86 mmol) were dissolved in 1,4-dioxane (50 mL) and water (10 mL), and $Pd(dppf)Cl_2$ (1.35 g, 1.84 mmol) was added thereto. After the addition was completed, the reaction system was stirred at 80°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 75%) to obtain 5 g of the title compound **237-6.** LC-MS (ESI): m/z 378.0 $[M+H]^+$.

**Preparation of compound 237-7**

Compound **237-6** (5 g, 13.2 mmol) and cesium carbonate (8.6 g, 26.52 mmol) were dissolved in acetonitrile (50 mL), and (2-(methylthio)pyrimidin-4-yl)methyl methanesulfonate (4.7 g, 19.89 mmol) was added thereto. After the addition was completed, the reaction system was stirred at 80°C for 2 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 70%) to obtain 5 g of the title compound **237-7.** LC-MS (ESI): m/z 516.2 $[M+H]^+$.

**Preparation of compound 237-8**

Compound **237-7** (5 g, 9.69 mmol) was dissolved in dichloromethane (50 mL), and m-chloroperoxybenzoic acid (5 g, 29.37 mmol) was added thereto. After the addition was completed, the reaction system was stirred at 25°C for 1 h. After the reaction was completed, saturated sodium carbonate solution (50 mL) was added, and the mixture was extracted three times with dichloromethane (50 mL). The organic phases were combined, washed twice with saturated sodium carbonate solution (50 mL), dried over anhydrous sodium sulfate, filtered, and the filtrate was concentrated to obtain a crude product. The crude product was purified by silica gel column chromatography (PE/EA = 100% to 70%) to obtain 5 g of the title compound **237-8.** LC-MS (ESI): m/z 548.2 $[M+H]^+$.

**Preparation of compound 237**

Compound **237-8** (100 mg, 0.18 mmol), dimethylphosphine oxide (43 mg, 0.54 mmol), and potassium carbonate (79 mg, 0.54 mmol) were dissolved in DMF (5 mL). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 37 mg of the title compound **237.** LC-MS (ESI): m/z 546.2 $[M+H]^+$. $^1H$ NMR (400 MHz, Chloroform-*d*) δ 8.88 (s, 1H), 7.69 (m, 1H), 7.55 (d, *J* = 8.7 Hz, 2H), 7.49 (d, *J* = 8.4 Hz, 2H), 7.47 (d, *J* = 2.4 Hz, 1H), 7.36 (d, *J* = 2.3 Hz, 1H), 7.23 (d, *J* = 8.4 Hz, 2H), 7.04 (d, *J* = 8.8 Hz, 2H), 5.30 (s, 2H), 4.05 (s, 3H), 1.91 (d, *J* = 13.6 Hz, 6H), 1.69 (s, 6H). $^{31}P$ NMR (162 MHz, Chloroform-*d*) δ 35.20 (s, 1P).

**Example 238: Preparation of compound 238**

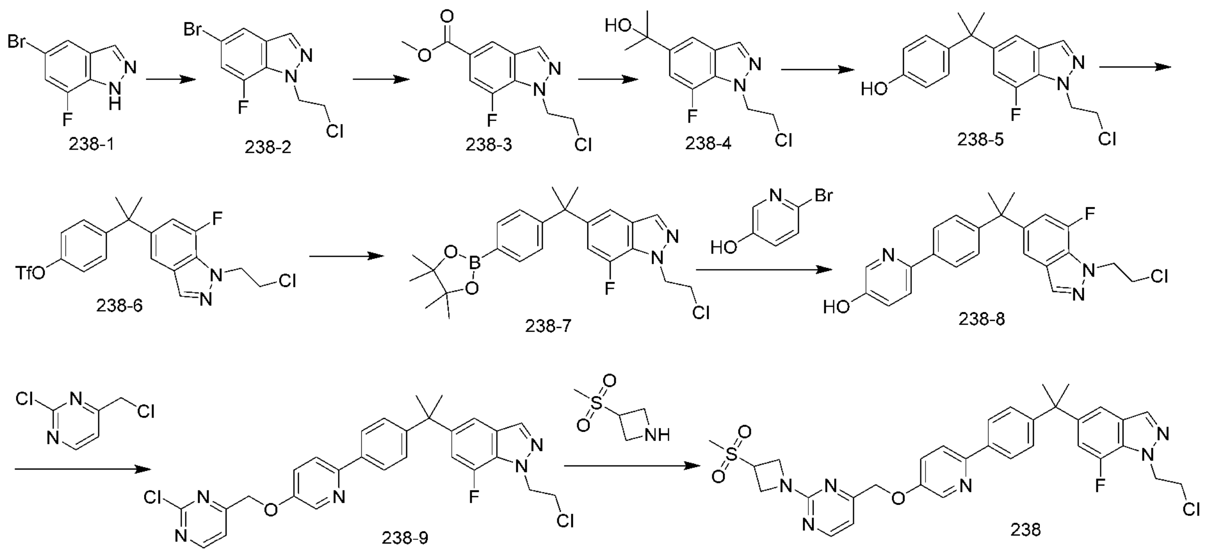


**Preparation of compound 238-2**

5-Bromo-7-fluoroindazole (9.5 g, 44.18 mmol) and 1-bromo-2-chloroethane (7.6 g, 53.02 mmol, 4.4 mL) were weighed and dissolved in acetonitrile (100 mL). Cesium carbonate (28.8 g, 88.36 mmol) was added. The reaction mixture was reacted at 80°C for 12 h. LCMS monitoring indicated the disappearance of the starting material and the formation of the product. The reaction mixture was cooled to room temperature, subjected to rotary evaporation until dryness, and purified by column chromatography (EtOAc/PE = 0 to 30%) to obtain 5 g of the title compound **238-2.** LC-MS (ESI): m/z 277.0 $[M+H]^+$.

## Preparation of compound 238-3

**238-2** (9.2 g, 33.15 mmol) was dissolved in methanol (100 mL). Triethylamine (13.8 mL) and Pd(dppf)$Cl_2$ (2.43 g, 3.31 mmol) were added at room temperature. The gas in the reaction mixture was replaced with carbon monoxide gas, and the mixture was stirred at 70°C for 16 h. The reaction was monitored by LCMS until completion. The reaction mixture was concentrated and purified by silica gel column chromatography (EA/PE = 0% to 20%) to obtain 4.3 g of the title compound **238-3** in 50.54% yield. LC-MS (ESI): m/z 257.0 $[M+H]^+$. $^1$H NMR (400 MHz, $CDCl_3$) δ 8.29 (d, *J* = 1.0 Hz, 1H), 8.16 (d, *J* = 2.1 Hz, 1H), 7.74 (dd, *J* = 12.5, 1.1 Hz, 1H), 4.87 (t, *J* = 6.3 Hz, 2H), 3.98 (t, *J* = 6.3 Hz, 2H), 3.95 (s, 3H).

## Preparation of compound 238-4

Under a nitrogen atmosphere, methylmagnesium bromide (26 mL, 77.92 mmol, 3 M) was added dropwise to a solution of compound **238-3** (4 g, 15.58 mmol) in tetrahydrofuran (30 mL) at 0°C. The reaction mixture was stirred at 0°C for 10 min. The reaction was monitored using a TLC plate (PE:EA = 3:1) until completion. The reaction mixture was quenched with saturated $Na_2CO_3$ solution and extracted twice with EtOAc (50 mL). The organic phases were combined, washed with saturated brine, dried over anhydrous sodium sulfate, filtered, and subjected to rotary evaporation until dryness. The residue was purified by an automated column chromatography system (PE/EA = 100 to 20%) to obtain 3.72 g of the title compound **238-4.** LC-MS (ESI): m/z 257.2 $[M+H]^+$.

## Preparation of compound 238-5

Under a nitrogen atmosphere at 0°C, boron trifluoride diethyl etherate (6.39 g, 21.62 mmol, 5.56 mL, 48% by mass) was added to a solution of compound **238-4** (3.7 g, 14.41 mmol) and phenol (1.63 g, 17.30 mmol) in 1,2-dichloroethane (30 mL). The reaction mixture was stirred at 0°C for 1 h. LCMS indicated the disappearance of the starting material and the formation of the product. The reaction mixture was poured into saturated ammonium chloride solution. The aqueous phase was extracted three times with dichloromethane (30 mL). The combined organic phases were washed with saturated brine, dried over anhydrous sodium sulfate, filtered, and subjected to rotary evaporation until dryness. The resulting residue was purified by column chromatography (EtOAc/PE = 0 to 15%) to obtain 3 g of the title compound **238-5.** LC-MS (ESI): m/z 333.2 $[M+H]^+$.

## Preparation of compound 238-6

Compound **238-5** (3 g, 9.01 mmol) and triethylamine (2.74 g, 27.04 mmol, 3.77 mL) were dissolved in dichloromethane (18 mL). After the system was purged three times with nitrogen, *N*-phenylbis(trifluoromethanesulfonyl)imide (3.86 g, 10.82 mmol, 2.3 mL) was added at 0°C. The reaction mixture was stirred at room temperature for 1 h. LCMS indicated the disappearance of the starting material and the formation of the product. The reaction mixture was subjected to rotary evaporation until dryness and purified by an automated column chromatography system (EtOAc/PE = 0 to 5%) to obtain 2.5 g of the title compound **238-6.** LC-MS (ESI): m/z 465.2 $[M+H]^+$.

## Preparation of compound 238-7

Compound **238-6** (300 mg, 677.57 μmol) and bis(pinacolato)diboron (123.8 mg, 711.45 μmol) were dissolved in dioxane (5 mL) and water (2.5 mL). Pd(dppf)$Cl_2$ (49.6 mg, 67.76 μmol) and potassium carbonate (187.3 mg, 1.36 mmol) were added. The system was purged three times with nitrogen, and the reaction mixture was heated at 100°C for 12 h. LCMS indicated the disappearance of the starting material and the formation of the product. The reaction mixture was subjected to rotary evaporation until dryness and purified by column chromatography (EtOAc/PE = 0 to 60%) to obtain 150 mg of the title compound **238-7.**

## Preparation of compound 238-8

Compound **238-7** (300 mg, 677.57 μmol) and 6-bromo-3-hydroxypyridine (123.8 mg, 711.45 μmol) were dissolved in 1,4-dioxane (5 mL) and water (2.5 mL). Pd(dppf)$Cl_2$ (49.6 mg, 67.76 μmol) and potassium carbonate (187.3 mg, 1.36 mmol) were added. The system was purged three times with nitrogen, and the reaction mixture was heated at 100°C for 12 h. After the reaction was complete, the reaction mixture was subjected to rotary evaporation until dryness and purified by column chromatography (EA/PE = 0 to 60%) to obtain 150 mg of the title compound **238-8** in 54% yield.

**Preparation of compound 238-9**

Compound **238-8** and 2-chloro-4-(chloromethyl)pyrimidine (65.6 mg, 402.55 μmol) were weighed and dissolved in acetonitrile (3 mL), and potassium carbonate (151.7 mg, 1.10 mmol) was added. The reaction mixture was stirred at 85°C for 12 h. LCMS indicated the disappearance of the starting material. The product was formed. The reaction mixture was used directly in the next step without purification. LC-MS (ESI): m/z 536.4 $[M+H]^+$.

**Preparation of compound 238**

Compound **238-9** (200 mg, 372.84 μmol) and 3-methylsulfonyl-azetidine (151.2 mg, 1.12 mmol) were dissolved in acetonitrile (3 mL), and TEA (226.4 mg, 2.24 mmol, 312.01 μL) was added. The reaction mixture was heated at 80°C for 6 h. LCMS indicated the disappearance of the starting material and the formation of the product. The reaction mixture was filtered and then subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 70.8 mg of the title compound **238.** LC-MS (ESI): m/z 635.3 $[M+H]^+$. $^1$H NMR (400 MHz, DMSO-$d_6$) δ 8.48 - 8.40 (m, 2H), 8.18 (d, *J* = 2.2 Hz, 1H), 7.92 (d, *J* = 8.5 Hz, 2H), 7.87 (d, *J* = 8.8 Hz, 1H), 7.58 - 7.49 (m, 2H), 7.32 (d, *J* = 8.5 Hz, 2H), 6.98 (dd, *J* = 14.1, 1.4 Hz, 1H), 6.90 (d, *J* = 5.0 Hz, 1H), 5.17 (s, 2H), 4.76 (t, *J* = 5.7 Hz, 2H), 4.47 - 4.36 (m, 1H), 4.34 (t, *J* = 8.7 Hz, 2H), 4.27 - 4.19 (m, 2H), 4.05 (t, *J* = 5.6 Hz, 2H), 3.06 (s, 3H), 1.72 (s, 6H). $^{19}$F NMR (376 MHz, DMSO-d6) δ -132.65 (s, 1F).

**Example 239: Preparation of compound 239**

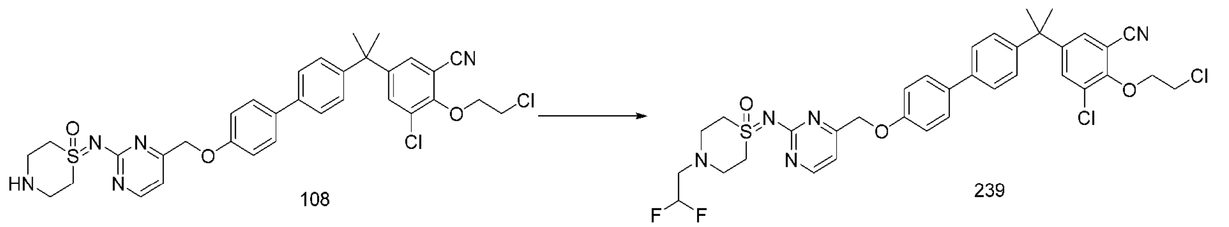

108

239

**Preparation of compound 239**

Compound **108** (100.00 mg, 0.153 mmol) and TEA (76 mg, 0.75 mmol) were dissolved in DCM (2 mL). Subsequently, 2,2-difluoroethyl trifluoromethanesulfonate (88.2 mg, 0.45 mmol) was added dropwise, and the reaction was carried out at room temperature for 5 h. LCMS monitoring indicated product formation. The reaction mixture was subjected to rotary evaporation until dryness and subjected to preparative purification (Waters 2767/QDA: column: Pursuit XRs C18, 21.2 × 250 mm, 10 μm; mobile phase: A: 0.1% FA in $H_2O$, B: ACN; flow rate: 20 mL/min; elution gradient: 75% to 85%; retention time: 8.0-9.8 min of 16 min) to obtain 3.6 mg of the title compound **239** in 3.5% yield. LC-MS (ESI): m/z 714.2 $[M+H]^+$. $^1$H NMR (400 MHz, Methanol-$d_4$) δ 8.44 (d, *J* = 5.2 Hz, 1H), 7.56 (d, *J* = 8.7 Hz, 2H), 7.56 (d, *J* = 2.3 Hz, 1H), 7.53 (d, *J* = 8.4 Hz, 2H), 7.53 (d, *J* = 2.4 Hz, 1H), 7.29 (d, *J* = 8.4 Hz, 2H), 7.09 (d, *J* = 5.2 Hz, 1H), 7.06 (d, *J* = 8.7 Hz, 2H), 5.93 (tt, *J* = 55.8, 4.1 Hz, 1H), 5.13 (s, 2H), 4.43 (t, *J*= 5.6 Hz, 2H), 3.90 (t, *J* = 5.6 Hz, 2H), 3.79 - 3.67 (m, 2H), 3.54 - 3.46 (m, 2H), 3.25 - 3.10 (m, 4H), 2.94 (td, *J* = 15.1, 4.2 Hz, 2H), 1.71 (s, 6H). $^{19}$F NMR (376 MHz, Methanol-$d_4$) δ -121.60 (s, 2F).

**Example 240: Preparation of compound 240**

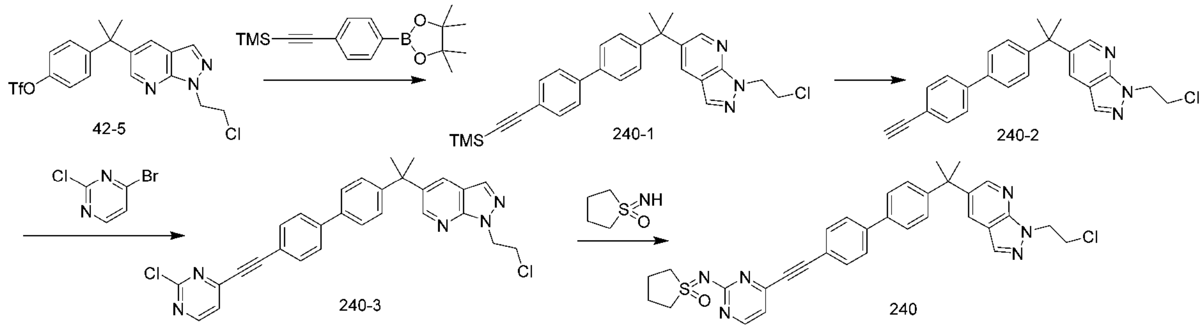


**Preparation of compound 240-1**

Compound **42-5** (1 g, 2.09 mmol) and 4-[(trimethylsilyl)ethynyl]phenylboronic acid pinacol ester (754 mg, 2.51 mmol) was dissolved in 1,4-dioxane (10 mL), followed by the sequential addition of cesium carbonate (1.69 g, 5.2 mmol) and [1,1'- bis(diphenylphosphino)ferrocene]dichloropalladium(II) dichloromethane complex (170 mg, 209 μmol). After the system was purged three times with nitrogen, the mixture was stirred at 105°C for 5 h. After the reaction was completed, the reaction system was filtered. Saturated aqueous sodium chloride solution (10 mL) was added to the filtrate, and the mixture was extracted three times with ethyl acetate (10 mL). The organic phases were combined, dried, and concentrated to obtain a crude product. The crude product was purified by silica gel column chromatography (EA/PE = 0 to 40%) to obtain720 mg of the title compound **240-1.** LC-MS (ESI): 472.3[M+H]$^+$.

**Preparation of compound 240-2**

Compound **240-1**(720 mg, 1.53 mmol) and potassium fluoride (164 mg, 2.82 mmol) were dissolved in ethanol (8 mL). The reaction system was purged three times with nitrogen and stirred at 50°C for 10 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 20%) to obtain 510 mg of the title compound **240-2.** LC-MS (ESI): m/z 400.2 [M+H]$^+$.

**Preparation of compound 240-3**

Under a nitrogen atmosphere, compound **240-2** (150 mg, 375.08 μmol), 4-bromo-2-chloropyrimidine (79.8 mg, 412.59 μmol), bis(triphenylphosphine)palladium(II) chloride (26.3 mg, 37.51 μmol), copper(I) iodide (7.1 mg, 37.51 μmol), and triethylamine (759.1 mg, 7.50 mmol, 1.05 mL) were added to acetonitrile (3 mL). The reaction mixture was stirred at 50°C for 12 h. LCMS indicated the disappearance of the starting material and the formation of the product. The reaction mixture was subjected to rotary evaporation until dryness and then purified by column chromatography (EtOAc/PE = 0 to 40%) to obtain 180 mg of the title compound **240-3.** LC-MS (ESI): m/z 512.2 [M+H]$^+$.

**Preparation of compound 240**

Compound **240-3** (180 mg, 351.27 μmol) and 1-iminotetrahydrothiophene 1-oxide (62.8 mg, 526.90 μmol) were dissolved in DMF (2 mL). $Pd_2(dba)_3$ (32.2 mg, 35.13 μmol), Xantphos (40.7 mg, 70.25 μmol), and DIPEA (136.2 mg, 1.05 mmol, 183.55 μL) were added. The system was purged three times with nitrogen, and the reaction mixture was heated under microwave irradiation at 120°C for 2 h. LCMS indicated the disappearance of the starting material and the formation of the product. The reaction mixture was cooled to room temperature and subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 16.6 mg of the title compound **240.** LC-MS (ESI): m/z 595.2 [M+H]$^+$. $^1$H NMR (400 MHz, DMSO-$d_6$) δ 8.52 (d, *J* = 5.0 Hz, 1H), 8.41 (d, *J* = 2.2 Hz, 1H), 8.20 (d, *J* = 2.3 Hz, 1H), 8.17 (s, 1H), 7.78 (d, *J* = 8.5 Hz, 2H), 7.71 (d, *J* = 8.4 Hz, 2H), 7.68 (d, *J* = 8.5 Hz, 2H), 7.38 (d, *J* = 8.5 Hz, 2H), 7.12 (d, *J* = 5.0 Hz, 1H), 4.75 (t, *J* = 5.8 Hz, 2H), 4.12 (t, *J* = 5.8 Hz, 2H), 3.66 - 3.55 (m, 2H), 3.44 - 3.38 (m, 2H), 2.30 - 2.18 (m, 2H), 2.16 - 2.05 (m, 2H), 1.79 (s, 6H).

**Example 241: Preparation of compound 241**

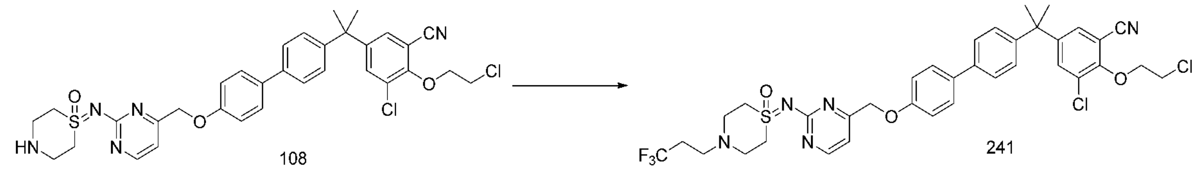


**Preparation of compound 241**

Compound **108** (100 mg, 0.15 mmol), 3,3,3-trifluoropropanal (84 mg, 0.75 mmol), and sodium triacetoxyborohydride (160 mg, 0.75 mmol) were dissolved in dichloromethane (2 mL), and the system was reacted at room temperature for 2 h. The mixture was concentrated, and the resulting residue was subjected to preparative purification (Waters 2767/Qda, column: Xbridge C18 19 * 250 mm, 10 μm; mobile phase: A: 0.1% FA/$H_2O$, B: ACN; flow rate: 20 mL/min; elution gradient: 79% to 89%; retention time: 8.8-9.8 min of 16 min) to obtain 8.27 mg of the title compound **241.** LC-MS (ESI): m/z 746.4 $[M+H]^+$. $^1H$ NMR (400 MHz, DMSO-$d_6$) δ 8.45 (d, *J* = 5.0 Hz, 1H), 7.70 (d, *J* = 2.2 Hz, 1H), 7.63 (d, *J* = 2.2 Hz, 1H), 7.60 (d, *J* = 8.7 Hz, 2H), 7.56 (d, *J* = 8.3 Hz, 2H), 7.30 (d, *J* = 8.3 Hz, 2H), 7.07 (d, *J* = 8.7 Hz, 2H), 6.98 (d, *J* = 5.0 Hz, 1H), 5.12 (s, 2H), 4.42 (t, *J* = 5.2 Hz, 2H), 3.96 (t, *J* = 5.2 Hz, 2H), 3.72 - 3.59 (m, 2H), 3.53 - 3.40 (m, 2H), 3.08 - 2.95 (m, 2H), 2.94 - 2.83 (m, 2H), 2.72 (t, *J* = 8 Hz, 2H), 2.49 - 2.37 (m, 2H), 1.68 (s, 6H). $^{19}F$ NMR (376 MHz, DMSO-d6) δ -63.50 (s, 3F).

**Example 242: Preparation of compounds 242, 242A, and 242B**

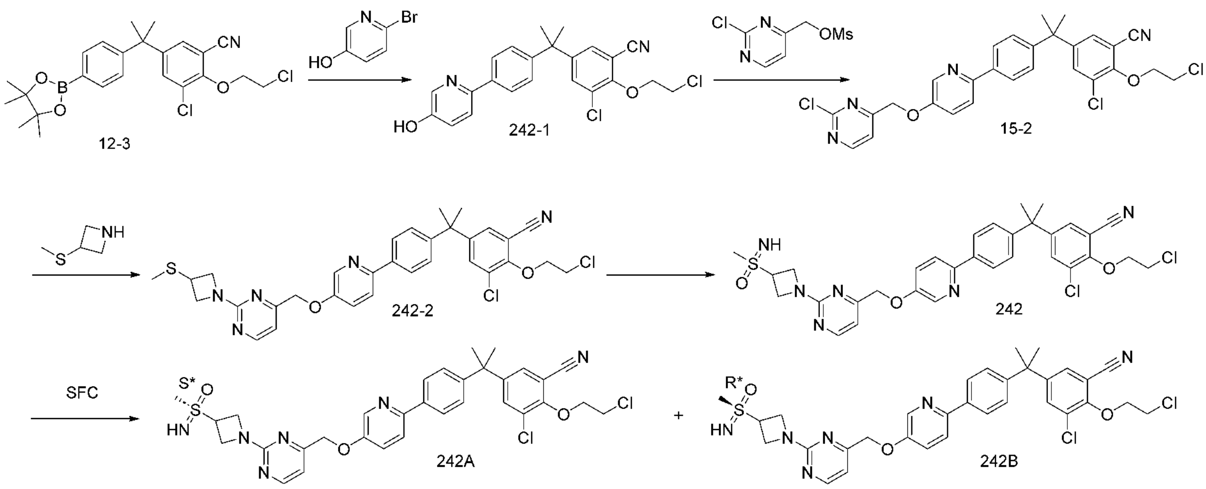


**Preparation of compound 242-1**

Compound **12-3** (1 g, 2.17 mmol) and 2-bromo-5-hydroxypyridine (0.45 g, 2.60 mmol) were dissolved in 1,4-dioxane (10 mL) and water. Pd(dppf)$Cl_2$ (0.16 g, 0.22 mmol) and potassium carbonate (0.9 g, 6.51 mmol) were added at room temperature, and the system was purged with argon. The mixture was stirred at 110°C for 3 h. The reaction was monitored by LCMS until completion. The reaction mixture was acidified with 1 mol/L hydrochloric acid and extracted three times with ethyl acetate (100 mL). The organic phases were combined, washed with saturated brine (100 mL), dried over anhydrous sodium sulfate, subjected to suction filtration, and the filtrate was concentrated. The resulting residue was purified by column chromatography (EA/PE = 0 to 45%) to obtain 0.75 g of the title compound **242-1** in 80.6% yield. LC-MS (ESI): m/z 426.6 $[M+H]^+$.

**Preparation of compound 15-2**

Compound **242-1** (0.3 g, 0.70 mmol) and (2-chloropyrimidin-4-yl)methyl methanesulfonate (0.19 g, 0.84 mmol) were dissolved in acetonitrile (2 mL), and cesium carbonate (0.68 g, 2.10 mmol) was added at room temperature. The mixture was stirred at 70°C for 2 h. The reaction was monitored by LCMS until completion. The reaction mixture was filtered, and the filtrate was concentrated. The resulting residue was subjected to column chromatography (EA/PE = 0 to 55%) to obtain 0.16 g of the title compound **15-2** in 41% yield. LC-MS (ESI): m/z 552.7 $[M+H]^+$.

**Preparation of compound 242-2**

Compound **15-2** (0.16 g, 0.29 mmol) and 3-(methylthio)azetidine (0.035 g, 0.35 mmol) were dissolved in ethanol (2 mL). Triethylamine (0.117 g, 1.16 mmol) was added at room temperature, and the mixture was stirred at 100°C for 2 h. The reaction was monitored by LCMS until completion. The mixture was cooled to room temperature and concentrated. The resulting residue was purified by silica gel column chromatography (EA/PE = 0 to 55%) to obtain 0.13 g of the title compound **242-2** in 72.5% yield. LC-MS (ESI): m/z 619.6 $[M+H]^+$.

**Preparation of compound 242**

Compound **242-2** (130 mg, 0.21 mmol) was dissolved in methanol (2 mL). (Diacetoxyiodo)benzene (271 mg, 0.84 mmol) and ammonium carbamate (41 mg, 0.53 mmol) were added at room temperature, and the mixture was stirred at room temperature for 16 h. The reaction was monitored by LCMS until completion. The reaction mixture was subjected to preparative purification (Waters 2767/Qda, column: Xbridge C18 19 * 250 mm, 10 μm; mobile phase: A: 0.1% FA/$H_2O$, B: ACN; flow rate: 20 mL/min; elution gradient: 68% to 78%; retention time: 6.8-8.4 min of 16 min) to obtain 41.7 mg of the title compound **242.** LC-MS (ESI): m/z 651.2 $[M+H]^+$. $^1H$ NMR (400 MHz, DMSO-$d_6$) δ 8.45 (d, *J* = 3.0 Hz, 1H), 8.42 (d, *J* = 5.0 Hz, 1H), 7.95 (d, *J* = 8.6 Hz, 2H), 7.89 (d, *J* = 8.8 Hz, 1H), 7.69 (d, *J* = 2.3 Hz, 1H), 7.62 (d, *J* = 2.3 Hz, 1H), 7.53 (dd, *J* = 8.8, 3.0 Hz, 1H), 7.33 (d, *J* = 8.6 Hz, 2H), 6.87 (d, *J* = 5.0 Hz, 1H), 5.16 (s, 2H), 4.42 (t, *J* = 5.2 Hz, 2H), 4.34 - 4.26 (m, 4H), 4.26 - 4.19 (m, 1H), 3.96 (t, *J* = 5.2 Hz, 2H), 2.95 (s, 3H), 1.69 (s, 6H).

**Preparation of compounds 242A and 242B**

Compound **242** (60 mg) was purified by preparative SFC (preparative conditions: instrument model: Waters SFC15; column model: ChiralPak AD-H, 250 * 20 mm I.D., 5 μm; mobile phase: A: $CO_2$; B: isopropanol (containing 30% acetonitrile); elution gradient: B 55%; flow rate: 15 mL/min; column pressure: 100 bar; column temperature: 40°C; detection wavelength: 214 nm; run time per injection: 20 min) to obtain 13.95 mg of title compound **242A** and 13.63 mg of title compound **242B.**

Compound **242A:** SFC analysis method (instrument model: Waters SFC15; column model: ChiralPak AD-H, 250 * 4.6 mm I.D., 5 μm; mobile phase: A: $CO_2$, B: isopropanol (0.05% DEA); elution gradient: B 55%; flow rate: 3 mL/min; column pressure: 100 bar; column temperature: 40°C; detection wavelength: 214 nm; Rt = 5.18 min). LC-MS (ESI): m/z 651.2 $[M+H]^+$. $^1H$ NMR (400 MHz, DMSO-$d_6$) δ 8.44 (d, *J* = 3.0 Hz, 1H), 8.42 (d, *J* = 5.0 Hz, 1H), 7.95 (d, *J* = 8.0 Hz, 2H), 7.89 (d, *J* = 8.8 Hz, 1H), 7.69 (s, 1H), 7.62 (s, 1H), 7.53 (dd, *J* = 8.9, 3.0 Hz, 1H), 7.33 (d, *J* = 8.0 Hz, 2H), 6.87 (d, *J*= 5.0 Hz, 1H), 5.16 (s, 2H), 4.42 (t, *J* = 5.1 Hz, 2H), 4.34 - 4.24 (m, 4H), 4.24 - 4.17 (m, 1H), 4.00 (s, 1H), 3.96 (t, *J* = 5.1 Hz, 2H), 2.91 (s, 3H), 1.68 (s, 6H).

Compound **242B:** SFC analysis method (instrument model: Waters SFC15; column model: ChiralPak AD-H, 250 * 4.6 mm I.D., 5 μm; mobile phase: A: $CO_2$, B: isopropanol (0.05% DEA); elution gradient: B 55%; flow rate: 3 mL/min; column pressure: 100 bar; column temperature: 40°C; detection wavelength: 214 nm; Rt = 6.20 min). LC-MS (ESI): m/z 651.2 $[M+H]^+$. $^1H$ NMR (400 MHz, DMSO-$d_6$) δ 8.45 (s, 1H), 8.42 (d, *J* = 5.0 Hz, 1H), 7.95 (d, *J* = 8.0 Hz, 2H), 7.89 (d, *J* = 8.8 Hz, 1H), 7.70 (s, 1H), 7.62 (s, 1H), 7.53 (d, *J* = 8.8 Hz, 1H), 7.33 (d, *J* = 8.0 Hz, 2H), 6.87 (d, *J* = 5.1 Hz, 1H), 5.16 (s, 2H), 4.42 (t, *J* = 5.2 Hz, 2H), 4.34 - 4.24 (m, 4H), 4.24 - 4.17 (m, 1H), 4.00 (s, 1H), 3.96 (t, *J*= 5.2 Hz, 2H), 2.91 (s, 3H), 1.69 (s, 6H).

**Example 243: Preparation of compound 243**

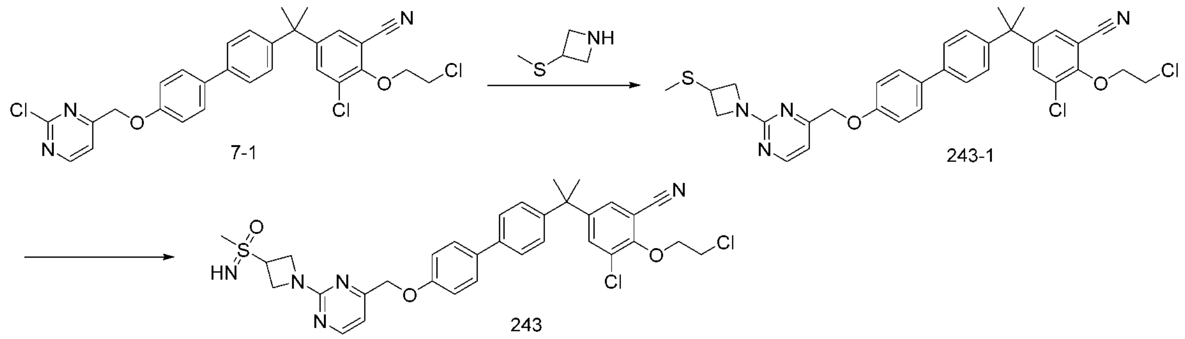

7-1 243-1 243

**Preparation of compound 243-1**

Compound **7-1** (200 mg, 0.36 mmol), 3-(methylthio)azetidine (50 mg, 0.36 mmol), and triethylamine (180 mg, 1.80 mmol) were dissolved in ethanol (2 mL), and the system was reacted at 100°C for 2 h. After the reaction was complete, the reaction mixture was cooled to room temperature and concentrated. The resulting residue was purified by silica gel column chromatography (EA/PE = 0 to 44%) to obtain 190 mg of the title compound **243-1** in 84.7% yield. LC-MS (ESI): m/z 619.1 $[M+H]^+$.

**Preparation of compound 243**

Compound **243-1** (190 mg, 0.31 mmol) was dissolved in methanol (2 mL). (Diacetoxyiodo)benzene (399 mg, 1.24 mmol) and ammonium carbamate (61 mg, 0.78 mmol) were added at room temperature, and the mixture was stirred at room temperature for 16 h. The reaction was monitored by LCMS until completion. The residue was concentrated and subjected to preparative purification (Waters 2767/Qda, Column: XBridge C18 19 * 250mm, 10 μm; mobile phase: A: 0.1% FA/$H_2O$, B: ACN; flow rate: 20 mL/min; elution gradient: 65% to 75%; retention time: 9.4-11.2 min of 16 min) to obtain 41.9 mg of the title compound **243.** LC-MS (ESI): m/z 690.4 $[M+H_2O+Na]^+$. $^1$H NMR (400 MHz, DMSO-$d_6$) δ 8.40 (d, *J* = 5.0 Hz, 1H), 7.70 (d, *J* = 2.3 Hz, 1H), 7.64 (d, *J* = 2.3 Hz, 1H), 7.60 (d, *J* = 8.8 Hz, 2H), 7.56 (d, *J* = 8.4 Hz, 2H), 7.30 (d, *J* = 8.5 Hz, 2H), 7.08 (d, *J* = 8.8 Hz, 2H), 6.83 (d, *J* = 5.0 Hz, 1H), 5.07 (s, 2H), 4.42 (t, *J* = 5.2 Hz, 2H), 4.33 - 4.21 (m, 5H), 3.98 (s, 1H), 3.96 (t, *J* = 5.2 Hz, 2H), 2.91 (s, 3H), 1.68 (s, 6H).

**Example 244: Preparation of compound 244**

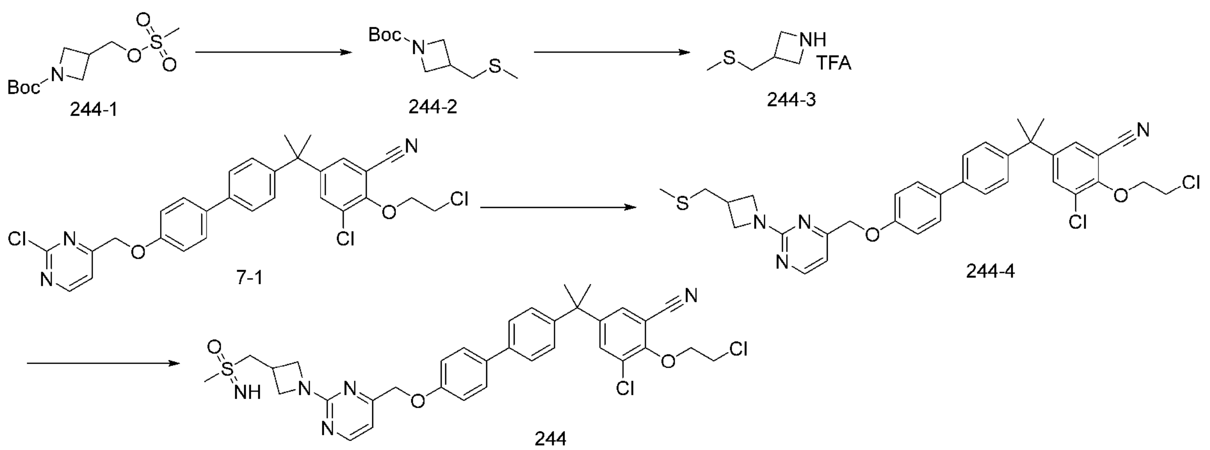

244-1 244-2 244-3 7-1 244-4 244

**Preparation of compound 244-2**

Compound **244-1** (100.0 mg, 0.38 mmol) was dissolved in DMF (3 mL), and sodium methanethiolate (67 mg, 0.95 mmol) was added. The mixture was heated to 70°C and stirred overnight. TLC indicated the disappearance of the starting material. The system was cooled to room temperature, quenched with ice water (10 mL), and extracted three times with ethyl acetate (20 mL). The organic phases were combined, dried over anhydrous sodium sulfate, subjected to suction

filtration, and the filtrate was concentrated to obtain 170 mg of the title compound **244-2.** The product was used directly in the next step without purification.

**Preparation of compound 244-3**

Compound **244-2** (170 mg, 0.78 mmol) was dissolved in DCM (2 mL), and TFA (1 mL) was added at room temperature. The mixture was stirred at room temperature for 10 min. TLC indicated the disappearance of the starting material. The mixture was concentrated to obtain 300 mg of the title compound **244-3.** $^1$H NMR (400 MHz, $CDCl_3$) δ 4.27 - 4.17 (m, 2H), 3.98 - 3.89 (m, 2H), 3.28 - 3.16 (m, 1H), 2.78 (d, *J* = 7.7 Hz, 2H), 2.12 (s, 3H).

**Preparation of compound 244-4**

Compound **7-1** (100.0 mg, 0.18 mmol) was dissolved in EtOH (5 mL). Compound **244-3** (84.4 mg, 0.72 mmol) and triethylamine (91.1 mg, 0.90 mmol) were added. The mixture was heated to 100°C and stirred for 6 h under an argon atmosphere. The reaction was monitored by LCMS until completion. The reaction mixture was cooled to room temperature, quenched by pouring into ice-cold ammonium chloride solution (10 mL), and extracted three times with ethyl acetate (15 mL). The organic phases were combined, dried over anhydrous sodium sulfate, subjected to suction filtration, and the filtrate was concentrated. The resulting organic phase was purified by normal-phase column chromatography (EA/PE = 0 to 60%) to obtain 30 mg of the title compound **244-4** in 26% yield. LC-MS (ESI): m/z 633.2 [M+H]$^+$.

**Preparation of compound 244**

Compound **244-4** (30.0 mg, 0.047 mmol), (diacetoxyiodo)benzene (60.6 mg, 0.19 mmol), and ammonium carbamate (9.2 mg, 0.12 mmol) were added to methanol (3 mL). The mixture was stirred at room temperature for 1 h. The reaction was monitored by LCMS until completion. The organic phase was concentrated, and the resulting residue was subjected to preparative purification (Waters 2767/QDA; column: Sunfire C18, 19 * 250 mm, 10 μm; mobile phase: A: 0.05% TFA in $H_2O$; B: ACN; flow rate: 20 mL/min; elution gradient: 57% to 57%; retention time: 7.2-8.6 min of 16 min) to obtain 5.2 mg of the title compound. LC-MS (ESI): m/z 664.4 [M+H]$^+$. $^1$H NMR (400 MHz, Methanol-*d*$_4$) δ 8.33 (d, *J* = 5.2 Hz, 1H), 7.58 - 7.51 (m, 6H), 7.29 (d, *J* = 8.0 Hz, 2H), 7.05 (d, *J* = 8.6 Hz, 2H), 6.91 (d, *J* = 5.2 Hz, 1H), 5.06 (s, 2H), 4.46 - 4.39 (m, 4H), 4.20 - 4.04 (m, 4H), 3.90 (t, *J* = 5.5 Hz, 2H), 3.55 (s, 3H), 3.50 - 3.42 (m, 1H), 1.71 (s, 6H).

**Example 245: Preparation of compound 245**

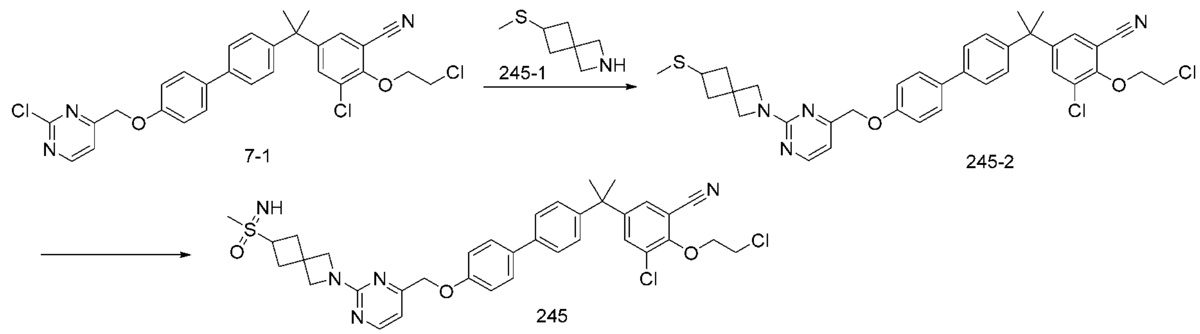


**Preparation of compound 245-2**

Compound **7-1** (200 mg, 0.36 mmol), compound **245-1** (52 mg, 0.36 mmol), and triethylamine (150 mg, 1.44 mmol) were dissolved in ethanol (2 mL). The system was reacted at 100°C for 2 h. After the reaction was completed, the reaction mixture was cooled to room temperature and concentrated. The resulting residue was purified by silica gel column chromatography (EA/PE = 0 to 60%) to obtain 200 mg of the title compound **245-2** in 83.8% yield. LC-MS (ESI): m/z 659.2 [M+H]$^+$.

**Preparation of compound 245**

Compound **245-2** (100 mg, 0.15 mmol) was dissolved in methanol (2 mL). (Diacetoxyiodo)benzene (190 mg,

0.60 mmol) and ammonium carbamate (30 mg, 0.39 mmol) were added at room temperature, and the mixture was stirred at room temperature for 16 h. The reaction was monitored by LCMS until completion. The reaction mixture was concentrated, and the resulting residue was subjected to preparative purification (Waters 2767/Qda; column: XBridge C18 19 * 250 mm, 10 μm; mobile phase: A: 0.1% FA/$H_2O$, B: ACN; flow rate: 20 mL/min; elution gradient: 65% to 75%; retention time: 9.4-11.2 min of 16 min) to obtain 41.97 mg of the title compound **245.** LC-MS (ESI): m/z 690.4 $[M+H]^+$. $^1H$ NMR (400 MHz, DMSO-$d_6$) δ 8.34 (d, *J*= 5.0 Hz, 1H), 7.70 (d, *J* = 2.2 Hz, 1H), 7.63 (d, *J* = 2.2 Hz, 1H), 7.59 (d, *J* = 8.7 Hz, 2H), 7.55 (d, *J* = 8.3 Hz, 2H), 7.30 (d, *J* = 8.3 Hz, 2H), 7.07 (d, *J* = 8.7 Hz, 2H), 6.75 (d, *J* = 5.0 Hz, 1H), 5.04 (s, 2H), 4.42 (t, *J* = 5.2 Hz, 2H), 4.09 (s, 2H), 4.04 - 3.93 (m, 4H), 3.87 - 3.72 (m, 1H), 3.61 (s, 1H), 2.78 (s, 3H), 2.63 - 2.44 (m, 4H), 1.68 (s, 6H).

**Example 246: Preparation of compound 246**

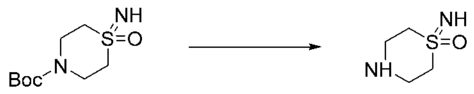

246-1 246-2

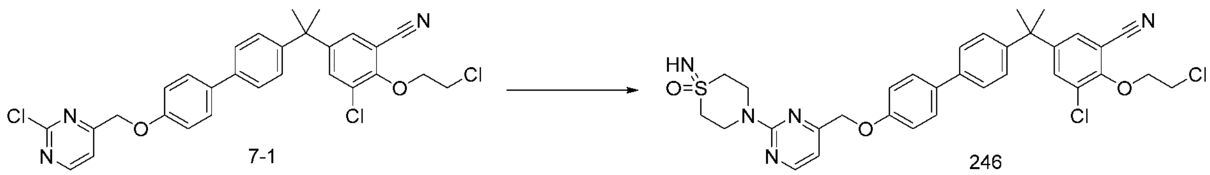

7-1 246

**Preparation of compound 246-2**

Compound **246-1** (1 g, 4.27 mmol) was dissolved in DCM (5 mL). A solution of hydrogen chloride in 1,4-dioxane (5 mL) was added at room temperature, and the mixture was stirred at room temperature for 2 h. The reaction was monitored by TLC until completion. Diethyl ether (50 mL) was added, and the mixture was filtered to obtain 200 mg of the title compound **246-2** in 87.3% yield. The product was used directly in the next step without purification.

**Preparation of compound 246**

Compound **7-1** (100 mg, 0.180 mmol), compound **246-2** (50 mg, 0.361 mmol), and triethylamine (55 mg, 0.542 mmol) were dissolved in ethanol (2 mL). The system was reacted at 100°C for 2 h. After the reaction was completed, the reaction mixture was cooled to room temperature and concentrated. The resulting residue was subjected to preparative purification (Waters 2767/Qda; column: SunFire C18 19 * 250 mm, 10 μm; mobile phase: A: 0.1% FA/$H_2O$, B: ACN; flow rate: 20 mL/min; elution gradient: 74% to 82%; retention time: 8.1-9.1 min of 16 min) to obtain 26.81 mg of the title compound **246.** LC-MS (ESI): m/z 650.2 $[M+H]^+$. $^1H$ NMR (400 MHz, DMSO-$d_6$) δ 8.44 (d, *J* = 5.0 Hz, 1H), 7.70 (d, *J* = 2.3 Hz, 1H), 7.63 (d, *J* = 2.3 Hz, 1H), 7.59 (d, *J*= 8.8 Hz, 2H), 7.55 (d, *J* = 8.4 Hz, 2H), 7.30 (d, *J* = 8.5 Hz, 2H), 7.08 (d, *J* = 8.8 Hz, 2H), 6.83 (d, *J* = 5.0 Hz, 1H), 5.10 (s, 2H), 4.47 - 4.35 (m, 4H), 3.99 - 3.90 (m, 4H), 3.08 - 2.96 (m, 4H), 1.68 (s, 6H).

**Example 247: Preparation of compound 247**

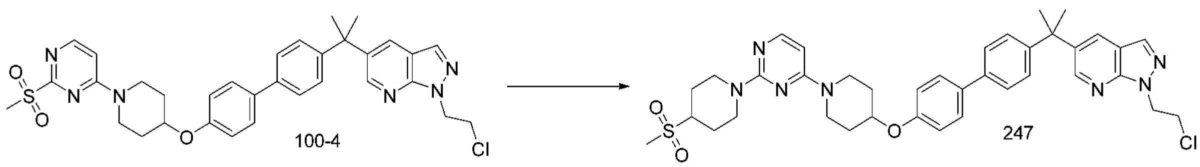

100-4 247

**Preparation of compound 247**

Compound **100-4** (90 mg, 142.5 μmol), 4-(methylsulfonyl)piperidine hydrochloride (48 mg, 279 μmol), and cesium carbonate (93 mg, 285 μmol) were added to acetonitrile (2 mL). The reaction system was stirred at 120°C for 16 h.

After the reaction was completed, the reaction mixture was cooled to room temperature. Saturated brine (5 mL) was added to the reaction mixture, and the mixture was extracted three times with ethyl acetate (8 mL). The organic phases were combined, dried, and subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 5% to 35% acetonitrile gradient elution over 12 min; flow rate: 30 mL/min) to obtain 23 mg of the title compound **247.** LC-MS (ESI): m/z 714.4 $[M+H]^+$. $^1H$ NMR (400 MHz, DMSO-$d_6$) δ 8.40 (d, *J* = 2.2 Hz, 1H), 8.19 (d, *J* = 2.2 Hz, 1H), 8.17 (s, 1H), 7.89 (d, *J* = 6.0 Hz, 1H), 7.57 (d, *J* = 8.8 Hz, 2H), 7.54 (d, *J* = 8.5 Hz, 2H), 7.31 (d, *J* = 8.5 Hz, 2H), 7.07 (d, *J* = 8.8 Hz, 2H), 6.15 (d, *J* = 6.1 Hz, 1H), 4.75 (t, *J* = 5.8 Hz, 2H), 4.73 - 4.63 (m, 1H), 4.12 (t, *J= 5.8* Hz, 2H), 4.04 - 3.87 (m, 1H), 3.48 - 3.24 (m, 8H), 2.93 (s, 3H), 2.10 - 1.93 (m, 4H), 1.77 (s, 6H), 1.67 - 1.54 (m, 2H), 1.54 - 1.36 (m, 2H).

**Example 248: Preparation of compound 248**

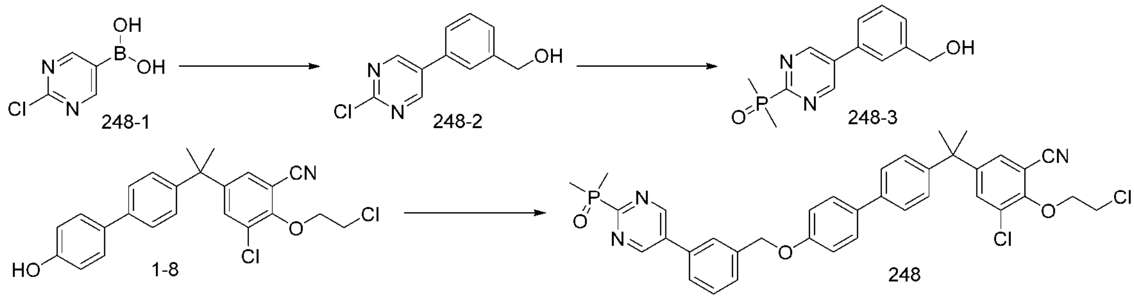

248-1 248-2 248-3

1-8 248

**Preparation of compound 248-2**

Compound **248-1** (2 g, 12.63 mmol), 3-bromobenzyl alcohol (2.36 g, 12.63 mmol), and potassium carbonate (1.75 g, 12.63 mmol) were dissolved in 1,4-dioxane (20 mL) and water (5 mL), and Pd(dppf)$Cl_2$ (924 mg, 1.26 mmol) was added thereto. After the addition was completed, the reaction system was stirred at 80°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 75%) to obtain 1.5 g of the title compound **248-2.** LC-MS (ESI): m/z 221.0 $[M+H]^+$.

**Preparation of compound 248-3**

Compound **248-2** (750 mg, 3.4 mmol), dimethylphosphine oxide (796 mg, 10.2 mmol), $Pd_2(dba)_3$ (311 mg, 0.339 mmol), Xantphos (196 mg, 0.339 mmol), and DIPEA (1.32 g, 10.20 mmol) were dissolved in DMF (5 mL). The reaction system was stirred at 128°C for 3 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (DCM/MeOH = 100% to 90%) to obtain 650 mg of the title compound **248-3.** LC-MS (ESI): m/z 263.1 $[M+H]^+$.

**Preparation of compound 248**

Compound **248-3** (45 mg, 0.172 mmol) was dissolved in dichloromethane (5 mL), followed by the sequential addition of compound **1-8** (73 mg, 0.172 mmol) and TMAD (59 mg, 0.343 mmol). A solution of tributylphosphine (69 mg, 0.343 mmol) in dichloromethane (0.5 mL) was added dropwise to the reaction system at 0°C. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 70 mg of

the title compound **248.** LC-MS (ESI): m/z 670.3 $[M+H]^+$. $^1H$ NMR (400 MHz, DMSO-$d_6$) δ 9.33 (s, 2H), 7.98 (s, 1H), 7.88 - 7.83 (m, 1H), 7.70 (d, *J* = 2.4 Hz, 1H), 7.65 - 7.59 (m, 5H), 7.56 (d, *J* = 8.5 Hz, 2H), 7.30 (d, *J* = 8.5 Hz, 2H), 7.13 (d, *J* = 8.8 Hz, 2H), 5.25 (s, 2H), 4.42 (t, *J* = 5.2 Hz, 2H), 3.96 (t, *J* = 5.2 Hz, 2H), 1.80 (d, *J* = 13.7 Hz, 6H), 1.68 (s, 6H). $^{31}P$ NMR (162 MHz, Chloroform-*d*) δ 33.86 (s, 1P).

**Example 249: Preparation of compound 249**

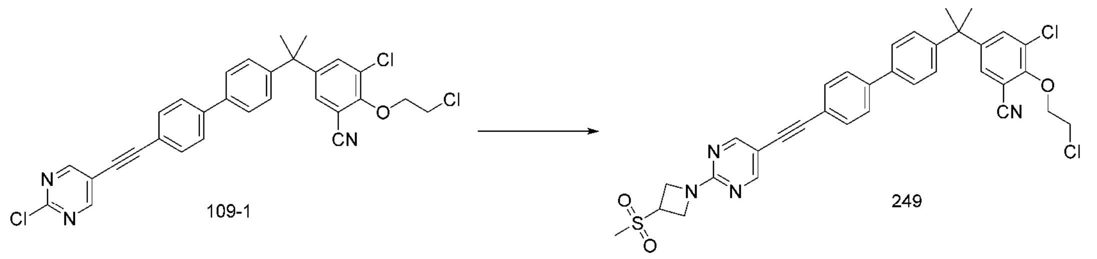

**Preparation of compound 249**

Compound **109-1** (0.1 g, 0.183 mmol) and DIEA(34 mg, 0.201 mmol) were dissolved in ethanol (2 mL), and compound 3-(methylsulfonyl)azetidine hydrochloride (34 mg, 0.201 mmol) was added thereto. After the addition was completed, the reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound **249.** LC-MS (ESI): m/z 645.2 $[M+H]^+$. $^1H$ NMR (400 MHz, DMSO-$d_6$) δ 8.62 (s, 2H), 7.73 (d, *J* = 8.4 Hz, 2H), 7.71 (d, *J* = 2.3 Hz, 1H), 7.67 (d, *J* = 8.5 Hz, 2H), 7.65 (d, *J* = 2.3 Hz, 1H), 7.60 (d, *J* = 8.4 Hz, 2H), 7.36 (d, *J* = 8.5 Hz, 2H), 4.45 - 4.39 (m, 5H), 4.30 - 4.26 (m, 2H), 3.98 - 3.94 (m, 2H), 3.09 (s, 3H), 1.70 (s, 6H).

**Example 250: Preparation of compound 250**

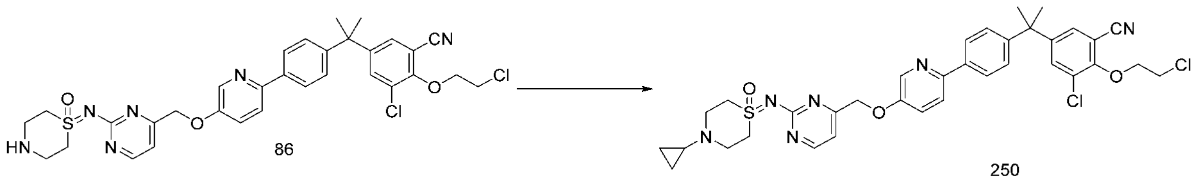

**Preparation of compound 250**

Compound **86** (200 mg, 306.93 μmol), bromocyclopropane (112 mg, 920.80 μmol), DIPEA (397 mg, 3.07 mmol, 534.63 μL), and acetonitrile (2 mL) were added to a reaction flask, and the flask was sealed and stirred at 100°C for 40 h. After the reaction was completed, the reaction mixture was cooled to room temperature, subjected to rotary evaporation until dryness, and concentrated to obtain a crude product. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 10% to 90% acetonitrile gradient elution over 20 min; flow rate: 30 mL/min) to obtain 20 mg of the title compound **250.** LC-MS (ESI): m/z 691.4 $[M+H]^+$. $^1H$ NMR (400 MHz, DMSO-$d_6$) δ 8.46 (d, *J*= 5.1 Hz, 1H), 8.44 (d, *J*= 3.0 Hz, 1H), 7.95 (d, *J* = 8.5 Hz, 2H), 7.89 (d, *J* = 8.8 Hz, 1H), 7.70 (d, *J*= 2.4 Hz, 1H), 7.62 (d, *J* = 2.3 Hz, 1H), 7.51 (dd, *J* = 8.8, 3.1 Hz, 1H), 7.33 (d, *J* = 8.6 Hz, 2H), 7.01 (d, *J* = 5.0 Hz, 1H), 5.84 - 5.68 (m, 1H), 5.25 - 5.09 (m, 4H), 4.42 (t, *J* = 5.2 Hz, 2H), 3.95 (t, *J* = 5.2 Hz, 2H), 3.73 - 3.60 (m, 2H), 3.50 - 3.45 (m, 2H), 3.09 (d, *J* = 6.4 Hz, 2H), 2.97 - 2.86 (m, 2H), 2.86 - 2.73 (m, 2H), 1.69 (s, 6H).

**Example 251: Preparation of compound 251**

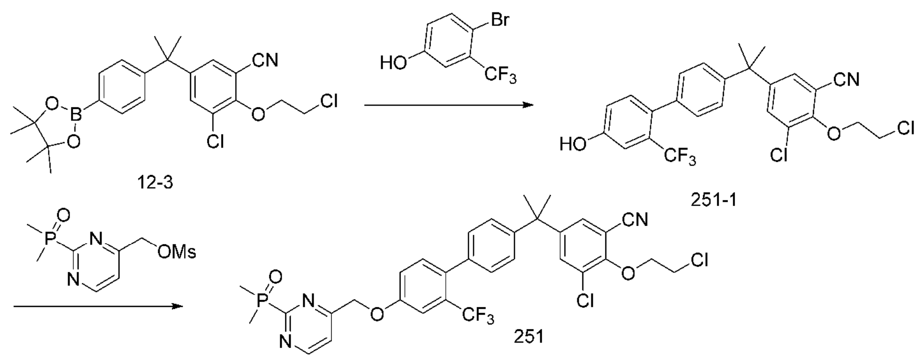

12-3 251-1 251

**Preparation of compound 251-1**

Compound **12-3** (200 mg, 0.829 mmol) was dissolved in 1,4-dioxane (6 mL) and water (2 mL), followed by the sequential addition of 4-bromo-3-(trifluoromethyl)phenol (382 mg, 0.829 mmol), potassium carbonate (228 mg, 1.658 mmol), and Pd(dppf)$Cl_2$-DCM (68 mg, 0.083 mmol). The reaction system was purged three times with nitrogen and stirred at 91°C for 2 h. After the reaction was completed, water (30 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 75%) to obtain 380 mg of the title compound **251-1.** LC-MS (ESI): m/z 494.0 $[M+H]^+$.

**Preparation of compound 251**

Compound **251-1** (63 mg, 0.127 mmol) was dissolved in acetonitrile (2 mL), followed by the sequential addition of potassium carbonate (53 mg, 0.382 mmol) and compound (2-(dimethylphosphoryl)pyrimidin-4-yl)methyl methanesulfonate (34 mg, 0.127 mmol). The reaction system was stirred at 70°C for 0.5 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 53 mg of the title compound **251.** LC-MS (ESI): m/z 661.8 $[M+H]^+$. $^1$H NMR (400 MHz, DMSO-$d_6$) δ 9.02 (d, $J$ = 5.2 Hz, 1H), 7.80 (dd, $J$ = 5.2, 3.2 Hz, 1H), 7.70 (d, $J$ = 2.3 Hz, 1H), 7.63 (d, $J$ = 2.3 Hz, 1H), 7.49 (d, $J$ = 2.5 Hz, 1H), 7.42 - 7.34 (m, 2H), 7.31 (d, $J$ = 8.4 Hz, 2H), 7.24 (d, $J$ = 8.2 Hz, 2H), 5.45 (s, 2H), 4.43 (t, $J$ = 5.2 Hz, 2H), 3.96 (t, $J$ = 5.2 Hz, 2H), 1.75 (d, $J$ = 13.8 Hz, 6H), 1.70 (s, 6H). $^{31}$P NMR (162 MHz, DMSO-$d_6$) δ 34.16 (s, 1P). $^{19}$F NMR (376 MHz, DMSO-$d_6$) δ -55.59 (s, 3F).

**Example 252: Preparation of compound 252**

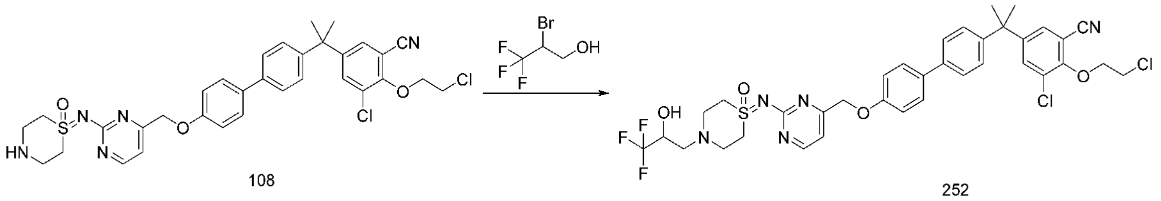

108 252

**Preparation of compound 252**

2-Bromo-3,3,3-trifluoropropan-1-ol (60 mg, 307.40 μmol), compound **108** (100 mg, 153.70 μmol), DIPEA (60 mg, 461.10 μmol), and acetonitrile (1 mL)were added to a reaction flask, and the mixture was stirred under microwave irradiation at 120°C for 3 h. The mixture was subjected to rotary evaporation until dryness and concentrated to obtain a

crude product. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 20% to 90% acetonitrile gradient elution over 20 min; flow rate: 30 mL/min) to obtain 15 mg of the title compound **252.** LC-MS (ESI): m/z 762.2 [M+H]+. $^1$H NMR (400 MHz, DMSO-$d_6$) δ 8.45 (d, *J* = 5.0 Hz, 1H), 7.70 (d, *J* = 2.3 Hz, 1H), 7.63 (d, *J* = 2.4 Hz, 1H), 7.59 (d, *J* = 8.8 Hz, 2H), 7.55 (d, *J* = 8.5 Hz, 2H), 7.29 (d, *J* = 8.3 Hz, 2H), 7.07 (d, *J* = 8.8 Hz, 2H), 6.98 (d, *J* = 5.1 Hz, 1H), 6.24 (d, *J* = 6.2 Hz, 1H), 5.11 (s, 2H), 4.42 (t, *J* = 5.2 Hz, 2H), 4.18 - 4.08 (m, 1H), 3.95 *(t, J* = 5.2 Hz, 2H), 3.72 - 3.60 (m, 2H), 3.51 - 3.44 (m, 2H), 3.16 - 3.05 (m, 2H), 3.05 - 2.94 (m, 2H), 2.79 (dd, *J* = 13.8, 2.9 Hz, 1H), 2.66 (dd, *J* = 13.7, 8.5 Hz, 1H), 1.68 (s, 6H). $^{19}$F NMR (376 MHz, DMSO-$d_6$) δ - 76.97 (s, 3F).

**Example 253: Preparation of compound 253**

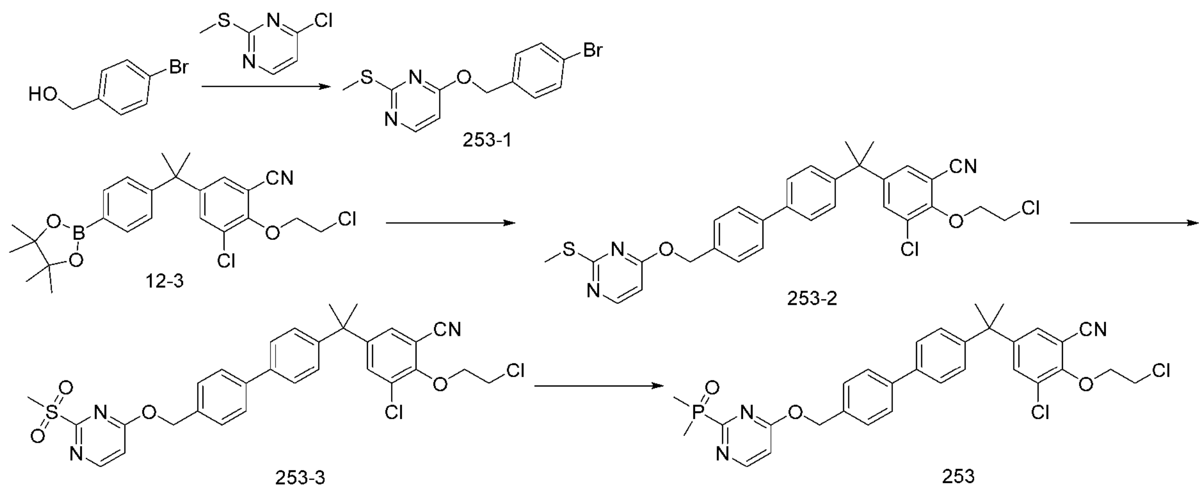

253-1

12-3

253-2

253-3

253

**Preparation of compound 253-1**

4-Bromobenzyl alcohol (2 g, 10.69 mmol) and 2-methylthio-4-chloropyrimidine (1.55 g, 9.62 mmol) were dissolved in DMF (30 mL), and cesium fluoride (1.06 g, 16.04 mmol) was added thereto. After the addition was completed, the reaction system was stirred at 50°C for 1 h. After the reaction was completed, a 5% aqueous lithium chloride solution (100 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 85%) to obtain 2.4 g of the title compound **253-1.** LC-MS (ESI): m/z 311.0 [M+H]+.

**Preparation of compound 253-2**

Compound **253-1** (0.2 g, 0.642 mmol), compound **12-3** (296 mg, 0.642 mmol), and potassium carbonate (177 mg, 1.29 mmol) were dissolved in 1,4-dioxane (10 mL) and water (3 mL), and Pd(dppf)$Cl_2$ (52 mg, 0.064 mmol) was added thereto. After the addition was completed, the reaction system was stirred at 91°C for 2 h. After the reaction was completed, water (50 mL) was added, and the mixture was extracted three times with ethyl acetate (20 mL). The organic phases were combined, washed twice with saturated brine (30 mL), dried over anhydrous sodium sulfate, filtered, and the filtrate was concentrated to obtain a crude product. The crude product was purified by silica gel column chromatography (PE/EA = 100% to 75%) to obtain 200 mg of the title compound **253-2.** LC-MS (ESI): m/z 564.2 [M+H]+.

**Preparation of compound 253-3**

Compound **253-2** (0.2 g, 0.354 mmol) was dissolved in dichloromethane (30 mL), and m-chloroperoxybenzoic acid (0.134 g, 0.779 mmol) was added thereto. After the addition was completed, the reaction system was stirred at room temperature for 1 h. After the reaction was completed, the reaction mixture was filtered, and the filtrate was concentrated to obtain a crude product. The crude product was purified by silica gel column chromatography (PE/EA = 100% to 85%) to obtain 0.17 g of the title compound **253-3.** LC-MS (ESI): m/z 596.0 [M+H]+.

**Preparation of compound 253**

Compound **253-3** (45 mg, 0.075 mmol), dimethylphosphine oxide (6 mg, 0.076 mmol), and potassium carbonate (10 mg, 0.075 mmol) were placed in a sealed tube, and acetonitrile (1 mL) was added. The reaction system was stirred at 90°C for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound 253. LC-MS (ESI): m/z 594.2 $[M+H]^+$. $^1H$ NMR (400 MHz, DMSO-$d_6$) δ 8.70 (d, *J* = 5.8 Hz, 1H), 7.72 (d, *J* = 2.4 Hz, 1H), 7.69 (d, *J* = 8.0 Hz, 2H), 7.65 (d, *J* = 2.3 Hz, 1H), 7.62 (d, *J* = 8.2 Hz, 2H), 7.57 (d, *J* = 8.1 Hz, 2H), 7.34 (d, *J* = 8.2 Hz, 2H), 7.13 (dd, *J* = 5.9, 2.7 Hz, 1H), 5.53 (s, 2H), 4.42 (t, *J* = 5.1 Hz, 2H), 3.96 (t, *J* = 5.1 Hz, 2H), 1.74 (d, *J* = 13.7 Hz, 6H), 1.69 (s, 6H). $^{31}P$ NMR (162 MHz, Chloroform-*d*) δ 34.10 (s, 1P).

**Example 254: Preparation of compound 254**

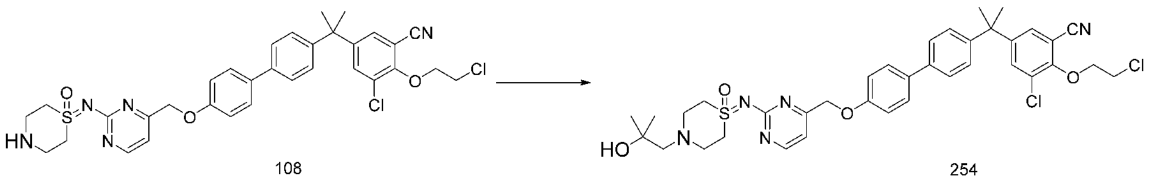

108 254

**Preparation of compound 254**

Compound **108** (160 mg, 245.92 μmol) and 1-bromo-2-methyl-2-propanol (376.3 mg, 2.46 mmol) were dissolved in acetonitrile (2 mL), and cesium carbonate (400.6 mg, 1.23 mmol) was added. The reaction mixture was stirred at 80°C for 12 h. LCMS indicated the disappearance of the starting material and the formation of the product. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 1.3 mg of the title compound **254.** LC-MS (ESI): m/z 722.2 $[M+H]^+$. $^1H$ NMR (400 MHz, DMSO-$d_6$) δ 8.49 (s, 1H), 7.70 (d, *J* = 2.4 Hz, 1H), 7.64 (d, *J* = 2.3 Hz, 1H), 7.63 - 7.52 (m, 5H), 7.30 (d, *J* = 8.0 Hz, 2H), 7.07 (d, *J* = 8.2 Hz, 2H), 6.99 (s, 1H), 5.10 (s, 2H), 4.42 (t, *J* = 5.1 Hz, 2H), 4.20 (s, 1H), 3.96 (t, *J* = 5.1 Hz, 2H), 3.64 (s, 2H), 3.37 - 5.55 (m, 2H), 3.04 (d, *J* = 37.6 Hz, 5H), 1.68 (s, 6H), 1.06 (s, 6H).

**Example 255: Preparation of compound 255**

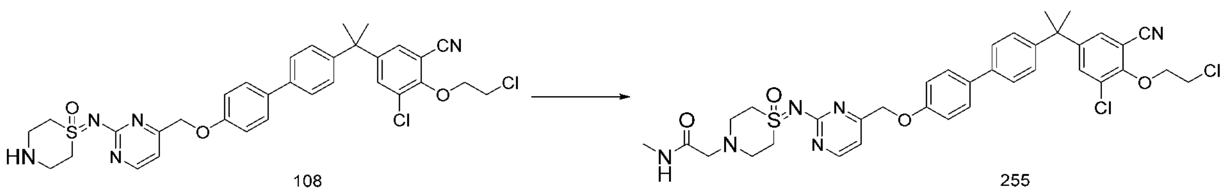

108 255

**Preparation of compound 255**

Compound **108** (100 mg, 153.70 μmol) and 2-bromo-N-methylacetamide (70.1 mg, 461.10 μmol) were dissolved in acetonitrile (2 mL), and cesium carbonate (250.4 mg, 768.50 μmol) was added. The reaction mixture was stirred at 80°C for 12 h. LCMS indicated the disappearance of the starting material and the formation of the product. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 1.41 mg of the title compound **255.** LC-MS (ESI): m/z 721.2 $[M+H]^+$. $^1H$ NMR (400 MHz, DMSO-$d_6$) δ 8.45 (d, *J* = 5.0 Hz, 1H), 7.92 (d, *J* = 5.0 Hz, 1H), 7.70 (d, *J* = 2.4 Hz, 1H), 7.64 (d, *J* = 2.3 Hz, 1H), 7.60 (d, *J* = 8.7 Hz, 2H), 7.55 (d, *J* = 8.4 Hz, 2H), 7.30 *(d, J* = 8.3 Hz, 2H), 7.07 (d, *J* = 8.8 Hz, 2H), 6.98 (d, *J* = 5.1 Hz, 1H), 5.11 (s, 2H), 4.42 (t, *J* = 5.1 Hz, 2H), 3.96 (t, *J* = 5.1 Hz, 2H), 3.79 - 3.68 (m, 2H), 3.63 -

3.54 (m, 2H), 3.09 (s, 2H), 3.02 - 2.91 (m, 4H), 2.60 (d, *J* = 4.7 Hz, 3H), 1.68 (s, 6H).

**Example 256: Preparation of compound 256**

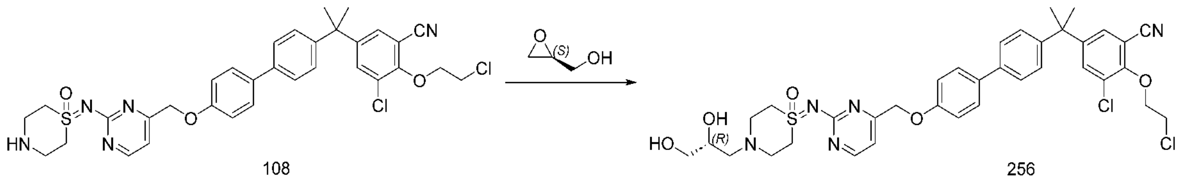


108

256

**Preparation of compound 256**

Compound 108 (100 mg, 153.70 μmol), (S)-glycidol (57 mg, 768.50 μmol), $K_2CO_3$ (106 mg, 768.50 μmol), and acetonitrile (1 mL) were added to a reaction flask, and the mixture was stirred at 60°C for 16 h. The mixture was filtered, subjected to rotary evaporation until dryness, and concentrated to obtain a crude product. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 20% to 90% acetonitrile gradient elution over 20 min; flow rate: 30 mL/min) to obtain 20 mg of the title compound **256.** LC-MS (ESI): m/z 724.3 $[M+H]^+$. $^1H$ NMR (400 MHz, DMSO-$d_6$) δ 8.45 (d, *J* = 5.1 Hz, 1H), 7.71 (d, *J* = 2.3 Hz, 1H), 7.64 (d, *J* = 2.4 Hz, 1H), 7.60 (d, *J* = 8.8 Hz, 2H), 7.56 (d, *J* = 8.5 Hz, 2H), 7.30 (d, *J* = 8.4 Hz, 2H), 7.08 (d, *J* = 8.8 Hz, 2H), 6.97 (d, *J* = 5.0 Hz, 1H), 5.11 (s, 2H), 4.51 (d, *J* = 4.2 Hz, 2H), 4.42 (t, *J* = 5.2 Hz, 2H), 3.96 (t, *J* = 5.2 Hz, 2H), 3.75 - 3.62 (m, 2H), 3.62 - 3.53 (m, 1H), 3.52 - 3.41 (m, 2H), 3.30 (s, 2H), 3.10 - 2.98 (m, 2H), 2.98 - 2.86 (m, 2H), 1.68 (s, 6H).

**Example 257: Preparation of compound 257**

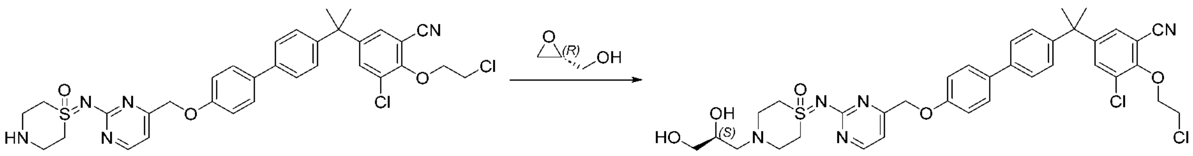


108

257

**Preparation of compound 257**

Compound **108** (100 mg, 153.70 μmol), (R)-glycidol (57 mg, 768.50 μmol), $K_2CO_3$ (106 mg, 768.50 μmol), and acetonitrile (1 mL) were added to a reaction flask, and the mixture was stirred at 60°C for 16 h. The mixture was filtered, subjected to rotary evaporation until dryness, and concentrated to obtain a crude product. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 20% to 90% acetonitrile gradient elution over 20 min; flow rate: 30 mL/min) to obtain 30 mg of the title compound 257. LC-MS (ESI): m/z 724.3 $[M+H]^+$. $^1H$ NMR (400 MHz, DMSO-$d_6$) δ 8.44 (d, *J* = 5.0 Hz, 1H), 7.70 (d, *J* = 2.4 Hz, 1H), 7.64 (d, *J* = 2.4 Hz, 1H), 7.60 (d, *J* = 8.8 Hz, 2H), 7.55 (d, *J* = 8.2 Hz, 2H), 7.30 (d, *J* = 8.2 Hz, 2H), 7.07 (d, *J* = 8.8 Hz, 2H), 6.97 *(d, J=* 5.0 Hz, 1H), 5.11 (s, 2H), 4.51 (s, 2H), 4.42 (t, *J* = 5.2 Hz, 2H), 3.96 (t, *J* = 5.2 Hz, 2H), 3.73 - 3.61 (m, 2H), 3.61 - 3.53 (m, 1H), 3.52 - 3.41 (m, 3H), 3.06 - 2.98 (m, 2H), 2.98 - 2.88 (m, 2H), 2.60 - 2.31 (m, 3H), 1.68 (s, 6H).

**Example 258: Preparation of compound 258**

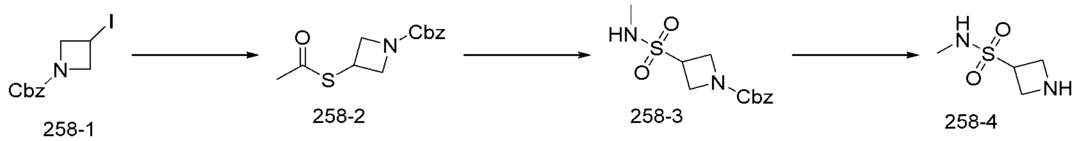

258-1 258-2 258-3 258-4

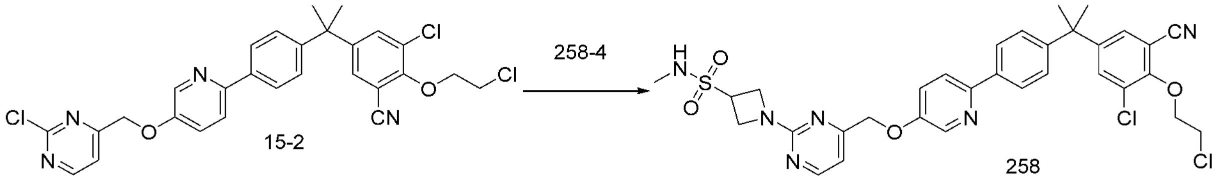


15-2 258

### Preparation of compound 258-2

Compound **258-1** (3 g, 9.46 mmol), potassium thioacetate (2.16 g, 18.92 mmol), cesium carbonate (3.08 g, 9.46 mmol), and DMF (30 mL)were added to a reaction flask, and the mixture was stirred at 70°C for 2 h. The mixture was cooled to room temperature, followed by the addition of ethyl acetate (50 mL). The organic phase was washed three times with saturated aqueous ammonium chloride solution (50 mL), subjected to rotary evaporation until dryness, and the crude product was purified by silica gel column chromatography (EA/PE = 0 to 20%) to obtain 2.5 g of compound258-2. LC-MS (ESI): m/z 266.2 $[M+H]^+$.

### Preparation of compound 258-3

Compound258-2 (2.5 g, 9.42 mmol), water (5 mL), and acetic acid (50 mL)were added to a reaction flask, followed by the portionwise addition of NCS (3.77 g, 28.27 mmol). The mixture was stirred at room temperature for 0.5 h. DCM (100 mL) was added. The organic phase was washed twice with water (100 mL), washed with saturated aqueous sodium chloride solution (100 mL), dried over anhydrous sodium sulfate, and subjected to suction filtration. Methylamine hydrochloride (4.66 g, 69.03 mmol) and DIEA (5 mL) were added to the resulting dichloromethane solution at room temperature, and the mixture was stirred at room temperature for 2 h. The mixture was subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 20% to 55% acetonitrile gradient elution over 20 min; flow rate: 30 mL/min) to obtain 200 mg of the title compound **258-3.**

### Preparation of compound 258-4

Compound**258-3** (100 mg, 351.70 μmol), 10% Pd/C (42.7 mg, 35.17 μmol,), and methanol (6 mL)were added to a reaction flask. The system was purged three times with hydrogen and stirred at 50°C for 16 h. The mixture was filtered, subjected to rotary evaporation until dryness, and used directly in the next step. LC-MS (ESI): m/z 151.0 $[M+H]^+$.

### Preparation of compound 258

Compound **258-4** (41 mg, 270.82 μmol), compound **15-2** (100 mg, 180.55 μmol), DIPEA(233 mg, 1.81 mmol, 314.5 μL), and acetonitrile (927 μL)were added to a reaction flask, and the mixture was stirred at 90°C for 2 h. The mixture was subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 85% acetonitrile gradient elution over 12 min; flow rate: 30 mL/min) to obtain 60 mg of compound **258.** LC-MS (ESI): m/z 667.2 $[M+H]^+$. $^1$H NMR (400 MHz, DMSO-$d_6$) δ 8.45 (d, *J* = 3.0 Hz, 1H), 8.43 (d, *J* = 5.0 Hz, 1H), 7.95 (d, *J* = 8.5 Hz, 2H), 7.89 (d, *J* = 8.8 Hz, 1H), 7.70 (d, *J* = 2.3 Hz, 1H), 7.62 (d, *J* = 2.4 Hz, 1H), 7.53 (dd, *J* = 8.8, 3.0 Hz, 1H), 7.33 (d, *J* = 8.5 Hz, 2H), 7.31 - 7.24 (m, 1H), 6.89 (d, *J* = 5.0 Hz, 1H), 5.17 (s, 2H), 4.42 (t, *J* = 5.2 Hz, 2H), 4.39 - 4.25 (m, 3H), 4.22 - 4.10 (m, 2H), 3.95 (t, *J* = 5.2 Hz, 2H), 2.62 (d, *J* = 4.8 Hz, 3H), 1.68 (s, 6H).

### Example 259: Preparation of compound 259

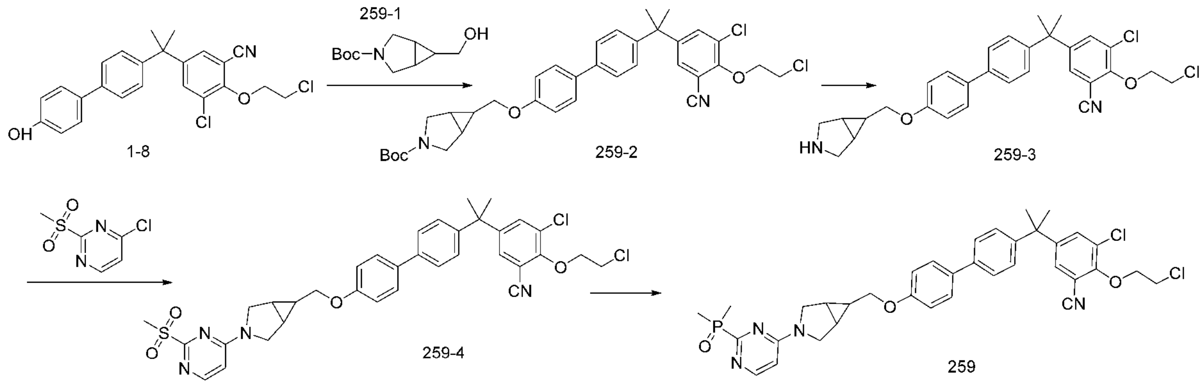

1-8, 259-2, 259-3, 259-4, 259

### Preparation of compound 259-2

Compound **1-8** (700 mg, 1.64 mmol) and compound **259-1** (628.9 mg, 2.46 mmol) were dissolved in toluene (10 mL), and cyanomethylene(tri-n-butyl)phosphorane (792.6 mg, 3.28 mmol) was added. The system was purged three times with nitrogen. The reaction mixture was stirred at 100°C for 16 h. LCMS indicated the disappearance of the starting material and the formation of the product. The reaction mixture was subjected to rotary evaporation until dryness and purified by silica gel column chromatography (EA/PE = 0 to 10%) to obtain 1 g of the title compound **259-2.** LC-MS (ESI): m/z 565.2 $[M\text{-}t\text{-}Bu+H]^+$.

### Preparation of compound 259-3

Compound **259-2** (1 g, 1.51 mmol) was dissolved in dichloromethane (10 mL), and trifluoroacetic acid (859 mg, 7.53 mmol) was added dropwise. The reaction mixture was stirred at room temperature for 12 h. LCMS indicated the disappearance of the starting material and the formation of the product. The reaction mixture was poured into saturated sodium carbonate solution (30 mL) and extracted three times with dichloromethane (30 mL). The organic phases were combined, washed with brine (30 mL), dried over anhydrous sodium sulfate, filtered, and the filtrate was subjected to rotary evaporation until dryness to obtain 800 mg of the title compound **259-3.** The crude product was used directly in the next step without purification. LC-MS (ESI): m/z 521.4 $[M+H]^+$.

### Preparation of compound 259-4

Compound **259-3** (1 g, 1.77 mmol) and 4-chloro-2-(methylsulfonyl)pyrimidine (410.2 mg, 2.13 mmol) were dissolved in dichloromethane (10 mL), and DIPEA (458.7 mg, 3.55 mmol, 619 μL) was added. The reaction mixture was stirred at 0°C for 2 h. LCMS indicated the disappearance of the starting material and the formation of the product. The reaction mixture was subjected to rotary evaporation until dryness. The resulting residue was purified by silica gel column chromatography (EA/PE = 0 to 75%) to obtain 450 mg of the title compound **259-4.** LC-MS (ESI): m/z 677.2 $[M+H]^+$.

### Preparation of compound 259

Compound **259-4** (70 mg, 103.30 μmol) and dimethylphosphine oxide (40.3 mg, 516.50 μmol) were dissolved in acetonitrile (2 mL), and cesium carbonate (101 mg, 309.90 μmol) was added. The reaction mixture was heated under microwave irradiation at 90°C for 4 h. LCMS indicated the formation of the product. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 21.2 mg of the title compound 259. LC-MS (ESI): m/z 675.2 $[M+H]^+$. $^1H$ NMR (400 MHz, DMSO-$d_6$) δ 8.26 (d, *J* = 6.1 Hz, 1H), 7.70 (d, *J* = 2.3 Hz, 1H), 7.64 (d, *J* = 2.3 Hz, 1H), 7.57 (d, *J* = 9.2 Hz, 2H), 7.55 (d, *J* = 8.9 Hz, 2H), 7.30 (d, *J* = 8.4 Hz, 2H), 7.01 (d, *J* = 8.8 Hz, 2H), 6.57 (dd, *J* = 6.2, 3.1 Hz, 1H), 4.42 (t, *J* = 5.8, 4.3 Hz, 2H), 4.03 - 3.88 (m, 5H), 3.72 - 3.59 (m, 1H), 3.58 - 3.47 (m, 2H), 1.90 - 1.80 (m, 2H), 1.68 (s, 6H), 1.65 (d, *J*= 13.6 Hz, 6H), 1.14 - 1.07 (m, 1H). $^{31}P$ NMR (162 MHz, DMSO-$d_6$) δ 33.48 (s, 1P).

### Example 260: Preparation of compound 260

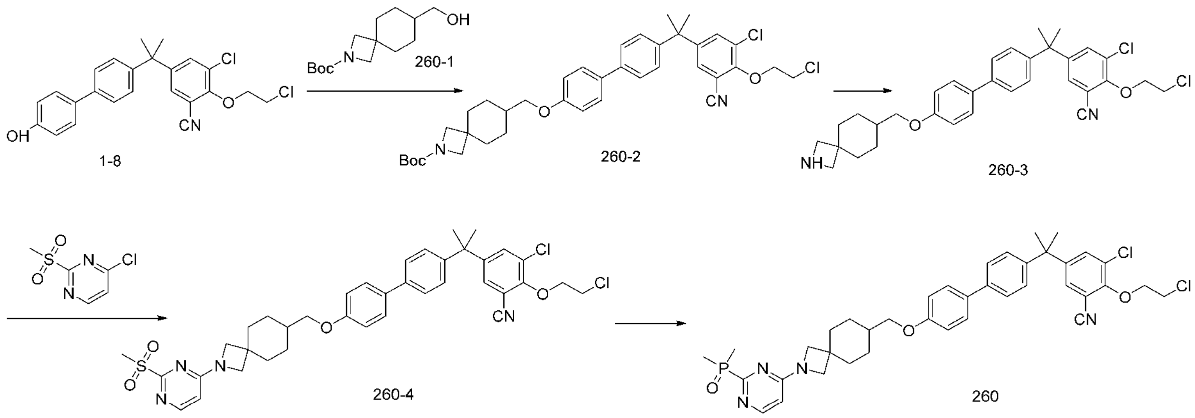

**Preparation of compound 260-2**

Compound **1-8** (700 mg, 1.64 mmol) and compound **260-1** (628.9 mg, 2.46 mmol) were dissolved in toluene (10 mL), and cyanomethylene(tri-n-butyl)phosphorane (792.6 mg, 3.28 mmol) was added. The system was purged three times with nitrogen. The reaction mixture was stirred at 100°C for 16 h. LCMS indicated the disappearance of the starting material and the formation of the product. The reaction mixture was subjected to rotary evaporation until dryness. The resulting residue was purified by silica gel column chromatography (EA/PE = 0 to 10%) to obtain 1 g of the title compound **260-2.** LC-MS (ESI): m/z 563.2 $[M-100+H]^+$.

**Preparation of compound 260-3**

Compound **260-2** (1 g, 1.51 mmol) was dissolved in dichloromethane (10 mL), and trifluoroacetic acid (859 mg, 7.53 mmol) was added dropwise. The reaction mixture was stirred at room temperature for 12 h. LCMS indicated the disappearance of the starting material and the formation of the product. The reaction mixture was poured into saturated sodium carbonate solution (30 mL) and extracted three times with dichloromethane (30 mL). The organic phases were combined, washed with saturated brine (30 mL), dried over anhydrous sodium sulfate, subjected to suction filtration, and the filtrate was subjected to rotary evaporation until dryness to obtain 800 mg of a crude product of the title compound **260-3.** The crude product was used directly in the next step without purification. LC-MS (ESI): m/z 563.2 $[M+H]^+$.

**Preparation of compound 260-4**

Compound **260-3** (1 g, 1.77 mmol) and compound 4-chloro-2-(methylsulfonyl)pyrimidine (410.2 mg, 2.13 mmol) were dissolved in dichloromethane (10 mL), and DIPEA (458.7 mg, 3.55 mmol, 619 μL) was added. The reaction mixture was stirred at 0°C for 2 h. LCMS indicated the disappearance of the starting material and the formation of the product. The reaction mixture was subjected to rotary evaporation until dryness. The resulting residue was purified by silica gel column chromatography (EA/PE = 0 to 75%) to obtain 450 mg of the title compound **260-4.** LC-MS (ESI): m/z 719.2 $[M+H]^+$.

**Preparation of compound 260**

Compound **260-4** (100 mg, 138.94 μmol) and dimethylphosphine oxide (54.2 mg, 694.72 μmol) were dissolved in acetonitrile (2 mL), and cesium carbonate (135.8 mg, 416.83 μmol) was added. The reaction mixture was heated under microwave irradiation at 90°C for 4 h. LCMS indicated the formation of the product. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 28.65 mg of the title compound **260.** LC-MS (ESI): m/z 717.2 $[M+H]^+$. $^1H$ NMR (400 MHz, DMSO-$d_6$) δ 8.25 (d, *J* = 6.0 Hz, 1H), 7.70 (d, *J* = 2.3 Hz, 1H), 7.64 (d, *J* = 2.3 Hz, 1H), 7.56 (t, *J* = 8.6 Hz, 4H), 7.30 (d, *J* = 8.5 Hz, 2H), 7.00 (d, *J* = 8.8 Hz, 2H), 6.45 (dd, *J* = 6.0, 3.1 Hz, 1H), 4.42 (t, *J* = 5.2 Hz, 2H), 3.96 (t, *J* = 5.2 Hz, 2H), 3.84 (d, *J*= 6.0 Hz, 2H), 3.79 (s, 2H), 3.72 (s, 2H), 2.00 - 1.90 (m, 2H), 1.85 - 1.73 (m, 3H), 1.68 (s, 6H), 1.64 (d, *J* = 13.5 Hz, 6H), 1.59 - 1.49 (m, 2H), 1.21 - 1.09 (m, 2H). $^{31}P$ NMR (162 MHz, DMSO-*d6*) δ 33.30 (s, 1P).

**Example 261: Preparation of compound 261**

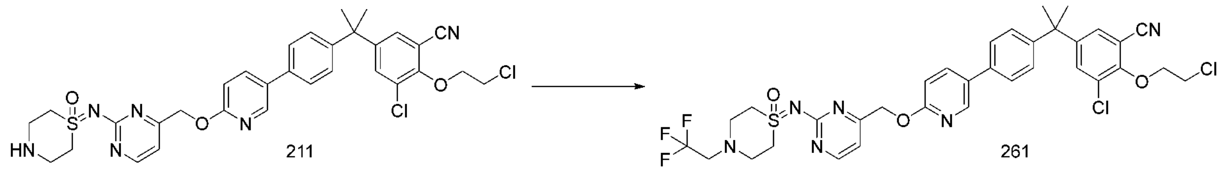

211

261

**Preparation of compound 261**

Compound **211** (30 mg, 0.05 mmol) and DIPEA (29.8 mg, 0.23 mmol) were weighed and added to DMF (2 mL), stirred for 5 min, and then 2,2,2-trifluoroethyl trifluoromethanesulfonate (21.4 mg, 0.09 mmol) was added. After the addition was completed, the reaction was carried out at room temperature for 5 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5 mg of the title compound **261.** LC-MS (ESI): m/z 733.6 $[M+H]^+$. $^1H$ NMR (400 MHz, Chloroform-*d*) δ 8.42 (d, *J* = 5.1 Hz, 1H), 8.34 (d, *J* = 2.3 Hz, 1H), 7.84 (dd, *J* = 8.6, 2.5 Hz, 1H), 7.51 - 7.47 (m, 1H), 7.47 (d, *J* = 8.9 Hz, 2H), 7.36 (d, *J* = 2.3 Hz, 1H), 7.25 (d, *J* = 8.5 Hz, 2H), 6.99 - 6.90 (m, 2H), 5.43 (s, 2H), 4.43 (t, *J* = 6.1 Hz, 2H), 3.88 (t, *J* = 6.2 Hz, 2H), 3.82 - 3.70 (m, 2H), 3.55 - 3.44 (m, 2H), 3.37 - 3.24 (m, 4H), 3.16 (q, *J* = 9.2 Hz, 2H), 1.70 (s, 6H). $^{19}F$ NMR (376 MHz, Chloroform-*d*) δ -69.72 (s, 3H).

**Example 262: Preparation of compound 262**

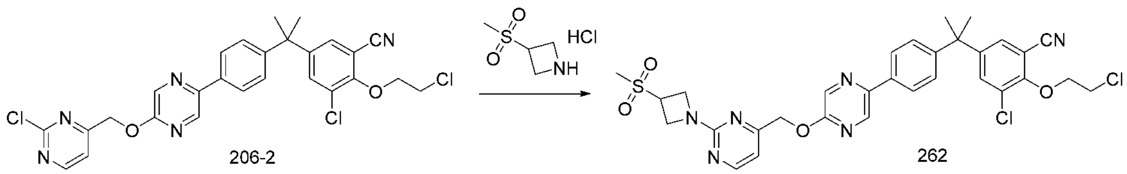


206-2

262

**Preparation of compound 262**

Compound **206-2** (25 mg, 0.05 mmol) was added to a microwave tube, followed by the sequential addition of anhydrous ethanol (2 mL), compound 3-(methylsulfonyl)azetidine hydrochloride (11.6 mg, 0.07 mmol), and DIPEA (17.5 mg, 0.14 mmol). The system was purged three times with nitrogen, and the reaction was carried out under microwave irradiation at 90°C for 2 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 13 mg of the title compound **262.** LC-MS (ESI): m/z 653.5 $[M+H]^+$. $^1H$ NMR (400 MHz, DMSO-$d_6$) δ 8.75 (d, *J* = 1.4 Hz, 1H), 8.54 (d, *J* = 1.4 Hz, 1H), 8.38 (d, *J* = 5.1 Hz, 1H), 7.96 (d, *J* = 8.5 Hz, 2H), 7.70 (d, *J* = 2.3 Hz, 1H), 7.63 (d, *J* = 2.3 Hz, 1H), 7.37 (d, *J* = 8.5 Hz, 2H), 6.79 (d, *J*= 5.0 Hz, 1H), 5.37 (s, 2H), 4.42 (t, *J* = 5.2 Hz, 2H), 4.41 - 4.35 (m, 1H), 4.31 (t, *J* = 8.8 Hz, 2H), 4.25 - 4.15 (m, 2H), 3.95 (t, *J* = 5.2 Hz, 2H), 3.05 (s, 3H), 1.69 (s, 6H).

**Examples 263 and 264: Preparation of compounds 263 and 264**

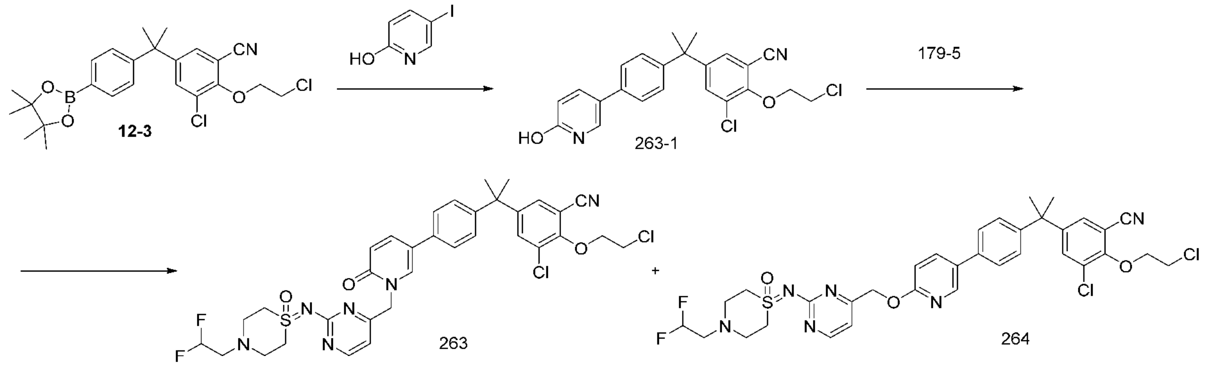


12-3 263-1 263 + 264

**Preparation of compound 263-1**

Compound **12-3** (0.5 g, 2.26 mmol), compound 2-hydroxy-5-iodopyridine (1.04 g, 2.26 mmol), $Pd(dppf)Cl_2$ (165.6 mg, 0.23 mmol), and potassium carbonate (780.6 mg, 5.66 mmol) were weighed and added to a mixed solvent of 1,4-dioxane (15 mL) and water (5 mL). The system was purged three times with nitrogen, and the reaction was carried out at 90°C for 4 h. After the reaction was monitored by LCMS until completion, the reaction mixture was poured into ice water and extracted twice with ethyl acetate (20 mL). The organic phases were combined, dried over anhydrous sodium sulfate, subjected to suction filtration, and the filtrate was concentrated to obtain a crude product. The crude product was purified by normal-phase silica gel column chromatography (EA/PE = 0 to 40%) to obtain 80 mg of the title compound **263-1** in 8% yield. LC-MS (ESI): m/z 427.2 $[M+H]^+$.

**Preparation of compounds 263 and 264**

Compound **263-1** (80 mg, 0.19 mmol) was added to a three-necked flask, followed by the sequential addition of anhydrous DCM (5 mL), compound **179-5** (57.4 mg, 0.19 mmol), and N,N,N,N-tetramethylazodicarboxamide (64.5 mg, 0.37 mmol). The system was purged three times with nitrogen, and tributylphosphine (75.8 mg, 0.37 mmol) was added at 0°C. The reaction was stirred at 25°C for 3 h. After the reaction was completed, water (20 mL) was added, and the mixture was extracted twice with DCM (20 mL). The organic phases were combined, dried, subjected to suction filtration, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **263** and 5 mg of the title compound **264,** respectively.

Compound **263:** LC-MS (ESI): m/z 715.2 $[M+H]^+$. $^1H$ NMR (400 MHz, DMSO-$d_6$) δ 8.38 (d, *J* = 5.1 Hz, 1H), 8.19 (d, *J* = 2.7 Hz, 1H), 7.88 (dd, *J* = 9.5, 2.7 Hz, 1H), 7.68 (d, *J* = 2.3 Hz, 1H), 7.61 (d, *J* = 2.3 Hz, 1H), 7.53 (d, *J* = 8.5 Hz, 2H), 7.31 (d, *J* = 8.5 Hz, 2H), 6.77 (d, *J* = 5.1 Hz, 1H), 6.52 (d, *J* = 9.5 Hz, 1H), 6.08 (tt, *J* = 55.7, 4.2 Hz, 1H), 5.13 (s, 2H), 4.42 (t, *J* = 5.2 Hz, 2H), 3.95 (t, *J* = 5.2 Hz, 2H), 3.59 - 3.45 (m, 2H), 3.39 - 3.35 (m, 2H), 3.11 - 2.83 (m, 6H), 1.67 (s, 6H). $^{19}F$ NMR (376 MHz, DMSO-$d_6$) δ -119.55 (s, 2H).

Compound **264:** LC-MS (ESI): m/z 715.2$[M+H]^+$. $^1H$ NMR (400 MHz, DMSO-$d_6$) δ 8.43 (d, *J* = 2.6 Hz, 1H), 8.41 (d, *J* = 5.1 Hz, 1H), 8.07 (dd, *J* = 8.6, 2.6 Hz, 1H), 7.71 (d, *J* = 2.3 Hz, 1H), 7.65 (d, *J* = 2.3 Hz, 1H), 7.61 (d, *J* = 8.5 Hz, 2H), 7.33 (d, *J* = 8.5 Hz, 2H), 7.07 (d, *J* = 8.6 Hz, 1H), 6.89 (d, *J* = 5.1 Hz, 1H), 6.11 (tt, *J* = 55.7, 4.3 Hz, 1H), 5.34 (s, 2H), 4.42 (t, *J* = 5.2 Hz, 2H), 3.96 (t, *J* = 5.2 Hz, 2H), 3.71 - 3.58 (m, 2H), 3.51 - 3.41 (m, 2H), 3.19 - 3.08 (m, 2H), 3.09 - 2.90 (m, 4H), 1.68 (s, 6H). $^{19}F$ NMR (376 MHz, DMSO-$d_6$) δ -119.59 (s, 2F).

**Example 265: Preparation of compound 265**

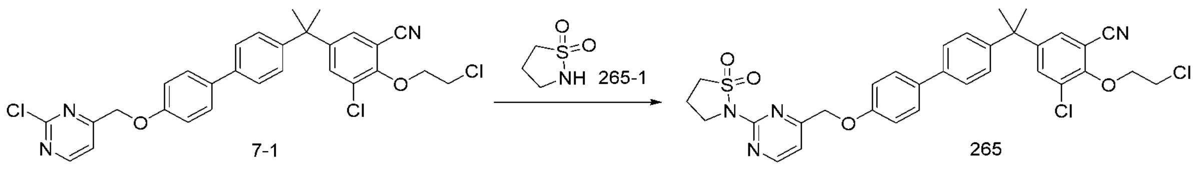


7-1 265

**Preparation of compound 265**

Compound **7-1** (150 mg, 0.27 mmol), compound **265-1** (65 mg, 0.54 mmol), DIEA (150 mg, 1.17 mmol), Xantphos (5.16 mg, 0.019 mmol), $Pd_2(dba)_3$ (8.16 mg, 9.92 mmol), and DMF (3 mL) were added to a microwave tube. The reaction was carried out under microwave irradiation at 120°C for 2 h under a nitrogen atmosphere. The mixture was filtered, and the resulting crude product was purified by preparative liquid chromatography (preparative method: mobile phase: A: 0.1% formic acid in water; B: acetonitrile; chromatographic column: Agilent 10 Prep-C18 250 × 21.2 mm; column temperature: 25°C; gradient: 5% to 80% acetonitrile gradient elution over 30 min; flow rate: 30 mL/min) to obtain 14 mg of title compound **265.** LC-MS (ESI): m/z 637.2 $[M+H]^+$. $^1H$ NMR (400 MHz, DMSO-$d_6$) δ 8.64 (d, *J* = 5.1 Hz, 1H), 7.70 (d, *J* = 2.3 Hz, 1H), 7.64 (d, *J* = 2.3 Hz, 1H), 7.60 (d, *J* = 8.8 Hz, 2H), 7.56 (d, *J* = 8.4 Hz, 2H), 7.30 (d, *J* = 8.5 Hz, 2H), 7.22 (d, *J* = 5.0 Hz, 1H), 7.11 (d, *J* = 8.9 Hz, 2H), 5.18 (s, 2H), 4.42 (t, *J* = 5.2 Hz, 2H), 3.97 - 3.91 (m, 2H), 3.96 (t, *J* = 5.2 Hz, 2H), 3.56 (t, *J* = 7.1 Hz, 2H), 2.41 - 2.29 (m, 2H), 1.68 (s, 6H).

**Example 266: Preparation of compound 266**

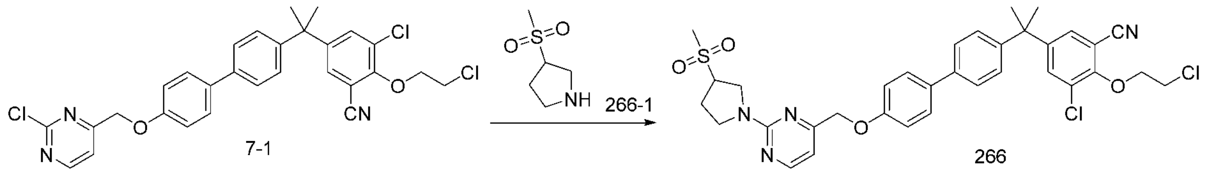


**Preparation of compound 266**

Compound **7-1** (130 mg, 0.23 mmol) was dissolved in EtOH (10 mL), and DIEA (129 mg, 1 mmol) and **266-1** (74.5 mg, 0.50 mmol) were added. The reaction was carried out at 80°C for 1 h. After the reaction was monitored by LCMS until completion, the reaction mixture was filtered. The crude filtrate was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 79.1 mg of the title compound **266.** LC-MS (ESI): m/z 665.2 $[M+H]^+$. $^1H$ NMR (400 MHz, DMSO-$d_6$) δ 8.39 (d, *J* = 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.5 Hz, 2H), 7.30 (d, *J* = 8.5 Hz, 2H), 7.08 (d, *J*= 8.9 Hz, 2H), 6.76 (d, *J*= 5.0 Hz, 1H), 5.08 (s, 2H), 4.42 (t, *J* = 5.2 Hz, 2H), 4.15 - 4.03 (m, 1H), 3.96 (t, *J* = 5.2 Hz, 2H), 3.96 - 3.79 (m, 2H), 3.74 - 3.63 (m, 1H), 3.64 - 3.54 (m, 1H), 3.07 (s, 3H), 2.46 - 2.32 (m, 2H), 1.68 (s, 6H).

**Example 267: Preparation of compound 267**

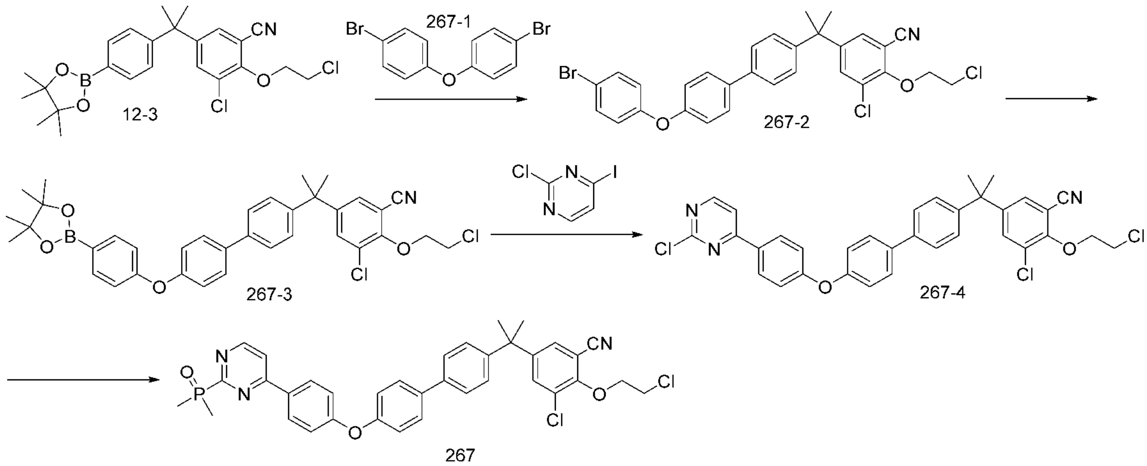

**Preparation of compound 267-2**

Compound **12-3** (3 g, 6.52 mmol) and compound **267-1** (2.14 g, 6.52 mmol) were weighed and dissolved in 1,4-dioxane (20 mL) and water (5 mL). Pd(dppf)$Cl_2$ (476.99 mg, 651.89 μmol) and potassium carbonate (2.25 g, 16.30 mmol) were added. After the system was purged three times with nitrogen, the reaction mixture was heated at 100°C for 16 h. LCMS indicated the disappearance of the starting material and the formation of the product. The reaction mixture was subjected to rotary evaporation until dryness and then purified by silica gel column chromatography (EA/PE = 0 to 20%) to obtain 2.3 g of the title compound **267-2.**

**Preparation of compound 267-3**

Compound **267-2** (2.1 g, 3.61 mmol) and bis(pinacolato)diboron (1.83 g, 7.22 mmol) were dissolved in 1,4-dioxane (30 mL). Potassium acetate (1.06 g, 10.84 mmol) and Pd(dppf)$Cl_2$ (264.3 mg, 361.24 μmol) were added. The system was purged three times with nitrogen. The reaction mixture was stirred at 110°C for 16 h. LCMS indicated the disappearance of the starting material and the formation of the product. The reaction mixture was subjected to rotary evaporation until dryness and then purified by silica gel column chromatography (EA/PE = 0 to 10%) to obtain 800 mg of the title compound **267-3.**

**Preparation of compound 267-4**

Compound **267-3** (250 mg, 397.84 μmol) and 2-chloro-4-iodopyrimidine (87 mg, 361.67 μmol) were dissolved in 1,4-dioxane (5 mL) and water (2 mL). Pd(dppf)$Cl_2$ (26.5 mg, 36.17 μmol) and potassium carbonate (125 mg, 904.19 μmol) were added. The system was purged three times with nitrogen. The reaction mixture was heated at 100°C for 2 h. LCMS indicated the disappearance of the starting material and the formation of the product. The reaction mixture was subjected to rotary evaporation until dryness and then purified by column chromatography (EA/PE = 0 to 30%) to obtain 130 mg of the title compound **267-4.** LC-MS (ESI): m/z 614.0 $[M+H]^+$.

**Preparation of compound 267**

Compound **267-4** (130 mg, 211.40 μmol) was dissolved in DMF (445 μL), followed by the sequential addition of $Pd_2(dba)_3$ (19.4 mg, 21.14 μmol), Xantphos (24.5 mg, 42.28 μmol), DIPEA (54.6 mg, 422.80 μmol, 73.64 μL), and dimethylphosphine oxide (82.50 mg, 1.06 mmol). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 60.34 mg of the title compound **267.** LC-MS (ESI): m/z 656.2 $[M+H]^+$. $^1H$ NMR (400 MHz, DMSO-$d_6$) δ 8.99 (d, *J* = 5.5 Hz, 1H), 8.33 *(d, J*= 8.8 Hz, 2H), 8.17 (q, *J* = 5.5, 3.2 Hz, 1H), 7.73 (d, *J* = 8.7 Hz, 2H), 7.72 (d, *J* = 2.3 Hz, 1H), 7.65 (d, *J* = 2.3 Hz, 1H), 7.62 (d, *J* = 8.4 Hz, 2H), 7.3 5 (d, *J* = 8.5 Hz, 2H), 7.22 (d, *J*= 8.7 Hz, 2H), 7.21 (d, *J* = 8.5 Hz, 2H), 4.43 (t, *J* = 5.2 Hz, 2H), 3.96 (t, *J* = 5.2 Hz, 2H), 1.82 (d, *J*= 13.7 Hz, 6H), 1.70 (s, 6H). $^{31}P$ NMR (162 MHz, DMSO-*d6*) δ 34.33 (s, 1P).

**Example 268: Preparation of compound 268**

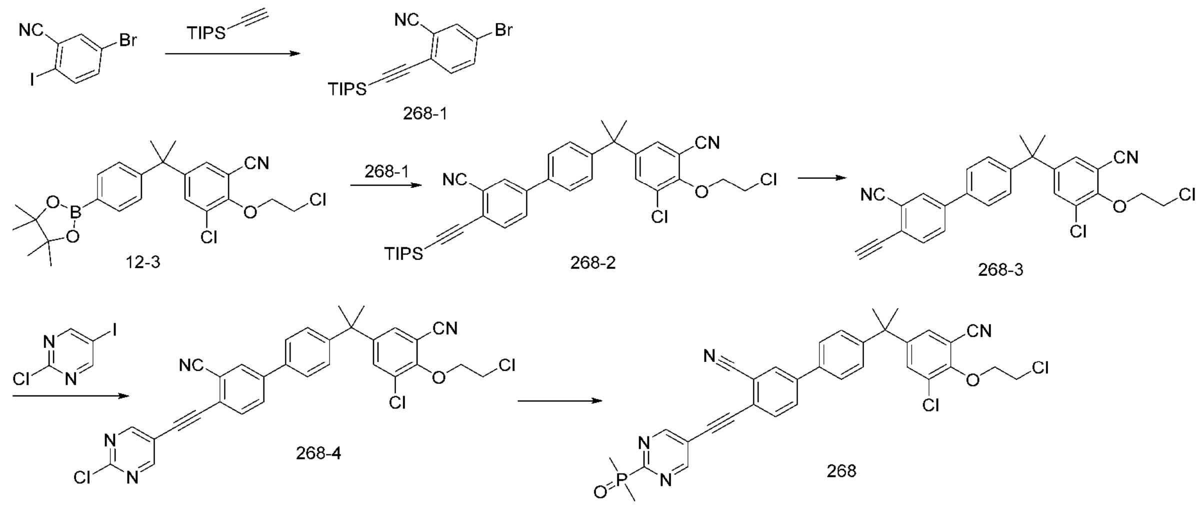


268-1, 12-3, 268-2, 268-3, 268-4, 268

**Preparation of compound 268-1**

Compound 5-bromo-2-iodobenzonitrile (3 g, 9.74 mmol) was dissolved in tetrahydrofuran (50 mL). Triisopropylsilylacetylene (1.78 g, 9.74 mmol), DIEA (6.3 g, 48.72 mmol), copper(I) iodide (0.371 g, 1.95 mmol), and bis(triphenylphosphine)palladium(II) chloride (1.13 g, 0.974 mmol) were added thereto. After the addition was completed, the reaction system was stirred at 50°C for 1 h. After the reaction was completed, the reaction mixture was filtered, and the filtrate was concentrated to obtain a crude product. The crude product was purified by silica gel column chromatography (PE/EA = 100% to 85%) to obtain 2.6 g of the title compound **268-1.** $^1$H NMR (400 MHz, DMSO-$d_6$) δ 8.24 (d, *J* = 2.1 Hz, 1H), 7.92 (dd, *J* = 8.5, 2.2 Hz, 1H), 7.62 (d, *J* = 8.4 Hz, 1H), 1.13 - 1.08 (m, 21H).

**Preparation of compound 268-2**

Compound **268-1** (1.57 g, 4.35 mmol), compound **12-3** (2 g, 4.35 mmol), and sodium carbonate (1.38 g, 13.04 mmol) were dissolved in 1,4-dioxane (50 mL) and water (10 mL), and tetrakis(triphenylphosphine)palladium (355 mg, 0.435 mmol) was added thereto. After the addition was completed, the reaction system was stirred at 90°C for 12 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 75%) to obtain 2.2 g of the title compound **268-2.** $^1$H NMR (400 MHz, Chloroform-*d*) δ 7.85 (d, *J* = 1.9 Hz, 1H), 7.73 (dd, *J* = 8.2, 1.9 Hz, 1H), 7.64 (d, *J* = 8.2 Hz, 1H), 7.50 (d, *J* = 8.4 Hz, 2H), 7.48 (d, *J* = 2.3 Hz, 1H), 7.36 (d, *J* = 2.3 Hz, 1H), 7.29 (d, *J* = 8.4 Hz, 2H), 4.43 (t, *J* = 6.1 Hz, 2H), 3.88 (t, *J* = 6.1 Hz, 2H), 1.70 (s, 6H), 1.20 - 1.14 (m, 21H).

**Preparation of compound 268-3**

Compound **268-2** (2.2 g, 3.57 mmol) was dissolved in tetrahydrofuran (20 mL), and TBAF (7.2 mL, 1 mol/L in THF) was added dropwise thereto. After the addition was completed, the reaction system was stirred at room temperature for 12 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 75%) to obtain 1.6 g of the title compound **268-3.** $^1$H NMR (400 MHz, Chloroform-*d*) δ 7.86 (dd, *J* = 1.9, 0.5 Hz, 1H), 7.76 (dd, *J* = 8.2, 1.9 Hz, 1H), 7.67 (d, *J* = 8.2 Hz, 1H), 7.51 (d, *J* = 8.5 Hz, 2H), 7.47 (d, *J* = 2.4 Hz, 1H), 7.35 (d, *J* = 2.3 Hz, 1H), 7.29 (d, *J* = 8.5 Hz, 2H), 4.43 (t, *J* = 6.1 Hz, 2H), 3.88 (t, *J* = 6.1 Hz, 2H), 3.52 (s, 1H), 1.70 (s, 6H).

**Preparation of compound 268-4**

Compound **268-3** (500 mg, 1.09 mmol), 2-chloro-5-iodopyrimidine (314 mg, 1.31 mmol), bis(triphenylphosphine) palladium(II) chloride (76 mg, 0.108 mmol), copper(I) iodide (21 mg, 0.108 mmol), and DIPEA (0.703 g, 5.44 mmol) were placed in a single-necked flask, and acetonitrile (10 mL) was added. The system was purged three times with nitrogen. The reaction system was stirred at 50°C for 2 h. After the reaction was completed, saturated sodium chloride solution (50 mL)

was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 85%) to obtain 500 mg of the title compound **268-4.** LC-MS (ESI): m/z 571.3 $[M+H]^+$.

**Preparation of compound 268**

Compound **268-4** (200 mg, 0.349 mmol), dimethylphosphine oxide (82 mg, 1.05 mmol), $Pd_2(dba)_3$ (32 mg, 0.035 mmol), Xantphos (20 mg, 0.035 mmol), and DIPEA (136 mg, 1.05 mmol) were dissolved in ethanol (5 mL). The reaction system was stirred at 110°C for 0.5 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 22 mg of the title compound **268.** LC-MS (ESI): m/z 613.2 $[M+H]^+$. $^1H$ NMR (400 MHz, Chloroform-*d*) δ 9.04 (s, 2H), 7.93 (d, *J* = 1.9 Hz, 1H), 7.84 (dd, *J* = 8.3, 1.9 Hz, 1H), 7.75 (d, *J* = 8.2 Hz, 1H), 7.54 (d, *J* = 8.1 Hz, 2H), 7.47 (d, *J* = 2.4 Hz, 1H), 7.33 (d, *J* = 2.3 Hz, 1H), 7.31 (d, *J* = 8.1 Hz, 2H), 4.42 (t, *J* = 6.1 Hz, 2H), 3.87 (t, *J* = 6.1 Hz, 2H), 1.89 (d, *J* = 13.6 Hz, 6H), 1.70 (s, 6H). $^{31}P$ NMR (162 MHz, Chloroform-*d*) δ 35.39 (s, 1P).

**Example 269: Preparation of compound 269**

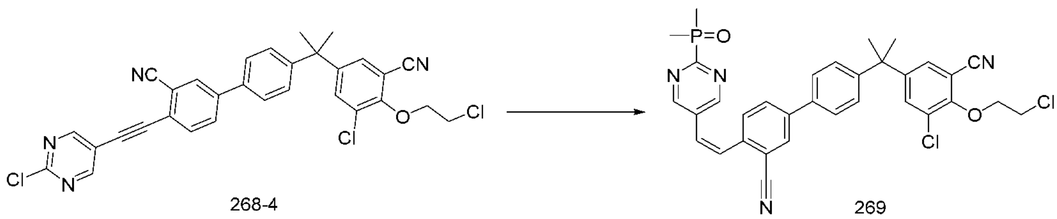

268-4

269

**Preparation of compound 269**

Compound **268-4** (200 mg, 0.349 mmol), dimethylphosphine oxide (82 mg, 1.05 mmol), $Pd_2(dba)_3$ (32 mg, 0.035 mmol), Xantphos (20 mg, 0.035 mmol), and DIPEA (136 mg, 1.05 mmol) were dissolved in ethanol (5 mL). The reaction system was stirred at 110°C for 0.5 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2.8 mg of the title compound **269.** LC-MS (ESI): m/z 615.2 $[M+H]^+$. $^1H$ NMR (400 MHz, Chloroform-*d*) δ 8.67 (s, 2H), 7.91 (d, *J* = 1.9 Hz, 1H), 7.70 (dd, *J* = 8.2, 1.9 Hz, 1H), 7.51 (d, *J* = 8.5 Hz, 2H), 7.47 (d, *J* = 2.4 Hz, 1H), 7.38 - 7.3 3 (m, 2H), 7.30 (d, *J* = 8.4 Hz, 2H), 7.15 (d, *J* = 12.2 Hz, 1H), 6.80 (d, *J* = 12.2 Hz, 1H), 4.43 (t, *J* = 6.1 Hz, 2H), 3.88 (t, *J* = 6.1 Hz, 2H), 1.86 (d, *J* = 13.4 Hz, 6H), 1.71 (s, 6H). $^{31}P$ NMR (162 MHz, Chloroform-*d*) δ 35.45 (s, 1P).

**Example 270: Preparation of compound 270**

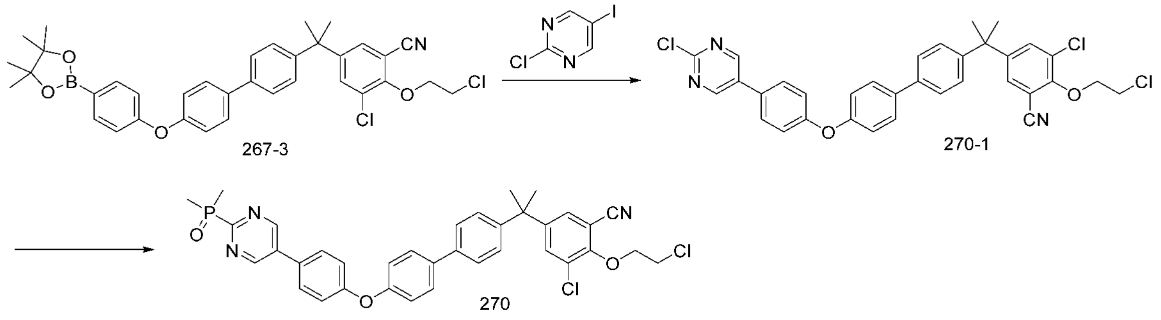

267-3

270-1

270

**Preparation of compound 270-1**

2-Chloro-5-iodopyrimidine (92 mg, 0.382 mmol), compound **267-3** (0.2 g, 0.318 mmol), and potassium carbonate (110 mg, 0.756 mmol) were dissolved in 1,4-dioxane (10 mL) and water (2 mL). Pd(dppf)$Cl_2$-DCM (26 mg, 0.032 mmol) was added thereto. After the addition was completed, the reaction system was stirred at 90°C for 12 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 75%) to obtain 110 mg of the title compound **270-1.** LC-MS (ESI): m/z 614.1 $[M+H]^+$.

**Preparation of compound 270**

Compound **270-1** (90 mg, 0.146 mmol), dimethylphosphine oxide (27 mg, 0.439 mmol), $Pd_2(dba)_3$ (13 mg, 0.014 mmol), Xantphos (8 mg, 0.014 mmol), and DIPEA (94 mg, 0.731 mmol) were dissolved in ethanol (5 mL). The reaction system was stirred at 110°C for 0.5 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 11 mg of the title compound **270.** LC-MS (ESI): m/z 656.2 $[M+H]^+$. $^1H$ NMR (400 MHz, Chloroform-*d*) δ 9.05 (s, 2H), 7.60 (d, *J* = 8.7 Hz, 2H), 7.59 (d, *J* = 8.7 Hz, 2H), 7.53 (d, *J* = 8.3 Hz, 2H), 7.50 (d, *J* = 2.3 Hz, 1H), 7.37 (d, *J* = 2.4 Hz, 1H), 7.26 (d, *J* = 8.4 Hz, 2H), 7.20 (d, *J* = 8.6 Hz, 2H), 7.15 (d, *J*= 8.7 Hz, 2H), 4.43 (t, *J* = 6.2 Hz, 2H), 3.88 (t, *J* = 6.1 Hz, 2H), 1.91 (d, *J* = 13.5 Hz, 6H), 1.71 (s, 6H). $^{31}P$ NMR (162 MHz, Chloroform-*d*) δ 34.65 (s, 1P).

**Example 271: Preparation of compound 271**

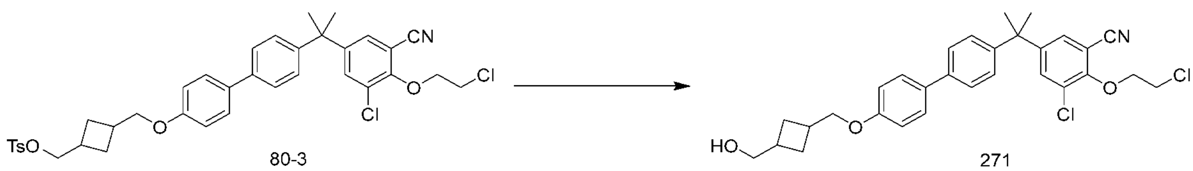

80-3

271

**Preparation of compound 271**

Compound **80-3** (75.7 mg, 111.54 μmol) was dissolved in acetonitrile (1.5 mL), and compound 2-thia-6-azaspiro [3.3]heptane 2,2-dioxide hydrochloride (22.5 mg, 122.70 μmol) and DIPEA (43.3 mg, 334.63 μmol, 58.3 μL) were added. The reaction mixture was stirred overnight at 90°C in a sealed tube. After the reaction was completed, the reaction mixture was cooled to room temperature, diluted with a small amount of acetonitrile, and purified by preparative chromatography (preparative method: mobile phase: A: 10 mM aqueous ammonium bicarbonate solution; B: acetonitrile; chromatographic column: Welch Ultimate AQ-C18 250 × 21.2 mm; column temperature: 25°C; gradient: B%: 80%-90% 8 min, 95% 6 min; flow rate: 30 mL/min) to obtain 2.19 mg of the title compound **271.** LC-MS (ESI): m/z 506.2 $[M-OH]^+$. $^1H$ NMR (400 MHz, Chloroform-*d*) δ 7.54 - 7.47 (m, 5H), 7.38 (d, *J* = 2.3 Hz, 1H), 7.24 - 7.19 (m, 2H), 7.00 - 6.92 (m, 2H), 4.43 (t, *J* = 6.2 Hz, 2H),

4.05 - 3.98 (m, 1H), 3.93 (d, *J* = 6.1 Hz, 1H), 3.88 (t, *J* = 6.2 Hz, 2H), 3.70 (d, *J* = 6.9 Hz, 1H), 3.62 (d, *J* = 6.3 Hz, 1H), 2.82 - 2.65 (m, 1H), 2.60 - 2.44 (m, 1H), 2.34 - 2.15 (m, 2H), 2.06 - 1.96 (m, 1H), 1.76 - 1.64 (m, 1H), 1.69 (s, 6H).

**Example 272: Preparation of compound 272**

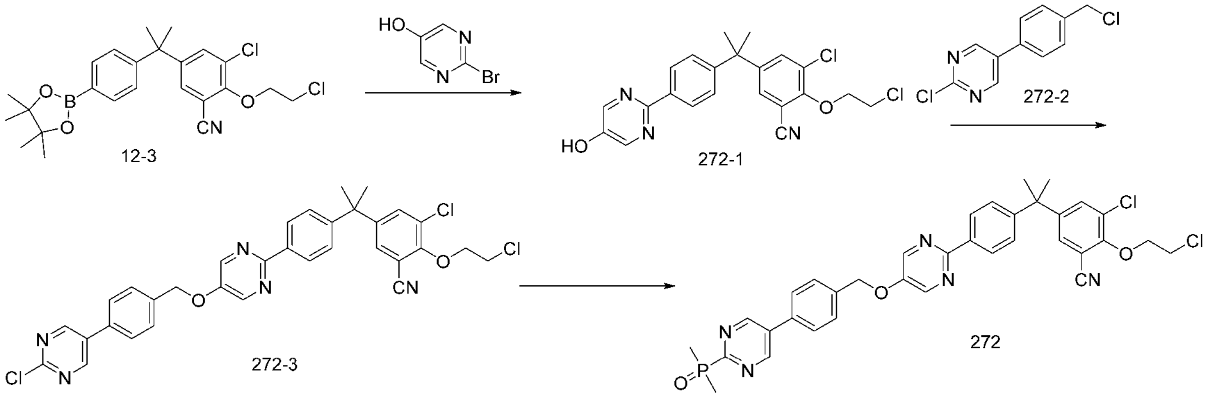
12-3
272-1
272-2
272-3
272

**Preparation of compound 272-1**

Compound **12-3** (200 mg, 0.43 mmol) was dissolved in dioxane (5 mL), followed by the sequential addition of 2-bromo-5-hydroxypyrimidine (84 mg, 0.48 mmol), potassium carbonate (180 mg, 1.30 mmol), and water (1 mL). The system was purged three times with nitrogen. Pd(dppf)$Cl_2$ (31.8 mg, 0.04 mmol) was added, and the reaction system was stirred at 110°C for 12 h. After the reaction was completed, water (25 mL) was added, and the mixture was extracted three times with dichloromethane (25 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 55%) to obtain 32 mg of the title compound **272-1.** LC-MS (ESI): m/z 428.0 $[M+H]^+$.

**Preparation of compound 272-3**

Compound **272-1** (100 mg, 0.23 mmol) was dissolved in DMF (3 mL), followed by the sequential addition of cesium carbonate (228 mg, 0.70 mmol) and compound **272-2** (61 mg, 0.26 mmol). The reaction system was stirred at 60°C for 4 h. After the reaction was completed, water (10 mL) was added, and the mixture was extracted three times with ethyl acetate (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (PE/EA = 0 to 50%) to obtain 55 mg of the title compound **272-3.** LC-MS (ESI): m/z 630.1 $[M+H]^+$.

**Preparation of compound 272**

Compound **272-3** (60 mg, 0.01 mmol) was dissolved in DMF (2 mL), followed by the sequential addition of dimethylphosphine oxide (15 mg, 0.19 mmol), DIEA (61 mg, 0.48 mmol), and Xantphos (11 mg, 0.02 mmol). The system was purged three times with nitrogen. $Pd_2(dba)_3$ (9 mg, 0.01 mmol) was added, and the reaction system was stirred under microwave irradiation at 120°C for 2 h. After the reaction was completed, insoluble solids were removed by filtration. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **272.** LC-MS (ESI): m/z 672.3 $[M+H]^+$. $^1$H NMR (400 MHz, Chloroform-*d*) δ 9.10 (s, 2H), 8.64 (s, 2H), 8.38 (d, *J* = 8.4 Hz, 2H), 7.72 - 7.62 (m, 4H), 7.44 (d, *J* = 2.4 Hz, 1H), 7.36 (d, *J* = 2.3 Hz, 1H), 7.33 (d, *J* = 8.4 Hz, 2H), 5.33 (s, 2H), 4.43 (t, *J* = 6.1 Hz, 2H), 3.88 (t, *J* = 6.1 Hz, 2H), 1.93 (d, *J* = 13.5 Hz, 6H), 1.71 (s, 6H). $^{31}$P NMR (162 MHz, Chloroform-*d*) δ 34.85 (s, 1P).

**Example 273: Preparation of compound 273**

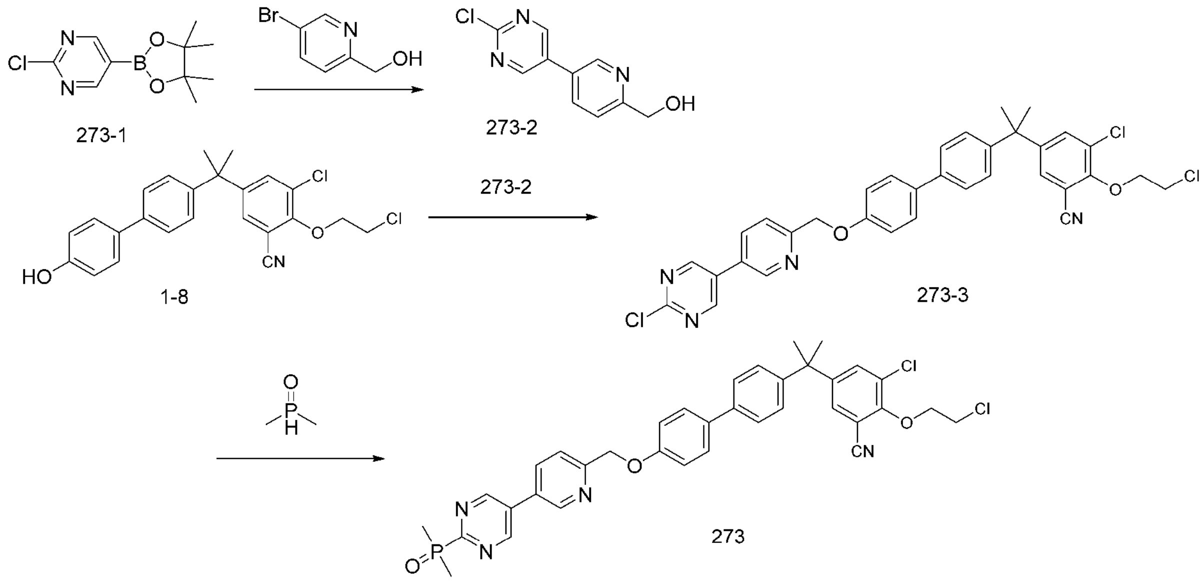

273-1
273-2
1-8
273-3
273

### Preparation of compound 273-2

(5-Bromopyridin-2-yl)methanol (752.1 mg, 4.0 mmol), compound **273-1** (1.92 g, 8.0 mmol), and sodium carbonate (847.9 mg, 8.0 mmol) were dissolved in 1,4-dioxane (16 mL) and water (4 mL), and Pd(dppf)$Cl_2$ (292.7 mg, 0.4 mmol) was added thereto. After the addition was completed, the reaction system was stirred at 100°C for 6 h. After the reaction was completed, water (50 mL) was added, and the mixture was extracted six times with dichloromethane (50 mL). The organic phases were combined, dried over anhydrous sodium sulfate, filtered, and the filtrate was concentrated to obtain a crude product. The crude product was purified by silica gel column chromatography (PE/EA = 100% to 5%) to obtain 285 mg of the title compound **273-2.** LC-MS (ESI): m/z 222.2 $[M+H]^+$.

### Preparation of compound 273-3

Compound **273-2** (222 mg, 1.0 mmol), compound **1-8** (284.7 mg, 0.668 mmol), and triphenylphosphine (350.3 mg, 1.34 mmol) were dissolved in acetonitrile (50 mL), and DIAD (269.9 mg, 1.34 mmol) was added thereto at 100°C. After the addition was completed, the reaction system was stirred at 100°C for 1 h. After the reaction was completed, the reaction mixture was concentrated to obtain a crude product. The crude product was purified by silica gel column chromatography (PE/EA = 100% to 80%) to obtain 380 mg of the title compound 273-3. LC-MS (ESI): m/z 629.2 $[M+H]^+$.

### Preparation of compound 273

Compound **273-3** (315 mg, 0.50 mmol), dimethylphosphine oxide (195 mg, 2.5 mmol), $Pd_2(dba)_3$ (45.8 mg, 0.05 mmol), Xantphos (57.8 mg, 0.10 mmol), and DIPEA (323 mg, 2.5 mmol) were dissolved in DMF (5 mL). The reaction system was stirred in a microwave reactor at 110°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 110 mg of the title compound **273.** LC-MS (ESI): m/z 671.3 $[M+H]^+$. $^1H$ NMR (400 MHz, DMSO-$d_6$) δ 9.40 (s, 2H), 9.09 (d, *J* = 2.3 Hz, 1H), 8.35 (dd, *J* = 8.2, 2.4 Hz, 1H), 7.75 - 7.67 (m, 2H), 7.64 (d, *J* = 2.3 Hz, 1H), 7.61 (d, *J* = 8.8 Hz, 2H), 7.56 (d, *J* = 8.4 Hz, 2H), 7.30 (d, *J* = 8.5 Hz, 2H), 7.13 (d, *J* = 8.8 Hz, 2H), 5.33 (s, 2H), 4.42 (t, *J* = 5.2 Hz, 2H), 3.96 (t, *J* = 5.2 Hz, 2H), 1.81 (d, *J* = 13.6 Hz, 6H), 1.68 (s, 6H). $^{31}P$ NMR (162 MHz, DMSO-$d_6$) δ 33.96 (s, 1P).

### Example 274: Preparation of compound 274

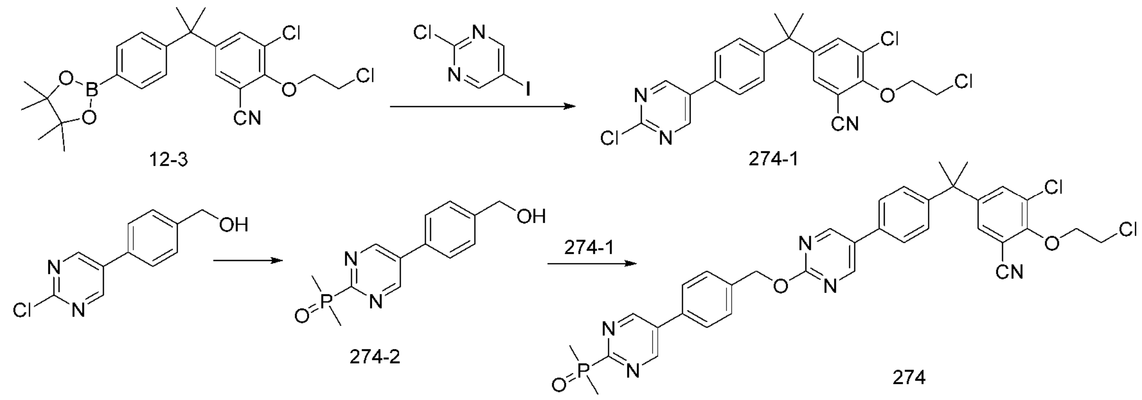


**Preparation of compound 274-1**

Compound **12-3** (200 mg, 0.43 mmol) was dissolved in dioxane (2 mL), followed by the sequential addition of 2-chloro-5-iodopyrimidine (105 mg, 0.43 mmol), potassium carbonate (120 mg, 0.87 mmol), and water (0.4 mL). The system was purged three times with nitrogen. Pd(dppf)$Cl_2$ (32 mg, 0.04 mmol) was added, and the reaction system was stirred at 110°C for 12 h. After the reaction was completed, water (25 mL) was added, and the mixture was extracted three times with ethyl acetate (25 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (PE/EA = 100 to 55%) to obtain 32 mg of the title compound **274-1.** LC-MS (ESI): m/z 446.0 $[M+H]^+$.

**Preparation of compound 274-2**

(4-(2-Chloropyrimidin-5-yl)phenyl)methanol (400 mg, 1.81 mmol) was dissolved in dioxane (10 mL), followed by the sequential addition of dimethylphosphine oxide (707 mg, 9.06 mmol), Xantphos (210 mg, 0.36 mmol), and DIPEA (702 mg, 5.44 mmol). The system was purged three times with nitrogen. $Pd_2(dba)_3$ (166 mg, 0.18 mmol) was added, and the reaction system was stirred under microwave irradiation at 130°C for 2 h. After the reaction was completed, water (25 mL) was added, and the mixture was extracted three times with dichloromethane (25 mL). The aqueous phase was concentrated to obtain 262 mg of a crude product of the title compound **274-2.** LC-MS (ESI): m/z 263.1 $[M+H]^+$.

**Preparation of compound 274**

Compound **274-2** (30 mg, 0.11 mmol) was dissolved in THF (3 mL), and the mixture was cooled to 0°C. NaH (23 mg, 0.57 mmol) was added, and the reaction system was stirred at 25°C for 0.5 h. Compound **274-1** (51 mg, 0.11 mmol) was added, and the reaction system was stirred at 70°C for 12 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **274.** LC-MS (ESI): m/z 672.2 $[M+H]^+$. $^1H$ NMR (400 MHz, DMSO-$d_6$) δ 9.33 (s, 2H), 8.96 (s, 2H), 7.91 (d, *J* = 8.3 Hz, 2H), 7.71 (d, *J* = 2.3 Hz, 1H), 7.70 - 7.62 (m, 5H), 7.38 (d, *J* = 8.4 Hz, 2H), 5.54 (s, 2H), 4.42 (t, *J* = 5.2 Hz, 2H), 3.96 (t, *J* = 5.2 Hz, 2H), 1.80 (d, *J* = 13.7 Hz, 6H), 1.69 (s, 6H). $^{31}P$ NMR (162 MHz, DMSO-$d_6$) δ 33.81 (s, 1P).

**Example 275: Preparation of compound 275**

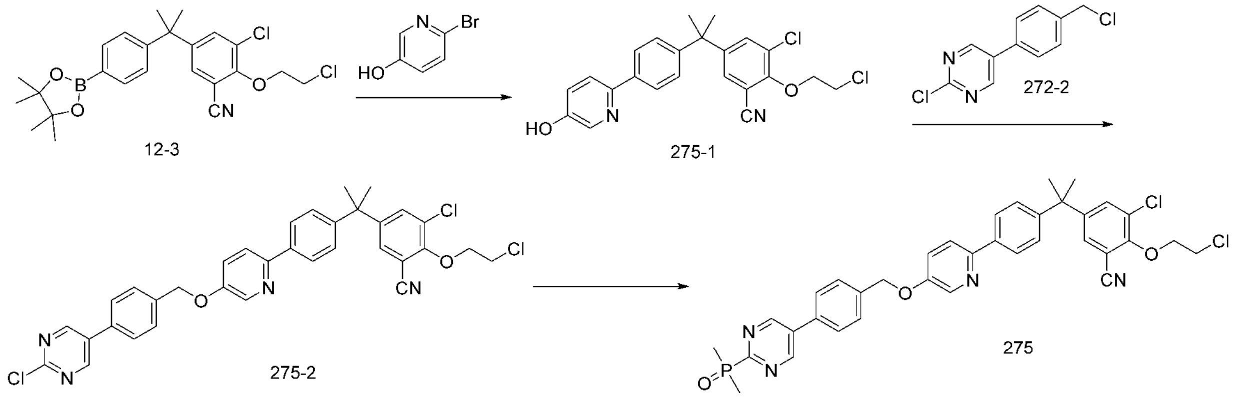

12-3 275-1 272-2 275-2 275

**Preparation of compound 275-1**

Compound **12-3** (500 mg, 1.09 mmol) was dissolved in dioxane (10 mL), followed by the sequential addition of 2-bromo-5-hydroxypyridine (189 mg, 1.09 mmol), potassium carbonate (451 mg, 3.26 mmol), and water (2 mL). The system was purged three times with nitrogen. $Pd(PPh_3)Cl_2$ (76 mg, 0.11 mmol) was added, and the reaction system was stirred at 110°C for 12 h. After the reaction was completed, water (25 mL) was added, and the mixture was extracted three times with ethyl acetate (25 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (PE/EA = 100 to 55%) to obtain 68 mg of the title compound **275-1.** LC-MS (ESI): m/z 427.2 $[M+H]^+$.

**Preparation of compound 275-2**

Compound **275-1** (100 mg, 0.24 mmol) was dissolved in DMF (10 mL). Compound **272-2** (62 mg, 0.26 mmol) and cesium carbonate (254 mg, 0.47 mmol) were added. The reaction system was stirred at 60°C for 3 h. After the reaction was completed, water (25 mL) was added, and the mixture was extracted three times with ethyl acetate (25 mL). The organic phases were combined, washed twice with saturated brine (25 mL), dried over anhydrous sodium sulfate, subjected to suction filtration, and the filtrate was concentrated to obtain a crude product. The crude product was purified by column chromatography (PE/EA = 100 to 45%) to obtain 60 mg of the title compound **275-2.** LC-MS (ESI): m/z 629.2 $[M+H]^+$.

**Preparation of compound 275**

Compound **275-2** (50 mg, 0.08 mmol) was dissolved in DMF (2 mL), followed by the sequential addition of dimethylphosphine oxide (15 mg, 0.16 mmol), DIEA (51 mg, 0.40 mmol), and Xantphos (9.2 mg, 0.02 mmol). The system was purged three times with nitrogen. $Pd_2(dba)_3$ (7 mg, 0.01 mmol) was added, and the reaction system was stirred under microwave irradiation at 120°C for 2 h. After the reaction was completed, insoluble solids were removed by filtration. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 23 mg of the title compound 275. LC-MS (ESI): m/z 671.2 $[M+H]^+$. $^1H$ NMR (400 MHz, Chloroform-d) δ 9.09 (s, 2H), 8.48 (d, *J* = 2.9 Hz, 1H), 7.89 (d, *J* = 8.2 Hz, 2H), 7.74 - 7.61 (m, 5H), 7.45 (d, *J* = 2.4 Hz, 1H), 7.37 (d, *J* = 2.3 Hz, 1H), 7.30 - 7.26 (m, 3H), 5.26 (s, 2H), 4.42 (t, *J* = 6.2 Hz, 2H), 3.87 (t, *J* = 6.2 Hz, 2H), 1.93 (d, *J* = 13.5 Hz, 6H), 1.70 (s, 6H). $^{31}P$ NMR (162 MHz, Chloroform-d) δ 34.55 (s, 1P).

**Example 276: Preparation of compound 276**

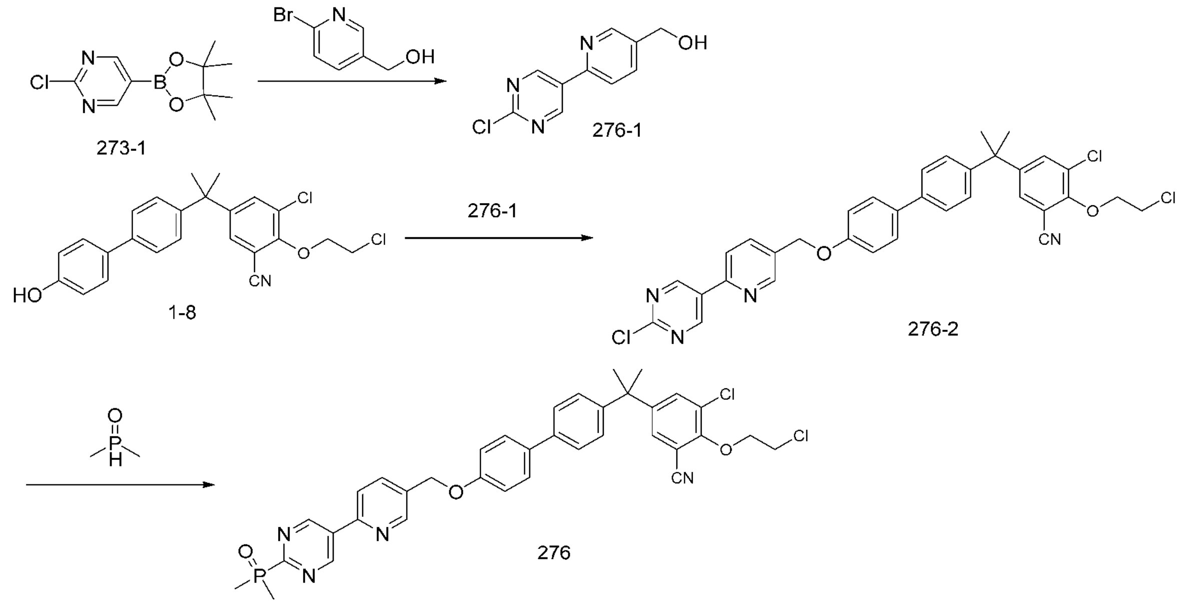


273-1

276-1

1-8

276-2

276

**Preparation of compound 276-1**

(6-Bromopyridin-3-yl)methanol (752.1 mg, 4.0 mmol), compound 273-1 (1.92 g, 8.0 mmol), and sodium carbonate (847.9 mg, 8.0 mmol) were dissolved in 1,4-dioxane (16 mL) and water (4 mL), and Pd(dppf)$Cl_2$ (292.7 mg, 0.4 mmol) was added thereto. After the addition was completed, the reaction system was stirred at 100°C for 6 h. After the reaction was completed, water (50 mL) was added, and the mixture was extracted six times with dichloromethane (50 mL). The organic phases were combined, dried over anhydrous sodium sulfate, filtered, and the filtrate was concentrated to obtain a crude product. The crude product was purified by silica gel column chromatography (PE/EA = 100% to 5%) to obtain 280 mg of the title compound **276-1.**

**Preparation of compound 276-2**

Compound **276-1** (222 mg, 1.0 mmol), compound **1-8** (284.7 mg, 0.668 mmol), and triphenylphosphine (350.3 mg, 1.34 mmol) were dissolved in acetonitrile (50 mL), and DIAD (269.9 mg, 1.34 mmol) was added thereto at 100°C. After the addition was completed, the reaction system was stirred at 100°C for 1 h. After the reaction was completed, the reaction mixture was concentrated to obtain a crude product. The crude product was purified by silica gel column chromatography (PE/EA = 100% to 80%) to obtain 370 mg of the title compound **276-2.** LC-MS (ESI): m/z 629.2 $[M+H]^+$.

**Preparation of compound 276**

Compound **276-2** (350 mg, 0.56 mmol), dimethylphosphine oxide (216.8 mg, 2.78 mmol), $Pd_2(dba)_3$ (50.9 mg, 0.06 mmol), Xantphos (64.2 mg, 0.11 mmol), and DIPEA (359 mg, 2.78 mmol) were dissolved in DMF (5 mL). The reaction system was stirred in a microwave reactor at 110°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 105 mg of the title compound **276.** LC-MS (ESI): m/z 671.2 $[M+H]^+$. $^1H$ NMR (400 MHz, DMSO-$d_6$) δ 9.61 (s, 2H), 8.89 (d, *J* = 2.3 Hz, 1H), 8.25 (dd, *J* = 8.1, 0.9 Hz, 1H), 8.11 (dd, *J* = 8.2, 2.2 Hz, 1H), 7.70 (d, *J* = 2.4 Hz, 1H), 7.64 (d, *J* = 2.3 Hz, 1H), 7.62 (d, *J* = 8.8 Hz, 2H), 7.57 (d, *J* = 8.5 Hz, 2H), 7.30 (d, *J* = 8.5 Hz, 2H), 7.14 (d, *J* = 8.8 Hz, 2H), 5.31 (s, 2H), 4.42 (t, *J* = 5.2 Hz, 2H), 3.96 (t, *J* = 5.2 Hz, 2H), 1.81 (d, *J* = 13.7 Hz, 6H), 1.68 (s, 6H). $^{31}P$ NMR (162 MHz, DMSO-$d_6$) δ 34.07 (s, 1P).

**Example 277: Preparation of compound 277**

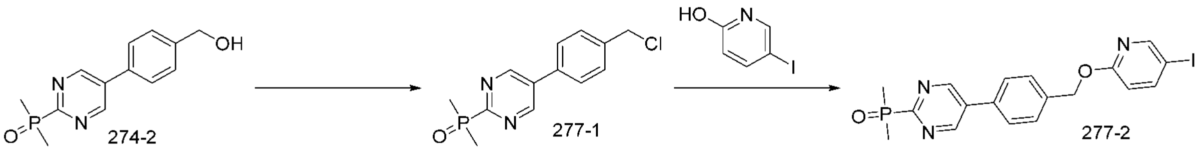

274-2 277-1 277-2

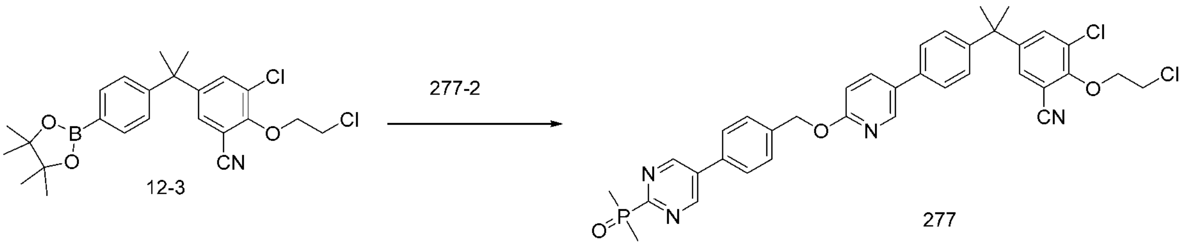


12-3 277

## Preparation of compound 277-1

Compound **274-2** (300 mg, 1.36 mmol) was dissolved in dichloromethane (10 mL). The mixture was cooled to 0°C, and thionyl chloride (485 mg, 4.08 mmol) was added dropwise. The reaction system was stirred at 25°C for 2 h. After the reaction was completed, the reaction mixture was concentrated to obtain 239 mg of a crude product of the title compound **277-1.** LC-MS (ESI): m/z 281.2 $[M+H]^+$.

## Preparation of compound 277-2

Compound **277-1** (700 mg, 2.49 mmol) was dissolved in DMF (10 mL), followed by the sequential addition of potassium carbonate (1.03 g, 7.48 mmol), silver carbonate (688 mg, 2.49 mmol), and 2-hydroxy-5-iodopyridine (551 mg, 2.49 mmol). The reaction system was stirred at 90°C for 2 h. After the reaction was completed, water (25 mL) was added, and the mixture was extracted three times with ethyl acetate (25 mL). The organic phases were combined, washed twice with saturated brine (20 mL), dried over anhydrous sodium sulfate, subjected to suction filtration, and the filtrate was concentrated to obtain a crude product. The crude product was purified by column chromatography (PE/EA = 100 to 45%) to obtain 80 mg of the compound **277-2.** LC-MS (ESI): m/z 465.8 $[M+H]^+$.

## Preparation of compound 277

Compound **277-2** (35 mg, 0.08 mmol) was dissolved in dioxane (2 mL), followed by the sequential addition of compound **12-3** (35 mg, 0.08 mmol) and potassium carbonate (31 mg, 0.23 mmol). The system was purged three times with nitrogen. Pd(dppf)$Cl_2$ (6 mg, 0.07 mmol) was added, and the reaction system was stirred at 110°C for 12 h. After the reaction was completed, insoluble solids were removed by filtration. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of compound **277.** LC-MS (ESI): m/z 671.2 $[M+H]^+$. $^1H$ NMR (400 MHz, Chloroform-d) δ 9.08 (s, 2H), 8.41 (s, 1H), 7.88 (d, *J* = 9.8 Hz, 1H), 7.71 - 7.60 (m, 4H), 7.52 - 7.45 (m, 3H), 7.36 (d, *J* = 1.3 Hz, 1H), 7.27 (d, *J* = 8.7 Hz, 2H), 6.95 (d, *J* = 8.5 Hz, 1H), 5.55 (s, 2H), 4.43 (t, *J* = 5.9 Hz, 2H), 3.88 (t, *J* = 6.1 Hz, 2H), 1.92 (d, *J* = 13.3 Hz, 6H), 1.70 (s, 6H). $^{31}P$ NMR (162 MHz, Chloroform-d) δ 34.67 (s, 1P).

## Example 278: Preparation of compound 278

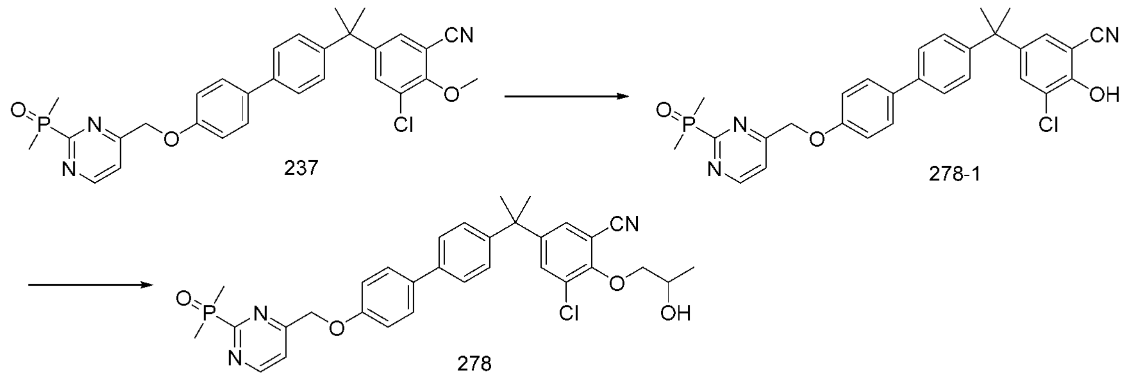

237

278-1

278

**Preparation of compound 278-1**

Compound **237** (2 g, 3.66 mmol) was dissolved in DMF (30 mL), and lithium chloride (1.55 g, 36.63 mmol) was added thereto. After the addition was completed, the reaction system was stirred at 130°C for 2 h. After the reaction was completed, water (100 mL) was added, and the mixture was extracted three times with dichloromethane (50 mL). The organic phases were combined, washed twice with saturated brine (100 mL), dried over anhydrous sodium sulfate, filtered, and the filtrate was concentrated to obtain a crude product. The crude product was purified by silica gel column chromatography (DCM/MeOH = 100% to 90%) to obtain 1.2 g of the title compound 278-1. LC-MS (ESI): m/z 532.2 $[M+H]^+$.

**Preparation of compound 278**

Compound **278-1** (50 mg, 0.09 mmol), 1-bromo-2-propanol (39 mg, 0.28 mmol), and potassium carbonate (39 mg, 0.28 mmol) were dissolved in DMF (5 mL). The reaction system was stirred at 80°C for 36 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 40% to 60% acetonitrile gradient elution over 12 min; flow rate: 30 mL/min) to obtain 33.5 mg of the title compound 278. LC-MS (ESI): m/z 590.2 $[M+H]^+$. $^1H$ NMR (400 MHz, Chloroform-d) δ 8.87 (d, *J* = 5.1 Hz, 1H), 7.71 - 7.66 (m, 1H), 7.58 - 7.52 (m, 2H), 7.52 - 7.46 (m, 3H), 7.38 - 7.35 (m, 1H), 7.25 - 7.20 (m, 2H), 7.07 - 7.00 (m, 2H), 5.30 (s, 2H), 4.29 - 4.20 (m, 1H), 4.04 - 3.97 (m, 1H), 3.82 (dd, *J* = 29.6 Hz, 2.8Hz, 1H), 1.91 (d, *J* = 13.6 Hz, 6H), 1.69 (d, *J* = 1.3 Hz, 6H), 1.27 (d, *J* = 6.4 Hz, 3H). $^{31}P$ NMR (162 MHz, Chloroform-d) δ 35.22 (s, 1P).

**Example 279: Preparation of compound 279**

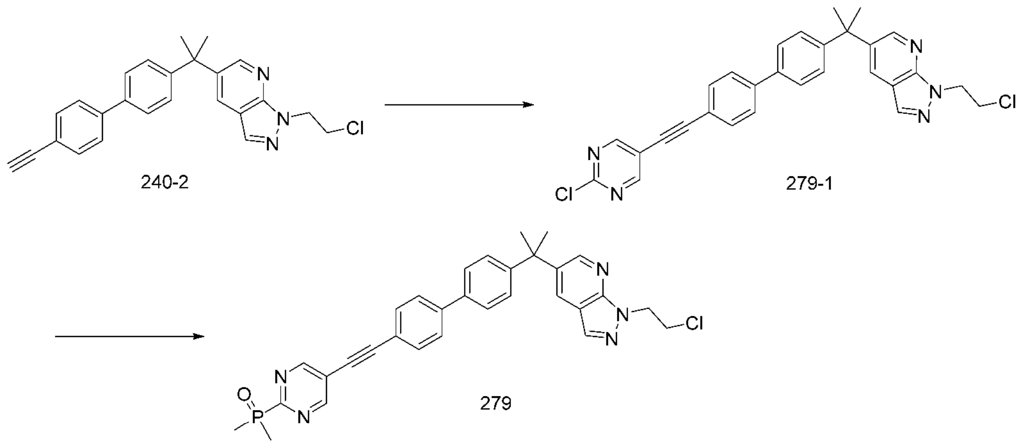

240-2

279-1

279

**Preparation of compound 279-1**

Compound **240-2** (400 mg, 1.0 mmol), 2-chloro-5-iodopyrimidine (245 mg, 1.11 mmol), triethylamine (205 mg, 2.02 mmol), copper(I) iodide (10 mg, 51.2 μmol), and bis(triphenylphosphine)palladium(II) chloride (35 mg, 50 μmol) were dissolved in acetonitrile (5 mL). The system was purged three times with nitrogen, and then the reaction was stirred at 25°C for 3 h. After the reaction was completed, water (15 mL) was added, and the mixture was extracted three times with ethyl acetate (10 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 35%) to obtain 295 mg of the title compound **279-1.**

**Preparation of compound 279**

Compound **279-1** (182 mg, 355 μmol) was dissolved in *N,N*-dimethylformamide (2 mL), followed by the sequential addition of dimethylphosphine oxide (138 mg, 1.77 mmol), triethylamine (90 mg, 887 μmol), $Pd_2(dba)_3$ (46 mg, 0.05 mmol), and Xantphos (30 mg, 0.05 mmol). The reaction system was purged three times with nitrogen and stirred under microwave irradiation at 120°C for 2 h. After the reaction was completed, the reaction system was filtered. Water (20 mL) was added to the filtrate, and the mixture was extracted three times with dichloromethane (20 mL). The organic phases were combined, dried, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 42 mg of the title compound **279.** LC-MS (ESI): m/z 554.2 $[M+H]^+$. $^1H$ NMR (400 MHz, DMSO-$d_6$) δ 9.19 (s, 2H), 8.40 (d, *J* = 2.2 Hz, 1H), 8.20 (d, *J* = 2.2 Hz, 1H), 8.17 (s, 1H), 7.79 (d, *J* = 8.5 Hz, 2H), 7.71 (d, *J* = 8.5 Hz, 2H), 7.69 (d, *J* = 8.5 Hz, 2H), 7.39 (d, *J* = 8.5 Hz, 2H), 4.75 (t, *J* = 5.8 Hz, 2H), 4.12 (t, *J* = 5.8 Hz, 2H), 1.79 (d, *J* = 13.6 Hz, 6H), 1.79 (s, 6H). $^{31}P$ NMR (162 MHz, DMSO-$d_6$) δ 34.39 (s, 1P).

**Example 280: Preparation of compound 280**

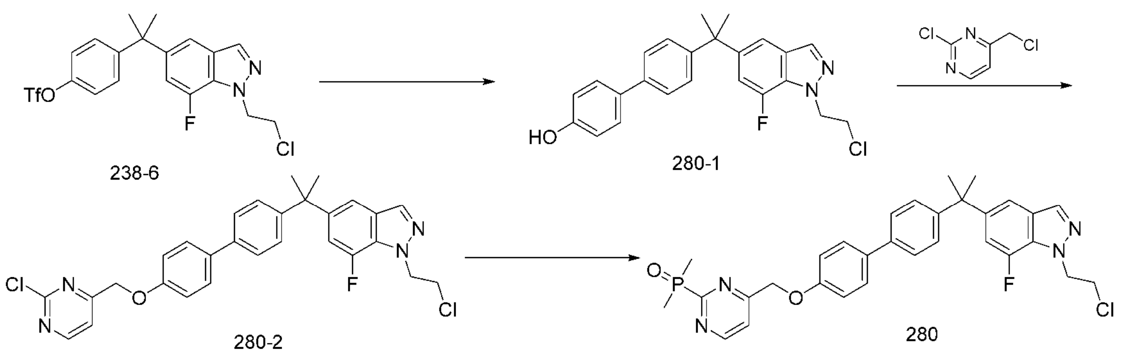
238-6
280-1
280-2
280

**Preparation of compound 280-1**

Compound **238-6** (1 g, 2.15 mmol) and 4-hydroxyphenylboronic acid (356 mg, 2.58 mmol) was dissolved in 1,4-dioxane (10 mL), followed by the sequential addition of cesium carbonate (1.5 g, 4.46 mmol), [1,1'-bis(diphenylphosphino) ferrocene]dichloropalladium(II) dichloromethane complex (136 mg, 186.5 μmol). The system was purged three times with nitrogen and then stirred at 105°C for 3 h. After the reaction was completed, the reaction mixture was cooled to room temperature and filtered. Saturated aqueous sodium chloride solution (20 mL) was added to the filtrate, and the mixture was extracted three times with ethyl acetate (10 mL). The organic phases were combined, dried, subjected to suction filtration, and the filtrate was concentrated to obtain a crude product. The crude product was purified by silica gel column chromatography (EA/PE = 0 to 40%) to obtain 580 mg of the title compound **280-1.** LC-MS (ESI): 409.2 $[M+H]^+$.

**Preparation of compound 280-2**

Compound **280-1** (580 mg, 1.42 mmol), 2-chloro-4-(chloromethyl)pyrimidine (231 mg, 1.42 mmol), and cesium carbonate (916 mg, 2.82 mol) were dissolved in acetonitrile (5 mL). The system was purged three times with nitrogen, and then the reaction was stirred at 50°C for 3 h. After the reaction was completed, water (15 mL) was added, and the mixture was extracted three times with ethyl acetate (10 mL). The organic phases were combined, washed twice with saturated

brine (20 mL), dried over anhydrous sodium sulfate, subjected to suction filtration, and the filtrate was concentrated to obtain a crude product. The crude product was purified by column chromatography (EA/PE = 0 to 25%) to obtain 365 mg of the title compound **280-2.**

### Preparation of compound 280

Compound **280-2** (100 mg, 1.87 mmol) was dissolved in N,N-dimethylformamide (2 mL), followed by the sequential addition of dimethylphosphine oxide (291 mg, 3.74 mmol), triethylamine (379 mg, 3.74 $\mu$mol), $Pd_2(dba)_3$ (85 mg, 93 $\mu$mol), and Xantphos (54 mg, 93 $\mu$mol). The reaction system was purged three times with nitrogen and stirred under microwave irradiation at 120°C for 2 h. After the reaction was completed, the reaction system was filtered and then extracted three times with dichloromethane (20 mL). The organic phases were combined, dried, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 42 mg of the title compound **280.** LC-MS (ESI): m/z 577.4 $[M+H]^+$. $^1$H NMR (400 MHz, DMSO-$d_6$) $\delta$ 9.00 (d, $J$ = 5.2 Hz, 1H), 8.19 (d, $J$ = 2.3 Hz, 1H), 7.73 (dd, $J$ = 5.2, 3.2 Hz, 1H), 7.60 (d, $J$ = 8.8 Hz, 2H), 7.56 (d, $J$ = 1.4 Hz, 1H), 7.54 (d, $J$ = 8.5 Hz, 2H), 7.30 (d, $J$ = 8.5 Hz, 2H), 7.13 (d, $J$ = 8.8 Hz, 2H), 6.99 (dd, $J$ = 14.1, 1.4 Hz, 1H), 5.34 (s, 2H), 4.76 (t, $J$ = 5.7 Hz, 2H), 4.06 (t, $J$ = 5.6 Hz, 2H), 1.76 (d, $J$ = 13.7 Hz, 6H), 1.72 (s, 6H). $^{31}$P NMR (162 MHz, DMSO-$d_6$) $\delta$ 34.07 (s, 1P). $^{19}$F NMR (376 MHz, DMSO-$d_6$) $\delta$ -132.64 (1F).

### Example 281: Preparation of compound 281

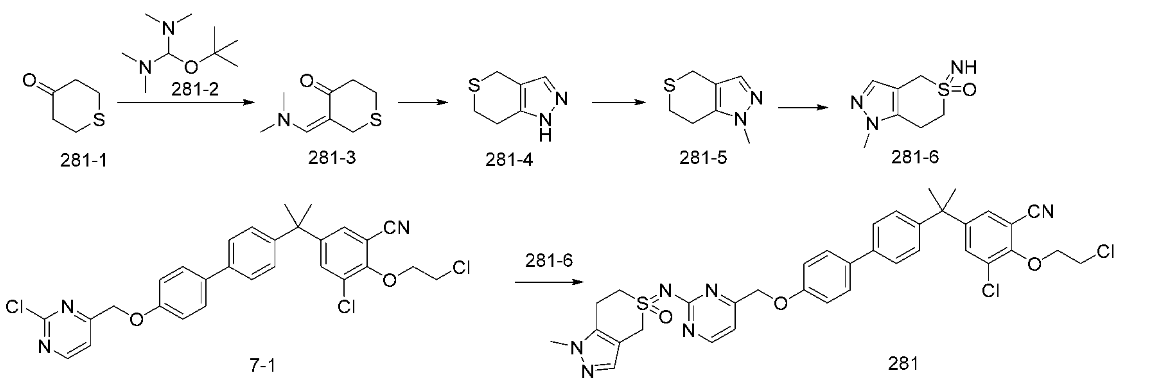


### Preparation of compound 281-3

Compound **281-1** (5 g, 43.04 mmol) was dissolved in toluene (50 mL), and compound **281-2** (8.3 g, 47.34 mmol) was added thereto. After the addition was completed, the reaction system was stirred at 25°C for 16 h. After the reaction was completed, the reaction mixture was directly concentrated to obtain 7 g of a crude product of the title compound **281-3.** The product was used directly in the next step without purification.

### Preparation of compound 281-4

Compound **281-3** (7 g, crude) was dissolved in ethanol (60 mL), and hydrazine hydrate (7.02 g, 175.17 mmol) was added thereto. After the addition was completed, the reaction system was stirred at 90°C for 16 h. After the reaction was completed, the reaction mixture was directly concentrated to obtain 7 g of a crude product of the title compound **281-4.** The product was used directly in the next step without purification.

### Preparation of compound 281-5

Compound **281-4** (4 g, 28.53 mmol) was dissolved in tetrahydrofuran (50 mL), and NaH (1.03 g, 42.80 mmol) was added thereto at 0°C. After the addition was completed, the reaction system was stirred at 0°C for 1 h. Then, iodomethane (4.05 g, 28.53 mmol) was added thereto. After the addition was completed, the reaction system was stirred at 25°C for 1 h. After the reaction was completed, saturated ammonium chloride (50 mL) was added, and the mixture was extracted three times with ethyl acetate (50 mL). The organic phases were combined, washed twice with saturated brine (50 mL), dried

over anhydrous sodium sulfate, filtered, and the filtrate was concentrated to obtain a crude product. The crude product was purified by silica gel column chromatography (PE/EA = 100% to 50%) to obtain 1.8 g of the title compound **281-3.** LC-MS (ESI): m/z 155.0 $[M+H]^+$.

**Preparation of compound 281-6**

Compound **281-5** (1 g, 6.49 mmol) and ammonium carbamate (1.52 g, 19.47 mmol) were dissolved in methanol (50 mL), and (diacetoxyiodo)benzene (6.27 g, 19.47 mmol) was added thereto. After the addition was completed, the reaction system was stirred at 25°C for 1 h. After the reaction was completed, saturated sodium chloride (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (DCM/MeOH = 100% to 90%) to obtain 1.6 g of the title compound **281-6.** LC-MS (ESI): m/z 185.9 $[M+H]^+$.

**Preparation of compound 281**

Compound **7-1** (100 mg, 0.18 mmol), compound **281-6** (100 mg, 0.54 mmol), $Pd_2(dba)_3$ (33 mg, 0.04 mmol), Xantphos (52 mg, 0.09 mmol), and DIPEA (24 mg, 0.31 mmol) were dissolved in DMF (50 mL). After the addition was completed, the reaction system was stirred at 120°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 21.3 mg of the title compound **281.** LC-MS (ESI): m/z 701.2 $[M+H]^+$. $^1H$ NMR (400 MHz, Chloroform-*d*) δ 8.48 (dd, *J* = 5.2, 1.2 Hz, 1H), 7.52 (d, *J* = 8.9 Hz, 2H), 7.48 (d, *J* = 8.4 Hz, 2H), 7.48 (d, *J* = 2.4 Hz, 1H), 7.37 (d, *J* = 2.3 Hz, 1H), 7.27 (s, 1H), 7.22 (d, *J* = 8.5 Hz, 2H), 7.10 (t, *J* = 5.5 Hz, 1H), 7.00 (d, *J* = 8.8 Hz, 2H), 5.13 (d, *J* = 2.0 Hz, 2H), 5.07 (d, *J* = 15.8 Hz, 1H), 4.94 (d, *J* = 15.7 Hz, 1H), 4.60 (d, *J* = 6.4 Hz, 1H), 4.43 (t, *J* = 6.2 Hz, 2H), 3.88 (t, *J* = 6.1 Hz, 2H), 3.85 (s, 1H), 3.78 (s, 3H), 3.38 (dt, *J* = 8.7, 4.7 Hz, 1H), 3.30 - 3.22 (m, 1H), 1.69 (s, 6H).

**Example 282: Preparation of compound 282**

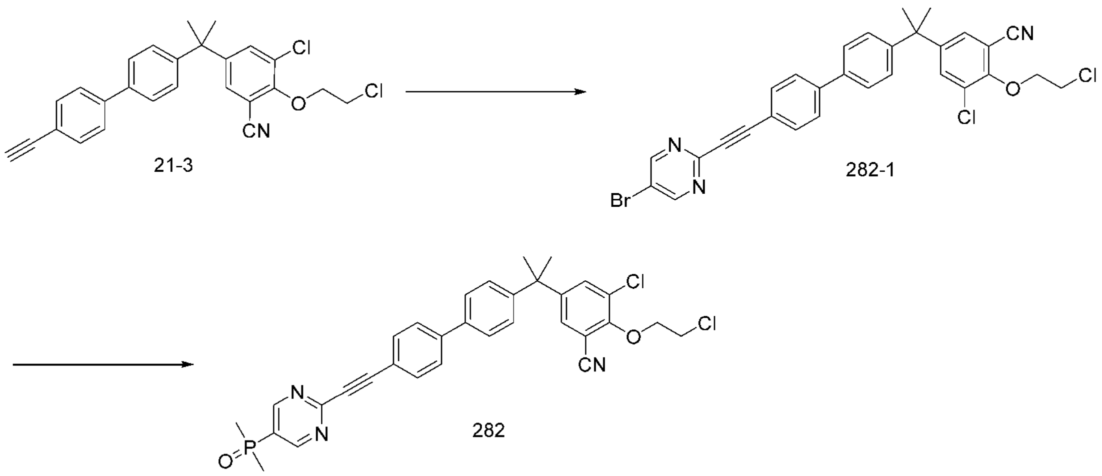

21-3

282-1

282

**Preparation of compound 282-1**

Compound **21-3** (400 mg, 0.92 mmol), 2-iodo-5-bromopyrimidine (314 mg, 1.1 mmol), triethylamine (205 mg, 2.02 mmol), copper(I) iodide (10 mg, 51.2 μmol), and $Pd(PPh_3)_2Cl_2$ (35 mg, 50 μmol) were dissolved in acetonitrile (5 mL). The system was purged three times with nitrogen, and then the reaction was stirred at 25°C for 3 h. After the reaction was completed, water (15 mL) was added, and the mixture was extracted three times with ethyl acetate (10 mL). The organic

phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 35%) to obtain 210 mg of the title compound **282-1.**

**Preparation of compound 282**

Compound **282-1** (210 mg, 355 μmol) was dissolved in *N,N*-dimethylformamide (2 mL), followed by the sequential addition of dimethylphosphine oxide (138 mg, 1.77 mmol), triethylamine (90 mg, 887 μmol), $Pd_2(dba)_3$ (46 mg, 0.05 mmol), and Xantphos (30 mg, 0.05 mmol). The reaction system was purged three times with nitrogen and stirred under microwave irradiation at 100°C for 2 h. After the reaction was completed, the reaction mixture was cooled to room temperature and filtered. Water (20 mL) was added, and the mixture was extracted three times with dichloromethane (20 mL). The organic phases were combined, dried, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 62 mg of the title compound **282.** LC-MS (ESI): m/z 588.3 $[M+H]^+$. $^1H$ NMR (400 MHz, DMSO-$d_6$) δ 9.14 (d, *J* = 5.7 Hz, 2H), 7.84 - 7.75 (m, 4H), 7.73 (d, *J* = 2.3 Hz, 1H), 7.71 (d, *J* = 8.5 Hz, 2H), 7.66 (d, *J* = 2.3 Hz, 1H), 7.38 (d, *J* = 8.5 Hz, 2H), 4.43 (t, *J* = 5.2 Hz, 2H), 3.96 (t, *J* = 5.2 Hz, 2H), 1.80 (d, *J* = 13.9 Hz, 6H), 1.70 (s, 6H). $^{31}P$ NMR (162 MHz, DMSO-$d_6$) δ 30.90 (s, 1P).

**Example 283: Preparation of compound 283**

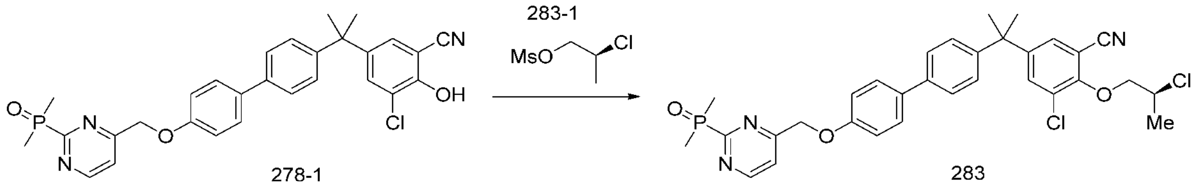


**Preparation of compound 283**

Compound **278-1** (50 mg, 0.09 mmol) and compound **283-1** (33 mg, 0.18 mmol) were dissolved in DMF (5 mL), and potassium carbonate (37 mg, 2.7 mmol) was added thereto. After the addition was completed, the reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 9.58 mg of the title compound **283.** LC-MS (ESI): m/z 608.2 $[M+H]^+$. $^1H$ NMR (400 MHz, Chloroform-*d*) δ 8.90 (s, 1H), 7.70 (s, 1H), 7.54 (d, *J* = 8.3 Hz, 2H), 7.49 (d, *J* = 8.4 Hz, 2H), 7.48 (d, *J* = 2.3 Hz, 1H), 7.36 (d, *J* = 2.3 Hz, 1H), 7.23 (d, *J* = 8.4 Hz, 2H), 7.04 (d, *J* = 8.4 Hz, 2H), 5.29 (s, 2H), 4.42 - 4.34 (m, 1H), 4.31 (dd, *J* = 9.5, 5.4 Hz, 1H), 4.22 (dd, *J* = 9.5, 6.8 Hz, 1H), 1.92 (d, *J* = 13.5 Hz, 6H), 1.71 (d, *J* = 6.5 Hz, 3H), 1.69 (s, 6H). $^{31}P$ NMR (162 MHz, Chloroform-d) δ 35.59 (s, 1P).

**Example 284: Preparation of compound 284**

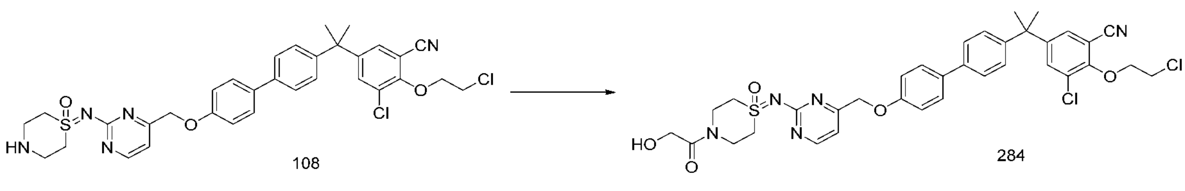

**Preparation of compound 284**

Compound **108** (133 mg, 0.204 mmol), HATU (155 mg, 0.409 mmol), *N,N*-diisopropylethylamine (53 mg, 0.409 mmol) and dichloromethane (2 mL) were added to a three-necked flask, and the reaction was stirred at 25°C for 3 h. After the reaction was completed, saturated sodium chloride solution (10 mL) was added to the reaction system. The mixture was extracted three times with DCM (10 mL). The organic phases were combined, dried, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 45% acetonitrile gradient elution over 12 min; flow rate: 30 mL/min) to obtain 21 mg of the title compound **284.** LC-MS (ESI): m/z 708.3 $[M+H]^+$. $^1H$ NMR (400 MHz, DMSO-$d_6$) δ 8.47 (d, *J* = 5.1 Hz, 1H), 7.70 (d, *J* = 2.3 Hz, 1H), 7.64 (d, *J* = 2.4 Hz, 1H), 7.60 (d, *J* = 8.8 Hz, 2H), 7.56 (d, *J* = 8.4 Hz, 2H), 7.30 (d, *J* = 8.5 Hz, 2H), 7.08 (d, *J* = 8.8 Hz, 2H), 7.00 (d, *J* = 5.1 Hz, 1H), 5.12 (s, 2H), 4.81 (t, *J* = 5.6 Hz, 1H), 4.42 (t, *J* = 5.2 Hz, 2H), 4.16 (d, *J* = 5.3 Hz, 2H), 4.13 - 4.02 (m, 1H), 3.96 (t, *J* = 5.2 Hz, 2H), 3.93 - 3.84 (m, 1H), 3.83 - 3.58 (m, 5H), 3.54 - 3.42 (m, 1H), 1.68 (s, 6H).

**Example 285: Preparation of compound 285**

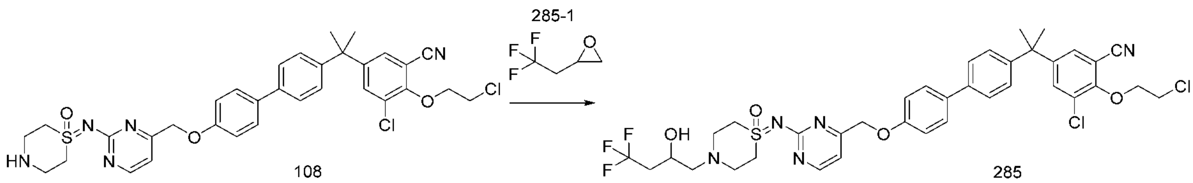


108 285

**Preparation of compound 285**

Compound **108** (120 mg, 0.185 mmol), compound **285-1** (117 mg, 0.925 mmol), cesium carbonate (120 mg, 0.37 mmol), and methanol (2 mL) were added to a three-necked flask, and the reaction was stirred at 25°C for 3 h. After the reaction was completed, saturated aqueous sodium chloride solution (10 mL) was added to the reaction system. The mixture was extracted three times with DCM (10 mL). The organic phases were combined, dried, subjected to suction filtration, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 45% acetonitrile gradient elution over 12 min; flow rate: 30 mL/min) to obtain 19 mg of the title compound **285.** LC-MS (ESI): m/z 776.4 $[M+H]^+$. $^1H$ NMR (400 MHz, Chloroform-d) δ 8.46 (s, 1H), 7.52 (d, *J* = 8.5 Hz, 2H), 7.48 (d, *J* = 8.3 Hz, 2H), 7.48 (d, *J* = 2.5 Hz, 1H), 7.37 (d, *J* = 2.4 Hz, 1H), 7.22 (d, *J* = 8.3 Hz, 2H), 7.15 - 7.06 (m, 1H), 7.00 (d, *J* = 8.4 Hz, 2H), 5.11 (s, 2H), 4.42 (t, *J* = 6.2 Hz, 2H), 4.10 (s, 1H), 3.88 (t, *J* = 6.2 Hz, 2H), 3.85 - 3.74 (m, 2H), 3.73 - 3.49 (m, 2H), 3.43 - 3.26 (m, 2H), 3.26 - 3.05 (m, 2H), 2.79 - 2.66 (m, 1H), 2.62 - 2.48 (m, 1H), 2.41 - 2.29 (m, 1H), 2.27 - 2.16 (m, 1H), 1.69 (s, 6H). $^{19}F$ NMR (376 MHz, Chloroform-d) δ -63.31 (3F).

**Example 286: Preparation of compound 286**

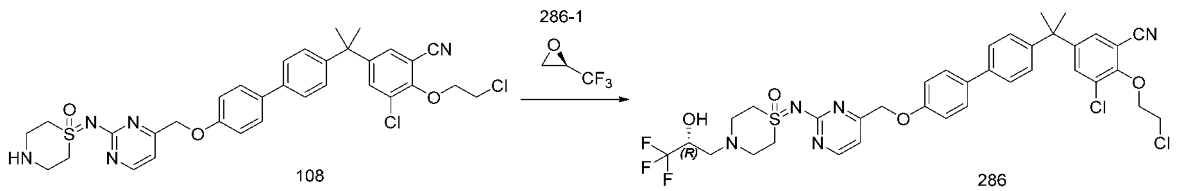


108 286

**Preparation of compound 286**

Compound **108** (110 mg, 0.169 mmol) was dissolved in DMF (8 mL). Cesium carbonate (130 mg, 0.4 mmol) and compound **286-1** (224 mg, 2.0 mmol) were added, and the reaction was carried out at 50°C for 1 h. After the reaction was monitored by LCMS until completion, the reaction mixture was filtered. The crude filtrate was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over

12 min; flow rate: 30 mL/min) to obtain 18.1 mg of the title compound **286.** LC-MS (ESI): m/z 762.2 $[M+H]^+$. $^1H$ NMR (400 MHz, DMSO-$d_6$) δ 8.50 - 8.41 (m, 1H), 7.70 (d, *J* = 2.4 Hz, 1H), 7.64 (d, *J* = 2.3 Hz, 1H), 7.60 (d, *J* = 8.7 Hz, 2H), 7.56 (d, *J* = 8.4 Hz, 2H), 7.30 (d, *J* = 8.2 Hz, 2H), 7.07 (d, *J* = 8.8 Hz, 2H), 6.99 (dd, *J* = 10.3, 5.1 Hz, 1H), 5.12 (d, *J* = 3.5 Hz, 2H), 4.42 (t, *J* = 5.3 Hz, 2H), 4.35 - 4.23 (m, 1H), 4.19 - 4.09 (m, 1H), 3.95 (t, *J* = 5.3 Hz, 2H), 3.80 - 3.73 (m, 1H), 3.72 - 3.63 (m, 2H), 3.62 - 3.52 (m, 1H), 3.52 - 3.42 (m, 2H), 3.14 - 3.07 (m, 1H), 3.04 - 2.97 (m, 1H), 2.85 - 2.75 (m, 1H), 2.72 - 2.62 (m, 1H), 1.68 (s, 6H).

**Example 287: Preparation of compound 287**

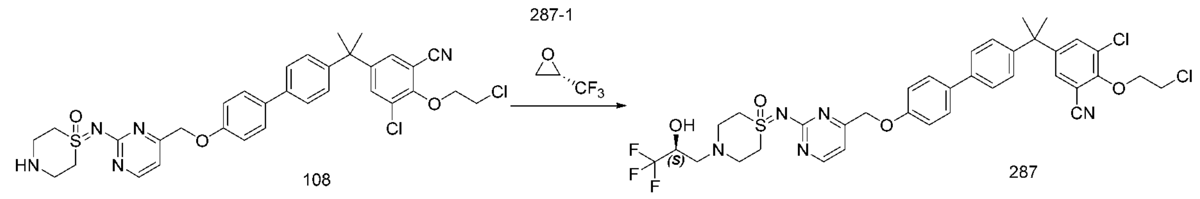


108 287

**Preparation of compound 287**

Compound **108** (110 mg, 0.169 mmol) was dissolved in DMF (8 mL). Cesium carbonate (130 mg, 0.4 mmol) and **287-1** (224 mg, 2.0 mmol) were added, and the reaction was carried out at 50°C for 1 h. After the reaction was monitored by LCMS until completion, the reaction mixture was filtered. The crude filtrate was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 15 mg of the title compound 287. LC-MS (ESI): m/z 762.2 $[M+H]^+$. $^1H$ NMR (400 MHz, DMSO-$d_6$) δ 8.47 (d, *J* = 5.0 Hz, 1H), 7.70 (d, *J* = 2.3 Hz, 1H), 7.64 (d, *J* = 2.3 Hz, 1H), 7.60 (d, *J* = 8.8 Hz, 2H), 7.56 (d, *J* = 8.5 Hz, 2H), 7.30 (d, *J* = 8.2 Hz, 2H), 7.07 (d, *J* = 8.9 Hz, 2H), 6.99 (d, *J* = 1.2 Hz, 1H), 5.12 (s, 2H), 4.42 (t, *J* = 5.2 Hz, 2H), 4.35 - 4.22 (m, 1H), 3.96 (t, *J* = 5.2 Hz, 2H), 3.79 - 3.73 (m, 1H), 3.70 - 3.64 (m, 1H), 3.61 - 3.55 (m, 1H), 3.18 - 3.15 (m, 3H), 2.22 - 2.11 (m, 2H), 2.07 - 1.99 (m, 2H), 1.68 (s, 6H).

**Example 288: Preparation of compound 288**

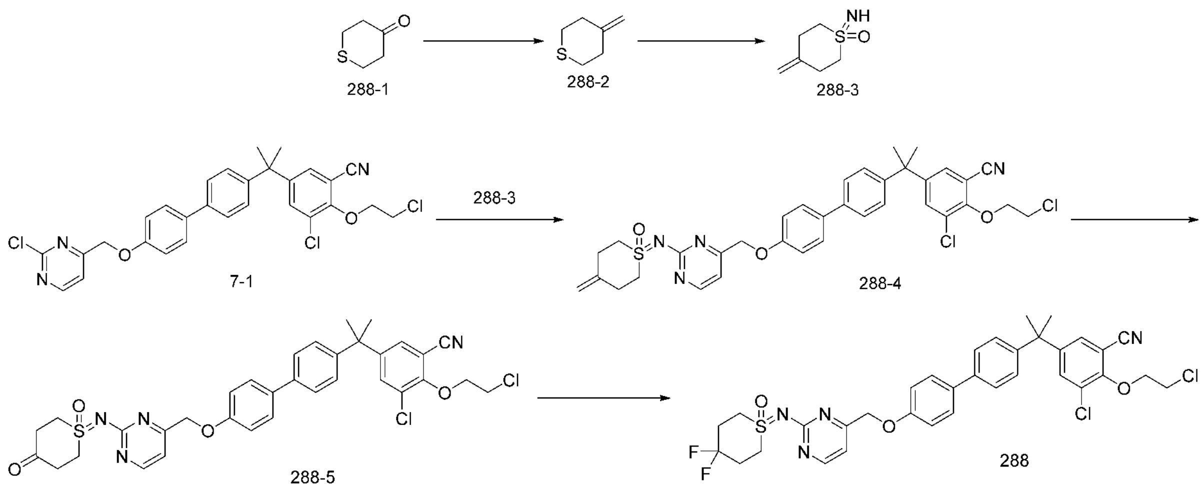


288-1 288-2 288-3 7-1 288-4 288-5 288

**Preparation of compound 288-2**

Methyltriphenylphosphonium bromide (46.1 g, 129.11 mmol) was dissolved in THF (150 mL). The mixture was cooled to 0°C, and potassium *tert*-butoxide (14.5 g, 129.11 mmol) was added. The mixture was stirred at 0°C for 1 h. Then, compound **288-1** (15 g, 129.11 mmol, 20 mL, THF solution) was added, and the mixture was stirred at room temperature for 1 h. TLC indicated the disappearance of the starting material. The reaction was quenched with ice water (100 mL) and

extracted three times with ethyl acetate (200 mL). The organic phases were combined, dried over anhydrous sodium sulfate, subjected to suction filtration, and the filtrate was concentrated. The resulting crude product was purified by silica gel column chromatography (PE/EA = 100% to 90%) to obtain 1.2 g of the title compound **288-2.** $^1$H NMR (400 MHz, Chloroform-d) δ 4.70 (s, 2H), 2.68 - 2.65 (m, 4H), 2.48 - 2.45 (m, 4H).

**Preparation of compound 288-3**

Compound **288-2** (1.2 g, 10.51 mmol) was dissolved in methanol (10 mL), and aminoformamide (820 mg, 10.51 mmol) and (diacetoxyiodo)benzene (7.1 g, 22.07 mmol) were added. The mixture was stirred at room temperature open to air for 1 h. TLC indicated the disappearance of the starting material. The mixture was directly concentrated to obtain a crude product. The crude product was purified by silica gel column chromatography (DCM/MeOH = 100% to 90%) to obtain 700 mg of the title compound **288-3.** $^1$H NMR (400 MHz, $CDCl_3$) δ 4.86 (s, 2H), 3.07 - 3.04 (m, 4H), 2.65 - 2.62 (m, 4H).

**Preparation of compound 288-4**

Compound **7-1** (650 mg, 1.14 mmol), compound **288-3** (308 mg, 2.28 mmol), and t-Bu-XPhos-Pd-G3 (90.6 mg, 0.11 mmol) were dissolved in 1,4-dioxane (6 mL), and cesium carbonate (1.1 g, 3.42 mmol) was added. The system was purged with argon, heated to 100°C, and reacted under microwave irradiation for 1 h. After the reaction was complete, the system was cooled to 0°C. The reaction was quenched with saturated sodium chloride solution (6 mL), and the mixture was extracted three times with ethyl acetate (10 mL). The organic phases were combined, dried over anhydrous sodium sulfate, subjected to suction filtration, and the filtrate was concentrated to obtain a crude product. The crude product was purified by silica gel column chromatography (PE/EA = 100% to 55%) to obtain 350 mg of the title compound **288-4.** LC-MS (ESI): m/z 661.2 $[M+H]^+$.

**Preparation of compound 288-5**

Compound **288-4** (200 mg, 0.30 mmol) was dissolved in DCM (10 mL), and the mixture was cooled to -78°C. Ozone was introduced into the reaction system with stirring until the reaction mixture turned blue. Dimethyl sulfide solution was then slowly added dropwise to the reaction until the blue color disappeared. The reaction was stopped, and the mixture was stirred at room temperature for 1 h. The reaction mixture was concentrated to obtain 150 mg of a crude product of the title compound **288-5,** which was used directly in the next step. LC-MS (ESI): m/z 663.0 $[M+H]^+$.

**Preparation of compound 288**

Compound **288-5** (50 mg, 0.075 mmol) was dissolved in DCM (3 mL). Under a nitrogen atmosphere, the temperature was lowered to 0°C, and DAST (24.2 mg, 0.15 mmol) was added. The mixture was stirred at 0°C for 1 h. The reaction was monitored by LCMS until completion. The organic phase was concentrated to obtain a crude product. The crude product was subjected to preparative purification (preparative method: mobile phase: A: 0.1% formic acid in water; B: acetonitrile; chromatographic column: Sunfire C18, 19 * 250 mm, 10 μm; column temperature: 25°C; gradient: 76% to 76% acetonitrile gradient elution over 16 min; flow rate: 20 mL/min) to obtain 0.63 mg of the title compound **288.** LC-MS (ESI): m/z 685.4 $[M+H]^+$. $^1$H NMR (400 MHz, Methanol-$d_4$) δ 8.47 (d, $J$ = 5.1 Hz, 1H), 7.56 (d, $J$ = 8.8 Hz, 2H), 7.56 (d, $J$ = 2.3 Hz, 1H), 7.53 (d, $J$ = 2.4 Hz, 1H), 7.53 (d, $J$ = 8.4 Hz, 2H), 7.29 (d, $J$ = 8.5 Hz, 2H), 7.12 (d, $J$ = 5.2 Hz, 1H), 7.06 (d, $J$ = 8.8 Hz, 2H), 5.14 (s, 2H), 4.43 (t, $J$ = 5.6 Hz, 2H), 3.90 (t, $J$ = 5.6 Hz, 2H), 2.58 - 2.47 (m, 4H), 2.19 (t, $J$ = 7.6 Hz, 2H), 2.05 - 2.00 (m, 2H), 1.71 (s, 6H). $^{19}$F NMR (376 MHz, Methanol-$d_4$) δ -101.36 (s, 2F).

**Example 289: Preparation of compound 289**

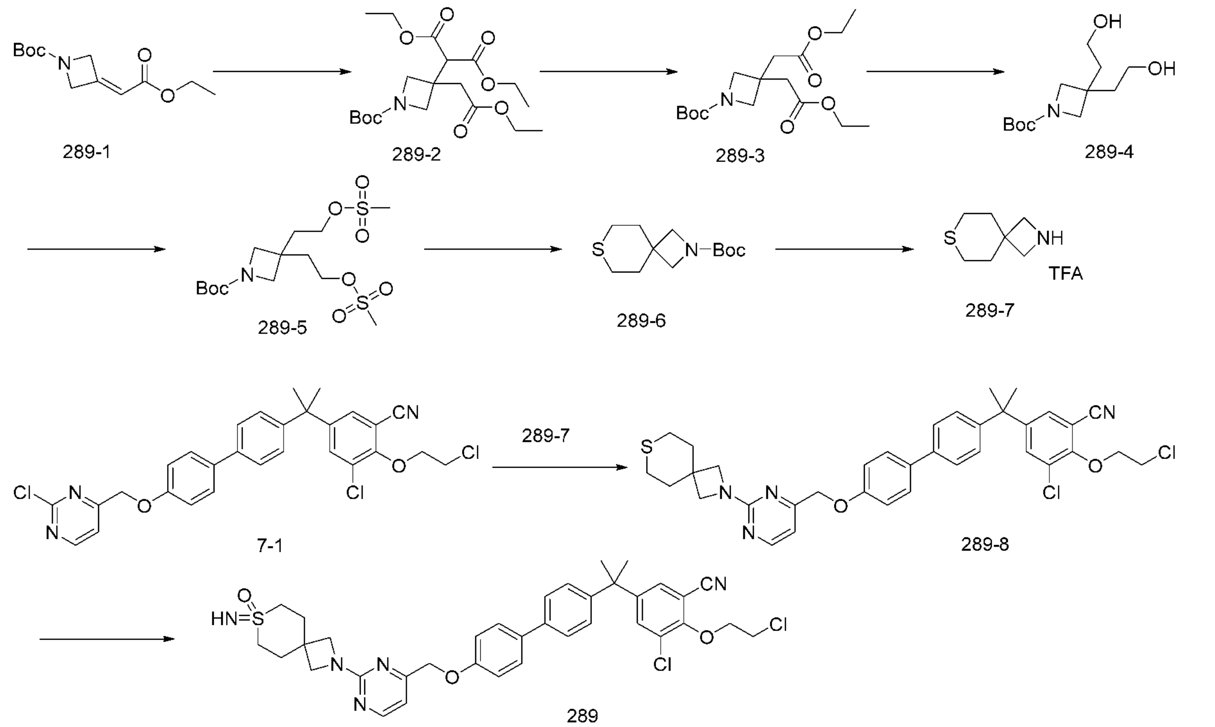

289-1 289-2 289-3 289-4
289-5 289-6 289-7
7-1 289-8
289

**Preparation of compound 289-2**

Under a nitrogen atmosphere, sodium hydride (8.3 g, 207.25 mmol, 60% wt) was added to dry THF (100 mL). The mixture was cooled to 0°C and stirred for 10 min. Diethyl malonate (22.1 mL, 145.08 mmol) was then slowly added dropwise to the reaction mixture. After stirring at room temperature for 30 min, the mixture was cooled again to 0°C. Tetrabutylammonium bromide (5.4 g, 16.58 mmol, 50 mL, THF solution) and compound 289-1 (10 g, 41.45 mmol) were added dropwise. The mixture was stirred at room temperature for 16 h. TLC indicated that the starting material was completely consumed. Acetic acid (15 mL) was added dropwise to quench the reaction. Ethyl acetate (400 mL) was added, and the mixture was washed twice with saturated sodium chloride (600 mL). The organic phase was dried over anhydrous sodium sulfate, subjected to suction filtration, and the filtrate was concentrated. The resulting crude product was purified by silica gel column chromatography (PE/EA = 100% to 40%) to obtain 11.6 g of the title compound **289-2.** $^1$H NMR (400 MHz, $CDCl_3$) δ 4.24 - 4.19 (m, 6H), 4.17 - 4.0 (m, 2H), 3.91 (s, 1H), 3.84 (d, *J* = 9.4 Hz, 2H), 2.87 (s, 2H), 1.43 (s, 9H), 1.30 - 1.23 (m, 9H).

**Preparation of compound 289-3**

Compound **289-2** (11.6 g, 28.90 mmol) was dissolved in DMSO (100 mL), followed by the addition of sodium chloride (3.4 g, 57.80 mmol) and water (1.4 mL). The mixture was heated to 160°C and stirred for 3.5 h. TLC indicated the disappearance of the starting material. The mixture was cooled to 0°C, quenched with water (200 mL), and extracted three times with ethyl acetate (400 mL). The organic phases were combined, washed three times with saturated sodium chloride solution (600 mL), dried over anhydrous sodium sulfate, subjected to suction filtration, and the filtrate was concentrated to obtain 10.5 g of the title compound **289-3.** $^1$H NMR (400 MHz, $CDCl_3$) δ 4.16 - 4.11 (q, *J* = 7.2 Hz, 4H), 3.82 (s, 4H), 2.80 (s, 4H), 1.43 (s, 9H), 1.27 - 1.24 (t, *J* = 7.2 Hz, 6H).

**Preparation of compound 289-4**

Compound **289-3** (9.5 g, 28.84 mmol) was dissolved in dry THF (100 mL). The system was purged with argon and cooled to 0°C. Lithium aluminum hydride (4.38 g, 115.36 mmol) was slowly added to the reaction mixture, and the mixture was stirred for 2 h. TLC indicated the disappearance of the starting material. The reaction was quenched with ice water (100 mL) and extracted three times with ethyl acetate (200 mL). The organic phases were combined, dried over anhydrous sodium sulfate, subjected to suction filtration, and the filtrate was concentrated to obtain a crude product. The crude product was purified by silica gel column chromatography (DCM/MeOH = 100% to 85%) to obtain 1.3 g of the title compound **289-4.** $^1$H NMR (400 MHz, $CDCl_3$) δ 3.75 (t, *J* = 6.3 Hz, 4H), 3.68 (s, 4H), 1.96 (t, *J* = 6.3 Hz, 4H), 1.43 (s, 9H).

**Preparation of compound 289-5**

Compound **289-4** (1.2 g, 4.89 mmol) was dissolved in DCM (10 mL). The mixture was cooled to 0°C, followed by the sequential addition of triethylamine (2.5 g, 24.45 mmol) and methanesulfonyl chloride (1.23 mL, 15.89 mmol). The reaction mixture was stirred at room temperature for 1 h. TLC indicated the disappearance of the starting material and the formation of a new spot, indicating completion of the reaction. The reaction was quenched with ice water (10 mL), and the system was extracted three times with DCM (20 mL). The organic phases were combined, dried over anhydrous sodium sulfate, subjected to suction filtration, and the filtrate was concentrated to obtain a crude product. The crude product was purified by silica gel column chromatography (PE/EA = 100% to 40%) to obtain 850 mg of the title compound **289-5.** $^1$H NMR (400 MHz, $CDCl_3$) δ 4.32 (t, *J* = 6.4 Hz, 4H), 3.73 (s, 4H), 3.04 (s, 6H), 2.16 (t, *J* = 6.5 Hz, 4H), 1.44 (s, 9H).

**Preparation of compound 289-6**

Compound **289-5** (200 mg, 0.50 mmol) was dissolved in DMF (3 mL), and sodium sulfide (47 mg, 0.60 mmol) was added. The mixture was stirred at 50°C for 4 h. TLC indicated the disappearance of the starting material and the formation of a new spot, indicating completion of the reaction. The reaction was quenched with ice water (5 mL), and the system was extracted three times with ethyl acetate (10 mL). The organic phases were combined, washed three times with saturated sodium chloride solution (30 mL), dried over anhydrous sodium sulfate, subjected to suction filtration, and the filtrate was concentrated to obtain a crude product. The crude product was purified by silica gel column chromatography (PE/EA = 100% to 75%) to obtain 40 mg of the title compound **289-6.** $^1$H NMR (400 MHz, $CDCl_3$) δ 3.56 (s, 4H), 2.57 - 2.54 (m, 4H), 1.97 - 1.94 (m, 4H), 1.44 (s, 9H).

**Preparation of compound 289-7**

Compound **289-6** (40 mg, 0.16 mmol) was dissolved in DCM (2 mL) and cooled to 0°C. Trifluoroacetic acid (1 mL) was added, and the mixture was stirred at room temperature for 1 h. TLC indicated the disappearance of the starting material and the formation of a new spot, indicating completion of the reaction. The mixture was directly concentrated to obtain 100 mg of a crude product of the title compound, which was used directly in the next step.

**Preparation of compound 289-8**

Compound 7-1 (100 mg, 0.18 mmol) and compound **289-7** (25.8 mg, 0.18 mmol) were dissolved in ethanol (3 mL). Triethylamine (0.25 mL, 1.80 mmol) was added, and the mixture was heated to 100°C and stirred for 2 h. LCMS indicated that the reaction was complete. The reaction was quenched with ice water (10 mL), and the system was extracted three times with ethyl acetate (20 mL). The organic phases were combined, dried over anhydrous sodium sulfate, and concentrated to obtain a crude product. The crude product was purified by silica gel column chromatography (PE/EA = 100% to 0%) to obtain 40 mg of the title compound **289-8.** LC-MS (ESI): m/z 659.2 $[M+H]^+$.

**Preparation of compound 289**

Compound **289-8** (35 mg, 0.053 mmol) was dissolved in methanol (1 mL), and (diacetoxyiodo)benzene (51.2 mg, 0.16 mmol) and ammonium acetate (16.3 mg, 0.21 mmol) were added. The mixture was stirred at room temperature open to air for 1 h. The reaction was monitored by LCMS until completion. The organic phase was concentrated to obtain a crude product. The crude product was subjected to preparative purification (preparative method: mobile phase: A: 0.1% formic acid in water; B: acetonitrile; chromatographic column: Sunfire C18, 19 * 250 mm, 10 μm; column temperature: 25°C; gradient: 70% to 70% acetonitrile gradient elution over 16 min; flow rate: 20 mL/min) to obtain 10.79 mg of the title compound **289.** LC-MS (ESI): m/z 690.4 $[M+H]^+$. $^1$H NMR (400 MHz, DMSO-$d_6$) δ 8.35 (d, *J* = 5.0 Hz, 1H), 7.70 (d, *J* = 2.3 Hz, 1H), 7.64 (d, *J* = 2.3 Hz, 1H), 7.59 (d, *J* = 8.7 Hz, 2H), 7.55 (d, *J* = 8.4 Hz, 2H), 7.30 (d, *J* = 8.4 Hz, 2H), 7.07 (d, *J* = 8.8 Hz, 2H), 6.75 (d, *J* = 5.0 Hz, 1H), 5.03 (s, 2H), 4.42 (t, *J* = 5.2 Hz, 2H), 3.96 (t, *J* = 5.2 Hz, 2H), 3.85 (s, 2H), 3.81 (s, 2H), 3.57 (s, 1H), 3.06 - 2.97 (m, 4H), 2.16 (t, *J* = 5.5 Hz, 4H), 1.68 (s, 6H).

**Example 290: Preparation of compound 290**

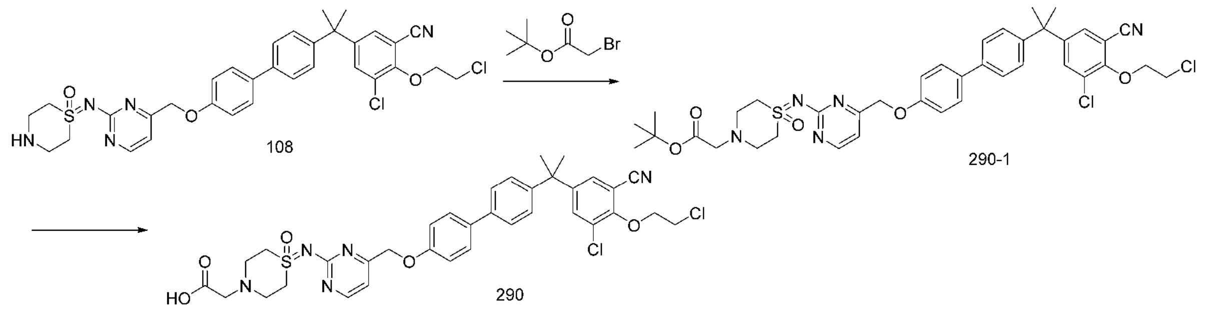

108, 290-1, 290

### Preparation of compound 290-1

Compound 108 (150 mg, 0.23 mmol) was dissolved in DMF (8 mL), and cesium carbonate (130 mg, 0.5 mmol) and *tert*-butyl 2-bromoacetate (97 mg, 0.5 mmol) were added. The reaction was carried out at 50°C for 1 h. The reaction was monitored by LCMS until completion. The reaction mixture was filtered, and the filtrate was poured into water (50 mL) and extracted with EA (50 mL). The organic phase was concentrated, and the residue was purified silica gel column chromatography to obtain 120 mg of the title compound **290-1** in 68.2% yield. LC-MS (ESI): m/z 764.2 $[M+H]^+$.

### Preparation of compound 290

Compound **290-1** (120 mg, 0.15 mmol) was dissolved in DCM (8 mL), and TFA (1.14 g, 10 mmol) was added. The reaction was carried out at 20°C for 12 h. After the reaction was monitored by LCMS until completion, the reaction mixture was concentrated. The crude product was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 11.5 mg of the title compound **290.** LC-MS (ESI): m/z 708.2 $[M+H]^+$. $^1H$ NMR (400 MHz, DMSO-$d_6$) δ 12.46 (br.s, 1H), 8.45 (d, *J* = 5.1 Hz, 1H), 7.70 (d, *J* = 2.3 Hz, 1H), 7.63 (d, *J* = 2.3 Hz, 1H), 7.59 (d, *J* = 8.8 Hz, 2H), 7.55 (d, *J* = 8.4 Hz, 2H), 7.30 (d, *J* = 8.5 Hz, 2H), 7.07 (d, *J* = 8.8 Hz, 2H), 6.98 (d, *J* = 5.1 Hz, 1H), 5.11 (s, 2H), 4.42 (t, *J* = 5.2 Hz, 2H), 3.95 (t, *J* = 5.2 Hz, 2H), 3.73 - 3.60 (m, 2H), 3.58 - 3.45 (m, 4H), 3.21 - 3.06 (m, 4H), 1.68 (s, 6H).

### Examples 291 and 292: Preparation of compounds 291 and 292

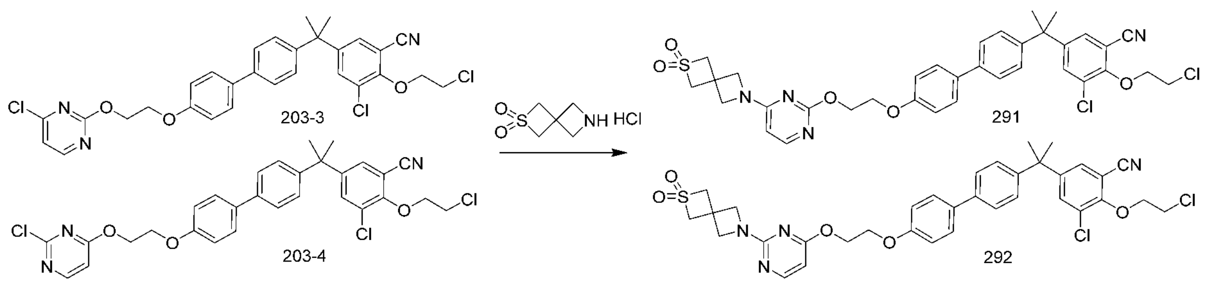

203-3, 203-4, 291, 292

### Preparation of compounds 291 and 292

A mixture of compounds **203-3 and 203-4** (120 mg, 0.21 mmol) was dissolved in *N,N*-dimethylformamide (5 mL), followed by the sequential addition of 2-thia-6-azaspiro[3.3]heptane 2,2-dioxide hydrochloride (76 mg, 0.41 mmol) and cesium carbonate (335 mg, 1.03 mmol). The reaction system was purged three times with nitrogen and stirred at 50°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5 mg of the title compound **291** and 25 mg of the title compound **292.**

Compound **291:** LC-MS (ESI): m/z 693.4 $[M+H]^+$. $^1H$ NMR (400 MHz, DMSO-$d_6$) δ 8.02 (d, *J* = 5.8 Hz, 1H), 7.71

(d, $J$ = 2.3 Hz, 1H), 7.64 (d, $J$ = 2.3 Hz, 1H), 7.58 (dd, $J$ = 12.9, 8.6 Hz, 4H), 7.30 (d, $J$ = 8.5 Hz, 2H), 7.05 (d, $J$ = 8.8 Hz, 2H), 6.13 (d, $J$ = 5.8 Hz, 1H), 4.53 (t, $J$ = 4.6 Hz, 2H), 4.50 (s, 4H), 4.42 (t, $J$ = 5.2 Hz, 2H), 4.31 (t, $J$ = 4.6 Hz, 2H), 4.26 (s, 4H), 3.96 (t, $J$ = 5.2 Hz, 2H), 1.68 (s, 6H).

Compound **292:** LC-MS (ESI): m/z 693.3 $[M+H]^+$. $^1$H NMR (400 MHz, DMSO-$d_6$) δ 8.11 (d, $J$ = 5.6 Hz, 1H), 7.70 (d, $J$ = 2.3 Hz, 1H), 7.64 (d, $J$ = 2.3 Hz, 1H), 7.60 (d, $J$ = 8.8 Hz, 2H), 7.56 (d, $J$ = 8.5 Hz, 2H), 7.30 (d, $J$ = 8.5 Hz, 2H), 7.05 (d, $J$ = 8.8 Hz, 2H), 6.21 (d, $J$ = 5.6 Hz, 1H), 4.60 (t, $J$ = 4.6 Hz, 2H), 4.48 (s, 4H), 4.42 (t, $J$ = 5.2 Hz, 2H), 4.35 (t, $J$ = 4.6 Hz, 2H), 4.24 (s, 4H), 3.96 (t, $J$ = 5.2 Hz, 2H), 1.68 (s, 6H).

**Example 293: Preparation of compound 293**

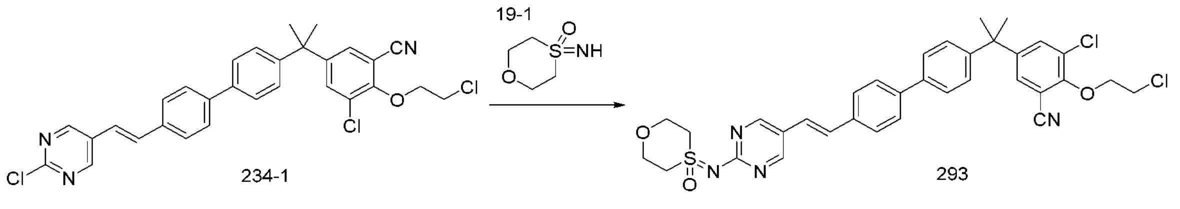


234-1 293

**Preparation of compound 293**

Compound **234-1** (50 mg, 0.09 mmol), compound **19-1** (24.6 mg, 0.18 mmol), $Pd_2(dba)_3$ (8.3 mg, 0.009 mmol), Xantphos (10.5 mg, 0.018 mmol), and DIPEA (58.9 mg, 0.46 mmol) were dissolved in DMF (2 mL). Under a nitrogen atmosphere, the reaction system was stirred at 120°C for 2 h in a microwave reactor. After the reaction was completed, insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 12 mg of the title compound **293.** LC-MS (ESI): m/z 647.2 $[M+H]^+$. $^1$H NMR (400 MHz, Chloroform-$d$) δ 8.63 (s, 2H), 7.63 - 7.54 (m, 6H), 7.49 (d, $J$ = 2.4 Hz, 1H), 7.38 (d, $J$ = 2.4 Hz, 1H), 7.25 (d, 2H), 7.09 (d, $J$ = 16.4 Hz, 1H), 6.96 (d, $J$ = 16.4 Hz, 1H), 4.43 (t, $J$ = 6.2 Hz, 2H), 4.29 - 4.21 (m, 2H), 4.21 - 4.12 (m, 2H), 3.93 - 3.80 (m, 4H), 3.58 - 3.47 (m, 2H), 1.70 (s, 6H).

**Example 294: Preparation of compound 294**

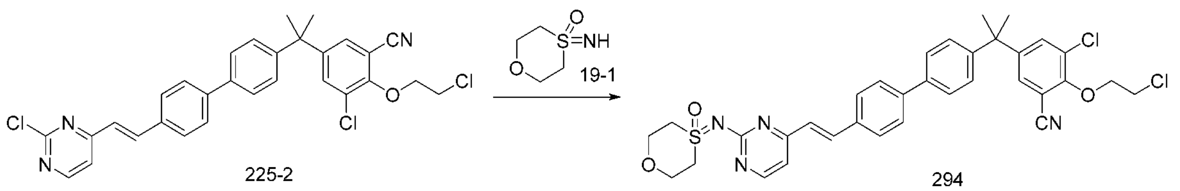


225-2 294

**Preparation of compound 294**

Compound **225-2** (50.0 mg, 0.09 mmol), compound **19-1** (24.6 mg, 0.18 mmol), $Pd_2(dba)_3$ (8.3 mg, 0.009 mmol), Xantphos (10.5 mg, 0.018 mmol), and DIPEA (58.9 mg, 0.46 mmol) were dissolved in DMF (2 mL). Under a nitrogen atmosphere, the reaction system was stirred at 120°C for 2 h in a microwave reactor. After the reaction was completed, insoluble solids were removed by filtration. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15 mg of compound **294.** LC-MS (ESI): m/z 647.3 $[M+H]^+$. $^1$H NMR (400 MHz, Chloroform-d) δ 8.41 (s, 1H), 7.90 (d, $J$ = 15.9 Hz, 1H), 7.64 (m, 4H), 7.57 (d, $J$ = 7.9 Hz, 2H), 7.49 (d, $J$ = 2.4 Hz, 2H), 7.43 - 7.33 (m, 2H), 7.04 (d, $J$ = 15.9 Hz, 1H), 6.90 (d, $J$ = 5.3 Hz, 1H), 4.43 (t, $J$ = 6.2 Hz, 2H), 4.29 - 4.18 (m, 4H), 3.92 - 3.84 (m, 4H), 3.60 - 3.51 (m, 2H), 1.71 (s, 6H).

**Example 295: Preparation of compounds 295, 295A, and 295B**

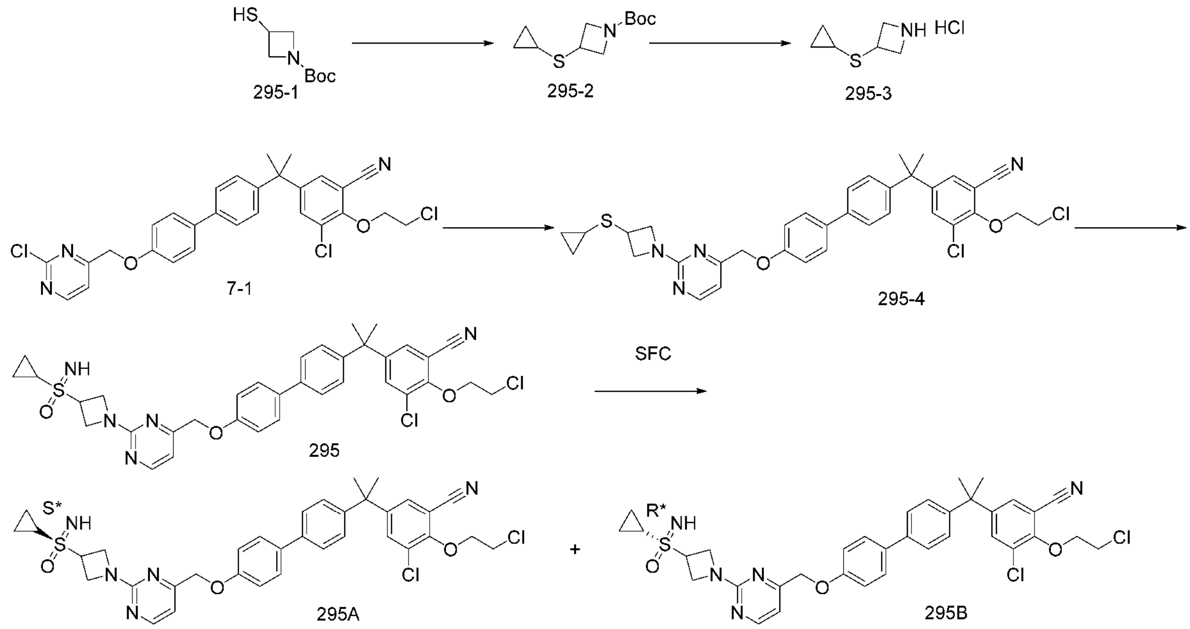


295-1 295-2 295-3 7-1 295-4 295 295A 295B

### Preparation of compound 295-2

*tert*-Butyl 3-mercaptoazetidine-1-carboxylate **295-1** (5 g, 26.42 mmol) was added to DMSO (10 mL), followed by the addition of bromocyclopropane (6.4 g, 52.84 mmol) and potassium *tert*-butoxide (4.5 g, 39.63 mmol) to the reaction system. After purging with argon for 30 seconds, the reaction system was sealed and stirred at 80°C for 16 h. TLC indicated the disappearance of the starting material. The reaction was quenched with water (10 mL), and ethyl acetate (50 mL) was added. The mixture was washed with saturated sodium chloride (100 mL). The organic phase was dried over anhydrous sodium sulfate, subjected to suction filtration, and the filtrate was concentrated. The resulting residue was purified by silica gel column chromatography (PE/EA = 100% to 20%) to obtain 5 g of the title compound **295-2.** $^1$H NMR (400 MHz, $CDCl_3$) δ 4.25 (t, *J* = 8.6 Hz, 2H), 3.89 (dd, *J* = 9.1, 5.6 Hz, 2H), 3.76 - 3.61 (m, 1H), 1.93 - 1.84 (m, 1H), 1.44 (s, 9H), 0.89 - 0.82 (m, 2H), 0.60 - 0.53 (m, 2H).

### Preparation of compound 295-3

Compound **295-2** (2 g, 8.73 mmol) was dissolved in DCM (10 mL), and hydrogen chloride in 1,4-dioxane (5 mL, 4 mol/L) was added. The mixture was stirred at room temperature for 2 h, and TLC indicated the disappearance of the starting material. The mixture was concentrated to obtain 600 mg of a crude product of the title compound **295-3.** The product was used directly in the next step without purification.

### Preparation of compound 295-4

Compound **7-1** (200 mg, 0.36 mmol) was dissolved in dry ethanol (5 mL), followed by the sequential addition of triethylamine (180 mg, 1.80 mmol) and compound **295-3** (100 mg, 0.72 mmol). The mixture was stirred at 100°C for 2 h, and TLC indicated the disappearance of the starting material. The reaction was quenched with ice water (10 mL) and extracted three times with ethyl acetate (50 mL). The organic phases were combined, dried over anhydrous sodium sulfate, subjected to suction filtration, and concentrated. The resulting residue was purified by silica gel column chromatography (PE/EA = 100% to 20%) to obtain 160 mg of the title compound **295-4.** $^1$H NMR (400 MHz, $CDCl_3$) δ 8.32 (d, *J* = 5.0 Hz, 1H), 7.57 - 7.44 (m, 5H), 7.38 (d, *J* = 2.2 Hz, 1H), 7.22 (d, *J* = 8.3 Hz, 2H), 7.01 (d, *J* = 8.7 Hz, 2H), 6.81 (d, *J* = 5.0 Hz, 1H), 5.02 (s, 2H), 4.51 (t, *J* = 8.6 Hz, 2H), 4.43 (t, *J* = 6.2 Hz, 2H), 4.15 - 4.11 (m, 2H), 3.95 - 3.84 (m, 3H), 1.98 - 1.90 (m, 1H), 1.69 (s, 6H), 0.91 - 0.83 (m, 2H), 0.65 - 0.56 (m, 2H).

### Preparation of compound 295

Compound **295-4** (160 mg, 0.25 mmol) was dissolved in methanol (3 mL). (Diacetoxyiodo)benzene (322 mg, 1.0 mmol) and ammonium carbamate (49 mg, 0.63 mmol) were added. The mixture was stirred at room temperature open to air for 1 h. The reaction was monitored by LCMS until completion. The organic phase was concentrated to obtain a crude product. The crude product was subjected to preparative purification (preparative method: mobile phase: A: 0.1% formic

acid in water; B: acetonitrile; chromatographic column: pursuit XRs C18, 19 * 250 mm, 10 μm; column temperature: 25°C; gradient: 85% to 90% acetonitrile gradient elution over 17 min; flow rate: 20 mL/min) to obtain 90.6 mg of the title compound **295.** LC-MS (ESI): m/z 676.4 [M+H]+. $^1$H NMR (400 MHz, DMSO-$d_6$) δ 8.40 (d, *J* = 5.0 Hz, 1H), 7.70 (d, *J* = 2.3 Hz, 1H), 7.64 (d, *J* = 2.3 Hz, 1H), 7.60 (d, *J* = 8.8 Hz, 2H), 7.56 (d, *J* = 8.4 Hz, 2H), 7.30 (d, *J* = 8.5 Hz, 2H), 7.08 (d, *J* = 8.8 Hz, 2H), 6.83 (d, *J* = 5.0 Hz, 1H), 5.08 (s, 2H), 4.42 (t, *J* = 5.2 Hz, 2H), 4.34 - 4.23 (m, 5H), 3.96 (t, *J* = 5.2 Hz, 2H), 3.89 (s, 1H), 2.67 - 2.59 (m, 1H), 1.68 (s, 6H), 1.11 - 1.02 (m, 1H), 0.96 - 0.89 (m, 2H), 0.88 - 0.82 (m, 1H).

**Preparation of compounds 295A and 295B**

Compound **295** (72 mg) was purified by preparative SFC (preparative conditions: instrument model: Waters SFC15; column model: ChiralPak OX-H, 250 * 20 mm I.D., 5 μm; mobile phase: A: $CO_2$; B: isopropanol (40% acetonitrile); elution gradient: B 55%; flow rate: 15 mL/min; column pressure: 100 bar; column temperature: 40°C; detection wavelength: 214 nm; run time per injection: 30 min) to obtain 25 mg of title compound **295A** and 19 mg of title compound **295B.**

Compound **295A:** SFC analysis method (instrument model: Waters SFC15; column model: ChiralPak OX-3, 100 * 4.6 mm I.D., 3 μm; mobile phase: A: $CO_2$, B: isopropanol (30% acetonitrile); elution gradient: B 40%; flow rate: 3 mL/min; column pressure: 100 bar; column temperature: 40°C; detection wavelength: 214 nm; Rt = 7.85 min). LC-MS (ESI): m/z 676.2 [M+H]+. $^1$H NMR (400 MHz, DMSO-$d_6$) δ 8.40 (d, *J* = 5.0 Hz, 1H), 7.70 (d, *J* = 2.4 Hz, 1H), 7.64 (d, *J* = 2.3 Hz, 1H), 7.60 (d, *J* = 8.8 Hz, 2H), 7.56 (d, *J* = 8.4 Hz, 2H), 7.30 (d, *J* = 8.4 Hz, 2H), 7.08 (d, *J* = 8.9 Hz, 2H), 6.83 (d, *J* = 5.0 Hz, 1H), 5.08 (s, 2H), 4.42 (t, *J* = 5.2 Hz, 2H), 4.35 - 4.22 (m, 5H), 3.96 (t, *J* = 5.2 Hz, 2H), 3.91 (s, 1H), 2.67 - 2.59 (m, 1H), 1.68 (s, 6H), 1.11 - 1.02 (m, 1H), 0.96 - 0.89 (m, 2H), 0.88 - 0.82 (m, 1H).

Compound **295B:** SFC analysis method (instrument model: Waters SFC15; column model: ChiralPak OX-3, 100 * 4.6 mm I.D., 3 μm; mobile phase: A: $CO_2$, B: isopropanol (30% acetonitrile); elution gradient: B 40%; flow rate: 3 mL/min; column pressure: 100 bar; column temperature: 40°C; detection wavelength: 214 nm; Rt = 9.07 min). LC-MS (ESI): m/z 676.2 [M+H]+. $^1$H NMR (400 MHz, DMSO-$d_6$) δ 8.40 (d, *J* = 5.0 Hz, 1H), 7.70 (d, *J* = 2.4 Hz, 1H), 7.64 (d, *J* = 2.3 Hz, 1H), 7.60 (d, *J* = 8.8 Hz, 2H), 7.56 (d, *J* = 8.4 Hz, 2H), 7.30 (d, *J* = 8.4 Hz, 2H), 7.08 (d, *J* = 8.9 Hz, 2H), 6.83 (d, *J* = 5.0 Hz, 1H), 5.08 (s, 2H), 4.42 (t, *J* = 5.1 Hz, 2H), 4.35 - 4.22 (m, 5H), 3.96 (t, *J* = 5.1 Hz, 2H), 3.91 (s, 1H), 2.67 - 2.59 (m, 1H), 1.68 (s, 6H), 1.11 - 1.02 (m, 1H), 0.96 - 0.89 (m, 2H), 0.88 - 0.82 (m, 1H).

**Example 296: Preparation of compound 296**

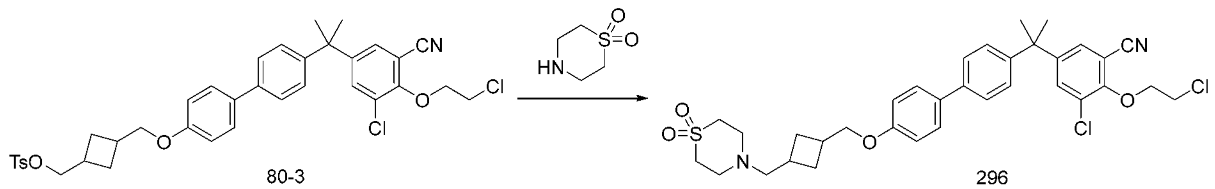

**Preparation of compound 296**

Compound **80-3** (67.4 mg, 99.31 μmol) was dissolved in acetonitrile (1 mL). Thiomorpholine 1,1-dioxide (40.3 mg, 148.97 μmol) and DIPEA (64.2 mg, 297.94 μmol, 87 μL) were added separately. The reaction mixture was reacted under microwave irradiation at 100°C for 1.5 h in a sealed tube. After the reaction was completed, the reaction mixture was diluted with a small amount of acetonitrile and filtered. The crude product was purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 40 to 60% over 4 min, 60 to 80% over 8 min, 80 to 95% over 0.1 min, hold at 95% for 4 min; flow rate: 30 mL/min) to obtain 1.71 mg of the title compound **296.** LC-MS (ESI): m/z 641.4 [M+H]+. $^1$H NMR (400 MHz, Chloroform-*d*) δ 7.56 - 7.46 (m, 5H), 7.37 (s, 1H), 7.22 (d, *J* = 7.8 Hz, 2H), 7.02 - 6.91 (m, 2H), 4.55 - 4.46 (m, 1H), 4.42 (t, *J* = 6.2 Hz, 2H), 4.12 - 3.96 (m, 2H), 3.93 - 3.84 (m, 3H), 3.42 - 2.92 (m, 8H), 2.83 - 2.60 (m, 3H), 2.42 - 2.19 (m, 3H), 1.69 (s, 6H).

**Example 297: Preparation of compound 297**

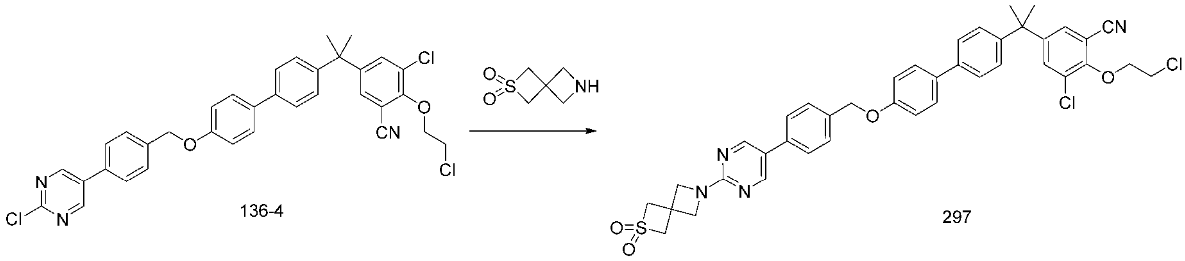

136-4

297

### Preparation of compound 297

Compound **136-4** (50 mg, 0.08 mmol), 2-thia-6-azaspiro[3.3]heptane 2,2-dioxide (18 mg, 0.10 mmol), and DIEA (52 mg, 0.4 mmol) were dissolved in EtOH (3 mL). The reaction system was stirred under microwave irradiation at 90°C for 2 h. After the reaction was completed, the reaction mixture was directly concentrated to obtain a crude product. The crude product was subjected to preparative purification (preparative method: mobile phase: A: 0.1 % formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 13 mg of the title compound 297. LC-MS (ESI): m/z 739.4 $[M+H]^+$. $^1H$ NMR (400 MHz, DMSO-$d_6$) δ 8.73 (s, 2H), 7.70 (d, *J* = 2.3 Hz, 1H), 7.67 (d, *J* = 8.1 Hz, 2H), 7.63 (d, *J* = 2.3 Hz, 1H), 7.59 (d, *J* = 8.8 Hz, 2H), 7.57 - 7.50 (m, 4H), 7.29 (d, *J* = 8.2 Hz, 2H), 7.09 (d, *J* = 8.8 Hz, 2H), 5.19 (s, 2H), 4.52 (s, 4H), 4.42 (t, *J* = 5.2 Hz, 2H), 4.32 (s, 4H), 3.95 (t, *J* = 5.2 Hz, 2H), 1.68 (s, 6H).

### Example 298: Preparation of compound 298

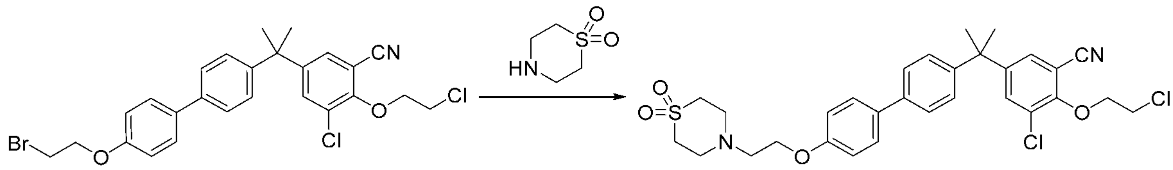

229-1

298

### Preparation of compound 298

Compound **229-1** (60 mg, 0.11 mmol) and potassium carbonate (78 mg, 0.56 mmol) were dissolved in *N*,*N*-dimethylformamide (5 mL), and thiomorpholine-1,1-dioxide (46 mg, 0.34 mmol) was added thereto. After the addition was completed, the reaction system was stirred at 80°C for 1 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 8.7 mg of the title compound **298.** LC-MS (ESI): m/z 587.2 $[M+H]^+$. $^1H$ NMR (400 MHz, DMSO-$d_6$) δ 7.70 (d, *J* = 2.4 Hz, 1H), 7.64 (d, *J* = 2.4 Hz, 1H), 7.58 (d, *J* = 8.8 Hz, 2H), 7.55 (d, *J* = 8.5 Hz, 2H), 7.30 (d, *J* = 8.5 Hz, 2H), 7.02 (d, *J* = 8.8 Hz, 2H), 4.42 (t, *J* = 5.2 Hz, 2H), 4.12 (t, *J* = 5.6 Hz, 2H), 3.96 (t, *J* = 5.2 Hz, 2H), 3.12 - 3.07 (m, 4H), 3.06 - 3.01 (m, 4H), 2.94 (t, *J* = 5.6 Hz, 2H), 1.68 (s, 6H).

### Example 299: Preparation of compound 299

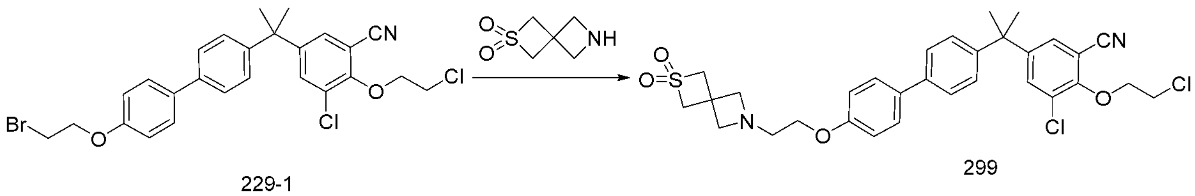

229-1

299

**Preparation of compound 299**

Compound **229-1** (60 mg, 0.11 mmol) and cesium carbonate (110 mg, 0.34 mmol) were dissolved in *N*,*N*-dimethylformamide (5 mL), and 2-thia-6-azaspiro[3.3]heptane 2,2-dioxide (20 mg, 0.14 mmol) was added thereto. After the addition was completed, the reaction system was stirred at 40°C for 3 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 13 mg of the title compound **299.** LC-MS (ESI): m/z 599.4 $[M+H]^+$. $^1H$ NMR (400 MHz, Chloroform-d) δ 7.51 (d, *J* = 8.7 Hz, 2H), 7.49 (d, *J* = 2.4 Hz, 1H), 7.48 (d, *J* = 8.6 Hz, 2H), 7.37 (d, *J* = 2.4 Hz, 1H), 7.22 (d, *J* = 8.4 Hz, 2H), 6.94 (d, *J* = 8.7 Hz, 2H), 4.42 (t, *J* = 6.2 Hz, 2H), 4.31 (s, 4H), 4.13 (t, *J* = 5.0 Hz, 2H), 3.88 (t, *J* = 6.2 Hz, 2H), 3.80 (s, 4H), 3.05 (t, *J* = 5.2 Hz, 2H), 1.69 (s, 6H).

**Example 300: Preparation of compound 300**

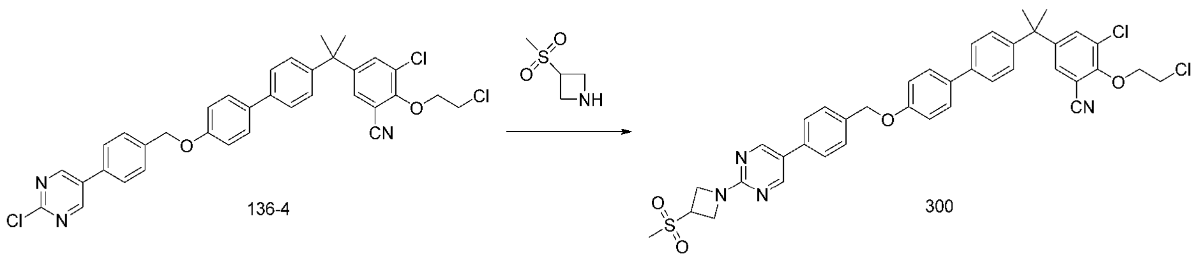

136-4

300

**Preparation of compound 300**

Compound **136-4** (50 mg, 0.08 mmol), 3-(methylsulfonyl)azetidine (18 mg, 0.10 mmol), and DIEA (52 mg, 0.4 mmol) were dissolved in EtOH (3 mL). The reaction system was stirred under microwave irradiation at 90°C for 2 h. After the reaction was completed, the reaction mixture was directly concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 17 mg of the title compound **300.** LC-MS (ESI): m/z 727.2 $[M+H]^+$. $^1H$ NMR (400 MHz, DMSO-$d_6$) δ 8.76 (s, 2H), 7.73 - 7.66 (m, 3H), 7.64 (d, *J* = 2.3 Hz, 1H), 7.59 (d, *J* = 8.5 Hz, 2H), 7.57 - 7.49 (m, 4H), 7.30 (d, *J* = 8.2 Hz, 2H), 7.09 (d, *J* = 8.5 Hz, 2H), 5.20 (s, 2H), 4.52 - 4.34 (m, 5H), 4.27 (dd, *J* = 9.5, 4.6 Hz, 2H), 3.96 (t, *J* = 5.2 Hz, 2H), 3.08 (s, 3H), 1.68 (s, 6H).

**Example 301: Preparation of compound 301**

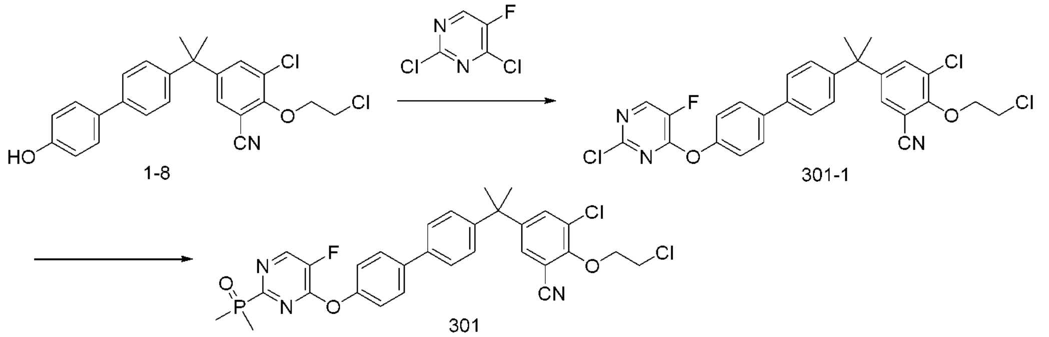

1-8 301-1 301

**Preparation of compound 301**

Compound **1-8** (70 mg, 0.16 mmol) was dissolved in tetrahydrofuran (2 mL) and cooled to 0°C. Sodium hydride (13 mg, 0.33 mmol) was added, and the mixture was stirred for 15 min. 2,4-Dichloro-5-fluoropyrimidine (30 mg, 0.33 mmol) was added, and the reaction system was stirred at 25°C for 2 h. After the reaction was completed, water (5 mL) was added, and the mixture was extracted three times with ethyl acetate (10 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (PE/EA= 100% to 45%) to obtain 14 mg of the title compound **301-1.** LC-MS (ESI): m/z 578.0 $[M+Na]^+$.

**Preparation of compound 301**

Compound **301-1** (50 mg, 0.10 mmol) was dissolved in dioxane (2 mL), followed by the sequential addition of dimethylphosphine oxide (8 mg, 0.11 mmol), Xantphos (10 mg, 0.02 mmol), and triethylamine (45 mg, 0.45 mmol). The system was purged three times with nitrogen, and $Pd_2(dba)_3$ (61 mg, 0.19 mmol) was added. The reaction system was stirred at 90°C for 12 h. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 7 mg of the title compound **301.** LC-MS (ESI): m/z 598.2. $[M+H]^+$. $^1H$ NMR (400 MHz, Chloroform-d) δ 7.67 - 7.61 (m, 3H), 7.56 (d, *J* = 8.4 Hz, 2H), 7.50 (d, *J* = 2.4 Hz, 1H), 7.44 - 7.41 (m, 2H), 7.37 (d, *J* = 2.3 Hz, 1H), 7.29 - 7.27 (m, 2H), 4.43 (t, *J* = 6.2 Hz, 2H), 3.88 (t, *J* = 6.1 Hz, 2H), 1.72 (d, *J* = 13.2 Hz, 6H), 1.71 (s, 6H). $^{31}P$ NMR (162 MHz, Chloroform-d) δ 35.00 (s, 1P). $^{19}F$ NMR (376 MHz, Chloroform-d) δ -146.80 (s, 3F).

**Example 302: Preparation of compound 302**

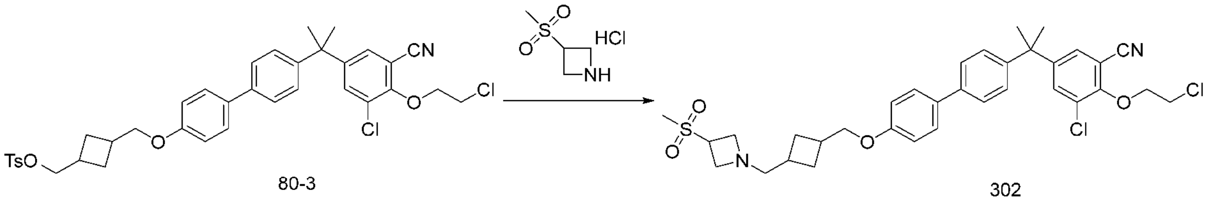

80-3 302

Preparation of compound **302**

Compound **80-3** (78 mg, 114.93 μmol) was added to acetonitrile (1 mL). 3-(Methanesulfonyl)azetidine hydrochloride (29.6 mg, 172.40 μmol) and potassium carbonate (47.7 mg, 344.80 μmol) were added separately. The reaction mixture was stirred in a sealed tube at 90°C overnight. After the reaction was completed, the reaction mixture was cooled to room temperature, diluted with acetonitrile, filtered, and then purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 30-70% 8 min, 70-95% 4 min; flow rate: 30 mL/min) to obtain 16 mg of compound **302.** LC-MS (ESI): m/z 641.2 $[M+H]^+$.

**Example 303: Preparation of compound 303**

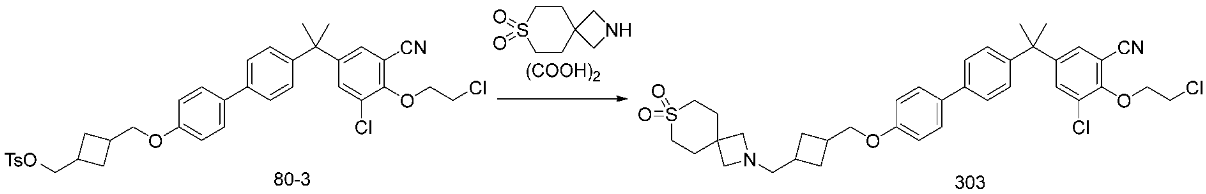


80-3

303

Preparation of compound **303**

Compound **80-3** (73.9 mg, 108.89 μmol) was dissolved in acetonitrile (1 mL). 7-Thia-2-azaspiro[3.5]nonane 7,7-dioxide monooxalate (37.6 mg, 141.56 μmol) and potassium carbonate (60.2 mg, 435.56 μmol) were added. The reaction mixture was stirred at 90°C overnight in a sealed tube. After the reaction was completed, the reaction mixture was cooled to room temperature, diluted with acetonitrile, and filtered. The filtrate was purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 30-70% 8 min, 70-95% 4 min; flow rate: 30 mL/min) to obtain 15.8 mg of the title compound **303.** LC-MS (ESI): m/z 681.4 $[M+H]^+$. $^1H$ NMR (400 MHz, Chloroform-*d*) δ 7.52 - 7.46 (m, 5H), 7.37 (t, *J* = 2.6 Hz, 1H), 7.24 - 7.20 (m, 2H), 6.97 - 6.92 (m, 2H), 4.42 (t, *J* = 6.2 Hz, 2H), 4.01 (d, *J* = 6.7 Hz, 1H), 3.90 - 3.85 (m, 3H), 3.74 - 3.51 (m, 5H), 3.01 - 2.94 (m, 5H), 2.77 - 2.72 (m, 1H), 2.49 (s, 3H), 2.36 - 2.29 (m, 2H), 2.25 - 2.18 (m, 1H), 2.07 - 2.01 (m, 1H), 1.79 - 1.76 (m, 2H), 1.69 (s, 6H).

**Example 304: Preparation of compound 304**

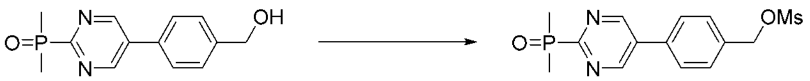

274-2

304-1

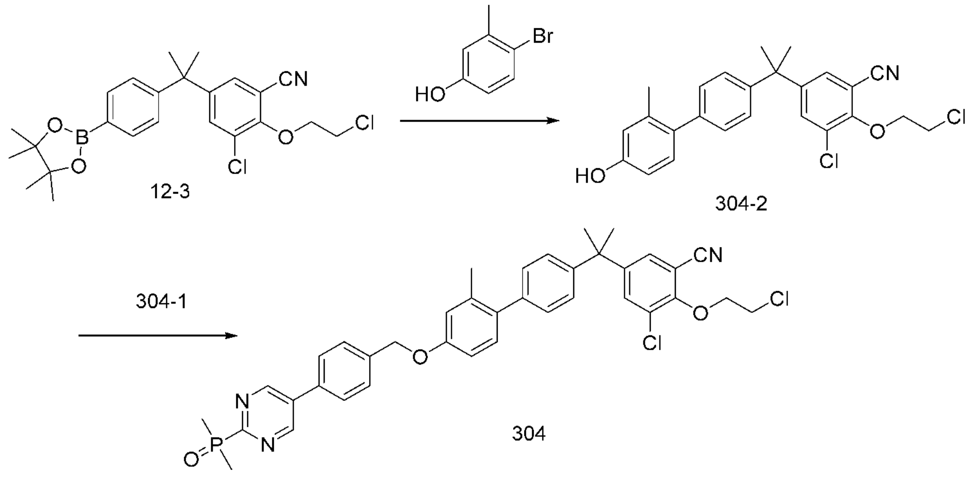

12-3

304-2

304-1

304

**Preparation of compound 304-2**

Compound **12-3** (1.0 g, 2.17 mmol) was dissolved in dioxane (30 mL) and water (10 mL). Potassium carbonate (414 mg, 3.0 mmol), 3-methyl-4-bromophenol (409 mg, 2.2 mmol), and Pd(dppf)$Cl_2$ (146 mg, 0.2 mmol) were added. The system was purged three times with nitrogen, heated to 100°C, and the reaction was carried out for 3 h. After the reaction was monitored by LCMS until completion, the reaction mixture was filtered, poured into water (50 mL), and extracted with EA (100 mL). The organic phase was concentrated, and the residue was purified silica gel column chromatography to obtain 680 mg of the title compound **304-2** in 71.3% yield. LC-MS (ESI): m/z 440.2 $[M+H]^+$.

**Preparation of compound 304**

Compound **304-2** (100 mg, 0.22 mmol) was dissolved in acetonitrile (8 mL). Potassium carbonate (55.2 mg, 0.4 mmol) and compound **304-1** (104 mg, 0.30 mmol) were added, and the reaction was carried out at 70°C for 2 h. After the reaction was monitored by LCMS until completion, the reaction mixture was concentrated. The crude product was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 38 mg of the title compound **304.** LC-MS (ESI): m/z 684.0 $[M+H]^+$. $^1H$ NMR (400 MHz, DMSO-$d_6$) δ 9.33 (s, 2H), 7.91 (d, *J* = 8.3 Hz, 2H), 7.72 (d, *J* = 2.4 Hz, 1H), 7.69 - 7.63 (m, 3H), 7.33 - 7.22 (m, 4H), 7.13 (d, *J* = 8.4 Hz, 1H), 6.99 (d, *J* = 2.7 Hz, 1H), 6.90 (dd, *J* = 8.5, 2.6 Hz, 1H), 5.23 (s, 2H), 4.43 (t, *J* = 5.2 Hz, 2H), 3.96 (t, *J* = 5.2 Hz, 2H), 2.22 (s, 3H), 1.80 (d, *J* = 13.7 Hz, 6H), 1.69 (s, 6H).

**Example 305: Preparation of compound 305**

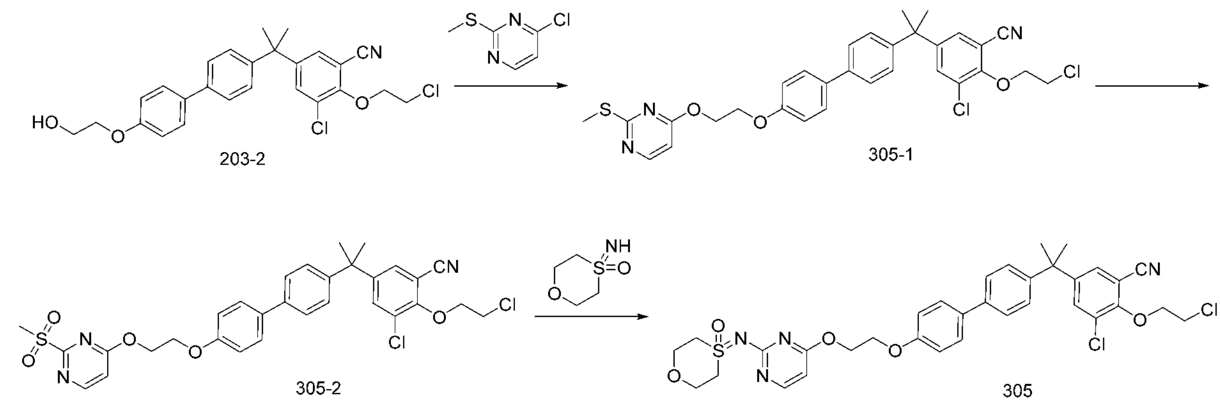

203-2 305-1 305-2 305

**Preparation of compound 305-1**

Compound **203-2** (600 mg, 1.28 mmol) was dissolved in *N,N*-dimethylformamide (10 mL), followed by the sequential addition of 2-methylthio-4-chloropyrimidine (410 mg, 2.55 mmol) and cesium carbonate (1.66 g, 5.10 mmol). The reaction system was purged three times with nitrogen and stirred at 70°C for 16 h. After the reaction was completed, water (10 mL) was added, and the mixture was extracted three times with dichloromethane (50 mL) and methanol (50 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (DCM/PE = 0 to 90%) to obtain 1.2 g of the title compound **305-1** in 94.6% yield. LC-MS (ESI): m/z 594.3 $[M+H]^+$.

**Preparation of compound 305-2**

Compound **305-1** (600 mg, 1.01 mmol) was dissolved in dichloromethane (50 mL), and *m*-chloroperoxybenzoic acid (615 mg, 3.03 mmol, 85%) was added. After the addition was completed, the mixture was stirred at room temperature for 5 h. After the reaction was completed, the reaction mixture was concentrated to obtain a crude product. The crude product was purified by silica gel column chromatography (DCM/PE = 0 to 90%) to obtain 500 mg of the title compound **305-2.** LC-MS (ESI): m/z 626.3 $[M+H]^+$.

**Preparation of compound 305**

Compound **305-2** (120 mg, 0.19 mmol) was dissolved in *N*,*N*-dimethylformamide (3 mL), followed by the sequential addition of 1,4-oxathiane sulfoximine (52 mg, 0.38 mmol) and cesium carbonate (187 mg, 0.57 mmol). The reaction system was purged three times with nitrogen and stirred at 60°C for 8 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in

water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound **305.** LC-MS (ESI): m/z 681.3 $[M+H]^+$. $^1$H NMR (400 MHz, DMSO-$d_6$) δ 8.18 (d, *J* = 5.7 Hz, 1H), 7.70 (d, *J* = 2.3 Hz, 1H), 7.64 (d, *J* = 2.3 Hz, 1H), 7.62 - 7.50 (m, 4H), 7.30 (d, *J* = 8.0 Hz, 2H), 7.05 (d, *J* = 8.3 Hz, 2H), 6.41 (d, *J* = 5.7 Hz, 1H), 4.59 (t, *J* = 4.2 Hz, 2H), 4.42 (t, *J* = 5.1 Hz, 2H), 4.35 (t, *J* = 4.4 Hz, 2H), 4.14 - 4.04 (m, 2H), 4.00 - 3.89 (m, 4H), 3.86 - 3.74 (m, 2H), 3.64 - 3.53 (m, 2H), 1.68 (s, 6H).

**Example 306: Preparation of compound 306**

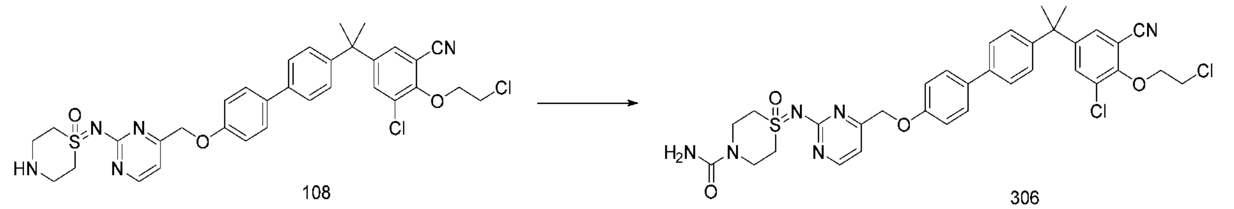

108 306

**Preparation of compound 306**

Compound **108** (100 mg, 0.15 mmol) was dissolved in isopropanol (4 mL), and potassium carbonate (42 mg, 0.30 mmol) was added. The system was purged with argon, and a solution of trimethylsilyl isocyanate in isopropanol (5 mL) was added dropwise. The mixture was stirred at room temperature for 2 h. The reaction was monitored by LCMS until completion. The reaction was quenched with ice water (10 mL) and extracted three times with ethyl acetate (10 mL). The organic phases were combined, dried over anhydrous sodium sulfate, subjected to suction filtration, and the filtrate was concentrated. The resulting residue was purified by reversed-phase column chromatography (0.1% formic acid in water/acetonitrile = 95% to 25%) to obtain 23.6 mg of the title compound **306.** LC-MS (ESI): m/z 693.2 $[M+H]^+$. $^1$H NMR (400 MHz, DMSO-$d_6$) δ 8.46 (d, *J* = 5.0 Hz, 1H), 7.70 (d, *J* = 2.3 Hz, 1H), 7.64 (d, *J* = 2.3 Hz, 1H), 7.60 (d, *J* = 8.8 Hz, 2H), 7.56 (d, *J* = 8.5 Hz, 2H), 7.30 (d, *J* = 8.4 Hz, 2H), 7.08 (d, *J* = 8.8 Hz, 2H), 6.99 (d, *J* = 5.0 Hz, 1H), 6.31 (s, 2H), 5.11 (s, 2H), 4.42 (t, *J* = 5.2 Hz, 2H), 4.03 - 3.85 (m, 4H), 3.76 - 3.65 (m, 2H), 3.65 - 3.54 (m, 2H), 3.50 - 3.40 (m, 2H), 1.68 (s, 6H).

**Example 307: Preparation of compound 307**

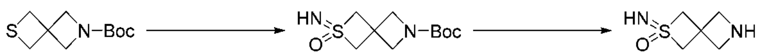

307-1 307-2 307-3

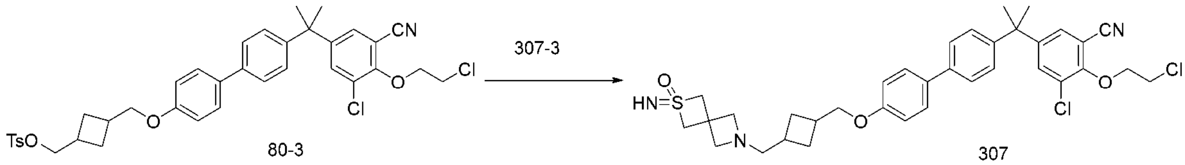


80-3 307

**Preparation of compound 307-2**

Compound **307-1** (400 mg, 1.86 mmol) was dissolved in methanol (8 mL). (Diacetoxyiodo)benzene (1.80 g, 5.57 mmol) and ammonium carbamate (580.2 mg, 7.43 mmol) were added separately in an ice-water bath. The reaction mixture was stirred at room temperature for 1 h. The reaction mixture was concentrated, and the resulting residue was purified by silica gel column chromatography (DCM/MeOH = 9: 1) to obtain 395 mg of the title compound **307-2.** LC-MS (ESI): m/z 247.0 $[M+H]^+$.

**Preparation of compound 307-3**

Compound **307-2** (85 mg, 345.07 μmol) was dissolved in DCM (1.5 mL). TFA (0.5 mL) was added, and the mixture was stirred at room temperature for 1.5 h. After the reaction was completed, the reaction mixture was concentrated

to dryness to obtain a crude product of the title compound **307-3.** LC-MS (ESI): m/z 147.2 [M+H]+.

Preparation of compound **307**

Compound **80-3** (160 mg, 235.76 μmol) was dissolved in acetonitrile (2.8 mL). The crude product of compound **307-3** and potassium carbonate (162.9 mg, 1.18 mmol) were added separately. The reaction mixture was stirred at 90°C overnight in a sealed tube. After the reaction was completed, the reaction mixture was cooled to room temperature, diluted with a small amount of acetonitrile, filtered, and then purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 30-50% 8 min, 50-60% 4 min; flow rate: 30 mL/min) to obtain 5.7 mg of the title compound **307.** LC-MS (ESI): m/z 652.3 [M+H]+. $^1$H NMR (400 MHz, DMSO-$d_6$) δ 7.70 (d, *J* = 2.3 Hz, 1H), 7.64 (d, *J* = 2.3 Hz, 1H), 7.59 - 7.51 (m, 4H), 7.32 - 7.27 (m, 2H), 7.02 - 6.96 (m, 2H), 4.42 (t, *J* = 5.2 Hz, 2H), 4.38 (s, 1H), 4.14 - 4.06 (m, 2H), 4.05 - 3.98 (m, 3H), 3.96 (t, *J* = 5.2 Hz, 2H), 3.90 (d, *J* = 6.3 Hz, 1H), 3.25 (t, *J* = 4.4 Hz, 4H), 2.70 - 2.65 (m, 1H), 2.38 (d, *J* = 6.6 Hz, 1H), 2.34 - 2.30 (m, 1H), 2.19 - 2.07 (m, 2H), 2.03 - 1.96 (m, 1H), 1.91 - 1.73 (m, 2H), 1.68 (s, 6H).

**Example 308: Preparation of compound 308**

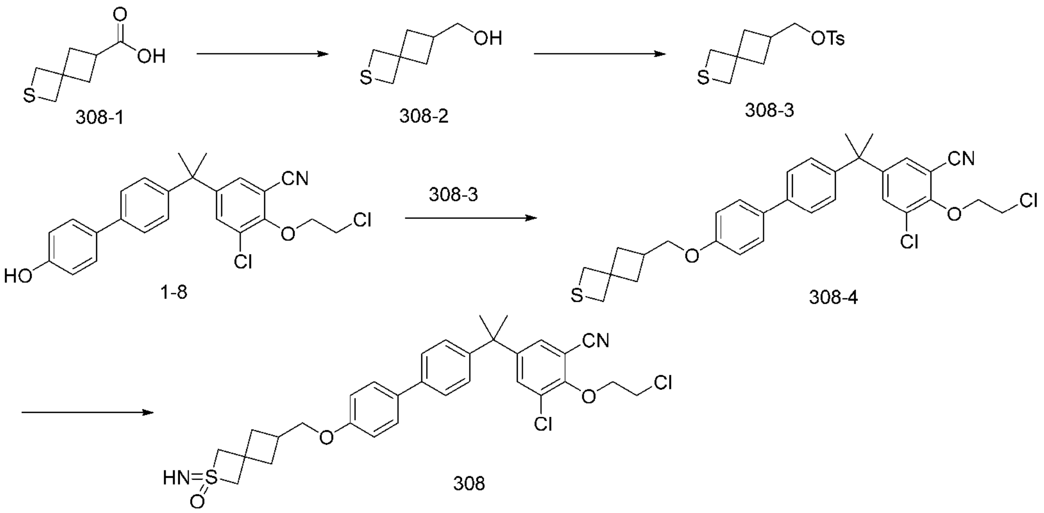

308-1 308-2 308-3 1-8 308-4 308

**Preparation of compound 308-2**

In a 25 mL two-necked flask, compound **308-1** (450 mg, 2.84 mmol) was dissolved in tetrahydrofuran (8 mL), and the mixture was cooled to 0°C under a nitrogen atmosphere. A solution of borane in tetrahydrofuran (4.3 mL, 4.3 mmol, 1 mol/L) was slowly added. The reaction mixture was stirred at 0°C for 2 h. After the reaction was completed, the reaction mixture was quenched with methanol and then concentrated under reduced pressure to obtain 400 mg of a crude product of the title compound **308-2.** The crude product was used directly in the next step without purification.

**Preparation of compound 308-3**

In a 25 mL single-necked flask, compound **308-2** (400 mg, 2.77 mmol) was dissolved in dichloromethane (2 mL), followed by the sequential addition of DMAP (34 mg, 0.28 mmol) and triethylamine (842 mg, 8.32 mmol). Under a nitrogen atmosphere, p-toluenesulfonyl chloride (1.06 g, 5.55 mmol) was added at 0°C, and the reaction mixture was stirred at room temperature for 16 h. After the reaction was completed, the reaction mixture was concentrated under reduced pressure, and the crude product was purified by column chromatography (PE/EA = 5:1) to obtain 620 mg of the title compound **308-3.** $^1$H NMR (400 MHz, Chloroform-d) δ 7.80 - 7.74 (m, 2H), 7.38 - 7.32 (m, 2H), 3.93 (d, *J* = 6.3 Hz, 2H), 3.20 (s, 2H), 3.08 (s, 2H), 2.49 - 2.37 (m, 1H), 2.45 (s, 3H), 2.29 - 2.20 (m, 2H), 1.85 - 1.76 (m, 2H).

**Preparation of compound 308-4**

In a 25 mL single-necked flask, compound **308-3** (100 mg, 0.34 mmol) was dissolved in N,Ndimethylformamide

(2 mL), followed by the sequential addition of compound **1-8** (143 mg, 0.34 mmol) and cesium carbonate (218 mg, 0.67 mmol). The reaction mixture was stirred at 60°C for 2 h. After the reaction was completed, water (5 mL) was added to the reaction mixture, and the mixture was extracted three times with ethyl acetate (5 mL). The organic phases were combined, washed once with saturated brine (5 mL), dried over anhydrous sodium sulfate, then filtered, and the filtrate was concentrated under reduced pressure. The resulting crude product was purified by silica gel column chromatography (petroleum ether: ethyl acetate = 2:1) to obtain 70 mg of the title compound **308-4.** LC-MS (ESI): m/z 552.0 [M+H]$^+$.

**Preparation of compound 308**

In a 25 mL single-necked flask, compound **308-4** (50 mg, 0.09 mmol) was dissolved in methanol (0.5 mL) and dichloromethane (0.2 mL), followed by the sequential addition of ammonium carbamate (28 mg, 0.36 mmol) and iodobenzene diacetate (88 mg, 0.27 mmol). The reaction mixture was stirred at 25°C for 16 h. After the reaction was completed, the reaction mixture was subjected to rotary evaporation until dryness under reduced pressure. The crude product was purified by column chromatography (dichloromethane: methanol = 100% to 10%) to obtain a crude product. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 17.45 mg of the title compound **308.** LC-MS (ESI): m/z 583.2 [M+H]$^+$. $^1$H NMR (400 MHz, DMSO-$d_6$) δ 7.70 (d, *J* = 2.3 Hz, 1H), 7.64 (d, *J* = 2.4 Hz, 1H), 7.61 - 7.51 (m, 4H), 7.33 - 7.27 (m, 2H), 7.02 - 6.96 (m, 2H), 4.42 (t, *J* = 5.2 Hz, 2H), 4.33 (s, 1H), 4.11 - 4.02 (m, 2H), 4.01 - 3.93 (m, 5H), 2.75 - 2.59 (m, 2H), 2.44 - 2.36 (m, 2H), 2.22 - 2.09 (m, 2H), 1.68 (s, 6H).

**Example 309: Preparation of compound 309**

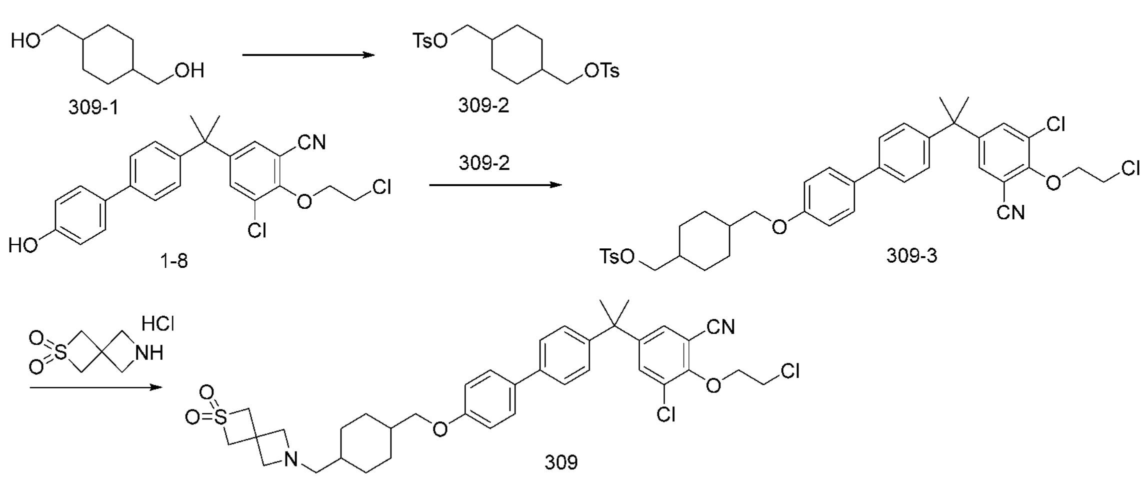


309-1 309-2 1-8 309-3 309

**Preparation of compound 309-2**

Compound **309-1** (1 g, 6.93 mmol) was dissolved in DCM (25 mL). Triethylamine (2.11 g, 20.80 mmol, 2.90 mL) and DMAP (169.4 mg, 1.39 mmol) were added at room temperature, followed by the slow, portionwise addition of *p*-toluenesulfonyl chloride (2.91 g, 15.26 mmol). The reaction mixture was stirred at room temperature overnight. After the reaction was completed, water and dichloromethane were added to the reaction mixture. The organic phase was separated, washed with saturated brine, dried over anhydrous sodium sulfate, filtered, and the filtrate was concentrated to dryness by rotary evaporation to obtain 2.85 g of a crude product of the title compound **309-2.** The product was used directly in the next step without purification.

**Preparation of compound 309-3**

Compound **1-8** (0.9 g, 2.11 mmol) was dissolved in DMF (12 mL), and compound **309-2** (1.05 g, 2.32 mmol) and cesium carbonate (1.03 g, 3.17 mmol) were added separately. The reaction mixture was stirred at 65°C overnight. After the reaction was completed, the reaction mixture was cooled to room temperature. Water and saturated aqueous ammonium chloride solution were added, and the mixture was extracted with ethyl acetate. The organic phase was washed with saturated brine, dried over anhydrous sodium sulfate, filtered, and concentrated to obtain a crude product. The crude

product was purified by silica gel column chromatography (PE/EA = 4:1) to obtain 899.9 mg of the title compound **309-3.** LC-MS (ESI): m/z 723.4 [M+18]$^+$.

**Preparation of compound 309**

Compound **309-3** (100 mg, 141.50 μmol) was suspended in acetonitrile (1.3 mL). 2-Thia-6-azaspiro[3.3]heptane 2,2-dioxide hydrochloride (33.8 mg, 183.95 μmol) and potassium carbonate (58.7 mg, 424.50 μmol) were added separately. The reaction mixture was stirred at 90°C overnight in a sealed tube. LC-MS monitoring indicated that a significant amount of starting material remained unreacted. Additional 2-thia-6-azaspiro[3.3]heptane 2,2-dioxide hydrochloride (0.2 eq) and potassium carbonate (1 eq) were added. The temperature was increased to 100°C and the mixture was stirred for 2.5 h. After the reaction was completed, the reaction mixture was cooled to room temperature, diluted with a small amount of acetonitrile, filtered, and then purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 40 to 60% gradient elution over 12 min; flow rate: 30 mL/min) to obtain 8.6 mg of the title compound 309. LC-MS (ESI): m/z 681.4 [M+H]$^+$. $^1$H NMR (400 MHz, Chloroform-d) δ 7.54 - 7.45 (m, 5H), 7.37 (d, *J* = 2.3 Hz, 1H), 7.21 (d, *J* = 8.1 Hz, 2H), 6.94 (dd, *J* = 8.6, 2.3 Hz, 2H), 4.42 (t, *J* = 6.2 Hz, 2H), 4.39 - 4.21 (m, 4H), 4.01 - 3.90 (m, 2H), 3.88 (t, *J* = 6.3 Hz, 2H), 3.79 (d, *J* = 6.3 Hz, 2H), 3.63 - 3.39 (m, 4H), 2.92 - 2.55 (m, 5H), 2.26 - 2.18 (m, 1H), 2.02 - 1.86 (m, 4H), 1.69 (s, 6H).

**Example 310: Preparation of compound 310**

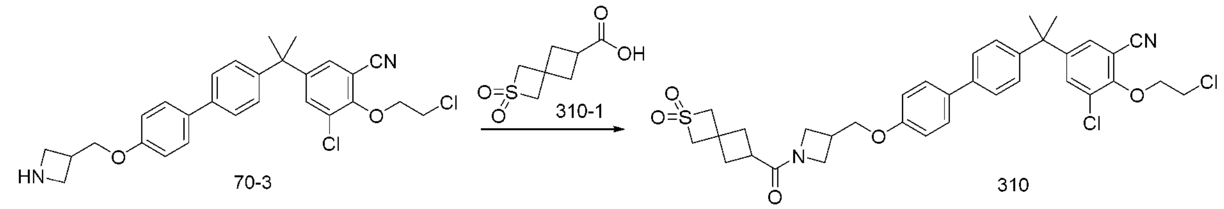

70-3

310-1

310

**Preparation of compound 310**

Compound **70-3** (95 mg, 0.192 mmol) was dissolved in DMF (2 mL), and then compound **310-1** (36.5 mg, 0.192 mmol), DIEA (124 mg, 0.959 mmol), and HATU (109.4 mg, 0.288 mmol) were added thereto. The reaction system was then stirred at 15°C for 12 h. After the reaction was completed, the solvent was removed by rotary evaporation to dryness. 20 mg of the crude product was taken, dissolved in acetonitrile and dioxane, and subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2.61 mg of the title compound **310.** LC-MS (ESI): m/z 667.2 [M+H]$^+$. $^1$H NMR (400 MHz, Chloroform-d) δ 7.55 - 7.46 (m, 5H), 7.37 (d, *J* = 2.3 Hz, 1H), 7.22 (d, *J* = 8.4 Hz, 2H), 6.96 (d, *J* = 8.7 Hz, 2H), 4.42 (t, *J* = 6.2 Hz, 2H), 4.23 (t, *J* = 8.4 Hz, 1H), 4.19 - 4.07 (m, 7H), 3.99 (dd, *J* = 8.5, 5.3 Hz, 1H), 3.95 - 3.90 (m, 1H), 3.88 (t, *J* = 6.2 Hz, 2H), 3.15 - 2.99 (m, 2H), 2.72 - 2.63 (m, 2H), 2.54 - 2.46 (m, 2H), 1.69 (s, 6H).

**Example 311: Preparation of compound 311**

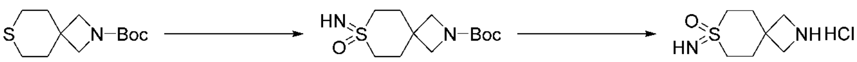

289-6 311-1 311-2

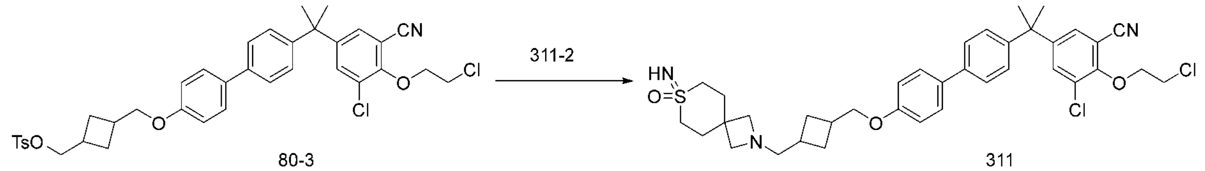


80-3 311

**Preparation of compound 311-1**

Compound **289-6** (196.6 mg, 807.84 μmol) was dissolved in methanol (4 mL). (Diacetoxyiodo)benzene (780.6 mg, 2.42 mmol) and ammonium carbamate (252.3 mg, 3.23 mmol) were added separately in an ice-water bath. The mixture was gradually warmed to room temperature and stirred for 1 h. The reaction mixture was concentrated and purified by silica gel column chromatography (DCM/MeOH = 9:1) to obtain 266.7 mg of a crude product of the title compound **311-1.** LC-MS (ESI): m/z 275.2 $[M+H]^+$.

**Preparation of compound 311-2**

The crude product of compound **311-1** (266.8 mg, 972.38 μmol) was dissolved in DCM (3 mL), and a solution of hydrogen chloride in 1,4-dioxane (4.0 mmol, 1.0 mL, 4 mol/L) was added. The mixture was stirred at room temperature for 1.5 h. The mixture was concentrated to dryness by rotary evaporation to obtain 205 mg of a crude product of the title compound **311-2,** which was used directly in the next step. LC-MS (ESI): m/z 175.0 $[M+H]^+$.

**Preparation of compound 311**

Compound **80-3** (129 mg, 190.08 μmol) was suspended in acetonitrile (2.5 mL). The crude product of compound **311-2** (80.1 mg, 380.16 μmol) and potassium carbonate (170.8 mg, 1.24 mmol) were added separately. The reaction mixture was stirred at 90°C overnight in a sealed tube. After the reaction was completed, the reaction mixture was cooled to room temperature, diluted with acetonitrile, and filtered through a membrane filter. The filtrate was purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 30 to 50% for 8 min, held at 50% for 2 min; flow rate: 30 mL/min) to obtain 19.6 mg of the title compound **311.** LC-MS (ESI): m/z 680.4 $[M+H]^+$. $^1H$ NMR (400 MHz, DMSO-$d_6$) δ 7.70 (d, $J$ = 2.3 Hz, 1H), 7.64 (d, $J$ = 2.3 Hz, 1H), 7.56 (d, $J$ = 8.8 Hz, 2H), 7.55 (d, $J$ = 8.4 Hz, 2H), 7.29 (d, $J$ = 8.4 Hz, 2H), 6.99 (d, $J$ = 8.8 Hz, 2H), 4.42 (t, $J$ = 5.2 Hz, 2H), 4.01 (d, $J$ = 7.1 Hz, 1H), 3.96 (t, $J$ = 5.2 Hz, 2H), 3.90 (d, $J$ = 6.4 Hz, 1H), 3.50 (s, 1H), 2.95 (d, $J$ = 15.2 Hz, 3H), 2.91 - 2.86 (m, 3H), 2.43 (d, $J$ = 6.6 Hz, 1H), 2.34 - 2.27 (m, 1H), 2.30 - 2.07 (m, 3H), 2.07 - 1.96 (m, 4H), 1.92 - 1.77 (m, 2H), 1.68 (s, 6H), 1.58 - 1.45 (m, 2H).

**Example 312: Preparation of compound 312**

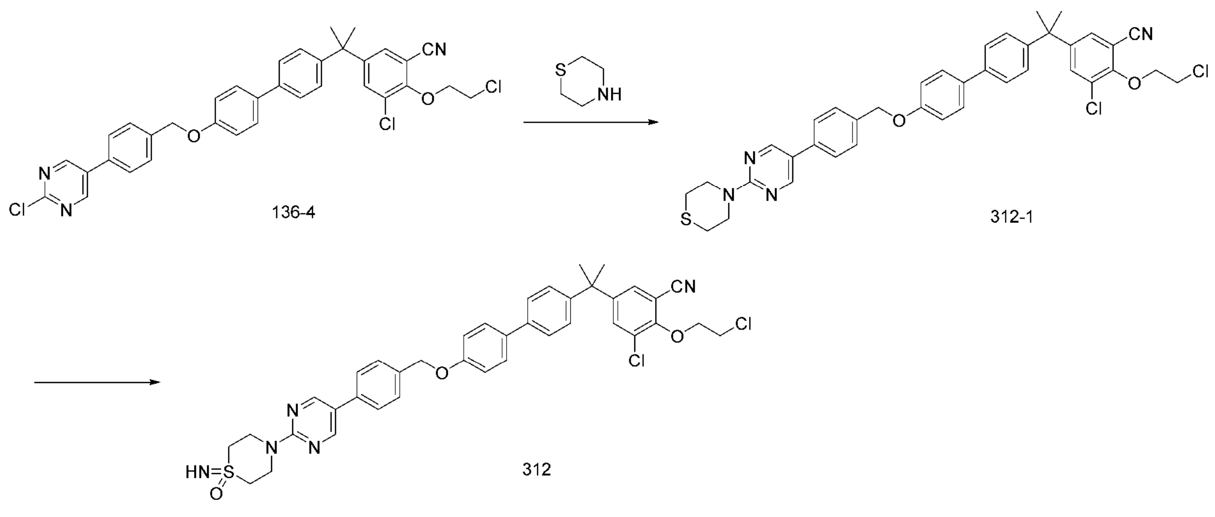

136-4 312-1

312

**Preparation of compound 312-1**

Compound **136-4** (110 mg, 0.16 mmol), thiomorpholine (20 mg, 0.19 mmol), and DIEA (105 mg, 0.8 mmol) were dissolved in 1,4-dioxane (3 mL). The reaction system was stirred under microwave irradiation at 110°C for 2 h. After the reaction was completed, water (50 mL) was added to the reaction mixture, and the mixture was extracted with ethyl acetate (100 mL). The organic phase was washed once with saturated brine (50 mL), dried over anhydrous sodium sulfate, subjected to suction filtration, and concentrated to obtain 100 mg of a crude product of the title compound **312-1.** LC-MS (ESI): m/z 695.3 $[M+H]^+$.

**Preparation of compound 312**

Compound **312-1** (125 mg, 0.18 mmol) and (diacetoxyiodo)benzene (174 mg, 0.5 mmol) were added to MeOH (3 mL). The mixture was cooled to 0°C, and ammonium carbamate (56.06 mg, 0.7 mmol) was added. The mixture was slowly warmed to room temperature and stirred for 3 h. After the reaction was completed, water (50 mL) was added to the reaction mixture, and the mixture was extracted with ethyl acetate (100 mL). The organic phase was washed once with saturated brine (50 mL), dried over anhydrous sodium sulfate, subjected to suction filtration, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 10 mg of the title compound **312.** LC-MS (ESI): m/z 726.2 $[M+H]^+$. $^1H$ NMR (400 MHz, DMSO-$d_6$) δ 8.79 (s, 2H), 7.74 - 7.66 (m, 3H), 7.64 (d, *J* = 2.3 Hz, 1H), 7.61 - 7.50 (m, 6H), 7.30 (d, *J* = 8.4 Hz, 2H), 7.10 (d, *J* = 8.9 Hz, 2H), 5.20 (s, 2H), 4.50 - 4.36 (m, 4H), 4.05 - 3.91 (m, 4H), 3.84 (s, 1H), 3.09 - 3.00 (m, 4H), 1.68 (s, 6H).

**Example 313: Preparation of compound 313**

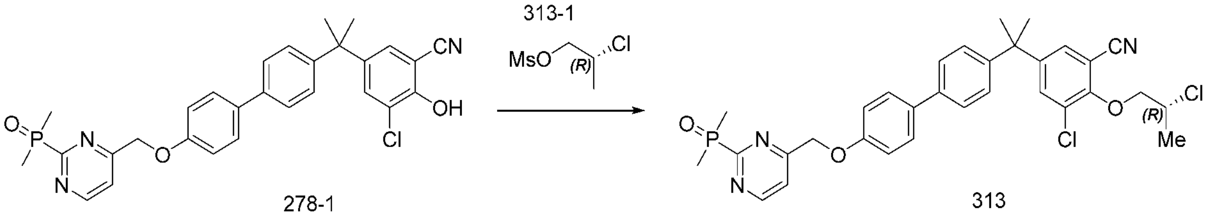


278-1

313

**Preparation of compound 313**

Compound **278-1** (50 mg, 0.09 mmol) and compound **313-1** (33 mg, 0.18 mmol) were dissolved in DMF (5 mL), and potassium carbonate (37 mg, 2.7 mmol) was added. After the addition was completed, the reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 4.09 mg of the title compound **313.** LC-MS (ESI): m/z 608.2 $[M+H]^+$. $^1H$ NMR (400 MHz, Chloroform-d) δ 8.88 (s, 1H), 7.69 (s, 1H), 7.54 (d, *J* = 8.4 Hz, 2H), 7.49 (d, *J* = 8.4 Hz, 2H), 7.48 - 7.45 (m, 1H), 7.37 (d, *J* = 2.3 Hz, 1H), 7.23 (d, *J* = 8.4 Hz, 2H), 7.04 (d, *J* = 8.4 Hz, 2H), 5.30 (s, 2H), 4.43 - 4.27 (m, 2H), 4.22 (dd, *J* = 9.5, 6.8 Hz, 1H), 1.91 (d, *J* = 13.5 Hz, 6H), 1.71 (d, *J* = 6.5 Hz, 3H), 1.69 (s, 6H). $^{31}P$ NMR (162 MHz, Chloroform-d) δ 35.20 (s, 1P).

**Example 314: Preparation of compound 314**

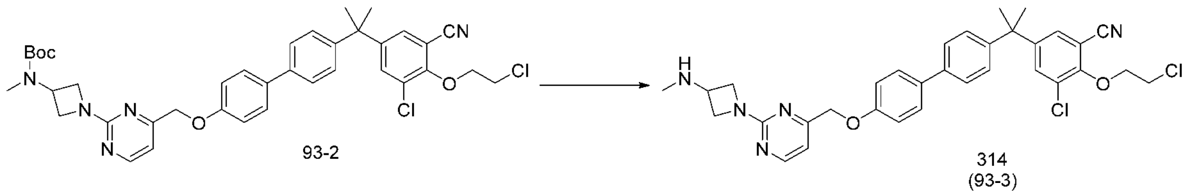

93-2

314 (93-3)

**Preparation of compound 314**

Compound **93-2** (120 mg, 0.17 mmol) was dissolved in DCM (5 mL), and a solution of hydrogen chloride in 1,4-dioxane (3 mol/L, 5 mL) was added thereto. After the addition was completed, the reaction system was stirred at room temperature for 3 h. After the reaction was completed, the solvent was removed by rotary evaporation to dryness. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 83.7 mg of the title compound **314.** LC-MS (ESI): m/z 602.2 $[M+H]^+$. $^1H$ NMR (400 MHz, Chloroform-d) δ 8.30 (d, *J* = 5.5 Hz, 1H), 7.55 - 7.44 (m, 5H), 7.36 (d, *J* = 2.3 Hz, 1H), 7.25 - 7.18 (m, 2H), 7.02 - 6.94 (m, 3H), 5.04 (s, 2H), 4.51 (d, *J* = 6.0 Hz, 4H), 4.42 (t, *J* = 6.2 Hz, 2H), 4.15 - 4.07 (m, 1H), 3.87 (t, *J* = 6.1 Hz, 2H), 2.74 (s, 3H), 1.68 (s, 6H).

**Example 315: Preparation of compound 315**

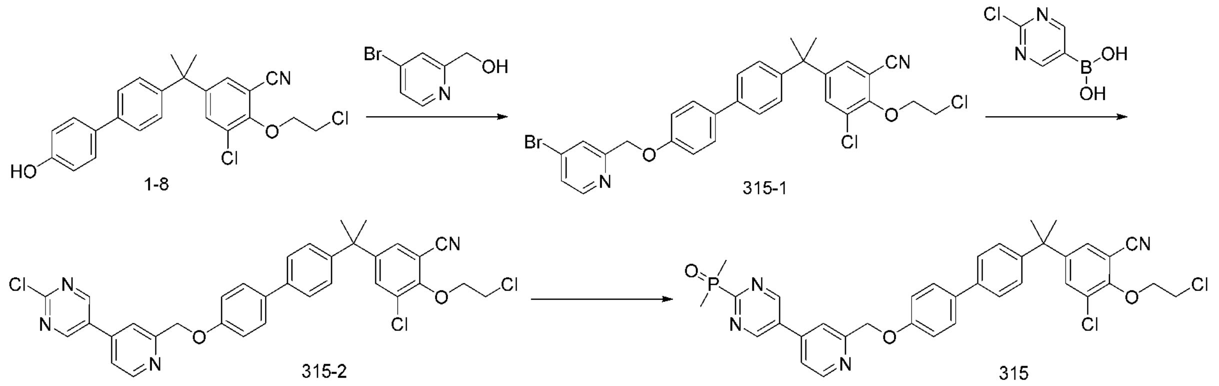

1-8

315-1

315-2

315

**Preparation of compound 315-1**

Compound **1-8** (200 mg, 0.47 mmol), (4-bromopyridin-2-yl)methanol (133 mg, 0.71 mmol), and *N*,*N*,*N'*,*N'*-tetramethylazodicarboxamide (162 mg, 0.94 mmol) were dissolved in dichloromethane (20 mL), and tri-n-butylphosphine (285 mg, 1.41 mmol) was added thereto at 0°C. After the addition was completed, the reaction system was stirred at 25°C for 1 h. After the reaction was completed, saturated brine (20 mL) was added, and the mixture was extracted three times with dichloromethane (20 mL). The organic phases were combined, washed twice with saturated brine (20 mL), dried over anhydrous sodium sulfate, filtered, and the filtrate was concentrated to obtain a crude product. The crude product was purified by silica gel column chromatography (PE/EA = 100% to 70%) to obtain 200 mg of the title compound **315-1.** LC-MS (ESI): m/z 595.2 $[M+H]^+$.

**Preparation of compound 315-2**

Compound **315-1** (200 mg, 0.34 mmol), (2-chloropyrimidin-5-yl)boronic acid (54 mg, 0.34 mmol), and potassium carbonate (95 mg, 0.68 mmol) were dissolved in 1,4-dioxane (10 mL) and water (2 mL), and $Pd(dppf)Cl_2$ (51 mg, 0.07 mmol) was added thereto. After the addition was completed, the reaction system was stirred at 80°C for 2 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 50%) to obtain 50 mg of the title compound **315-2.** LC-MS (ESI): m/z 629.2 $[M+H]^+$.

**Preparation of compound 315**

Compound **315-2** (50 mg, 0.08 mmol) was dissolved in DMF (5 mL). Dimethylphosphine oxide (45 mg, 0.57 mmol), $Pd_2(dba)_3$ (18 mg, 0.02 mmol), Xantphos (27 mg, 0.05 mmol), and DIPEA (74 mg, 0.57 mmol) were dissolved in DMF (5 mL). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 55.52 mg of the title compound 315. LC-MS (ESI): m/z 671.3 $[M+H]^+$. $^1H$ NMR (400 MHz, Chloroform-d) δ 9.13 (s, 2H), 8.80 (s, 1H), 7.81 (s, 1H), 7.55 (d, *J* = 8.0 Hz, 2H), 7.51 - 7.44 (m, 4H), 7.37 (d, *J* = 2.4 Hz, 1H), 7.22 (d, *J* = 8.1 Hz, 2H), 7.09 (d, *J* = 8.1 Hz, 2H), 5.36 (s, 2H), 4.42 (t, *J* = 6.1 Hz, 2H), 3.88 (t, *J* = 6.2 Hz, 2H), 1.94 (d, *J* = 13.4 Hz, 6H), 1.69 (s, 6H). $^{31}P$ NMR (162 MHz, Chloroform-d) δ 35.39 (s, 1P).

**Example 316: Preparation of compound 316**

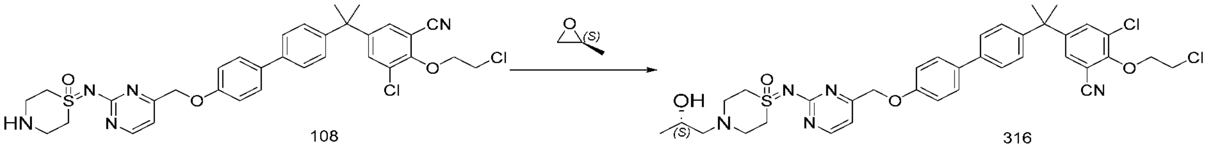

108

316

**Preparation of compound 316**

Compound **108** (100 mg, 0.15 mmol), (S)-2-epoxypropane (87 mg, 1.5 mmol), and cesium carbonate (146 mg, 0.45 mmol) were dissolved in DMF (5 mL). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2.39 mg of the title compound **316.** LC-MS (ESI): m/z 708.3 $[M+H]^+$. $^1H$ NMR (400 MHz, Chloroform-d) δ 8.47 (d, *J* = 5.2 Hz, 1H), 7.57 - 7.48 (m, 5H), 7.40 (d, *J* = 2.3 Hz, 1H), 7.25 (dd, *J* = 8.4, 3.5 Hz, 2H), 7.10 (d, *J* = 5.1 Hz, 1H), 7.03 (d, *J* = 8.7 Hz, 2H), 5.13 (s, 2H), 4.45 (t, *J* = 6.2 Hz, 2H), 3.97 (s, 1H), 3.93 - 3.82 (m, 4H), 3.72 - 3.61 (m, 2H), 3.40 - 3.30 (m, 2H), 3.28 - 3.16 (m, 2H), 2.67 (d, *J*= 12.6 Hz, 1H), 2.50 (d, *J*= 11.7 Hz, 2H), 1.72 (s, 6H), 1.20 (d, *J* = 6.1 Hz, 3H).

**Example 317: Preparation of compound 317**

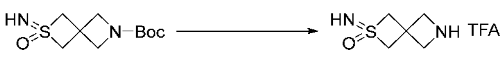

317-1

317-2

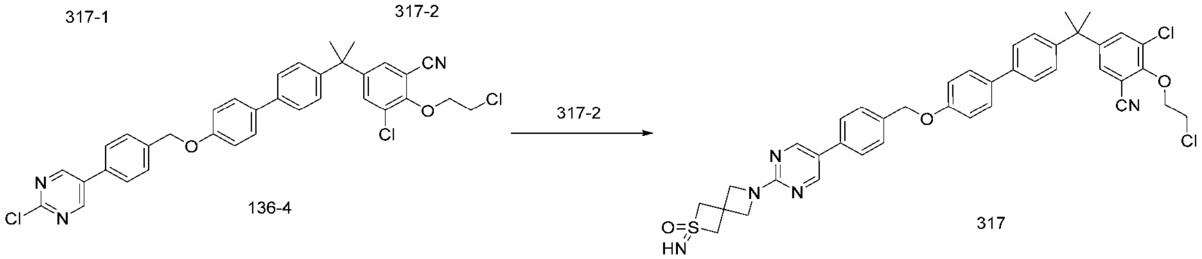


136-4

317

**Preparation of compound 317-2**

Compound **317-1** (30 mg, 0.12 mmol) was dissolved in TFA/DCM (3 mL), and the reaction system was stirred at room temperature for 1 h. After the reaction was completed, the reaction mixture was directly concentrated to obtain a crude product of the title compound **317-2.** LC-MS (ESI): m/z 146.9 [M+H]$^+$.

**Preparation of compound 317**

Compound **317-2** (18 mg, 0.12 mmol), **136-4** (60 mg, 0.1 mmol), and DIEA (66 mg, 0.5 mmol) were added to EtOH (5 mL), and the mixture was stirred under microwave irradiation at 100°C for 2 h. After the reaction was completed, the reaction mixture was directly concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 11 mg of the title compound **317.** LC-MS (ESI): m/z 738.2 [M+H]$^+$. $^1$H NMR (400 MHz, DMSO-$d_6$) δ 8.73 (s, 2H), 7.70 (d, *J* = 2.3 Hz, 1H), 7.67 (d, *J* = 8.0 Hz, 2H), 7.64 (d, *J* = 2.3 Hz, 1H), 7.59 (d, *J* = 8.8 Hz, 2H), 7.57 - 7.52 (m, 4H), 7.29 (d, *J* = 8.1 Hz, 2H), 7.09 (d, *J* = 8.8 Hz, 2H), 5.19 (s, 2H), 4.50 (s, 110), 4.42 (t, *J* = 5.1 Hz, 2H), 4.37 - 4.19 (m, 8H), 3.96 (t, *J* = 5.1 Hz, 2H), 1.68 (s, 6H).

**Example 318: Preparation of compound 318**

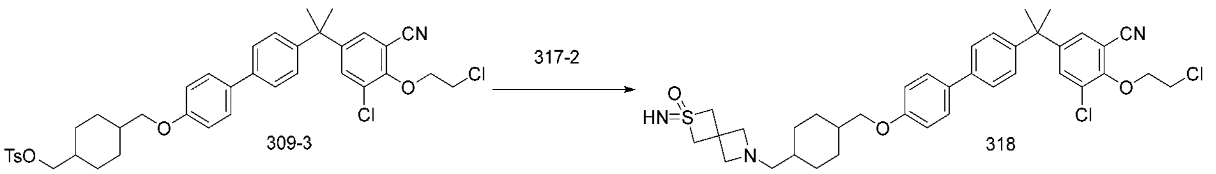


309-3

318

**Preparation of compound 318**

Compound **309-3** (149 mg, 210.83 μmol) was dissolved in acetonitrile (1.8 mL). Compound **317-2** (109.7 mg, 421.67 μmol) and potassium carbonate (204 mg, 1.48 mmol) were added separately. The reaction mixture was heated and stirred at 92°C overnight in a sealed tube. After the reaction was completed, the reaction mixture was cooled to room temperature, diluted with acetonitrile, and filtered. The filtrate was purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 40-60% 8 min, 60-80% 4 min; flow rate: 30 mL/min) to obtain 12 mg of the title compound **318.** LC-MS (ESI): m/z 680.4 [M+H]$^+$. $^1$H NMR (400 MHz, DMSO-$d_6$) δ 7.70 (d, *J* = 2.4 Hz, 1H), 7.64 (d, *J* = 2.4 Hz, 1H), 7.61 - 7.50 (m, 4H), 7.29 (d, *J* = 8.5 Hz, 2H), 7.03 - 6.95 (m, 2H), 4.42 (t, *J* = 5.2 Hz, 2H), 4.39 (s, 1H), 4.06 (q, *J* = 13.2 Hz, 4H), 3.96 (t, *J* = 5.2 Hz, 2H), 3.80 (d, *J* = 6.3 Hz, 1H), 3.28 - 3.23 (m, 4H), 1.91 - 1.71 (m, 4H), 1.68 (s, 6H), 1.56 - 1.33 (m, 4H), 1.11 - 0.95 (m, 2H), 0.93 - 0.76 (m, 2H).

**Example 319: Preparation of compound 319**

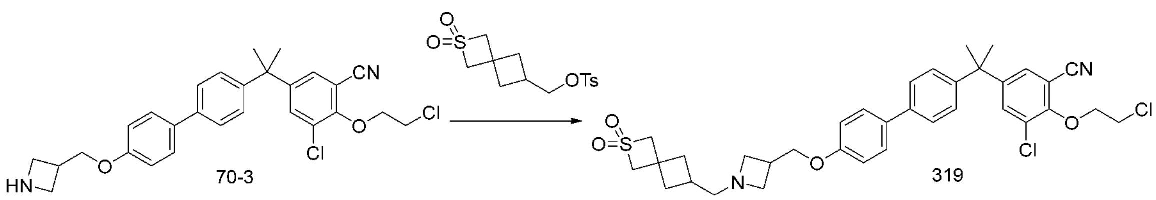

70-3

319

**Preparation of compound 319**

Compound **70-3** (48 mg, 0.097 mmol) was dissolved in acetonitrile (5 mL). (2,2-Dioxide-2-thiaspiro[3.3]heptan-6-yl)methyl 4-methylbenzenesulfonate (38.4 mg, 0.116 mmol) was added thereto, and the mixture was stirred at room temperature for 5 min. Potassium carbonate (26.7 mg, 0.194 mmol) was added, and the reaction system was stirred at

90°C for 15 h. After the reaction was completed, acetonitrile was removed by evaporation under reduced pressure. The residue was dissolved in acetonitrile and dioxane, filtered, and the filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15 mg of compound **319.** LC-MS (ESI): m/z 653.4 [M+H]+. $^1$H NMR (400 MHz, DMSO-$d_6$) δ 7.70 (d, $J$ = 2.3 Hz, 1H), 7.64 (d, $J$ = 2.3 Hz, 1H), 7.62 - 7.51 (m, 4H), 7.30 (d, $J$ = 8.5 Hz, 2H), 7.02 (d, $J$ = 8.5 Hz, 2H), 4.42 (t, $J$ = 5.2 Hz, 2H), 4.25 - 4.21 (m, 1H), 4.19 - 4.08 (m, 4H), 4.06 (s, 1H), 3.96 (t, $J$ = 5.2 Hz, 2H), 3.72 - 3.55 (m, 2H), 3.49 (d, $J$ = 5.7 Hz, 1H), 3.02 - 2.88 (m, 1H), 2.74 (s, 2H), 2.40 - 2.26 (m, 4H), 2.07 - 1.98 (m, 1H), 1.97 - 1.85 (m, 1H), 1.68 (s, 6H).

**Example 320: Preparation of compound 320**

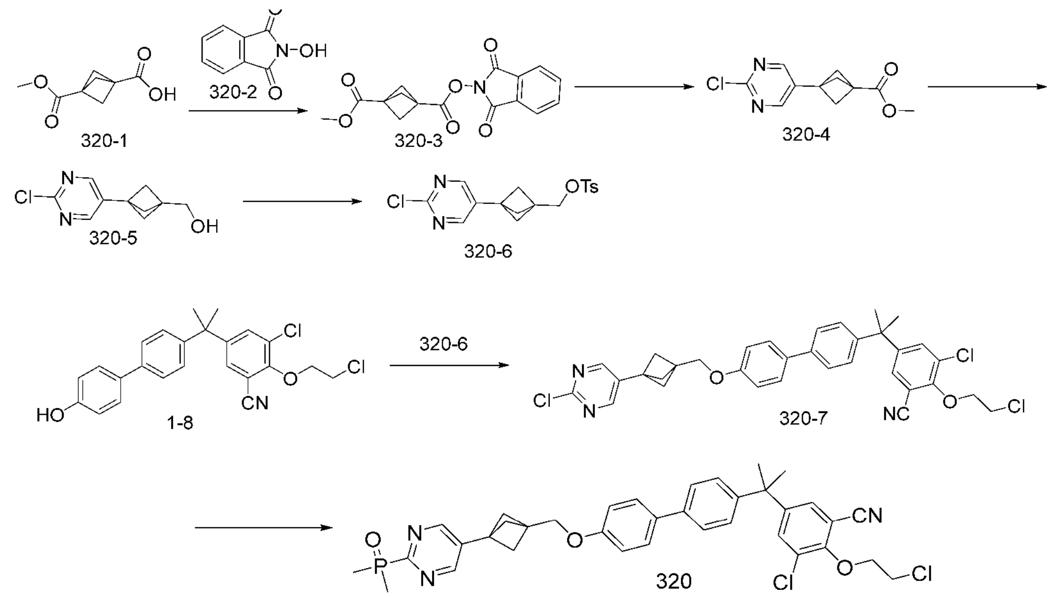


**Preparation of compound 320-3**

Compound **320-1** (10 g, 58.77 mmol) was added to dichloromethane (100 mL). The system was purged with argon, and the temperature was lowered to 0°C. Compound **320-2** (10.6 g, 64.65 mmol) and DMAP (720 mg, 5.88 mmol) were added to the reaction system, and the mixture was stirred for 10 min. *N, N'*-Diisopropylcarbodiimide (8.9 g, 70.52 mmol) was then slowly added dropwise to the reaction mixture, and the mixture was stirred at room temperature for 16 h. Dichloromethane (200 mL) was added, and the mixture was washed with saturated sodium chloride (600 mL). The organic phase was dried over anhydrous sodium sulfate, subjected to suction filtration, and the filtrate was concentrated to obtain a crude product. The crude product was purified by silica gel column chromatography (PE/EA = 100% to 30%) to obtain 8 g of the title compound **320-3.** $^1$H NMR (400 MHz, DMSO-*d6*) δ 8.00 - 7.94 (m, 4H), 3.66 (s, 3H), 2.53 (s, 6H).

**Preparation of compound 320-4**

Compound **320-3** (1.4 g, 4.44 mmol) was dissolved in *N,N*-dimethylacetamide (20 mL), followed by the sequential addition of 5-bromo-2-chloropyrimidine (911 mg, 4.44 mmol), diethyl 2,6-dimethyl-1,4-dihydro-3,5-pyridinedicarboxylate (2.25 g, 8.88 mmol), (2,2'-bipyridine)nickel(II) dibromide (890 mg, 0.89 mmol), and sodium bicarbonate (1.49 g, 17.76 mmol). The mixture was stirred at 25°C for 16 h under irradiation with a 380-385 nm UV lamp. TLC indicated the disappearance of the starting material. The reaction was quenched with water (50 mL) and extracted three times with ethyl acetate (50 mL). The organic phases were combined, washed three times with saturated sodium chloride solution (100 mL), dried over anhydrous sodium sulfate, subjected to suction filtration, and the filtrate was concentrated. The resulting residue was purified by silica gel column chromatography (PE/EA = 100% to 85%) to obtain 280 mg of the title compound **320-4.** LC-MS (ESI): m/z 239.0 [M+H]+.

**Preparation of compound 320-5**

Compound **320-4** (280 mg, 1.12 mmol) was dissolved in methanol (10 mL). The system was purged with argon and cooled to 0°C. Sodium borohydride (64 mg, 1.68 mmol) was slowly added to the reaction mixture, and the mixture was stirred for 2 h. TLC indicated the disappearance of the starting material. The reaction was quenched with acetone (10 mL) and concentrated to obtain a crude product. The crude product was purified by silica gel column chromatography (DCM/MeOH = 100% to 5%) to obtain 100 mg of the title compound **320-5.** LC-MS (ESI): m/z 211.1 $[M+H]^+$.

**Preparation of compound 320-6**

Compound **320-5** (100 mg, 0.47 mmol) was dissolved in DCM (3 mL). The mixture was cooled to 0°C, followed by the sequential addition of triethylamine (71 mg, 0.70 mmol) and *p*-toluenesulfonyl chloride (99 mg, 0.52 mmol). The reaction mixture was stirred at room temperature for 1 h. TLC indicated the disappearance of the starting material. The reaction was quenched with ice water (10 mL), and the reaction system was extracted three times with DCM (10 mL). The organic phases were combined, dried over anhydrous sodium sulfate, subjected to suction filtration, and the filtrate was concentrated. The resulting residue was purified by silica gel column chromatography (PE/EA = 100% to 40%) to obtain 95 mg of the title compound **320-6.** LC-MS (ESI): m/z 365.0 $[M+H]^+$.

**Preparation of compound 320-7**

Compound **320-6** (95 mg, 0.26 mmol) was dissolved in acetonitrile (5 mL), and compound **1-8** (221 mg, 0.52 mmol) and cesium carbonate (254 mg, 0.78 mmol) were added. The mixture was stirred at 70°C for 2 h. TLC indicated the disappearance of the starting material. The reaction was quenched with ice water (5 mL), and the system was extracted three times with ethyl acetate (10 mL). The organic phases were combined, washed three times with saturated sodium chloride solution (30 mL), dried over anhydrous sodium sulfate, subjected to suction filtration, and the filtrate was concentrated. The resulting residue purified by silica gel column chromatography (PE/EA = 100% to 75%) to obtain 45 mg of the title compound **320-7.** $^1$H NMR (400 MHz, $CDCl_3$) δ 8.40 (s, 2H), 7.46 - 7.42 (m, 5H), 7.30 (d, *J* = 2.2 Hz, 1H), 7.15 (d, *J* = 8.3 Hz, 2H), 6.90 (d, *J* = 8.7 Hz, 2H), 4.35 (t, *J* = 6.2 Hz, 2H), 4.04 (s, 2H), 3.81 (t, *J* = 6.2 Hz, 2H), 2.14 (s, 6H), 1.62 (s, 6H).

**Preparation of compound 320**

Compound **320-7** (45 mg, 0.073 mmol), dimethylphosphine oxide (23 mg, 0.29 mmol), palladium acetate (4 mg, 0.015 mmol), 1,3-bis(diphenylphosphino)propane (6 mg, 0.015 mmol), and *N,N*-diisopropylethylamine (47 mg, 0.29 mmol) were dissolved in *N,N*-dimethylformamide (1 mL). The system was purged with argon, and the reaction was carried out under microwave irradiation at 120°C for half an hour. After filtration, the mixture was subjected to preparative purification (preparative method: mobile phase: A: 0.03% aqueous ammonia; B: acetonitrile; chromatographic column: XBridge C18, 19 * 250 mm, 10 μm; column temperature: 25°C; gradient: 72% to 72% acetonitrile gradient elution over 16 min; flow rate: 20 mL/min) to obtain 12.17 mg of the title compound **320.** LC-MS (ESI): m/z 660.2 $[M+H]^+$. $^1$H NMR (400 MHz, DMSO-$d_6$) δ 8.89 (s, 2H), 7.70 (d, *J* = 2.3 Hz, 1H), 7.64 (d, *J* = 2.3 Hz, 1H), 7.59 (d, *J* = 8.8 Hz, 2H), 7.56 (d, *J* = 8.4 Hz, 2H), 7.30 (d, *J* = 8.4 Hz, 2H), 7.04 (d, *J*= 8.8 Hz, 2H), 4.42 (t, *J* = 5.2 Hz, 2H), 4.16 (s, 2H), 3.96 (t, *J*= *5.2* Hz, 2H), 2.18 (s, 6H), 1.74 (d, *J* = 13.7 Hz, 6H), 1.68 (s, 6H). $^{31}$P NMR (162 MHz, DMSO-*d6*) δ 33.56 (s, 1P).

**Example 321: Preparation of compound 321**

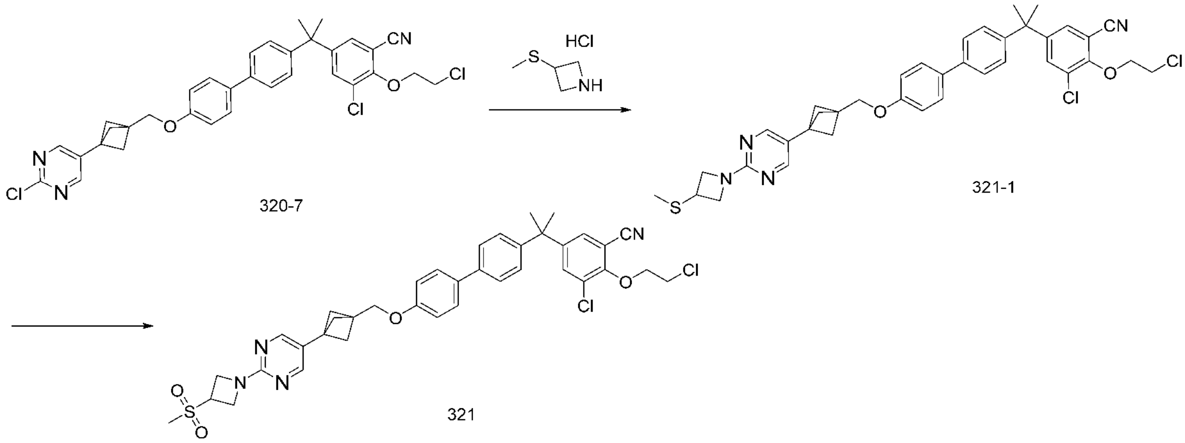


320-7

321-1

321

## Preparation of compound 321-1

Compound **320-7** (20.0 mg, 0.032 mmol), 3-(methylthio)azetidine hydrochloride (5.4 mg, 0.038 mmol), triethylamine (9.7 mg, 0.096 mmol), and ethanol (1 mL) were added to an eggplant-shaped flask, and the system was reacted at 100°C for 2 h. The reaction mixture was subjected to rotary evaporation until dryness. Water (10 mL) was added, and the mixture was extracted with ethyl acetate (10 mL). The organic phase was washed with saturated brine (10 mL), dried over anhydrous sodium sulfate, subjected to suction filtration, and the filtrate was concentrated to obtain 40 mg of a crude product of the title compound **321-1.** LC-MS (ESI): m/z 685.0 $[M+H]^+$.

## Preparation of compound 321

Compound **321-1** (40.0 mg, 0.23 mmol), potassium peroxymonosulfate (40.0 mg, 0.12 mmol), tetrahydrofuran (1 mL), and water (1 mL) were added to an eggplant-shaped flask, and the system was reacted at room temperature for 16 h. The reaction was monitored by LCMS until completion. The reaction mixture was concentrated, and the resulting residue was purified by preparative liquid chromatography (Waters 2767/Qda: column: XBridge C18 19 × 250 mm, 10 μm; mobile phase: A: 0.1% FA/$H_2O$, B: ACN; flow rate: 20 mL/min; elution gradient: 82% to 92%; retention time: 8.8-9.6 min of 16 min) to obtain 14.46 mg of the title compound **321.** LC-MS (ESI): m/z 717.2 $[M+H]^+$. $^1H$ NMR (400 MHz, DMSO-$d_6$) δ 8.31 (s, 2H), 7.70 (d, *J* = 2.3 Hz, 1H), 7.63 (d, *J* = 2.3 Hz, 1H), 7.58 (d, *J* = 8.5 Hz, 2H), 7.56 (d, *J* = 7.9 Hz, 2H), 7.30 (d, *J* = 8.4 Hz, 2H), 7.02 (d, *J* = 8.8 Hz, 2H), 4.42 (t, *J* = 5.2 Hz, 2H), 4.40 - 4.35 (m, 1H), 4.29 (t, *J* = 8.7 Hz, 2H), 4.22 - 4.15 (m, 2H), 4.12 (s, 2H), 3.96 (t, *J* = 5.2 Hz, 2H), 3.05 (s, 3H), 2.05 (s, 6H), 1.68 (s, 6H).

## Example 322: Preparation of compound 322

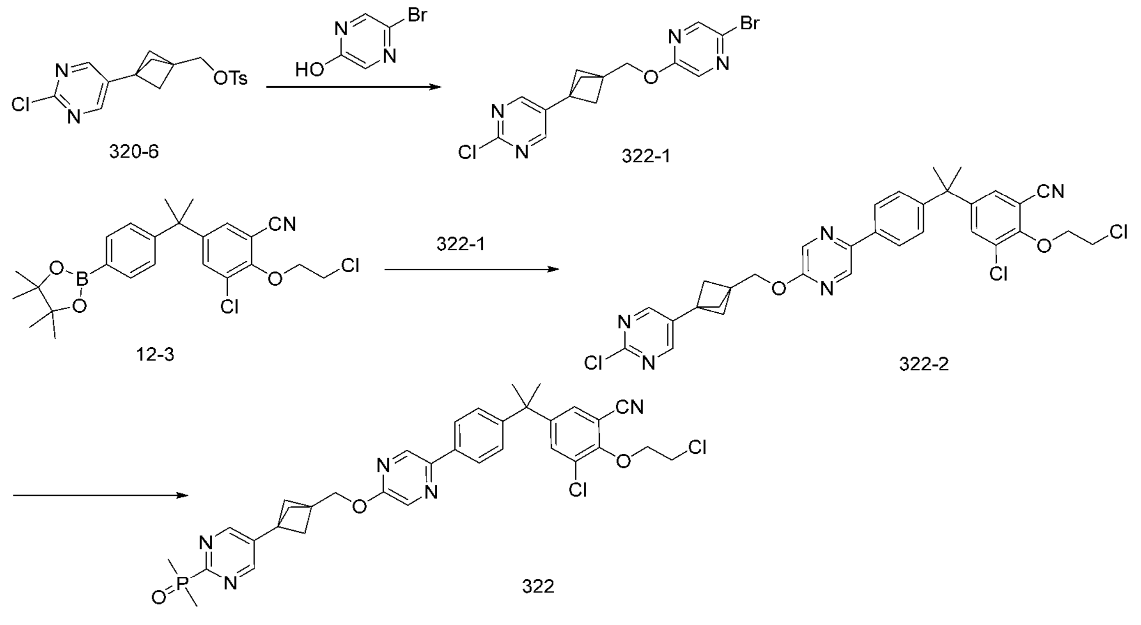

320-6 322-1 12-3 322-2 322

### Preparation of compound 322-1

Compound **320-6** (50 mg, 0.14 mmol) and 2-bromo-5-hydroxypyrazine (29.4 mg, 0.17 mmol) were dissolved in acetonitrile (3 mL). Cesium carbonate (68.4 mg, 0.21 mmol) was added, and the mixture was stirred at 70°C for 1 h. The reaction was monitored by LCMS until completion. The reaction was quenched with ice water (5 mL) and extracted three times with ethyl acetate (10 mL). The organic phases were combined, dried over anhydrous sodium sulfate, subjected to suction filtration, and the filtrate was concentrated. The resulting residue was purified by preparative TLC (PE/EA = 4/1) to obtain 18 mg of the title compound **322-1.** $^1$H NMR (400 MHz, $CDCl_3$) δ 8.45 (s, 2H), 8.16 (s, 1H), 8.05 (s, 1H), 4.41 (s, 2H), 2.17 (s, 6H).

### Preparation of compound 322-2

Compound **322-1** (18 mg, 0.05 mmol) and compound **12-3** (30 mg, 0.06 mmol) were dissolved in 1,4-dioxane (3 mL) and water (0.6 mL). Pd(dppf)$Cl_2$ (3.6 mg, 0.005 mmol) and potassium carbonate (21 mg, 0.15 mmol) were added at room temperature, and the mixture was stirred at 100°C for 2 h. The reaction was monitored by LCMS until completion. The reaction was quenched with ice water (5 mL) and extracted three times with ethyl acetate (10 mL). The organic phases were combined, dried over anhydrous sodium sulfate, subjected to suction filtration, and the filtrate was concentrated. The resulting residue was purified by preparative TLC (PE/EA = 4/1) to obtain 6 mg of the title compound **322-2.** LC-MS (ESI): m/z 620.2 $[M+H]^+$.

### Preparation of compound 322

Compound **322-2** (6 mg, 0.0097 mmol) was dissolved in *N,N*-dimethylformamide (2 mL). Dimethylphosphine oxide (1.5 mg, 0.019 mmol), palladium acetate (0.5 mg, 0.0019 mmol), 1,3-bis(diphenylphosphino)propane (0.6 mg, 0.0015 mmol), and *N,N*-diisopropylethylamine (6.3 mg, 0.05 mmol) were added. After purging with argon for 30 seconds, the mixture was stirred under microwave irradiation at 120°C for 0.5 h. The reaction was monitored by LCMS until completion. The reaction mixture was subjected to preparative purification (preparative method: mobile phase: A: 0.03% aqueous ammonia; B: acetonitrile; chromatographic column: XBridge C18, 19 * 250 mm, 10 μm; column temperature: 25°C; gradient: 67% to 67% acetonitrile gradient elution over 17 min; flow rate: 20 mL/min) to obtain 1.28 mg of the target compound **322.** LC-MS (ESI): m/z 662.3 $[M+H]^+$. $^1$H NMR (400 MHz, Methanol-$d_4$) δ 8.83 (s, 2H), 8.61 (d, *J* = 1.4 Hz, 1H), 8.28 (d, *J* = 1.3 Hz, 1H), 7.92 (d, *J* = 8.5 Hz, 2H), 7.57 (d, *J* = 2.3 Hz, 1H), 7.54 (d, *J* = 2.3 Hz, 1H), 7.37 (d, *J* = 8.5 Hz, 2H), 4.53 (s, 2H), 4.44 (t, *J* = 5.5 Hz, 2H), 3.90 (t, *J* = 5.5 Hz, 2H), 2.25 (s, 6H), 1.88 (d, *J* = 13.8 Hz, 6H), 1.73 (s, 6H). $^{31}$P NMR (162 MHz, Methanol-$d_4$) δ 40.81 (s, 1P).

### Example 323: Preparation of compounds 323, 323A, and 323B

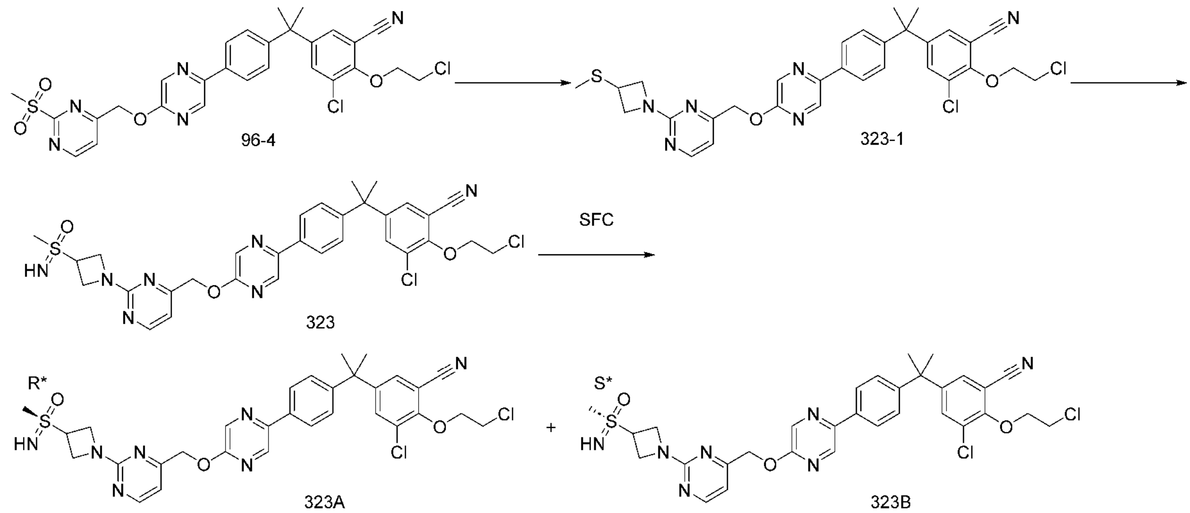


96-4

323-1

323

323A

323B

**Preparation of compound 323-1**

Compound **96-4** (100 mg, 0.17 mmol) was dissolved in acetonitrile (5 mL), followed by the sequential addition of 3-(methylthio)azetidine (26 mg, 0.26 mmol) and DIPEA (44 mg, 0.34 mmol). The reaction system was stirred at 80°C for 2 h. After the reaction was completed, water (5 mL) was added to the filtrate, and the mixture was extracted three times with dichloromethane (5 mL). The organic phases were combined, washed twice with saturated brine (5 mL), dried over anhydrous sodium sulfate, subjected to suction filtration, and the filtrate was concentrated to obtain a crude product. The crude product was purified by silica gel column chromatography (PE/EA = 100% to 30%) to obtain 100 mg of the title compound **323-1.** LC-MS (ESI): m/z 621.0 $[M+H]^+$.

**Preparation of compound 323**

Compound **323-1** (100 mg, 0.16 mmol) was dissolved in methanol (3 mL), followed by the sequential addition of ammonium carbonate (46 mg, 0.48 mmol) and (diacetoxyiodo)benzene (260 mg, 0.81 mmol). The reaction system was stirred at room temperature overnight. After the reaction was completed, water (3 mL) was added, and the mixture was extracted three times with dichloromethane (5 mL). The organic phases were combined, washed twice with saturated brine (3 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2.41 mg of the title compound 323. LC-MS (ESI): m/z 652.0 $[M+H]^+$.

**Preparation of compounds 323A and 323B**

Compound **323** (60 mg) was purified by preparative SFC (preparative conditions: instrument model: WATERS 150 preparative SFC; column model: ChiralPak AD, 250 × 30 mm I.D., 10 μm; mobile phase: A: $CO_2$, B: ethanol (0.1% $NH_3H_2O$); elution gradient: B 45%; flow rate: 120 mL/min; column pressure: 100 bar; column temperature: 38°C; detection wavelength: 220 nm; run time per injection: 5 min) to obtain 26.2 mg of the title compound **323A** and 29.3 mg of the title compound **323B.**

Compound **323A:** SFC analysis method (instrument model: Waters UPC2 analytical SFC; column model: Chiralpak AD-3 50 × 4.6 mm I.D., 3 μm; mobile phase: A: $CO_2$, B: ethanol/acetonitrile (2/1) (0.05% DEA); elution gradient: B 40%; flow rate: 2.5 mL/min; column pressure: 100 bar; column temperature: 35°C; detection wavelength: 220 nm; Rt = 8.85 min). LC-MS (ESI): m/z 652.5 $[M+H]^+$. $^1H$ NMR (400 MHz, DMSO-$d_6$) δ 8.75 (d, $J$ = 1.4 Hz, 1H), 8.53 (d, $J$ = 1.4 Hz, 1H), 8.36 (d, $J$ = 5.1 Hz, 1H), 7.96 (d, $J$ = 8.4 Hz, 2H), 7.70 (d, $J$ = 2.3 Hz, 1H), 7.63 (d, $J$ = 2.4 Hz, 1H), 7.37 (d, $J$ = 8.6 Hz, 2H), 6.75 (d, $J$ = 5.1 Hz, 1H), 5.36 (s, 2H), 4.42 (t, $J$ = 5.2 Hz, 2H), 4.31 - 4.22 (m, 4H), 4.21 - 4.16 (m, 1H), 3.99 (s, 1H), 3.95 (t, $J$ = 5.2 Hz, 2H), 2.90 (s, 3H), 1.69 (s, 6H).

Compound **323B:** SFC analysis method (instrument model: Waters UPC2 analytical SFC; column model: Chiralpak AD-3 50 × 4.6 mm I.D., 3 μm; mobile phase: A: $CO_2$, B: ethanol/acetonitrile (2/1) (0.05% DEA); elution gradient: B 40%; flow rate: 2.5 mL/min; column pressure: 100 bar; column temperature: 35°C; detection wavelength: 220 nm; Rt = 10.72 min). LC-MS (ESI): m/z 652.5 $[M+H]^+$. $^1H$ NMR (400 MHz, DMSO-$d_6$) δ 8.76 (d, $J$ = 1.5 Hz, 1H), 8.54 (d, $J$ = 1.4 Hz,

1H), 8.36 (d, *J* = 5.0 Hz, 1H), 7.96 (d, *J* = 8.4 Hz, 2H), 7.71 (d, *J* = 2.3 Hz, 1H), 7.64 (d, *J* = 2.3 Hz, 1H), 7.37 (d, *J* = 8.6 Hz, 2H), 6.76 (d, *J* = 5.0 Hz, 1H), 5.36 (s, 2H), 4.42 (t, *J* = 5.2 Hz, 2H), 4.31 - 4.22 (m, 4H), 4.21 - 4.16 (m, 1H), 3.99 (s, 1H), 3.96 (t, *J* = 5.2 Hz, 2H), 2.90 (s, 3H), 1.69 (s, 6H).

**Example 324: Preparation of compound 324**

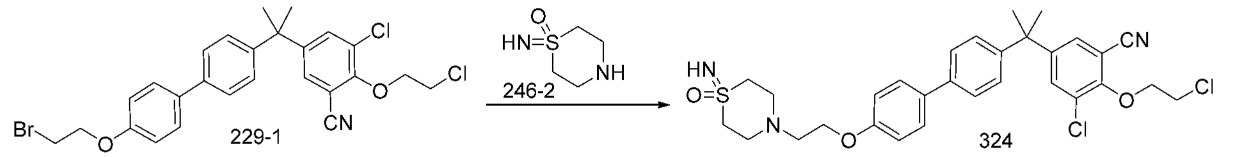


**Preparation of compound 324**

Compound **229-1** (440 mg, 0.82 mmol) and potassium carbonate (228 mg, 1.65 mmol) were dissolved in *N,N*-dimethylformamide (10 mL), and compound **246-2** (169 mg, 0.99 mmol) was added thereto. After the addition was completed, the reaction system was stirred at 80°C for 12 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 26 mg of the title compound **324.** LC-MS (ESI): m/z 586.0 $[M+H]^+$. $^1H$ NMR (400 MHz, DMSO-$d_6$) δ 7.71 (d, *J*= 2.3 Hz, 1H), 7.64 (d, *J* = 2.3 Hz, 1H), 7.58 (d, *J* = 8.8 Hz, 2H), 7.56 (d, *J* = 8.4 Hz, 2H), 7.30 (d, *J* = 8.5 Hz, 2H), 7.02 (d, *J* = 8.8 Hz, 2H), 4.42 (t, *J* = 5.2 Hz, 2H), 4.12 (t, *J* = 5.6 Hz, 2H), 3.96 (t, *J* = 5.2 Hz, 2H), 3.61 (s, 1H), 3.08 - 2.94 (m, 7H), 2.94 - 2.85 (m, 3H), 1.68 (s, 6H).

**Example 325: Preparation of compound 325**

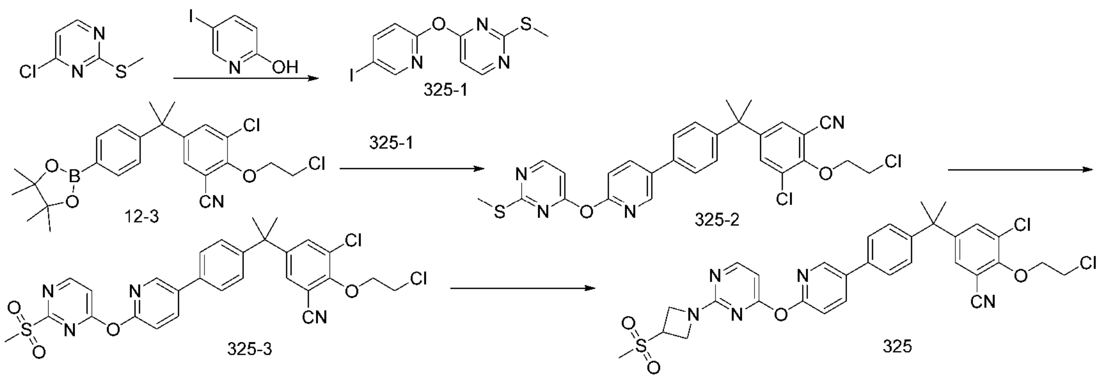


**Preparation of compound 325-1**

2-Hydroxy-5-iodopyridine (1 g, 4.52 mmol), 4-chloro-2-methylthiopyrimidine (872 mg, 5.43 mmol), $K_2CO_3$ (1.25 g, 9.05 mmol) and acetonitrile (10 mL) were added to a reaction flask, and the mixture was stirred at 80°C for 16 h. The mixture was filtered, subjected to rotary evaporation until dryness, and the resulting crude product was purified by silica gel column chromatography (PE/EA = 100% to 70%) to obtain 230 mg of the title compound **325-1**. $^1H$ NMR (400 MHz, DMSO-$d_6$) δ 8.60 (dd, *J* = 2.4, 0.6 Hz, 1H), 8.58 (d, *J* = 5.6 Hz, 1H), 8.31 (dd, *J* = 8.5, 2.4 Hz, 1H), 7.19 (dd, *J* = 8.5, 0.6 Hz, 1H), 6.93 (d, *J* = 5.6 Hz, 1H), 2.36 (s, 3H).

**Preparation of compound 325-2**

Under a nitrogen atmosphere, compound **325-1** (368 mg, 799.63 μmol), compound **12-3** (230 mg, 666.36 μmol), [1,1'-bis(diphenylphosphino)ferrocene]dichloropalladium(II) dichloromethane complex (49 mg, 66.64 μmol), $K_2CO_3$ (277 mg, 2.00 mmol), dioxane (5 mL) and $H_2O$ (1 mL) were added to a reaction flask. The system was purged three times with nitrogen, and the reaction was carried out at 100°C for 16 h. The mixture was subjected to rotary evaporation until dryness, and the crude product was purified by silica gel column chromatography (PE/EA = 100% to 50%) to obtain 300 mg of the title compound **325-2.** LC-MS (ESI): m/z 551.2 $[M+H]^+$.

**Preparation of compound 325-3**

Compound **325-2** (350 mg, 634.65 μmol) was dissolved in DCM (10 mL). *m*-CPBA (387 mg, 1.90 mmol) was added slowly. The mixture was stirred at room temperature for 1 h. The reaction mixture was poured into saturated $Na_2SO_3$ solution (20 mL) and extracted three times with DCM (30 mL). The organic phases were combined, washed three times with saturated aqueous sodium carbonate solution (20 mL), dried over anhydrous sodium sulfate, subjected to suction filtration, and the filtrate was subjected to rotary evaporation until dryness to obtain 300 mg of a crude product of the title compound**325-3.** LC-MS (ESI): m/z 583.2 $[M+H]^+$.

**Preparation of compound 325**

3-(Methylsulfonyl)azetidine hydrochloride (71 mg, 411.32 μmol), compound**325-3** (200 mg, 342.77 μmol), $K_2CO_3$ (95 mg, 685.54 μmol), and acetonitrile (2 mL) were added to a reaction flask. The mixture was stirred at 80°C for 1 h. The mixture was filtered, and the crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 35% to 85% acetonitrile gradient elution over 12 min; flow rate: 30 mL/min) to obtain 20 mg of the title compound **325.** LC-MS (ESI): m/z 638.2 $[M+H]^+$. $^1H$ NMR (400 MHz, DMSO-$d_6$) δ 8.64 (d, *J* = 2.6 Hz, 1H), 8.35 (d, *J* = 5.6 Hz, 1H), 8.24 (dd, *J* = 8.5, 2.6 Hz, 1H), 7.80 - 7.58 (m, 4H), 7.38 (d, *J* = 8.1 Hz, 2H), 7.32 (d, *J* = 8.4 Hz, 1H), 6.42 (d, *J* = 5.6 Hz, 1H), 4.42 *(t, J=* 5.1 Hz, 2H), 4.39 - 4.29 (m, 1H), 4.28 - 4.17 (m, 2H), 4.16 - 4.04 (m, 2H), 3.96 (t, *J* = 5.1 Hz, 2H), 3.03 (s, 3H), 1.70 (s, 6H).

**Example 326: Preparation of compound 326**

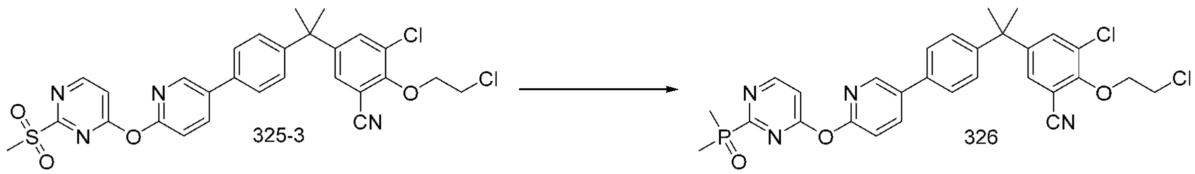

325-3

326

**Preparation of compound 326**

Dimethylphosphine oxide (24 mg, 308.49 μmol) and compound **325-3** (60 mg, 102.83 μmol), $K_2CO_3$ (43 mg, 308.49 μmol), and acetonitrile (2 mL) were added to a reaction flask. The mixture was stirred at 80°C for 1 h. The mixture was filtered, and the crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 35% to 85% acetonitrile gradient elution over 12 min; flow rate: 30 mL/min) to obtain 10 mg of the title compound **326.** LC-MS (ESI): m/z 581.2 $[M+H]^+$. $^1H$ NMR (400 MHz, DMSO-$d_6$) δ 8.92 (dd, *J* = 5.8, 1.0 Hz, 1H), 8.68 (d, *J* = 2.6 Hz, 1H), 8.31 (dd, *J* = 8.4, 2.6 Hz, 1H), 7.76 - 7.70 (m, 3H), 7.66 (d, *J* = 2.3 Hz, 1H), 7.45 - 7.36 (m, 4H), 4.43 (t, *J* = 5.2 Hz, 2H), 3.96 (t, *J* = 5.2 Hz, 2H), 1.71 (s, 6H), 1.66 (d, *J* = 13.8 Hz, 6H). $^{31}P$ NMR (162 MHz, DMSO-$d_6$) δ 34.47 (s, 1P).

**Example 327: Preparation of compound 327**

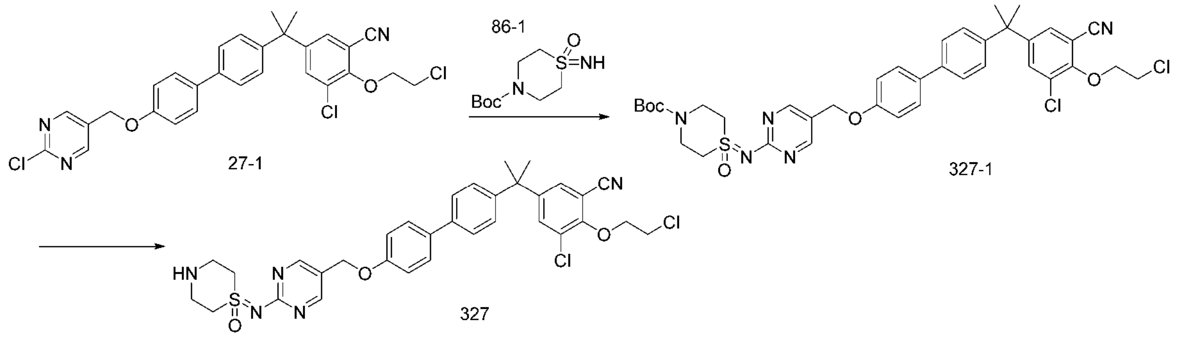

86-1
27-1
327-1
327

**Preparation of compound 327-1**

Compound **27-1** (190 mg, 0.34 mmol) was dissolved in DMF (3 mL), followed by the sequential addition of tris(dibenzylideneacetone)dipalladium (31.5 mg, 0.03 mmol), 4,5-bis(diphenylphosphino)-9,9-dimethylxanthene (39.7 mg, 0.07 mmol), DIEA (133.3 mg, 1.0 mmol), and compound **86-1** (161.1 mg, 0.69 mmol). The reaction system was stirred at 120°C for 3 h. After the reaction was monitored by LCMS until completion, the reaction mixture was poured into ice water and extracted twice with ethyl acetate (20 mL). The organic phases were combined, dried over anhydrous sodium sulfate, subjected to suction filtration, and the filtrate was concentrated to obtain a crude product. The crude product was purified by normal-phase silica gel column chromatography (EA/PE = 0 to 40%) to obtain 150 mg of the title compound **327-1** in 58% yield. LC-MS (ESI): m/z 750.4 $[M+H]^+$.

**Preparation of compound 327**

Compound **327-1** (130 mg, 0.17 mmol) was dissolved in dichloromethane (5 mL), and a solution of hydrogen chloride in 1,4-dioxane (4 mol/L, 5 mL) was added dropwise with stirring. After the addition was completed, the reaction was carried out at room temperature overnight. After the reaction was monitored by LCMS until completion, the reaction mixture was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 56 mg of the title compound **327.** LC-MS (ESI): m/z 650.4 $[M+H]^+$. $^1H$ NMR (400 MHz, DMSO-$d_6$) δ 8.55 (s, 2H), 7.71 (d, *J* = 2.3 Hz, 1H), 7.64 (d, *J* = 2.3 Hz, 1H), 7.60 (d, *J* = 8.7 Hz, 2H), 7.56 (d, *J* = 8.4 Hz, 2H), 7.30 (d, *J* = 8.3 Hz, 2H), 7.10 (d, *J* = 8.8 Hz, 2H), 5.03 (s, 2H), 4.42 (t, *J= 5.2* Hz, 2H), 3.96 (t, *J= 5.2* Hz, 2H), 3.69 - 3.55 (m, 2H), 3.44 - 3.25 (m, 2H), 3.23 - 3.11 (m, 2H), 3.06 - 2.96 (m, 2H), 1.68 (s, 6H).

**Example 328: Preparation of compound 328**

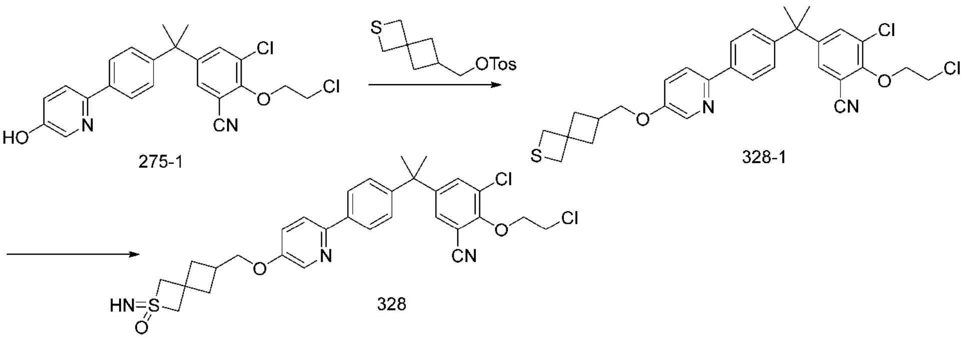

275-1
328-1
328

**Preparation of compound 328-1**

Compound **275-1** (100 mg, 0.23 mmol) was dissolved in DMF (5 mL), and (2-thiaspiro[3.3]heptan-6-yl)methyl 4-methylbenzenesulfonate (70 mg, 0.23 mmol) was added. The reaction system was stirred at 90°C for 4 h. After the reaction was completed, water (25 mL) was added, and the mixture was extracted three times with ethyl acetate (25 mL). The organic phases were combined, washed twice with saturated brine (20 mL), dried over anhydrous sodium sulfate,

subjected to suction filtration, and the filtrate was concentrated to obtain a crude product. The crude product was purified by silica gel column chromatography (PE/EA = 100% to 35%) to obtain 101 mg of the title compound **328-1.** LC-MS (ESI): m/z 553.2 $[M+H]^+$.

**Preparation of compound 328**

Compound **328-1** (100 mg, 0.18 mmol) was dissolved in anhydrous methanol (5 mL), followed by the sequential addition of (diacetoxyiodo)benzene (175 mg, 0.54 mmol), ammonium carbamate (56 mg, 0.72 mmol), and dichloromethane (0.5 mL). The reaction system was stirred at 25°C for 2 h. After the reaction was completed, the reaction mixture was filtered to remove insoluble solids, and then subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 14 mg of the title compound **328.** LC-MS (ESI): m/z 584.0 $[M+H]^+$. $^1H$ NMR (400 MHz, Chloroform-*d*) δ 8.36 (d, *J* = 2.9 Hz, 1H), 7.86 (d, *J* = 8.3 Hz, 2H), 7.65 (d, *J* = 8.7 Hz, 1H), 7.45 (d, *J* = 2.3 Hz, 1H), 7.37 (d, *J* = 2.3 Hz, 1H), 7.31 - 7.21 (m, 3H), 4.42 (t, *J* = 6.2 Hz, 2H), 4.28 - 4.05 (m, 4H), 4.02 (d, *J* = 5.5 Hz, 2H), 3.87 (t, *J* = 6.2 Hz, 2H), 2.90 - 2.73 (m, 1H), 2.67 - 2.47 (m, 2H), 2.44 - 2.29 (m, 2H), 1.69 (s, 6H).

**Example 329: Preparation of compound 329**

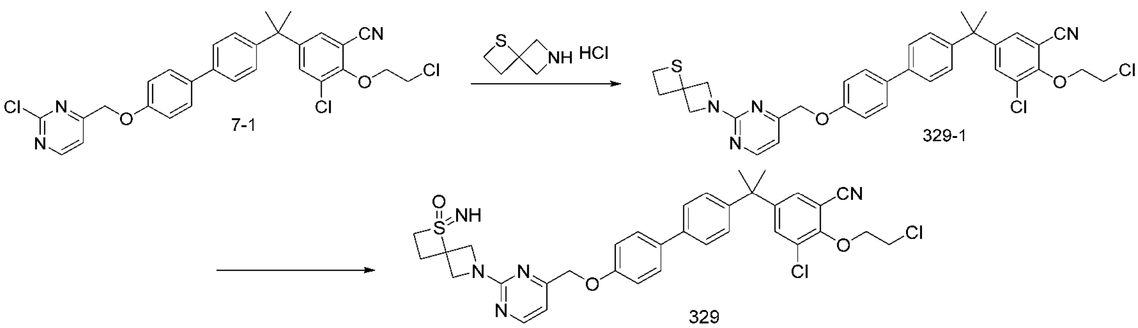

7-1
329-1
329

**Preparation of compound 329-1**

Compound **7-1** (200 mg, 0.36 mmol), 1-thia-6-azaspiro[3.3]heptane hydrochloride (41 mg, 0.36 mmol), and triethylamine (180 mg, 1.80 mmol) were dissolved in ethanol (2 mL). The system was reacted at 100°C for 2 h. The reaction mixture was concentrated. The resulting residue was subjected to silica gel column chromatography (EA/PE = 0 to 80%) to obtain 150 mg of the title compound **329-1** in 65.6% yield. LC-MS (ESI): m/z 631.1 $[M+H]^+$.

**Preparation of compound 329**

Compound **329-1** (150 mg, 0.24 mmol) was dissolved in methanol (5 mL). (Diacetoxyiodo)benzene (310 mg, 0.96 mmol) and ammonium carbamate (47 mg, 0.6 mmol) were added at room temperature, and the mixture was stirred at room temperature for 16 h. The reaction was monitored by LCMS until completion. The reaction mixture was concentrated, and the resulting residue was subjected to preparative purification (Waters 2767/Qda, column: XBridge XBridge C18 19 * 250 mm, 10 μm; mobile phase A: 0.1% FA/$H_2O$, B: acetonitrile; flow rate: 20 mL/min; elution gradient: 73% to 75%; retention time: 8.7-10.0 min of 18 min) to obtain 40.42 mg of the title compound **329.** LC-MS (ESI): m/z 662.3 $[M+H]^+$. $^1H$ NMR (400 MHz, DMSO-$d_6$) δ 8.41 (d, *J* = 5.0 Hz, 1H), 7.70 (d, *J* = 2.3 Hz, 1H), 7.64 (d, *J* = 2.3 Hz, 1H), 7.60 (d, *J* = 8.8 Hz, 2H), 7.56 (d, *J* = 8.4 Hz, 2H), 7.30 (d, *J* = 8.4 Hz, 2H), 7.08 (d, *J* = 8.8 Hz, 2H), 6.84 (d, *J* = 5.0 Hz, 1H), 5.08 (s, 2H), 4.79 (s, 1H), 4.52 (d, *J* = 10.3 Hz, 1H), 4.47 - 4.36 (m, 3H), 4.26 (d, *J* = 10.2 Hz, 1H), 4.20 (d, *J* = 10.2 Hz, 1H), 3.96 (t, *J* = 5.2 Hz, 2H), 3.93 - 3.79 (m, 2H), 2.47 - 2.31 (m, 2H), 1.68 (s, 6H).

**Examples 330 and 331: Preparation of compounds 330 and 331**

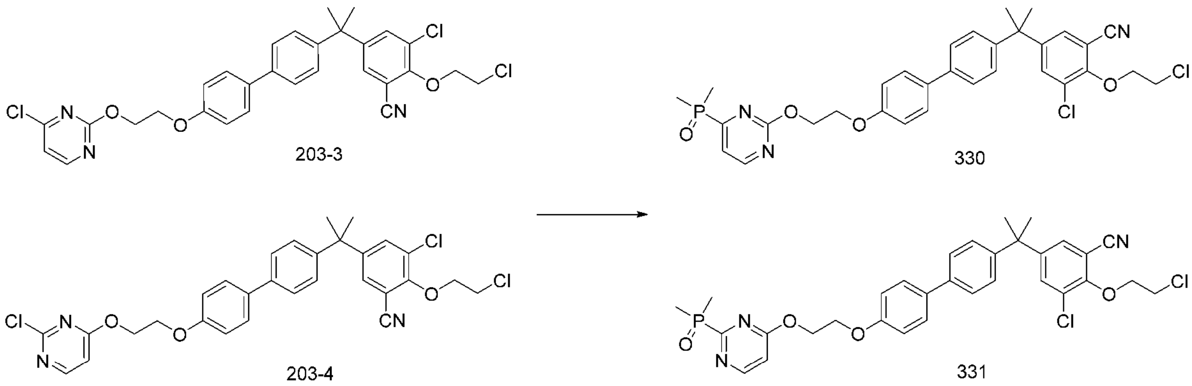

203-3 330 203-4 331

**Preparation of compounds 330 and 331**

A mixture of compounds **203-3** and **203-4** (200 mg, 0.34 mmol), dimethylphosphine oxide (134 mg, 1.72 mmol), $Pd_2(dba)_3$ (32 mg, 0.03 mmol), Xantphos (40 mg, 0.07 mmol), and DIPEA (222 mg, 1.72 mmol) were dissolved in DMF (4 mL). The reaction system was stirred under microwave irradiation at 120°C under an argon atmosphere for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 6 mg of the title compound 330 and 70 mg of the title compound 331.

Compound **330:** LC-MS (ESI): m/z 624.2 $[M+H]^+$. $^1H$ NMR (400 MHz, DMSO-$d_6$) δ 8.85 (t, *J* = 5.0 Hz, 1H), 7.70 (d, *J* = 2.3 Hz, 1H), 7.64 (d, *J* = 2.3 Hz, 1H), 7.62 (d, *J* = 5.0 Hz, 1H), 7.60 (d, *J* = 8.7 Hz, 2H), 7.56 (d, *J* = 8.4 Hz, 2H), 7.30 (d, *J* = 8.5 Hz, 2H), 7.06 (d, *J* = 8.8 Hz, 2H), 4.74 - 4.66 (m, 2H), 4.44 - 4.37 (m, 4H), 3.96 (t, *J* = 5.2 Hz, 2H), 1.69 (d, *J*= 13.6 Hz, 6H), 1.68 (s, 6H). $^{31}P$ NMR (162 MHz, DMSO-$d_6$) δ 34.56 (s, 1P).

Compound **331:** LC-MS (ESI): m/z 624.2 $[M+H]^+$. $^1H$ NMR (400 MHz, DMSO-$d_6$) δ 8.69 (dd, *J* = 5.9, 1.0 Hz, 1H), 7.70 (d, *J* = 2.3 Hz, 1H), 7.64 (d, *J* = 2.3 Hz, 1H), 7.59 (d, *J* = 8.8 Hz, 2H), 7.56 (d, *J* = 8.5 Hz, 2H), 7.30 (d, *J* = 8.5 Hz, 2H), 7.14 (dd, *J* = 5.9, 2.7 Hz, 1H), 7.05 (d, *J* = 8.8 Hz, 2H), 4.81 - 4.73 (m, 2H), 4.45 - 4.38 (m, 4H), 3.96 (t, *J* = 5.2 Hz, 2H), 1.74 (d, *J*= 13.7 Hz, 6H), 1.68 (s, 6H). $^{31}P$ NMR (162 MHz, DMSO-$d_6$) δ 34.24 (s, 1P).

**Example 332: Preparation of compound 332**

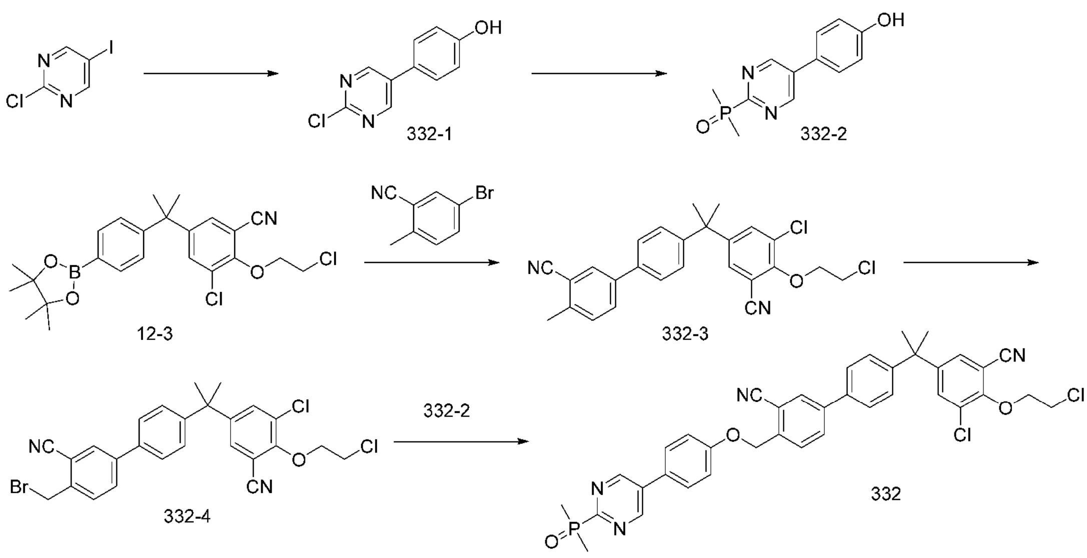


332-1 332-2 12-3 332-3 332-4 332

**Preparation of compound 332-1**

Under a nitrogen atmosphere, 4-hydroxyphenylboronic acid (1 g, 7.25 mmol), 2-chloro-5-iodopyrimidine (1.92 g, 7.98 mmol), [1,1'-bis(diphenylphosphino)ferrocene]dichloropalladium(II) dichloromethane complex (592.1 mg, 725.01 μmol), $K_2CO_3$ (2.51 g, 18.13 mmol), 1,4-dioxane (10 mL), and $H_2O$ (2 mL) were added to a reaction flask. The system was purged three times with nitrogen, and the reaction was carried out at 100°C for 16 h. The mixture was subjected to rotary evaporation until dryness. The crude product was purified by silica gel column chromatography (PE/EA = 100% to 0%) to obtain 800 mg of title compound **332-1.** LC-MS (ESI): m/z 207.0 $[M+H]^+$.

**Preparation of compound 332-2**

Compound **332-1** (300 mg, 1.45 mmol) was dissolved in DMF (3 mL), followed by the sequential addition of dimethylphosphine oxide (340 mg, 4.36 mmol), DIEA (563 mg, 4.36 mmol, 758.68 μL), tris(dibenzylideneacetone) dipalladium (133 mg, 145.19 μmol), and Xantphos (168 mg, 290.38 μmol). The system was purged three times with nitrogen, then heated to 120°C and stirred for 8 h. The mixture was filtered, subjected to rotary evaporation until dryness, and the crude product was purified by silica gel column chromatography (PE/EA = 100% to 0%) to obtain 280 mg of the title compound **332-2.** LC-MS (ESI): m/z 249.0 $[M+H]^+$.

**Preparation of compound 332-3**

Under a nitrogen atmosphere, compound **332-2** (300 mg, 651.89 μmol), 2-methyl-5-bromobenzonitrile (154 mg, 782.27 μmol), [1,1'-bis(diphenylphosphino)ferrocene]dichloropalladium(II) dichloromethane complex (53.2 mg, 65.19 μmol), $K_2CO_3$ (270.3 mg, 1.96 mmol), 1,4-dioxane (5 mL), and $H_2O$ (1 mL) were added to a reaction flask. The system was purged three times with nitrogen, and the reaction was carried out at 100°C for 16 h. The mixture was subjected to rotary evaporation until dryness, and the crude product was purified by silica gel column chromatography (PE/EA = 100% to 30%) to obtain 200 mg of the title compound **332-3.**LC-MS (ESI): m/z 466.2 $[M+18]^+$.

**Preparation of compound 332-4**

Compound **332-3** (150 mg, 333.80 μmol), NBS (63 mg, 350.49 μmol), ATBN (6 mg, 33.38 μmol), and $CCl_4$ (1 mL) were added to a sealed tube and stirred at 80°C for 16 h. Water (5 mL) was added, and the mixture was extracted three times with DCM (5 mL). The organic phases were combined, dried over anhydrous sodium sulfate, filtered, and the filtrate was subjected to rotary evaporation until dryness to obtain 80 mg of a crude product of the title compound **332-4.**

**Preparation of compound 332**

Compound **332-4** (60 mg, 113.58 μmol), compound **332-2** (43 mg, 170.37 μmol), $K_2CO_3$ (48 mg, 340.74 μmol), and acetonitrile (1 mL)were added to a reaction flask, and the mixture was stirred at 80°C for 2 h, filtered, and subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 35% to 85% acetonitrile gradient elution over 12 min; flow rate: 30 mL/min) to obtain 50 mg of the title compound **332.** LC-MS (ESI): m/z 695.2 $[M+H]^+$. $^1H$ NMR (400 MHz, DMSO-$d_6$) δ 9.29 (s, 2H), 8.24 (d, *J* = 2.0 Hz, 1H), 8.06 (dd, *J*= 8.1, 2.0 Hz, 1H), 7.88 (d, *J* = 8.8 Hz, 2H), 7.83 (d, *J*= 8.2 Hz, 1H), 7.76 - 7.70 (m, 3H), 7.66 (d, *J* = 2.4 Hz, 1H), 7.39 (d, *J* = 8.5 Hz, 2H), 7.26 (d, *J* = 8.9 Hz, 2H), 5.38 (s, 2H), 4.42 (t, *J* = 5.2 Hz, 2H), 3.96 (t, *J* = 5.2 Hz, 2H), 1.79 (d, *J* = 13.7 Hz, 6H), 1.70 (s, 6H). $^{31}P$ NMR (162 MHz, DMSO-$d_6$) δ 33.75 (s, 1P).

**Example 333: Preparation of compound 333**

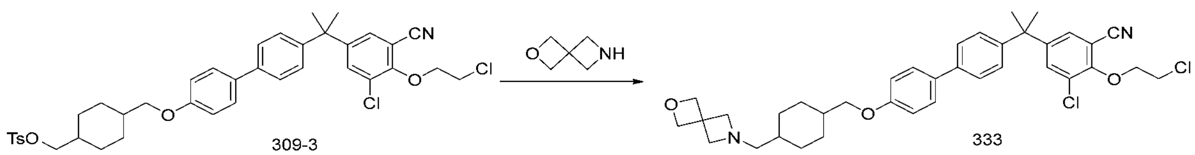

309-3

333

**Preparation of compound 333**

Compound **309-3** (72.4 mg, 102.45 μmol) was suspended in acetonitrile (1.5 mL), and 2-oxa-6-thiaspiro[3.3] heptane (13.2 mg, 133.18 μmol) and potassium carbonate (28.3 mg, 204.89 μmol) were added. The reaction mixture was stirred at 90°C overnight in a sealed tube. After the reaction was completed, the reaction mixture was cooled to room temperature, diluted with a small amount of acetonitrile, and filtered. The filtrate was purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 30-50% 8 min, 50-92% 4 min; flow rate: 30 mL/min) to obtain 14.6 mg of the title compound **333.** LC-MS (ESI): m/z 633.3 $[M+H]^+$. $^1$H NMR (400 MHz, Chloroform-*d*) δ 7.53 - 7.46 (m, 5H), 7.38 (d, *J* = 2.3 Hz, 1H), 7.21 (d, *J* = 8.2 Hz, 2H), 6.97 - 6.91 (m, 2H), 4.79 (s, 4H), 4.42 (t, *J* = 6.2 Hz, 2H), 3.88 (t, *J* = 6.2 Hz, 2H), 3.87 - 3.84 (m, 1H), 3.83 - 3.62 (m, 4H), 2.71 - 2.43 (m, 2H), 2.03 - 1.84 (m, 4H), 1.69 (s, 6H), 1.57 - 1.39 (m, 4H), 1.17 - 0.94 (m, 3H).

**Example 334: Preparation of compound 334**

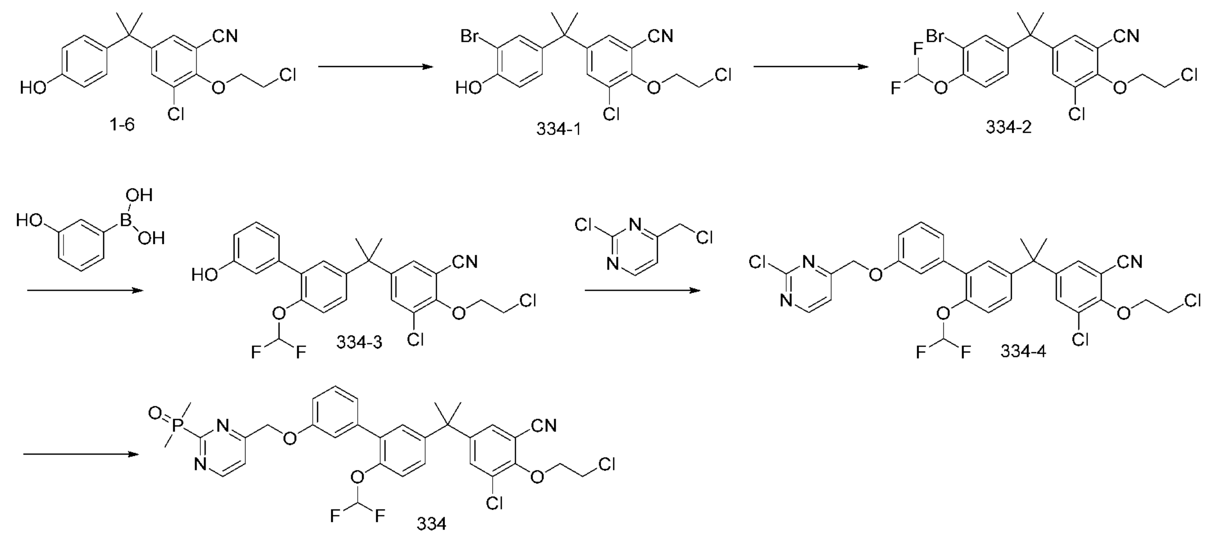

1-6

334-1

334-2

334-3

334-4

334

**Preparation of compound 334-1**

Compound **1-6** (10 g, 28.55 mmol) and *p*-toluenesulfonic acid (0.49 g, 2.86 mmol) were dissolved in DCM (100 mL), and NBS (5 g, 28.55 mmol) was added at room temperature. The mixture was stirred at room temperature for 16 h. Water (100 mL) was added, and the mixture was extracted three times with DCM (100 mL). The organic phase was washed with saturated brine (100 mL), dried over anhydrous sodium sulfate, subjected to suction filtration, and the filtrate was concentrated. The resulting residue was purified by silica gel column chromatography (EA/PE = 0 to 45%) to obtain 5 g of the title compound **334-1** in 40.8% yield. LC-MS (ESI): m/z 425.7 $[M-H]^-$.

**Preparation of compound 334-2**

Compound **334-1** (5 g, 11.65 mmol) was dissolved in acetonitrile (50 mL) and water (50 mL). Potassium carbonate (4.58 g, 81.55 mmol) was added at room temperature, followed by the addition of diethyl phosphate (6.22 g, 23.3 mmol) at 0°C. The mixture was stirred at room temperature for 16 h. The reaction was monitored by LCMS until completion. The reaction mixture was subjected to rotary evaporation until dryness, and ethyl acetate (200 mL) was added. The organic phase was washed with saturated sodium chloride (600 mL), concentrated, and the resulting residue was subjected to silica gel column chromatography (EA/PE = 0 to 50%) to obtain 3 g of the title compound **334-2** in 53.7% yield. $^1$H NMR (400 MHz, $CDCl_3$) δ 7.49 - 7.39 (m, 2H), 7.34 (d, *J* = 2.4 Hz, 1H), 7.21 - 7.04 (m, 2H), 6.54 (t, *J* = 73.4 Hz, 1H), 4.44 (t, *J* = 6.1 Hz, 2H), 3.88 (t, *J* = 6.1 Hz, 2H), 1.66 (s, 6H).

**Preparation of compound 334-3**

Compound **334-2** (1 g, 2.09 mmol) and 3-hydroxyphenylboronic acid (350 mg, 2.51 mmol) were dissolved in 1,4-dioxane (10 mL) and water (1 mL). Pd(dppf)$Cl_2$ (150 mg, 0.21 mmol) and potassium carbonate (870 mg, 6.27 mmol) were added at room temperature. The system was purged with argon and stirred at 100°C for 16 h. The reaction was monitored by LCMS until completion. The reaction mixture was concentrated. The resulting residue was subjected to silica gel column chromatography (EA/PE = 0 to 45%) to obtain 600 mg of the title compound **334-3** in 58.2% yield. $^1$H NMR (400 MHz, $CDCl_3$) δ 7.47 (d, *J* = 2.3 Hz, 1H), 7.36 (d, *J* = 2.3 Hz, 1H), 7.31 - 7.25 (m, 1H), 7.21 - 7.18 (m, 1H), 7.17 - 7.12 (m, 2H), 7.02 - 6.97 (m, 1H), 6.95 - 6.93 (m, 1H), 6.87 - 6.83 (m, 1H), 6.43 (d, *J* = 74.2 Hz, 1H), 4.44 (t, *J* = 6.1 Hz, 2H), 3.87 (t, *J* = 6.1 Hz, 2H), 1.68 (s, 6H).

**Preparation of compound 334-4**

Compound **334-3** (200 mg, 0.41 mmol) was dissolved in acetonitrile (2 mL). 2-Chloro-4-(chloromethyl)pyrimidine (80 mg, 0.49 mmol) and cesium carbonate (400 mg, 1.23 mmol) were added at room temperature. The mixture was stirred at 70°C for 2 h. The reaction was monitored by TLC until completion. The reaction mixture was subjected to rotary evaporation until dryness, and ethyl acetate (20 mL) was added. The organic phase was washed with saturated sodium chloride (60 mL), concentrated, and the resulting residue was subjected to silica gel column chromatography (EA/PE = 0 to 50%) to obtain 100 mg of a crude product of the title compound **334-4** in 39.8% yield. The product was used directly in the next step without purification.

**Preparation of compound 334**

Compound **334-4** (100 mg, 0.16 mmol) was dissolved in *N,N*-dimethylformamide (2 mL). Dimethylphosphine oxide (50 mg, 0.64 mmol), palladium acetate (4 mg, 0.016 mmol), 1,3-bis(diphenylphosphino)propane (7 mg, 0.016 mmol), and *N,N*-diisopropylethylamine (100 mg, 0.80 mmol) were added at room temperature. The system was purged with argon for 30 seconds. The reaction was then stirred under microwave irradiation at 100°C for 0.5 h. The reaction was monitored by LCMS until completion. The reaction mixture was purified by preparative liquid chromatography (Waters 2767/Qda: column: XBridge Xbridge C18 19 * 250 mm, 10 μm; mobile phase A: 0.1% FA/$H_2O$, B: acetonitrile; flow rate: 20 mL/min; gradient: 67% to 67%; retention time: 8.4-9.6 min of 18 min) to obtain 13.53 mg of the title compound **334.** LC-MS (ESI): m/z 660.3 $[M+H]^+$. $^1$H NMR (400 MHz, DMSO-$d_6$) δ 9.00 (d, *J* = 5.2 Hz, 1H), 7.76 - 7.72 (m, 2H), 7.69 (d, *J* = 2.3 Hz, 1H), 7.39 (t, *J* = 8.0 Hz, 1H), 7.34 (d, *J* = 2.4 Hz, 1H), 7.29 - 7.15 (m, 3H), 7.12 (t, *J* = 74 Hz, 1H), 7.11 - 7.06 (m, 2H), 5.34 (s, 2H), 4.42 (t, *J* = 5.2 Hz, 2H), 3.95 (t, *J* = 5.2 Hz, 2H), 1.75 (d, *J* = 13.7 Hz, 6H), 1.69 (s, 6H). $^{19}$F NMR (376 MHz, DMSO-d6) δ -81.07 (s). $^{31}$P NMR (162 MHz, DMSO-$d_6$) δ 33.92 (s, 1P).

**Example 335: Preparation of compound 335**

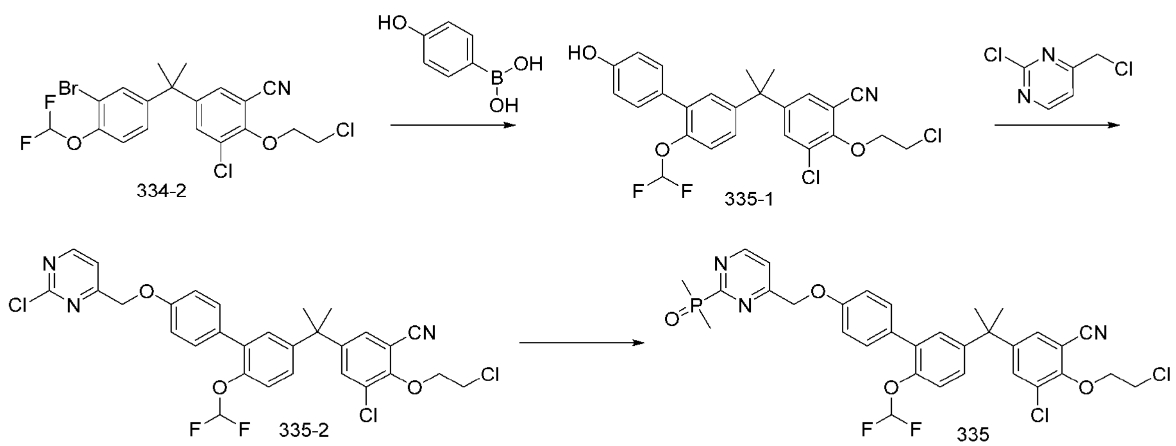

334-2

335-1

335-2

335

**Preparation of compound 335-1**

Compound **334-2** (1 g, 2.09 mmol) and 4-hydroxyphenylboronic acid (350 mg, 2.51 mmol) were dissolved in 1,4-dioxane (10 mL) and water (1 mL). Pd(dppf)$Cl_2$ (150 mg, 0.21 mmol) and potassium carbonate (870 mg, 6.27 mmol) were added at room temperature. The system was purged with argon and then stirred at 110°C for 16 h. The reaction was monitored by LCMS until completion. The reaction was quenched with ice water (10 mL) and acidified with dilute hydrochloric acid (1 mol/L). The mixture was extracted three times with ethyl acetate (20 mL). The organic phases were

combined, dried over anhydrous sodium sulfate, subjected to suction filtration, and the filtrate was concentrated. The resulting residue was purified by silica gel column chromatography (PE/EA = 100% to 60%) to obtain 500 mg of the title compound **335-1.** LC-MS (ESI): m/z 490.0 $[M-H]^-$.

**Preparation of compound 335-2**

Compound **335-2** (200 mg, 0.41 mmol) was dissolved in acetonitrile (3 mL). 2-Chloro-4-(chloromethyl)pyrimidine (74 mg, 0.45 mmol) and cesium carbonate (200 mg, 0.61 mmol) were added at room temperature. The mixture was stirred at 70°C for 1 h. The reaction was monitored by LCMS until completion. Ice water (5 mL) was added to quench the reaction, and the mixture was extracted three times with ethyl acetate (10 mL). The organic phase was washed once with saturated sodium chloride (20 mL), dried over anhydrous sodium sulfate, subjected to suction filtration, and the filtrate was concentrated. The resulting residue was purified by silica gel column chromatography (PE/EA = 100% to 75%) to obtain 160 mg of the title compound **335-2.** LC-MS (ESI): m/z 618.5 $[M+H]^+$.

**Preparation of compound 335**

Compound **335-2** (160 mg, 0.26 mmol) was dissolved in *N,N*-dimethylformamide (2 mL). Dimethylphosphine oxide (82 mg, 1.04 mmol), palladium acetate (16.1 mg, 0.039 mmol), 1,3-bis(diphenylphosphino)propane (11.7 mg, 0.052 mmol), and *N,N*-diisopropylethylamine (168 mg, 1.30 mmol) were added at room temperature. After purging with argon for 30 seconds, the mixture was stirred under microwave irradiation at 120°C for 0.5 h. The reaction was monitored by LCMS until completion. The reaction mixture was subjected to preparative purification (preparative method: mobile phase: A: 0.1% formic acid in water; B: acetonitrile; chromatographic column: Sunfire C18, 19 * 250 mm, 10 μm; column temperature: 25°C; gradient: 67% to 67% acetonitrile gradient elution over 16 min; flow rate: 20 mL/min) to obtain 63.52 mg of the title compound **335.** LC-MS (ESI): m/z 660.4 $[M+H]^+$. $^1H$ NMR (400 MHz, DMSO-$d_6$) δ 9.01 (d, $J$ = 5.2 Hz, 1H), 7.74 (dd, $J$ = 5.1, 3.2 Hz, 1H), 7.72 (d, $J$ = 2.3 Hz, 1H), 7.69 (d, $J$ = 2.3 Hz, 1H), 7.44 (d, $J$ = 8.8 Hz, 2H), 7.30 (d, $J$ = 2.4 Hz, 1H), 7.26 - 7.16 (m, 2H), 7.14 (d, $J$ = 8.8 Hz, 2H), 7.12 (t, $J$ = 74.1 Hz, 1H), 5.35 (s, 2H), 4.42 (t, $J$ = 5.2 Hz, 2H), 3.95 (t, $J$ = 5.2 Hz, 2H), 1.77 (d, $J$ = 13.7 Hz, 6H), 1.69 (s, 6H). $^{19}F$ NMR (376 MHz, DMSO-$d_6$) δ -76.19 (s). $^{31}P$ NMR (162 MHz, DMSO-$d_6$) δ 34.00 (s, 1P).

**Example 336: Preparation of compound 336**

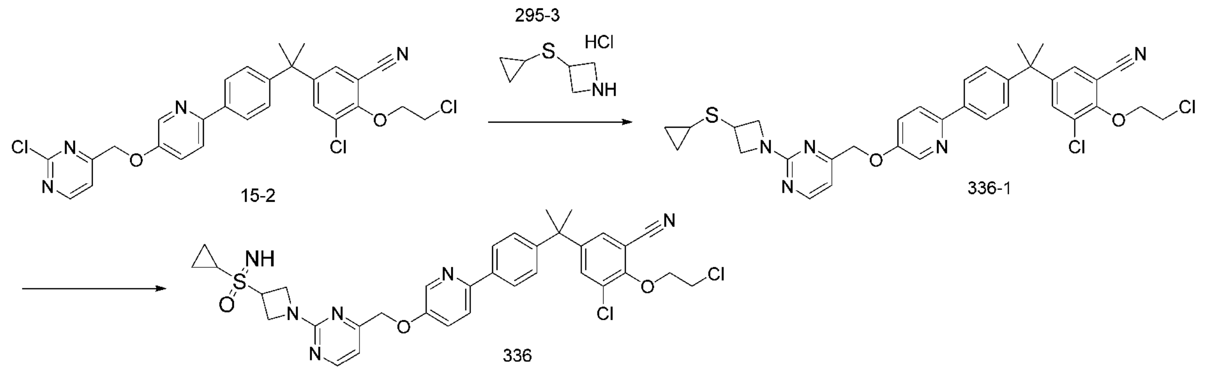


15-2

336-1

336

**Preparation of compound 336-1**

Compound **15-2** (80 mg, 0.14 mmol), compound **295-3** (20 mg, 0.15 mmol), and triethylamine (71 mg, 0.7 mmol) were dissolved in ethanol (3 mL). The reaction was carried out at 100°C for 2 h. The mixture was cooled to room temperature and concentrated. The resulting residue was subjected to silica gel column chromatography (EA/PE = 0 to 50%) to obtain 130 mg of the title compound **336-1.** LC-MS (ESI): m/z 645.5 $[M+H]^+$.

**Preparation of compound 336**

Compound **336-1** (130 mg, 0.19 mmol) was dissolved in methanol (2 mL). (Diacetoxyiodo)benzene (64 mg, 0.2 mmol) and ammonium carbamate (16 mg, 0.2 mmol) were added at room temperature, and the mixture was stirred at room temperature for 16 h. The reaction was monitored by LCMS until completion. The reaction mixture was concentrated, and

the resulting residue was purified by preparative liquid chromatography (Waters 2767/Qda: column: XBridge XBridge C18 19 × 250 mm, 10 μm; mobile phase: A: 0.1% FA/$H_2O$, B: acetonitrile; flow rate: 20 mL/min; elution gradient: 80% to 85%; retention time: 7.2-8.5 min of 17 min) to obtain 40.42 mg of the title compound **336.** LC-MS (ESI): m/z 677.3 $[M+H]^+$. $^1H$ NMR (400 MHz, DMSO-$d_6$) δ 8.45 (d, *J* = 3.0 Hz, 1H), 8.42 (d, *J* = 5.0 Hz, 1H), 7.95 (d, *J* = 8.5 Hz, 2H), 7.89 (d, *J* = 8.8 Hz, 1H), 7.69 (d, *J* = 2.3 Hz, 1H), 7.62 (d, *J* = 2.3 Hz, 1H), 7.53 (dd, *J* = 8.8, 3.0 Hz, 1H), 7.33 (d, *J* = 8.5 Hz, 2H), 6.87 (d, *J* = 5.0 Hz, 1H), 5.17 (s, 2H), 4.42 (t, *J* = 5.2 Hz, 2H), 4.33 - 4.22 (m, 5H), 3.96 (t, *J* = 5.2 Hz, 2H), 3.89 (s, 1H), 2.67 - 2.57 (m, 1H), 1.69 (s, 6H), 1.09 - 1.01 (m, 1H), 0.94 - 0.80 (m, 3H).

**Example 337: Preparation of compound 337**

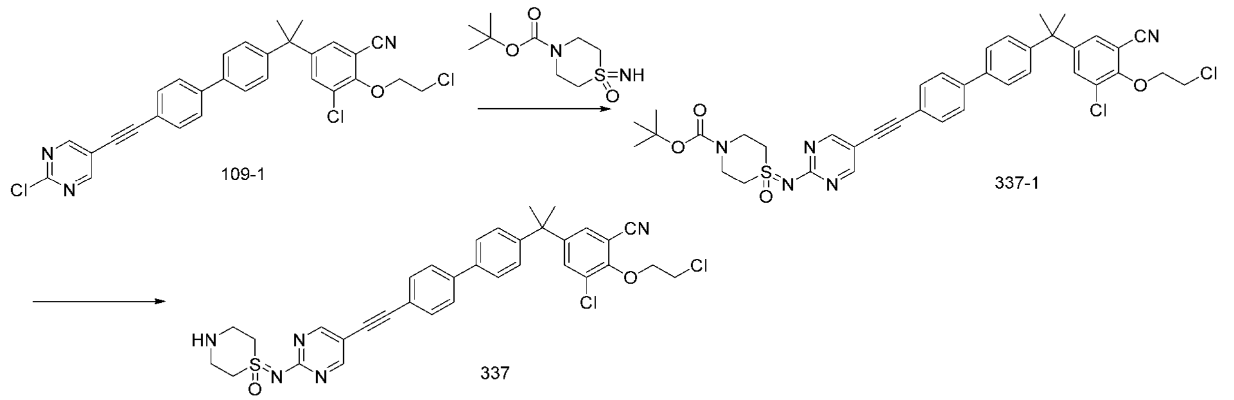

109-1

337-1

337

**Preparation of compound 337-1**

Compound **109-1** (150 mg, 0.274 mmol), *tert*-butyl 1-iminothiomorpholine-4-carboxylate 1-oxide (64 mg, 0.274 mmol), $Pd_2(dba)_3$ (25 mg, 0.027 mmol), Xantphos (16 mg, 0.027 mmol), and DIPEA (106 mg, 0.823 mmol) were dissolved in 1,4-dioxane (5 mL). The reaction system was purged three times with nitrogen and stirred at 100°C for 0.5 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (DCM/MeOH = 100% to 5%) to obtain 150 mg of the title compound **337-1.** LC-MS (ESI): m/z 744.2 $[M+H]^+$.

**Preparation of compound 337**

Compound **337-1** (140 mg, 0.188 mmol) was dissolved in dichloromethane (5 mL). The reaction system was cooled to 0°C, and a solution of hydrogen chloride in 1,4-dioxane (1 mL, 4 mol/L) was added dropwise. After the addition was completed, the reaction system was stirred at 0°C for 0.5 h. After the reaction was completed, the reaction mixture was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 80 mg of the title compound **337.** LC-MS (ESI): m/z 644.2 $[M+H]^+$. $^1H$ NMR (400 MHz, DMSO-$d_6$) δ 8.65 (s, 2H), 7.74 (d, *J* = 8.5 Hz, 2H), 7.71 (d, *J* = 2.3 Hz, 1H), 7.67 (d, *J* = 8.5 Hz, 2H), 7.65 (d, *J* = 2.3 Hz, 1H), 7.61 (d, *J* = 8.4 Hz, 2H), 7.36 (d, *J* = 8.5 Hz, 2H), 4.42 (t, *J* = 5.2 Hz, 2H), 3.96 (t, *J* = 5.2 Hz, 2H), 3.71 - 3.61 (m, 2H), 3.43 - 3.37 (m, 2H), 3.25 - 3.17 (m, 2H), 3.09 - 3.00 (m, 2H), 1.70 (s, 6H).

**Example 338: Preparation of compound 338**

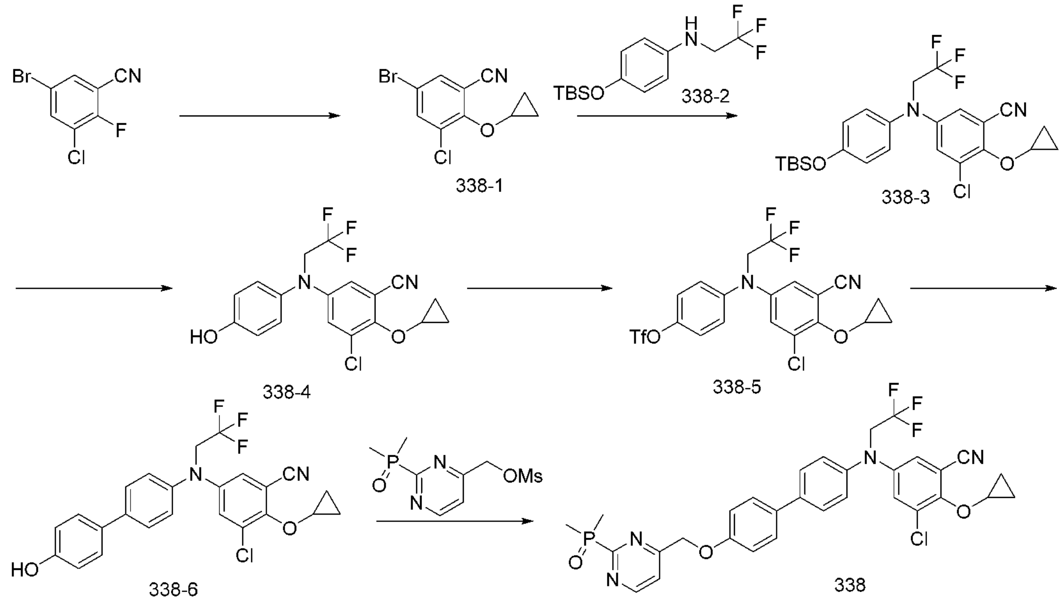


### Preparation of compound 338-1

Under a nitrogen atmosphere, cyclopropanol (298 mg, 5.12 mmol), ,2-fluoro-3-chloro-5-bromobenzonitrile (1 g, 4.27 mmol), $Cs_2CO_3$ (2.78 g, 8.53 mmol), and acetonitrile (10 mL) were added to a reaction flask. The reaction system was heated to 90°C and stirred for 2 h, filtered, and subjected to rotary evaporation until dryness. The crude product was purified by silica gel column chromatography (PE/EA = 100% to 20%) to obtain 800 mg of the title compound **338-1.** $^1H$ NMR (400 MHz, Chloroform-*d*) δ 7.73 (d, *J* = 2.4 Hz, 1H), 7.61 (d, *J* = 2.4 Hz, 1H), 4.55 - 4.46 (m, 1H), 1.05 - 0.92 (m, 2H), 0.83 - 0.70 (m, 2H).

### Preparation of compound 338-3

Compound **338-2** (0.5 g, 1.64 mmol), compound **338-1** (535 mg, 1.96 mmol), tris(dibenzylideneacetone) palladium (150 mg, 163.71 μmol), sodium *tert*-butoxide (189 mg, 1.96 mmol), and toluene (5 mL) were added to a reaction flask. The reaction system was purged three times with nitrogen and reacted at 70°C for 12 h. The mixture was filtered and subjected to rotary evaporation until dryness. The resulting crude product was purified by silica gel column chromatography (PE/EA = 100% to 20%) to obtain 470 mg of the title compound **338-3.** LC-MS (ESI): m/z 497.2 $[M+H]^+$.

### Preparation of compound 338-4

Compound **338-3** (470 mg, 945.63 μmol) was dissolved in THF (2 mL), followed by the addition of TBAF (1.89 mmol, 2 mL, 1 mol/L in THF), and the mixture was stirred at room temperature for 1 h. The mixture was subjected to rotary evaporation until dryness, and the crude product was purified by silica gel column chromatography (PE/EA = 100% to 20%) to obtain 120 mg of the title compound **338-4.** LC-MS (ESI): m/z 383.0 $[M+H]^+$.

### Preparation of compound 338-5

Compound **338-4** (120 mg, 313.51 μmol), *N*-phenylbis(trifluoromethanesulfonyl)imide (135 mg, 376.21 μmol), and TEA (64 mg, 627.02 μmol, 87.45 μL) were dissolved in DCM (2 mL), and the mixture was stirred at room temperature for 1 h. The mixture was subjected to rotary evaporation until dryness, and the crude product was purified by silica gel column chromatography (PE/EA = 100% to 20%) to obtain 100 mg of the title compound **338-5.** LC-MS (ESI): m/z 515.0 $[M+H]^+$.

### Preparation of compound 338-6

Under a nitrogen atmosphere, compound **338-5** (50 mg, 97.12 μmol), 4-hydroxyphenylboronic acid pinacol ester (26 mg, 116.54 μmol), [1,1'-bis(diphenylphosphino)ferrocene]dichloropalladium(II) dichloromethane complex (7 mg, 9.71 μmol), $K_2CO_3$ (40 mg, 291.36 μmol), 1,4-dioxane (1 mL) and $H_2O$ (0.2 mL) were added to a reaction flask. The system was

purged three times with nitrogen, and the reaction was carried out at 100°C for 16 h. The mixture was subjected to rotary evaporation until dryness, and the crude product was purified by silica gel column chromatography (PE/EA = 100% to 20%) to obtain 25 mg of the title compound **338-6.** LC-MS (ESI): m/z 459.0 $[M+H]^+$.

**Preparation of compound 338**

Compound **338-6** (25 mg, 54.48 μmol), (2-(dimethylphosphoryl)pyrimidin-4-yl)methyl methanesulfonate (19 mg, 70.83 μmol), potassium carbonate (23 mg, 163.45 μmol) and acetonitrile (1 mL) were added to a reaction flask, and the mixture was stirred at 75°C for 1 h. The mixture was filtered and subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 85% acetonitrile gradient elution over 25 min; flow rate: 30 mL/min) to obtain 20 mg of the title compound **338.** LC-MS (ESI): m/z 627.2 $[M+H]^+$. $^1H$ NMR (400 MHz, DMSO-$d_6$) δ 9.01 (d, *J= 5.2* Hz, 1H), 7.74 (dd, *J* = 5.2, 3.2 Hz, 1H), 7.72 - 7.62 (m, 4H), 7.34 (d, *J* = 3.0 Hz, 1H), 7.31 (d, *J* = 3.0 Hz, 1H), 7.26 (d, *J* = 8.6 Hz, 2H), 7.16 (d, *J* = 8.8 Hz, 2H), 5.36 (s, 2H), 4.73 (q, *J* = 9.2 Hz, 2H), 4.37 - 4.28 (m, 1H), 1.77 (d, *J* = 13.7 Hz, 6H), 0.91 - 0.83 (m, 2H), 0.72 - 0.63 (m, 2H). $^{31}P$ NMR (162 MHz, DMSO-$d_6$) δ 34.09 (s, 1P).

**Example 339: Preparation of compound 339**

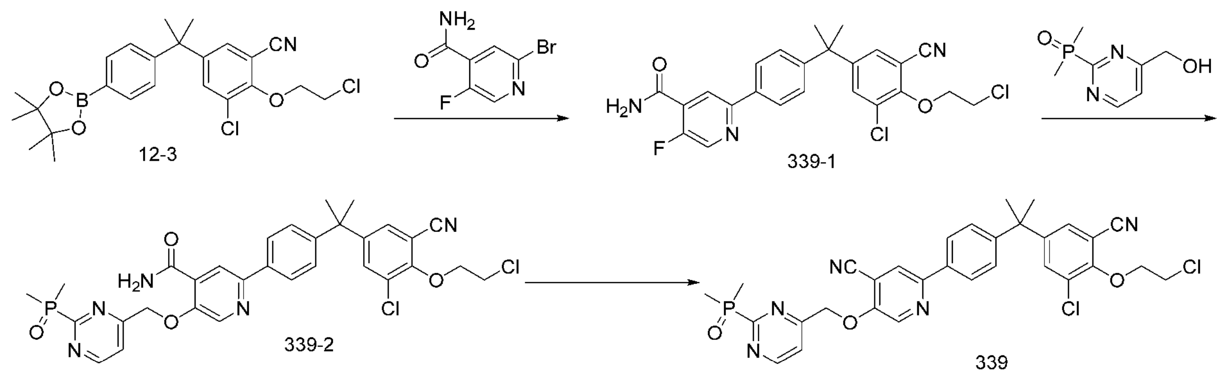

12-3

339-1

339-2

339

**Preparation of compound 339-1**

Under a nitrogen atmosphere, compound **12-3** (500 mg, 1.09 mmol), 2-bromo-5-fluoroisonicotinamide (286 mg, 1.30 mmol), [1,1'-bis(diphenylphosphino)ferrocene]dichloropalladium(II) dichloromethane complex (80 mg, 108.65 μmol), $K_2CO_3$ (451 mg, 3.26 mmol), 1,4-dioxane (5 mL), and $H_2O$ (1 mL) were added to a reaction flask. The system was purged three times with nitrogen, and the reaction was carried out at 100°C for 16 h. The mixture was subjected to rotary evaporation until dryness, and the crude product was purified by silica gel column chromatography (PE/EA = 100% to 20%) to obtain 300 mg of the title compound **339-1.** LC-MS (ESI): m/z 472.0 $[M+H]^+$.

**Preparation of compound 339-2**

Compound **339-1** (178 mg, 952.71 μmol), 2-(dimethylphosphoryl)pyrimidine-4-methanol (300 mg, 635.14 μmol), $Cs_2CO_3$ (310.41 mg, 952.71 μmol), and DMF (3 mL) were added to a reaction flask. The mixture was heated to 80°C and stirred for 1 h. The mixture was filtered, subjected to rotary evaporation until dryness, and the crude product was purified by silica gel column chromatography (DCM/MeOH = 100% to 10%) to obtain 80 mg of the title compound **339-2.** LC-MS (ESI): m/z 638.0 $[M+H]^+$.

**Preparation of compound 339**

Compound **339-2** (50 mg, 78.31 μmol) was dissolved in THF (2 mL), followed by the addition of pyridine (0.5 mL). The mixture was cooled to 0°C, and trifluoromethanesulfonic anhydride (0.5 mL) was slowly added dropwise. The mixture was stirred at 0°C for 1 h. Water (10 mL) was added, and the system was extracted three times with DCM (5 mL). The organic phases were combined, subjected to rotary evaporation until dryness, and the crude product was subjected to preparative purification (preparative method: mobile phase: A: 0.1% formic acid in water; B: acetonitrile; chromatographic

column: Welch Ultimate AQ-C18 250 × 21.2 mm; column temperature: 25°C; gradient: 25% to 95% acetonitrile gradient elution over 20 min; flow rate: 30 mL/min) to obtain 20 mg of the title compound **339.** LC-MS (ESI): m/z 620.2 [M+H]+. $^1$H NMR (400 MHz, DMSO-$d_6$) δ 9.08 (d, *J* = 5.2 Hz, 1H), 8.82 (s, 1H), 8.46 (s, 1H), 8.02 (d, *J* = 8.2 Hz, 2H), 7.77 (dd, *J* = 5.3, 3.1 Hz, 1H), 7.70 (d, *J* = 2.4 Hz, 1H), 7.63 (d, *J* = 2.3 Hz, 1H), 7.37 (d, *J* = 8.1 Hz, 2H), 5.71 (s, 2H), 4.42 (t, *J* = 5.1 Hz, 2H), 3.96 (t, *J* = 5.1 Hz, 2H), 1.73 (d, *J* = 13.8 Hz, 6H), 1.69 (s, 6H). $^{31}$P NMR (162 MHz, DMSO-$d_6$) δ 34.22 (s, 1P).

**Example 340: Preparation of compound 340**

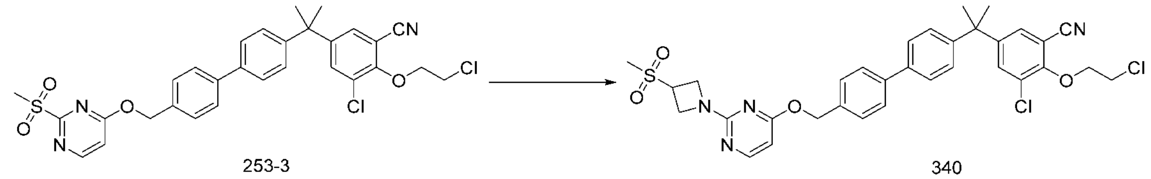

253-3

340

**Preparation of compound 340**

Compound **253-3** (90 mg, 0.151 mmol), 3-(methylsulfonyl)azetidine hydrochloride (52 mg, 0.302 mmol), and DIEA (98 mg, 0.755 mmol) were placed in a sealed tube, and acetonitrile (2 mL) was added. The reaction system was stirred at 90°C for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 50 mg of the title compound **340.** LC-MS (ESI): m/z 651.2 [M+H]+. $^1$H NMR (400 MHz, Chloroform-*d*) δ 8.08 (d, *J* = 5.8 Hz, 1H), 7.59 (d, *J* = 8.2 Hz, 2H), 7.53 (d, *J* = 8.4 Hz, 2H), 7.51 - 7.45 (m, 3H), 7.38 (d, *J* = 2.3 Hz, 1H), 7.25 (d, *J* = 8.7 Hz, 2H), 6.19 (d, *J* = 5.8 Hz, 1H), 5.39 (s, 2H), 4.53 - 4.45 (m, 4H), 4.43 (t, *J*= 6.2 Hz, 2H), 4.13 - 4.04 (m, 1H), 3.88 (t, *J* = 6.1 Hz, 2H), 2.95 (s, 3H), 1.70 (s, 6H).

**Example 341: Preparation of compound 341**

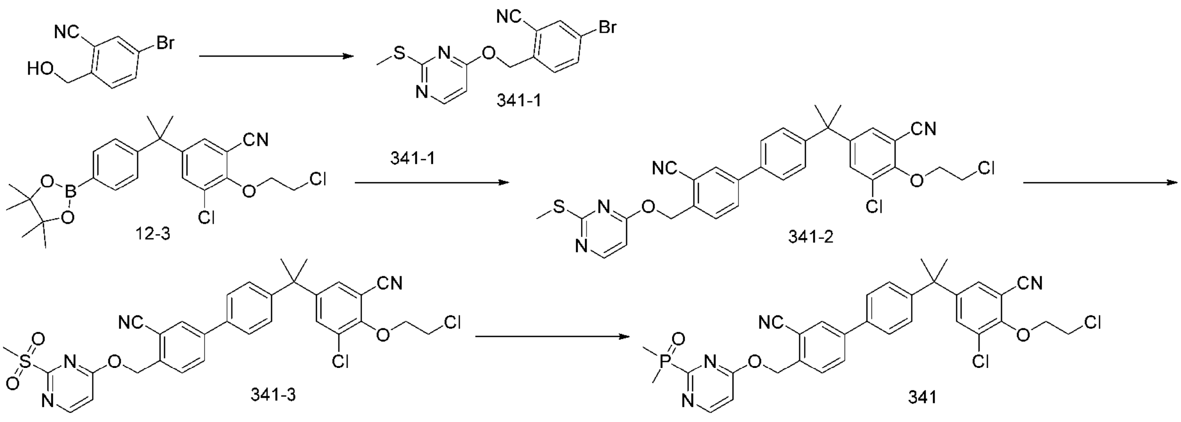


**Preparation of compound 341-1**

2-(Hydroxymethyl)-5-bromobenzonitrile (240 mg, 1.13 mmol) and 2-(methylthio)-4-chloropyrimidine (182 mg, 1.13 mmol) were dissolved in acetonitrile (3 mL), and cesium carbonate (1.11 g, 3.4 mmol) was added. After the addition was completed, the reaction system was stirred at 50°C for 1 h. After the reaction was completed, a 5% aqueous sodium chloride solution (50 mL) was added, and the mixture was extracted three times with ethyl acetate (30 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 85%) to obtain 350 mg of the title compound **341-1.** LC-MS (ESI): m/z 336.0 [M+H]+.

**Preparation of compound 341-2**

Compound **341-1** (0.3 g, 0.892 mmol), compound **12-3** (431 mg, 0.937 mmol), and potassium carbonate (370 mg, 2.68 mmol) were dissolved in 1,4-dioxane (10 mL) and water (3 mL), and Pd(dppf)$Cl_2$ (65 mg, 0.089 mmol) was added thereto. After the addition was completed, the reaction system was stirred at 91°C for 2 h. After the reaction was completed, water (50 mL) was added, and the mixture was extracted three times with ethyl acetate (20 mL). The organic phases were combined, washed twice with saturated brine (30 mL), dried over anhydrous sodium sulfate, filtered, and the filtrate was concentrated to obtain a crude product. The crude product was purified by silica gel column chromatography (PE/EA = 100% to 75%) to obtain 380 mg of the title compound **341-2.** LC-MS (ESI): m/z 589.2 $[M+H]^+$.

**Preparation of compound 341-3**

Compound **341-2** (0.3 g, 0.508 mmol) was dissolved in dichloromethane (10 mL), and m-chloroperoxybenzoic acid (0.263 g, 1.53 mmol) was added. After the addition was completed, the reaction system was stirred at room temperature for 1 h. After the reaction was completed, the reaction mixture was filtered, and the filtrate was concentrated. The resulting crude product was purified by silica gel column chromatography (PE/EA = 100% to 85%) to obtain 0.24 g of the title compound **341-3.** LC-MS (ESI): m/z 621.2$[M+H]^+$.

**Preparation of compound 341**

Compound **341-3** (80 mg, 0.129 mmol), dimethylphosphine oxide (30 mg, 0.386 mmol), and cesium carbonate (126 mg, 0.386 mmol) were placed in a sealed tube, and acetonitrile (3 mL) was added. The reaction system was stirred at 70°C for 2 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **341.** LC-MS (ESI): m/z 619.2 $[M+H]^+$. $^1$H NMR (400 MHz, Chloroform-*d*) δ 8.62 (s, 1H), 7.92 (d, *J* = 1.7 Hz, 1H), 7.82 (d, *J* = 7.0 Hz, 1H), 7.72 (d, *J* = 8.0 Hz, 1H), 7.51 (d, *J* = 8.4 Hz, 2H), 7.48 (d, *J* = 2.4 Hz, 1H), 7.35 (d, *J* = 2.4 Hz, 1H), 7.29 (d, *J* = 8.3 Hz, 2H), 6.93 (s, 1H), 5.72 (s, 2H), 4.43 (t, *J* = 6.1 Hz, 2H), 3.88 (t, *J* = 6.2 Hz, 2H), 1.89 (d, *J* = 13.5 Hz, 6H), 1.70 (s, 6H). $^{31}$P NMR (162 MHz, Chloroform-*d*) δ 36.01 (s, 1P).

**Example 342: Preparation of compound 342**

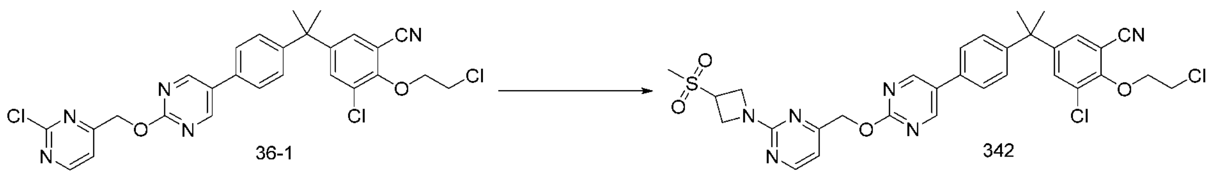

**Preparation of compound 342**

Compound **36-1** (0.14 g, 252.32 μmol), 3-(methylsulfonyl)azetidine hydrochloride (65 mg, 378.48 μmol), DIPEA (163 mg, 1.26 mmol), and acetonitrile (2 mL)were added to a reaction flask, and the mixture was stirred under microwave irradiation at 90°C for 2 h. The mixture was subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 85% acetonitrile gradient elution over 25 min; flow rate: 30 mL/min) to obtain 50 mg of the title compound **342.** LC-MS (ESI): m/z 653.2 $[M+H]^+$. $^1$H NMR (400 MHz, DMSO-$d_6$) δ 8.94 (s, 2H), 8.37 (d, *J*= 5.0 Hz, 1H), 7.71 (d, *J* = 2.3 Hz, 1H), 7.67 (d, *J* = 8.5 Hz, 2H), 7.65 (d, *J* = 2.4 Hz, 1H), 7.37 (d, *J* = 8.5 Hz, 2H), 6.73 (d, *J* = 5.1 Hz, 1H), 5.38 (s, 2H), 4.45 - 4.36 (m, 3H), 4.31 (t, *J* = 8.8 Hz, 2H), 4.20 (dd, *J* = 9.7, 5.0 Hz, 2H), 3.96 (t, *J* = 5.2 Hz, 2H), 3.05 (s, 3H), 1.69 (s, 6H).

**Example 343: Preparation of compound 343**

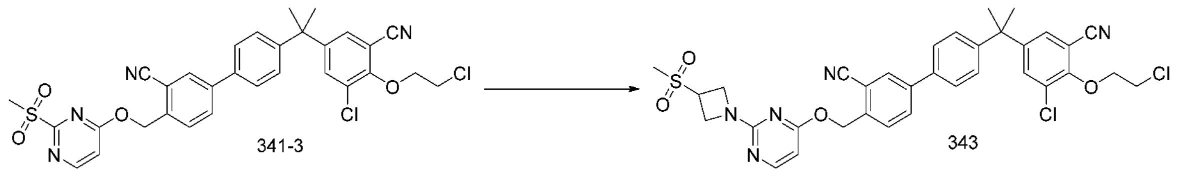
341-3
343

**Preparation of compound 343**

Compound **341-3** (80 mg, 0.129 mmol), 3-(methylsulfonyl)azetidine hydrochloride (22 mg, 0.129 mmol), and DIEA (32 mg, 0.258 mmol) were placed in a sealed tube, and acetonitrile (3 mL) was added. The reaction system was stirred at 70°C for 2 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **343.** LC-MS (ESI): m/z 676.2 $[M+H]^+$. $^1H$ NMR (400 MHz, Chloroform-*d*) δ 8.11 (d, *J* = 5.7 Hz, 1H), 7.88 (d, *J* = 1.9 Hz, 1H), 7.80 (dd, *J* = 8.1, 2.0 Hz, 1H), 7.65 (d, *J* = 8.2 Hz, 1H), 7.51 (d, *J* = 8.4 Hz, 2H), 7.48 (d, *J* = 2.3 Hz, 1H), 7.35 (d, *J* = 2.3 Hz, 1H), 7.28 (d, *J* = 8.4 Hz, 2H), 6.22 (d, *J* = 5.6 Hz, 1H), 5.59 (s, 2H), 4.56 - 4.36 (m, 6H), 4.13 - 4.02 (m, 1H), 3.88 (t, *J* = 6.1 Hz, 2H), 2.94 (s, 3H), 1.70 (s, 6H).

**Example 344: Preparation of compound 344**

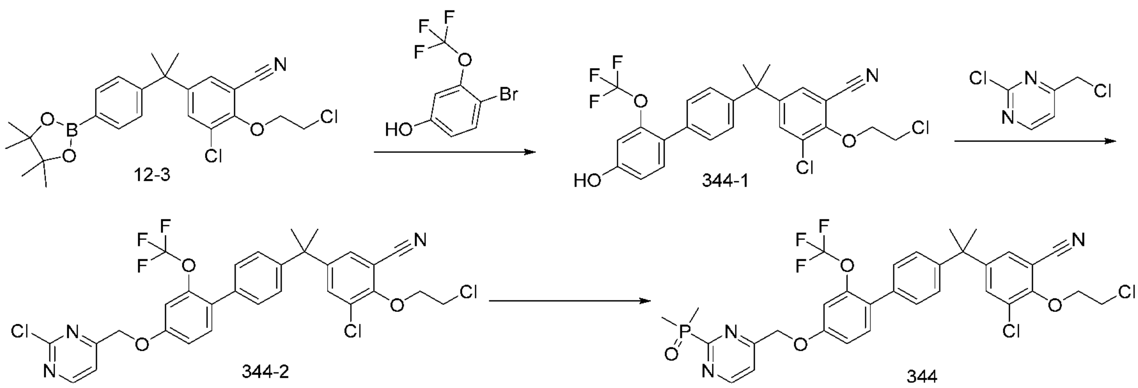
12-3
344-1
344-2
344

**Preparation of compound 344-1**

Compound **12-3** (300 mg, 651.89 μmol) was dissolved in 1,4-dioxane (10 mL). 4-Bromo-3-(trifluoromethoxy) phenol (201 mg, 782.27 μmol), potassium carbonate (270 mg, 1.96 mmol), and [1,1'-bis(diphenylphosphino)ferrocene] dichloropalladium(II) (95 mg, 130.38 μmol) were added, followed by water (2 mL). The reaction system was purged three times with nitrogen and stirred at 100°C for 5 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 50%) to obtain 300 mg of the title compound **344-1.** LC-MS (ESI): m/z 510.2 $[M+H]^+$.

**Preparation of compound 344-2**

Compound **344-1** (300 mg, 587.85 μmol), acetonitrile (4 mL), 2-chloro-4-(chloromethyl)pyrimidine (144 mg, 881.78 μmol), and $K_2CO_3$ (162 mg, 1.18 mmol) were added to a 100 mL single-necked flask, and the mixture was stirred at 90°C for 2 h. TLC indicated the reaction was essentially complete. Water (5 mL) was added, and the mixture was extracted twice with ethyl acetate (4 mL). The organic phases were combined, washed with water (5 mL), dried over anhydrous sodium sulfate, subjected to suction filtration, and the filtrate was evaporated to dryness under reduced pressure. The residue was purified by an automated column chromatography system (EA/PE = 0 to 20%) to obtain 350 mg of the title compound **344-2.** LC-MS (ESI): m/z 636.0 $[M+H]^+$.

**Preparation of compound 344**

Dimethylphosphine oxide (19 mg, 235.52 μmol) and compound **344-2** (100 mg, 157.02 μmol) were dissolved in DMF (1 mL), then $Pd_2(dba)_3$ (14.38 mg, 15.70 μmol), Xantphos (18 mg, 31.40 μmol), and DIEA (61 mg, 471.05 μmol) were added. The system was purged three times with nitrogen and reacted under microwave irradiation at 120°C for 2 h. The mixture was filtered and subjected to rotary evaporation until dryness to obtain a crude product. The crude product was subjected to preparative purification (preparative method: mobile phase: A: 0.1% formic acid in water; B: acetonitrile; chromatographic column: Agilent 10 Prep-C18 250 × 21.2 mm; column temperature: 25°C; gradient: 10% to 80% acetonitrile gradient elution over 30 min; flow rate: 30 mL/min) to obtain 50 mg of the title compound 344. LC-MS (ESI): m/z 678.2 $[M+H]^+$. $^1H$ NMR (400 MHz, DMSO-$d_6$) δ 9.02 (d, *J* = 5.2 Hz, 1H), 7.78 (dd, *J* = 5.2, 3.2 Hz, 1H), 7.68 (d, *J* = 2.3 Hz, 1H), 7.62 (d, *J* = 2.3 Hz, 1H), 7.50 (d, *J* = 8.5 Hz, 1H), 7.42 - 7.30 (m, 4H), 7.24 - 7.15 (m, 2H), 5.41 (s, 2H), 4.42 (t, *J* = 5.2 Hz, 2H), 3.96 (t, *J* = 5.2 Hz, 2H), 1.75 (d, *J* = 13.7 Hz, 6H), 1.70 (s, 6H). $^{19}F$ NMR (376 MHz, DMSO-$d_6$) δ -56.08 (s, 3F). $^{31}P$ NMR (162 MHz, DMSO-$d_6$) δ 34.07 (s, 1P).

**Example 345: Preparation of compound 345**

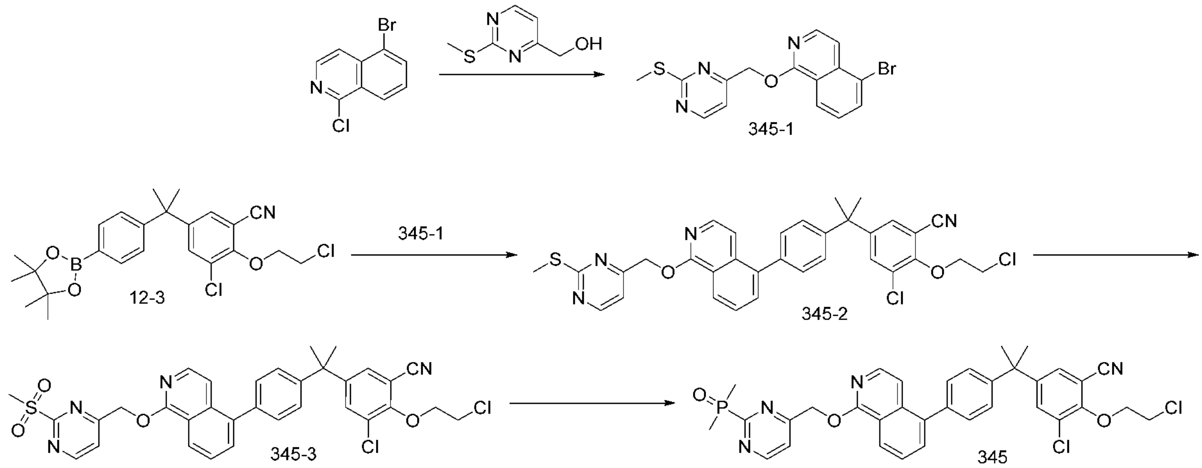

**Preparation of compound 345-1**

1-Chloro-5-bromoisoquinoline (500 mg, 2.06 mmol) and (2-(methylthio)pyrimidin-4-yl)methanol (644.2 mg, 4.12 mmol) were dissolved in toluene (10 mL). Potassium carbonate (427.4 mg, 3.09 mmol), potassium hydroxide (173.5 mg, 3.09 mmol), and tris(3,6-dioxaheptyl)amine (666.9 mg, 2.06 mmol) were added. The reaction mixture was heated and stirred at 120°C for 12 h. LCMS indicated the formation of the product. The reaction mixture was poured into water (30 mL) and extracted three times with dichloromethane (30 mL). The organic phases were washed three times with saturated brine (30 mL), combined, dried over anhydrous sodium sulfate, filtered, and subjected to rotary evaporation until dryness. The resulting residue was purified by silica gel column chromatography (EA/PE = 0 to 20%) to obtain 580 mg of the title compound **345-1.** LC-MS (ESI): m/z 362.0 $[M+H]^+$.

**Preparation of compound 345-2**

Compound **12-3** (600 mg, 1.30 mmol) and compound **345-1** (519.5 mg, 1.43 mmol) were dissolved in 1,4-dioxane (5 mL) and water (2 mL), and $Pd(dppf)Cl_2$ (95.4 mg, 130.38 μmol) and $K_2CO_3$ (450.5 mg, 3.26 mmol) were added. The system was purged three times with nitrogen. The reaction mixture was heated and stirred under microwave irradiation at 120°C for 2 h. LCMS indicated the disappearance of the starting material and the formation of the product. The reaction mixture was subjected to rotary evaporation until dryness and then purified by silica gel column chromatography (EA/PE = 0 to 18%) to obtain 610 mg of the title compound **345-2.** LC-MS (ESI): m/z 615.2 $[M+H]^+$.

**Preparation of compound 345-3**

Compound **345-2** (500 mg, 812.25 μmol) was dissolved in DCM (10 mL), and m-CPBA (659.6 mg, 3.25 mmol, 85% purity) was added. The reaction mixture was stirred at room temperature for 4 h. LCMS indicated the disappearance

of the starting material and the formation of the product. The reaction mixture was poured into saturated sodium sulfite solution (30 mL) and extracted three times with dichloromethane (30 mL). The organic phases were combined, washed three times with saturated brine (30 mL), dried over anhydrous sodium sulfate, filtered, and subjected to rotary evaporation until dryness. The resulting residue was purified by silica gel column chromatography (EA/PE = 0 to 70%) to obtain 440 mg of the title compound **345-3.** LC-MS (ESI): m/z 647.2 [M+H]+.

**Preparation of compound 345**

Compound **345-3** (220 mg, 339.73 μmol) and dimethylphosphine oxide (105.4 mg, 1.70 mmol) were dissolved in acetonitrile (2 mL), and $K_2CO_3$ (140.9 mg, 1.02 mmol) was added. The reaction mixture was heated at 80°C for 4 h. LCMS indicated the disappearance of the starting material and the formation of the product. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 68.16 mg of the title compound **345.** LC-MS (ESI): m/z 645.2 [M+H]+. $^1H$ NMR (400 MHz, DMSO-$d_6$) δ 8.94 (d, *J* = 5.3 Hz, 1H), 8.47 - 8.42 (m, 1H), 7.95 (d, *J* = 6.1 Hz, 1H), 7.83 - 7.75 (m, 3H), 7.75 - 7.69 (m, 2H), 7.51 - 7.38 (m, 4H), 7.29 (dd, *J* = 6.1, 0.9 Hz, 1H), 5.78 (s, 2H), 4.44 (t, *J* = 5.2 Hz, 2H), 3.97 (t, *J* = 5.2 Hz, 2H), 1.74 (s, 6H), 1.73 (d, *J*= 14.0 Hz, 6H). $^{31}P$ NMR (162 MHz, DMSO-$d_6$) δ 34.25 (s, 1P).

**Example 346: Preparation of compound 346**

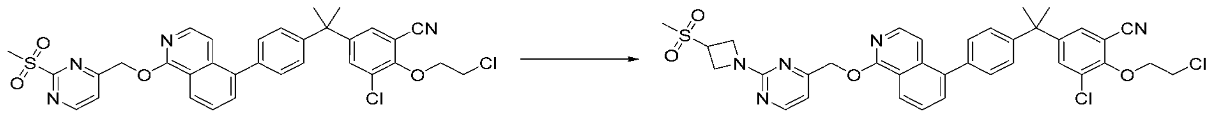

345-3 346

**Preparation of compound 346**

Compound **345-3** (220 mg, 339.73 μmol) and 3-(methylsulfonyl)azetidine hydrochloride (116.6 mg, 679.46 μmol) were dissolved in acetonitrile (2 mL), and $K_2CO_3$ (140.9 mg, 1.02 mmol) was added. The reaction mixture was heated at 80°C for 4 h. LCMS indicated the disappearance of the starting material and the formation of the product. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30.37 mg of the title compound **346.** LC-MS (ESI): m/z 702.2 [M+H]+. $^1H$ NMR (400 MHz, DMSO-$d_6$) δ 8.42 - 8.36 (m, 2H), 7.96 (d, *J* = 6.1 Hz, 1H), 7.79 - 7.72 (m, 4H), 7.48 - 7.40 (m, 4H), 7.27 (d, *J* = 6.1 Hz, 1H), 6.84 (d, *J* = 5.1 Hz, 1H), 5.53 (s, 2H), 4.44 (t, *J* = 5.2 Hz, 2H), 4.42 - 4.37 (m, 1H), 4.33 (t, *J* = 8.8 Hz, 2H), 4.22 (dd, *J* = 9.7, 4.9 Hz, 2H), 3.97 (t, *J* = 5.2 Hz, 2H), 3.07 (s, 3H), 1.74 (s, 6H).

**Examples 347 and 348: Preparation of compounds 347 and 348**

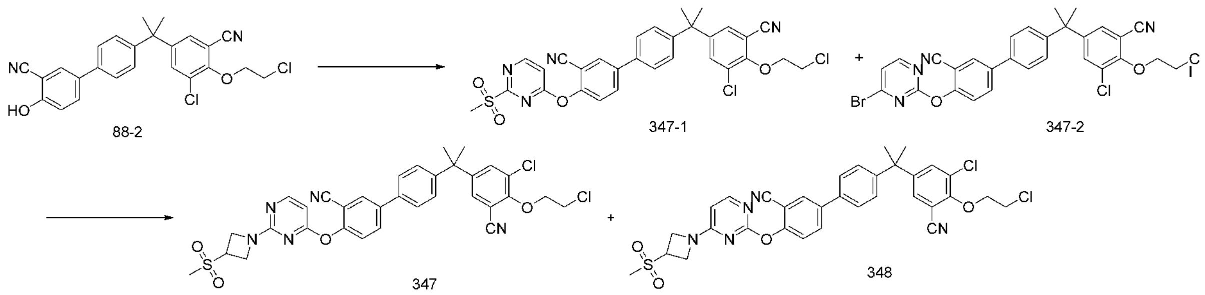
88-2 347-1 347-2
347 348

**Preparation of compounds 347-1 and 347-2**

Compound **88-2** (200 mg, 0.44 mmol) was dissolved in acetonitrile (5 mL), followed by the sequential addition of 4-bromo-2-(methylsulfonyl)pyrimidine (157 mg, 0.66 mmol) and potassium carbonate (120 mg, 0.88 mmol). The reaction system was stirred at 80°C for 2 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated. The resulting crude product was purified by silica gel column chromatography (EA/PE = 0 to 50%) to obtain 200 mg of a mixture of the title compounds 347-1 and 347-2. LC-MS (ESI): m/z 607.0 $[M+H]^+$.

**Preparation of compounds 347 and 348**

The mixture of compounds **347-1** and **347-2** (200 mg, 0.33 mmol) was dissolved in acetonitrile (5 mL), followed by the sequential addition of 3-(methylsulfonyl)azetidine hydrochloride (69 mg, 0.49 mmol) and cesium carbonate (215 mg, 0.66 mmol). The reaction system was stirred at 80°C for 2 h. After the reaction was completed, water (10 mL) was added and the mixture was extracted three times with dichloromethane (20 mL). The organic phases were combined, washed twice with saturated brine (20 mL), dried over anhydrous sodium sulfate, subjected to suction filtration, and the filtrate was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 6.37 mg of the title compound **347** and 1.52 mg of the title compound **348.**

Compound **347:** LC-MS (ESI): m/z 662.2 $[M+H]^+$. $^1H$ NMR (400 MHz, DMSO-$d_6$) δ 8.38 (d, *J* = 5.6 Hz, 1H), 8.28 (d, *J* = 2.4 Hz, 1H), 8.09 (dd, *J* = 8.7, 2.3 Hz, 1H), 7.74 (d, *J* = 8.4 Hz, 2H), 7.71 (d, *J* = 2.4 Hz, 1H), 7.64 (d, *J* = 2.4 Hz, 1H), 7.57 (d, *J* = 8.7 Hz, 1H), 7.38 (d, *J* = 8.5 Hz, 2H), 6.52 (d, *J* = 5.6 Hz, 1H), 4.42 (t, *J* = 5.2 Hz, 2H), 4.38 - 4.27 (m, 1H), 4.27 - 4.12 (m, 2H), 4.12 - 3.99 (m, 2H), 3.96 (t, *J* = 5.2 Hz, 2H), 3.01 (s, 3H), 1.70 (s, 6H).

Compound **348:** LC-MS (ESI): m/z 662.2 $[M+H]^+$. $^1H$ NMR (400 MHz, DMSO-$d_6$) δ 8.20 (d, *J* = 2.4 Hz, 1H), 8.08 - 7.99 (m, 2H), 7.75 - 7.68 (m, 3H), 7.66 (d, *J* = 2.3 Hz, 1H), 7.49 (d, *J* = 8.6 Hz, 1H), 7.38 (d, *J* = 8.5 Hz, 2H), 6.34 (d, *J* = 5.8 Hz, 1H), 4.48 - 4.41 (m, 3H), 4.40 - 4.33 (m, 2H), 4.29 - 4.18 (m, 2H), 3.96 *(t, J*= 5.2 Hz, 2H), 3.08 (s, 3H), 1.70 (s, 6H).

**Example 349: Preparation of compound 349**

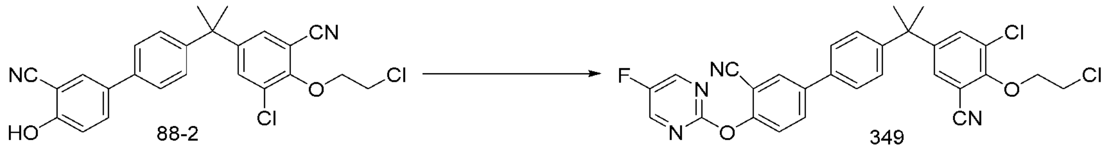
88-2 349

**Preparation of compound 349**

Compound **88-2** (100 mg, 0.22 mmol) was dissolved in acetonitrile (5 mL), followed by the sequential addition of 2-chloro-5-fluoropyrimidine (44 mg, 0.33 mmol) and potassium carbonate (60 mg, 0.44 mmol). The reaction system was stirred at 80°C for 2 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated. The resulting crude product was subjected to preparative purification (preparative method: mobile phase: A: 0.1% formic acid in water; B:

acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 11.13 mg of the title compound **349.** LC-MS (ESI): m/z 547.2 [M+H]$^+$. $^1$H NMR (400 MHz, DMSO-$d_6$) δ 8.84 (s, 2H), 8.26 (d, *J* = 2.4 Hz, 1H), 8.08 (dd, *J* = 8.7, 2.4 Hz, 1H), 7.75 - 7.69 (m, 3H), 7.67 (d, *J* = 2.4 Hz, 1H), 7.60 (d, *J* = 8.7 Hz, 1H), 7.38 (d, *J* = 8.5 Hz, 2H), 4.43 (t, *J* = 5.2 Hz, 2H), 3.96 (t, *J* = 5.2 Hz, 2H), 1.70 (s, 6H). $^{19}$F NMR (376 MHz, DMSO-$d_6$) δ - 146.25 (s, 1H).

**Example 350: Preparation of compounds 350, 350A, and 350B**

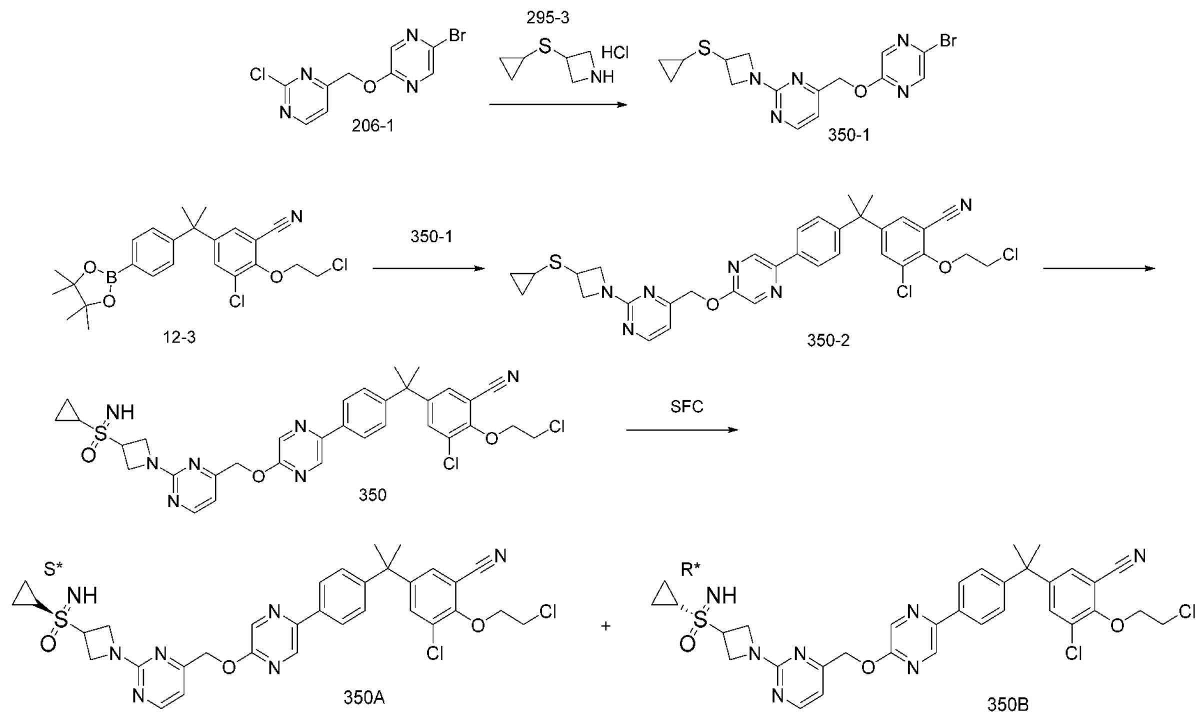

206-1
350-1
12-3
350-2
350
S*
350A
+
R*
350B

**Preparation of compound 350-1**

Compound **206-1** (150 mg, 0.50 mmol) and compound **295-3** (83 mg, 0.50 mmol) were dissolved in ethanol (3 mL). Triethylamine (506 mg, 5.0 mmol) was added at room temperature, and the mixture was stirred at 100°C for 3 h. The reaction was monitored by LCMS until completion. Ice water (5 mL) was added to quench the reaction, and the mixture was extracted three times with ethyl acetate (8 mL). The organic phases were combined, washed once with saturated sodium chloride (10 mL), dried over anhydrous sodium sulfate, subjected to suction filtration, and concentrated to obtain 300 mg of a crude product of the title compound **350-1.** LC-MS (ESI): m/z 394.0 [M+H]$^+$.

**Preparation of compound 350-2**

Compound **350-1** (100 mg, 0.25 mmol) and compound **12-3** (127 mg, 0.28 mmol) were dissolved in 1,4-dioxane (3 mL) and water (0.6 mL). Pd(dppf)$Cl_2$ (18 mg, 0.025 mmol) and potassium carbonate (104 mg, 0.75 mmol) were added at room temperature, and the mixture was stirred at 100°C for 2 h. The reaction was monitored by LCMS until completion. The reaction was quenched with ice water (5 mL) and extracted three times with ethyl acetate (10 mL). The organic phases were combined, dried over anhydrous sodium sulfate, subjected to suction filtration, and concentrated. The resulting residue was purified by silica gel column chromatography (PE/EA = 100% to 50%) to obtain 170 mg of the title compound **350-2.** LC-MS (ESI): m/z 647.1 [M+H]$^+$.

**Preparation of compound 350**

Compound **350-2** (120 mg, 0.19 mmol) was dissolved in methanol (3 mL). (Diacetoxyiodo)benzene (245 mg, 0.76 mmol) and ammonium carbamate (37 mg, 0.47 mmol) were added at room temperature, and the mixture was stirred at room temperature for 1 h. The reaction was monitored by LCMS until completion. The reaction mixture was subjected to

preparative purification (preparative method: mobile phase: A: 0.1% formic acid in water; B: acetonitrile; chromatographic column: Sunfire C18, 19 * 250 mm, 10 μm; column temperature: 25°C; gradient: 65% to 65% acetonitrile gradient elution over 16 min; flow rate: 20 mL/min) to obtain 83.52 mg of the title compound **350.** LC-MS (ESI): m/z 678.4 $[M+H]^+$. $^1H$ NMR (400 MHz, DMSO-$d_6$) δ 8.75 (d, *J*= 1.4 Hz, 1H), 8.53 (d, *J* = 1.3 Hz, 1H), 8.36 (d, *J* = 5.0 Hz, 1H), 7.96 (d, *J* = 8.5 Hz, 2H), 7.70 (d, *J* = 2.3 Hz, 1H), 7.63 (d, *J* = 2.3 Hz, 1H), 7.37 (d, *J*= 8.5 Hz, 2H), 6.76 (d, *J* = 5.0 Hz, 1H), 5.36 (s, 2H), 4.42 (t, *J* = 5.2 Hz, 2H), 4.33 - 4.18 (m, 5H), 3.95 (t, *J* = 5.2 Hz, 2H), 3.88 (s, 1H), 2.64 - 2.57 (m, 1H), 1.69 (s, 6H), 1.08 - 1.01 (m, 1H), 0.93 - 0.87 (m, 2H), 0.86 - 0.80 (m, 1H).

**Preparation of compounds 350A and 350B**

Compound **350** (70 mg) was purified by preparative SFC (preparative conditions: instrument model: WATERS 150 preparative SFC; column model: ChiralCel OJ, 250 × 30 mm I.D., 10 μm; mobile phase: A: $CO_2$, B: ethanol/acetonitrile = 2:1 (0.1% $NH_3H_2O$); elution gradient: B 40%; flow rate: 120 mL/min; column pressure: 100 bar; column temperature: 38°C; detection wavelength: 220 nm; cycle time: about 5 min.) to obtain 14.1 mg of the title compound **350A** and 25.9 mg of the title compound **350B.**

Compound **350A:** SFC analysis method (instrument model: Waters UPC2 analytical SFC; column model: Chiralcel OJ-3 150 * 4.6 mm I.D., 3 μm; mobile phase: A: $CO_2$, B: ethanol/acetonitrile = 2:1 (0.05% DEA); elution gradient: B 40%; flow rate: 2.5 mL/min; column pressure: 100 bar; column temperature: 35°C; detection wavelength: 220 nm; Rt = 2.933 min). LC-MS (ESI): m/z 678.0 $[M+H]^+$. $^1H$ NMR (400 MHz, DMSO-$d_6$) δ 8.76 (d, *J* = 1.4 Hz, 1H), 8.54 (d, *J* = 1.4 Hz, 1H), 8.36 (d, *J* = 5.0 Hz, 1H), 7.96 (d, *J* = 8.5 Hz, 2H), 7.70 (d, *J* = 2.3 Hz, 1H), 7.64 (d, *J* = 2.3 Hz, 1H), 7.37 (d, *J* = 8.6 Hz, 2H), 6.76 (d, *J* = 5.0 Hz, 1H), 5.37 (s, 2H), 4.42 (t, *J* = 5.2 Hz, 2H), 4.36 - 4.16 (m, 5H), 3.96 (t, *J* = 5.2 Hz, 2H), 3.90 (s, 1H), 2.65 - 2.57 (m, 1H), 1.69 (s, 6H), 1.10 - 1.00 (m, 1H), 0.93 - 0.87 (m, 2H), 0.86 - 0.80 (m, 1H).

Compound **350B:** SFC analysis method (instrument model: Waters UPC2 analytical SFC; column model: Chiralcel OJ-3 150 * 4.6 mm I.D., 3 μm; mobile phase: A: $CO_2$, B: ethanol/acetonitrile = 2:1 (0.05% DEA); elution gradient: B 40%; flow rate: 2.5 mL/min; column pressure: 100 bar; column temperature: 35°C; detection wavelength: 220 nm; Rt = 3.467 min). LC-MS (ESI): m/z 678.0 $[M+H]^+$. $^1H$ NMR (400 MHz, DMSO-$d_6$) δ 8.76 (d, *J* = 1.4 Hz, 1H), 8.54 (d, *J* = 1.4 Hz, 1H), 8.37 (d, *J* = 5.0 Hz, 1H), 7.97 (d, *J* = 8.5 Hz, 2H), 7.71 (d, *J* = 2.3 Hz, 1H), 7.64 (d, *J* = 2.4 Hz, 1H), 7.38 (d, *J* = 8.5 Hz, 2H), 6.77 (d, *J* = 5.0 Hz, 1H), 5.37 (s, 2H), 4.43 (t, *J* = 5.2 Hz, 2H), 4.36 - 4.18 (m, 5H), 3.96 (t, *J* = 5.2 Hz, 2H), 3.90 (s, 1H), 2.66 - 2.58 (m, 1H), 1.70 (s, 6H), 1.11 - 0.99 (m, 1H), 0.93 - 0.87 (m, 2H), 0.86 - 0.80 (m, 1H).

**Example 351: Preparation of compound 351**

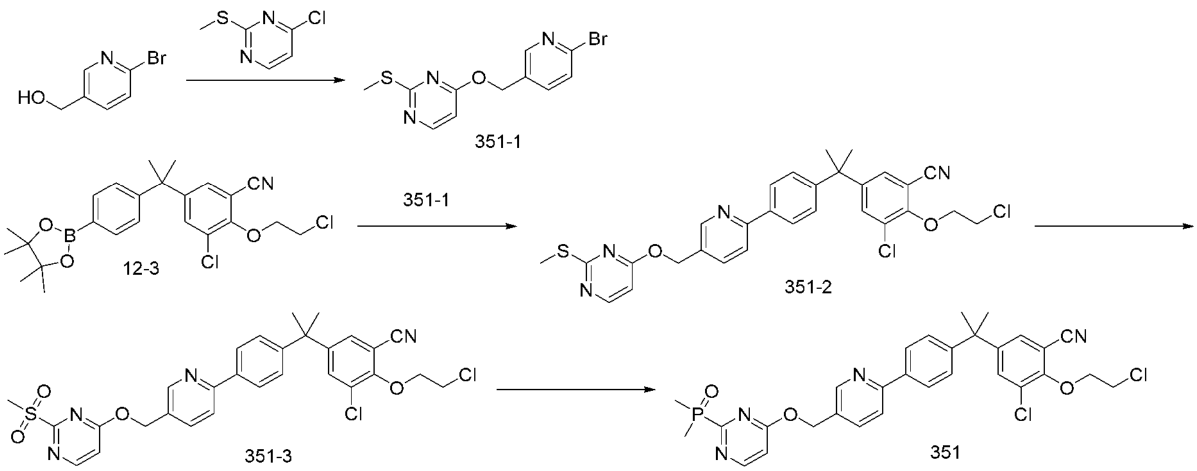


**Preparation of compound 351-1**

(6-Bromopyridin-3-yl)methanol (1 g, 5.32 mmol) and 2-(methylthio)-4-chloropyrimidine (0.85 g, 5.32 mmol) were dissolved in DMF (30 mL), and cesium fluoride (1.73 g, 5.32 mmol) was added thereto. After the addition was completed, the reaction system was stirred at 50°C for 1 h. After the reaction was completed, a 5% aqueous lithium chloride solution (100 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA =

100% to 85%) to obtain 1.6 g of the title compound **351-1.** LC-MS (ESI): m/z 312.0 $[M+H]^+$.

**Preparation of compound 351-2**

Compound **351-1** (1.2 g, 3.84 mmol), compound **12-3** (1.77 g, 3.84 mmol), and potassium carbonate (1.06 g, 7.69 mmol) were dissolved in 1,4-dioxane (30 mL) and water (10 mL), and $Pd(dppf)Cl_2(DCM)$ (314 mg, 0.384 mmol) was added thereto. After the addition was completed, the reaction system was stirred at 91°C for 2 h. After the reaction was completed, water (50 mL) was added, and the mixture was extracted three times with ethyl acetate (20 mL). The organic phases were combined, washed twice with saturated brine (30 mL), dried over anhydrous sodium sulfate, filtered, and the filtrate was concentrated to obtain a crude product. The crude product was purified by silica gel column chromatography (PE/EA = 100% to 75%) to obtain 2 g of the title compound **351-2.** LC-MS (ESI): m/z 565.2 $[M+H]^+$.

**Preparation of compound 351-3**

Compound **351-2** (0.5 g, 0.884 mmol) was dissolved in dichloromethane (30 mL), and m-chloroperoxybenzoic acid (0.538 g, 2.65 mmol) was added thereto. After the addition was completed, the reaction system was stirred at room temperature for 1 h. After the reaction was completed, the reaction mixture was filtered, and the filtrate was concentrated to obtain a crude product. The crude product was purified by silica gel column chromatography (PE/EA = 100% to 85%) to obtain 0.35 g of the title compound **351-3.** LC-MS (ESI): m/z 597.2$[M+H]^+$.

**Preparation of compound 351**

Compound **351-3** (70 mg, 0.117 mmol), dimethylphosphine oxide (27 mg, 0.351 mmol), and cesium carbonate (114 mg, 0.351 mmol) were placed in a sealed tube, and acetonitrile (2 mL) was added. The reaction system was stirred at 90°C for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 40 mg of the title compound **351.** LC-MS (ESI): m/z 595.2 $[M+H]^+$. $^1H$ NMR (400 MHz, DMSO-$d_6$) δ 8.79 (d, *J* = 2.1 Hz, 1H), 8.70 (d, *J* = 5.8 Hz, 1H), 8.10 - 7.93 (m, 4H), 7.71 (d, *J* = 2.4 Hz, 1H), 7.64 (d, *J* = 2.4 Hz, 1H), 7.37 (d, *J* = 8.6 Hz, 2H), 7.14 (dd, *J* = 5.8, 2.7 Hz, 1H), 5.57 (s, 2H), 4.42 (t, *J* = 5.2 Hz, 2H), 3.96 (t, *J* = 5.2 Hz, 2H), 1.75 (d, *J* = 13.7 Hz, 6H), 1.69 (s, 6H). $^{31}P$ NMR (162 MHz, Chloroform-*d*) δ 34.20 (s, 1P).

**Example 352: Preparation of compound 352**

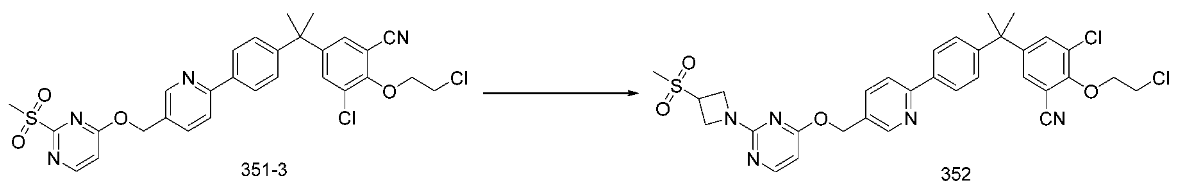

351-3

352

**Preparation of compound 352**

Compound **351-3** (70 mg, 0.117 mmol), 3-(methylsulfonyl)azetidine hydrochloride (40 mg, 0.234 mmol), and DIEA (45 mg, 0.351 mmol) were placed in a sealed tube, and acetonitrile (2 mL) was added. The reaction system was stirred at 90°C for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 40 mg of the title compound **352.** LC-MS (ESI): m/z 652.2 $[M+H]^+$. $^1H$ NMR (400 MHz, DMSO-$d_6$) δ 8.75 (d, *J* = 1.6 Hz, 1H), 8.14 (d, *J* = 5.7 Hz, 1H), 8.03 (d, *J* = 8.5 Hz, 2H), 7.98 - 7.93 (m, 2H), 7.71 (d, *J* = 2.4 Hz, 1H), 7.64 (d, *J* = 2.3 Hz, 1H), 7.37 (d, *J* = 8.5 Hz, 2H), 6.26 (d, *J* = 5.6 Hz, 1H), 5.43 (s, 2H), 4.42 (t, *J* = 5.2 Hz, 2H), 4.40 - 4.36 (m, 1H), 4.31 (t, *J* = 8.8 Hz, 2H), 4.26 - 4.19 (m, 2H), 3.96 (t, *J* = 5.2 Hz, 2H), 3.06 (s, 3H), 1.70 (s, 6H).

**Example 353: Preparation of compound 353**

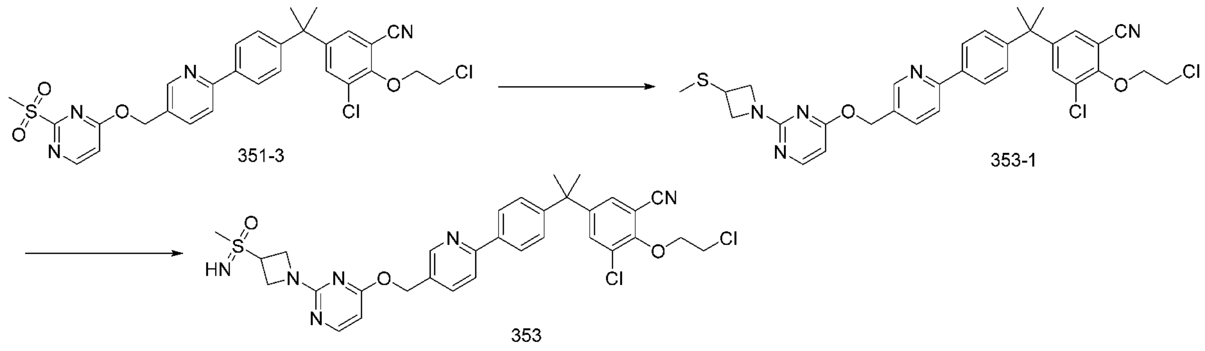

**Preparation of compound 353-1**

Compound **351-3** (120 mg, 0.201 mmol), 3-(methylthio)azetidine hydrochloride (56 mg, 0.401 mmol), and DIEA (79 mg, 0.602 mmol) were placed in a sealed tube, and acetonitrile (5 mL) was added. The reaction system was stirred at 90°C for 16 h. After the reaction was completed, insoluble solids were removed by filtration. A 5% aqueous lithium chloride solution (20 mL) was added, and the mixture was extracted three times with ethyl acetate (20 mL). The organic phases were combined, washed twice with saturated brine (20 mL), dried over anhydrous sodium sulfate, filtered, and the filtrate was concentrated to obtain a crude product. The crude product was purified by silica gel column chromatography (PE/EA = 100% to 85%) to obtain 120 mg of the title compound **353-1.** LC-MS (ESI): m/z 620.2 $[M+H]^+$.

**Preparation of compound 353**

Compound **353-1** (120 mg, 0.580 mmol), (diacetoxyiodo)benzene (187 mg, 0.580 mmol), and ammonium carbonate (76 mg, 0.966 mmol) were placed in a sealed tube, and methanol (3 mL) was added. The reaction system was stirred at room temperature for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 80 mg of the title compound **353.** LC-MS (ESI): m/z 651.2 $[M+H]^+$. $^1$H NMR (400 MHz, DMSO-$d_6$) δ 8.75 (t, *J* = 1.5 Hz, 1H), 8.12 (d, *J* = 5.6 Hz, 1H), 8.03 (d, *J* = 8.5 Hz, 2H), 7.97 - 7.94 (m, 2H), 7.71 (d, *J* = 2.3 Hz, 1H), 7.63 (d, *J* = 2.3 Hz, 1H), 7.37 (d, *J* = 8.5 Hz, 2H), 6.22 (d, *J* = 5.7 Hz, 1H), 5.42 (s, 2H), 4.42 (t, *J* = 5.2 Hz, 2H), 4.31 - 4.20 (m, 5H), 3.99 (s, 1H), 3.96 (t, *J* = 5.2 Hz, 2H), 2.91 (s, 3H), 1.69 (s, 6H).

**Example 354: Preparation of compound 354**

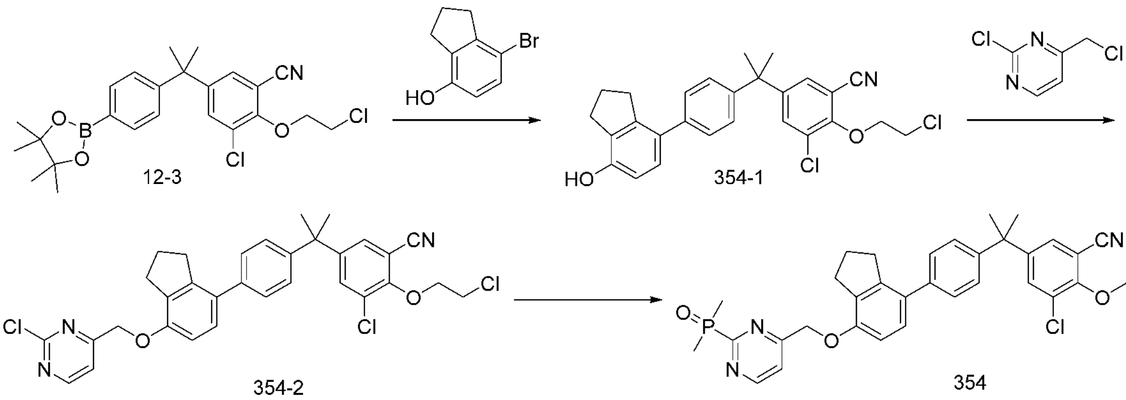

**Preparation of compound 354-1**

Compound **12-3** (260 mg, 564.97 μmol) was dissolved in a solution of 1,4-dioxane (5 mL) and water (2 mL), followed by the sequential addition of Pd(dppf)$Cl_2$ (41.3 mg, 56.50 μmol), $K_2CO_3$ (195.2 mg, 1.41 mmol), and 7-bromo-2,3-dihydro-1*H*-inden-4-ol (120.4 mg, 564.97 μmol). The reaction system was purged three times with nitrogen and stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 20%) to obtain 180 mg of the title compound **354-1.** LC-MS (ESI): m/z 466.0 $[M+H]^+$.

**Preparation of compound 354-2**

Compound **354-1** (180 mg, 385.94 μmol) and 2-chloro-4-(chloromethyl)pyrimidine (69.2 mg, 424.53 μmol) were dissolved in acetonitrile (2 mL), and potassium carbonate (106.7 mg, 771.87 μmol) was added. The reaction mixture was heated and stirred at 80°C for 16 h. LCMS indicated the disappearance of the starting material and the formation of the product. The reaction mixture was poured into water (30 mL) and extracted three times with ethyl acetate (30 mL). The organic phases were combined, washed twice with saturated brine (20 mL), dried over anhydrous sodium sulfate, filtered, and subjected to rotary evaporation until dryness. The resulting residue was purified by silica gel column chromatography (EA/PE = 0 to 25%) to obtain 200 mg of the title compound **354-2.** LC-MS (ESI): m/z 592.0 $[M+H]^+$.

**Preparation of compound 354**

Compound **354-2** (200 mg, 337.30 μmol) was dissolved in DMF (2 mL), followed by the sequential addition of $Pd_2(dba)_3$ (30.9 mg, 33.73 μmol), Xantphos (39.1 mg, 67.46 μmol), DIPEA (87.2 mg, 674.60 μmol, 117.50 μL), and dimethylphosphine oxide (131.6 mg, 1.69 mmol). The reaction system was stirred at 120°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated. The resulting crude product was purified by preparative liquid chromatography (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 50.11 mg of the title compound **354.** LC-MS (ESI): m/z 634.2 $[M+H]^+$. $^1H$ NMR (400 MHz, DMSO-$d_6$) δ 8.99 (d, 1H), 7.77 - 7.68 (m, 2H), 7.66 (d, *J* = 2.3 Hz, 1H), 7.37 (d, *J* = 8.4 Hz, 2H), 7.28 (d, *J* = 8.5 Hz, 2H), 7.15 (d, *J* = 8.4 Hz, 1H), 6.89 (d, *J* = 8.5 Hz, 1H), 5.35 (s, 2H), 4.42 (t, *J* = 5.2 Hz, 2H), 3.96 (t, *J* = 5.2 Hz, 2H), 2.96 (q, *J*= 7.1 Hz, 4H), 2.10 - 1.97 (m, 2H), 1.77 (d, *J* = 13.8 Hz, 6H), 1.69 (s, 6H). $^{31}P$ NMR (162 MHz, DMSO-$d_6$) δ 34.12 (s, 1P).

**Example 355: Preparation of compound 355**

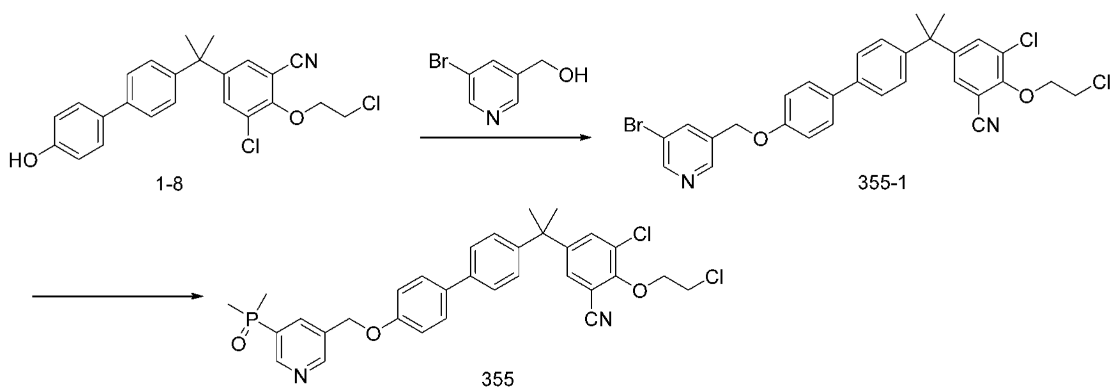

1-8

355-1

355

**Preparation of compound 355-1**

Compound **1-8** (250 mg, 0.59 mmol) was added to a three-necked flask, followed by the sequential addition of

anhydrous DCM (10 mL), (5-bromopyridin-3-yl)methanol (143 mg, 0.76 mmol), and *N,N,N,N*-tetramethylazodicarboxamide (202 mg, 1.17 mmol). The system was purged three times with nitrogen, and tributylphosphine (237 mg, 1.17 mmol) was added at 0°C. The reaction was stirred at 25°C for 3 h. After the reaction was completed, water (20 mL) was added, and the mixture was extracted with DCM (20 mL × 2). The organic phases were combined, dried, subjected to suction filtration, and concentrated to obtain a crude product. The crude product was purified by normal-phase silica gel column chromatography (EtOAc/PE = 0 to 30%) to obtain 280 mg of the title compound **355-1** in 80% yield. LC-MS (ESI): m/z 595.0 $[M+H]^+$.

**Preparation of compound 355**

Compound **355-1** (80 mg, 0.13 mmol) was dissolved in DMF (3 mL), followed by the sequential addition of tris(dibenzylideneacetone)dipalladium (12 mg, 0.01 mmol), 4,5-bis(diphenylphosphino)-9,9-dimethylxanthene (16 mg, 0.03 mmol), DIEA (52 mg, 0.40 mmol), and dimethylphosphine oxide (31 mg, 0.40 mmol). The reaction system was stirred at 120°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 56 mg of the title compound **355.** LC-MS (ESI): m/z 593.0 $[M+H]^+$. $^1$H NMR (400 MHz, DMSO-$d_6$) δ 8.90 (dd, *J* = 5.7, 1.9 Hz, 1H), 8.85 *(t, J*= 2.1 Hz, 1H), 8.27 (dt, *J* = 11.4, 2.1 Hz, 1H), 7.71 (d, *J* = 2.3 Hz, 1H), 7.67 - 7.59 (m, 3H), 7.57 (d, *J* = 8.5 Hz, 2H), 7.30 (d, *J* = 8.5 Hz, 2H), 7.13 (d, *J* = 8.8 Hz, 2H), 5.26 (s, 2H), 4.42 (t, *J* = 5.2 Hz, 2H), 3.96 *(t, J*= 5.2 Hz, 2H), 1.74 (d, *J* = 13.6 Hz, 6H), 1.68 (s, 6H). $^{31}$P NMR (162 MHz, DMSO-$d_6$) δ 31.55 (s, 1P).

**Example 356: Preparation of compound 356**

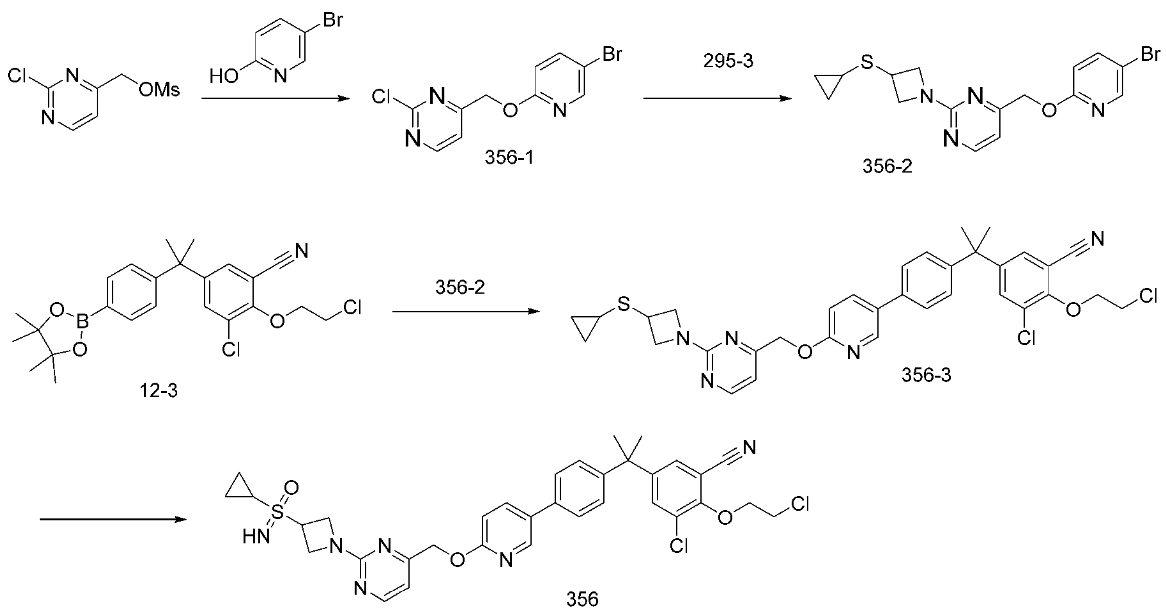


356-1

356-2

12-3

356-3

356

**Preparation of compound 356-1**

(2-Chloropyrimidin-4-yl)methyl methanesulfonate (1.0 g, 4.49 mmol), 2-hydroxy-5-bromopyridine (940 mg, 5.39 mmol), and cesium carbonate (2.93 g, 8.98 mmol) were dissolved in acetonitrile (10 mL). The system was reacted at 70°C for 2 h. The reaction mixture was filtered, and the filtrate was concentrated. The resulting residue was purified by silica gel column chromatography (EA/PE = 0% to 40%) to obtain 250 mg of the title compound **356-1.** $^1$H NMR (400 MHz, DMSO-$d_6$) δ 8.75 (d, 1H), 8.26 (d, *J* = 2.4 Hz, 1H), 8.00 (dd, *J* = 8.8, 2.6 Hz, 1H), 7.55 (d, *J* = 5.1 Hz, 1H), 7.05 (d, *J* = 9.0 Hz, 1H), 5.45 (s, 2H).

**Preparation of compound 356-2**

Compound **356-1** (100 mg, 0.33 mmol), compound **295-3** (110 mg, 0.66 mmol), and triethylamine (100 mg, 0.99 mmol) were dissolved in ethanol (2 mL). The system was reacted at 100°C for 2 h. The reaction mixture was subjected to rotary evaporation until dryness. Water (20 mL) was added, and the mixture was extracted with ethyl acetate (20 mL). The organic phase was washed twice with saturated brine (20 mL), dried over anhydrous sodium sulfate, filtered, and subjected to rotary evaporation until dryness to obtain 150 mg of a crude product of the title compound **356-2.** LC-MS (ESI): m/z 392.9 $[M+H]^+$.

**Preparation of compound 356-3**

Compound **356-2** (150 mg, 0.38 mmol), compound **12-3** (210 mg, 0.46 mmol), Pd(dppf)$Cl_2$ (28 mg, 0.038 mmol), and potassium carbonate (110 mg, 0.76 mmol) were dissolved in 1,4-dioxane (2 mL) and water (1 mL). The system was purged with argon and reacted at 100°C for 2 h. The reaction mixture was subjected to rotary evaporation until dryness. Water (20 mL) was added, and the mixture was extracted with ethyl acetate (20 mL). The organic phase was washed with saturated brine (20 mL), dried over anhydrous sodium sulfate, subjected to suction filtration, and the filtrate was concentrated. The resulting residue was purified by silica gel column chromatography (EA/PE = 0% to 48%) to obtain 200 mg of the title compound **356-3** in 81% yield. LC-MS (ESI): m/z 646.2 $[M+H]^+$.

**Preparation of compound 356**

Compound **356-3** (200 mg, 0.31 mmol) was dissolved in methanol (2 mL). (Diacetoxyiodo)benzene (400 mg, 1.24 mmol) and ammonium carbamate (61 mg, 0.78 mmol) were added at room temperature, and then the mixture was stirred at room temperature for 16 h. The reaction was monitored by LCMS until completion. The residue was concentrated and subjected to preparative purification (Waters 2767/Qda; column: XBridge C18 19 * 250 mm, 10 μm; mobile phase A: 0.1% FA/$H_2O$, B: acetonitrile; flow rate: 20 mL/min; elution gradient: 87% to 92%; retention time: 8.5-9.7 min of 17 min) to obtain 57.62 mg of the title compound **356.** LC-MS (ESI): m/z 677.3 $[M+H]^+$. $^1H$ NMR (400 MHz, DMSO-$d_6$) δ 8.43 (d, *J* = 2.5 Hz, 1H), 8.34 (d, *J* = 5.0 Hz, 1H), 8.07 (dd, *J* = 8.6, 2.5 Hz, 1H), 7.70 (d, *J* = 2.3 Hz, 1H), 7.64 (d, *J* = 2.3 Hz, 1H), 7.60 (d, *J* = 8.4 Hz, 2H), 7.33 (d, *J* = 8.4 Hz, 2H), 7.06 (d, *J* = 8.6 Hz, 1H), 6.71 (d, *J* = 5.0 Hz, 1H), 5.32 (s, 2H), 4.42 (t, *J* = 5.2 Hz, 2H), 4.33 - 4.21 (m, 5H), 3.96 (t, *J* = 5.2 Hz, 2H), 3.88 (s, 1H), 2.68 - 2.57 (m, 1H), 1.68 (s, 6H), 1.11 - 0.99 (m, 1H), 0.95 - 0.88 (m, 2H), 0.88 - 0.81 (m, 1H).

**Example 357: Preparation of compound 357**

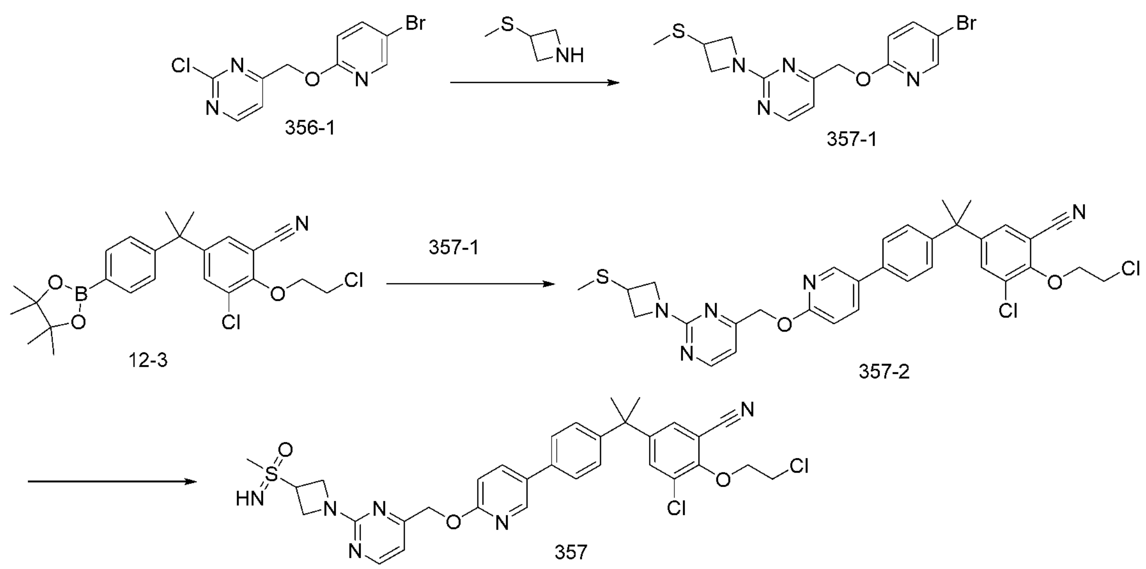


**Preparation of compound 357-1**

Compound **356-1** (100 mg, 0.33 mmol), 3-(methylthio)azetidine (55 mg, 0.40 mmol), and triethylamine (100 mg,

0.99 mmol) were dissolved in ethanol (2 mL). The system was reacted at 100°C for 2 h. The reaction mixture was subjected to rotary evaporation until dryness. Water (10 mL) was added, and the mixture was extracted with ethyl acetate (10 mL). The organic phase was washed with saturated brine (20 mL), dried over anhydrous sodium sulfate, subjected to suction filtration, and concentrated to obtain 150 mg of a crude product of the title compound **357-1.** LC-MS (ESI): m/z 366.9 $[M+H]^+$.

**Preparation of compound 357-2**

Compound **12-3** (150 mg, 0.41 mmol), compound **357-1** (230 mg, 0.49 mmol), Pd(dppf)$Cl_2$ (30 mg, 0.041 mmol), and potassium carbonate (110 mg, 0.82 mmol) were dissolved in 1,4-dioxane (2 mL) and water (1 mL). The system was reacted at 100°C for 2 h. The reaction mixture was subjected to rotary evaporation until dryness. Water (10 mL) was added, and the mixture was extracted with ethyl acetate (10 mL). The organic phase was washed with saturated brine (20 mL), dried over anhydrous sodium sulfate, subjected to suction filtration, and the filtrate was concentrated. The resulting residue was purified by silica gel column chromatography (EA/PE = 0% to 55%) to obtain 130 mg of the title compound **357-2** in 51% yield. LC-MS (ESI): m/z 619.8 $[M+H]^+$.

**Preparation of compound 357**

Compound **357-2** (130 mg, 0.21 mmol) was dissolved in methanol (2 mL). (Diacetoxyiodo)benzene (270 mg, 0.84 mmol) and ammonium carbamate (41 mg, 0.53 mmol) were added at room temperature, and the mixture was stirred at room temperature for 16 h. The reaction was monitored by LCMS until completion. The mixture was concentrated, and the resulting residue was purified by preparative liquid chromatography (Waters 2767/Qda, column: XBridge C18 19 * 250 mm, 10 $\mu$m; mobile phase A: 0.1% FA/$H_2O$, B: ACN; flow rate: 20 mL/min; elution gradient: 70% to 75%; retention time: 7.2-8.5 min of 17 min) to obtain 45.54 mg of the title compound **357.** LC-MS (ESI): m/z 651.3 $[M+H]^+$. $^1$H NMR (400 MHz, DMSO-$d_6$) $\delta$ 8.43 (d, *J* = 2.5 Hz, 1H), 8.34 (d, *J* = 5.0 Hz, 1H), 8.07 (dd, *J* = 8.6, 2.6 Hz, 1H), 7.70 (d, *J* = 2.3 Hz, 1H), 7.64 (d, *J* = 2.3 Hz, 1H), 7.60 (d, *J* = 8.4 Hz, 2H), 7.33 (d, *J* = 8.5 Hz, 2H), 7.06 (d, *J* = 8.7 Hz, 1H), 6.70 (d, *J* = 5.0 Hz, 1H), 5.31 (s, 2H), 4.42 (t, *J* = 5.2 Hz, 2H), 4.34 - 4.17 (m, 5H), 4.02 - 3.95 (m, 1H), 3.96 (t, *J* = 5.2 Hz, 2H), 2.91 (s, 3H), 1.68 (s, 6H).

**Example 358: Preparation of compound 358**

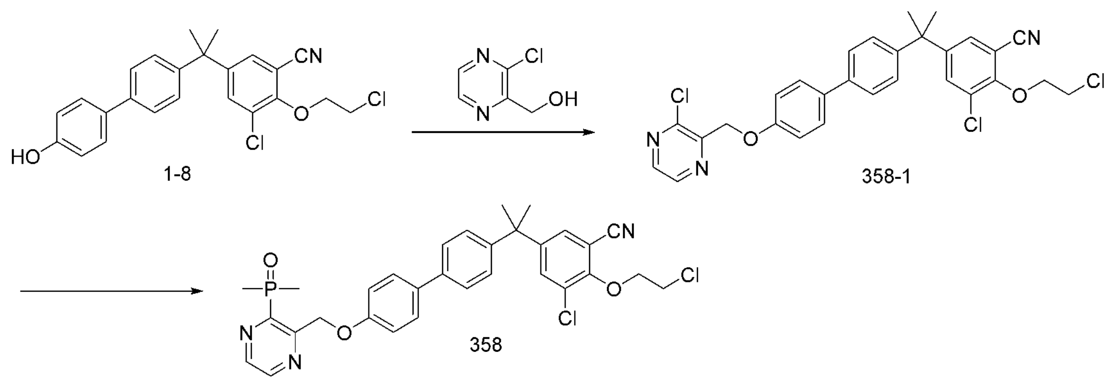

1-8

358-1

358

**Preparation of compound 358-1**

Compound **1-8** (250 mg, 0.59 mmol) was added to a three-necked flask, followed by the sequential addition of anhydrous DCM (10 mL), (3-chloropyrazin-2-yl)methanol (110 mg, 0.76 mmol), and *N,N,N,N*-tetramethylazodicarboxamide (202 mg, 1.17 mmol). The system was purged three times with nitrogen, and tributylphosphine (237 mg, 1.17 mmol) was added at 0°C. The reaction was stirred at 25°C for 3 h. After the reaction was completed, water (20 mL) was added, and the mixture was extracted twice with DCM (20 mL). The organic phases were combined, dried, subjected to suction filtration, and the filtrate was concentrated to obtain a crude product. The crude product was purified by normal-phase silica gel column chromatography (EtOAc/PE = 0 to 30%) to obtain 250 mg of the title compound **358-1** in 77% yield. LC-MS (ESI): m/z 552.2 $[M+H]^+$.

### Preparation of compound 358

Compound **358-1** (120 mg, 0.22 mmol) was dissolved in DMF (3 mL), followed by the sequential addition of tris(dibenzylideneacetone)dipalladium (20 mg, 0.02 mmol), 4,5-bis(diphenylphosphino)-9,9-dimethylxanthene (25 mg, 0.04 mmol), DIEA (84 mg, 0.65 mmol), and dimethylphosphine oxide (51 mg, 0.65 mmol). The reaction system was stirred at 120°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 35 mg of the title compound **358.** LC-MS (ESI): m/z 594.4 $[M+H]^+$. $^1H$ NMR (400 MHz, DMSO-$d_6$) δ 8.89 - 8.74 (m, 2H), 7.71 (d, $J$ = 2.3 Hz, 1H), 7.64 (d, $J$ = 2.3 Hz, 1H), 7.63 - 7.51 (m, 4H), 7.30 (d, $J$ = 8.3 Hz, 2H), 7.11 (d, $J$ = 8.7 Hz, 2H), 5.69 (s, 2H), 4.42 (t, $J$ = 5.1 Hz, 2H), 3.96 (t, $J$ = 5.1 Hz, 2H), 1.78 (d, $J$ = 13.7 Hz, 6H), 1.68 (s, 6H). $^{31}P$ NMR (162 MHz, DMSO-$d_6$) δ 39.33 (s, 1P).

### Example 359: Preparation of compound 359

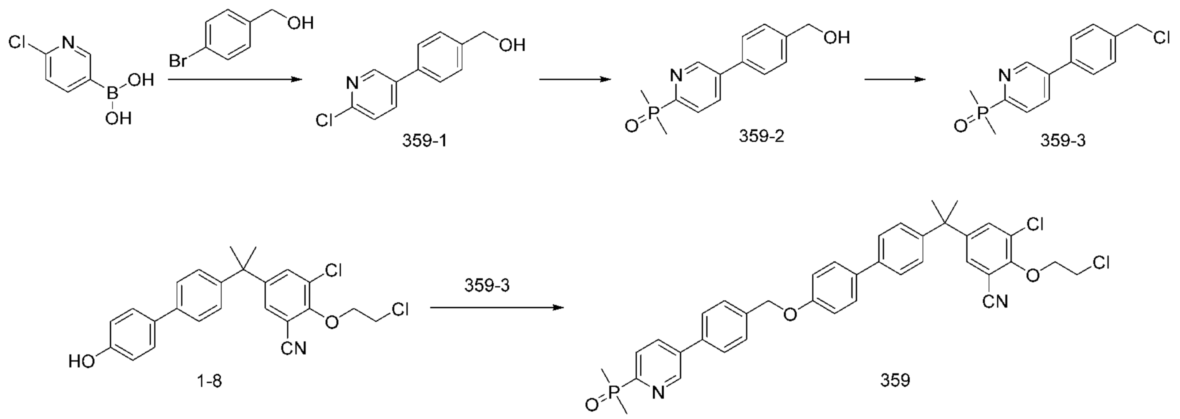


### Preparation of compound 359-1

(6-Chloropyridin-3-yl)boronic acid (3 g, 16.04 mmol), 4-bromobenzyl alcohol (2.65 g, 16.84 mmol), Pd(dppf)$Cl_2$ (1.17 g, 1.60 mmol), and potassium carbonate (6.65 g, 48.12 mmol) were added to a mixed solution of 1,4-dioxane (40 mL) and water (10 mL). The reaction system was purged three times with nitrogen and stirred at 90°C for 8 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (DCM/PE = 0 to 90%) to obtain 1 g of the title compound **359-1.** LC-MS (ESI): m/z 220.0 $[M+H]^+$.

### Preparation of compound 359-2

Compound **359-1** (500 mg, 2.28 mmol), dimethylphosphine oxide (888 mg, 11.38 mmol), $Pd_2(dba)_3$ (208 mg, 0.227 mmol), XantPhos (263 mg, 0.455 mmol), and DIEA (1.47 g, 11.38 mmol) were dissolved in N,N-dimethylformamide (10 mL). The reaction system was purged three times with nitrogen and stirred at 120°C for 16 h. After the reaction was completed, insoluble solids were removed by filtration. The pH was adjusted to 3 with 1 mol/L hydrochloric acid. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (DCM/PE = 0 to 90%) to obtain 400 mg of the title compound **359-2.** LC-MS (ESI): m/z 262.0 $[M+H]^+$.

**Preparation of compound 359-3**

Compound **359-2** (400 mg, 1.53 mmol) was dissolved in dichloromethane (10 mL). The system was purged three times with argon, and thionyl chloride (1.82, 15.31 mmol) was added at 0°C. After the addition was completed, the reaction system was stirred at room temperature for 2 h. After the reaction was completed, the reaction mixture was concentrated to obtain a crude product. The crude product was purified by silica gel column chromatography (DCM/PE = 0 to 90%) to obtain 300 mg of the title compound **359-3.** LC-MS (ESI): m/z 280.0 $[M+H]^+$.

**Preparation of compound 359**

Compound **359-3** (394 mg, 1.41 mmol), compound **1-8** (300 mg, 0.703 mmol), and cesium carbonate (688 mg, 2.11 mmol) were dissolved in *N,N*-dimethylformamide (5 mL). The reaction system was purged three times with nitrogen and stirred at room temperature for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 152 mg of the title compound **359.** LC-MS (ESI): m/z 669.2. $[M+H]^+$. $^1H$ NMR (400 MHz, DMSO-$d_6$) δ 9.10 (d, *J* = 2.2 Hz, 1H), 8.27 (dt, *J* = 8.0, 2.7 Hz, 1H), 8.02 (dd, *J* = 8.0, 5.0 Hz, 1H), 7.83 (d, *J* = 8.3 Hz, 2H), 7.70 (d, *J* = 2.3 Hz, 1H), 7.66 - 7.57 (m, 5H), 7.56 (d, *J* = 8.5 Hz, 2H), 7.30 (d, *J* = 8.4 Hz, 2H), 7.11 (d, *J* = 8.8 Hz, 2H), 5.24 (s, 2H), 4.42 (t, *J* = 5.2 Hz, 2H), 3.96 (t, *J* = 5.2 Hz, 2H), 1.69 (d, *J* = 13.2 Hz, 6H), 1.68 (s, 6H). $^{31}P$ NMR (162 MHz, DMSO-$d_6$) δ 34.05 (s, 1P).

**Example 360: Preparation of compound 360**

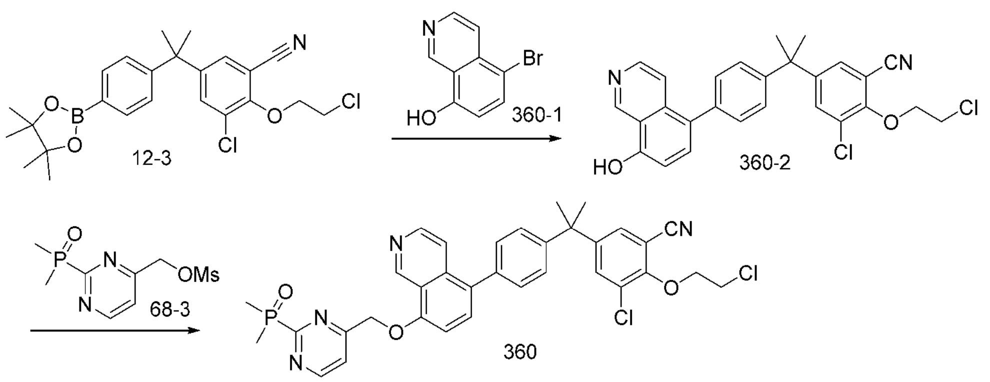


**Preparation of compound 360-2**

Compound **360-1** (0.045 g, 0.2 mmol), compound 12-3 (0.097 g, 0.210 mmol), and potassium carbonate (0.078 g, 0.6 mmol) were added to 1,4-dioxane (10 mL) and water (3 mL), and Pd(dppf)$Cl_2$-DCM (16 mg, 0.020 mmol) was added thereto. After the addition was completed, the reaction system was stirred at 90°C for 12 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 75%) to obtain 65 mg of the title compound **360-2.** LC-MS (ESI): m/z 477.2 $[M+H]^+$.

**Preparation of compound 360**

Compound **360-2** (60 mg, 0.125 mmol), compound **68-3** (40 mg, 0.150 mmol), and potassium carbonate (35 mg, 0.252 mmol) were added to acetonitrile (3 mL). The reaction system was stirred at 100°C for 5 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to

obtain a crude product. The crude product was subjected to preparative purification (preparative method: mobile phase A: 0.1% formic acid in water; mobile phase B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **360.** LC-MS (ESI): m/z 645.2 [M+H]+. $^1$H NMR (400 MHz, Chloroform-d) δ 9.85 (s, 1H), 8.94 (d, *J* = 5.2 Hz, 1H), 8.58 (d, *J* = 6.0 Hz, 1H), 7.81 (dd, *J* = *5.2,* 3.2 Hz, 1H), 7.76 (d, *J* = 5.9 Hz, 1H), 7.59 - 7.53 (m, 2H), 7.42 - 7.37 (m, 3H), 7.32 - 7.28 (m, 2H), 6.98 (d, *J* = 8.0 Hz, 1H), 5.53 (s, 2H), 4.44 (t, *J* = 6.1 Hz, 2H), 3.89 (t, *J* = 6.2 Hz, 2H), 1.92 (d, *J* = 13.6 Hz, 6H), 1.74 (s, 6H). $^{31}$P NMR (162 MHz, Chloroform-d) δ 35.11 (s, 1P).

**Example 361: Preparation of compound 361**

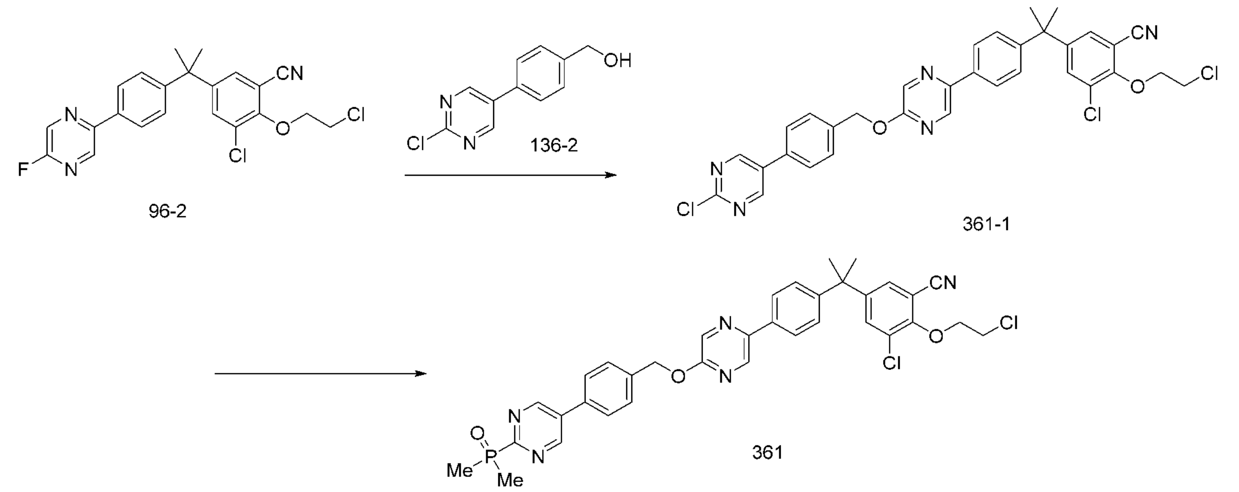

96-2

136-2

361-1

361

**Preparation of compound 361-1**

Compound **136-2** (147 mg, 0.66 mmol) was weighed and added to anhydrous tetrahydrofuran (20 mL), and sodium hydride (48 mg, 1.21 mmol, 60% purity) was added in an ice-water bath. The reaction was carried out for 5 min, and then compound **96-2** (260 mg, 0.60 mmol) was added. After the addition was completed, the reaction was carried out at room temperature for 2 h. After the reaction was monitored by LCMS until completion, the reaction mixture was poured into ice water and extracted twice with ethyl acetate (20 mL). The organic phases were combined, dried over anhydrous sodium sulfate, and concentrated to obtain a crude product. The crude product was purified by normal-phase silica gel column chromatography (EA/PE = 0 to 40%) to obtain 160 mg of the title compound **361-1** in 42% yield. LC-MS (ESI): m/z 630.2 [M+H]+.

**Preparation of compound 361**

Compound **361-1** (80 mg, 0.13 mmol) was dissolved in *N,N*-dimethylacetamide (2 mL), followed by the sequential addition of tris(dibenzylideneacetone)dipalladium (12 mg, 0.01 mmol), 4,5-bis(diphenylphosphino)-9,9-dimethylxanthene (15 mg, 0.03 mmol), DIEA (49 mg, 0.38 mmol), and dimethylphosphine oxide (30 mg, 0.38 mmol). The reaction system was stirred at 120°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **361.** LC-MS (ESI): m/z 672.3 [M+H]+. $^1$H NMR (400 MHz, DMSO-$d_6$) δ 9.33 (s, 2H), 8.81 (d, *J* = 1.4 Hz, 1H), 8.48 (d, *J* = 1.4 Hz, 1H), 7.97 (d, *J* = 8.6 Hz, 2H), 7.91 (d, *J* = 8.3 Hz, 2H), 7.71 (d, *J* = 2.3 Hz, 1H), 7.68 (d, *J* = 8.2 Hz, 2H), 7.64 (d, *J* = 2.3 Hz, 1H), 7.38 (d, *J* = 8.5 Hz, 2H), 5.53 (s, 2H), 4.42 (t, *J* = 5.2 Hz, 2H), 3.96 (t, *J* = 5.2 Hz, 2H), 1.80 (d, *J* = 13.7 Hz, 6H), 1.70 (s, 6H). $^{31}$P NMR (162 MHz, DMSO-$d_6$) δ 33.84 (s, 1P).

**Example 362: Preparation of compound 362**

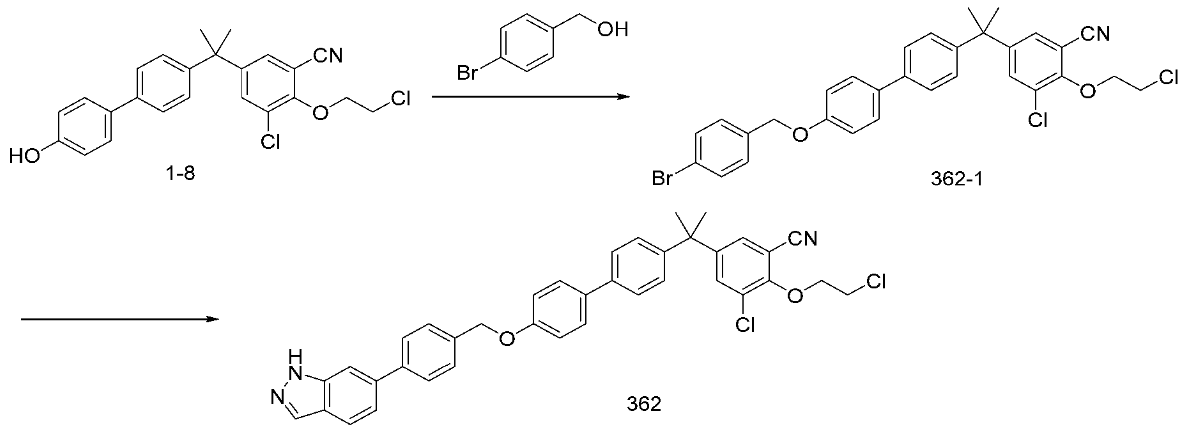

1-8

362-1

362

**Preparation of compound 362-1**

Compound **1-8** (0.2 g, 0.469 mmol), 4-bromobenzyl alcohol (87 mg, 0.469 mmol), and *N,N,N', N'*-tetramethylazodicarboxamide (81 mg, 0.469 mmol) were dissolved in dichloromethane (5 mL). A solution of tributylphosphine (95 mg, 0.469 mmol) in dichloromethane (1 mL) was added thereto. After the addition was completed, the reaction system was stirred at 0°C for 1 h. After the reaction was completed, the reaction mixture was filtered, and the filtrate was concentrated to obtain a crude product. The crude product was purified by silica gel column chromatography (PE/EA = 100% to 85%) to obtain 250 mg of the title compound **362-1.** LC-MS (ESI): m/z 594.0 $[M+H]^+$.

**Preparation of compound 362**

Compound **362-1** (0.1 g, 0.168 mmol), 1H-indazole-6-boronic acid pinacol ester (0.082 g, 0.336 mmol), and potassium carbonate (0.070 g, 0.504 mmol) were dissolved in 1,4-dioxane (10 mL) and water (3 mL), and Pd(dppf)$Cl_2$ (12 mg, 0.017 mmol) was added thereto. After the addition was completed, the reaction system was stirred at 90°C for 12 h. After the reaction was completed, water (20 mL) was added, and the mixture was extracted three times with ethyl acetate (20 mL). The organic phases were combined, washed twice with saturated brine (20 mL), dried over anhydrous sodium sulfate, filtered, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 60 mg of the title compound 362. LC-MS (ESI): m/z 632.2 $[M+H]^+$. $^1H$ NMR (400 MHz, DMSO-$d_6$) δ 13.13 (s, 1H), 8.13 (t, *J* = 1.2 Hz, 1H), 8.04 (t, *J* = 1.2 Hz, 1H), 7.75 - 7.71 (m, 2H), 7.71 - 7.69 (m, 1H), 7.67 (d, *J* = 1.7 Hz, 1H), 7.65 - 7.59 (m, 4H), 7.58 - 7.52 (m, 4H), 7.30 (d, *J* = 8.5 Hz, 2H), 7.12 (d, *J* = 8.8 Hz, 2H), 5.20 (s, 2H), 4.42 (t, *J* = 5.2 Hz, 2H), 3.96 (t, *J* = 5.2 Hz, 2H), 1.68 (s, 6H).

**Example 363: Preparation of compound 363**

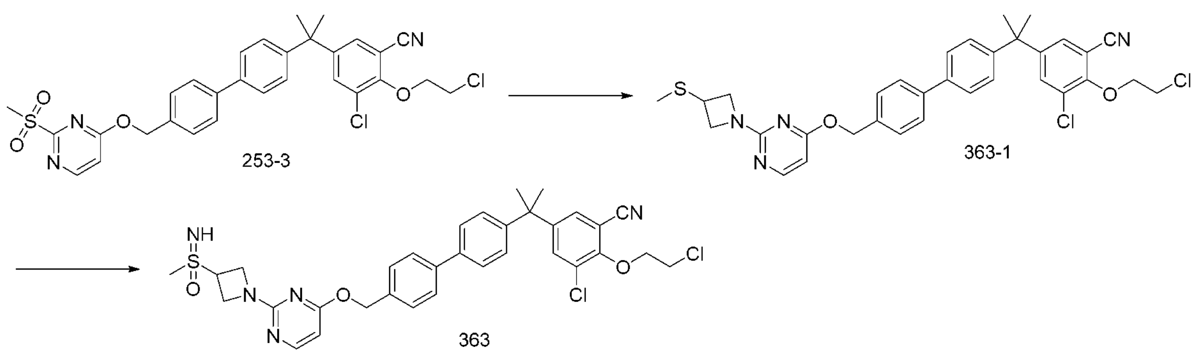

253-3

363-1

363

**Preparation of compound 363-1**

Compound **253-3** (300 mg, 0.503 mmol), 3-(methylthio)azetidine hydrochloride (140 mg, 1.01 mmol), and cesium carbonate (491 mg, 1.51 mmol) were placed in a sealed tube, and acetonitrile (5 mL) was added. The reaction system was stirred at 90°C for 16 h. After the reaction was completed, insoluble solids were removed by filtration. A 5% aqueous lithium chloride solution (20 mL) was added, and the mixture was extracted three times with ethyl acetate (20 mL). The organic phases were combined, washed twice with saturated brine (20 mL), dried over anhydrous sodium sulfate, filtered, and the filtrate was concentrated. The resulting crude product was purified by silica gel column chromatography (PE/EA = 100% to 85%) to obtain 300 mg of the title compound **363-1.** LC-MS (ESI): m/z 619.2 $[M+H]^+$.

**Preparation of compound 363**

Compound **363-1** (300 mg, 0.484 mmol), (diacetoxyiodo)benzene (467 mg, 1.45 mmol), and ammonium carbonate (232 mg, 2.42 mmol) were placed in a sealed tube, and methanol (1 mL) was added. The reaction system was stirred at room temperature for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 60 mg of the title compound **363.** LC-MS (ESI): m/z 650.2 $[M+H]^+$. $^1$H NMR (400 MHz, DMSO-$d_6$) δ 8.12 (d, *J* = 5.6 Hz, 1H), 7.72 (d, *J* = 2.3 Hz, 1H), 7.69 - 7.64 (m, 3H), 7.62 (d, *J* = 8.5 Hz, 2H), 7.53 (d, *J* = 8.2 Hz, 2H), 7.34 (d, *J* = 8.4 Hz, 2H), 6.22 (d, *J* = 5.6 Hz, 1H), 5.39 (s, 2H), 4.42 (t, *J* = 5.2 Hz, 2H), 4.31 - 4.19 (m, 5H), 3.99 (s, 1H), 3.96 (t, *J* = 5.2 Hz, 2H), 2.91 (s, 3H), 1.69 (s, 6H).

**Example 364: Preparation of compound 364**

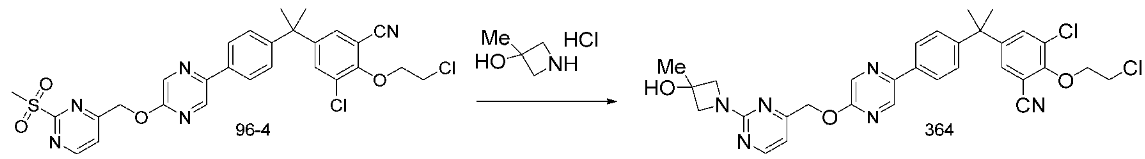

**Preparation of compound 364**

Compound **96-4** (80 mg, 0.13 mmol) was added to a microwave tube, followed by the sequential addition of anhydrous ethanol (2 mL), 3-methylazetidin-3-ol hydrochloride (25 mg, 0.20 mmol), and DIPEA (52 mg, 0.40 mmol). The system was purged three times with nitrogen, and the reaction was carried out under microwave irradiation at 90°C for 2 h. After the reaction was monitored by LCMS until completion, the reaction mixture was filtered. The filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 55 mg of the title compound **364.** LC-MS (ESI): m/z 605.2 $[M+H]^+$. $^1$H NMR (400 MHz, DMSO-$d_6$) δ 8.74 (d, *J* = 1.4 Hz, 1H), 8.53 (d, *J* = 1.4 Hz, 1H), 8.30 (d, *J* = 5.0 Hz, 1H), 7.96 (d, *J* = 8.5 Hz, 2H), 7.70 (d, *J* = 2.3 Hz, 1H), 7.63 (d, *J* = 2.3 Hz, 1H), 7.37 (d, *J* = 8.5 Hz, 2H), 6.68 (d, *J* = 5.0 Hz, 1H), 5.61 (s, 1H), 5.33 (s, 2H), 4.42 (t, *J* = 5.2 Hz, 2H), 3.96 (t, *J* = 5.2 Hz, 2H), 3.92 - 3.79 (m, 4H), 1.69 (s, 6H), 1.41 (s, 3H).

**Example 365: Preparation of compound 365**

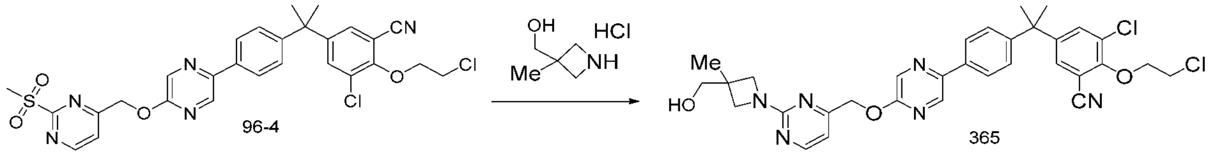

**Preparation of compound 365**

Compound **96-4** (80 mg, 0.13 mmol) was added to a microwave tube, followed by the sequential addition of anhydrous ethanol (2 mL), (3-methylazetidin-3-yl)methanol hydrochloride (28 mg, 0.20 mmol), and DIPEA (52 mg, 0.40 mmol). The system was purged three times with nitrogen, and the reaction was carried out under microwave irradiation at 90°C for 2 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 45 mg of the title compound **365.** LC-MS (ESI): m/z 619.2 $[M+H]^+$. $^1$H NMR (400 MHz, DMSO-$d_6$) δ 8.75 (d, *J* = 1.4 Hz, 1H), 8.52 (d, *J* = 1.4 Hz, 1H), 8.28 (d, *J* = 5.0 Hz, 1H), 7.96 (d, *J* = 8.5 Hz, 2H), 7.70 (d, *J* = 2.4 Hz, 1H), 7.63 (d, *J* = 2.3 Hz, 1H), 7.37 (d, *J* = 8.6 Hz, 2H), 6.64 (d, *J* = 5.0 Hz, 1H), 5.31 (s, 2H), 4.93 (t, *J* = 5.4 Hz, 1H), 4.42 (t, *J* = 5.2 Hz, 2H), 3.95 (t, *J* = 5.2 Hz, 2H), 3.86 (d, *J* = 8.5 Hz, 2H), 3.56 (d, *J* = 8.4 Hz, 2H), 3.39 (d, *J* = 5.4 Hz, 2H), 1.69 (s, 6H), 1.22 (s, 3H).

**Example 366: Preparation of compounds 366, 366A, and 366B**

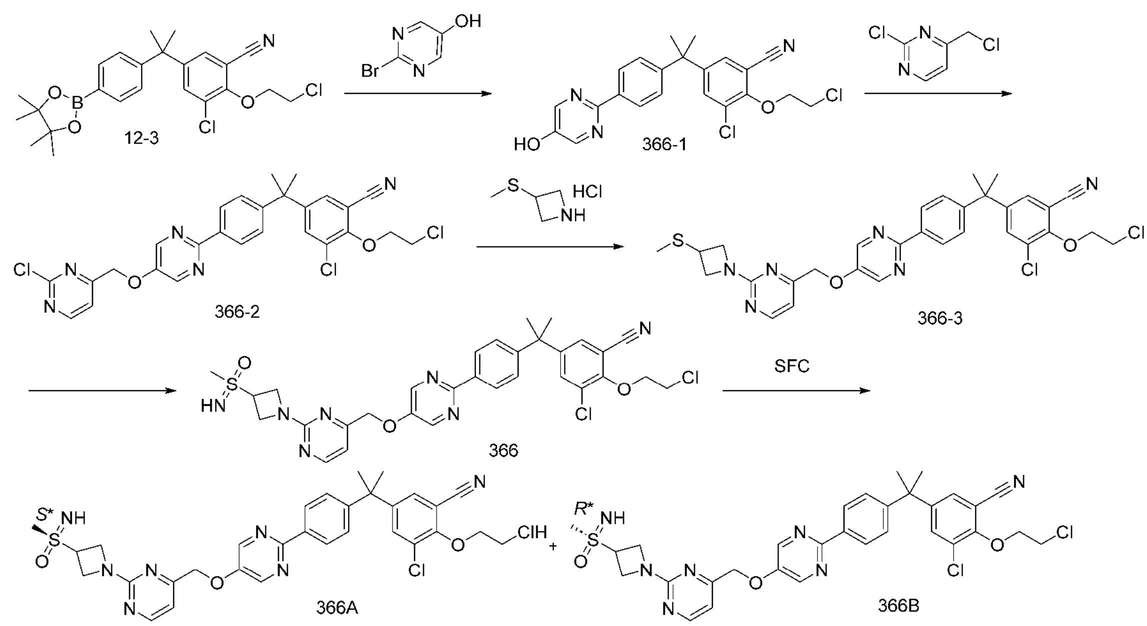


12-3, 366-1, 366-2, 366-3, 366, 366A, 366B

**Preparation of compound 366-1**

Compound **12-3** (2 g, 4.35 mmol), 2-bromo-5-hydroxypyrimidine (1.14 g, 6.52 mmol), Pd(dppf)$Cl_2$ (320 mg, 0.43 mmol), potassium carbonate (1.2 g, 8.7 mmol), 1,4-dioxane (50 mL), and water (5 mL) were added to an eggplant-shaped flask. The reaction system was purged with argon and reacted at 110°C for 16 h. The reaction mixture was cooled to room temperature. Water (20 mL) was added, and the mixture was extracted with ethyl acetate (20 mL). The organic phase was washed with saturated brine (20 mL), dried over anhydrous sodium sulfate, subjected to suction filtration, and the filtrate was concentrated. The resulting residue was purified by silica gel column chromatography (EA/PE = 0% to 66%) to obtain 700 mg of the title compound **366-1.** LC-MS (ESI): m/z 426.0 $[M-H]^-$.

**Preparation of compound 366-2**

Compound **366-1** (400 mg, 0.93 mmol), 2-chloro-4-(chloromethyl)pyrimidine (150 mg, 0.93 mmol), cesium carbonate (610 mg, 1.86 mmol), and acetonitrile (4 mL) were added to an eggplant-shaped flask. The system was reacted at 70°C for 2 h. The reaction mixture was subjected to rotary evaporation until dryness. Water (10 mL) was added, and the mixture was extracted with ethyl acetate (10 mL). The organic phase was washed with saturated brine (10 mL), dried over anhydrous sodium sulfate, subjected to suction filtration, and the filtrate was concentrated. The resulting residue was purified by silica gel column chromatography (EA/PE = 0% to 65%) to obtain 140 mg of the title compound **366-2.** LC-MS (ESI): m/z 554.1 $[M+H]^+$.

**Preparation of compound 366-3**

Compound **366-2** (140 mg, 0.23 mmol), 3-(methylthio)azetidine hydrochloride (39 mg, 0.28 mmol), triethylamine (70 mg, 0.69 mmol), and ethanol (2 mL) were added to an eggplant-shaped flask, and the system was reacted at 100°C for 1 h. The reaction mixture was subjected to rotary evaporation until dryness. Water (10 mL) was added, and the mixture was extracted with ethyl acetate (10 mL). The organic phase was washed with saturated brine (10 mL), dried over anhydrous sodium sulfate, subjected to suction filtration, and the filtrate was subjected to rotary evaporation until dryness to obtain 130 mg of a crude product of compound **366-3.** LC-MS (ESI): m/z 620.8 $[M+H]^+$.

**Preparation of compound 366**

Compound **366-3** (130 mg, 0.21 mmol) and methanol (3 mL) were added to an eggplant-shaped flask. (Diacetoxyiodo)benzene (270 mg, 0.84 mmol) and ammonium carbamate (41 mg, 0.53 mmol) were added at room temperature, and the mixture was stirred at room temperature for 16 h. The reaction was monitored by LCMS until completion. The reaction mixture was concentrated, and the residue was subjected to preparative purification (Waters 2767/Qda, column: XBridge C18 19 × 250 mm, 10 μm; mobile phase: A: 0.1%FA/$H_2O$, B: CAN; flow rate: 20 mL/min; elution gradient: 65% to 65%; retention time: 8.5-9.9 min of 16 min) to obtain 34.6 mg of the title compound **366.** LC-MS (ESI): m/z 652.2 $[M+H]^+$. $^1H$ NMR (400 MHz, DMSO-$d_6$) δ 8.72 (s, 2H), 8.44 (d, $J$ = 5.0 Hz, 1H), 8.24 (d, $J$ = 8.5 Hz, 2H), 7.69 (d, $J$ = 2.3 Hz, 1H), 7.62 (d, $J$ = 2.3 Hz, 1H), 7.38 (d, $J$ = 8.5 Hz, 2H), 6.90 (d, $J$ = 5.0 Hz, 1H), 5.26 (s, 2H), 4.42 (t, $J$ = 5.2 Hz, 2H), 4.32 - 4.15 (m, 5H), 4.01 - 3.92 (m, 3H), 2.90 (s, 3H), 1.70 (s, 6H).

**Preparation of compounds 366A and 366B**

Compound **366** (27 mg) was purified by preparative SFC (preparative conditions: instrument model: Waters SFC15; column model: CHIRAL ART Cellulose-C 250 * 20 mm I.D.S-5 μm; mobile phase: A: $CO_2$, B: isopropanol (50% acetonitrile); elution gradient: B 50%; flow rate: 15 mL/min; column pressure: 100 bar; column temperature: 40°C; detection wavelength: 214 nm; cycle time: 24 min.) to obtain 12.97 mg of the title compound 366A and 13.60 mg of the title compound **366B.**

Compound **366A:** SFC analysis method (instrument model: Waters SFC15; column model: CHIRALCEL OD-H 4.6 mm I.D. *150 mmL 5 μm; mobile phase: A: $CO_2$, B: isopropanol (50% acetonitrile); elution gradient: B 40%; flow rate: 3 mL/min; column pressure: 100 bar; column temperature: 40°C; detection wavelength: 214 nm; Rt = 6.40 min). LC-MS (ESI): m/z 652.0 $[M+H]^+$. $^1H$ NMR (400 MHz, DMSO-$d_6$) δ 8.72 (s, 2H), 8.44 (d, $J$ = 5.0 Hz, 1H), 8.24 (d, $J$ = 8.1 Hz, 2H), 7.70 (d, $J$ = 2.2 Hz, 1H), 7.63 (d, $J$ = 2.3 Hz, 1H), 7.38 (d, $J$ = 8.2 Hz, 2H), 6.90 (d, $J$ = 5.0 Hz, 1H), 5.26 (s, 2H), 4.42 (t, $J$ = 5.2 Hz, 2H), 4.33 - 4.16 (m, 5H), 3.99 (s, 1H), 3.96 (t, $J$ = 5.1 Hz, 2H), 2.90 (s, 3H), 1.70 (s, 6H).

Compound **366B:** SFC analysis method (instrument model: Waters SFC15; column model: CHIRALCEL OD-H 4.6 mm I.D. *150 mmL 5 μm; mobile phase: A: $CO_2$, B: isopropanol (50% acetonitrile); elution gradient: B 40%; flow rate: 3 mL/min; column pressure: 100 bar; column temperature: 40°C; detection wavelength: 214 nm; Rt = 7.60 min). LC-MS (ESI): m/z 652.0 $[M+H]^+$. $^1H$ NMR (400 MHz, DMSO-$d_6$) δ 8.72 (s, 2H), 8.44 (d, $J$ = 5.0 Hz, 1H), 8.24 (d, $J$ = 8.5 Hz, 2H), 7.70 (d, $J$ = 2.3 Hz, 1H), 7.63 (d, $J$ = 2.4 Hz, 1H), 7.38 (d, $J$ = 8.6 Hz, 2H), 6.90 (d, $J$ = 5.0 Hz, 1H), 5.26 (s, 2H), 4.42 (t, $J$ = 5.2 Hz, 2H), 4.24 (d, $J$ = 21.8 Hz, 5H), 3.99 (s, 1H), 3.96 (t, $J$ = 5.2 Hz, 2H), 2.90 (s, 3H), 1.70 (s, 6H).

**Example 367: Preparation of compound 367**

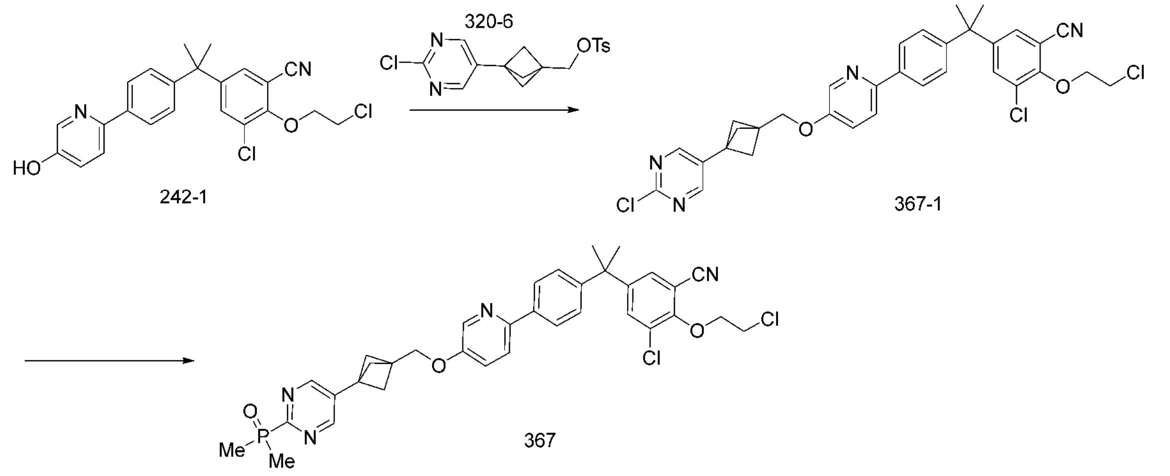


242-1

367-1

367

**Preparation of compound 367-1**

Compound **242-1** (50 mg, 0.14 mmol) and acetonitrile (2 mL) were added to an eggplant-shaped flask. **320-6** (66 mg, 0.15 mmol) and cesium carbonate (91 mg, 0.32 mmol) were added at room temperature, and the mixture was stirred at 70°C for 2 h. The reaction was monitored by TLC until completion. The reaction mixture was subjected to rotary evaporation until dryness and then purified by silica gel column chromatography (EA/PE = 0 to 50%) to obtain 24 mg of the title compound **367-1.** LC-MS (ESI): m/z 619.1 $[M+H]^+$.

**Preparation of compound 367**

Compound **367-1** (24 mg, 0.039 mmol) and N,N-dimethylformamide (2 mL) were added to an eggplant-shaped flask. Dimethylphosphine oxide (12 mg, 0.16 mmol), palladium acetate (2 mg, 0.0078 mmol), 1,3-bis(diphenylphosphino) propane (3 mg, 0.0059 mmol), and N,N-diisopropylethylamine (25 mg, 0.20 mmol) were added at room temperature. After purging with argon for 30 seconds, the mixture was stirred under microwave irradiation at 120°C for 0.5 h. The reaction was monitored by LCMS until completion. The reaction mixture was subjected to preparative purification (Waters 2767/Qda: column: Atalatis T3 prep OBD C18 19 × 250 mm, 10 μm; mobile phase: A: 0.1% FA/$H_2O$, B: CAN; flow rate: 20 mL/min; elution gradient: 72% to 77%; retention time: 8.4-9.2 min of 16 min) to obtain 4.47 mg of the title compound **367.** LC-MS (ESI): m/z 661.3 $[M+H]^+$. $^1H$ NMR (400 MHz, Methanol-$d_4$) δ 8.85 (s, 2H), 8.31 (d, *J* = 2.9 Hz, 1H), 7.84 (d, *J* = 8.5 Hz, 2H), 7.78 (d, *J* = 8.8 Hz, 1H), 7.56 (d, *J* = 2.3 Hz, 1H), 7.53 (d, *J* = 2.3 Hz, 1H), 7.50 (dd, *J* = 8.8, 3.0 Hz, 1H), 7.35 (d, *J* = 8.5 Hz, 2H), 4.43 (t, *J* = 5.6 Hz, 2H), 4.25 (s, 2H), 3.90 (t, *J* = 5.5 Hz, 2H), 2.28 (s, 6H), 1.89 (d, *J* = 13.8 Hz, 6H), 1.73 (s, 6H). $^{31}P$ NMR (162 MHz, Methanol-$d_4$) δ 40.81 (s,1P).

**Example 368: Preparation of compound 368**

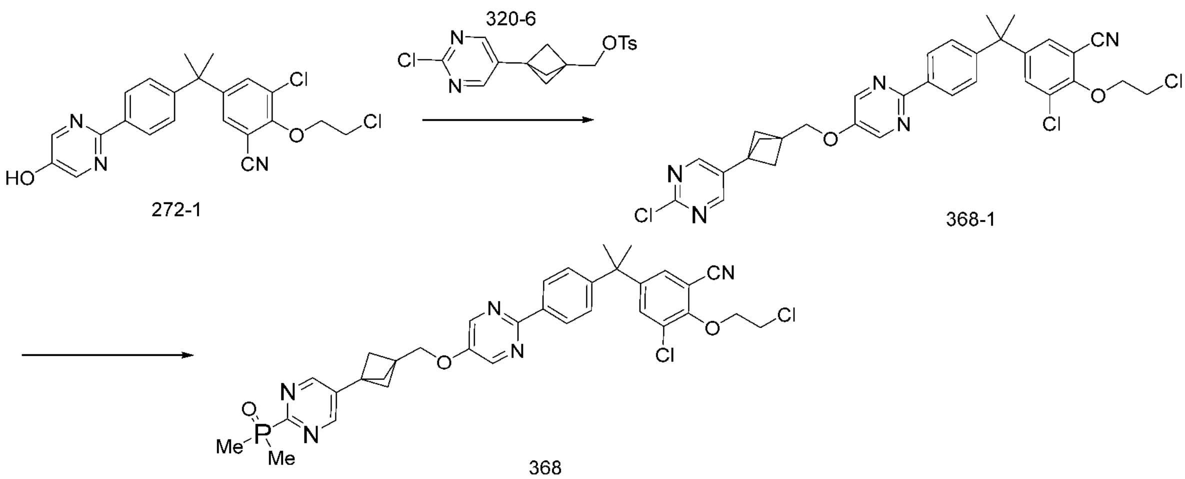


272-1

368-1

368

**Preparation of compound 368-1**

Compound **272-1** (60 mg, 0.16 mmol) and acetonitrile (2 mL) were added to an eggplant-shaped flask. **320-6** (75 mg, 0.18 mmol) and cesium carbonate (100 mg, 0.32 mmol) were added at room temperature, and the mixture was stirred at 70°C for 2 h. The reaction was monitored by TLC until completion. The reaction mixture was subjected to rotary evaporation until dryness, and the resulting residue was purified by silica gel column chromatography (EA/PE = 0 to 50%) to obtain 26 mg of the title compound **368-1.** LC-MS (ESI): m/z 620.1 $[M+H]^+$.

**Preparation of compound 368**

Compound **368-1** (26 mg, 0.042 mmol) and N,N-dimethylformamide (2 mL) were added to an eggplant-shaped flask. Dimethylphosphine oxide (13 mg, 0.17 mmol), palladium acetate (2 mg, 0.0084 mmol), 1,3-bis(diphenylphosphino) propane (3 mg, 0.0063 mmol), and N,N-diisopropylethylamine (27 mg, 0.21 mmol) were added at room temperature. After purging with argon for 30 seconds, the mixture was stirred under microwave irradiation at 120 for 0.5 h. The reaction was monitored by LCMS until completion. The reaction mixture was subjected to preparative purification (Waters 2767/Qda: column: Atalatis T3 prep OBD C18 19 × 250 mm, 10 μm; mobile phase: A: 0.1% FA/$H_2O$, B: ACN; flow rate: 20 mL/min; elution gradient: 75% to 80%; retention time: 7.4-8.6 min of 16 min) to obtain 6.97 mg of the title compound **368.** LC-MS (ESI): m/z 662.2 $[M+H]^+$. $^1H$ NMR (400 MHz, Methanol-$d_4$) δ 8.85 (s, 2H), 8.57 (s, 2H), 8.26 (d, *J* = 8.5 Hz, 2H), 7.56 (d, *J* = 2.3 Hz, 1H), 7.54 (d, *J* = 2.3 Hz, 1H), 7.36 (d, *J* = 8.5 Hz, 2H), 4.43 (t, *J* = 5.5 Hz, 2H), 4.33 (s, 2H), 3.90 (t, *J* = 5.5 Hz, 2H), 2.29 (s, 6H), 1.89 (d, *J* = 13.8 Hz, 6H), 1.73 (s, 6H). $^{31}P$ NMR (162 MHz, Methanol-$d_4$) δ 40.81 (s, 1P).

**Example 369: Preparation of compound 369**

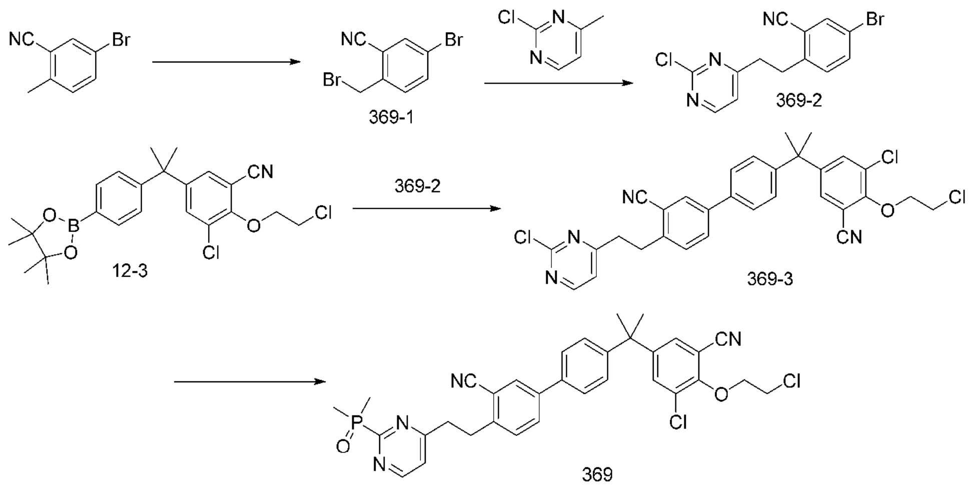

369-1
369-2
12-3
369-3
369

**Preparation of compound 369-1**

2-Methyl-5-bromobenzonitrile (3 g, 15.30 mmol), NBS (2.86 g, 16.07 mmol), AIBN (251 mg, 1.53 mmol) and $CCl_4$ (30 mL) were added to a reaction flask, and the mixture was stirred at 80°C for 16 h. Water (50 mL) was added, and the mixture was extracted three times with DCM (50 mL). The organic phases were combined, dried over anhydrous sodium sulfate, filtered, and subjected to rotary evaporation until dryness. The resulting crude product was purified by silica gel column chromatography (EA/PE = 0 to 20%) to obtain 2.5 g of the title compound **369-1.** $^1H$ NMR (400 MHz, Chloroform-d) δ 7.80 (d, *J* = 2.1 Hz, 1H), 7.72 (dd, *J* = 8.4, 2.1 Hz, 1H), 7.43 (d, *J*= 8.4 Hz, 1H), 4.59 (s, 2H).

**Preparation of compound 369-2**

Under a nitrogen atmosphere, 2-chloro-4-methylpyrimidine (1 g, 7.78 mmol) and THF (10 mL) were added to a reaction flask, and LiHMDS (1 M, 8.56 mmol, 8.6 mL) was added dropwise at -78°C. After stirring for 30 min, a solution of compound **369-1** (2.14 g, 7.78 mmol) in THF (10 mL) was added dropwise to the reaction flask. The temperature was slowly raised to room temperature, and the mixture was stirred for 1 h. The reaction was quenched with water (20 mL) and extracted three times with dichloromethane (50 mL). The organic phases were combined and subjected to rotary

evaporation until dryness. The crude product was purified by preparative liquid chromatography to obtain 500 mg of the title compound **369-2.** $^1$H NMR (400 MHz, Chloroform-d) δ 8.51 (d, *J* = 5.0 Hz, 1H), 7.76 (d, *J* = 2.1 Hz, 1H), 7.63 (dd, *J* = 8.4, 2.1 Hz, 1H), 7.20 (d, *J* = 8.3 Hz, 1H), 7.11 (d, *J* = 5.1 Hz, 1H), 3.27 (t, *J*= 8.0 Hz, 2H), 3.12 (t, *J* = 8.0 Hz, 2H).

**Preparation of compound 369-3**

Under a nitrogen atmosphere, compound **369-2** (350 mg, 1.08 mmol), compound **12-3** (500 mg, 1.08 mmol), [1,1'-bis(diphenylphosphino)ferrocene]dichloropalladium(II) dichloromethane complex (80 mg, 108.65 μmol), $K_2CO_3$ (451 mg, 3.26 mmol), 1,2-dichloroethane (5 mL) and $H_2O$ (1 mL) were added to a reaction flask. The system was purged three times with nitrogen, and the reaction was carried out under microwave irradiation at 90°C for 1 h. The reaction mixture was subjected to rotary evaporation until dryness, and the resulting crude product was purified by silica gel column chromatography (PE/EA = 100% to 20%) to obtain 400 mg of the title compound **369-3.** LC-MS (ESI): m/z 575.2 [M+H]$^+$. $^1$H NMR (400 MHz, Chloroform-d) δ 8.51 (d, *J* = 5.0 Hz, 1H), 7.84 (d, *J* = 2.0 Hz, 1H), 7.71 (dd, *J* = 8.1, 2.0 Hz, 1H), 7.49 (d, *J* = 8.5 Hz, 2H), 7.48 (d, *J* = 2.4 Hz, 1H), 7.37 (d, *J* = 8.1 Hz, 1H), 7.35 (d, *J* = 2.3 Hz, 1H), 7.28 (d, *J* = 8.4 Hz, 2H), 7.14 (d, *J* = 5.0 Hz, 1H), 4.43 (t, *J* = 6.1 Hz, 2H), 3.88 (t, *J* = 6.1 Hz, 2H), 3.34 (dd, *J* = 9.2, 6.4 Hz, 2H), 3.18 (dd, *J* = 9.2, 6.5 Hz, 2H), 1.70 (s, 6H).

**Preparation of compound 369**

Compound **369-3** (100 mg, 173.64 μmol) was dissolved in DMF (1 mL), followed by the sequential addition of dimethylphosphine oxide (41 mg, 520.91 μmol), DIEA (68 mg, 520.91 μmol, 90.73 μL), tris(dibenzylideneacetone) dipalladium (16 mg, 17.36 μmol), and Xantphos (20 mg, 34.73 μmol). The system was purged three times with nitrogen, and then the reaction was carried out under microwave irradiation at 120°C for 2 h. The mixture was filtered and subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 85% acetonitrile gradient elution over 25 min; flow rate: 30 mL/min) to obtain 30 mg of the title compound **369.** LC-MS (ESI): m/z 617.2 [M+H]$^+$. $^1$H NMR (400 MHz, DMSO-$d_6$) δ 8.84 (d, *J* = 5.2 Hz, 1H), 8.08 (d, *J* = 2.0 Hz, 1H), 7.92 (dd, *J* = 8.2, 2.1 Hz, 1H), 7.71 (d, *J* = 2.3 Hz, 1H), 7.67 (d, *J* = 8.5 Hz, 2H), 7.64 (d, *J* = 2.3 Hz, 1H), 7.55 (d, *J* = 8.3 Hz, 1H), 7.53 (dd, *J* = 5.2, 3.2 Hz, 1H), 7.35 (d, *J* = 8.5 Hz, 2H), 4.42 (t, *J*= *5.2* Hz, 2H), 3.96 (t, *J* = 5.2 Hz, 2H), 3.33 - 3.26 (m, 2H), 3.27 - 3.20 (m, 2H), 1.70 (d, *J* = 13.7 Hz, 6H), 1.69 (s, 6H). $^{31}$P NMR (162 MHz, DMSO-$d_6$) δ 33.89 (s, 1P).

**Example 370: Preparation of compound 370**

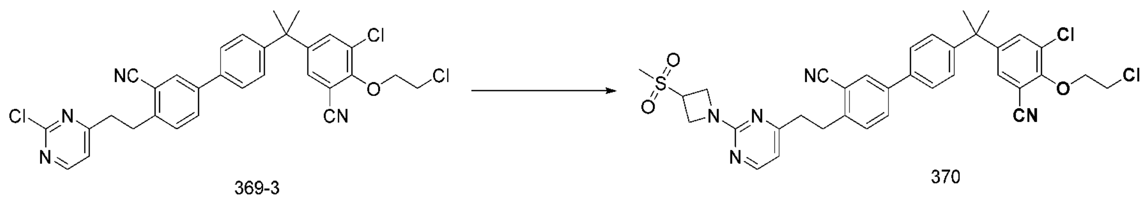

369-3

370

**Preparation of compound 370**

Compound **369-3** (50 mg, 86.82 μmol), 3-(methylsulfonyl)azetidine hydrochloride (22.35 mg, 130.23 μmol), DIPEA (34 mg, 260.46 μmol), and acetonitrile (1 mL)were added to a reaction flask, and the mixture was stirred under microwave irradiation at 80°C for 2 h. The reaction mixture was subjected to rotary evaporation until dryness, and the resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 95% acetonitrile gradient elution over 25 min; flow rate: 30 mL/min) to obtain 20 mg of the title compound **370.** LC-MS (ESI): m/z 673.8 [M+H]$^+$. $^1$H NMR (400 MHz, DMSO-$d_6$) δ 8.27 (d, *J* = 5.0 Hz, 1H), 8.08 (d, *J* = 2.0 Hz, 1H), 7.91 (dd, *J* = 8.2, 2.1 Hz, 1H), 7.71 (d, *J* = 2.3 Hz, 1H), 7.68 (d, *J* = 8.5 Hz, 2H), 7.64 (d, *J* = 2.4 Hz, 1H), 7.55 (d, *J* = 8.2 Hz, 1H), 7.35 (d, *J* = 8.6 Hz, 2H), 6.67 (d, *J* = 5.0 Hz, 1H), 4.42 (t, *J*= 5.2 Hz, 2H), 4.40 - 4.35 (m, 1H), 4.30 (t, *J* = 8.8 Hz, 2H), 4.24 - 4.16 (m, 2H), 3.96 (t, *J* = 5.2 Hz, 2H), 3.24 - 3.18 (m, 2H), 3.04 (s, 3H), 2.99 (t, *J* = 7.6 Hz, 2H), 1.69 (s, 6H).

**Example 371: Preparation of compound 371**

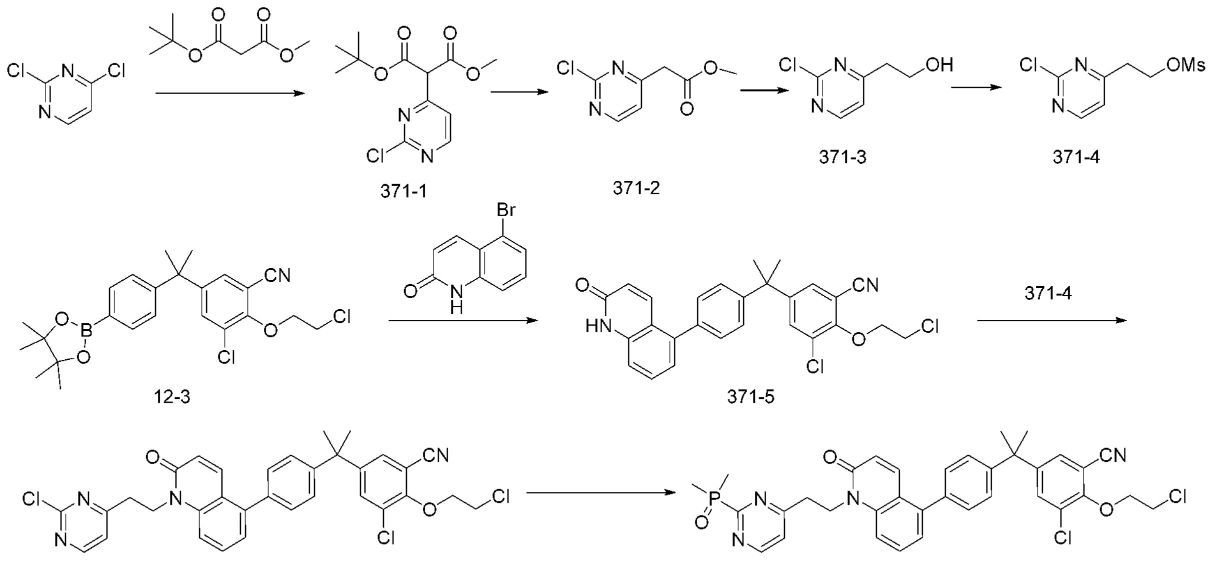


371-1 371-2 371-3 371-4 12-3 371-5 371-6 371

**Preparation of compound 371-1**

Under a nitrogen atmosphere, *tert*-butyl methyl malonate (25.3 g, 144.99 mmol) was dissolved in anhydrous tetrahydrofuran (200 mL), and the mixture was cooled to 0°C. Sodium hydride (6.44 g, 161.10 mmol, 60% by mass) was added slowly. After the addition was completed, the mixture was stirred for 30 min, then stirred at room temperature for 1 h. Subsequently, 2,4-dichloropyrimidine (12 g, 80.55 mmol) was added. After the addition was completed, the temperature was raised to 70°C and the mixture was stirred for 3 h. The reaction was monitored by LCMS until completion. The reaction mixture was cooled to 0°C and quenched with ammonium chloride solution (100 mL). The mixture was extracted three times with ethyl acetate (150 mL). The organic phases were combined, dried over anhydrous sodium sulfate, subjected to suction filtration, and the filtrate was concentrated to obtain 30 g of a crude product of the title compound **371-1.** LC-MS (ESI): m/z 287.1 $[M+H]^+$.

**Preparation of compound 371-2**

Compound **371-1** (25 g, 87.20 mmol) was dissolved in DCM (200 mL). The system was purged with nitrogen, cooled to 0°C, and trifluoroacetic acid (65 mL, 872.00 mmol) was slowly added dropwise. After the addition was completed, the mixture was stirred at room temperature for 1 h. The reaction was monitored by LCMS until completion. The reaction was quenched with saturated sodium bicarbonate solution (300 mL). The mixture was extracted three times with DCM (300 mL). The organic phases were combined, dried over anhydrous sodium sulfate, subjected to suction filtration, and the filtrate was concentrated. The resulting residue was purified by silica gel column chromatography (PE/EA = 100% to 60%) to obtain 12 g of the title compound **371-2.** LC-MS (ESI): m/z 187.1 $[M+H]^+$.

**Preparation of compound 371-3**

Compound **371-2** (5.5 g, 29.47 mmol) was dissolved in anhydrous THF (100 mL). The system was purged with nitrogen and cooled to 0°C. Diisobutylaluminum hydride (1.5 mol/L in toluene, 49 mL, 73.67 mmol) was slowly added dropwise. After the addition was completed, the mixture was stirred at 0°C for 1 h. The reaction was monitored by LCMS until completion. The mixture was cooled to -10°C, and methanol (50 mL) was added dropwise to quench the reaction. The pH was adjusted to approximately 3 with 1 mol/L dilute hydrochloric acid. The mixture was extracted three times with ethyl acetate (300 mL). The organic phases were combined, dried over anhydrous sodium sulfate, subjected to suction filtration, and the filtrate was concentrated. The resulting residue was purified by silica gel column chromatography (PE/EA = 100% to 60%) to obtain 2 g of the title compound **371-3.** LC-MS (ESI): m/z 159.1 $[M+H]^+$.

**Preparation of compound 371-4**

Compound **371-3** (1 g, 6.31 mmol) was dissolved in DCM (20 mL). The system was purged with nitrogen and cooled to 0°C. Triethylamine (2.62 mL, 18.92 mmol) was added, and methanesulfonyl chloride (0.73 mL, 9.46 mmol) was slowly added dropwise. After the addition was completed, the mixture was stirred at 0°C for 1 h. The reaction was monitored by LCMS until completion. The reaction was quenched with ice water (20 mL) and extracted three times with ethyl acetate (50 mL). The organic phases were combined, dried over anhydrous sodium sulfate, subjected to suction filtration, and the filtrate was concentrated. The resulting residue was purified by silica gel column chromatography (PE/EA = 100% to 50%) to obtain 500 mg of the title compound **371-4.** LC-MS (ESI): m/z 237.0 $[M+H]^+$.

**Preparation of compound 371-5**

Compound **12-3** (500 mg, 1.1 mmol), 5-bromoquinolin-2(1H)-one (246 mg, 1.1 mmol), Pd(dppf)$Cl_2$ (80 mg, 0.11 mmol), potassium carbonate (456 mg, 3.3 mmol), 1,4-dioxane (2 mL), and water (1 mL) were added to an eggplant-shaped flask. The system was purged with argon. The system was reacted at 100°C for 2 h. The reaction mixture was subjected to rotary evaporation until dryness. Water (10 mL) was added, and the mixture was extracted with ethyl acetate (10 mL). The organic phase was washed with saturated brine (20 mL), dried over anhydrous sodium sulfate, subjected to suction filtration, and the filtrate was concentrated. The resulting residue was purified by silica gel column chromatography (PE/EA = 0% to 50%) to obtain 230 mg of the title compound **371-5.** LC-MS (ESI): m/z 477.1 $[M+H]^+$.

**Preparation of compound 371-6**

Compound **371-5** (200 mg, 0.43 mmol), compound **371-4** (101 mg, 0.43 mmol), cesium carbonate (280 mg, 0.86 mmol), and DMF (5 mL) were added to an eggplant-shaped flask. The system was reacted at 60°C for 2 h. Water (10 mL) was added, and the mixture was extracted with ethyl acetate (10 mL). The organic phase was washed with saturated brine (20 mL), concentrated, and the resulting residue was purified by silica gel column chromatography (PE/EA = 0% to 40%) to obtain 50 mg of the title compound **371-6.** LC-MS (ESI): m/z 617.1 $[M+H]^+$.

**Preparation of compound 371**

Compound **371-6** (50 mg, 0.081 mmol) and *N,N*-dimethylformamide (2 mL) were added to a microwave tube. Dimethylphosphine oxide (25 mg, 0.32 mmol), palladium acetate (4 mg, 0.016 mmol), 1,3-bis(diphenylphosphino) propane (5 mg, 0.012 mmol), and *N,N*-diisopropylethylamine (52 mg, 0.41 mmol) were added at room temperature, and the system was purged with argon for 30 seconds. The reaction was then stirred under microwave irradiation at 120°C for 0.5 h. The reaction was monitored by LCMS until completion. The reaction mixture was purified by preparative liquid chromatography (Waters 2767/Qda, Column: Atalatis T3 prep OBD C18 19 × 250 mm, 10 μm; mobile phase: A: 0.03% $NH_3H_2O/H_2O$, B: ACN; flow rate: 20 mL/min; elution gradient: 79% to 79%; retention time: 9.11-10.35 min of 16 min) to obtain 8.19 mg of the title compound **371.** LC-MS (ESI): m/z $[M+H]^+$: 659.2. $^1H$ NMR (400 MHz, DMSO-$d_6$) δ 8.86 (d, *J* = 5.2 Hz, 1H), 7.77 (d, *J* = 2.3 Hz, 1H), 7.74 - 7.61 (m, 5H), 7.41 (d, *J* = 8.4 Hz, 2H), 7.36 (d, *J* = 8.4 Hz, 2H), 7.20 (dd, *J* = 6.7, 1.3 Hz, 1H), 6.57 (d, *J* = 9.8 Hz, 1H), 4.71 (t, *J* = 7.4 Hz, 2H), 4.44 (t, *J* = 5.2 Hz, 2H), 3.97 (t, *J* = 5.2 Hz, 2H), 3.21 (t, *J* = 7.3 Hz, 2H), 1.73 (s, 6H), 1.69 (d, *J* = 13.7 Hz, 6H). $^{31}P$ NMR (162 MHz, DMSO-$d_6$) δ 33.65 (s, 1P).

**Example 372: Preparation of compound 372**

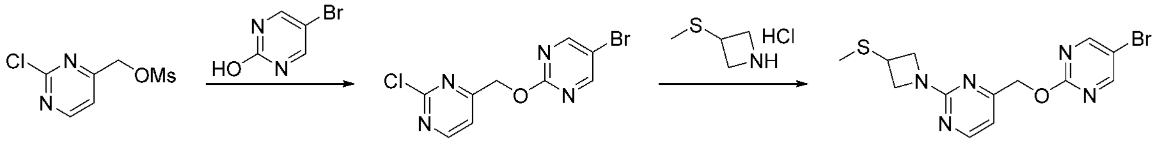

372-1

372-2

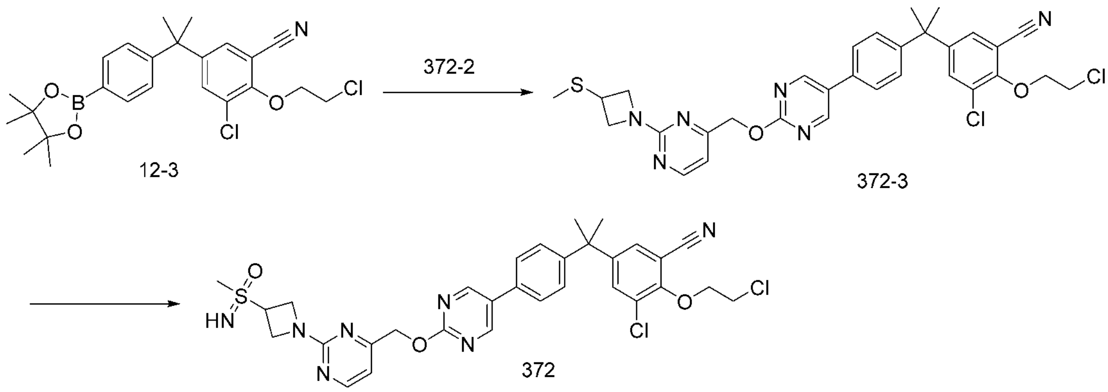


12-3 372-3 372

### Preparation of compound 372-1

(2-Chloropyrimidin-4-yl)methyl methanesulfonate (1.0 g, 5.71 mmol), 2-hydroxy-5-bromopyrimidine (1.27 g, 5.71 mmol), cesium carbonate (3.72 g, 11.42 mmol), and acetonitrile (10 mL) were added to an eggplant-shaped flask. The system was reacted at 70°C for 2 h. The reaction mixture was filtered and concentrated. The resulting residue was purified by silica gel column chromatography (EA/PE = 0% to 45%) to obtain 140 mg of the title compound 372-1. $^1$H NMR (400 MHz, $CDCl_3$) δ 8.63 (d, *J* = 5.1 Hz, 1H), 8.58 (s, 2H), 7.46 (d, *J* = 5.0 Hz, 1H), 5.50 (s, 2H).

### Preparation of compound 372-2

Compound **372-1** (140 mg, 0.46 mmol), 3-(methylthio)azetidine hydrochloride (77 mg, 0.55 mmol), triethylamine (140 mg, 1.38 mmol), and ethanol (2 mL) were added to an eggplant-shaped flask, and the system was reacted at 100°C for 2 h. The reaction mixture was concentrated, and the resulting residue was purified by silica gel column chromatography (PE/EA = 0% to 66%) to obtain 120 mg of the title compound **372-2.** LC-MS (ESI): m/z 368.0 $[M+H]^+$.

### Preparation of compound 372-3

Compound **12-3** (150 mg, 0.41 mmol), compound **372-2** (190 mg, 0.41 mmol), $Pd(dppf)Cl_2$ (30 mg, 0.041 mmol), potassium carbonate (110 mg, 0.82 mmol), 1,4-dioxane (2 mL), and water (1 mL) were added to an eggplant-shaped flask. The system was reacted at 100°C for 1 h. The reaction mixture was subjected to rotary evaporation until dryness. Water (10 mL) was added, and the mixture was extracted with ethyl acetate (10 mL). The organic phase was washed with saturated brine (20 mL), dried over anhydrous sodium sulfate, subjected to suction filtration, and concentrated. The resulting residue was purified by silica gel column chromatography (PE/EA = 0% to 44%) to obtain 150 mg of the title compound **372-3** in 59% yield. LC-MS (ESI): m/z 621.3 $[M+H]^+$.

### Preparation of compound 372

Compound **372-3** (150 mg, 0.24 mmol) was dissolved in methanol (2 mL). (Diacetoxyiodo)benzene (310 mg, 0.96 mmol) and ammonium carbamate (47 mg, 0.6 mmol) were added at room temperature, and the mixture was stirred at room temperature for 16 h. The reaction was monitored by LCMS until completion. The reaction mixture was concentrated, and the resulting residue was purified by preparative liquid chromatography (Waters 2767/Qda: column: XBridge C18 19 $\times$ 250 mm, 10 $\mu$m; mobile phase: A: 0.1% FA/$H_2O$; B: ACN; flow rate: 20 mL/min; elution gradient: 61% to 63%; retention time: 7.6-8.6 min of 16 min) to obtain 48.4 mg of the title compound **372.** LC-MS (ESI): m/z 652.2 $[M+H]^+$. $^1$H NMR (400 MHz, DMSO-$d_6$) δ 8.94 (s, 2H), 8.36 (d, *J* = 5.0 Hz, 1H), 7.70 (d, *J* = 2.3 Hz, 1H), 7.67 (d, *J* = 8.3 Hz, 2H), 7.64 (d, *J* = 2.3 Hz, 1H), 7.37 (d, *J* = 8.4 Hz, 2H), 6.70 (d, *J* = 5.0 Hz, 1H), 5.36 (s, 2H), 4.42 (t, *J* = 5.2 Hz, 2H), 4.29 - 4.14 (m, 5H), 3.99 - 3.87 (m, 3H), 2.90 (s, 3H), 1.69 (s, 6H).

### Example 373: Preparation of compound 373

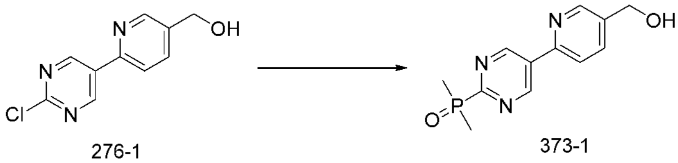

276-1 373-1

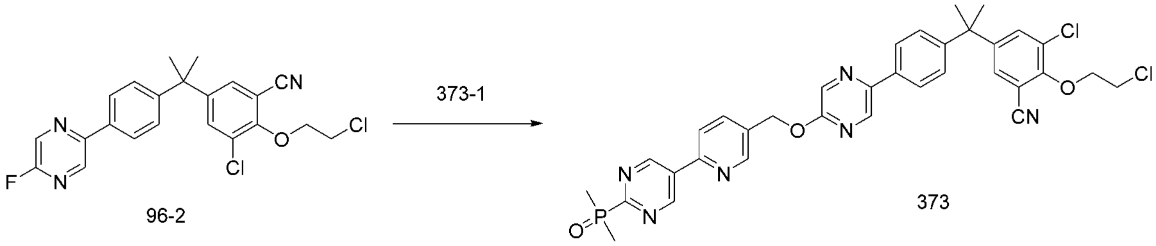


96-2 373

## Preparation of compound 373-1

Compound **276-1** (879.3 mg, 3.97 mmol) was dissolved in DMF (10 mL). Xantphos (459.1 mg, 793.44 μmol), $Pd_2(dba)_3$ (363.3 mg, 396.72 μmol), dimethylphosphine oxide (928.9 mg, 11.90 mmol), and DIPEA (2.57 g, 19.84 mmol, 3.45 mL) were added. The mixture was bubbled with nitrogen for 1 min and stirred under microwave irradiation at 120°C for 2 h in a sealed tube. After the reaction was completed, the reaction mixture was filtered through diatomite, and the filter cake was washed with ethyl acetate and dichloromethane. The filtrate was concentrated to dryness by rotary evaporation. Deionized water (30 mL) was added, and the mixture was extracted with dichloromethane. The organic phase was separated and then back-extracted once with deionized water (10 mL). The aqueous phases were combined and lyophilized to obtain a crude product. The crude product was purified by silica gel column chromatography (DCM/MeOH = 17 : 3) to obtain 545.5 mg of the title compound **373-1** as a crude product. LC-MS (ESI): m/z 264.0 $[M+H]^+$.

## Preparation of compound 373

Compound **96-2** (98 mg, 0.23 mmol) was dissolved in DMF (3 mL), followed by the sequential addition of cesium carbonate (228 mg, 0.68 mmol) and compound **373-1** (60 mg, 0.23 mmol). The reaction system was stirred under microwave irradiation at 80°C for 2 h. After the reaction was completed, insoluble solids were removed by filtration. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **373.** LC-MS (ESI): m/z 673.2 $[M+H]^+$. $^1H$ NMR (400 MHz, DMSO-$d_6$) δ 9.60 (s, 2H), 8.92 (s, 1H), 8.82 (s, 1H), 8.48 (d, *J* = 1.4 Hz, 1H), 8.25 (d, *J* = 8.1 Hz, 1H), 8.14 (dd, *J* = 8.3, 2.3 Hz, 1H), 7.98 (d, *J* = 8.5 Hz, 2H), 7.70 (d, *J* = 2.4 Hz, 1H), 7.64 (d, *J* = 2.3 Hz, 1H), 7.38 (d, *J* = 8.5 Hz, 2H), 5.57 (s, 2H), 4.42 (t, *J* = 5.1 Hz, 2H), 3.96 (t, *J* = 5.1 Hz, 2H), 1.81 (d, *J* = 13.8 Hz, 6H), 1.69 (s, 6H). $^{31}P$ NMR (162 MHz, DMSO-$d_6$) δ 34.91 (s, 1P).

## Example 374: Preparation of compound 374

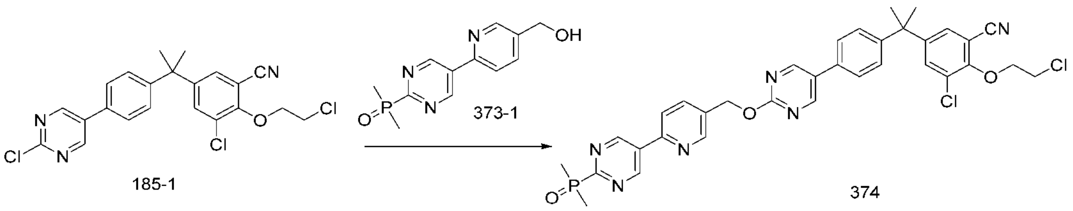

185-1 373-1 374

## Preparation of compound 374

Compound **185-1** (116 mg, 440.68 μmol) was dissolved in DMF (3 mL), and compound 373-1 (157.5 mg, 352.54 μmol) and cesium carbonate (287.2 mg, 881.35 μmol) were added. The reaction mixture was stirred under microwave irradiation at 90°C for 2 h in a sealed tube. After the reaction was completed, the reaction mixture was diluted with a small

amount of 1,4-dioxane and purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 60-80% 8 min, 80-95% 4 min; flow rate: 30 mL/min) to obtain 15 mg of compound **374.** LC-MS (ESI): m/z 673.3 $[M+H]^+$. $^1$H NMR (400 MHz, DMSO-$d_6$) δ 9.61 (s, 2H), 8.97 (s, 2H), 8.91 (d, *J* = 2.0 Hz, 1H), 8.26 (d, *J* = 8.2 Hz, 1H), 8.13 (dd, *J* = 8.2, 2.3 Hz, 1H), 7.74 - 7.65 (m, 3H), 7.65 (d, *J* = 2.4 Hz, 1H), 7.38 (d, *J* = 8.4 Hz, 2H), 5.58 (s, 2H), 4.42 (t, *J* = 5.2 Hz, 2H), 3.96 (t, *J* = 5.2 Hz, 2H), 1.81 (d, *J* = 13.7 Hz, 6H), 1.69 (s, 6H). $^{31}$P NMR (162 MHz, DMSO-$d_6$) δ 34.11 (s, 1P).

**Example 375: Preparation of compound 375**

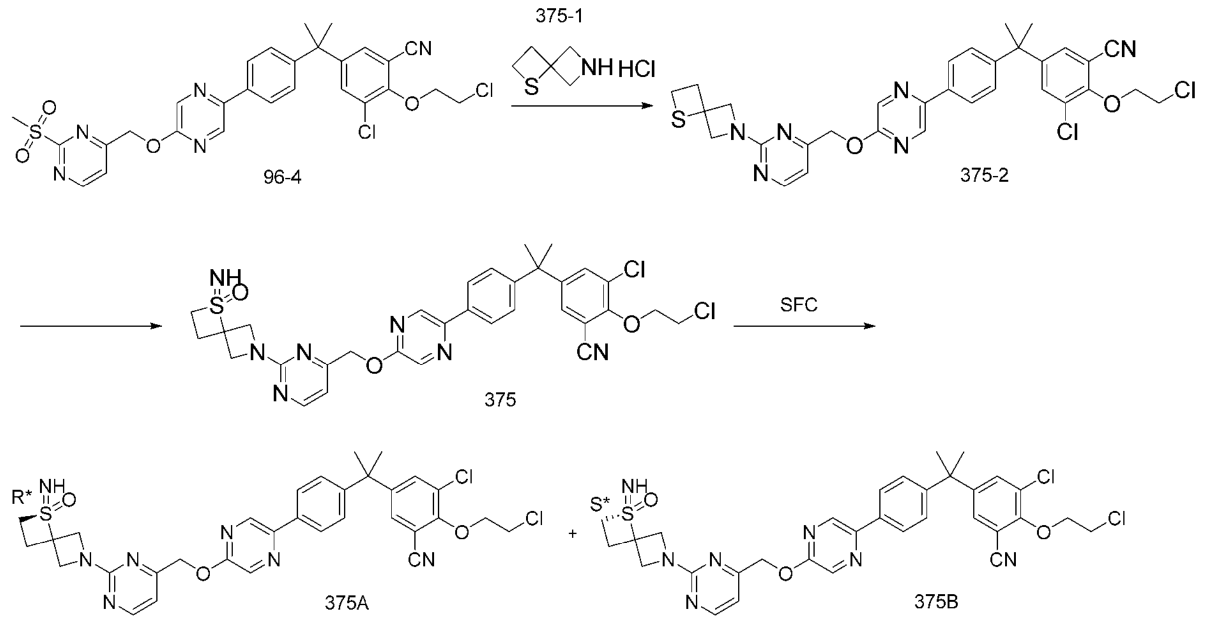


**Preparation of compound 375-2**

Compound **96-4** (80 mg, 0.14 mmol) was added to a microwave tube, followed by the sequential addition of anhydrous ethanol (2 mL), compound **375-1** (30 mg, 0.20 mmol), and DIPEA (52 mg, 0.40 mmol). The system was purged three times with nitrogen, and the reaction was carried out under microwave irradiation at 90°C for 2 h. After the reaction was monitored by LCMS until completion, the reaction mixture was filtered. The crude filtrate was purified by normal-phase silica gel column chromatography (EA/PE = 0 to 60%) to obtain 70 mg of the title compound **375-2** in 83% yield. LC-MS (ESI): m/z 633.2 $[M+H]^+$.

**Preparation of compound 375**

Compound **375-2** (70 mg, 0.11 mmol) and ammonium carbonate (32 mg, 0.33 mmol) were dissolved in methanol (5 mL). (Diacetoxyiodo)benzene (178 mg, 0.55 mmol) was added thereto at 0°C. After the addition was completed, the reaction was carried out at room temperature overnight. After the reaction was monitored the next day by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 26 mg of the title compound **375.** LC-MS (ESI): m/z 664.0 $[M+H]^+$. $^1$H NMR (400 MHz, DMSO-$d_6$) δ 8.75 (d, *J* = 1.4 Hz, 1H), 8.54 (d, *J*= 1.4 Hz, 1H), 8.37 (d, *J* = 5.0 Hz, 1H), 7.96 (d, *J* = 8.5 Hz, 2H), 7.70 (d, *J* = 2.4 Hz, 1H), 7.63 (d, *J* = 2.3 Hz, 1H), 7.37 (d, *J* = 8.6 Hz, 2H), 6.78 (d, *J* = 5.0 Hz, 1H), 5.36 (s, 2H), 4.79 (d, *J* = 1.5 Hz, 1H), 4.49 (dd, *J* = 10.3, 1.4 Hz, 1H), 4.42 (t, *J* = 5.2 Hz, 2H), 4.36 *(d, J*= 10.2 Hz, 1H), 4.23 (d, *J* = 10.3 Hz, 1H), 4.17 (d, *J* = 10.3 Hz, 1H), 3.96 (t, *J* = 5.2 Hz, 2H), 3.93 - 3.78 (m, 2H), 2.44 - 2.31 (m, 2H), 1.69 (s, 6H).

**Preparation of compounds 375A and 375B**

Compound **375** (22 mg) was purified by preparative SFC (preparative conditions: instrument model: WATERS

150 preparative SFC (SFC15); column: CHIRALCEL OX-H/SFC 20 mm I.D.*250 mmL 5 μm; mobile phase: A: $CO_2$, B: isopropanol: acetonitrile = 1:1 (0.05% DEA); elution gradient: B 45%; flow rate: 15 mL/min; column pressure: 100 bar; column temperature: 40°C; detection wavelength: 214 nm; cycle time: 38 min) to obtain 10.2 mg of the title compound **375A** and 9.4 mg of the title compound **375B.**

Compound **375A:** SFC analysis method (instrument model: Waters SFC15; column model: CHIRALCEL OX-3 4.6 mm I.D.*100 mm L 3 μm; mobile phase: A: $CO_2$, B: ethanol: acetonitrile = 1:1 (0.05% DEA); elution gradient: B 35%; flow rate: 3 mL/min; column pressure: 100 bar; column temperature: 40°C; detection wavelength: 214 nm; Rt = 9.90 min). LC-MS (ESI): m/z 664.2 $[M+H]^+$. $^1H$ NMR (400 MHz, DMSO-$d_6$) δ 8.74 (d, $J$ = 1.5 Hz, 1H), 8.53 (d, $J$ = 1.4 Hz, 1H), 8.37 (d, $J$ = 5.0 Hz, 1H), 7.96 (d, $J$ = 8.4 Hz, 2H), 7.70 (d, $J$ = 2.3 Hz, 1H), 7.63 (d, $J$ = 2.3 Hz, 1H), 7.37 (d, $J$ = 8.2 Hz, 2H), 6.78 (d, $J$ = 5.1 Hz, 1H), 5.36 (s, 2H), 4.79 (s, 1H), 4.49 (d, $J$ = 10.2 Hz, 1H), 4.42 (t, $J$ = 5.1 Hz, 2H), 4.38 (d, $J$ = 10.2 Hz, 1H), 4.23 (d, $J$ = 10.2 Hz, 1H), 4.16 (d, $J$ = 10.3 Hz, 1H), 3.95 (t, $J$ = 5.1 Hz, 2H), 3.92 - 3.77 (m, 2H), 2.44 - 2.27 (m, 2H), 1.69 (s, 6H).

Compound **387B:** SFC analysis method (instrument model: Waters SFC15; column model: CHIRALCEL OX-3 4.6 mm I.D.*100 mm L 3 μm; mobile phase: A: $CO_2$, B: ethanol: acetonitrile = 1:1 (0.05% DEA); elution gradient: B 35%; flow rate: 3 mL/min; column pressure: 100 bar; column temperature: 40°C; detection wavelength: 214 nm; Rt = 11.15 min). LC-MS (ESI): m/z 664.2 $[M+H]^+$. $^1H$ NMR (400 MHz, DMSO-$d_6$) δ 8.74 (s, 1H), 8.53 (s, 1H), 8.37 (d, $J$ = 5.1 Hz, 1H), 7.96 (d, $J$ = 8.1 Hz, 2H), 7.70 (d, $J$ = 2.4 Hz, 1H), 7.63 (d, $J$ = 2.3 Hz, 1H), 7.37 (d, $J$ = 8.2 Hz, 2H), 6.78 (d, $J$ = 5.1 Hz, 1H), 5.36 (s, 2H), 4.79 (s, 1H), 4.49 (d, $J$ = 10.3 Hz, 1H), 4.42 (t, $J$ = 5.1 Hz, 2H), 4.37 (d, $J$ = 10.2 Hz, 1H), 4.23 (d, $J$ = 10.3 Hz, 1H), 4.16 (d, $J$ = 10.2 Hz, 1H), 3.95 (t, $J$= 5.1 Hz, 2H), 3.92 - 3.77 (m, 2H), 2.43 - 2.29 (m, 2H), 1.69 (s, 6H).

## Example 376: Preparation of compound 376

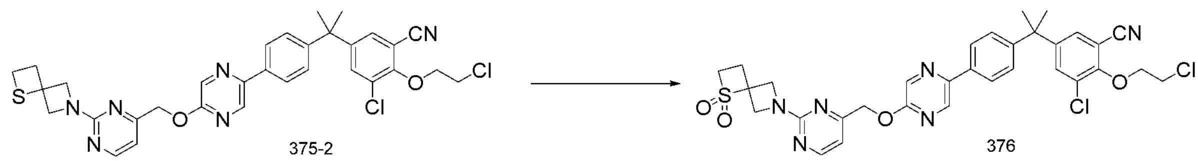

375-2

376

## Preparation of compound 376

Compound **375-2** (70 mg, 0.11 mmol) was weighed and dissolved in dichloromethane (5 mL). m-CPBA (76 mg, 0.44 mmol) was added thereto at 0°C. After the addition was completed, the reaction was carried out at room temperature for 2 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 40 mg of the title compound **376.** LC-MS (ESI): m/z 665.0 $[M+H]^+$. $^1H$ NMR (400 MHz, DMSO-$d_6$) δ 8.74 (d, $J$ = 1.4 Hz, 1H), 8.54 (d, $J$ = 1.4 Hz, 1H), 8.39 (d, $J$ = 5.0 Hz, 1H), 7.96 (d, $J$ = 8.5 Hz, 2H), 7.70 (d, $J$ = 2.3 Hz, 1H), 7.63 (d, $J$ = 2.3 Hz, 1H), 7.37 (d, $J$ = 8.6 Hz, 2H), 6.82 (d, $J$ = 5.0 Hz, 1H), 5.38 (s, 2H), 4.46 - 4.37 (m, 4H), 4.30 (d, $J$ = 10.3 Hz, 2H), 4.17 - 4.07 (m, 2H), 3.95 (t, $J$ = 5.2 Hz, 2H), 2.46 - 2.38 (m, 2H), 1.69 (s, 6H).

## Example 377: Preparation of compound 377

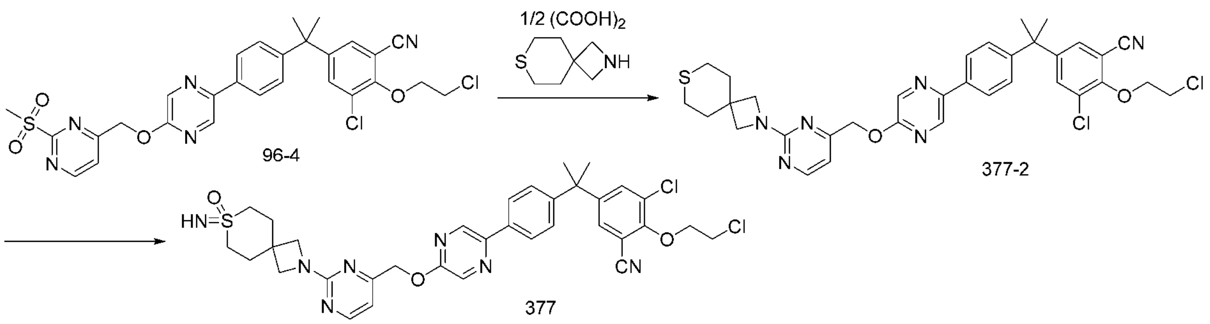


96-4

377-2

377

**Preparation of compound 377-2**

Compound **96-4** (80 mg, 0.14 mmol) was added to a microwave tube, followed by the sequential addition of anhydrous ethanol (2 mL), compound **377-1** (76 mg, 0.20 mmol), and DIPEA (52 mg, 0.40 mmol). The system was purged three times with nitrogen, and the reaction was carried out under microwave irradiation at 90°C for 2 h. After the reaction was monitored by LCMS until completion, the reaction mixture was filtered. The crude filtrate was purified by normal-phase silica gel column chromatography (EA/PE = 0 to 60%) to obtain 80 mg of the title compound **377-2** in 90% yield. LC-MS (ESI): m/z 661.0 $[M+H]^+$.

**Preparation of compound 377**

Compound **377-2** (80 mg, 0.12 mmol) and ammonium carbonate (35 mg, 0.36 mmol) were dissolved in methanol (5 mL). (Diacetoxyiodo)benzene (194 mg, 0.60 mmol) was added thereto at 0°C. After the addition was completed, the reaction was carried out at room temperature overnight. After the reaction was monitored the next day by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 70 mg of the title compound 377. LC-MS (ESI): m/z 692.2 $[M+H]^+$. $^1$H NMR (400 MHz, DMSO-$d_6$) δ 8.74 (d, *J* = 1.4 Hz, 1H), 8.53 (d, *J* = 1.4 Hz, 1H), 8.31 (d, *J* = 5.0 Hz, 1H), 7.96 (d, *J* = 8.6 Hz, 2H), 7.70 (d, *J* = 2.4 Hz, 1H), 7.63 (d, *J* = 2.3 Hz, 1H), 7.37 (d, *J* = 8.6 Hz, 2H), 6.69 (d, *J* = 5.0 Hz, 1H), 5.32 (s, 2H), 4.42 (t, *J* = 5.2 Hz, 2H), 3.96 (t, *J* = 5.2 Hz, 2H), 3.83 (s, 2H), 3.78 (s, 2H), 3.57 (s, 1H), 3.06 - 2.91 (m, 4H), 2.14 (t, *J* = 5.9 Hz, 4H), 1.69 (s, 6H).

**Example 378: Preparation of compound 378**

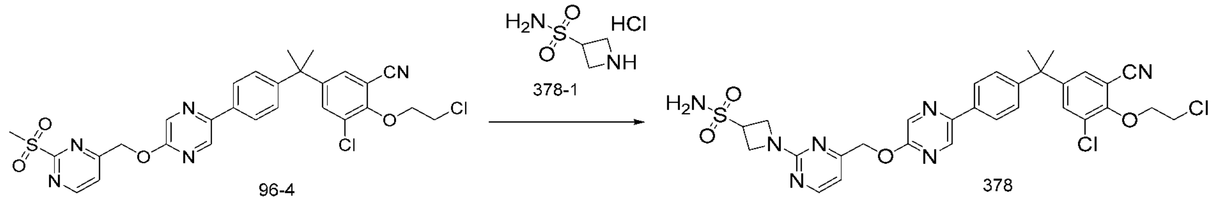


96-4 378

**Preparation of compound 378**

Compound **96-4** (80 mg, 0.13 mmol) was added to a microwave tube, followed by the sequential addition of anhydrous ethanol (2 mL), **378-1** (azetidine-3-sulfonamide hydrochloride, 35 mg, 0.20 mmol), and DIPEA (52 mg, 0.40 mmol). The system was purged three times with nitrogen, and the reaction was carried out under microwave irradiation at 90°C for 2 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 38 mg of the title compound **378.** LC-MS (ESI): m/z 654.0 $[M+H]^+$. $^1$H NMR (400 MHz, DMSO-$d_6$) δ 8.75 (d, *J* = 1.4 Hz, 1H), 8.53 (d, *J* = 1.4 Hz, 1H), 8.36 (d, *J* = 5.1 Hz, 1H), 7.96 (d, *J* = 8.6 Hz, 2H), 7.70 (d, *J* = 2.3 Hz, 1H), 7.63 (d, *J* = 2.4 Hz, 1H), 7.37 (d, *J* = 8.6 Hz, 2H), 7.18 (s, 2H), 6.76 (d, *J* = 5.0 Hz, 1H), 5.36 (s, 2H), 4.42 (t, *J* = 5.2 Hz, 2H), 4.34 - 4.23 (m, 2H), 4.23 - 4.12 (m, 3H), 3.96 (t, *J* = 5.2 Hz, 2H), 1.69 (s, 6H).

**Example 379: Preparation of compound 379**

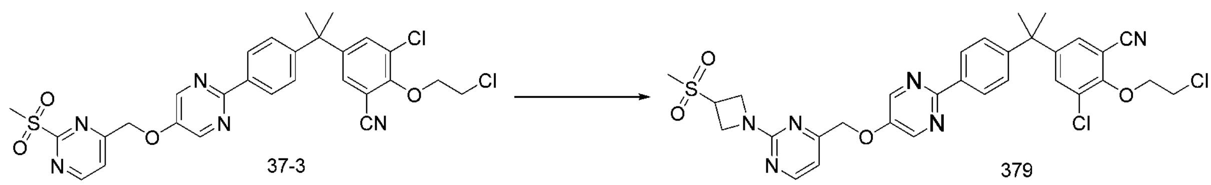

37-3 379

**Preparation of compound 379**

Compound **37-3** (220 mg, 367.59 μmol) and 3-(methylsulfonyl)azetidine hydrochloride (94.6 mg, 551.38 μmol) were dissolved in $CH_3CN$ (2 mL), and TEA (111.6 mg, 1.10 mmol, 154 μL) was added. The reaction mixture was heated at 60°C for 12 h. LCMS indicated the disappearance of the starting material and the formation of the product. The reaction mixture was filtered and then subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 100 mg of the title compound **379.** LC-MS (ESI): m/z 653.2 $[M+H]^+$. $^1H$ NMR (400 MHz, DMSO-$d_6$) δ 8.72 (s, 2H), 8.46 (d, *J* = 5.0 Hz, 1H), 8.24 (d, *J* = 8.3 Hz, 2H), 7.70 (d, *J* = 2.3 Hz, 1H), 7.63 (d, *J* = 2.3 Hz, 1H), 7.38 (d, *J*= 8.2 Hz, 2H), 6.94 (d, *J* = 5.0 Hz, 1H), 5.27 (s, 2H), 4.48 - 4.37 (m, 3H), 4.33 (t, *J* = 8.8 Hz, 2H), 4.25 - 4.18 (m, 2H), 3.96 (t, *J* = 5.2 Hz, 2H), 3.06 (s, 3H), 1.69 (s, 6H).

**Example 380: Preparation of compound 380**

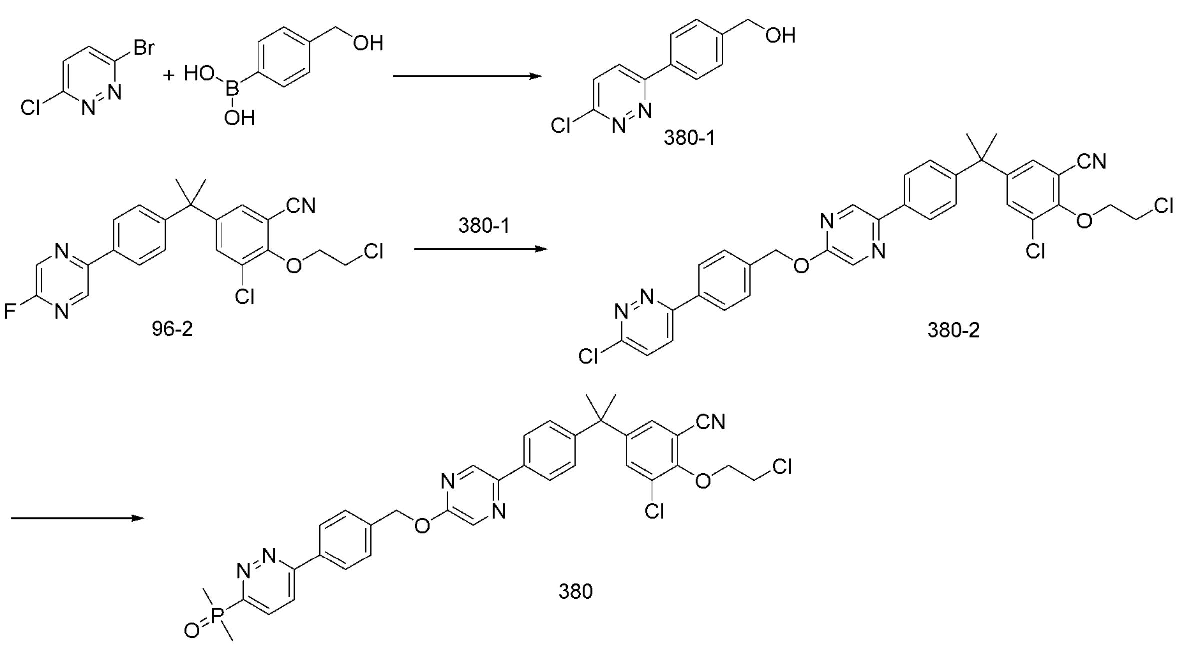


**Preparation of compound 380-1**

3-Bromo-6-chloropyridazine (0.5 g, 2.58 mmol), 4-(hydroxymethyl)phenylboronic acid (510.6 mg, 3.36 mmol), potassium carbonate (714.5 mg, 5.17 mmol), and Pd(dppf)$Cl_2$ (189.1 mg, 258.49 μmol) were sequentially added to a 100 mL round-bottom flask, followed by the addition of 1,4-dioxane (12 mL) and water (3 mL). The reaction system was purged three times with nitrogen, and the mixture was stirred at 95°C under a nitrogen atmosphere for 8 h. After the reaction was completed, the reaction mixture was cooled to room temperature, diluted with ethyl acetate, and filtered through diatomite. The filtrate was adsorbed onto silica gel, concentrated to dryness by rotary evaporation, and purified by silica gel column chromatography (PE/EA = 1/3) to obtain 376 mg of the title compound **380-1.** LC-MS (ESI): m/z 221.0 $[M+H]^+$.

**Preparation of compound 380-2**

Compound **380-1** (60 mg, 271.92 μmol), compound **96-2** (117 mg, 271.92 μmol), and cesium carbonate (177.2 mg, 543.84 μmol) were sequentially added to a 10 mL microwave tube, followed by the final addition of DMF (2 mL). The reaction mixture was stirred under microwave irradiation at 100°C for 2 h in a sealed tube. After the reaction was completed, the reaction mixture was diluted with dichloromethane and water. The organic phase was separated, washed with saturated brine, dried over anhydrous sodium sulfate, filtered, and concentrated. The crude product was purified by silica gel column chromatography (DCM/MeOH = 97/3) to obtain 90.7 mg of the title compound **380-2.** LC-MS (ESI): m/z 630.2 $[M+H]^+$.

**Preparation of compound 380**

Compound **380-2** (90.7 mg, 143.75 μmol) was dissolved in DMF (2.5 mL). Xantphos (16.6 mg, 28.75 μmol), $Pd_2(dba)_3$ (13.2 mg, 14.38 μmol), dimethylphosphine oxide (56.1 mg, 718.76 μmol), and DIPEA (111.5 mg, 862.51 μmol, 150 μL) were added separately. The reaction mixture was immediately bubbled with nitrogen for 1 min and stirred under microwave irradiation at 120°C for 2 h. After the reaction was completed, the reaction mixture was diluted with a small amount of 1,4-dioxane, filtered through a membrane filter, and purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 60-80% 8 min, 80-95% 4 min; flow rate: 30 mL/min) to obtain 60 mg of the title compound **380.** LC-MS (ESI): m/z 672.2 $[M+H]^+$. $^1H$ NMR (400 MHz, Chloroform-d) δ 8.53 (d, *J* = 1.4 Hz, 1H), 8.39 (d, *J* = 1.4 Hz, 1H), 8.30 (s, 1H), 8.19 (d, *J* = 8.1 Hz, 2H), 8.04 (d, *J* = 6.4 Hz, 1H), 7.87 (d, *J* = 8.5 Hz, 2H), 7.68 (d, *J* = 8.0 Hz, 2H), 7.46 (d, *J* = 2.4 Hz, 1H), 7.37 (d, *J* = 2.3 Hz, 1H), 7.30 (d, *J* = 8.5 Hz, 2H), 5.54 (s, 2H), 4.43 (t, *J* = 6.2 Hz, 2H), 3.88 (t, *J* = 6.2 Hz, 2H), 1.96 (d, *J* = 12.6 Hz, 6H), 1.70 (s, 6H). $^{31}P$ NMR (162 MHz, Chloroform-d) δ 36.65 (s, 1P).

**Example 381: Preparation of compound 381**

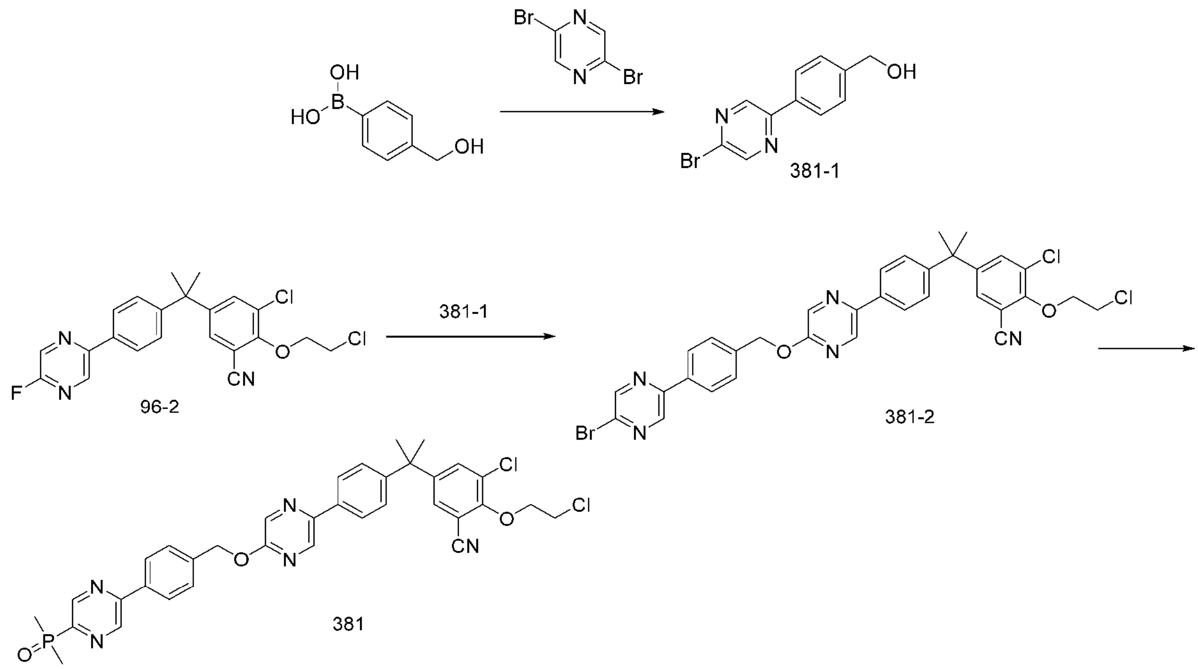

381-1
96-2
381-2
381

**Preparation of compound 381-1**

(4-(Hydroxymethyl)phenyl)boronic acid (1.0 g, 4.20 mmol), 2,5-dibromopyrazine (639 mg, 4.20 mmol), potassium carbonate (1.2 g, 8.4 mmol), and $Pd(dppf)Cl_2$ (308 mg, 0.42 mmol) were dissolved in 1,4-dioxane (24 mL) and water (6 mL). The reaction system was purged three times with nitrogen and stirred at 90°C overnight. After the reaction was completed, the reaction mixture was filtered through a pad of diatomite, and the filter cake was rinsed with dichloromethane (50 mL). Water (30 mL) was added to the filtrate, and the mixture was extracted three times with dichloromethane (70 mL). The organic phases were combined, washed once with saturated brine (70 mL), dried over anhydrous sodium sulfate, subjected to suction filtration, and the filtrate was concentrated. The resulting crude product was purified by silica gel column chromatography (PE/EA = 1/1) to obtain 115 mg of the title compound **381-1.** LC-MS (ESI): m/z 265.0 $[M+H]^+$.

**Preparation of compound 381-2**

Compound **381-1** (72.1 mg, 0.27 mmol), compound **96-2** (97.5 mg, 0.23 mmol), and cesium carbonate (147.7 mg, 0.45 mmol) were dissolved in DMF (2 mL). After addition was completed, the reaction system was stirred at 100°C for 2 h. After the reaction was completed, the reaction mixture was concentrated to obtain a crude product. The crude product was purified by silica gel column chromatography (PE/EA = 100% to 80%) to obtain 75 mg of the title compound **381-2.** LC-MS (ESI): m/z 674.1 [M+H]+.

**Preparation of compound 381**

Compound **381-2** (75 mg, 0.11 mmol), dimethylphosphine oxide (43.3 mg, 0.55 mmol), $Pd_2(dba)_3$ (10.2 mg, 0.01 mmol), Xantphos (12.9 mg, 0.02 mmol), and DIPEA (86.1 mg, 0.55 mmol) were dissolved in DMF (2 mL). The reaction system was stirred in a microwave reactor at 110°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 42 mg of the title compound **381.** LC-MS (ESI): m/z 672.2 [M+H]+. $^1$H NMR (400 MHz, DMSO-$d_6$) δ 9.46 (s, 1H), 9.11 (s, 1H), 8.80 (s, 1H), 8.49 (s, 1H), 8.24 (d, *J* = 7.9 Hz, 2H), 7.97 (d, *J* = 8.1 Hz, 2H), 7.77 - 7.56 (m, 4H), 7.37 (d, *J* = 8.1 Hz, 2H), 5.54 (s, 2H), 4.42 (t, *J* = 5.1 Hz, 2H), 3.95 (t, *J* = 5.0 Hz, 2H), 1.76 (d, *J* = 13.7 Hz, 6H), 1.69 (s, 6H). $^{31}$P NMR (162 MHz, DMSO-$d_6$) δ 34.22 (s, 1P).

**Example 382: Preparation of compound 382**

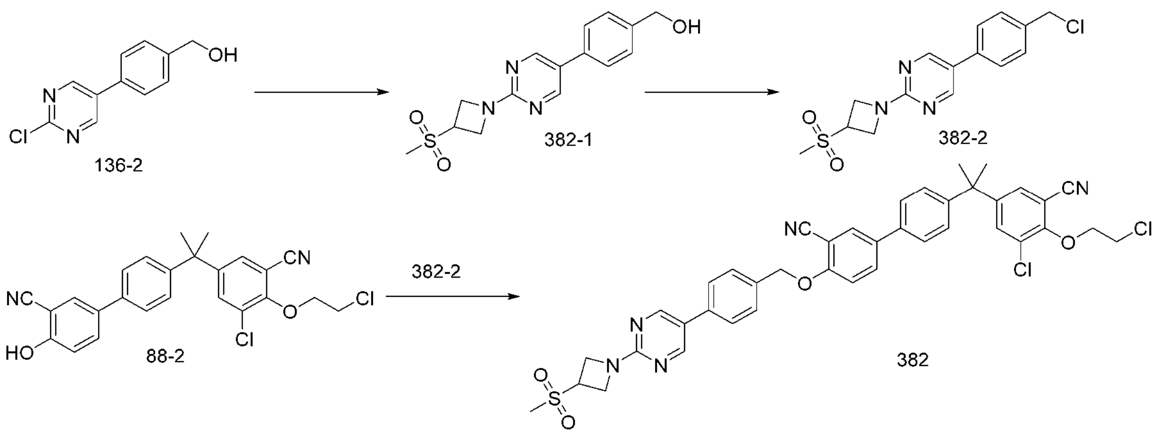


136-2 382-1 382-2

88-2 382

**Preparation of compound 382-1**

Compound **136-2** (300 mg, 1.36 mmol), 3-(methylsulfonyl)azetidine hydrochloride (467 mg, 2.72 mmol), DIEA (530 mg, 4.10 μmol), and acetonitrile (3 mL) were added to a three-necked flask, and the reaction was stirred at 85°C for 3 h. After the reaction was completed, saturated aqueous sodium chloride solution (10 mL) was added to the reaction system, and the mixture was extracted three times with DCM (10 mL). The organic phases were combined, dried, subjected to suction filtration, and concentrated. The resulting crude product was purified by silica gel column chromatography (EA/PE = 0 to 60%) to obtain 110 mg of the title compound **382-1.** LC-MS (ESI): m/z 320.3 [M+H]+.

**Preparation of compound 382-2**

Compound **382**-**1**(110 mg, 0.344 mmol), triethylamine (104 mg, 1.0 mmol) and dichloromethane (3 mL) were added to a three-necked flask. The reaction system was purged three times with nitrogen, and methanesulfonyl chloride (47 mg, 0.41 mmol) was added dropwise in an ice bath. The mixture was stirred for 3 h. After the reaction was completed, saturated aqueous sodium chloride solution (10 mL) was added to the reaction system, and the mixture was extracted three times with DCM (10 mL). The organic phases were combined, dried, subjected to suction filtration, and the filtrate was concentrated. The resulting crude product was purified by silica gel column chromatography (EA/PE = 0 to 50%) to obtain 105 mg of the title compound **382-2.** LC-MS (ESI): m/z 338.4 [M+H]+.

**Preparation of compound 382**

Compound **382-2** (30 mg, 88.8 μmol), compound **88-2** (44 mg, 97.7 μmol), cesium carbonate (58 mg, 177.6

μmol), and acetonitrile (3 mL) were added to a three-necked flask. The reaction system was purged three times with nitrogen and stirred at 75°C for 3 h. After the reaction was completed, saturated aqueous sodium chloride solution (10 mL) was added to the reaction system. The mixture was extracted three times with DCM (10 mL). The organic phases were combined, dried, subjected to suction filtration, and the filtrate was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 50% acetonitrile gradient elution over 12 min; flow rate: 30 mL/min) to obtain 12 mg of the title compound **382.** LC-MS (ESI): m/z 752.2 $[M+H]^+$. $^1H$ NMR (400 MHz, DMSO-$d_6$) δ 8.78 (s, 2H), 8.07 (d, *J* = 2.4 Hz, 1H), 7.97 (dd, *J* = 8.9, 2.4 Hz, 1H), 7.76 - 7.69 (m, 2H), 7.70 (d, *J* = 2.4 Hz, 1H), 7.67 - 7.61 (m, 3H), 7.58 (d, *J* = 8.4 Hz, 2H), 7.43 (d, *J* = 9.0 Hz, 1H), 7.33 (d, *J* = 8.5 Hz, 2H), 5.38 (s, 2H), 4.48 - 4.34 (m, 5H), 4.27 (dd, *J* = 9.5, 4.3 Hz, 2H), 3.96 (t, *J* = 5.2 Hz, 2H), 3.08 (s, 3H), 1.68 (s, 6H).

**Example 383: Preparation of compound 383**

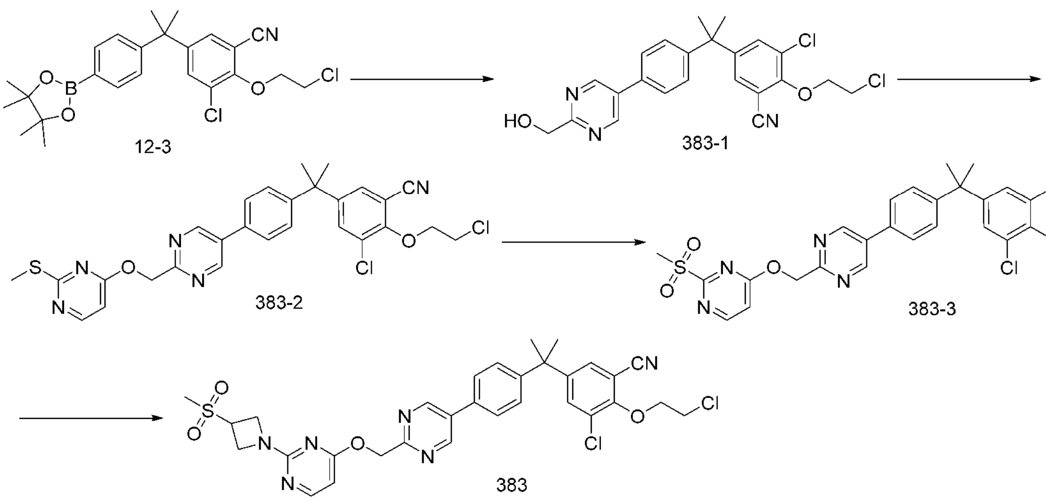
12-3
383-1
383-2
383-3
383

**Preparation of compound 383-1**

Compound **12-3** (800 mg, 1.74 mmol) and 5-bromopyrimidine-2-methanol (395 mg, 2.1 mmol) were dissolved in 1,4-dioxane (8 mL). Potassium carbonate (600 mg, 4.35 mmol), [1,1'-bis(diphenylphosphino)ferrocene]dichloropalladium(II) (150 mg, 174 μmol), and water (0.5 mL) were added. The reaction system was purged three times with nitrogen and stirred at 100°C for 3 h. After the reaction was completed, water (15 mL) was added, and the mixture was extracted three times with dichloromethane (20 mL). The organic phases were combined, washed twice with saturated brine (30 mL), dried over anhydrous sodium sulfate, subjected to suction filtration, and the filtrate was concentrated. The resulting crude product was purified by silica gel column chromatography (EA/PE = 0 to 50%) to obtain 620 mg of the title compound **383-1.** LC-MS (ESI): m/z 442.3 $[M+H]^+$.

**Preparation of compound 383-2**

Compound **383-1** (620 mg, 1.40 mmol) and 4-chloro-2-(methylthio)pyrimidine (269 mg, 1.68 mmol) were dissolved in acetonitrile (8 mL), and potassium carbonate (104 mg, 1.0 mmol) was added. The reaction system was purged three times with nitrogen and stirred at 65°C for 3 h. After the reaction was completed, the reaction mixture was cooled to room temperature. Saturated aqueous sodium chloride solution (20 mL) was added to the reaction system, and the mixture was extracted three times with dichloromethane (15 mL). The organic phases were combined, washed twice with saturated brine (30 mL), dried over anhydrous sodium sulfate, filtered, and the filtrate was concentrated. The resulting crude product was purified by silica gel column chromatography (PE/EA = 100% to 40%) to obtain 450 mg of the title compound **383-2.**

**Preparation of compound 383-3**

Compound **383-2** (450 mg, 0.80 mmol) was dissolved in tetrahydrofuran (8 mL), and an aqueous solution of potassium peroxymonosulfate (1.38 g, 4.0 mmol) was added. The reaction system was stirred at 25°C for 5 h. After the

reaction was completed, the reaction mixture was cooled to room temperature. Saturated aqueous sodium chloride solution (20 mL) was added to the reaction system, and the mixture was extracted three times with dichloromethane (15 mL). The organic phases were combined, washed twice with saturated brine (30 mL), dried over anhydrous sodium sulfate, filtered, and the filtrate was concentrated to obtain a crude product. The crude product was purified by silica gel column chromatography (PE/EA = 100% to 40%) to obtain 450 mg of the title compound **383-3.** LC-MS (ESI): m/z 598.0 $[M+H]^+$.

**Preparation of compound 383**

Compound **383-3** (50 mg, 83.5 μmol), 3-(methylsulfonyl)azetidine hydrochloride (44 mg, 97.7 μmol), and *N,N*-diisopropylethylamine (27 mg, 209 μmol) were dissolved in acetonitrile (3 mL). The reaction system was stirred under microwave irradiation at 80°C for 2 h. After the reaction was completed, saturated aqueous sodium chloride (10 mL) solution was added, and the mixture was extracted three times with dichloromethane (10 mL). The organic phases were combined, washed twice with saturated brine (20 mL), dried over anhydrous sodium sulfate, filtered, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 50% acetonitrile gradient elution over 12 min; flow rate: 30 mL/min) to obtain 12 mg of the title compound **383.** LC-MS (ESI): m/z 653.2 $[M+H]^+$. $^1H$ NMR (400 MHz, DMSO-$d_6$) δ 9.10 (s, 2H), 8.15 (d, *J* = 5.7 Hz, 1H), 7.75 (d, *J* = 8.5 Hz, 2H), 7.72 (d, *J* = 2.3 Hz, 1H), 7.66 (d, *J* = 2.3 Hz, 1H), 7.40 (d, *J* = 8.6 Hz, 2H), 6.33 (d, *J* = 5.7 Hz, 1H), 5.56 (s, 2H), 4.42 (t, *J* = 5.2 Hz, 2H), 4.36 - 4.25 (m, 1H), 4.23 - 4.07 (m, 2H), 4.09 - 3.98 (m, 2H), 3.96 (t, *J* = 5.2 Hz, 2H), 2.98 (s, 3H), 1.70 (s, 6H).

**Example 384: Preparation of compound 384**

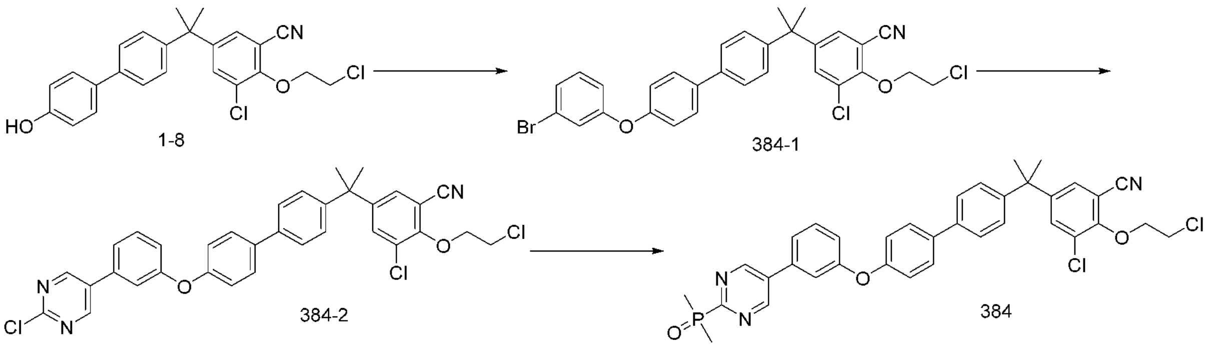


**Preparation of compound 384-1**

Compound **1-8** (1.5 g, 3.52 mmol), *m*-bromophenylboronic acid (1.13 g, 5.63 mmol), triethylamine (713 mg, 7.04 mmol), and copper(II) acetate (70 mg, 0.35 mmol) were weighed and added to dichloromethane (15 mL). The reaction system was stirred at room temperature for 24 h under an air atmosphere. After the reaction was completed, water (30 mL) was added, and the mixture was extracted three times with dichloromethane (20 mL). The organic phases were combined, washed twice with saturated brine (40 mL), dried over anhydrous sodium sulfate, subjected to suction filtration, and the filtrate was concentrated. The resulting crude product was purified by silica gel column chromatography (EA/PE = 0 to 25%) to obtain 130 mg of the title compound **384-1.** $^1H$ NMR (400 MHz, DMSO-$d_6$) δ 7.74 - 7.67 (m, 3H), 7.64 (d, *J* = 2.3 Hz, 1H), 7.60 (d, *J* = 8.5 Hz, 2H), 7.40 - 7.31 (m, 4H), 7.25 - 7.21 (m, 1H), 7.16 - 7.11 (m, 2H), 7.08 - 7.04 (m, 1H), 4.42 (t, *J* = 5.2 Hz, 2H), 3.96 (t, *J* = 5.2 Hz, 2H), 1.69 (s, 6H).

**Preparation of compound 384-2**

Compound **384-1** (130 mg, 223 μmol) was dissolved in 1,4-dioxane (3 mL). 2-Chloropyrimidine-5-boronic acid (42 mg, 0.261 μmol), potassium carbonate (62 mg, 449 μmol), [1,1'-bis(diphenylphosphino)ferrocene]dichloropalladium(II) (19 mg, 22 μmol), and water (0.2 mL) were added. The reaction system was purged three times with nitrogen and stirred at 100°C for 3 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product.

The crude product was purified by silica gel column chromatography (EA/PE = 0 to 30%) to obtain 85 mg of the title compound **384-2.**

**Preparation of compound 384**

Compound **384-2** (85 mg, 138 μmol) was dissolved in DMF (1 mL), followed by the sequential addition of tris(dibenzylideneacetone)dipalladium (5.06 mg, 6.9 μmol), 4,5-bis(diphenylphosphino)-9,9-dimethylxanthene (4 mg, 6.9 μmol), DIEA (36 mg, 276 μmol), and dimethylphosphine oxide (41 mg, 522 μmol). The reaction system was stirred at 120°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 50% acetonitrile gradient elution over 12 min; flow rate: 30 mL/min) to obtain 12 mg of the title compound **384.** LC-MS (ESI): m/z 656.3 $[M+H]^+$. $^1$H NMR (400 MHz, DMSO-$d_6$) δ 9.33 (s, 2H), 7.74 - 7.70 (m, 2H), 7.70 - 7.66 (m, 3H), 7.65 (d, *J* = 2.3 Hz, 1H), 7.63 - 7.56 (m, 3H), 7.37 - 7.29 (m, 2H), 7.21 - 7.11 (m, 3H), 4.42 (t, *J* = 5.2 Hz, 2H), 3.96 (t, *J* = 5.2 Hz, 2H), 1.79 (d, *J* = 13.7 Hz, 6H), 1.69 (s, 6H). $^{31}$P NMR (162 MHz, DMSO-$d_6$) δ 33.90 (s, 1P).

**Example 385: Preparation of compound 385**

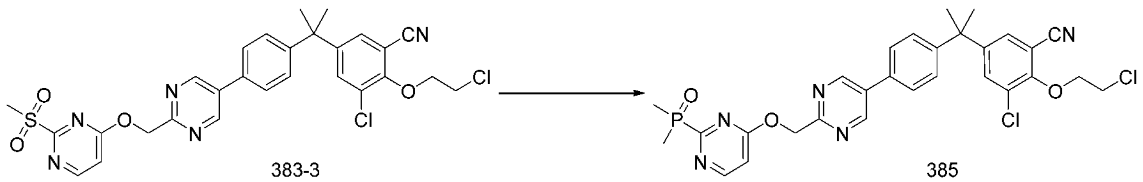

383-3

385

**Preparation of compound 385**

Compound **383-3** (100 mg, 167 μmol), cesium carbonate (135 mg, 417 μmol), and dimethylphosphine oxide (39 mg, 501 μmol) were dissolved in acetonitrile (2 mL), and the reaction system was stirred under microwave irradiation at 90°C for 2 h. After the reaction was completed, saturated aqueous sodium chloride (10 mL) solution was added, and the mixture was extracted three times with dichloromethane (10 mL). The organic phases were combined, washed twice with saturated brine (20 mL), dried over anhydrous sodium sulfate, filtered, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 50% acetonitrile gradient elution over 12 min; flow rate: 30 mL/min) to obtain 12 mg of the title compound **385.** LC-MS (ESI): m/z 596.0 $[M+H]^+$. $^1$H NMR (400 MHz, DMSO-$d_6$) δ 9.10 (s, 2H), 8.72 (d, *J* = 5.9 Hz, 1H), 7.84 - 7.69 (m, 3H), 7.66 (d, *J* = 2.4 Hz, 1H), 7.40 (d, *J* = 8.0 Hz, 2H), 7.24 (dd, *J* = 5.9, 2.7 Hz, 1H), 5.70 (s, 2H), 4.42 (t, *J* = 5.0 Hz, 2H), 3.96 (t, *J* = 5.1 Hz, 2H), 1.69 (s, 6H), 1.51 (d, *J* = 13.7 Hz, 6H). $^{31}$P NMR (162 MHz, DMSO-$d_6$) δ 33.96 (s, 1P).

**Example 386: Preparation of compound 386**

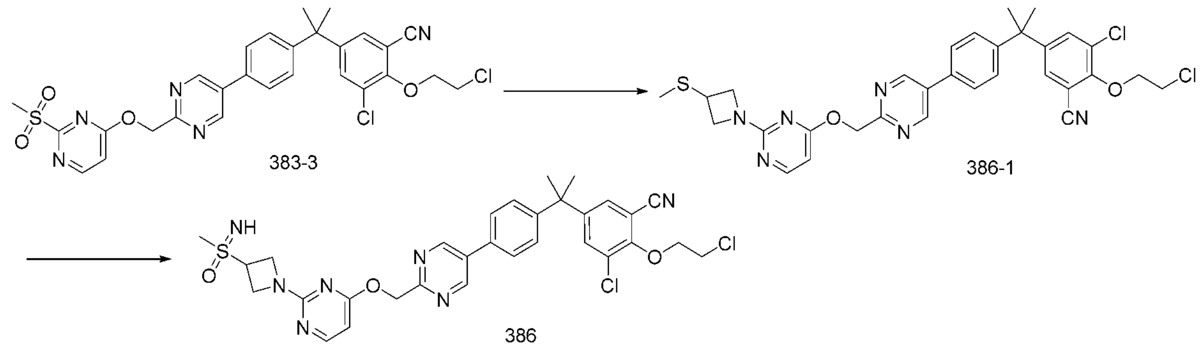

383-3 386-1 386

### Preparation of compound 386-1

Compound **383-3** (130 mg, 0.22 mmol), N,N-diisopropylethylamine (71 mg, 0.55 mmol), and 3-(methylthio) azetidine hydrochloride (41 mg, 0.29 mmol) were dissolved in acetonitrile (10 mL), and the reaction system was stirred at 75°C for 3 h. After the reaction was completed, saturated sodium chloride solution (30 mL) was added, and the mixture was extracted three times with dichloromethane (20 mL). The organic phases were combined, washed twice with saturated brine (40 mL), dried over anhydrous sodium sulfate, filtered, and the filtrate was concentrated to obtain a crude product. The crude product was purified by silica gel column chromatography (PE/EA = 100% to 40%) to obtain 105 mg of the title compound **386-1.**

### Preparation of compound 386

Compound **386-1** (105 mg, 169 μmol) and ammonium carbonate (48 mg, 507 μmol) were dissolved in methanol (3 mL), and (diacetoxyiodo)benzene (163 mg, 507 μmol) was added thereto at 0°C. After the addition was completed, the reaction system was stirred at 25°C for 3 h. After the reaction was completed, water (5 mL) was added, and the mixture was extracted three times with ethyl acetate (10 mL). The organic phases were combined, washed twice with saturated brine (20 mL), dried over anhydrous sodium sulfate, filtered, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 50% acetonitrile gradient elution over 12 min; flow rate: 30 mL/min) to obtain 12 mg of the title compound **386.** LC-MS (ESI): m/z 652.0 $[M+H]^+$. $^1$H NMR (400 MHz, Chloroform-d) δ 8.94 (s, 2H), 8.12 (d, *J* = 5.9 Hz, 1H), 7.54 (d, *J* = 8.4 Hz, 2H), 7.48 (d, *J* = 2.4 Hz, 1H), 7.38 - 7.30 (m, 3H), 6.37 (d, *J* = 5.9 Hz, 1H), 5.64 (s, 2H), 4.43 (t, *J* = 6.1 Hz, 2H), 4.36 (s, 4H), 4.08 (q, *J* = 6.6 Hz, 1H), 3.88 (t, *J* = 6.1 Hz, 2H), 2.95 (s, 3H), 1.71 (s, 6H).

### Example 387: Preparation of compound 387

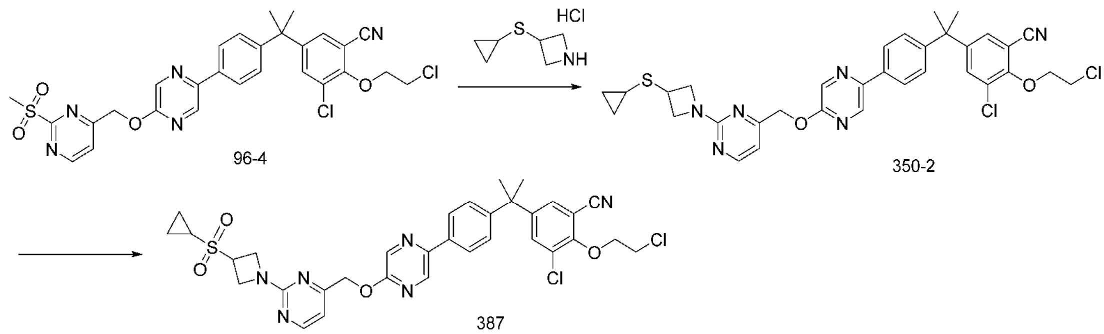


96-4 350-2 387

### Preparation of compound 350-2

Compound **96-4** (80 mg, 0.13 mmol) was added to a microwave tube, followed by the sequential addition of anhydrous ethanol (2 mL), compound 2 (33 mg, 0.20 mmol), and DIPEA (52 mg, 0.40 mmol). The system was purged three times with nitrogen, and the reaction was carried out under microwave irradiation at 90°C for 2 h. After the reaction was monitored by LCMS until completion, the reaction mixture was filtered. The crude filtrate was purified by normal-phase

silica gel column chromatography (EA/PE = 0 to 60%) to obtain 70 mg of the title compound **350-2** in 81% yield. LC-MS (ESI): m/z 647.2 $[M+H]^+$.

### Preparation of compound 387

Compound **350-2** (70 mg, 0.11 mmol) was added to dichloromethane (5 mL), and *m*-CPBA (56 mg, 0.32 mmol) was added with stirring. After the addition was completed, the reaction was carried out at room temperature for 2 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 10 mg of the title compound **387.** LC-MS (ESI): m/z 679.4 $[M+H]^+$. $^1H$ NMR (400 MHz, DMSO-$d_6$) δ 8.75 (d, *J* = 1.4 Hz, 1H), 8.54 (d, *J* = 1.3 Hz, 1H), 8.38 (d, *J* = 5.0 Hz, 1H), 7.96 (d, *J* = 8.1 Hz, 2H), 7.70 (d, *J* = 2.3 Hz, 1H), 7.63 (d, *J* = 2.3 Hz, 1H), 7.37 (d, *J* = 8.2 Hz, 2H), 6.80 (d, *J* = 5.0 Hz, 1H), 5.37 (s, 2H), 4.53 - 4.44 (m, 1H), 4.42 (t, *J* = 5.1 Hz, 2H), 4.34 (t, *J* = 8.9 Hz, 2H), 4.28 - 4.17 (m, 2H), 3.95 (t, *J* = 5.1 Hz, 2H), 2.88 - 2.74 (m, 1H), 1.69 (s, 6H), 1.07 - 0.96 (m, 4H).

### Example 388: Preparation of compound 388

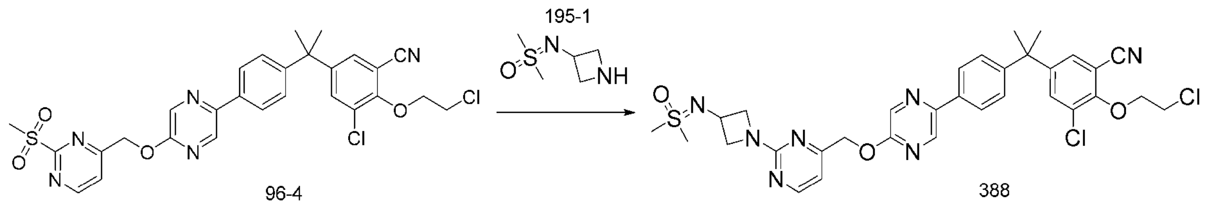


96-4 388

### Preparation of compound 388

Compound **96-4** (80 mg, 0.13 mmol) was added to a microwave tube, followed by the sequential addition of anhydrous ethanol (2 mL), compound **195-1** (37 mg, 0.20 mmol), and DIPEA (52 mg, 0.40 mmol). The system was purged three times with nitrogen, and the reaction was carried out under microwave irradiation at 90°C for 2 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **388.** LC-MS (ESI): m/z 666.5 $[M+H]^+$. $^1H$ NMR (400 MHz, DMSO-$d_6$) δ 8.75 (d, *J* = 1.4 Hz, 1H), 8.52 (d, *J* = 1.4 Hz, 1H), 8.29 (d, *J* = 5.0 Hz, 1H), 7.96 (d, *J* = 8.6 Hz, 2H), 7.70 (d, *J* = 2.3 Hz, 1H), 7.63 (d, J = 2.3 Hz, 1H), 7.37 (d, *J* = 8.5 Hz, 2H), 6.67 (d, *J* = 5.0 Hz, 1H), 5.33 (s, 2H), 4.42 (t, *J* = 5.2 Hz, 2H), 4.34 - 4.22 (m, 3H), 3.96 (t, *J* = 5.2 Hz, 2H), 3.74 (dd, *J* = 8.4, 4.8 Hz, 2H), 3.02 (s, 6H), 1.69 (s, 6H).

### Examples 389 and 390: Preparation of compounds 389 and 390

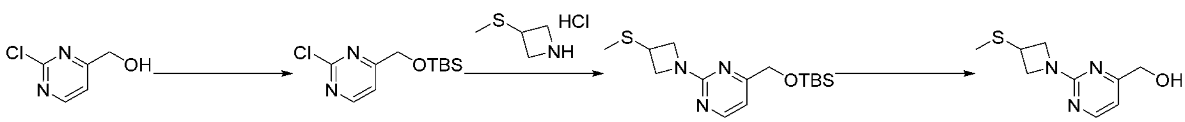

389-1 389-2 389-3

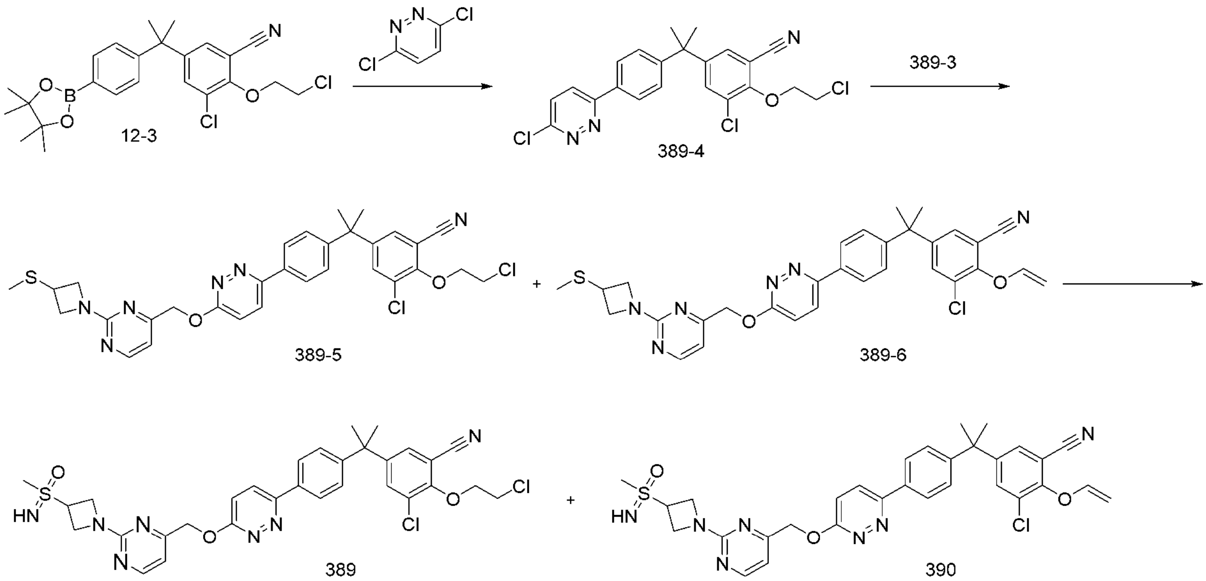


12-3 389-4 389-5 389-6 389 390

## Preparation of compound 389-1

(2-Chloropyrimidin-4-yl)methanol (3.7 g, 25.59 mmol), *tert*-butyldimethylsilyl chloride (5.79 g, 38.38 mmol), imidazole (3.48 g, 51.18 mmol), and dichloromethane (40 mL) were added to an eggplant-shaped flask. The reaction system was reacted at room temperature for 2 h. Water (50 mL) was added, and the mixture was extracted with dichloromethane (60 mL). The organic phase was washed with saturated brine (50 mL), dried over anhydrous sodium sulfate, subjected to suction filtration, and the filtrate was evaporated to dryness under reduced pressure. The resulting residue was purified by silica gel column chromatography (EA/PE = 0% to 20%) to obtain 2.5 g of the title compound **389-1.** $^1$H NMR (400 MHz, Chloroform-d) δ 8.49 (d, *J* = 5.0 Hz, 1H), 7.38 (d, *J* = 5.0 Hz, 1H), 4.64 (s, 2H), 0.83 (s, 9H), 0.00 (s, 6H).

## Preparation of compound 389-2

Compound **389-1** (500 mg, 1.93 mmol) and 3-(methylthio)azetidine hydrochloride (270 mg, 1.93 mmol) were dissolved in ethanol (10 mL). Triethylamine (1.95 g, 19.3 mmol) was added, and the mixture was heated to 100°C and stirred for 2 h. The reaction was monitored by LCMS until completion. The reaction was quenched with ice water (10 mL) and extracted three times with ethyl acetate (20 mL). The organic phases were combined, dried over anhydrous sodium sulfate, subjected to suction filtration, and the filtrate was concentrated to obtain 550 mg of the title compound **389-2.** LC-MS (ESI): m/z 326.2 $[M+H]^+$.

## Preparation of compound 389-3

Compound **389-2** (450 mg, 1.38 mmol) was dissolved in tetrahydrofuran (3 mL). Tetrabutylammonium fluoride solution (1 mol/L in THF, 1.4 mL, 1.38 mmol) was added at room temperature, and the mixture was stirred at room temperature for 2 h. The reaction was monitored by LCMS until completion. The mixture was directly concentrated, and the resulting residue was purified by normal-phase silica gel column chromatography (PE/EA = 100% to 50%) to obtain 350 mg of the title compound **389-3.** LC-MS (ESI): m/z 212.1 $[M+H]^+$.

## Preparation of compound 389-4

Compound **12-3** (1 g, 1.98 mmol) and 3,6-dichloropyridazine (384 mg, 2.57 mmol) were dissolved in 1,4-dioxane (10 mL) and water (2 mL). Pd(dppf)$Cl_2$ (145 mg, 0.20 mmol) and potassium carbonate (821 mg, 5.94 mmol) were added. The system was purged four times with argon and stirred at 100°C for 16 h. The reaction was monitored by LCMS until completion. The reaction was quenched with ice water (10 mL) and extracted three times with ethyl acetate (20 mL). The organic phases were combined, dried over anhydrous sodium sulfate, subjected to suction filtration, and the filtrate was concentrated. The resulting residue was purified by normal-phase silica gel column chromatography (PE/EA = 100% to 65%) to obtain 700 mg of the title compound **389-4.** LC-MS (ESI): m/z 446.0 $[M+H]^+$.

**Preparation of compounds 389-5 and 389-6**

Compound **389-4** (300 mg, 1.42 mmol) was dissolved in DMF (10 mL). Sodium hydride (170 mg, 4.26 mmol, 60% by mass) was added at 0°C, and the mixture was stirred for 20 min. Then, compound **389-3** (634 mg, 1.42 mmol) was added, and the mixture was stirred for another 1 h. The reaction was monitored by LCMS until completion. The reaction was quenched with ice water (15 mL) and extracted three times with ethyl acetate (30 mL). The organic phase was washed three times with saturated sodium chloride (50 mL), dried over anhydrous sodium sulfate, subjected to suction filtration, and the filtrate was concentrated. The resulting residue was purified by normal-phase silica gel column chromatography (PE/EA = 100% to 0%) to obtain 250 mg of the title compound **389-5,** LC-MS (ESI): m/z 643.2 $[M+Na]^+$; and the title compound **389-6,** LC-MS (ESI): m/z 585.3 $[M+H]^+$.

**Preparation of compounds 389 and 390**

Compounds **389-5 and 389-6** (250 mg, 0.38 mmol) were dissolved in methanol (10 mL). (Diacetoxyiodo) benzene (490 mg, 1.52 mmol) and ammonium carbamate (74 mg, 0.95 mmol) were added at room temperature, and the mixture was stirred at room temperature for 1 h. The reaction was monitored by LCMS until completion. The reaction mixture was subjected to preparative purification (preparative method: mobile phase: A: 0.03% aqueous ammonia; B: acetonitrile; chromatographic column: XBridge C18, 19 * 250 mm, 10 μm; column temperature: 25°C; gradient: 54% to 54% acetonitrile gradient elution over 16 min; flow rate: 20 mL/min) to obtain 12.66 mg of the title compound **389** and 25.65 mg of the title compound **390.**

Compound **389:** LC-MS (ESI): m/z 616.1 $[M+H]^+$. $^1$H NMR (400 MHz, Chloroform-d) δ 8.34 (d, *J* = 5.1 Hz, 1H), 7.99 - 7.93 (m, 2H), 7.85 (d, *J* = 9.2 Hz, 1H), 7.51 (d, *J* = 2.3 Hz, 1H), 7.42 (d, *J* = 2.3 Hz, 1H), 7.35 - 7.29 (m, 2H), 7.20 (d, *J* = 9.2 Hz, 1H), 6.80 (d, *J* = 5.0 Hz, 1H), 6.66 (dd, *J* = 13.9, 6.2 Hz, 1H), 5.56 (s, 2H), 4.49 - 4.39 (m, 6H), 4.36 (dd, *J* = 13.9, 3.0 Hz, 1H), 4.18 - 4.10 (m, 1H), 3.00 (s, 3H), 1.73 (s, 6H).

Compound **390:** LC-MS (ESI): m/z 652.1 $[M+H]^+$. $^1$H NMR (400 MHz, Chloroform-d) δ 8.34 (d, *J* = 5.0 Hz, 1H), 7.98 - 7.94 (m, 2H), 7.84 (d, *J* = 9.2 Hz, 1H), 7.46 (d, *J* = 2.3 Hz, 1H), 7.37 (d, *J* = 2.3 Hz, 1H), 7.33 - 7.30 (m, 2H), 7.19 (d, *J* = 9.2 Hz, 1H), 6.80 (d, *J* = 5.1 Hz, 1H), 5.56 (s, 2H), 4.46 - 4.41 (m, 6H), 4.18 - 4.11 (m, 1H), 3.88 (t, *J* = 6.2 Hz, 2H), 3.00 (s, 3H), 1.71 (s, 6H).

**Example 391: Preparation of compound 391**

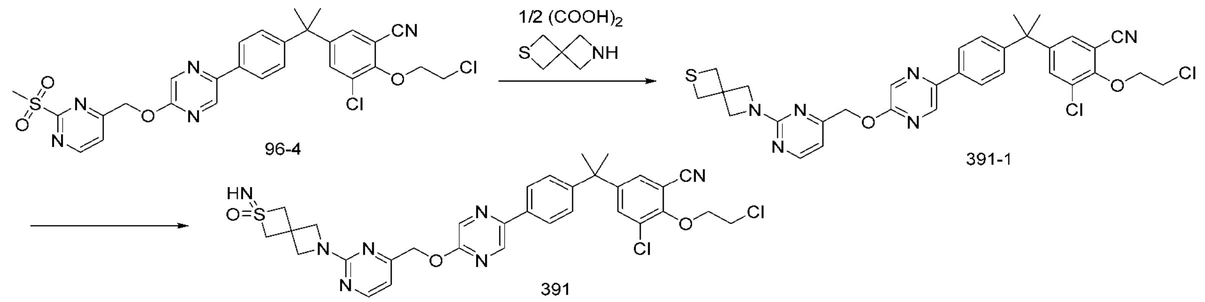


96-4

391-1

391

**Preparation of compound 391-1**

Compound **96-4** (80 mg, 0.14 mmol) was added to a microwave tube, followed by the sequential addition of anhydrous ethanol (2 mL), 2-thia-6-azaspiro[3.3]heptane hemioxalate (128 mg, 0.40 mmol), and DIPEA (52 mg, 0.40 mmol). The system was purged three times with nitrogen, and the reaction was carried out under microwave irradiation at 90°C for 2 h. After the reaction was monitored by LCMS until completion, the reaction mixture was filtered. The crude filtrate was purified by normal-phase silica gel column chromatography (EA/PE = 0 to 60%) to obtain 80 mg of the title compound **391-1** in 94.5% yield. LC-MS (ESI): m/z 633.0 $[M+H]^+$.

**Preparation of compound 391**

Compound **391-1** (80 mg, 0.13 mmol) and ammonium carbonate (36 mg, 0.38 mmol) were dissolved in methanol (5 mL). (Diacetoxyiodo)benzene (203 mg, 0.63 mmol) was added thereto at 0°C. After the addition was completed, the reaction was carried out at room temperature overnight. After the reaction was monitored the next day by LCMS until

completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **391.** LC-MS (ESI): m/z 664.2 $[M+H]^+$. $^1H$ NMR (400 MHz, DMSO-$d_6$) δ 8.74 (d, *J* = 1.4 Hz, 1H), 8.53 (d, *J* = 1.4 Hz, 1H), 8.34 (d, *J* = 5.0 Hz, 1H), 7.96 (d, *J* = 8.6 Hz, 2H), 7.70 (d, *J* = 2.3 Hz, 1H), 7.63 (d, *J* = 2.3 Hz, 1H), 7.37 (d, *J* = 8.6 Hz, 2H), 6.74 (d, *J* = 5.0 Hz, 1H), 5.34 (s, 2H), 4.48 (s, 1H), 4.42 (t, *J* = 5.2 Hz, 2H), 4.30 (d, *J* = 13.0 Hz, 2H), 4.26 - 4.17 (m, 6H), 3.96 (t, *J* = 5.2 Hz, 2H), 1.69 (s, 6H).

**Example 392: Preparation of compound 392**

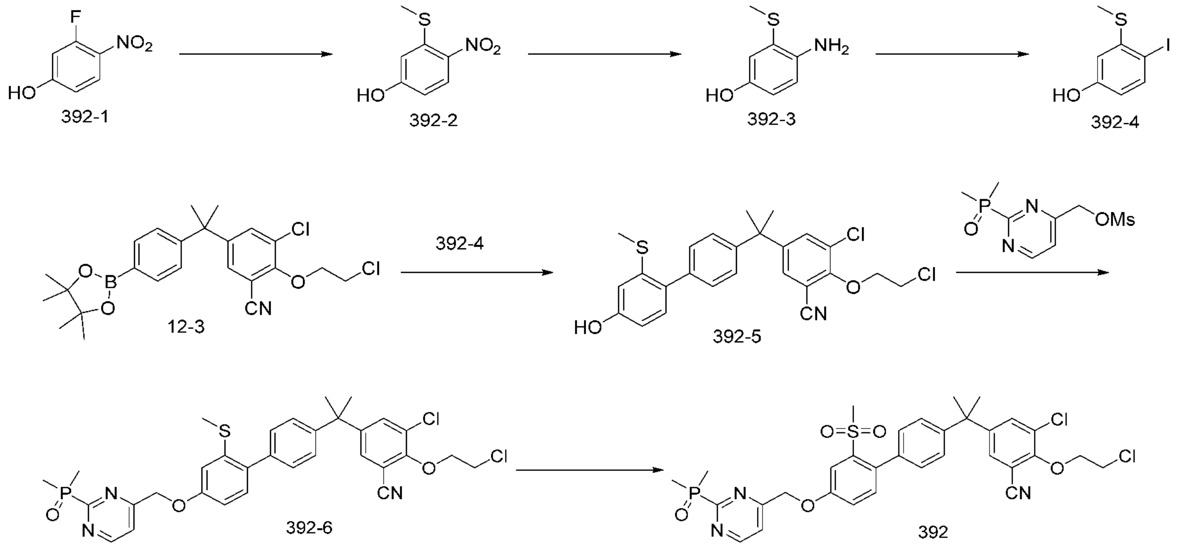

392-1, 392-2, 392-3, 392-4, 12-3, 392-5, 392-6, 392

**Preparation of compound 392-2**

Compound **392-1** (1 g, 6.37 mmol) was dissolved in anhydrous DMF (10 mL). Sodium methanethiolate (10 g, 20% aqueous solution, 28.5 mmol) was added, and the reaction was carried out at 60°C for 5 h. After the reaction was monitored by TLC until completion, the reaction mixture was poured into water (100 mL) and extracted with EA (50 mL). The phases were separated, and the organic phase was dried, subjected to suction filtration, and the filtrate was concentrated. The residue was purified by normal-phase silica gel column chromatography (EtOAc/PE = 20 to 40%) to obtain 600 mg of the title compound **392-2** in 51% yield. LC-MS (ESI): m/z 186.0 $[M+H]^+$.

**Preparation of compound 392-3**

Compound **392-2** (600 mg, 3.24 mmol) was dissolved in THF (30 mL). Saturated aqueous ammonium chloride solution (10 mL) was added, followed by the addition of zinc powder (2.1 g, 32 mmol), and the reaction was carried out at 70°C for 5 h. After the reaction was monitored by TLC until completion, the reaction mixture was filtered. The filtrate was poured into water (100 mL) and extracted with EA (50 mL). The phases were separated, and the organic phase was dried and subjected to suction filtration. The filtrate was concentrated to obtain 450 mg of the title compound **392-3** in 89.6% yield.

**Preparation of compound 392-4**

Compound **392-3** (450 mg, 2.90 mmol) was dissolved in THF (30 mL), and dilute hydrochloric acid (10 mL, 1 mol/L) was added. The mixture was cooled to 0°C, and sodium nitrite (400 mg, 5.80 mmol) was added. After stirring for 30 min, potassium iodide (963 mg, 5.80 mmol) was added, and the reaction was carried out at 80°C for 2 h. After the reaction was monitored by TLC until completion, the reaction mixture was poured into water (100 mL) and extracted with EA (100 mL). The phases were separated, and the organic phase was dried and subjected to suction filtration. The filtrate was concentrated. The resulting residue was purified by normal-phase silica gel column chromatography (EtOAc/PE = 10 to 20%) to obtain 320 mg of the title compound **392-4** in 41.4% yield.

**Preparation of compound 392-5**

Compound **12-3** (320 mg, 1.20 mmol) was dissolved in 1,4-dioxane (30 mL), followed by the addition of $H_2O$ (10 mL), potassium carbonate (248 mg, 1.80 mmol), **392-4** (662 mg, 1.44 mmol), and Pd(dppf)$Cl_2$ (183 mg, 0.20 mmol). The system was purged three times with nitrogen, and the reaction was carried out at 100°C for 2 h. After the reaction was monitored by TLC until completion, the reaction mixture was poured into water (100 mL) and extracted with EA (100 mL). The organic phase was dried, subjected to suction filtration, and the filtrate was concentrated. The resulting residue was purified by normal-phase silica gel column chromatography (EtOAc/PE = 10 to 20%) to obtain 300 mg of the title compound 392-**5** in 53% yield.LC-MS (ESI): m/z 472.0 $[M+H]^+$.

**Preparation of compound 392-6**

Compound **392-5** (150 mg, 0.32 mmol) was dissolved in DMF (8 mL). Cesium carbonate (130 mg, 0.5 mmol) and (2-(dimethylphosphoryl)pyrimidin-4-yl)methyl methanesulfonate (132 mg, 0.5 mmol) were added, and the reaction was carried out at 60°C for 1 h. After the reaction was monitored by LCMS until completion, the reaction mixture was poured into water (50 mL) and extracted with EA (50 mL). The phases were separated, and the organic phase was concentrated to obtain 120 mg of a crude product of the title compound **392-6.** LC-MS (ESI): m/z 640.2 $[M+H]^+$.

**Preparation of compound 392**

Compound **392-6** (120 mg, crude) was dissolved in DCM (20 mL), and m-CPBA (194 mg, 0.96 mmol, 85% purity) was added. The reaction was carried out at 40°C for 1 h. After the reaction was monitored by LCMS until completion, the reaction mixture was filtered. The crude filtrate was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 5.8 mg of the title compound **392.** LC-MS (ESI): m/z 671.80 $[M+H]^+$. $^1H$ NMR (400 MHz, DMSO-$d_6$) δ 9.03 (d, *J* = 5.2 Hz, 1H), 7.84 - 7.77 (m, 1H), 7.71 (d, *J* = 2.5 Hz, 2H), 7.63 (d, *J* = 2.3 Hz, 1H), 7.48 - 7.41 (m, 1H), 7.41 - 7.27 (m, 5H), 5.46 (s, 2H), 4.43 (t, *J* = 5.0 Hz, 2H), 3.96 (t, *J* = 5.1 Hz, 2H), 2.80 (s, 3H), 1.76 (d, *J* = 13.7 Hz, 6H), 1.71 (s, 6H). $^{31}P$ NMR (162 MHz, DMSO-$d_6$)

**Example 393: Preparation of compound 393**

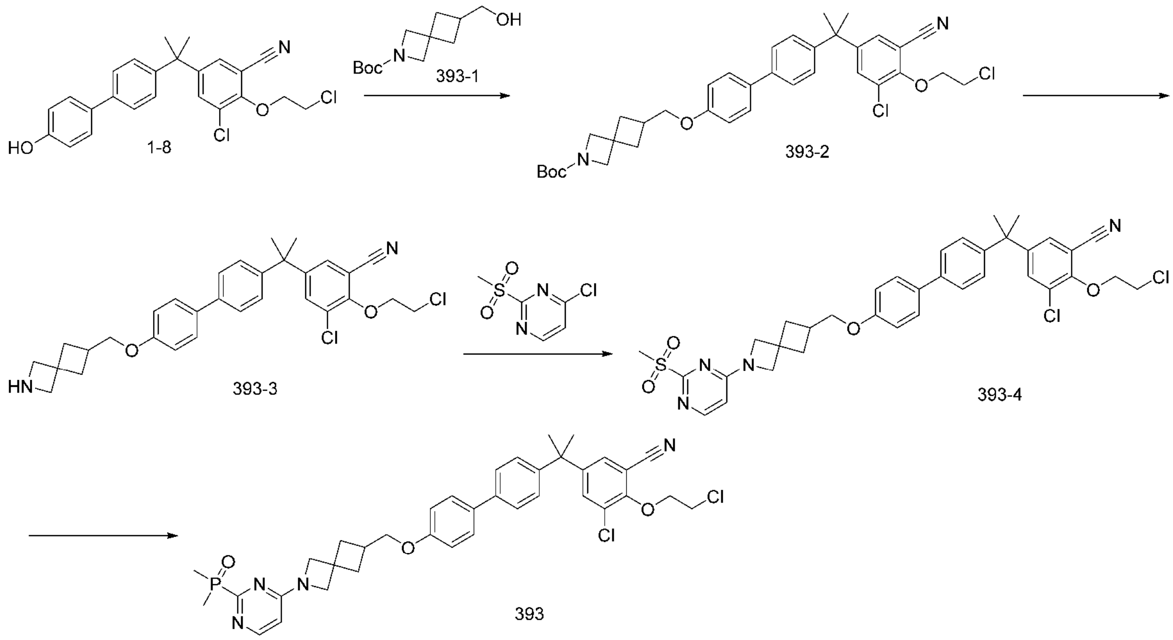

**Preparation of compound 393-2**

Compound **1-8** (500 mg, 1.17 mmol) and compound **393-1** (292.83 mg, 1.29 mmol) were weighed and added to a 30 mL microwave tube, followed by the addition of CMBP (566.1 mg, 2.35 mmol) and toluene (10 mL). The system was purged three times with nitrogen. The reaction mixture was heated at 100°C for 2 h. TLC indicated disappearance of the starting material and formation of the product. The reaction mixture was subjected to rotary evaporation until dryness, and purified by silica gel column chromatography (PE/EtOAc = 100% to 10%) to obtain 300 mg of the title compound **393-2** in 47.3% yield.

**Preparation of compound 393-3**

At 0°C, compound **393-2** (300 mg, 0.473 mmol) was dissolved in DCM (5 mL), and TFA (5 mL) was added dropwise. The reaction mixture was stirred at room temperature for 3 h. LCMS indicated the disappearance of the starting material and the formation of the product. The reaction mixture was poured into saturated $Na_2CO_3$ solution (50 mL), extracted three times with DCM (30 mL), washed three times with saturated brine (30 mL), dried over anhydrous $Na_2SO_4$, filtered, and subjected to rotary evaporation until dryness under reduced pressure to obtain 150 mg of the title compound **393-3** in 59.6% yield. The product was used directly in the next step. LC-MS (ESI): m/z 535.2 $[M+H]^+$.

**Preparation of compound 393-4**

Compound **393-3** (150 mg, 0.28 mmol) and 2-(methylsulfonyl)-4-chloropyrimidine (64.72 mg, 0.34 mmol) were weighed and dissolved in DCM (10 mL). DIPEA (87.04 mg, 0.68 mmol) was added, and the reaction mixture was stirred at 0°C for 2 h. TLC indicated the disappearance of the starting material. The reaction mixture was subjected to rotary evaporation until dryness and purified by silica gel column chromatography (PE/EtOAc = 0 to 50%) to obtain 120 mg of the title compound **393-4** in 62.1% yield. LC-MS (ESI): m/z 691.3 $[M+H]^+$.

**Preparation of compound 393**

Compound **393-4** (120 mg, 0.17 mmol) and dimethylphosphine oxide (359.4 mg, 4.60 mmol) were dissolved in $CH_3CN$ (8 mL), and $Cs_2CO_3$ (325.82 mg, 1.0 mmol) was added. The reaction mixture was stirred at 70°C for 2 h. LCMS indicated the disappearance of the starting material and the formation of the product. The reaction mixture was filtered, and the filtrate was poured into water (20 mL) and extracted three times with ethyl acetate (25 mL). The organic phases were combined, washed with water, washed three times with saturated brine (25 mL), dried over anhydrous sodium sulfate, filtered, and subjected to rotary evaporation until dryness. The resulting residue was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 55% to 75% acetonitrile gradient elution over 12 min; flow rate: 30 mL/min) to obtain 14.28 mg of the title compound 393. LC-MS (ESI): m/z 689.4 $[M+H]^+$. $^1$H NMR (400 MHz, DMSO-$d_6$) δ 8.25 (dd, *J* = 6.0, 1.0 Hz, 1H), 7.71 (d, *J* = 2.3 Hz, 1H), 7.64 (d, *J* = 2.3 Hz, 1H), 7.61 - 7.53 (m, 4H), 7.36 - 7.24 (m, 2H), 7.07 - 6.95 (m, 2H), 6.44 (dd, *J* = 6.0, 3.2 Hz, 1H), 4.42 (t, *J= 5.2* Hz,2H), 4.12 (s, 2H), 4.06 (s, 2H), 4.01 - 3.92 (m, 4H), 2.72 - 2.58 (m, 1H), 2.45 - 2.38 (m, 2H), 2.17 - 2.03 (m, 2H), 1.68 (s, 6H), 1.64 (d, *J* = 13.5 Hz, 6H). $^{31}$P NMR (162 MHz, DMSO-$d_6$) δ 33.32 (s, 1P).

**Example 394: Preparation of compound 394**

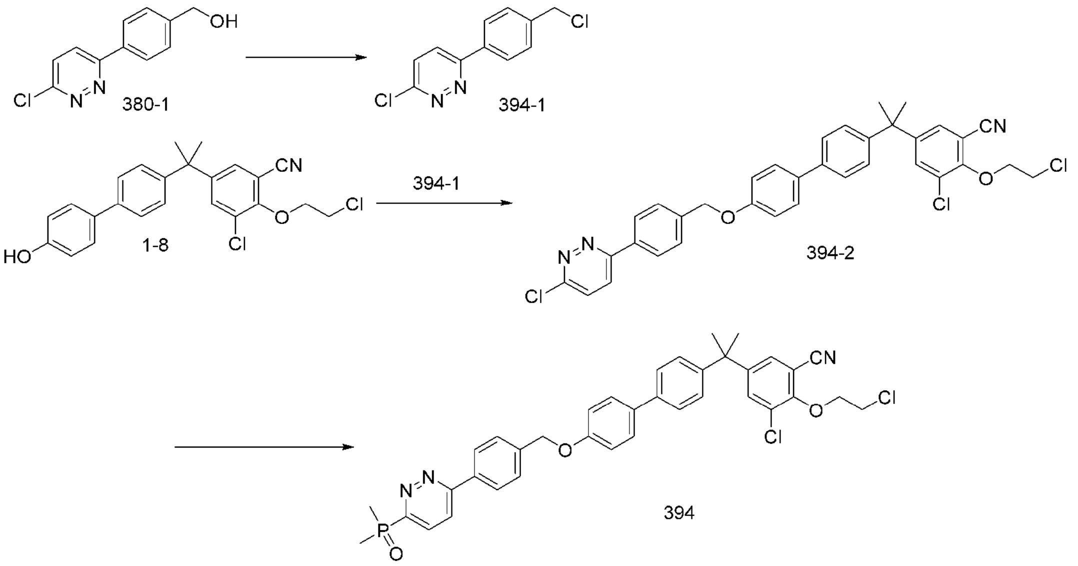


380-1 394-1 1-8 394-2 394

**Preparation of compound 394-1**

Compound **380-1** (100 mg, 453.20 μmol) was suspended in DCM, cooled to 0°C in an ice-water bath, and thionyl chloride (269.6 mg, 2.27 mmol, 164 μL) was added. The reaction mixture was gradually warmed to room temperature and stirred for 1.5 h. After the reaction was completed, the reaction mixture was concentrated to dryness by rotary evaporation to obtain a crude product of the title compound **394-1.** The product was used directly in the next step without purification. LC-MS (ESI): m/z 239.0 $[M+H]^+$.

**Preparation of compound 394-2**

Compound **1-8** (193 mg, 452.70 μmol) and compound **394-1** (108.2 mg, 452.70 μmol) were dissolved in DMF (3 mL), and cesium carbonate (295 mg, 905.39 μmol) was added. The reaction mixture was stirred at 70°C for 7 h. After the reaction was completed, the reaction mixture was cooled to room temperature. Saturated aqueous ammonium chloride solution was added, and the mixture was extracted with ethyl acetate. The organic phase was washed with saturated brine, dried over anhydrous sodium sulfate, filtered, and concentrated. The crude product was purified by silica gel column chromatography (PE/EA = 1.5/1 to 3/7) to obtain 247.4 mg of the title compound **394-2.** LC-MS (ESI): m/z 628.2 $[M+H]^+$.

**Preparation of compound 394**

Compound **394-2** (120 mg, 190.79 μmol) was dissolved in DMF (2.5 mL). Xantphos (22.1 mg, 38.16 μmol), $Pd_2(dba)_3$ (17.5 mg, 19.08 μmol), dimethylphosphine oxide (44.7 mg, 572.36 μmol), and DIPEA (123.3 mg, 953.94 μmol, 166 μL) were added separately. The mixture was immediately bubbled with nitrogen for 1 min and stirred under microwave irradiation at 120°C for 2 h in a sealed tube. After the reaction was completed, the reaction mixture was diluted with a small amount of 1,4-dioxane, filtered through a membrane filter, and then purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 60-80% 8 min, 80-95% 4 min; flow rate: 30 mL/min) to obtain 68 mg of the title compound **394.** LC-MS (ESI): m/z 670.2 $[M+H]^+$. $^1H$ NMR (400 MHz, DMSO-$d_6$) δ 8.42 (dd, *J* = 8.7, 2.6 Hz, 1H), 8.25 (d, *J* = 8.4 Hz, 2H), 8.22 (dd, *J* = 8.7, 5.0 Hz, 1H), 7.72 - 7.66 (m, 3H), 7.64 (d, *J* = 2.3 Hz, 1H), 7.61 (d, *J* = 8.4 Hz, 2H), 7.56 (d, *J* = 8.4 Hz, 2H), 7.30 (d, *J* = 8.4 Hz, 2H), 7.13 (d, *J* = 8.8 Hz, 2H), 5.28 (s, 2H), 4.42 (t, *J* = 5.2 Hz, 2H), 3.96 (t, *J* = 5.2 Hz, 2H), 1.83 (d, *J* = 13.8 Hz, 6H), 1.68 (s, 6H). $^{31}P$ NMR (162 MHz, DMSO-$d_6$) δ 34.54 (s, 1P).

**Example 395: Preparation of compound 395**

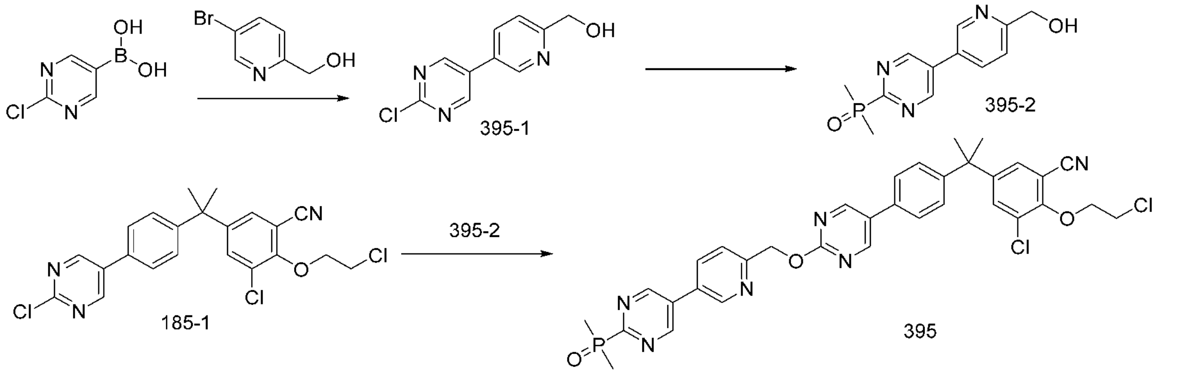

395-1 395-2 185-1 395

**Preparation of compound 395-1**

(2-Chloropyrimidin-5-yl)boronic acid (3.28 g, 20.74 mmol), (5-bromopyridin-2-yl)methanol (3.0 g, 15.96 mmol), potassium carbonate (4.41 g, 31.91 mmol), and Pd(dppf)$Cl_2$ (934.0 mg, 1.28 mmol) were sequentially added to a 100 mL round-bottom flask, followed by the final addition of 1,4-dioxane (32 mL) and water (8 mL). The reaction system was purged three times with nitrogen, and the mixture was stirred at 90°C under a nitrogen atmosphere for 5 h. After the reaction was completed, the reaction mixture was cooled to room temperature, diluted with ethyl acetate, and filtered through diatomite. The filtrate was adsorbed onto silica gel, concentrated to dryness by rotary evaporation, and purified by silica gel column chromatography (PE/EA = 1/7) to obtain 801.3 mg of the title compound **395-1.** LC-MS (ESI): m/z 221.9 $[M+H]^+$.

**Preparation of compound 395-2**

Compound **395-1** (400 mg, 1.80 mmol) was dissolved in DMF (8.0 mL). Xantphos (208.9 mg, 360.94 μmol), $Pd_2(dba)_3$ (165.3 mg, 180.47 μmol), dimethylphosphine oxide (422.6 mg, 5.41 mmol), and DIPEA (1.17 g, 9.02 mmol, 1.57 mL) were added separately. The mixture was bubbled with nitrogen for 1 min and stirred under microwave irradiation at 120°C for 2 h in a sealed tube. After the reaction was completed, the reaction mixture was filtered through diatomite, and the filter cake was washed with ethyl acetate and dichloromethane. The filtrate was concentrated to dryness by rotary evaporation. Deionized water (30 mL) was added, and the mixture was extracted with dichloromethane. The aqueous phase was separated. The organic phase was washed once with deionized water (10 mL). The aqueous phases were combined and lyophilized. The resulting crude product was purified by silica gel column chromatography (DCM/MeOH = 17 to 3) to obtain 100 mg of the title compound **395-2.** LC-MS (ESI): m/z 264.0 $[M+H]^+$.

**Preparation of compound 395**

Compound **395-2** (88.0 mg, 334.31 μmol) was dissolved in DMF (3 mL), and compound **395-2** (119.5 mg, 267.44 μmol) and cesium carbonate (217.9 mg, 668.61 μmol) were added separately. The reaction mixture was stirred under microwave irradiation at 90°C for 2 h in a sealed tube. After the reaction was completed, the reaction mixture was diluted with a small amount of 1,4-dioxane, filtered through a membrane filter, and purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 60-80% 8 min, 80-95% 4 min; flow rate: 30 mL/min) to obtain 49.8 mg of compound **395.** LC-MS (ESI): m/z 673.2 $[M+H]^+$. $^1$H NMR (400 MHz, DMSO-$d_6$) δ 9.39 (s, 2H), 9.07 (d, *J* = 2.0 Hz, 1H), 8.96 (s, 2H), 8.33 (dd, *J* = 8.2, 2.4 Hz, 1H), 7.71 (d, *J* = 2.3 Hz, 1H), 7.70 - 7.66 (m, 2H), 7.66 - 7.62 (m, 2H), 7.37 (d, *J* = 8.4 Hz, 2H), 5.62 (s, 2H), 4.42 (t, *J* = 5.2 Hz, 2H), 3.96 (t, *J* = 5.2 Hz, 2H), 1.80 (d, *J* = 13.8 Hz, 6H), 1.69 (s, 6H). $^{31}$P NMR (162 MHz, DMSO-$d_6$) δ 34.00 (s, 1P).

**Example 396: Preparation of compound 396**

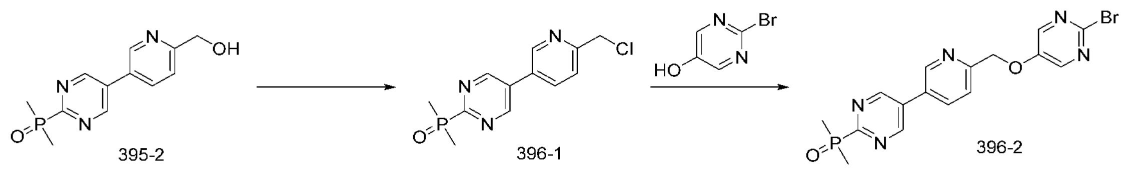
395-2 396-1 396-2

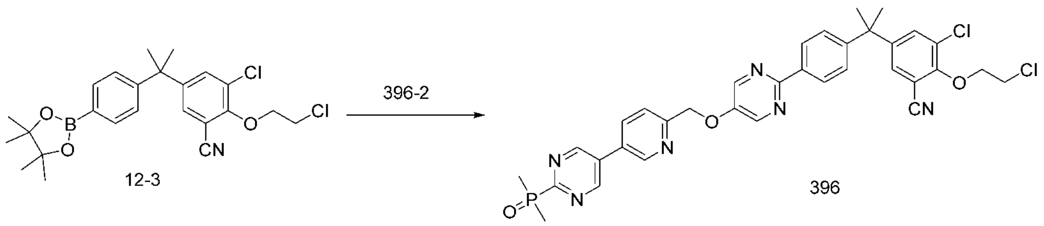


12-3

396

**Preparation of compound 396-1**

Compound **395-2** (100 mg, 0.38 mmol) and thionyl chloride (228 mg, 1.9 mmol) were dissolved in DCM (5 mL), and the reaction system was stirred at room temperature for 1.5 h. After the reaction was completed, the reaction mixture was directly concentrated to obtain a crude product, yielding 105 mg of the title compound **396-1.** LC-MS (ESI): m/z 282.0 $[M+H]^+$.

**Preparation of compound 396-2**

Compound **396-1** (107 mg, 0.38 mmol) was dissolved in DMF (5 mL), followed by the sequential addition of 2-bromo-5-hydroxypyrimidine (37.0 mg, 0.21 mmol) and cesium carbonate (344 mg, 1.06 mmol). The reaction system was stirred at 90°C for 1.5 h. After the reaction was completed, water (100 mL) was added to the reaction mixture, and the mixture was extracted three times with dichloromethane (150 mL). The organic phases were combined, washed once with saturated brine (100 mL), dried over anhydrous sodium sulfate, subjected to suction filtration, and the filtrate was concentrated to obtain a crude product. The crude product was purified by silica gel column chromatography (DCM/MeOH = 17/3) to obtain 80 mg of the title compound **396-2.** LC-MS (ESI): m/z 420.0 $[M+H]^+$.

**Preparation of compound 396**

Compound **396-2** (80 mg, 0.19 mmol), compound 12-3 (131 mg, 0.28 mmol), $K_2CO_3$ (53 mg, 0.38 mmol), and Pd(dppf)$Cl_2$ (14 mg, 0.02 mmol) were dissolved in 1,4-dioxane (4.5 mL) and $H_2O$ (1.5 mL). The reaction system was purged three times with nitrogen and stirred under microwave irradiation at 105°C for 2 h. After the reaction was completed, the reaction mixture was filtered through a pad of diatomite. The filter cake was rinsed with dichloromethane (70 mL). Water (100 mL) was added to the filtrate, and the mixture was extracted three times with dichloromethane (100 mL). The organic phases were combined, washed once with saturated brine (100 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 56 mg of the title compound **396.** LC-MS (ESI): m/z 673.3 $[M+H]^+$. $^1H$ NMR (400 MHz, DMSO-$d_6$) δ 9.41 (s, 2H), 9.11 (d, *J* = 2.4 Hz, 1H), 8.77 (s, 2H), 8.39 (dd, *J* = 8.1, 2.4 Hz, 1H), 8.24 (d, *J* = 8.5 Hz, 2H), 7.80 (d, *J* = 8.2 Hz, 1H), 7.70 (d, *J* = 2.4 Hz, 1H), 7.63 (d, *J* = 2.3 Hz, 1H), 7.39 (d, *J* = 8.5 Hz, 2H), 5.51 (s, 2H), 4.42 (t, *J* = 5.2 Hz, 2H), 3.96 (t, *J* = 5.2 Hz, 2H), 1.81 (d, *J* = 13.8 Hz, 6H), 1.70 (s, 6H). $^{31}P$ NMR (162 MHz, DMSO-$d_6$) δ 34.00 (s, 1P).

**Example 397: Preparation of compound 397**

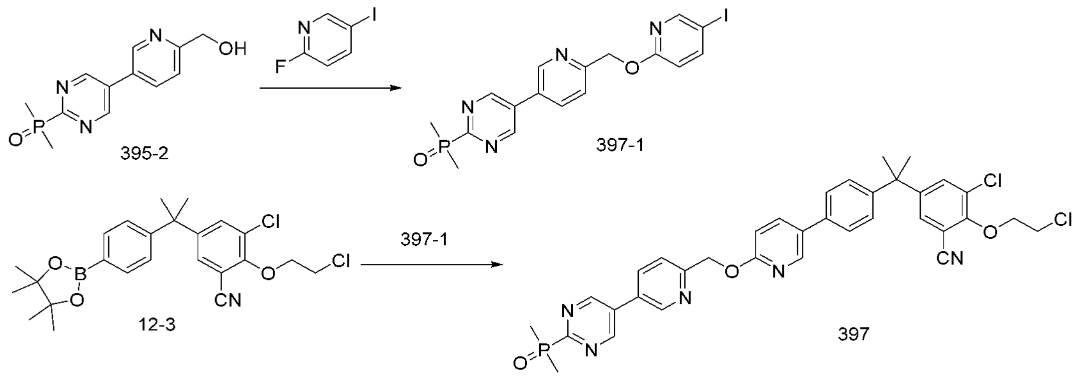

395-2
397-1
12-3
397

### Preparation of compound 397-1

Compound **395-2** (400 mg, 1.52 mmol) was dissolved in DMF (10.0 mL), followed by the sequential addition of 2-fluoro-5-iodopyridine (373 mg, 1.67 mmol) and cesium carbonate (1.07 g, 3.04 mmol). The reaction system was stirred under microwave irradiation at 120°C for 2 h. After the reaction was completed, water (10 mL) was added, and the mixture was extracted three times with ethyl acetate (20 mL). The organic phases were combined, washed twice with saturated brine (20 mL), dried over anhydrous sodium sulfate, and subjected to suction filtration, and the filtrate was concentrated to obtain a crude product. The crude product was purified by silica gel column chromatography (DCM/MeOH = 15 to 25%) to obtain 215 mg of the title compound **397-2.** LC-MS (ESI): m/z 467.0 [M+H]$^+$.

### Preparation of compound 397

Compound **397-1** (120 mg, 0.26 mmol) was dissolved in 1,4-dioxane (2 mL), followed by the sequential addition of compound **12-3** (142 mg, 0.31 mmol), potassium carbonate (71 mg, 0.51 mmol), and water (0.4 mL). The system was purged three times with nitrogen, and Pd(dppf)$Cl_2$ (19 mg, 0.03 mmol) was added. The reaction system was stirred at 90 °C for 12 h. After the reaction was completed, insoluble solids were removed by filtration. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 13 mg of the title compound **397.** LC-MS (ESI): m/z 672.3 [M+H]$^+$. $^1$H NMR (400 MHz, DMSO-$d_6$) δ 9.39 (s, 2H), 9.06 (d, *J* = 3.2 Hz, 1H), 8.46 (d, *J* = 2.5 Hz, 1H), 8.31 (dd, *J* = 8.1, 2.4 Hz, 1H), 8.07 (dd, *J* = 8.6, 2.6 Hz, 1H), 7.71 (d, *J* = 2.4 Hz, 1H), 7.66 - 7.57 (m, 4H), 7.34 (d, *J* = 8.5 Hz, 2H), 7.07 (d, *J* = 8.6 Hz, 1H), 5.57 (s, 2H), 4.42 (t, *J* = 5.2 Hz, 2H), 3.96 (t, *J* = 5.2 Hz, 2H), 1.80 (d, *J* = 13.7 Hz, 6H), 1.69 (s, 6H). $^{31}$P NMR (162 MHz, DMSO-$d_6$) δ 33.96 (s, 1P).

### Example 398: Preparation of compound 398

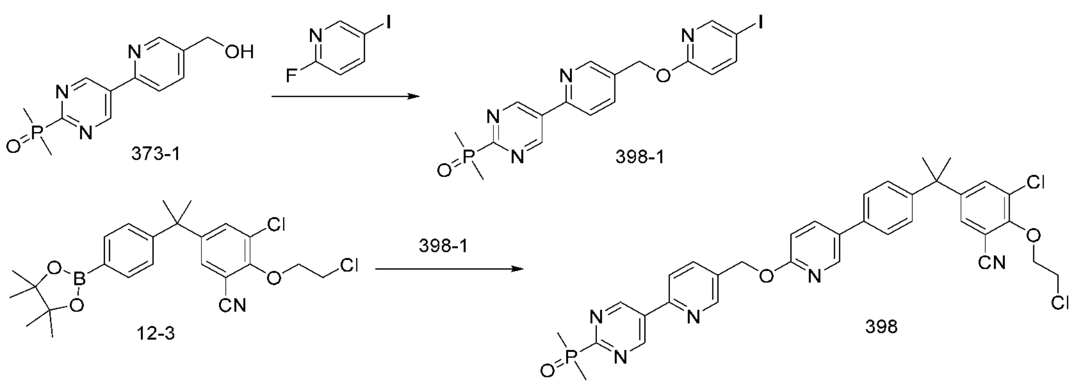

373-1
398-1
12-3
398

### Preparation of compound 398-1

Compound **373-1** (100 mg, 0.38 mmol) was dissolved in DMF (5 mL), followed by the sequential addition of 2-fluoro-5-iodopyridine (93 mg, 0.42 mmol) and cesium carbonate (268 mg, 0.76 mmol). The reaction system was stirred under microwave irradiation at 120°C for 2 h. After the reaction was completed, water (10 mL) was added, and the mixture was extracted three times with ethyl acetate (20 mL). The organic phases were combined, washed twice with saturated brine (20 mL), dried over anhydrous sodium sulfate, and subjected to suction filtration, and the filtrate was concentrated. The resulting crude product was purified by silica gel column chromatography (DCM/MeOH = 1/4) to obtain 215 mg of the title compound **398-2.** LC-MS (ESI): m/z 467.0 [M+H]$^+$.

**Preparation of compound 398**

Compound **398-1** (40 mg, 0.09 mmol) was dissolved in 1,4-dioxane (2 mL), followed by the sequential addition of compound **12-3** (47 mg, 0.10 mmol), potassium carbonate (24 mg, 0.17 mmol), and water (0.4 mL). The system was purged three times with nitrogen, and Pd(dppf)$Cl_2$ (6 mg, 0.09 mmol) was added. The reaction system was stirred at 90 °C for 12 h. After the reaction was completed, insoluble solids were removed by filtration. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 8.1 mg of the title compound **398.** LC-MS (ESI): m/z 672.2 [M+H]$^+$. $^1$H NMR (400 MHz, DMSO-$d_6$) δ 9.60 (s, 2H), 8.89 (d, *J* = 2.2 Hz, 1H), 8.50 (d, *J* = 3.4 Hz, 1H), 8.24 (d, *J* = 8.1 Hz, 1H), 8.11 (dd, *J* = 8.2, 2.2 Hz, 1H), 8.06 (dd, *J* = 8.6, 2.5 Hz, 1H), 7.71 (d, *J* = 2.3 Hz, 1H), 7.65 (d, *J* = 2.3 Hz, 1H), 7.62 (d, *J* = 8.5 Hz, 2H), 7.34 (d, *J* = 8.5 Hz, 2H), 7.02 (d, *J* = 9.3 Hz, 1H), 5.53 (s, 2H), 4.42 (t, *J* = 5.2 Hz, 2H), 3.96 (t, *J* = 5.2 Hz, 2H), 1.81 (d, *J* = 13.8 Hz, 6H), 1.69 (s, 6H). $^{31}$P NMR (162 MHz, DMSO-$d_6$) δ 34.97 (s, 1P).

**Example 399: Preparation of compound 399**

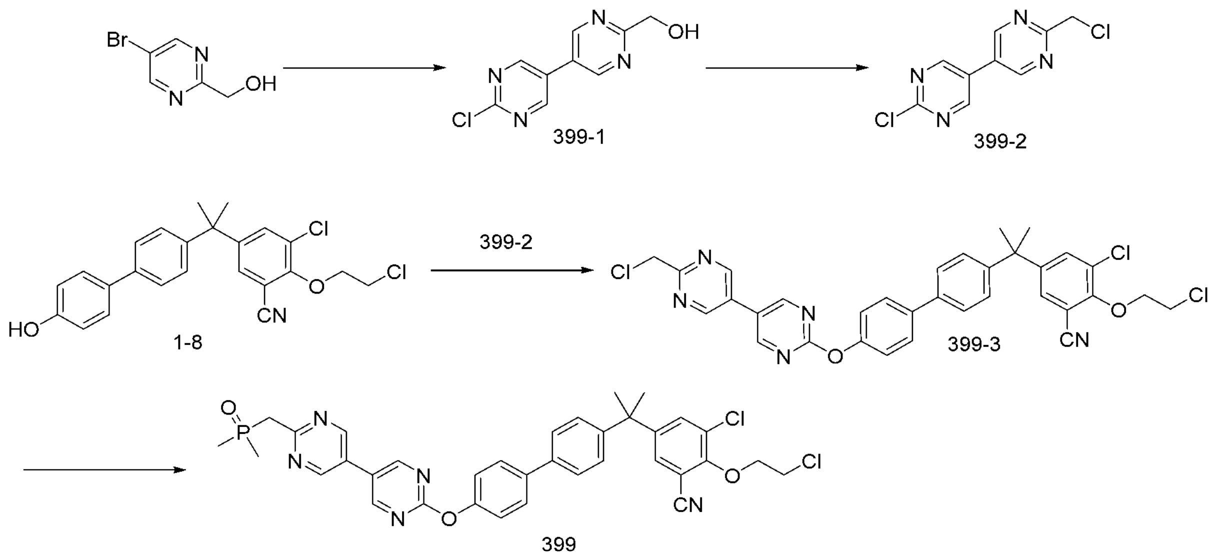


**Preparation of compound 399-1**

In a 100 mL single-necked flask, (5-bromopyrimidin-2-yl)methanol (1 g, 5.29 mmol) was dissolved in 1,4-dioxane (25 mL) and water (4 mL), followed by the sequential addition of 2-chloro-5-(4,4,5,5-tetramethyl-1,3,2-dioxaborolan-2-yl) pyrimidine (1.53 g, 6.35 mmol), potassium carbonate (1.46 g, 10.58 mmol), and Pd(dppf)$Cl_2$ (194 mg, 0.26 mmol). The reaction mixture was stirred at 90°C under a nitrogen atmosphere for 5 h. After the reaction was completed, the reaction mixture was filtered, and the filtrate was subjected to rotary evaporation until dryness under reduced pressure. The crude product was purified by silica gel column chromatography (DCM/MeOH = 9/1) to obtain 390 mg of the title compound **399-1.** LC-MS (ESI): m/z 223.0 [M+H]$^+$.

**Preparation of compound 399-2**

In a 50 mL single-necked flask, compound **399-1** (300 mg, 2.08 mmol) was dissolved in dichloromethane (1.5 mL), followed by the addition of thionyl chloride (1.5 mL). The reaction mixture was stirred at room temperature for 2 h. After

the reaction was completed, the reaction mixture was subjected to rotary evaporation until dryness under reduced pressure to obtain 340 mg of a crude product of the title compound **399-2.** The product was used directly in the next step without purification.

**Preparation of compound 399-3**

In a 25 mL single-necked flask, compound **399-2** (300 mg, 1.24 mmol) was dissolved in DMF (3 mL), followed by the sequential addition of compound **1-8** (265 mg, 0.62 mmol) and cesium carbonate (405 mg, 1.24 mmol). The reaction mixture was stirred at 60°C for 2 h. After the reaction was completed, water (10 mL) was added to the reaction mixture, and the aqueous phase was extracted three times with ethyl acetate (5 mL). The organic phases were combined. The organic phase was washed once with saturated brine, subjected to rotary evaporation until dryness under reduced pressure, and the residue was purified by silica gel column chromatography (DCM/MeOH = 10/1) to obtain 200 mg of the title compound **399-3.** LC-MS (ESI): m/z 630.2 $[M+H]^+$.

**Preparation of compound 399**

In a 50 mL single-necked flask, compound **399-3** (260 mg, 0.41 mmol) was dissolved in NMP (3 mL), followed by the sequential addition of dimethylphosphine oxide (96 mg, 1.24 mmol), $Pd_2(dba)_3$ (38 mg, 0.04 mmol), Xantphos (48 mg, 0.08 mmol), and DIEA (160 mg, 1.24 mmol). The reaction mixture was stirred under microwave irradiation at 120°C under a nitrogen atmosphere for 2 h. After the reaction was completed, water (10 mL) was added to the reaction mixture, and the mixture was extracted three times with ethyl acetate (10 mL). The organic phases were combined, washed once with brine, concentrated to dryness, and then purified by silica gel column chromatography (DCM/MeOH = 10/1) to obtain 20 mg of a crude product (70% purity). The crude product was purified by reversed-phase preparative chromatography (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2.11 mg of the title compound **399.** LC-MS (ESI): m/z 672.2 $[M+H]^+$. $^1$H NMR (400 MHz, Methanol-$d_4$) δ 9.08 (s, 2H), 8.97 (s, 2H), 7.74 - 7.67 (m, 2H), 7.65 - 7.59 (m, 2H), 7.58 (d, *J* = 2.4 Hz, 1H), 7.56 (d, *J* = 2.4 Hz, 1H), 7.39 - 7.32 (m, 2H), 7.32 - 7.26 (m, 2H), 4.43 (t, *J* = 5.5 Hz, 2H), 3.90 (t, *J* = 5.5 Hz, 2H), 3.72 (d, *J* = 15.8 Hz, 2H), 1.73 (s, 6H), 1.68 (d, *J* = 13.4 Hz, 6H). $^{31}$P NMR (162 MHz, Methanol-$d_4$) δ 48.16 (s, 1P).

**Example 400: Preparation of compound 400**

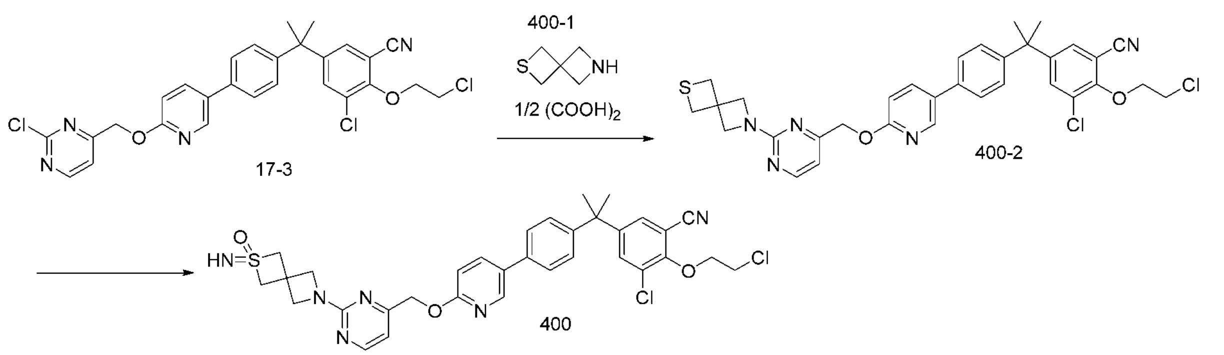


**Preparation of compound 400-2**

Compound **17-3** (80 mg, 0.14 mmol) was added to a microwave tube, followed by the sequential addition of anhydrous ethanol (2 mL), compound **400-1** (139 mg, 0.43 mmol), and DIPEA (56 mg, 0.43 mmol). The system was purged three times with nitrogen, and the reaction was carried out under microwave irradiation at 90°C for 2 h. After the reaction was monitored by LCMS until completion, the reaction mixture was filtered. The crude filtrate was purified by normal-phase silica gel column chromatography (EA/PE = 0 to 60%) to obtain 70 mg of the title compound **400-2** in 77% yield. LC-MS (ESI): m/z 632.0 $[M+H]^+$.

**Preparation of compound 400**

Compound **400-2** (70 mg, 0.11 mmol) and ammonium carbonate (32 mg, 0.33 mmol) were dissolved in methanol

(5 mL). (Diacetoxyiodo)benzene (178 mg, 0.55 mmol) was added thereto at 0°C. After the addition was completed, the reaction was carried out at room temperature overnight. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **400.** LC-MS (ESI): m/z 663.0 $[M+H]^+$. $^1H$ NMR (400 MHz, DMSO-$d_6$) δ 8.42 (dd, *J* = 2.5, 0.7 Hz, 1H), 8.32 (d, *J* = 5.0 Hz, 1H), 8.06 (dd, *J* = 8.6, 2.6 Hz, 1H), 7.71 (d, *J* = 2.4 Hz, 1H), 7.64 (d, *J* = 2.3 Hz, 1H), 7.60 (d, *J* = 8.5 Hz, 2H), 7.33 (d, *J* = 8.5 Hz, 2H), 7.06 (d, *J* = 8.9 Hz, 1H), 6.69 (d, *J* = 5.0 Hz, 1H), 5.29 (s, 2H), 4.49 (s, 1H), 4.42 (t, *J* = 5.2 Hz, 2H), 4.31 (d, *J* = 12.9 Hz, 2H), 4.26 - 4.17 (m, 6H), 3.96 (t, *J* = 5.2 Hz, 2H), 1.68 (s, 6H).

**Example 401: Preparation of compound 401**

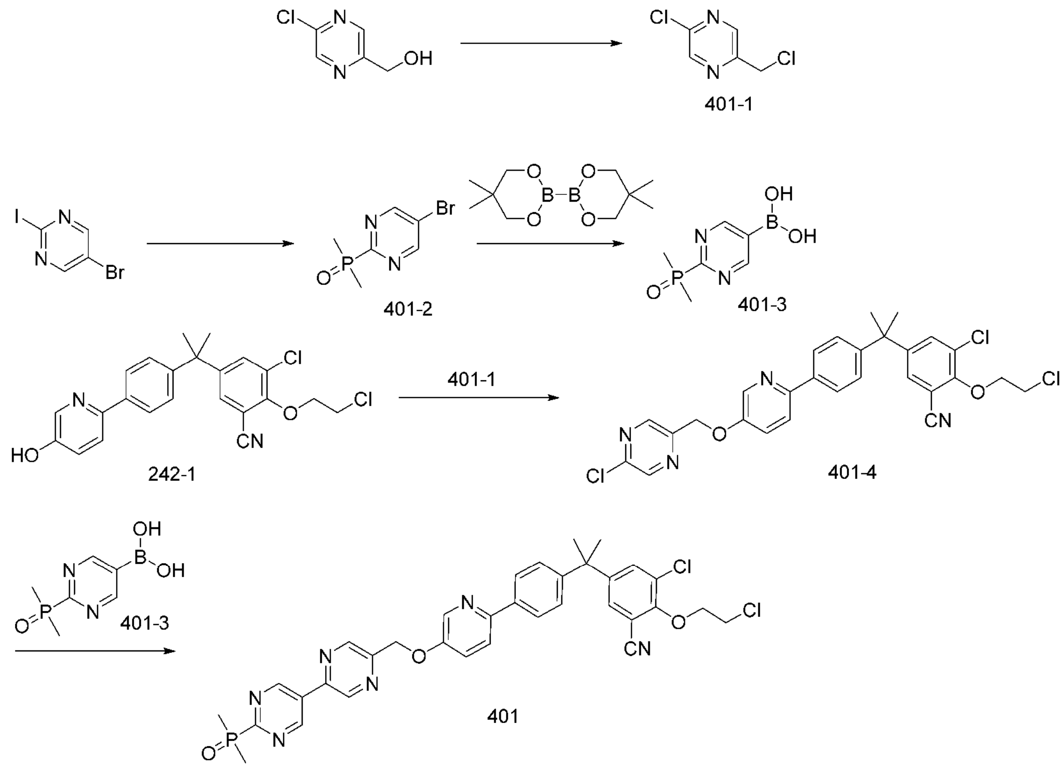

401-1
401-2
401-3
242-1
401-4
401-3
401

**Preparation of compound 401-1**

In a 100 mL single-necked flask, (5-chloropyrazin-2-yl)methanol (100 mg, 0.69 mmol) was dissolved in dichloromethane (0.5 mL). The reaction mixture was cooled to 0°C, and then thionyl chloride (0.5 mL) was added. The reaction mixture was stirred at 60°C for 2 h. After the reaction was completed, the reaction mixture was subjected to rotary evaporation until dryness under reduced pressure to obtain 170 mg of a crude product of the title compound **401-1.** The product was used directly in the next step without purification.

**Preparation of compound 401-2**

2-Iodo-5-bromopyrimidine (1.0 g, 3.51 mmol), dimethylphosphine oxide (301 mg, 3.86 mmol), $Pd_2(dba)_3$ (96 mg, 0.11 mmol), Xantphos (122 mg, 0.211 mmol), and potassium phosphate (820 mg, 3.86 mmol) were dissolved in 1,4-dioxane (20 mL). After the addition was completed, the system was purged three times with argon and stirred at 100°C for 4 h. After the reaction was completed, insoluble solids were removed by filtration, and the filtrate was concentrated to obtain a crude product. The crude product was purified by silica gel column chromatography (DCM/PE = 9/10) to obtain 400 mg of the title compound **401-2.** LC-MS (ESI): m/z 235.0 $[M+H]^+$.

**Preparation of compound 401-3**

Compound **401-2** (320 mg, 1.36 mmol), neopentyl glycol diboronate (615 mg, 2.72 mmol), Pd(dppf)$Cl_2$ (100 mg, 0.136 mmol), and potassium acetate (401 mg, 4.08 mmol) were dissolved in 1,4-dioxane (10 mL). After the addition was completed, the system was purged three times with argon and stirred at 110°C for 2 h. After the reaction was completed, insoluble solids were removed by filtration, and the filtrate was concentrated to obtain a crude product. The crude product was purified by silica gel column chromatography (DCM/PE = 9/10) to obtain 300 mg of the title compound **401-3.** LC-MS (ESI): m/z 201.0 $[M+H]^+$.

**Preparation of compound 401-4**

In a 50 mL single-necked flask, compound **401-1** (170 mg) was dissolved in DMF (5 mL), and **242-1** (295 mg, 0.69 mmol) and cesium carbonate (450 mg, 1.38 mmol) were added. The reaction mixture was stirred at 60°C for 2 h. After the reaction was completed, water (30 mL) was added to the reaction mixture. The aqueous phase was extracted three times with ethyl acetate (20 mL). The organic phases were combined, washed three times with saturated brine, and subjected to rotary evaporation until dryness under reduced pressure. The residue was purified by silica gel column chromatography (DCM/MeOH = 10/1) to obtain 200 mg of the title compound **401-4.** LC-MS (ESI): m/z 553.0 $[M+H]^+$.

**Preparation of compound 401**

In a 50 mL single-necked flask, compound **401-4** (111 mg, 0.20 mmol) was dissolved in 1,4-dioxane (4 mL) and water (1 mL), followed by the sequential addition of 5-(5,5-dimethyl-1,3,2-dioxaborolan-2-yl)-2-dimethylphosphorylpyrimidine (180 mg, 0.27 mmol), Pd(dppf)$Cl_2$ (20 mg, 0.03 mmol), and potassium carbonate (74 mg, 0.54 mmol). The reaction mixture was stirred at 110°C under a nitrogen atmosphere for 4 h. After the reaction was completed, the reaction mixture was filtered, and the filtrate was concentrated to dryness under reduced pressure. The resulting residue was purified by silica gel column chromatography (DCM/MeOH = 10/1), and then purified by reversed-phase preparative chromatography (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 11.34 mg of the title compound **401.** LC-MS (ESI): m/z 673.0 $[M+H]^+$. $^1H$ NMR (400 MHz, DMSO-$d_6$) δ 9.66 (s, 2H), 9.48 (d, *J* = 1.5 Hz, 1H), 9.05 (d, *J* = 1.5 Hz, 1H), 8.51 (d, *J* = 3.0 Hz, 1H), 7.96 (d, *J* = 8.6 Hz, 2H), 7.93 (d, *J* = 8.8 Hz, 1H), 7.70 (d, *J* = 2.3 Hz, 1H), 7.67 - 7.58 (m, 2H), 7.34 (d, *J* = 8.6 Hz, 2H), 5.51 (s, 2H), 4.42 (t, *J* = 5.2 Hz, 2H), 3.96 (t, *J* = 5.2 Hz, 2H), 1.82 (d, *J* = 13.7 Hz, 6H), 1.69 (s, 6H). $^{31}P$ NMR (162 MHz, DMSO-$d_6$) δ 34.28 (s, 1P).

**Example 402: Preparation of compound 402**

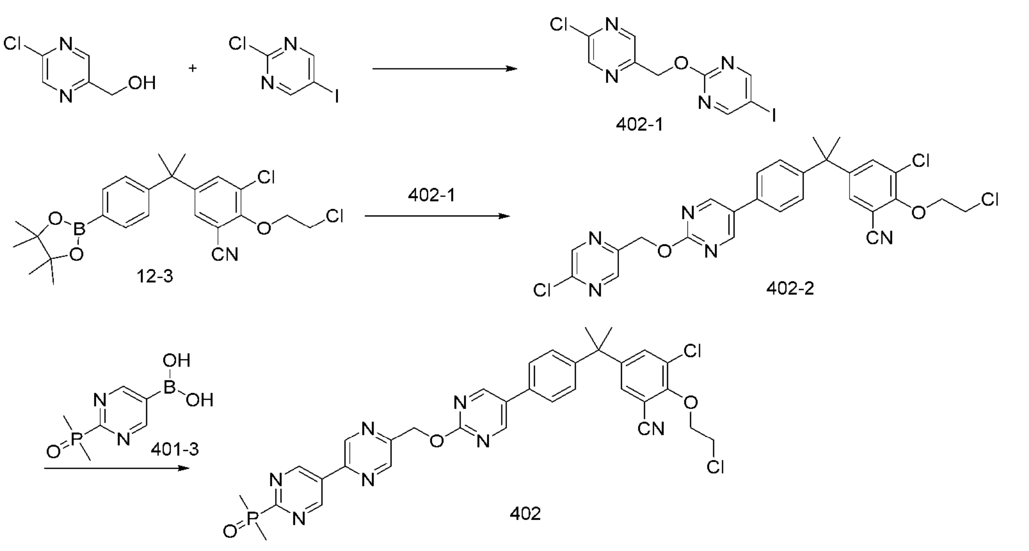

402-1
12-3
402-2
401-3
402

**Preparation of compound 402-1**

2-Chloro-5-iodopyrimidine (2.0 g, 8.32 mmol), (5-chloropyrazin-2-yl)methanol (1.20 g, 8.32 mmol), and cesium

carbonate (5.42 g, 16.64 mmol) were dissolved in DMF (10 mL). After the addition was completed, the system was purged three times with argon and stirred at 45°C for 2 h. After the reaction was completed, insoluble solids were removed by filtration, and the filtrate was concentrated to obtain a crude product. The crude product was purified by silica gel column chromatography (DCM/PE = 9/10) to obtain 2.5 g of the title compound 402-1. LC-MS (ESI): m/z 349.0 $[M+H]^+$.

**Preparation of compound 402-2**

Compound **402-1** (1.90 g, 5.45 mmol), compound **12-3** (2.51 g, 5.45 mmol), $Pd(dppf)Cl_2$ (399 mg, 0.545 mmol), and potassium carbonate (2.26 g, 16.35 mmol) were dissolved in a mixed solution of 1,4-dioxane (50 mL) and $H_2O$ (10 mL). After the addition was completed, the system was purged three times with argon and stirred at 90°C for 16 h. After the reaction was completed, insoluble solids were removed by filtration, and the filtrate was concentrated to obtain a crude product. The crude product was purified by silica gel column chromatography (DCM/PE = 9/10) to obtain 2 g of the title compound **402-2.** LC-MS (ESI): m/z 554.2 $[M+H]^+$.

**Preparation of compound 402**

Compound **402-2** (200 mg, 0.36 mmol), (2-(dimethylphosphoryl)pyrimidin-4-yl)boronic acid (72 mg, 0.36 mmol), $Pd(dppf)Cl_2$ (26 mg, 0.036 mmol), and potassium carbonate (150 mg, 1.08 mmol) were dissolved in a mixed solution of 1,4-dioxane (15 mL) and $H_2O$ (3 mL). After the addition was completed, the system was purged three times with argon and stirred at 110°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 58 mg of the title compound **402.** LC-MS (ESI): m/z 674.0 $[M+H]^+$. $^1H$ NMR (400 MHz, DMSO-$d_6$) δ 9.65 (s, 2H), 9.45 (d, *J* = 1.5 Hz, 1H), 9.00 - 8.92 (m, 3H), 7.71 (d, *J* = 2.3 Hz, 1H), 7.70 - 7.66 (m, 2H), 7.65 (d, *J* = 2.4 Hz, 1H), 7.37 (d, *J* = 8.3 Hz, 2H), 5.70 (s, 2H), 4.42 (t, *J* = 5.2 Hz, 2H), 3.96 (t, *J* = 5.2 Hz, 2H), 1.82 (d, *J* = 13.7 Hz, 6H), 1.69 (s, 6H). $^{31}P$ NMR (162 MHz, DMSO-$d_6$) δ 34.28 (s, 1P).

**Example 403: Preparation of compound 403**

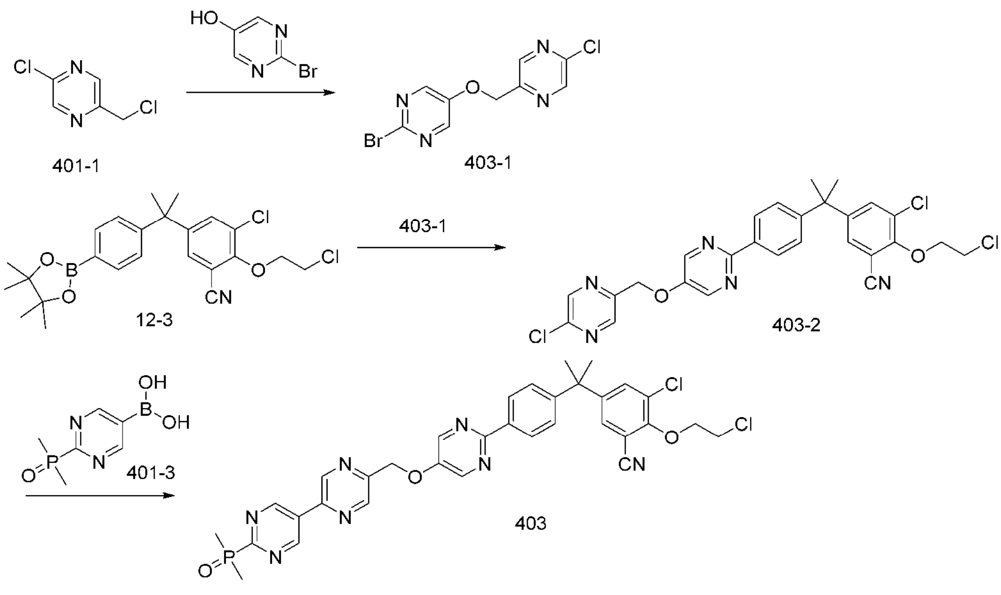


**Preparation of compound 403-1**

Compound **401-1** (1.35 g, 8.28 mmol), 2-bromo-5-hydroxypyrimidine (1.45 g, 8.28 mmol), and cesium carbonate (5.40 g, 16.56 mmol) were dissolved in DMF (10 mL). After the addition was completed, the system was purged three times

with argon and stirred at 40°C for 3 h. After the reaction was completed, insoluble solids were removed by filtration, and the filtrate was concentrated to obtain a crude product. The crude product was purified by silica gel column chromatography (DCM/PE = 9/10) to obtain 2 g of the title compound **403-1.** LC-MS (ESI): m/z 300.9 $[M+H]^+$.

**Preparation of compound 403-2**

Compound **403-1** (1.9 g, 6.30 mmol), compound **12-3** (2.90 g, 6.30 mmol), Pd(dppf)$Cl_2$ (461 mg, 0.63 mmol), and potassium carbonate (2.61 g, 18.90 mmol) were dissolved in a mixed solution of 1,4-dioxane (50 mL) and $H_2O$ (10 mL). After the addition was completed, the system was purged three times with argon and stirred at 110°C for 16 h. After the reaction was completed, insoluble solids were removed by filtration, and the filtrate was concentrated to obtain a crude product. The crude product was purified by silica gel column chromatography (DCM/PE = 9/10) to obtain 2 g of the title compound **403-2.** LC-MS (ESI): m/z 554.1 $[M+H]^+$.

**Preparation of compound 403**

Compound **403-2** (250 mg, 0.45 mmol), compound **401-3** (90 mg, 0.45 mmol), Pd(dppf)$Cl_2$ (33 mg, 0.045 mmol), and potassium carbonate (187 mg, 1.35 mmol) were dissolved in a mixed solution of 1,4-dioxane (5 mL) and $H_2O$ (1 mL). After the addition was completed, the system was purged three times with argon and stirred at 110°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 37 mg of the title compound **403.** LC-MS (ESI): m/z 674.0 $[M+H]^+$. $^1$H NMR (400 MHz, DMSO-$d_6$) δ 9.66 (s, 2H), 9.49 (d, *J* = 1.5 Hz, 1H), 9.07 (d, *J* = 1.5 Hz, 1H), 8.80 (s, 2H), 8.25 (d, *J* = 8.5 Hz, 2H), 7.70 (d, *J* = 2.4 Hz, 1H), 7.63 (d, *J* = 2.3 Hz, 1H), 7.39 (d, *J* = 8.6 Hz, 2H), 5.60 (s, 2H), 4.42 (t, *J* = 5.2 Hz, 2H), 3.96 (t, *J* = 5.2 Hz, 2H), 1.82 (d, *J* = 13.7 Hz, 6H), 1.70 (s, 6H). $^{31}$P NMR (162 MHz, DMSO-$d_6$) δ 34.31 (s, 1P).

**Example 404: Preparation of compound 404**

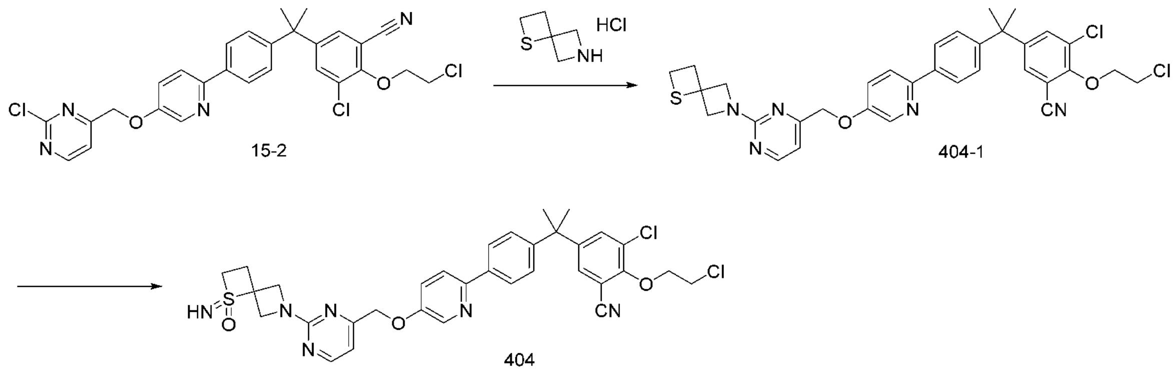


15-2

404-1

404

**Preparation of compound 404-1**

Compound **15-2** (100 mg, 180.55 μmol), 1-thia-6-azaspiro[3.3]heptane hydrochloride (41 mg, 270.82 μmol), DIPEA (70 mg, 541.65 μmol), and acetonitrile (1 mL) were added to a reaction flask, and the mixture was stirred under microwave irradiation at 80°C for 2 h. The mixture was subjected to rotary evaporation until dryness. The crude product was purified by silica gel column chromatography (MeOH/DCM = 0 to 5%) to obtain 100 mg of the title compound **404-1.** LC-MS (ESI): m/z 632.2 $[M+H]^+$.

**Preparation of compound 404**

Compound **404-1** (100 mg, 158.08 μmol), (diacetoxyiodo)benzene (153 mg, 474.23 μmol), and ammonium carbonate (62 mg, 0.79 mmol) were dissolved in methanol (10 mL). The mixture was stirred at room temperature for 16 h,

then subjected to rotary evaporation until dryness. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 95% acetonitrile gradient elution over 25 min; flow rate: 30 mL/min) to obtain 50 mg of the title compound **404.** LC-MS (ESI): m/z 663.2 $[M+H]^+$. $^1H$ NMR (400 MHz, DMSO-$d_6$) δ 8.44 (d, *J* = 3.0 Hz, 1H), 8.42 (d, *J* = 5.0 Hz, 1H), 7.94 (d, *J* = 8.5 Hz, 2H), 7.88 (d, *J* = 8.8 Hz, 1H), 7.69 (d, *J* = 2.4 Hz, 1H), 7.61 (d, *J* = 2.4 Hz, 1H), 7.53 (dd, *J* = 8.8, 3.1 Hz, 1H), 7.33 (d, *J* = 8.5 Hz, 2H), 6.88 (d, *J* = 5.0 Hz, 1H), 5.17 (s, 2H), 4.80 (d, *J* = 1.4 Hz, 1H), 4.51 (dd, *J* = 10.3, 1.3 Hz, 1H), 4.46 - 4.36 (m, 3H), 4.25 (d, *J* = 10.3 Hz, 1H), 4.19 (d, *J* = 10.3 Hz, 1H), 3.95 (t, *J* = 5.2 Hz, 2H), 3.92 - 3.80 (m, 2H), 2.48 - 2.29 (m, 2H), 1.68 (s, 6H).

**Example 405: Preparation of compound 405**

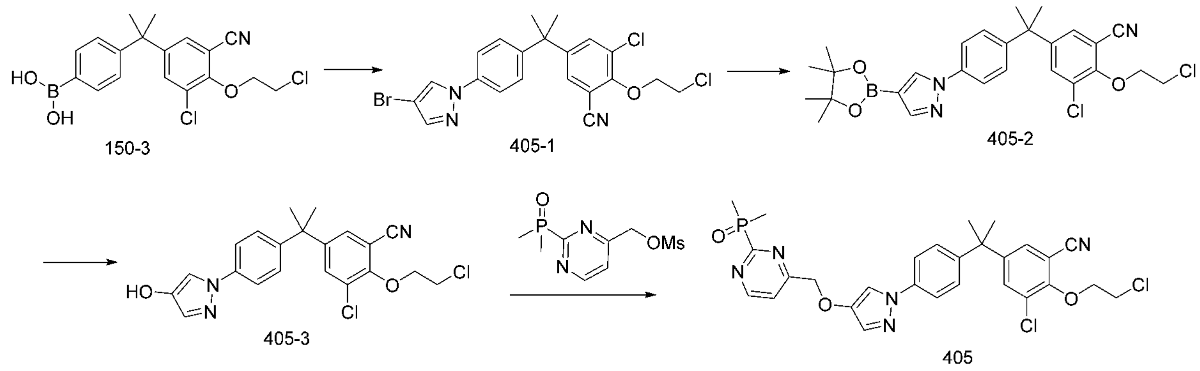

150-3 405-1 405-2 405-3 405

**Preparation of compound 405-1**

Compound **150-3** (420 mg, 1.11 mmol), 4-bromopyrazole (164 mg, 1.11 mmol), anhydrous copper(II) acetate (403 mg, 2.22 mmol), pyridine (176 mg, 2.22 mmol), and DCM (5 mL) were added to a reaction flask, and the mixture was stirred at room temperature for 16 h. Saturated aqueous ammonium chloride solution (10 mL) was added, and the mixture was extracted three times with dichloromethane (5 mL). The organic phases were combined, subjected to rotary evaporation until dryness, and the crude product was purified by normal-phase silica gel column chromatography (EA/PE = 0 to 30%) to obtain 340 mg of compound **405-1.** LC-MS (ESI): m/z 477.8 $[M+H]^+$.

**Preparation of compound 405-2**

Under a nitrogen atmosphere, compound **405-1** (340 mg, 709.52 μmol), bis(pinacolato)diboron (270 mg, 1.06 mmol), potassium acetate (209 mg, 2.13 mmol), Pd(dppf)$Cl_2$ (52 mg, 70.95 μmol), and 1,4-dioxane (5 mL) were added to a reaction flask, and the mixture was stirred at 100°C for 16 h. Water (10 mL) was added, and the mixture was extracted three times with ethyl acetate (10 mL). The organic phases were combined, dried over anhydrous sodium sulfate, filtered, and the filtrate was subjected to rotary evaporation until dryness to obtain 400 mg of a crude product of the title compound **405-2.** LC-MS (ESI): m/z 526.2 $[M+H]^+$.

**Preparation of compound 405-3**

Compound **405-2** (200 mg, 380.04 μmol), water (5 mL), THF (5 mL), and potassium carbonate (158 mg, 1.14 mmol) were added to a reaction flask. The system was cooled to 0°C, and then 30% hydrogen peroxide (0.5 mL) was added dropwise. After stirring for 2 h, saturated aqueous sodium sulfite solution (10 mL) was slowly added at 0°C to quench the reaction. The mixture was extracted three times with dichloromethane (10 mL). The organic phases were combined and subjected to rotary evaporation until dryness. The crude product was purified by silica gel column chromatography (EA/PE = 0% to 50%) to obtain 140 mg of the title compound **405-3.** LC-MS (ESI): m/z 415.8 $[M+H]^+$.

**Preparation of compound 405**

Compound **405-3** (20 mg, 48.04 μmol) , (2-(dimethylphosphoryl)pyrimidin-4-yl)methyl methanesulfonate (20 mg, 72.06 μmol), potassium carbonate (20 mg, 144.13 μmol), and acetonitrile (2 mL) were added to a reaction flask, and the mixture was stirred at 80°C for 1 h. The mixture was filtered and subjected to rotary evaporation until dryness. The

crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 85% acetonitrile gradient elution over 25 min; flow rate: 30 mL/min) to obtain 15 mg of the title compound **405.** LC-MS (ESI): m/z 584.2 [M+H]$^+$. $^1$H NMR (400 MHz, DMSO-$d_6$) δ 9.00 (d, *J* = 5.1 Hz, 1H), 8.43 (s, 1H), 7.75 - 7.65 (m, 5H), 7.61 (d, *J* = 2.3 Hz, 1H), 7.35 (d, *J* = 8.8 Hz, 2H), 5.24 (s, 2H), 4.42 (t, *J* = 5.2 Hz, 2H), 3.95 (t, *J* = 5.2 Hz, 2H), 1.76 (d, *J* = 13.8 Hz, 6H), 1.68 (s, 6H). $^{31}$P NMR (162 MHz, DMSO-$d_6$) δ 34.07 (s, 1P).

**Example 406: Preparation of compound 406**

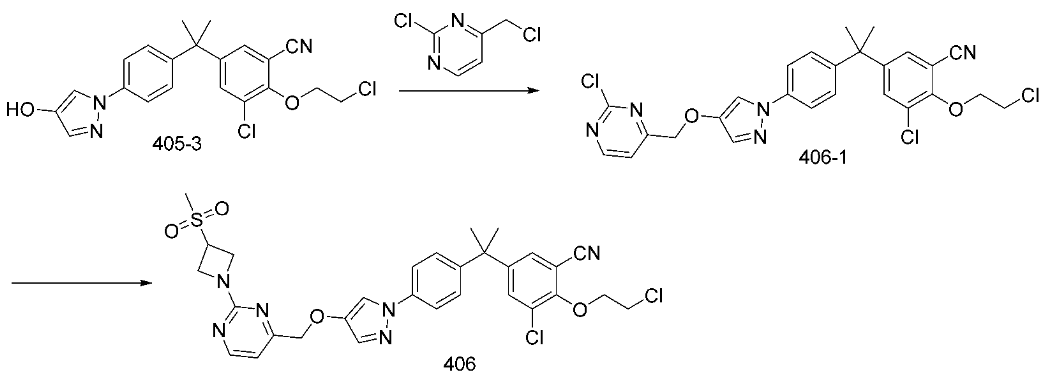

405-3

406-1

406

**Preparation of compound 406-1**

Compound **405-3** (100 mg, 240.21 μmol), 2-chloro-4-(chloromethyl)pyrimidine (59 mg, 360.32 μmol), potassium carbonate (100 mg, 720.63 μmol), and acetonitrile (2 mL) were added to a reaction flask. The reaction system was stirred at 75°C for 1 h and then at 80°C for 1 h. The mixture was cooled to room temperature, filtered, and the filtrate was subjected to rotary evaporation until dryness. The resulting crude product was purified by silica gel column chromatography (MeOH/DCM = 0 to 5%) to obtain 80 mg of the title compound **406-1.** LC-MS (ESI): m/z 542.0 [M+H]$^+$.

**Preparation of compound 406**

Compound **406-1** (40 mg, 73.69 μmol), 1-thia-6-azaspiro[3.3]heptane hydrochloride (38 mg, 221.06 μmol), DIPEA (29 mg, 221.06 μmol), and acetonitrile (1 mL) were added to a reaction flask, and the mixture was stirred under microwave irradiation at 80°C for 2 h. The mixture was cooled to room temperature, and the filtrate was subjected to rotary evaporation until dryness. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 30% to 90% acetonitrile gradient elution over 20 min; flow rate: 30 mL/min) to obtain 20 mg of the title compound **406.** LC-MS (ESI): m/z 641.2 [M+H]$^+$. $^1$H NMR (400 MHz, DMSO-$d_6$) δ 8.43 (d, *J* = 5.0 Hz, 1H), 8.37 (d, *J* = 0.8 Hz, 1H), 7.73 - 7.65 (m, 3H), 7.63 (d, *J* = 0.7 Hz, 1H), 7.61 (d, *J* = 2.4 Hz, 1H), 7.34 (d, *J* = 8.8 Hz, 2H), 6.87 (d, *J* = 5.0 Hz, 1H), 4.98 (s, 2H), 4.47 - 4.37 (m, 3H), 4.33 (t, *J* = 8.7 Hz, 2H), 4.27 - 4.14 (m, 2H), 3.95 (t, 2H), 3.06 (s, 3H), 1.67 (s, 6H).

**Example 407: Preparation of compound 407**

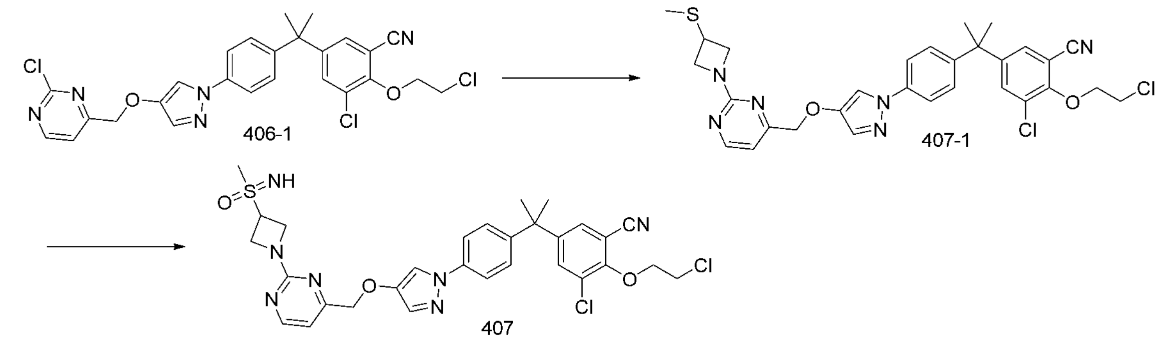

406-1 407-1 407

**Preparation of compound 407-1**

Compound **406-1** (40 mg, 73.69 μmol), 3-(methylthio)azetidine hydrochloride (31 mg, 221.06 μmol), DIPEA (29 mg, 221.06 μmol), and acetonitrile (1 mL) were added to a reaction flask, and the mixture was stirred under microwave irradiation at 80°C for 2 h. The mixture was subjected to rotary evaporation until dryness. The crude product was purified by silica gel column chromatography (MeOH/DCM = 0 to 5%) to obtain 30 mg of the title compound **407-1.** LC-MS (ESI): m/z 609.0 $[M+H]^+$.

**Preparation of compound 407**

Compound **407-1** (30 mg, 49.22 μmol) , (diacetoxyiodo)benzene (48 mg, 147.65 μmol) , and ammonium carbonate (30 mg, 0.4 mmol) were dissolved in methanol (2 mL). The mixture was stirred at room temperature for 1 h. Water (5 mL) was added, and the mixture was extracted three times with dichloromethane (5 mL). The organic phases were combined and subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 90% acetonitrile gradient elution over 20 min; flow rate: 30 mL/min) to obtain 15 mg of the title compound **407.** LC-MS (ESI): m/z 640.0 $[M+H]^+$. $^1H$ NMR (400 MHz, DMSO-$d_6$) δ 8.41 (d, *J* = 5.0 Hz, 1H), 8.37 (s, 1H), 7.73 - 7.65 (m, 3H), 7.63 (s, 1H), 7.61 (d, *J* = 2.3 Hz, 1H), 7.34 (d, *J* = 8.8 Hz, 2H), 6.84 (d, *J* = 5.0 Hz, 1H), 4.97 (s, 2H), 4.42 (t, *J* = 5.2 Hz, 2H), 4.34 - 4.17 (m, 5H), 3.98 (s, 1H), 3.95 (t, *J* = 5.2 Hz, 2H), 2.91 (s, 3H), 1.67 (s, 6H).

**Example 408: Preparation of compound 408**

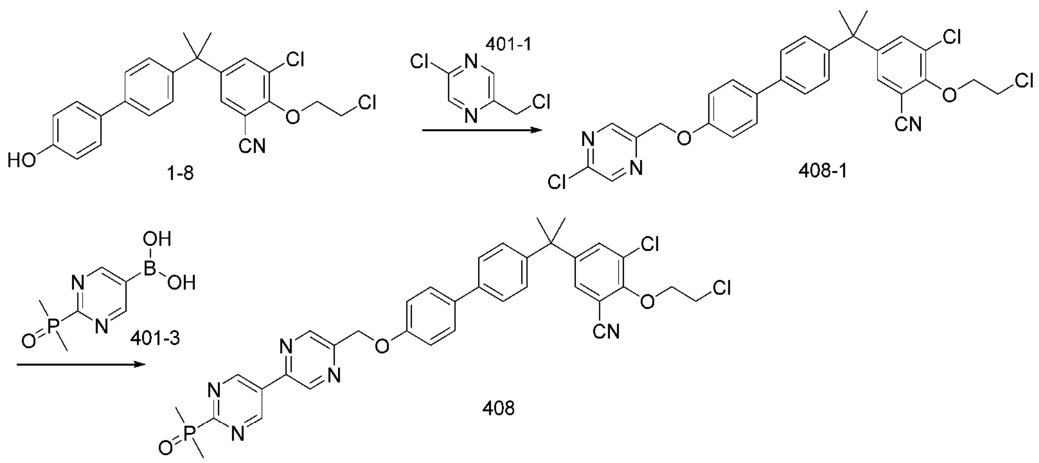

1-8 401-1 408-1 401-3 408

**Preparation of compound 408-1**

In a 50 mL single-necked flask, compound **401-1** (110 mg, crude) was dissolved in DMF (3 mL), followed by the addition of compound **1-8** (287 mg, 0.67 mmol) and cesium carbonate (440 mg, 1.35 mmol). The reaction mixture was stirred at 60°C for 2 h. After the reaction was completed, water (20 mL) was added to the reaction mixture. The aqueous

phase was extracted three times with ethyl acetate (20 mL). The organic phases were combined, washed three times with saturated brine, and subjected to rotary evaporation until dryness under reduced pressure. The residue was purified by silica gel column chromatography (DCM/MeOH = 10/1) to obtain 200 mg of the title compound **408-1.** LC-MS (ESI): m/z 552.0 $[M+H]^+$.

**Preparation of compound 408**

In a 50 mL single-necked flask, compound **408-1** (104 mg, 0.19 mmol) was dissolved in 1,4-dioxane (2 mL) and water (0.5 mL), followed by the sequential addition of compound **401-3** (180 mg, 0.27 mmol), $Pd(dppf)Cl_2$ (20 mg, 0.03 mmol), and potassium carbonate (74 mg, 0.54 mmol). The reaction mixture was stirred at 110°C under a nitrogen atmosphere for 4 h. After the reaction was completed, the reaction mixture was filtered, and the filtrate was concentrated to dryness under reduced pressure. The resulting residue was purified by silica gel column chromatography (DCM/MeOH = 10/1), and then purified by reversed-phase preparative chromatography (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 35.97 mg of the title compound **408.** LC-MS (ESI): m/z 672.0 $[M+H]^+$. $^1$H NMR (400 MHz, DMSO-$d_6$) δ 9.66 (s, 2H), 9.47 (d, *J*= 1.5 Hz, 1H), 9.01 (d, *J* = 1.5 Hz, 1H), 7.70 (d, *J* = 2.3 Hz, 1H), 7.66 - 7.59 (m, 3H), 7.59 - 7.54 (m, 2H), 7.34 - 7.28 (m, 2H), 7.21 - 7.14 (m, 2H), 5.42 (s, 2H), 4.42 (t, *J* = 5.2 Hz, 2H), 3.96 (t, *J* = 5.2 Hz, 2H), 1.82 (d, *J* = 13.8 Hz, 6H), 1.68 (s, 6H). $^{31}$P NMR (162 MHz, Methanol-$d_4$) δ 34.27(s, 1P).

**Example 409: Preparation of compound 409**

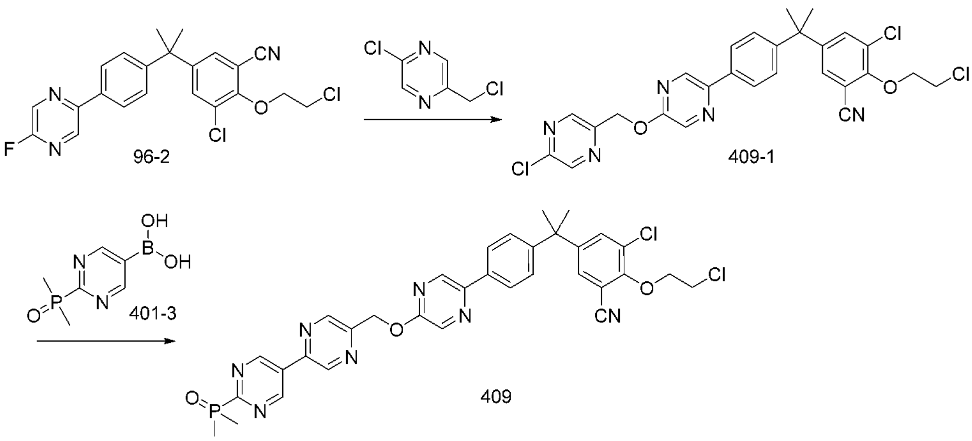

**Preparation of compound 409-1**

In a 50 mL single-necked flask, compound **96-2** (238 mg, 0.55 mmol) was dissolved in DMF (3 mL), followed by the addition of 2-chloro-5-(chloromethyl)pyrazine (80 mg, 0.55 mmol) and cesium carbonate (360 mg, 1.11 mmol). The reaction mixture was stirred at 80°C for 3 h. After the reaction was completed, water (20 mL) was added to the reaction mixture. The aqueous phase was extracted three times with ethyl acetate (20 mL). The organic phases were combined, washed three times with saturated brine, and subjected to rotary evaporation until dryness under reduced pressure. The residue was purified by silica gel column chromatography (DCM/MeOH = 10/1) to obtain 180 mg of the title compound **409-1.** LC-MS (ESI): m/z 553.9 $[M+H]^+$.

**Preparation of compound 409**

In a 50 mL single-necked flask, compound **409-1** (181 mg, 0.27 mmol) was dissolved in 1,4-dioxane (4 mL) and water (1 mL), followed by the sequential addition of compound **401-3** (150 mg, 0.27 mmol), $Pd(dppf)Cl_2$ (20 mg, 0.03 mmol), and potassium carbonate (74 mg, 0.54 mmol). The reaction mixture was stirred at 110°C under a nitrogen atmosphere for 4 h. After the reaction was completed, the reaction mixture was filtered, and the filtrate was concentrated to dryness under reduced pressure. The resulting residue was purified by column chromatography (DCM/MeOH = 10/1), and further purified by reversed-phase preparative chromatography (preparative method: mobile phase: A: 0.1% formic acid in water; B: acetonitrile; chromatographic column: Welch Ultimate AQ-C18 250 × 21.2 mm; column temperature: 25°C;

gradient: 45% to 65% acetonitrile gradient elution over 12 min; flow rate: 30 mL/min) to obtain 36.46 mg of the title compound **409.** LC-MS (ESI): m/z 674.0 [M+H]$^+$. $^1$H NMR (400 MHz, DMSO-$d_6$) δ 9.64 (s, 2H), 9.45 (d, *J* = 1.5 Hz, 1H), 9.00 (d, *J* = 1.5 Hz, 1H), 8.78 (d, *J* = 1.5 Hz, 1H), 8.54 (d, *J* = 1.4 Hz, 1H), 7.97 (d, *J* = 8.5 Hz, 2H), 7.70 (d, *J*= 2.3 Hz, 1H), 7.63 (d, *J* = 2.3 Hz, 1H), 7.37 (d, *J* = 8.4 Hz, 2H), 5.69 (s, 2H), 4.42 (t, *J* = 5.2 Hz, 2H), 3.95 (t, *J* = 5.2 Hz, 2H), 1.82 (d, *J* = 13.8 Hz, 6H), 1.69 (s, 6H). $^{31}$P NMR (162 MHz, DMSO-$d_6$) δ 34.25 (s, 1P).

**Example 410: Preparation of compound 410**

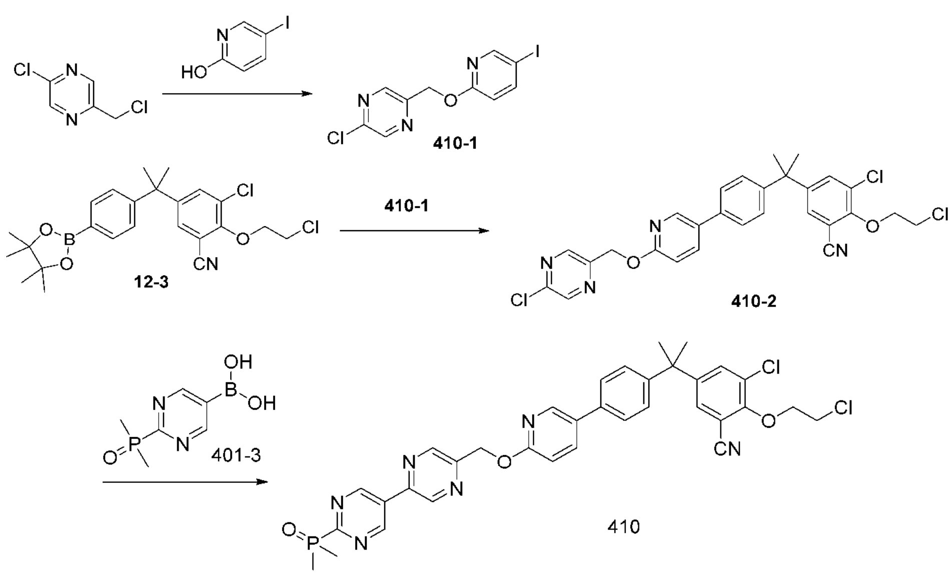


**Preparation of compound 410-1**

In a 50 mL single-necked flask, 2-chloro-5-(chloromethyl)pyrazine (350 mg, 2.15 mmol) was dissolved in DMF (5 mL), followed by addition of 2-hydroxy-5-iodopyridine (522 mg, 2.36 mmol) and cesium carbonate (1.40 g, 4.29 mmol). The reaction mixture was stirred at 60°C for 2 h. After the reaction was completed, water (20 m) was added to the reaction mixture. The aqueous phase was extracted three times with ethyl acetate (20 mL). The organic phases were combined, then washed three times with saturated brine, and subjected to rotary evaporation until dryness under reduced pressure. The residue was purified by silica gel column chromatography (DCM/MeOH = 9/1) to obtain 130 mg of the title compound **410-1.** $^1$H NMR (400 MHz, DMSO-$d_6$) δ 8.80 (d, *J* = 1.4 Hz, 1H), 8.62 (d, *J* = 1.4 Hz, 1H), 8.37 (dd, *J* = 2.4, 0.7 Hz, 1H), 8.05 (dd, *J* = 8.7, 2.3 Hz, 1H), 6.87 (dd, *J* = 8.7, 0.7 Hz, 1H), 5.47 (s, 2H).

**Preparation of compound 410-2**

In a 25 mL single-necked flask, compound **410-1** (130 mg, 0.37 mmol) was dissolved in 1,4-dioxane (2 mL) and water (0.5 mL), followed by the addition of compound **12-3** (172 mg, 0.37 mmol), Pd(dppf)$Cl_2$ (27 mg, 0.04 mmol), and potassium carbonate (103 mg, 0.75 mmol). The reaction mixture was stirred at 80°C for 4 h. After the reaction was completed, the reaction mixture was filtered, and the filtrate was subjected to rotary evaporation until dryness under reduced pressure. The crude product was purified by silica gel column chromatography (DCM/MeOH = 9/1) to obtain 180 mg of the title compound **410-2.** LC-MS (ESI): m/z 554.0 [M+H]$^+$.

**Preparation of compound 410**

In a 50 mL single-necked flask, compound **410-2** (150 mg, 0.27 mmol) was dissolved in 1,4-dioxane (2 mL) and water (0.5 mL), followed by the sequential addition of compound **401-3** (181 mg, 0.27 mmol), Pd(dppf)$Cl_2$ (20 mg, 0.03 mmol), and potassium carbonate (75 mg, 0.54 mmol). The reaction mixture was stirred at 110°C under a nitrogen atmosphere for 4 h. After the reaction was completed, the reaction mixture was filtered, and the filtrate was concentrated to dryness under reduced pressure. The resulting residue was purified by silica gel column chromatography (DCM/MeOH = 10/1), and then purified by reversed-phase preparative chromatography (preparative method: mobile phase: A: 0.1% formic acid in water; B: acetonitrile; chromatographic column: Welch Ultimate AQ-C18 250 × 21.2 mm; column

temperature: 25°C; gradient: 45% to 65% acetonitrile gradient elution over 12 min; flow rate: 30 mL/min) to obtain 60.62 mg of the title compound **410.** LC-MS (ESI): m/z 673.2 [M+H]+. $^{1}$H NMR (400 MHz, DMSO-$d_6$) δ 9.64 (s, 2H), 9.44 (d, *J* = 1.5 Hz, 1H), 8.95 (d, *J* = 1.5 Hz, 1H), 8.47 (d, *J* = 2.6 Hz, 1H), 8.08 (dd, *J* = 8.6, 2.6 Hz, 1H), 7.71 (d, *J* = 2.4 Hz, 1H), 7.64 (d, *J* = 2.3 Hz, 1H), 7.61 (d, *J* = 8.5 Hz, 2H), 7.34 (d, *J* = 8.5 Hz, 2H), 7.08 (d, *J* = 8.6 Hz, 1H), 5.64 (s, 2H), 4.42 (t, *J* = 5.2 Hz, 2H), 3.96 (t, *J* = 5.2 Hz, 2H), 1.82 (d, *J* = 13.8 Hz, 6H), 1.69 (s, 6H). $^{31}$P NMR (162 MHz, Methanol-$d_4$) δ 34.24 (s, 1P).

**Example 411: Preparation of compound 411**

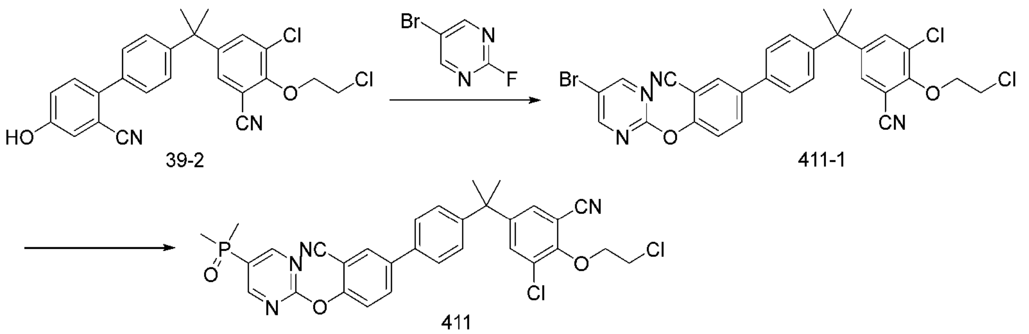

39-2

411-1

411

**Preparation of compound 411-1**

Compound **39-2** (300 mg, 0.66 mmol) was dissolved in acetonitrile (5 mL), followed by the sequential addition of 2-fluoro-5-bromopyrimidine (234 mg, 1.32 mmol) and potassium carbonate (180 mg, 1.32 mmol). The reaction system was stirred at 80°C for 2 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (PE/EA = 30%) to obtain 290 mg of the title compound **411-1.** LC-MS (ESI): m/z 607.3[M+H]+.

**Preparation of compound 411**

Compound **411-1** (145 mg, 0.24 mmol) was dissolved in DMF (3 mL), followed by the sequential addition of dimethylphosphine oxide (43 mg, 0.48 mmol), X-Phos (20 mg, 0.04 mmol), $Pd_2(dba)_3$ (18 mg, 0.02 mmol), and potassium carbonate (234 mg, 0.72 mmol). The reaction system was stirred at 80°C for 2 h. After the reaction was completed, water (3 mL) was added to the filtrate, and the mixture was extracted three times with dichloromethane (5 mL). The organic phases were combined, washed twice with saturated brine (3 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2 mg of the title compound **411.** LC-MS (ESI): m/z 604.9 [M+H]+. $^{1}$H NMR (400 MHz, DMSO-$d_6$) δ 8.92 (t, *J* = 4.8 Hz, 1H), 8.73 (d, *J* = 4.8 Hz, 1H), 8.29 (d, *J* = 2.4 Hz, 1H), 8.11 (dd, *J* = 8.7, 2.3 Hz, 1H), 7.84 (t, *J* = 4.9 Hz, 1H), 7.78 - 7.69 (m, 2H), 7.69 - 7.66 (m, 1H), 7.64 (d, *J* = 8.7 Hz, 1H), 7.42 - 7.36 (m, 2H), 4.43 (t, *J* = 5.2 Hz, 2H), 3.96 (t, *J* = 5.2 Hz, 2H), 1.71 (s, 6H), 1.68 (d, *J* = 13.8 Hz, 6H). $^{31}$P NMR (162 MHz, DMSO-$d_6$) δ 34.69 (s, 1P).

**Example 412: Preparation of compound 412**

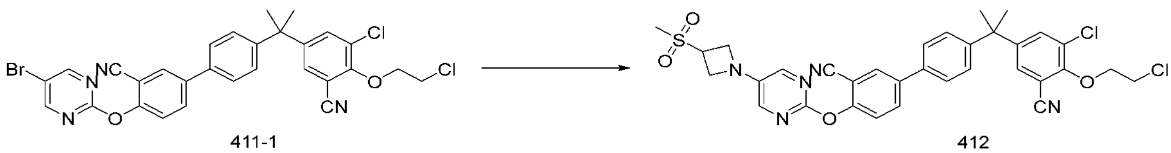

411-1

412

**Preparation of compound 412**

Compound **411-1** (145 mg, 0.24 mmol) was dissolved in acetonitrile (3 mL), followed by the sequential addition of 3-(methylsulfonyl)azetidine (65 mg, 0.48 mmol) and DIPEA (93 mg, 0.72 mmol). The reaction system was stirred at 80°C for 2 h. After the reaction was completed, water (3 mL) was added, and the mixture was extracted three times with dichloromethane (5 mL). The organic phases were combined, washed twice with saturated brine (3 mL), dried over anhydrous sodium sulfate, and concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2 mg of the title compound **411.** LC-MS (ESI): m/z 661.7 [M+H]+.

**Example 413: Preparation of compound 413**

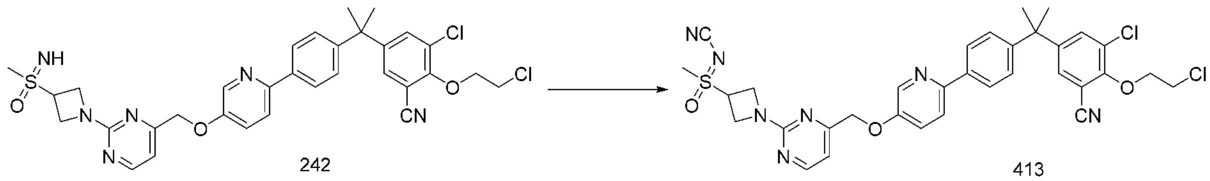

242 413

**Preparation of compound 413**

Compound **242** (100 mg, 0.15 mmol) was dissolved in acetonitrile (8 mL), followed by the addition of DIEA (11.4 mg, 0.8 mmol) and cyanogen bromide (21 mg, 0.20 mmol). The reaction was carried out at 20°C for 1 h. After the reaction was monitored by LCMS until completion, the reaction mixture was filtered. The filtrate was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 26.07 mg of the title compound **413.** LC-MS (ESI): m/z 676.2 [M+H]+. $^1$H NMR (400 MHz, DMSO-$d_6$) δ 8.50 - 8.42 (m, 2H), 7.95 (d, *J* = 8.5 Hz, 2H), 7.90 (d, *J* = 8.8 Hz, 1H), 7.70 (d, *J* = 2.4 Hz, 1H), 7.63 (d, *J* = 2.3 Hz, 1H), 7.54 (dd, *J* = 8.9, 3.0 Hz, 1H), 7.33 (d, *J* = 8.6 Hz, 2H), 6.95 (d, *J* = 5.0 Hz, 1H), 5.19 (s, 2H), 4.99 - 4.80 (m, 1H), 4.54 - 4.43 (m, 1H), 4.46 - 4.38 (m, 4H), 4.41 - 4.34 (m, 1H), 3.96 (t, *J* = 5.2 Hz, 2H), 3.60 (s, 3H), 1.69 (s, 6H).

Example **414:** Preparation of compound **414**

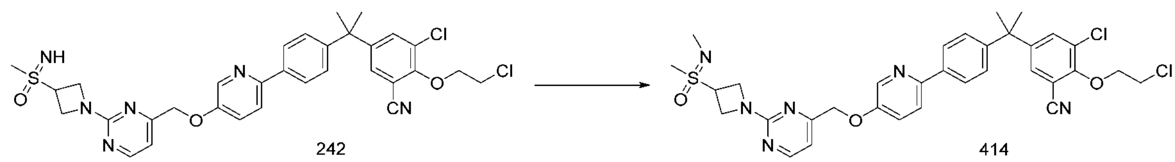

242 414

**Preparation of compound 414**

Compound **242** (100 mg, 0.15 mmol) was dissolved in THF (8 mL), cooled to 0°C, and NaH (7.6 mg, 0.20 mmol) was added. The reaction was carried out at 0°C for 1 h, and iodomethane (21 mg, 0.15 mmol) was added. After the reaction was monitored by LCMS until completion, the reaction mixture was filtered. The filtrate was subjected to preparative purification (preparative method: chromatographic column: Welch Xtimate C18 250 × 21.2 mm; column temperature: 5°C; mobile phase: water (10 mM/L $NH_4HCO_3$)-acetonitrile; mobile phase: 45% to 65% acetonitrile gradient elution over 12 min; flow rate: 30 mL/min) to obtain 24.5 mg of the title compound **414.** LC-MS (ESI): m/z 665.2 [M+H]+. $^1$H NMR (400 MHz, DMSO-$d_6$) δ 8.49 - 8.35 (m, 2H), 7.95 (d, *J* = 8.1 Hz, 2H), 7.89 (d, *J* = 9.4 Hz, 2H), 7.72 - 7.66 (m, 1H), 7.65 - 7.61 (m, 1H), 7.33 (d, *J* = 8.4 Hz, 2H), 6.88 (d, *J* = 5.1 Hz, 1H), 5.17 (s, 2H), 4.42 (t, *J* = 5.1 Hz, 3H), 4.38 - 4.22 (m, 4H), 3.95 (t, *J* = 5.1 Hz, 2H), 2.94 (s, 3H), 2.65 (s, 3H), 1.68 (s, 6H).

**Example 415: Preparation of compound 415**

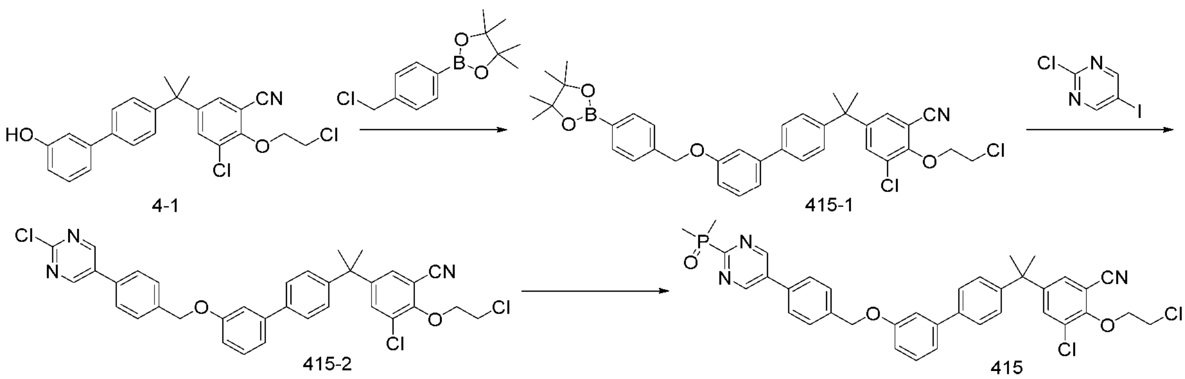

4-1 415-1 415-2 415

**Preparation of compound 415-2**

Compound **4-1** and 2-(4-(chloromethyl)phenyl)-4,4,5,5-tetramethyl-1,3,2-dioxaborolane (742.79 mg, 1.74 mmol) were weighed and dissolved in $CH_3CN$ (10 mL). $K_2CO_3$ (437.80 mg, 3.17 mmol) and 18-crown-6 (41.86 mg, 158.39 μmol) were added. The reaction mixture was heated at 80°C for 16 h. LCMS indicated the disappearance of the starting material and the formation of the product. The reaction mixture was subjected to rotary evaporation until dryness and then purified by silica gel column chromatography (EA/PE = 0 to 25%) to obtain 680 mg of the title compound **415-2.** LC-MS (ESI): m/z 642.7 $[M+H]^+$.

**Preparation of compound 415-2**

Compound **415-1** (300 mg, 466.99 μmol) and 2-chloro-5-iodopyrimidine (117.89 mg, 490.34 μmol) were weighed and dissolved in 1,4-dioxane (3 mL) and water (1 mL). Pd(dppf)$Cl_2$ (34.17 mg, 46.70 μmol) and $K_2CO_3$ (161.35 mg, 1.17 mmol) were added. The system was purged three times with nitrogen, and the reaction mixture was stirred at 100°C for 2 h. LCMS indicated the disappearance of the starting material and the formation of the product. The reaction mixture was subjected to rotary evaporation until dryness and then purified by silica gel column chromatography (EA/PE = 0 to 35%) to obtain 280 mg of the title compound **415-2.** LC-MS (ESI): m/z 628.2$[M+H]^+$.

**Preparation of compound 415**

Compound **415-2** (300 mg, 476.97 μmol), dimethylphosphine oxide (186.14 mg, 2.38 mmol), $Pd_2(dba)_3$ (43.68 mg, 47.70 μmol), Xantphos (27.60 mg, 47.70 μmol), and DIPEA (123.29 mg, 953.94 μmol, 166.16 μL) were dissolved in DMF (2 mL). The reaction system was stirred at 120°C for 12 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 39.37 mg of the title compound **415.** LC-MS (ESI): m/z 670.2 $[M+H]^+$. $^1H$ NMR (400 MHz, DMSO-$d_6$) δ 9.33 (s, 2H), 7.91 (d, *J* = 8.3 Hz, 2H), 7.71 (d, *J* = 2.3 Hz, 1H), 7.70 - 7.62 (m, 3H), 7.65 - 7.57 (m, 2H), 7.42 - 7.27 (m, 4H), 7.27 - 7.20 (m, 1H), 7.03 (dd, *J* = 7.7, 2.3 Hz, 1H), 5.29 (s, 2H), 4.42 (t, *J* = 5.2 Hz, 2H), 3.96 (t, *J* = 5.2 Hz, 2H), 1.80 (d, *J* = 13.7 Hz, 6H), 1.69 (s, 6H). $^{31}P$ NMR (162 MHz, DMSO-d6) δ 33.81 (s, 1P).

**Example 416: Preparation of compound 416**

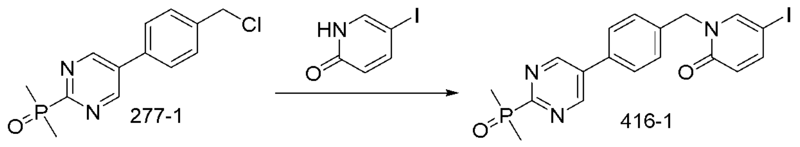

277-1 416-1

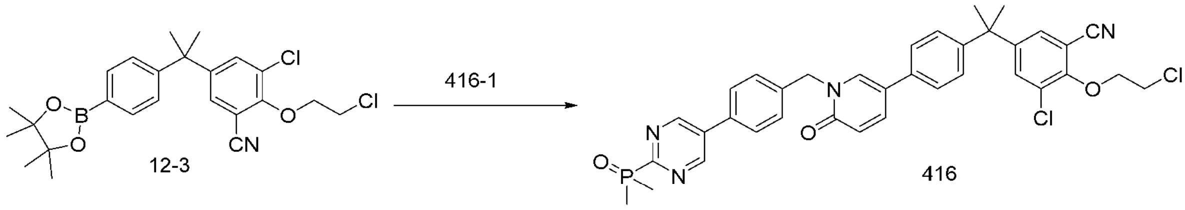


12-3 416

## Preparation of compound 416-1

Compound **277-1** (700 mg, 2.49 mmol) was dissolved in DMF (10 mL), followed by the sequential addition of potassium carbonate (1.03 g, 7.48 mmol), silver carbonate (688 mg, 2.49 mmol), and 2-hydroxy-5-iodopyridine (551 mg, 2.49 mmol). The reaction system was stirred at 90°C for 2 h. After the reaction was completed, water (25 mL) was added, and the mixture was extracted three times with ethyl acetate (25 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (PE/EA = 1/1) to obtain 580 mg of the title compound **416-1.** LC-MS (ESI): m/z 465.8 $[M+H]^+$.

## Preparation of compound 416

Compound **416-1** (35 mg, 0.08 mmol) was dissolved in 1,4-dioxane (2.0 mL), followed by the sequential addition of compound **12-3** (35 mg, 0.08 mmol) and potassium carbonate (31 mg, 0.23 mmol). The system was purged three times with nitrogen. $Pd(dppf)Cl_2$ (6 mg, 0.07 mmol) was added, and the reaction system was stirred at 110°C for 12 h. After the reaction was completed, insoluble solids were removed by filtration. The reaction mixture was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **416.** LC-MS (ESI): m/z 671.3 $[M+H]^+$. $^1H$ NMR (400 MHz, Chloroform-*d*) δ 9.08 (s, 2H), 8.46 (s, 1H), 7.99 (s, 1H), 7.77 - 7.57 (m, 4H), 7.55 - 7.44 (m, 3H), 7.35 (d, *J* = 2.3 Hz, 1H), 7.28 (d, *J* = 7.8 Hz, 2H), 7.04 (s, 1H), 5.64 (s, 2H), 4.43 (t, *J* = 6.1 Hz, 2H), 3.89 (t, *J* = 6.1 Hz, 2H), 1.93 (d, *J* = 12.9 Hz, 6H), 1.70 (s, 6H). $^{31}P$ NMR (162 MHz, Chloroform-*d*) δ 34.77 (s, 1P).

## Example 417: Preparation of compound 417

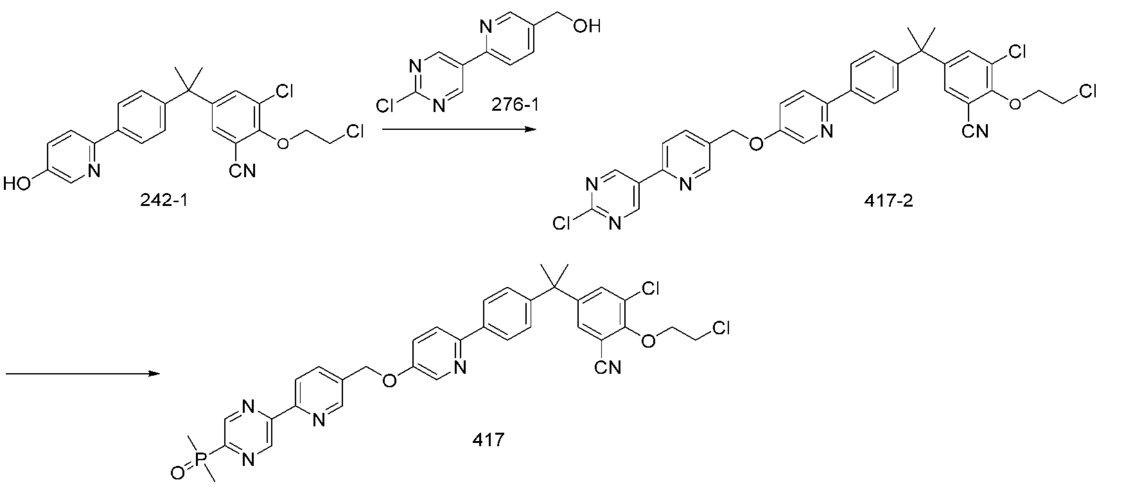

276-1 242-1 417-2 417

**Preparation of compound 417-2**

Compound **276-1** (88.7 mg, 0.40 mmol), compound **242-1** (114 mg, 0.27 mmol), and triphenylphosphine (209.8 mg, 0.8 mmol) were dissolved in acetonitrile (2 mL), and DIAD (161.6 mg, 0.8 mmol) was added thereto at 100°C. After the addition was completed, the reaction system was stirred at 100°C for 1 h. After the reaction was completed, the reaction mixture was concentrated to obtain a crude product. The crude product was purified by silica gel column chromatography (PE/EA = 100% to 80%) to obtain 125 mg of the title compound **417-2.** LC-MS (ESI): m/z 631.2 $[M+H]^+$.

**Preparation of compound 417**

Compound **417-2** (125.0 mg, 0.2 mmol), dimethylphosphine oxide (77.4 mg, 1.0 mmol), $Pd_2(dba)_3$ (18.2 mg, 0.02 mmol), Xantphos (22.9 mg, 0.04 mmol), and DIPEA (128.1 mg, 1.0 mmol) were dissolved in DMF (5 mL). The reaction system was stirred in a microwave reactor at 110°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **417.** LC-MS (ESI): m/z 672.2 $[M+H]^+$. $^1H$ NMR (400 MHz, DMSO-$d_6$) δ 9.61 (s, 2H), 8.91 (d, $J$ = 2.2 Hz, 1H), 8.48 (d, $J$ = 3.0 Hz, 1H), 8.26 (d, $J$ = 8.2 Hz, 1H), 8.15 (d, $J$ = 2.3 Hz, 1H), 7.94 (dd, $J$ = 14.0, 8.5 Hz, 3H), 7.70 (d, $J$ = 2.4 Hz, 1H), 7.62 (t, $J$ = 2.8 Hz, 2H), 7.33 (d, $J$ = 8.3 Hz, 2H), 5.38 (s, 2H), 4.42 (t, $J$ = 5.1 Hz, 2H), 3.95 (t, $J$ = 5.1 Hz, 2H), 1.81 (d, $J$ = 13.7 Hz, 6H), 1.69 (s, 6H). $^{31}P$ NMR (162 MHz, DMSO-$d_6$) δ 34.22 (s, 1P).

**Example 418: Preparation of compound 418**

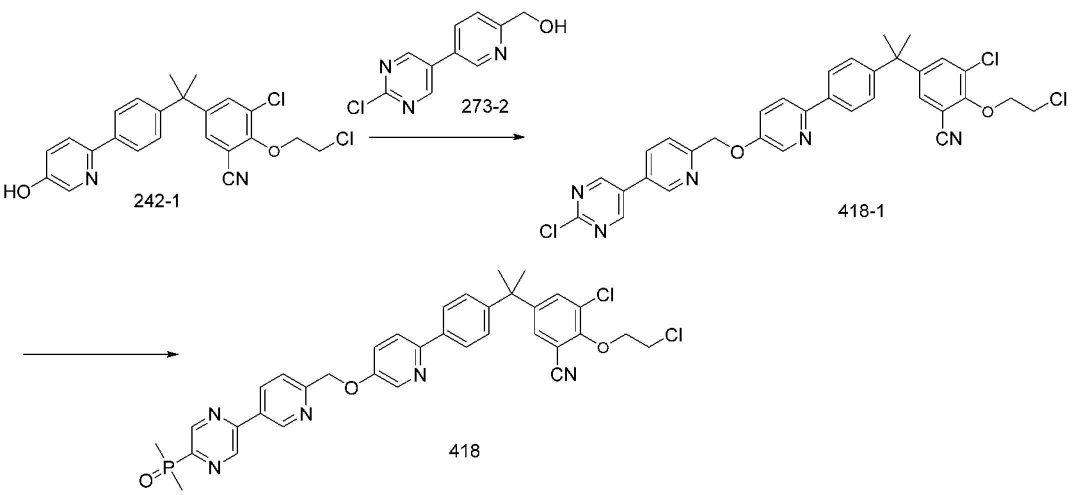
242-1
273-2
418-1
418

**Preparation of compound 418-1**

Compound **273-2** (88.7 mg, 0.40 mmol), compound **242-1** (114 mg, 0.27 mmol), and triphenylphosphine (209.8 mg, 0.8 mmol) were dissolved in acetonitrile (2 mL), and DIAD (161.6 mg, 0.8 mmol) was added thereto at 100°C. After the addition was completed, the reaction system was stirred at 100°C for 1 h. After the reaction was completed, the reaction mixture was concentrated to obtain a crude product. The crude product was purified by silica gel column chromatography (PE/EA = 100% to 80%) to obtain 100 mg of the title compound **418-1.** LC-MS (ESI): m/z 630.2 $[M+H]^+$.

**Preparation of compound 418**

Compound **418-1** (25.0 mg, 0.04 mmol), dimethylphosphine oxide (15.5 mg, 0.2 mmol), $Pd_2(dba)_3$ (3.6 mg, 0.004 mmol), Xantphos (4.6 mg, 0.008 mmol), and DIPEA (25.7 mg, 0.2 mmol) were dissolved in DMF (2 mL). The reaction system was stirred in a microwave reactor at 110°C for 2 h. After the reaction was completed, insoluble solids were

removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 10 mg of the title compound **418.** LC-MS (ESI): m/z 672.2 $[M+H]^+$. $^1H$ NMR (400 MHz, DMSO-*d6*) δ 9.38 (s, 2H), 9.08 (s, 2H), 8.46 (d, *J* = 3.0 Hz, 1H), 8.34 (d, *J* = 6.9 Hz, 1H), 7.93 (d, *J* = 8.0 Hz, 2H), 7.87 (d, *J* = 8.8 Hz, 1H), 7.74 (d, *J* = 8.2 Hz, 1H), 7.63 (d, *J* = 2.4 Hz, 1H), 7.60 - 7.52 (m, 1H), 7.31 (d, *J* = 8.2 Hz, 2H), 5.39 (s, 2H), 4.40 (t, *J* = 5.2 Hz, 2H), 3.93 (t, *J* = 5.2 Hz, 2H), 1.81 (d, *J* = 13.7 Hz, 6H), 1.68 (s, 6H). $^{31}P$ NMR (162 MHz, DMSO-$d_6$) δ 34.07 (s, 1P).

**Example 419: Preparation of compound 419**

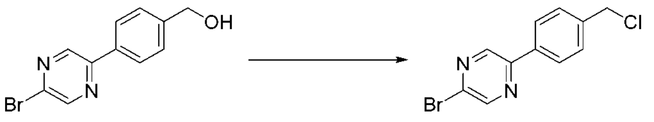

381-1 419-1

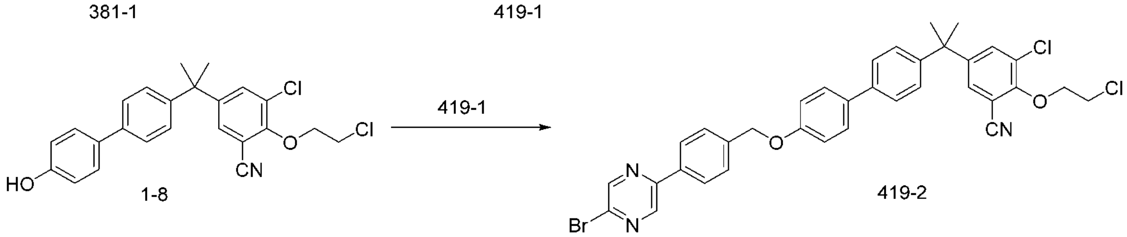


1-8 419-2

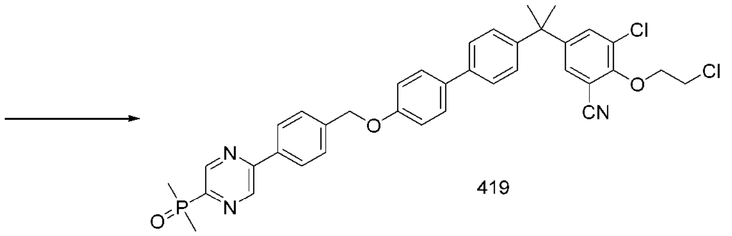

419

**Preparation of compound 419-1**

Compound **381-1** (78 mg, 0.29 mmol) and thionyl chloride (176 mg, 1.5 mmol) were added to dichloromethane (5 mL), and the mixture was stirred at room temperature for 2 h. After the reaction was completed, the reaction mixture was concentrated directly to obtain 80 mg of a crude product of the title compound **419-1.** LC-MS (ESI): m/z 283.0 $[M+H]^+$.

**Preparation of compound 419-2**

Compound **1-8** (107 mg, 0.25 mmol) was dissolved in DMF (5 mL), followed by the sequential addition of **419-1** (86 mg, 0.30 mmol) and cesium carbonate (409 mg, 1.3 mmol). The reaction system was stirred at 70°C for 1 h. After the reaction was completed, water (100 mL) was added to the reaction mixture, and the mixture was extracted three times with dichloromethane (150 mL). The organic phases were combined, washed once with saturated brine (100 mL), dried over anhydrous sodium sulfate, subjected to suction filtration, and the filtrate was concentrated. The resulting crude product was purified by silica gel column chromatography (PE/EA = 7/3) to obtain 120 mg of the title compound **419-2.** LC-MS (ESI): m/z 672.2 $[M+H]^+$.

**Preparation of compound 419**

Compound **419-2** (100 mg, 0.18 mmol), dimethylphosphine oxide (35 mg, 0.45 mmol), DIEA (96 mg, 0.74 mmol), X-phos (18 mg, 0.03 mmol), and $Pd(dba)_3$ (14 mg, 0.01 mmol) were dissolved in 1,4-dioxane (2 mL). The reaction system was purged three times with nitrogen and stirred under microwave irradiation at 120°C for 2 h. After the reaction was completed, the reaction mixture was filtered through diatomite. The filter cake was rinsed with dichloromethane (70 mL).

Water (100 mL) was added to the filtrate, and the mixture was extracted three times with dichloromethane (100 mL). The organic phases were combined, washed once with saturated brine (100 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 15.0 mg of the title compound **419.** LC-MS (ESI): m/z 670.2 $[M+H]^+$. $^1H$ NMR (400 MHz, DMSO-$d_6$) δ 9.46 (s, 1H), 9.11 (s, 1H), 8.25 (d, *J* = 8.2 Hz, 2H), 7.70 (d, *J* = 2.3 Hz, 1H), 7.67 (d, *J* = 8.1 Hz, 2H), 7.64 (d, *J* = 2.3 Hz, 1H), 7.60 (d, *J* = 8.8 Hz, 2H), 7.56 (d, *J* = 8.4 Hz, 2H), 7.30 (d, *J* = 8.3 Hz, 2H), 7.12 (d, *J* = 8.8 Hz, 2H), 5.27 (s, 2H), 4.42 (t, *J* = 5.2 Hz, 2H), 3.95 (t, *J* = 5.2 Hz, 2H), 1.76 (d, *J* = 13.7 Hz, 6H), 1.68 (s, 6H). $^{31}P$ NMR (162 MHz, DMSO-$d_6$) δ 34.12 (s, 1P).

**Example 420: Preparation of compound 420**

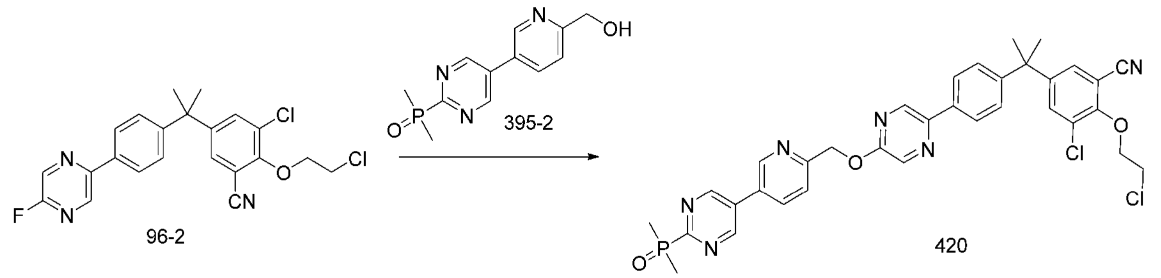

96-2 395-2 420

**Preparation of compound 420**

Compound **395-2** (20 mg, 0.076 mmol), compound **96-2** (32.7 mg, 0.076 mmol), and cesium carbonate (82.5 mg, 0.152 mmol) were dissolved in DMF (2 mL). The reaction system was stirred in a microwave reactor at 60°C for 1 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound **420.** LC-MS (ESI): m/z 673.3 $[M+H]^+$. $^1H$ NMR (400 MHz, DMSO-$d_6$) δ 9.39 (s, 2H), 9.07 (d, *J* = 2.4 Hz, 1H), 8.78 (d, *J* = 1.4 Hz, 1H), 8.54 (d, *J* = 1.4 Hz, 1H), 8.33 (dd, *J* = 8.2, 2.4 Hz, 1H), 7.97 (d, *J* = 8.5 Hz, 2H), 7.74 - 7.67 (m, 2H), 7.64 (d, *J* = 2.4 Hz, 1H), 7.38 (d, *J* = 8.6 Hz, 2H), 5.61 (s, 2H), 4.42 (t, *J* = 5.2 Hz, 2H), 3.96 (t, *J* = 5.2 Hz, 2H), 1.80 (d, *J* = 13.7 Hz, 6H), 1.70 (s, 6H). $^{31}P$ NMR (162 MHz, DMSO-$d_6$) δ 33.95 (s, 1P).

**Example 421: Preparation of compound 421**

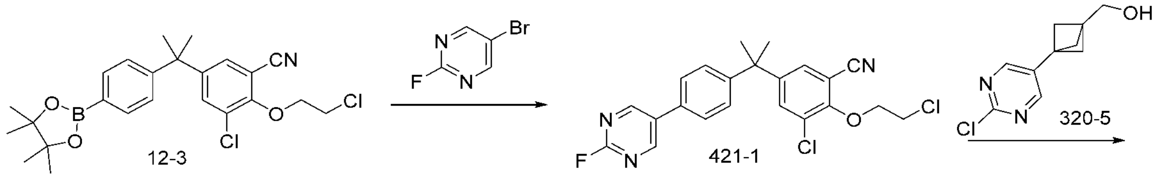

12-3 421-1 320-5

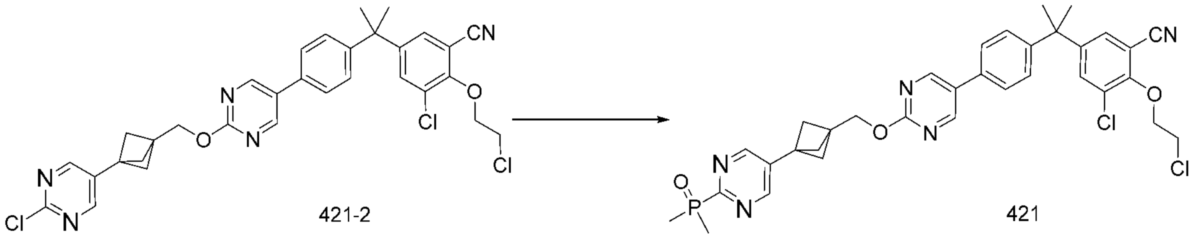

421-2

421

## Preparation of compound 421-1

Compound **12-3** (1 g, 2.17 mmol) and 2-fluoro-5-bromopyrimidine (460 mg, 2.60 mmol) were dissolved in 1,4-dioxane (10 mL) and water (1 mL). Pd(dppf)$Cl_2$ (160 mg, 0.22 mmol) and potassium carbonate (600 mg, 4.34 mmol) were added at room temperature. The system was purged with argon and stirred at 100°C for 2 h. The reaction was monitored by LCMS until completion. The reaction was quenched with ice water (10 mL) and extracted three times with ethyl acetate (30 mL). The organic phases were combined, dried over anhydrous sodium sulfate, and concentrated. The resulting residue was purified by silica gel column chromatography (EA/PE = 0 to 50%) to obtain 270 mg of the title compound **421-1.** LC-MS (ESI): m/z 430.1 $[M+H]^+$.

## Preparation of compound 421-2

Compound **421-1** (60 mg, 0.14 mmol) was dissolved in acetonitrile (5 mL). Cesium carbonate (91 mg, 0.28 mmol) and compound **320-5** (30 mg, 0.14 mmol) were added at room temperature, and the mixture was stirred at 100°C for 5 h. The reaction was monitored by LCMS until completion. The reaction mixture was filtered, and the filtrate was concentrated. The residue was purified by silica gel column chromatography (EA/PE = 0 to 40%) to obtain 20 mg of the title compound **421-2.** LC-MS (ESI): m/z 620.2 $[M+H]^+$.

## Preparation of compound 421

Compound **421-2** (20 mg, 0.032 mmol) was dissolved in N,N-dimethylformamide (2 mL). Dimethylphosphine oxide (10 mg, 0.13 mmol), palladium acetate (1 mg, 0.0032 mmol), 1,3-bis(diphenylphosphino)propane (1 mg, 0.0032 mmol), and *N,N*-diisopropylethylamine (21 mg, 0.16 mmol) were added at room temperature. After purging with argon for 30 seconds, the mixture was stirred under microwave irradiation at 120°C for 0.5 h. The reaction was monitored by LCMS until completion. The reaction mixture was purified by preparative liquid chromatography (Waters 2767/Qda; column: Xbridge C18 19 * 250 mm, 10 μm; mobile phase A: 0.1% FA/$H_2O$, B: acetonitrile; flow rate: 20 mL/min; elution gradient: 69% to 74%; retention time: 8.0-9.6 min of 16 min) to obtain 2.66 mg of the title compound **421.** LC-MS (ESI): m/z 662.2 $[M+H]^+$. $^1H$ NMR (400 MHz, DMSO-$d_6$) δ 8.92 (s, 2H), 8.88 (s, 2H), 7.70 (d, *J* = 2.3 Hz, 1H), 7.68 (d, *J* = 8.4 Hz, 2H), 7.64 (d, *J* = 2.3 Hz, 1H), 7.37 (d, *J* = 8.4 Hz, 2H), 4.50 (s, 2H), 4.42 (t, *J* = 5.2 Hz, 2H), 3.96 (t, *J* = 5.2 Hz, 2H), 2.17 (s, 6H), 1.73 (d, *J* = 13.7 Hz, 6H), 1.69 (s, 6H). $^{31}P$ NMR (162 MHz, DMSO-*d6*) δ 33.57 (s,1P).

## Example 422: Preparation of compound 422

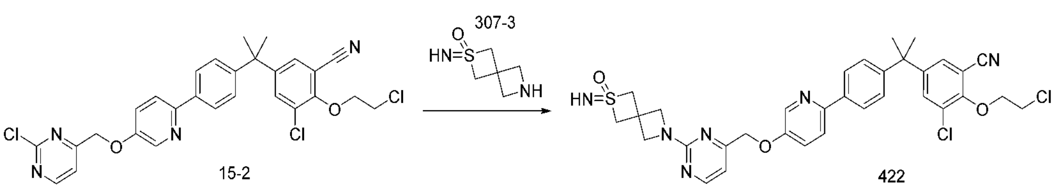


15-2

422

## Preparation of compound 422

Compound **15-2** (200 mg, 361.10 μmol) and compound **307-3** (52.8 mg, 361.10 μmol) were dissolved in $CH_3CN$ (5 mL), and DIPEA (233.4 mg, 1.81 mmol, 315 μL) was added. The reaction mixture was heated at 80°C for 4 h. LCMS indicated the disappearance of the starting material and the formation of the product. The reaction mixture was filtered and then subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65%

acetonitrile gradient elution over 12 min; flow rate: 30 mL/min) to obtain 93.4 mg of the title compound **422.** LC-MS (ESI): m/z 663.2 [M+H]$^+$. $^1$H NMR (400 MHz, DMSO-$d_6$) δ 8.44 (d, *J* = 3.0 Hz, 1H), 8.40 (d, *J* = 5.0 Hz, 1H), 7.95 (d, *J* = 8.2 Hz, 2H), 7.89 (d, *J* = 8.8 Hz, 1H), 7.70 (d, *J* = 2.3 Hz, 1H), 7.62 (d, *J* = 2.3 Hz, 1H), 7.52 (dd, *J* = 8.8, 3.1 Hz, 1H), 7.33 (d, *J* = 8.3 Hz, 2H), 6.84 (d, *J* = 5.0 Hz, 1H), 5.15 (s, 2H), 4.50 (s, 1H), 4.42 (t, *J* = 5.2 Hz, 2H), 4.36 - 4.18 (m, 8H), 3.95 (t, *J* = 5.2 Hz, 2H), 1.68 (s, 6H).

**Example 423: Preparation of compound 423**

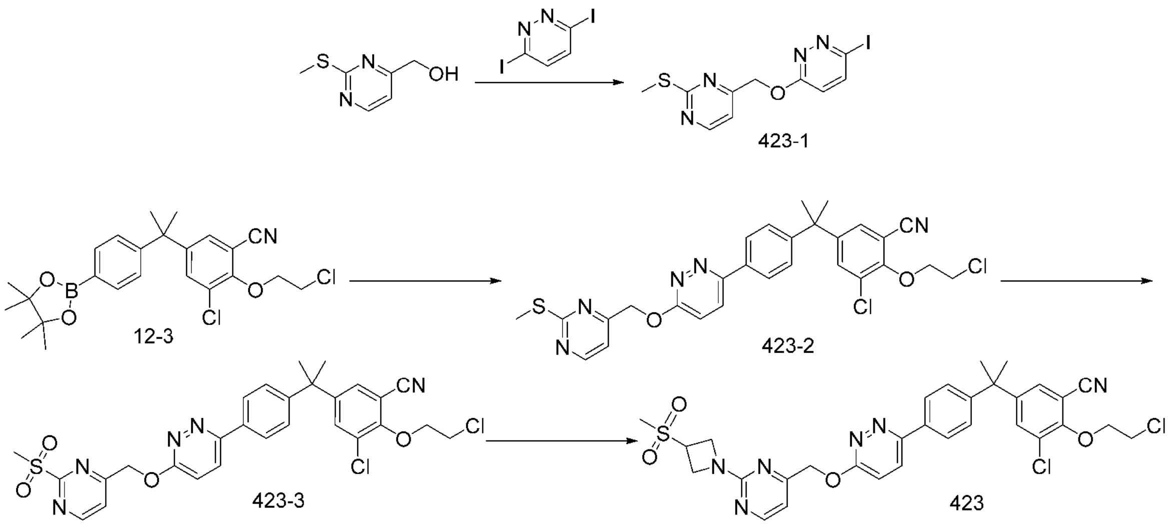

423-1

12-3

423-2

423-3

423

**Preparation of compound 423-1**

(2-(Methylthio)pyrimidin-4-yl)methanol (500 mg, 3.20 mmol) and 2,5-diiodopyridazine (1.06 g, 3.20 mmol) were dissolved in THF (10 mL). NaH (256.1 mg, 6.40 mmol, 60% purity) was added, and the reaction system was stirred at room temperature for 5 h. The reaction was quenched with saturated aqueous ammonium chloride solution and extracted three times with ethyl acetate (10 mL). The organic phases were combined and subjected to rotary evaporation until dryness. The resulting residue was purified by silica gel column chromatography (EA/PE = 0 to 40%) to obtain 440 mg of the title compound **423-1.** LC-MS (ESI): m/z 361.0 [M+H]$^+$.

**Preparation of compound 423-2**

Compound **423-1** (400 mg, 1.11 mmol) and compound **12-3** (464.62 mg, 1.01 mmol) were dissolved in 1,4-dioxane (3 mL) and water (1 mL). Pd(dppf)$Cl_2$ (74 mg, 100.96 μmol) and $K_2CO_3$ (348.8 mg, 2.52 mmol) were added, and the system was purged three times with nitrogen. The reaction mixture was heated and stirred at 100°C for 4 h. LCMS indicated the disappearance of the starting material and the formation of the product. The reaction mixture was subjected to rotary evaporation until dryness. The resulting residue was purified by silica gel column chromatography (EA/PE = 0 to 50%) to obtain 440 mg of the title compound **423-2.** LC-MS (ESI): m/z 566.2 [M+H]$^+$.

**Preparation of compound 423-3**

Compound **423-2** (450 mg, 794.35 μmol) was dissolved in DCM (10 mL), and *m*-CPBA (645.1 mg, 3.18 mmol, 85% purity) was added. The reaction mixture was stirred at room temperature for 4 h. LCMS indicated the disappearance of the starting material and the formation of the product. The reaction mixture was poured into saturated sodium sulfite solution (30 mL) and extracted three times with dichloromethane (30 mL). The organic phases were combined, washed three times with saturated brine (30 mL each time), dried over anhydrous sodium sulfate, filtered, and then subjected to rotary evaporation until dryness. The resulting residue was used directly in the next step. LC-MS (ESI): m/z 598.2 [M+H]$^+$.

**Preparation of compound 423**

Compound **423-3** (600 mg, 1.00 mmol) and 3-(methylsulfonyl)azetidine hydrochloride (258.1 mg, 1.50 mmol)

were dissolved in $CH_3CN$ (2 mL), and TEA (304.3 mg, 3.01 mmol, 420 μL) was added. The reaction mixture was heated at 60°C for 12 h. LCMS indicated the disappearance of the starting material and the formation of the product. The reaction mixture was filtered and then subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 79.89 mg of the title compound 423. LC-MS (ESI): m/z 653.2 $[M+H]^+$. $^1H$ NMR (400 MHz, DMSO-$d_6$) δ 8.39 (d, *J* = 5.0 Hz, 1H), 8.22 (d, *J* = 9.3 Hz, 1H), 8.00 (d, *J* = 8.3 Hz, 2H), 7.71 (d, *J* = 2.4 Hz, 1H), 7.64 (d, *J* = 2.3 Hz, 1H), 7.48 (d, *J* = 9.3 Hz, 1H), 7.40 (d, *J* = 8.2 Hz, 2H), 6.82 (d, *J* = 5.0 Hz, 1H), 5.49 (s, 2H), 4.46 - 4.37 (m, 3H), 4.33 (t, *J* = 8.8 Hz, 2H), 4.26 - 4.19 (m, 2H), 3.96 (t, *J* = 5.2 Hz, 2H), 3.06 (s, 3H), 1.70 (s, 6H).

**Example 424: Preparation of compound 424**

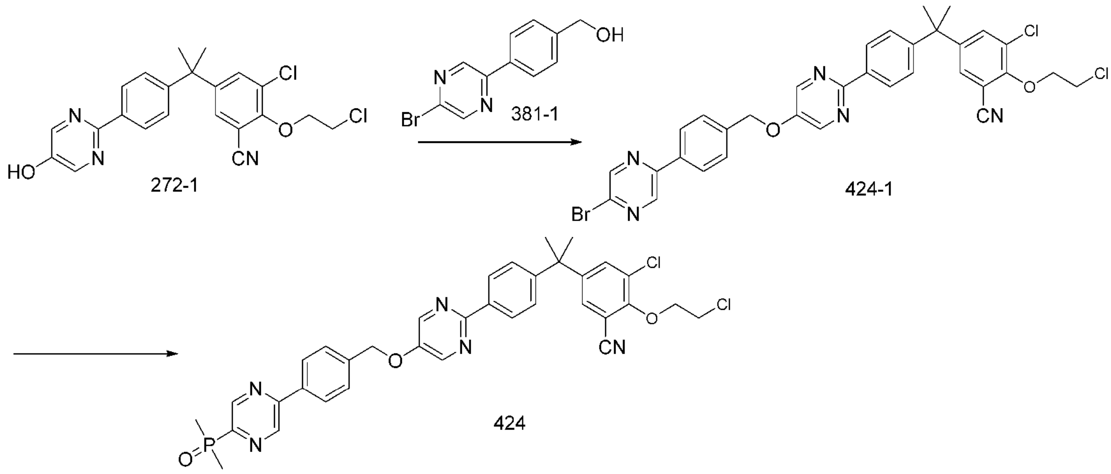

272-1

381-1

424-1

424

**Preparation of compound 424-1**

Compound **381-1** (53 mg, 0.20 mmol), compound **272-1** (57.1 mg, 0.13 mmol), and triphenylphosphine (104.9 mg, 0.4 mmol) were dissolved in acetonitrile (2 mL), and DIAD (80.8 mg, 0.4 mmol) was added thereto at 100°C. After the addition was completed, the reaction system was stirred at 100°C for 1 h. After the reaction was completed, the reaction mixture was concentrated to obtain a crude product. The crude product was purified by silica gel column chromatography (PE/EA = 100% to 80%) to obtain 75 mg of the title compound **424-1.** LC-MS (ESI): m/z 674.0 $[M+H]^+$.

**Preparation of compound 424**

Compound **424-1** (67.5 mg, 0.1 mmol), dimethylphosphine oxide (39.0 mg, 0.5 mmol), $Pd_2(dba)_3$ (9.2 mg, 0.01 mmol), Xantphos (11.6 mg, 0.02 mmol), and DIPEA (77.6 mg, 0.5 mmol) were dissolved in DMF (2 mL). The reaction system was stirred in a microwave reactor at 110°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 18 mg of the title compound **424.** LC-MS (ESI): m/z 672.2 $[M+H]^+$. $^1H$ NMR (400 MHz, DMSO-$d_6$) δ 9.46 (s, 1H), 9.12 (s, 1H), 8.74 (s, 2H), 8.30 - 8.20 (m, 4H), 7.74 - 7.67 (m, 3H), 7.62 (s, 1H), 7.38 (d, *J* = 8.1 Hz, 2H), 5.43 (s, 2H), 4.42 (t, *J* = 5.0 Hz, 2H), 3.95 (t, *J* = 5.0 Hz, 2H), 1.76 (d, *J* = 13.6 Hz, 6H), 1.69 (s, 6H). $^{31}P$ NMR (162 MHz, DMSO-$d_6$) δ 34.26 (s, 1P).

**Example 425: Preparation of compound 425**

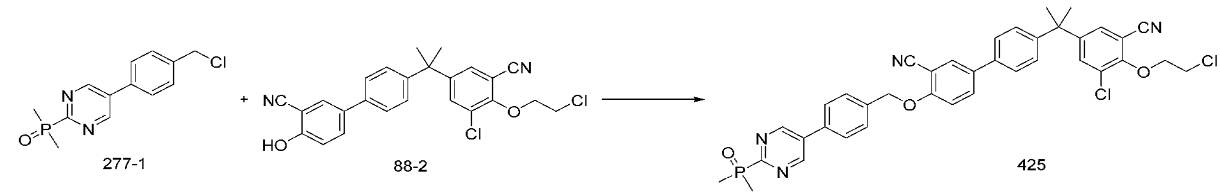

277-1 88-2 425

**Preparation of compound 425**

Compound **277-1** (50 mg, 178 μmol), compound **88-2** (88 mg, 195 μmol), cesium carbonate (116 mg, 357 μmol), and acetonitrile (2 mL) were added to a three-necked flask. The reaction system was purged three times with nitrogen and stirred at 75°C for 3 h. After the reaction was completed, saturated aqueous sodium chloride solution (10 mL) was added to the reaction system. The mixture was extracted three times with DCM (10 mL). The organic phases were combined, dried, subjected to suction filtration, and concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 50% acetonitrile gradient elution over 12 min; flow rate: 30 mL/min) to obtain 12 mg of the title compound **425.** LC-MS (ESI): m/z 695.2 $[M+H]^+$. $^1H$ NMR (400 MHz, DMSO-$d_6$) δ 9.34 (s, 2H), 8.09 (d, *J* = 2.4 Hz, 1H), 7.98 (dd, *J* = 8.9, 2.4 Hz, 1H), 7.96 - 7.92 (m, 2H), 7.74 - 7.67 (m, 3H), 7.67 - 7.62 (m, 3H), 7.44 (d, *J* = 9.0 Hz, 1H), 7.33 (d, *J* = 8.5 Hz, 2H), 5.44 (s, 2H), 4.42 (t, *J* = 5.2 Hz, 2H), 3.96 (t, *J* = 5.2 Hz, 2H), 1.80 (d, *J* = 13.7 Hz, 6H), 1.69 (s, 6H).

**Example 426: Preparation of compound 426**

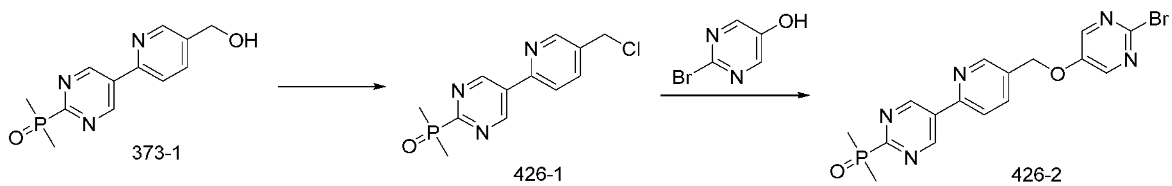

373-1 426-1 426-2

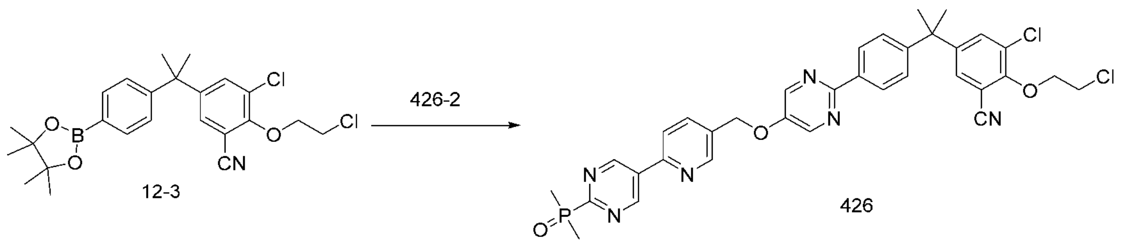


12-3 426

**Preparation of compound 426-1**

Compound **373-1** (200 mg, 0.76 mmol) and thionyl chloride (456 mg, 3.8 mmol) were dissolved in DCM (5 mL), and the reaction system was stirred at room temperature for 1.5 h. After the reaction was completed, the reaction mixture was directly concentrated to obtain a crude product, yielding 210 mg of the title compound **426-1.** LC-MS (ESI): m/z 282.0 $[M+H]^+$.

**Preparation of compound 426-2**

Compound **426-1** (100 mg, 0.38 mmol) was dissolved in DMF (5 mL), followed by the sequential addition of 2-bromo-5-hydroxypyrimidine (37.0 mg, 0.21 mmol) and cesium carbonate (344 mg, 1.06 mmol). The reaction system was stirred at 90°C for 1.5 h. After the reaction was completed, water (100 mL) was added to the reaction mixture, and the mixture was extracted three times with dichloromethane (150 mL). The organic phases were combined, washed once with saturated brine (100 mL), dried over anhydrous sodium sulfate, and concentrated. The resulting crude product was purified by silica gel column chromatography (DCM/MeOH = 3/17) to obtain 83 mg of the title compound **426-2.** LC-MS (ESI): m/z 420.2 $[M+H]^+$.

**Preparation of compound 426**

Compound **426-2** (83 mg, 0.19 mmol), compound **12-3** (142 mg, 0.28 mmol), $K_2CO_3$ (58 mg, 0.38 mmol), and Pd(dppf)$Cl_2$ (17 mg, 0.02 mmol) were dissolved in 1,4-dioxane (5 mL) and $H_2O$ (2 mL). The reaction system was purged three times with nitrogen and stirred under microwave irradiation at 105°C for 2 h. After the reaction was completed, the reaction mixture was filtered through diatomite. The filter cake was rinsed with dichloromethane (70 mL). Water (100 mL) was added to the filtrate, and the mixture was extracted three times with dichloromethane (100 mL). The organic phases were combined, washed once with saturated brine (100 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 22 mg of the title compound **426.** LC-MS (ESI): m/z 672.8[M+H]$^+$. $^1$H NMR (400 MHz, DMSO-$d_6$) δ 9.61 (s, 2H), 8.93 (d, *J* = 2.2 Hz, 1H), 8.76 (s, 2H), 8.28 (d, *J* = 8.1 Hz, 1H), 8.24 (d, *J* = 8.1 Hz, 2H), 8.16 (dd, *J* = 8.2, 2.2 Hz, 1H), 7.69 (d, *J* = 2.3 Hz, 1H), 7.62 (d, *J* = 2.3 Hz, 1H), 7.38 (d, *J* = 8.2 Hz, 2H), 5.46 (s, 2H), 4.42 (t, *J* = 5.1 Hz, 2H), 3.95 (t, *J* = 5.1 Hz, 2H), 1.81 (d, *J* = 13.7 Hz, 6H), 1.69 (s, 6H). $^{31}$P NMR (162 MHz, DMSO-$d_6$) δ 34.23 (s, 1P).

**Example 427: Preparation of compound 427**

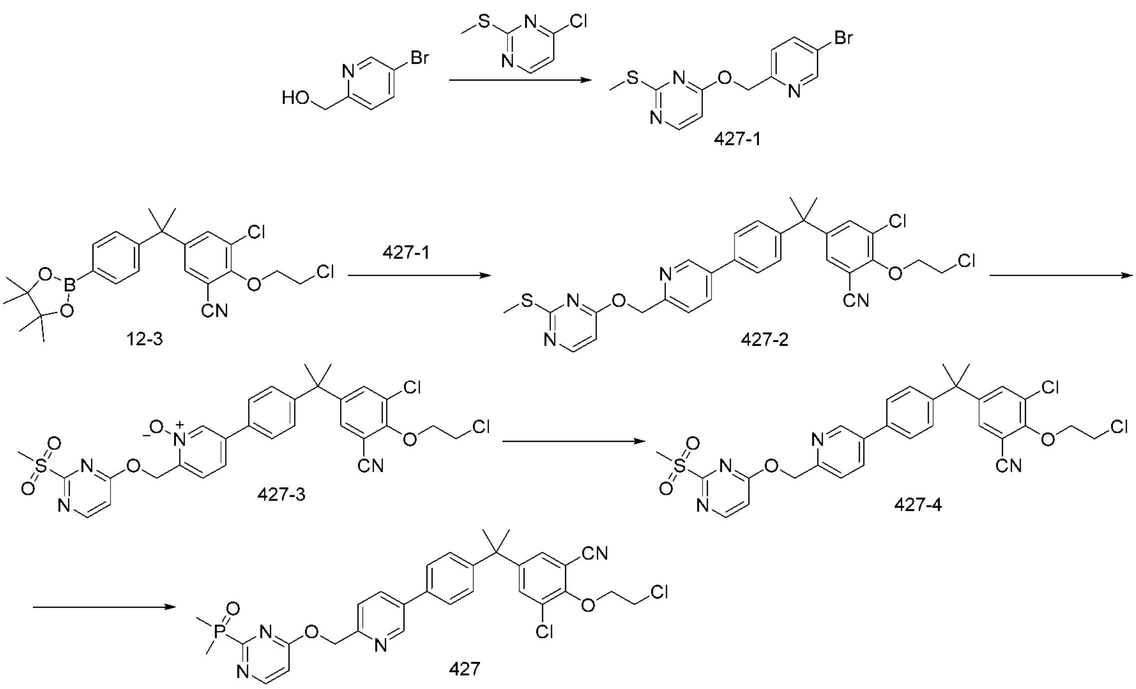

427-1
12-3
427-2
427-3
427-4
427

**Preparation of compound 427-1**

(5-Bromopyridin-2-yl)methanol (1.2 g, 6.38 mmol) and 2-(methylthio)-4-chloropyrimidine (1.03 g, 6.38 mmol) were dissolved in DMF (20 mL), and cesium carbonate (4.16 g, 12.76 mmol) was added thereto. After the addition was completed, the reaction system was stirred at 50°C for 1 h. After the reaction was completed, a 5% aqueous lithium chloride solution (100 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 85%) to obtain 1.99 g of the title compound **427-1.** LC-MS (ESI): m/z 312.0 [M+H]$^+$.

**Preparation of compound 427-2**

Compound **427-1** (1.2 g, 3.84 mmol), compound **12-3** (1.77 g, 3.84 mmol), and potassium carbonate (1.06 g, 7.69 mmol) were dissolved in 1,4-dioxane (30 mL) and water (10 mL), and Pd(dppf)$Cl_2$-DCM (314 mg, 0.384 mmol) was added thereto. After the addition was completed, the reaction system was stirred at 91°C for 2 h. After the reaction was completed,

water (50 mL) was added, and the mixture was extracted three times with ethyl acetate (20 mL). The organic phases were combined, washed twice with saturated brine (30 mL), dried over anhydrous sodium sulfate, filtered, and the filtrate was concentrated to obtain a crude product. The crude product was purified by silica gel column chromatography (PE/EA = 100% to 75%) to obtain 1.5 g of the title compound **427-2.** LC-MS (ESI): m/z 565.2 $[M+H]^+$.

**Preparation of compound 427-3**

Compound **427-2** (0.55 g, 0.972 mmol) was dissolved in dichloromethane (30 mL), and *m*-chloroperoxybenzoic acid (0.592 g, 2.92 mmol) was added thereto. After the addition was completed, the reaction system was stirred at room temperature for 1 h. After the reaction was completed, the reaction mixture was filtered, and the filtrate was concentrated to obtain a crude product. The crude product was purified by silica gel column chromatography (PE/EA = 100% to 85%) to obtain 0.55 g of the title compound **427-3.** LC-MS (ESI): m/z 613.2 $[M+H]^+$.

**Preparation of compound 427-4**

Compound **427-3** (0.55 g, 0.896 mmol) was dissolved in acetonitrile (10 mL), and bis(pinacolato)diboron (0.455 g, 1.79 mmol) was added thereto. After the addition was completed, the reaction system was stirred at 70°C for 1 h. After the reaction was completed, the reaction mixture was filtered, and the filtrate was concentrated. The resulting crude product was purified by silica gel column chromatography (PE/EA = 100% to 85%) to obtain 0.31 g of the title compound **427-4.** LC-MS (ESI): m/z 597.2 $[M+H]^+$.

**Preparation of compound 427**

Compound **427-4** (55 mg, 0.092 mmol), dimethylphosphine oxide (22 mg, 0.277 mmol), and cesium carbonate (90 mg, 0.276 mmol) were placed in a sealed tube, and acetonitrile (2 mL) was added. The reaction system was stirred at 90°C for 16 h. After the reaction was completed, insoluble solids were removed by filtration, and the filtrate was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **427.** LC-MS (ESI): m/z 595.2 $[M+H]^+$. $^1H$ NMR (400 MHz, DMSO-$d_6$) δ 8.87 (d, *J* = 2.4 Hz, 1H), 8.72 (d, *J* = 5.8 Hz, 1H), 8.11 (dd, *J* = 8.1, 2.4 Hz, 1H), 7.71 (d, *J* = 2.4 Hz, 1H), 7.70 - 7.63 (m, 3H), 7.59 (d, *J* = 8.2 Hz, 1H), 7.38 (d, *J* = 8.4 Hz, 2H), 7.19 (dd, *J* = 5.9, 2.7 Hz, 1H), 5.60 (s, 2H), 4.42 (t, *J* = 5.2 Hz, 2H), 3.96 (t, *J* = 5.2 Hz, 2H), 1.69 (s, 6H), 1.68 (d, *J* = 13.7 Hz, 6H). $^{31}P$ NMR (162 MHz, DMSO-$d_6$) δ 34.21 (s, 1P).

**Example 428: Preparation of compound 428**

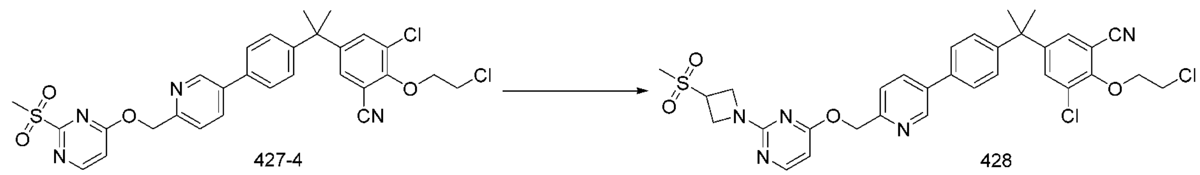

**Preparation of compound 428**

Compound **427-4** (40 mg, 0.067 mmol), 3-(methylsulfonyl)azetidine hydrochloride (23 mg, 0.133 mmol), and DIEA (26 mg, 0.20 mmol) were placed in a sealed tube, and acetonitrile (2 mL) was added. The reaction system was stirred at 90°C for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **428.** LC-MS (ESI): m/z 652.2 $[M+H]^+$. $^1H$ NMR (400 MHz, DMSO-$d_6$) δ 8.86 (d, *J* = 2.4 Hz, 1H), 8.16 (d, *J* = 5.6 Hz, 1H), 8.09 (dd, *J* = 8.1, 2.4 Hz, 1H), 7.71 (d, *J* = 2.3 Hz, 1H), 7.70 - 7.63 (m, 3H), 7.55 (d, *J* = 8.2 Hz, 1H), 7.38 (d, *J* = 8.5 Hz, 2H), 6.31 (d, *J* = 5.7 Hz, 1H), 5.46 (s, 2H), 4.42 (t, *J* = 5.2 Hz, 2H), 4.40 - 4.33 (m, 1H), 4.27 (t, *J* = 8.8 Hz, 2H), 4.21 - 4.15 (m, 2H), 3.96 (t, *J* = 5.2 Hz, 2H), 3.04 (s, 3H), 1.70 (s, 6H).

**Example 429: Preparation of compound 429**

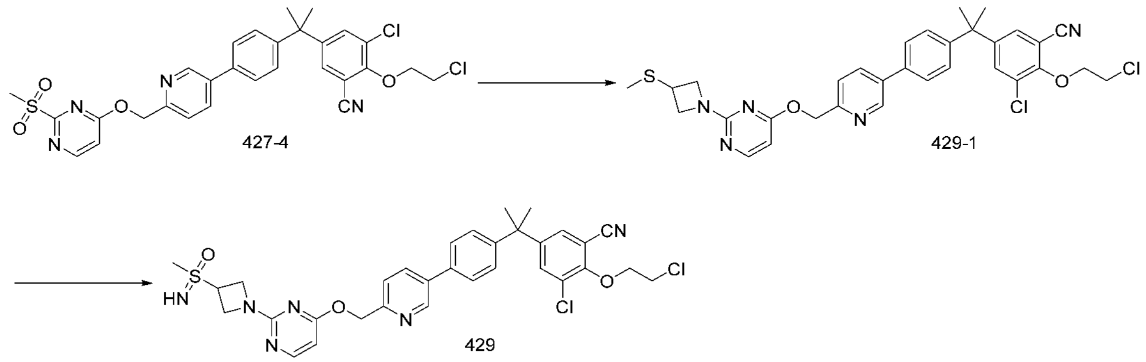

427-4

429-1

429

**Preparation of compound 429-1**

Compound **427-4** (70 mg, 0.117 mmol), 3-(methylthio)azetidine hydrochloride (33 mg, 0.234 mmol), and DIEA (45 mg, 0.351 mmol) were placed in a sealed tube, and acetonitrile (2 mL) was added. The reaction system was stirred at 90°C for 16 h. After the reaction was completed, insoluble solids were removed by filtration. A 5% aqueous lithium chloride solution (20 mL) was added, and the mixture was extracted three times with ethyl acetate (20 mL). The organic phases were combined, washed twice with saturated brine (20 mL), dried over anhydrous sodium sulfate, filtered, and the filtrate was concentrated to obtain a crude product. The crude product was purified by silica gel column chromatography (PE/EA = 100% to 85%) to obtain 70 mg of the title compound **429-1.** LC-MS (ESI): m/z 620.2 $[M+H]^+$.

**Preparation of compound 429**

Compound **429-1** (60 mg, 0.097 mmol), (diacetoxyiodo)benzene (93 mg, 0.290 mmol), and ammonium carbonate (38 mg, 0.483 mmol) were placed in a sealed tube, and methanol (2 mL) was added. The reaction system was stirred at room temperature for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **429.** LC-MS (ESI): m/z 651.2 $[M+H]^+$. $^1H$ NMR (400 MHz, DMSO-$d_6$) δ 8.86 (d, *J* = 2.3 Hz, 1H), 8.15 (d, *J* = 5.6 Hz, 1H), 8.09 (dd, *J* = 8.1, 2.4 Hz, 1H), 7.72 (d, *J* = 2.3 Hz, 1H), 7.68 (d, *J* = 8.5 Hz, 2H), 7.66 (d, *J* = 2.4 Hz, 1H), 7.54 (d, *J* = 8.1 Hz, 1H), 7.38 (d, *J* = 8.5 Hz, 2H), 6.28 (d, *J* = 5.6 Hz, 1H), 5.46 (s, 2H), 4.42 (t, *J* = 5.2 Hz, 2H), 4.27 - 4.14 (m, 5H), 3.98 - 3.93 (m, 3H), 2.89 (s, 3H), 1.70 (s, 6H).

**Example 430: Preparation of compound 430**

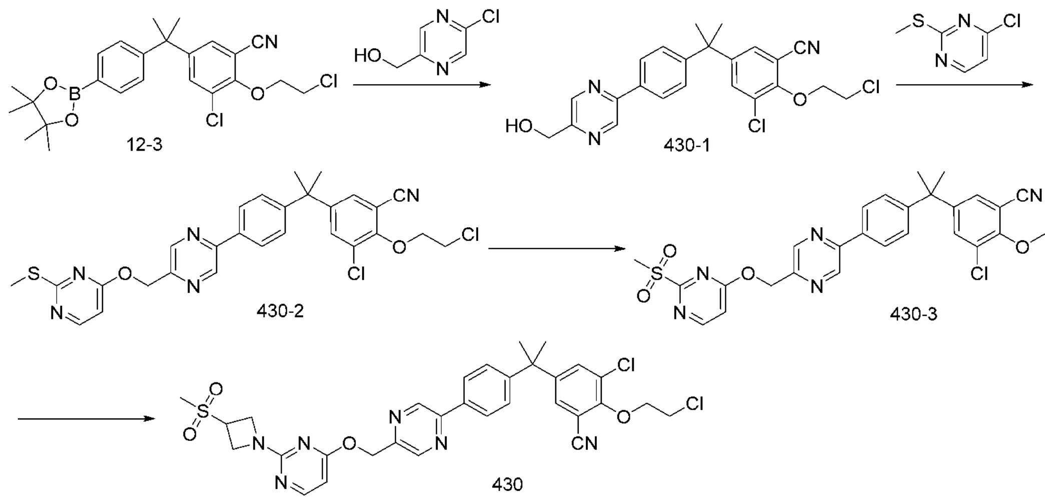

12-3

430-1

430-2

430-3

430

**Preparation of compound 430-1**

Compound **12-3** (1.0 g, 2.17 mmol) and (5-chloropyrazin-2-yl)methanol (392 mg, 2.71 mmol) were dissolved in 1,4-dioxane (12 mL). Potassium carbonate (748 mg, 5.43 mmol), [1,1'-bis(diphenylphosphino)ferrocene]dichloropalladium(II) (187 mg, 217 μmol), and water (0.5 mL) were added. The reaction system was purged three times with nitrogen and stirred at 100°C for 3 h. After the reaction was completed, water (15 mL) was added, and the mixture was extracted three times with dichloromethane (20 mL). The organic phases were combined, washed twice with saturated brine (30 mL), dried over anhydrous sodium sulfate, and concentrated. The resulting crude product was purified by silica gel column chromatography (EA/PE = 0 to 50%) to obtain 720 mg of the title compound **430-1.** LC-MS (ESI): m/z 442.3 $[M+H]^+$.

**Preparation of compound 430-2**

Compound **430-1** (720 mg, 1.63 mmol) and 2-(methylthio)-4-chloropyrimidine (312 mg, 1.95 mmol) were dissolved in acetonitrile (8 mL), and potassium carbonate (500 mg, 3.26 mmol) was added. The reaction system was purged three times with nitrogen and stirred at 65°C for 3 h. After the reaction was completed, the reaction mixture was cooled to room temperature. Saturated aqueous sodium chloride solution (20 mL) was added to the reaction system, and the mixture was extracted three times with dichloromethane (15 mL). The organic phases were combined, washed twice with saturated brine (30 mL), dried over anhydrous sodium sulfate, filtered, and the filtrate was concentrated to obtain a crude product. The crude product was purified by silica gel column chromatography (PE/EA = 100% to 40%) to obtain 520 mg of the title compound **430-2.**

**Preparation of compound 430-3**

Compound **430-2** (520 mg, 0.92 mmol) was dissolved in tetrahydrofuran (8 mL), and an aqueous solution of potassium peroxymonosulfate (1.59 g, 4.62 mmol) was added. The reaction system was stirred at 25°C for 5 h. After the reaction was completed, the reaction mixture was cooled to room temperature. Saturated aqueous sodium chloride solution (20 mL) was added to the reaction system, and the mixture was extracted three times with dichloromethane (15 mL). The organic phases were combined, washed twice with saturated brine (30 mL), dried over anhydrous sodium sulfate, filtered, and the filtrate was concentrated to obtain a crude product. The crude product was purified by silica gel column chromatography (PE/EA = 100% to 60%) to obtain 420 mg of the title compound **430-3.** LC-MS (ESI): m/z 598.0 $[M+H]^+$.

**Preparation of compound 430**

Compound **430-3** (50 mg, 83.5 μmol), 3-(methylsulfonyl)azetidine hydrochloride (44 mg, 97.7 μmol), and *N*,*N*-diisopropylethylamine (27 mg, 209 μmol) were dissolved in acetonitrile (3 mL). The reaction system was stirred under microwave irradiation at 80°C for 2 h. After the reaction was completed, saturated aqueous sodium chloride (10 mL) solution was added, and the mixture was extracted three times with dichloromethane (10 mL). The organic phases were combined, washed twice with saturated brine (20 mL), dried over anhydrous sodium sulfate, filtered, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2

mm; column temperature: 25°C; gradient: 15% to 55% acetonitrile gradient elution over 12 min; flow rate: 30 mL/min) to obtain 32 mg of the title compound **430.** LC-MS (ESI): m/z 653.0 $[M+H]^+$. $^1H$ NMR (400 MHz, DMSO-$d_6$) δ 9.22 (d, *J* = 1.5 Hz, 1H), 8.83 (d, *J* = 1.5 Hz, 1H), 8.16 (d, *J* = 5.7 Hz, 1H), 8.08 (d, *J* = 8.6 Hz, 2H), 7.71 (d, *J* = 2.4 Hz, 1H), 7.65 (d, *J* = 2.3 Hz, 1H), 7.42 (d, *J* = 8.5 Hz, 2H), 6.31 (d, *J* = 5.6 Hz, 1H), 5.52 (s, 2H), 4.42 (t, *J* = 5.2 Hz, 2H), 4.40 - 4.33 (m, 1H), 4.26 (t, *J* = 8.9 Hz, 2H), 4.21 - 4.14 (m, 2H), 3.96 (t, *J* = 5.2 Hz, 2H), 3.04 (s, 3H), 1.70 (s, 6H).

**Example 431: Preparation of compound 431**

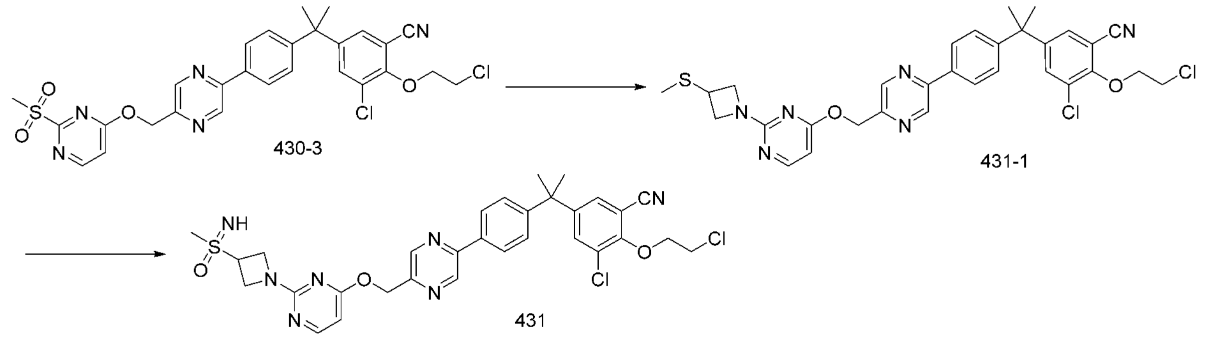

**Preparation of compound 431-1**

Compound **430-3** (110 mg, 0.19 mmol), *N,N*-diisopropylethylamine (61 mg, 0.48 mmol), and 3-(methylthio) azetidine hydrochloride (41 mg, 0.29 mmol) were dissolved in acetonitrile (10 mL), and the reaction system was stirred at 75°C for 3 h. After the reaction was completed, saturated sodium chloride solution (30 mL) was added, and the mixture was extracted three times with dichloromethane (20 mL). The organic phases were combined, washed twice with saturated brine (40 mL), dried over anhydrous sodium sulfate, filtered, and the filtrate was concentrated to obtain a crude product. The crude product was purified by silica gel column chromatography (PE/EA = 100% to 40%) to obtain 98 mg of the title compound **431-1.**

**Preparation of compound 431**

Compound **431-1** (98 mg, 158 μmol) and ammonium carbonate (45 mg, 473 μmol) were dissolved in methanol (3 mL), and (diacetoxyiodo)benzene (163 mg, 507 μmol) was added thereto at 0°C. After the addition was completed, the reaction system was stirred at 25°C for 3 h. After the reaction was completed, water (5 mL) was added, and the mixture was extracted three times with ethyl acetate (10 mL). The organic phases were combined, washed twice with saturated brine (20 mL), dried over anhydrous sodium sulfate, filtered, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 50% acetonitrile gradient elution over 12 min; flow rate: 30 mL/min) to obtain 12 mg of the title compound **431.** LC-MS (ESI): m/z 652.2 $[M+H]^+$. $^1H$ NMR (400 MHz, DMSO-$d_6$) δ 9.22 (d, *J* = 1.5 Hz, 1H), 8.82 (d, *J*= 1.5 Hz, 1H), 8.14 (d, *J* = 5.6 Hz, 1H), 8.08 (d, *J* = 8.5 Hz, 2H), 7.71 (d, *J* = 2.3 Hz, 1H), 7.65 (d, *J* = 2.4 Hz, 1H), 7.42 (d, *J* = 8.5 Hz, 2H), 6.28 (d, *J* = 5.6 Hz, 1H), 5.51 (s, 2H), 4.42 (t, *J* = 5.2 Hz, 2H), 4.29 - 4.12 (m, 5H), 4.02 - 3.90 (m, 3H), 2.88 (s, 3H), 1.70 (s, 6H).

**Example 432: Preparation of compound 432**

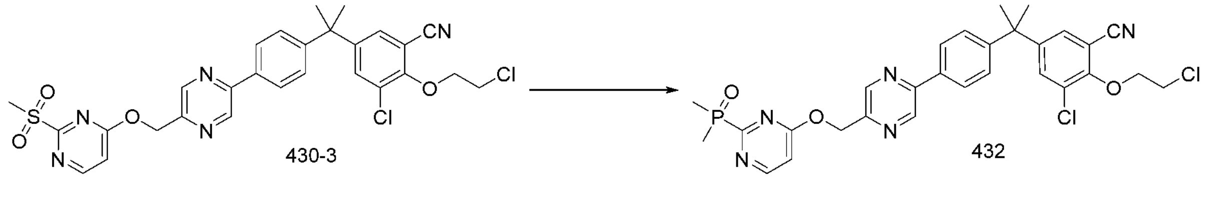

**Preparation of compound 432**

Compound **430-3** (120 mg, 200 μmol), cesium carbonate (162 mg, 500 μmol), and dimethylphosphine oxide (39 mg, 501 μmol) were dissolved in acetonitrile (2 mL), and the reaction system was stirred under microwave irradiation at 90°C for 2 h. After the reaction was completed, saturated aqueous sodium chloride (10 mL) solution was added, and the mixture was extracted three times with dichloromethane (10 mL). The organic phases were combined, washed twice with saturated brine (20 mL), dried over anhydrous sodium sulfate, filtered, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 50% acetonitrile gradient elution over 12 min; flow rate: 30 mL/min) to obtain 42 mg of the title compound 432. LC-MS (ESI): m/z 596.0 $[M+H]^+$. $^1H$ NMR (400 MHz, DMSO-$d_6$) δ 9.23 (d, *J* = 1.5 Hz, 1H), 8.88 (d, *J* = 1.5 Hz, 1H), 8.73 (dd, *J* = 5.9, 0.9 Hz, 1H), 8.08 (d, *J* = 8.5 Hz, 2H), 7.72 (d, *J* = 2.3 Hz, 1H), 7.65 (d, *J* = 2.3 Hz, 1H), 7.42 (d, *J* = 8.5 Hz, 2H), 7.20 (dd, *J* = 5.8, 2.7 Hz, 1H), 5.67 (s, 2H), 4.42 (t, *J* = 5.2 Hz, 2H), 3.96 (t, *J* = 5.2 Hz, 2H), 1.70 (s, 6H), 1.68 (d, *J* = 13.7 Hz, 6H). $^{31}P$ NMR (162 MHz, DMSO-$d_6$) δ 34.13 (s, 1P).

**Example 433: Preparation of compound 433**

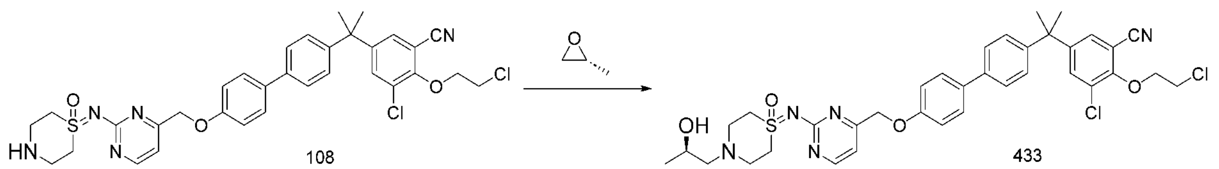


**Preparation of compound 433**

Compound **108** (100 mg, 0.15 mmol), (R)-2-methyloxirane (87 mg, 1.5 mmol), and cesium carbonate (146 mg, 0.45 mmol) were dissolved in DMF (5 mL). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2.39 mg of the title compound **433.** LC-MS (ESI): m/z 708.3 $[M+H]^+$. $^1H$ NMR (400 MHz, Chloroform-*d*) δ 8.45 (d, *J* = 5.0 Hz, 1H), 7.55 - 7.45 (m, 5H), 7.37 (d, *J* = 2.3 Hz, 1H), 7.22 (d, *J* = 8.4 Hz, 2H), 7.08 (d, *J* = 4.9 Hz, 1H), 7.00 (d, *J* = 8.6 Hz, 2H), 5.11 (s, 2H), 4.42 (t, *J* = 6.2 Hz, 2H), 4.00 - 3.90 (m, 1H), 3.90 - 3.75 (m, 4H), 3.69 - 3.55 (m, 2H), 3.36 - 3.27 (m, 2H), 3.26 - 3.11 (m, 2H), 2.67 - 2.59 (m, 1H), 2.48 - 2.39 (m, 1H), 1.69 (s, 6H), 1.17 (d, *J* = 6.0 Hz, 3H).

**Example 434: Preparation of compound 434**

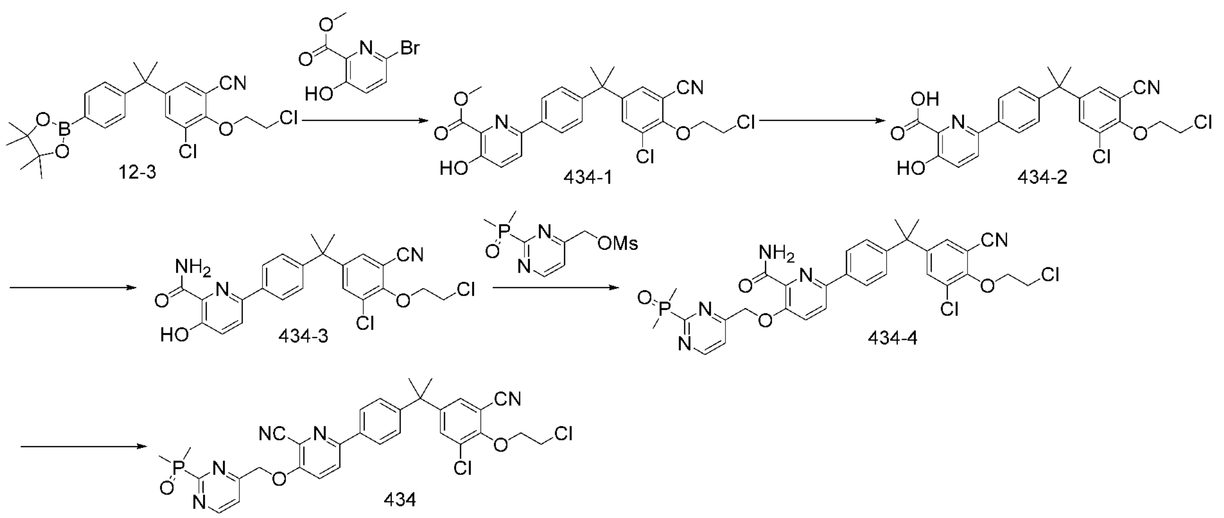

12-3, 434-1, 434-2, 434-3, 434-4, 434

**Preparation of compound 434-1**

Compound **12-3** (400 mg, 0.87 mmol), methyl 3-hydroxy-6-bromopyridine-1-carboxylate (242 mg, 1.04 mmol), and potassium carbonate (241 mg, 1.74 mmol) were dissolved in 1,4-dioxane (20 mL) and water (4 mL). Pd(dppf)$Cl_2$ (73 mg, 0.1 mmol) was added thereto. After the addition was completed, the reaction system was stirred at 80°C for 2 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 50%) to obtain 300 mg of the title compound **434-1.** LC-MS (ESI): m/z 485.2 $[M+H]^+$.

**Preparation of compound 434-2**

Compound **434-1** (300 mg, 0.62 mmol) was dissolved in tetrahydrofuran (10 mL) and water (10 mL), and lithium hydroxide (140 mg, 6.18 mmol) was added thereto. After the addition was completed, the reaction system was stirred at 25°C for 3 h. After the reaction was completed, saturated brine (20 mL) was added, and the mixture was extracted three times with dichloromethane (20 mL). The organic phases were combined, washed twice with saturated brine (20 mL), dried over anhydrous sodium sulfate, filtered, and the filtrate was concentrated to obtain a crude product of the title compound **434-2.** LC-MS (ESI): m/z 471.2 $[M+H]^+$.

**Preparation of compound 434-3**

Compound **434-2** (200 mg, 0.42 mmol), ammonium chloride (68 mg, 1.27 mmol), and triethylamine (128 mg, 1.27 mmol) were dissolved in dichloromethane (10 mL), and HATU (380 mg, 0.84 mmol) was added thereto. After the addition was completed, the reaction system was stirred at 25°C for 1 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 20%) to obtain 60 mg of the title compound **434-3.** LC-MS (ESI): m/z 470.2 $[M+H]^+$.

**Preparation of compound 434-4**

Compound **434-3** (60 mg, 0.13 mmol) and potassium carbonate (53 mg, 0.39 mmol) were dissolved in acetonitrile (5 mL), and (2-(dimethylphosphoryl)pyrimidin-4-yl)methyl methanesulfonate (69 mg, 0.26 mmol) was added thereto. After the addition was completed, the reaction system was stirred at 80°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (DCM/MeOH = 100% to 90%) to obtain 60 mg of the title compound **434-4.** LC-MS (ESI): m/z 638.2 $[M+H]^+$.

**Preparation of compound 434**

Compound **434-4** (50 mg, 0.08 mmol) was dissolved in acetonitrile (5 mL), and phosphorus oxychloride (34 mg, 0.24 mmol) was added thereto. The reaction system was stirred at 25°C for 2 h. After the reaction was completed, water (10 mL) was added, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 18.12 mg of the title compound **434.** LC-MS (ESI): m/z 620.2 $[M+H]^+$. $^1H$ NMR (400 MHz, Chloroform-*d*) δ 8.96 (s, 1H), 7.97 - 7.83 (m, 4H), 7.49 (d, *J* = 8.8 Hz, 1H), 7.45 (d, *J* = 2.3 Hz, 1H), 7.36 (d, *J* = 2.3 Hz, 1H), 7.30 (d, *J* = 8.5 Hz, 2H), 5.42 (s, 2H), 4.43 (t, *J* = 6.1 Hz, 2H), 3.88 (t, *J* = 6.1 Hz, 2H), 1.91 (d, *J* = 13.6 Hz, 6H), 1.70 (s, 6H). $^{31}P$ NMR (162 MHz, Chloroform-*d*) δ 30.78 (s, 1P).

**Example 435: Preparation of compound 435**

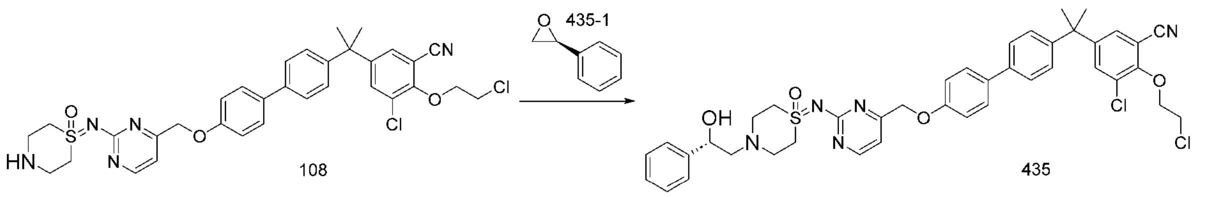

435-1

108

435

**Preparation of compound 435**

Compound **108** (100 mg, 0.15 mmol), compound **435-1** (180 mg, 1.5 mmol), and cesium carbonate (146 mg, 0.45 mmol) were dissolved in DMF (5 mL). The reaction system was stirred at 100°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 2.39 mg of the title compound **435.** LC-MS (ESI): m/z 770.3 $[M+H]^+$. $^1H$ NMR (400 MHz, Methanol-$d_4$) δ 8.46 (d, *J* = 5.2 Hz, 1H), 7.60 - 7.53 (m, 6H), 7.39 - 7.25 (m, 7H), 7.12 - 7.03 (m, 3H), 5.15 (s, 2H), 4.78 (dd, *J* = 8.3, 4.3 Hz, 1H), 4.64 (s, 1H), 4.44 (t, *J* = 5.2 Hz, 2H), 3.92 (t, *J* = 5.4 Hz, 2H), 3.86 - 3.66 (m, 3H), 3.59 - 3.43 (m, 2H), 3.25 - 3.17 (m, 1H), 3.17 - 3.09 (m, 2H), 3.09 - 2.98 (m, 1H), 1.72 (s, 6H).

**Example 436: Preparation of compound 436**

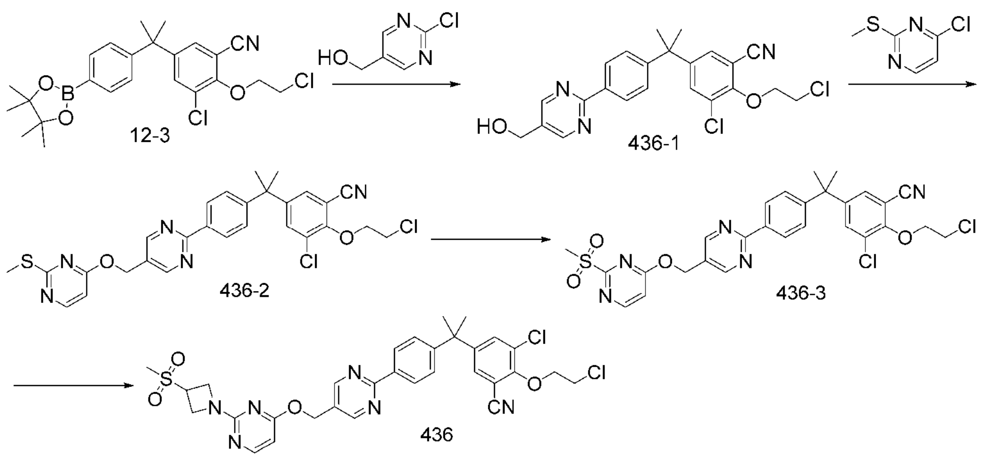

12-3

436-1

436-2

436-3

436

**Preparation of compound 436-1**

Compound **12-3** (500 mg, 1.09 mmol), (2-chloropyrimidin-5-yl)methanol (236 mg, 1.63 mmol), and potassium carbonate (301 mg, 2.18 mmol) were added to 1,4-dioxane (25 mL) and water (5 mL), and Pd(dppf)$Cl_2$ (146 mg, 0.2 mmol) was added thereto. After the addition was completed, the reaction system was stirred at 80°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 75%) to obtain 450 mg of the title compound **436-1.** LC-MS (ESI): m/z 442.2 $[M+H]^+$.

**Preparation of compound 436-2**

Compound **436-1** (450 mg, 1.02 mmol) and cesium carbonate (993 mg, 3.06 mmol) were dissolved in acetonitrile (50 mL), and 2-(methylthio)-4-chloropyrimidine (195 mg, 1.22 mmol) was added thereto. After the addition was completed, the reaction system was stirred at 80°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 80%) to obtain 350 mg of the title compound **436-2.** LC-MS (ESI): m/z 566.2 $[M+H]^+$.

**Preparation of compound 436-3**

Compound **436-2** (350 mg, 0.62 mmol) was dissolved in dichloromethane (30 mL), and m-chloroperoxybenzoic acid (3.21 mg, 1.86 mmol) was added thereto. After the addition was completed, the reaction system was stirred at 25°C for 2 h. After the reaction was completed, water (50 mL) was added, and the mixture was extracted three times with dichloromethane (50 mL). The organic phases were combined, washed twice with saturated sodium carbonate (50 mL), dried over anhydrous sodium sulfate, filtered, and the filtrate was concentrated to obtain a crude product. The crude product was purified by silica gel column chromatography (PE/EA = 100% to 30%) to obtain 300 mg of the title compound **436-3.** LC-MS (ESI): m/z 598.2 $[M+H]^+$.

**Preparation of compound 436**

Compound **436-3** (100 mg, 0.17 mmol), 3-(methylthio)azetidine hydrochloride (69 mg, 0.51 mmol), and potassium carbonate (71 mg, 0.51 mmol) were dissolved in DMF (5 mL). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 42 mg of the title compound **436.** LC-MS (ESI): m/z 653.2 $[M+H]^+$. $^1$H NMR (400 MHz, Chloroform-*d*) δ 8.86 (s, 2H), 8.40 - 8.35 (m, 2H), 8.11 (d, *J* = 5.7 Hz, 1H), 7.44 (d, *J* = 2.3 Hz, 1H), 7.37 (d, *J* = 2.3 Hz, 1H), 7.33 - 7.29 (m, 2H), 6.18 (d, *J* = 5.7 Hz, 1H), 5.40 (s, 2H), 4.53 - 4.38 (m, 6H), 4.14 - 4.03 (m, 1H), 3.87 (t, *J* = 6.2 Hz, 2H), 2.96 (s, 3H), 1.71 (s, 6H).

**Example 437: Preparation of compound 437**

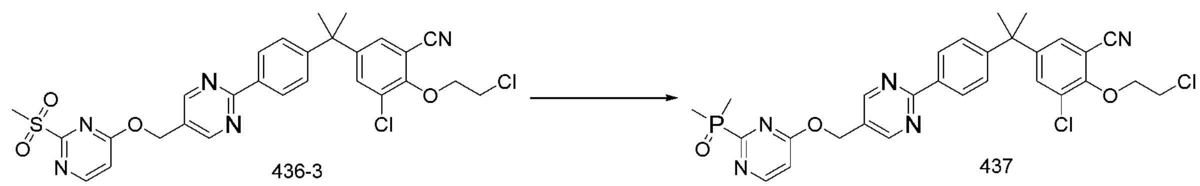

**Preparation of compound 437**

Compound **436-3** (100 mg, 0.17 mmol), dimethylphosphine oxide (40 mg, 0.51 mmol), and potassium carbonate

(71 mg, 0.51 mmol) were dissolved in DMF (5 mL). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 42 mg of the title compound **437.** LC-MS (ESI): m/z 596.2 $[M+H]^+$. $^1$H NMR (400 MHz, Chloroform-*d*) δ 8.92 (s, 2H), 8.60 (d, *J* = 5.8 Hz, 1H), 8.39 (d, *J* = 8.5 Hz, 2H), 7.44 (d, *J* = 2.3 Hz, 1H), 7.37 (d, *J* = 2.3 Hz, 1H), 7.31 (d, *J* = 8.6 Hz, 2H), 6.88 (dd, *J* = 5.8, 2.7 Hz, 1H), 5.57 (s, 2H), 4.42 (t, *J* = 6.2 Hz, 2H), 3.87 (t, *J* = 6.1 Hz, 2H), 1.87 (d, *J* = 13.5 Hz, 6H), 1.71 (s, 6H). $^{31}$P NMR (162 MHz, Chloroform-*d*) δ 35.07 (s, 1P).

**Example 438: Preparation of compound 438**

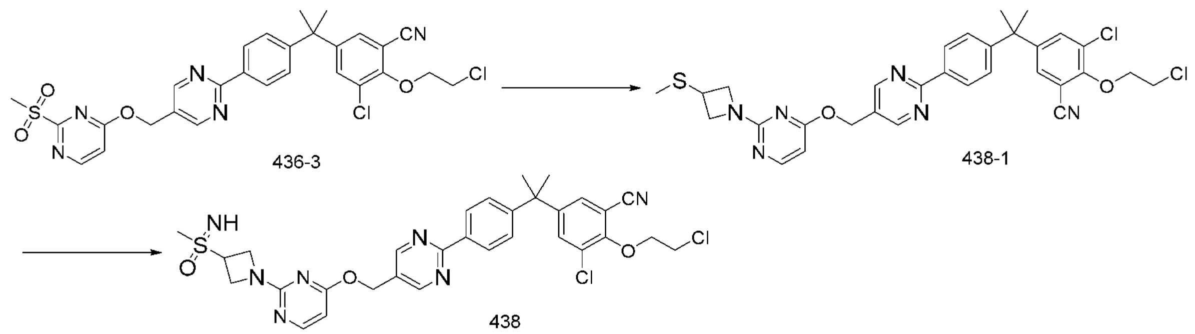
436-3
438-1
438

**Preparation of compound 438-1**

Compound **436-3** (100 mg, 0.17 mmol), 3-(methylthio)azetidine (53 mg, 0.51 mmol), and potassium carbonate (71 mg, 0.51 mmol) were dissolved in DMF (5 mL). The reaction system was stirred at 80°C for 16 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 50%) to obtain 90 mg of the title compound **438-1.** LC-MS (ESI): m/z 621.2 $[M+H]^+$.

**Preparation of compound 438**

Compound **438-1** (90 mg, 0.14 mmol), ammonium carbonate (27 mg, 0.28 mmol), and (diacetoxyiodo)benzene (113 mg, 0.35 mmol) were dissolved in methanol (5 mL). The reaction system was stirred at 25°C for 1 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 55.52 mg of the title compound **438.** LC-MS (ESI): m/z 652.2 $[M+H]^+$. $^1$H NMR (400 MHz, Chloroform-*d*) δ 8.86 (s, 2H), 8.37 (d, *J* = 8.6 Hz, 2H), 8.10 (d, *J* = 5.7 Hz, 1H), 7.44 (d, *J* = 2.3 Hz, 1H), 7.37 (d, *J* = 2.3 Hz, 1H), 7.31 (d, *J* = 8.6 Hz, 2H), 6.17 (d, *J* = 5.7 Hz, 1H), 5.40 (s, 2H), 4.48 - 4.39 (m, 6H), 4.18 - 4.10 (m, 1H), 3.87 (t, *J* = 6.2 Hz, 2H), 3.00 (s, 3H), 1.71 (s, 6H).

**Example 439: Preparation of compound 439**

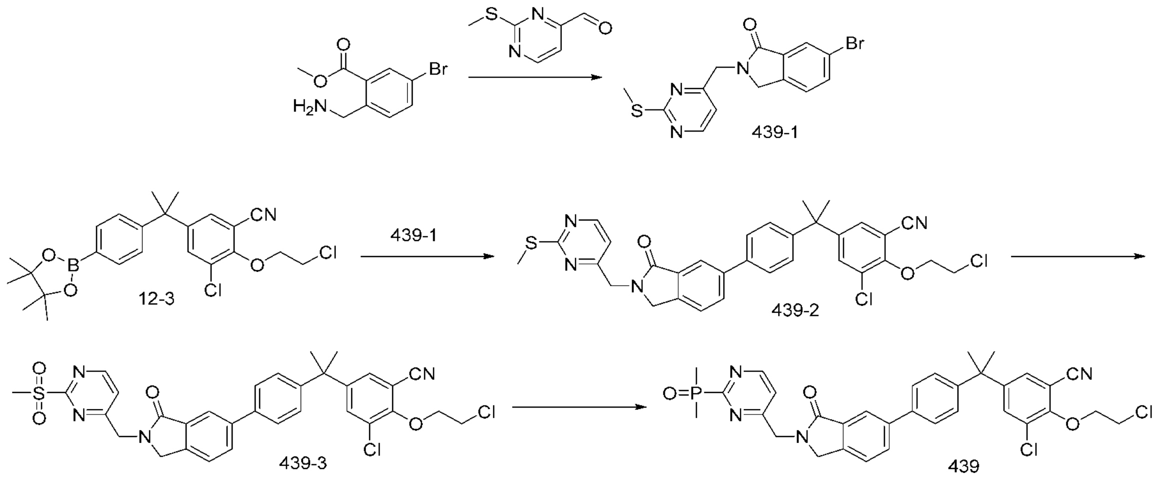


439-1

12-3

439-2

439-3

439

**Preparation of compound 439-1**

Methyl 2-(aminomethyl)-5-bromobenzoate (300 mg, 1.23 mmol) and 2-(methylthio)pyrimidine-4-carbaldehyde (189 mg, 1.23 mmol) were dissolved in ethanol (30 mL), and sodium triacetoxyborohydride (519 mg, 2.46 mmol) was added thereto. After the addition was completed, the reaction system was stirred at 25°C for 16 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 70%) to obtain 450 mg of the title compound **439.** LC-MS (ESI): m/z 350.0 $[M+H]^+$.

**Preparation of compound 439-2**

Compound **439-1** (400 mg, 1.14 mmol), compound **12-3** (236 mg, 1.14 mmol), and potassium carbonate (526 mg, 2.18 mmol) were dissolved in 1,4-dioxane (50 mL) and water (10 mL), and Pd(dppf)$Cl_2$ (146 mg, 0.2 mmol) was added thereto. After the addition was completed, the reaction system was stirred at 80°C for 3 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 60%) to obtain 600 mg of the title compound **439-2.** LC-MS (ESI): m/z 603.2 $[M+H]^+$.

**Preparation of compound 439-3**

Compound **439-2** (600 mg, 1.00 mmol) was dissolved in dichloromethane (30 mL), and *m*-chloroperoxybenzoic acid (518 mg, 3.00 mmol) was added thereto. After the addition was completed, the reaction system was stirred at 25°C for 2 h. After the reaction was completed, water (50 mL) was added, and the mixture was extracted three times with dichloromethane (50 mL). The organic phases were combined, washed twice with saturated sodium carbonate (50 mL), dried over anhydrous sodium sulfate, filtered, and the filtrate was concentrated to obtain a crude product. The crude product was purified by silica gel column chromatography (PE/EA = 100% to 30%) to obtain 300 mg of the title compound **439-3.** LC-MS (ESI): m/z 635.2 $[M+H]^+$.

**Preparation of compound 439**

Compound **439-3** (100 mg, 0.16 mmol), dimethylphosphine oxide (37 mg, 0.48 mmol), and potassium carbonate (66 mg, 0.48 mmol) were dissolved in DMF (5 mL). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 55.52 mg of the title compound **439.** LC-MS (ESI): m/z 633.2 $[M+H]^+$. $^1$H NMR (400 MHz, Chloroform-*d*) δ 8.81 (d, *J* = 5.1 Hz, 1H),

8.12 (d, $J$ = 1.6 Hz, 1H), 7.82 (dd, $J$ = 8.0, 1.8 Hz, 1H), 7.60 (d, $J$ = 8.5 Hz, 2H), 7.55 (d, $J$ = 7.9 Hz, 1H), 7.50 (d, $J$ = 2.4 Hz, 1H), 7.41 - 7.36 (m, 2H), 7.29 (d, $J$ = 8.4 Hz, 2H), 5.01 (s, 2H), 4.58 (s, 2H), 4.44 (t, $J$ = 6.2 Hz, 2H), 3.88 (t, $J$ = 6.2 Hz, 2H), 1.85 (d, $J$ = 13.5 Hz, 6H), 1.72 (s, 6H). $^{31}$P NMR (162 MHz, Chloroform-$d$) δ 35.24 (s, 1P).

**Example 440: Preparation of compound 440**

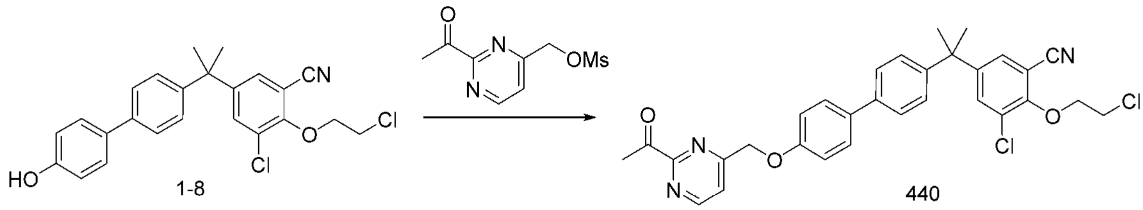

**Preparation of compound 440**

Compound **1-8** (100 mg, 0.23 mmol), (2-acetylpyrimidin-4-yl)methyl methanesulfonate (106 mg, 0.46 mmol), and potassium carbonate (95 mg, 0.69 mmol) were dissolved in DMF (5 mL). The reaction system was stirred at 80°C for 16 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 55 mg of the title compound **440.** LC-MS (ESI): m/z 560.2 [M+H]$^+$. $^1$H NMR (400 MHz, Chloroform-$d$) δ 8.94 (d, $J$ = 5.1 Hz, 1H), 7.76 (d, $J$ = 5.0 Hz, 1H), 7.58 - 7.52 (m, 2H), 7.52 - 7.46 (m, 3H), 7.37 (d, $J$ = 2.3 Hz, 1H), 7.25 - 7.20 (m, 2H), 7.07 - 7.01 (m, 2H), 5.33 (s, 2H), 4.43 (t, $J$ = 6.2 Hz, 2H), 3.88 (t, $J$ = 6.2 Hz, 2H), 2.82 (s, 3H), 1.69 (s, 6H).

**Example 441: Preparation of compound 441**

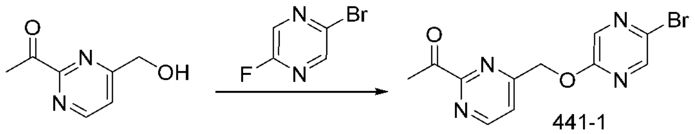

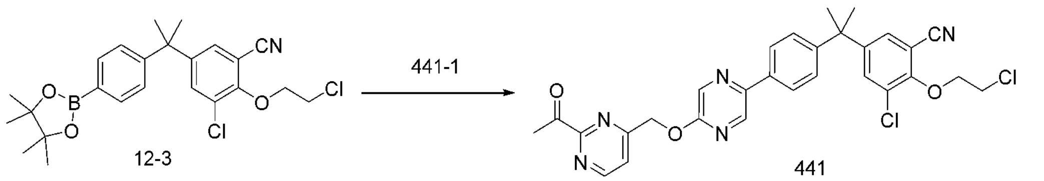

**Preparation of compound 441-1**

1-(4-(Hydroxymethyl)pyrimidin-2-yl)ethan-1-one (150 mg, 0.99 mmol), 2-fluoro-5-bromopyrazine (262 mg, 1.48 mmol), and potassium carbonate (410 mg, 2.97 mmol) were dissolved in DMF (5 mL). The reaction system was stirred at 80°C for 16 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 60%) to obtain 80 mg of the title compound **441-1.** LC-MS (ESI): m/z 309.0 [M+H]$^+$.

**Preparation of compound 441**

Compound **441-1** (80 mg, 0.26 mmol), compound **12-3** (119 mg, 0.26 mmol), and potassium carbonate (72 mg, 0.52 mmol) were dissolved in 1,4-dioxane (10 mL) and water (2 mL), and Pd(dppf)$Cl_2$ (73 mg, 0.1 mmol) was added thereto. After the addition was completed, the reaction system was stirred at 80°C for 3 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 27 mg of the title compound **441.** LC-MS (ESI): m/z 562.2 $[M+H]^+$. $^1H$ NMR (400 MHz, Chloroform-*d*) δ 8.92 (d, *J* = 4.9 Hz, 1H), 8.47 (d, *J* = 3.2 Hz, 2H), 7.87 (d, *J* = 8.0 Hz, 2H), 7.60 (d, *J* = 4.9 Hz, 1H), 7.46 (d, *J* = 2.3 Hz, 1H), 7.36 (d, *J* = 2.3 Hz, 1H), 7.30 (d, *J* = 8.0 Hz, 2H), 5.65 (s, 2H), 4.43 (t, *J* = 6.2 Hz, 2H), 3.88 (t, *J* = 6.2 Hz, 2H), 2.80 (s, 3H), 1.70 (s, 6H).

**Example 442: Preparation of compound 442**

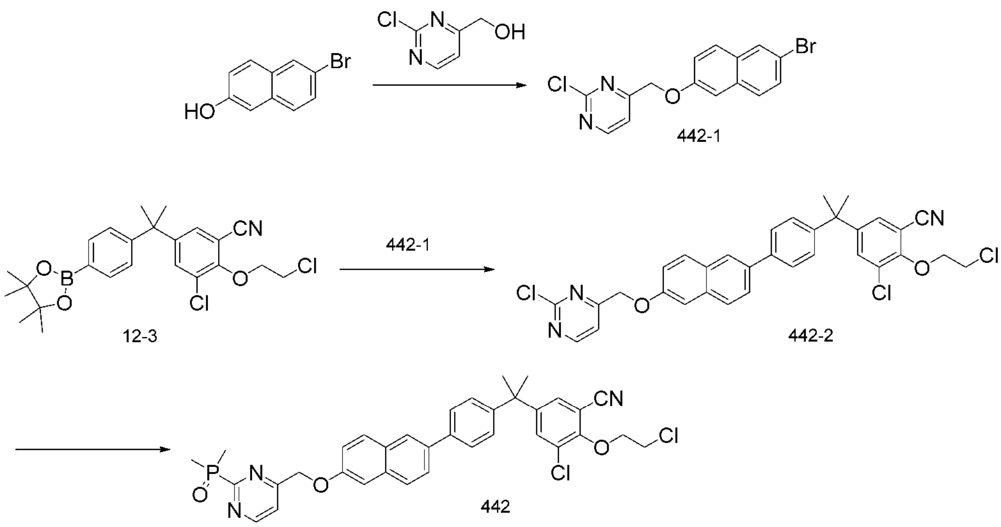


442-1

12-3

442-2

442

**Preparation of compound 442-1**

6-Bromo-2-naphthol (400 mg, 1.79 mmol) was added to a three-necked flask, followed by the sequential addition of anhydrous DCM (10 mL), (2-chloropyrimidin-4-yl)methanol (285 mg, 1.97 mmol), and *N*,*N*,*N*',*N*'-tetramethylazodicarboxamide (618 mg, 3.59 mmol). The system was purged three times with nitrogen, and tributylphosphine (726 mg, 3.59 mmol) was added at 0°C. The reaction was stirred at 25°C for 3 h. After the reaction was completed, water (20 mL) was added, and the mixture was extracted twice with DCM (20 mL). The organic phases were combined, dried, and concentrated to obtain a crude product, which was purified by normal-phase silica gel column chromatography (EtOAc/PE = 0 to 30%) to obtain 340 mg of the title compound **442-1** in 54% yield. LC-MS (ESI): m/z 348.9 $[M+H]^+$.

**Preparation of compound 442-2**

Compound **442-1** (200 mg, 0.57 mmol), compound **12-3** (263 mg, 0.57 mmol), [1,1'-bis(diphenylphosphino) ferrocene]dichloropalladium(II) (42 mg, 0.06 mmol), and potassium carbonate (198 mg, 1.43 mmol) were weighed and added to a mixed solvent of 1,4-dioxane (10 mL) and water (2 mL). The system was purged three times with nitrogen, and the reaction was carried out at 90°C for 4 h. After the reaction was monitored by LCMS until completion, the reaction mixture was poured into ice water and extracted twice with ethyl acetate (20 mL). The organic phases were combined, dried over anhydrous sodium sulfate, and concentrated to obtain a crude product. The crude product was purified by normal-phase silica gel column chromatography (EA/PE = 0 to 40%) to obtain 190 mg of the title compound **442-2** in 55% yield. LC-MS (ESI): m/z 602.2 $[M+H]^+$.

**Preparation of compound 442**

Compound **442-2** (80 mg, 0.13 mmol) was dissolved in DMF (3 mL), followed by the sequential addition of tris(dibenzylideneacetone)dipalladium (12 mg, 0.01 mmol), 4,5-bis(diphenylphosphino)-9,9-dimethylxanthene (15 mg, 0.03 mmol), DIEA (49 mg, 0.38 mmol), and dimethylphosphine oxide (31 mg, 0.4 mmol). The reaction system was stirred at 120°C for 2 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 40 mg of the title compound **442.** LC-MS (ESI): m/z 644.0 $[M+H]^+$. $^1$H NMR (400 MHz, DMSO-$d_6$) δ 9.02 (d, *J* = 5.2 Hz, 1H), 8.16 (d, *J* = 1.8 Hz, 1H), 7.96 (d, *J* = 9.0 Hz, 1H), 7.89 (d, *J* = 8.6 Hz, 1H), 7.84 - 7.77 (m, 2H), 7.76 - 7.70 (m, 3H), 7.66 (d, *J* = 2.3 Hz, 1H), 7.51 (d, *J* = 2.6 Hz, 1H), 7.41 - 7.33 (m, 3H), 5.44 (s, 2H), 4.43 (t, *J* = 5.2 Hz, 2H), 3.96 (t, *J* = 5.2 Hz, 2H), 1.78 (d, *J* = 13.7 Hz, 6H), 1.71 (s, 6H). $^{31}$P NMR (162 MHz, DMSO-d6) δ 34.05 (s, 1P).

**Example 443: Preparation of compound 443**

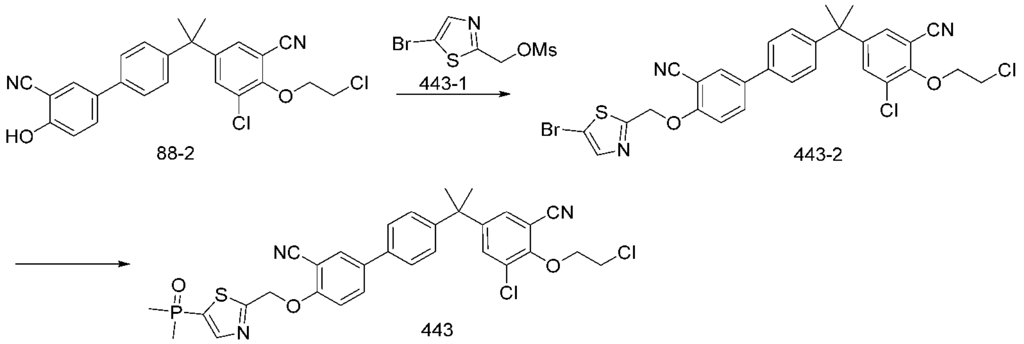


**Preparation of compound 443-2**

Compound **443-1** (65 mg, 241 μmol), compound **88-2** (120 mg, 265 μmol), cesium carbonate (156 mg, 482 μmol), and acetonitrile (2 mL) were added to a three-necked flask. The reaction system was purged three times with nitrogen and stirred at 75°C for 3 h. After the reaction was completed, saturated aqueous sodium chloride solution (10 mL) was added to the reaction system, and the mixture was extracted three times with DCM (10 mL). The organic phases were combined, dried, and concentrated to obtain a crude product. The crude product was purified by silica gel column chromatography (EA/PE = 0 to 60%) to obtain 110 mg of the title compound **443-2.**

**Preparation of compound 443**

Compound **443-2** (110 mg, 175 mmol) was dissolved in DMF (1.5 mL), followed by the sequential addition of tris(dibenzylideneacetone)dipalladium (32 mg, 35 μmol), 4,5-bis(diphenylphosphino)-9,9-dimethylxanthene (34 mg, 35 μmol), DIEA (68 mg, 525 μmol), and dimethylphosphine oxide (41 mg, 525 μmol). The reaction system was stirred under microwave irradiation at 120°C for 2 h. After the reaction was completed, saturated aqueous sodium chloride solution (10 mL) was added to the reaction system. The mixture was extracted three times with DCM (10 mL). The organic phases were combined, dried, subjected to suction filtration, and the filtrate was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 50% acetonitrile gradient elution over 12 min; flow rate: 30 mL/min) to obtain 28 mg of the title compound **443.** LC-MS (ESI): m/z 624.2 $[M+H]^+$. $^1$H NMR (400 MHz, ) δ 8.24 (d, *J* = 3.8 Hz, 1H), 8.12 (d, *J* = 2.4 Hz, 1H), 8.01 (dd, *J* = 8.9, 2.4 Hz, 1H), 7.71 (d, *J* = 2.4 Hz, 1H), 7.68 - 7.60 (m, 3H), 7.49 (d, *J* = 9.0 Hz, 1H), 7.34 (d, *J* = 8.5 Hz, 2H), 5.76 (s, 2H), 4.42 (t, *J* = 5.2 Hz, 2H), 3.96 (t, *J* = 5.2 Hz, 2H), 1.80 (d, *J* = 13.9 Hz, 6H), 1.69 (s, 6H). $^{31}$P NMR (162 MHz, DMSO-$d_6$) δ 28.02. (s, 1P).

**Example 444: Preparation of compound 444**

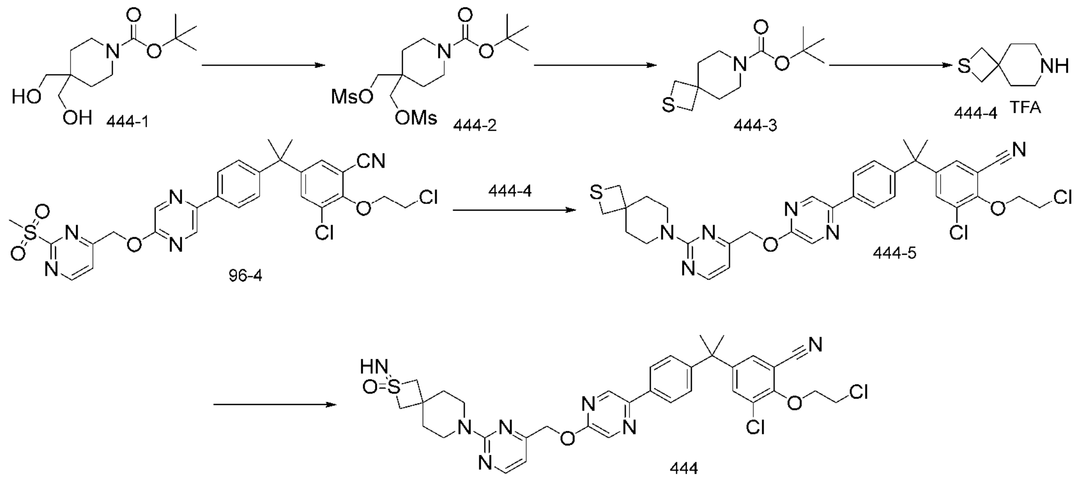


**Preparation of compound 444-2**

Compound **444-1** (1 g, 4.08 mmol) was weighed and dissolved in dichloromethane (20 mL), and triethylamine (1.65 g, 16.3 mmol) was added with stirring. Methanesulfonyl chloride (1.4 g, 12.2 mmol) was added dropwise at 0°C, and the reaction was carried out at room temperature for 2 h. After the reaction was monitored by LCMS until completion, water (20 mL) was added, and the mixture was extracted twice with DCM (20 mL). The organic phases were combined, dried, and concentrated to obtain 1.5 g of a crude product of the title compound **444-2.** The crude product was used directly in the next step without purification. LC-MS (ESI): m/z 346.0 $[M+H-56]^+$.

**Preparation of compound 444-3**

Compound **444-2** (1.5 g, 3.74 mmol) and sodium sulfide nonahydrate (1.08 g, 4.48 mmol) were weighed and added to anhydrous ethanol (15 mL), and the mixture was refluxed overnight. After the reaction was monitored by LCMS until completion, the reaction mixture was filtered. The crude filtrate was purified by normal-phase silica gel column chromatography (EA/PE = 0 to 60%) to obtain 250 mg of the title compound **444-3** in 28% yield. LC-MS (ESI): m/z 188.0 $[M+H-56]^+$.

**Preparation of compound 444-4**

Compound **444-3** (230 mg, 0.95 mmol) was weighed and dissolved in dichloromethane (2 mL), and trifluoroacetic acid (539 mg, 4.73 mmol) was added dropwise at 0°C. After the addition was completed, the reaction was carried out at room temperature for 3 h. After the reaction was monitored by LCMS until completion, the reaction mixture was concentrated to obtain 100 mg of a crude product of the title compound. The crude product was used directly in the next step without purification. LC-MS (ESI): m/z 144.0 $[M+H]^+$.

**Preparation of compound 444-5**

Compound **96-4** (80 mg, 0.14 mmol) was added to a microwave tube, followed by the sequential addition of anhydrous ethanol (2 mL), compound **444-4** (57 mg, 0.4 mmol), and DIPEA (52 mg, 0.40 mmol). The system was purged three times with nitrogen, and the reaction was carried out under microwave irradiation at 90°C for 2 h. After the reaction was monitored by LCMS until completion, the reaction mixture was filtered. The crude filtrate was purified by normal-phase silica gel column chromatography (EA/PE = 0 to 60%) to obtain 50 mg of the title compound **444-5** in 57% yield. LC-MS (ESI): m/z 661.2 $[M+H]^+$.

**Preparation of compound 444**

Compound **444-5** (50 mg, 0.08 mmol) and ammonium carbonate (22 mg, 0.23 mmol) were dissolved in methanol

(5 mL). (Diacetoxyiodo)benzene (122 mg, 0.38 mmol) was added thereto at 0°C. After the addition was completed, the reaction was carried out at room temperature overnight. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **444.** LC-MS (ESI): m/z 692.2 $[M+H]^+$. $^1H$ NMR (400 MHz, DMSO-$d_6$) δ 8.74 (d, *J* = 1.4 Hz, 1H), 8.53 (d, *J* = 1.4 Hz, 1H), 8.32 (d, *J* = 5.0 Hz, 1H), 7.96 (d, *J* = 8.5 Hz, 2H), 7.70 (d, *J* = 2.3 Hz, 1H), 7.63 (d, *J* = 2.4 Hz, 1H), 7.37 (d, *J* = 8.6 Hz, 2H), 6.63 (d, *J* = 5.0 Hz, 1H), 5.35 (s, 2H), 4.49 (s, 1H), 4.42 (t, *J* = 5.2 Hz, 2H), 3.96 (t, *J* = 5.2 Hz, 2H), 3.86 - 3.73 (m, 4H), 3.73 - 3.62 (m, 4H), 1.81 - 1.72 (m, 4H), 1.69 (s, 6H).

**Example 445: Preparation of compound 445**

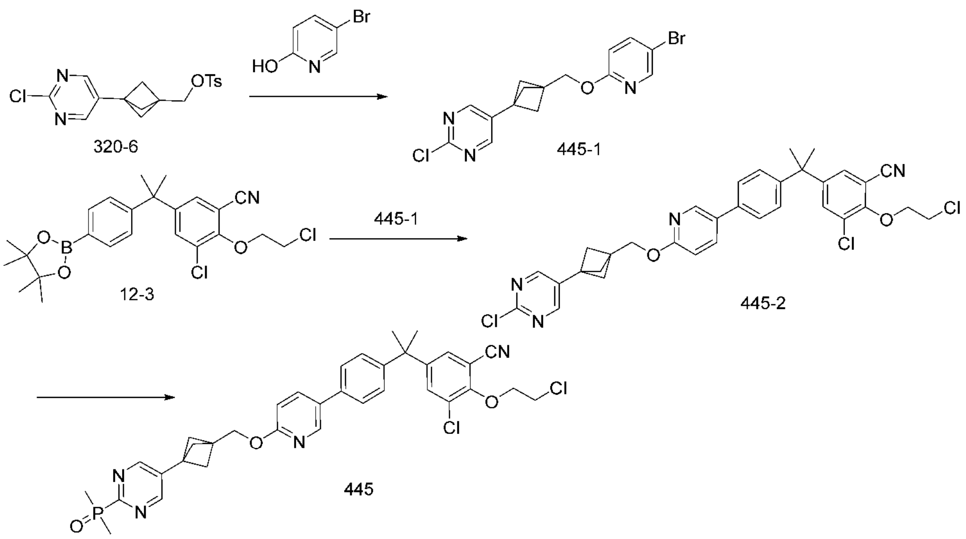


**Preparation of compound 445-1**

Compound **320-6** (150 mg, 0.41 mmol) and 2-hydroxy-5-bromopyridine (86 mg, 0.49 mmol) were dissolved in acetonitrile (5 mL), and cesium carbonate (400 mg, 1.23 mmol) was added at room temperature. The mixture was heated to 60°C and stirred for 2 h. The reaction mixture was filtered and concentrated. The resulting residue was purified by preparative TLC (ethyl acetate/petroleum ether = 1:4) to obtain 60 mg of the title compound **445-1.** LC-MS (ESI): m/z 366.0 $[M+H]^+$. $^1H$ NMR (400 MHz, $CDCl_3$) δ 8.38 (s, 2H), 8.09 (d, *J* = 2.4 Hz, 1H), 7.63 - 7.54 (m, 1H), 6.61 (d, *J* = 8.8 Hz, 1H), 4.31 (s, 2H), 2.08 (s, 6H).

**Preparation of compound 445-2**

Compound **445-1** (50 mg, 0.14 mmol) and compound **12-3** (77 mg, 0.17 mmol) were dissolved in 1,4-dioxane (5 mL). Pd(dppf)$Cl_2$ (10 mg, 0.014 mmol) and potassium carbonate (58 mg, 0.42 mmol) were added at room temperature. The system was purged with argon, and then the mixture was stirred at 100°C for 16 h. The reaction was monitored by LCMS until completion. The reaction mixture was subjected to rotary evaporation until dryness, and ethyl acetate (20 mL) was added. The organic phase was washed with saturated sodium chloride (20 mL) and concentrated. The residue was purified by preparative TLC (ethyl acetate/petroleum ether = 1/2) to obtain 25 mg of the title compound **445-2.** LC-MS (ESI): m/z 618.7 $[M+H]^+$.

**Preparation of compound 445**

Compound **445-2** (25 mg, 0.04 mmol) was dissolved in *N,N*-dimethylformamide (2 mL). Dimethylphosphine oxide (6 mg, 0.080 mmol), palladium acetate (1 mg, 0.004 mmol), 1,3-bis(diphenylphosphino)propane (2 mg, 0.0040 mmol), and *N,N*-diisopropylethylamine (16 mg, 0.12 mmol) were added at room temperature. After purging with argon for 30 seconds, the mixture was stirred under microwave irradiation at 120°C for 0.5 h. The reaction was monitored by LCMS until completion. The reaction mixture was filtered. The filtrate was subjected to preparative purification (Waters

2767/QDA; column: Atlatis T3 prep OBD, C18 19 * 250 mm, 10 μm; mobile phase A: 0.1% FA/$H_2O$, B: ACN; flow rate: 20 mL/min; elution gradient: 82% to 87%; retention time: 7.2-8.2 min of 16 min) to obtain 10.12 mg of the title compound **445.** LC-MS (ESI): m/z 661.3 $[M+H]^+$. $^1H$ NMR (400 MHz, DMSO-$d_6$) δ 8.87 (s, 2H), 8.46 (d, *J* = 2.5 Hz, 1H), 8.01 (dd, *J* = 8.6, 2.5 Hz, 1H), 7.70 (d, *J* = 2.3 Hz, 1H), 7.64 (d, *J* = 2.2 Hz, 1H), 7.61 (d, *J* = 8.4 Hz, 2H), 7.34 (d, *J* = 8.4 Hz, 2H), 6.92 (d, *J* = 8.6 Hz, 1H), 4.50 - 4.38 (m, 4H), 3.96 (t, *J* = 5.2 Hz, 2H), 2.15 (s, 6H), 1.73 (d, *J* = 13.7 Hz, 6H), 1.69 (s, 6H). $^{31}P$ NMR (162 MHz, DMSO-*d6*) δ 33.69 (s, 1P).

**Example 446: Preparation of compound 446**

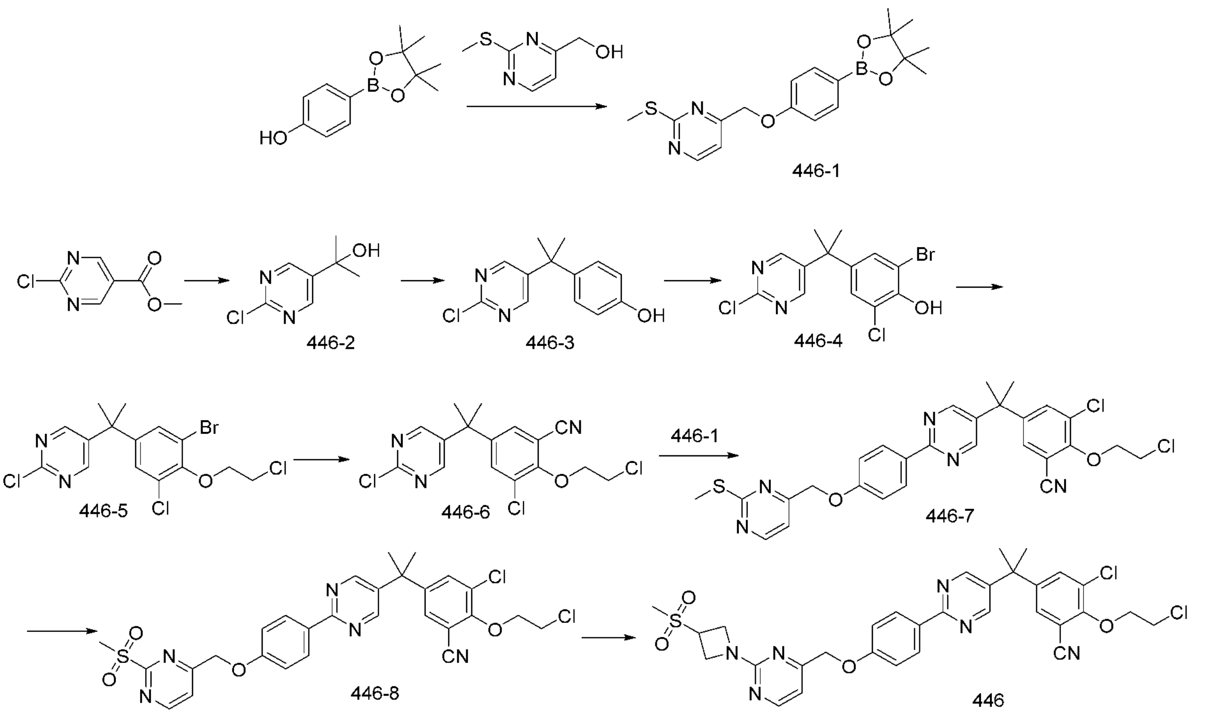

446-1

446-2 446-3 446-4

446-5 446-6 446-7

446-8 446

**Preparation of compound 446-1**

Under a nitrogen atmosphere, 4-(4,4,5,5-tetramethyl-1,3,2-dioxaborolan-2-yl)phenol (500 mg, 2.27 mmol), (2-(methylthio)pyrimidin-4-yl)methanol (355 mg, 2.27 mmol), TMAD (1.17 g, 6.82 mmol), and DCM (5 mL) were added to a reaction flask. Tributylphosphine (1.38 g, 6.82 mmol) was added dropwise at 0°C, and the mixture was stirred for 2 h. Water (10 mL) was added, and the mixture was extracted three times with DCM. The organic phases were combined and subjected to rotary evaporation until dryness. The resulting residue was purified by silica gel column chromatography (PE/EA = 0 to 50%) to obtain 700 mg of the title compound **446-1.** LC-MS (ESI): m/z 359.2 $[M+H]^+$.

**Preparation of compound 446-2**

Methyl 2-chloropyrimidine-5-carboxylate (9.5 g, 55.05 mmol) was added to THF (50 mL). The system was purged three times with argon. At -10°C, a solution of methylmagnesium bromide in tetrahydrofuran (3 M, 165.15 mmol, 55 mL) was slowly added dropwise. The mixture was maintained at -10°C for 2 h. The reaction mixture was poured into saturated aqueous ammonium chloride solution at 0°C to quench the reaction. Water (50 mL) was added, and the mixture was extracted three times with ethyl acetate (50 mL). The organic phase was subjected to rotary evaporation until dryness. The crude product was purified by silica gel column chromatography (EtOAc/PE = 0 to 50%) to obtain 5.3 g of compound **446-2.** LC-MS (ESI): m/z 173.0 $[M+H]^+$.

**Preparation of compound 446-3**

Phenol (15 g, 159.32 mmol), compound **446-2** (5.5 g, 31.86 mmol), $AlCl_3$ (8.50 g, 63.73 mmol), and DCE (50 mL) were added to a reaction flask. The mixture was stirred at 80°C for 2 h. After cooling to 0°C, saturated aqueous ammonium

chloride solution (100 mL) was slowly added dropwise. The mixture was extracted three times with dichloromethane (50 mL). The organic phases were combined and subjected to rotary evaporation until dryness. The resulting crude product was purified by silica gel column chromatography (EA/PE = 0 to 50%) to obtain 530 mg of the title compound **446-3.** $^1H$ NMR (400 MHz, DMSO-$d_6$) δ 9.33 (d, *J* = 2.8 Hz, 1H), 8.60 (s, 2H), 7.08 - 7.02 (m, 2H), 6.71 - 6.67 (m, 2H), 1.65 (s, 6H).

**Preparation of compound 446-4**

Compound **446-3** (430 mg, 1.73 mmol) and diisopropylamine hydrochloride (12 mg, 86.45 μmol) were dissolved in toluene (5 mL). The system was cooled to 0°C, followed by the slow addition of 1,3-dichloro-5,5-dimethylhydantoin (375 mg, 1.90 mmol). After stirring at 0°C for 1 h, NBS (338 mg, 1.90 mmol) was added. After stirring for 1 h, water (20 mL) was added to the system, and the mixture was extracted three times with dichloromethane (10 mL). The organic phases were combined and subjected to rotary evaporation until dryness. The crude product was purified by silica gel column chromatography (EA/PE = 0 to 50%) to obtain 300 mg of compound **446-4.** LC-MS (ESI): m/z 360.9 $[M+H]^+$.

**Preparation of compound 446-5**

Potassium carbonate (344 mg, 2.49 mmol) was added to a solution of compound **446-4** (300 mg, 828.62 μmol) and 1-bromo-2-chloroethane (357 mg, 2.49 mmol, 206.9 μL) in acetonitrile (3 mL). The reaction mixture was stirred at 70°C for 16 h. The mixture was filtered and subjected to rotary evaporation until dryness to obtain 330 mg of the title compound **446-5.** LC-MS (ESI): m/z 422.8 $[M+H]^+$.

**Preparation of compound 446-6**

Under a nitrogen atmosphere, compound **446-5** (350 mg, 824.41 μmol), zinc cyanide (194 mg, 1.65 mmol), tetrakis(triphenylphosphine)palladium (381 mg, 329.76 μmol), and DMF (8 mL) were added to a reaction flask. The mixture was stirred at 95°C for 2 h. Water (5 mL) was added, and the mixture was extracted three times with ethyl acetate (5 mL). The organic phases were combined and subjected to rotary evaporation until dryness. The crude product was purified by silica gel column chromatography (EA/PE = 0 to 50%) to obtain 40 mg of the title compound **446-6.** $^1H$ NMR (400 MHz, Chloroform-d) δ 8.68 (s, 2H), 7.40 (d, *J* = 2.5 Hz, 1H), 7.36 (d, *J* = 2.5 Hz, 1H), 4.48 (t, *J* = 5.9 Hz, 2H), 3.89 (t, *J* = 6.0 Hz, 2H), 1.77 (s, 6H).

**Preparation of compound 446-7**

Under a nitrogen atmosphere, compound **446-6** (58 mg, 161.87 μmol), compound **446-1** (50 mg, 134.89 μmol), Pd(dppf)$Cl_2$ (20 mg, 26.98 μmol), and potassium carbonate (56 mg, 404.68 μmol) were dissolved in water (0.5 mL) and dioxane (2 mL). The system was purged three times with nitrogen and stirred at 100°C for 16 h. Water (10 mL) was added, and the mixture was extracted three times with DCM (10 mL). The organic phases were combined and subjected to rotary evaporation until dryness. The residue was purified by silica gel column chromatography (EA/PE = 0 to 50%) to obtain 10 mg of the title compound **446-7.** LC-MS (ESI): m/z 566.2 $[M+H]^+$.

**Preparation of compound 446-8**

Compound **446-7** (10 mg, 17.65 μmol), DCM (2 mL), and *m*-CPBA (13 mg, 44.10 μmol) were stirred at room temperature for 2 h. Sodium bicarbonate solution (5 mL) was added, and the mixture was extracted three times with ethyl acetate (4 mL). The organic phases were combined, dried over anhydrous sodium sulfate, filtered, and subjected to rotary evaporation until dryness to obtain 10 mg of the title compound**446-8**. LC-MS (ESI): m/z 598.2 $[M+H]^+$.

**Preparation of compound 446**

3-(Methylsulfonyl)azetidine hydrochloride (4 mg, 25.06 μmol), compound **446-8** (10 mg, 16.71 μmol), DIPEA (7 mg, 50.13 μmol), and acetonitrile (2 mL) were added to a reaction flask, and the mixture was stirred at 80°C for 2 h. The reaction mixture was subjected to rotary evaporation until dryness, and the resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 90% acetonitrile gradient elution over 15 min; flow rate: 30 mL/min) to obtain 3 mg of the title compound **446.** LC-MS (ESI): m/z 653.2 $[M+H]^+$. $^1H$ NMR (400 MHz, DMSO-$d_6$) δ 8.74 (s, 2H), 8.43 (d, *J* = 5.0 Hz, 1H), 8.32 (d, *J* = 8.9 Hz, 2H), 7.80 (q, *J* = 2.4 Hz, 2H), 7.15 (d, *J* = 8.9 Hz, 2H), 6.87 (d, *J* = 5.0 Hz, 1H), 5.13 (s, 2H), 4.43 (t, *J* = 5.2 Hz, 2H), 4.40 (t, *J* = 4.8 Hz, 1H), 4.34 (t, *J* = 8.7 Hz, 2H), 4.27 - 4.15 (m, 2H), 3.96 (t, *J*= 5.2 Hz, 2H), 3.07 (s, 3H), 1.74 (s, 6H).

**Example 447: Preparation of compound 447**

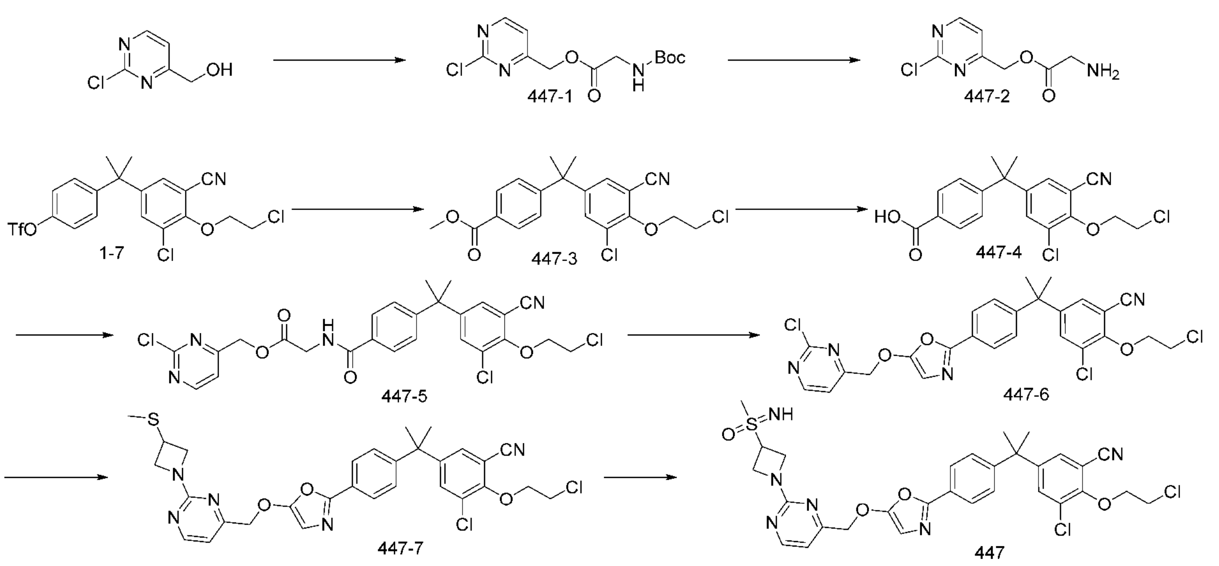

**Preparation of compound 447-1**

(2-Chloropyrimidin-4-yl)methanol (1.0 g, 6.92 mmol), *N*-(*tert*-butoxycarbonyl)glycinamide (1.21 g, 6.92 mmol), and DMAP (0.845 g, 6.92 mmol) were dissolved in dichloromethane (20 mL), and EDCI (1.99 g, 10.38 mmol) was added thereto. After the addition was completed, the reaction system was stirred at room temperature for 1 h. After the reaction was completed, 5% dilute hydrochloric acid solution (50 mL) was added, and the mixture was extracted three times with dichloromethane (25 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 85%) to obtain 1.99 g of the title compound **447-1.**

**Preparation of compound 447-2**

Compound **447-1** (3.4 g, 0.264 mmol) was dissolved in dichloromethane (20 mL), and trifluoroacetic acid (4 mL) was added thereto. After the addition was completed, the reaction system was stirred at room temperature for 1 h. After the reaction was completed, the reaction mixture was concentrated to obtain 2.31 g of a crude product of the title compound **447-2,** which was used directly in the next step. LC-MS (ESI): m/z 202.2 $[M+H]^+$.

**Preparation of compound 447-3**

Compound **1-7** (2 g, 4.15 mmol), dicobalt octacarbonyl (2.84 g, 8.29 mmol), Xantphos (0.24 g, 0.415 mmol), $Pd_2(dba)_3$ (0.38 g, 0.415 mmol), and TEA (1.26 g, 12.44 mmol) were dissolved in DMF (30 mL) and methanol (5 mL). The reaction system was stirred at 75°C for 12 h. After the reaction was completed, water (50 mL) was added, and the mixture was extracted three times with ethyl acetate (50 mL). The organic phases were combined, washed three times with 10% aqueous lithium chloride solution (30 mL), dried over anhydrous sodium sulfate, filtered, and the filtrate was concentrated to obtain a crude product. The crude product was purified by silica gel column chromatography (PE/EA = 100% to 75%) to obtain 1.15 g of the title compound **447-3.** LC-MS (ESI): m/z 392.2 $[M+H]^+$.

**Preparation of compound 447-4**

Compound **447-3** (1.2 g, 3.06 mmol) was dissolved in tetrahydrofuran (10 mL) and methanol (3 mL). A solution of lithium hydroxide monohydrate (0.244 g, 6.11 mmol) in water (3 mL) was added dropwise thereto. After the addition was completed, the reaction system was stirred at room temperature for 1 h. After the reaction was completed, the pH of the reaction system was adjusted to approximately 3 with 10% dilute hydrochloric acid solution, and the mixture was extracted three times with ethyl acetate (50 mL). The organic phases were combined, washed three times with saturated brine (30 mL), dried over anhydrous sodium sulfate, filtered, and the filtrate was concentrated to obtain 1.1 g of a crude product of the

title compound **447-4,** which was used directly in the next step. LC-MS (ESI): m/z 378.2 [M+H]+.

**Preparation of compound 447-5**

Compound **447-4** (500 mg, 1.32 mmol), compound **447-2** (266 mg, 1.32 mmol), and 1-methylimidazole (143 mg, 1.72 mmol) were placed in a single-necked flask, and acetonitrile (5 mL) was added. Subsequently, *N*,*N*,*N*',*N*'-tetramethylchloroformamidinium hexafluorophosphate (455 mg, 1.59 mmol) was added, and the reaction system was stirred at room temperature for 0.5 h. A 10% aqueous sodium chloride solution (20 mL) was added, and the mixture was extracted three times with dichloromethane (20 mL). The organic phases were combined, washed twice with saturated brine (20 mL), dried over anhydrous sodium sulfate, filtered, and the filtrate was concentrated to obtain a crude product. The crude product was purified by silica gel column chromatography (PE/EA = 100% to 75%) to obtain 0.7 g of the title compound **447-5.** LC-MS (ESI): m/z 561.1 [M+H]+.

**Preparation of compound 447-6**

Compound **447-5** (0.72 g, 1.28 mmol) was dissolved in acetonitrile (50 mL), and phosphorus pentoxide (0.364 g, 2.56 mmol) was added thereto. After the addition was completed, the reaction system was stirred at 70°C for 0.5 h. After the reaction was completed, the reaction mixture was filtered, and the filtrate was concentrated to obtain a crude product. The crude product was purified by silica gel column chromatography (PE/EA = 100% to 85%) to obtain 0.5 g of the title compound **447-6.** LC-MS (ESI): m/z 543.1[M+H]+.

**Preparation of compound 447-7**

Compound **447-6** (100 mg, 0.184 mmol), 3-(methylsulfanyl)azetidine hydrochloride (31 mg, 0.221 mmol), and DIEA (47 mg, 0.368 mmol) were placed in a sealed tube, and acetonitrile (2 mL) was added. The reaction system was stirred at 90°C for 16 h. After the reaction was completed, insoluble solids were removed by filtration. A 5% aqueous lithium chloride solution (20 mL) was added, and the mixture was extracted three times with ethyl acetate (20 mL). The organic phases were combined, washed twice with saturated brine (20 mL), dried over anhydrous sodium sulfate, filtered, and the filtrate was concentrated to obtain a crude product. The crude product was purified by silica gel column chromatography (PE/EA = 100% to 85%) to obtain 100 mg of the title compound **447-7.** LC-MS (ESI): m/z 610.2 [M+H]+.

**Preparation of compound 447**

Compound **447-7** (100 mg, 0.164 mmol), (diacetoxyiodo)benzene (53 mg, 0.164 mmol), and ammonium carbonate (13 mg, 0.164 mmol) were placed in a sealed tube, and methanol (2 mL) was added. The reaction system was stirred at room temperature for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 50 mg of the title compound **447.** LC-MS (ESI): m/z 642.2 [M+H]+. $^1$H NMR (400 MHz, Chloroform-*d*) δ 8.40 (d, *J* = 5.1 Hz, 1H), 7.86 (d, *J* = 8.5 Hz, 2H), 7.42 (d, *J* = 2.3 Hz, 1H), 7.35 (d, *J* = 2.3 Hz, 1H), 7.25 (d, *J* = 8.5 Hz, 2H), 6.89 (d, *J* = 5.1 Hz, 1H), 6.28 (s, 1H), 5.06 (s, 2H), 4.52 - 4.38 (m, 6H), 4.23 - 4.13 (m, 1H), 3.87 (t, *J* = 6.1 Hz, 2H), 3.01 (s, 3H), 1.68 (s, 6H).

**Example 448: Preparation of compound 448**

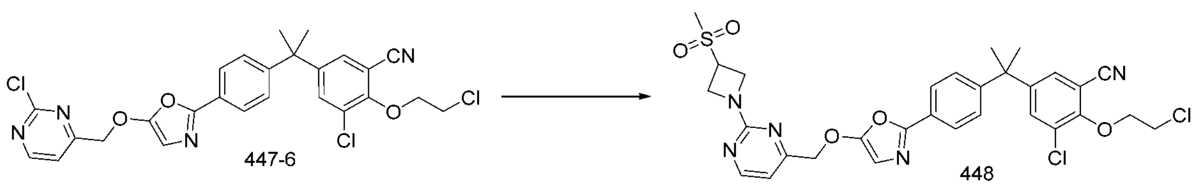
447-6
448

**Preparation of compound 448**

Compound **447-6** (50 mg, 0.092 mmol), 3-(methylsulfonyl)azetidine hydrochloride (16 mg, 0.092 mmol), and

DIEA (12 mg, 0.092 mmol) were placed in a sealed tube, and acetonitrile (2 mL) was added. The reaction system was stirred at 90°C for 16 h. After the reaction was completed, insoluble solids were removed by filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **448.** LC-MS (ESI): m/z 641.2 $[M+H]^+$. $^1H$ NMR (400 MHz, Chloroform-*d*) δ 8.34 (dd, *J* = 5.0, 1.8 Hz, 1H), 7.79 (dd, *J* = 8.6, 1.8 Hz, 2H), 7.36 (q, *J* = 2.3 Hz, 1H), 7.28 (q, *J* = 2.3 Hz, 1H), 7.19 - 7.13 (m, 2H), 6.83 (dd, *J* = 5.1, 1.8 Hz, 1H), 6.21 (s, 1H), 4.99 (s, 2H), 4.46 - 4.29 (m, 6H), 4.08 - 3.99 (m, 1H), 3.83 - 3.77 (m, 2H), 2.88 (s, 3H), 1.61 (s, 6H).

**Example 449: Preparation of compound 449**

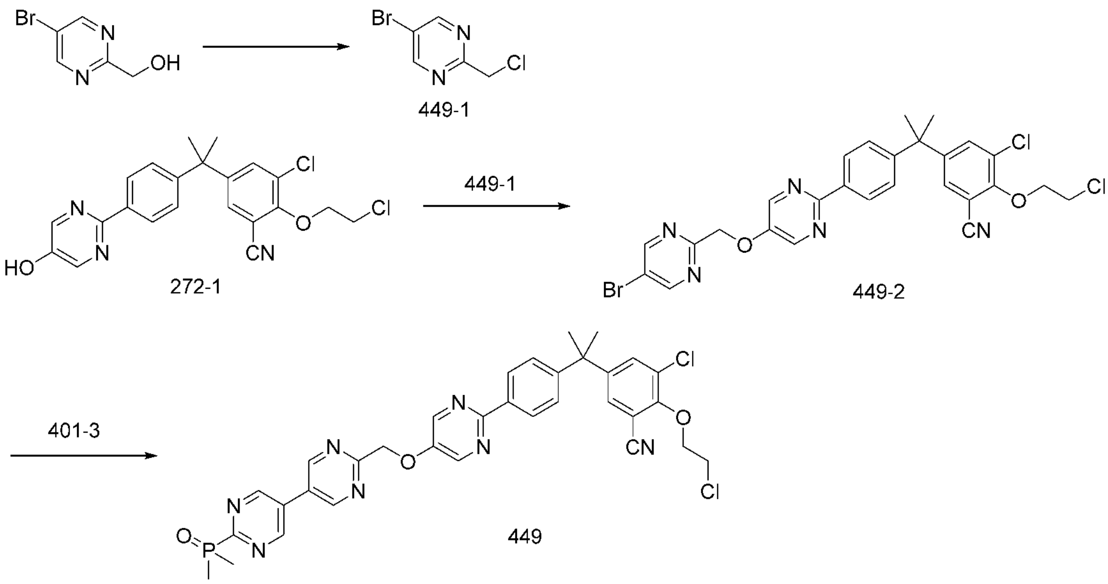

449-1
272-1
449-2
449

**Preparation of compound 449-1**

(5-Bromopyrimidin-2-yl)methanol (300 mg, 0.76 mmol) and thionyl chloride (456 mg, 3.8 mmol) were dissolved in DCM (5 mL), and the reaction system was stirred at room temperature for 1 h. After the reaction was completed, the reaction mixture was directly concentrated to obtain a crude product, yielding 325 mg of the title compound **449-1.** LC-MS (ESI): m/z 206.8 $[M+H]^+$.

**Preparation of compound 449-2**

Compound **272-1** (566 mg, 1.32 mmol) was dissolved in DMF (5 mL), followed by the sequential addition of **449-1** (329 mg, 1.59 mmol) and cesium carbonate (861 mg, 2.64 mmol). The reaction system was stirred at 90°C for 2 h. After the reaction was completed, water (100 mL) was added to the reaction mixture, and the mixture was extracted three times with dichloromethane (150 mL). The organic phases were combined, washed once with saturated brine (100 mL), dried over anhydrous sodium sulfate, subjected to suction filtration, and the filtrate was concentrated. The resulting crude product was purified by silica gel column chromatography (DCM/MeOH = 100 to 20%) to obtain 300 mg of the title compound **449-2.** LC-MS (ESI): m/z 598.2 $[M+H]^+$.

**Preparation of compound 449**

Compound **449-2** (68 mg, 0.10 mmol), compound **401-3** (46 mg, 0.20 mmol), potassium carbonate (28 mg, 0.2 mmol), and $Pd(dppf)Cl_2$ (7.3 mg, 0.01 mmol) were dissolved in 1,4-dioxane (12 mL) and water (3 mL). The reaction system was purged three times with nitrogen and stirred at 105°C overnight. After the reaction was completed, the reaction mixture was filtered through diatomite, and the filter cake was rinsed with dichloromethane (50 mL). Water (30 mL) was added to the filtrate, and the mixture was extracted three times with dichloromethane (70 mL). The organic phases were combined,

washed once with saturated brine (70 mL), dried over anhydrous sodium sulfate, subjected to suction filtration, and the filtrate was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 11.2 mg of the title compound **449.** LC-MS (ESI): m/z 674.2 $[M+H]^+$. $^1$H NMR (400 MHz, DMSO-$d_6$) δ 9.45 (s, 2H), 9.36 (s, 2H), 8.72 (s, 2H), 8.23 (d, *J* = 8.5 Hz, 2H), 7.69 (d, *J* = 2.4 Hz, 1H), 7.62 (d, *J* = 2.3 Hz, 1H), 7.38 (d, *J* = 8.6 Hz, 2H), 5.66 (s, 2H), 4.42 (t, *J* = 5.2 Hz, 2H), 3.95 (t, *J* = 5.2 Hz, 2H), 1.81 (d, *J* = 13.7 Hz, 6H), 1.69 (s, 6H). $^{31}$P NMR (162 MHz, DMSO-$d_6$) δ 34.20 (s, 1P).

**Example 450: Preparation of compound 450**

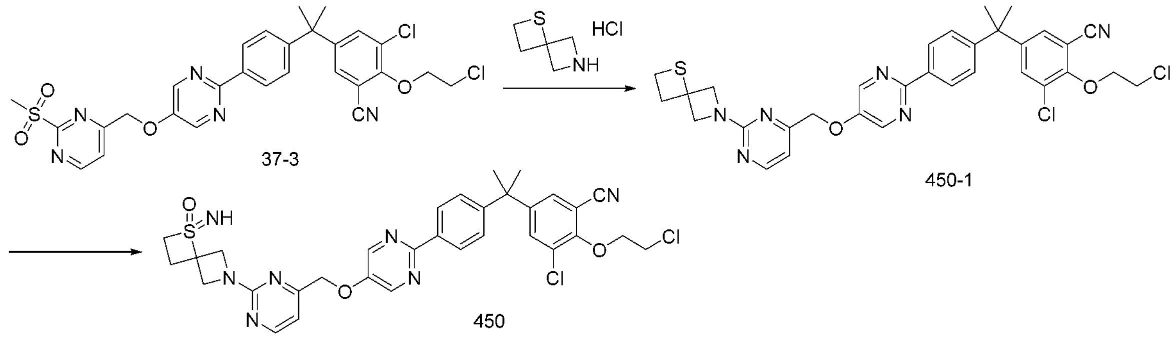

37-3

450-1

450

**Preparation of compound 450-1**

Compound **37-3** (200 mg, 0.34 mmol), 1-thia-6-azaspiro[3.3]heptane hydrochloride (39 mg, 0.34 mmol), and potassium carbonate (142 mg, 1.02 mmol) were dissolved in DMF (5 mL). The reaction system was stirred at 80°C for 16 h. After the reaction was completed, water (50 mL) was added, and the mixture was extracted three times with ethyl acetate (50 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 30%) to obtain 120 mg of the title compound **450-1.** LC-MS (ESI): m/z 633.2 $[M+H]^+$.

**Preparation of compound 450**

Compound **450-1** (120 mg, 0.19 mmol), ammonium carbonate (37 mg, 0.38 mmol), and (diacetoxyiodo)benzene (155 mg, 0.48 mmol) were dissolved in methanol (5 mL). The reaction system was stirred at 25°C for 1 h. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 91.76 mg of the title compound **450.** LC-MS (ESI): m/z 664.2 $[M+H]^+$. $^1$H NMR (400 MHz, Chloroform-*d*) δ 8.52 (s, 2H), 8.38 (d, *J* = 5.0 Hz, 1H), 8.31 - 8.25 (m, 2H), 7.44 (d, *J* = 2.4 Hz, 1H), 7.37 (d, *J* = 2.3 Hz, 1H), 7.30 - 7.27 (m, 2H), 6.84 (d, *J* = 5.0 Hz, 1H), 5.11 (s, 2H), 4.78 (dd, *J* = 10.5, 1.5 Hz, 1H), 4.73 (dd, *J* = 10.7, 1.5 Hz, 1H), 4.42 (t, *J* = 6.2 Hz, 2H), 4.26 (t, *J* = 10.7 Hz, 2H), 4.02 (td, *J* = 8.2, 3.0 Hz, 2H), 3.87 (t, *J* = 6.1 Hz, 2H), 2.51 (t, *J* = 8.8 Hz, 2H), 1.70 (s, 6H).

**Example 451: Preparation of compound 451**

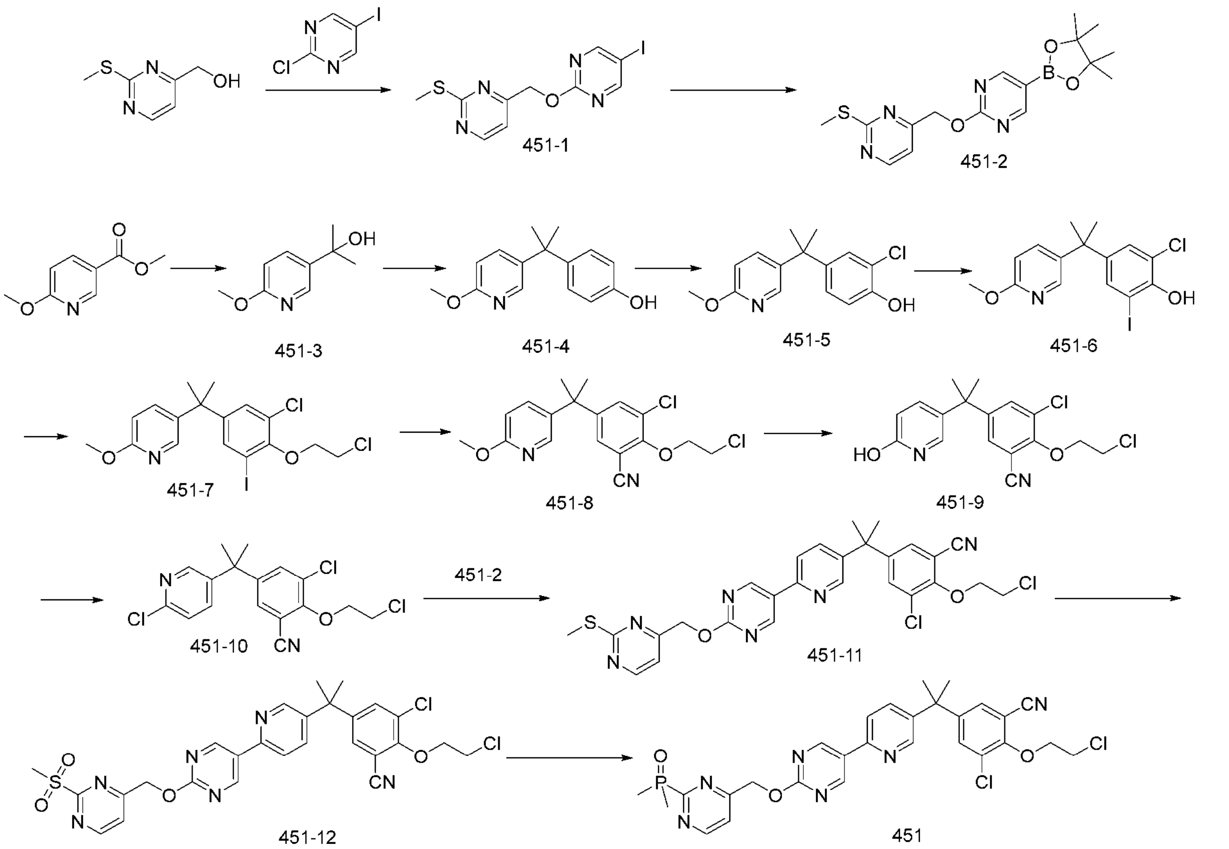


451-1, 451-2, 451-3, 451-4, 451-5, 451-6, 451-7, 451-8, 451-9, 451-10, 451-11, 451-12, 451

**Preparation of compound 451-1**

(2-(Methylthio)pyrimidin-4-yl)methanol (1.1 g, 6.41 mmol) was dissolved in THF (100 mL), cooled to 0°C, and then NaH (309 mg, 7.68 mmol, 60% purity) was added. After stirring at room temperature for 30 min, 2-chloro-5-iodopyrimidine (1.69 g, 7.05 mmol) was added, and the reaction system was stirred at 40°C for 2 h. The reaction was quenched with saturated ammonium chloride (100 mL) and extracted three times with dichloromethane (100 mL). The organic phases were combined, washed twice with saturated brine (20 mL), dried over anhydrous sodium sulfate, subjected to suction filtration, and the filtrate was concentrated to obtain 1.5 g of the title compound **451-1.**

**Preparation of compound 451-2**

Compound **451-1** (1.5 g, 4.16 mmol) was dissolved in 1,4-dioxane (80 mL). Bis(pinacolato)diboron (1.16 g, 4.58 mmol), potassium acetate (611 mg, 6.24 mmol), and Pd(dppf)$Cl_2$ (375 mg, 0.41 mmol) were added. The reaction system was purged three times with nitrogen and then stirred at 110°C for 12 h. After the reaction was monitored by LCMS until completion, the reaction mixture was concentrated to obtain 3.2 g of a crude product of the title compound **451-2,** which was used directly in the next step.

**Preparation of compound 451-3**

Methyl 6-methoxynicotinate (1 g, 5.98 mmol) was placed in a single-necked flask, and tetrahydrofuran (10 mL) was added. The reaction system was cooled to 0°C, and a solution of methylmagnesium bromide (5.98 mL, 3.0 M in diethyl ether) was slowly added dropwise. After the addition was completed, the reaction system was stirred at 0°C for additional 2 h. After the reaction was completed, the reaction was quenched with 20% aqueous ammonium chloride solution (20 mL), and the mixture was extracted three times with ethyl acetate (20 mL). The organic phases were combined, washed twice with saturated brine (20 mL), dried over anhydrous sodium sulfate, filtered, and the filtrate was concentrated to obtain a crude product. The crude product was purified by silica gel column chromatography (PE/EA = 100% to 100%) to obtain 1 g of the title compound **451-3.** LC-MS (ESI): m/z 168.2 $[M+H]^+$.

**Preparation of compound 451-4**

Compound **451-3** (1 g, 5.98 mmol) and phenol (0.732 g, 7.77 mmol) were placed in a single-necked flask, and dichloromethane (10 mL) was added. The reaction system was cooled to 0°C, and trifluoromethanesulfonic acid (2.24 g, 7.18 mmol) was slowly added dropwise. After the addition was completed, the reaction system was stirred at room temperature for 16 h. After the reaction was completed, saturated brine (30 mL) was added, and the mixture was extracted three times with dichloromethane (20 mL). The organic phases were combined, washed twice with saturated brine (20 mL), dried over anhydrous sodium sulfate, filtered, and the filtrate was concentrated to obtain a crude product. The crude product was purified by silica gel column chromatography (PE/EA = 100% to 100%) to obtain 0.7 g of the title compound **451-4.** $^1$H NMR (400 MHz, Chloroform-*d*) δ 8.01 (dd, *J* = 2.6, 0.7 Hz, 1H), 7.46 (dd, *J* = 8.7, 2.6 Hz, 1H), 7.07 - 7.02 (m, 2H), 6.74 - 6.70 (m, 2H), 6.68 (dd, *J* = 8.7, 0.7 Hz, 1H), 3.93 (s, 3H), 1.63 (s, 6H).

**Preparation of compound 451-5**

Compound **451-4** (0.771 g, 3.17 mmol) and aluminum trichloride (42 mg, 0.317 mmol) were placed in a single-necked flask, and dichloromethane (10 mL) was added. The reaction system was cooled to 0°C, and NCS (0.423 g, 3.17 mmol) was added in portions. After the addition was completed, the reaction system was stirred at room temperature for 16 h. After the reaction was completed, saturated aqueous ammonium chloride solution (30 mL) was added, and the mixture was extracted three times with dichloromethane (20 mL). The organic phases were combined, washed twice with saturated brine (20 mL), dried over anhydrous sodium sulfate, filtered, and the filtrate was concentrated to obtain a crude product. The crude product was purified by silica gel column chromatography (PE/EA = 100% to 100%) to obtain 0.88 g of the title compound **451-5.** LC-MS (ESI): m/z 278.0 $[M+H]^+$.

**Preparation of compound 451-6**

Compound **451-5** (0.75 g, 2.70 mmol) and silver sulfate (550 mg, 2.70 mmol) were placed in a single-necked flask, and ethanol (3 mL) was added. The reaction system was cooled to 0°C, and iodine (607 mg, 2.39 mmol) was added in portions. After the addition was completed, the reaction system was stirred at room temperature for 16 h. After the reaction was completed, saturated aqueous sodium sulfite solution (30 mL) was added, and the mixture was extracted three times with ethyl acetate (20 mL). The organic phases were combined, washed twice with saturated brine (20 mL), dried over anhydrous sodium sulfate, filtered, and the filtrate was concentrated to obtain a crude product. The crude product was purified by silica gel column chromatography (PE/EA = 100% to 100%) to obtain 0.66 g of the title compound **451-6.** LC-MS (ESI): m/z 404.0 $[M+H]^+$.

**Preparation of compound 451-7**

Compound **451-6** (600 mg, 1.49 mmol), 1-bromo-2-chloroethane (426 mg, 2.97 mmol), and cesium carbonate (1.45 g, 4.46 mmol) were placed in a sealed tube, and acetonitrile (2 mL) was added. The reaction system was stirred at room temperature for 2 h. After the reaction was completed, insoluble solids were removed by filtration. A 5% aqueous lithium chloride solution (20 mL) was added, and the mixture was extracted three times with ethyl acetate (20 mL). The organic phases were combined, washed twice with saturated brine (20 mL), dried over anhydrous sodium sulfate, filtered, and the filtrate was concentrated to obtain a crude product. The crude product was purified by silica gel column chromatography (PE/EA = 100% to 100%) to obtain 600 mg of the title compound **451-7.** LC-MS (ESI): m/z 466.0 $[M+H]^+$.

**Preparation of compound 451-8**

Compound **451-7** (280 mg, 0.600 mmol), copper(I) iodide (12 mg, 0.060 mmol), and copper(I) cyanide (112 mg, 1.26 mmol) were dissolved in N,N-dimethylformamide (5 mL). After the addition was completed, the reaction system was stirred at 125°C for 2 h. After the reaction was completed, insoluble solids were removed by filtration. Water (30 mL) was added, and the mixture was extracted three times with ethyl acetate (30 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 75%) to obtain 160 mg of the title compound **451-8.** LC-MS (ESI): m/z 365.0 $[M+H]^+$.

**Preparation of compound 451-9**

Compound **451-8** (160 mg, 0.438 mmol), p-toluenesulfonic acid monohydrate (377 mg, 2.19 mmol), and lithium chloride (93 mg, 2.19 mmol) were dissolved in *N,N*-dimethylformamide (5 mL). After the addition was completed, the

reaction system was stirred at 125°C for 2 h. After the reaction was completed, insoluble solids were removed by filtration. Water (30 mL) was added, and the mixture was extracted three times with ethyl acetate (30 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 75%) to obtain 140 mg of the title compound **451-9.** LC-MS (ESI): m/z 351.2 $[M+H]^+$.

**Preparation of compound 451-11**

Compound **451-10** (3.2 g, crude) was dissolved in 1,4-dioxane (100 mL). **451-2** (1.16 g, 4.58 mmol), potassium carbonate (948 mg, 6.87 mmol), and Pd(dppf)$Cl_2$ (457 mg, 0.5 mmol) were added. The reaction system was purged three times with nitrogen and then stirred at 100°C for 5 h. After the reaction was monitored by LCMS until completion, the reaction mixture was concentrated and purified by silica gel column chromatography (PE/EA = 100% to 50%) to obtain 1.2 g of the title compound **451-11.** LC-MS (ESI): m/z 567.2 $[M+H]^+$.

**Preparation of compound 451-12**

Compound **451-11** (1.2 g, 2.12 mmol) was dissolved in THF (50 mL) and water (50 mL). Potassium peroxymonosulfate triple salt (3.91 g, 6.36 mmol) was added, and the reaction system was stirred at 20°C for 1 h. After the reaction was monitored by LCMS until completion, the reaction mixture was poured into water (300 mL) and extracted with dichloromethane (100 mL). The phases were separated, and the organic phase was concentrated to obtain 920 mg of the title compound **451-12.**

**Preparation of compound 451**

Compound **451-12** (200 mg, 0.33 mmol) was dissolved in acetonitrile (10 mL). Cesium carbonate (160 mg, 0.49 mmol) and dimethylphosphine oxide (77 mg, 1.00 mmol) were added, and the reaction system was stirred at 70°C for 2 h. After the reaction was monitored by LCMS until completion, the reaction mixture was filtered. The crude filtrate was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 8.2 mg of the title compound **451.** LC-MS (ESI): m/z 597.2 $[M+H]^+$. $^1H$ NMR (400 MHz, DMSO-$d_6$) δ 9.25 (s, 2H), 8.95 (d, *J* = 5.2 Hz, 1H), 8.60 (d, *J* = 2.5 Hz, 1H), 7.97 (d, *J* = 8.3 Hz, 1H), 7.79 (dd, *J* = 8.4, 2.5 Hz, 1H), 7.76 (d, *J* = 2.4 Hz, 1H), 7.72 (d, *J* = 2.3 Hz, 1H), 7.64 (dd, *J* = 5.2, 3.1 Hz, 1H), 5.66 (s, 2H), 4.43 (t, *J* = 5.2 Hz, 2H), 3.96 (t, *J* = 5.2 Hz, 2H), 1.73 (s, 6H), 1.71 (d, *J* = 13.8 Hz, 6H). $^{31}P$ NMR (162 MHz, DMSO-$d_6$) δ 38.27 (s, 1P).

**Example 452: Preparation of compound 452**

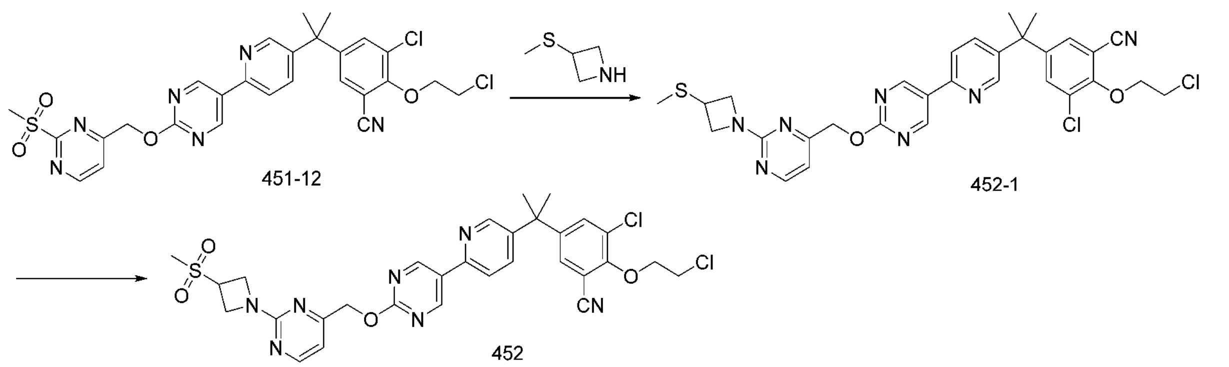

**Preparation of compound 452-1**

Compound **451-12** (200 mg, 0.33 mmol) was dissolved in acetonitrile (10 mL). Cesium carbonate (160 mg, 0.49 mmol) and 3-(methylthio)azetidine (51 mg, 0.49 mmol) were added. The reaction system was purged three times with nitrogen and stirred at 70°C for 5 h. The reaction mixture was filtered, and the filtrate was concentrated to obtain 230 mg of the title compound **452-1.**

**Preparation of compound 452**

Compound **452-1** (230 mg, 0.37 mmol) was dissolved in THF (10 mL) and water (10 mL). Potassium peroxymonosulfate triple salt (683 mg, 1.11 mmol) was added, and the reaction system was stirred at 20°C for 1 h. After the reaction was monitored by LCMS until completion, the reaction mixture was poured into water (300 mL) and extracted with dichloromethane (100 mL). The phases were separated, and the organic phase was concentrated. The resulting crude product was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 16.5 mg of the title compound **452.** LC-MS (ESI): m/z 654.2 $[M+H]^+$. $^1$H NMR (400 MHz, DMSO-$d_6$) δ 9.24 (s, 2H), 8.60 (d, *J* = 2.4 Hz, 1H), 8.38 (d, *J* = 5.1 Hz, 1H), 7.97 (d, *J* = 8.4 Hz, 1H), 7.79 (dd, *J* = 8.4, 2.5 Hz, 1H), 7.76 (d, *J* = 2.4 Hz, 1H), 7.72 (d, *J* = 2.3 Hz, 1H), 6.74 (d, *J* = 5.0 Hz, 1H), 5.40 (s, 2H), 4.43 (t, *J* = 5.2 Hz, 2H), 4.41 - 4.36 (m, 1H), 4.31 (t, *J* = 8.8 Hz, 2H), 4.23 - 4.17 (m, 2H), 3.96 (t, *J* = 5.2 Hz, 2H), 3.05 (s, 3H), 1.73 (s, 6H).

**Example 453: Preparation of compound 453**

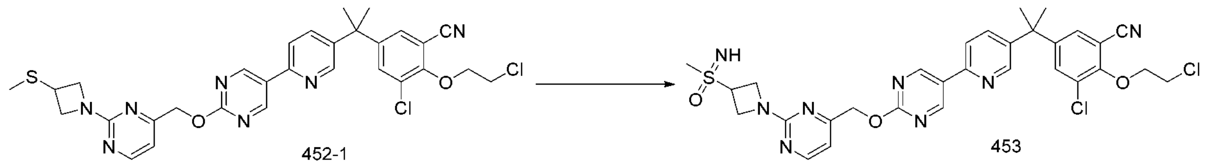

452-1

453

**Preparation of compound 453**

Compound **452-1** (100 mg, 0.16 mmol) was dissolved in methanol (10 mL). (Diacetoxyiodo)benzene (155 mg, 0.48 mmol) and ammonium carbonate (77 mg, 0.80 mmol) were added, and the reaction system was stirred at 20°C for 1 h. After the reaction was monitored by LCMS until completion, the reaction mixture was filtered. The filtrate was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 17.6 mg of the title compound **453.** LC-MS (ESI): m/z 653.2 $[M+H]^+$. $^1$H NMR (400 MHz, DMSO-$d_6$) δ 9.24 (s, 2H), 8.60 (d, *J* = 2.5 Hz, 1H), 8.36 (d, *J* = 5.1 Hz, 1H), 7.97 (d, *J* = 8.4 Hz, 1H), 7.79 (dd, *J* = 8.4, 2.5 Hz, 1H), 7.76 (d, *J* = 2.4 Hz, 1H), 7.72 (d, *J* = 2.4 Hz, 1H), 6.71 (d, *J* = 5.1 Hz, 1H), 5.39 (s, 2H), 4.43 (t, *J* = 5.2 Hz, 2H), 4.38 - 4.31 (m, 1H), 4.30 - 4.22 (m, 4H), 4.01 - 3.92 (m, 3H), 2.90 (s, 3H), 1.73 (s, 6H).

**Example 454: Preparation of compound 454**

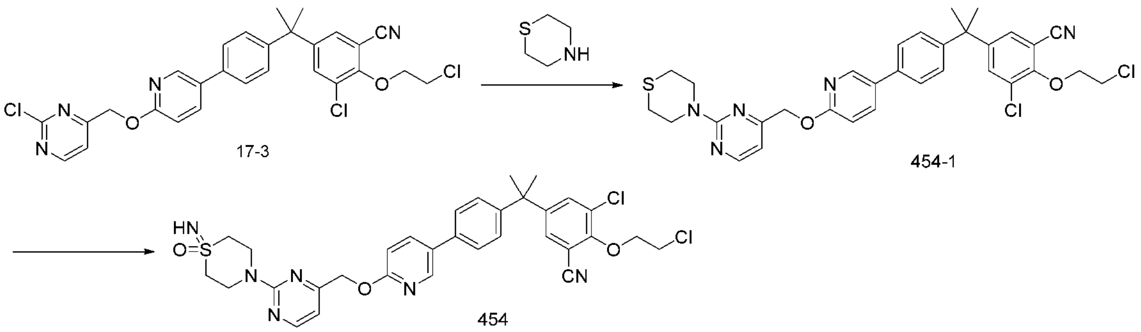

17-3

454-1

454

**Preparation of compound 454-1**

Compound **17-3** (25 mg, 0.045 mmol) was dissolved in anhydrous ethanol (10 mL). DIEA (13 mg, 0.1 mmol) and thiomorpholine (10 mg, 0.10 mmol) were added, and the reaction system was stirred at 80°C for 12 h. After the reaction was completed, insoluble solids were removed by filtration. Water (100 mL) was added to the filtrate, and the mixture was

extracted three times with dichloromethane (100 mL). The organic phases were combined, washed twice with saturated brine (20 mL), dried over anhydrous sodium sulfate, subjected to suction filtration, and the filtrate was concentrated to obtain 35 mg of a crude product of the title compound **454-1.** The product was used directly in the next step without purification.

**Preparation of compound 454**

Compound **454-1** (35 mg, 0.056 mmol) was dissolved in MeOH (8 mL), and (diacetoxyiodo)benzene (54 mg, 0.16 mmol) and ammonium carbonate (27 mg, 0.28 mmol) were added. The reaction was carried out at room temperature for 5 h. After the reaction was monitored by LCMS until completion, the reaction mixture was filtered. The crude filtrate was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 9.5 mg of the title compound **454.** LC-MS (ESI): m/z 651.4 $[M+H]^+$. $^1H$ NMR (400 MHz, DMSO-$d_6$) δ 8.42 (d, *J* = 2.6 Hz, 1H), 8.39 (d, *J* = 5.0 Hz, 1H), 8.07 (dd, *J* = 8.6, 2.6 Hz, 1H), 7.71 (d, *J* = 2.3 Hz, 1H), 7.65 (d, *J* = 2.4 Hz, 1H), 7.60 (d, *J* = 8.3 Hz, 2H), 7.34 (d, *J* = 8.3 Hz, 2H), 7.07 (d, *J* = 8.6 Hz, 1H), 6.72 (d, *J* = 5.0 Hz, 1H), 5.35 (s, 2H), 4.42 (t, *J* = 5.2 Hz, 2H), 4.40 - 4.31 (m, 2H), 3.96 (t, *J* = 5.2 Hz, 2H), 3.94 - 3.85 (m, 2H), 3.82 (s, 1H), 3.06 - 2.91 (m, 4H), 1.69 (s, 6H).

**Example 455: Preparation of compound 455**

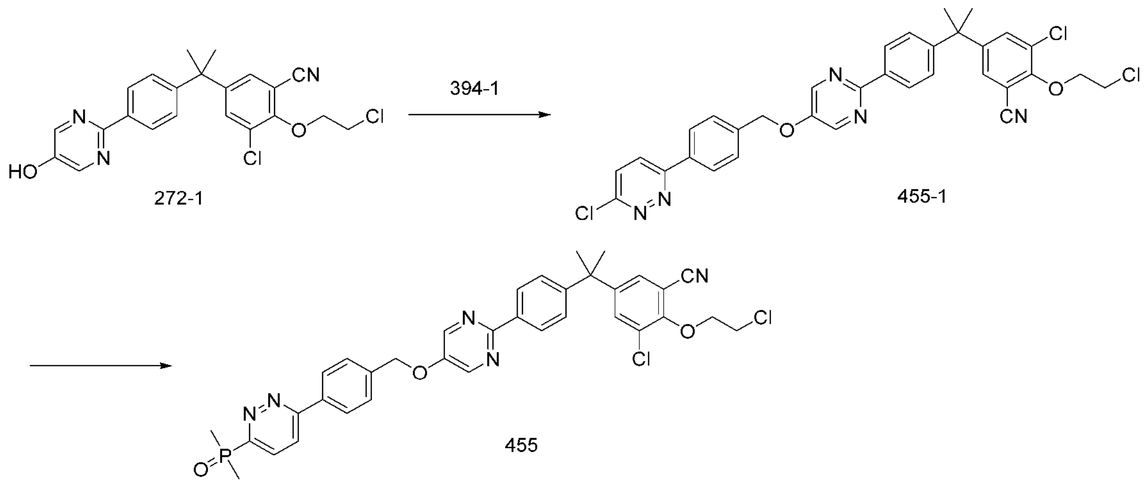


272-1

455-1

455

**Preparation of compound 455-1**

Compound **272-1** (145 mg, 338.54 μmol) and compound **394-1** (81.3 mg, 340.02 μmol) were dissolved in DMF (3 mL), and cesium carbonate (220.6 mg, 677.08 μmol) was added. The reaction mixture was stirred at 70°C for 7 h. After the reaction was completed, the reaction mixture was cooled to room temperature. Saturated aqueous ammonium chloride solution and water were added, and the mixture was extracted with ethyl acetate. The organic phase was washed with saturated brine, dried over anhydrous sodium sulfate, filtered, and the filtrate was concentrated. The residue was purified by silica gel column chromatography (DCM/MeOH = 24/1) to obtain 105.2 mg of the title compound **455-1.** LC-MS (ESI): m/z 630.2 $[M+H]^+$.

**Preparation of compound 455**

Compound **455-1** (105.2 mg, 166.73 μmol) was dissolved in DMF (3 mL). Xantphos (19.3 mg, 33.35 μmol), $Pd_2(dba)_3$ (15.3 mg, 16.67 μmol), dimethylphosphine oxide (39.0 mg, 500.20 μmol), and DIPEA (107.8 mg, 833.66 μmol, 145 μL) were added. The mixture was immediately bubbled with nitrogen for 1 min and stirred under microwave irradiation at 120°C for 2 h in a sealed tube. After the reaction was completed, the reaction mixture was diluted with a small amount of 1,4-dioxane, filtered, and then purified by preparative chromatography (preparative method: mobile phase: A: 0.1% formic acid in water; B: acetonitrile; chromatographic column: Welch Ultimate AQ-C18 250 × 21.2 mm; column temperature: 25°C; gradient: B%: 60-80% 8 min, 80-95% 4 min; flow rate: 30 mL/min) to obtain 67.5 mg of the title compound **455.** LC-MS (ESI): m/z 672.0 [M+H]+. $^1$H NMR (400 MHz, Chloroform-*d*) δ 8.58 (s, 2H), 8.30 (d, *J* = 8.6 Hz, 2H), 8.21 (d, *J* = 8.3 Hz, 2H), 8.05 (dd, *J* = 8.6, 2.6 Hz, 1H), 7.66 (d, *J* = 8.3 Hz, 2H), 7.46 - 7.40 (m, 2H), 7.37 (d, *J* = 2.3 Hz, 1H), 7.29 (d, *J* = 8.5 Hz, 2H), 5.32 (s, 2H), 4.42 (t, *J* = 6.2 Hz, 2H), 3.87 (t, *J* = 6.2 Hz, 2H), 1.95 (d, *J* = 13.7 Hz, 6H), 1.70 (s, 6H). $^{31}$P NMR (162 MHz, Chloroform-d) δ 36.51 (s, 1P).

**Example 456: Preparation of compound 456**

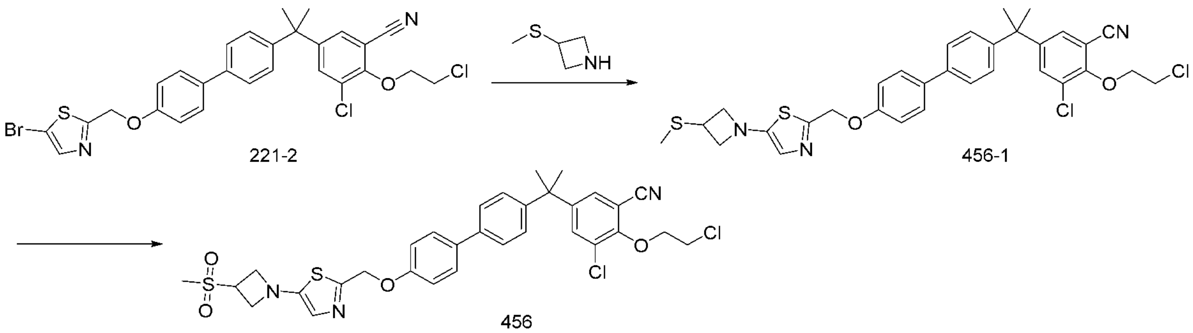
221-2

456-1

456

**Preparation of compound 456-1**

Compound **221-2** (230 mg, 381.83 μmol) and compound 3-(methylthio)azetidine (47.3 mg, 458.19 μmol) were dissolved in 1,4-dioxane (3 mL). $Pd_2(dba)_3$ (69.9 mg, 76.37 μmol), Xantphos (88.4 mg, 152.73 μmol), and $Cs_2CO_3$ (248.8 mg, 763.65 μmol) were added. The reaction mixture was purged three times with nitrogen and then heated under microwave irradiation at 110°C for 4 h. LCMS indicated the formation of the product. The reaction mixture was subjected to rotary evaporation until dryness. The resulting residue was purified by silica gel column chromatography (EA/PE = 0 to 45%) to obtain 90 mg of the title compound **456-1.** LC-MS (ESI): m/z 624.23 [M+H]+.

**Preparation of compound 456**

Compound **456-1** (90 mg, 144.08 μmol) was dissolved in THF (3 mL) and $H_2O$ (3 mL), and oxone (442.33 mg, 720.41 μmol) was added. The reaction mixture was stirred at room temperature for 2 h. LCMS indicated the disappearance of the starting material and the formation of the product. After the reaction mixture was filtered, the filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 1.02 mg of the title compound **456.** LC-MS (ESI): m/z 656.2 [M+H]+.

$^1$H NMR (400 MHz, DMSO-$d_6$) δ 7.83 - 7.78 (m, 1H), 7.71 (d, *J* = 2.3 Hz, 1H), 7.64 (d, *J* = 2.3 Hz, 1H), 7.62 - 7.53 (m, 3H), 7.50 - 7.45 (m, 1H), 7.33 - 7.29 (m, 2H), 7.04 - 6.97 (m, 2H), 4.99 (s, 2H), 4.79 (dd, *J* = 11.4, 7.9 Hz, 1H), 4.63 (dd, *J* = 11.4, 4.8 Hz, 1H), 4.42 (t, *J* = 5.2 Hz, 2H), 4.40 - 4.29 (m, 2H), 4.20 (dd, *J* = 11.4, 4.9 Hz, 1H), 3.96 (t, *J* = 5.2 Hz, 2H), 3.08 (s, 3H), 1.68 (s, 6H).

**Example 457: Preparation of compound 457**

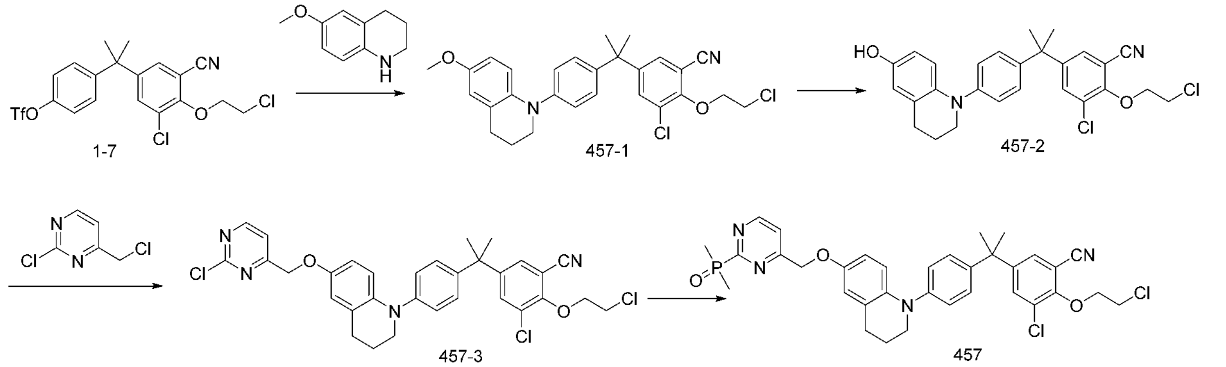

1-7 457-1 457-2 457-3 457

**Preparation of compound 457-1**

Compound **1-7** (827.4 mg, 1.72 mmol) and 6-methoxy-1,2,3,4-tetrahydroisoquinoline (200 mg, 1.23 mmol) were dissolved in 1,4-dioxane (10 mL). $Pd_2(dba)_3$ (112.2 mg, 122.54 μmol), XPhos (233.7 mg, 490.15 μmol), and $Cs_2CO_3$ (598.9 mg, 1.84 mmol) were added. The reaction mixture was purged three times with nitrogen and then heated under microwave irradiation at 100°C for 2 h. LCMS indicated the formation of the product. The reaction mixture was subjected to rotary evaporation until dryness and purified by silica gel column chromatography (EA/PE = 0 to 20%) to obtain 500 mg of the title compound **457-1.** LC-MS (ESI): m/z 495.2 $[M+H]^+$.

**Preparation of compound 457-2**

Compound **457-1** (250 mg, 504.60 μmol) was dissolved in DCM (5 mL), and $BBr_3$ (379.2 mg, 1.51 mmol) was added dropwise at 0°C. The reaction mixture was stirred at 0°C for 1 h. LCMS indicated the disappearance of the starting material and the formation of the product. Methanol (5 mL) was slowly added dropwise to the reaction mixture to quench the reaction. The organic phase was washed twice with saturated $NaHCO_3$ solution (15 mL). The organic phases were combined, dried over anhydrous $Na_2SO_4$, subjected to suction filtration, and the filtrate was subjected to rotary evaporation until dryness. The resulting residue was purified by silica gel column chromatography (EA/PE = 0 to 45%) to obtain 110 mg of the title compound **457-2.** LC-MS (ESI): m/z 481.2 $[M+H]^+$.

**Preparation of compound 457-3**

Compound **457-2** (110 mg, 228.49 μmol) and 2-chloro-4-(chloromethyl)pyrimidine (41 mg, 251.34 μmol) were dissolved in acetonitrile (5 mL), and potassium carbonate (94.7 mg, 685.48 μmol) was added. The reaction mixture was heated at 70°C for 5 h. LCMS indicated the disappearance of the starting material and the formation of the product. The reaction mixture was subjected to rotary evaporation until dryness and then purified by silica gel column chromatography (EA/PE = 0 to 40%) to obtain 90 mg of the title compound **457-3.** LC-MS (ESI): m/z 607.2 $[M+H]^+$.

**Preparation of compound 457**

Compound **457-3** (80 mg, 131.59 μmol) and dimethylphosphine oxide (51.4 mg, 657.94 μmol) were weighed and dissolved in 1,4-dioxane (1 mL). $Pd_2(dba)_3$ (12.1 mg, 13.16 μmol), Xantphos (15.2 mg, 26.32 μmol), and DIPEA (51 mg, 394.77 μmol, 69 μL) were added. The system was purged three times with nitrogen. The reaction mixture was heated under microwave irradiation at 120°C for 2 h. LCMS indicated the disappearance of the starting material and the formation of the product. The reaction mixture was subjected to rotary evaporation until dryness, filtered, and then subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 50% to 70% acetonitrile gradient elution over 12 min; flow rate: 30 mL/min) to obtain 10.39 mg of the title compound **457.** LC-MS (ESI): m/z 667.2 $[M+H+H_2O]^+$.

**Example 458: Preparation of compounds 458, 458A, and 458B**

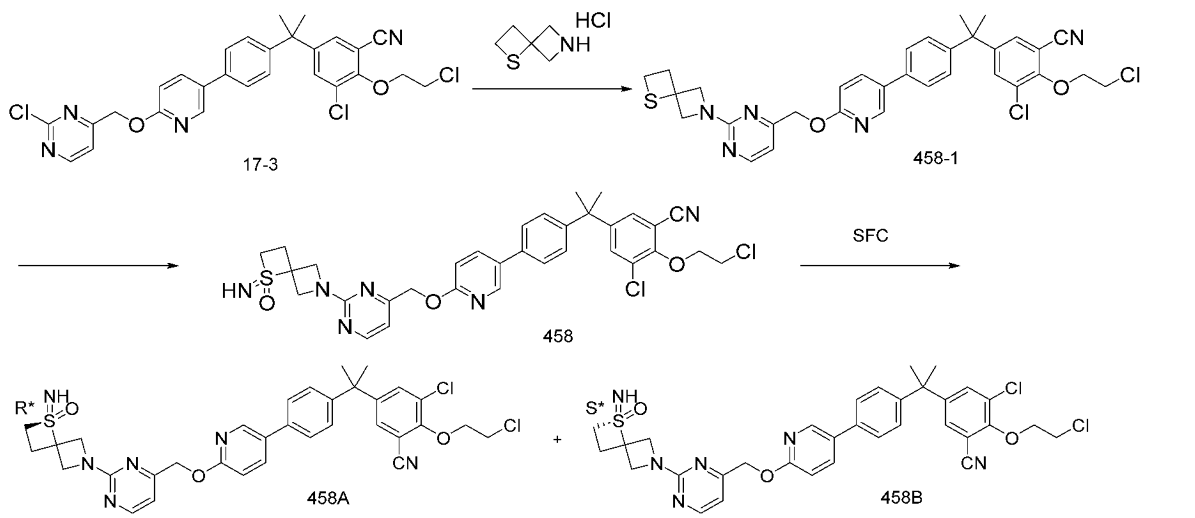


17-3, 458-1, 458, 458A + 458B

**Preparation of compound 458-1**

Compound **17-3** (80 mg, 0.14 mmol) was added to a microwave tube, followed by the sequential addition of anhydrous ethanol (2 mL), 1-thia-6-azaspiro[3.3]heptane (33 mg, 0.22 mmol), and DIPEA (56 mg, 0.43 mmol). The system was purged three times with nitrogen, and the reaction was carried out under microwave irradiation at 90°C for 2 h. After the reaction was monitored by LCMS until completion, the reaction mixture was filtered. The crude filtrate was purified by normal-phase silica gel column chromatography (EA/PE = 0 to 60%) to obtain 80 mg of the title compound **458-1** in 88% yield. LC-MS (ESI): m/z 632.2 $[M+H]^+$.

**Preparation of compound 458**

Compound **458-1** (80 mg, 0.13 mmol) and ammonium carbonate (36 mg, 0.38 mmol) were dissolved in methanol (5 mL). (Diacetoxyiodo)benzene (204 mg, 0.63 mmol) was added thereto at 0°C. After the addition was completed, the reaction was carried out at room temperature overnight. After the reaction was monitored the next day by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 30 mg of the title compound **458.** LC-MS (ESI): m/z 663.2 $[M+H]^+$. $^1$H NMR (400 MHz, DMSO-$d_6$) δ 8.43 (d, *J* = 2.5 Hz, 1H), 8.35 (d, *J* = 5.1 Hz, 1H), 8.07 (dd, *J* = 8.6, 2.5 Hz, 1H), 7.71 (d, *J* = 2.3 Hz, 1H), 7.65 (d, *J* = 2.4 Hz, 1H), 7.60 (d, *J* = 8.5 Hz, 2H), 7.33 (d, *J* = 8.5 Hz, 2H), 7.07 (d, *J* = 8.6 Hz, 1H), 6.72 (d, *J* = 5.0 Hz, 1H), 5.32 (s, 2H), 4.80 (d, *J* = 1.4 Hz, 1H), 4.50 (dd, *J* = 10.3, 1.4 Hz, 1H), 4.42 (t, *J* = 5.3 Hz, 2H), 4.40 - 4.34 (m, 1H), 4.24 (d, *J* = 10.3 Hz, 1H), 4.18 (d, *J* = 10.3 Hz, 1H), 3.96 (t, *J* = 5.3 Hz, 2H), 3.93 - 3.79 (m, 2H), 2.45 - 2.32 (m, 2H), 1.68 (s, 6H).

**Preparation of compounds 458A and 458B**

Compound **458** (22 mg) was purified by preparative SFC (preparative conditions: instrument model: Waters SFC15; column: ChiralPak AD-H, 250 * 20 mm I.D., 5 μm; mobile phase: A: $CO_2$, B: isopropanol (30% acetonitrile); elution gradient: B 55%; flow rate: 15 mL/min; column pressure: 100 bar; column temperature: 40°C; detection wavelength: 214 nm; cycle time: 22 min.) to obtain 7.87 mg of the title compound **458B** and 7.54 mg of the title compound **458A.**

Compound **458B:** SFC analysis method (instrument model: Waters SFC15; column model: ChiralPak AD-H, 250 * 4.6 mm I.D., 5 um; mobile phase: A: $CO_2$, B: isopropanol (30% acetonitrile); elution gradient: B 50%; flow rate: 3 mL/min; column pressure: 100 bar; column temperature: 40°C; detection wavelength: 214 nm; Rt = 6.57 min). LC-MS (ESI): m/z 663.4 $[M+H]^+$. $^1$H NMR (400 MHz, Chloroform-d) δ 8.34 (t, *J* = 2.5 Hz, 1H), 8.32 (d, *J* = 3.6 Hz, 1H), 7.84 (dt, *J* = 8.5, 2.3 Hz, 1H), 7.54 - 7.41 (m, 3H), 7.36 (t, *J* = 2.2 Hz, 1H), 7.26 - 7.18 (m, 2H), 6.95 (d, *J* = 8.5 Hz, 1H), 6.78 (d, *J* = 4.2 Hz, 1H), 5.37 (s, 2H), 4.74 (dd, *J* = 20.6, 10.5 Hz, 2H), 4.49 - 4.36 (m, 2H), 4.25 (t, *J* = 11.2 Hz, 2H), 4.10 - 3.95 (m, 2H), 3.94 - 3.81 (m, 2H), 3.10 (s, 1H), 2.49 (t, *J* = 8.8 Hz, 2H), 1.70 (s, 6H).

Compound **458A:** SFC analysis method (instrument model: Waters SFC15; column model: ChiralPak AD-H, 250 * 4.6 mm I.D., 5 um; mobile phase: A: $CO_2$, B: isopropanol (30% acetonitrile); elution gradient: B 50%; flow rate: 3 mL/min; column pressure: 100 bar; column temperature: 40°C; detection wavelength: 214 nm; Rt = 5.47 min). LC-MS (ESI): m/z 663.4 $[M+H]^+$. $^1$H NMR (400 MHz, Chloroform-*d*) δ 8.34 (d, *J* = 2.5 Hz, 1H), 8.32 (d, *J* = 5.1 Hz, 1H), 7.84 (d, *J* = 8.4 Hz, 1H),

7.52 - 7.42 (m, 3H), 7.36 (s, 1H), 7.28 - 7.18 (m, 2H), 6.95 (d, *J* = 8.5 Hz, 1H), 6.77 (d, *J* = 5.0 Hz, 1H), 5.37 (s, 2H), 4.74 (dd, *J* = 20.6, 10.5 Hz, 2H), 4.49 - 4.37 (m, 2H), 4.25 (t, *J* = 11.2 Hz, 2H), 4.09 - 3.94 (m, 2H), 3.94 - 3.80 (m, 2H), 3.10 (s, 1H), 2.49 (t, *J* = 8.6 Hz, 2H), 1.69 (s, 6H).

**Example 459: Preparation of compound 459**

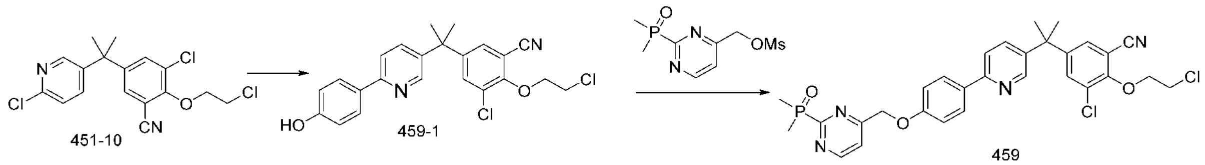

**Preparation of compound 459-1**

Compound **451-10** (180 mg, 486.92 μmol) and 4-hydroxyphenylboronic acid (73.9 mg, 535.61 μmol) were weighed and dissolved in 1,4-dioxane (5 mL) and water (1.5 mL). Pd(dppf)$Cl_2$ (31.4 mg, 48.69 μmol) and $K_2CO_3$ (168.2 mg, 1.22 mmol) were added. The system was purged three times with nitrogen, and then the reaction mixture was stirred at 100°C for 16 h. LCMS indicated the disappearance of the starting material and the formation of the product. The reaction mixture was subjected to rotary evaporation until dryness and then purified by silica gel column chromatography (EA/PE = 0 to 50%) to obtain 100 mg of the title compound **459-1.** LC-MS (ESI): m/z 427.2 $[M+H]^+$.

**Preparation of compound 459**

Compound **459-1** (100 mg, 234.02 μmol) and (2-(dimethylphosphoryl)pyrimidin-4-yl)methyl methanesulfonate (80.4 mg, 304.22 μmol) were dissolved in $CH_3CN$ (3 mL). $K_2CO_3$ (97 mg, 702.05 μmol) was added. The reaction mixture was stirred at 80°C for 12 h. LCMS indicated the disappearance of the starting material and the formation of the product. After the reaction was completed, insoluble solids were removed by filtration. Water (10 mL) was added to the filtrate, and the mixture was extracted three times with dichloromethane (20 mL). The organic phases were combined, washed twice with saturated brine (20 mL), dried over anhydrous sodium sulfate, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 21.60 mg of the title compound **459.** LC-MS (ESI): m/z 595.2 $[M+H]^+$. $^1$H NMR (400 MHz, DMSO-$d_6$) δ 9.00 (d, *J* = 5.2 Hz, 1H), 8.52 (d, *J* = 2.5 Hz, 1H), 8.04 (d, *J* = 8.5 Hz, 2H), 7.84 (d, *J* = 8.5 Hz, 1H), 7.78 - 7.73 (m, 2H), 7.73 - 7.64 (m, 2H), 7.17 (d, *J* = 8.6 Hz, 2H), 5.37 (s, 2H), 4.42 (t, *J* = 5.1 Hz, 2H), 3.96 (t, *J* = 5.1 Hz, 2H), 1.77 (d, *J* = 13.7 Hz, 6H), 1.72 (s, 6H). $^{31}$P NMR (162 MHz, DMSO-*d6*) δ 34.29 (s, 1P).

**Example 460: Preparation of compound 460**

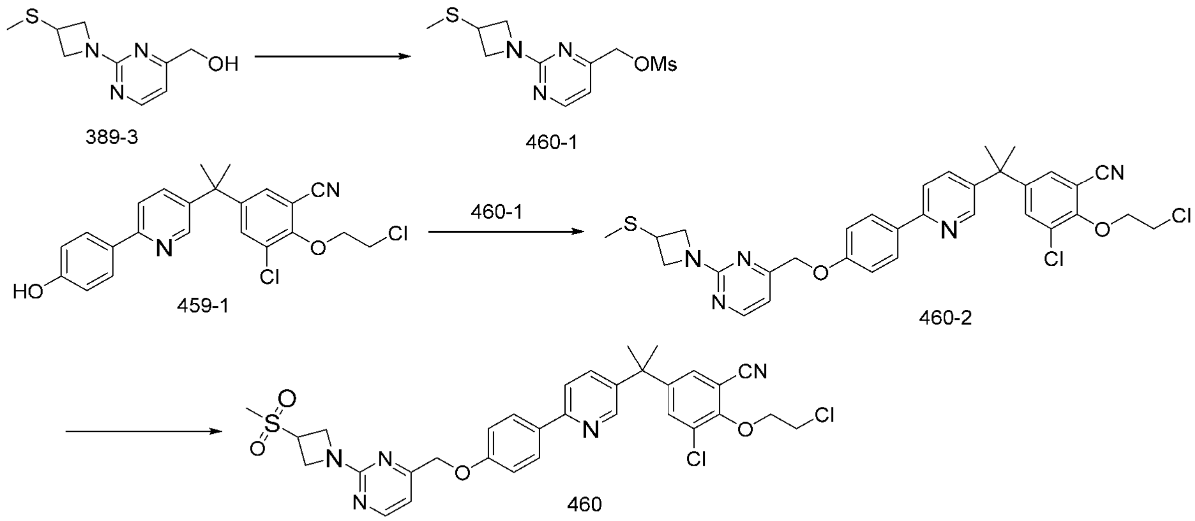


389-3

460-1

459-1

460-2

460

**Preparation of compound 460-1**

Compound **389-3** (10 g, 47.33 mmol) was dissolved in DCM (100 mL), and TEA (5.75 g, 56.80 mmol, 7.9 mL) was added. The reaction mixture was cooled to 0°C, and methanesulfonyl chloride (6.51 g, 56.80 mmol, 4.40 mL) was added dropwise. The reaction mixture was stirred at room temperature for 1 h. LCMS indicated the disappearance of the starting material and the formation of the product. The reaction mixture was poured into water and extracted three times with DCM. The organic phases were combined, washed three times with brine, dried, filtered, and subjected to rotary evaporation until dryness. The resulting residue was used directly in the next step without purification. LC-MS (ESI): m/z 290.0 $[M+H]^+$.

**Preparation of compound 460-2**

Compound **459-1** (90 mg, 210.61 μmol) and compound **460-1** (79.2 mg, 273.80 μmol) were dissolved in acetonitrile (5 mL), and potassium carbonate (58.2 mg, 421.23 μmol) was added. The reaction mixture was reacted at 80°C for 6 h. LCMS indicated the disappearance of the starting material and the formation of the product. The reaction mixture was poured into water (100 mL) and extracted three times with ethyl acetate (20 mL). The organic phases were combined, washed three times with saturated brine (20 mL), dried over anhydrous sodium sulfate, filtered, and subjected to rotary evaporation until dryness. The resulting residue was purified by silica gel column chromatography (EA/PE = 0 to 60%) to obtain 100 mg of the title compound **460-2.** LC-MS (ESI): m/z 620.4 $[M+H]^+$.

**Preparation of compound 460**

Compound **460-2** (35 mg, 56.40 μmol) was dissolved in tetrahydrofuran (2 mL) and water (0.5 mL). Potassium peroxymonosulfate (172.3 mg, 281.99 μmol) was added, and the reaction mixture was stirred at 25°C for 2 h. LCMS indicated the disappearance of the starting material and the formation of the product. The reaction mixture was poured into saturated sodium sulfite solution and extracted three times with dichloromethane. The organic phases were combined, washed three times with saturated brine, dried over anhydrous sodium sulfate, subjected to suction filtration, and the filtrate was subjected to rotary evaporation until dryness. The resulting residue was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 3.46 mg of the title compound 460. LC-MS (ESI): m/z 652.2 $[M+H]^+$. $^1H$ NMR (400 MHz, DMSO-$d_6$) δ 8.52 (d, *J* = 2.4 Hz, 1H), 8.42 (d, *J* = 5.0 Hz, 1H), 8.07 - 7.99 (m, 2H), 7.84 (d, *J* = 8.4 Hz, 1H), 7.76 (d, *J* = 2.4 Hz, 1H), 7.74 - 7.66 (m, 2H), 7.14 - 7.06 (m, 2H), 6.87 (d, *J* = 5.0 Hz, 1H), 5.11 (s, 2H), 4.47 - 4.36 (m, 3H), 4.34 (t, *J* = 8.7 Hz, 2H), 4.27 - 4.19 (m, 2H), 3.96 (t, *J*= 5.2 Hz, 2H), 3.07 (s, 3H), 1.72 (s, 6H).

**Example 461: Preparation of compound 461**

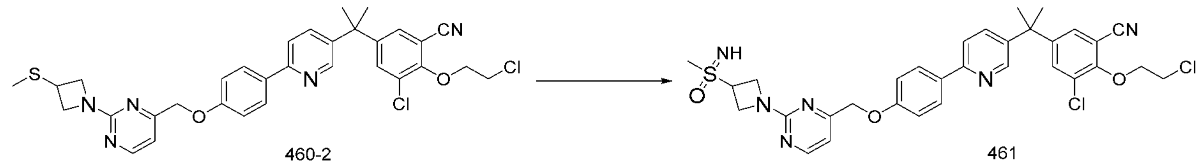

460-2 461

**Preparation of compound 461**

Compound **460-2** (35 mg, 56.40 μmol) was dissolved in tetrahydrofuran (1 mL) and methanol (2 mL). (Diacetoxyiodo)benzene (90.8 mg, 281.99 μmol) and ammonium carbonate (27.1 mg, 281.99 μmol) were added, and the reaction mixture was stirred at 25°C for 2 h. LCMS indicated the disappearance of the starting material and the formation of the product. The reaction mixture was poured into saturated sodium sulfite solution and extracted three times with dichloromethane. The organic phases were combined, washed three times with saturated brine, dried over anhydrous sodium sulfate, subjected to suction filtration, and the filtrate was subjected to rotary evaporation until dryness. The resulting residue was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 5.63 mg of the title compound **461.** LC-MS (ESI): m/z 651.2 $[M+H]^+$. $^1H$ NMR (400 MHz, DMSO-$d_6$) δ 8.52 (d, *J* = 2.4 Hz, 1H), 8.40 (d, *J* = 5.0 Hz, 1H), 8.05 - 8.00 (m, 2H), 7.83 (d, *J* = 8.5 Hz, 1H), 7.76 (d, *J* = 2.4 Hz, 1H), 7.71 (d, *J* = 2.3 Hz, 1H), 7.69 (dd, *J* = 8.4, 2.5 Hz, 1H), 7.14 - 7.08 (m, 2H), 6.83 (d, *J* = 5.0 Hz, 1H), 5.09 (s, 2H), 4.43 (t, *J* = 5.2 Hz, 2H), 4.34 - 4.19 (m, 5H), 3.96 (t, *J* = 5.2 Hz, 3H), 2.91 (s, 3H), 1.72 (s, 6H).

**Example 462: Preparation of compound 462**

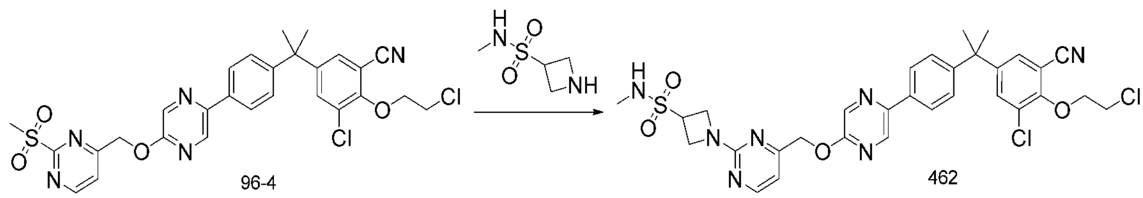

96-4 462

**Preparation of compound 462**

Compound **96-4** (80 mg, 0.13 mmol) was added to a microwave tube, followed by the sequential addition of anhydrous ethanol (2 mL), *N*-methylazetidine-3-methanesulfonamide (40 mg, 0.27 mmol), and DIPEA (52 mg, 0.40 mmol). The system was purged three times with nitrogen, and the reaction was carried out under microwave irradiation at 90°C for 2 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 50 mg of the title compound **462.** LC-MS (ESI): m/z 668.7 $[M+H]^+$. $^1H$ NMR (400 MHz, DMSO-$d_6$) δ 8.75 (s, 1H), 8.54 (s, 1H), 8.37 (dd, *J* = 5.1, 1.5 Hz, 1H), 7.96 (d, *J* = 7.4 Hz, 2H), 7.70 (t, *J* = 1.8 Hz, 1H), 7.63 (t, *J* = 1.9 Hz, 1H), 7.37 (d, *J* = 7.5 Hz, 2H), 7.27 (q, *J* = 4.8 Hz, 1H), 6.78 (d, *J* = 5.0 Hz, 1H), 5.37 (s, 2H), 4.42 (t, *J* = 5.2 Hz, 2H), 4.39 - 4.32 (m, 1H), 4.29 (t, *J* = 8.6 Hz, 2H), 4.19 - 4.09 (m, 2H), 3.96 (t, *J* = 5.2 Hz, 2H), 2.61 (d, *J* = 4.8 Hz, 3H), 1.69 (s, 6H).

**Example 463: Preparation of compound 463**

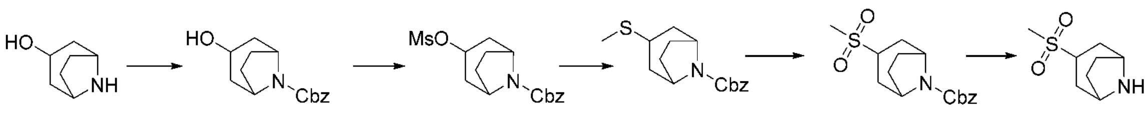

463-1 463-2 463-3 463-4 463-5 463-6

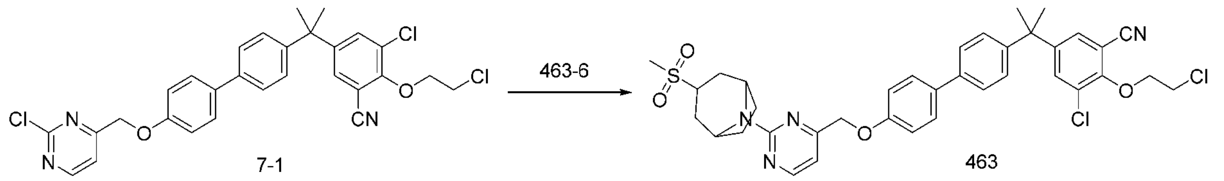


**Preparation of compound 463-2**

Compound **463-1** (2.0 g, 15.7 mmol) was dissolved in THF (50 mL) and water (20 mL), followed by the addition of sodium carbonate (2.5 g, 23.5 mmol). The system was purged three times with nitrogen, then cooled to 0°C, and benzyl chloroformate (3.2 g, 18.8 mmol) was added. The reaction system was stirred at 25°C for 5 h. The reaction mixture was filtered, and the filtrate was concentrated. The resulting residue was subjected to silica gel column chromatography (PE/EA = 100% to 50%) to obtain 1.8 g of the title compound **463-2.**

**Preparation of compound 463-3**

Compound **463-2** (800 mg, 3.06 mmol) was dissolved in DCM (10 mL) and cooled to 0°C. Triethylamine (0.45 g, 4.50 mmol) was added, followed by the addition of MsCl (418 mg, 3.67 mmol). The reaction was carried out at room temperature for 2 h. The reaction mixture was poured into water (20 mL) and extracted with DCM (100 mL). The phases were separated, and the organic phase was concentrated to obtain 780 mg of a crude product of the title compound **463-3.**

**Preparation of compound 463-4**

Compound **463-3** (780 mg, crude) was dissolved in DMF (10 mL). Sodium methanethiolate (5 mL, 20% aqueous solution) was added, and the reaction was carried out at 50°C for 12 h. After the reaction was completed, the reaction mixture was poured into water (200 mL) and extracted with DCM (100 mL). The phases were separated, and the organic phase was concentrated. The residue was purified by silica gel column chromatography (PE/EA = 100% to 30%) to obtain 430 mg of the title compound **463-4.** LC-MS (ESI): m/z 292.1 $[M+H]^+$.

**Preparation of compound 463-5**

Compound **463-4** (430 mg, 1.47 mmol) was dissolved in THF (20 mL) and water (10 mL). Potassium peroxymonosulfate triple salt (2.72 g, 4.43 mmol) was added, and the reaction system was stirred at 20°C for 1 h. After the reaction was monitored by LCMS until completion, the reaction mixture was poured into water (300 mL) and extracted with dichloromethane (100 mL). The phases were separated, and the organic phase was concentrated to obtain 430 mg of the title compound **463-5.** LC-MS (ESI): m/z 647.4 $[2M+H]^+$.

**Preparation of compound 463-6**

Compound **463-5** (430 mg, 1.33 mmol) was dissolved in methanol (20 mL). Pd/C (2 g, 80% water content) was added, and the reaction system was stirred at 60°C for 12 h under a hydrogen atmosphere. After the reaction was monitored by TLC until completion, the reaction mixture was filtered, and the filtrate was concentrated to obtain 220 mg of the title compound **463-6.**

**Preparation of compound 463**

Compound **463-6** (220 mg, 1.16 mmol) was dissolved in ethanol (20 mL). Compound **7-1** (150 mg, 0.27 mmol) and DIEA (150 mg, 1.16 mmol) were added, and the reaction system was stirred at 90°C for 12 h in a sealed tube. The reaction mixture was filtered, and the filtrate was concentrated. The resulting crude product was subjected to preparative purification (preparative method: chromatographic column: Welch Xtimate C18 250 × 21.2 mm; column temperature: 25°C; mobile phase: water (10 mM/L $NH_4HCO_3$)-acetonitrile; gradient elution with the acetonitrile proportion in the mobile phase from 45% to 65% over 12 min; flow rate: 30 mL/min) to obtain 53.64 mg of the title compound **463.** LC-MS (ESI): m/z 705.2 $[M+H]^+$. $^1H$ NMR (400 MHz, DMSO-$d_6$) δ 8.41 (d, *J* = 5.0 Hz, 1H), 7.70 (d, *J* = 2.3 Hz, 1H), 7.64 (d, *J* = 2.3 Hz, 1H), 7.59 (d, *J* = 8.8 Hz, 2H), 7.55 (d, *J* = 8.4 Hz, 2H), 7.30 (d, *J* = 8.4 Hz, 2H), 7.08 (d, *J* = 8.8 Hz, 2H), 6.77 (d, *J* = 5.0 Hz, 1H), 5.08 (s, 2H), 4.75 (s, 2H), 4.42 (t, *J* = 5.2 Hz, 2H), 3.96 (t, *J* = 5.2 Hz, 2H), 3.91 - 3.68 (m, 1H), 2.87 (s, 3H), 2.05 - 1.91 (m, 2H), 1.92 - 1.73 (m, 6H), 1.68 (s, 6H).

**Example 464: Preparation of compound 464**

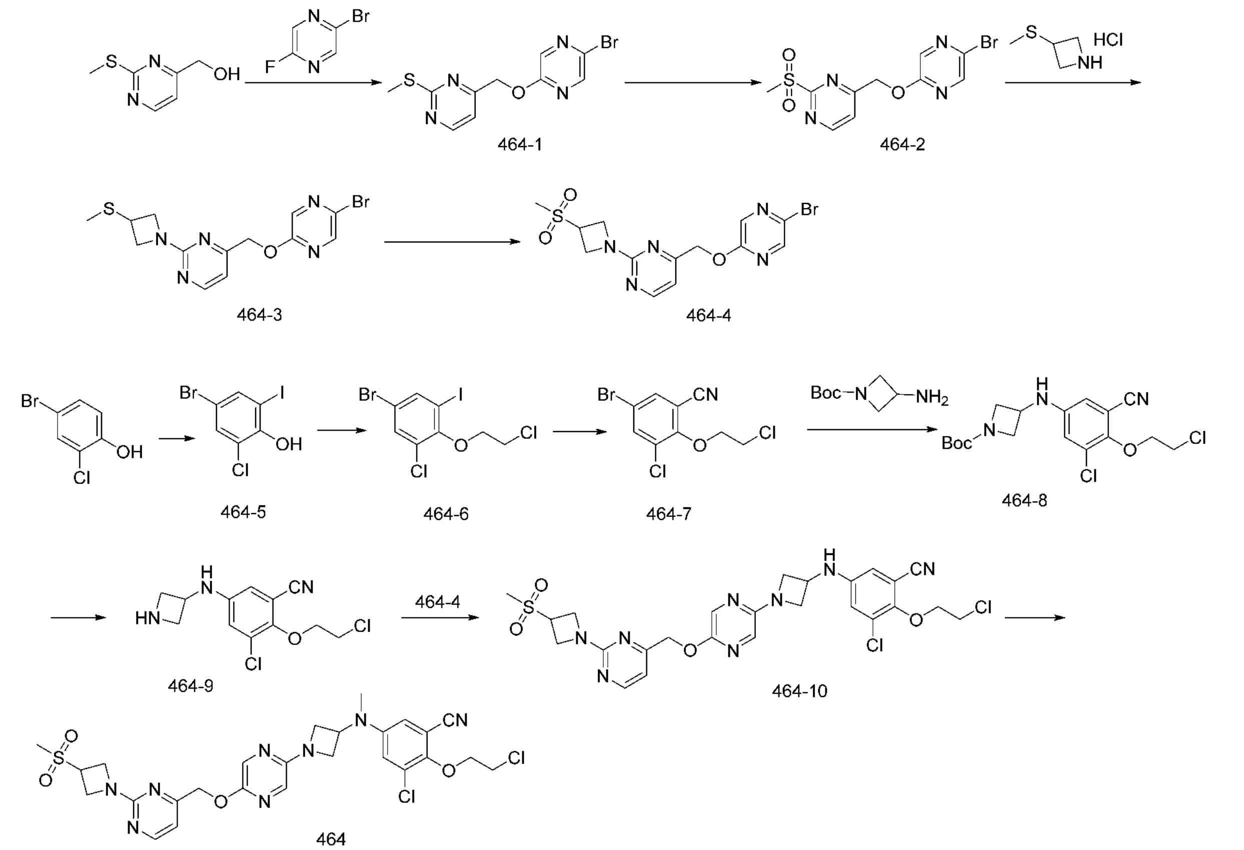


464-1 464-2

464-3 464-4

464-5 464-6 464-7 464-8

464-9 464-10

464

**Preparation of compound 464-1**

(2-(Methylthio)pyrimidin-4-yl)methanol (1.0 g, 6.41 mmol) was dissolved in tetrahydrofuran (2 mL), cooled to 0°C, and NaH (1.0 g, 9.61 mmol, 60% purity) was added. The system was purged three times with nitrogen, then warmed to room temperature and stirred for 0.5 h. 2-Fluoro-5-bromopyrazine (1.2 g, 7.05 mmol) was added, and the reaction system was stirred at 25°C for 5 h. After the reaction was completed, water (100 mL) was added, and the mixture was extracted three times with dichloromethane (200 mL). The organic phases were combined, washed twice with saturated brine (20 mL), dried over anhydrous sodium sulfate, subjected to suction filtration, and the filtrate was concentrated to obtain a crude product. The crude product was purified by silica gel column chromatography (EA/PE = 0 to 30%) to obtain 1.5 g of the title compound **464-1.**

**Preparation of compound 464-2**

Compound **464-1** (1.5 g, 4.80 mmol) was dissolved in a solution of THF (100 mL) and water (100 mL), followed by the addition of potassium peroxymonosulfate triple salt (8.8 g, 14.40 mmol). The reaction system was stirred at 25°C for 2 h. After the reaction was completed, insoluble solids were removed by filtration. Water (100 mL) was added to the filtrate, and the mixture was extracted three times with dichloromethane (200 mL). The organic phases were combined, washed twice with saturated brine (20 mL), dried over anhydrous sodium sulfate, subjected to suction filtration, and the filtrate was concentrated to obtain 1.4 g of the title compound **464-2.** LC-MS (ESI): m/z 345.0 $[M+H]^+$.

**Preparation of compound 464-3**

Compound **464-2** (1.4 g, 4.07 mmol) was dissolved in ethanol (50 mL), followed by the sequential addition of 3-(methylthio)azetidine hydrochloride (678 mg, 4.88 mmol) and DIEA (673 mg, 4.88 mmol). The reaction system was stirred at 75°C for 2 h. The reaction mixture was concentrated and purified by silica gel column chromatography (EA/PE = 0 to 50%) to obtain 1.1 g of the title compound **464-3.** LC-MS (ESI): m/z 368.0 $[M+H]^+$.

**Preparation of compound 464-4**

Compound **464-3** (500 mg, 1.36 mmol) was dissolved in THF (20 mL) and water (10 mL). Potassium peroxymonosulfate triple salt (2.51 g, 4.08 mmol) was added, and the reaction system was stirred at 20°C for 1 h. After the reaction was monitored by TLC until completion, the reaction mixture was poured into water (300 mL) and extracted with dichloromethane (100 mL). The phases were separated, and the organic phase was concentrated to obtain 410 mg of the title compound **464-4.**

**Preparation of compound 464-5**

2-Chloro-4-bromophenol (33.0 g, 176 mmol) was dissolved in acetonitrile (500 mL). The mixture was cooled to 0°C in an ice-water bath, and p-toluenesulfonic acid monohydrate (30 g, 176 mmol) was added. The mixture was stirred at this temperature for 30 min, followed by the addition of NIS (43.6 g, 194 mmol). After the addition was completed, the ice-water bath was removed, and the mixture was stirred at room temperature for 16 h. The reaction system was quenched with sodium sulfite solution. Water (2 L) was added, and the mixture was extracted twice with ethyl acetate (2 L). The organic phases were combined, washed three times with saturated brine (2 L), dried over anhydrous sodium sulfate, subjected to suction filtration, and the filtrate was concentrated. The residue was purified by normal-phase silica gel column chromatography (EA/PE = 0 to 30%) to obtain 45 g of the title compound **464-5** in 77% yield. $^1$H NMR (400 MHz, DMSO-$d_6$) δ 10.36 (s, 1H), 7.86 (d, *J* = 2.3 Hz, 1H), 7.66 (d, *J* = 2.3 Hz, 1H).

**Preparation of compound 464-6**

Compound **464-5** (45 g, 135 mol) was dissolved in DMF (500 mL), followed by the sequential addition of cesium carbonate (97 g, 297 mmol) and compound 1-bromo-2-chloroethane (28.8 g, 202 mmol). The mixture was heated to 80°C and reacted for 16 h. After the reaction was completed, the reaction mixture was filtered. Water (3 L) was added to the filtrate, and the pH was adjusted to approximately 5 with dilute hydrochloric acid. The mixture was extracted twice with ethyl acetate (2 L). The organic phases were combined, washed three times with saturated brine (2 L), dried over anhydrous sodium sulfate, and concentrated. The resulting residue was purified by normal-phase silica gel column chromatography (EA/PE = 0 to 30%) to obtain 35 g of the title compound **464-6** in 66% yield. LC-MS (ESI): m/z 394.3.

**Preparation of compound 464-7**

Compound **464-6** (35 g, 88.0 mmol) was dissolved in DMF (500 mL), and CuCN (8.6 g, 96.8 mmol) and CuI (1.6 g, 8.8 mmol) were added. The system was purged three times with nitrogen, heated to 100°C, and reacted for 5 h. The reaction was monitored by TLC until completion. The solids were removed by filtration through a pad of diatomite, and the filter cake was rinsed with ethyl acetate. The filtrates were combined, water (2 L) was added, and the mixture was extracted twice with ethyl acetate (0.5 L). The organic phases were combined, washed three times with saturated brine (1 L), dried over anhydrous sodium sulfate, subjected to suction filtration, and the filtrate was concentrated. The resulting residue was purified by normal-phase silica gel column chromatography (EA/PE = 0 to 20%) to obtain 15 g of the title compound **464-7** in 58% yield. $^1$H NMR (400 MHz, DMSO-$d_6$) δ 8.20 (d, *J* = 2.4 Hz, 1H), 8.16 (d, *J* = 2.4 Hz, 1H), 4.45 (t, *J* = 5.2 Hz, 2H), 3.97 (t, *J* = 5.2 Hz, 2H).

**Preparation of compound 464-8**

Compound **464-7** (2.00 g, 6.82 mmol) was dissolved in dioxane (50 mL). *tert*-Butyl 3-aminoazetidine-1-carboxylate (1.40 g, 8.18 mmol), cesium carbonate (3.33 g, 10.23 mmol), $Pd_2(dba)_3$ (622 mg, 0.68 mmol), and BINAP (846 mg, 1.36 mmol) were added. After purging three times with nitrogen, the reaction system was stirred at 110°C for 3 h. The reaction mixture was filtered, and the filtrate was concentrated. The resulting residue was subjected to silica gel column chromatography (PE/EA = 100% to 30%) to obtain 1.2 g of the title compound **464-8.**

**Preparation of compound 464-9**

Compound **464-8** (1.2 g, 3.11 mmol) was dissolved in DCM (20 mL) and cooled to 0°C. Trifluoroacetic acid (3.5 g, 31.1 mmol) was added, and the reaction was carried out at room temperature for 2 h. The reaction mixture was concentrated, then poured into saturated aqueous sodium carbonate solution (100 mL), and extracted with DCM (100 mL). The phases were separated, and the organic phase was concentrated to obtain 620 mg of the title compound **464-9.** LC-MS (ESI): m/z 286.2 $[M+H]^+$.

**Preparation of compound 464-10**

Compound **464-9** (100 mg, 0.35 mmol) was dissolved in 1,4-dioxane (10 mL). Compound **464-4** (167 mg, 0.42 mmol), cesium carbonate (171 mg, 0.52 mmol), $Pd_2(dba)_3$ (32 mg, 0.035 mmol), and BINAP (44 mg, 0.07 mmol) were added. The reaction was carried out at 110°C for 3 h. After the reaction was completed, the reaction mixture was filtered, and the filtrate was concentrated. The resulting residue was purified by silica gel column chromatography (PE/EA = 100% to 70%) to obtain 55 mg of the title compound **464-10.**

**Preparation of compound 464**

Compound **464-10** (55 mg, 0.09 mmol) was dissolved in DMF (10 mL), and NaH (5 mg, 1.36 mmol) was added. The reaction system was stirred at 0°C for 1 h in a sealed tube. Iodomethane (17 mg, 0.12 mmol) was added, and the reaction was carried out at room temperature for 2 h. After the reaction was completed, saturated aqueous ammonium chloride solution (0.5 mL) was added to quench the reaction. The reaction mixture was filtered, and the filtrate was subjected to preparative purification (preparative method: chromatographic column: Welch Xtimate C18 250 × 21.2 mm; column temperature: 25°C; mobile phase: water (10 mM/L $NH_4HCO_3$)-acetonitrile; mobile phase: 45% to 65% acetonitrile gradient elution over 12 min; flow rate: 30 mL/min) to obtain 22.22 mg of the title compound **464.** LC-MS (ESI): m/z 619.2 $[M+H]^+$. $^1H$ NMR (400 MHz, DMSO-$d_6$) δ 8.36 (d, *J* = 5.0 Hz, 1H), 8.02 (d, *J* = 1.5 Hz, 1H), 7.50 (d, *J* = 1.5 Hz, 1H), 7.24 (d, *J* = 3.0 Hz, 1H), 7.18 (d, *J* = 3.0 Hz, 1H), 6.73 (d, *J* = 5.1 Hz, 1H), 5.20 (s, 2H), 4.81 - 4.68 (m, 1H), 4.46 - 4.36 (m, 1H), 4.35 - 4.24 (m, 5H), 4.24 - 4.14 (m, 3H), 4.00 - 3.84 (m, 4H), 3.06 (s, 3H), 2.94 (s, 3H).

**Example 465: Preparation of compound 465**

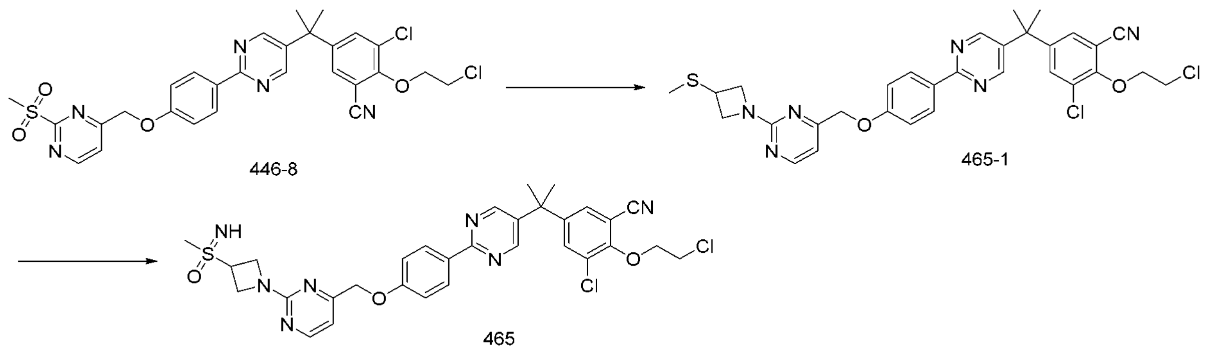

**Preparation of compound 465-1**

3-(Methylthio)azetidine hydrochloride (24 mg, 167.08 μmol), compound **446-8** (50 mg, 83.54 μmol), DIPEA (54 mg, 417.71 μmol), and acetonitrile (2 mL) were added to a reaction flask, and the mixture was stirred at 80°C for 1 h. The mixture was subjected to rotary evaporation until dryness to obtain 50 mg of a crude product of the title compound **465-1,** which was used directly in the next step without purification. LC-MS (ESI): m/z 621.2 $[M+H]^+$.

**Preparation of compound 465**

Compound **465-1** (50 mg, 80.44 μmol), (diacetoxyiodo)benzene (130 mg, 402.20 μmol), and ammonium carbonate (24 mg, 241.32 μmol) were dissolved in methanol (3 mL). The mixture was stirred at room temperature for 2 h and subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 95% acetonitrile gradient elution over 25 min; flow rate: 30 mL/min) to obtain 10 mg of the title compound **465.** LC-MS (ESI): m/z 652.2 $[M+H]^+$. $^1H$ NMR (400 MHz, DMSO-$d_6$) δ 8.74 (s, 2H), 8.41 (d, *J* = 5.0 Hz, 1H), 8.32 (d, *J* = 8.9 Hz, 2H), 7.80 (q, *J* = 2.4 Hz, 2H), 7.15 (d, *J* = 8.9 Hz, 2H), 6.84 (d, *J* = 5.0 Hz, 1H), 5.11 (s, 2H), 4.43 (t, *J* = 5.2 Hz, 2H), 4.34 - 4.18 (m, 5H), 3.99 (s, 1H), 3.96 (t, *J* = 5.0 Hz, 2H), 2.91 (s, 3H), 1.75 (s, 6H).

**Example 466: Preparation of compound 466**

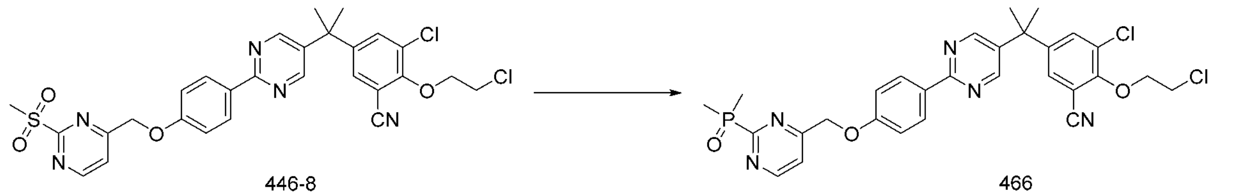

446-8 466

**Preparation of compound 466**

Dimethylphosphine oxide (13 mg, 167.08 μmol), compound **446-8** (50 mg, 83.54 μmol), cesium carbonate (55 mg, 167.08 μmol), and acetonitrile (2 mL) were added to a reaction flask, and the mixture was stirred at 80°C for 1 h. The mixture was filtered, and the filtrate was subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 90% acetonitrile gradient elution over 25 min; flow rate: 30 mL/min) to obtain 3 mg of the title compound **466.** LC-MS (ESI): m/z 596.2 $[M+H]^+$. $^1H$ NMR (400 MHz, DMSO-$d_6$) δ 9.01 (d, *J* = 5.2 Hz, 1H), 8.74 (s, 2H), 8.34 (d, *J* = 8.9 Hz, 2H), 7.80 (q, *J* = 2.4 Hz, 2H), 7.75 (dd, *J* = 5.2, 3.2 Hz, 1H), 7.20 (d, *J* = 8.9 Hz, 2H), 5.39 (s, 2H), 4.43 (t, *J* = 5.2 Hz, 2H), 3.96 (t, *J* = 5.2 Hz, 2H), 1.76 (d, *J* = 13.6 Hz, 6H), 1.74 (s, 6H). $^{31}P$ NMR (162 MHz, DMSO-$d_6$) δ 34.18 (s, 1P).

**Example 467: Preparation of compound 467**

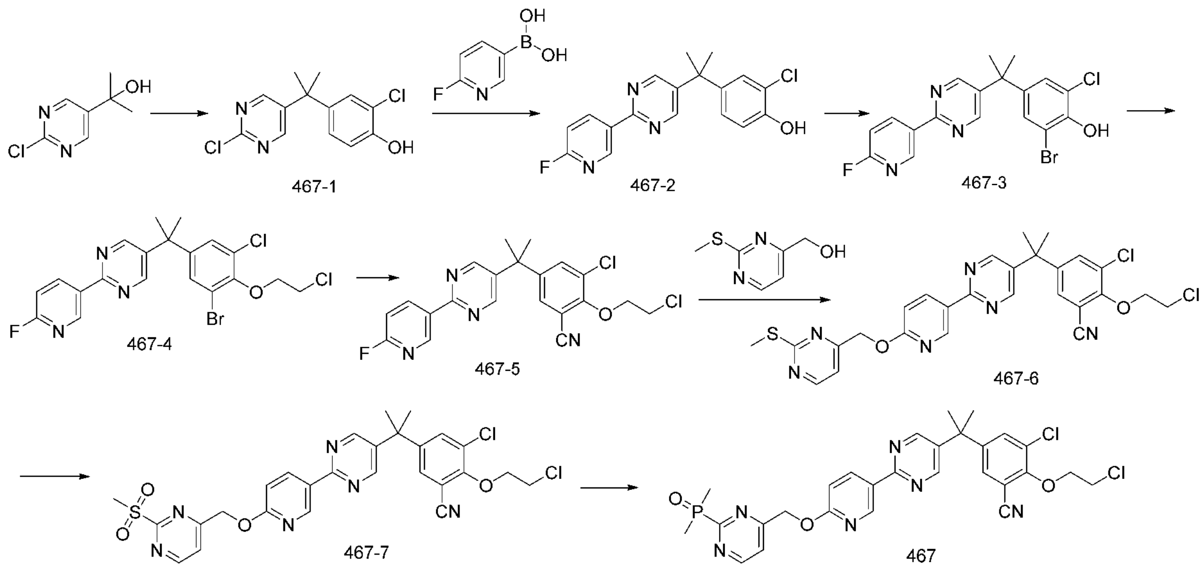

467-1 467-2 467-3 467-4 467-5 467-6 467-7 467

**Preparation of compound 467-1**

Compound 2-chlorophenol (31 g, 243.32 mmol), 2-(2-chloropyrimidin-5-yl)propan-2-ol (14 g, 81.11 mmol), and $AlCl_3$ (21.63 g, 162.21 mmol) were added to a reaction flask. The mixture was stirred at 80°C for 2 h. After cooling to 0°C, saturated aqueous ammonium chloride solution (500 mL) was slowly added dropwise. The mixture was extracted three times with DCM (500 mL). The organic phases were combined and subjected to rotary evaporation until dryness. The crude product was purified by silica gel column chromatography (EA/PE = 0 to 50%) to obtain 3.8 g of compound **467-1.** LC-MS (ESI): m/z 283.2 $[M+H]^+$.

**Preparation of compound 467-2**

Under a nitrogen atmosphere, 2-fluoro-5-pyridineboronic acid (747 mg, 5.30 mmol), compound **467-1** (1 g, 3.53 mmol), Pd(dppf)$Cl_2$ (517 mg, 706.33 μmol), potassium carbonate (1.46 g, 10.59 mmol), 1,4-dioxane (10 mL), and water (2 mL) were added to a reaction flask. The mixture was stirred at 110°C for 6 h and then subjected to rotary evaporation until dryness. The crude product was purified by silica gel column chromatography (EtOAc/PE = 0 to 50%) to obtain 800 mg of compound **467-2.**

**Preparation of compound 467-3**

Compound **467-2** (400 mg, 1.16 mmol) and acetonitrile (20 mL) were added to a reaction flask. NBS (228 mg, 1.28 mmol) was added in portions at 0°C. The mixture was slowly warmed to room temperature and stirred for 2 h. After cooling to 0°C, saturated aqueous sodium sulfite solution (50 mL) was slowly added dropwise. The mixture was extracted three times with dichloromethane (50 mL). The organic phases were combined and subjected to rotary evaporation until dryness. The crude product was purified by silica gel column chromatography (EA/PE = 0 to 50%) to obtain 450 mg of the title compound **467-3.** LC-MS (ESI): m/z 422.0 $[M+H]^+$.

**Preparation of compound 467-4**

Potassium carbonate (393 mg, 2.84 mmol) was added to a solution of compound **467-3** (400 mg, 946.35 μmol) and 1-bromo-2-chloroethane (408 mg, 2.84 mmol) in acetonitrile (5 mL). The reaction mixture was stirred at 70°C for 16 h. The reaction mixture was filtered, and the filtrate was subjected to rotary evaporation until dryness to obtain 450 mg of the title compound **467-4.** LC-MS (ESI): m/z 484.0 $[M+H]^+$.

**Preparation of compound 467-5**

Under a nitrogen atmosphere, compound **467-4** (500 mg, 1.03 mmol), zinc cyanide (363 mg, 3.09 mmol), tetrakis(triphenylphosphine)palladium (119 mg, 103.06 μmol), and DMF (5 mL) were added to a reaction flask. The mixture was stirred at 120°C for 16 h. Water (10 mL) was added, and the mixture was extracted three times with ethyl acetate (5 mL). The organic phases were combined and subjected to rotary evaporation until dryness. The resulting crude product was purified by silica gel column chromatography (EA/PE = 0 to 50%) to obtain 400 mg of the title compound **467-5.** LC-MS (ESI): m/z 431.0 $[M+H]^+$.

**Preparation of compound 467-6**

NaH (73 mg, 1.81 mmol, 60% purity) was slowly added to a solution of (2-(methylthio)pyrimidin-4-yl)methanol (283 mg, 1.81 mmol) in THF (5 mL) at 0°C. After stirring for 20 min, a solution of compound **467-5** (520 mg, 1.21 mmol) in THF (5 mL) was added dropwise to the reaction system. The mixture was stirred at room temperature for 2 h. Water (20 mL) was added, and the mixture was extracted three times with ethyl acetate (20 mL). The organic phases were combined and subjected to rotary evaporation until dryness. The crude product was subjected to silica gel column chromatography (MeOH/DCM = 0 to 5%) to obtain 550 mg of compound**467-6**. LC-MS (ESI): m/z 567.0 $[M+H]^+$.

**Preparation of compound 467-7**

Compound **467-6** (550 mg, 969.18 μmol), DCM (10 mL), and *m*-CPBA (787 mg, 3.88 mmol, 85% purity) were stirred at room temperature for 2 h. The reaction mixture was poured into saturated sodium sulfite solution (30 mL) and extracted three times with dichloromethane (30 mL). The organic phases were combined, washed three times with saturated brine (30 mL), dried over anhydrous sodium sulfate, filtered, and the filtrate was subjected to rotary evaporation until dryness. The resulting residue was purified by silica gel column chromatography (EA/PE = 0 to 70%) to obtain 500 mg of the title compound **467-7.** LC-MS (ESI): m/z 599.1 $[M+H]^+$.

**Preparation of compound 467**

Dimethylphosphine oxide (26 mg, 333.62 μmol), compound **467-7** (100 mg, 166.81 μmol), cesium carbonate (109 mg, 333.62 μmol), and acetonitrile (2 mL) were added to a reaction flask, and the mixture was stirred at 80°C for 1 h. The mixture was filtered, and the filtrate was subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 90%

acetonitrile gradient elution over 25 min; flow rate: 30 mL/min) to obtain 3 mg of the title compound **467.** LC-MS (ESI): m/z 597.2 [M+H]$^+$. $^1$H NMR (400 MHz, DMSO-$d_6$) δ 9.05 (d, *J* = 2.3 Hz, 1H), 8.95 (d, *J* = 5.3 Hz, 1H), 8.79 (s, 2H), 8.64 (dd, *J* = 8.9, 2.4 Hz, 1H), 7.80 (s, 2H), 7.67 - 7.60 (m, 1H), 7.18 (d, *J* = 8.8 Hz, 1H), 5.63 (s, 2H), 4.43 (t, *J* = 5.1 Hz, 2H), 3.96 (t, *J* = 5.1 Hz, 2H), 1.75 (s, 6H), 1.73 (d, *J* = 13.6 Hz, 6H). $^{31}$P NMR (162 MHz, DMSO-$d_6$) δ 34.20 (s, 1P).

**Example 468: Preparation of compound 468**

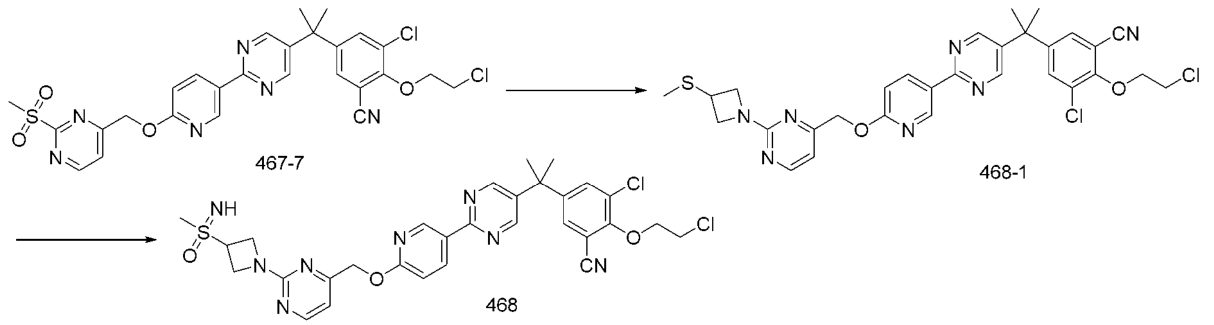

467-7

468-1

468

**Preparation of compound 468-1**

3-(Methylthio)azetidine hydrochloride (47 mg, 333.62 μmol), compound **467-7** (100 mg, 166.81 μmol), DIPEA (108 mg, 834.05 μmol), and acetonitrile (2 mL) were added to a reaction flask, and the mixture was stirred at 80°C for 1 h. The mixture was subjected to rotary evaporation until dryness to obtain 80 mg of a crude product of the title compound **468-8.** LC-MS (ESI): m/z 622.2 [M+H]$^+$.

**Preparation of compound 468**

Compound **468-1** (100 mg, 160.63 μmol), (diacetoxyiodo)benzene (259 mg, 803.13 μmol), and ammonium carbonate (77 mg, 803.13 μmol) were dissolved in methanol (3 mL). The mixture was stirred at room temperature for 2 h and subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 95% acetonitrile gradient elution over 25 min; flow rate: 30 mL/min) to obtain 30 mg of the title compound **468.** LC-MS (ESI): m/z 653.2 [M+H]$^+$. $^1$H NMR (400 MHz, DMSO-$d_6$) δ 9.06 (d, *J* = 2.4 Hz, 1H), 8.78 (s, 2H), 8.61 (dd, *J* = 8.7, 2.4 Hz, 1H), 8.35 (d, *J* = 5.1 Hz, 1H), 7.80 (s, 2H), 7.13 (d, *J* = 8.7 Hz, 1H), 6.72 (d, *J* = 5.0 Hz, 1H), 5.36 (s, 2H), 4.43 (t, *J* = 5.2 Hz, 2H), 4.31 - 4.16 (m, 5H), 3.98 (s, 1H), 3.96 (t, *J* = 5.2 Hz, 2H), 2.90 (s, 3H), 1.75 (s, 6H).

**Example 469: Preparation of compound 469**

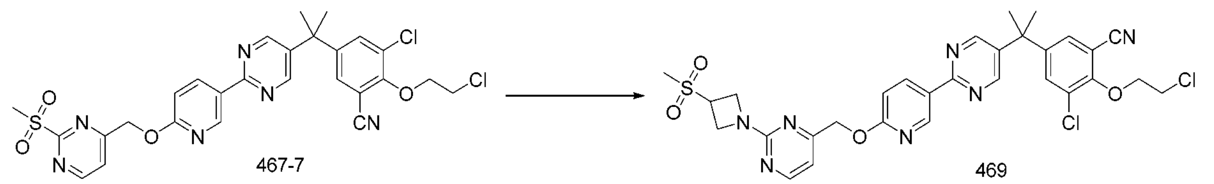

467-7

469

**Preparation of compound 469**

3-(Methylsulfonyl)azetidine hydrochloride (57 mg, 333.62 μmol), compound **467-7** (100 mg, 166.81 μmol), DIPEA (108 mg, 834.05 μmol), and acetonitrile (2 mL) were added to a reaction flask, and the mixture was stirred at 80°C for 2 h. The mixture was subjected to rotary evaporation until dryness. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 25% to 90% acetonitrile gradient elution

over 15 min; flow rate: 30 mL/min) to obtain 3 mg of the title compound **469.** LC-MS (ESI): m/z 654.2 [M+H]+. $^1$H NMR (400 MHz, DMSO-$d_6$) δ 9.06 (dd, *J* = 2.4, 0.7 Hz, 1H), 8.78 (s, 2H), 8.61 (dd, *J* = 8.7, 2.4 Hz, 1H), 8.37 (d, *J* = 5.0 Hz, 1H), 7.80 (s, 2H), 7.13 (dd, *J* = 8.7, 0.7 Hz, 1H), 6.76 (d, *J*= 5.1 Hz, 1H), 5.37 (s, 2H), 4.43 (t, *J* = 5.2 Hz, 2H), 4.43 - 4.35 (m, 1H), 4.32 (t, *J* = 8.8 Hz, 2H), 4.25 - 4.17 (m, 2H), 3.96 (t, *J* = 5.2 Hz, 2H), 3.06 (s, 3H), 1.75 (s, 6H).

### Example 470: Preparation of compound 470

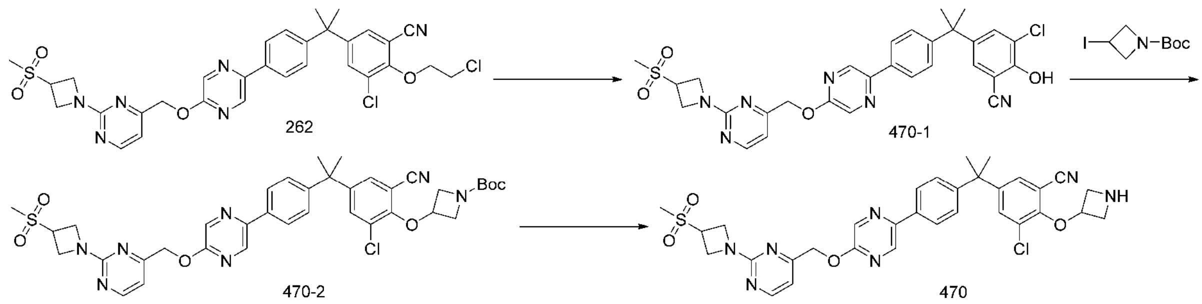
262
470-1
470-2
470

### Preparation of compound 470-1

Compound **262** (250 mg, 0.44 mmol) was weighed and added to DMF (3 mL), and sodium hydrosulfide hydrate (98 mg, 1.32 mmol) was added with stirring. After the addition was completed, the reaction was carried out at room temperature for 2 h. After the reaction was monitored by LCMS until completion, the reaction mixture was filtered, and the filtrate was subjected to rotary evaporation until dryness. The resulting crude product was purified by normal-phase silica gel column chromatography (EA/PE = 0 to 60%) to obtain 180 mg of the title compound **470-1.** LC-MS (ESI): m/z 591.2 [M+H]+.

### Preparation of compound 470-2

Compound **470-1** (140 mg, 0.24 mmol) was added to a microwave tube, followed by the sequential addition of anhydrous DMF (2 mL), *tert*-butyl 3-iodoazetidine-1-carboxylate (101 mg, 0.36 mmol), and cesium carbonate (232 mg, 0.71 mmol). The system was purged three times with nitrogen, and the reaction was carried out under microwave irradiation at 120°C for 2 h. After the reaction was monitored by LCMS until completion, the reaction mixture was filtered, and the filtrate was subjected to rotary evaporation until dryness. The resulting crude product was purified by normal-phase silica gel column chromatography (EA/PE = 0 to 60%) to obtain 65 mg of the title compound **470-2.** LC-MS (ESI): m/z 746.4 [M+H]+.

### Preparation of compound 470

Compound **470-2** (65 mg, 0.09 mmol) was dissolved in dichloromethane (2 mL), and trifluoroacetic acid (0.5 mL) was added dropwise with stirring. After the addition was completed, the reaction was carried out at room temperature for 2 h. After the reaction was monitored by LCMS until completion, the reaction mixture was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 25 mg of the title compound **470.** LC-MS (ESI): m/z 646.4 [M+H]+. $^1$H NMR (400 MHz, DMSO-$d_6$) δ 8.75 (d, *J* = 1.4 Hz, 1H), 8.54 (d,*J* = 1.4 Hz, 1H), 8.38 (d, *J*= 5.1 Hz, 1H), 7.96 (d, *J* = 8.6 Hz, 2H), 7.72 (d, *J* = 2.3 Hz, 1H), 7.65 (d, *J* = 2.3 Hz, 1H), 7.36 (d, *J* = 8.6 Hz, 2H), 6.79 (d, *J* = 5.0 Hz, 1H), 5.37 (s, 2H), 5.12 - 5.02 (m, 1H), 4.44 - 4.36 (m, 1H), 4.31 (t, *J* = 8.8 Hz, 2H), 4.24 - 4.17 (m, 2H), 4.17 - 4.10 (m, 2H), 4.10 - 4.02 (m, 2H), 3.05 (s, 3H), 1.69 (s, 6H).

### Example 471: Preparation of compound 471

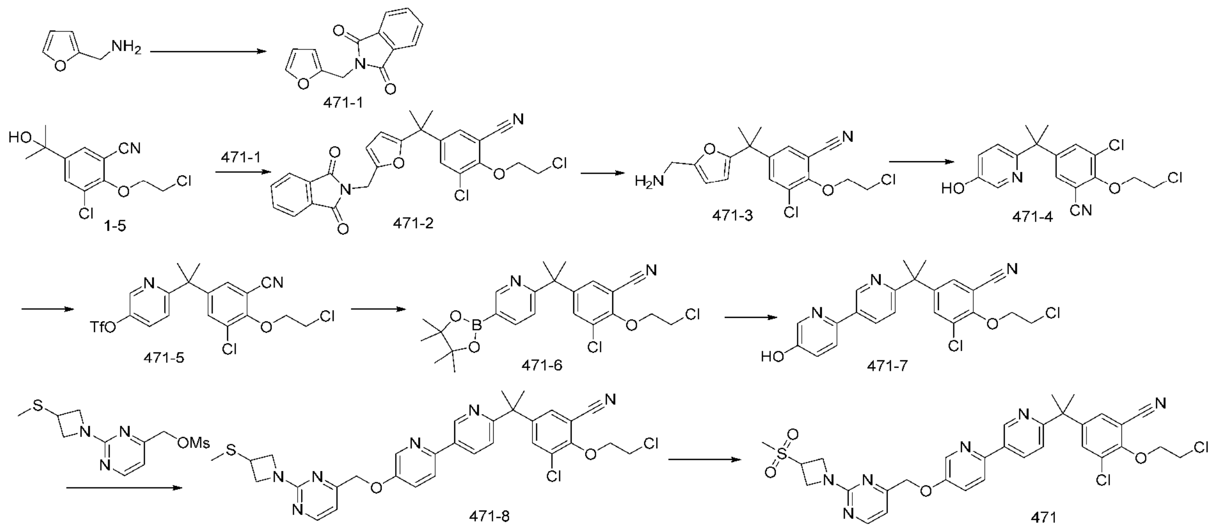

**Preparation of compound 471-1**

Furan-2-ylmethanamine (10 g, 103.0 mmol) and phthalic anhydride (18.3 g, 123.6 mmol) were dissolved in glacial acetic acid (12 mL). The reaction system was stirred at 120°C for 3 h. After the reaction was completed, water (80 mL) was added, and the mixture was extracted three times with dichloromethane (30 mL). The organic phases were combined, washed twice with saturated brine (30 mL), dried over anhydrous sodium sulfate, subjected to suction filtration, and the filtrate was concentrated to obtain a crude product. The crude product was purified by silica gel column chromatography (EA/PE = 0 to 30%) to obtain 17.8 g of the title compound **471-1.** LC-MS (ESI): m/z 228.0 $[M+H]^+$.

**Preparation of compound 471-2**

Compound **1-5** (12 g, 32.1 mmol) was dissolved in dichloromethane (120 mL), and a solution of tetrafluoroboric acid in diethyl ether (17 mL, 64.2 mmol) was added dropwise at - 78°C. After 15 min, **471-1** (8.29 g, 32.1 mmol) was added in one portion. The mixture was maintained at this temperature for 30 min, then slowly warmed to room temperature and stirred for 1 h. After the reaction was completed, saturated aqueous sodium bicarbonate solution (50 mL) was added to the reaction system. The mixture was extracted three times with dichloromethane (35 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 40%) to obtain 13.8 g of the title compound **471-2.** LC-MS (ESI): m/z 483.0 $[M+H]^+$.

**Preparation of compound 471-3**

Compound **471-2** (6.2 g, 12.8 mmol) was dissolved in ethanol (70 mL), and hydrazine hydrate (4 mL, 128 mmol) was added dropwise. The reaction system was stirred at 85°C for 3 h. After the reaction was completed, the reaction mixture was cooled to room temperature. Saturated aqueous sodium chloride solution (50 mL) was added to the reaction system, and the mixture was extracted twice with dichloromethane (30 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 60%) to obtain 3.8 g of the title compound **471-3.** LC-MS (ESI): m/z 336.0 $[M-NH_2]^+$.

**Preparation of compound 471-4**

Compound **471-3** (3.8 g, 10.76 mmol) was dissolved in methanol (40 mL) and water (10 mL). Bromine (0.63 mL, 11.8 mmol) was added dropwise in an ice bath. The reaction system was stirred at room temperature for 3 h. After the reaction was completed, saturated aqueous sodium chloride (30 mL) solution was added to the reaction system, and the mixture was extracted twice with dichloromethane (30 mL). The organic phases were combined, washed twice with saturated brine (40 mL), dried over anhydrous sodium sulfate, filtered, and concentrated to obtain a crude product. The crude product was purified by silica gel column chromatography (PE/EA = 100% to 60%) to obtain 2.6 g of the title compound **471-4.** LC-MS (ESI): m/z 351.2 $[M+H]^+$.

**Preparation of compound 471-5**

Compound **471-4** (2.6 g, 7.40 mmol), triethylamine (1.87 g, 18.5 mmol), and *N*-phenylbis(trifluoromethanesulfonyl)imide (2.42 g, 8.14 mmol) were dissolved in dichloromethane (30 mL), and the mixture was stirred in an ice bath for 3 h. After the reaction was completed, the reaction mixture was cooled to room temperature. Saturated aqueous sodium chloride (50 mL) solution was added to the reaction system, and the mixture was extracted twice with dichloromethane (30 mL). The organic phases were combined, washed twice with saturated brine (50 mL), dried over anhydrous sodium sulfate, filtered, and the filtrate was concentrated to obtain a crude product. The crude product was purified by silica gel column chromatography (PE/EA = 100% to 60%) to obtain 2.8 g of the title compound **471-5.**

**Preparation of compound 471-6**

Compound **471-5** (2 g, 4.14 mmol) and bis(pinacolato)diboron (1.89 g, 7.45 mmol) were dissolved in 1,4-dioxane (20 mL), followed by the sequential addition of potassium carbonate (1.43 g, 10.4 mmol) and [1,1'-bis(diphenylphosphino) ferrocene]dichloropalladium(II) dichloromethane complex (54 mg, 66.34 μmol). The system was purged three times with nitrogen and stirred at 115°C for 5 h. After the reaction was completed, the reaction mixture was cooled to room temperature and filtered. Saturated aqueous sodium chloride solution (25 mL) was added to the filtrate, and the mixture was extracted three times with ethyl acetate (20 mL). The organic phases were combined, dried, and concentrated to obtain a crude product. The crude product was purified by normal-phase silica gel column chromatography (EtOAc/PE = 0 to 45%) to obtain 1.4 g of the title compound **471-6.** LC-MS (ESI): m/z 483.4 $[M+Na]^+$.

**Preparation of compound 471-7**

Compound **471-6** (600 mg, 1.30 mmol) and 2-bromo-5-hydroxypyridine (249 mg, 1.43 mmol) were dissolved in 1,4-dioxane (8 mL), followed by the sequential addition of potassium carbonate (448 mg, 3.25 mmol) and [1,1'-bis(diphenylphosphino)ferrocene]dichloropalladium(II) dichloromethane complex (27 mg, 33.17 μmol). The system was purged three times with nitrogen and stirred at 105°C for 5 h. After the reaction was completed, the reaction mixture was cooled to room temperature and filtered. Saturated aqueous sodium chloride solution (25 mL) was added to the filtrate, and the mixture was extracted three times with ethyl acetate (20 mL). The organic phases were combined, dried, and concentrated to obtain a crude product. The crude product was purified by normal-phase silica gel column chromatography (EtOAc/PE = 0 to 45%) to obtain 285 mg of the title compound **471-7.** LC-MS (ESI): m/z 428.2 $[M+H]^+$.

**Preparation of compound 471-8**

Compound **471-7** (180 mg, 421 μmol), (2-(3-(methylthio)azetidin-1-yl)pyrimidin-4-yl)methyl methanesulfonate (134 mg, 463 μmol), cesium carbonate (342 mg, 1.05 mmol), and acetonitrile (4 mL) were added to a three-necked flask. The system was purged three times with nitrogen and stirred at 75°C for 3 h. After the reaction was completed, saturated aqueous sodium chloride solution (10 mL) was added to the reaction system, and the mixture was extracted three times with DCM (10 mL). The organic phases were combined, dried, and concentrated to obtain a crude product. The crude product was purified by normal-phase silica gel column chromatography (EtOAc/PE = 0 to 65%) to obtain 196 mg of the title compound **471-8.**

**Preparation of compound 471**

Compound **471-8** (80 mg, 129 μmol) was added to a three-necked flask, and tetrahydrofuran (5 mL) was added as a solvent. Water (1 mL) was added to another conical flask, followed by the addition of potassium peroxymonosulfate (213 mg, 650 μmol). After stirring to dissolve, the mixture was added dropwise to the reaction system in an ice bath. The reaction system was stirred at room temperature for 16 h. The reaction mixture was filtered and subjected to rotary evaporation until dryness to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 55% acetonitrile gradient elution over 12 min; flow rate: 30 mL/min) to obtain 32 mg of the title compound **471.** LC-MS (ESI): m/z 653.4 $[M+H]^+$. $^1H$ NMR (400 MHz, DMSO-$d_6$) δ 9.12 (dd, *J* = 2.4, 0.7 Hz, 1H), 8.49 (d, *J* = 3.0 Hz, 1H), 8.44 (d, *J* = 5.0 Hz, 1H), 8.32 (dd, *J* = 8.4, 2.4 Hz, 1H), 7.99 (d, *J* = 8.8 Hz, 1H), 7.70 (d, *J* = 2.3 Hz, 1H), 7.65 (d, *J* = 2.4 Hz, 1H), 7.58 (dd, *J* = 8.9, 3.0 Hz, 1H), 7.45 (d, *J* = 8.3 Hz, 1H), 6.90 (d, *J* = 5.0 Hz, 1H), 5.19 (s, 2H), 4.50 - 4.37 (m, 3H), 4.34 (t, *J* = 8.7 Hz, 2H), 4.28 - 4.18 (m, 2H), 3.95 (t, *J* = 5.2 Hz, 2H), 3.06 (s, 3H), 1.73 (s, 6H).

**Example 472: Preparation of compound 472**

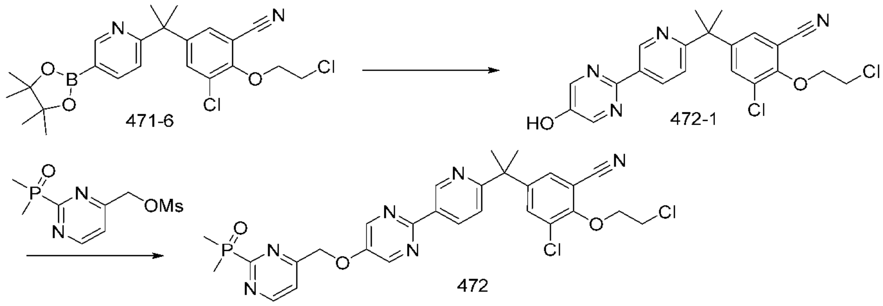
471-6

472-1

472

**Preparation of compound 472-1**

Compound **471-6** (620 mg, 1.28 mmol) and 2-bromo-5-hydroxypyrimidine (246 mg, 1.41 mmol) were dissolved in 1,4-dioxane (10 mL), followed by the sequential addition of potassium carbonate (442 mg, 3.2 mmol) and [1,1'-bis(diphenylphosphino)ferrocene]dichloropalladium(II) dichloromethane complex (56 mg, 664 μmol). The system was purged three times with nitrogen and stirred at 105°C for 5 h. After the reaction was completed, the reaction mixture was cooled to room temperature and filtered. Saturated aqueous sodium chloride solution (25 mL) was added to the filtrate, and the mixture was extracted three times with ethyl acetate (20 mL). The organic phases were combined, dried, subjected to suction filtration, and the filtrate was concentrated to obtain a crude product. The crude product was purified by normal-phase silica gel column chromatography (EtOAc/PE = 0 to 45%) to obtain 150 mg of the title compound **472-1.** LC-MS (ESI): m/z 429.2 $[M+H]^+$.

**Preparation of compound 472**

Compound **472-1** (100 mg, 233 μmol), (2-(dimethylphosphoryl)pyrimidin-4-yl)methyl methanesulfonate (74 mg, 2.80 μmol), cesium carbonate (189 mg, 582 μmol), and acetonitrile (4 mL) were added to a three-necked flask. The reaction system was purged three times with nitrogen and stirred at 75°C for 3 h. After the reaction was completed, saturated aqueous sodium chloride solution (10 mL) was added to the reaction system. The mixture was extracted three times with DCM (10 mL). The organic phases were combined, dried, subjected to suction filtration, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 55% acetonitrile gradient elution over 12 min; flow rate: 30 mL/min) to obtain 32 mg of the title compound **472.** LC-MS (ESI): m/z 597.4 $[M+H]^+$. $^1H$ NMR (400 MHz, DMSO-$d_6$) δ 9.36 (d, *J* = 2.3 Hz, 1H), 9.04 (d, *J* = 5.2 Hz, 1H), 8.82 (s, 2H), 8.54 (dd, *J* = 8.4, 2.4 Hz, 1H), 7.81 (dd, *J* = 5.2, 3.2 Hz, 1H), 7.70 (d, *J* = 2.3 Hz, 1H), 7.66 (d, *J* = 2.4 Hz, 1H), 7.50 (d, *J* = 8.4 Hz, 1H), 5.56 (s, 2H), 4.42 (t, *J* = 5.2 Hz, 2H), 3.95 (t, *J* = 5.2 Hz, 2H), 1.74 (d, *J* = 13.6 Hz, 6H), 1.74 (s, 6H). $^{31}P$ NMR (162 MHz, DMSO-$d_6$) δ 34.15 (s, 1P).

**Example 473: Preparation of compound 473**

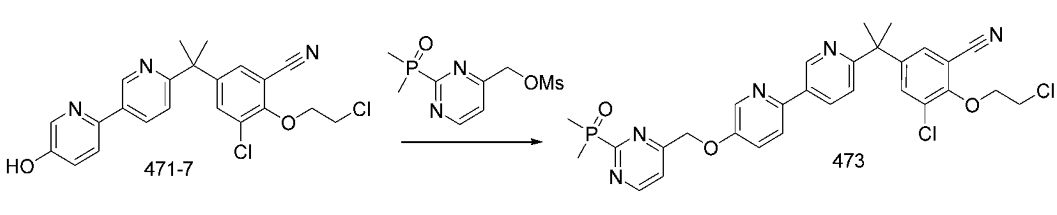
471-7

473

**Preparation of compound 473**

Compound **471-7** (80 mg, 187 μmol), (2-(dimethylphosphoryl)pyrimidin-4-yl)methyl methanesulfonate (60 mg, 224 μmol), cesium carbonate (152 mg, 467 mmol), and acetonitrile (4 mL) were added to a three-necked flask. The system was purged three times with nitrogen and stirred at 75°C for 3 h. After the reaction was completed, saturated aqueous

sodium chloride solution (10 mL) was added to the reaction system. The mixture was extracted three times with DCM (10 mL). The organic phases were combined, dried, subjected to suction filtration, and the filtrate was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 50% acetonitrile gradient elution over 12 min; flow rate: 30 mL/min) to obtain 24 mg of the title compound 473. LC-MS (ESI): m/z 596.4 $[M+H]^+$. $^1$H NMR (400 MHz, DMSO-$d_6$) δ 9.13 (d, *J* = 2.4 Hz, 1H), 9.02 (d, *J* = 5.1 Hz, 1H), 8.55 (d, *J* = 3.0 Hz, 1H), 8.33 (dd, *J* = 8.4, 2.4 Hz, 1H), 8.02 (d, *J* = 8.7 Hz, 1H), 7.78 (dd, *J* = 5.2, 3.2 Hz, 1H), 7.70 (d, *J* = 2.3 Hz, 1H), 7.69 - 7.60 (m, 2H), 7.45 (d, *J* = 8.3 Hz, 1H), 5.46 (s, 2H), 4.41 (t, *J* = 5.2 Hz, 2H), 3.95 (t, *J* = 5.2 Hz, 2H), 1.75 (d, *J* = 13.8 Hz, 6H), 1.74 (s, 6H). $^{31}$P NMR (162 MHz, DMSO-$d_6$) δ 34.10 (s, 1P).

**Example 474: Preparation of compound 474**

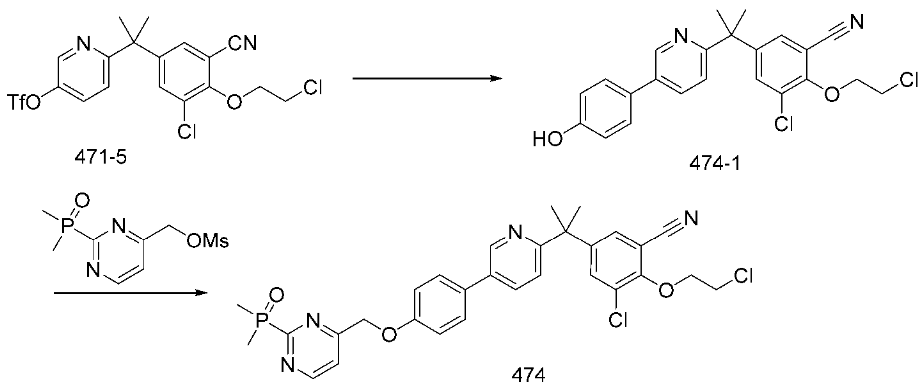
471-5

474-1

474

**Preparation of compound 474-1**

Compound **471-5** (500 mg, 1.03 mmol) and 4-hydroxyphenylboronic acid (157 mg, 1.13 mmol) were dissolved in 1,4-dioxane (8 mL), followed by the sequential addition of potassium carbonate (356 mg, 2.58 mmol) and [1,1'-bis(diphenylphosphino)ferrocene]dichloropalladium(II) dichloromethane complex (27 mg, 33.17 μmol). The system was purged three times with nitrogen and stirred at 105°C for 5 h. After the reaction was completed, the reaction mixture was cooled to room temperature and filtered. Saturated aqueous sodium chloride solution (25 mL) was added to the filtrate, and the mixture was extracted three times with ethyl acetate (20 mL). The organic phases were combined, dried, and concentrated to obtain a crude product. The crude product was purified by normal-phase silica gel column chromatography (EtOAc/PE = 0 to 45%) to obtain 310 mg of the title compound **474-1.** LC-MS (ESI): m/z 427.4 $[M+H]^+$.

**Preparation of compound 474**

Compound **474-1** (90 mg, 211 μmol), (2-(dimethylphosphoryl)pyrimidin-4-yl)methyl methanesulfonate (61 mg, 232 μmol), and cesium carbonate (171 mg, 527 μmol) were dissolved in acetonitrile (3 mL). The reaction system was purged three times with nitrogen and stirred at 75°C for 3 h. After the reaction was completed, saturated aqueous sodium chloride solution (10 mL) was added to the reaction system. The mixture was extracted three times with DCM (10 mL). The organic phases were combined, dried, subjected to suction filtration, and the filtrate was concentrated. The resulting crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 60% acetonitrile gradient elution over 12 min; flow rate: 30 mL/min) to obtain 24 mg of the title compound **474.** LC-MS (ESI): m/z 595.4 $[M+H]^+$. $^1$H NMR (400 MHz, DMSO-$d_6$) δ 9.00 (d, *J* = 5.2 Hz, 1H), 8.80 (d, *J* = 2.5 Hz, 1H), 7.99 (dd, *J* = 8.3, 2.5 Hz, 1H), 7.74 (dd, *J* = 5.2, 3.2 Hz, 1H), 7.72 - 7.68 (m, 3H), 7.67 (d, *J* = 2.3 Hz, 1H), 7.41 (d, *J* = 8.4 Hz, 1H), 7.24 - 7.13 (m, 2H), 5.37 (s, 2H), 4.41 (t, *J* = 5.2 Hz, 2H), 3.96 (t, *J* = 5.2 Hz, 2H), 1.77 (d, *J* = 13.7 Hz, 6H), 1.73 (s, 6H). $^{31}$P NMR (162 MHz, DMSO-$d_6$) δ 34.07 (s, 1P).

**Example 475: Preparation of compound 475**

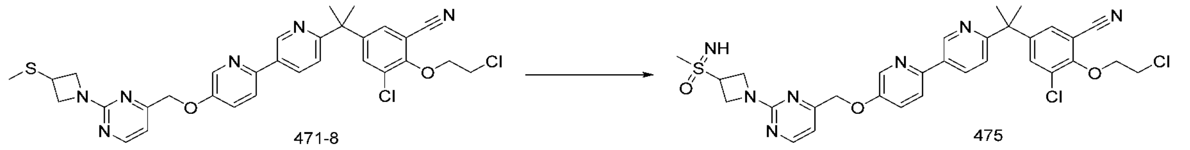

471-8 475

**Preparation of compound 475**

Compound **471-8** (70 mg, 113 μmol) and ammonium carbonate (54 mg, 567 μmol) were dissolved in methanol (3 mL), and (diacetoxyiodo)benzene (73 mg, 226 μmol) was added thereto at 0°C. After the addition was completed, the reaction system was stirred at 25°C for 3 h. After the reaction was completed, water (5 mL) was added, and the mixture was extracted three times with ethyl acetate (10 mL). The organic phases were combined, washed twice with saturated brine (20 mL), dried over anhydrous sodium sulfate, filtered, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 70% acetonitrile gradient elution over 12 min; flow rate: 30 mL/min) to obtain 12 mg of the title compound 475. LC-MS (ESI): m/z 652.4 $[M+H]^+$. $^1H$ NMR (400 MHz, DMSO-$d_6$) δ 9.12 (d, *J* = 2.4 Hz, 1H), 8.49 (d, *J* = 3.0 Hz, 1H), 8.42 (d, *J* = 5.0 Hz, 1H), 8.32 (dd, *J* = 8.4, 2.4 Hz, 1H), 7.99 (d, *J* = 8.8 Hz, 1H), 7.70 (d, *J* = 2.3 Hz, 1H), 7.65 (d, *J* = 2.4 Hz, 1H), 7.58 (dd, *J* = 8.8, 3.0 Hz, 1H), 7.45 (d, *J* = 8.3 Hz, 1H), 6.87 (d, *J* = 5.0 Hz, 1H), 5.18 (s, 2H), 4.41 (t, *J* = 5.2 Hz, 2H), 4.32 - 4.20 (m, 5H), 3.99 (s, 1H), 3.95 (t, *J* = 5.2 Hz, 2H), 2.91 (s, 3H), 1.73 (s, 6H).

**Example 476: Preparation of compound 476**

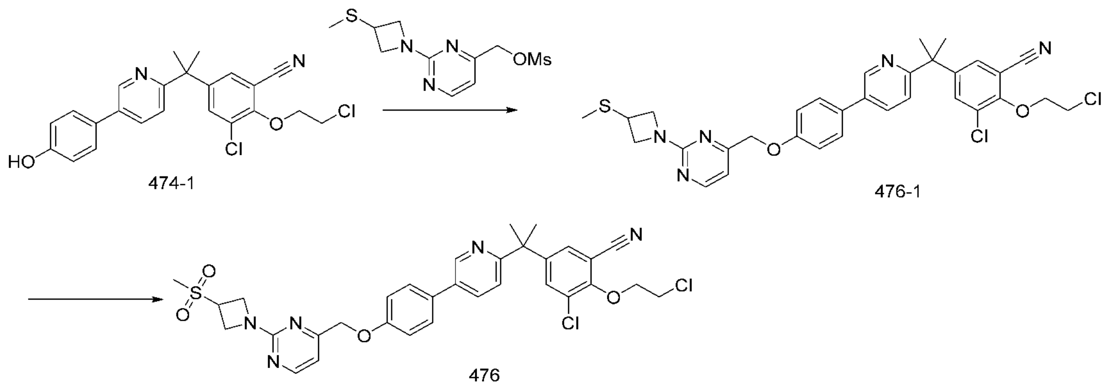

474-1 476-1 476

**Preparation of compound 476-1**

Compound **474-1** (210 mg, 491 μmol), (2-(3-(methylthio)azetidin-1-yl)pyrimidin-4-yl)methyl methanesulfonate (134 mg, 540 μmol), cesium carbonate (342 mg, 1.05 mmol), and acetonitrile (4 mL) were added to a three-necked flask. The system was purged three times with nitrogen and stirred at 75°C for 3 h. After the reaction was completed, saturated aqueous sodium chloride solution (10 mL) was added to the reaction system, and the mixture was extracted three times with DCM (10 mL). The organic phases were combined, dried, subjected to suction filtration, and the filtrate was concentrated to obtain a crude product. The crude product was purified by normal-phase silica gel column chromatography (EtOAc/PE = 0 to 45%) to obtain 196 mg of the title compound **476-1.**

**Preparation of compound 476**

Compound **476-1** (80 mg, 129 μmol) was added to a three-necked flask, followed by the addition of tetrahydrofuran (5 mL). Water (1 mL) was added to a conical flask, followed by the addition of potassium peroxymonosulfate (213 mg, 650 μmol). After stirring to dissolve, the mixture was added dropwise to the reaction system in an ice bath. The reaction system was stirred at room temperature for 16 h. The reaction mixture was filtered, and the filtrate was subjected to rotary evaporation until dryness to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 55% acetonitrile gradient elution over 12 min; flow

rate: 30 mL/min) to obtain 32 mg of the title compound **476.** LC-MS (ESI): m/z 652.4 $[M+H]^+$. $^1H$ NMR (400 MHz, DMSO-$d_6$) δ 8.79 (d, *J* = 2.5 Hz, 1H), 8.42 (d, *J* = 5.0 Hz, 1H), 7.98 (dd, *J* = 8.4, 2.5 Hz, 1H), 7.71 (d, *J* = 2.3 Hz, 1H), 7.70 - 7.63 (m, 3H), 7.40 (d, *J*= *8.3* Hz, 1H), 7.22 - 7.07 (m, 2H), 6.86 (d, *J* = 5.0 Hz, 1H), 5.10 (s, 2H), 4.46 - 4.38 (m, 3H), 4.34 (t, *J* = 8.7 Hz, 2H), 4.23 (dd, *J* = 9.9, 5.0 Hz, 2H), 3.95 (t, *J* = 5.2 Hz, 2H), 3.07 (s, 3H), 1.73 (s, 6H).

**Example 477: Preparation of compound 477**

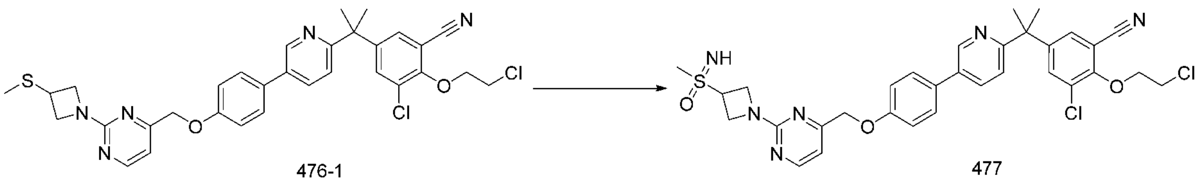

476-1 477

**Preparation of compound 476**

Compound **476-1** (85 mg, 117 μmol) and ammonium carbonate (65 mg, 685 μmol) were dissolved in methanol (3 mL), and (diacetoxyiodo)benzene (73 mg, 226 μmol) was added thereto at 0°C. After the addition was completed, the reaction system was stirred at 25°C for 3 h. After the reaction was completed, water (5 mL) was added, and the mixture was extracted three times with ethyl acetate (10 mL). The organic phases were combined, washed twice with saturated brine (20 mL), dried over anhydrous sodium sulfate, filtered, and the filtrate was concentrated to obtain a crude product. The crude product was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 15% to 70% acetonitrile gradient elution over 12 min; flow rate: 30 mL/min) to obtain 12 mg of the title compound 477. LC-MS (ESI): m/z 651.4 $[M+H]^+$. $^1H$ NMR (400 MHz, DMSO-$d_6$) δ 8.79 (d, *J* = 2.4 Hz, 1H), 8.40 (d, *J* = 5.0 Hz, 1H), 7.98 (dd, *J* = 8.3, 2.5 Hz, 1H), 7.71 (d, *J* = 2.3 Hz, 1H), 7.70 - 7.61 (m, 3H), 7.40 (d, *J* = 8.3 Hz, 1H), 7.11 (d, *J* = 8.8 Hz, 2H), 6.83 (d, *J* = 5.0 Hz, 1H), 5.09 (s, 2H), 4.41 (t, *J* = 5.2 Hz, 2H), 4.34 - 4.18 (m, 5H), 3.99 (s, 1H), 3.95 (t, *J*= 5.2 Hz, 2H), 2.91 (s, 3H), 1.73 (s, 6H).

**Example 478: Preparation of compound 478**

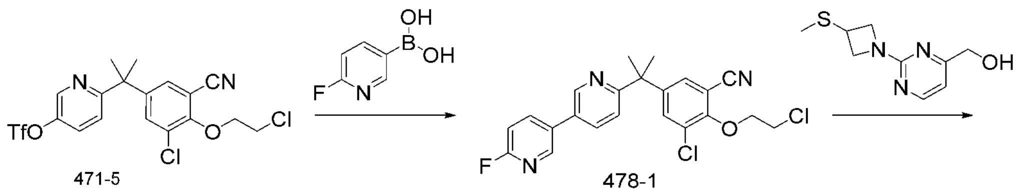

471-5 478-1

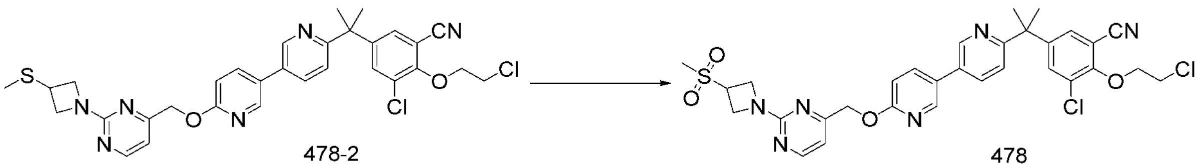

478-2 478

**Preparation of compound 478-1**

Compound **471-5** (870 mg, 1.8 mmol), (6-fluoropyridin-3-yl)boronic acid (254 mg, 1.8 mmol), [1,1'-bis(diphenylphosphino)ferrocene]dichloropalladium(II) (132 mg, 0.18 mmol), and potassium carbonate (621 mg, 4.5 mmol) were weighed and added to a mixed solvent of $H_2O$ (5 mL) and 1,4-dioxane (20 mL). The system was purged three times with nitrogen, and the reaction was carried out at 100°C for 5 h. After the reaction was monitored by LCMS until completion, the reaction mixture was poured into ice water and extracted twice with ethyl acetate (20 mL). The organic phases were combined, dried over anhydrous sodium sulfate, subjected to suction filtration, and the filtrate was concentrated to obtain a crude product. The crude product was purified by normal-phase silica gel column chromatography (EA/PE = 0 to 40%) to

obtain 650 mg of the title compound **478-1.** LC-MS (ESI): m/z 430.2 [M+H]+.

**Preparation of compound 478-2**

Compound **478-1** (300 mg, 0.7 mmol) was weighed and dissolved in anhydrous tetrahydrofuran (15 mL), and sodium hydride (56 mg, 1.39 mmol, 60% purity) was added in an ice-water bath. The reaction was carried out for 5 min, and then (2-(3-(methylthio)azetidin-1-yl)pyrimidin-4-yl)methanol (191 mg, 0.91 mmol) was added. After the addition was completed, the reaction was carried out at room temperature for 2 h. After the reaction was monitored by LCMS until completion, the reaction mixture was poured into ice water and extracted twice with ethyl acetate (20 mL). The organic phases were combined, dried over anhydrous sodium sulfate, subjected to suction filtration, and the filtrate was concentrated to obtain a crude product. The crude product was purified by normal-phase silica gel column chromatography (EA/PE = 0 to 60%) to obtain 220 mg of the title compound **478-2.** LC-MS (ESI): m/z 621.4 [M+H]+.

**Preparation of compound 478**

Compound **478-2** (80 mg, 0.13 mmol) was weighed and dissolved in dichloromethane (5 mL). *m*-CPBA (89 mg, 0.51 mmol) was added thereto at 0°C. After the addition was completed, the reaction was carried out at room temperature for 2 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 35 mg of the title compound **478.** LC-MS (ESI): m/z 653.4 [M+H]+. $^1$H NMR (400 MHz, DMSO-$d_6$) δ 8.83 (dd, *J* = 2.4, 0.8 Hz, 1H), 8.51 (dd, *J* = 2.5, 0.8 Hz, 1H), 8.36 (d, *J* = 5.0 Hz, 1H), 8.15 (dd, *J* = 8.6, 2.6 Hz, 1H), 8.04 (dd, *J* = 8.3, 2.5 Hz, 1H), 7.71 (d, *J* = 2.3 Hz, 1H), 7.67 (d, *J* = 2.3 Hz, 1H), 7.44 (dd, *J* = 8.3, 0.8 Hz, 1H), 7.11 (dd, *J* = 8.6, 0.7 Hz, 1H), 6.73 (d, *J* = 5.0 Hz, 1H), 5.33 (s, 2H), 4.45 - 4.36 (m, 3H), 4.32 (t, *J* = 8.8 Hz, 2H), 4.22 (dd, *J* = 9.9, 5.0 Hz, 2H), 3.95 (t, *J* = 5.2 Hz, 2H), 3.06 (s, 3H), 1.73 (s, 6H).

**Example 479: Preparation of compound 479**

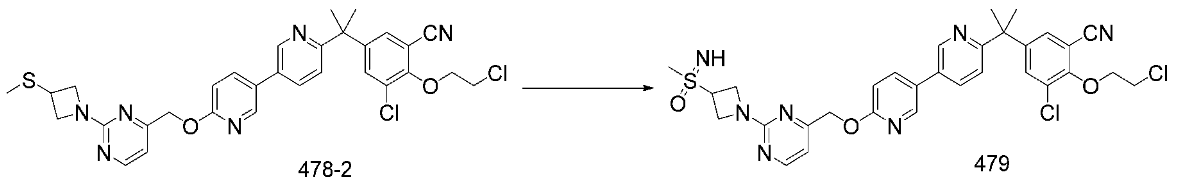

478-2

479

**Preparation of compound 479**

Compound **478-2** (80 mg, 0.13 mmol) and ammonium carbonate (37 mg, 0.39 mmol) were dissolved in methanol (5 mL). (Diacetoxyiodo)benzene (207 mg, 0.64 mmol) was added thereto at 0°C. After the addition was completed, the reaction was carried out at room temperature overnight. The reaction was monitored by LCMS until completion. The reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 50 mg of the title compound **479.** LC-MS (ESI): m/z 652.4 [M+H]+. $^1$H NMR (400 MHz, DMSO-$d_6$) δ 8.83 (dd, *J* = 2.5, 0.8 Hz, 1H), 8.55 - 8.48 (m, 1H), 8.34 (d, *J* = 5.0 Hz, 1H), 8.15 (dd, *J* = 8.7, 2.5 Hz, 1H), 8.05 (dd, *J* = 8.3, 2.5 Hz, 1H), 7.71 (d, *J* = 2.3 Hz, 1H), 7.67 (d, *J* = 2.4 Hz, 1H), 7.44 (dd, *J* = 8.3, 0.8 Hz, 1H), 7.11 (d, *J* = 8.7 Hz, 1H), 6.70 (d, *J* = 5.0 Hz, 1H), 5.32 (s, 2H), 4.41 (t, *J* = 5.2 Hz, 2H), 4.31 - 4.17 (m, 5H), 3.98 (s, 1H), 3.95 (t, *J* = 5.2 Hz, 2H), 2.91 (s, 3H), 1.73 (s, 6H).

**Example 480: Preparation of compound 480**

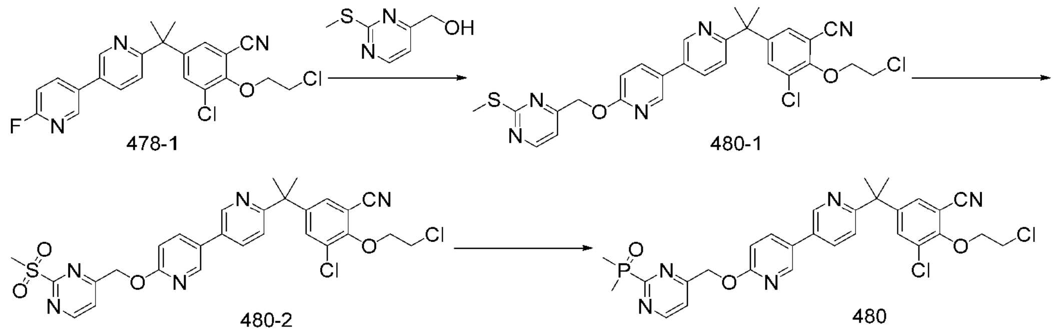

478-1 480-1 480-2 480

**Preparation of compound 480-1**

(2-(Methylthio)pyrimidin-4-yl)methanol (94 mg, 0.6 mmol) was weighed and added to anhydrous tetrahydrofuran (15 mL), and sodium hydride (37 mg, 0.93 mmol, 60% purity) was added in an ice-water bath. The reaction was carried out for 5 min, and then compound **478-1** (200 mg, 0.46 mmol) was added. After the addition was completed, the reaction was carried out at room temperature for 2 h. After the reaction was monitored by LCMS until completion, the reaction mixture was poured into ice water and extracted twice with ethyl acetate (20 mL). The organic phases were combined, dried over anhydrous sodium sulfate, subjected to suction filtration, and the filtrate was concentrated to obtain a crude product. The crude product was purified by normal-phase silica gel column chromatography (EA/PE = 0 to 60%) to obtain 180 mg of the title compound **480-1.** LC-MS (ESI): m/z 566.2 $[M+H]^+$.

**Preparation of compound 480-2**

Compound **480-1** (180 mg, 0.32 mmol) was weighed and dissolved in dichloromethane (5 mL). *m*-CPBA (219 mg, 1.27 mmol) was added thereto at 0°C. After the addition was completed, the reaction was carried out at room temperature for 2 h. After the reaction was monitored by LCMS until completion, the reaction was quenched with saturated aqueous sodium bicarbonate solution. Water (20 mL) was added, and the mixture was extracted twice with dichloromethane (20 mL). The organic phases were combined, dried over anhydrous sodium sulfate, subjected to suction filtration, and the filtrate was concentrated to obtain a crude product. The crude product was purified by normal-phase silica gel column chromatography (EA/PE = 0 to 60%) to obtain 120 mg of the title compound **480-2.** LC-MS (ESI): m/z 598.4 $[M+H]^+$.

**Preparation of compound 480**

Compound **480-2** (80 mg, 0.13 mmol) was added to a microwave tube, followed by the sequential addition of anhydrous acetonitrile (3 mL), dimethylphosphine oxide (31 mg, 0.40 mmol), and cesium carbonate (131 mg, 0.40 mmol). The system was purged three times with nitrogen, and the reaction was carried out under microwave irradiation at 60°C for 2 h. After the reaction was monitored by LCMS until completion, the reaction mixture was filtered. The crude filtrate was subjected to preparative purification (preparative method: mobile phase: A: 0.1% formic acid in water; B: acetonitrile; chromatographic column: Welch Ultimate AQ-C18 250 × 21.2 mm; column temperature: 25°C; gradient: 45% to 65% acetonitrile gradient elution over 12 min; flow rate: 30 mL/min) to obtain 20 mg of the title compound **480.** LC-MS (ESI): m/z 596.2 $[M+H]^+$. $^1$H NMR (400 MHz, DMSO-$d_6$) δ 8.94 (d, *J* = 5.2 Hz, 1H), 8.83 (d, *J* = 2.4 Hz, 1H), 8.50 (d, *J* = 2.5 Hz, 1H), 8.18 (dd, *J* = 8.7, 2.6 Hz, 1H), 8.04 (dd, *J* = 8.3, 2.5 Hz, 1H), 7.71 (d, *J* = 2.3 Hz, 1H), 7.67 (d, *J* = 2.3 Hz, 1H), 7.60 (dd, *J* = 5.3, 3.2 Hz, 1H), 7.44 (d, *J* = 8.3 Hz, 1H), 7.16 (d, *J* = 8.6 Hz, 1H), 5.59 (s, 2H), 4.41 (t, *J* = 5.2 Hz, 2H), 3.95 (t, *J* = 5.2 Hz, 2H), 1.74 (d, *J* = 13.6 Hz, 6H), 1.73 (s, 6H). $^{31}$P NMR (162 MHz, DMSO-$d_6$) δ 34.16 (s, 1P).

**Test Example 1: Cell Proliferation Inhibition Assay for 22RV1 and LNCaP Cells:**

1. The human prostate cancer cell lines 22RV1 and LNCaP were purchased from ATCC. The cell culture medium was RPMI-1640 + 10% FBS, and the cells were cultured in an incubator at 37°C, 100% relative humidity, and 5% $CO_2$.
2. On the first day, cells in the logarithmic growth phase were collected, counted, and resuspended in phenol red-free RPMI-1640 medium containing 10% CD-FBS. The cell concentration was adjusted to an appropriate concentration (determined based on the results of cell density optimization experiments), and the cells were seeded into a 96-well plate. A 100 μL cell suspension was added, resulting in 3000 cells per well. The cells were incubated at 37°C in a 5%

$CO_2$ incubator for 24 h.
3. On the second day, for 22Rv1 cells, test compounds at different concentrations were added, and the cells were incubated at 37°C in a 5% $CO_2$ incubator for 7 days; for LNCaP cells, test compounds at different concentrations were added first, and after 1 hour of treatment, 0.2 nM DHT was added. The cells were then incubated at 37°C in a 5% $CO_2$ incubator for 7 days.
4. After the incubation period, a volume of CellTiter-Glo detection reagent equal to the volume of the culture medium was added to each well. The plate was shaken and mixed thoroughly for 5 minutes, then incubated at room temperature for 10 minutes. Readings were taken using a PerkinElmer EnVision microplate reader.
5. The inhibition rate of the drug on cell growth was calculated according to the following formula:

$$\text{Cell growth inhibition rate } \% = [(Ac - As) / (Ac - Ab)] \times 100\%$$

As: OA of the sample (cells + test compound)
Ac: OA of the normal growth cell control (cells + DMSO)
Ab: OA of the blank control (culture medium + DMSO)

The $IC_{50}$ curve was fitted and the $IC_{50}$ value was calculated using the software GraphPad Prism 8 with the calculation formula XY-analysis/Nonlinear regression (curve fit)/Dose response-Inhibition/log (inhibitor) vs. response-Variable slope (four parameters).

**Table 1: Results of the cell proliferation inhibition** assays **of the compounds of the present disclosure in 22RV1 cells**

| Compound No. | $IC_{50}$ (μM) | Compound No. | $IC_{50}$ (μM) | Compound No. | $IC_{50}$ (μM) |
|---|---|---|---|---|---|
| 1 | 1.07 | 190 | 0.63 | 309 | 0.46 |
| 2 | 0.77 | 191 | 0.41 | 310 | 0.94 |
| 4 | 1.38 | 195 | 0.038 | 311 | 0.54 |
| 7 | 0.46 | 196 | 0.86 | 313 | 0.89 |
| 15 | 1.35 | 198 | 0.53 | 314 | 1.14 |
| 16 | 0.94 | 199 | 1.01 | 316 | 0.68 |
| 17 | 0.96 | 200 | 0.58 | 317 | 1.04 |
| 18 | 1.46 | 201 | 0.19 | 318 | 0.73 |
| 19 | 0.86 | 202 | 0.66 | 320 | 0.079 |
| 21 | 0.34 | 203 | 0.047 | 321 | 1.20 |
| 24 | 1.03 | 205 | 0.43 | 322 | 1.05 |
| 26 | 1.42 | 206 | 0.41 | 323 | 0.57 |
| 35 | 0.84 | 207 | 0.34 | 324 | 0.30 |
| 36 | 1.35 | 208 | 0.99 | 326 | 0.98 |
| 43 | 0.30 | 209 | 0.29 | 328 | 1.11 |
| 44 | 0.94 | 210 | 0.23 | 329 | 0.39 |
| 45 | 0.99 | 211 | 0.39 | 330 | 0.28 |
| 46 | 0.59 | 212 | 0.44 | 333 | 0.32 |
| 48 | 0.44 | 217 | 0.43 | 336 | 0.68 |
| 53 | 0.28 | 218 | 0.37 | 342 | 0.86 |
| 54 | 1.42 | 219 | 0.21 | 350 | 0.46 |
| 56 | 0.27 | 220 | 0.17 | 356 | 0.79 |
| 61 | 0.38 | 221 | 1.04 | 357 | 0.83 |
| 62 | 0.26 | 223 | 0.51 | 358 | 0.57 |

(continued)

| Compound No. | $IC_{50}$ (μM) | Compound No. | $IC_{50}$ (μM) | Compound No. | $IC_{50}$ (μM) |
|---|---|---|---|---|---|
| 65 | 1.18 | 224 | 1.33 | 359 | 0.23 |
| 66 | 0.60 | 230 | 0.52 | 361 | 0.25 |
| 70 | 0.50 | 234 | 0.35 | 365 | 0.93 |
| 74 | 0.29 | 235 | 0.61 | 373 | 0.64 |
| 77 | 1.47 | 237 | 0.86 | 375 | 0.12 |
| 78 | 0.71 | 239 | 1.01 | 375A | 0.089 |
| 80 | 0.11 | 240 | 0.65 | 375B | 0.12 |
| 81 | 0.86 | 241 | 0.79 | 376 | 0.27 |
| 82 | 0.86 | 242 | 0.49 | 377 | 0.38 |
| 84 | 1.05 | 242A | 0.12 | 378 | 0.17 |
| 85 | 0.34 | 242B | 0.14 | 379 | 0.68 |
| 86 | 0.63 | 243 | 1.27 | 380 | 0.23 |
| 90 | 1.32 | 245 | 0.99 | 381 | 0.35 |
| 93 | 1.01 | 250 | 0.86 | 382 | 0.86 |
| 94 | 0.49 | 252 | 0.72 | 385 | 0.57 |
| 103 | 0.69 | 253 | 1.06 | 387 | 0.22 |
| 104 | 0.92 | 254 | 0.16 | 388 | 0.37 |
| 105 | 0.30 | 255 | 0.56 | 391 | 0.46 |
| 106 | 0.19 | 258 | 0.63 | 393 | 0.88 |
| 108 | 0.06 | 260 | 0.17 | 394 | 0.11 |
| 109 | 0.12 | 261 | 0.83 | 397 | 0.95 |
| 117 | 0.68 | 262 | 0.14 | 398 | 0.51 |
| 119 | 0.74 | 264 | 0.25 | 402 | 0.66 |
| 121 | 0.19 | 265 | 0.54 | 410 | 0.88 |
| 122 | 1.28 | 266 | 0.77 | 411 | 0.79 |
| 135 | 0.23 | 267 | 0.17 | 414 | 0.94 |
| 136 | 0.23 | 269 | 0.39 | 419 | 0.13 |
| 139 | 0.65 | 270 | 0.89 | 421 | 0.35 |
| 140 | 0.53 | 272 | 0.65 | 422 | 0.69 |
| 143 | 0.90 | 274 | 0.13 | 424 | 0.66 |
| 144 | 0.74 | 275 | 0.46 | 432 | 0.84 |
| 145 | 0.60 | 276 | 0.11 | 435 | 0.18 |
| 148 | 0.77 | 277 | 1.31 | 440 | 0.99 |
| 152 | 0.33 | 280 | 1.27 | 442 | 0.38 |
| 153 | 0.78 | 281 | 0.50 | 444 | 0.12 |
| 154 | 0.26 | 282 | 0.82 | 445 | 0.13 |
| 157 | 0.48 | 283 | 0.84 | 446 | 0.31 |
| 160 | 0.23 | 285 | 0.21 | 458A | 0.18 |
| 161 | 0.45 | 286 | 0.70 | 458B | 0.32 |

(continued)

| Compound No. | $IC_{50}$ (μM) | Compound No. | $IC_{50}$ (μM) | Compound No. | $IC_{50}$ (μM) |
|---|---|---|---|---|---|
| 163 | 0.28 | 287 | 0.67 | 462 | 0.16 |
| 164 | 0.045 | 288 | 0.074 | 463 | 0.088 |
| 165 | 0.39 | 289 | 0.83 | 471 | 0.95 |
| 166 | 1.21 | 293 | 0.074 | 476 | 0.17 |
| 169 | 0.36 | 294 | 0.82 | 477 | 0.69 |
| 171 | 1.24 | 295 | 0.33 | | |
| 172 | 1.23 | 295A | 0.063 | | |
| 173 | 0.40 | 295B | 0.48 | | |
| 174 | 0.61 | 296 | 0.48 | | |
| 175 | 0.49 | 298 | 0.19 | | |
| 176 | 0.39 | 299 | 0.97 | | |
| 177 | 0.21 | 302 | 1.41 | | |
| 179 | 0.41 | 303 | 0.49 | | |
| 180 | 0.80 | 304 | 0.81 | | |
| 184 | 0.92 | 307 | 0.66 | | |
| 188 | 1.03 | 308 | 0.75 | | |

As shown by the experimental data in Table 1, the compounds of the present disclosure exhibited excellent activity in the 22RV1 cell proliferation inhibition assay.

**Table 2: Results of the cell proliferation inhibition assays of the compounds of the present disclosure in LnCaP cells**

| Compound No. | $IC_{50}$ (μM) | Compound No. | $IC_{50}$ (μM) | Compound No. | $IC_{50}$ (μM) |
|---|---|---|---|---|---|
| 62 | 0.83 | 361 | 0.40 | 424 | 0.31 |
| 85 | 0.33 | 365 | 0.55 | 426 | 0.94 |
| 94 | 0.79 | 366A | 0.97 | 427 | 0.83 |
| 108 | 0.30 | 367 | 0.91 | 432 | 0.66 |
| 109 | 0.57 | 372 | 0.66 | 435 | 0.65 |
| 119 | 0.94 | 373 | 0.25 | 439 | 0.47 |
| 136 | 0.52 | 374 | 0.92 | 440 | 0.57 |
| 164 | 0.48 | 375 | 0.059 | 441 | 0.95 |
| 179 | 0.53 | 375A | 0.058 | 442 | 0.16 |
| 195 | 0.46 | 375B | 0.10 | 444 | 0.13 |
| 203 | 0.44 | 376 | 0.10 | 445 | 0.080 |
| 206 | 0.98 | 377 | 0.10 | 446 | 0.14 |
| 209 | 0.42 | 378 | 0.061 | 455 | 0.16 |
| 234 | 0.58 | 379 | 0.12 | 457 | 0.97 |
| 242A | 0.11 | 380 | 0.15 | 458A | 0.042 |
| 242B | 0.12 | 381 | 0.11 | 458B | 0.11 |
| 254 | 0.44 | 382 | 0.41 | 459 | 0.99 |
| 260 | 0.62 | 383 | 0.58 | 460 | 0.45 |

(continued)

| Compound No. | $IC_{50}$ (μM) | Compound No. | $IC_{50}$ (μM) | Compound No. | $IC_{50}$ (μM) |
|---|---|---|---|---|---|
| 262 | 0.34 | 385 | 0.22 | 461 | 0.58 |
| 264 | 0.98 | 387 | 0.050 | 462 | 0.042 |
| 276 | 0.51 | 388 | 0.53 | 463 | 0.093 |
| 288 | 0.55 | 391 | 0.091 | 471 | 0.43 |
| 295A | 0.063 | 394 | 0.076 | 474 | 0.68 |
| 295B | 0.23 | 397 | 0.11 | 475 | 0.70 |
| 298 | 0.76 | 398 | 0.12 | 476 | 0.044 |
| 317 | 0.27 | 399 | 0.97 | 477 | 0.24 |
| 320 | 0.26 | 400 | 0.69 | 478 | 0.78 |
| 322 | 0.12 | 402 | 0.099 | 479 | 0.70 |
| 323A | 0.58 | 403 | 0.26 | | |
| 323B | 0.58 | 404 | 0.49 | | |
| 333 | 0.68 | 408 | 0.18 | | |
| 342 | 0.50 | 410 | 027 | | |
| 345 | 0.23 | 411 | 0.21 | | |
| 350A | 0.055 | 413 | 0.80 | | |
| 350B | 0.50 | 414 | 0.36 | | |
| 356 | 0.41 | 419 | 0.13 | | |
| 357 | 0.44 | 421 | 0.60 | | |
| 358 | 0.24 | 422 | 0.53 | | |
| 359 | 0.074 | 423 | 0.46 | | |

As shown by the experimental data in Table 2, the compounds of the present disclosure exhibited excellent activity in the LnCaP cell proliferation inhibition assay.

**Test Example 2: Mouse Pharmacokinetic Test**

Formulation preparation: The dosing solutions were prepared on the day of administration. 2 mg of the compound was weighed and dissolved in 3% DMSO + 1.5% Tween80 + 95.5% Saline to obtain an intravenous dosing solution with a concentration of 0.1 mg/mL; 3 mg of the compound was weighed and dissolved in 5% DMSO + 5% NMP + 10% Solutol + 80% PEG400 to obtain an oral dosing solution with a concentration of 3.0 mg/mL.

Six healthy male naive ICR mice weighing 25-30 g were selected and divided into two groups (intravenous and oral groups), with three mice in each group, and administered a single dose. After a 3-day acclimatization period, the mice were fasted overnight (10-12 h) before the experiment, with free access to water during the experiment. Feeding was resumed 4 h after administration. Timing was initiated following intravenous and oral administration. Whole blood samples were collected from the mice at the predetermined time points (IV&PO 0.083, 0.25, 0.5, 1, 2, 4, 6, 8, 24 h) into 1.5 mL EP tubes containing odium heparin. The collected whole blood was briefly vortexed twice for mixing, placed on wet ice, and centrifuged at 4°C and 8000 rpm for 5 min within 1 h. The supernatant plasma was collected and stored at -80°C until processing and analysis.

**Table 3: Pharmacokinetics in mice**

| Compound No. | Route of administration | $C_0$ (IV) or $C_{max}$ (PO) (ng/mL) | CL (mL/min/kg, IV) | $AUC_i$ (ng*hr/mL) | $T_{1/2}$ (hr) | $T_{max}$ (hr, PO) | Bioavailability (%) |
|---|---|---|---|---|---|---|---|
| 2 | IV-0.5mpk | 1203 | 1.09 | 7650 | 8.9 | / | / |
| | PO-30mpk | 31740 | / | 618691 | 12.0 | 3.3 | 134.8 |
| 6 | PO-30mpk | 62798 | / | 447415 | 2.2 | 2.7 | / |
| 7 | PO-30mpk | 12284 | / | 161135 | 5.0 | 3.3 | / |
| 10 | PO-30mpk | 31039 | / | 315824 | 5.4 | 3.3 | / |
| 16 | PO-30mpk | 36136 | / | 210922 | 1.46 | 1.67 | / |
| 17 | IV-0.5mpk | 982 | 1.10 | 7588 | 5.6 | / | / |
| | PO-30mpk | 35719 | / | 419897 | 5.82 | 4.67 | 92.2 |
| 26 | PO-30mpk | 29497 | / | 368071 | 7.81 | 3.33 | / |
| 42 | PO-30mpk | 34280 | / | 421750 | 4.42 | 3.33 | / |
| 44 | PO-30mpk | 11692 | / | 218923 | 9.35 | 4.67 | / |
| 46 | PO-30mpk | 20743 | / | 220237 | 3.58 | 4.0 | / |
| 48 | PO-30mpk | 13029 | / | 358085 | 18.03 | 4.0 | / |
| 53 | IV-0.5mpk | 2095 | 0.59 | 10243 | 33.6 | / | / |
| | PO-30mpk | 13186 | / | 420634 | 28.14 | 4.0 | 68.4 |
| 56 | IV-0.5mpk | 6188 | 1.02 | 8303 | 6.1 | / | / |
| | PO-30mpk | 29617 | / | 277015 | 8.57 | 3.33 | 55.6 |
| 61 | PO-30mpk | 15617 | / | 241586 | 7.6 | 6.0 | / |
| 62 | PO-30mpk | 19938 | / | 188471 | 2.0 | 2.0 | / |
| 70 | PO-30mpk | 14737 | / | 187044 | 5.13 | 4.0 | / |
| 82 | PO-30mpk | 24852 | / | 171343 | 8.3 | 2.7 | / |
| 85 | IV-0.5mpk | 3107 | 0.63 | 13725 | 6.12 | / | / |
| | PO-30mpk | 42989 | / | 480422 | 8.9 | 2.0 | 58.3 |
| 103 | PO-30mpk | 18124 | / | 247320 | 7.5 | 4.0 | / |
| 109 | IV-0.5mpk | 1582 | 0.56 | 15507 | 13.8 | / | / |
| | PO-30mpk | 29249 | / | 870375 | 27.1 | 4.0 | 93.6 |
| 117 | PO-30mpk | 15706 | / | 303526 | 12.1 | 4.0 | / |
| 119 | PO-30mpk | 17439 | / | 333263 | 11.8 | 4.7 | / |
| 135 | PO-30mpk | 13611 | / | 206659 | 5.6 | 6.0 | / |
| 136 | IV-0.5mpk | 3729 | 0.49 | 17122 | 14.6 | / | / |
| | PO-30mpk | 10081 | / | 241513 | 16.6 | 7.0 | 23.5 |
| 145 | PO-30mpk | 15757 | / | 273008 | 15.3 | 4.0 | / |
| 161 | PO-30mpk | 11816 | / | 375600 | 22.7 | 4.0 | / |
| 175 | PO-30mpk | 9199 | / | 320177 | 21.1 | 4.0 | / |
| 176 | PO-30mpk | 21274 | / | 406805 | 19.63 | 4.0 | / |
| 177 | PO-30mpk | 17809 | / | 557664 | 33.71 | 6.0 | / |
| 180 | PO-30mpk | 34603 | / | 308945 | 5.17 | 3.0 | / |
| 188 | PO-30mpk | 17339 | / | 211479 | 7.5 | 3.0 | / |

(continued)

| Compound No. | Route of administration | $C_0$ (IV) or $C_{max}$ (PO) (ng/mL) | CL (mL/min/kg, IV) | $AUC_i$ (ng*hr/mL) | $T_{1/2}$ (hr) | $T_{max}$ (hr, PO) | Bioavailability (%) |
|---|---|---|---|---|---|---|---|
| 191 | PO-30mpk | 25035 | / | 480955 | 13.2 | 4.0 | / |
| 202 | PO-30mpk | 8885 | / | 202270 | 18.5 | 4.0 | / |
| 206 | PO-30mpk | 32104 | / | 236367 | 1.7 | 2.0 | / |
| 207 | IV-0.5mpk | 1620 | 1.10 | 7607 | 16.1 | / | / |
| | PO-30mpk | 33936 | / | 445902 | 9.8 | 3.0 | 97.7 |
| 221 | PO-30mpk | 19251 | / | 463507 | 10.4 | 5.0 | / |
| 228 | PO-30mpk | 14516 | / | 241511 | 6.0 | 4.0 | / |
| 234 | PO-30mpk | 13401 | / | 306902 | 11.0 | 7.0 | / |
| 237 | PO-30mpk | 16217 | / | 212951 | 4.2 | 3.0 | / |
| 242 | PO-30mpk | 77969 | / | 607315 | 4.4 | 4.0 | / |
| 242A | IV-0.5mpk | 4219 | 0.64 | 13773 | 4.9 | / | / |
| | PO-30mpk | 56516 | / | 600129 | 5.70 | 4.0 | 72.6 |
| 242B | IV-0.5mpk | 2163 | 0.68 | 12346 | 5.4 | / | / |
| | PO-30mpk | 60756 | / | 739462 | 5.71 | 4.0 | 99.8 |
| 245 | PO-30mpk | 10959 | / | 181304 | 7.3 | 5.0 | / |
| 253 | PO-30mpk | 20806 | / | 298209 | 7.3 | 5.0 | / |
| 258 | PO-30mpk | 39223 | / | 323205 | 5.2 | 3.0 | / |
| 262 | IV-0.5mpk | 1789 | 0.55 | 15155 | 10.8 | / | / |
| | PO-30mpk | 74739 | / | 647552 | 5.2 | 2.0 | 71.2 |
| 272 | PO-30mpk | 53175 | / | 755276 | 5.8 | 4.0 | / |
| 276 | PO-30mpk | 13207 | / | 215532 | 9.0 | 4.0 | / |
| 283 | PO-30mpk | 18667 | / | 276981 | 9.3 | 4.0 | / |
| 289 | PO-30mpk | 10034 | / | 239937 | 10.7 | 4.0 | / |
| 308 | PO-30mpk | 14306 | / | 186103 | 4.45 | 3.0 | / |
| 375A | PO-30mpk | 109007 | / | 1307079 | 6.82 | 3.3 | / |
| 323A | PO-30mpk | 130913 | / | 1394244 | 5.94 | 3.3 | / |
| 323B | PO-30mpk | 108249 | / | 1184647 | 6.05 | 2.7 | / |
| 375B | PO-30mpk | 153425 | / | 2620877 | 8.67 | 4.0 | / |
| 376 | PO-30mpk | 131295 | / | 2300527 | 23.0 | 4.0 | / |

As shown by the experimental data in Table 3, the compounds of the present disclosure exhibited favorable pharmacokinetic results in the mouse oral PK experiment.

**Test Example 3: CYP Inhibition** Assay

An *in vitro* assay system was used to evaluate the effects of the compounds on the activities of several human liver microsomal cytochrome P450 (CYP) isozymes (CYP1A2, CYP2C9, CYP2D6, CYP3A4 (testosterone/midazolam), CYP2C19, and CYP2C8). Specific probe substrates for CYP450 isozymes were co-incubated with human liver microsomes and the compound at various concentrations (0, 0.0300, 0.100, 0.300, 1.00, 3.00, 10.0 μM). The reaction was initiated by adding the coenzyme NADPH. After the reaction was completed, the samples were processed, and the metabolites generated from the probe substrates were detected by liquid chromatography-tandem mass spectrometry

(LC-MS/MS). Based on the resulting dose-response curves, the $IC_{50}$ values of the test compounds for the probe substrate reactions catalyzed by each CYP isozyme were calculated.

Table 4: **CYP inhibition test results of partial compounds**

| CYPs (μM) / Compound No. | 1A2 | 2C9 | 2D6 | 3A4 (Testosterone) | 3A4 (Midazolam) | 2C19 | 2C8 |
|---|---|---|---|---|---|---|---|
| 2 | >10 | 6.9 | >10 | >10 | >10 | >10 | / |
| 7 | / | 7.3 | / | >10 | >10 | / | >10 |
| 46 | / | 6.7 | / | >10 | 4.5 | >10 | >10 |
| 48 | / | 5.4 | / | >10 | >10 | >10 | 1.7 |
| 53 | / | >10 | / | >10 | >10 | >10 | >10 |
| 56 | >10 | >10 | >10 | >10 | >10 | >10 | >10 |
| 61 | / | >10 | / | >10 | >10 | >10 | >10 |
| 65 | / | >10 | / | >10 | >10 | >10 | 4.3 |
| 85 | / | 6.5 | / | >10 | >10 | >10 | >10 |
| 94 | / | >10 | / | >10 | >10 | >10 | >10 |
| 109 | / | >10 | / | >10 | >10 | >10 | 8.8 |
| 136 | / | >10 | / | >10 | >10 | >10 | >10 |
| 176 | / | >10 | / | >10 | >10 | >10 | >10 |
| 177 | / | >10 | / | >10 | >10 | >10 | >10 |
| 207 | / | >10 | / | >10 | >10 | >10 | >10 |
| 242A | >10 | / | >10 | >10 | >10 | >10 | >10 |
| 242B | >10 | / | >10 | >10 | >10 | >10 | >10 |
| 262 | >10 | 8.3 | >10 | >10 | >10 | >10 | >10 |
| 276 | / | >10 | / | >10 | >10 | >10 | >10 |
| 289 | / | >10 | / | >10 | >10 | >10 | >10 |
| 295A | / | >10 | / | >10 | >10 | >10 | >10 |
| 295B | / | >10 | / | >10 | >10 | >10 | >10 |

| | | | | | | | |
|---|---|---|---|---|---|---|---|
| 302 | / | >10 | / | >10 | >10 | >10 | >10 |
| 320 | / | >10 | / | >10 | >10 | >10 | 9.1 |
| 323A | >10 | 8.0 | >10 | >10 | >10 | >10 | >10 |
| 323B | >10 | 2.9 | >10 | >10 | >10 | >10 | >10 |
| 350A | | / | / | >10 | >10 | >10 | >10 |
| 375A | / | / | / | >10 | >10 | >10 | >10 |
| 375B | / | / | / | >10 | >10 | >10 | >10 |
| 376 | / | >10 | / | >10 | >10 | >10 | >10 |
| 378 | / | / | / | >10 | >10 | >10 | >10 |
| 379 | / | >10 | / | >10 | >10 | >10 | >10 |
| 380 | / | >10 | / | >10 | >10 | >10 | >10 |
| 381 | / | 5.8 | / | >10 | >10 | >10 | >10 |
| 387 | / | 1.9 | / | >10 | >10 | >10 | >10 |
| 391 | / | 10 | / | >10 | >10 | >10 | >10 |
| 403 | / | >10 | / | >10 | >10 | >10 | 1.3 |

Conclusion: Under the test conditions, the compound of the present disclosure exhibited no inhibition or weak inhibition against these enzymes.

In the description of this specification, references to the terms "an embodiment," "some embodiments," "example," "specific example," or "some examples," *etc.,* mean that specific features, structures, materials, or characteristics described in connection with the embodiment or example are included in at least one embodiment or example of the present disclosure. In this specification, the illustrative expressions of the aforementioned terms do not necessarily refer to the same embodiment or example. Moreover, the specific features, structures, materials, or characteristics described may be combined in any suitable manner in any one or more embodiments or examples. Furthermore, without mutual contradiction, those skilled in the art may combine and integrate the different embodiments or examples described in this specification as well as the features of different embodiments or examples.

Although examples of the present disclosure have been shown and described above, it is to be understood that the above examples are illustrative and are not to be construed as limiting the present disclosure. Those of ordinary skill in the art may make variations, modifications, substitutions, and alterations to the above examples within the scope of the present disclosure.

## Claims

**1.** A compound of formula (I), an optical isomer thereof, or a pharmaceutically acceptable salt thereof,

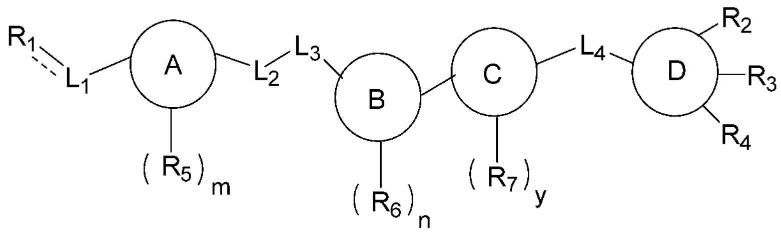

( I )

wherein

$R_1$ is selected from the group consisting of H, halogen, OH, CN, $NH_2$, $H_2N$-$S(=O)_2$-, $C_{1-6}$ alkyl, $C_{2-6}$ alkenyl, $C_{2-6}$ alkynyl, $C_{3-6}$ cycloalkyl, $C_{3-6}$ cycloalkenyl, $C_{3-6}$ cycloalkyl-$S(=O)_2$-, $C_{3-6}$ cycloalkyl-S(=O)(=NH)-, $C_{6-10}$ aryl, 5- to 10-membered heteroaryl, $C_{1-6}$ heteroalkyl, and 3- to 10-membered heterocycloalkyl, wherein the $C_{1-6}$ alkyl, $C_{2-6}$ alkenyl, $C_{2-6}$ alkynyl, $C_{3-6}$ cycloalkyl, $C_{3-6}$ cycloalkenyl, $C_{3-6}$ cycloalkyl-$S(=O)_2$-, $C_{3-6}$ cycloalkyl-S(=O)(=NH)-, $C_{6-10}$ aryl, 5- to 10-membered heteroaryl, $C_{1-6}$ heteroalkyl, and 3- to 10-membered heterocycloalkyl are each optionally substituted with 1, 2, or 3 R;

$R_2$, $R_3$, and $R_4$ are each independently selected from the group consisting of H, CN, F, Cl, Br, OH, $NH_2$, $C_{1-6}$ alkyl, $C_{3-6}$ cycloalkyl, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, 4- to 6-membered heterocycloalkyl-O-, 4- to 6-membered heterocycloalkyl-S-, and 4- to 6-membered heterocycloalkyl-NH-, wherein the $C_{1-6}$ alkyl, $C_{3-6}$ cycloalkyl, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, 4- to 6-membered heterocycloalkyl-O-, 4- to 6-membered heterocycloalkyl-S-, and 4- to 6-membered heterocycloalkyl-NH- are each optionally substituted with 1, 2, or 3 R;

$R_5$, $R_6$, and $R_7$ are each independently selected from the group consisting of H, CN, F, Cl, Br, OH, $NH_2$, C(=O)OH, $C(=O)OCH_3$, -$C(=O)NH_2$, -$C(=O)NHCH_3$, -$C(=O)N(CH_3)_2$, $C_{1-6}$ alkyl, $C_{3-6}$ cycloalkyl, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, 4- to 6-membered heterocycloalkyl-O-, 4- to 6-membered heterocycloalkyl-S-, 4- to 6-membered heterocycloalkyl-NH-, $C_{6-10}$ aryl, 5- to 10-membered heteroaryl, and 3- to 10-membered heterocycloalkyl, wherein the $C(=O)OCH_3$, -$C(=O)NH_2$, -$C(=O)NHCH_3$, -$C(=O)N(CH_3)_2$, $C_{1-6}$ alkyl, $C_{3-6}$ cycloalkyl, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, 4-to 6-membered heterocycloalkyl-O-, 4- to 6-membered heterocycloalkyl-S-, 4- to 6-membered heterocycloalkyl-NH-, $C_{6-10}$ aryl, 5- to 10-membered heteroaryl, and 3- to 10-membered heterocycloalkyl are each optionally substituted with 1, 2, or 3 R;

m, n, and y are each independently selected from the group consisting of 0, 1, 2, 3, and 4;

$L_1$ is selected from the group consisting of a single bond, -NH-, =N-, -O-, -C≡C-, -S-, - C(=O)-, -S(=O)-, -$S(=O)_2$-, -$CH_2$-, -$CH_2CH_2$-, -$OCH_2$-, $C_{3-6}$ cycloalkyl, and 3- to 10-membered heterocycloalkyl, wherein the -NH-, -$CH_2$-, -$CH_2CH_2$-, -$OCH_2$-, $C_{3-6}$ cycloalkyl, and 3- to 10-membered heterocycloalkyl are each optionally substituted with 1 or 2 R;

 is selected from the group consisting of 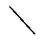 and 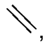, and when 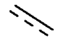 is 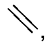, $L_1$ is selected from the group consisting of =N- and 3- to 10-membered heterocycloalkyl;

$L_2$ is selected from the group consisting of a single bond, -C=C-, -CH=CH-, -$(CR_8R_9)$x-, -O-, -S-, -C(=O)-, -S(=O)-, -$S(=O)_2$-, -$NR_{10}$-, -$C_{1-3}$ alkyl-O-, $C_{3-6}$ cycloalkyl, 4- to 10-membered heterocycloalkyl, $C_{6-10}$ aryl, and 5- to 10-membered heteroaryl, wherein the -$C_{1-3}$ alkyl-O-, $C_{3-6}$ cycloalkyl, 4- to 10-membered heterocycloalkyl, $C_{6-10}$ aryl, and 5- to 10-membered heteroaryl are each optionally substituted with 1, 2, or 3 R;

$L_3$ is selected from the group consisting of a single bond, -C=C-, -CH=CH-, -$(CR_8R_9)$x-, -O-, -S-, -C(=O)-, -S(=O)-, -$S(=O)_2$-, -$NR_{10}$-, and -$C_{1-3}$alkyl-O-, wherein the -$C_{1-3}$alkyl-O- is optionally substituted with 1, 2, or 3 R;

and, $L_2$ and $L_3$ are not simultaneously a single bond;

and, when $L_2$ is selected from the group consisting of $C_{3-6}$ cycloalkyl, 4- to 10-membered heterocycloalkyl, $C_{6-10}$ aryl, and 5- to 10-membered heteroaryl, $L_3$ is not a single bond;

$L_4$ is selected from the group consisting of -$(CR_8R_9)$x-, -O-, -S-, -C(=O)-, -S(=O)-, - $S(=O)_2$-, and -$NR_{10}$-;

$R_8$ and $R_9$ are each independently selected from the group consisting of H, CN, F, Cl, Br, OH, $NH_2$, $C_{1-6}$ alkyl, $C_{3-6}$ cycloalkyl, $C_{6-10}$ aryl, 5- to 10-membered heteroaryl, $C_{1-6}$ heteroalkyl, and 3- to 10-membered heterocycloalkyl, wherein the $C_{1-6}$ alkyl, $C_{3-6}$ cycloalkyl, $C_{6-10}$ aryl, 5-to 10-membered heteroaryl, $C_{1-6}$ heteroalkyl, and 3- to 10-membered heterocycloalkyl are each optionally substituted with 1, 2, or 3 R;

$R_{10}$ is selected from the group consisting of H, $C_{1-6}$ alkyl, $C_{3-6}$ cycloalkyl, $C_{6-10}$ aryl, 5- to 10-membered heteroaryl,

$C_{1-6}$ heteroalkyl, and 3- to 10-membered heterocycloalkyl, wherein the $C_{1-6}$ alkyl, $C_{3-6}$ cycloalkyl, $C_{6-10}$ aryl, 5- to 10-membered heteroaryl, $C_{1-6}$ heteroalkyl, and 3- to 10-membered heterocycloalkyl are each optionally substituted with 1, 2, or 3 R;

x is selected from the group consisting of 1, 2, and 3;

ring A is selected from the group consisting of $C_{4-10}$ cycloalkyl, 4- to 10-membered heterocycloalkyl, phenyl, and 5- to 10-membered heteroaryl;

ring B, ring C, and ring D are each independently selected from the group consisting of $C_{4-10}$ cycloalkyl, 4- to 10-membered heterocycloalkyl, phenyl, 5- to 10-membered heteroaryl, benzo-fused $C_{5-6}$ cycloalkyl, benzo-fused 5- to 7-membered heterocycloalkyl, 5- to 6-membered heteroaryl-fused $C_{5-6}$ cycloalkyl, and 5- to 6-membered heteroaryl-fused 5- to 6-membered heterocycloalkyl;

each R is independently selected from the group consisting of H, halogen, =O, =NR', OH, $NH_2$, CN,

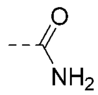

, $C_{1-6}$ alkyl, $C_{3-6}$ cycloalkyl, 3- to 6-membered heterocycloalkyl, $C_{1-6}$ alkyl-$S(=O)_2$-, ($C_{1-6}$ alkyl)$_2$-P(=O)-, $C_{1-6}$ alkyl-C(=O)-, $C_{1-6}$ alkyl-C(=O)O-, $C_{1-6}$ alkyl-O-C(=O)-, $C_{1-6}$ alkyl-C(=O)NH-, $C_{1-6}$ alkyl-NH-C(=O)-, $C_{1-6}$ alkyl-S(=O)NH-, $C_{1-6}$ alkyl-$S(=O)_2$NH-, $C_{1-6}$ alkyl-NHS$(=O)_2$-, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, $C_{3-6}$ cycloalkyl-C(=O)-, 4- to 6-membered heterocycloalkyl-O-, 4- to 6-membered heterocycloalkyl-S-, 4- to 6-membered heterocycloalkyl-NH-, and $C_{6-10}$ aryl-$C_{1-6}$ alkyl-, wherein the $C_{1-6}$ alkyl, $C_{3-6}$ cycloalkyl, 3- to 6-membered heterocycloalkyl, $C_{1-6}$ alkyl-$S(=O)_2$-, ($C_{1-6}$ alkyl)$_2$-P(=O)-, $C_{1-6}$ alkyl-C(=O)-, $C_{1-6}$ alkyl-C(=O)O-, $C_{1-6}$ alkyl-O-C(=O)-, $C_{1-6}$ alkyl-C(=O)NH-, $C_{1-6}$ alkyl-NH-C(=O)-, $C_{1-6}$ alkyl-S(=O)NH-, $C_{1-6}$ alkyl-$S(=O)_2$NH-, $C_{1-6}$ alkyl-NHS$(=O)_2$-, $C_{1-6}$ alkyl-O-, $C_{1-6}$ alkyl-S-, $C_{1-6}$ alkyl-NH-, $C_{2-6}$ alkenyl-O-, $C_{2-6}$ alkenyl-S-, $C_{2-6}$ alkenyl-NH-, $C_{3-6}$ cycloalkyl-O-, $C_{3-6}$ cycloalkyl-S-, $C_{3-6}$ cycloalkyl-NH-, $C_{3-6}$ cycloalkyl-C(=O)-, 4- to 6-membered heterocycloalkyl-O-, 4- to 6-membered heterocycloalkyl-S-, 4- to 6-membered heterocycloalkyl-NH-, and $C_{6-10}$ aryl-$C_{1-6}$ alkyl- are each optionally substituted with 1, 2, or 3 R';

R' is selected from the group consisting of H, F, Cl, Br, I, OH, $NH_2$, CN,

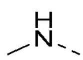 , 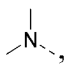 ,

$CH_3$, $CH_2F$, $CHF_2$, $CF_3$, and $C_{1-6}$ alkyl-$S(=O)_2$-;

the heteroaryl, heteroalkyl, or heterocycloalkyl comprises 1, 2, or 3 heteroatoms or heteroatomic groups independently selected from the group consisting of O, NH, S, C(=O), C(=O)O, C(=O)NH, S(=O), $S(=O)_2$, P(=O), $S(=O)_2$NH, and N.

**2.** The compound, the optical isomer thereof, or the pharmaceutically acceptable salt thereof according to claim 1, wherein each R is independently selected from the group consisting of H, halogen, OH, $NH_2$, CN,

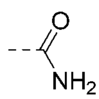 ,

=O, =NR', $C_{1-3}$ alkyl, $C_{3-6}$ cycloalkyl, 3- to 6-membered heterocycloalkyl, $C_{1-3}$ alkyl-C(=O)-, $C_{1-3}$ alkyl-$S(=O)_2$-, ($C_{1-3}$ alkyl)$_2$-P(=O)-, $C_{1-3}$ alkyl-C(=O)O-, $C_{1-3}$ alkyl-O-C(=O)-, $C_{1-3}$ alkyl-C(=O)NH-, $C_{1-3}$ alkyl-NH-C(=O)-, $C_{1-3}$ alkyl-S(=O)NH-, $C_{1-3}$ alkyl-$S(=O)_2$NH-, $C_{1-3}$ alkyl-NHS$(=O)_2$-, $C_{1-3}$ alkyl-O-, $C_{1-3}$ alkyl-S-, $C_{1-3}$ alkyl-NH-, $C_{3-6}$ cycloalkyl-C(=O)-, 4- to 6-membered heterocycloalkyl-O-, 4- to 6-membered heterocycloalkyl-S-, 4- to 6-membered heterocycloalkyl-NH-, and $C_{6-10}$ aryl-$C_{1-3}$ alkyl-, wherein the $C_{1-3}$ alkyl, $C_{3-6}$ cycloalkyl, 3- to 6-membered heterocycloalkyl, $C_{1-3}$ alkyl-C(=O)-, $C_{1-3}$ alkyl-$S(=O)_2$-, ($C_{1-3}$ alkyl)$_2$-P(=O)-, $C_{1-3}$ alkyl-C(=O)O-, $C_{1-3}$ alkyl-O-C(=O)-, $C_{1-3}$ alkyl-C(=O)NH-, $C_{1-3}$ alkyl-NH-C(=O)-, $C_{1-3}$ alkyl-S(=O)NH-, $C_{1-3}$ alkyl-$S(=O)_2$NH-, $C_{1-3}$ alkyl-NHS$(=O)_2$-, $C_{1-3}$ alkyl-O-, $C_{1-3}$ alkyl-S-, $C_{1-3}$ alkyl-NH-, $C_{3-6}$ cycloalkyl-C(=O)-, 4- to 6-membered heterocycloalkyl-O-, 4- to 6-membered heterocycloalkyl-S-, 4- to 6-membered heterocycloalkyl-NH-, $C_{6-10}$ aryl-$C_{1-3}$ alkyl-, and $C_{6-10}$ aryl-$C_{1-3}$ alkyl- are each optionally substituted with 1, 2, or 3 R'.

**3.** The compound, the optical isomer thereof, or the pharmaceutically acceptable salt thereof according to claim 2,

wherein each R is independently selected from the group consisting of H, F, Cl, Br, I, OH, $NH_2$, CN, =O, =NH, =N-CN,

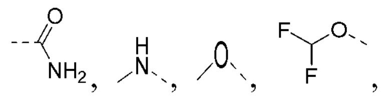,

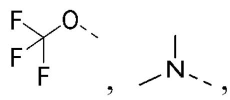,

$CH_3$, $CH_2F$, $CHF_2$, $CF_3$,

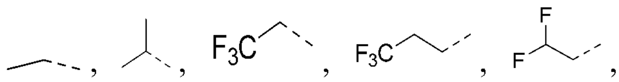,

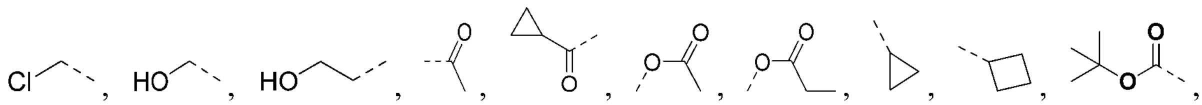,

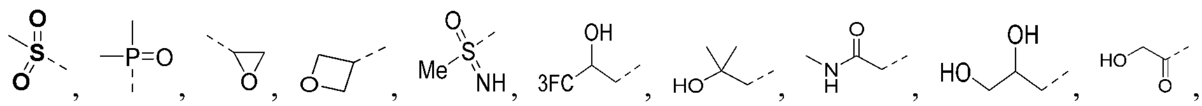,

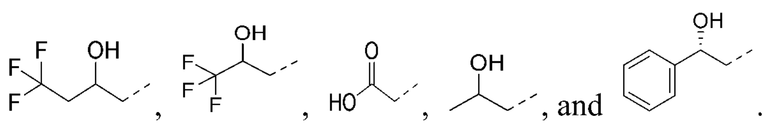.

4. The compound, the optical isomer thereof, or the pharmaceutically acceptable salt thereof according to any one of claims 1 to 3, wherein $R_1$ is selected from the group consisting of H, halogen, OH, CN, $NH_2$, $H_2N$-S(=O)$_2$-, $C_{1-6}$ alkyl, $C_{2-6}$ alkenyl, $C_{2-6}$ alkynyl, $C_{1-6}$ alkyl-O-, $C_{1-6}$ alkyl-S-, $C_{1-6}$ alkyl-S(=O)$_2$-, ($C_{1-6}$ alkyl)$_2$-S(=O)=, ($C_{1-6}$ alkyl)$_2$-P(=O)-, ($C_{1-6}$ alkyl)$_2$-S(=O)=N-, $C_{1-6}$ alkyl-S(=O)$_2$NH-, $C_{1-6}$ alkyl-NHS(=O)$_2$-, $C_{1-6}$ alkyl-NH-, $C_{1-6}$ alkyl-S(=O)(=NH)-, $C_{3-6}$ cycloalkyl, $C_{3-6}$ cycloalkenyl, $C_{3-6}$ cycloalkyl-S(=O)$_2$-, $C_{3-6}$ cycloalkyl-S(=O)(=NH)-, $C_{6-10}$ aryl, 5- to 10-membered heteroaryl, and 3- to 10-membered heterocycloalkyl, wherein the $C_{1-6}$ alkyl, $C_{2-6}$ alkenyl, $C_{2-6}$ alkynyl, $C_{1-6}$ alkyl-O-, $C_{1-6}$ alkyl-S-, $C_{1-6}$ alkyl-S(=O)$_2$-, ($C_{1-6}$ alkyl)$_2$-S(=O)=, ($C_{1-6}$ alkyl)$_2$-P(=O)-, ($C_{1-6}$ alkyl)$_2$-S(=O)=N-, $C_{1-6}$ alkyl-S(=O)$_2$NH-, $C_{1-6}$ alkyl-NHS(=O)$_2$-, $C_{1-6}$ alkyl-NH-, $C_{1-6}$ alkyl-S(=O)(=NH)-, $C_{3-6}$ cycloalkyl, $C_{3-6}$ cycloalkenyl, $C_{3-6}$ cycloalkyl-S(=O)$_2$-, $C_{3-6}$ cycloalkyl-S(=O)(=NH)-, $C_{6-10}$ aryl, 5- to 10-membered heteroaryl, and 3- to 10-membered heterocycloalkyl are each optionally substituted with 1, 2, or 3 R.

5. The compound, the optical isomer thereof, or the pharmaceutically acceptable salt thereof according to claim 4, wherein $R_1$ is selected from the group consisting of H, F, Cl, Br, I, Me,

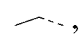,

CN, OH, $NH_2$, $H_2N$-S(=O)$_2$-,

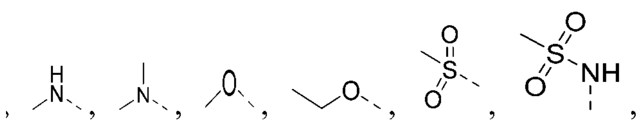,

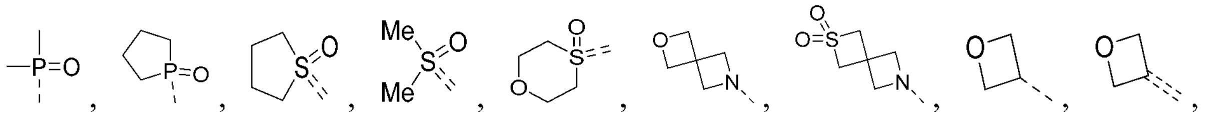

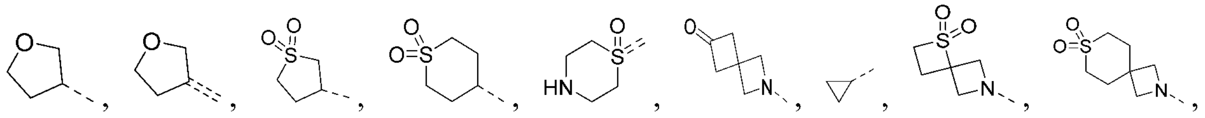

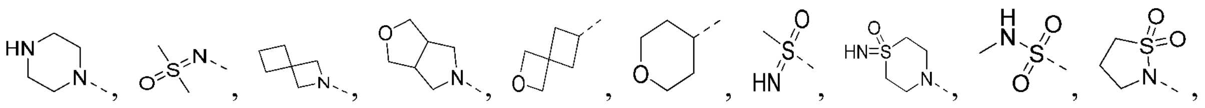

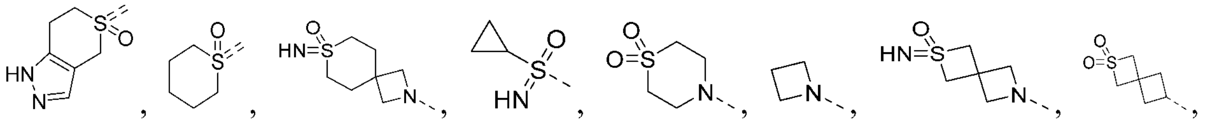

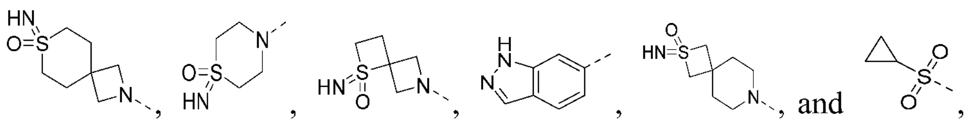
and

wherein the

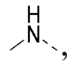

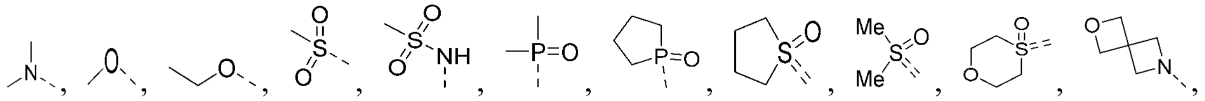

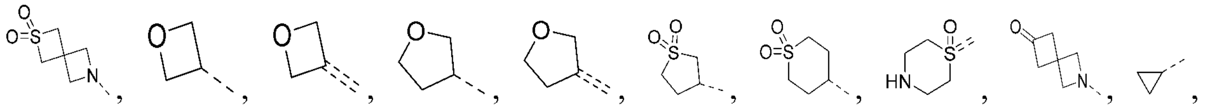

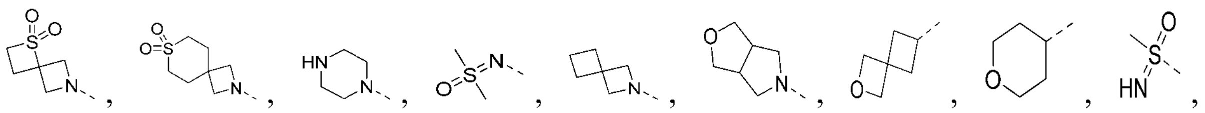

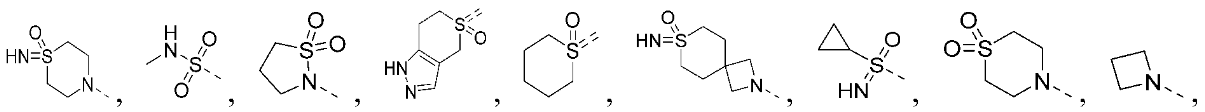

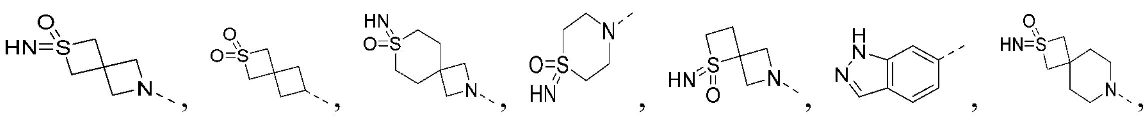

and

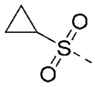

are each optionally substituted with 1, 2, or 3 R.

**6.** The compound, the optical isomer thereof, or the pharmaceutically acceptable salt thereof according to claim 5, wherein $R_1$ is selected from the group consisting of H, F, Cl, Br, I, Me,

,

CN, OH, $NH_2$, $H_2N\text{-}S(=O)_2\text{-}$,

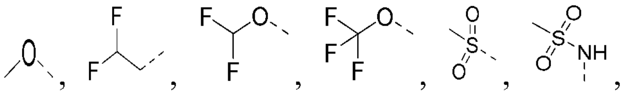

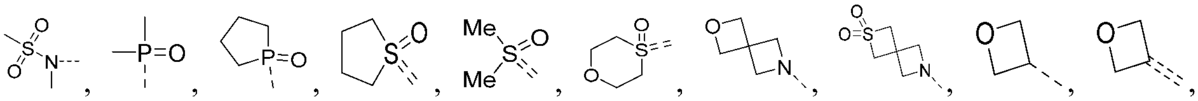

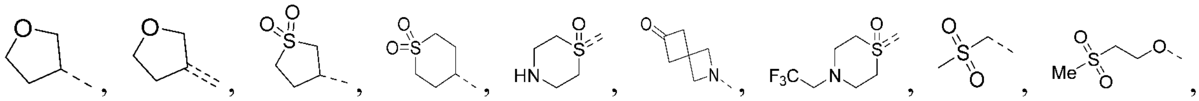

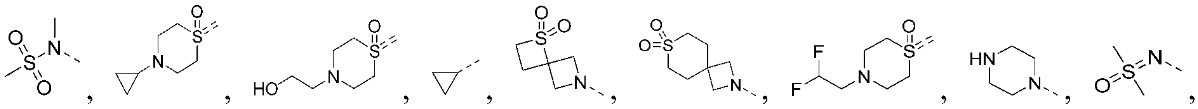

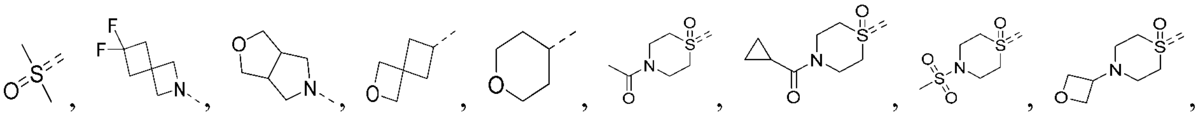

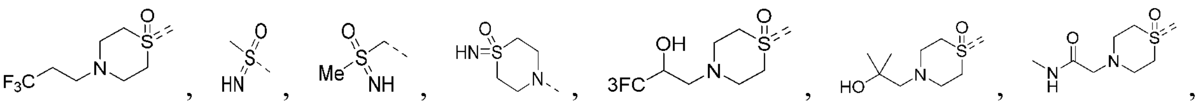

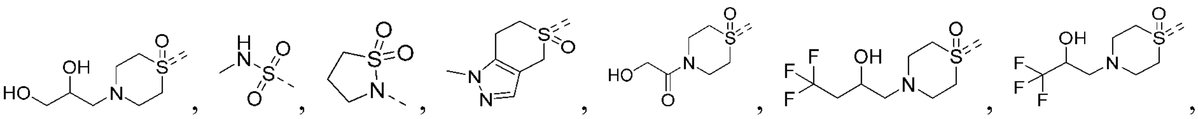

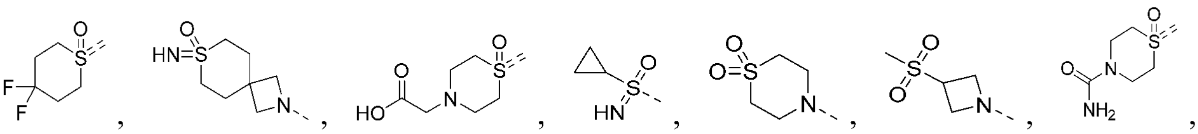

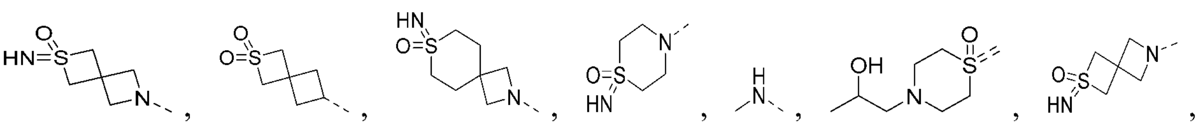

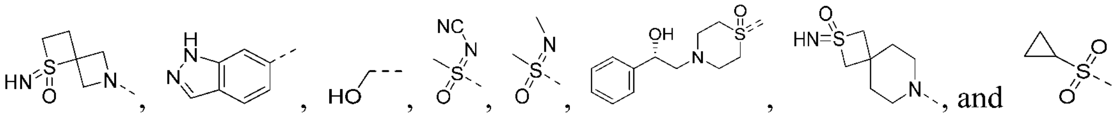, and .

**7.** The compound, the optical isomer thereof, or the pharmaceutically acceptable salt thereof according to any one of claims 1 to 3, wherein $L_1$ is selected from the group consisting of a single bond, $-CH_2-$, -NH-, =N-, -O-, -C≡C-, -S-, -C(=O)-, -S(=O)-, $-S(=O)_2-$,

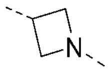,

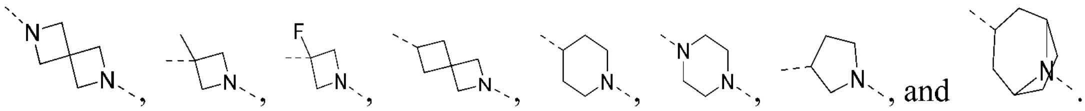, and .

**8.** The compound, the optical isomer thereof, or the pharmaceutically acceptable salt thereof according to any one of claims 1 to 3, wherein the structural moiety

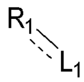

is selected from the group consisting of H, F, Cl, Br, I, $H_2N-S(=O)_2-$,

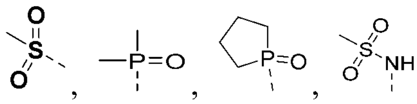,

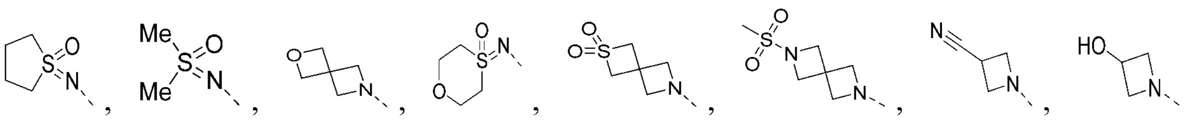,

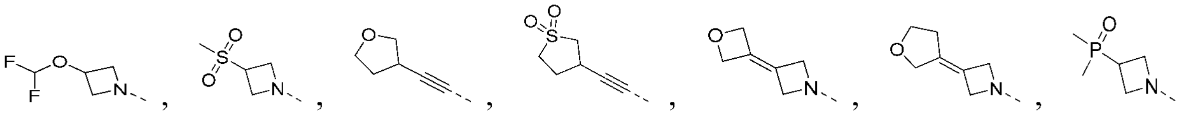,

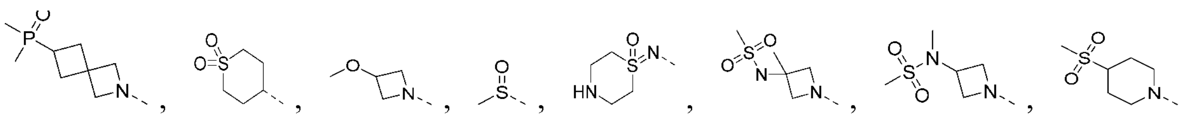,

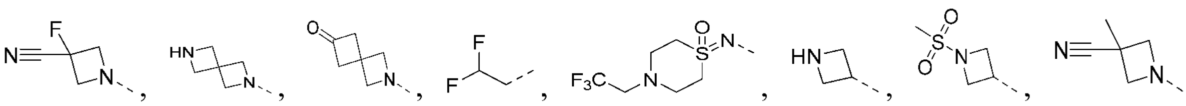,

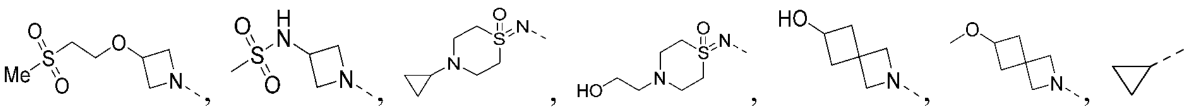,

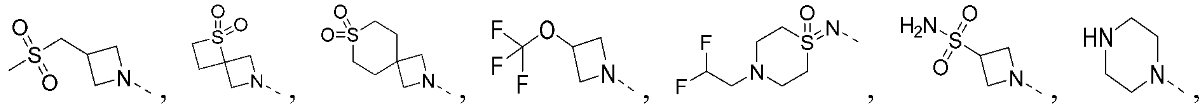

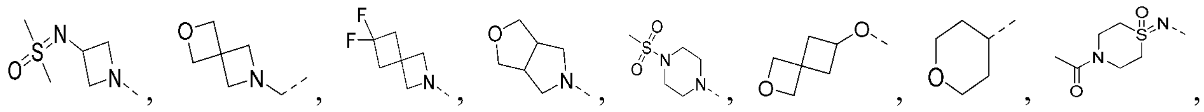

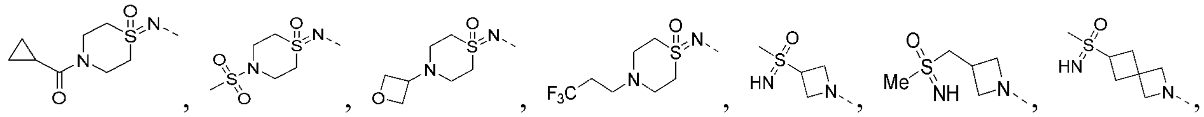

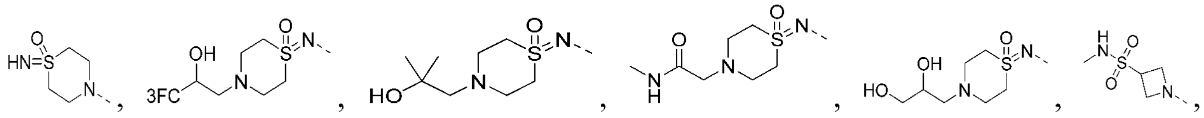

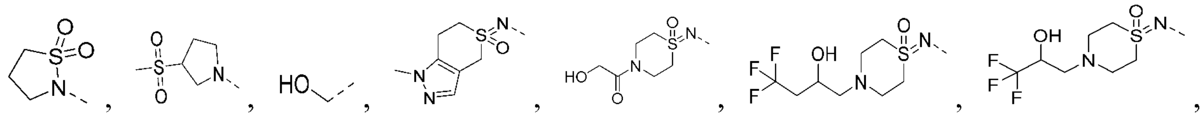

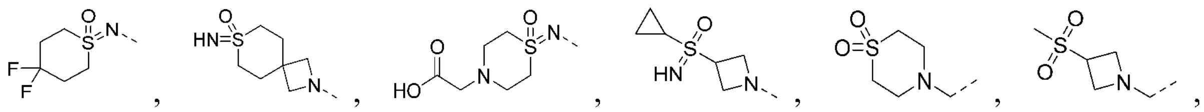

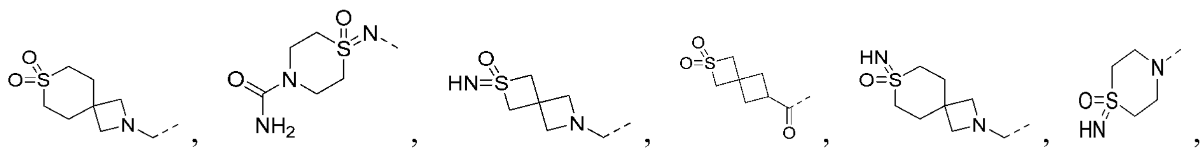

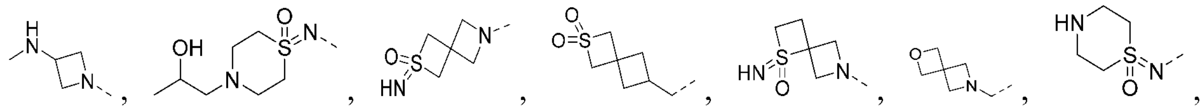

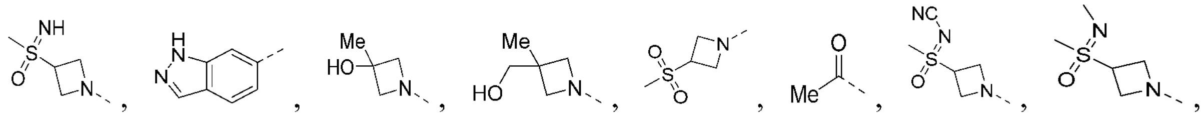

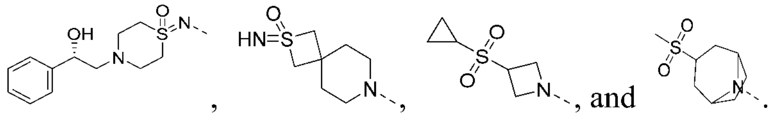
and .

9. The compound, the optical isomer thereof, or the pharmaceutically acceptable salt thereof according to any one of claims 1 to 3, wherein $R_2$, $R_3$, and $R_4$ are each independently selected from the group consisting of H, CN, F, Cl, Br, OH, $NH_2$, $C_{1-3}$ alkyl, $C_{1-3}$ alkyl-O-, $C_{1-3}$ alkyl-S-, $C_{1-3}$ alkyl-NH-, $C_{2-3}$ alkenyl-O-, $C_{2-3}$ alkenyl-S-, $C_{2-3}$ alkenyl-NH-, $C_{3-6}$ cycloalkyl-O-, $C_{3-6}$ cycloalkyl-S-, $C_{3-6}$ cycloalkyl-NH-, oxiranyl-O-, and azetidinyl-O-, wherein the $C_{1-3}$ alkyl, $C_{1-3}$ alkyl-O-, $C_{1-3}$ alkyl-S-, $C_{1-3}$ alkyl-NH-, $C_{2-3}$ alkenyl-O-, $C_{2-3}$ alkenyl-S-, $C_{2-3}$ alkenyl-NH-, $C_{3-6}$ cycloalkyl-O-, $C_{3-6}$ cycloalkyl-S-, $C_{3-6}$ cycloalkyl-NH-, oxiranyl-O-, and azetidinyl-O- are each optionally substituted with 1, 2, or 3 R.

**10.** The compound, the optical isomer thereof, or the pharmaceutically acceptable salt thereof according to claim 9, wherein $R_2$, $R_3$, and $R_4$ are each independently selected from the group consisting of H, CN, F, Cl, Br, OH, $NH_2$, CN, Me,

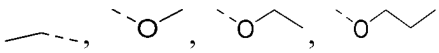,

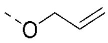, 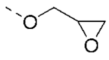, 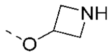, 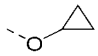, and 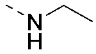,

wherein the Me,

, 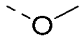,

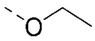, 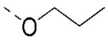, 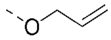, 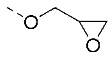, 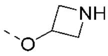, 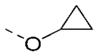, and 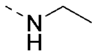

are each optionally substituted with 1, 2, or 3 R.

**11.** The compound, the optical isomer thereof, or the pharmaceutically acceptable salt thereof according to claim 10, wherein $R_2$, $R_3$, and $R_4$ are each independently selected from the group consisting of H, CN, F, Cl, Br, OH, $NH_2$, CN, Me,

, 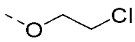, 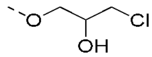,

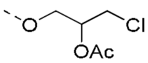, 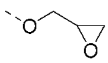, 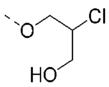, 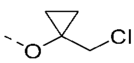, 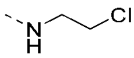, 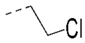, 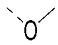, 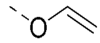, 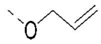,

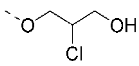, 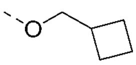, 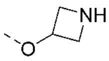, 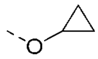, 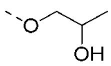, and 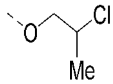.

**12.** The compound, the optical isomer thereof, or the pharmaceutically acceptable salt thereof according to any one of claims 1 to 3, wherein $R_5$, $R_6$, and $R_7$ are each independently selected from the group consisting of H, CN, F, Cl, Br, OH, $NH_2$, -COOH, $-COOCH_3$, - C(=O)$NH_2$, -C(=O)$NHCH_3$, -C(=O)N$(CH_3)_2$, $C_{1-3}$ alkyl, $C_{1-3}$ alkyl-O-, $C_{1-3}$ alkyl-S-, $C_{1-3}$ alkyl-NH-, $C_{2-3}$ alkenyl-O-, $C_{2-3}$ alkenyl-S-, $C_{2-3}$ alkenyl-NH-, $C_{3-6}$ cycloalkyl-O-, $C_{3-6}$ cycloalkyl-S-, $C_{3-6}$ cycloalkyl-NH-, and oxiranyl-O-, wherein the $C_{1-3}$ alkyl, $C_{1-3}$ alkyl-O-, $C_{1-3}$ alkyl-S-, $C_{1-3}$ alkyl-NH-, $C_{2-3}$ alkenyl-O-, $C_{2-3}$ alkenyl-S-, $C_{2-3}$ alkenyl-NH-, $C_{3-6}$ cycloalkyl-O-, $C_{3-6}$ cycloalkyl-S-, $C_{3-6}$ cycloalkyl-NH-, and oxiranyl-O- are each optionally substituted with 1, 2, or 3 R.

**13.** The compound, the optical isomer thereof, or the pharmaceutically acceptable salt thereof according to claim 12, wherein $R_5$, $R_6$, and $R_7$ are each independently selected from the group consisting of H, CN, F, Cl, Br, OH, $NH_2$, CN, Me,

,

$CH_2F$, $CHF_2$, $CF_3$, COOH, - COOMe,

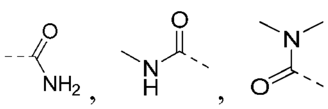, 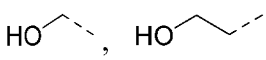, 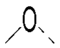, 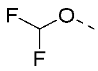, 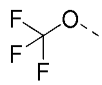, 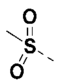,

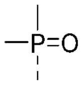 , and 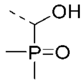 .

**14.** The compound, the optical isomer thereof, or the pharmaceutically acceptable salt thereof according to any one of claims 1 to 3, wherein $R_8$ and $R_9$ are each independently selected from the group consisting of H, CN, F, Cl, Br, OH, $NH_2$, Me, and

 .

**15.** The compound, the optical isomer thereof, or the pharmaceutically acceptable salt thereof according to any one of claims 1 to 3, wherein $R_{10}$ is selected from the group consisting of H, Me,

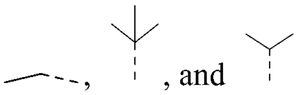 , , and .

**16.** The compound, the optical isomer thereof, or the pharmaceutically acceptable salt thereof according to any one of claims 1 to 3, wherein $L_2$ is selected from the group consisting of a single bond, $-CH_2-$, $-CH(CH_3)-$, $-OCH_2-$, -C≡C-, -CH=CH-, -O-, -S-, -C(=O)-, -S(=O)-, - $S(=O)_2-$, -NH-,

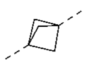 , 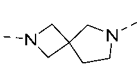 , 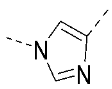 , 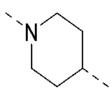 , 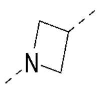 , 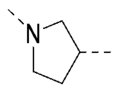 , 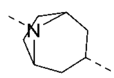 ,

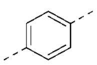 , 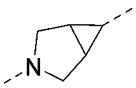 , 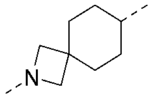 , 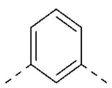 , 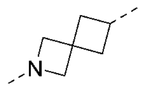 , 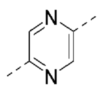 , 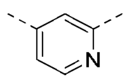 , 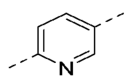 ,

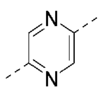 , and 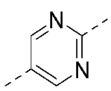 .

**17.** The compound, the optical isomer thereof, or the pharmaceutically acceptable salt thereof according to any one of claims 1 to 3, wherein $L_3$ is selected from the group consisting of a single bond, $-CH_2-$, $-CH(CH_3)-$, $-OCH_2-$, -C≡C-, -CH=CH-, -O-, -S-, -C(=O)-, -S(=O)-, - $S(=O)_2-$, and -NH-.

**18.** The compound, the optical isomer thereof, or the pharmaceutically acceptable salt thereof according to any one of claims 1 to 3, wherein $L_2$-$L_3$ is selected from the group consisting of $-CH_2-$, $-CH_2CH_2-$, $-CH(CH_3)-$, -C≡C-, -CH=CH-, -O-, $-OCH_2-$, $-OCH_2CH_2-$, - $OCH(CH_3)-$, $-OCH_2OCH_2-$, $-OCH_2CH_2O-$, -S-, -C(=O)-, -C(=O)O-, -S(=O)-, $-S(=O)_2-$, -NH-, $-CH_2NH-$,

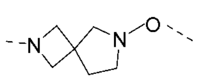 , 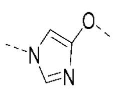 , 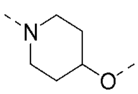 , 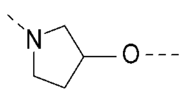 , 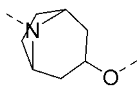 , 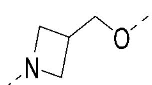 ,

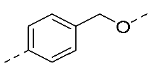 , 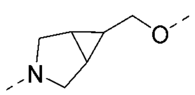 , 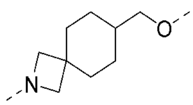 , 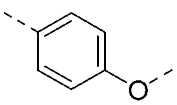 , 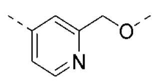 , 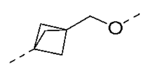 ,

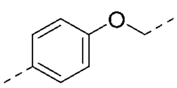 , 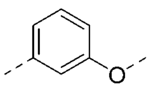 , 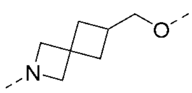 , 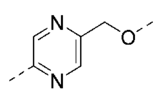 , 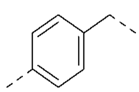 , 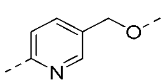 ,

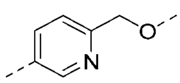, and 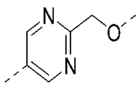.

**19.** The compound, the optical isomer thereof, or the pharmaceutically acceptable salt thereof according to any one of claims 1 to 3, wherein $L_4$ is selected from the group consisting of $-CH_2-$, -O-, -S-, -C(=O)-, -S(=O)-, $-S(=O)_2-$, -NH-,

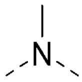, 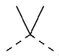, 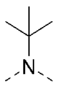, 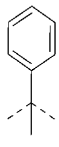, and 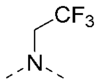.

**20.** The compound, the optical isomer thereof, or the pharmaceutically acceptable salt thereof according to any one of claims 1 to 3, wherein ring A is selected from the group consisting of phenyl, pyridinyl, pyrimidinyl, pyrazinyl, thiazolyl, thienyl, oxazolyl, pyridazinyl, cyclobutyl, oxetanyl, tetrahydropyranyl, 2-oxaspiro[3.3]heptanyl, 2,2-dioxo-2-thia-6-azaspiro[3.3]heptyl, tetrahydrofuranyl, azetidinyl, piperidinyl, 1,1-dioxothiomorpholinyl, 2,2-dioxo-2-thia-6-azaspiro[3.3]heptyl, 2-oxo-2-imino-2λ$^6$-thiaspiro[3.3]heptyl, cyclohexyl, and 1-oxo-1-imino-1λ$^6$-thiomorpholinyl.

**21.** The compound, the optical isomer thereof, or the pharmaceutically acceptable salt thereof according to claim 20, wherein the structural moiety

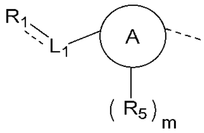

is selected from the group consisting of

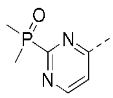, 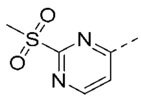, 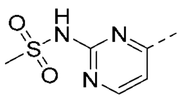, 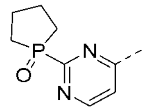, 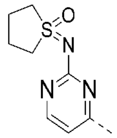,

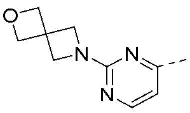, 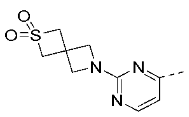, 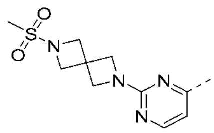, 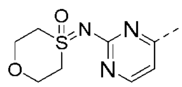, 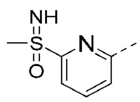, 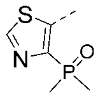,

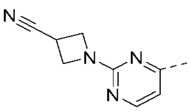, 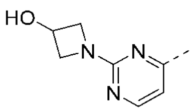, 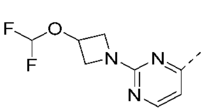, 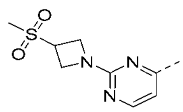, 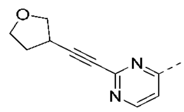,

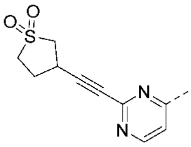, 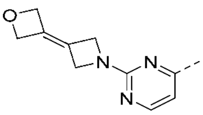, 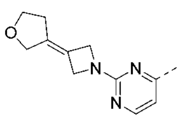, 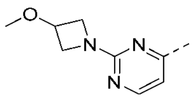, 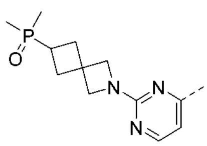, 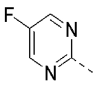,

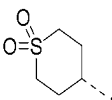, 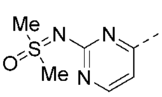, 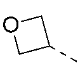, 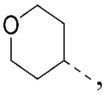, 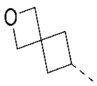, 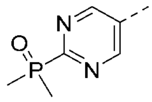, 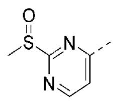, 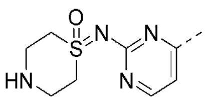,

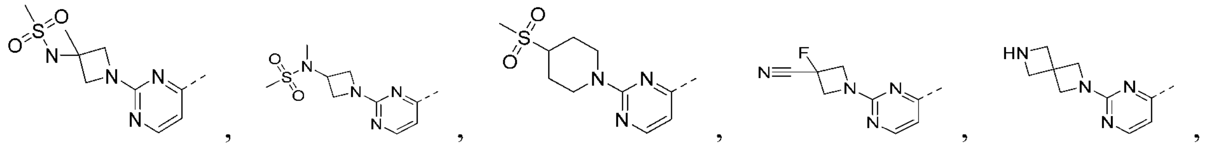
, , , , ,

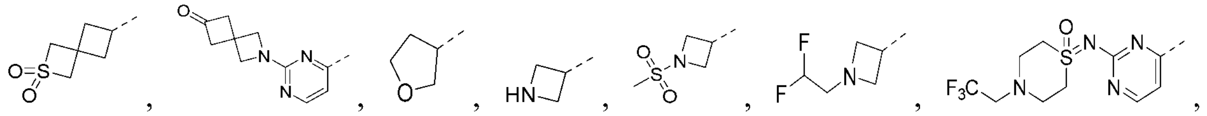
, , , , , , ,

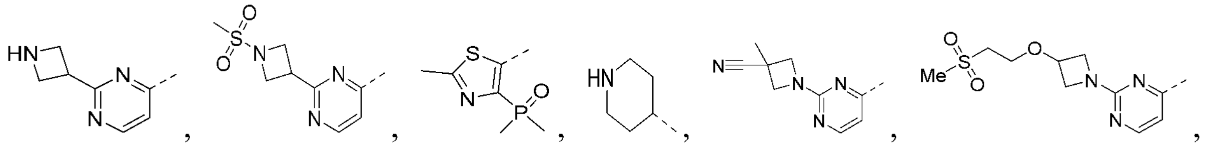
, , , , , ,

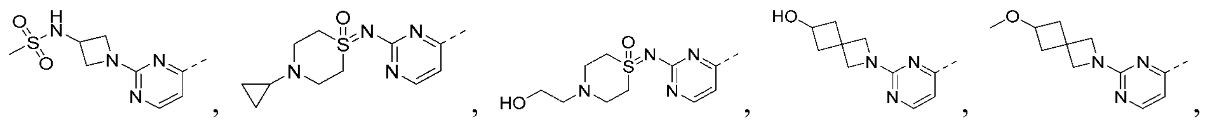
, , , , ,

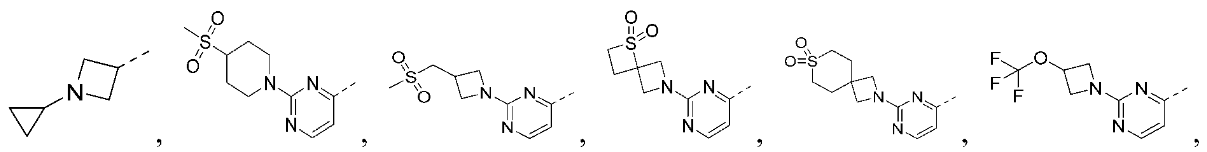
, , , , , ,

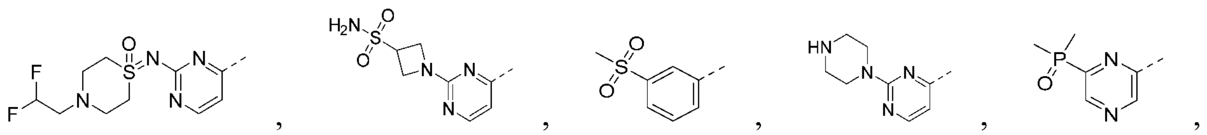
, , , , ,

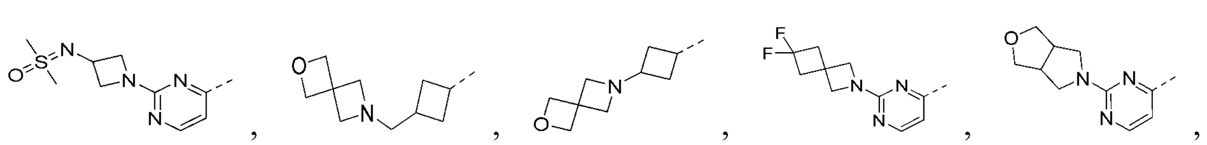
, , , , ,

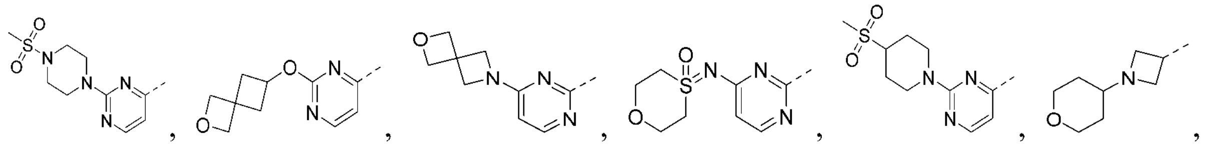
, , , , , ,

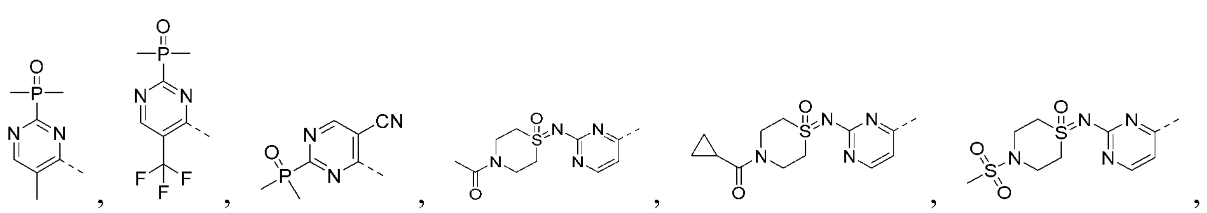
, , , , , ,

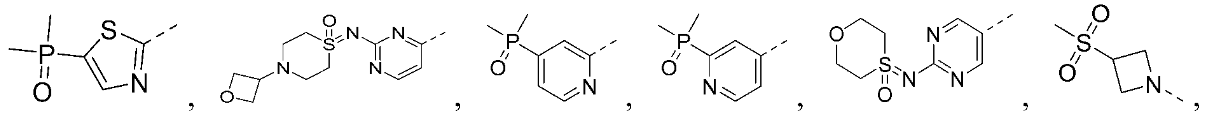
, , , , , ,

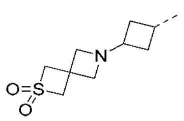 , 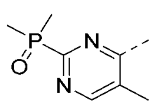 , 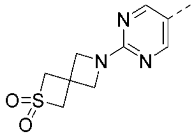 , 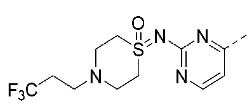 , 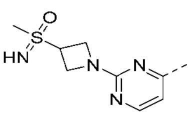 ,

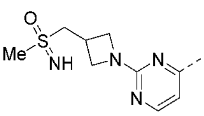 , 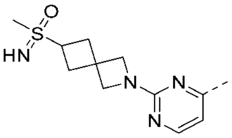 , 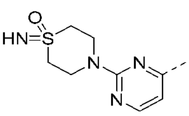 , 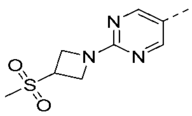 , 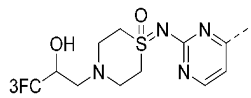 ,

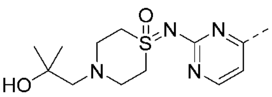 , 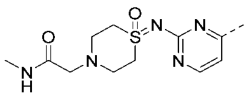 , 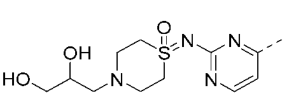 , 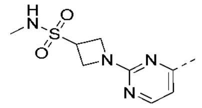 ,

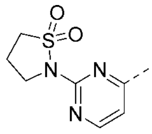 , 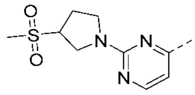 , 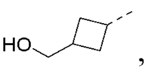 , 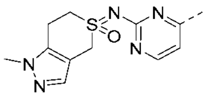 , 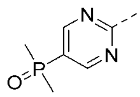 , 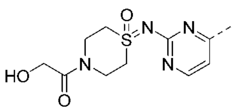 ,

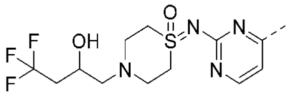 , 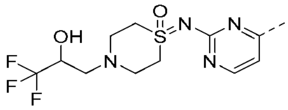 , 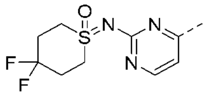 , 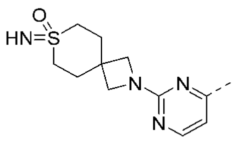 ,

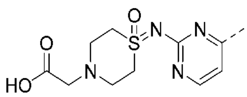 , 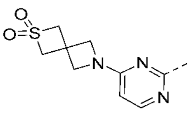 , 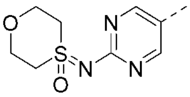 , 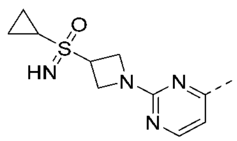 , 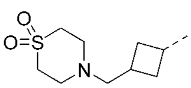 ,

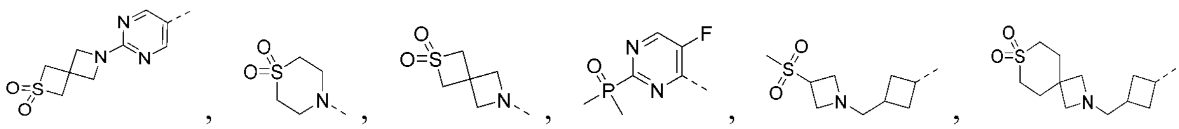
, , , , , ,

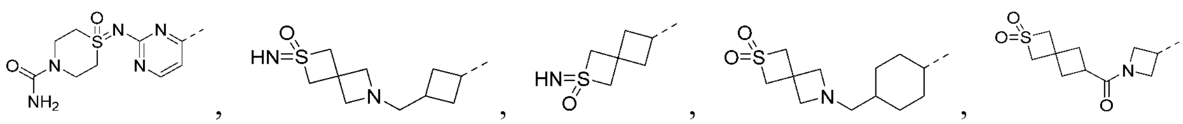
, , , , ,

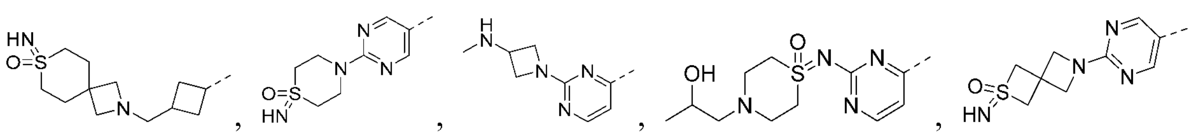
, , , , ,

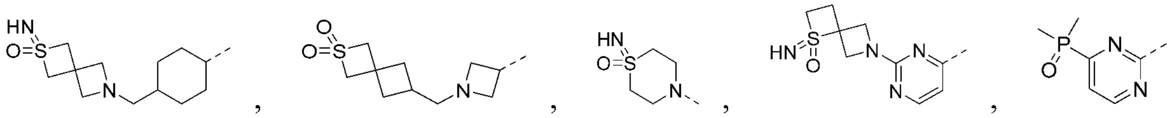
, , , , ,

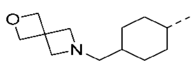, 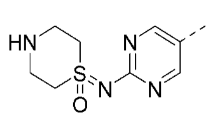, 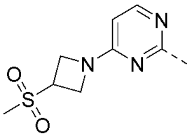, 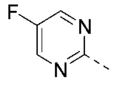, 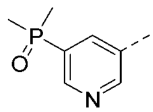, 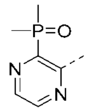,

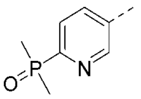, 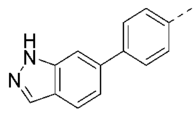, 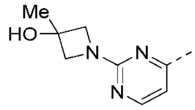, 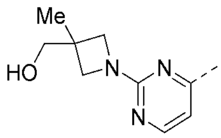, 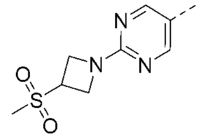,

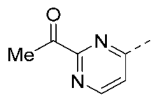, 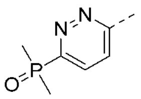, 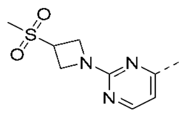, 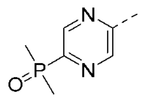, 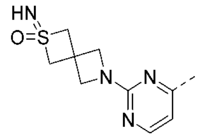, 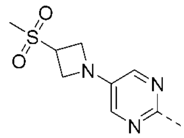,

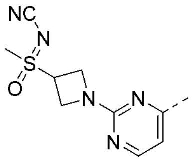, 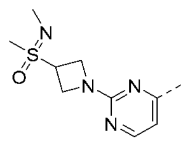, 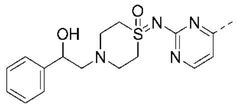, 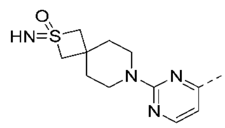, 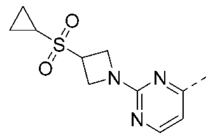,

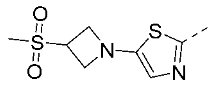, and 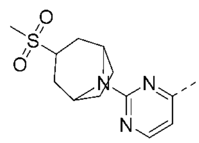.

**22.** The compound, the optical isomer thereof, or the pharmaceutically acceptable salt thereof according to any one of claims 1 to 3, wherein ring B is selected from the group consisting of bicyclo[1.1.1]pentyl, cyclobutyl, cyclopentyl, 2,6-diazaspiro[3.3]heptyl, pyrazolyl, piperidinyl, thiazolyl, phenyl, pyridinyl, pyrimidinyl, pyridazinyl, pyrazinyl, oxazolyl, benzocyclopentyl, benzocyclohexyl, 1,2,3,4-tetrahydroquinolinyl, naphthyl, indolyl, isoindolinyl, spiro[cyclopropane-1,3'-indolin]-2'-one, 3(2H)-pyridazinone, 2(1H)-pyridinone, isoquinolinyl, and quinolin-2(1H)-one.

**23.** The compound, the optical isomer thereof, or the pharmaceutically acceptable salt thereof according to claim 22, wherein the structural moiety

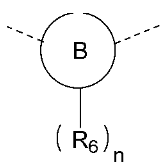

is selected from the group consisting of

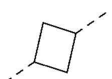, 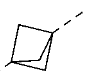, 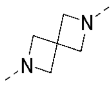, 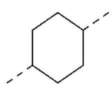, 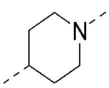, 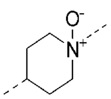, 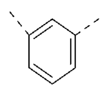,

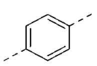, 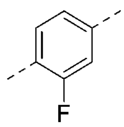, 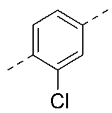, 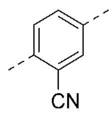, 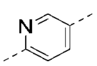, 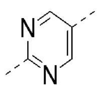, 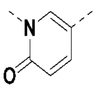, 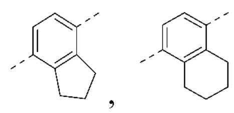,

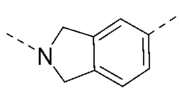, 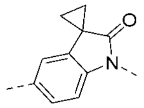, 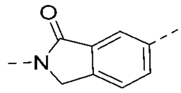, 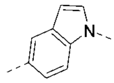, 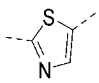, 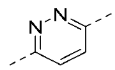, 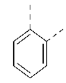, 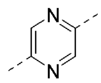,

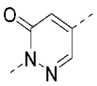, 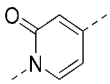, 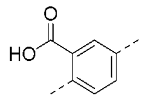, 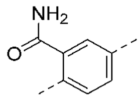, 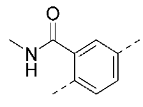, 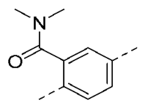, 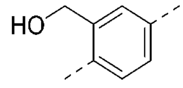,

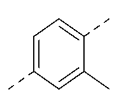, 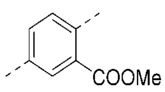, 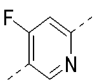, 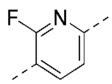, 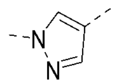, 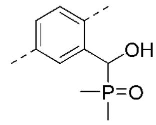, 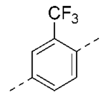, 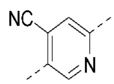,

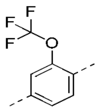, 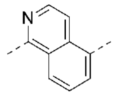, 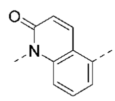, 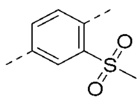, 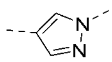, 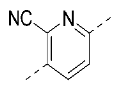, 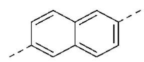, 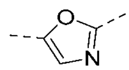,

and

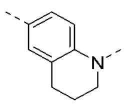.

**24.** The compound, the optical isomer thereof, or the pharmaceutically acceptable salt thereof according to any one of claims 1 to 3, wherein ring C is selected from the group consisting of azetidinyl, phenyl, pyridinyl, pyrimidinyl, pyridazinyl, pyrazinyl, indolyl, and spiro[cyclopropane-1,3'-indolin]-2'-one.

**25.** The compound, the optical isomer thereof, or the pharmaceutically acceptable salt thereof according to claim 24, wherein the structural moiety

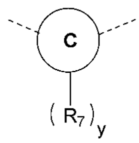

is selected from the group consisting of

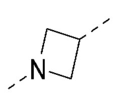, 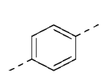, 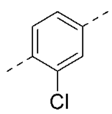, 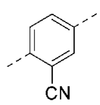, 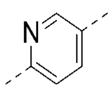, 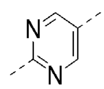, 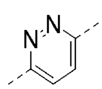,

, , , and .

**26.** The compound, the optical isomer thereof, or the pharmaceutically acceptable salt thereof according to any one of claims 1 to 3, wherein ring D is selected from the group consisting of phenyl, pyridinyl, benzocyclopentyl, benzocyclohexyl, 1H-indazolyl, 2H-indazolyl, and 1H-benzo[d]imidazolyl.

**27.** The compound, the optical isomer thereof, or the pharmaceutically acceptable salt thereof according to claim 11 or 26,

wherein the structural moiety

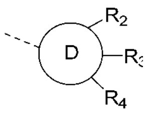

is selected from the group consisting of

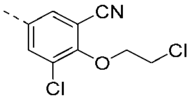, 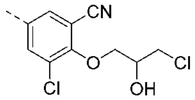, 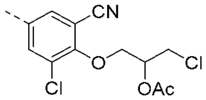, 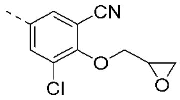,

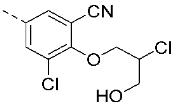, 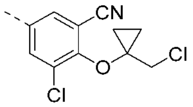, 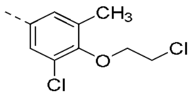, 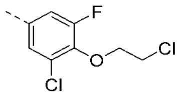, 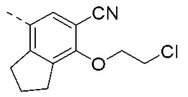,

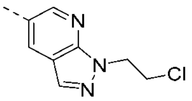, 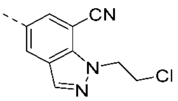, 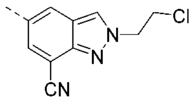, 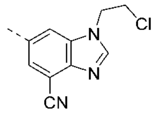, 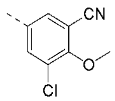, 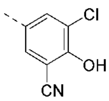,

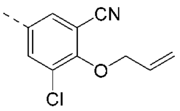, 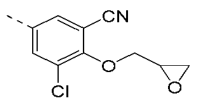, 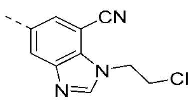, 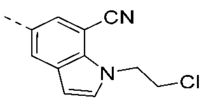, 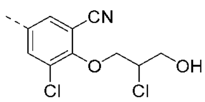,

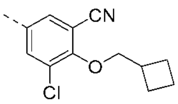, 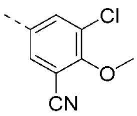, 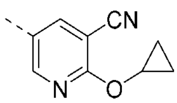, 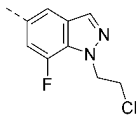, 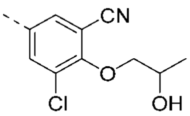, 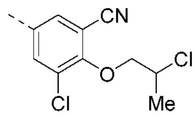,

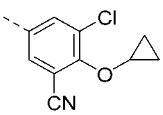, 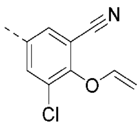, and 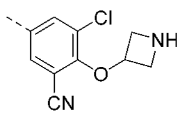.

**28.** A compound of the following formula, an optical isomer thereof, or a pharmaceutically acceptable salt thereof, selected from the group consisting of:

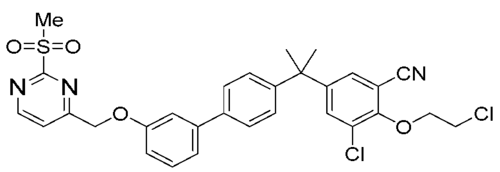

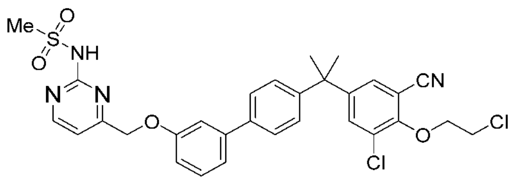

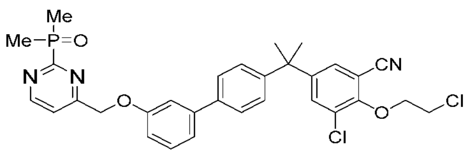

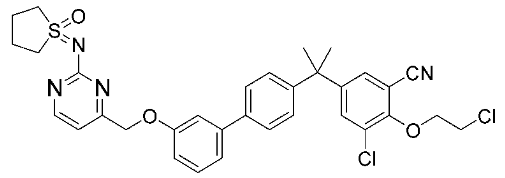

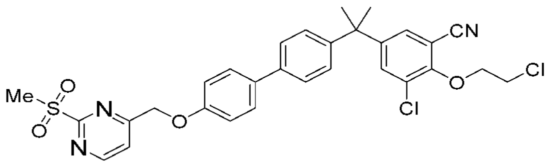

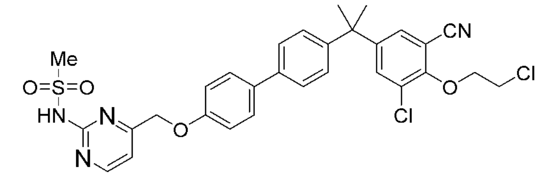

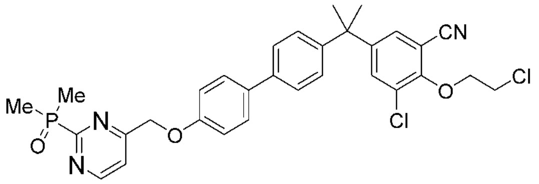

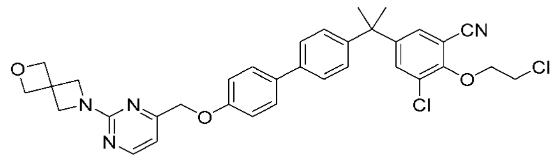

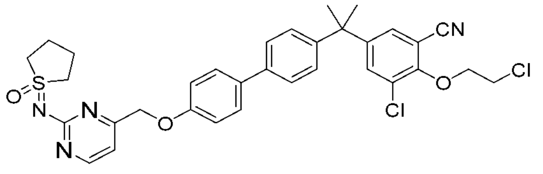

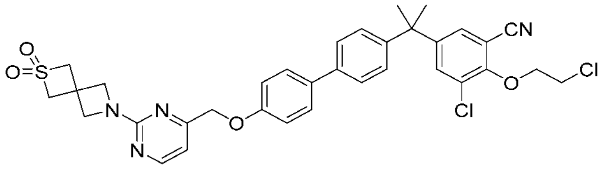

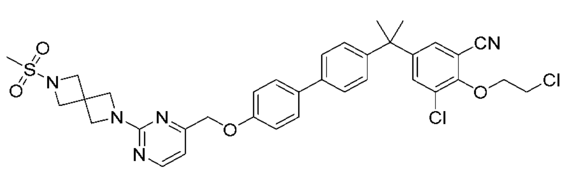

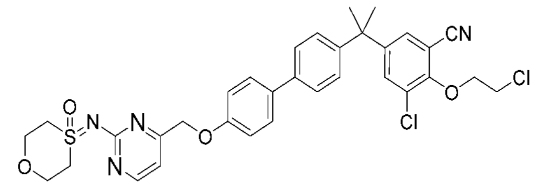

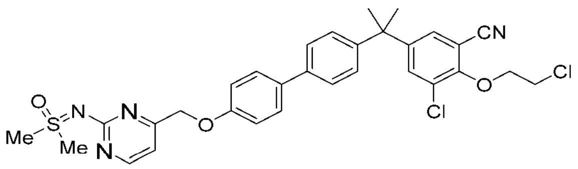

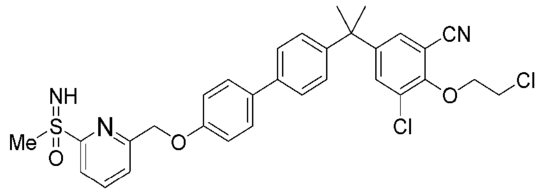

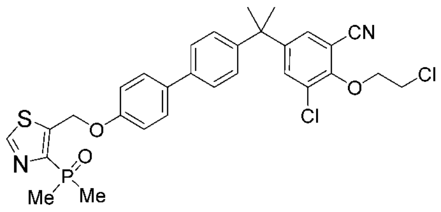

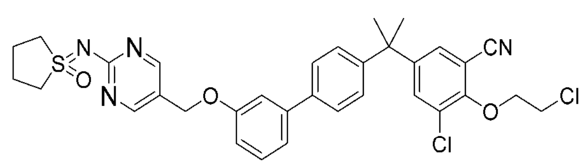

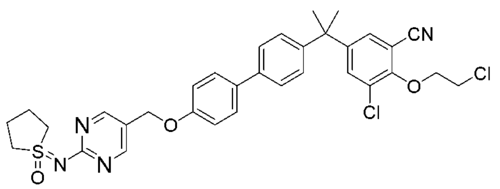

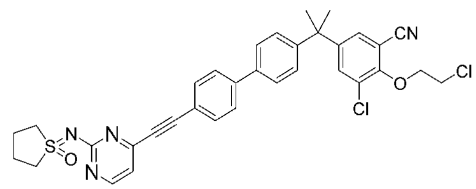

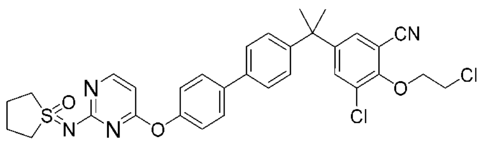

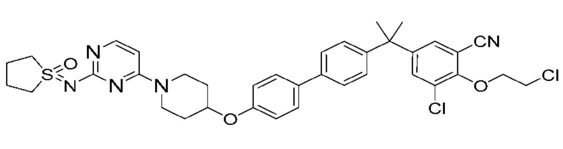

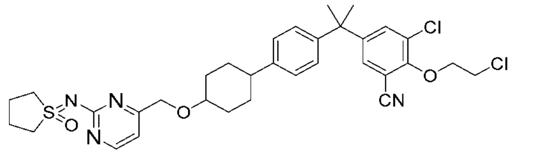

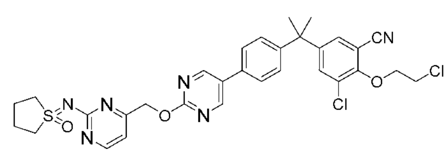

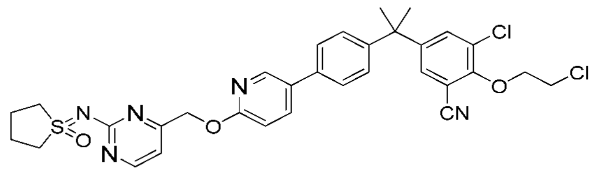

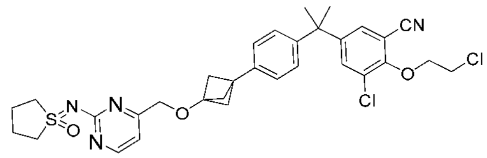

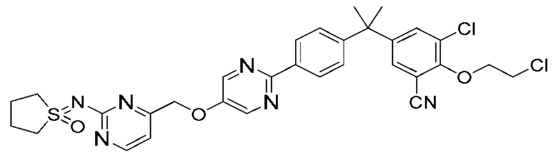

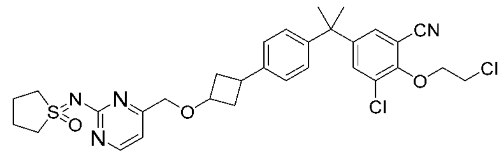

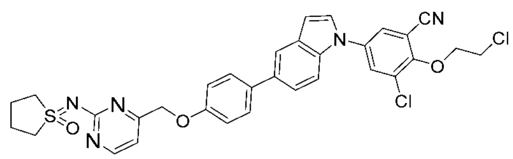

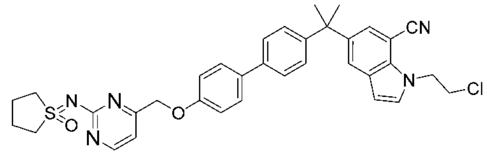

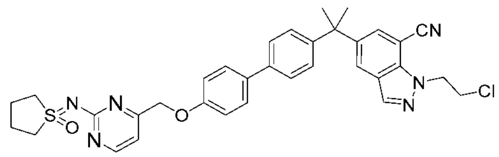

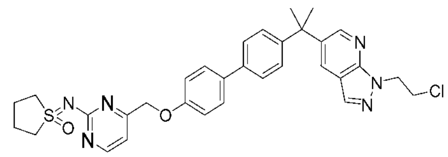

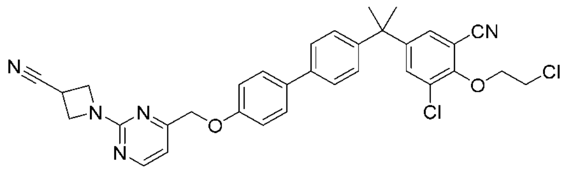

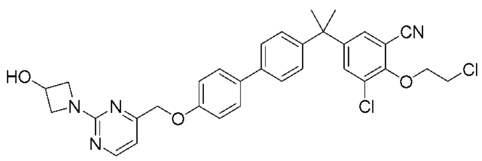

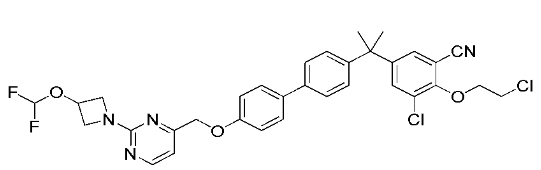

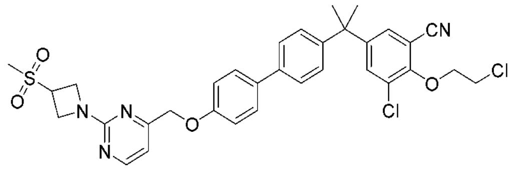

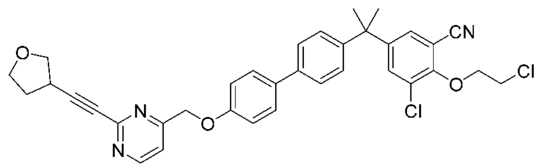

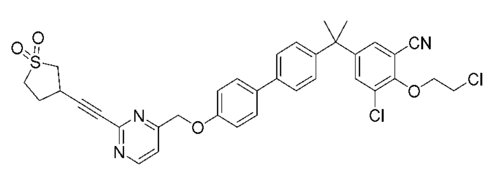

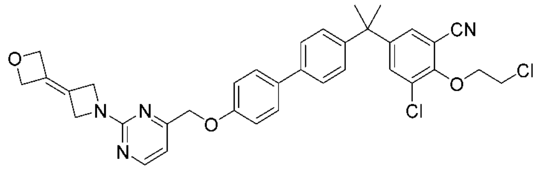

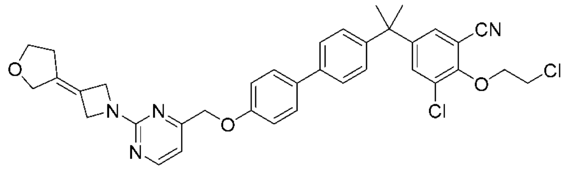

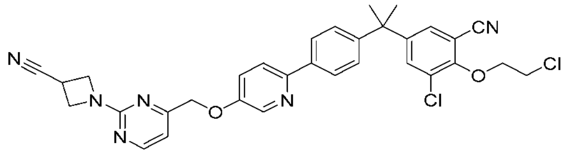

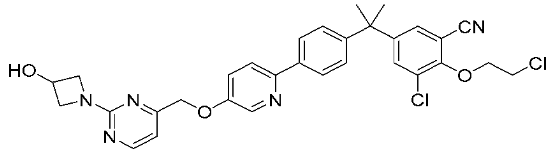

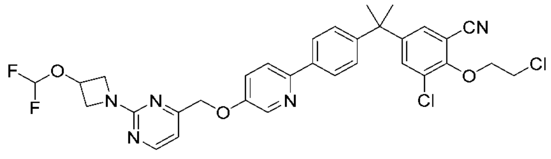

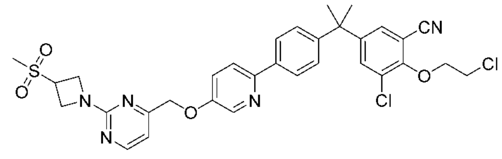

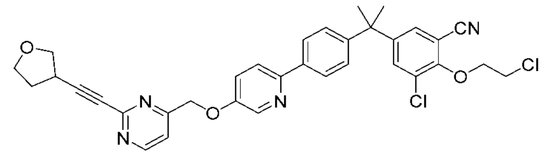

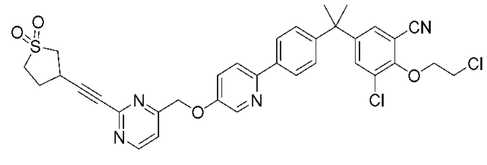

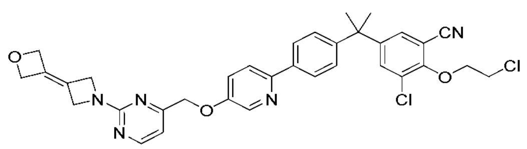

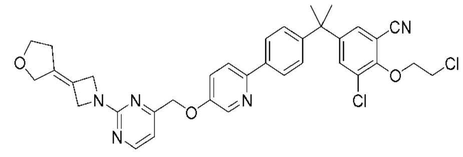

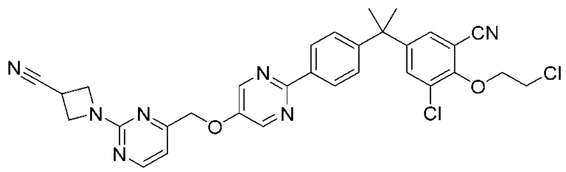

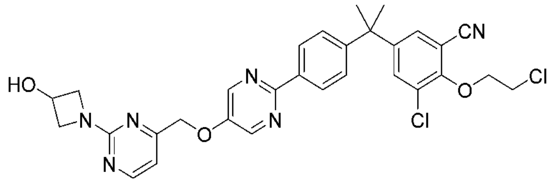

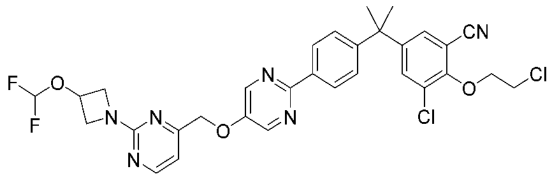

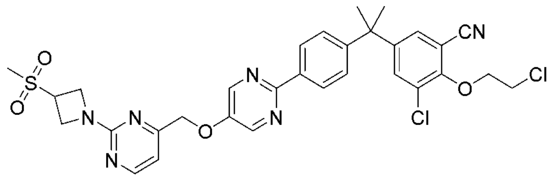

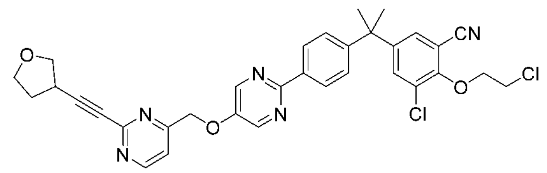

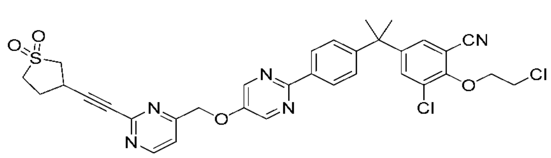

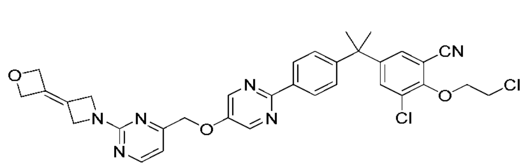

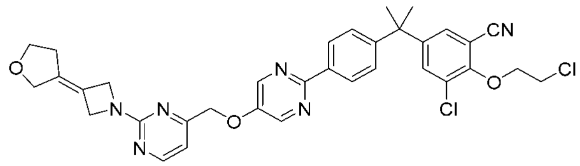

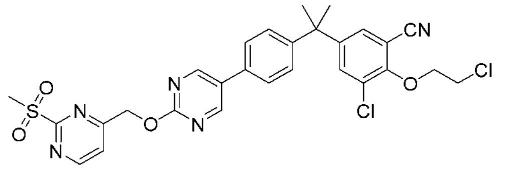
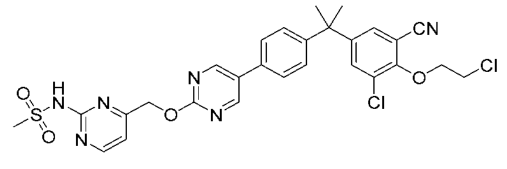
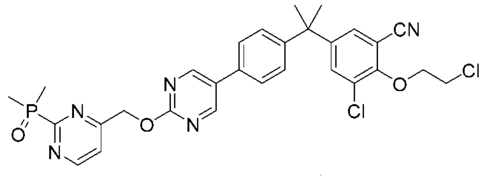
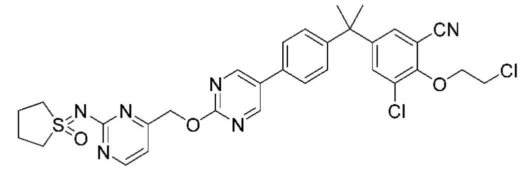
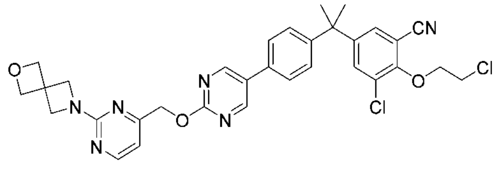
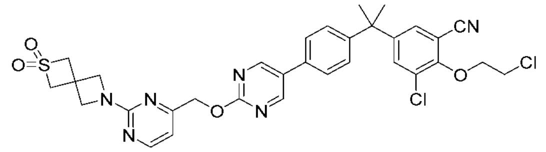
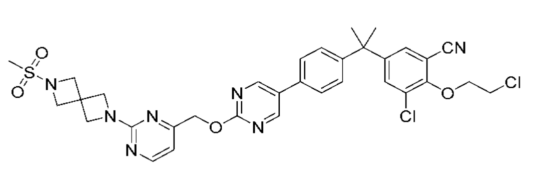
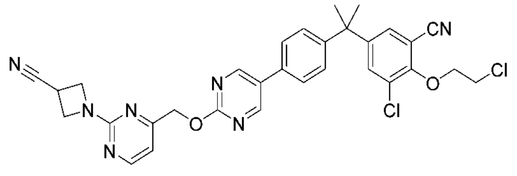
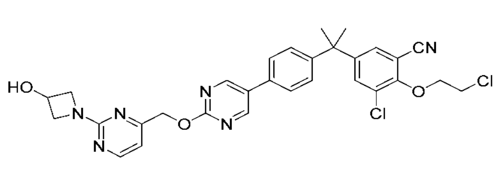
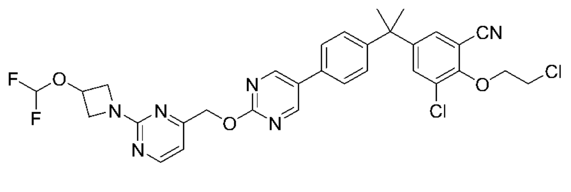
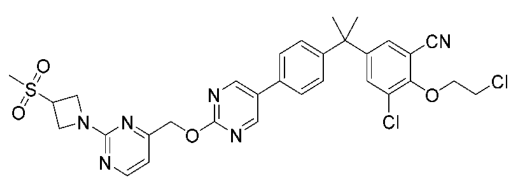
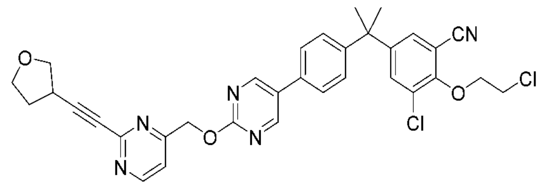
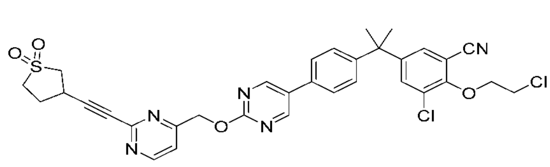
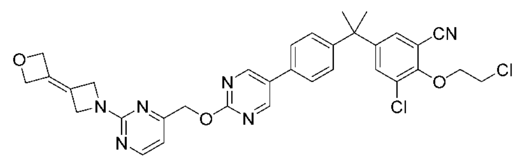
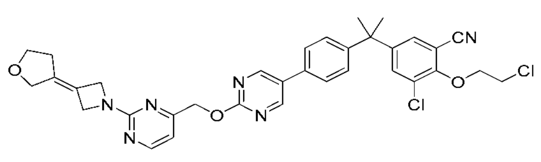
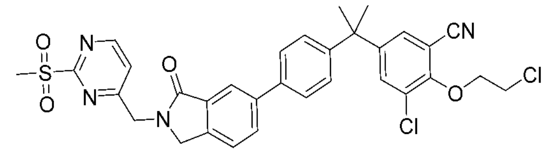
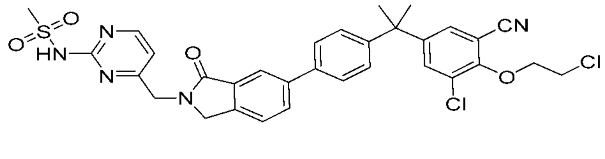
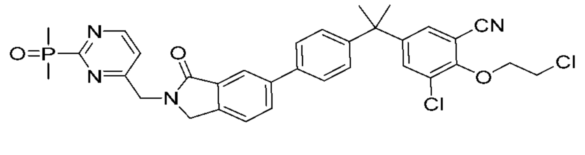

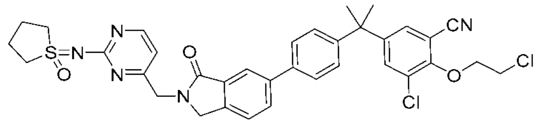
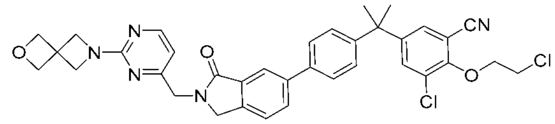
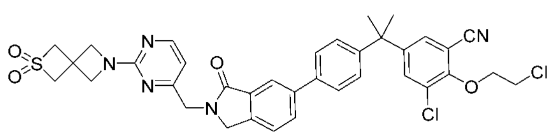
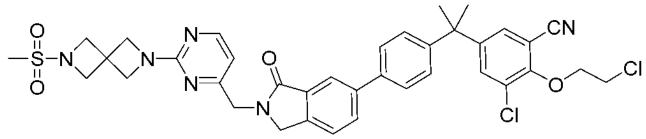
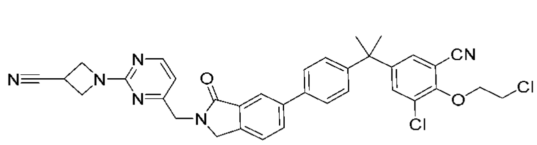
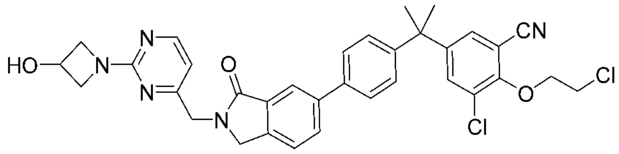
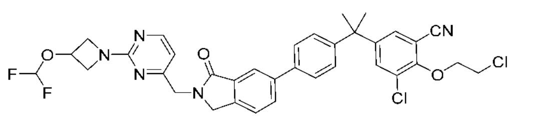
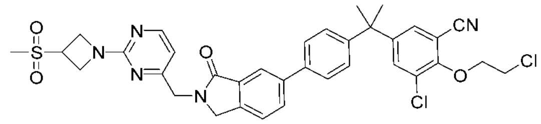
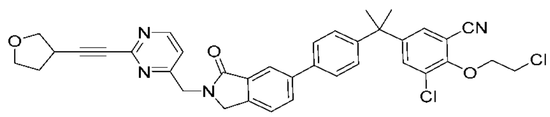
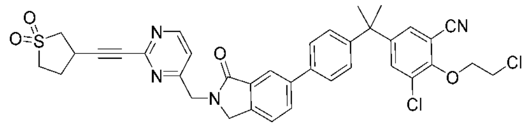
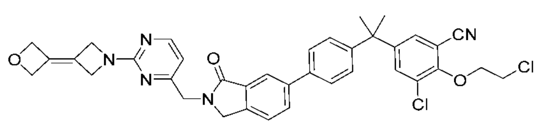
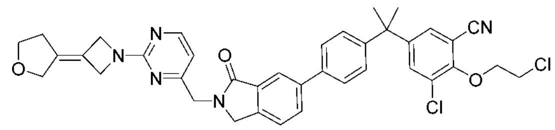
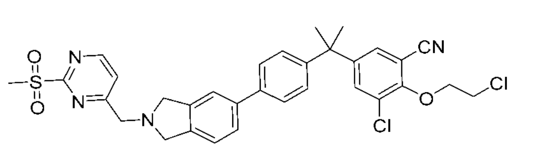
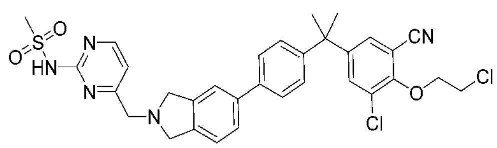
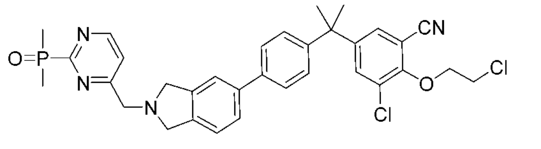
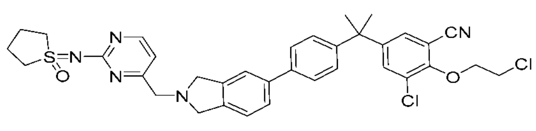
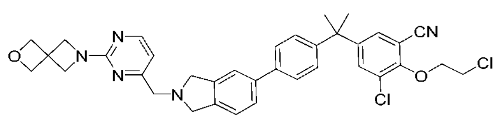
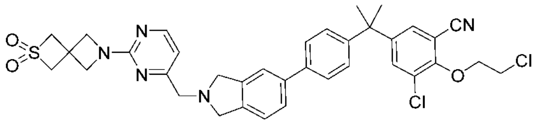
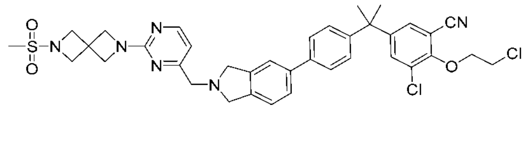
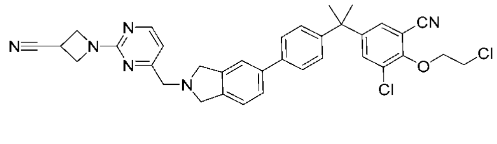

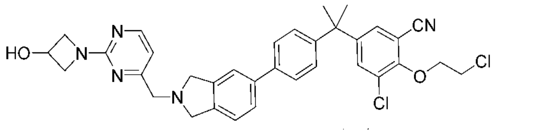
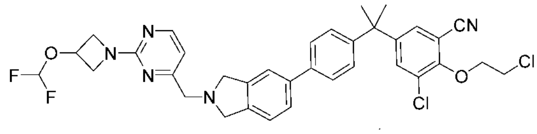
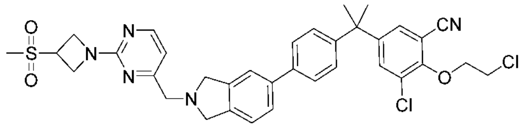
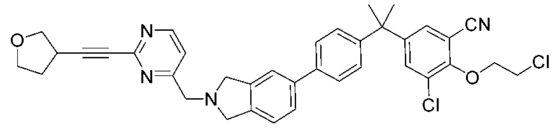
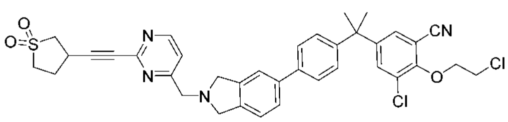
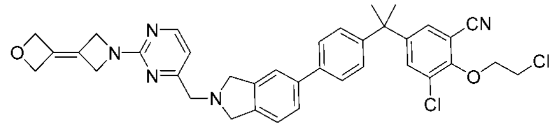
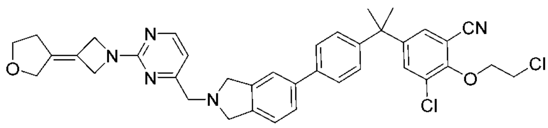
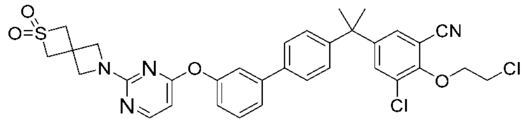
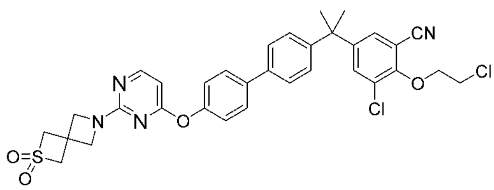
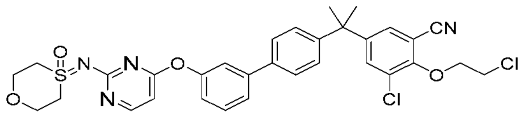
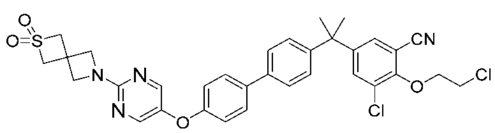
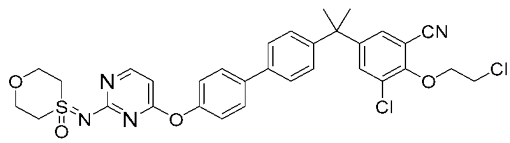
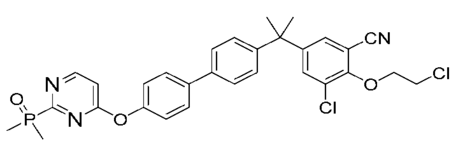
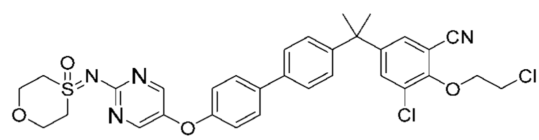
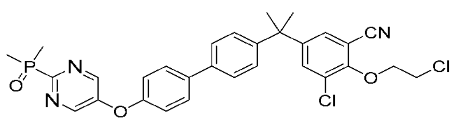
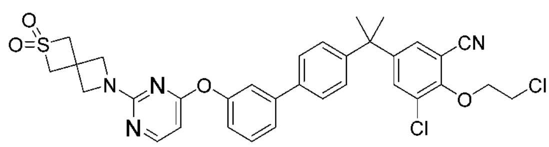
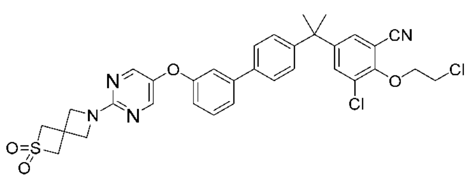
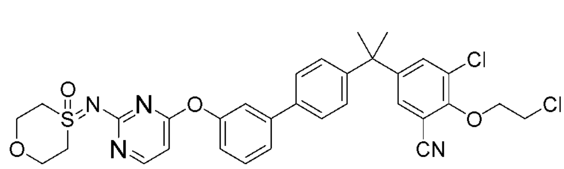
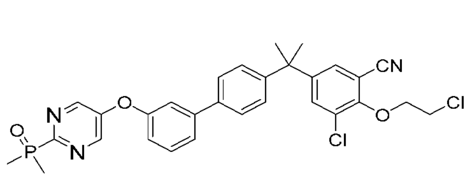
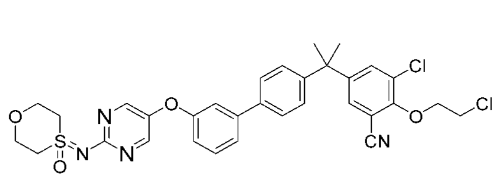

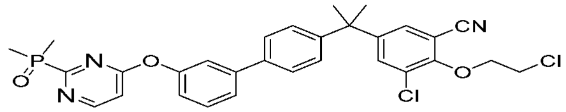

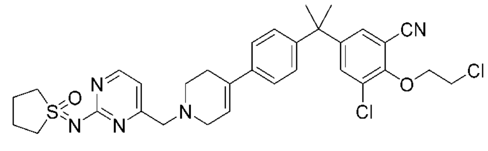

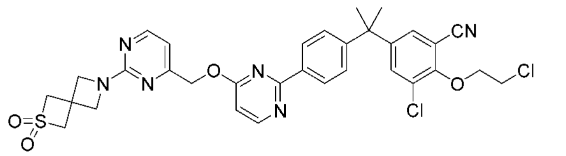

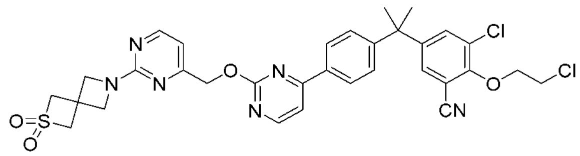

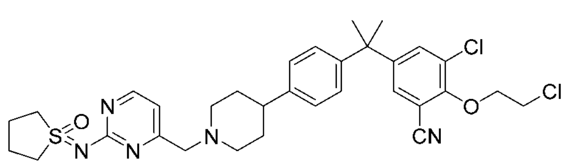

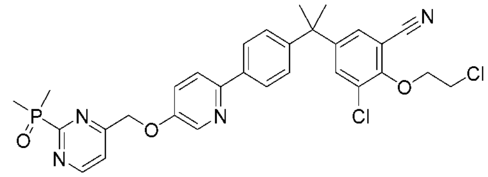

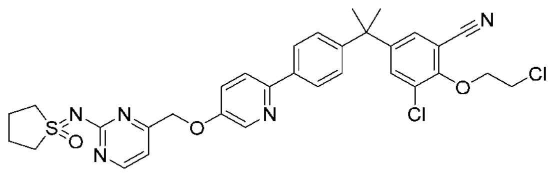

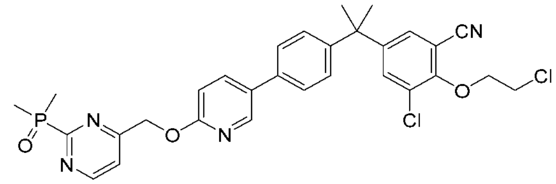

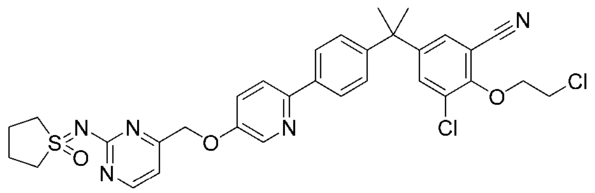

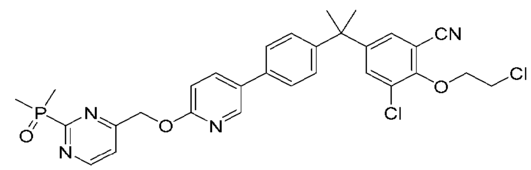

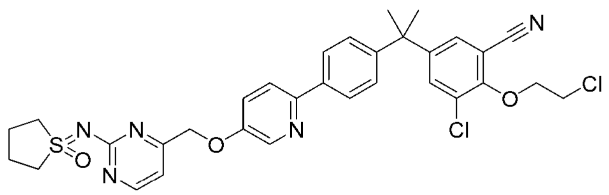

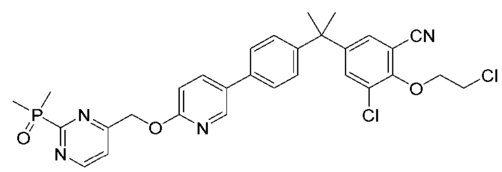

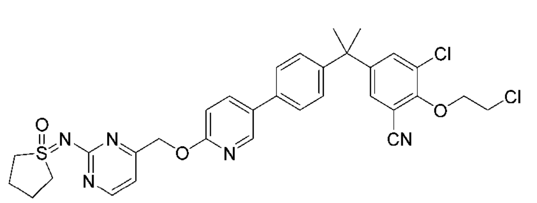

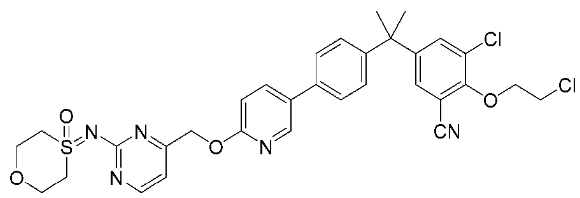

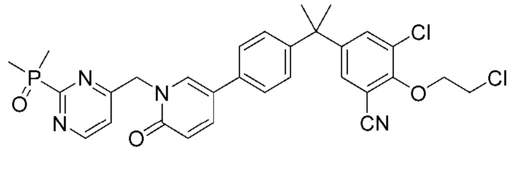

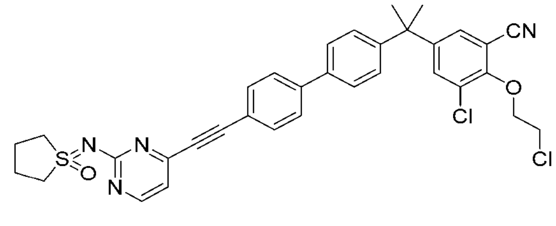

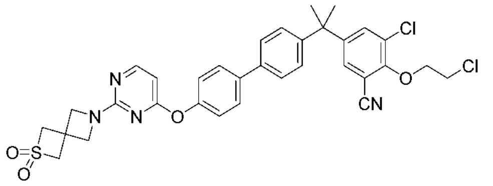

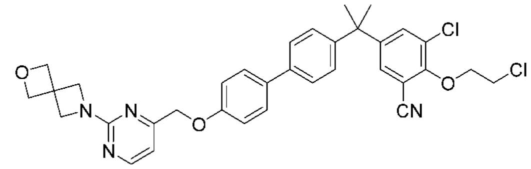

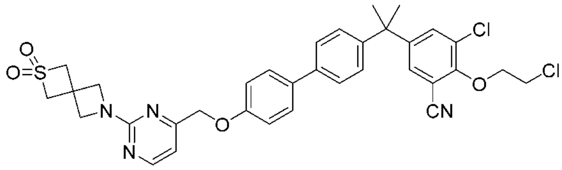

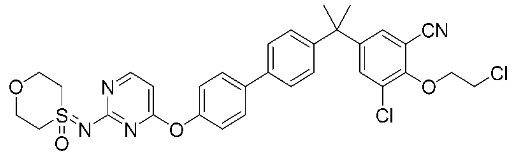

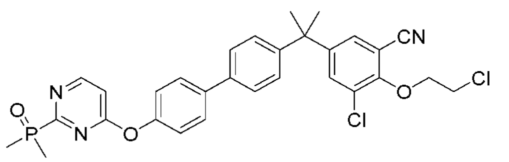

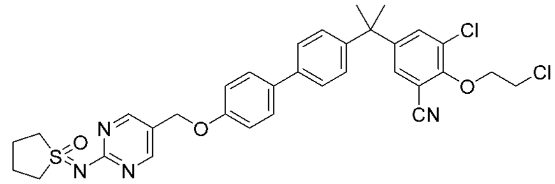

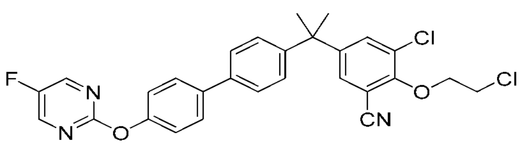

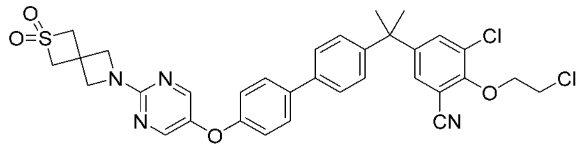

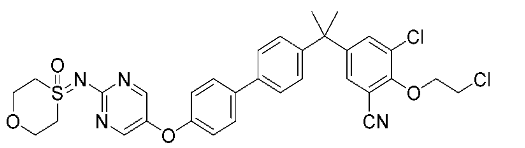

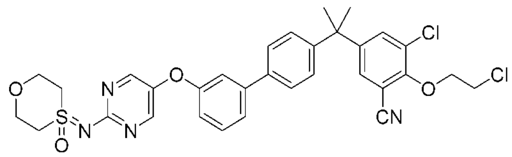

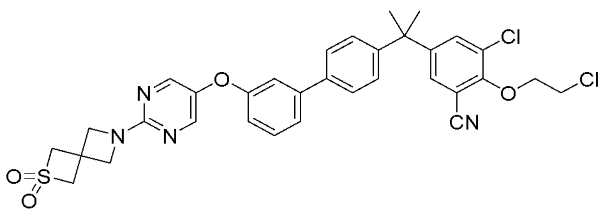

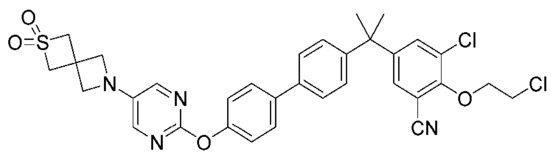

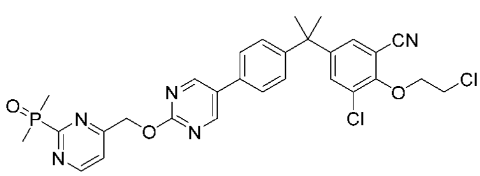

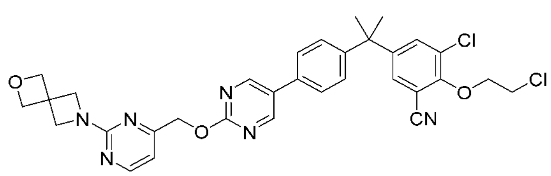

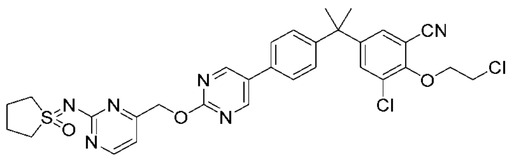

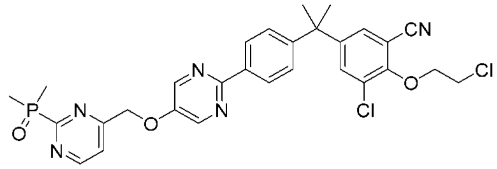

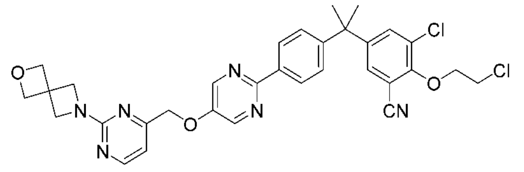

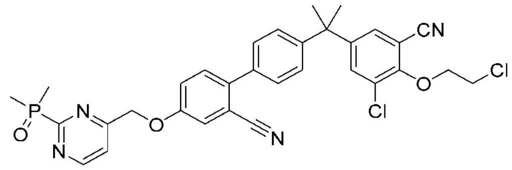

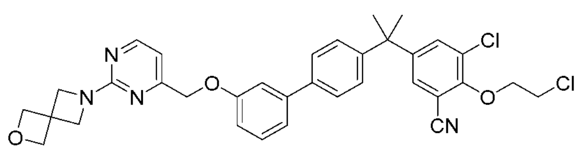

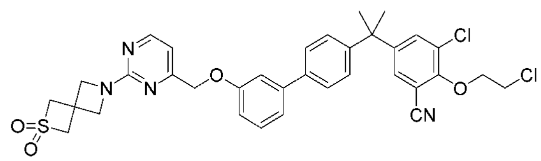

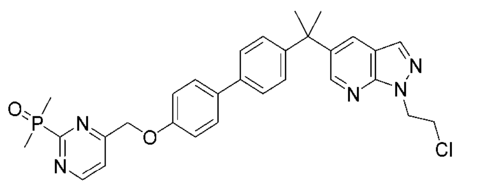

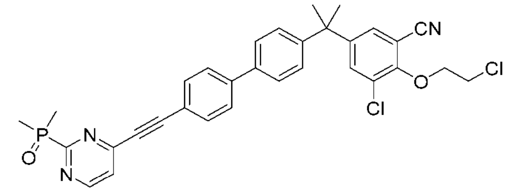

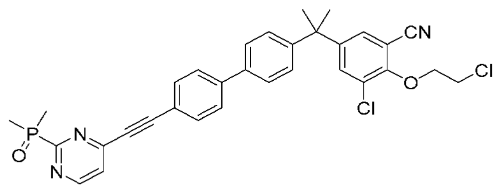

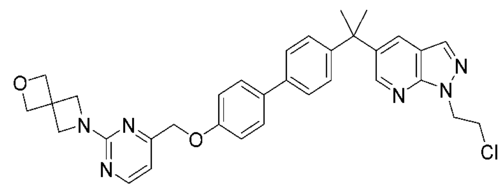

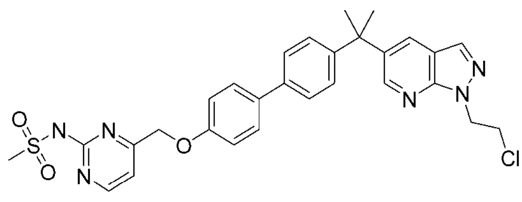

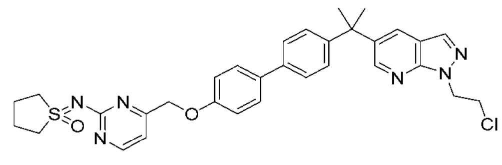

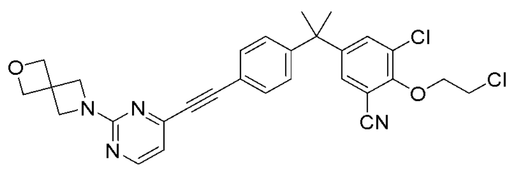

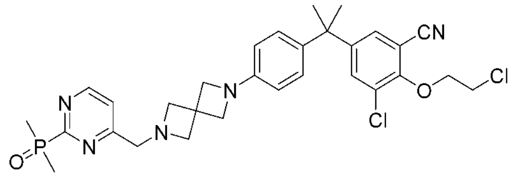

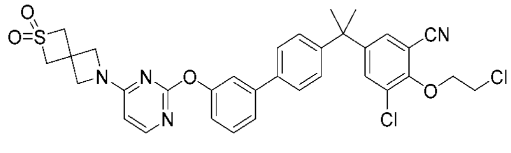

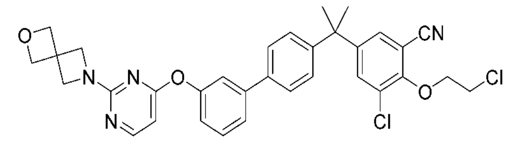

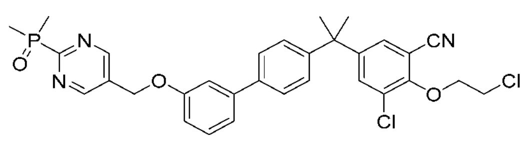

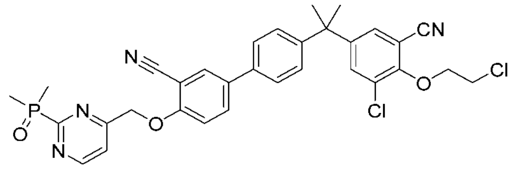

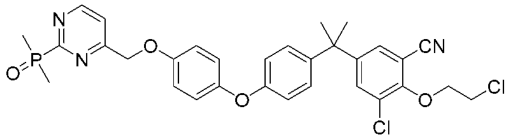
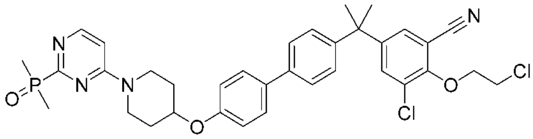
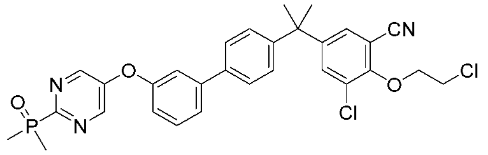
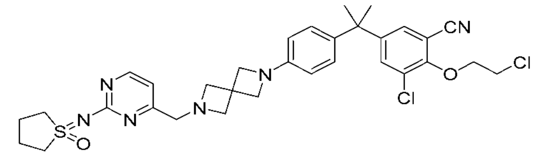
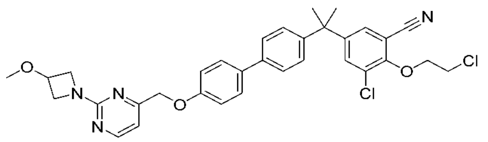
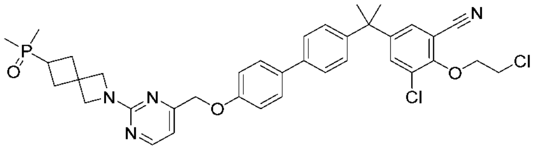
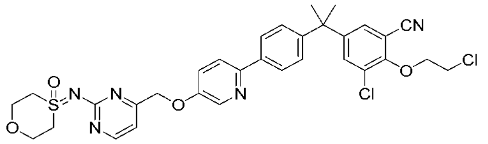
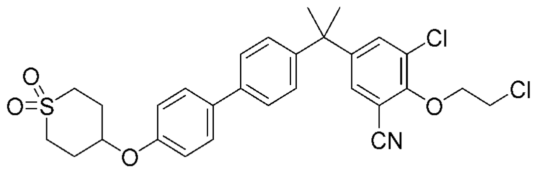
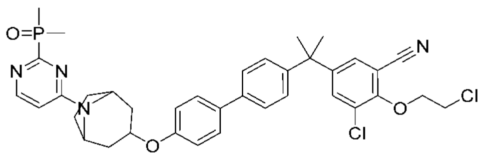
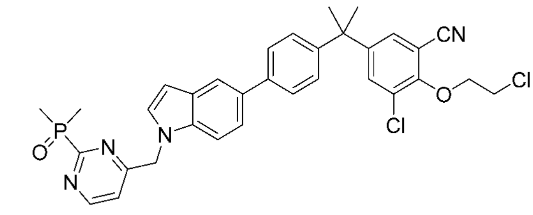
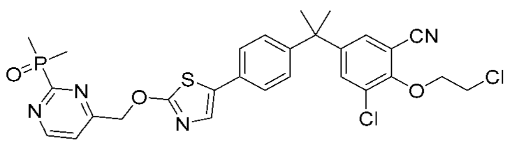
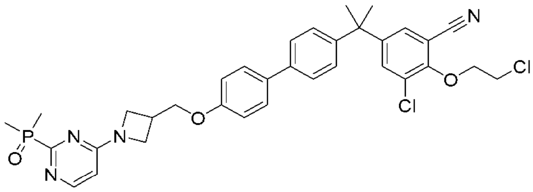
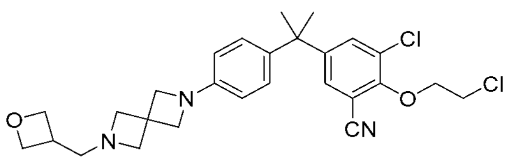
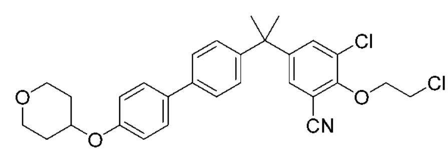
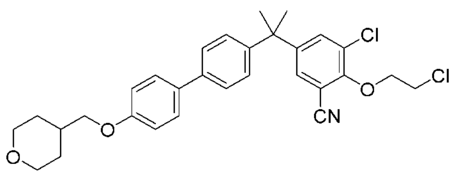
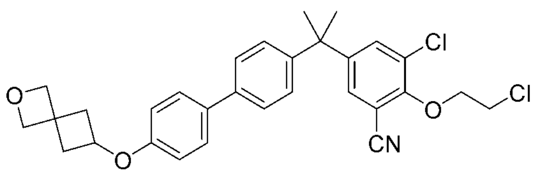

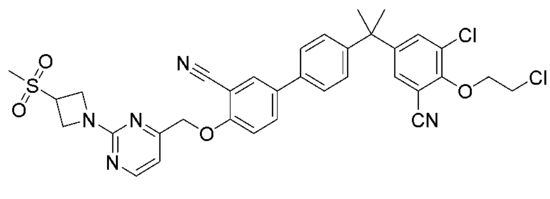

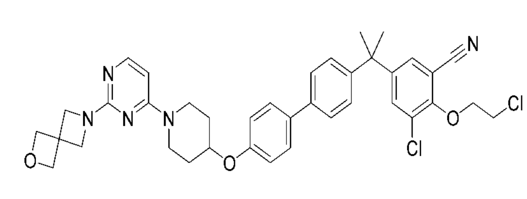

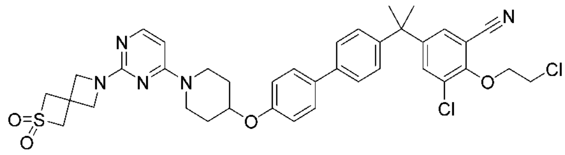

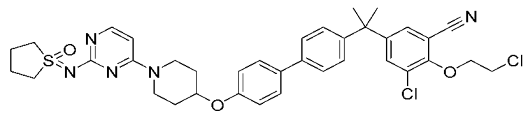

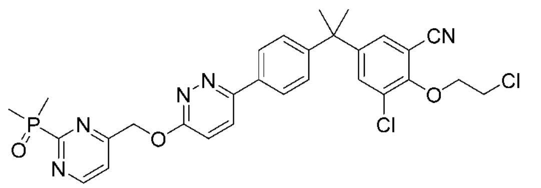

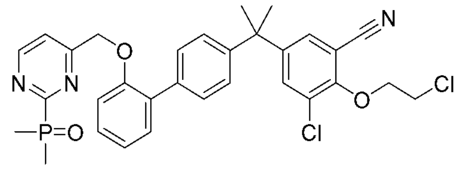

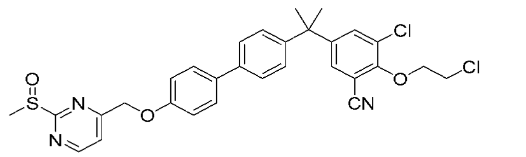

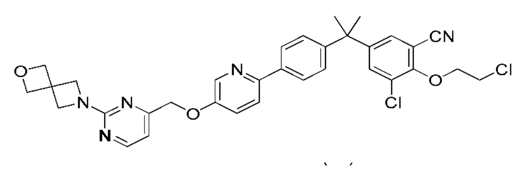

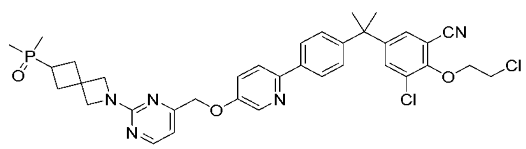

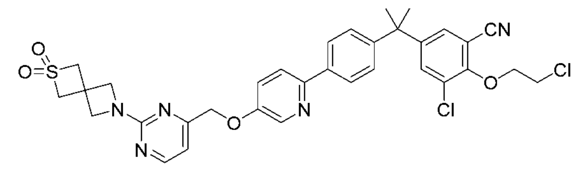

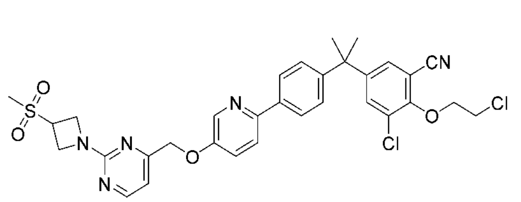

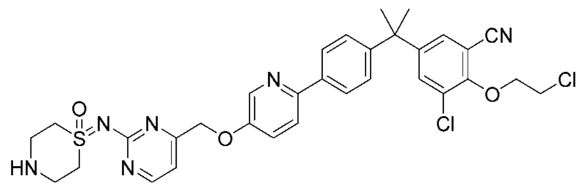

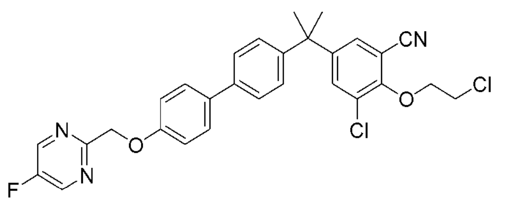

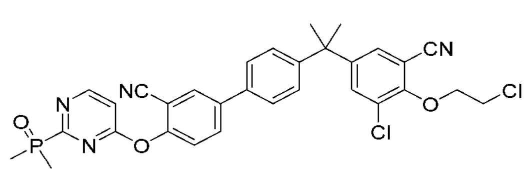

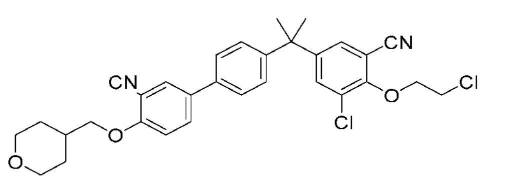

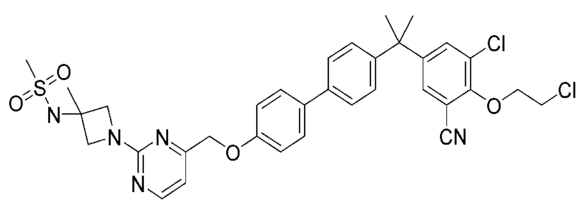

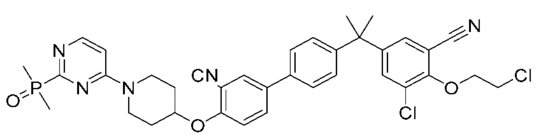

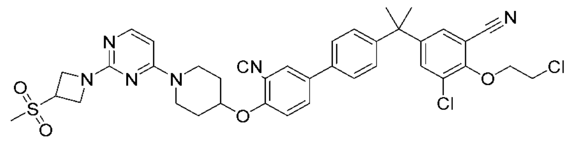

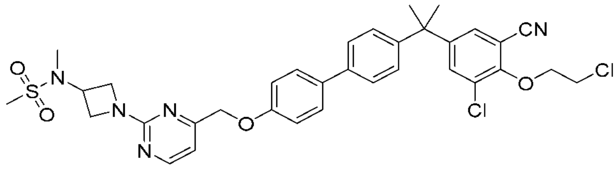

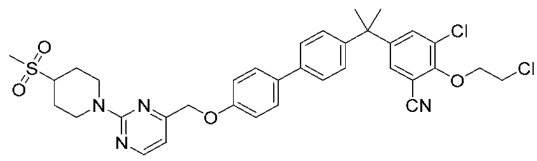

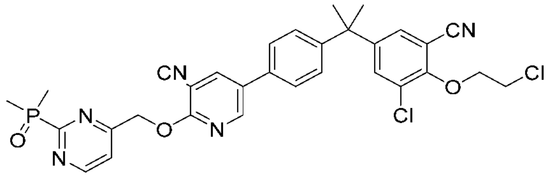

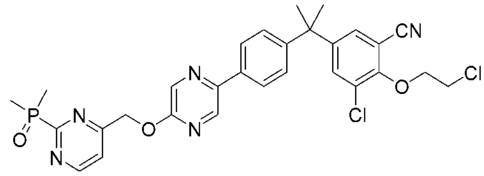

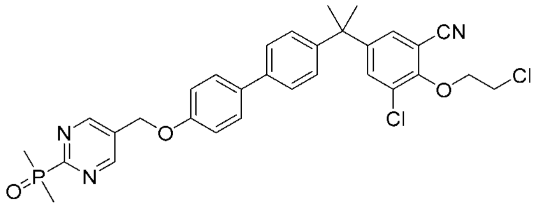

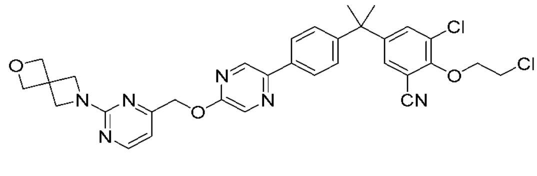

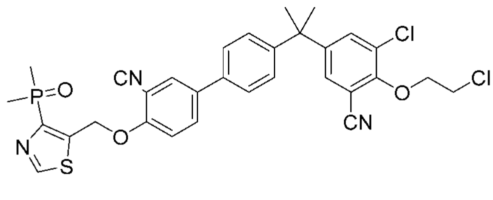

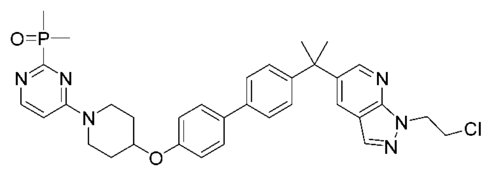

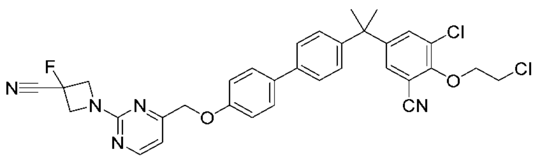

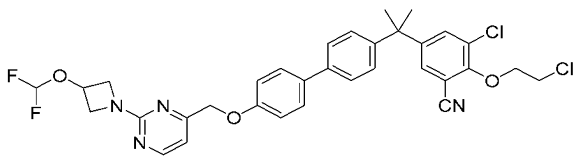

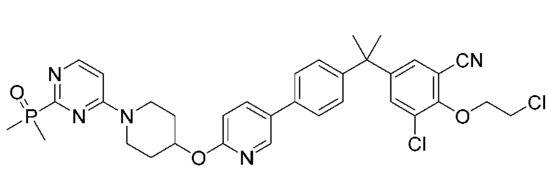

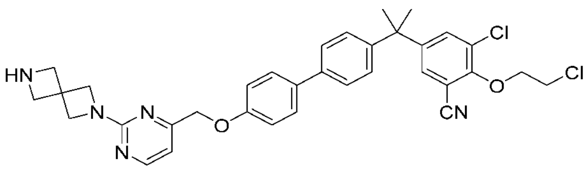

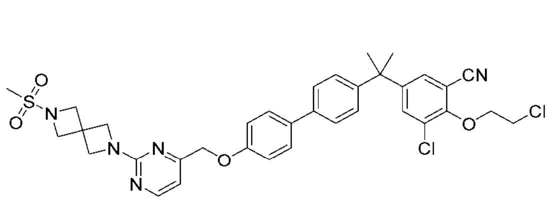

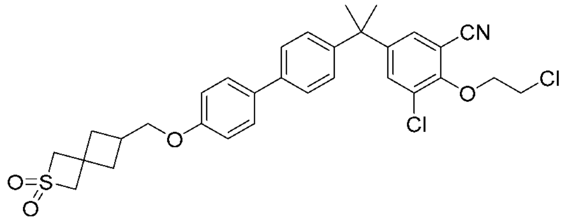

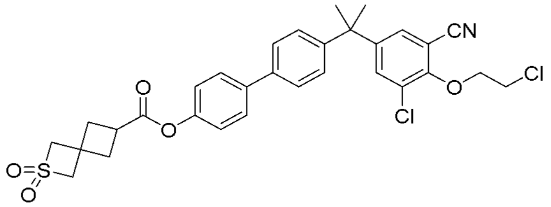

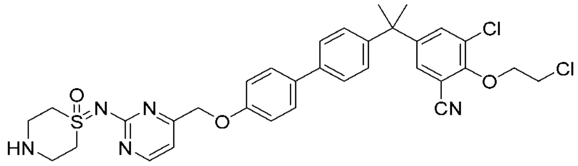

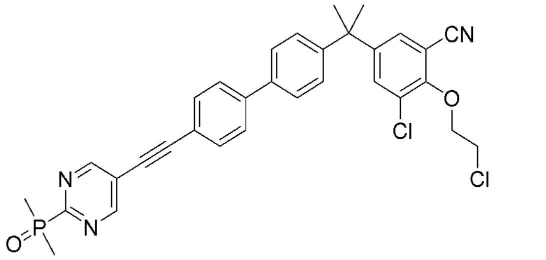

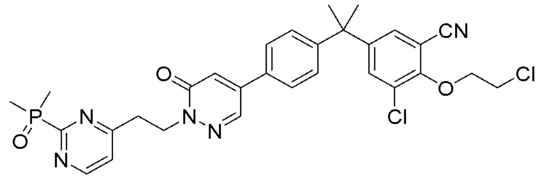

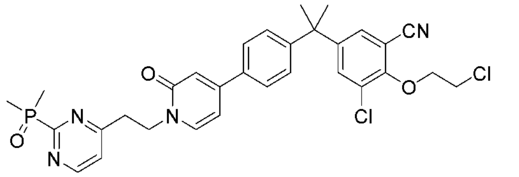

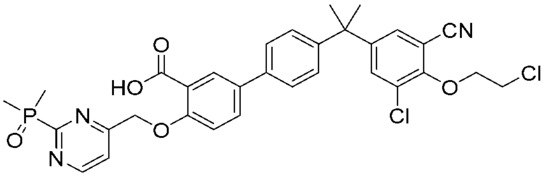

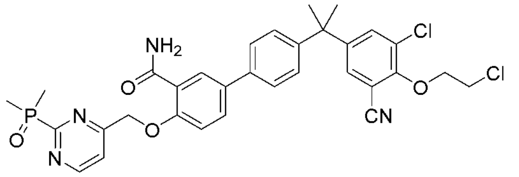

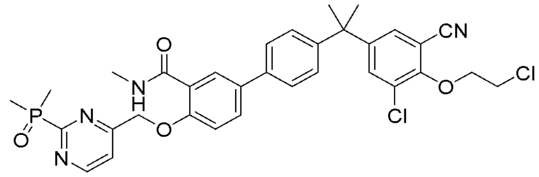

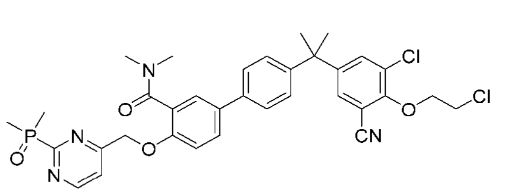

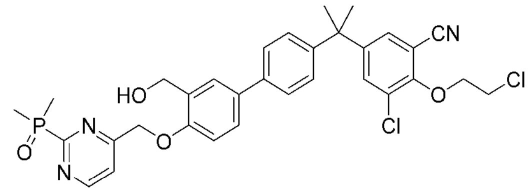

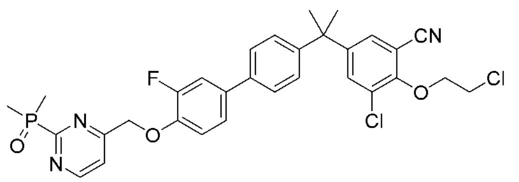

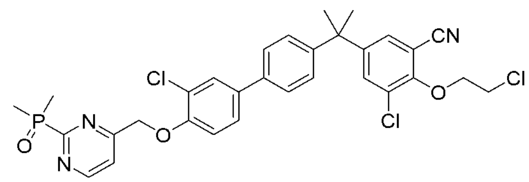

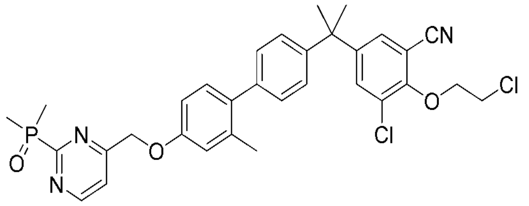

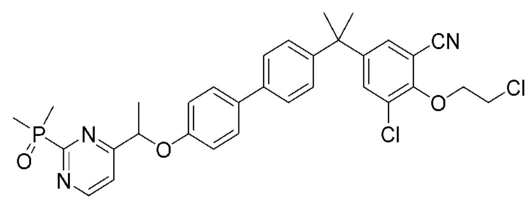

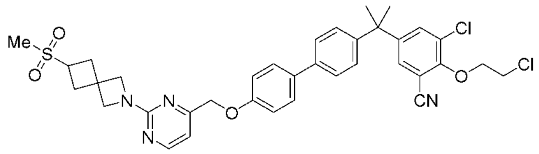

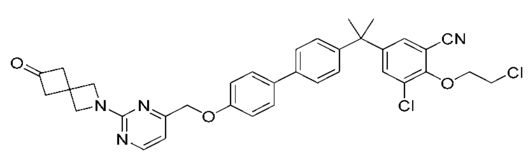

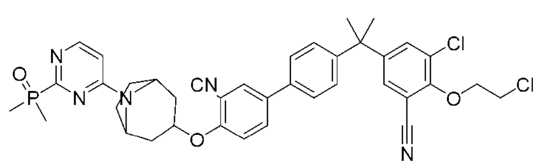

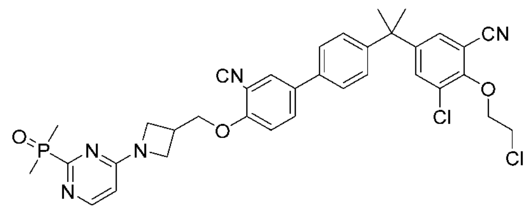

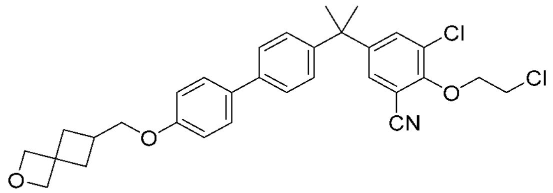

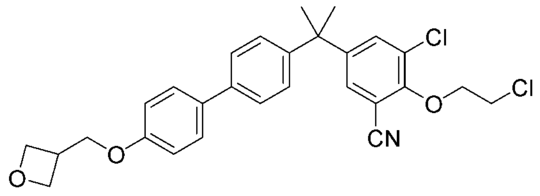

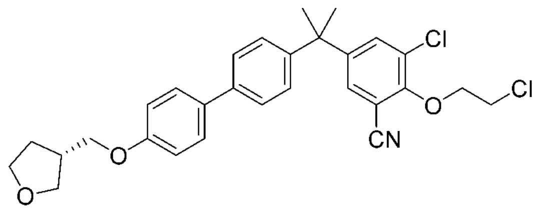

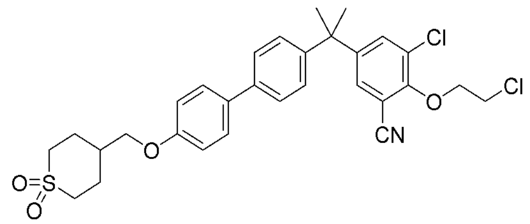

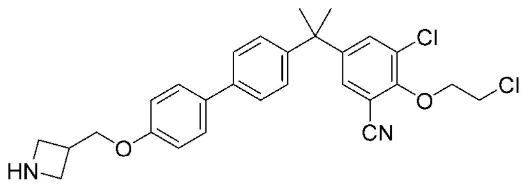

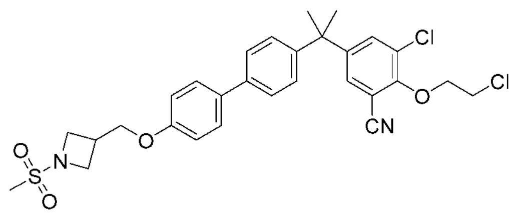

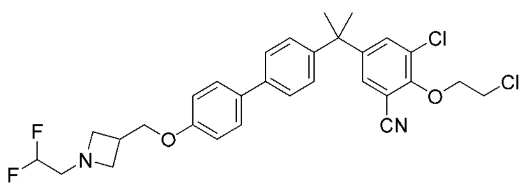

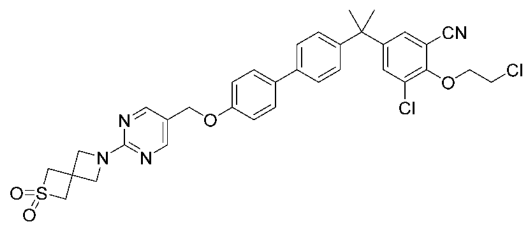

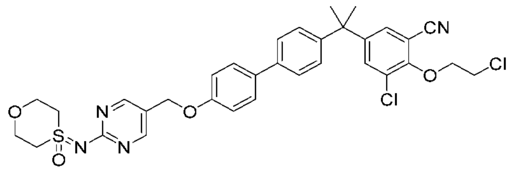

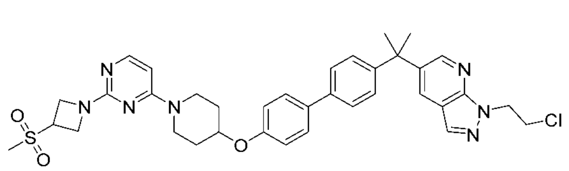

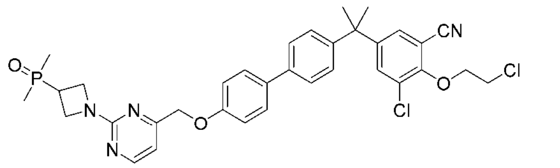

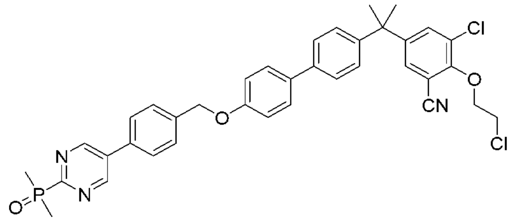

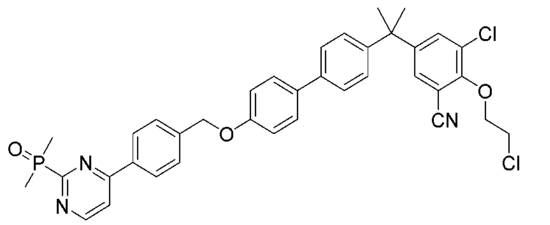

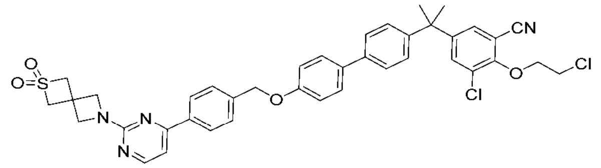

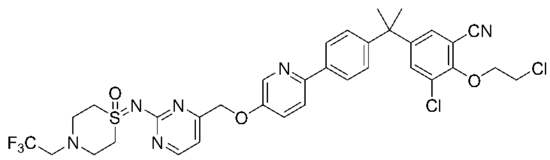

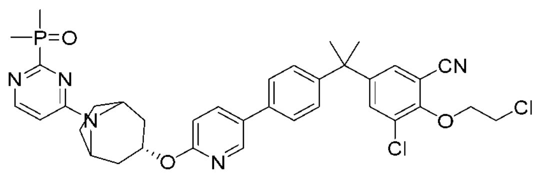

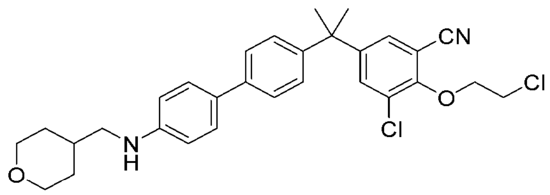

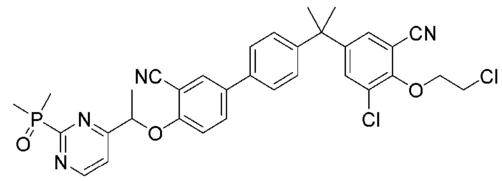

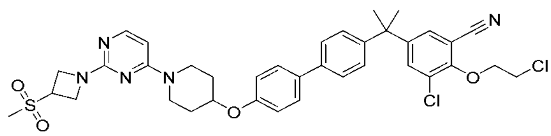

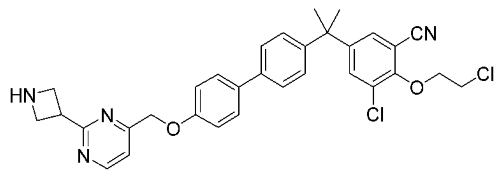

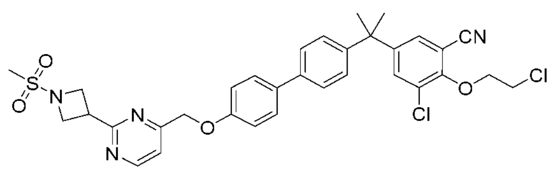

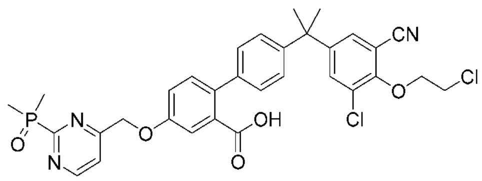

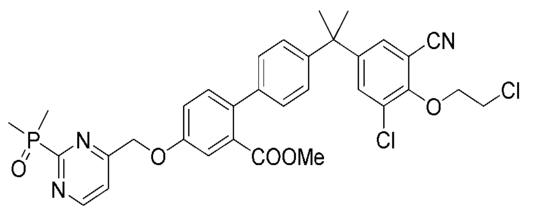

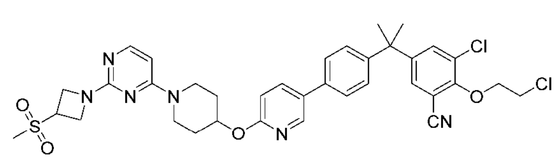

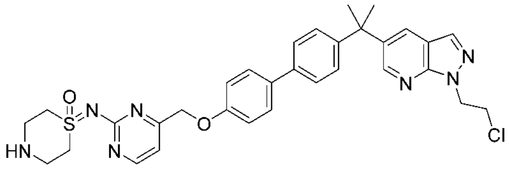

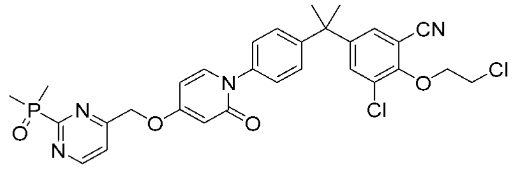

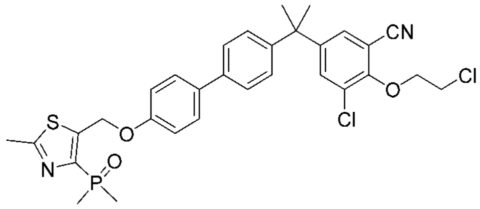

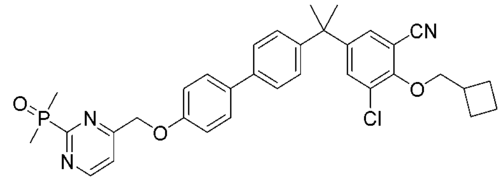

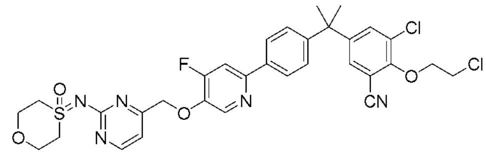

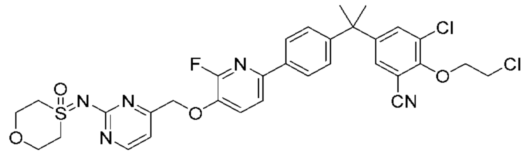

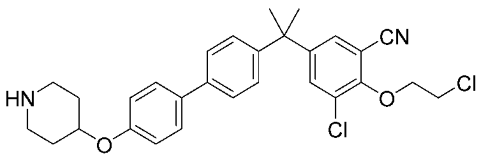

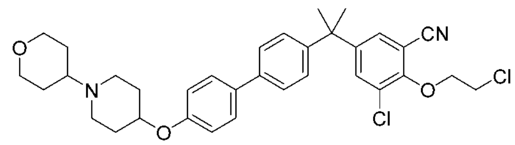

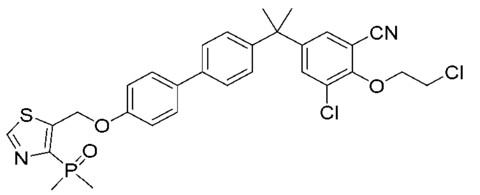

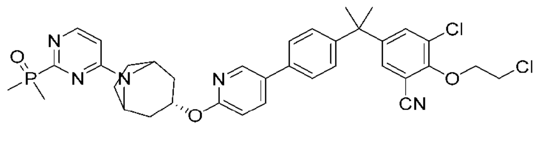

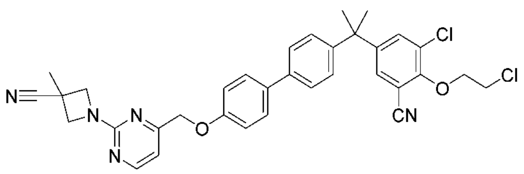

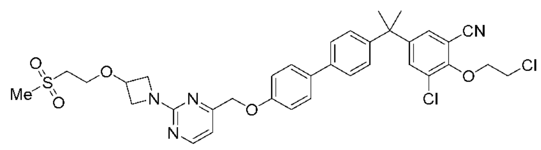

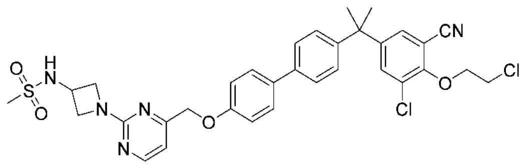

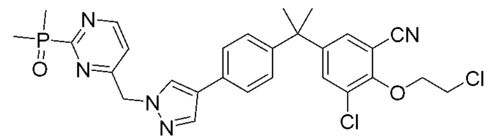

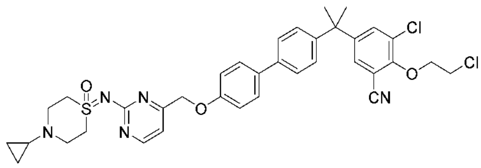

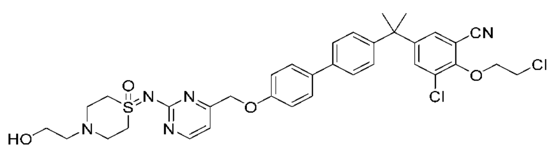

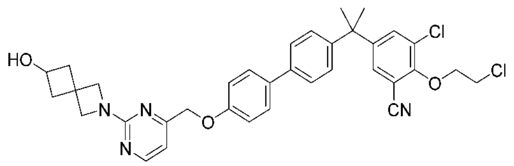

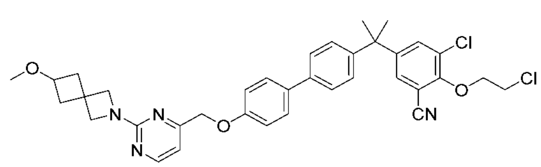

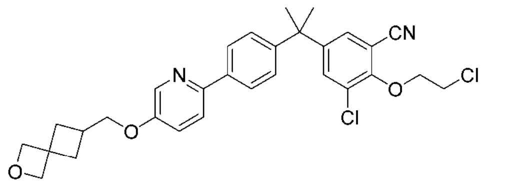

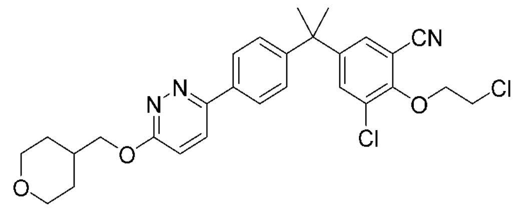

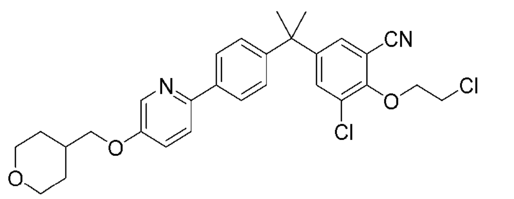

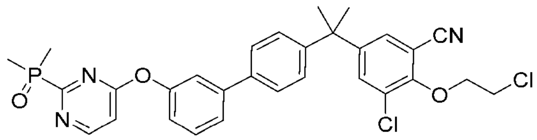

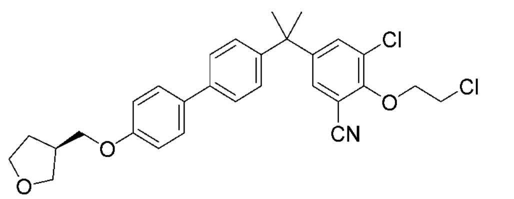

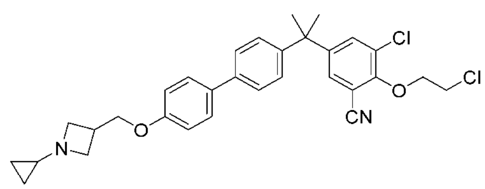

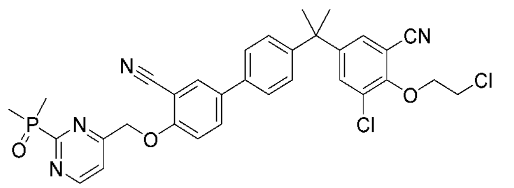

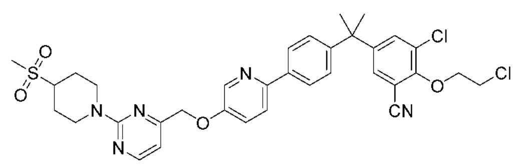

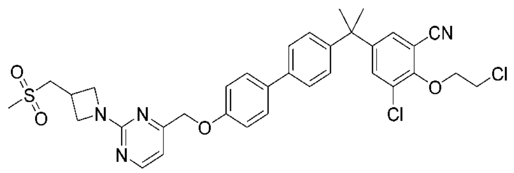

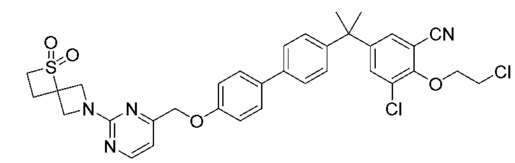

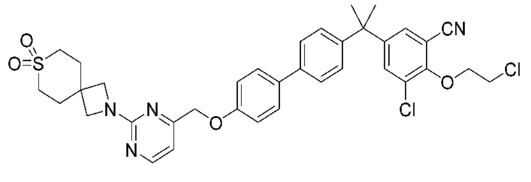

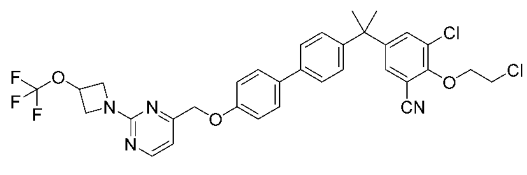

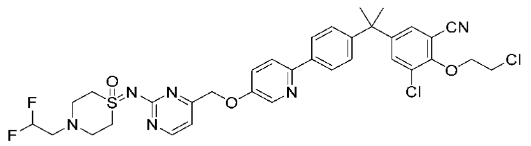

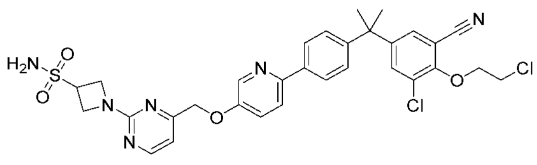

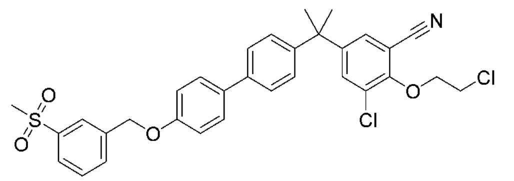

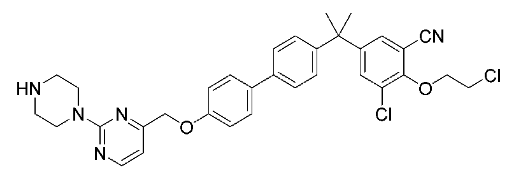

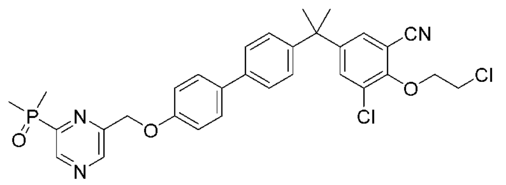

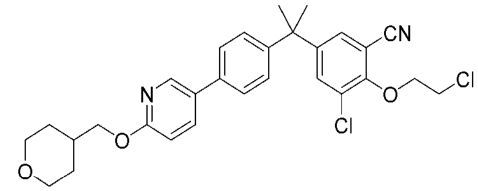

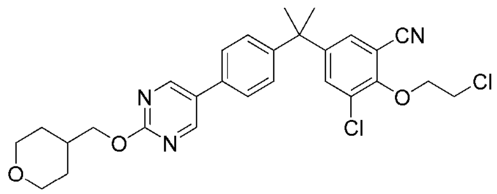

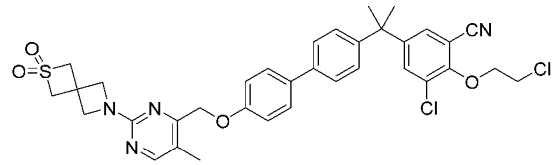

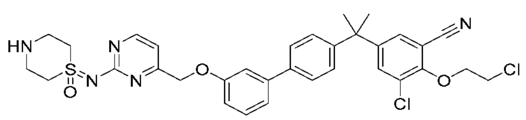

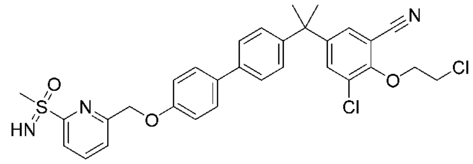

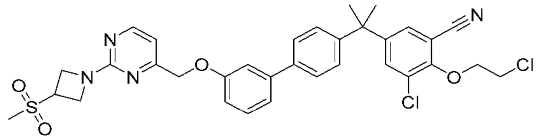

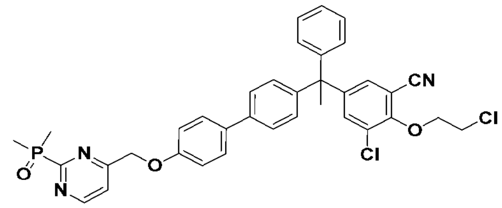

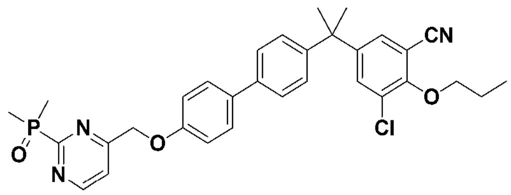

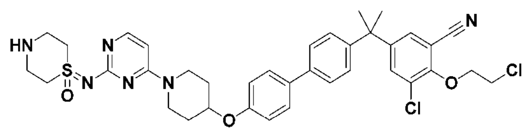

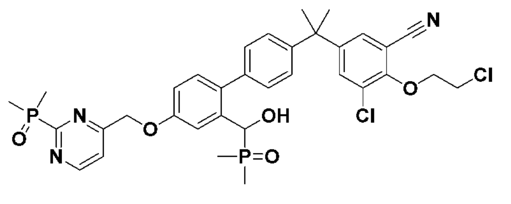

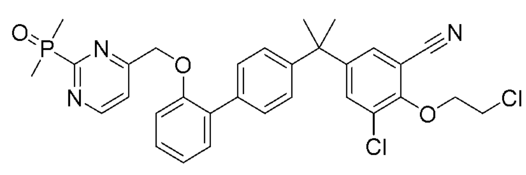

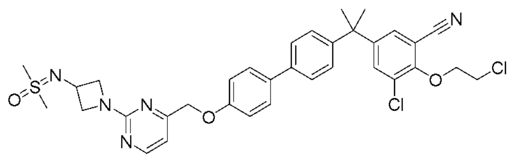

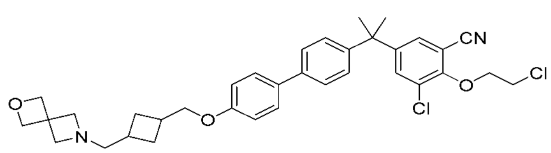

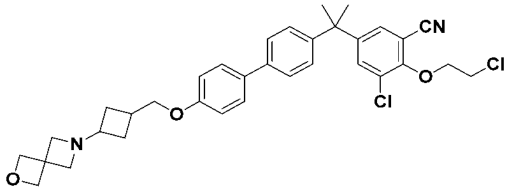

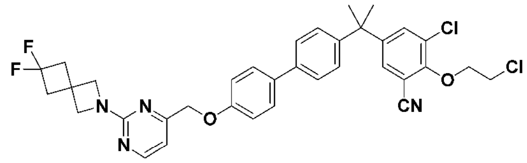

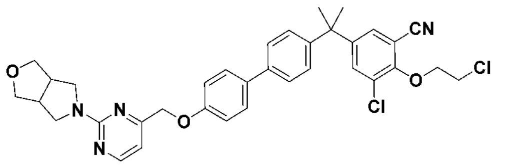

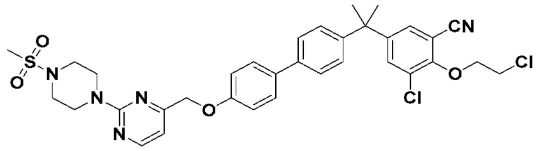

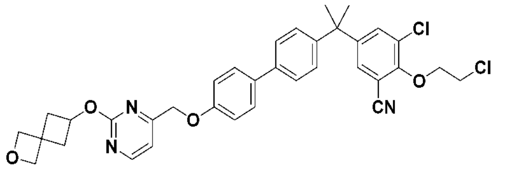

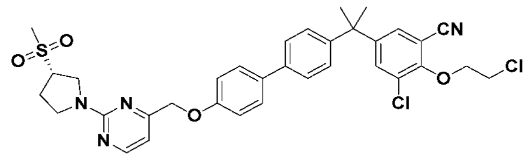

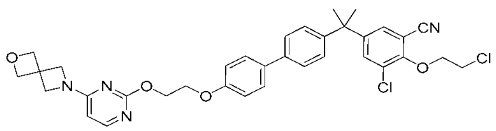

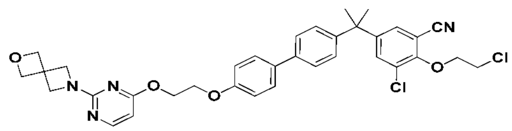

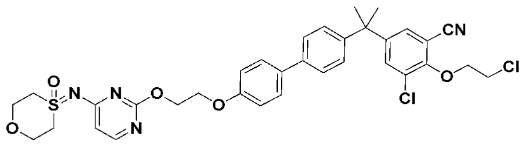

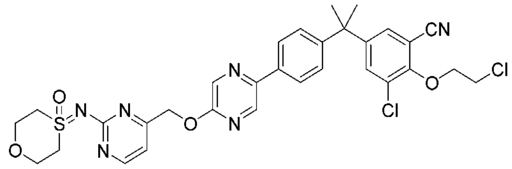

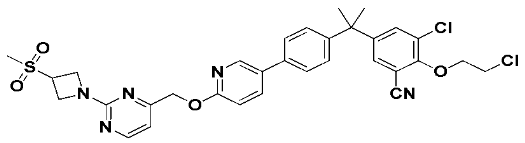

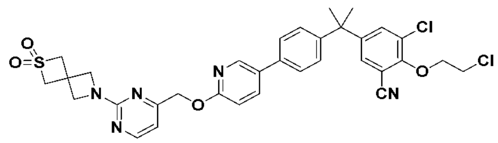

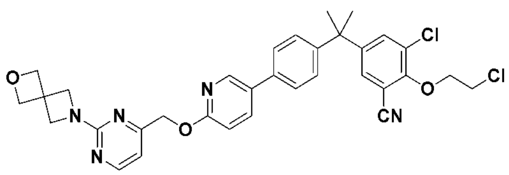

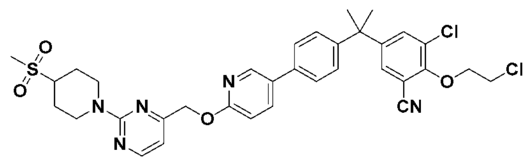

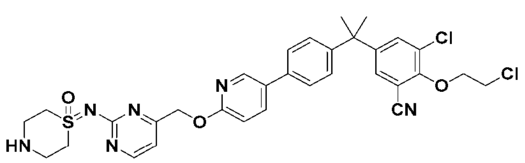

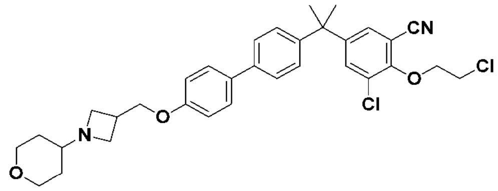

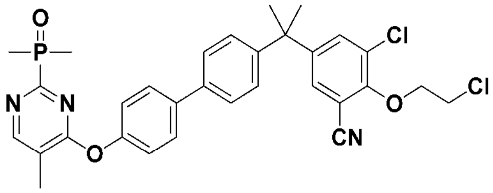

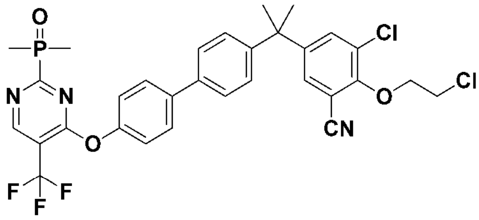

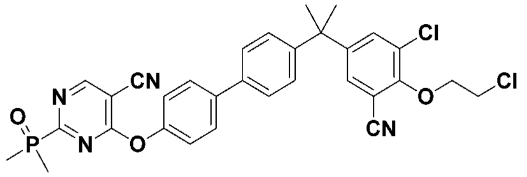

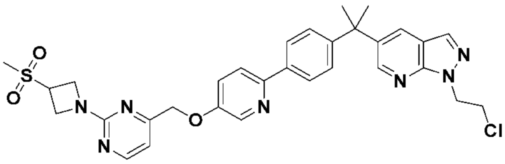

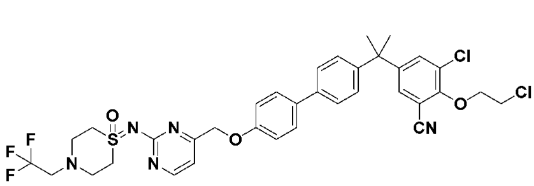

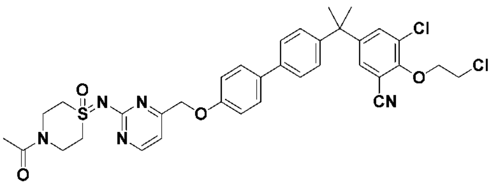

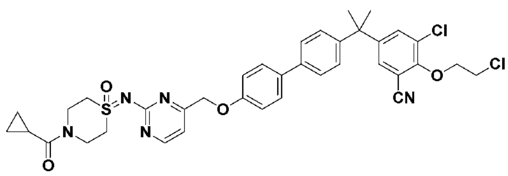

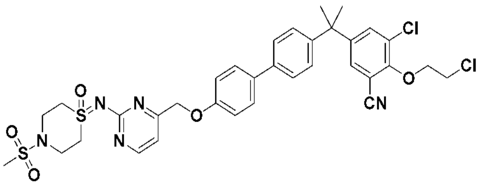

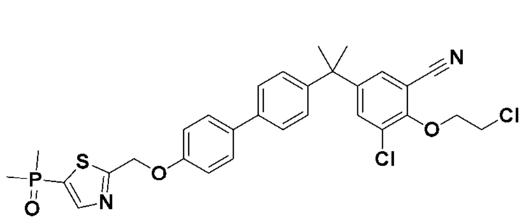

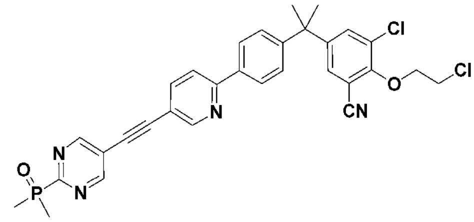

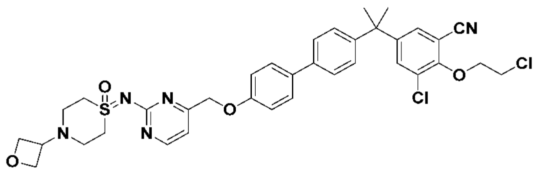

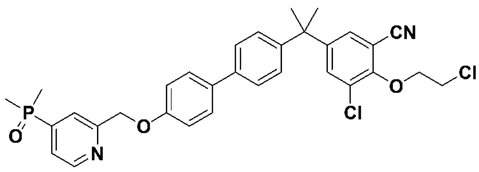

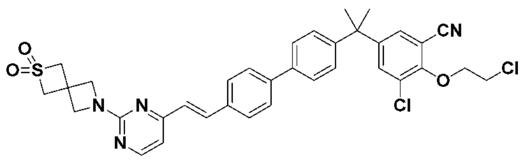

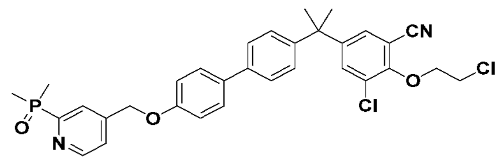

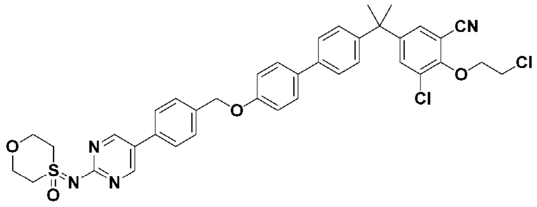

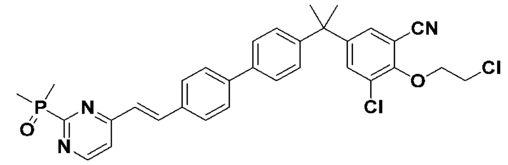

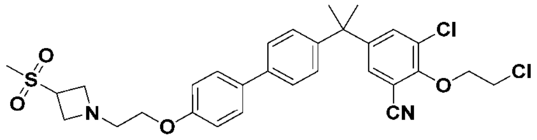

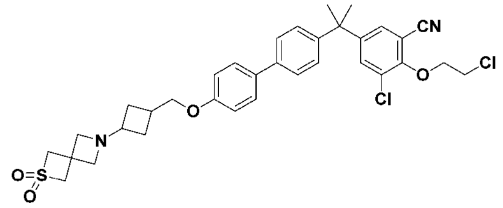

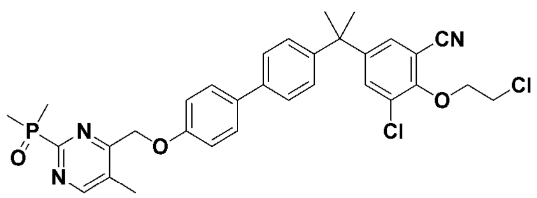

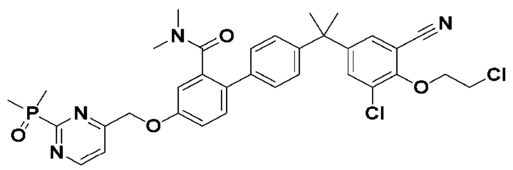

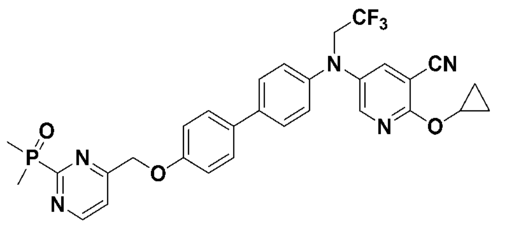

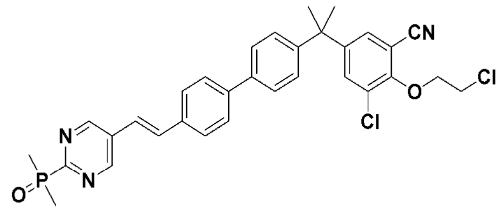

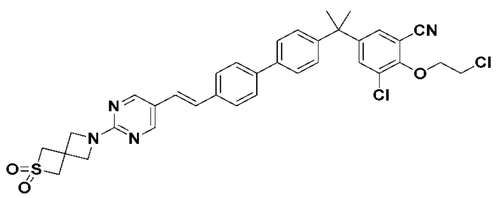

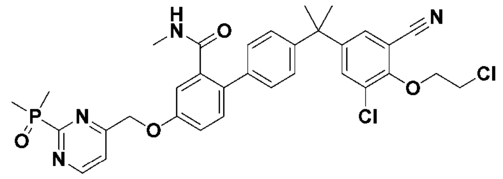

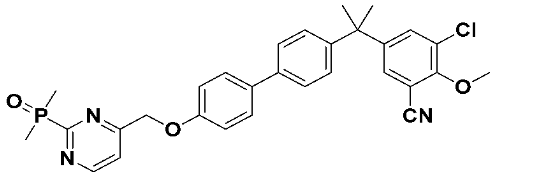

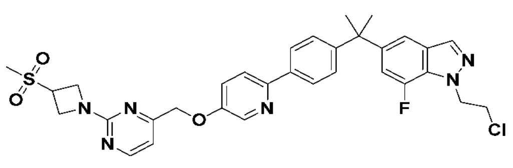

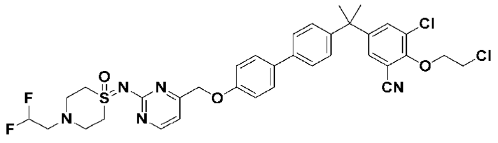

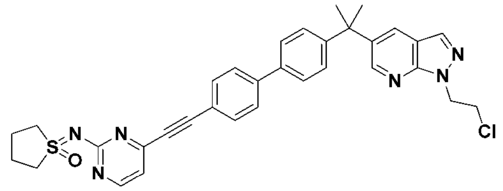

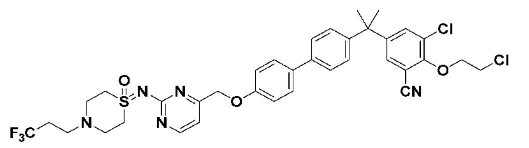

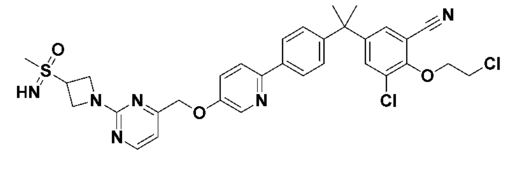

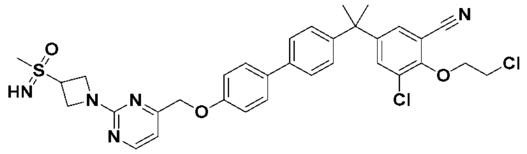

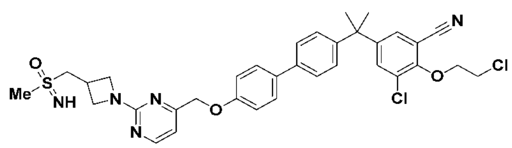

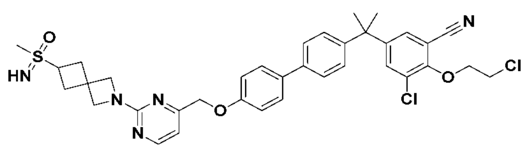

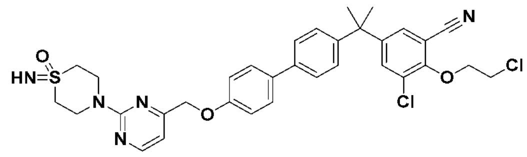

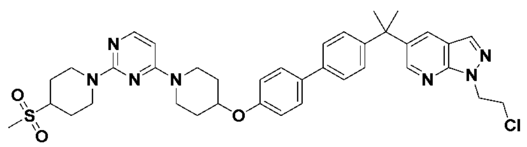

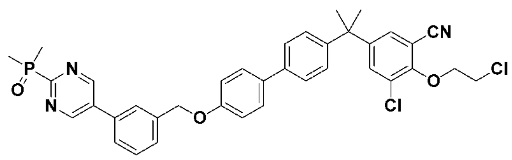

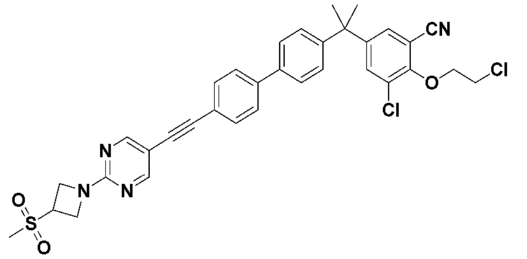

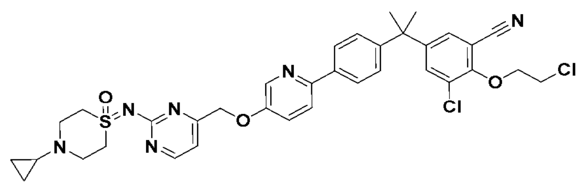

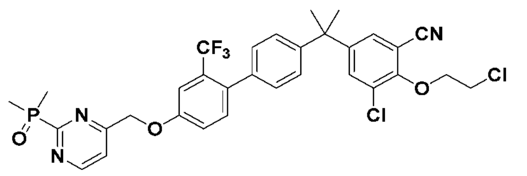

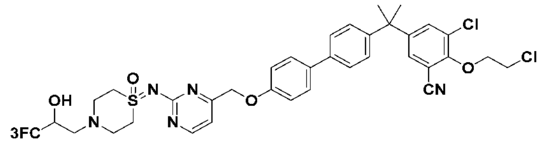

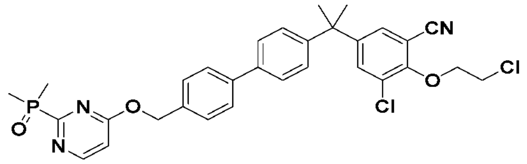

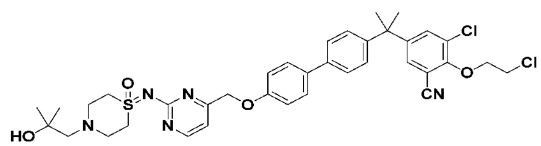

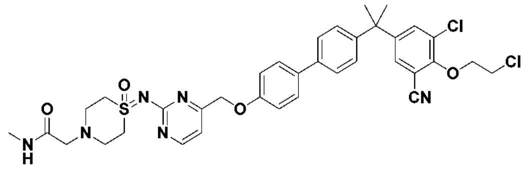

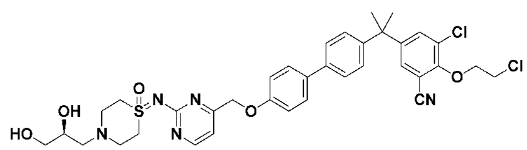

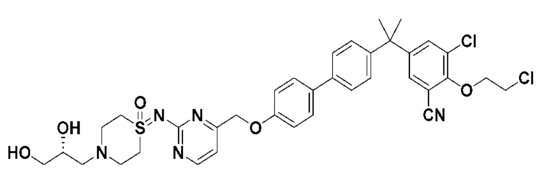

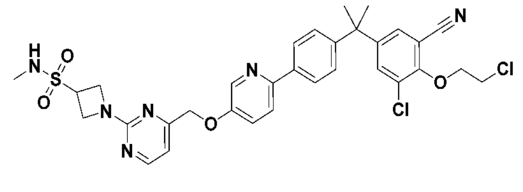

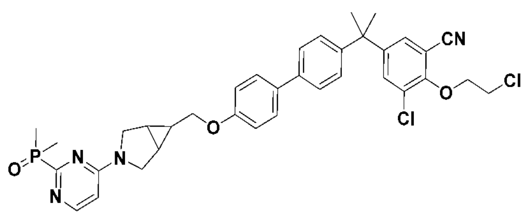

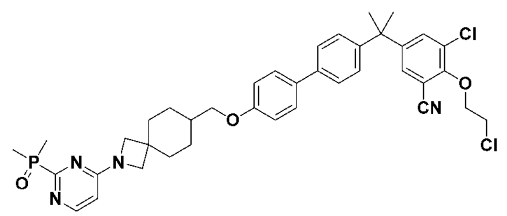

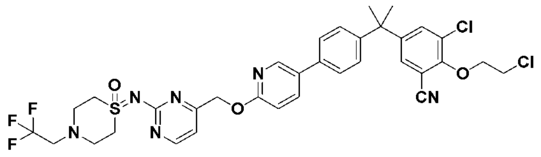

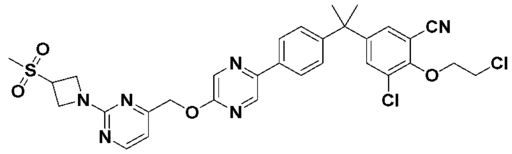

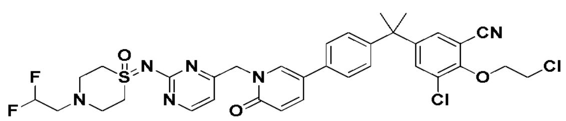

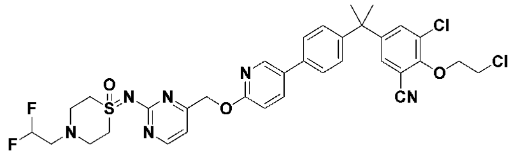

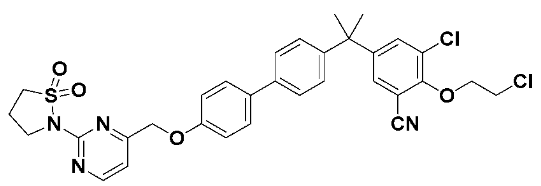

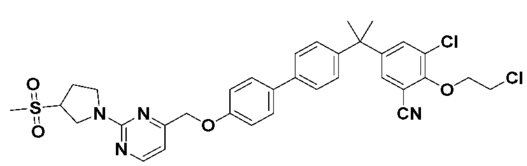

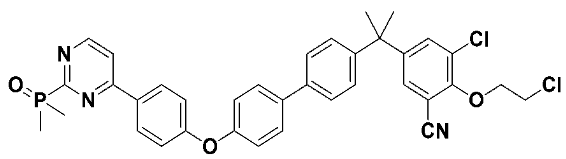

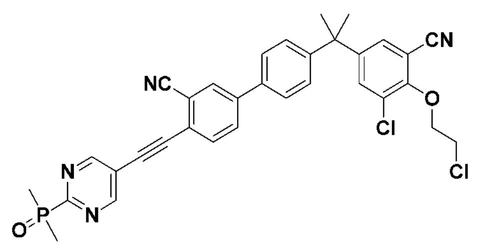

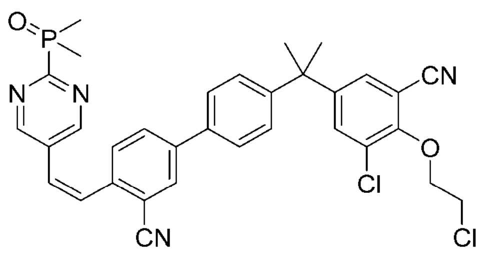

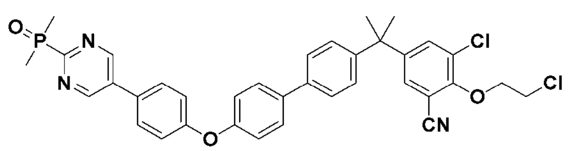

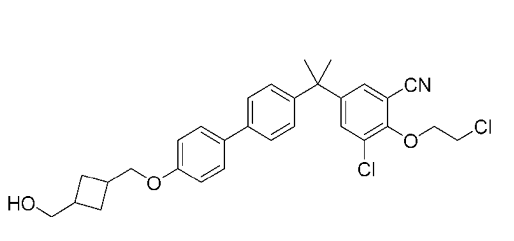

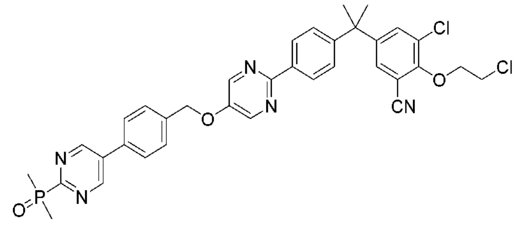

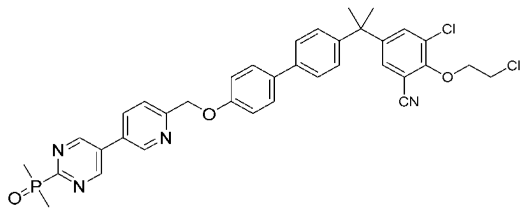

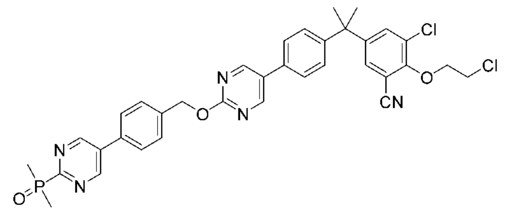

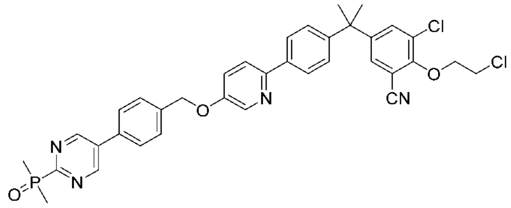

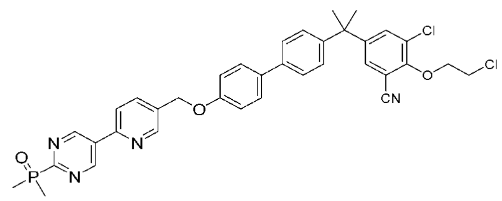

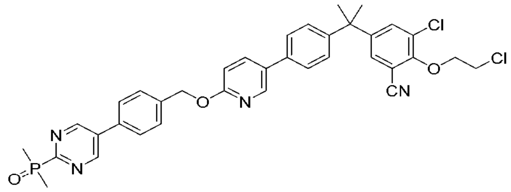

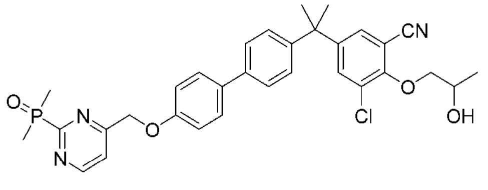

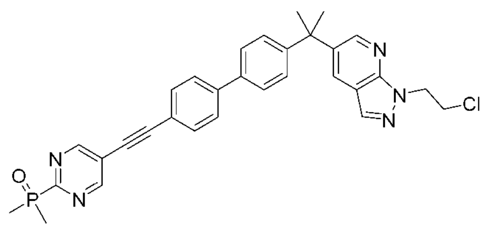

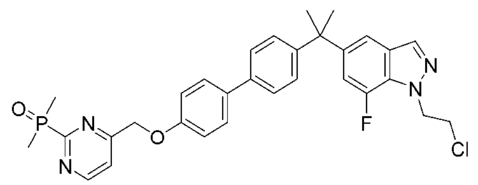

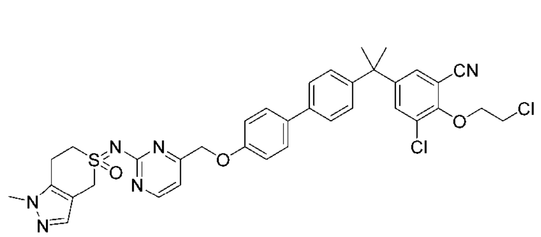

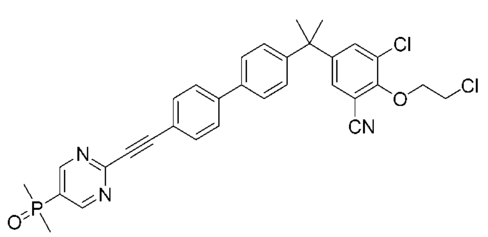

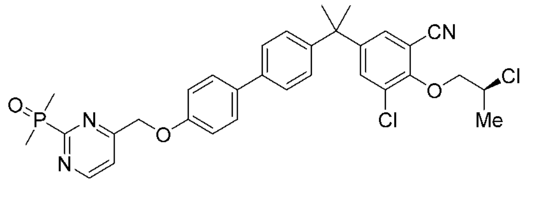

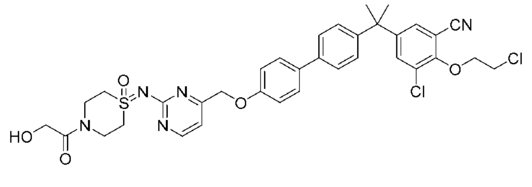

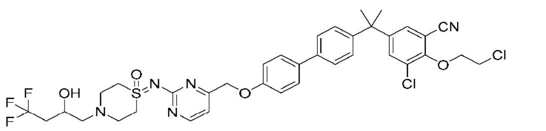

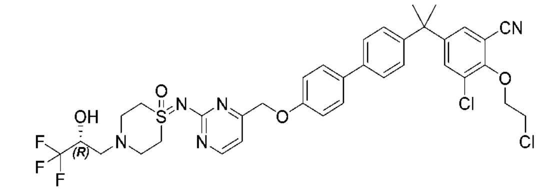

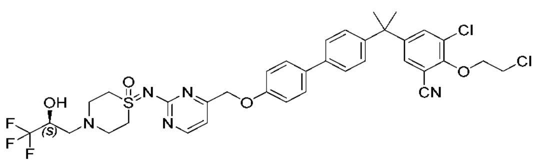

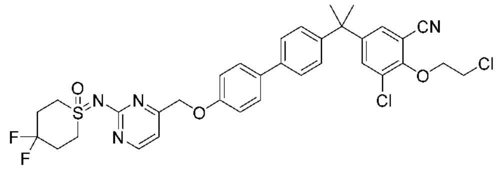

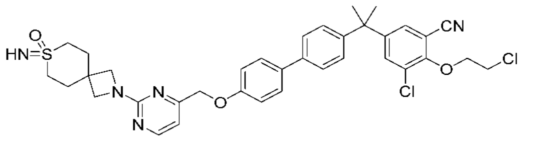

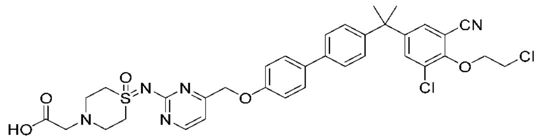

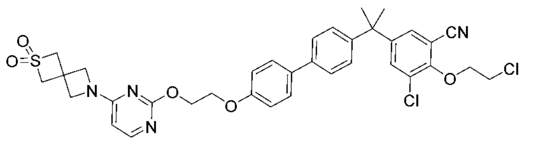

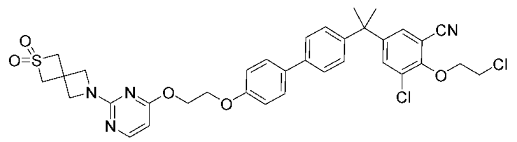

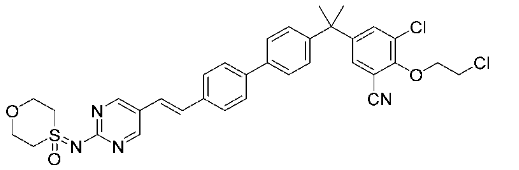

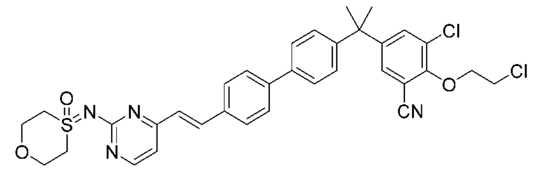

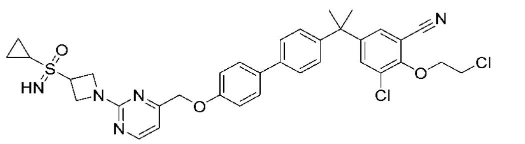

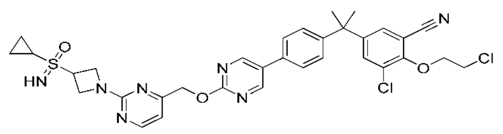

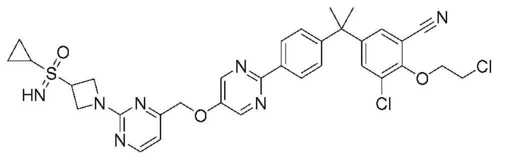

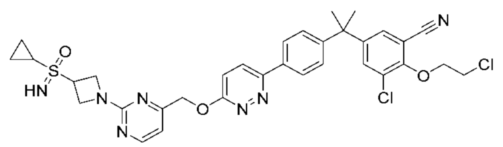

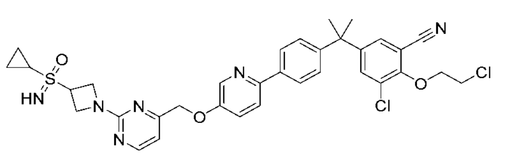

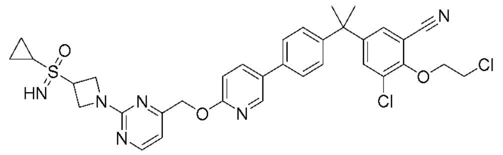

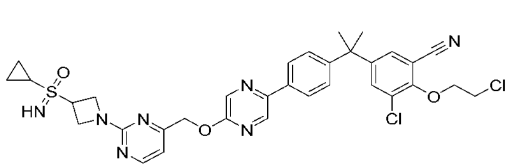

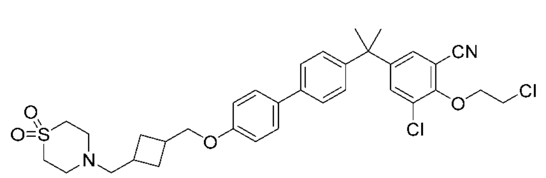

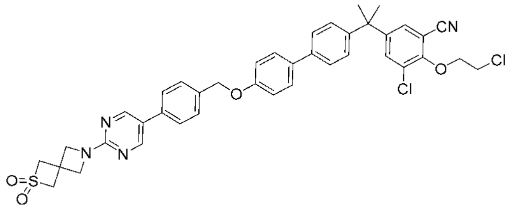

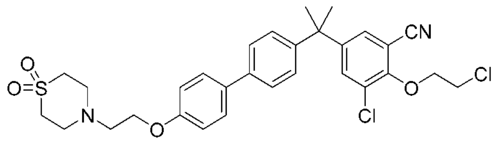

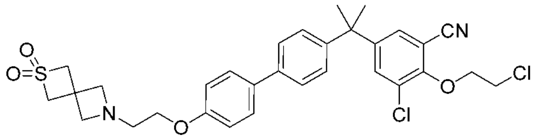

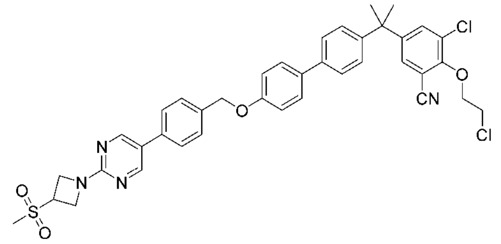

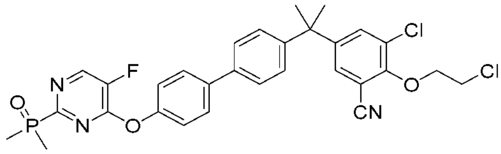

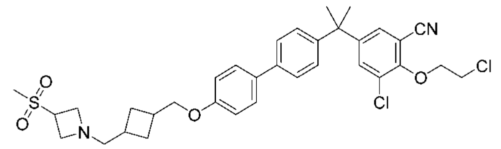

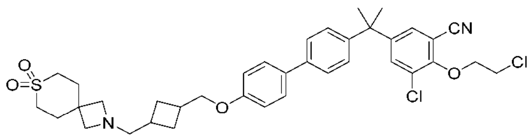

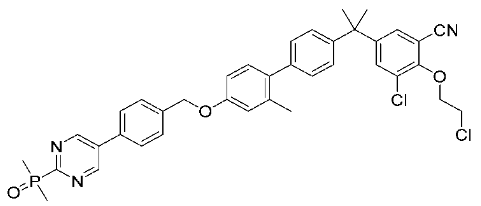

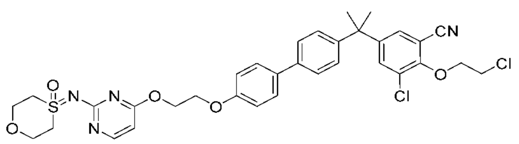

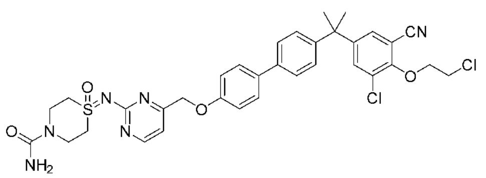

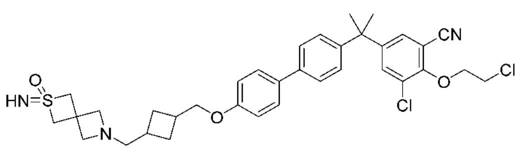

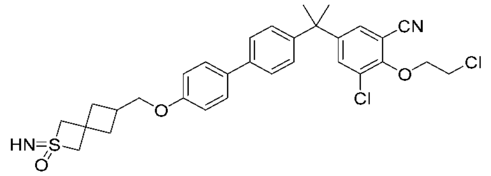

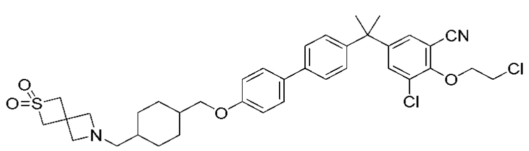

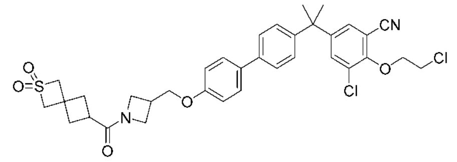

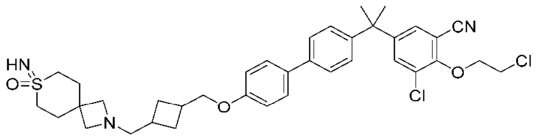

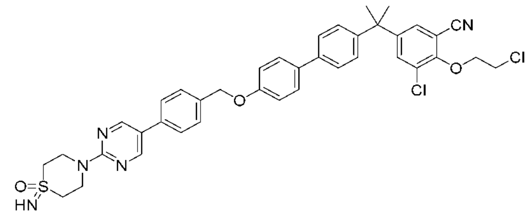

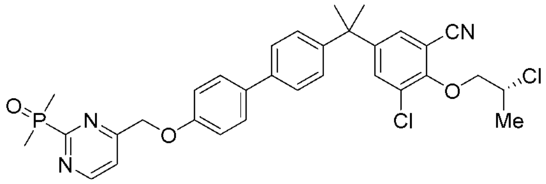

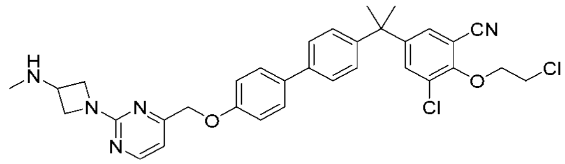

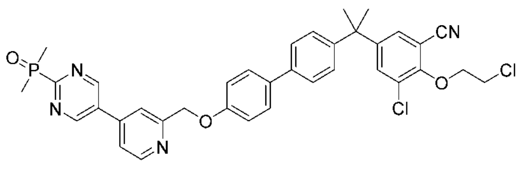

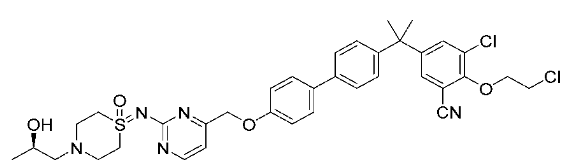

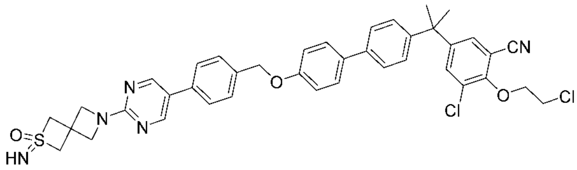

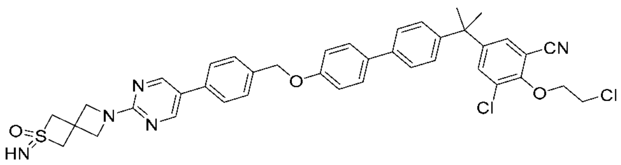

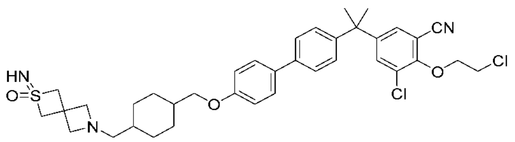

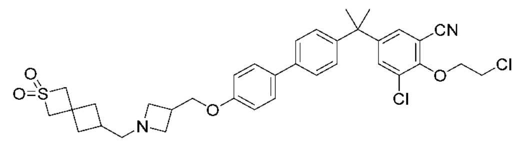

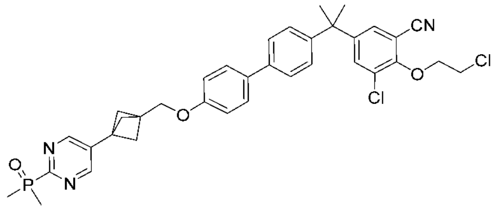

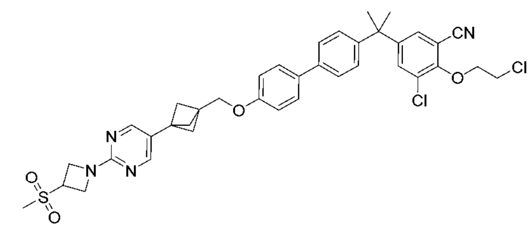

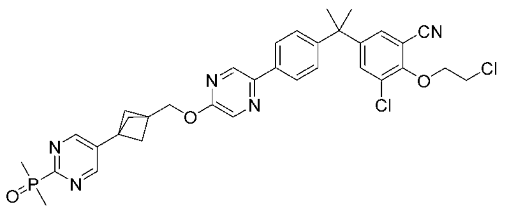

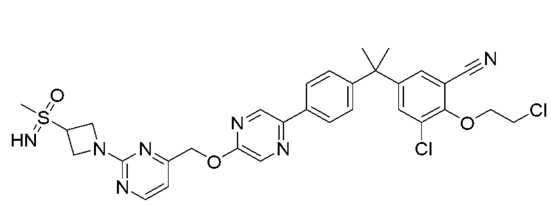

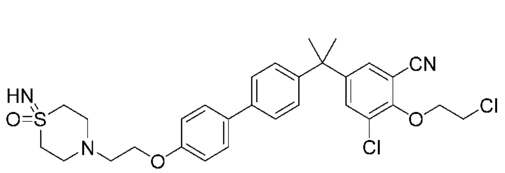

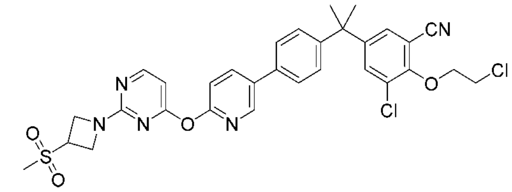

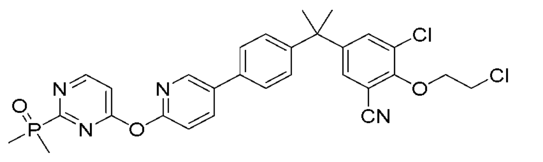

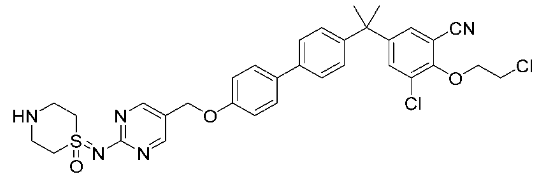

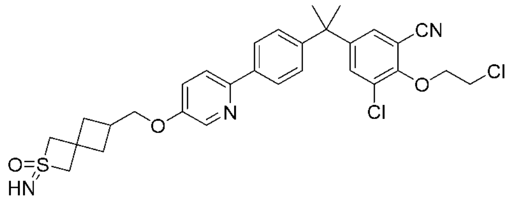

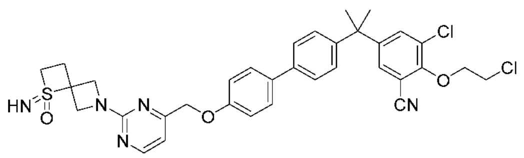

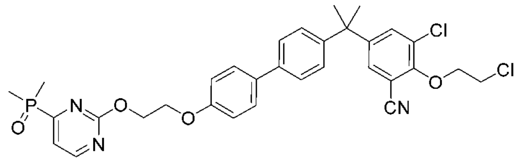

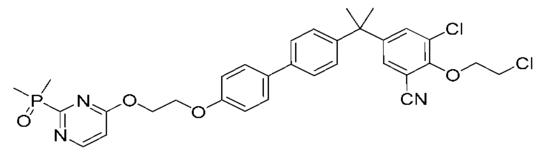

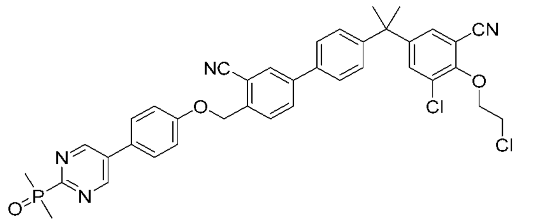

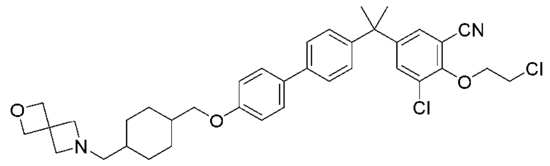

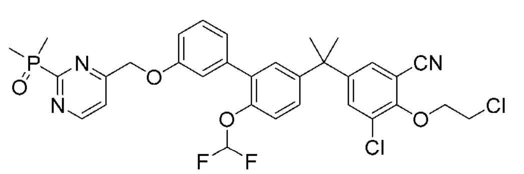

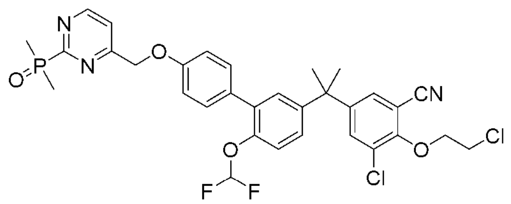

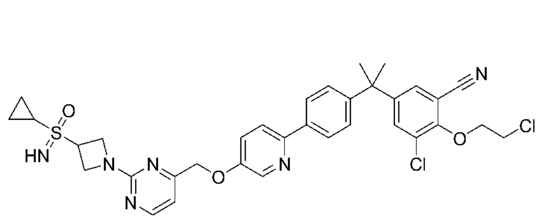

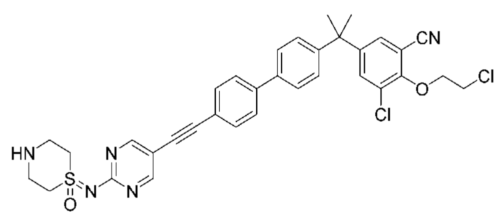

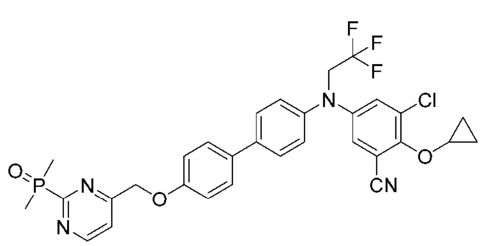

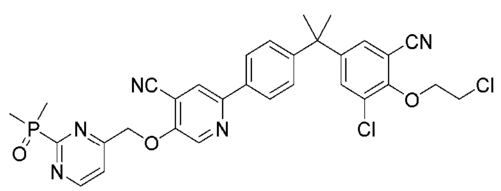

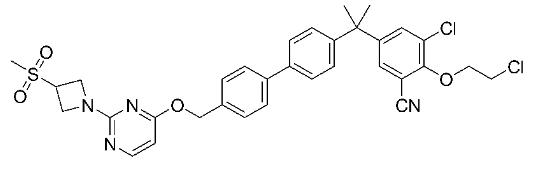

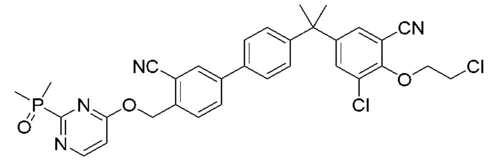

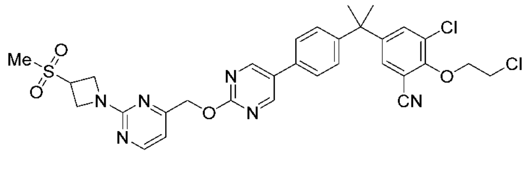

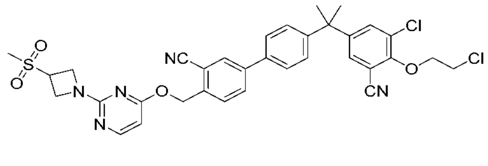

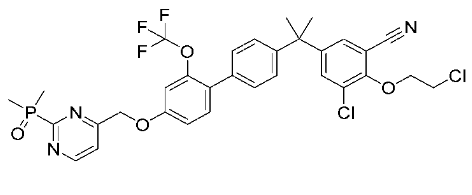

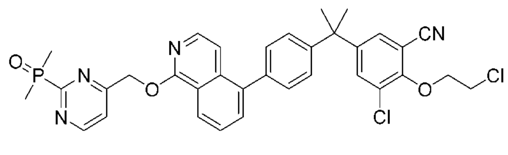

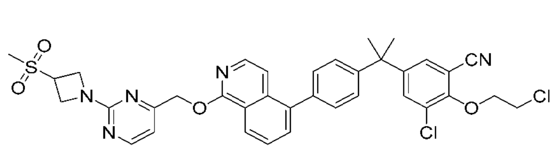

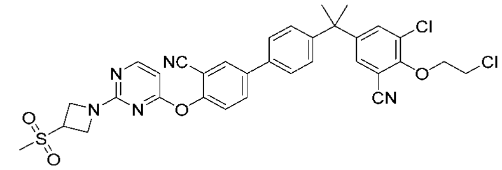

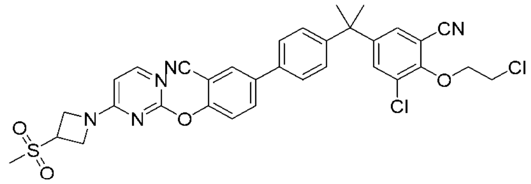

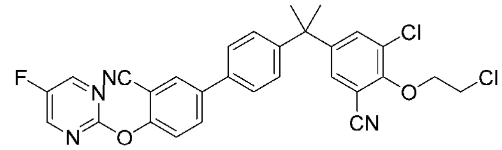

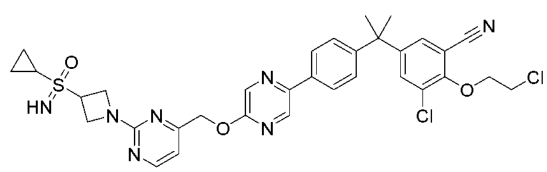

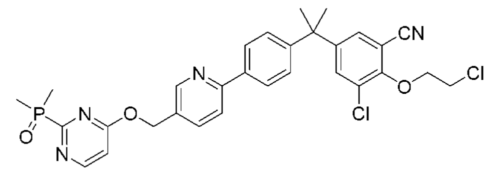

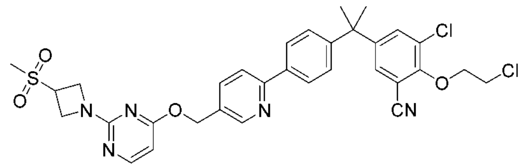

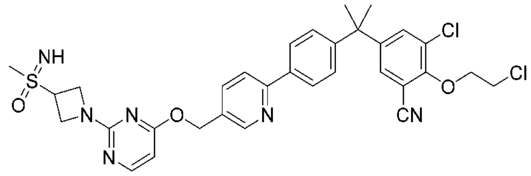

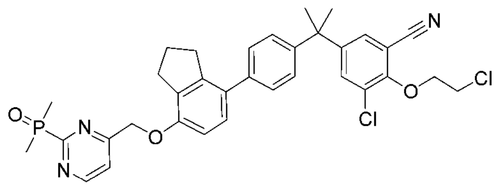

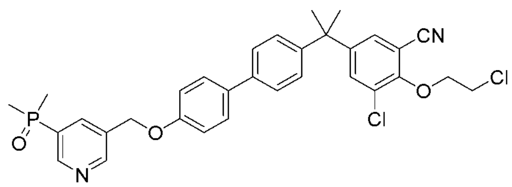

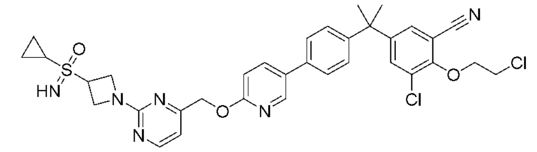

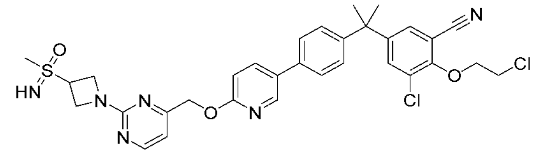

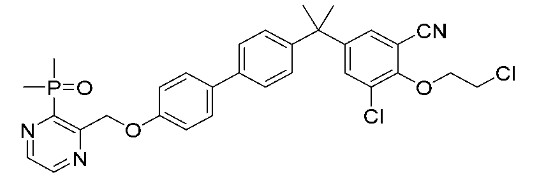

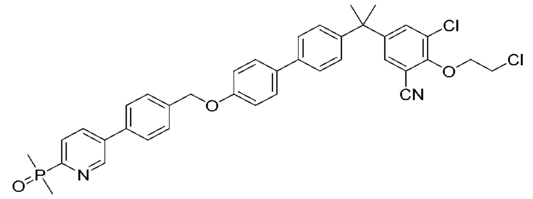

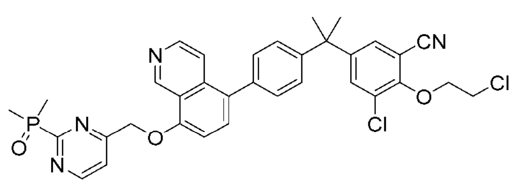

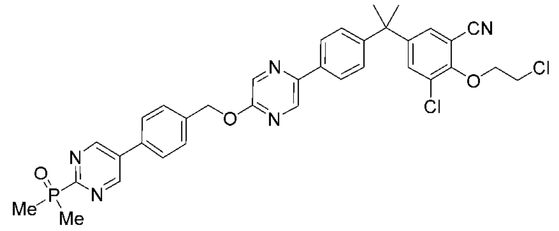

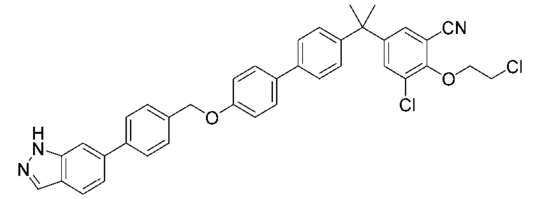

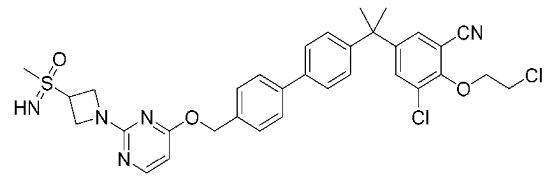

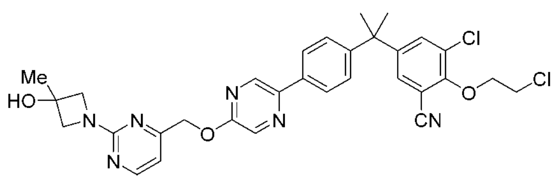

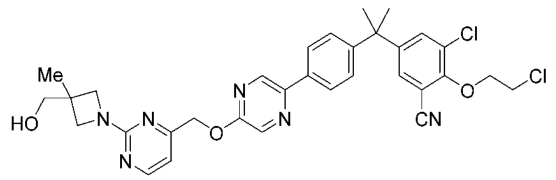

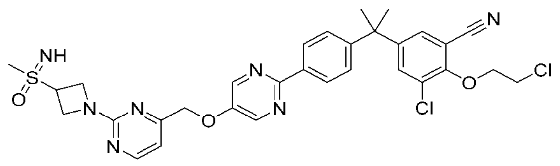

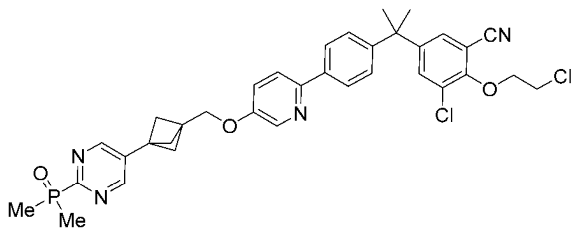

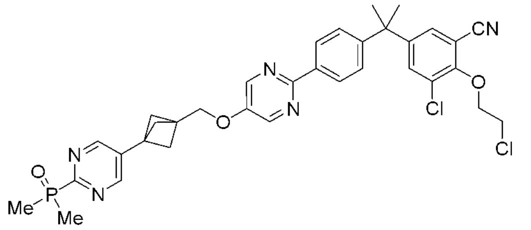

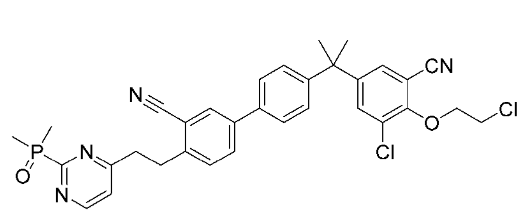

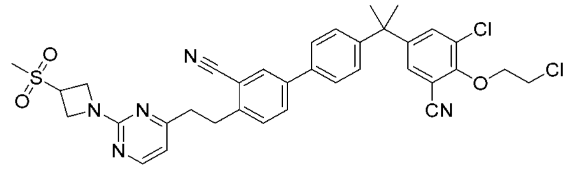

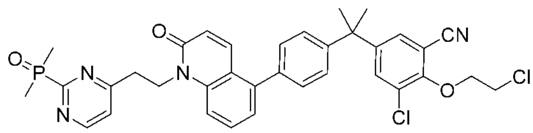

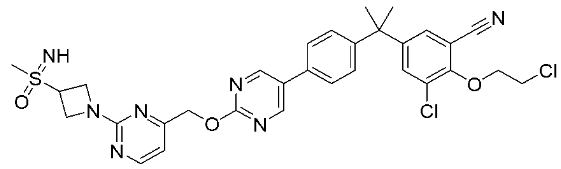

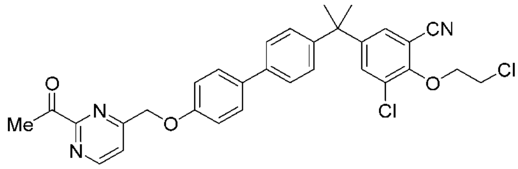

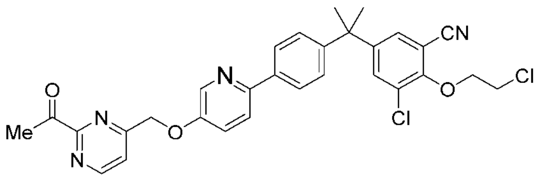

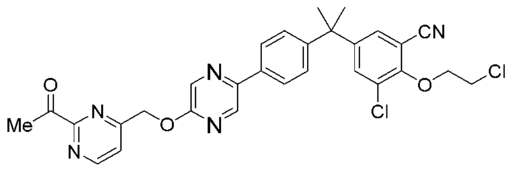

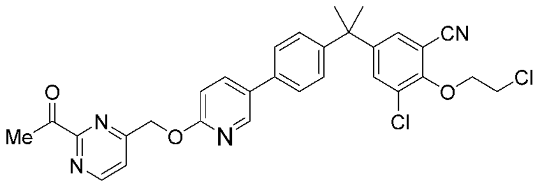

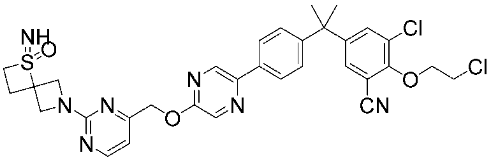

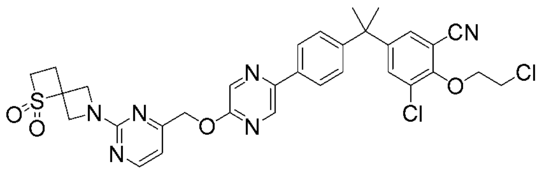

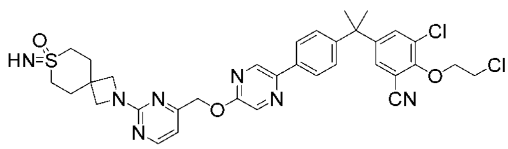

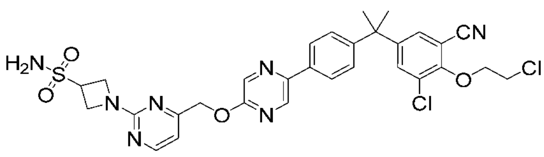

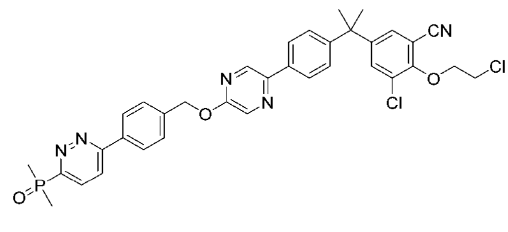

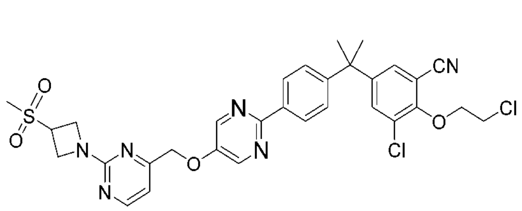

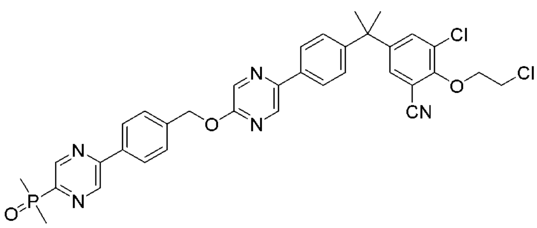

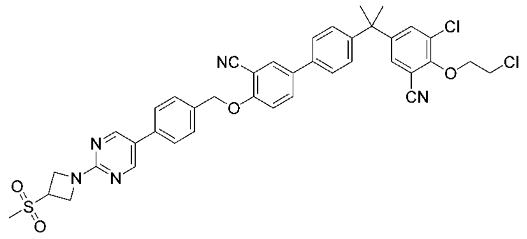

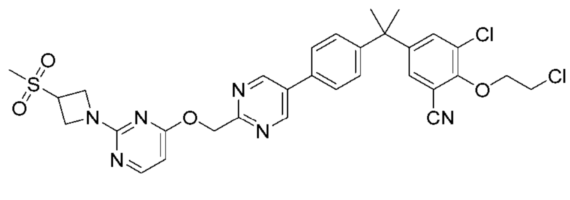

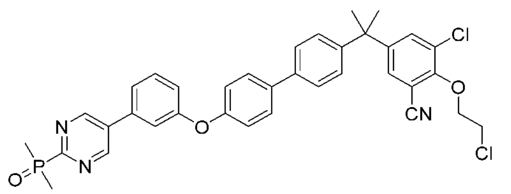

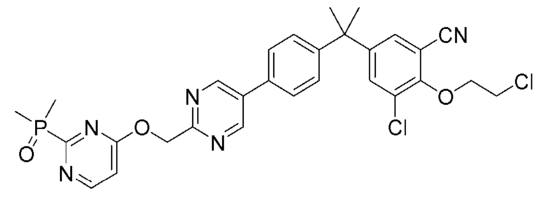

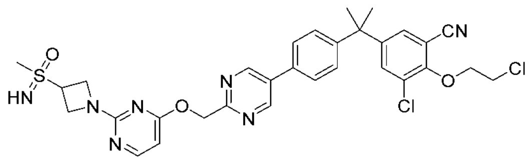

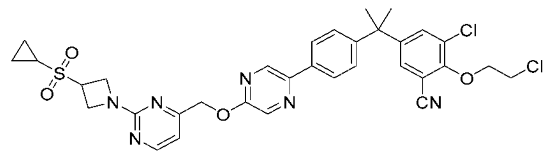

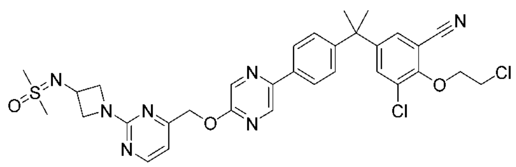

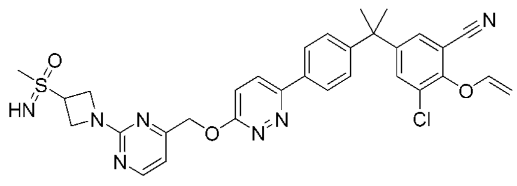

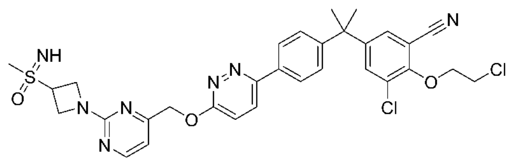

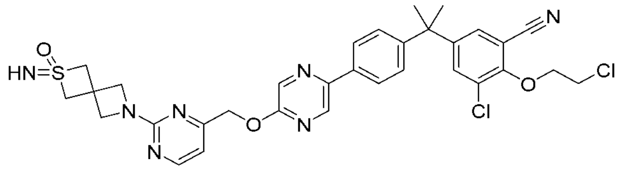

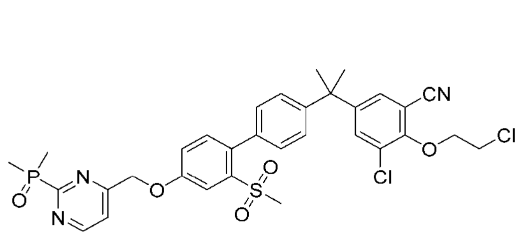

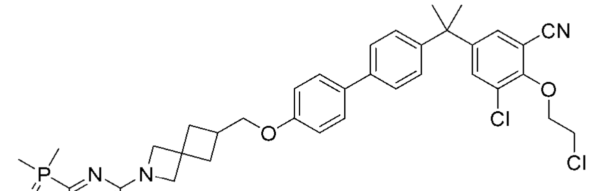

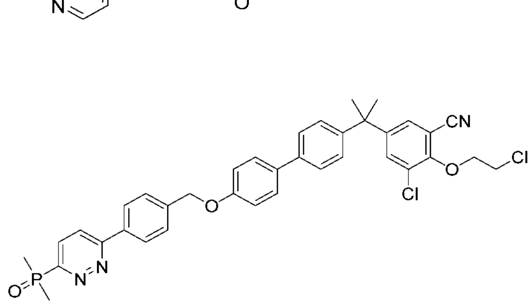

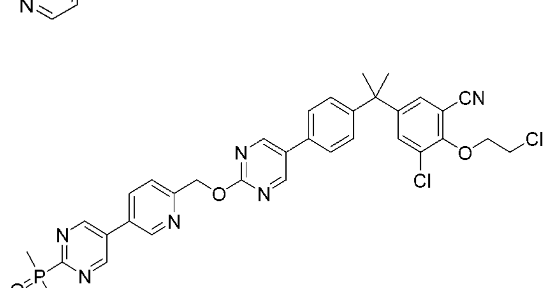

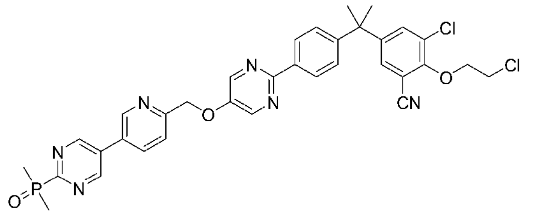

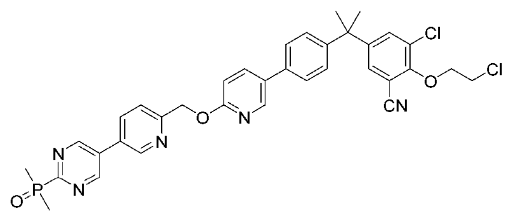

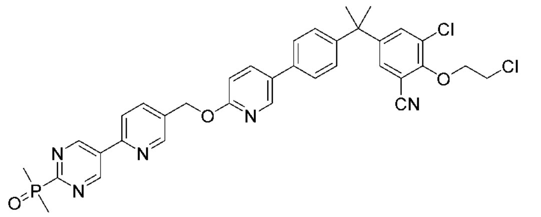

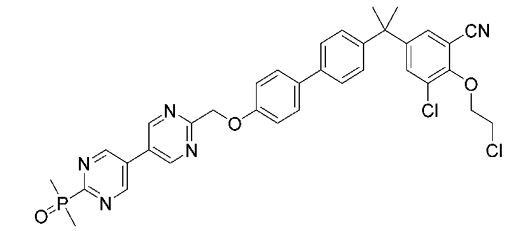

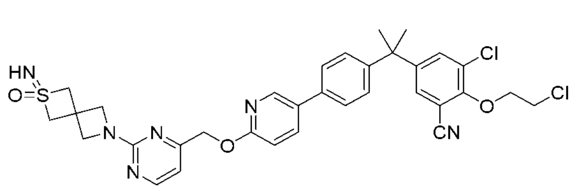

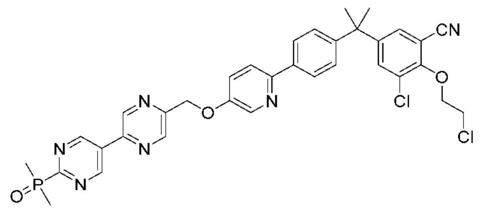

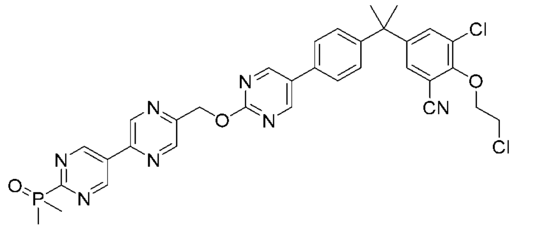

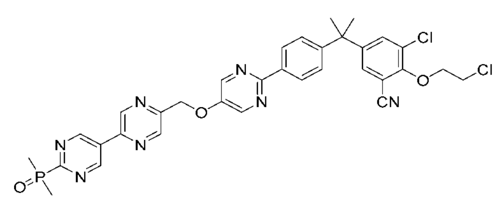

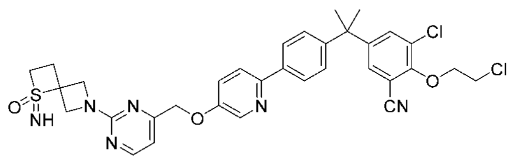

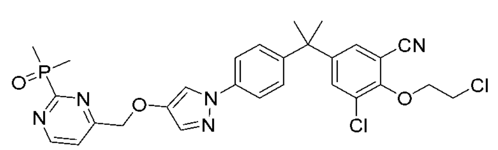

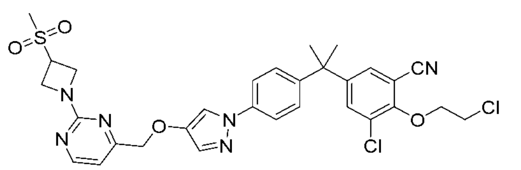

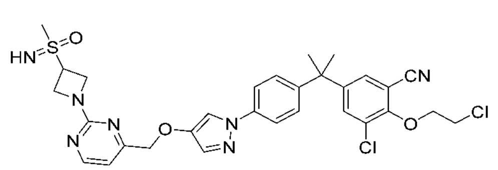

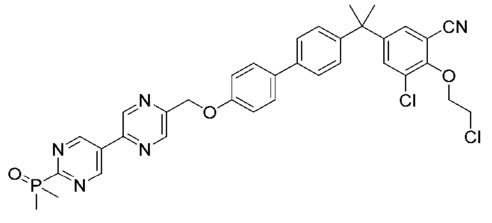

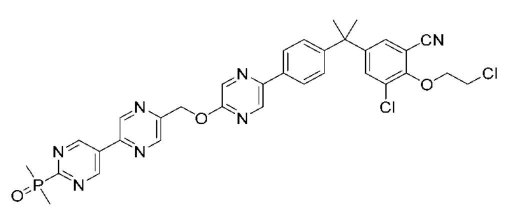

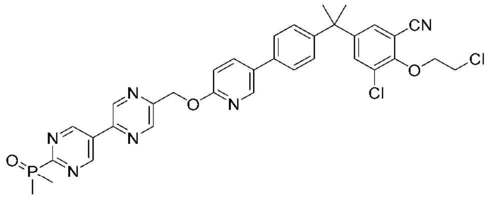

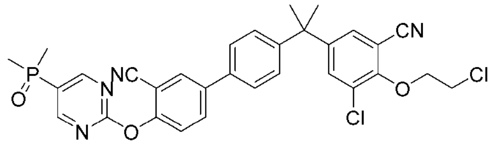

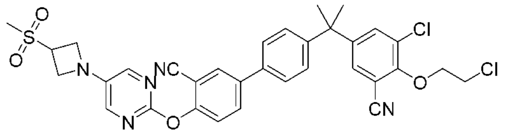

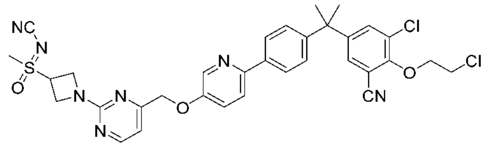

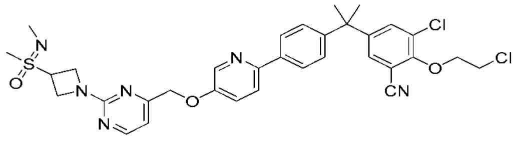

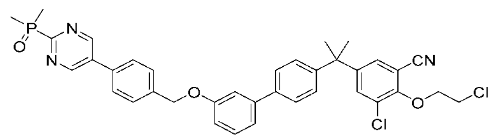

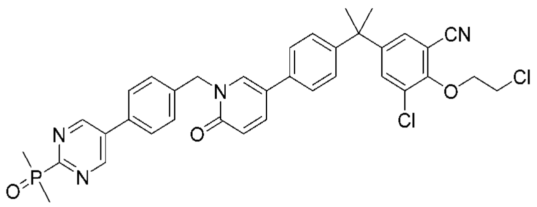

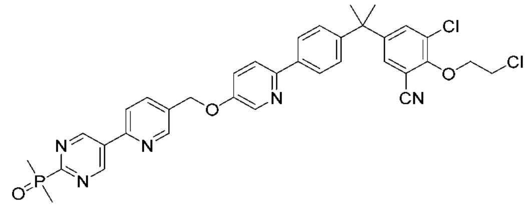

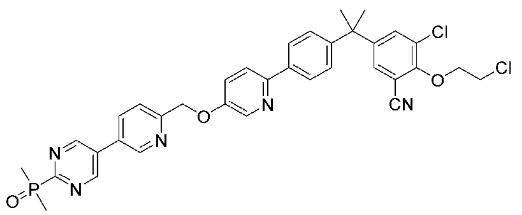

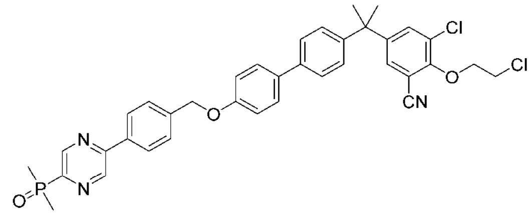

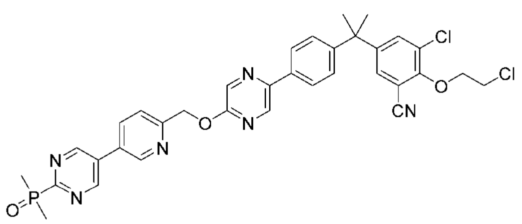

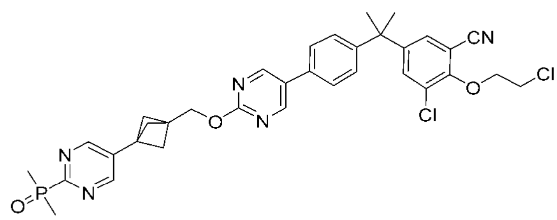

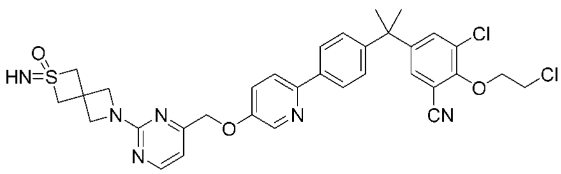

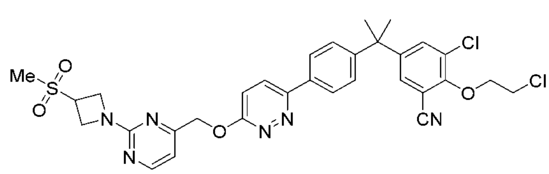

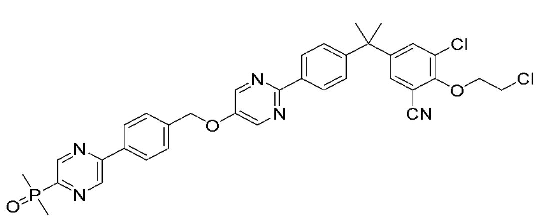

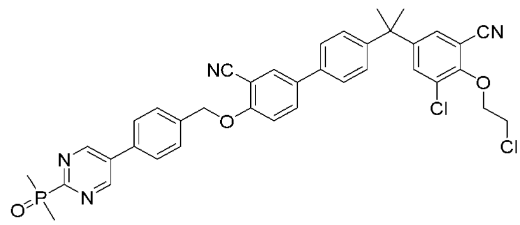

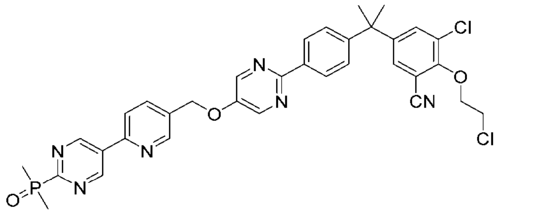
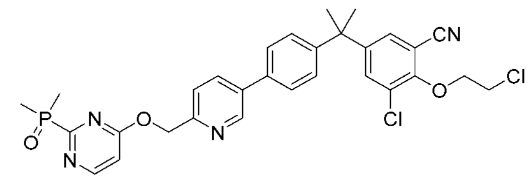

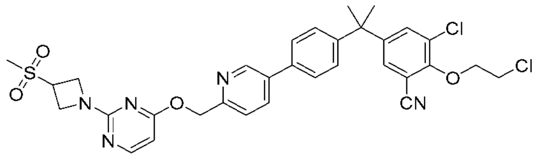
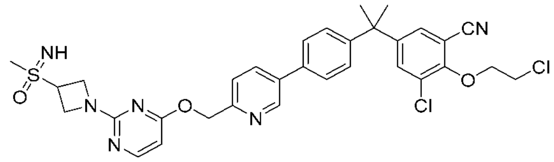

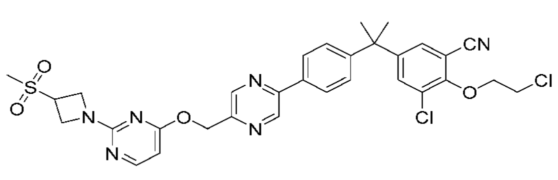
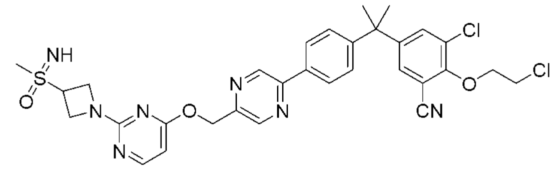

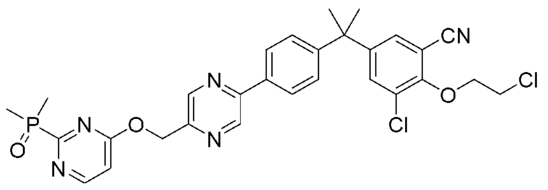
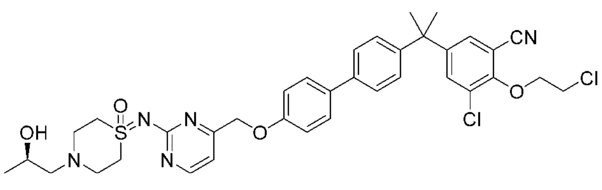

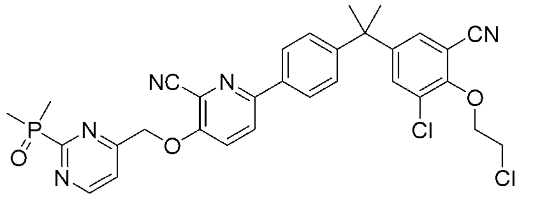
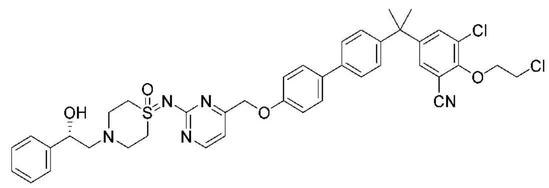

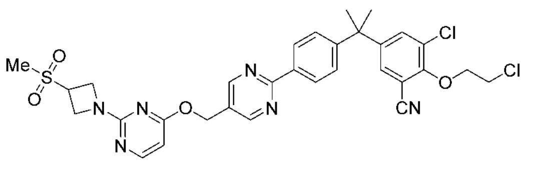
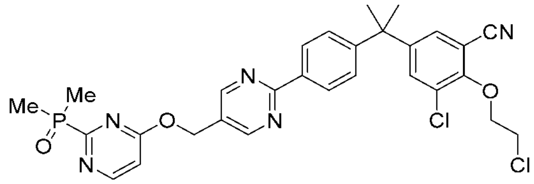

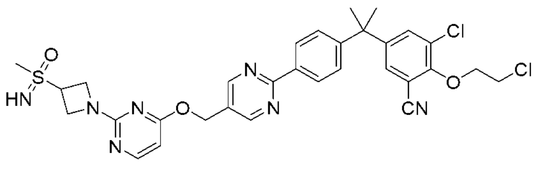
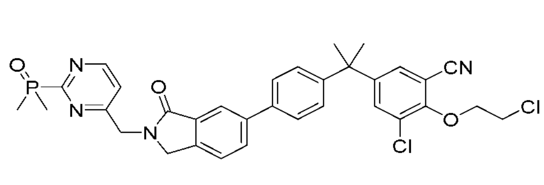

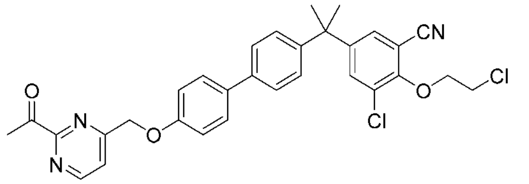
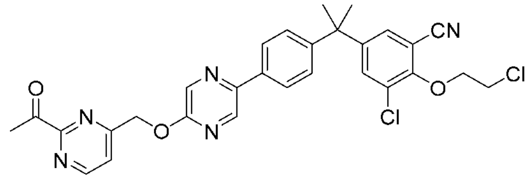

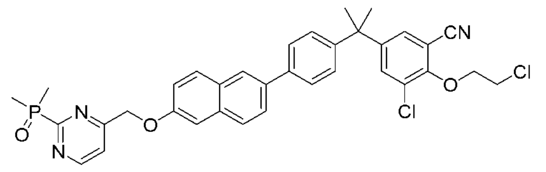

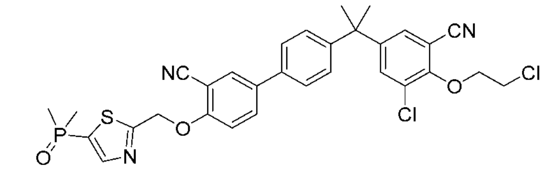

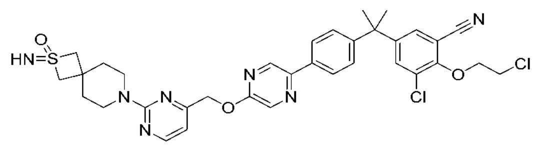

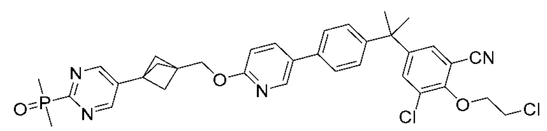

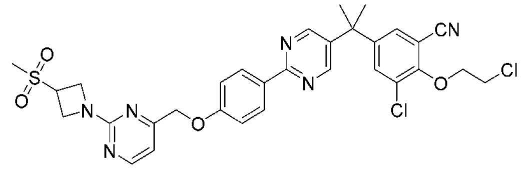

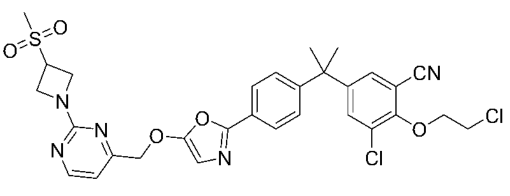

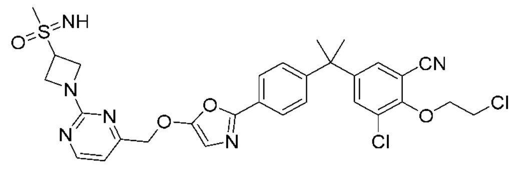

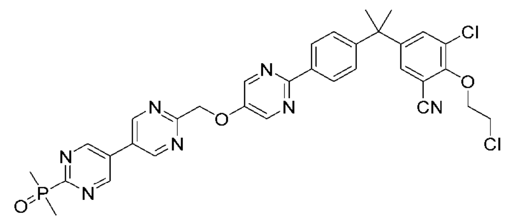

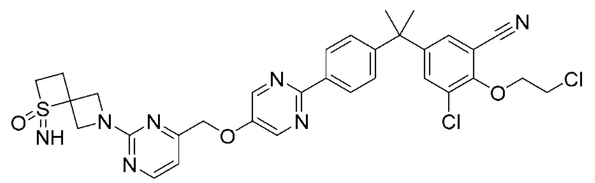

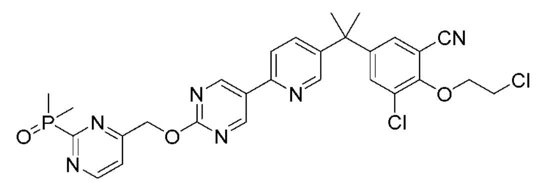

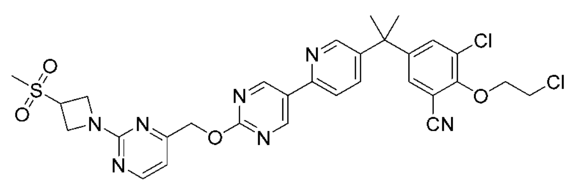

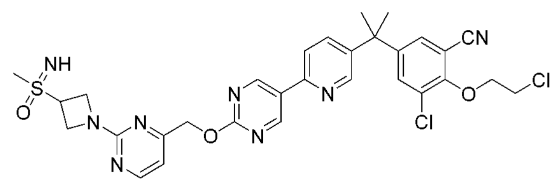

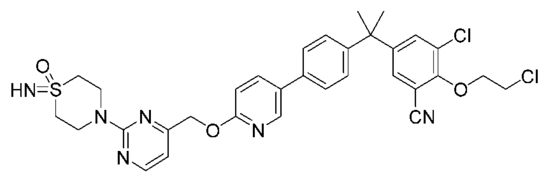

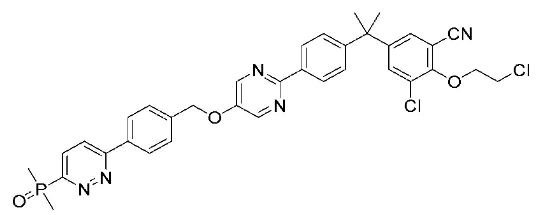

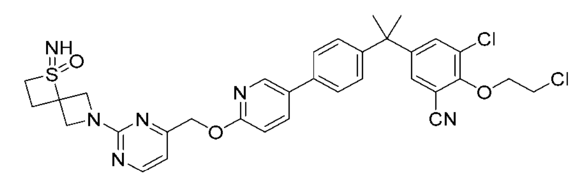
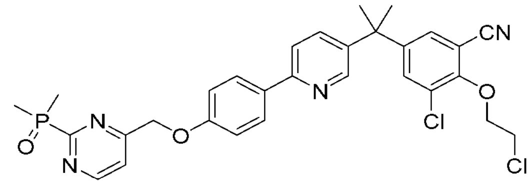

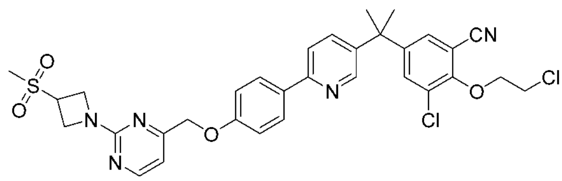
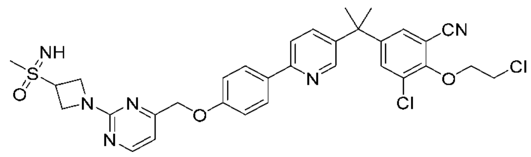

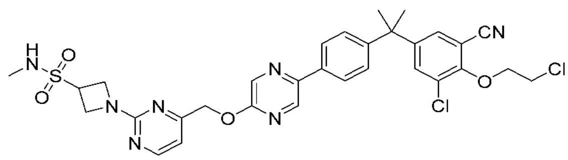
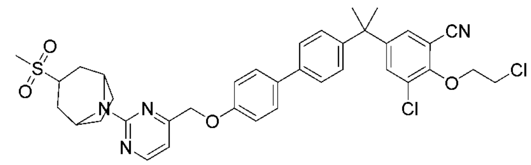

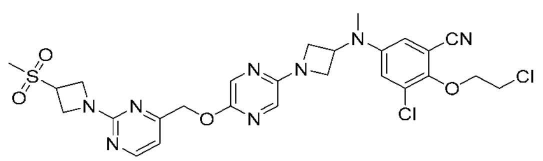
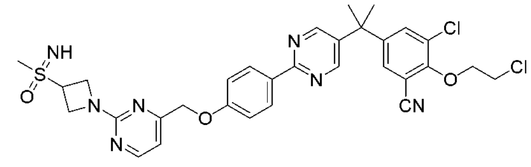

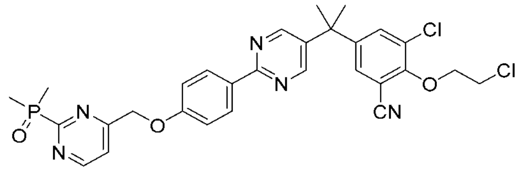
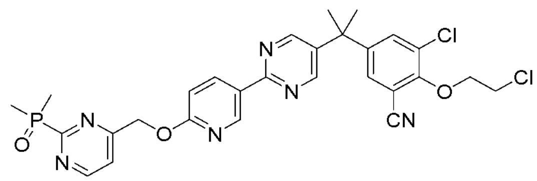

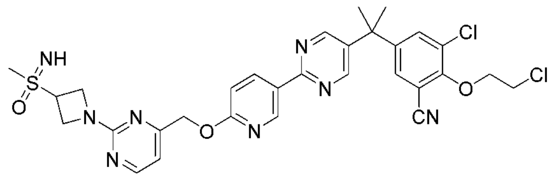
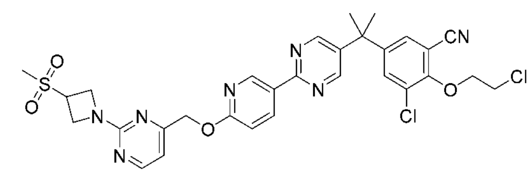

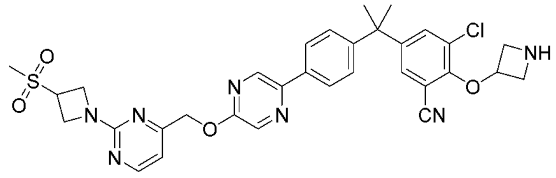
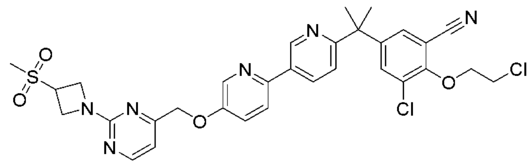

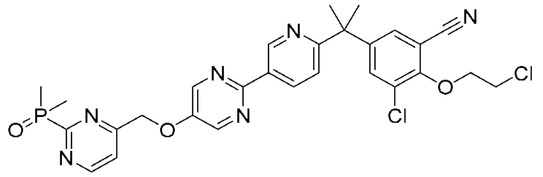
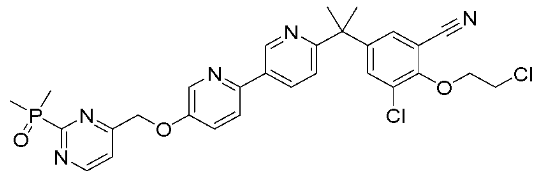

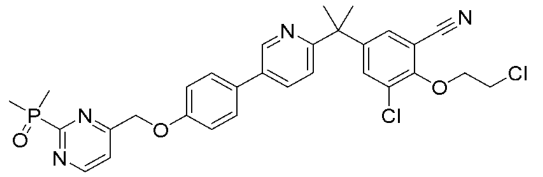

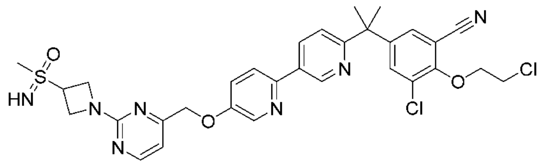

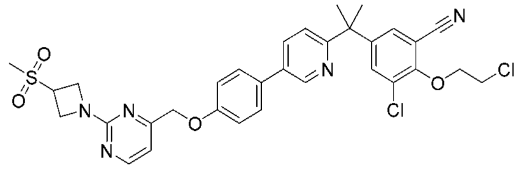

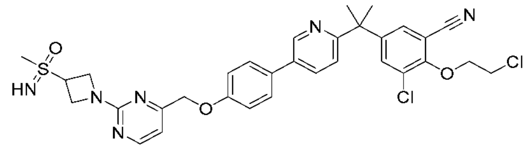

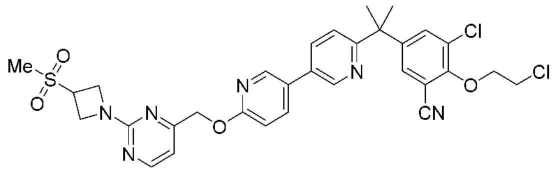

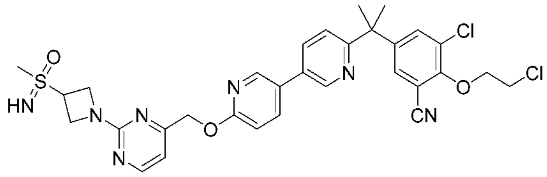

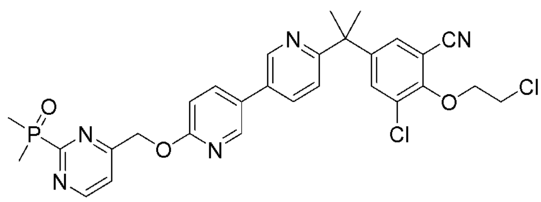

.

**29.** Use of the compound, the optical isomer thereof, or the pharmaceutically acceptable salt thereof according to any one of claims 1-28 in the manufacture of a medicament for treating a disease associated with androgen receptor (AR) activity or expression.

**30.** The disease associated with androgen receptor (AR) activity or expression according to claim 29, selected from the group consisting of prostate cancer, ovarian cancer, breast cancer, bladder cancer, pancreatic cancer, endometrial cancer, hepatocellular carcinoma, renal cell carcinoma, melanoma, mantle cell lymphoma, glioblastoma, salivary gland carcinoma, alopecia, acne, hirsutism, ovarian cysts, polycystic ovary syndrome, precocious puberty, spinal and bulbar muscular atrophy, and age-related macular degeneration.

**INTERNATIONAL SEARCH REPORT**

International application No. **PCT/CN2024/119149**

**A. CLASSIFICATION OF SUBJECT MATTER**

C07D403/02(2006.01)i； C07D205/02(2006.01)i； C07D239/38(2006.01)i； C07D239/42(2006.01)i； C07D239/34(2006.01)i； A61P35/00(2006.01)i； A61K31/506(2006.01)i

According to International Patent Classification (IPC) or to both national classification and IPC

**B. FIELDS SEARCHED**

Minimum documentation searched (classification system followed by classification symbols)

IPC：C07D，A61P，A61K

Documentation searched other than minimum documentation to the extent that such documents are included in the fields searched

Electronic data base consulted during the international search (name of data base and, where practicable, search terms used)

CNTXT, ENTXT, ENTXTC, VEN, CNKI, 万方, WANFANG, 百度学术, BAIDU SCHOLAR, 必应, BING, ISI web of Science, STN: 上海济煜, 邓刚, 黄火明, 林宪峰, 彭建彪, 雄激素受体, 癌, 肿瘤, 前列腺, androgen, cancer, tumor, prostate, 结构式检索, structural formula search.

**C. DOCUMENTS CONSIDERED TO BE RELEVANT**

| Category* | Citation of document, with indication, where appropriate, of the relevant passages | Relevant to claim No. |
|---|---|---|
| PX | WO 2023205507 A1 (ESSA PHARMA INC.) 26 October 2023 (2023-10-26)<br>description, pages 136, 139, and 149, table compounds, and pages 33-38 and 163 | 1-30 |
| PX | WO 2024067783 A1 (SHANGHAI JEMINCARE PHARMACEUTICAL CO., LTD.) 04 April 2024 (2024-04-04)<br>claims 1 and 22-23, and embodiment 45 | 1-30 |
| PX | ACS. "RN 3000473-89-9"<br>*STNEXT Registry,* 08 November 2023 (2023-11-08),<br>page 1 | 1-2, 4-7, 9-20, 22-27 |
| X | WO 2023056423 A1 (ESSA PHARMA INC. et al.) 06 April 2023 (2023-04-06)<br>description, pages 221 and 291-293, and claim 1 | 1-27, 29-30 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 December 2024** | **03 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/119149**

| Patent document cited in search report | Publication date (day/month/year) | Patent family member(s) | Publication date (day/month/year) |
|---|---|---|---|
| WO 2023205507 A1 | 26 October 2023 | US 2023373934 A1 | 23 November 2023 |
| | | AU 2023255575 A1 | 31 October 2024 |
| WO 2024067783 A1 | 04 April 2024 | TW 202428285 A | 16 July 2024 |
| WO 2023056423 A1 | 06 April 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311201930
- CN 202311547952
- CN 202311588615
- CN 202311698673
- CN 202410239612X
- CN 202410546567
- CN 202410734815
- CN 202411040155
- CN 202411238360